# EUROPEAN PATENT APPLICATION

(11) **EP 4 667 054 A2**
(43) Date of publication of application: **24.12.2025**
(21) Application number: 25186277.7
(22) Date of filing: 11.11.2019
(51) Int. Cl.: A61P 35/00

(54) **COMPOUNDS AND COMPOSITIONS FOR TREATING CONDITIONS ASSOCIATED WITH NLRP ACTIVITY**

(30) Priority: 13.11.2018 US 201862760244 P; 13.11.2018 US 201862760209 P; 13.11.2018 US 201862760248 P; 16.11.2018 US 201862768811 P; 19.11.2018 US 201862769151 P; 19.11.2018 US 201862769165 P; 23.01.2019 US 201962795919 P; 24.01.2019 US 201962796356 P; 24.01.2019 US 201962796361 P; 19.04.2019 US 201962836585 P; 19.04.2019 US 201962836577 P; 19.04.2019 US 201962836575 P; 04.09.2019 US 201962895595 P
(62) Divisional of application: 19817831.1
(71) Applicant: Novartis AG, 4056 Basel (CH)
(72) Inventor: GHOSH, Shomir, Boston, 02116 (US); GLICK, Gary, Boston, 02116 (US); KATZ, Jason, Boston, 02116 (US); ROUSH, William, Boston, 02116 (US); SEIDEL, Hans Martin, Boston, 02116 (US); SHEN, Dong-ming, Boston, 02116 (US); VENKATRAMAN, Shankar, Boston, 02116 (US)
(74) Representative: Mueller, Philippe

(57) **Abstract**

In one aspect, compounds of Formula AA, or a pharmaceutically acceptable salt thereof, are featured: 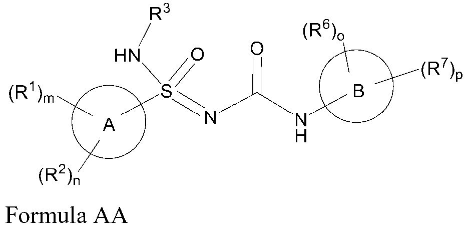 or a pharmaceutically acceptable salt thereof, wherein the variables shown in Formula A can be as defined anywhere herein.

## Description

### TECHNICAL FIELD

This disclosure features chemical entities (e.g., a compound that modulates (e.g., antagonizes) NLRP3, or a pharmaceutically acceptable salt, and/or hydrate, and/or cocrystal, and/or drug combination of the compound) that are useful, e.g., for treating a condition, disease or disorder in which a decrease or increase in NLRP3 activity (e.g., an increase, e.g., a condition, disease or disorder associated with NLRP3 signaling) contributes to the pathology and/or symptoms and/or progression of the condition, disease or disorder in a subject (e.g., a human). This disclosure also features compositions as well as other methods of using and making the same.

The present disclosure also relates to, in part, methods and compositions for treating anti-TNFα resistance in a subject with an NLRP3 antagonist. The present disclosure also relates, in part, to methods, combinations and compositions for treating TFNα related diseases and anti-TNFα resistance in a subject that include administration of an NLRP3 antagonist, an NLRP3 antagonist and an anti-TNFα agent, or a composition encompassing an NLRP3 antagonist and an anti-TNFα agent.

### BACKGROUND

The NLRP3 inflammasome is a component of the inflammatory process and its aberrant activation is pathogenic in inherited disorders such as the cryopyrin associated periodic syndromes (CAPS). The inherited CAPS Muckle-Wells syndrome (MWS), familial cold autoinflammatory syndrome (FCAS) and neonatal onset multi-system inflammatory disease (NOMID) are examples of indications that have been reported to be associated with gain of function mutations in NLRP3.

NLRP3 can form a complex and has been implicated in the pathogenesis of a number of complex diseases, including but not limited to metabolic disorders such as type 2 diabetes, atherosclerosis, obesity and gout, as well as diseases of the central nervous system, such as Alzheimer's disease and multiple sclerosis and Amyotrophic Lateral Sclerosis and Parkinson disease, lung disease, such as asthma and COPD and pulmonary idiopathic fibrosis, liver disease, such as NASH syndrome, viral hepatitis and cirrhosis, pancreatic disease, such as acute and chronic pancreatitis, kidney disease, such as acute and chronic kidney injury, intestinal disease such as Crohn's disease and Ulcerative Colitis, skin disease such as psoriasis, musculoskeletal disease such as scleroderma, vessel disorders, such as giant cell arteritis, disorders of the bones, such as Osteoarthritis , osteoporosis and osteopetrosis disorders eye disease, such as glaucoma and macular degeneration, diseased caused by viral infection such as HIV and AIDS, autoimmune disease such as Rheumatoid Arthritis, Systemic Lupus Erythematosus, Autoimmune Thyroiditis, Addison's disease, pernicious anemia, cancer and aging.

In light of the above, it would be desirable to provide compounds that modulate (e.g., antagonize) NLRP3.

Several patients having inflammatory or autoimmune diseases are treated with anti-TNFα agents. A subpopulation of such patients develop resistance to treatment with the anti-TNFα agents. It is desirable to develop methods for reducing a patient's resistance to anti-TNFα agents. In light of the this, it would also be desirable to provide alternative therapies for treating inflammatory or autoimmune diseases (for example NLRP3 inflammasome inhibitors) to avoid or minimise the use of anti-TNFα agents.

Intestinal bowel disease (IBD), encompassing Ulcerative Colitis (UC) and Crohn's disease (CD), are chronic diseases characterized by barrier dysfunction and uncontrolled inflammation and mucosal immune reactions in the gut. A number of inflammatory pathways have been implicated in the progression of IBD, and anti-inflammatory therapy such as tumor necrosis factor-alpha (TNF-α) blockade has shown efficacy in the clinic (Rutgeerts P et al N Engl J Med 2005; 353:2462-76). Anti-TNFa therapies, however, do not show complete efficacy, however, other cytokines such as IL-1β, IL-6, IL-12, IL-18, IL-21, and IL-23 have been shown to drive inflammatory disease pathology in IBD (Neurath MF Nat Rev Immunol 2014;14;329-42). IL-1β and IL-18 are produced by the NLRP3 inflammasome in response to pathogenic danger signals, and have been shown to play a role in IBD. Anti-IL-1β therapy is efficacious in patients with IBD driven by genetic mutations in CARD8 or IL-10R (Mao L et al, J Clin Invest 2018;238:1793-1806*,* Shouval DS et al, Gastroenterology 2016;151:1100-1104)*,* IL-18 genetic polymorphisms have been linked to UC (Kanai T et al, Curr Drug Targets 2013;14:1392-9), and NLRP3 inflammasome inhibitors have been shown to be efficacious in murine models of IBD (Perera AP et al, Sci Rep 2018;8:8618)*.* Resident gut immune cells isolated from the lamina propria of IBD patients can produce IL-1β, either spontaneously or when stimulated by LPS, and this IL-1β production can be blocked by the ex vivo addition of a NLRP3 antagonist. Based on strong clinical and preclinical evidence showing that inflammasome-driven IL-1β and IL-18 play a role in IBD pathology, it is clear that NLRP3 inflammasome inhibitors could be an efficacious treatment option for UC, Crohn's disease, or subsets of IBD patients. These subsets of patients could be defined by their peripheral or gut levels of inflammasome related cytokines including IL-1β, IL-6, and IL-18, by genetic factors that pre-dispose IBD patients to having NLRP3 inflammasome activation such as mutations in genes including ATG16L1, CARD8, IL-10R, or PTPN2 (Saitoh T et al, Nature 2008;456:264*,* Spalinger MR, Cell Rep 2018;22:1835), or by other clinical rationale such as non-response to TNF therapy.

Though anti-TNF therapy is an effective treatment option for Crohn's disease, 40% of patients fail to respond. One-third of non-responsive CD patients fail to respond to anti-TNF therapy at the onset of treatment, while another third lose response to treatment over time (secondary non-response). Secondary non-response can be due to the generation of anti-drug antibodies, or a change in the immune compartment that desensitizes the patient to anti-TNF (Ben-Horin S et al, Autoimmun Rev 2014;13:24-30*,* Steenholdt C et al Gut 2014;63:919-27). Anti-TNF reduces inflammation in IBD by causing pathogenic T cell apoptosis in the intestine, therefore eliminating the T cell mediated inflammatory response (Van den Brande et al Gut 2007:56:509-17). There is increased NLRP3 expression and increased production of IL-1β in the gut of TNF-non-responsive CD patients (Leal RF et al Gut 2015;64:233-42) compared to TNF-responsive patients, suggesting NLRP3 inflammasome pathway activation. Furthermore, there is increased expression of TNF-receptor 2 (TNF-R2), which allows for TNF-mediated proliferation of T cells (Schmitt H et al Gut 2018;0:1-15)*.* IL-1β signaling in the gut promotes T cell differentiation toward Th1/17 cells which can escape anti-TNF-α mediated apoptosis. It is therefore likely that NLRP3 inflammasome activation can cause non-responsiveness in CD patients to anti-TNF-α therapy by sensitizing pathogenic T cells in the gut to anti-TNF-α mediated apoptosis. Experimental data from immune cells isolated from the gut of TNF-resistant Crohn's patients show that these cells spontaneously release IL-1β, which can be inhibited by the addition of an NLRP3 antagonist. NLRP3 inflammasome antagonists - in part by blocking IL-1β secretion - would be expected to inhibit the mechanism leading to anti-TNF non-responsiveness, re-sensitizing the patient to anti-TNF therapy. In IBD patients who are naive to anti-TNF therapy, treatment with an NLRP3 antagonist would be expected to prevent primary- and secondary-non responsiveness by blocking the mechanism leading to non-response.

NLRP3 antagonists that are efficacious locally in the gut can be efficacious drugs to treat IBD; in particular in the treatment of TNF-resistant CD alone or in combination with anti-TNF therapy. Systemic inhibition of both IL-1β and TNF-α has been shown to increase the risk of opportunistic infections (Genovese MC et al, Arthritis Rheum 2004;50:1412), therefore, only blocking the NLRP3 inflammasome at the site of inflammation would reduce the infection risk inherent in neutralizing both IL-1β and TNF-α. NLRP3 antagonists that are potent in NLRP3-inflammasome driven cytokine secretion assays in cells, but have low permeability in vitro in a permeability assay such as an MDCK assay, have poor systemic bioavailability in a rat or mouse pharmacokinetic experiment, but high levels of compound in the colon and/or small intestine could be a useful therapeutic option for gut restricted purposes.

The present invention also provides alternative therapies for the treatment of inflammatory or autoimmune diseases, including IBD, that solves the above problems associated with anti-TNFα agents.

### SUMMARY

This disclosure features chemical entities (e.g., a compound that modulates (e.g., antagonizes) NLRP3, or a pharmaceutically acceptable salt, and/or hydrate, and/or cocrystal, and/or drug combination of the compound) that are useful, e.g., for treating a condition, disease or disorder in which a decrease or increase in NLRP3 activity (e.g., an increase, e.g., a condition, disease or disorder associated with NLRP3 signaling).

In some embodiments, provided herein is a compound of Formula AA 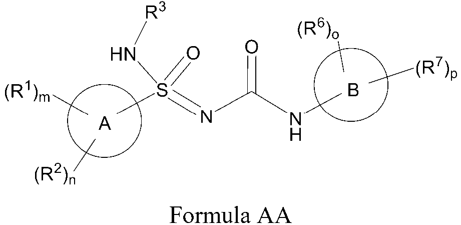 or a pharmaceutically acceptable salt thereof, wherein the variables in Formula AA can be as defined anywhere herein.

This disclosure also features compositions as well as other methods of using and making the same.

The present invention is also relates to the Applicant's discovery that inhibition of NLRP3 inflammasomes can increase a subject's sensitivity to an anti-TNFα agent or can overcome resistance to an anti-TNFα agent in a subject, or indeed provide an alternative therapy to anti-TNFα agents.

Provided herein are methods of treating a subject that include: (a) identifying a subject having a cell that has an elevated level of NLRP3 inflammasome activity and/or expression as compared to a reference level; and (b) administering to the identified subject a therapeutically effective amount of an compound of Formula I or a pharmaceutically acceptable salt, solvate, or co-crystal thereof.

Provided herein are methods for the treatment of inflammatory or autoimmune disease including IBD, such as UC and CD in a subject in need thereof, comprising administering to said subject a therapeutically effective amount a compound for Formula I or a pharmaceutically acceptable salt, solvate, or co-crystal thereof, wherein the NLRP3 antagonist is a gut-targeted NLRP3 antagonist.

Provided herein are methods of treating a subject in need thereof, that include: (a) identifying a subject having resistance to an anti-TNFα agent; and (b) administering a treatment comprising a therapeutically effective amount of a compound for Formula I, or a pharmaceutically acceptable salt, solvate, or co-crystal thereof to the identified subject.

Provided herein are methods of treating a subject in need thereof, that include: administering a treatment comprising a therapeutically effective amount of a compound for Formula I or a pharmaceutically acceptable salt, solvate, or co-crystal thereof to a subject identified as having resistance to an anti-TNFα agent.

Provided herein are methods of selecting a treatment for a subject in need thereof, that include: (a) identifying a subject having resistance to an anti-TNFα agent; and (b) selecting for the identified subject a treatment comprising a therapeutically effective amount of a compound for Formula I or a pharmaceutically acceptable salt, solvate, or co-crystal thereof.

Provided herein are methods of selecting a treatment for a subject in need thereof, that include selecting a treatment comprising a therapeutically effective amount of a compound for Formula I or a pharmaceutically acceptable salt, solvate, or co-crystal thereof for a subject identified as having resistance to an anti-TNFα agent.

In some embodiments of any of the methods described herein, the treatment further includes a therapeutically effective amount of an anti-TNFα agent, in addition to the NLRP3 antagonist.

An "antagonist" of NLRP3 includes compounds that inhibit the ability of NLRP3 to induce the production of IL-1β and/or IL-18 by directly binding to NLRP3, or by inactivating, destabilizing, altering distribution, of NLRP3 or otherwise.

In one aspect, pharmaceutical compositions are featured that include a chemical entity described herein (e.g., a compound described generically or specifically herein or a pharmaceutically acceptable salt thereof or compositions containing the same) and one or more pharmaceutically acceptable excipients.

In one aspect, methods for modulating (e.g., agonizing, partially agonizing, antagonizing) NLRP3 activity are featured that include contacting NLRP3 with a chemical entity described herein (e.g., a compound described generically or specifically herein or a pharmaceutically acceptable salt thereof or compositions containing the same). Methods include *in vitro* methods, e.g., contacting a sample that includes one or more cells comprising NLRP3, as well as *in vivo* methods.

In a further aspect, methods of treatment of a disease in which NLRP3 signaling contributes to the pathology and/or symptoms and/or progression of the disease are featured that include administering to a subject in need of such treatment an effective amount of a chemical entity described herein (e.g., a compound described generically or specifically herein or a pharmaceutically acceptable salt thereof or compositions containing the same).

In a further aspect, methods of treatment are featured that include administering to a subject a chemical entity described herein (e.g., a compound described generically or specifically herein or a pharmaceutically acceptable salt thereof or compositions containing the same), wherein the chemical entity is administered in an amount effective to treat a disease in which NLRP3 signaling contributes to the pathology and/or symptoms and/or progression of the disease, thereby treating the disease.

Embodiments can include one or more of the following features.

The chemical entity can be administered in combination with one or more additional therapies with one or more agents suitable for the treatment of the condition, disease or disorder.

Examples of the indications that may be treated by the compounds disclosed herein include but are not limited to metabolic disorders such as type 2 diabetes, atherosclerosis, obesity and gout, as well as diseases of the central nervous system, such as Alzheimer's disease and multiple sclerosis and Amyotrophic Lateral Sclerosis and Parkinson disease, lung disease, such as asthma and COPD and pulmonary idiopathic fibrosis, liver disease, such as NASH syndrome, viral hepatitis and cirrhosis, pancreatic disease, such as acute and chronic pancreatitis, kidney disease, such as acute and chronic kidney injury, intestinal disease such as Crohn's disease and Ulcerative Colitis, skin disease such as psoriasis, musculoskeletal disease such as scleroderma, vessel disorders, such as giant cell arteritis, disorders of the bones, such as osteoarthritis , osteoporosis and osteopetrosis disorders, eye disease, such as glaucoma and macular degeneration, diseases caused by viral infection such as HIV and AIDS, autoimmune disease such as rheumatoid arthritis, systemic Lupus erythematosus, autoimmune thyroiditis; Addison's disease, pernicious anemia, cancer and aging.

The methods can further include identifying the subject.

Other embodiments include those described in the Detailed Description and/or in the claims.

### Additional Definitions

To facilitate understanding of the disclosure set forth herein, a number of additional terms are defined below. Generally, the nomenclature used herein and the laboratory procedures in organic chemistry, medicinal chemistry, and pharmacology described herein are those well-known and commonly employed in the art. Unless defined otherwise, all technical and scientific terms used herein generally have the same meaning as commonly understood by one of ordinary skill in the art to which this disclosure belongs. Each of the patents, applications, published applications, and other publications that are mentioned throughout the specification and the attached appendices are incorporated herein by reference in their entireties.

As used herein, the term "NLRP3" is meant to include, without limitation, nucleic acids, polynucleotides, oligonucleotides, sense and antisense polynucleotide strands, complementary sequences, peptides, polypeptides, proteins, homologous and/or orthologous NLRP3 molecules, isoforms, precursors, mutants, variants, derivatives, splice variants, alleles, different species, and active fragments thereof.

The term "acceptable" with respect to a formulation, composition or ingredient, as used herein, means having no persistent detrimental effect on the general health of the subject being treated.

"API" refers to an active pharmaceutical ingredient.

The terms "effective amount" or "therapeutically effective amount," as used herein, refer to a sufficient amount of a chemical entity (e.g., a compound exhibiting activity as a modulator of NLRP3, or a pharmaceutically acceptable salt and/or hydrate and/or cocrystal thereof;) being administered which will relieve to some extent one or more of the symptoms of the disease or condition being treated. The result includes reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case is determined using any suitable technique, such as a dose escalation study.

The term "excipient" or "pharmaceutically acceptable excipient" means a pharmaceutically-acceptable material, composition, or vehicle, such as a liquid or solid filler, diluent, carrier, solvent, or encapsulating material. In one embodiment, each component is " pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. *See, e.g.,* Remington: The Science and Practice of Pharmacy, 21st ed.; Lippincott Williams & Wilkins: Philadelphia, PA, 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, FL, 2009.

The term "pharmaceutically acceptable salt" may refer to pharmaceutically acceptable addition salts prepared from pharmaceutically acceptable non-toxic acids including inorganic and organic acids. In certain instances, pharmaceutically acceptable salts are obtained by reacting a compound described herein, with acids such as hydrochloric acid, hydrobromic acid, sulfuric acid, nitric acid, phosphoric acid, methanesulfonic acid, ethanesulfonic acid, p-toluenesulfonic acid, salicylic acid and the like. The term "pharmaceutically acceptable salt" may also refer to pharmaceutically acceptable addition salts prepared by reacting a compound having an acidic group with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, and salts with amino acids such as arginine, lysine, and the like, or by other methods previously determined. The pharmacologically acceptable salt s not specifically limited as far as it can be used in medicaments. Examples of a salt that the compounds described hereinform with a base include the following: salts thereof with inorganic bases such as sodium, potassium, magnesium, calcium, and aluminum; salts thereof with organic bases such as methylamine, ethylamine and ethanolamine; salts thereof with basic amino acids such as lysine and ornithine; and ammonium salt. The salts may be acid addition salts, which are specifically exemplified by acid addition salts with the following: mineral acids such as hydrochloric acid, hydrobromic acid, hydroiodic acid, sulfuric acid, nitric acid, and phosphoric acid:organic acids such as formic acid, acetic acid, propionic acid, oxalic acid, malonic acid, succinic acid, fumaric acid, maleic acid, lactic acid, malic acid, tartaric acid, citric acid, methanesulfonic acid, and ethanesulfonic acid; acidic amino acids such as aspartic acid and glutamic acid.

The term "pharmaceutical composition" refers to a mixture of a compound described herein with other chemical components (referred to collectively herein as "excipients"), such as carriers, stabilizers, diluents, dispersing agents, suspending agents, and/or thickening agents. The pharmaceutical composition facilitates administration of the compound to an organism. Multiple techniques of administering a compound exist in the art including, but not limited to: rectal, oral, intravenous, aerosol, parenteral, ophthalmic, pulmonary, and topical administration.

The term "subject" refers to an animal, including, but not limited to, a primate (*e.g.,* human), monkey, cow, pig, sheep, goat, horse, dog, cat, rabbit, rat, or mouse. The terms "subject" and "patient" are used interchangeably herein in reference, for example, to a mammalian subject, such as a human.

The terms "treat," "treating," and "treatment," in the context of treating a disease or disorder, are meant to include alleviating or abrogating a disorder, disease, or condition, or one or more of the symptoms associated with the disorder, disease, or condition; or to slowing the progression, spread or worsening of a disease, disorder or condition or of one or more symptoms thereof.

The term "prevent", "preventing" or "prevention" in connection to a disease or disorder refers to the prophylactic treatment of a subject who is at risk of developing a condition (e.g., specific disease or disorder or clinical symptom thereof) resulting in a decrease in the probability that the subject will develop the condition.

The terms "hydrogen" and "H" are used interchangeably herein.

The term "halo" refers to fluoro (F), chloro (Cl), bromo (Br), or iodo (I).

The term "alkyl" refers to a hydrocarbon chain that may be a straight chain or branched chain, saturated or unsaturated, containing the indicated number of carbon atoms. For example, C₁₋₁₀ indicates that the group may have from 1 to 10 (inclusive) carbon atoms in it. Non-limiting examples include methyl, ethyl, *iso*-propyl, *tert-*butyl*, n*-hexyl.

The term "haloalkyl" refers to an alkyl, in which one or more hydrogen atoms is/are replaced with an independently selected halo.

The term "alkoxy" refers to an -O-alkyl radical (e.g., -OCH₃).

The term "carbocyclic ring" as used herein includes an aromatic or nonaromatic cyclic hydrocarbon group having 3 to 10 carbons unless otherwise noted, such as 3 to 8 carbons, such as 3 to 7 carbons, which may be optionally substituted. Carbocyclic rings may be monocyclic or bicyclic, and when bicyclic, can be fused bicyclic, bridged bicyclic, or spirocyclic. Examples of carbocyclic rings include five-membered, six-membered, and seven-membered carbocyclic rings.

The term "heterocyclic ring" refers to an aromatic or nonaromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein 0, 1, 2, or 3 atoms of each ring may be substituted by a substituent. When heterocyclic rings are bicyclic or tricyclic, any two connected rings of the bicycle or tricycle may be fused bicyclic, bridged bicyclic, or spirocyclic. Examples of heterocyclic rings include five-membered, six-membered, and seven-membered heterocyclic rings.

The term "cycloalkyl" as used herein includes an nonaromatic cyclic, bicylic, fused, or spiro hydrocarbon radical having 3 to 10 carbons, such as 3 to 8 carbons, such as 3 to 7 carbons, wherein the cycloalkyl group which may be optionally substituted. Examples of cycloalkyls include five-membered, six-membered, and seven-membered rings. Examples include cyclopropyl, cyclobutyl, cyclopentyl, cyclopentenyl, cyclohexyl, cyclohexenyl, cycloheptyl, and cyclooctyl.

The term "heterocycloalkyl" refers to an nonaromatic 5-8 membered monocyclic, 8-12 membered bicyclic, or 11-14 membered tricyclic ring, fused, or spiro system radical having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein 0, 1, 2, or 3 atoms of each ring may be substituted by a substituent. Examples of heterocycloalkyls include five-membered, six-membered, and seven-membered heterocyclic rings. Examples include piperazinyl, pyrrolidinyl, dioxanyl, morpholinyl, tetrahydrofuranyl, and the like.

The term "aryl" is intended to mean an aromatic ring radical containing 6 to 10 ring carbons. Examples include phenyl and naphthyl.

The term "heteroaryl" is intended to mean an aromatic ring system containing 5 to 14 aromatic ring atoms that may be a single ring, two fused rings or three fused rings wherein at least one aromatic ring atom is a heteroatom selected from, but not limited to, the group consisting of O, S and N. Examples include furanyl, thienyl, pyrrolyl, imidazolyl, oxazolyl, thiazolyl, isoxazolyl, pyrazolyl, isothiazolyl, oxadiazolyl, triazolyl, thiadiazolyl, pyridinyl, pyrazinyl, pyrimidinyl, pyridazinyl, triazinyl and the like. Examples also include carbazolyl, quinolizinyl, quinolinyl, isoquinolinyl, cinnolinyl, phthalazinyl, quinazolinyl, quinoxalinyl, triazinyl, indolyl, isoindolyl, indazolyl, indolizinyl, purinyl, naphthyridinyl, pteridinyl, carbazolyl, acridinyl. phenazinyl, phenothiazinyl, phenoxazinyl, benzoxazolyl, benzothiazolyl, 1H-benzimidazolyl, imidazopyridinyl, benzothienyl, benzofuranyl, isobenzofuran and the like.

The term "hydroxy" refers to an OH group.

The term "amino" refers to an NH₂ group.

The term "oxo" refers to O. By way of example, substitution of a CH₂ a group with oxo gives a C=O group.

As used herein, the terms "the ring A" or "A" are used interchangeably to denote 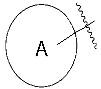 in formula AA, wherein the bond that is shown as being broken by the wavy line 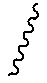 connects A to the S(O)(NHR³)=N moiety of Formula AA.

As used herein, the terms "the ring A'" or "A'" are used interchangeably to denote 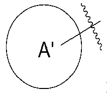 in formula AA-1, wherein the bond that is shown as being broken by the wavy line 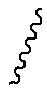 connects A' to the S(O)(NHR³)=N moiety of Formula AA-1.

As used herein, the terms "the ring A'" or "A'" are used interchangeably to denote 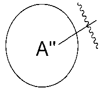 in formula AA-2, formula AA-3, and formula AA-4, wherein the bond that is shown as being broken by the wavy line 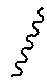 connects A" to the S(O)(NHR³)=N moiety of formula AA-2, formula AA-3, or formula AA-4.

As used herein, the terms "the ring B" or "B" are used interchangeably to denote 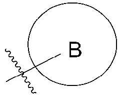 in formula AA wherein the bond that is shown as being broken by the wavy line 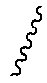 connects B to the NHC(O) group of Formula AA.

As used herein, the terms "the ring B'" or "B'" are used interchangeably to denote 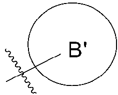 in formula AA-4 wherein the bond that is shown as being broken by the wavy line 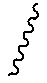 connects B' to the NHC(O) group of Formula AA-4.

As used herein, the terms "the ring B"" or "B"" are used interchangeably to denote 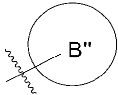 in formula AA-5 wherein the bond that is shown as being broken by the wavy line 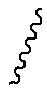 connects B" to the NHC(O) group of Formula AA-5.

As used herein, the term "the optionally substituted ring A" is used to denote 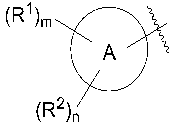 in formula AA, wherein the bond that is shown as being broken by the wavy line 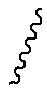 connects A to the S(O)(NHR³)=N moiety of Formula AA.

As used herein, the term "the optionally substituted ring A'" is used to denote 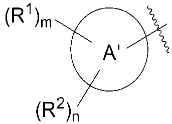 in formula AA-1, wherein the bond that is shown as being broken by the wavy line 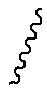 connects A' to the S(O)(NHR³)=N moiety of Formula AA-1.

As used herein, the term "the optionally substituted ring A"", is used to denote 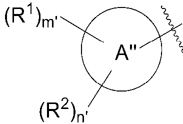 in formula AA-2, 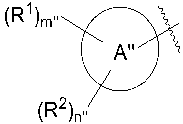 in formula AA-3, and 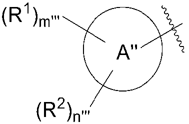 in formula AA-4, wherein the bond that is shown as being broken by the wavy line 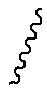 connects A" to the S(O)(NHR³)=N moiety of Formula AA-2, formula AA-3, or formula AA-4.

As used herein, the term "the substituted ring B" is used to denote 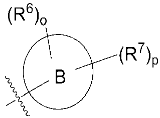 in formula AA and 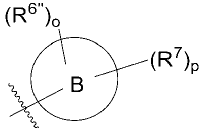 in formula AA-1, wherein the bond that is shown as being broken by the wavy line 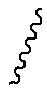 connects B to the NHC(O) group of Formula AA and Formula AA-1.

As used herein, the term "the substituted ring B"' is used to denote 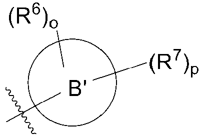 in formula AA-4, wherein the bond that is shown as being broken by the wavy line 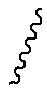 connects B' to the NHC(O) group of Formula AA-4.

As used herein, the term "the substituted ring B"" is used to denote 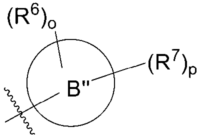 in formula AA-5, wherein the bond that is shown as being broken by the wavy line 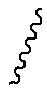 connects B" to the NHC(O) group of Formula AA-5.

As used herein, the recitation "S(O₂)", alone or as part of a larger recitation, refers to the group

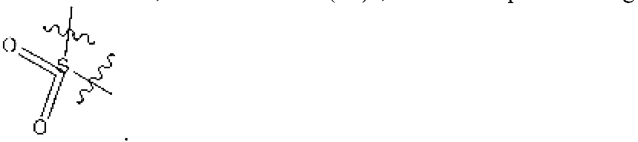

In addition, atoms making up the compounds of the present embodiments are intended to include all isotopic forms of such atoms. Isotopes, as used herein, include those atoms having the same atomic number but different mass numbers. By way of general example and without limitation, isotopes of hydrogen include tritium and deuterium, and isotopes of carbon include ¹³C and ¹⁴C.

The scope of the compounds disclosed herein includes tautomeric form of the compounds. Thus, by way of example, a compound that is represented as containing the moiety 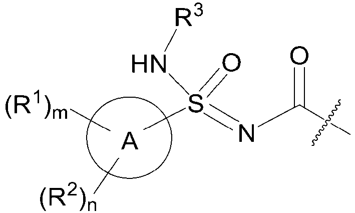 is also intended to include the tautomeric form containing the moiety 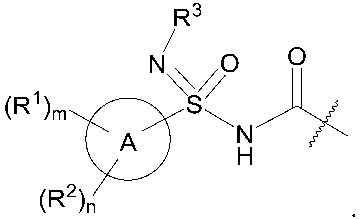 In addition, by way of example, a compound that is represented as containing the moiety 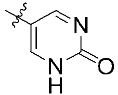 is also intended to include the tautomeric form containing the moiety

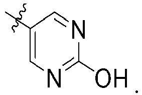

Non-limiting exemplified compounds of the formulae described herein include a stereogenic sulfur atom and optionally one or more stereogenic carbon atoms. This disclosure provides examples of stereoisomer mixtures (e.g., racemic or scalemic mixture of enantiomers; mixture of diastereomers). This disclosure also describes and exemplifies methods for separating individual components of said stereoisomer mixtures (e.g., resolving the enantiomers of a racemic mixture). In cases of compounds containing only a stereogenic sulfur atom, resolved enantiomers are graphically depicted using one of the two following formats: formulas A/B (hashed and solid wedge three-dimensional representation); and formula C ("flat structures with *-labelled stereogenic sulfur).

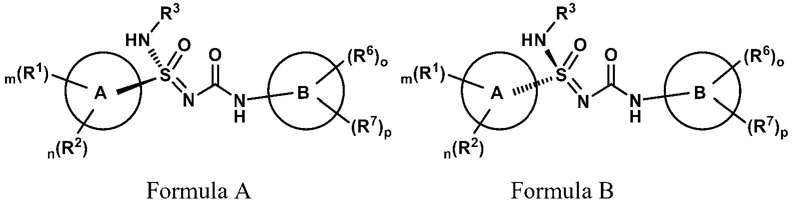

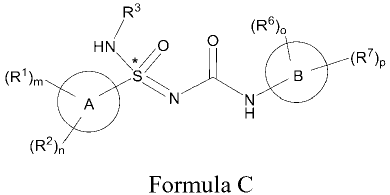

In reaction schemes showing resolution of a racemic mixture, Formulas A/B and C are intended only to convey that the constituent enantiomers were resolved in enantiopure pure form (about 98% ee or greater). The schemes that show resolution products using the formula A/B format are not intended to disclose or imply any correlation between absolute configuration and order of elution.

Analogous formulas are used for compounds containing both stereogenic sulfur and carbon atoms.

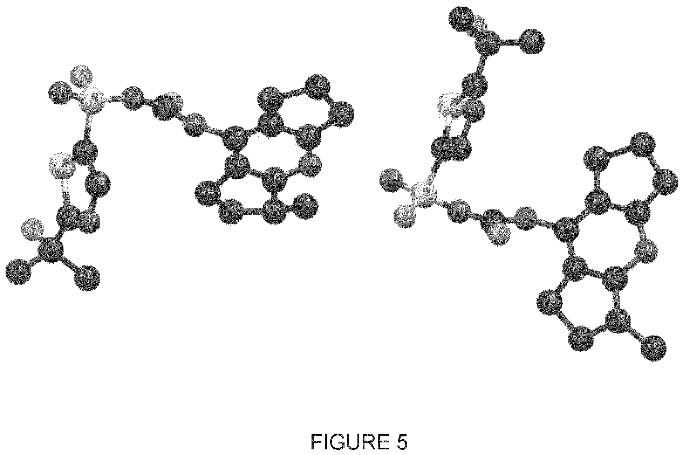

Some of the compounds shown in the tables below are graphically represented using the formula A/B format. However, unless otherwise indicated (e.g., when the compound is synthesized from enantiomerically enriched starting materials (see e.g., compound **964a, Example 573),** the stereogenic center is assigned based on the starting materials), the depicted stereochemistry at sulfur shown for each of the tabulated compounds drawn in the formula A/B format is a tentative assignment and based, by analogy, on the absolute stereochemistry assigned to compound **162bb** herein (see Figure 5).

In some embodiments, for two enantiomers or two epimers of Formula AA compounds which differ in the stereochemical configuration at the sulfur atom of the S(O)NHR³(=N) moiety, one of the two enantiomers or epimers has greater NLRP3 antagonistic activity than the other.

In certain embodiments, when **R³**=H, for two enantiomers or two epimers of Formula AA compounds which differ in the stereochemical configuration at the sulfur atom of the S(O)NHR³(=N) moiety, the enantiomer or epimer with **(*R*)**-stereochemical configuration at the sulfur atom has greater NLRP3 antagonistic activity than the enantiomer or epimer with **(*S*)-**stereochemical configuration at the sulfur atom. For example, the enantiomer or epimer with **(*R*)-**stereochemical configuration at the sulfur atom exhibits a lower IC₅₀ value in the hTHP-1 assay described herein.

The details of one or more embodiments of the invention are set forth in the accompanying drawings and the description below. Other features and advantages of the invention will be apparent from the description and drawings, and from the claims.

### DESCRIPTION OF DRAWINGS

Figure 1: Expression levels of RNA encoding NLRP3 in Crohn's Disease patients who are responsive and non-responsive to infliximab.
Figure 2: Expression levels of RNA encoding IL-1β in Crohn's Disease patients who are responsive and non-responsive to infliximab.
Figure 3: Expression levels of RNA encoding NLRP3 in Ulcerative Colitis (UC) patients who are responsive and non-responsive to infliximab.
Figure 4: Expression levels of RNA encoding IL-1β in Ulcerative Colitis (UC) patients who are responsive and non-responsive to infliximab.
Figure 5 depicts ball-and-stick representations of two crystallographically independent molecules of compound **162bb** in the asymmetrical unit.

### DETAILED DESCRIPTION

In one aspect, provided herein is a compound of Formula AA 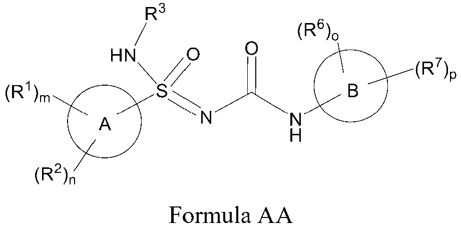 wherein
m = 0, 1, or 2;
n = 0, 1, or 2;
o = 1 or 2;
p = 0, 1, 2, or 3; wherein the sum of o and p is from 1 to 4;
wherein
A is a 5- to 10-membered heteroaryl or a C₆-C₁₀ aryl;
B is a 6-membered heteroaromatic ring containing 1-3 N atoms, or an N-oxide thereof; wherein at least one R⁶ is *ortho* to the bond connecting the B ring to the NHC(O) group of Formula AA;
R¹ and R² are each independently selected from C₁-C₆ alkyl, C₁-C₆ haloalkyl, C₁-C₆ alkoxy, C₁-C₆ haloalkoxy, halo, CN, NO₂, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NR⁸R⁹, C(O)R¹³, CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ alkoxy, C₁-C₆ haloalkyl, C₆-C₁₀ aryl, 5- to 10-membered heteroaryl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, R¹⁵, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), and OCO(3- to 7-membered heterocycloalkyl);
wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, -O(C₀-C₃ alkylene)C₆-C₁₀ aryl, halo, NR⁸R⁹, or oxo;
wherein the 3- to 7-membered heterocycloalkyl, C₆-C₁₀ aryl, and 5- to 10-membered heteroaryl are each optionally substituted with one or more substituents independently selected from halo, C₁-C₆ alkyl, and OC₁-C₆ alkyl;
or one pair of R¹ and R² on adjacent atoms, taken together with the atoms connecting them, independently form at least one monocyclic or bicyclic C₄-C₁₂ carbocyclic ring or at least one monocyclic or bicyclic 5- to-12-membered heterocyclic ring wherein:
   a) when each of the adjacent atoms is a carbon atom, then the heterocyclic ring includes from 1-3 heteroatoms and/or heteroatomic groups independently selected from O, NH, NR¹³, S, S(O), and S(O)₂; and
   b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²), and
wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R⁶ and R⁷ are each independently selected from C₁-C₆ alkyl, C₁-C₆ haloalkyl, C₁-C₆ alkoxy, C₁-C₆ haloalkoxy, halo, hydroxy, oxo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or at least one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms independently selected from O, NH, NR¹³, and S, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R¹⁰ is C₁-C₆ alkyl;
each of R⁸ and R⁹ at each occurrence is independently selected from hydrogen, C₁-C₆ alkyl, C₂-C₆ alkenyl, C₃-C₇ cycloalkyl, C₁-C₆ haloalkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl, 3- to 7-membered heterocycloalkyl, or NR¹¹R¹²;
or R⁸ and R⁹ taken together with the nitrogen they are attached to form a 3- to 10-membered monocyclic or bicyclic ring optionally containing one or more heteroatoms in addition to the nitrogen they are attached to, wherein the ring is optionally substituted with one or more substituents independently selected from halo, C₁-C₆ alkyl, C₁-C₆ haloalkyl, C₁-C₆ alkoxy, oxo, N(C₁-C₆ alkyl)₂, NH₂, NH(C₁-C₆ alkyl), and hydroxy;
R¹³ is C₁-C₆ alkyl, C₁-C₆ haloalkyl, or -(Z¹-Z²)ₐ₁-Z³;
each of R¹¹ and R¹² at each occurrence is independently selected from hydrogen, C₁-C₆ alkyl, and -(Z¹-Z²)ₐ₁-Z³;
a1 is an integer selected from 0-10 (e.g., 0-5);
each Z¹ is independently C₁-C₆ alkylene optionally substituted with one or more substituents independently selected from oxo, halo, and hydroxy;
each Z² is independently a bond, NH, N(C₁-C₆ alkyl), -O-, -S-, or 5-10 membered heteroarylene;
Z³ is independently C₆-C₁₀ aryl, C₂-C₆ alkyenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
R³ is selected from hydrogen, cyano, hydroxy, CO₂C₁-C₆ alkyl, C₁-C₆ alkoxy, C₁-C₆ alkyl, and 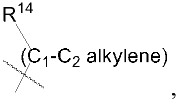 wherein the C₁-C₂ alkylene group is optionally substituted with oxo;
R¹⁴ is hydrogen, C₁-C₆ alkyl, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶;
R¹⁵ is -(Z⁴-Z⁵)ₐ₂-Z⁶;
a2 is an integer selected from 1-10 (e.g., 1-5 (e.g., 2-5));
each Z⁴ is independently selected from -O-, -S-, -NH-, and -N(C₁-C₃ alkyl)-;
provided that the Z⁴ group directly attached to R¹ or R² is -O- or -S-;
each Z⁵ is independently C₁-C₆ alkylene optionally substituted with one or more substituents independently selected from oxo, halo, and hydroxy; and
Z⁶ is OH, OC₁-C₆ alkyl, NH₂, NH(C₁-C₆ alkyl), N(C₁-C₆ alkyl)₂, NHC(O)(C₁-C₆ alkyl), NHC(O)(C₁-C₆ alkoxy), or an optionally substituted group selected from the group consisting of:
   C₆-C₁₀ aryl, C₂-C₆ alkenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
   with the proviso that the compound of Formula AA is not a compound selected from the group consisting of: 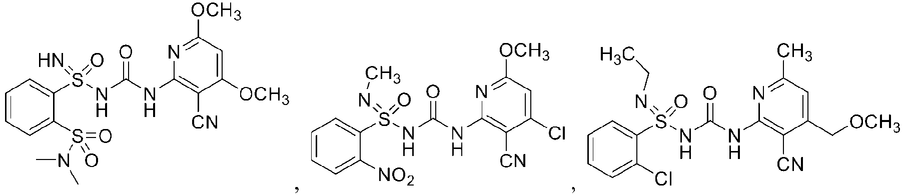 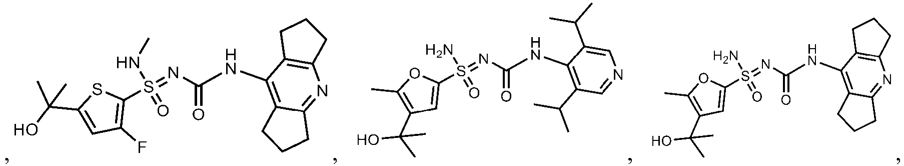 and 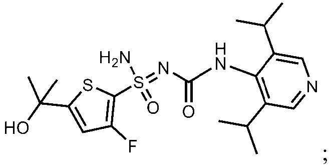
   or a pharmaceutically acceptable salt thereof.
In one aspect, provided herein is a compound of Formula AA: 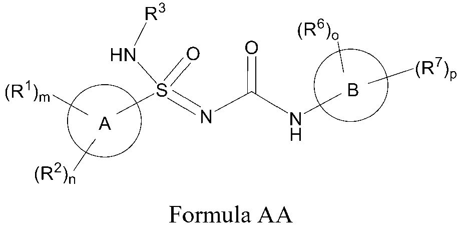 wherein
m = 0, 1, or 2;
n = 0, 1, or 2;
o = 1 or 2;
p = 0, 1, 2, or 3; wherein the sum of o and p is from 1 to 4; wherein
A is a 5- to 10-membered heteroaryl or a C₆-C₁₀ aryl;
B is a 6-membered heteroaromatic ring containing 1-3 N atoms, or an N-oxide thereof; wherein at least one R⁶ is *ortho* to the bond connecting the B ring to the NHC(O) group of Formula AA;
R¹ and R² are each independently selected from C₁-C₆ alkyl, C₁-C₆ haloalkyl, C₁-C₆ alkoxy, C₁-C₆ haloalkoxy, halo, CN, NO₂, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NR⁸R⁹, C(O)R¹³, CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ alkoxy, C₁-C₆ haloalkyl, C₆-C₁₀ aryl, 5- to 10-membered heteroaryl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, R¹⁵, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), and OCO(3- to 7-membered heterocycloalkyl);
wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, -O(C₀-C₃ alkylene)C₆-C₁₀ aryl, halo, NR⁸R⁹, or oxo;
wherein the 3- to 7-membered heterocycloalkyl, C₆-C₁₀ aryl, and 5- to 10-membered heteroaryl are each optionally substituted with one or more substituents independently selected from halo, C₁-C₆ alkyl, and OC₁-C₆ alkyl;
or one pair of R¹ and R² on adjacent atoms, taken together with the atoms connecting them, independently form at least one monocyclic or bicyclic C₄-C₁₂ carbocyclic ring or at least one monocyclic or bicyclic 5- to-12-membered heterocyclic ring wherein:
   a) when each of the adjacent atoms is a carbon atom, then the heterocyclic ring includes from 1-3 heteroatoms and/or heteroatomic groups independently selected from O, NH, NR¹³, S, S(O), and S(O)₂; and
   b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²), and
wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R⁶ and R⁷ are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or at least one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms independently selected from O, NH, NR¹³, and S, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R¹⁰ is C₁-C₆ alkyl;
each of R⁸ and R⁹ at each occurrence is independently selected from hydrogen, C₁-C₆ alkyl, C₂-C₆ alkenyl, C₃-C₇ cycloalkyl, C₁-C₆ haloalkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl, 3- to 7-membered heterocycloalkyl, or NR¹¹R¹²;
or R⁸ and R⁹ taken together with the nitrogen they are attached to form a 3- to 10-membered monocyclic or bicyclic ring optionally containing one or more heteroatoms in addition to the nitrogen they are attached to, wherein the ring is optionally substituted with one or more substituents independently selected from halo, C₁-C₆ alkyl, C₁-C₆ haloalkyl, C₁-C₆ alkoxy, oxo, N(C₁-C₆ alkyl)₂, NH₂, NH(C₁-C₆ alkyl), and hydroxy;
R¹³ is C₁-C₆ alkyl, C₁-C₆ haloalkyl, or -(Z¹-Z²)ₐ₁-Z³;
each of R¹¹ and R¹² at each occurrence is independently selected from hydrogen, C₁-C₆ alkyl, and -(Z¹-Z²)ₐ₁-Z³;
a1 is an integer selected from 0-10 (e.g., 0-5);
each Z¹ is independently C₁-C₆ alkylene optionally substituted with one or more substituents independently selected from oxo, halo, and hydroxy;
each Z² is independently a bond, NH, N(C₁-C₆ alkyl), -O-, -S-, or 5-10 membered heteroarylene;
Z³ is independently C₆-C₁₀ aryl, C₂-C₆ alkyenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
R³ is selected from hydrogen, cyano, hydroxy, CO₂C₁-C₆ alkyl, C₁-C₆ alkoxy, C₁-C₆ alkyl, and 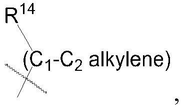 wherein the C₁-C₂ alkylene group is optionally substituted with oxo;
R¹⁴ is hydrogen, C₁-C₆ alkyl, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶;
R¹⁵ is -(Z⁴-Z⁵)ₐ₂-Z⁶;
a2 is an integer selected from 1-10 (e.g., 1-5 (e.g., 2-5));
each Z⁴ is independently selected from -O-, -S-, -NH-, and -N(C₁-C₃ alkyl)-;
provided that the Z⁴ group directly attached to R¹ or R² is -O- or -S-;
each Z⁵ is independently C₁-C₆ alkylene optionally substituted with one or more substituents independently selected from oxo, halo, and hydroxy; and
Z⁶ is OH, OC₁-C₆ alkyl, NH₂, NH(C₁-C₆ alkyl), N(C₁-C₆ alkyl)₂, NHC(O)(C₁-C₆ alkyl), NHC(O)(C₁-C₆ alkoxy), or an optionally substituted group selected from the group consisting of:
   C₆-C₁₀ aryl, C₂-C₆ alkenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
   with the proviso that the compound of Formula AA is not a compound selected from the group consisting of: 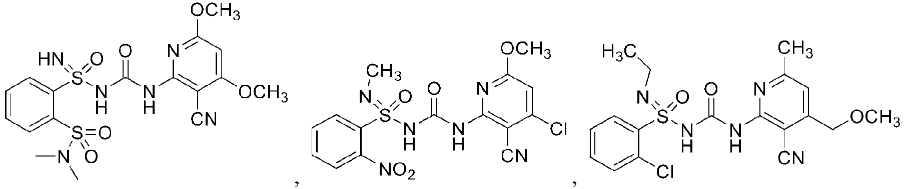 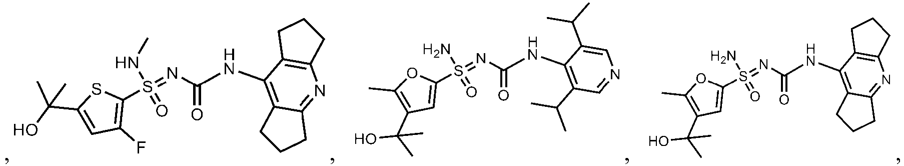 and 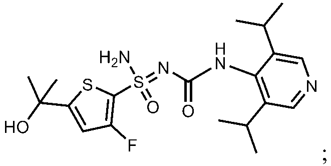
   or a pharmaceutically acceptable salt thereof.
In one aspect, provided herein is a compound of Formula AA 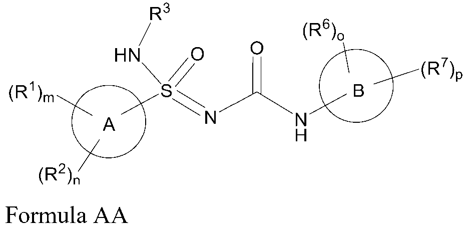 wherein
m = 0, 1, or 2;
n = 0, 1, or 2;
o = 1 or 2;
p = 0, 1, 2, or 3;
wherein the sum of o and p is from 1 to 4;
wherein
A is a 5- to 10-membered heteroaryl or a C₆-C₁₀ aryl;
B is a 6-membered heteroaromatic ring containing 1-3 N atoms, or an N-oxide thereof;
wherein
at least one R⁶ is *ortho* to the bond connecting the B ring to the NHC(O) group of Formula AA; R¹ and R² are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO-C₆-C₁₀ aryl, CO(5- to 10-membered heteroaryl), CO₂C₁-C₆ alkyl, CO₂C₃-C₈ cycloalkyl, OCOC₁-C₆ alkyl, OCOC₆-C₁₀ aryl, OCO(5-to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ haloalkyl, C₃-C₇ cycloalkyl and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, halo, NR⁸R⁹, or oxo;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R⁶ and R⁷ are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹;
R¹⁰ is C₁-C₆ alkyl;
each of R⁸ and R⁹ at each occurrence is independently selected from hydrogen, C₁-C₆ alkyl, C₁-C₆ haloalkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl or 3- to 7-membered heterocycloalkyl; or R⁸ and R⁹ taken together with the nitrogen they are attached to form a 3- to 7-membered ring optionally containing one or more heteroatoms in addition to the nitrogen they are attached to;
R¹³ is C₁-C₆ alkyl, C₁-C₆ haloalkyl, C₆-C₁₀ aryl, or 5- to 10-membered heteroaryl;
each of R¹¹ and R¹² at each occurrence is independently selected from hydrogen and C₁-C₆ alkyl; and
R³ is selected from hydrogen, cyano, hydroxy, CO₂C₁-C₆ alkyl, C₁-C₆ alkoxy, C₁-C₆ alkyl, and 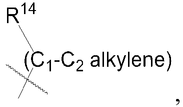 wherein the C₁-C₂ alkylene group is optionally substituted with oxo;
R¹⁴ is hydrogen, C₁-C₆ alkyl, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶;
with the proviso that the compound of Formula AA is not a compound selected from the group consisting of: 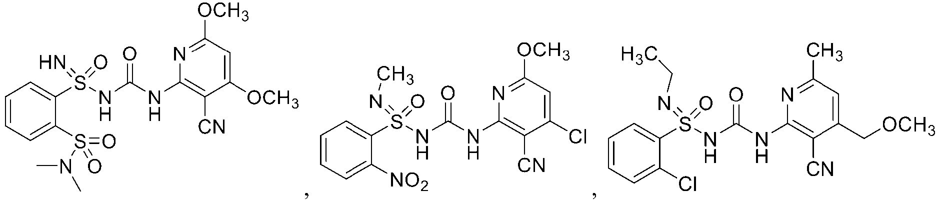 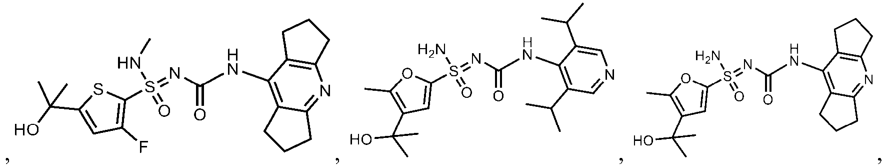 and 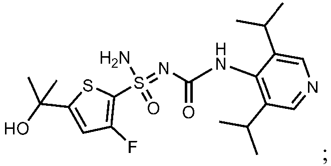
or a pharmaceutically acceptable salt thereof.

In another aspect, provided herein is a compound of Formula AA 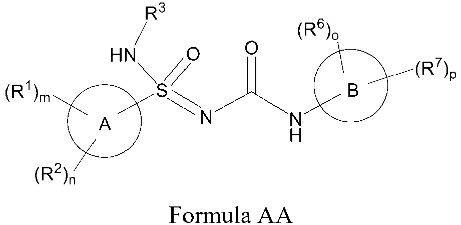 wherein the compound of Formula AA is selected from 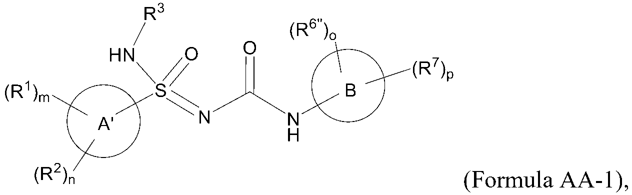 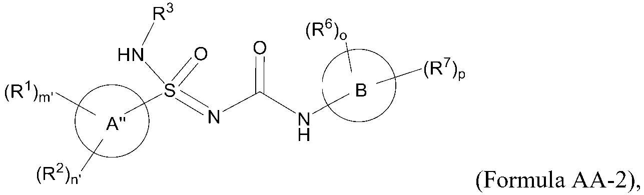 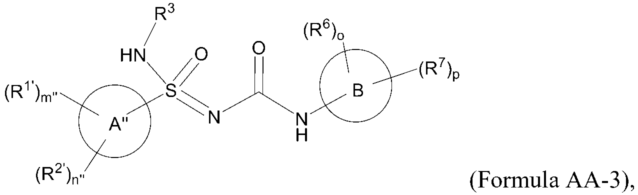 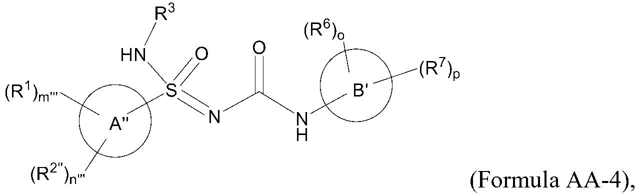 and 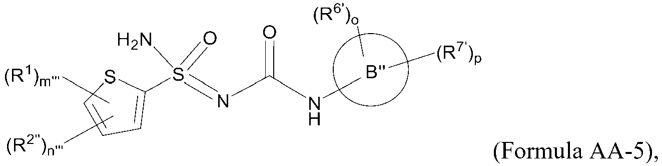 wherein
m = 0, 1, or 2;
n = 0, 1, or 2;
m' = 0, 1, or 2;
n' = 0, 1, or 2; wherein the sum of m' and n' is 0, 1, or 3;
m" = 0, 1, or 2;
n" = 0, 1, or 2; wherein the sum of m" and n'' is 2;
m‴ = 1;
n''' = 1;
o = 1 or 2;
p = 0, 1, 2, or 3; wherein the sum of o and p is from 1 to 4;
wherein
A' is selected from:
a 6- to 10-membered heteroaryl,
a C₆-C₁₀ aryl,
a 5-membered heteroaryl comprising 2 or more heteroatoms,
a 5-membered heteroaryl comprising 1 heteroatom or heteroatomic group selected from N, NH, and NR¹, and
a 5-membered heteroaryl comprising 1 heteroatom selected from O and S, wherein the heteroatom is not bonded to the position of the heteroaryl that is bonded to the S(O)(NHR³)=N moiety;
A" is a 5-membered heteroaryl comprising 1 heteroatom selected from O and S, wherein the heteroatom is bonded to the position of the heteroaryl that is bonded to the S(O)(NHR³)=N moiety;
B is a 6-membered heteroaromatic ring containing 1-3 N atoms, or an N-oxide thereof;
B' is 2-pyridyl, 3-pyridyl, or an N-oxide thereof;
B" is 4-pyridyl or an N-oxide thereof;
wherein
at least one R⁶ is *ortho* to the bond connecting the B ring to the NHC(O) group of Formula AA-2, Formula AA-3, and Formula AA-4;
at least one R^{6'} is *ortho* to the bond connecting the B ring to the NHC(O) group of Formula AA-5;
at least one R^{6"} is *ortho* to the bond connecting the B ring to the NHC(O) group of Formula AA-1;
R¹ and R² are each independently selected from C₁-C₆ alkyl, C₁-C₆ haloalkyl, C₁-C₆ alkoxy, C₁-C₆ haloalkoxy, halo, CN, NO₂, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NR⁸R⁹, C(O)R¹³, CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ alkoxy, C₁-C₆ haloalkyl, C₆-C₁₀ aryl, 5- to 10-membered heteroaryl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, R¹⁵, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), and OCO(3- to 7-membered heterocycloalkyl);
wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, -O(C₀-C₃ alkylene)C₆-C₁₀ aryl, halo, NR⁸R⁹, or oxo;
wherein the 3- to 7-membered heterocycloalkyl, C₆-C₁₀ aryl, and 5- to 10-membered heteroaryl, are each optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring wherein:
   a) when each of the adjacent atoms is a carbon atom, then the heterocyclic ring includes from 1-3 heteroatoms and/or heteroatomic groups independently selected from O, NH, NR¹³, S, S(O), and S(O)₂; and
   b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²), and
wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R^{1'} and R^{2'} are each independently selected from C₂-C₆ alkyl, C₁-C₆ haloalkyl, C₁-C₆ alkoxy, C₁-C₆ haloalkoxy, Cl, Br, I, CN, NO₂, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NR⁸R⁹, C(O)R¹³, CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₂-C₆ alkyl, C₁-C₆ alkoxy, C₁-C₆ haloalkyl, C₆-C₁₀ aryl, 5- to 10-membered heteroaryl, C₃-C₇ cycloalkyl, and 3- to 7-membered heterocycloalkyl is optionally substituted with one or more substituents each independently selected from hydroxy, halo, CN, oxo, C₁-C₆ alkyl, C₁-C₆ alkoxy, R¹⁵, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), and OCO(3- to 7-membered heterocycloalkyl);
wherein each C₁-C₆ alkyl substituent and each C₁-C₆ alkoxy substituent of the R^{1'} or R^{2'} C₃-C₇ cycloalkyl or of the R^{1'} or R^{2'} 3- to 7-membered heterocycloalkyl is further optionally independently substituted with one to three hydroxy, -O(C₀-C₃ alkylene)C₆-C₁₀ aryl, halo, NR⁸R⁹, or oxo;
wherein the 3- to 7-membered heterocycloalkyl, C₆-C₁₀ aryl, and 5- to 10-membered heteroaryl are optionally substituted with one or more substituents independently selected from halo, C₁-C₆ alkyl, and OC₁-C₆ alkyl;
or one pair of R^{1'} and R^{2'} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and
wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R^{2"} is F or CH₃; or
when the compound of Formula AA is a compound of Formula AA-4, one pair of R¹ and R^{2"} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and
wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R⁶ and R⁷ are each independently selected from C₁-C₆ alkyl, C₁-C₆ haloalkyl, C₁-C₆ alkoxy, C₁-C₆ haloalkoxy, halo, hydroxy, oxo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R^{6'} and R^{7'} are each independently selected from unbranched C₁-C₆ alkyl, C₁-C₆ haloalkyl, C₁-C₆ alkoxy, C₁-C₆ haloalkoxy, halo, hydroxy, oxo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R^{6'} and R^{7'} are each optionally substituted with one or more substituents independently selected from hydroxy, halo, CN, oxo, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkoxy that R^{6'} or R^{7'} is substituted with is optionally substituted with one or more hydroxyl, C₆-C₁₀ aryl or NR⁸R⁹, or wherein R^{6'} or R^{7'} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R^{6"} is selected from C₁-C₆ alkyl, C₁-C₆ haloalkyl, C₁-C₆ alkoxy, C₁-C₆ haloalkoxy, halo, hydroxy, oxo, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl, 3- to 10-membered heterocycloalkyl, and C₂-C₆ alkenyl,
wherein R^{6"} is optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and
wherein the C₁-C₆ alkyl or C₁-C₆ alkoxy that R^{6"} is substituted with is optionally substituted with one or more hydroxyl, C₆-C₁₀ aryl or NR⁸R⁹, or wherein R^{6"} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R¹⁰ is C₁-C₆ alkyl;
each of R⁸ and R⁹ at each occurrence is independently selected from hydrogen, C₁-C₆ alkyl, C₂-C₆ alkenyl, C₃-C₇ cycloalkyl, C₁-C₆ haloalkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl, 3- to 7-membered heterocycloalkyl, or NR¹¹R¹²;
or R⁸ and R⁹ taken together with the nitrogen they are attached to form a 3- to 10-membered monocyclic or bicyclic ring optionally containing one or more heteroatoms in addition to the nitrogen they are attached to, wherein the ring is optionally substituted with one or more substituents independently selected from halo, C₁-C₆ alkyl, C₁-C₆ haloalkyl, C₁-C₆ alkoxy, oxo, N(C₁-C₆ alkyl)₂, NH₂, NH(C₁-C₆ alkyl), and hydroxy;
R¹³ is C₁-C₆ alkyl, C₁-C₆ haloalkyl, or -(Z¹-Z²)ₐ₁-Z³;
each of R¹¹ and R¹² at each occurrence is independently selected from hydrogen, C₁-C₆ alkyl, and -(Z¹-Z²)ₐ₁-Z³;
a1 is an integer selected from 0-10 (e.g., 0-5);
each Z¹ is independently C₁-C₆ alkylene optionally substituted with one or more substituents independently selected from oxo, halo, and hydroxy;
each Z² is independently a bond, NH, N(C₁-C₆ alkyl), -O-, -S-, or 5-10 membered heteroarylene;
Z³ is independently C₆-C₁₀ aryl, C₂-C₆ alkyenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
R³ is selected from hydrogen, cyano, hydroxy, CO₂C₁-C₆ alkyl, C₁-C₆ alkoxy, C₁-C₆ alkyl, and 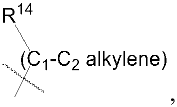 wherein the C₁-C₂ alkylene group is optionally substituted with oxo;
R¹⁴ is hydrogen, C₁-C₆ alkyl, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶;
R¹⁵ is -(Z⁴-Z⁵)ₐ₂-Z⁶;
a2 is an integer selected from 1-10 (e.g., 1-5 (e.g., 2-5));
each Z⁴ is independently selected from -O-, -S-, -NH-, and -N(C₁-C₃ alkyl)-;
provided that the Z⁴ group directly attached to R¹ or R² is -O- or -S-;
each Z⁵ is independently C₁-C₆ alkylene optionally substituted with one or more substituents independently selected from oxo, halo, and hydroxy; and
Z⁶ is OH, OC₁-C₆ alkyl, NH₂, NH(C₁-C₆ alkyl), N(C₁-C₆ alkyl)₂, NHC(O)(C₁-C₆ alkyl), NHC(O)(C₁-C₆ alkoxy), or an optionally substituted group selected from the group consisting of:
   C₆-C₁₀ aryl, C₂-C₆ alkenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
   or a pharmaceutically acceptable salt thereof.

In another aspect, provided herein is a compound of Formula AA: 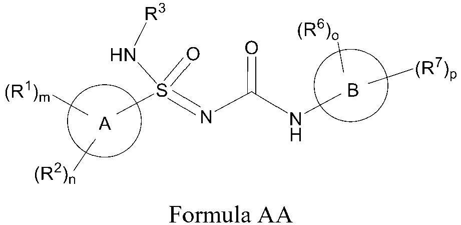 wherein the compound of Formula AA is selected from 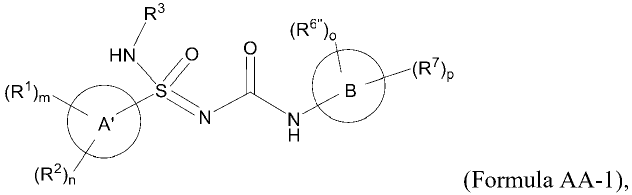 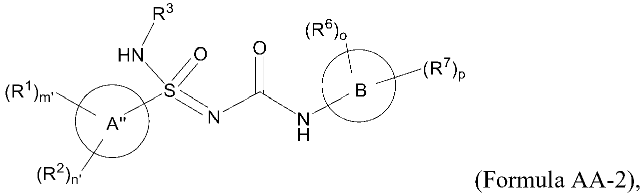 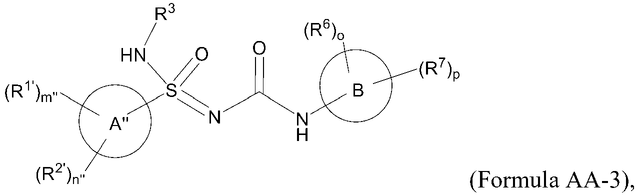 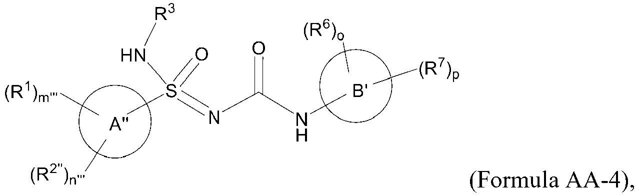 and 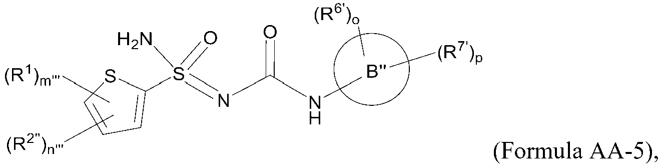 wherein
m = 0, 1, or 2;
n = 0, 1, or 2;
m' = 0, 1, or 2;
n' = 0, 1, or 2; wherein the sum of m' and n' is 0, 1, or 3;
m" = 0, 1, or 2;
n" = 0, 1, or 2; wherein the sum of m'' and n'' is 2;
m‴ = 1;
n‴ = 1;
o = 1 or 2;
p = 0, 1, 2, or 3; wherein the sum of o and p is from 1 to 4;
wherein
A' is selected from:
a 6- to 10-membered heteroaryl,
a C₆-C₁₀ aryl,
a 5-membered heteroaryl comprising 2 or more heteroatoms,
a 5-membered heteroaryl comprising 1 heteroatom or heteroatomic group selected from N, NH, and NR¹, and
a 5-membered heteroaryl comprising 1 heteroatom selected from O and S, wherein the heteroatom is not bonded to the position of the heteroaryl that is bonded to the S(O)(NHR³)=N moiety;
A" is a 5-membered heteroaryl comprising 1 heteroatom selected from O and S, wherein the heteroatom is bonded to the position of the heteroaryl that is bonded to the S(O)(NHR³)=N moiety;
B is a 6-membered heteroaromatic ring containing 1-3 N atoms, or an N-oxide thereof;
B' is 2-pyridyl, 3-pyridyl, or an N-oxide thereof;
B" is 4-pyridyl or an N-oxide thereof;
wherein
at least one R⁶ is *ortho* to the bond connecting the B ring to the NHC(O) group of Formula AA-2, Formula AA-3, and Formula AA-4;
at least one R^{6'} is *ortho* to the bond connecting the B ring to the NHC(O) group of Formula AA-5;
at least one R^{6"} is *ortho* to the bond connecting the B ring to the NHC(O) group of Formula AA-1;
R¹ and R² are each independently selected from C₁-C₆ alkyl, C₁-C₆ haloalkyl, C₁-C₆ alkoxy, C₁-C₆ haloalkoxy, halo, CN, NO₂, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NR⁸R⁹, C(O)R¹³, CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ alkoxy, C₁-C₆ haloalkyl, C₆-C₁₀ aryl, 5- to 10-membered heteroaryl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, R¹⁵, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), and OCO(3- to 7-membered heterocycloalkyl);
wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, -O(C₀-C₃ alkylene)C₆-C₁₀ aryl, halo, NR⁸R⁹, or oxo;
wherein the 3- to 7-membered heterocycloalkyl, C₆-C₁₀ aryl, and 5- to 10-membered heteroaryl, are each optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring wherein:
   a) when each of the adjacent atoms is a carbon atom, then the heterocyclic ring includes from 1-3 heteroatoms and/or heteroatomic groups independently selected from O, NH, NR¹³, S, S(O), and S(O)₂; and
   b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂(in addition to the aforementioned nitrogen atom(s) attached to R¹ and/or R²), and
wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R^{1'} and R^{2'} are each independently selected from C₂-C₆ alkyl, C₁-C₆ haloalkyl, C₁-C₆ alkoxy, C₁-C₆ haloalkoxy, Cl, Br, I, CN, NO₂, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NR⁸R⁹, C(O)R¹³, CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₂-C₆ alkyl, C₁-C₆ alkoxy, C₁-C₆ haloalkyl, C₆-C₁₀ aryl, 5- to 10-membered heteroaryl, C₃-C₇ cycloalkyl, and 3- to 7-membered heterocycloalkyl is optionally substituted with one or more substituents each independently selected from hydroxy, halo, CN, oxo, C₁-C₆ alkyl, C₁-C₆ alkoxy, R¹⁵, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), and OCO(3- to 7-membered heterocycloalkyl);
wherein each C₁-C₆ alkyl substituent and each C₁-C₆ alkoxy substituent of the R^{1'} or R^{2'} C₃-C₇ cycloalkyl or of the R^{1'} or R^{2'} 3- to 7-membered heterocycloalkyl is further optionally independently substituted with one to three hydroxy, -O(C₀-C₃ alkylene)C₆-C₁₀ aryl, halo, NR⁸R⁹, or oxo;
wherein the 3- to 7-membered heterocycloalkyl, C₆-C₁₀ aryl, and 5- to 10-membered heteroaryl are optionally substituted with one or more substituents independently selected from halo, C₁-C₆ alkyl, and OC₁-C₆ alkyl;
or one pair of R^{1'} and R^{2'} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and
wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R^{2"} is F or CH₃; or
when the compound of Formula AA is a compound of Formula AA-4, one pair of R¹ and R^{2"} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and
wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R⁶ and R⁷ are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R^{6'} and R^{7'} are each independently selected from unbranched C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R^{6'} and R^{7'} are each optionally substituted with one or more substituents independently selected from hydroxy, halo, CN, oxo, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkoxy that R^{6'} or R^{7'} is substituted with is optionally substituted with one or more hydroxyl, C₆-C₁₀ aryl or NR⁸R⁹, or wherein R^{6'} or R^{7'} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R^{6"} is selected from C₁-C₆ alkyl, C₁-C₆ haloalkyl, C₁-C₆ alkoxy, C₁-C₆ haloalkoxy, halo, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5-to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl, 3- to 10-membered heterocycloalkyl, and C₂-C₆ alkenyl,
wherein R^{6"} is optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and
wherein the C₁-C₆ alkyl or C₁-C₆ alkoxy that R^{6"} is substituted with is optionally substituted with one or more hydroxyl, C₆-C₁₀ aryl or NR⁸R⁹, or wherein R^{6"} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R¹⁰ is C₁-C₆ alkyl;
each of R⁸ and R⁹ at each occurrence is independently selected from hydrogen, C₁-C₆ alkyl, C₂-C₆ alkenyl, C₃-C₇ cycloalkyl, C₁-C₆ haloalkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl, 3- to 7-membered heterocycloalkyl, or NR¹¹R¹²;
or R⁸ and R⁹ taken together with the nitrogen they are attached to form a 3- to 10-membered monocyclic or bicyclic ring optionally containing one or more heteroatoms in addition to the nitrogen they are attached to, wherein the ring is optionally substituted with one or more substituents independently selected from halo, C₁-C₆ alkyl, C₁-C₆ haloalkyl, C₁-C₆ alkoxy, oxo, N(C₁-C₆ alkyl)₂, NH₂, NH(C₁-C₆ alkyl), and hydroxy;
R¹³ is C₁-C₆ alkyl, C₁-C₆ haloalkyl, or -(Z¹-Z²)ₐ₁-Z³;
each of R¹¹ and R¹² at each occurrence is independently selected from hydrogen, C₁-C₆ alkyl, and -(Z¹-Z²)ₐ₁-Z³;
a1 is an integer selected from 0-10 (e.g., 0-5);
each Z¹ is independently C₁-C₆ alkylene optionally substituted with one or more substituents independently selected from oxo, halo, and hydroxy;
each Z² is independently a bond, NH, N(C₁-C₆ alkyl), -O-, -S-, or 5-10 membered heteroarylene;
Z³ is independently C₆-C₁₀ aryl, C₂-C₆ alkyenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
R³ is selected from hydrogen, cyano, hydroxy, CO₂C₁-C₆ alkyl, C₁-C₆ alkoxy, C₁-C₆ alkyl, and 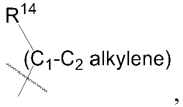 wherein the C₁-C₂ alkylene group is optionally substituted with oxo;
R¹⁴ is hydrogen, C₁-C₆ alkyl, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶;
R¹⁵ is -(Z⁴-Z⁵)ₐ₂-Z⁶;
a2 is an integer selected from 1-10 (e.g., 1-5 (e.g., 2-5));
each Z⁴ is independently selected from -O-, -S-, -NH-, and -N(C₁-C₃ alkyl)-;
provided that the Z⁴ group directly attached to R¹ or R² is -O- or -S-;
each Z⁵ is independently C₁-C₆ alkylene optionally substituted with one or more substituents independently selected from oxo, halo, and hydroxy; and
Z⁶ is OH, OC₁-C₆ alkyl, NH₂, NH(C₁-C₆ alkyl), N(C₁-C₆ alkyl)₂, NHC(O)(C₁-C₆ alkyl), NHC(O)(C₁-C₆ alkoxy), or an optionally substituted group selected from the group consisting of:
   C₆-C₁₀ aryl, C₂-C₆ alkenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
   or a pharmaceutically acceptable salt thereof.

In another aspect, described herein is a compound of Formula AA 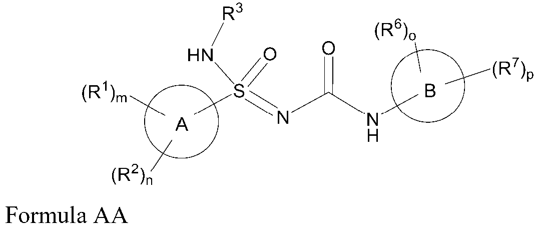 wherein the compound of Formula AA is selected from 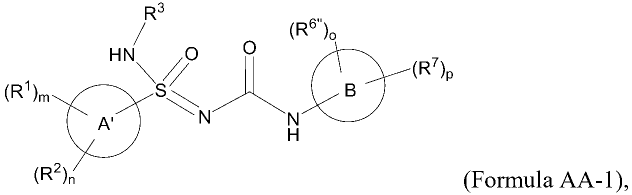 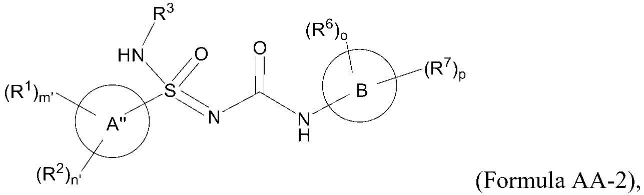 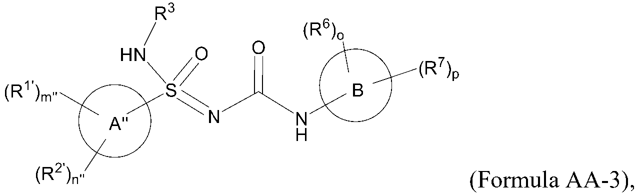 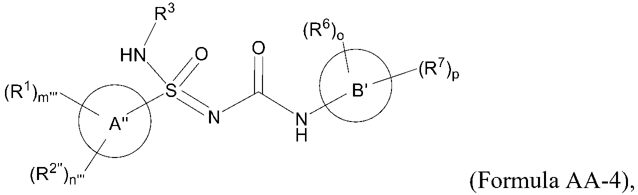 and 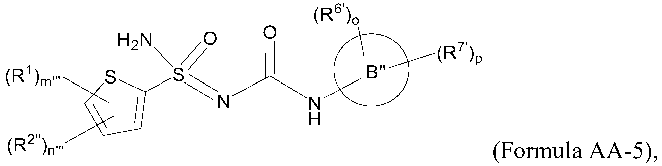 wherein
m = 0, 1, or 2;
n = 0, 1, or 2;
m' = 0, 1, or 2;
n' = 0, 1, or 2;
wherein the sum of m' and n' is 0, 1, or 3;
m" = 0, 1, or 2;
n" = 0, 1, or 2;
wherein the sum of m'' and n'' is 2;
m''' = 1;
n''' = 1;
o = 1 or 2;
p = 0, 1, 2, or 3;
wherein the sum of o and p is from 1 to 4;
wherein
A' is selected from:
   a 6- to 10-membered heteroaryl,
   a C₆-C₁₀ aryl,
   a 5-membered heteroaryl comprising 2 or more heteroatoms,
   a 5-membered heteroaryl comprising 1 heteroatom or heteroatomic group selected from N, NH, and NR¹, and
   a 5-membered heteroaryl comprising 1 heteroatom selected from O and S, wherein the heteroatom is not bonded to the position of the heteroaryl that is bonded to the S(O)(NHR³)=N moiety;
   A'' is a 5-membered heteroaryl comprising 1 heteroatom selected from O and S, wherein the heteroatom is bonded to the position of the heteroaryl that is bonded to the S(O)(NHR³)=N moiety;
   B is a 6-membered heteroaromatic ring containing 1-3 N atoms, or an N-oxide thereof; B' is 2-pyridyl, 3-pyridyl, or an N-oxide thereof;
   B'' is 4-pyridyl or an N-oxide thereof;
   wherein
   at least one R⁶ is *ortho* to the bond connecting the B ring to the NHC(O) group of Formula AA-2, Formula AA-3, and Formula AA-4;
   at least one R^{6'} is *ortho* to the bond connecting the B ring to the NHC(O) group of Formula AA-5;
   at least one R^{6"} is *ortho* to the bond connecting the B ring to the NHC(O) group of Formula AA-1;
   R¹ and R² are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO-C₆-C₁₀ aryl, CO(5- to 10-membered heteroaryl), CO₂C₁-C₆ alkyl, CO₂C₃-C₈ cycloalkyl, OCOC₁-C₆ alkyl, OCOC₆-C₁₀ aryl, OCO(5-to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
   wherein the C₁-C₆ alkyl, C₁-C₆ haloalkyl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, halo, NR⁸R⁹, or oxo;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
   or one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a
   carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²) , and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆
   alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
   R^{1'} and R^{2'} are each independently selected from C₂-C₆ alkyl, C₁-C₆ haloalkyl, C₁-C₆ alkoxy, C₁-C₆ haloalkoxy, Cl, Br, I, CN, NO₂, COC₁-C₆ alkyl, CO-C₆-C₁₀ aryl, CO(5- to 10-membered heteroaryl), CO₂C₁-C₆ alkyl, CO₂C₃-C₈ cycloalkyl, OCOC₁-C₆ alkyl, OCOC₆-C₁₀ aryl, OCO(5-to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
   wherein the C₂-C₆ alkyl, C₁-C₆ haloalkyl, C₃-C₇ cycloalkyl and 3- to 7-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R^{1'} or R^{2'} C₃-C₇ cycloalkyl or of the R^{1'} or R^{2'} 3- to 7-membered heterocycloalkyl is further optionally independently substituted with one to three hydroxy, halo, NR⁸R⁹, or oxo;
      wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
   or one pair of R^{1'} and R^{2'} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
   R^{2"} is F or CH₃;
   or, when the compound of Formula AA is a compound of Formula AA-4, one pair of R¹ and R^{2"} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂ and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
   R⁶ and R⁷ are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
   wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
   or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹;
   R^{6'} and R^{7'} are each independently selected from unbranched C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
   wherein R^{6'} and R^{7'} are each optionally substituted with one or more substituents independently selected from hydroxy, halo, CN, oxo, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkoxy that R^{6'} or R^{7'} is substituted with is optionally substituted with one or more hydroxyl, C₆-C₁₀ aryl or NR⁸R⁹, or wherein R^{6'} or R^{7'} is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
   or one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹;
   R^{6"} is selected from C₁-C₆ alkyl, C₁-C₆ haloalkyl, C₁-C₆ alkoxy, C₁-C₆ haloalkoxy, halo, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5-to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl, 3- to 10-membered heterocycloalkyl, and C₂-C₆ alkenyl, wherein R^{6"} is optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R^{6"} is substituted with is optionally substituted with one or more hydroxyl, C₆-C₁₀ aryl or NR⁸R⁹, or wherein R^{6"} is optionally fused to a five- to -seven-membered carbocyclic ring or heterocyclic ring containing one or two heteroatoms independently selected from oxygen, sulfur and nitrogen;
      wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
   or one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹;
   R¹⁰ is C₁-C₆ alkyl;
   each of R⁸ and R⁹ at each occurrence is independently selected from hydrogen, C₁-C₆ alkyl, C₁-C₆haloalkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl or 3- to 7-membered heterocycloalkyl; or R⁸ and R⁹ taken together with the nitrogen they are attached to form a 3- to 7-membered ring optionally containing one or more heteroatoms in addition to the nitrogen they are attached to;
   R¹³ is C₁-C₆ alkyl, C₁-C₆ haloalkyl, C₆-C₁₀ aryl, or 5- to 10-membered heteroaryl;
   each of R¹¹ and R¹² at each occurrence is independently selected from hydrogen and C₁-C₆ alkyl; and
   R³ is selected from hydrogen, cyano, hydroxy, CO₂C₁-C₆ alkyl, C₁-C₆ alkoxy, C₁-C₆ alkyl, and 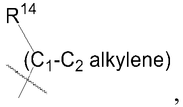 wherein the C₁-C₂ alkylene group is optionally substituted with oxo;
   R¹⁴ is hydrogen, C₁-C₆ alkyl, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶;
   or a pharmaceutically acceptable salt thereof.

### The Formula AA

In some embodiments, Formula AA is Formula AA-1

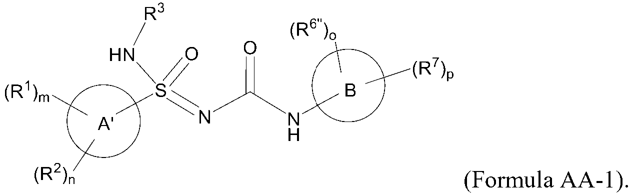

In some embodiments, Formula AA is Formula AA-2

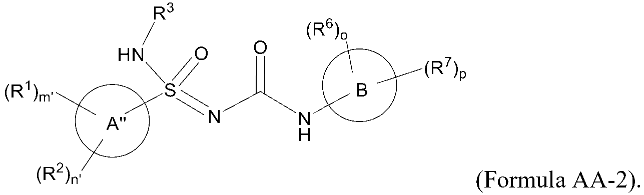

In some embodiments, Formula AA is Formula AA-3

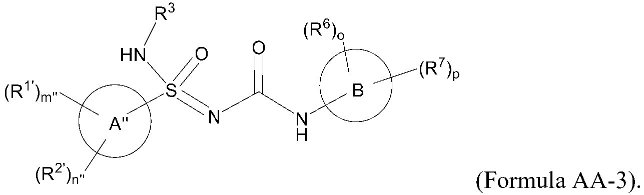

In some embodiments, Formula AA is Formula AA-4

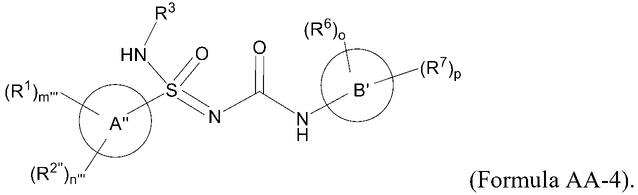

In some embodiments, Formula AA is Formula AA-5

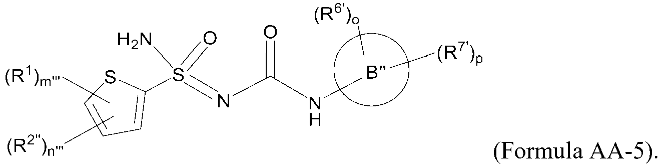

In some embodiments the variables shown in the formulae herein are as follows:
**The variables m, m', m", n, n', and n"**

In some embodiments m=0, 1, or 2.

In some embodiments m=0 or 1.

In some embodiments m=1 or 2.

In some embodiments m=0 or 2.

In some embodiments m=0.

In some embodiments m=1.

In some embodiments m=2.

In some embodiments n=0, 1, or 2.

In some embodiments n=0 or 1.

In some embodiments n=1 or 2.

In some embodiments n=0 or 2.

In some embodiments n=0.

In some embodiments n=1.

In some embodiments n=2.

In some embodiments, m=0 and n=0.

In some embodiments, m=1 and n=0.

In some embodiments, m=1 and n=1.

In some embodiments of the compound of Formula AA-1, m=1, and n=0.

In some embodiments of the compound of Formula AA-1, m=1, and n=1.

In some embodiments m'=0, 1, or 2.

In some embodiments m'=0 or 1.

In some embodiments m'=1 or 2.

In some embodiments m'=0 or 2.

In some embodiments m'=0.

In some embodiments m'=1.

In some embodiments m'=2.

In some embodiments n'=0, 1, or 2.

In some embodiments n'=0 or 1.

In some embodiments n'=1 or 2.

In some embodiments n'=0 or 2.

In some embodiments n'=0.

In some embodiments n'=1.

In some embodiments n'=2.

In some embodiments, m'=0 and n'=0.

In some embodiments, m'=1 and n'=0.

In some embodiments, m'=2 and n'=1.

In some embodiments, m'=1 and n'=2.
wherein the sum of m' and n' is 0, 1, or 3.

In some embodiments of the compound of Formula AA-2, m'=1 and n'=0.

In some embodiments m"=0, 1, or 2.

In some embodiments m"=0 or 1.

In some embodiments m"=1 or 2.

In some embodiments m''=0 or 2.

In some embodiments m''=0.

In some embodiments m"=1.

In some embodiments m''=2,
In some embodiments n"=0, 1, or 2.

In some embodiments n"=0 or 1.

In some embodiments n"=1 or 2.

In some embodiments n"=0 or 2.

In some embodiments n''=0.

In some embodiments n''=1.

In some embodiments n''=2.

In some embodiments, m"=0 and n''=2.

In some embodiments, m"=2 and n"=0.

In some embodiments, m"=1 and n"=1.
wherein the sum of m" and n" is 2.

In some embodiments of the compound of Formula AA-3, m"=1 and n"=1.

**The Ring A, A', and A" and substitutions on the ring A, A', and A"**

### The Ring A

In some embodiments, A is a 5- to 10-membered heteroaryl or a C₇-C₁₀ aryl

In some embodiments, A is a 5-membered heteroaryl.

In some embodiments, A is a 6- to 10-membered heteroaryl.

In some embodiments, A is a 6-membered heteroaryl.

In some embodiments, A is a C₆-C₁₀ aryl.

In some embodiments, A is a 5-membered heteroaryl comprising 1 heteroatom or heteroatomic group selected from N, NH, and NR¹.

In some embodiments, A is a 5-membered heteroaryl comprising 1 heteroatom selected from O and S, wherein the heteroatom is not bonded to the position of the heteroaryl that is bonded to the S(O)(NHR³)=N moiety.

In some embodiments, A is other than a moiety selected from the group consisting of: pyrazolyl, 2-thiophenyl, 2-furanyl, or phenyl monosubstituted with an R¹ or R² at the position *ortho* to the bond connecting the phenyl to the S(O)(NH) moiety.

In some embodiments, A is selected from the group consisting of: 3-furanyl, 3-thiophenyl, pyrrolyl, imidazoyl, triazolyl, thiazolyl, thiadiazolyl, oxadiazolyl, oxazolyl, pyridyl, pyrimidinyl, triazinyl, benzimidazolyl, benzothiophene, benzofuranyl, indazolyl, benzotriazolyl, quinolinyl, quinazolinyl, and benzotriazinyl.

In some embodiments, A is thiophenyl.

In some embodiments, A is thiazolyl.

In some embodiments, A is pyrazolyl.

In some embodiments, A is imidazolyl.

In some embodiments, A is pyrrolyl.

In some embodiments, A is oxazolyl.

In some embodiments, A is furanyl.

In some embodiments, A is isoxazolyl.

In some embodiments, A is isothiazolyl.

In some embodiments, A is triazolyl (e.g., 1,2,3-triazolyl or 1,2,4-triazolyl).

In some embodiments, A is pyridinyl.

In some embodiments, A is pyridimidinyl.

In some embodiments, A is pyrazinyl.

In some embodiments, A is pyridazinyl.

In some embodiments, A is triazinyl.

In some embodiments, A is phenyl.

In some embodiments, A is phenyl optionally substituted with 1 or 2 R¹ and optionally substituted with 1 or 2 R².

In some embodiments, A is naphthyl optionally substituted with 1 or 2 R¹ and optionally substituted with 1 or 2 R².

In some embodiments, A is furanyl optionally substituted with 1 or 2 R¹ and optionally substituted with 1 R².

In some embodiments, A is furanyl optionally substituted with 1 R¹ and optionally substituted with 1 or 2 R².

In some embodiments, A is thiophenyl optionally substituted with 1 or 2 R¹ and optionally substituted with 1 or 2 R².

In some embodiments, A is oxazolyl optionally substituted with 1 or 2 R¹ and optionally substituted with 1 or 2 R².

In some embodiments, A is thiazolyl optionally substituted with 1 or 2 R¹ and optionally substituted with 1 or 2 R².

In some embodiments, A is oxazolyl optionally substituted with 2 R¹ or optionally substituted with 2 R².

In some embodiments, A is thiazolyl optionally substituted with 2 R¹ or optionally substituted with 2 R².

In some embodiments, A is pyrazolyl optionally substituted with 1 or 2 R¹ and optionally substituted with 1 or 2 R².

In some embodiments, A is pyrazolyl optionally substituted with 1 R¹ and optionally substituted with 1 or 2 R².

In some embodiments, A is pyrazolyl optionally substituted with 1 or 2 R¹ and optionally substituted with 1 R².

In some embodiments, A is pyridyl optionally substituted with 1 or 2 R¹ and optionally substituted with 1 or 2 R².

In some embodiments, A is indazolyl optionally substituted with 1 or 2 R¹ and optionally substituted with 1 or 2 R².

In some embodiments, A is imidazolyl optionally substituted with 1 or 2 R¹ and optionally substituted with 1 or 2 R².

In some embodiments, A is pyrrolyl optionally substituted with 1 or 2 R¹ and optionally substituted with 1 or 2 R².

In some embodiments, A is oxazolyl optionally substituted with 1 or 2 R¹ and optionally substituted with 1 or 2 R².

In some embodiments, A is furanyl optionally substituted with 1 or 2 R¹ and optionally substituted with 1 or 2 R².

In some embodiments, A is isoxazolyl optionally substituted with 1 or 2 R¹ and optionally substituted with 1 or 2 R².

In some embodiments, A is isothiazolyl optionally substituted with 1 or 2 R¹ and optionally substituted with 1 or 2 R².

In some embodiments, A is triazolyl (e.g., 1,2,3-triazolyl or 1,2,4-triazolyl) optionally substituted with 1 R¹ and optionally substituted with 1 R².

In some embodiments, A is pyridinyl optionally substituted with 1 or 2 R¹ and optionally substituted with 1 or 2 R².

In some embodiments, A is pyridimidinyl optionally substituted with 1 or 2 R¹ and optionally substituted with 1 or 2 R².

In some embodiments, A is pyrazinyl optionally substituted with 1 or 2 R¹ and optionally substituted with 1 or 2 R².

In some embodiments, A is pyridazinyl optionally substituted with 1 or 2 R¹ and optionally substituted with 1 or 2 R².

In some embodiments, A is triazinyl optionally substituted with 1 or 2 R¹ and optionally substituted with 1 or 2 R².

In some embodiments, A is phenyl substituted with 1 R¹ and optionally substituted with 1 R².

In some embodiments, A is naphthyl substituted with 1 R¹ and optionally substituted with 1 R².

In some embodiments, A is furanyl substituted with 1 R¹ and optionally substituted with 1 R².

In some embodiments, A is thiophenyl substituted with 1 R¹ and optionally substituted with 1 R².

In some embodiments, A is oxazolyl substituted with 1 R¹ and optionally substituted with 1 R².

In some embodiments, A is thiazolyl substituted with 1 R¹ and optionally substituted with 1 R².

In some embodiments, A is pyrazolyl substituted with 1 R¹ and optionally substituted with 1 R².

In some embodiments, A is pyridyl substituted with 1 R¹ and optionally substituted with 1 R².

In some embodiments, A is indazolyl optionally substituted with 1 R¹ and optionally substituted with 1 R².

In some embodiments, A is phenyl substituted with 1 R¹ and substituted with 1 R².

In some embodiments, A is furanyl substituted with 1 R¹ and substituted with 1 R².

In some embodiments, A is thiophenyl substituted with 1 R¹ and substituted with 1 R².

In some embodiments, A is oxazolyl substituted with 1 R¹ and substituted with 1 R².

In some embodiments, A is thiazolyl substituted with 1 R¹ and substituted with 1 R².

In some embodiments, A is pyrazolyl substituted with 1 R¹ and substituted with 1 R².

In some embodiments, A is pyridyl substituted with 1 R¹ and substituted with 1 R².

In some embodiments, A is imidazolyl substituted with 1 R¹ and substituted with 1 R².

In some embodiments, A is pyrrolyl substituted with 1 R¹ and substituted with 1 R².

In some embodiments, A is oxazolyl substituted with 1 R¹ and substituted with 1 R².

In some embodiments, A is furanyl substituted with 1 R¹ and substituted with 1 R².

In some embodiments, A is isoxazolyl substituted with 1 R¹ and substituted with 1 R².

In some embodiments, A is isothiazolyl substituted with 1 R¹ and substituted with 1 R².

In some embodiments, A is triazolyl (e.g., 1,2,3-triazolyl or 1,2,4-triazolyl) substituted with 1 R¹ and substituted with 1 R².

In some embodiments, A is pyridimidinyl substituted with 1 R¹ and substituted with 1 R².

In some embodiments, A is pyrazinyl substituted with 1 R¹ and substituted with 1 R².

In some embodiments, A is pyridazinyl substituted with 1 R¹ and substituted with 1 R².

In some embodiments, A is triazinyl substituted with 1 R¹ and substituted with 1 R².

In some embodiments, A is phenyl, m is 0 or 1, and n is 0, 1, or 2.

In some embodiments, A is furanyl, m is 0 or 1, and n is 0, 1, or 2.

In some embodiments, A is thiophenyl, m is 0 or 1, and n is 0, 1, or 2.

In some embodiments, A is oxazolyl, m is 0 or 1, and n is 0, 1, or 2.

In some embodiments, A is thiazolyl, m is 0 or 1, and n is 0, 1, or 2.

In some embodiments, A is pyrazolyl, m is 0 or 1, and n is 0, 1, or 2.

In some embodiments, A is pyridyl, m is 0 or 1, and n is 0, 1, or 2.

In some embodiments, A is indazolyl, m is 0 or 1, and n is 0, 1, or 2.

In some embodiments, A is phenyl, m is 0, and n is 0 or 1.

In some embodiments, A is furanyl, m is 0, and n is 0 or 1.

In some embodiments, A is thiophenyl, m is 0, and n is 0 or 1.

In some embodiments, A is oxazolyl, m is 0, and n is 0 or 1.

In some embodiments, A is thiazolyl, m is 0, and n is 0 or 1.

In some embodiments, A is pyrazolyl, m is 0, and n is 0 or 1.

In some embodiments, A is pyridyl, m is 0, and n is 0 or 1.

In some embodiments, A is thiazolyl, m is 1, and n is 1.

In some embodiments, A is pyrazolyl, m is 1 or 2, and n is 1 or 2.

In some embodiments, A is imidazolyl, m is 1 or 2, and n is 1 or 2.

In some embodiments, A is pyrrolyl, m is 1 or 2, and n is 1 or 2.

In some embodiments, A is oxazolyl, m is 1, and n is 1.

In some embodiments, A is furanyl, m is 1 or 2, and n is 1 or 2.

In some embodiments, A is isoxazolyl, m is 1, and n is 1.

In some embodiments, A is isothiazolyl, m is 1, and n is 1..

In some embodiments, A is triazolyl (e.g., 1,2,3-triazolyl or 1,2,4-triazolyl), m is 1, and n is 1.

In some embodiments, A is pyridinyl, m is 1 or 2, and n is 1 or 2.

In some embodiments, A is pyridimidinyl, m is 1 or 2, and n is 1 or 2.

In some embodiments, A is pyrazinyl, m is 1 or 2, and n is 1 or 2.

In some embodiments, A is pyridazinyl, m is 1 or 2, and n is 1 or 2.

In some embodiments, A is triazinyl, m is 1, and n is 1.

In some embodiments, A is one of the rings disclosed hereinbelow optionally substituted as disclosed hereinbelow, wherein in each case the bond that is shown as being broken by the wavy line 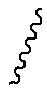 connects A to the S(O)(NHR³)=N moiety of Formula AA.

In some embodiments, the optionally substituted ring A 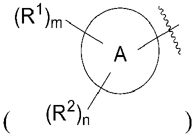 is

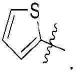

In some embodiments, the optionally substituted ring A is

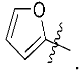

In some embodiments, the optionally substituted ring A is

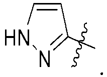

In some embodiments, the optionally substituted ring A is

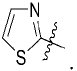

In some embodiments, the optionally substituted ring A is

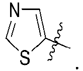

In some embodiments, the optionally substituted ring A is

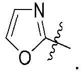

In some embodiments, the optionally substituted ring A is

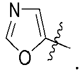

In some embodiments, the optionally substituted ring A is

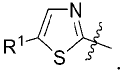

In some embodiments, the optionally substituted ring A is

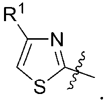

In some embodiments, the optionally substituted ring A is

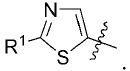

In some embodiments, the optionally substituted ring A is

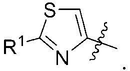

In some embodiments, the optionally substituted ring A is

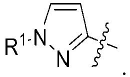

In some embodiments, the optionally substituted ring A is

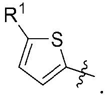

In some embodiments, the optionally substituted ring A is

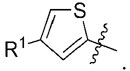

In some embodiments, the optionally substituted ring A is

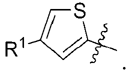

In some embodiments, the optionally substituted ring A is

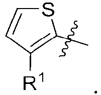

In some embodiments, the optionally substituted ring A is

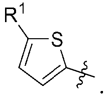

In some embodiments, the optionally substituted ring A is

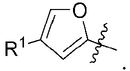

In some embodiments, the optionally substituted ring A is

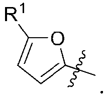

In some embodiments, the optionally substituted ring A is

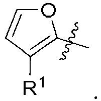

In some embodiments, the optionally substituted ring A is

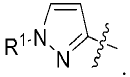

In some embodiments, the optionally substituted ring A is

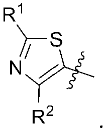

In some embodiments, the optionally substituted ring A is

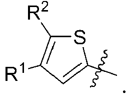

In some embodiments, the optionally substituted ring A is

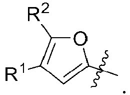

In some embodiments, the optionally substituted ring A is

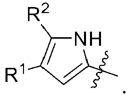

In some embodiments, the optionally substituted ring A is

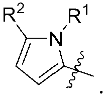

In some embodiments, the optionally substituted ring A is

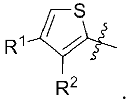

In some embodiments, the optionally substituted ring A is

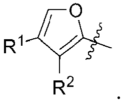

In some embodiments, the optionally substituted ring A is

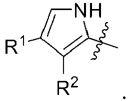

In some embodiments, the optionally substituted ring A is

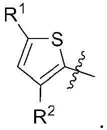

In some embodiments, the optionally substituted ring A is

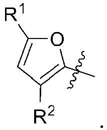

In some embodiments, the substituted ring A is

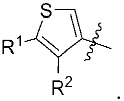

In some embodiments, the substituted ring A is

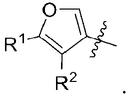

In some embodiments, the substituted ring A is

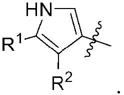

In some embodiments, the substituted ring A is

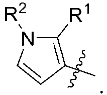

In some embodiments, the substituted ring A is

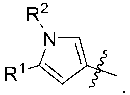

In some embodiments, the optionally substituted ring A is

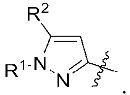

In some embodiments, the optionally substituted ring A is

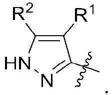

In some embodiments, the optionally substituted ring A is

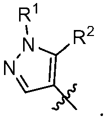

In some embodiments, the optionally substituted ring A is

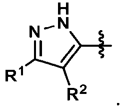

In some embodiments, the optionally substituted ring A is

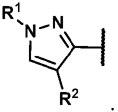

In some embodiments, the optionally substituted ring A is

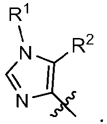

In some embodiments, the optionally substituted ring A is

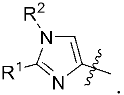

In some embodiments, the optionally substituted ring A is

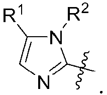

In some embodiments, the optionally substituted ring A is

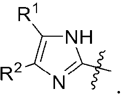

In some embodiments, the optionally substituted ring A is

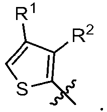

In some embodiments, the optionally substituted ring A is

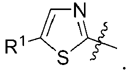

In some embodiments, the optionally substituted ring A is

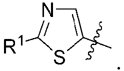

In some embodiments, the optionally substituted ring A is

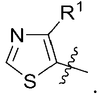

In some embodiments, the optionally substituted ring A is

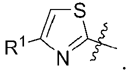

In some embodiments, the optionally substituted ring A is

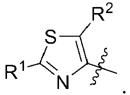

In some embodiments, the optionally substituted ring A is

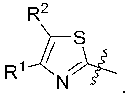

In some embodiments, the optionally substituted ring A is

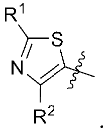

In some embodiments, the optionally substituted ring A is

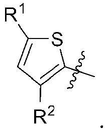

In some embodiments, the optionally substituted ring A is

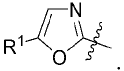

In some embodiments, the optionally substituted ring A is

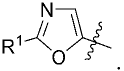

In some embodiments, the optionally substituted ring A is

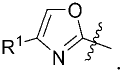

In some embodiments, the optionally substituted ring A is

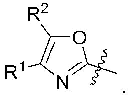

In some embodiments, the optionally substituted ring A is

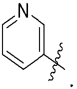

In some embodiments, the optionally substituted ring A is

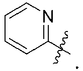

In some embodiments, the optionally substituted ring A is

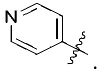

In some embodiments, the optionally substituted ring A is

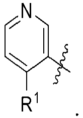

In some embodiments, the optionally substituted ring A is

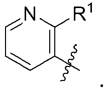

In some embodiments, the optionally substituted ring A is

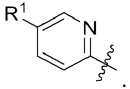

In some embodiments, the optionally substituted ring A is

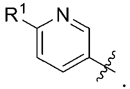

In some embodiments, the optionally substituted ring A is

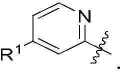

In some embodiments, the optionally substituted ring A is

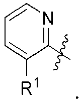

In some embodiments, the optionally substituted ring A is

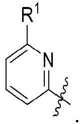

In some embodiments, the optionally substituted ring A is

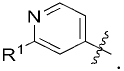

In some embodiments, the optionally substituted ring A is

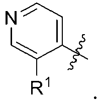

In some embodiments, the optionally substituted ring A is

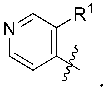

In some embodiments, the optionally substituted ring A is

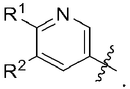

In some embodiments, the optionally substituted ring A is

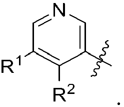

In some embodiments, the optionally substituted ring A is

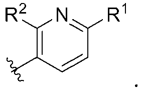

In some embodiments, the optionally substituted ring A is

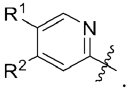

In some embodiments, the optionally substituted ring A is

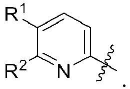

In some embodiments, the optionally substituted ring A is

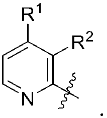

In some embodiments, the optionally substituted ring A is

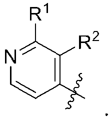

In some embodiments, the optionally substituted ring A is

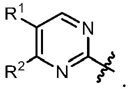

In some embodiments, the optionally substituted ring A is

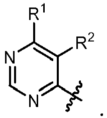

In some embodiments, the optionally substituted ring A is

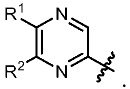

In some embodiments, the optionally substituted ring A is

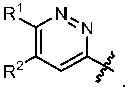

In some embodiments, the optionally substituted ring A is

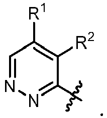

In some embodiments, the optionally substituted ring A is

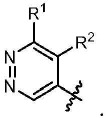

In some embodiments, the optionally substituted ring A is

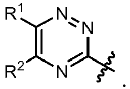

In some embodiments, the optionally substituted ring A is

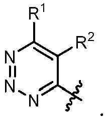

In some embodiments, the optionally substituted ring A is

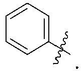

In some embodiments, the optionally substituted ring A is

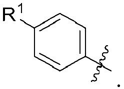

In some embodiments, the optionally substituted ring A is

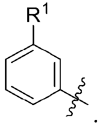

In some embodiments, the optionally substituted ring A is

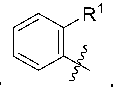

In some embodiments, the optionally substituted ring A is

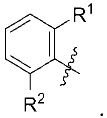

In some embodiments, the optionally substituted ring A is

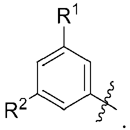

In some embodiments, the optionally substituted ring A is

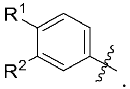

In some embodiments, the optionally substituted ring A is

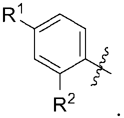

In some embodiments, the optionally substituted ring A is

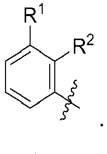

In some embodiments, the optionally substituted ring A is

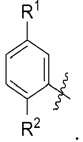

In some embodiments, the optionally substituted ring A is

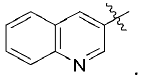

In some embodiments, the optionally substituted ring A is

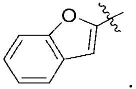

In some embodiments, the optionally substituted ring A is

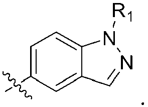

In some embodiments, the optionally substituted ring A is

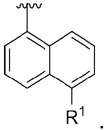

In some embodiments, the optionally substituted ring A is

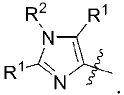

In some embodiments, the optionally substituted ring A is

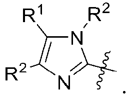

In some embodiments, the optionally substituted ring A is

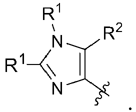

In some embodiments, the optionally substituted ring A is

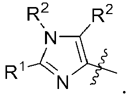

In some embodiments, the optionally substituted ring A is

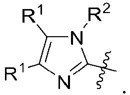

In some embodiments, the optionally substituted ring A is

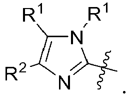

In some embodiments, the optionally substituted ring A is

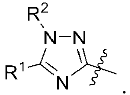

In some embodiments, the optionally substituted ring A is

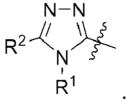

In some embodiments, the optionally substituted ring A is

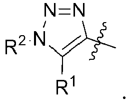

In some embodiments, the optionally substituted ring A is

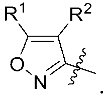

In some embodiments, the optionally substituted ring A is

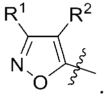

In some embodiments, the optionally substituted ring A is

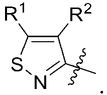

In some embodiments, the optionally substituted ring A is

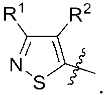

In some embodiments, the substituted ring A is

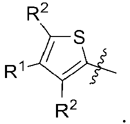

In some embodiments, the substituted ring A is

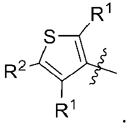

In some embodiments, the substituted ring A is

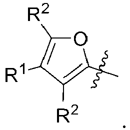

In some embodiments, the substituted ring A is

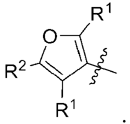

In some embodiments, the substituted ring A is

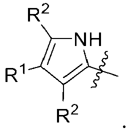

In some embodiments, the substituted ring A is

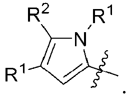

In some embodiments, the substituted ring A is

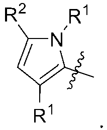

In some embodiments, the substituted ring A is

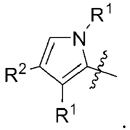

In some embodiments, the substituted ring A is

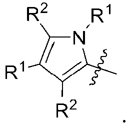

In some embodiments, the substituted ring A is

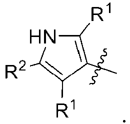

In some embodiments, the substituted ring A is

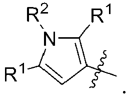

In some embodiments, the substituted ring A is

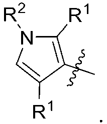

In some embodiments, the substituted ring A is

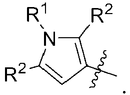

In some embodiments, the substituted ring A is

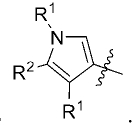

In some embodiments, the substituted ring A is

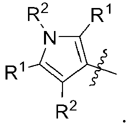

In some embodiments, the optionally substituted ring A is

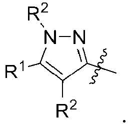

In some embodiments, the optionally substituted ring A is

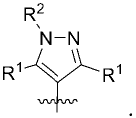

In some embodiments, the optionally substituted ring A is

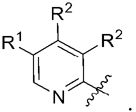

In some embodiments, the optionally substituted ring A is

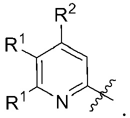

In some embodiments, the optionally substituted ring A is

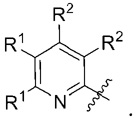

In some embodiments, the optionally substituted ring A is

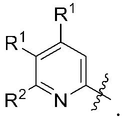

In some embodiments, the optionally substituted ring A is

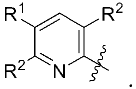

In some embodiments, the optionally substituted ring A is

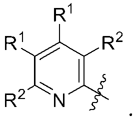

In some embodiments, the optionally substituted ring A is

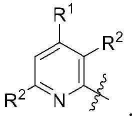

In some embodiments, the optionally substituted ring A is

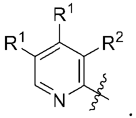

In some embodiments, the optionally substituted ring A is

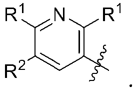

In some embodiments, the optionally substituted ring A is

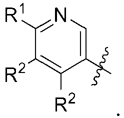

In some embodiments, the optionally substituted ring A is

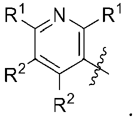

In some embodiments, the optionally substituted ring A is

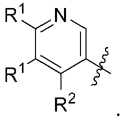

In some embodiments, the optionally substituted ring A is

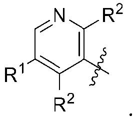

In some embodiments, the optionally substituted ring A is

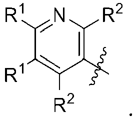

In some embodiments, the optionally substituted ring A is

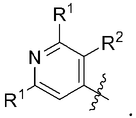

In some embodiments, the optionally substituted ring A is

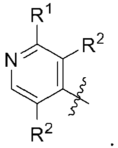

In some embodiments, the optionally substituted ring A is

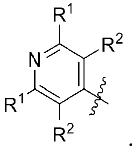

In some embodiments, the optionally substituted ring A is

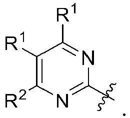

In some embodiments, the optionally substituted ring A is

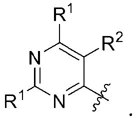

In some embodiments, the optionally substituted ring A is

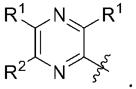

In some embodiments, the optionally substituted ring A is

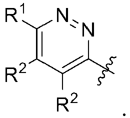

In some embodiments, the optionally substituted ring A is

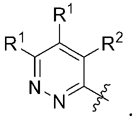

In some embodiments, the optionally substituted ring A is

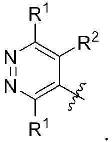

In some embodiments, the optionally substituted ring A is

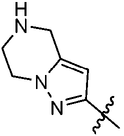

In some embodiments, the optionally substituted ring A is

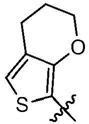

In some embodiments, the optionally substituted ring A is

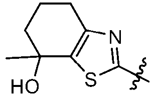

In some embodiments, the optionally substituted ring A is selected from the group consisting of:

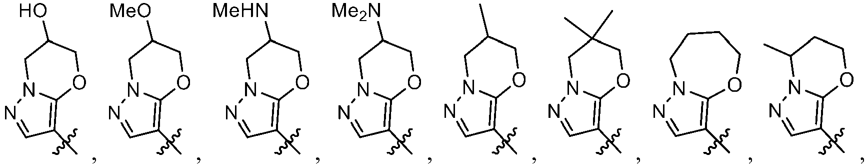

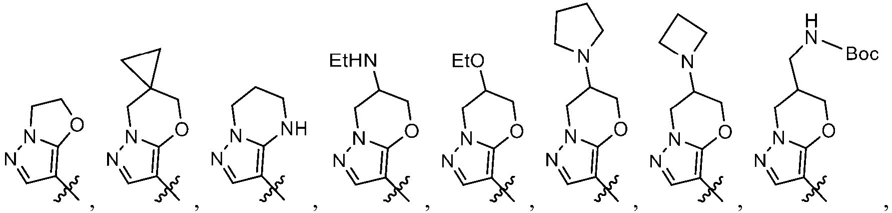

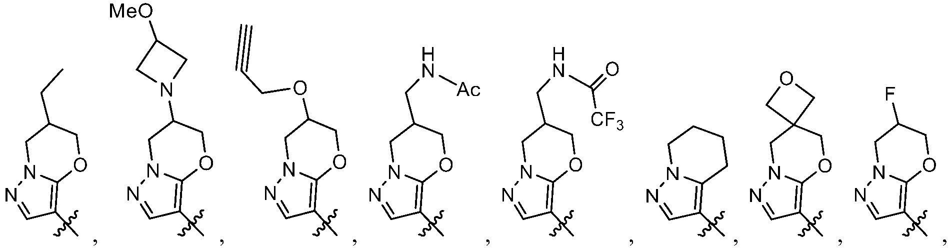

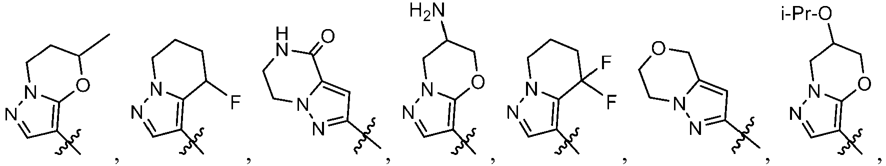

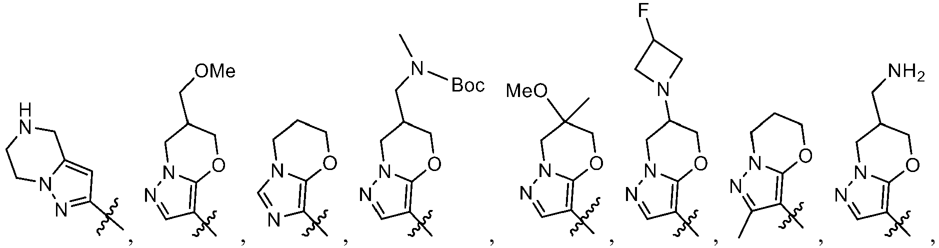

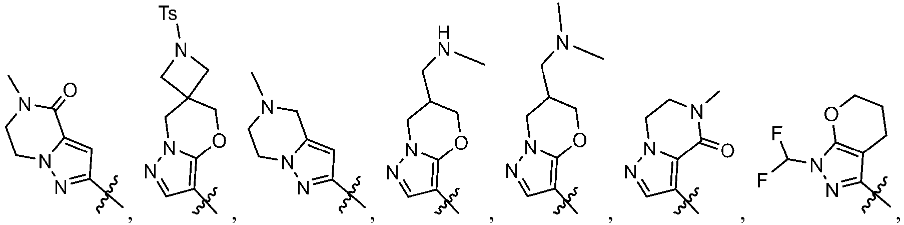

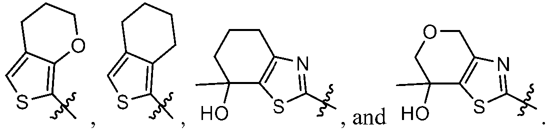

In some embodiments, the optionally substituted ring A is selected from the group consisting of:

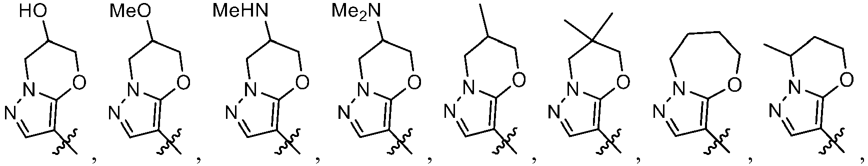

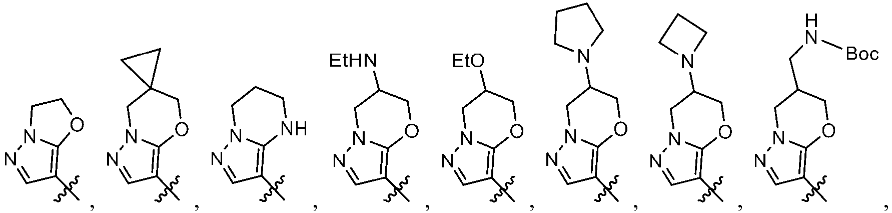

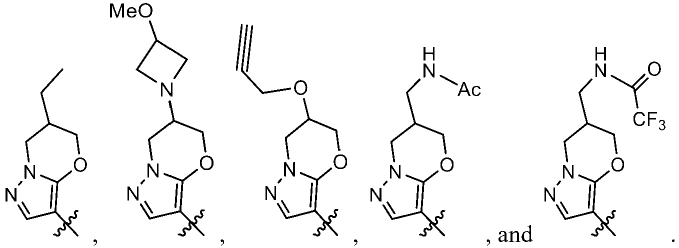

In some embodiments, the optionally substituted ring A is selected from the group consisting of:

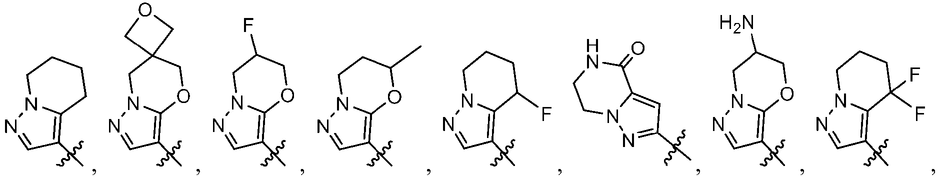

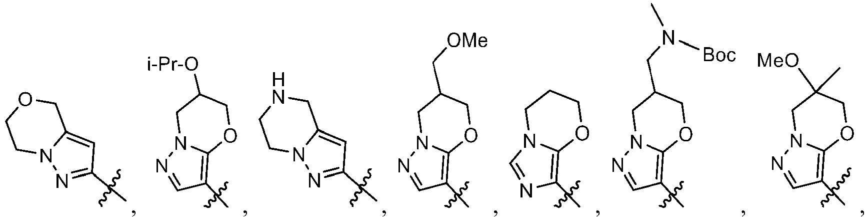

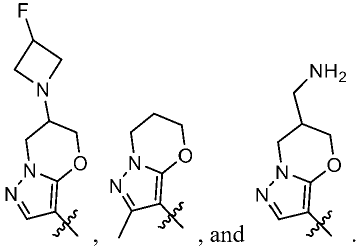

In some embodiments, the optionally substituted ring A is selected from the group consisting of:

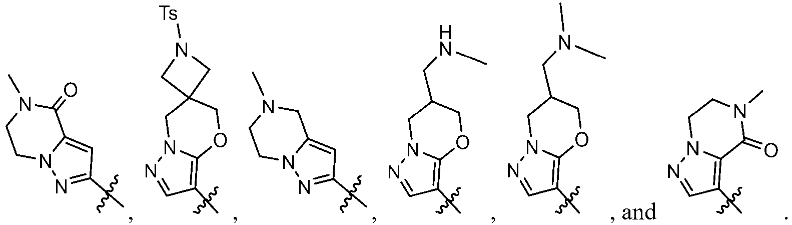

In some embodiments, the optionally substituted ring A is selected from the group consisting of:

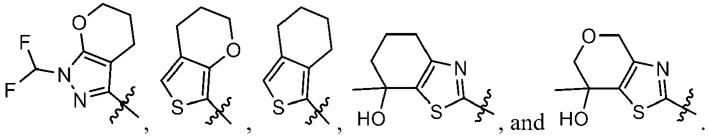

In some embodiments, the optionally substituted ring A is selected from the following:

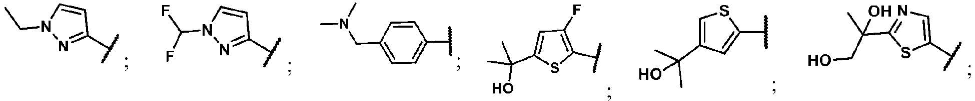

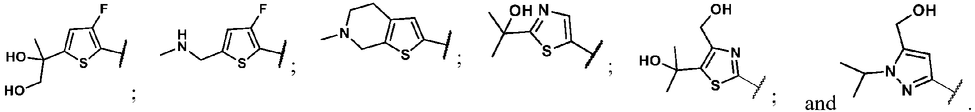

In some embodiments of the compound of Formula AA, when ring A is phenyl, then R¹ and R² are each independently selected from C₃ alkyl, C₅-C₆ alkyl, C₁-C₆ haloalkyl, C₁-C₆ alkoxy, C₁-C₆ haloalkoxy, F, I, CN, NO₂, COC₂-C₆ alkyl, CO-C₆-C₁₀ aryl, CO(5- to 10-membered heteroaryl), CO₂C₁-C₆ alkyl, CO₂C₃-C₈ cycloalkyl, OCOC₂-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ haloalkyl, C₃-C₇ cycloalkyl and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further
optionally independently substituted with one to three hydroxy, halo, NR⁸R⁹, or oxo; wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄ or C₆-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA, when ring A is pyridyl, then R¹ and R² are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO-C₆-C₁₀ aryl, CO(5- to 10-membered heteroaryl), CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₂-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl, and 3- to 7-membered heterocycloalkyl,
wherein the C₁-C₆ alkyl, C₁-C₆ haloalkyl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 5-membered heterocycloalkyl, 5-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, halo, NR⁸R⁹, or oxo;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

### The Ring A' when Formula AA is Formula AA-1

In some embodiments, A' is a 6- to 10-membered (e.g., 6-membered) heteroaryl or a C₆-C₁₀ (e.g., C₆) monocyclic or bicyclic aryl, such as phenyl.

In some embodiments, A' is a 6- to 10-membered (e.g., 6-membered) heteroaryl or a C₇-C₁₀ (e.g., C₆) monocyclic or bicyclic aryl.

In some embodiments, A' is a 6- to 10-membered (e.g., 6-membered) heteroaryl.

In some embodiments of the compound of Formula AA-1, A' is a C₆-C₁₀ aryl.

In some embodiments, A' is a 5-membered heteroaryl comprising 2 or more heteroatoms.

In some embodiments, A' is a 5-membered heteroaryl comprising 1 heteroatom and/or heteroatomic group selected from N, NH, and NR¹.

In some embodiments, A' is a 5-membered heteroaryl comprising 1 heteroatom selected from O and S, wherein the heteroatom is not bonded to the position of the heteroaryl that is bonded to the S(O)(NHR³)=N moiety.

In some embodiments, A' is a 5-membered heteroaryl containing a sulfur and optionally one or more nitrogens.

In some embodiments, A' is a C₆-C₁₀ aryl.

In some embodiments, A' is other than pyrazolyl.

In some embodiments, A' is thiophenyl (e.g., thiophen-3-yl).

In some embodiments, A' is thiazolyl (e.g., thiazol-2-yl or thiazol-3-yl).

In some embodiments, A' is pyrazolyl (e.g., pyrazol-2-yl).

In some embodiments, A' is imidazolyl (e.g., imidazol-2-yl).

In some embodiments, A' is naphthyl.

In some embodiments, A' is furanyl.

In some embodiments, A' is pyridyl.

In some embodiments, A' is indazolyl.

In some embodiments, A' is phenyl, m is 0 or 1, and n is 0, 1, or 2.

In some embodiments, A' is furanyl, m is 0 or 1, and n is 0, 1, or 2.

In some embodiments, A' is thiophenyl, m is 0 or 1, and n is 0, 1, or 2.

In some embodiments, A' is thiazolyl, m is 0 or 1, and n is 0, 1, or 2.

In some embodiments, A' is pyrazolyl, m is 0 or 1, and n is 0, 1, or 2.

In some embodiments, A' is pyridyl, m is 0 or 1, and n is 0, 1, or 2.

In some embodiments, A' is indazolyl, m is 0 or 1, and n is 0, 1, or 2.

In some embodiments, A' is thiazolyl, m is 1, and n is 1.

In some embodiments, A' is pyrazolyl, m is 1 or 2, and n is 1 or 2.

In some embodiments, A' is imidazolyl, m is 1 or 2, and n is 1 or 2.

In some embodiments, A' is pyrrolyl, m is 1 or 2, and n is 1 or 2.

In some embodiments, A' is oxazolyl, m is 1, and n is 1.

In some embodiments, A' is furanyl, m is 1 or 2, and n is 1 or 2.

In some embodiments, A' is isoxazolyl, m is 1, and n is 1.

In some embodiments, A' is isothiazolyl, m is 1, and n is 1..

In some embodiments, A' is triazolyl (e.g., 1,2,3-triazolyl or 1,2,4-triazolyl), m is 1, and n is 1.

In some embodiments, A' is pyridinyl, m is 1 or 2, and n is 1 or 2.

In some embodiments, A' is pyridimidinyl, m is 1 or 2, and n is 1 or 2.

In some embodiments, A' is pyrazinyl, m is 1 or 2, and n is 1 or 2.

In some embodiments, A' is pyridazinyl, m is 1 or 2, and n is 1 or 2.

In some embodiments, A' is triazinyl, m is 1, and n is 1.

In some embodiments, A' is one of the rings disclosed hereinbelow optionally substituted as disclosed hereinbelow, wherein in each case the bond that is shown as being broken by the wavy line 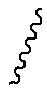 connects A to the S(O)(NHR³)=N moiety of Formula AA.

In some embodiments, the optionally substituted ring A' 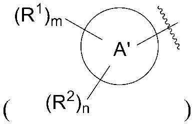 is

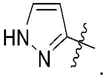

In some embodiments, the optionally substituted ring A' is

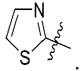

In some embodiments, the optionally substituted ring A' is

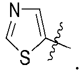

In some embodiments, the optionally substituted ring A' is

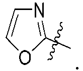

In some embodiments, the optionally substituted ring A' is

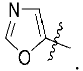

In some embodiments, the optionally substituted ring A' is

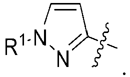

In some embodiments, the optionally substituted ring A' is

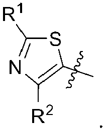

In some embodiments, the optionally substituted ring A' is

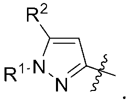

In some embodiments, the optionally substituted ring A' is

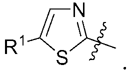

In some embodiments, the optionally substituted ring A' is

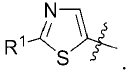

In some embodiments, the optionally substituted ring A' is

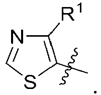

In some embodiments, the optionally substituted ring A' is

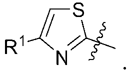

In some embodiments, the optionally substituted ring A' is

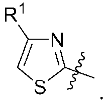

In some embodiments, the optionally substituted ring A' is

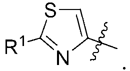

In some embodiments, the optionally substituted ring A' is

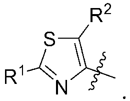

In some embodiments, the optionally substituted ring A' is

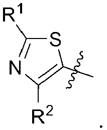

In some embodiments, the optionally substituted ring A' is

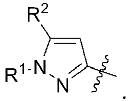

In some embodiments, the optionally substituted ring A' is

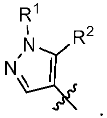

In some embodiments, the optionally substituted ring A' is

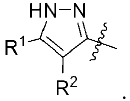

In some embodiments, the optionally substituted ring A' is

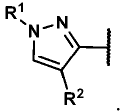

In some embodiments, the optionally substituted ring A' is

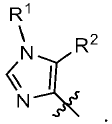

In some embodiments, the optionally substituted ring A' is

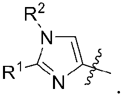

In some embodiments, the optionally substituted ring A' is

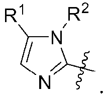

In some embodiments, the optionally substituted ring A' is

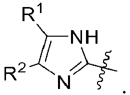

In some embodiments, the optionally substituted ring A' is

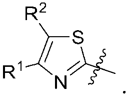

In some embodiments, the optionally substituted ring A' is

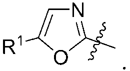

In some embodiments, the optionally substituted ring A' is

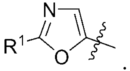

In some embodiments, the optionally substituted ring A' is

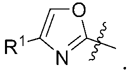

In some embodiments, the optionally substituted ring A' is

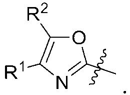

In some embodiments, the optionally substituted ring A' is

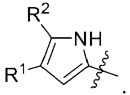

In some embodiments, the optionally substituted ring A' is

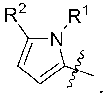

In some embodiments, the optionally substituted ring A' is

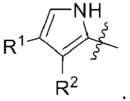

In so In some embodiments, the substituted ring A' is

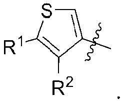

In some embodiments, the substituted ring A' is

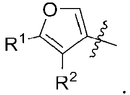

In some embodiments, the substituted ring A' is

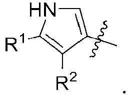

In some embodiments, the substituted ring A' is

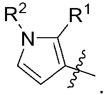

In some embodiments, the substituted ring A' is

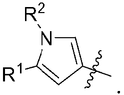

In some embodiments, the optionally substituted ring A' is

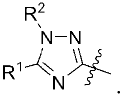

In some embodiments, the optionally substituted ring A' is

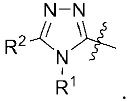

In some embodiments, the optionally substituted ring A' is

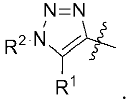

In some embodiments, the optionally substituted ring A' is

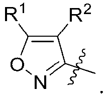

In some embodiments, the optionally substituted ring A' is

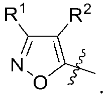

In some embodiments, the optionally substituted ring A' is

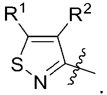

In some embodiments, the optionally substituted ring A' is

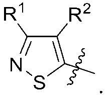

me embodiments, the optionally substituted ring A' is

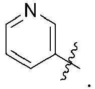

In some embodiments, the optionally substituted ring A' is

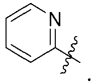

In some embodiments, the optionally substituted ring A' is

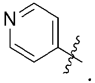

In some embodiments, the optionally substituted ring A' is

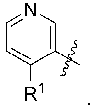

In some embodiments, the optionally substituted ring A' is

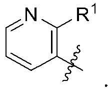

In some embodiments, the optionally substituted ring A' is

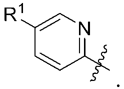

In some embodiments, the optionally substituted ring A' is

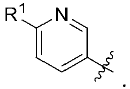

In some embodiments, the optionally substituted ring A' is

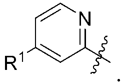

In some embodiments, the optionally substituted ring A' is

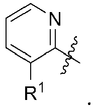

In some embodiments, the optionally substituted ring A' is

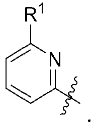

In some embodiments, the optionally substituted ring A' is

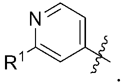

In some embodiments, the optionally substituted ring A' is

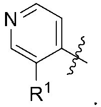

In some embodiments, the optionally substituted ring A' is

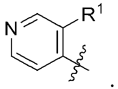

In some embodiments, the optionally substituted ring A' is

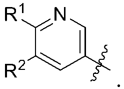

In some embodiments, the optionally substituted ring A' is

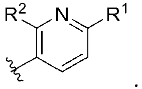

In some embodiments, the optionally substituted ring A' is

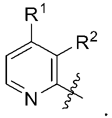

In some embodiments, the optionally substituted ring A' is

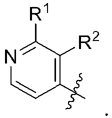

In some embodiments, the optionally substituted ring A' is

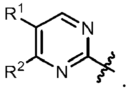

In some embodiments, the optionally substituted ring A' is

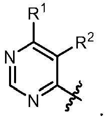

In some embodiments, the optionally substituted ring A' is

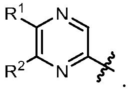

In some embodiments, the optionally substituted ring A' is

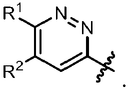

In some embodiments, the optionally substituted ring A' is

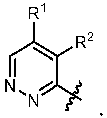

In some embodiments, the optionally substituted ring A' is

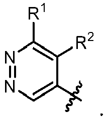

In some embodiments, the optionally substituted ring A' is

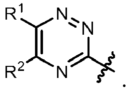

In some embodiments, the optionally substituted ring A' is

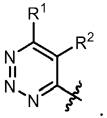

In some embodiments, the optionally substituted ring A' is

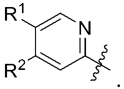

In some embodiments, the optionally substituted ring A' is

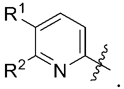

In some embodiments, the optionally substituted ring A' is

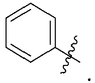

In some embodiments, the optionally substituted ring A' is

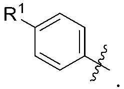

In some embodiments, the optionally substituted ring A' is

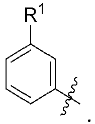

In some embodiments, the optionally substituted ring A' is

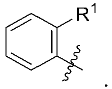

In some embodiments, the optionally substituted ring A' is

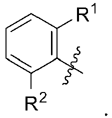

In some embodiments, the optionally substituted ring A' is

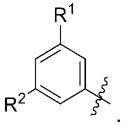

In some embodiments, the optionally substituted ring A' is

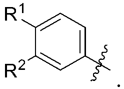

In some embodiments, the optionally substituted ring A' is

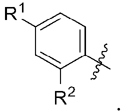

In some embodiments, the optionally substituted ring A' is

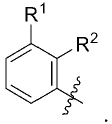

In some embodiments, the optionally substituted ring A' is

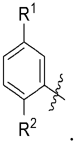

In some embodiments, the optionally substituted ring A' is

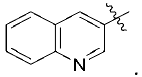

In some embodiments, the optionally substituted ring A' is

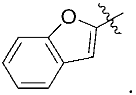

In some embodiments, the optionally substituted ring A' is

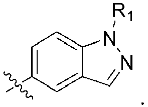

In some embodiments, the optionally substituted ring A' is

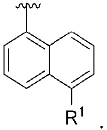

In some embodiments, the optionally substituted ring A' is

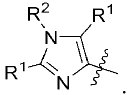

In some embodiments, the optionally substituted ring A' is

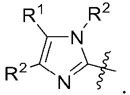

In some embodiments, the substituted ring A' is

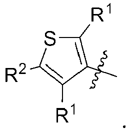

In some embodiments, the substituted ring A' is

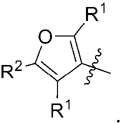

In some embodiments, the substituted ring A' is

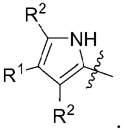

In some embodiments, the substituted ring A' is

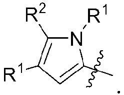

In some embodiments, the substituted ring A' is

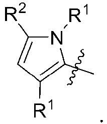

In some embodiments, the substituted ring A' is

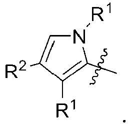

In some embodiments, the substituted ring A' is

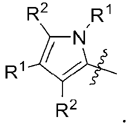

In some embodiments, the substituted ring A' is

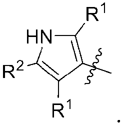

In some embodiments, the substituted ring A' is

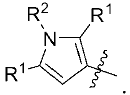

In some embodiments, the substituted ring A' is

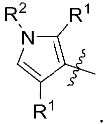

In some embodiments, the substituted ring A' is

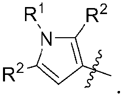

In some embodiments, the substituted ring A' is

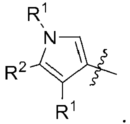

In some embodiments, the substituted ring A' is

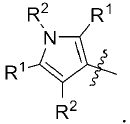

In some embodiments, the optionally substituted ring A' is

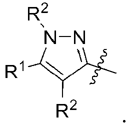

In some embodiments, the optionally substituted ring A' is

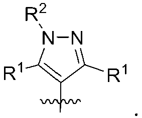

In some embodiments, the optionally substituted ring A' is

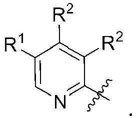

In some embodiments, the optionally substituted ring A' is

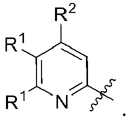

In some embodiments, the optionally substituted ring A' is

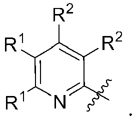

In some embodiments, the optionally substituted ring A' is

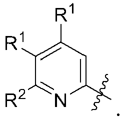

In some embodiments, the optionally substituted ring A' is

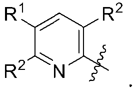

In some embodiments, the optionally substituted ring A' is

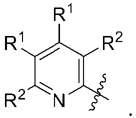

In some embodiments, the optionally substituted ring A' is

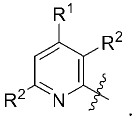

In some embodiments, the optionally substituted ring A' is

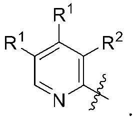

In some embodiments, the optionally substituted ring A' is

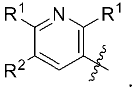

In some embodiments, the optionally substituted ring A' is

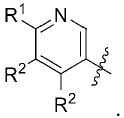

In some embodiments, the optionally substituted ring A' is

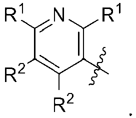

In some embodiments, the optionally substituted ring A' is

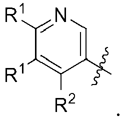

In some embodiments, the optionally substituted ring A' is

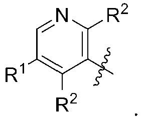

In some embodiments, the optionally substituted ring A' is

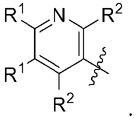

In some embodiments, the optionally substituted ring A' is

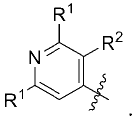

In some embodiments, the optionally substituted ring A' is

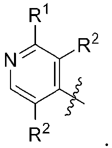

In some embodiments, the optionally substituted ring A' is

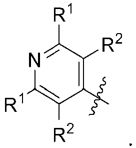

In some embodiments, the optionally substituted ring A' is

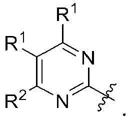

In some embodiments, the optionally substituted ring A' is

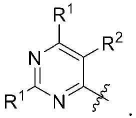

In some embodiments, the optionally substituted ring A' is

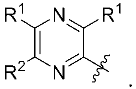

In some embodiments, the optionally substituted ring A' is

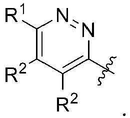

In some embodiments, the optionally substituted ring A' is

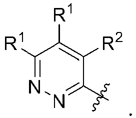

In some embodiments, the optionally substituted ring A' is

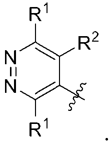

In some embodiments, the optionally substituted ring A' is selected from the group consisting of:

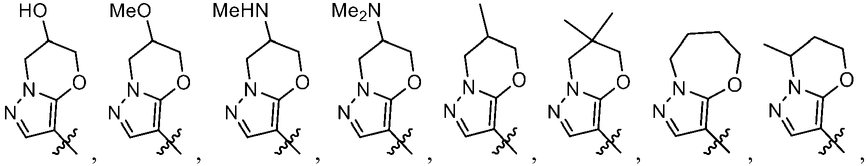

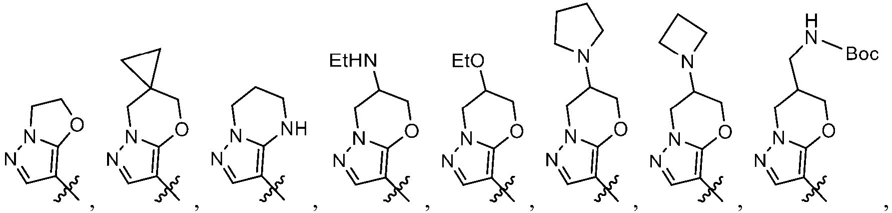

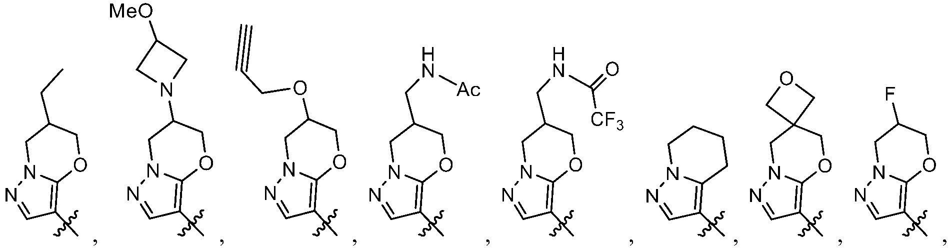

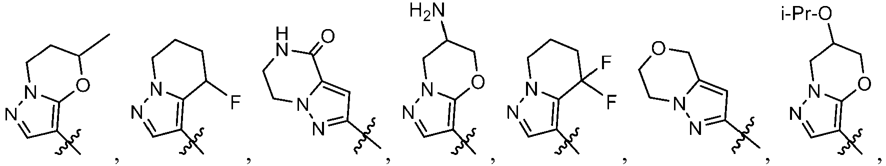

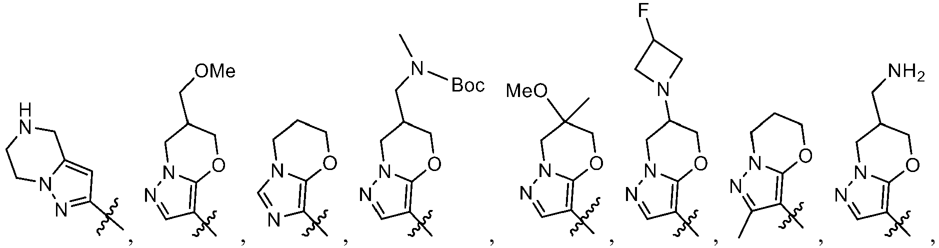

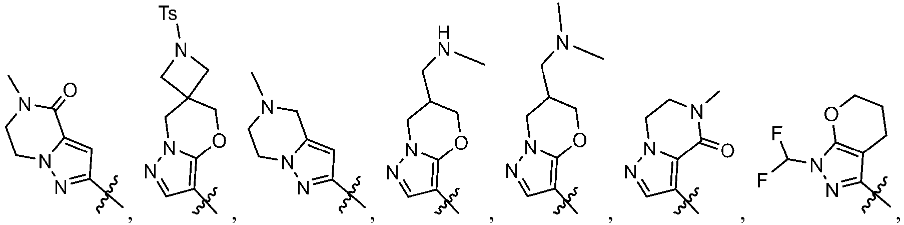

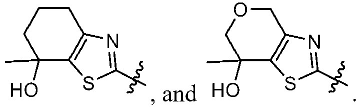

In some embodiments, the optionally substituted ring A' is selected from the group consisting of:

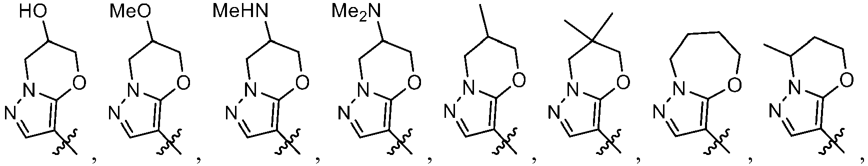

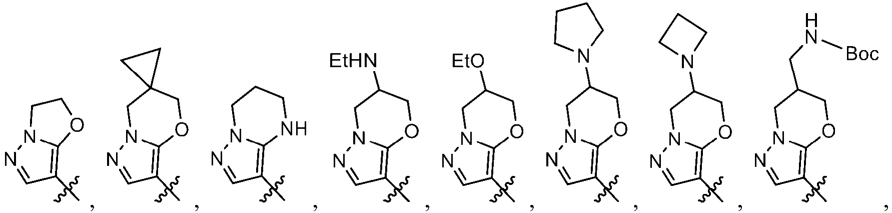

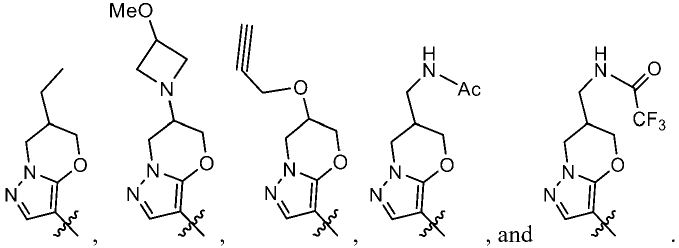

In some embodiments, the optionally substituted ring A' is selected from the group consisting of:

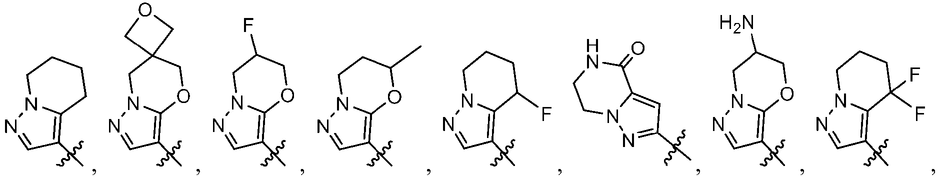

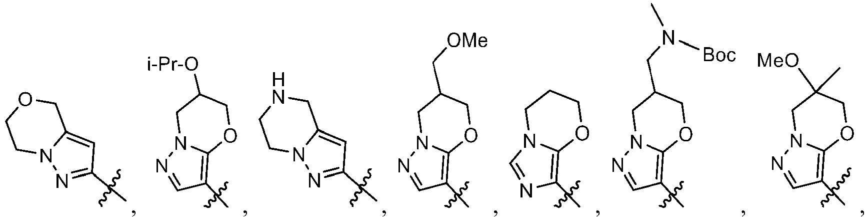

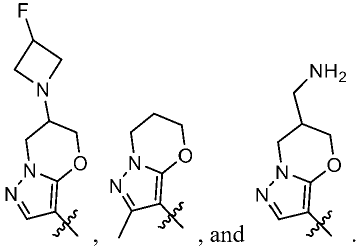

In some embodiments, the optionally substituted ring A' is selected from the group consisting of:

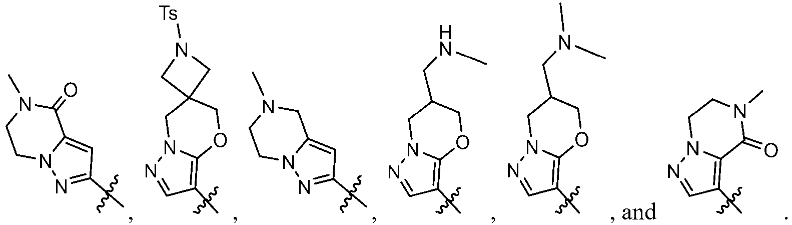

In some embodiments, the optionally substituted ring A' is selected from the group consisting of:

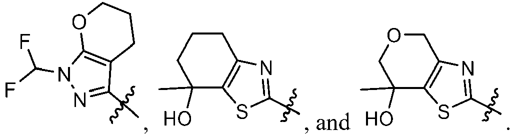

In some embodiments, the optionally substituted ring A' is selected from the following:

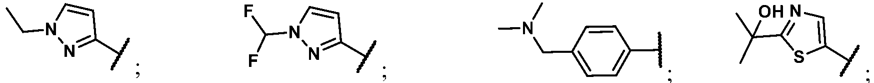

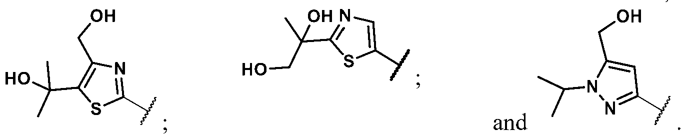

In some embodiments of the compound of Formula AA-1, when ring A' is phenyl, then R¹ and R² are each independently selected from C₃ alkyl, C₅-C₆ alkyl, C₁-C₆ haloalkyl, C₁-C₆ alkoxy, C₁-C₆ haloalkoxy, F, I, CN, NO₂, COC₂-C₆ alkyl, CO-C₆-C₁₀ aryl, CO(5- to 10-membered heteroaryl), CO₂C₁-C₆ alkyl, CO₂C₃-C₈ cycloalkyl, OCOC₂-C₆ alkyl, OCOC₆-C₁₀ aryl, OCO(5-to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ haloalkyl, C₃-C₇ cycloalkyl and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further
optionally independently substituted with one to three hydroxy, halo, NR⁸R⁹, or oxo; wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄ or C₆-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA-1, when ring A is pyridyl, then R¹ and R² are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO-C₆-C₁₀ aryl, CO(5- to 10-membered heteroaryl), CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₂-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl, and 3- to 7-membered heterocycloalkyl,
wherein the C₁-C₆ alkyl, C₁-C₆ haloalkyl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 5-membered heterocycloalkyl, 5-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, halo, NR⁸R⁹, or oxo;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, , and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

### The Ring A" when Formula AA is Formula AA-2

In some embodiments, A" is a 5-membered heteroaryl comprising 1 heteroatom selected from O and S, wherein the heteroatom is bonded to the position of the heteroaryl that is bonded to the S(O)(NHR³)=N moiety.

In some embodiments, A" is thiophen-2-yl.

In some embodiments, A" is furan-2-yl.

In some embodiments, A" is one of the rings disclosed hereinbelow optionally substituted as disclosed hereinbelow, wherein in each case the bond that is shown as being broken by the wavy line 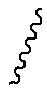 connects A to the S(O)(NHR³)=N moiety of Formula AA.

In some embodiments, the optionally substituted ring A" 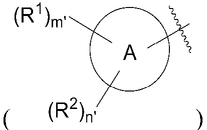 is

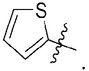

In some embodiments, the optionally substituted ring A" is

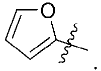

In some embodiments, the optionally substituted ring A" is

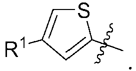

In some embodiments, the optionally substituted ring A" is

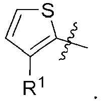

In some embodiments, the optionally substituted ring A" is

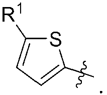

In some embodiments, the optionally substituted ring A" is

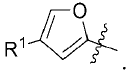

In some embodiments, the optionally substituted ring A" is

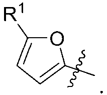

In some embodiments, the optionally substituted ring A" is

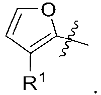

In some embodiments, the optionally substituted ring A" is

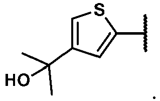

### The Ring A" when Formula AA is Formula AA-3

In some embodiments, A" is a 5-membered heteroaryl comprising 1 heteroatom selected from O and S, wherein the heteroatom is bonded to the position of the heteroaryl that is bonded to the S(O)(NHR³)=N moiety.

In some embodiments, A" is thiophen-2-yl.

In some embodiments, A" is furan-2-yl.

In some embodiments, A" is furan-2-yl substituted with 2 R^{1'}.

In some embodiments, A" is furan-2-yl substituted with 2 R^{2'}.

In some embodiments, A" is furan-2-yl substituted with 1 R^{1'} and substituted with 1 R^{2'}.

In some embodiments, A" is thiophen-2-yl substituted with 2 R^{1'}.

In some embodiments, A" is thiophen-2-yl substituted with 2 R^{2'}.

In some embodiments, A" is thiophen-2-yl substituted with 1 R^{1'} and substituted with 1 R^{2'}.

In some embodiments, A" is one of the rings disclosed hereinbelow optionally substituted as disclosed hereinbelow, wherein in each case the bond that is shown as being broken by the wavy line 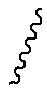 connects A" to the S(O)(NHR³)=N moiety of Formula AA.

In some embodiments, the optionally substituted ring A 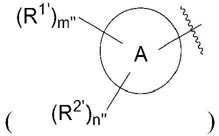 is

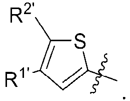

In some embodiments, the optionally substituted ring A" is

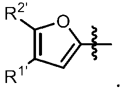

In some embodiments, the optionally substituted ring A" is

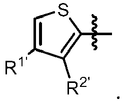

In some embodiments, the optionally substituted ring A" is

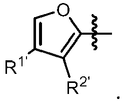

In some embodiments, the optionally substituted ring A" is

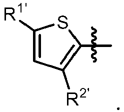

In some embodiments, the optionally substituted ring A" is

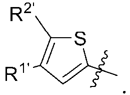

In some embodiments, the optionally substituted ring A" is

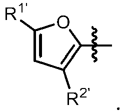

In some embodiments, the optionally substituted ring A is selected from the group consisting of:

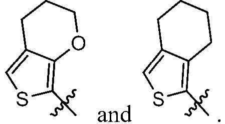

In some embodiments, the optionally substituted ring A is

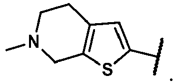

### The Ring A" when the compound is a compound of Formula AA-4

In some embodiments, A" is a 5-membered heteroaryl comprising 1 heteroatom selected from O and S, wherein the heteroatom is bonded to the position of the heteroaryl that is bonded to the S(O)(NHR³)=N moiety.

In some embodiments, A" is thiophen-2-yl.

In some embodiments, A" is furan-2-yl.

In some embodiments, A" is one of the rings disclosed hereinbelow optionally substituted as disclosed hereinbelow, wherein in each case the bond that is shown as being broken by the wavy line 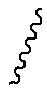 connects A to the S(O)(NHR³)=N moiety of Formula AA.

In some embodiments, the optionally substituted ring A 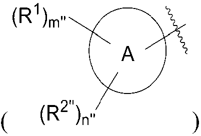 is

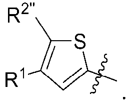

In some embodiments, the optionally substituted ring A" is

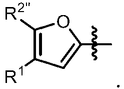

In some embodiments, the optionally substituted ring A" is

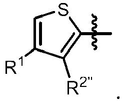

In some embodiments, the optionally substituted ring A" is

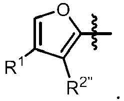

In some embodiments, the optionally substituted ring A" is

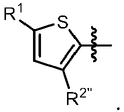

In some embodiments, the optionally substituted ring A" is

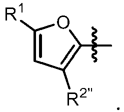

In some embodiments, the optionally substituted ring A" is

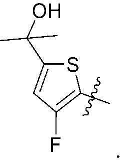

In some embodiments, the optionally substituted ring A" is

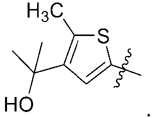

In some embodiments, the optionally substituted ring A" is

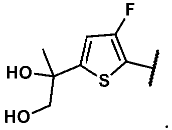

In some embodiments, the optionally substituted ring A" is

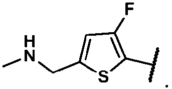

*The Ring* 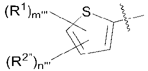 *when Formula AA is Formula AA-5*

In some embodiments, 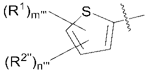 is

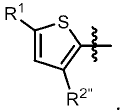

In some embodiments, 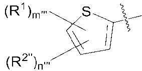 is

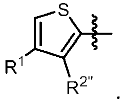

In some embodiments, 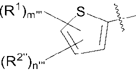 is

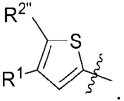

In some embodiments, 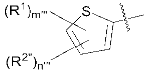 is

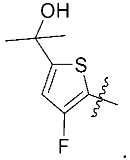

In some embodiments, 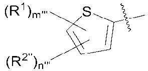 is

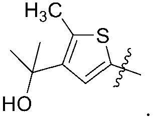

In some embodiments 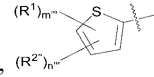 is

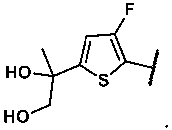

In some embodiments, 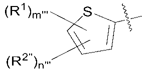 is

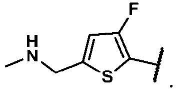

### The Groups R¹, R^{1'}, R², R^{2'}, and R^{2"}

### The Groups R¹ and R²

In some embodiments, R¹, when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R¹, when present, is independently selected from the group consisting of C₁-C₆ alkyl optionally substituted with one or more hydroxyl, halo, or NR⁸R⁹.

In some embodiments, R¹, when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².

In some embodiments, R¹, when present, is selected from the group consisting of 1-hydroxy-2-methylpropan-2-yl; methyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; 1-(dimethylamino)ethyl; fluoro; chloro; phenyl; pyridyl; pyrazolyl; S(O₂)CH₃; and S(O₂)NR¹¹R¹².

In some embodiments, R¹, when present, is selected from the group consisting of methyl; difluoromethyl; 2-hydroxy-2-propyl; hydroxymethyl; (dimethylamino)methyl; and fluoro. For example, R¹ is 2-hydroxy-2-propyl. For example, R¹ is fluoro.

In some embodiments, R¹, when present, is selected from the group consisting of methyl; ethyl; difluoromethyl; 2-hydroxy-2-propyl; 1,2-dihydroxy-2-propyl; hydroxymethyl; (dimethylamino)methyl; (methylamino)methyl; and fluoro. For example, R¹ is 2-hydroxy-2-propyl or 1,2-dihydroxy-2-propyl (e.g., R¹ is 2-hydroxy-2-propyl; or R¹ is 1,2-dihydroxy-2-propyl).

In some embodiments, R², when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R², when present, is independently selected from the group consisting of C₁-C₆ alkyl optionally substituted with one or more hydroxyl, halo, or NR⁸R⁹.

In some embodiments, R², when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².

In some embodiments, R², when present, is selected from the group consisting of 1-hydroxy-2-methylpropan-2-yl; methyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; 1-(dimethylamino)ethyl; fluoro; chloro; phenyl; pyridyl; pyrazolyl; S(O₂)CH₃; and S(O₂)NR¹¹R¹².

In some embodiments, R², when present, is selected from the group consisting of methyl; ethyl; difluoromethyl; 2-hydroxy-2-propyl; hydroxymethyl; 1,2-dihydroxy-2-propyl; (dimethylamino)methyl; (methylamino)methyl; and fluoro.

In some embodiments, R², when present, is selected from the group consisting of methyl; difluoromethyl; 2-hydroxy-2-propyl; hydroxymethyl; (dimethylamino)methyl; and fluoro.

In some embodiments, one or more R¹ when present is independently a C₁-C₆ alkyl substituted with one or more hydroxy.

In certain of these embodiments, one or more R¹ is independently selected from 1-hydroxy-2-methylpropan-2-yl; 2-hydroxy-2-propyl; hydroxymethyl; 1-hydroxyethyl; 2-hydroxyethyl; 1-hydroxy-2-propyl; 1,2-dihydroxy-2-propyl; and 1,2,3-trihydroxy-2-propyl.

In some embodiments, one or more R¹ when present is independently a C₁-C₆ alkyl substituted with one or more hydroxy and further substituted with one or more (e.g., one) NR⁸R⁹.

In certain of these embodiments, one or more R¹ is independently selected from 1-amino-2-hydroxy-prop-2-yl; 1-acetamido-2-hydroxy-prop-2-yl; and 1-(tert-butoxycarbonyl)amino-2-hydroxy-prop-2-yl.

In some embodiments, one or more R¹ when present is independently a C₁-C₆ alkyl substituted with one or more hydroxy and further substituted with one or more (e.g., one) R¹⁵.

In certain of these embodiments (e.g., a2 = 1 or 2), one or more R¹ is independently selected from 1-(2-hydroxyethoxy)-2-hydroxy-2-propyl; 1-(2-benzyloxyethoxy)-2-hydroxy-2-propyl; and 1-(2-methoxyethoxy)-2-hydroxy-2-propyl.

In certain of these embodiments (e.g., a2 = 1), one or more R¹ is independently selected from 1-(2-hydroxyethoxy)-2-hydroxy-2-propyl and 1-(2-methoxyethoxy)-2-hydroxy-2-propyl.

In certain embodiments (e.g., a2 = 1), one or more R¹ is independently selected from:

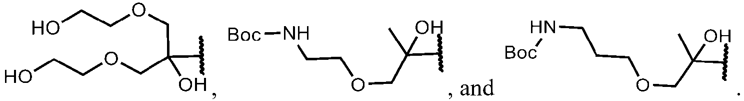

In certain embodiments (e.g., a2 > 1), one or more R¹ is

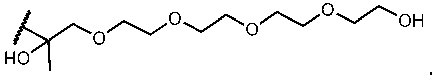

In some embodiments, one or more R¹ is independently C₁-C₆ alkyl substituted with one or more (e.g., one) NR⁸R⁹ and further optionally substituted with one or more halo.

In certain of these embodiments, one or more R¹ is independently selected from: (methylamino)methyl; (2,2-difluoroeth-1-yl)(methyl)aminomethyl; (2,2,2-trifluoroeth-1-yl)(methyl)aminomethyl; (dimethylamino)methyl; 1-(dimethylamino)ethyl; 2-((methyl)aminomethyl)-prop-2-yl; 2-((methyl)amino)-prop-2-yl; (methyl)(cyclopropylmethyl)aminomethyl; (methyl)(2-(dimethylamino)eth-1-yl)aminomethyl; (cyclobutyl)(methyl)aminomethyl; 1-(cyclobutyl)amino-eth-1-yl; isopropylaminomethyl; (cyclobutyl)aminomethyl; cycloheptylaminomethyl; tetrahydropyranylaminomethyl; sec-butylaminomethyl; ethylaminomethyl; allylaminomethyl; (2,2-difluoroeth-1-yl)aminomethyl; (2-methoxy-eth-1-yl)aminomethyl; (2-methoxy-eth-1-yl)(methyl)aminomethyl; 2-fluoro-1-dimethylamino-eth-1-yl; 1-dimethylamino-2,2-difluoroeth-1-yl; 1-dimethylamino-2,2,2-trifluoroeth-1-yl; 1-dimethylamino-2,2,2-trimethyleth-1-yl; and dimethylamino(cyclopropyl)methyl (e.g., one or more R¹ is dimethylaminomethyl or methylaminomethyl).

In some embodiments, one or more R¹ is C₁-C₆ alkyl that is optionally substituted with one or more halo. In certain of these embodiments, one or more R¹ is C₂-C₆ alkyl that is optionally substituted with one or more halo. As non-limiting examples, R¹ is ethyl or difluoromethyl.

In some embodiments, one or more R¹ when present is 3- to 7-membered heterocycloalkyl optionally substituted with one or more oxo and further optionally substituted with one or more C1-C6 alkyl. For example, R¹ is 5-methyl-oxazolidin-2-one-5-yl.

In certain of any of the foregoing embodiments of R¹, one or more R² is independently selected from C₁-C₆ alkyl, C₁-C₆ alkyl optionally substituted with one or more hydroxy, C₁-C₆ alkyl optionally substituted with one or more C₁-C₆ alkoxy, and halo.

In some embodiments, R¹ and R² are on adjacent atoms, and taken together with the atoms connecting them, independently form one monocyclic or bicyclic C₄-C₁₂ carbocyclic ring (e.g., C₅ or C₆ carbocyclic ring) or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), and wherein the carbocyclic ring or heterocyclic ring is optionally independently substituted with one or more substituents each independently selected from hydroxy, halo, oxo, C₁-C₆ alkyl (e.g., methyl), C₂-C₆ alkenyl, C₂-C₆ alkynyl, C₁-C₆ alkoxy (e.g., isopropoxyl), OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl (e.g., azetidinyl or oxetanyl), and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo (e.g., fluoro), C₁-C₆ alkyl, C₂-C₆ alkenyl, C₂-C₆ alkynyl, C₃-C₁₀ cycloalkyl, C₁-C₆ alkoxy, oxo, NR⁸R⁹ (e.g., amino, methylamino, or dimethylamino), =NR¹⁰, COOC₁-C₆ alkyl, C₆-C₁₀ aryl, and CONR⁸R⁹.

In some embodiments, R¹ and R² are on adjacent atoms, and taken together with the atoms connecting them, independently form one monocyclic or bicyclic C₅-C₆ carbocyclic ring optionally independently substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino; or
R¹ and R² are on adjacent atoms, and taken together, independently form 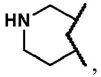 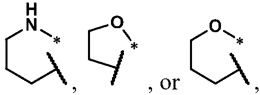 each of which is optionally substituted with one or more substituents independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino; wherein the asterisk represents a point of attachment to a carbon atom; and the 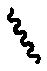 represents a point of attachment to a carbon or a nitrogen atom.

In some embodiments, R¹ and R² are on adjacent atoms, and taken together with the atoms connecting them, independently form at least one bicyclic spirocyclic C₄-C₁₂ carbocyclic ring, wherein the carbocyclic ring is optionally substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino.

In some embodiments, R¹ and R² are on adjacent atoms, and taken together with the atoms connecting them, independently form at least one bicyclic spirocyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic or heterocyclic ring is optionally substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino.

In some embodiments, R¹ and R² are different.

In some embodiments, R¹ and R² are the same.

In some embodiments, R¹ is *para* or *meta* to R².

In some embodiments, R¹ is *para* or *ortho* to R².

In some embodiments, R¹ is *ortho* or *meta* to R².

In some embodiments, R¹ is *para* to R².

In some embodiments, R¹ is *meta* to R².

In some embodiments, R¹ is *ortho* to R².

### The Groups R¹ and R² when Formula AA is Formula AA-1

In some embodiments, R¹, when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R¹, when present, is independently selected from the group consisting of C₁-C₆ alkyl optionally substituted with one or more hydroxyl, halo, or NR⁸R⁹.

In some embodiments, R¹, when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².

In some embodiments, R¹, when present, is selected from the group consisting of 1-hydroxy-2-methylpropan-2-yl; methyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; 1-(dimethylamino)ethyl; fluoro; chloro; phenyl; pyridyl; pyrazolyl; S(O₂)CH₃; and S(O₂)NR¹¹R¹².

In some embodiments, R¹, when present, is selected from the group consisting of methyl; ethyl; difluoromethyl; 2-hydroxy-2-propyl; 1,2-dihydroxy-2-propyl; hydroxymethyl; (dimethylamino)methyl; (methylamino)methyl; and fluoro. For example, R¹ is 2-hydroxy-2-propyl or 1,2-dihydroxy-2-propyl (e.g., R¹ is 2-hydroxy-2-propyl; or R¹ is 1,2-dihydroxy-2-propyl).

In some embodiments, R¹, when present, is selected from the group consisting of methyl; difluoromethyl; 2-hydroxy-2-propyl; hydroxymethyl; (dimethylamino)methyl; and fluoro. For example, R¹ is 2-hydroxy-2-propyl. For example, R¹ is fluoro.

In some embodiments, R², when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R², when present, is independently selected from the group consisting of C₁-C₆ alkyl optionally substituted with one or more hydroxyl, halo, or NR⁸R⁹.

In some embodiments, R², when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².

In some embodiments, R², when present, is selected from the group consisting of 1-hydroxy-2-methylpropan-2-yl; methyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; 1-(dimethylamino)ethyl; fluoro; chloro; phenyl; pyridyl; pyrazolyl; S(O₂)CH₃; and S(O₂)NR¹¹R¹².

In some embodiments, R², when present, is selected from the group consisting of methyl; ethyl; difluoromethyl; 2-hydroxy-2-propyl; hydroxymethyl; 1,2-dihydroxy-2-propyl; (dimethylamino)methyl; (methylamino)methyl; and fluoro.

In some embodiments of the compound of Formula AA-1, R², when present, is selected from the group consisting of methyl; difluoromethyl; 2-hydroxy-2-propyl; hydroxymethyl; (dimethylamino)methyl; and fluoro.

In some embodiments, one or more R¹ when present is independently a C₁-C₆ alkyl substituted with one or more hydroxy.

In certain of these embodiments, one or more R¹ is independently selected from 1-hydroxy-2-methylpropan-2-yl; 2-hydroxy-2-propyl; hydroxymethyl; 1-hydroxyethyl; 2-hydroxyethyl; 1-hydroxy-2-propyl; 1,2-dihydroxy-2-propyl; and 1,2,3-trihydroxy-2-propyl.

In some embodiments, one or more R¹ when present is independently a C₁-C₆ alkyl substituted with one or more hydroxy and further substituted with one or more (e.g., one) NR⁸R⁹.

In certain of these embodiments, one or more R¹ is independently selected from 1-amino-2-hydroxy-prop-2-yl; 1-acetamido-2-hydroxy-prop-2-yl; and 1-(*tert*-butoxycarbonyl)amino-2-hydroxy-prop-2-yl.

In some embodiments, one or more R¹ when present is independently a C₁-C₆ alkyl substituted with one or more hydroxy and further substituted with one or more (e.g., one) R¹⁵.

In certain of these embodiments (e.g., a2 = 1 or 2), one or more R¹ is independently selected from 1-(2-hydroxyethoxy)-2-hydroxy-2-propyl; 1-(2-benzyloxyethoxy)-2-hydroxy-2-propyl; and 1-(2-methoxyethoxy)-2-hydroxy-2-propyl.

In certain of these embodiments (e.g., a2 = 1), one or more R¹ is independently selected from 1-(2-hydroxyethoxy)-2-hydroxy-2-propyl and 1-(2-methoxyethoxy)-2-hydroxy-2-propyl.

In certain embodiments (e.g., a2 = 1), one or more R¹ is independently selected from:

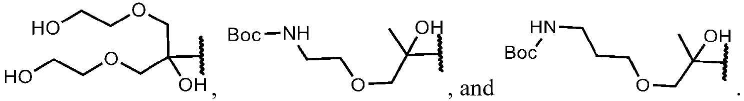

In certain embodiments (e.g., a2 > 1), one or more R¹ is

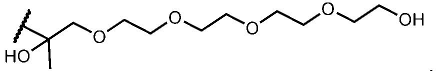

In some embodiments, one or more R¹ is independently C₁-C₆ alkyl substituted with one or more (e.g., one) NR⁸R⁹ and further optionally substituted with one or more halo.

In certain of these embodiments, one or more R¹ is independently selected from: (methylamino)methyl; (2,2-difluoroeth-1-yl)(methyl)aminomethyl; (2,2,2-trifluoroeth-1-yl)(methyl)aminomethyl; (dimethylamino)methyl; 1-(dimethylamino)ethyl; 2-((methyl)aminomethyl)-prop-2-yl; 2-((methyl)amino)-prop-2-yl; (methyl)(cyclopropylmethyl)aminomethyl; (methyl)(2-(dimethylamino)eth-1-yl)aminomethyl; (cyclobutyl)(methyl)aminomethyl; 1-(cyclobutyl)amino-eth-1-yl; isopropylaminomethyl; (cyclobutyl)aminomethyl; cycloheptylaminomethyl; tetrahydropyranylaminomethyl; sec-butylaminomethyl; ethylaminomethyl; allylaminomethyl; (2,2-difluoroeth-1-yl)aminomethyl; (2-methoxy-eth-1-yl)aminomethyl; (2-methoxy-eth-1-yl)(methyl)aminomethyl; 2-fluoro-1-dimethylamino-eth-1-yl; 1-dimethylamino-2,2-difluoroeth-1-yl; 1-dimethylamino-2,2,2-trifluoroeth-1-yl; 1-dimethylamino-2,2,2-trimethyleth-1-yl; and dimethylamino(cyclopropyl)methyl (e.g., one or more R¹ is dimethylaminomethyl or methylaminomethyl).

In some embodiments, one or more R¹ is C₁-C₆ alkyl that is optionally substituted with one or more halo. In certain of these embodiments, one or more R¹ is C₂-C₆ alkyl that is optionally substituted with one or more halo. As non-limiting examples, R¹ is ethyl or difluoromethyl.

In some embodiments, one or more R¹ when present is 3- to 7-membered heterocycloalkyl optionally substituted with one or more oxo and further optionally substituted with one or more C1-C6 alkyl. For example, R¹ is 5-methyl-oxazolidin-2-one-5-yl.

In certain of any of the foregoing embodiments of R¹, one or more R² is independently selected from C₁-C₆ alkyl, C₁-C₆ alkyl optionally substituted with one or more hydroxy, C₁-C₆ alkyl optionally substituted with one or more C₁-C₆ alkoxy, and halo.

In some embodiments of the compound of Formula AA-1, R¹ and R² are on adjacent atoms, and taken together with the atoms connecting them, independently form one monocyclic or bicyclic C₄-C₁₂ carbocyclic ring (e.g., C₅ or C₆ carbocyclic ring) or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), and wherein the carbocyclic ring or heterocyclic ring is optionally independently substituted with one or more substituents each independently selected from hydroxy, halo, oxo, C₁-C₆ alkyl (e.g., methyl), C₂-C₆ alkenyl, C₂-C₆ alkynyl, C₁-C₆ alkoxy (e.g., isopropoxyl), OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl (e.g., azetidinyl or oxetanyl), and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo (e.g., fluoro), C₁-C₆ alkyl, C₂-C₆ alkenyl, C₂-C₆ alkynyl, C₃-C₁₀ cycloalkyl, C₁-C₆ alkoxy, oxo, NR⁸R⁹ (e.g., amino, methylamino, or dimethylamino), =NR¹⁰, COOC₁-C₆ alkyl, C₆-C₁₀ aryl, and CONR⁸R⁹.

In some embodiments of the compound of Formula AA-1, R¹ and R² are on adjacent atoms, and taken together with the atoms connecting them, independently form one monocyclic or bicyclic C₅-C₆ carbocyclic ring optionally independently substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino; or
R¹ and R² are on adjacent atoms, and taken together, independently form 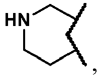 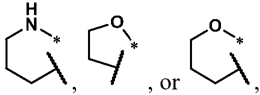 each of which is optionally substituted with one or more substituents independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino; wherein the asterisk represents a point of attachment to a carbon atom; and the 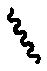 represents a point of attachment to a carbon or a nitrogen atom.

In some embodiments of the compound of Formula AA-1, R¹ and R² are on adjacent atoms, and taken together with the atoms connecting them, independently form at least one bicyclic spirocyclic C₄-C₁₂ carbocyclic ring, wherein the carbocyclic ring is optionally substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino.

In some embodiments of the compound of Formula AA-1, R¹ and R² are on adjacent atoms, and taken together with the atoms connecting them, independently form at least one bicyclic spirocyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic or heterocyclic ring is optionally substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino.

### The Groups R¹ and R² when Formula AA is Formula AA-2

In some embodiments, R¹, when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R¹, when present, is independently selected from the group consisting of C₁-C₆ alkyl optionally substituted with one or more hydroxyl, halo, or NR⁸R⁹.

In some embodiments, R¹, when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².

In some embodiments, R¹, when present, is selected from the group consisting of 1-hydroxy-2-methylpropan-2-yl; methyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; 1-(dimethylamino)ethyl; fluoro; chloro; phenyl; pyridyl; pyrazolyl; S(O₂)CH₃; and S(O₂)NR¹¹R¹².

In some embodiments, R¹, when present, is selected from the group consisting of methyl; difluoromethyl; 2-hydroxy-2-propyl; hydroxymethyl; (dimethylamino)methyl; and fluoro. For example, R¹ is 2-hydroxy-2-propyl. For example, R¹ is fluoro.

In some embodiments, R¹, when present, is selected from the group consisting of methyl; ethyl; difluoromethyl; 2-hydroxy-2-propyl; 1,2-dihydroxy-2-propyl; hydroxymethyl; (dimethylamino)methyl; (methylamino)methyl; and fluoro. For example, R¹, when present, is 2-hydroxy-2-propyl or 1,2-dihydroxy-2-propyl (e.g., R¹ is 2-hydroxy-2-propyl; or R¹ is 1,2-dihydroxy-2-propyl).

In some embodiments, R², when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R², when present, is independently selected from the group consisting of C₁-C₆ alkyl optionally substituted with one or more hydroxyl, halo, or NR⁸R⁹. In some embodiments, R², when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².

In some embodiments, R², when present, is selected from the group consisting of 1-hydroxy-2-methylpropan-2-yl; methyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; 1-(dimethylamino)ethyl; fluoro; chloro; phenyl; pyridyl; pyrazolyl; S(O₂)CH₃; and S(O₂)NR¹¹R¹².

In some embodiments, R², when present, is selected from the group consisting of methyl; ethyl; difluoromethyl; 2-hydroxy-2-propyl; 1,2-dihydroxy-2-propyl; hydroxymethyl; (dimethylamino)methyl; (methylamino)methyl; and fluoro.

In some embodiments, R², when present, is selected from the group consisting of methyl; difluoromethyl; 2-hydroxy-2-propyl; hydroxymethyl; (dimethylamino)methyl; and fluoro.

In some embodiments, one or more R¹ when present is independently a C₁-C₆ alkyl substituted with one or more hydroxy.

In certain of these embodiments, one or more R¹ is independently selected from 1-hydroxy-2-methylpropan-2-yl; 2-hydroxy-2-propyl; hydroxymethyl; 1-hydroxyethyl; 2-hydroxyethyl; 1-hydroxy-2-propyl; 1,2-dihydroxy-2-propyl; and 1,2,3-trihydroxy-2-propyl.

In some embodiments, one or more R¹ when present is independently a C₁-C₆ alkyl substituted with one or more hydroxy and further substituted with one or more (e.g., one) NR⁸R⁹.

In certain of these embodiments, one or more R¹ is independently selected from 1-amino-2-hydroxy-prop-2-yl; 1-acetamido-2-hydroxy-prop-2-yl; and 1-(tert-butoxycarbonyl)amino-2-hydroxy-prop-2-yl.

In some embodiments, one or more R¹ when present is independently a C₁-C₆ alkyl substituted with one or more hydroxy and further substituted with one or more (e.g., one) R¹⁵.

In certain of these embodiments (e.g., a2 = 1 or 2), one or more R¹ is independently selected from 1-(2-hydroxyethoxy)-2-hydroxy-2-propyl; 1-(2-benzyloxyethoxy)-2-hydroxy-2-propyl; and 1-(2-methoxyethoxy)-2-hydroxy-2-propyl.

In certain of these embodiments (e.g., a2 = 1), one or more R¹ is independently selected from 1-(2-hydroxyethoxy)-2-hydroxy-2-propyl and 1-(2-methoxyethoxy)-2-hydroxy-2-propyl.

In certain embodiments (e.g., a2 = 1), one or more R¹ is independently selected from:

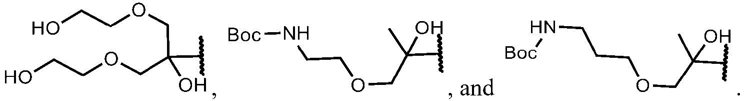

In certain embodiments (e.g., a2 > 1), one or more R¹ is

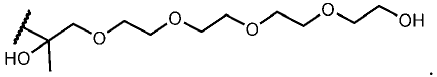

In some embodiments, one or more R¹ is independently C₁-C₆ alkyl substituted with one or more (e.g., one) NR⁸R⁹ and further optionally substituted with one or more halo.

In certain of these embodiments, one or more R¹ is independently selected from: (methylamino)methyl; (2,2-difluoroeth-1-yl)(methyl)aminomethyl; (2,2,2-trifluoroeth-1-yl)(methyl)aminomethyl; (dimethylamino)methyl; 1-(dimethylamino)ethyl; 2-((methyl)aminomethyl)-prop-2-yl; 2-((methyl)amino)-prop-2-yl; (methyl)(cyclopropylmethyl)aminomethyl; (methyl)(2-(dimethylamino)eth-1-yl)aminomethyl; (cyclobutyl)(methyl)aminomethyl; 1-(cyclobutyl)amino-eth-1-yl; isopropylaminomethyl; (cyclobutyl)aminomethyl; cycloheptylaminomethyl; tetrahydropyranylaminomethyl; sec-butylaminomethyl; ethylaminomethyl; allylaminomethyl; (2,2-difluoroeth-1-yl)aminomethyl; (2-methoxy-eth-1-yl)aminomethyl; (2-methoxy-eth-1-yl)(methyl)aminomethyl; 2-fluoro-1-dimethylamino-eth-1-yl; 1-dimethylamino-2,2-difluoroeth-1-yl; 1-dimethylamino-2,2,2-trifluoroeth-1-yl; 1-dimethylamino-2,2,2-trimethyleth-1-yl; and dimethylamino(cyclopropyl)methyl (e.g., one or more R¹ is dimethylaminomethyl or methylaminomethyl).

In some embodiments, one or more R¹ is C₁-C₆ alkyl that is optionally substituted with one or more halo. In certain of these embodiments, one or more R¹ is C₂-C₆ alkyl that is optionally substituted with one or more halo. As non-limiting examples, R¹ is ethyl or difluoromethyl.

In some embodiments, one or more R¹ when present is 3- to 7-membered heterocycloalkyl optionally substituted with one or more oxo and further optionally substituted with one or more C1-C6 alkyl. For example, R¹ is 5-methyl-oxazolidin-2-one-5-yl.

In certain of any of the foregoing embodiments of R¹, one or more R² is independently selected from C₁-C₆ alkyl, C₁-C₆ alkyl optionally substituted with one or more hydroxy, C₁-C₆ alkyl optionally substituted with one or more C₁-C₆ alkoxy, and halo.

In some embodiments, one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, when the compound of Formula AA is a compound of Formula AA-2, and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

### The Groups R^{1'} and R^{2'} when Formula AA is Formula AA-3

In some embodiments, R^{1'}, when present, is independently selected from the group consisting of C₂-C₆ alkyl optionally substituted with one or more hydroxy, C₂-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; Cl; Br; I; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R^{1'}, when present, is independently selected from the group consisting of C₂-C₆ alkyl optionally substituted with one or more hydroxyl, or NR⁸R⁹.

In some embodiments, R^{1'}, when present, is selected from the group consisting of 1-hydroxy-2-methylpropan-2-yl; 1,2-dihydroxy-2-propyl; isopropyl; ethyl; 2-hydroxy-2-propyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; 1-(dimethylamino)ethyl; chloro; phenyl; pyridyl; pyrazolyl; S(O₂)CH₃; and S(O₂)NR¹¹R¹². In certain of these embodiments, R^{1'} is 2-hydroxy-2-propyl or 1,2-dihydroxy-2-propyl. For example, R^{1'} is 2-hydroxy-2-propyl. As another example, R^{1'} is 1,2-dihydroxy-2-propyl.

In some embodiments, R^{1'}, when present, is selected from the group consisting of 1-hydroxy-2-methylpropan-2-yl; isopropyl; 2-hydroxy-2-propyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; 1-(dimethylamino)ethyl; chloro; phenyl; pyridyl; pyrazolyl; S(O₂)CH₃; and S(O₂)NR¹¹R¹². For example, R^{1'} is 2-hydroxy-2-propyl.

In some embodiments, R^{2'}, when present, is independently selected from the group consisting of C₂-C₆ alkyl optionally substituted with one or more hydroxy, C₂-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; Cl; Br; I; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R^{2'}, when present, is independently selected from the group consisting of C₂-C₆ alkyl optionally substituted with one or more hydroxyl, halo, or NR⁸R⁹.

In some embodiments, R^{2'}, when present, is selected from the group consisting of 1-hydroxy-2-methylpropan-2-yl; 1,2-dihydroxy-2-propyl; isopropyl; ethyl; 2-hydroxy-2-propyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; 1-(dimethylamino)ethyl; chloro; phenyl; pyridyl; pyrazolyl; S(O₂)CH₃; and S(O₂)NR¹¹R¹². For example, R^{2'} is 2-hydroxy-2-propyl or 1,2-dihydroxy-2-propyl (e.g., R^{2'} is 2-hydroxy-2-propyl; or R^{2'} is 1,2-dihydroxy-2-propyl).

In some embodiments, R^{2'}, when present, is selected from the group consisting of 1-hydroxy-2-methylpropan-2-yl; isopropyl; 2-hydroxy-2-propyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; 1-(dimethylamino)ethyl; chloro; phenyl; pyridyl; pyrazolyl; S(O₂)CH₃; and S(O₂)NR¹¹R¹². For example, R^{2'} is 2-hydroxy-2-propyl.

In some embodiments, one or more R^{1'} when present is independently a C₂-C₆ alkyl substituted with one or more hydroxy.

In certain of these embodiments, one or more R^{1'} is independently selected from 1-hydroxy-2-methylpropan-2-yl; 2-hydroxy-2-propyl; 1-hydroxyethyl; 2-hydroxyethyl; 1-hydroxy-2-propyl; 1,2-dihydroxy-2-propyl; and 1,2,3-trihydroxy-2-propyl.

In some embodiments, one or more R^{1'} when present is independently a C₂-C₆ alkyl substituted with one or more hydroxy and further substituted with one or more (e.g., one) NR⁸R⁹.

In certain of these embodiments, one or more R^{1'} is independently selected from 1-amino-2-hydroxy-prop-2-yl; 1-acetamido-2-hydroxy-prop-2-yl; and 1-(*tert*-butoxycarbonyl)amino-2-hydroxy-prop-2-yl.

In some embodiments, one or more R^{1'} when present is independently a C₂-C₆ alkyl substituted with one or more hydroxy and further substituted with one or more (e.g., one) R¹⁵.

In certain of these embodiments (e.g., a2 = 1 or 2), one or more R^{1'} is independently selected from 1-(2-hydroxyethoxy)-2-hydroxy-2-propyl; 1-(2-benzyloxyethoxy)-2-hydroxy-2-propyl; and 1-(2-methoxyethoxy)-2-hydroxy-2-propyl.

In certain of these embodiments (e.g., a2 = 1), one or more R^{1'} is independently selected from 1-(2-hydroxyethoxy)-2-hydroxy-2-propyl and 1-(2-methoxyethoxy)-2-hydroxy-2-propyl.

In certain embodiments (e.g., a2 = 1), one or more R^{1'} is independently selected from:

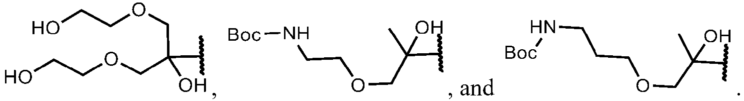

In certain embodiments (e.g., a2 > 1), one or more R^{1'} is

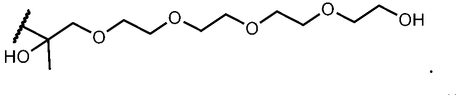

In some embodiments, one or more R^{1'} is independently C₂-C₆ alkyl substituted with one or more (e.g., one) NR⁸R⁹ and further optionally substituted with one or more halo.

In certain of these embodiments, one or more R^{1'} is independently selected from: 1-(dimethylamino)ethyl; 2-((methyl)aminomethyl)-prop-2-yl; 2-((methyl)amino)-prop-2-yl; (1-(cyclobutyl)amino-eth-1-yl; 2-fluoro-1-dimethylamino-eth-1-yl; 1-dimethylamino-2,2-difluoroeth-1-yl; 1-dimethylamino-2,2,2-trifluoroeth-1-yl; and 1-dimethylamino-2,2,2-trimethyleth-1-yl.

In some embodiments, one or more R^{1'} is C₂-C₆ alkyl that is optionally substituted with one or more halo. In certain of these embodiments, one or more R^{1'} is C₂-C₆ alkyl that is optionally substituted with one or more halo. As non-limiting examples, R^{1'} is ethyl or difluoroethyl.

In some embodiments, one or more R^{1'} when present is 3- to 7-membered heterocycloalkyl optionally substituted with one or more oxo and further optionally substituted with one or more C₁-C₆ alkyl. For example, R^{1'} is 5-methyl-oxazolidin-2-one-5-yl.

In certain of any of the foregoing embodiments of R^{1'}, one or more R^{2'} is independently selected from C₂-C₆ alkyl, C₂-C₆ alkyl optionally substituted with one or more hydroxy, C₂-C₆ alkyl optionally substituted with one or more C₁-C₆ alkoxy, Br, Cl, and I.

In some embodiments, one pair of R^{1'} and R^{2'} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂ and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, R^{1'} and R^{2'} are the same.

In some embodiments, R^{1'} and R^{2'} are different.

In some embodiments, R^{1'} is *meta* to R^{2'}.

In some embodiments, R^{1'} is *ortho* to R^{2'}.

### R¹ and R^{2"} when Formula AA is Formula AA-4

In some embodiments, R¹ is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R¹ is independently selected from the group consisting of C₁-C₆ alkyl optionally substituted with one or more hydroxyl, halo, or NR⁸R⁹.

In some embodiments, R¹ is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².

In some embodiments, R¹ is selected from the group consisting of 1-hydroxy-2-methylpropan-2-yl; methyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; 1-(dimethylamino)ethyl; fluoro; chloro; phenyl; pyridyl; pyrazolyl; S(O₂)CH₃; and S(O₂)NR¹¹R¹².

In certain embodiments, R¹ is selected from the group consisting of methyl; ethyl; difluoromethyl; 2-hydroxy-2-propyl; 1,2-dihydroxy-2-propyl; hydroxymethyl; (methylamino)methyl; (dimethylamino)methyl; and fluoro. As a non-limiting example of the foregoing embodiments, R¹ is 2-hydroxy-2-propyl or 1,2-dihydroxy-2-propyl (e.g., R¹ is 2-hydroxy-2-propyl; or R¹ is 1,2-dihydroxy-2-propyl).

In some embodiments, R¹ is selected from the group consisting of methyl; difluoromethyl; 2-hydroxy-2-propyl; hydroxymethyl; (dimethylamino)methyl; and fluoro. In some embodiments of the compound of Formula AA-4, R¹ is 2-hydroxy-2-propyl. n some embodiments of the compound of Formula AA-4, R¹ is fluoro.

In some embodiments, R^{2"} is fluoro.

In some embodiments, R^{2"} is methyl.

In some embodiments, one or more R¹ when present is independently a C₁-C₆ alkyl substituted with one or more hydroxy.

In certain of these embodiments, one or more R¹ is independently selected from 1-hydroxy-2-methylpropan-2-yl; 2-hydroxy-2-propyl; hydroxymethyl; 1-hydroxyethyl; 2-hydroxyethyl; 1-hydroxy-2-propyl; 1,2-dihydroxy-2-propyl; and 1,2,3-trihydroxy-2-propyl.

In some embodiments, one or more R¹ when present is independently a C₁-C₆ alkyl substituted with one or more hydroxy and further substituted with one or more (e.g., one) NR⁸R⁹.

In certain of these embodiments, one or more R¹ is independently selected from 1-amino-2-hydroxy-prop-2-yl; 1-acetamido-2-hydroxy-prop-2-yl; and 1-(*tert*-butoxycarbonyl)amino-2-hydroxy-prop-2-yl.

In some embodiments, one or more R¹ when present is independently a C₁-C₆ alkyl substituted with one or more hydroxy and further substituted with one or more (e.g., one) R¹⁵.

In certain of these embodiments (e.g., a2 = 1 or 2), one or more R¹ is independently selected from 1-(2-hydroxyethoxy)-2-hydroxy-2-propyl; 1-(2-benzyloxyethoxy)-2-hydroxy-2-propyl; and 1-(2-methoxyethoxy)-2-hydroxy-2-propyl.

In certain of these embodiments (e.g., a2 = 1), one or more R¹ is independently selected from 1-(2-hydroxyethoxy)-2-hydroxy-2-propyl and 1-(2-methoxyethoxy)-2-hydroxy-2-propyl.

In certain embodiments (e.g., a2 = 1), one or more R¹ is independently selected from:

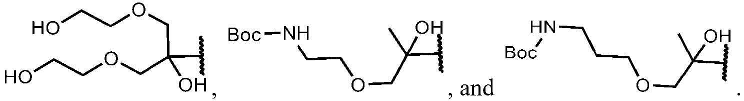

In certain embodiments (e.g., a2 > 1), one or more R¹ is

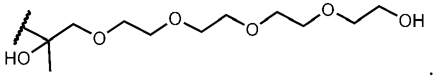

In some embodiments, one or more R¹ is independently C₁-C₆ alkyl substituted with one or more (e.g., one) NR⁸R⁹ and further optionally substituted with one or more halo.

In certain of these embodiments, one or more R¹ is independently selected from: (methylamino)methyl; (2,2-difluoroeth-1-yl)(methyl)aminomethyl; (2,2,2-trifluoroeth-1-yl)(methyl)aminomethyl; (dimethylamino)methyl; 1-(dimethylamino)ethyl; 2-((methyl)aminomethyl)-prop-2-yl; 2-((methyl)amino)-prop-2-yl; (methyl)(cyclopropylmethyl)aminomethyl; (methyl)(2-(dimethylamino)eth-1-yl)aminomethyl; (cyclobutyl)(methyl)aminomethyl; 1-(cyclobutyl)amino-eth-1-yl; isopropylaminomethyl; (cyclobutyl)aminomethyl; cycloheptylaminomethyl; tetrahydropyranylaminomethyl; sec-butylaminomethyl; ethylaminomethyl; allylaminomethyl; (2,2-difluoroeth-1-yl)aminomethyl; (2-methoxy-eth-1-yl)aminomethyl; (2-methoxy-eth-1-yl)(methyl)aminomethyl; 2-fluoro-1-dimethylamino-eth-1-yl; 1-dimethylamino-2,2-difluoroeth-1-yl; 1-dimethylamino-2,2,2-trifluoroeth-1-yl; 1-dimethylamino-2,2,2-trimethyleth-1-yl; and dimethylamino(cyclopropyl)methyl (e.g., one or more R¹ is dimethylaminomethyl or methylaminomethyl).

In some embodiments, one or more R¹ is C₁-C₆ alkyl that is optionally substituted with one or more halo. In certain of these embodiments, one or more R¹ is C₂-C₆ alkyl that is optionally substituted with one or more halo. As non-limiting examples, R¹ is ethyl or difluoromethyl.

In some embodiments, one or more R¹ when present is 3- to 7-membered heterocycloalkyl optionally substituted with one or more oxo and further optionally substituted with one or more C1-C6 alkyl. For example, R¹ is 5-methyl-oxazolidin-2-one-5-yl.

In some embodiments, one pair of R¹ and R^{2"} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, R¹ and R^{2"} are the same.

In some embodiments, R¹ and R^{2"} are different.

In some embodiments, R¹ is *meta* to R^{2"}.

In some embodiments, R¹ is *ortho* to R^{2"}.

### R¹ and R^{2"} when Formula AA is Formula AA-5

In some embodiments, R¹ is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.

In some embodiments, R¹ is independently selected from the group consisting of C₁-C₆ alkyl optionally substituted with one or more hydroxyl, halo, or NR⁸R⁹.

In some embodiments, R¹ is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².

In some embodiments, R¹ is selected from the group consisting of 1-hydroxy-2-methylpropan-2-yl; methyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; 1-(dimethylamino)ethyl; fluoro; chloro; phenyl; pyridyl; pyrazolyl; S(O₂)CH₃; and S(O₂)NR¹¹R¹².

In some embodiments, R¹ is selected from the group consisting of methyl; ethyl; difluoromethyl; 2-hydroxy-2-propyl; hydroxymethyl; 1,2-dihydroxy-2-propyl; (dimethylamino)methyl; (methylamino)methyl; and fluoro. For example, R¹ is 2-hydroxy-2-propyl or 1,2-dihydroxy-2-propyl (e.g., R¹ is 2-hydroxy-2-propyl; or R¹ is 1,2-dihydroxy-2-propyl).

In some embodiments, R¹ is selected from the group consisting of methyl; difluoromethyl; 2-hydroxy-2-propyl; hydroxymethyl; (dimethylamino)methyl; and fluoro. For example, R¹ is 2-hydroxy-2-propyl. For example, R¹ is fluoro.

In some embodiments, one or more R¹ when present is independently a C₁-C₆ alkyl substituted with one or more hydroxy.

In certain of these embodiments, one or more R¹ is independently selected from 1-hydroxy-2-methylpropan-2-yl; 2-hydroxy-2-propyl; hydroxymethyl; 1-hydroxyethyl; 2-hydroxyethyl; 1-hydroxy-2-propyl; 1,2-dihydroxy-2-propyl; and 1,2,3-trihydroxy-2-propyl.

In some embodiments, one or more R¹ when present is independently a C₁-C₆ alkyl substituted with one or more hydroxy and further substituted with one or more (e.g., one) NR⁸R⁹.

In certain of these embodiments, one or more R¹ is independently selected from 1-amino-2-hydroxy-prop-2-yl; 1-acetamido-2-hydroxy-prop-2-yl; and 1-(tert-butoxycarbonyl)amino-2-hydroxy-prop-2-yl.

In some embodiments, one or more R¹ when present is independently a C₁-C₆ alkyl substituted with one or more hydroxy and further substituted with one or more (e.g., one) R¹⁵.

In certain of these embodiments (e.g., a2 = 1 or 2), one or more R¹ is independently selected from 1-(2-hydroxyethoxy)-2-hydroxy-2-propyl; 1-(2-benzyloxyethoxy)-2-hydroxy-2-propyl; and 1-(2-methoxyethoxy)-2-hydroxy-2-propyl.

In certain of these embodiments (e.g., a2 = 1), one or more R¹ is independently selected from 1-(2-hydroxyethoxy)-2-hydroxy-2-propyl and 1-(2-methoxyethoxy)-2-hydroxy-2-propyl.

In certain embodiments (e.g., a2 = 1), one or more R¹ is independently selected from:

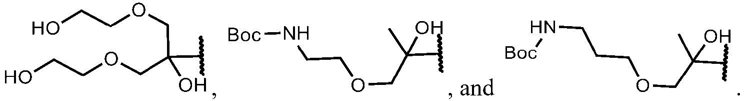

In certain embodiments (e.g., a2 > 1), one or more R¹ is

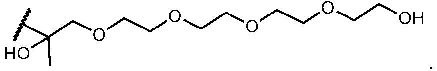

In some embodiments, one or more R¹ is independently C₁-C₆ alkyl substituted with one or more (e.g., one) NR⁸R⁹ and further optionally substituted with one or more halo.

In certain of these embodiments, one or more R¹ is independently selected from: (methylamino)methyl; (2,2-difluoroeth-1-yl)(methyl)aminomethyl; (2,2,2-trifluoroeth-1-yl)(methyl)aminomethyl; (dimethylamino)methyl; 1-(dimethylamino)ethyl; 2-((methyl)aminomethyl)-prop-2-yl; 2-((methyl)amino)-prop-2-yl; (methyl)(cyclopropylmethyl)aminomethyl; (methyl)(2-(dimethylamino)eth-1-yl)aminomethyl; (cyclobutyl)(methyl)aminomethyl; 1-(cyclobutyl)amino-eth-1-yl; isopropylaminomethyl; (cyclobutyl)aminomethyl; cycloheptylaminomethyl; tetrahydropyranylaminomethyl; sec-butylaminomethyl; ethylaminomethyl; allylaminomethyl; (2,2-difluoroeth-1-yl)aminomethyl; (2-methoxy-eth-1-yl)aminomethyl; (2-methoxy-eth-1-yl)(methyl)aminomethyl; 2-fluoro-1-dimethylamino-eth-1-yl; 1-dimethylamino-2,2-difluoroeth-1-yl; 1-dimethylamino-2,2,2-trifluoroeth-1-yl; 1-dimethylamino-2,2,2-trimethyleth-1-yl; and dimethylamino(cyclopropyl)methyl (e.g., one or more R¹ is dimethylaminomethyl or methylaminomethyl).

In some embodiments, one or more R¹ is C₁-C₆ alkyl that is optionally substituted with one or more halo. In certain of these embodiments, one or more R¹ is C₂-C₆ alkyl that is optionally substituted with one or more halo. As non-limiting examples, R¹ is ethyl or difluoromethyl.

In some embodiments, one or more R¹ when present is 3- to 7-membered heterocycloalkyl optionally substituted with one or more oxo and further optionally substituted with one or more C1-C6 alkyl. For example, R¹ is 5-methyl-oxazolidin-2-one-5-yl.

In certain of any of the foregoing embodiments of R¹, one or more R² is independently selected from C₁-C₆ alkyl, C₁-C₆ alkyl optionally substituted with one or more hydroxy, C₁-C₆ alkyl optionally substituted with one or more C₁-C₆ alkoxy, and halo.

In some embodiments, R^{2"} is fluoro.

In some embodiments, R^{2"} is methyl.

### The variables o and p

In some embodiments, o=1 or 2.

In some embodiments, o=1.

In some embodiments, o=2.

In some embodiments, p=0, 1, 2, or 3.

In some embodiments, p=0.

In some embodiments, p=1.

In some embodiments, p=2.

In some embodiments, o=1 and p=0.

In some embodiments, o=2 and p=0.

In some embodiments, o=1 and p=1.

In some embodiments, o=1 and p=2.

In some embodiments, o=2 and p=1.

In some embodiments, o=2 and p=2.

In some embodiments, o=2 and p=3.

### The ring B and substitutions on the ring B

In some embodiments, B is pyridyl or an N-oxide thereof (e.g., 2-pyridyl or an N-oxide thereof, 3-pyridyl or an N-oxide thereof, or 4-pyridyl or an N-oxide thereof).

In some embodiments, B is pyridyl (e.g., 2-pyridyl, 3-pyridyl, or 4-pyridyl).

In some embodiments, B is a pyridyl N-oxide (e.g., 2-pyridyl N-oxide, 3-pyridyl N-oxide, or 4-pyridyl N-oxide).

In some embodiments, B is pyrimidinyl or an N-oxide thereof (e.g., 4-pyrimidinyl or an N-oxide thereof, or 5-pyrimidinyl or an N-oxide thereof).

In some embodiments, B is pyridazinyl.

In some embodiments, B is pyrazinyl.

In some embodiments, B is triazinyl.

In some embodiments, B is one of the rings disclosed hereinbelow, substituted as disclosed hereinbelow, wherein in each case the bond that is shown as being broken by the wavy line 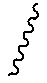 connects B to the NHC(O)group of Formula AA.

In some embodiments, the substituted ring B is

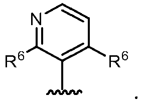

In some embodiments, the substituted ring B is

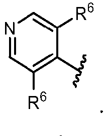

In some embodiments, the substituted ring B is

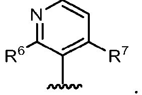

In some embodiments, the substituted ring B is

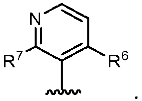

In some embodiments, the substituted ring B is

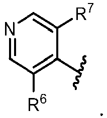

In some embodiments, the substituted ring B is

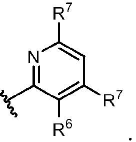

In some embodiments, the substituted ring B is

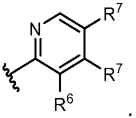

In some embodiments, the substituted ring B is

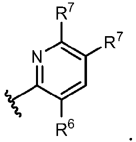

In some embodiments, the substituted ring B is

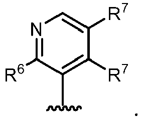

In some embodiments, the substituted ring B is

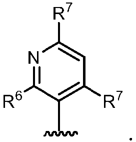

In some embodiments, the substituted ring B is

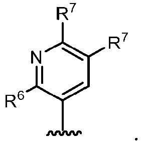

In some embodiments, the substituted ring B is

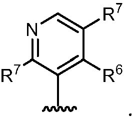

In some embodiments, the substituted ring B is

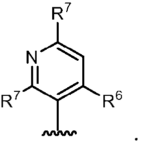

In some embodiments, the substituted ring B is

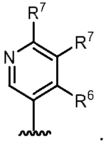

In some embodiments, the substituted ring B is

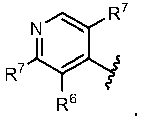

In some embodiments, the substituted ring B is

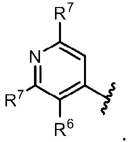

In some embodiments, the substituted ring B is

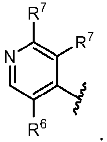

In some embodiments, the substituted ring B is

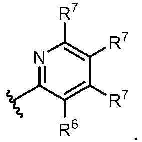

In some embodiments, the substituted ring B is

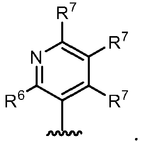

In some embodiments, the substituted ring B is

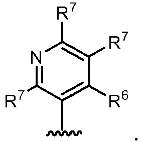

In some embodiments, the substituted ring B is

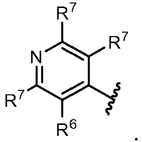

In some embodiments, the substituted ring B is

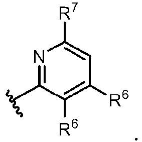

In some embodiments, the substituted ring B is

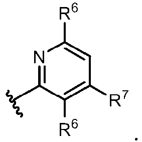

In some embodiments, the substituted ring B is

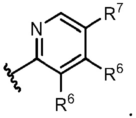

In some embodiments, the substituted ring B is

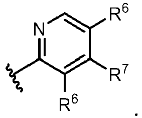

In some embodiments, the substituted ring B is

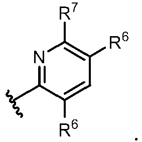

In some embodiments, the substituted ring B is

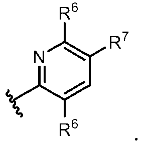

In some embodiments, the substituted ring B is

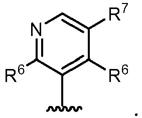

In some embodiments, the substituted ring B is

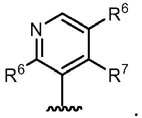

In some embodiments, the substituted ring B is

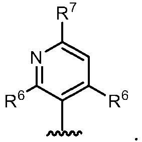

In some embodiments, the substituted ring B is

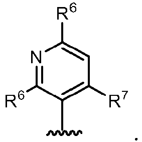

In some embodiments, the substituted ring B is

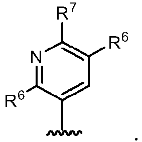

In some embodiments, the substituted ring B is

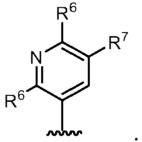

In some embodiments, the substituted ring B is

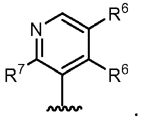

In some embodiments, the substituted ring B is

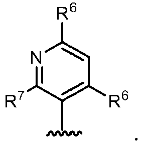

In some embodiments, the substituted ring B is

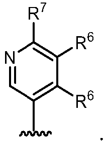

In some embodiments, the substituted ring B is

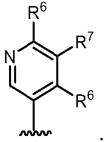

In some embodiments, the substituted ring B is

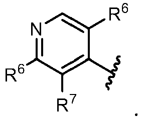

In some embodiments, the substituted ring B is

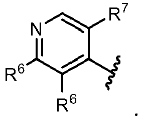

In some embodiments, the substituted ring B is

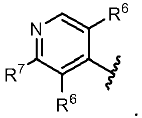

In some embodiments, the substituted ring B is

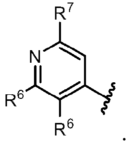

In some embodiments, the substituted ring B is

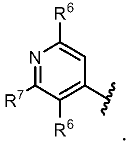

In some embodiments, the substituted ring B is

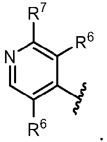

In some embodiments, the substituted ring B is

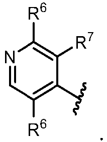

In some embodiments, the substituted ring B is

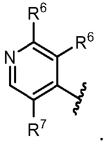

In some embodiments, the substituted ring B is

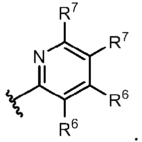

In some embodiments, the substituted ring B is

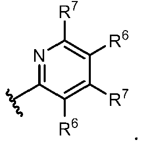

In some embodiments, the substituted ring B is

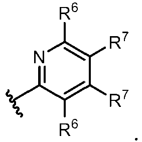

In some embodiments, the substituted ring B is

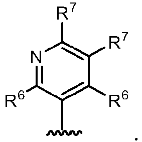

In some embodiments, the substituted ring B is

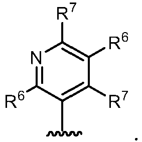

In some embodiments, the substituted ring B is

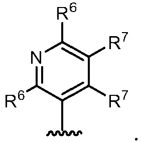

In some embodiments, the substituted ring B is

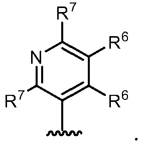

In some embodiments, the substituted ring B is

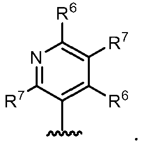

In some embodiments, the substituted ring B is

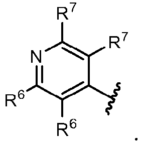

In some embodiments, the substituted ring B is

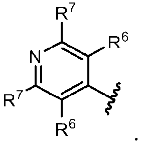

In some embodiments, the substituted ring B is

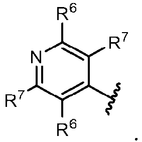

In some embodiments, the substituted ring B is

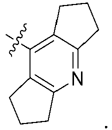

In some embodiments, the substituted ring B is

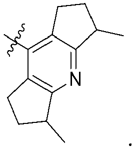

In some embodiments, the substituted ring B is

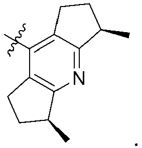

In some embodiments, the substituted ring B is

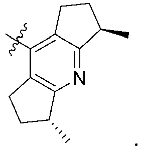

In some embodiments, the substituted ring B is

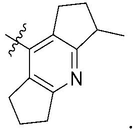

In some embodiments, the substituted ring B is

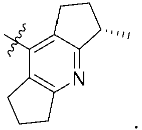

In some embodiments, the substituted ring B is

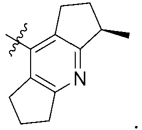

In some embodiments, the substituted ring B is

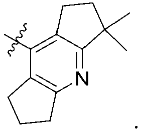

In some embodiments, the substituted ring B is

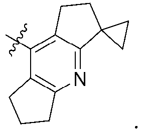

In some embodiments, the substituted ring B is

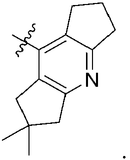

In some embodiments, the substituted ring B is

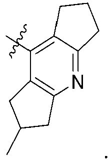

In some embodiments, the substituted ring B is

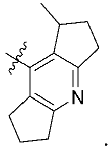

In some embodiments, the substituted ring B is

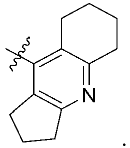

In some embodiments, the substituted ring B is

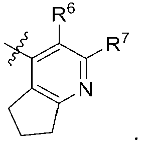

In some embodiments, the substituted ring B is

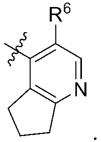

In some embodiments, the substituted ring B is

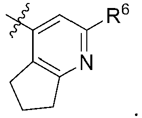

In some embodiments, the substituted ring B is

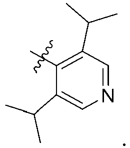

In some embodiments, the substituted ring B is

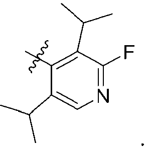

In some embodiments, the substituted ring B is

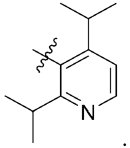

In some embodiments, the substituted ring B is

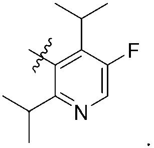

In some embodiments, the substituted ring B is

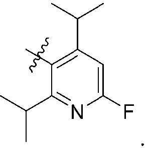

In some embodiments, the substituted ring B is selected from the group consisting of: 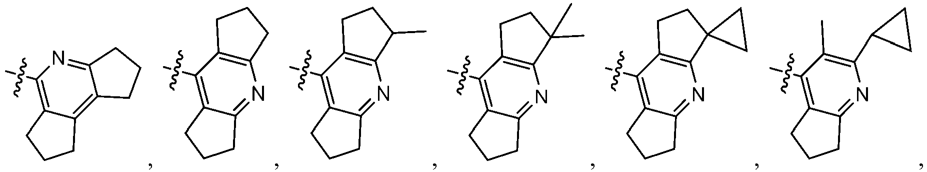 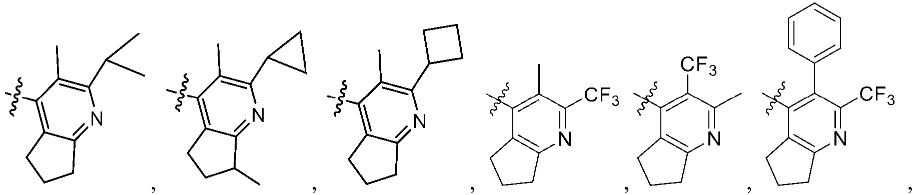 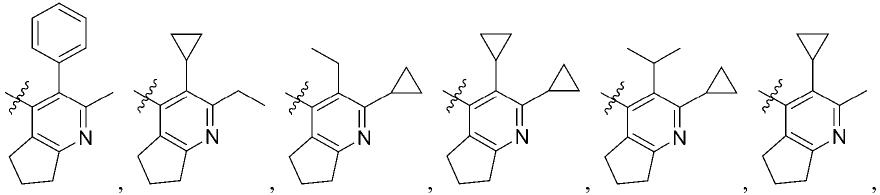 and

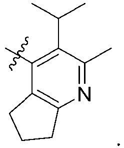

In some embodiments, the substituted ring B is selected from the group consisting of: 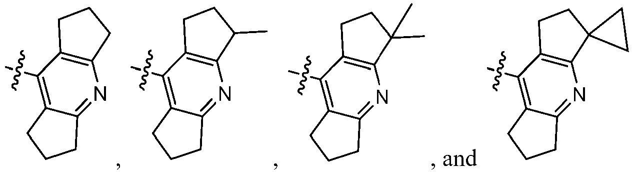 (e.g., 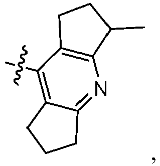 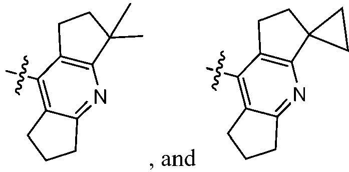 ).

In some embodiments, the substituted ring B is selected from the group consisting of:

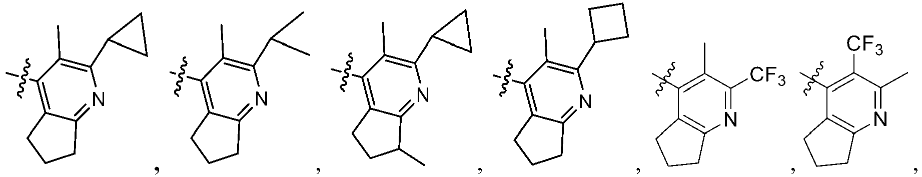

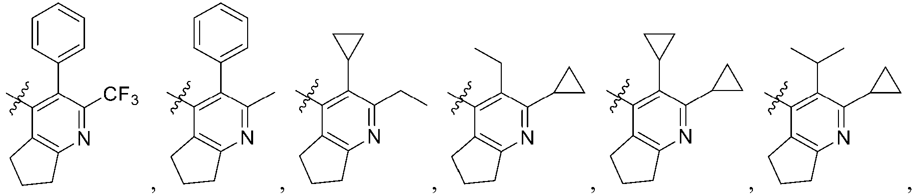

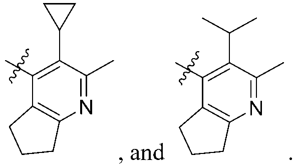

In some embodiments, the substituted ring B is selected from the group consisting of:

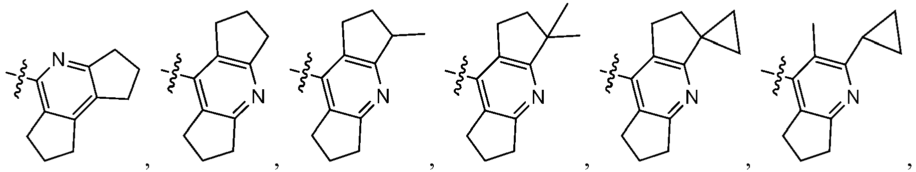

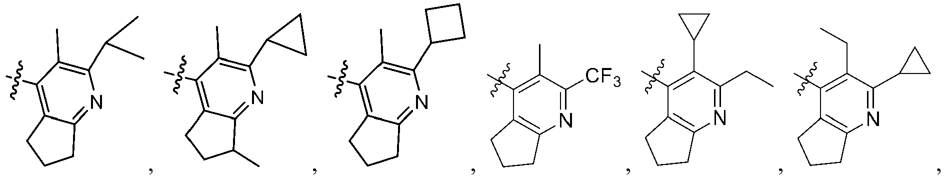

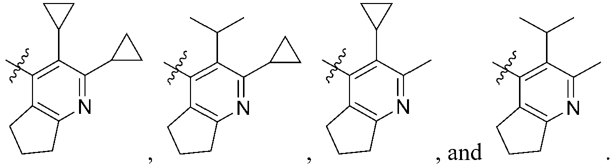

In some embodiments, the substituted ring B is:

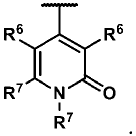

In certain of these embodiments, the substituted ring B is:

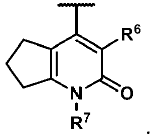

In some embodiments, the substituted ring B is

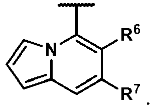

In certain of these embodiments, the substituted ring B is

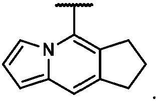

In some embodiments, the substituted ring B is

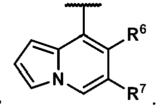

In certain of these embodiments, the substituted ring B is

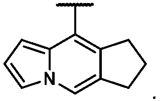

### The Ring B when Formula AA is Formula AA-1

In some embodiments, B is pyridyl or an N-oxide thereof (e.g., 2-pyridyl or an N-oxide thereof, 3-pyridyl or an N-oxide thereof, or 4-pyridyl or an N-oxide thereof).

In some embodiments, B is pyridyl (e.g., 2-pyridyl, 3-pyridyl, or 4-pyridyl).

In some embodiments, B is a pyridyl N-oxide (e.g., 2-pyridyl N-oxide, 3-pyridyl N-oxide, or 4-pyridyl N-oxide).

In some embodiments, B is pyrimidinyl or an N-oxide thereof (e.g., 4-pyrimidinyl or an N-oxide thereof, or 5-pyrimidinyl or an N-oxide thereof).

In some embodiments, B is pyridazinyl.

In some embodiments, B is pyrazinyl.

In some embodiments, B is triazinyl.

In some embodiments, the substituted ring B is

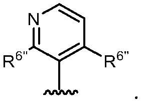

In some embodiments, the substituted ring B is

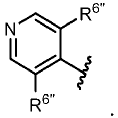

In some embodiments, the substituted ring B is

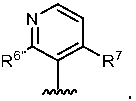

In some embodiments, the substituted ring B is

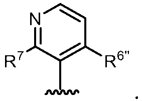

In some embodiments, the substituted ring B is

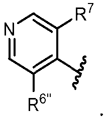

In some embodiments, the substituted ring B is

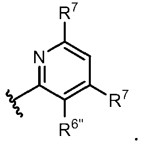

In some embodiments, the substituted ring B is

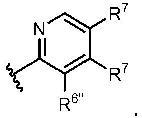

In some embodiments, the substituted ring B is

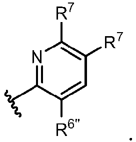

In some embodiments, the substituted ring B is

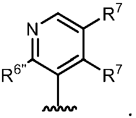

In some embodiments, the substituted ring B is

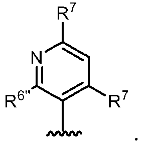

In some embodiments, the substituted ring B is

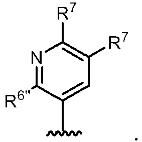

In some embodiments, the substituted ring B is

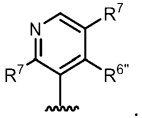

In some embodiments, the substituted ring B is

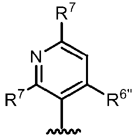

In some embodiments, the substituted ring B is

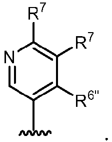

In some embodiments, the substituted ring B is

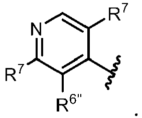

In some embodiments, the substituted ring B is

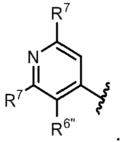

In some embodiments, the substituted ring B is

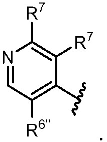

In some embodiments, the substituted ring B is

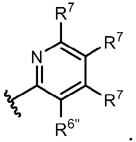

In some embodiments, the substituted ring B is

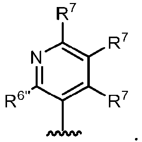

In some embodiments, the substituted ring B is

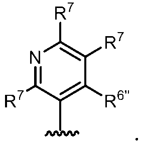

In some embodiments, the substituted ring B is

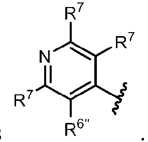

In some embodiments, the substituted ring B is

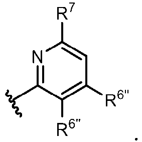

In some embodiments, the substituted ring B is

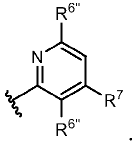

In some embodiments, the substituted ring B is

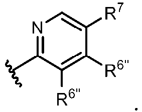

In some embodiments, the substituted ring B is

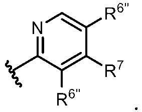

In some embodiments, the substituted ring B is

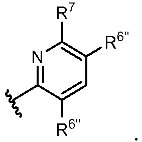

In some embodiments, the substituted ring B is

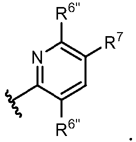

In some embodiments, the substituted ring B is

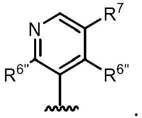

In some embodiments, the substituted ring B is

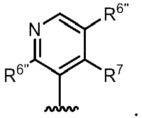

In some embodiments, the substituted ring B is

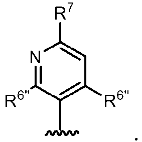

In some embodiments, the substituted ring B is

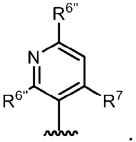

In some embodiments, the substituted ring B is

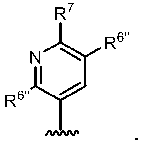

In some embodiments, the substituted ring B is

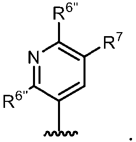

In some embodiments, the substituted ring B is

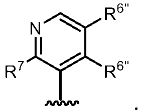

In some embodiments, the substituted ring B is

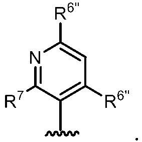

In some embodiments, the substituted ring B is

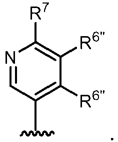

In some embodiments, the substituted ring B is

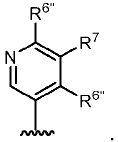

In some embodiments, the substituted ring B is

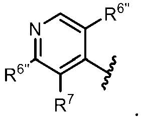

In some embodiments, the substituted ring B is

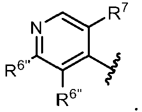

In some embodiments, the substituted ring B is

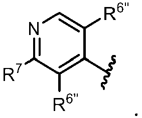

In some embodiments, the substituted ring B is

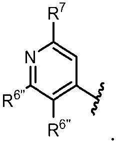

In some embodiments, the substituted ring B is

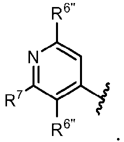

In some embodiments, the substituted ring B is

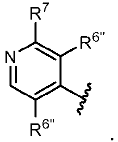

In some embodiments, the substituted ring B is

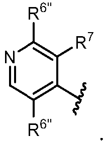

In some embodiments, the substituted ring B is

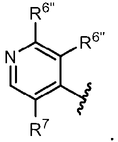

In some embodiments, the substituted ring B is

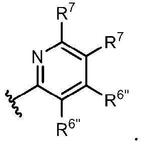

In some embodiments, the substituted ring B is

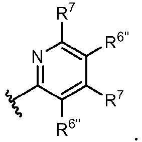

In some embodiments, the substituted ring B is

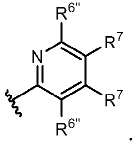

In some embodiments, the substituted ring B is

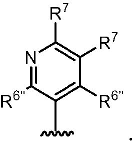

In some embodiments, the substituted ring B is

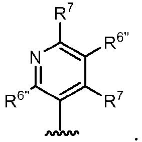

In some embodiments, the substituted ring B is

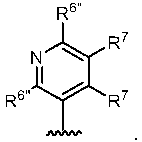

In some embodiments, the substituted ring B is

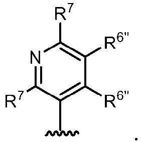

In some embodiments, the substituted ring B is

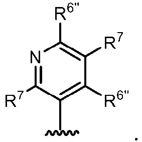

In some embodiments, the substituted ring B is

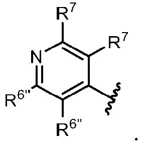

In some embodiments, the substituted ring B is

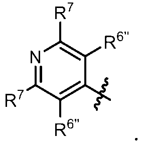

In some embodiments, the substituted ring B is

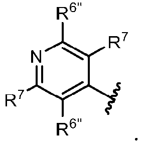

In some embodiments, the substituted ring B is

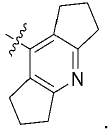

In some embodiments, the substituted ring B is

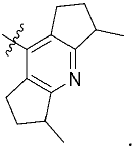

In some embodiments, the substituted ring B is

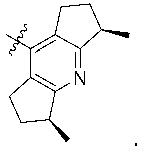

In some embodiments, the substituted ring B is

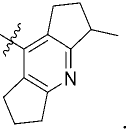

In some embodiments, the substituted ring B is

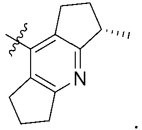

In some embodiments, the substituted ring B is

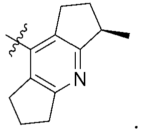

In some embodiments, the substituted ring B is

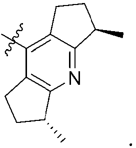

In some embodiments, the substituted ring B is

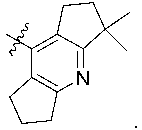

In some embodiments, the substituted ring B is

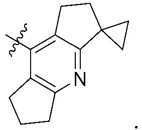

In some embodiments, the substituted ring B is

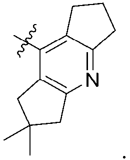

In some embodiments, the substituted ring B is

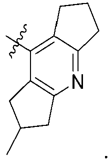

In some embodiments, the substituted ring B is

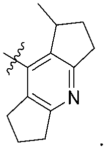

In some embodiments, the substituted ring B is

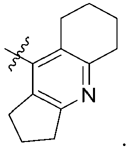

In some embodiments, the substituted ring B is

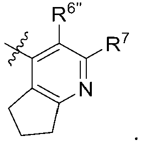

In some embodiments, the substituted ring B is

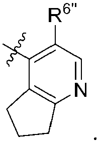

In some embodiments, the substituted ring B is

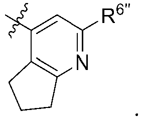

In some embodiments, the substituted ring B is

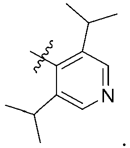

In some embodiments, the substituted ring B is

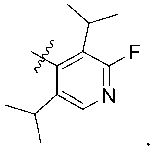

In some embodiments, the substituted ring B is

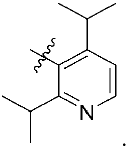

In some embodiments, the substituted ring B is

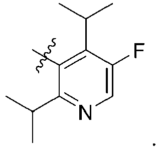

In some embodiments, the substituted ring B is

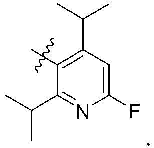

In some embodiments, the substituted ring B is selected from the group consisting of: 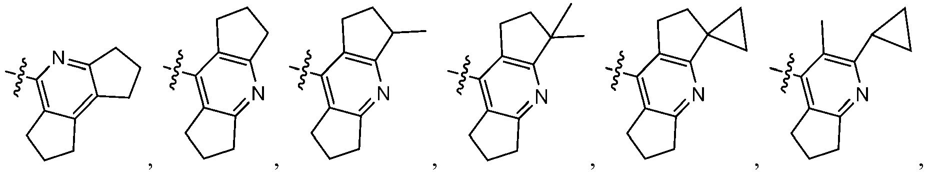 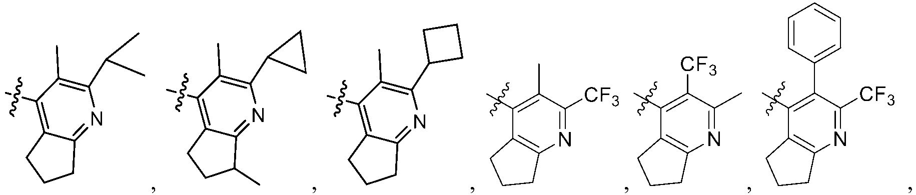 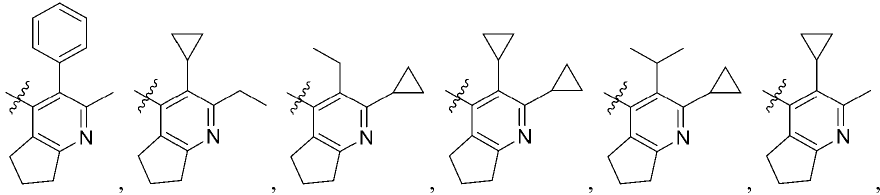 and

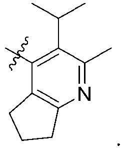

In some embodiments, the substituted ring B is selected from the group consisting of:

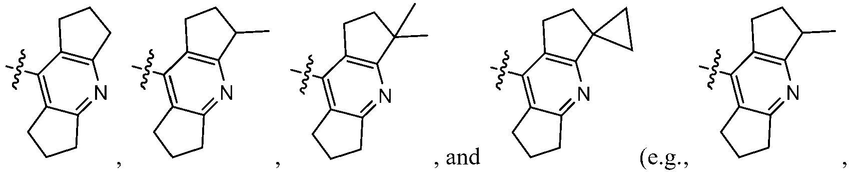

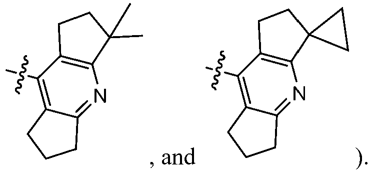

In some embodiments, the substituted ring B is selected from the group consisting of:

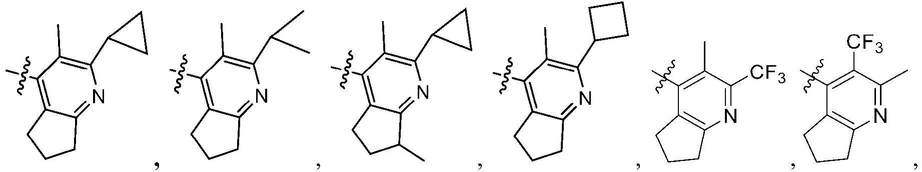

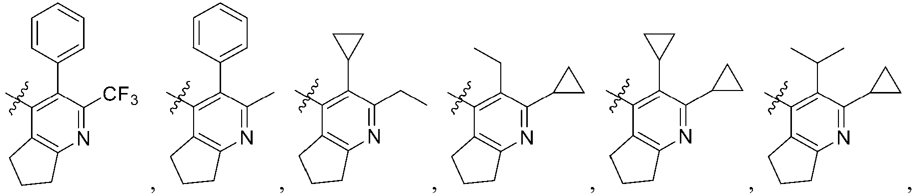

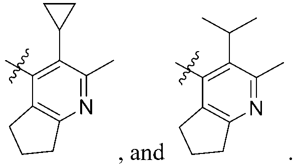

In some embodiments, the substituted ring B is selected from the group consisting of:

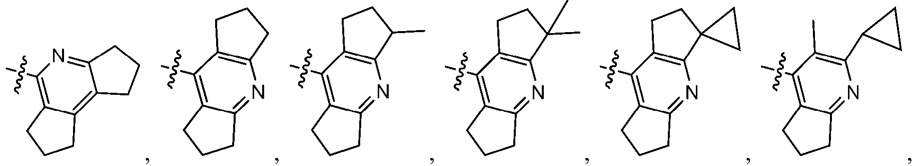

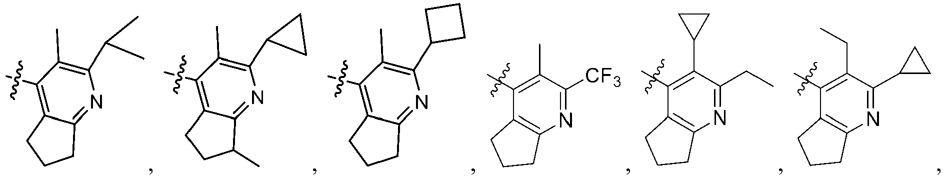

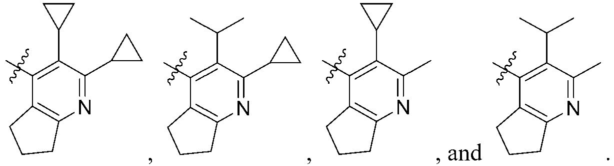

In some embodiments, the substituted ring B is:

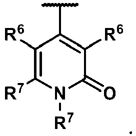

In certain of these embodiments, the substituted ring B is:

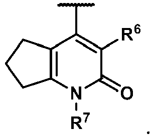

In some embodiments, the substituted ring B is

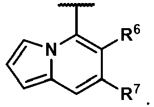

In certain of these embodiments, the substituted ring B is

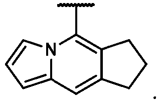

In some embodiments, the substituted ring B is

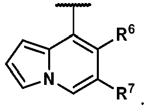

In certain of these embodiments, the substituted ring B is

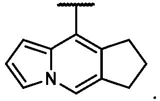

### The Ring B when Formula AA is Formula AA-2

In some embodiments, B is pyridyl or an N-oxide thereof (e.g., 2-pyridyl or an N-oxide thereof, 3-pyridyl or an N-oxide thereof, or 4-pyridyl or an N-oxide thereof).

In some embodiments, B is pyridyl (e.g., 2-pyridyl, 3-pyridyl, or 4-pyridyl).

In some embodiments, B is a pyridyl N-oxide (e.g., 2-pyridyl N-oxide, 3-pyridyl N-oxide, or 4-pyridyl N-oxide).

In some embodiments, B is pyrimidinyl or an N-oxide thereof (e.g., 4-pyrimidinyl or an N-oxide thereof, or 5-pyrimidinyl or an N-oxide thereof).

In some embodiments, B is pyridazinyl.

In some embodiments, B is pyrazinyl.

In some embodiments, B is triazinyl.

In some embodiments, B is a 6-membered heteroaryl including from 1-2 optionally substituted nitrogen atoms.

In some embodiments, B is a N-substituted pyridonyl (e.g., N-substituted pyrid-2-on-4-yl).

In some embodiments, B is one of the rings disclosed hereinbelow, substituted as disclosed hereinbelow, wherein in each case the bond that is shown as being broken by the wavy line 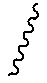 connects B to the NHC(O)group of Formula AA.

In some embodiments, the substituted ring B is

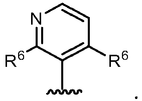

In some embodiments, the substituted ring B is

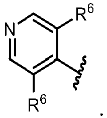

In some embodiments, the substituted ring B is

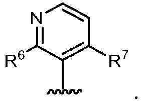

In some embodiments, the substituted ring B is

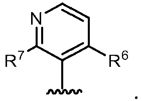

In some embodiments, the substituted ring B is

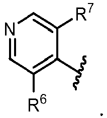

In some embodiments, the substituted ring B is

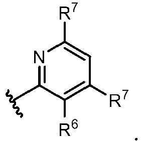

In some embodiments, the substituted ring B is

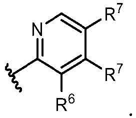

In some embodiments, the substituted ring B is

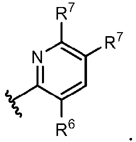

In some embodiments, the substituted ring B is

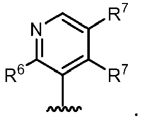

In some embodiments, the substituted ring B is

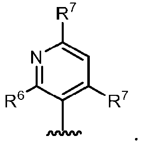

In some embodiments, the substituted ring B is

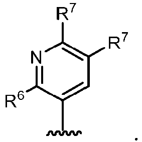

In some embodiments, the substituted ring B is

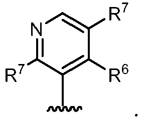

In some embodiments, the substituted ring B is

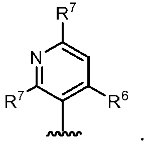

In some embodiments, the substituted ring B is

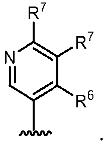

In some embodiments, the substituted ring B is

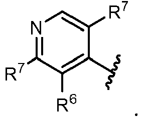

In some embodiments, the substituted ring B is

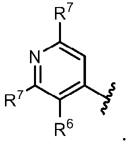

In some embodiments, the substituted ring B is

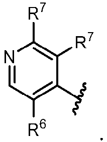

In some embodiments, the substituted ring B is

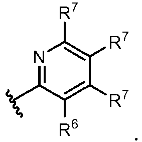

In some embodiments, the substituted ring B is

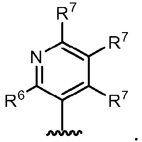

In some embodiments, the substituted ring B is

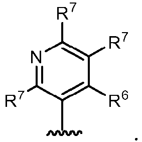

In some embodiments, the substituted ring B is

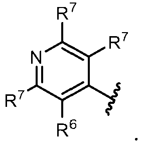

In some embodiments, the substituted ring B is

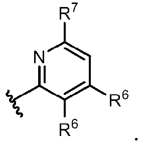

In some embodiments, the substituted ring B is

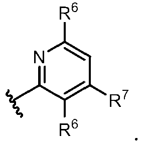

In some embodiments, the substituted ring B is

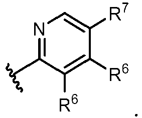

In some embodiments, the substituted ring B is

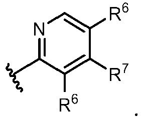

In some embodiments, the substituted ring B is

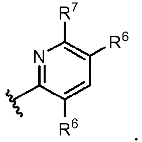

In some embodiments, the substituted ring B is

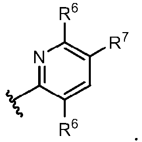

In some embodiments, the substituted ring B is

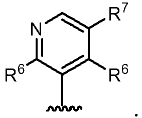

In some embodiments, the substituted ring B is

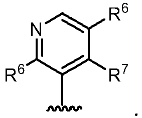

In some embodiments, the substituted ring B is

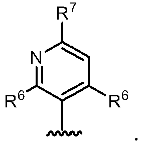

In some embodiments, the substituted ring B is

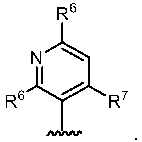

In some embodiments, the substituted ring B is

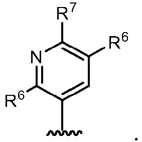

In some embodiments, the substituted ring B is

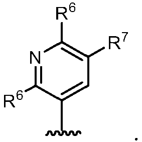

In some embodiments, the substituted ring B is

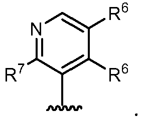

In some embodiments, the substituted ring B is

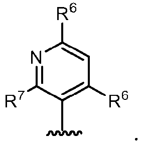

In some embodiments, the substituted ring B is

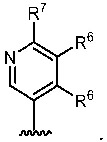

In some embodiments, the substituted ring B is

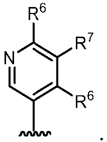

In some embodiments, the substituted ring B is

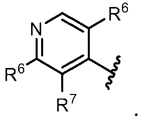

In some embodiments, the substituted ring B is

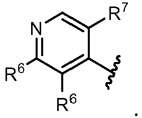

In some embodiments, the substituted ring B is

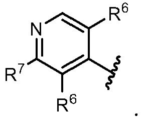

In some embodiments, the substituted ring B is

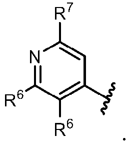

In some embodiments, the substituted ring B is

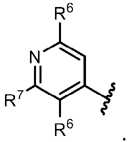

In some embodiments, the substituted ring B is

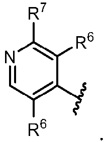

In some embodiments, the substituted ring B is

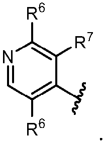

In some embodiments, the substituted ring B is

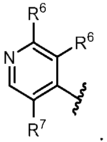

In some embodiments, the substituted ring B is

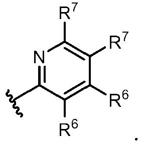

In some embodiments, the substituted ring B is

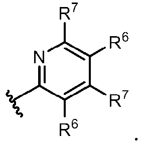

In some embodiments, the substituted ring B is

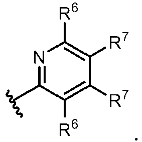

In some embodiments, the substituted ring B is

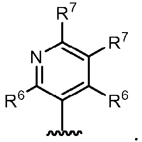

In some embodiments, the substituted ring B is

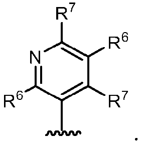

In some embodiments, the substituted ring B is

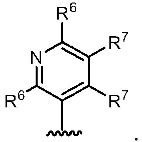

In some embodiments, the substituted ring B is

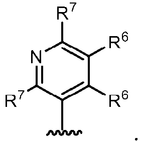

In some embodiments, the substituted ring B is

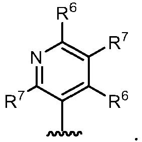

In some embodiments, the substituted ring B is

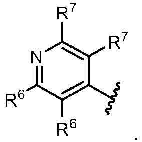

In some embodiments, the substituted ring B is

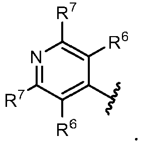

In some embodiments, the substituted ring B is

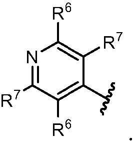

In some embodiments, the substituted ring B is

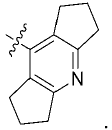

In some embodiments, the substituted ring B is

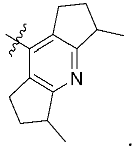

In some embodiments, the substituted ring B is

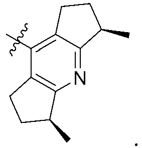

In some embodiments, the substituted ring B is

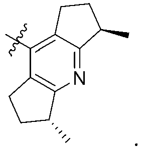

In some embodiments, the substituted ring B is

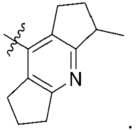

In some embodiments, the substituted ring B is

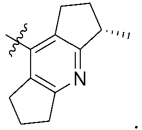

In some embodiments, the substituted ring B is

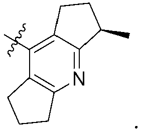

In some embodiments, the substituted ring B is

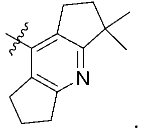

In some embodiments, the substituted ring B is

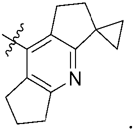

In some embodiments, the substituted ring B is

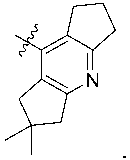

In some embodiments, the substituted ring B is

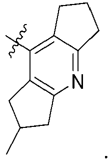

In some embodiments, the substituted ring B is

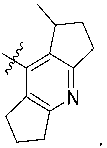

In some embodiments, the substituted ring B is

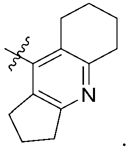

In some embodiments, the substituted ring B is

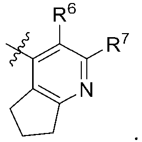

In some embodiments, the substituted ring B is

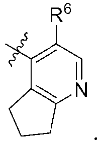

In some embodiments, the substituted ring B is

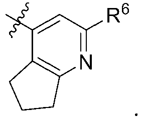

In some embodiments, the substituted ring B is

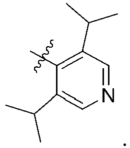

In some embodiments, the substituted ring B is

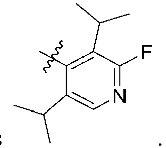

In some embodiments, the substituted ring B is

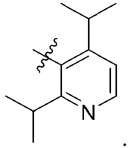

In some embodiments, the substituted ring B is

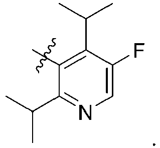

In some embodiments, the substituted ring B is

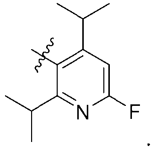

In some embodiments, the substituted ring B is:

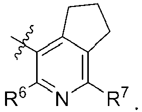

In some embodiments, the substituted ring B is:

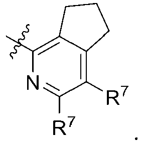

In some embodiments, the substituted ring B is selected from the group consisting of: 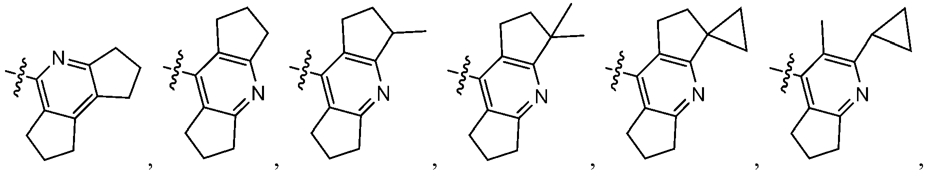 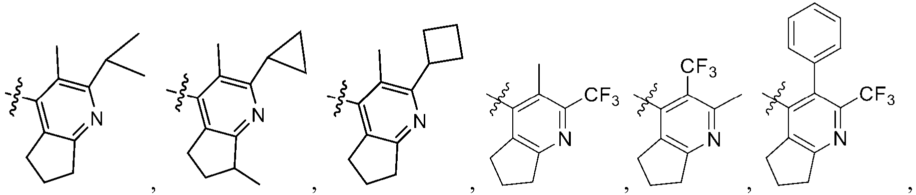 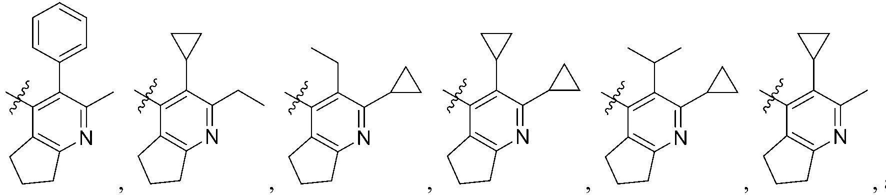 and

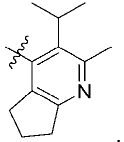

In some embodiments, the substituted ring B is selected from the group consisting of: 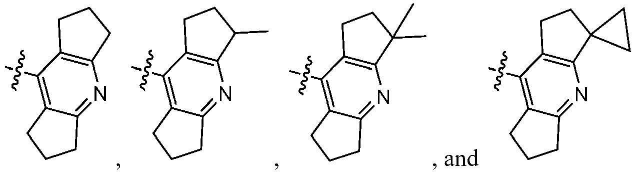 (e.g., 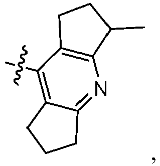 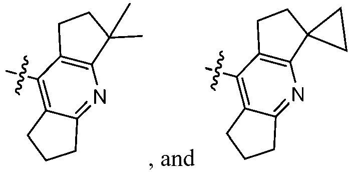 ).

In some embodiments, the substituted ring B is selected from the group consisting of:

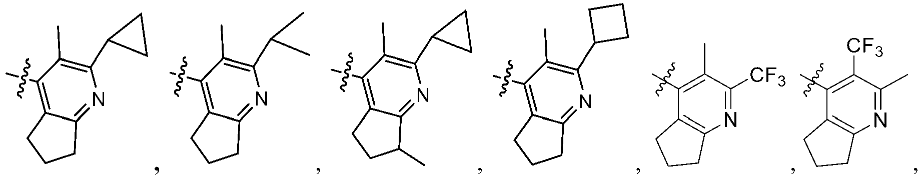

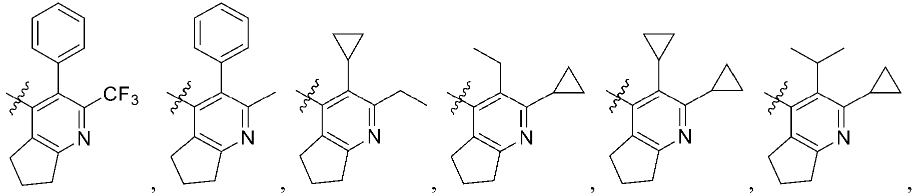

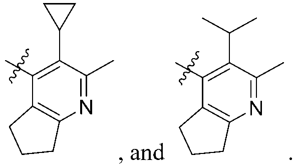

In some embodiments, the substituted ring B is selected from the group consisting of:

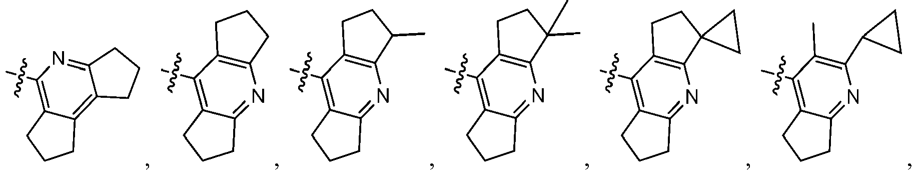

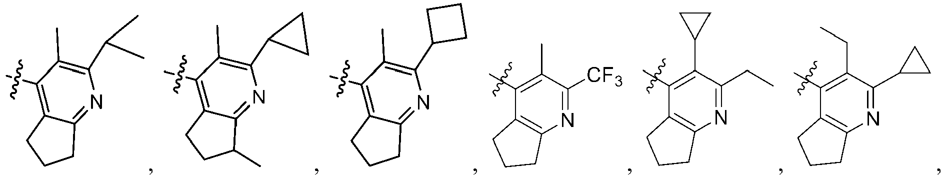

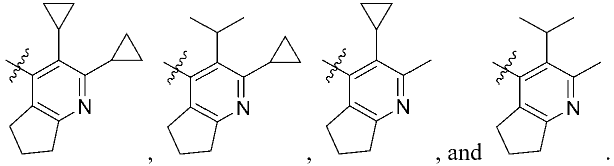

In some embodiments, the substituted ring B is:

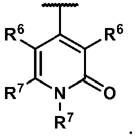

In certain of these embodiments, the substituted ring B is:

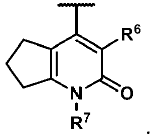

In some embodiments, the substituted ring B is

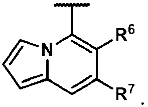

In certain of these embodiments, the substituted ring B is

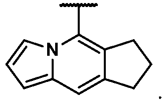

In some embodiments, the substituted ring B is

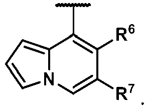

In certain of these embodiments, the substituted ring B is

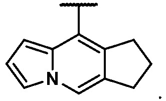

### The Ring B when Formula AA is Formula AA-3

In some embodiments, B is pyridyl or an N-oxide thereof (e.g., 2-pyridyl or an N-oxide thereof, 3-pyridyl or an N-oxide thereof, or 4-pyridyl or an N-oxide thereof).

In some embodiments, B is pyridyl (e.g., 2-pyridyl, 3-pyridyl, or 4-pyridyl).

In some embodiments, B is a pyridyl N-oxide (e.g., 2-pyridyl N-oxide, 3-pyridyl N-oxide, or 4-pyridyl N-oxide).

In some embodiments, B is pyrimidinyl or an N-oxide thereof (e.g., 4-pyrimidinyl or an N-oxide thereof, or 5-pyrimidinyl or an N-oxide thereof).

In some embodiments, B is pyridazinyl.

In some embodiments, B is pyrazinyl.

In some embodiments, B is triazinyl.

In some embodiments, B is one of the rings disclosed hereinbelow, substituted as disclosed hereinbelow, wherein in each case the bond that is shown as being broken by the wavy line 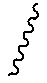 connects B to the NHC(O)group of Formula AA.

In some embodiments, the substituted ring B is

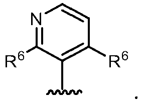

In some embodiments, the substituted ring B is

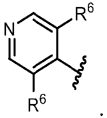

In some embodiments, the substituted ring B is

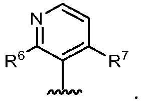

In some embodiments, the substituted ring B is

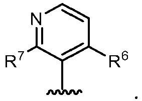

In some embodiments, the substituted ring B is

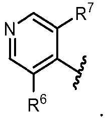

In some embodiments, the substituted ring B is

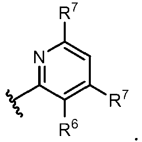

In some embodiments, the substituted ring B is

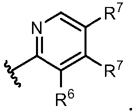

In some embodiments, the substituted ring B is

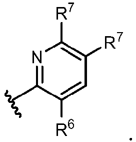

In some embodiments, the substituted ring B is

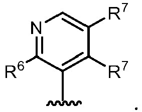

In some embodiments, the substituted ring B is

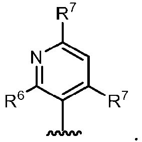

In some embodiments, the substituted ring B is

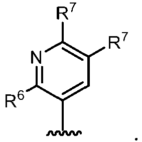

In some embodiments, the substituted ring B is

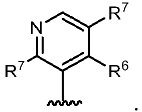

In some embodiments, the substituted ring B is

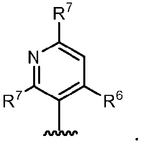

In some embodiments, the substituted ring B is

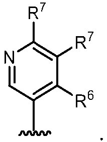

In some embodiments, the substituted ring B is

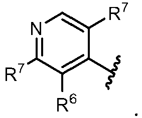

In some embodiments, the substituted ring B is

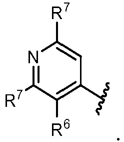

In some embodiments, the substituted ring B is

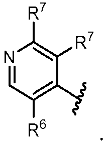

In some embodiments, the substituted ring B is

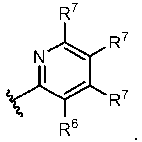

In some embodiments, the substituted ring B is

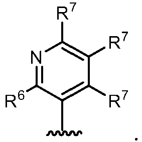

In some embodiments, the substituted ring B is

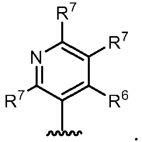

In some embodiments, the substituted ring B is

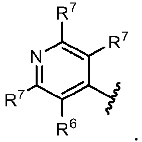

In some embodiments, the substituted ring B is

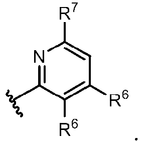

In some embodiments, the substituted ring B is

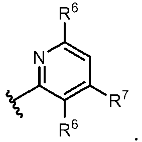

In some embodiments, the substituted ring B is

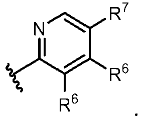

In some embodiments, the substituted ring B is

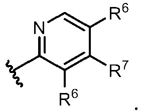

In some embodiments, the substituted ring B is

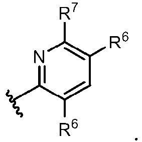

In some embodiments, the substituted ring B is

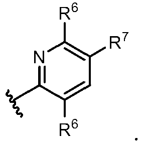

In some embodiments, the substituted ring B is

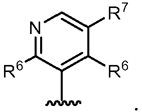

In some embodiments, the substituted ring B is

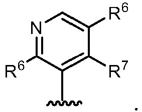

In some embodiments, the substituted ring B is

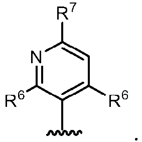

In some embodiments, the substituted ring B is

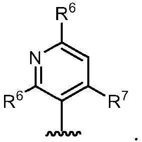

In some embodiments, the substituted ring B is

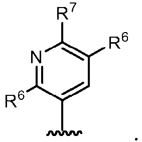

In some embodiments, the substituted ring B is

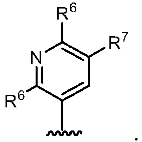

In some embodiments, the substituted ring B is

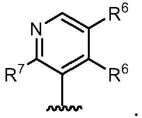

In some embodiments, the substituted ring B is

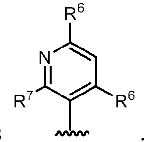

In some embodiments, the substituted ring B is

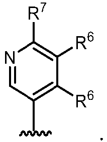

In some embodiments, the substituted ring B is

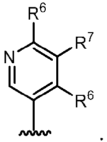

In some embodiments, the substituted ring B is

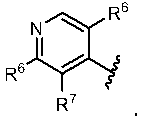

In some embodiments, the substituted ring B is

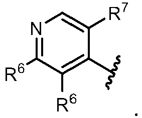

In some embodiments, the substituted ring B is

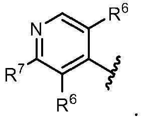

In some embodiments, the substituted ring B is

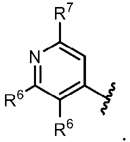

In some embodiments, the substituted ring B is

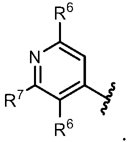

In some embodiments, the substituted ring B is

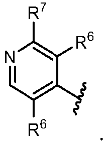

In some embodiments, the substituted ring B is

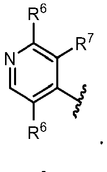

In some embodiments, the substituted ring B is

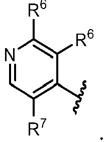

In some embodiments, the substituted ring B is

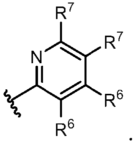

In some embodiments, the substituted ring B is

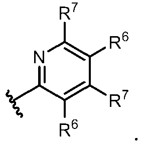

In some embodiments, the substituted ring B is

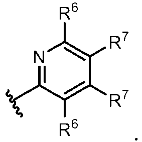

In some embodiments, the substituted ring B is

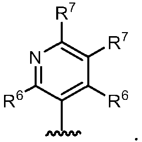

In some embodiments, the substituted ring B is

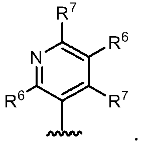

In some embodiments, the substituted ring B is

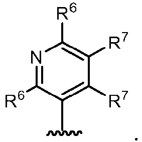

In some embodiments, the substituted ring B is

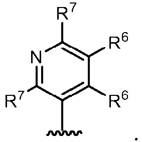

In some embodiments, the substituted ring B is

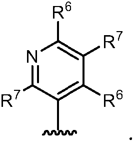

In some embodiments, the substituted ring B is

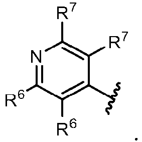

In some embodiments, the substituted ring B is

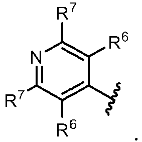

In some embodiments, the substituted ring B is

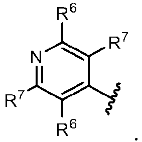

In some embodiments, the substituted ring B is

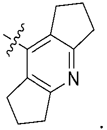

In some embodiments, the substituted ring B is

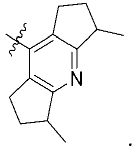

In some embodiments, the substituted ring B is

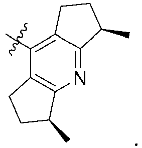

In some embodiments, the substituted ring B is

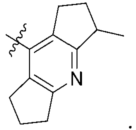

In some embodiments, the substituted ring B is

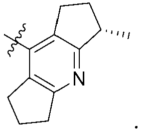

In some embodiments, the substituted ring B is

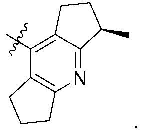

In some embodiments, the substituted ring B is

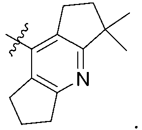

In some embodiments, the substituted ring B is

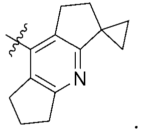

In some embodiments, the substituted ring B is

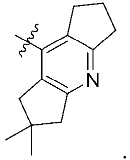

In some embodiments, the substituted ring B is

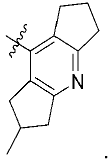

In some embodiments, the substituted ring B is

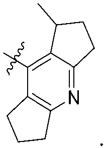

In some embodiments, the substituted ring B is

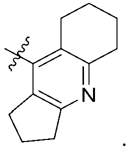

In some embodiments, the substituted ring B is

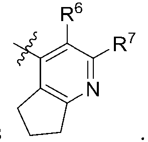

In some embodiments, the substituted ring B is

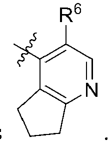

In some embodiments, the substituted ring B is

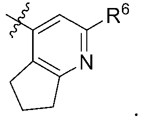

In some embodiments, the substituted ring B is

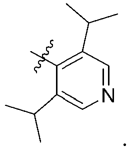

In some embodiments, the substituted ring B is

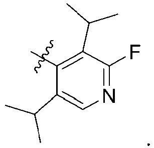

In some embodiments, the substituted ring B is

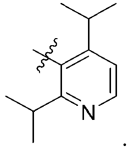

In some embodiments, the substituted ring B is

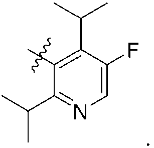

In some embodiments, the substituted ring B is

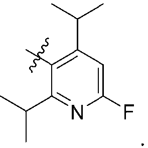

In some embodiments, the substituted ring B is selected from the group consisting of: 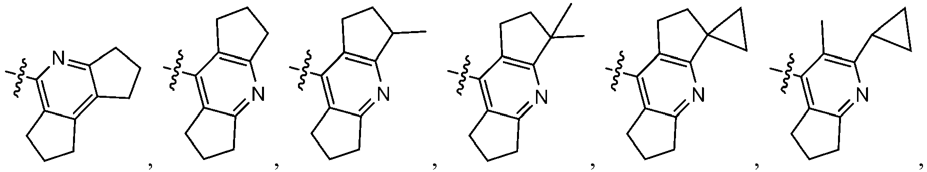 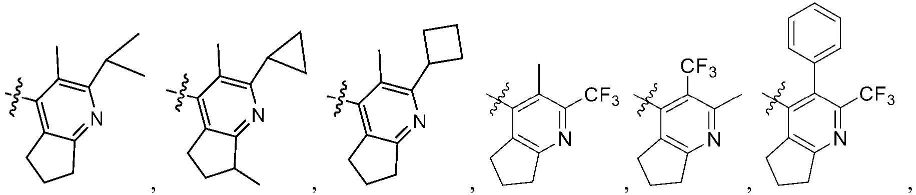 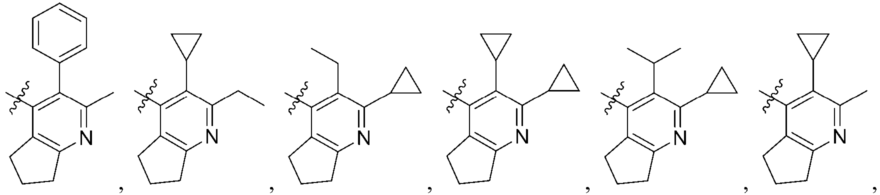 and

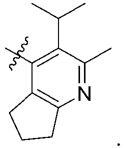

In some embodiments, the substituted ring B is selected from the group consisting of: 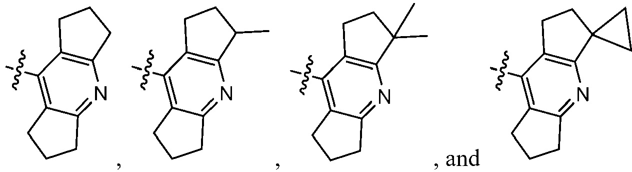 (e.g., 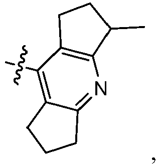 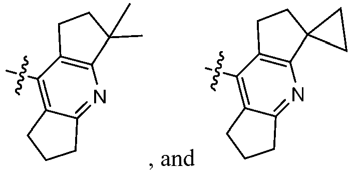 ).

In some embodiments, the substituted ring B is selected from the group consisting of:

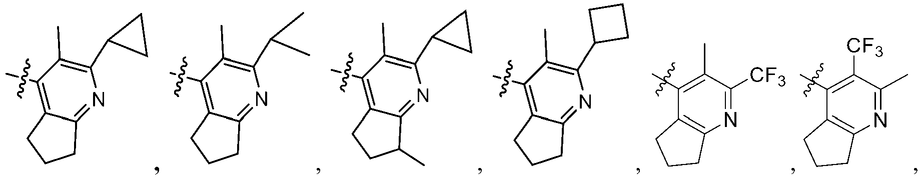

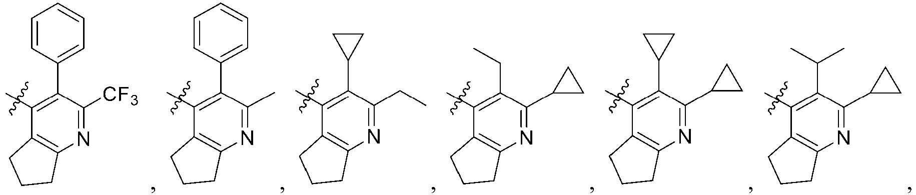

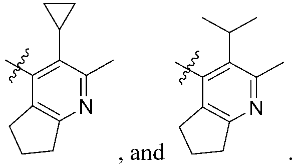

In some embodiments, the substituted ring B is selected from the group consisting of:

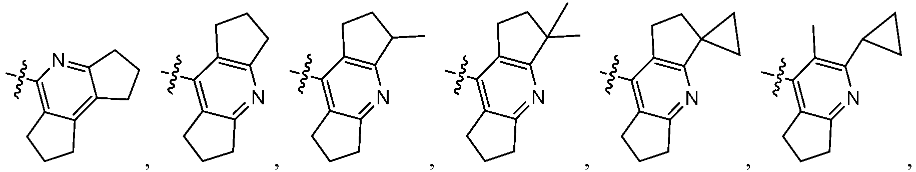

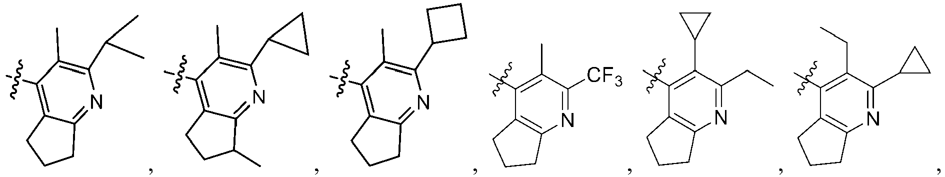

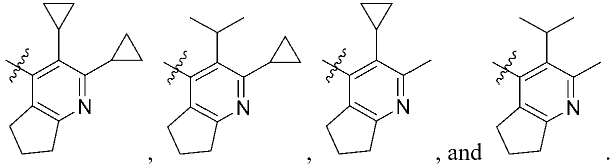

### The Ring B' when Formula AA is Formula AA-4

In some embodiments, B' is 2-pyridyl or 3-pyridyl, or an N-oxide thereof.

In some embodiments, B' is 2-pyridyl.

In some embodiments, B' is 3-pyridyl.

In some embodiments, B' is 2-pyridyl N-oxide.

In some embodiments, B' is 3-pyridyl N-oxide.

In some embodiments, B' is one of the rings disclosed hereinbelow, substituted as disclosed hereinbelow, wherein in each case the bond that is shown as being broken by the wavy line 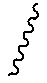 connects B' to the NHC(O)group of Formula AA.

In some embodiments, the substituted ring B' is

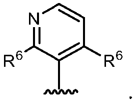

In some embodiments, the substituted ring B' is

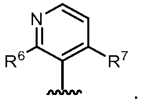

In some embodiments, the substituted ring B' is

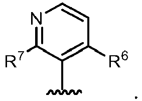

In some embodiments, the substituted ring B' is

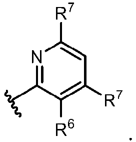

In some embodiments, the substituted ring B' is

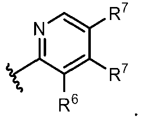

In some embodiments, the substituted ring B' is

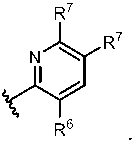

In some embodiments, the substituted ring B' is

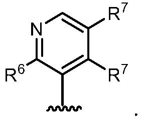

In some embodiments, the substituted ring B' is

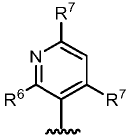

In some embodiments, the substituted ring B' is

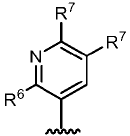

In some embodiments, the substituted ring B' is

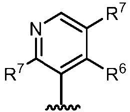

In some embodiments, the substituted ring B' is

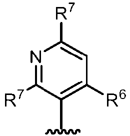

In some embodiments, the substituted ring B' is

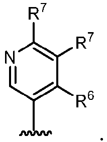

In some embodiments, the substituted ring B' is

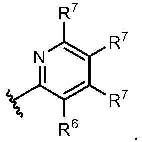

In some embodiments, the substituted ring B' is

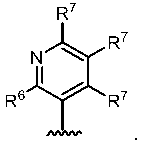

In some embodiments, the substituted ring B' is

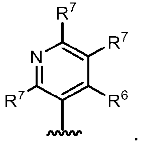

In some embodiments, the substituted ring B' is

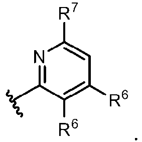

In some embodiments, the substituted ring B' is

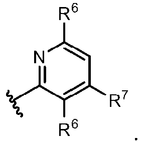

In some embodiments, the substituted ring B' is

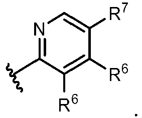

In some embodiments, the substituted ring B' is

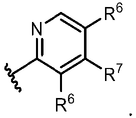

In some embodiments, the substituted ring B' is

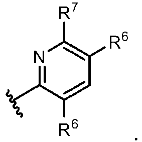

In some embodiments, the substituted ring B' is

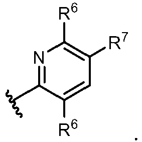

In some embodiments, the substituted ring B' is

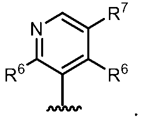

In some embodiments, the substituted ring B' is

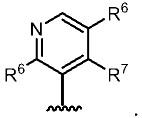

In some embodiments, the substituted ring B' is

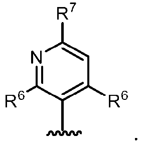

In some embodiments, the substituted ring B' is

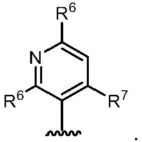

In some embodiments, the substituted ring B' is

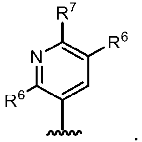

In some embodiments, the substituted ring B' is

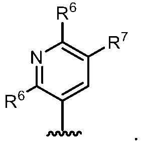

In some embodiments, the substituted ring B' is

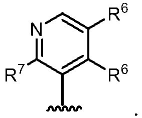

In some embodiments, the substituted ring B' is

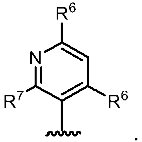

In some embodiments, the substituted ring B' is

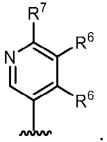

In some embodiments, the substituted ring B' is

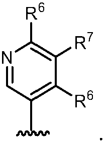

In some embodiments, the substituted ring B' is

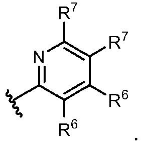

In some embodiments, the substituted ring B' is

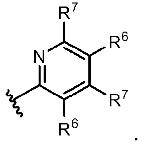

In some embodiments, the substituted ring B' is

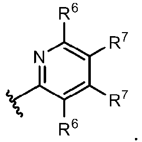

In some embodiments, the substituted ring B' is

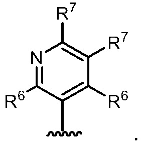

In some embodiments, the substituted ring B' is

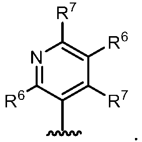

In some embodiments, the substituted ring B' is

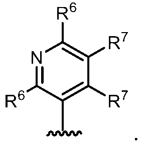

In some embodiments, the substituted ring B' is

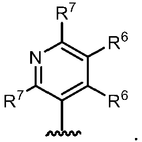

In some embodiments, the substituted ring B' is

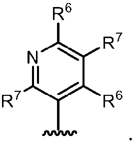

In some embodiments, the substituted ring B' is

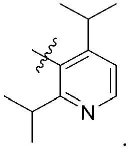

In some embodiments, the substituted ring B' is

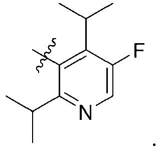

In some embodiments, the substituted ring B' is

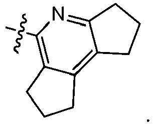

In some embodiments, the substituted ring B' is:

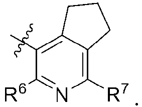

In some embodiments, the substituted ring B' is:

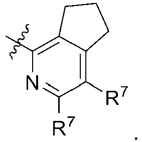

In some embodiments, the substituted ring B' is

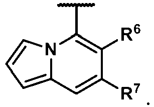

In certain of these embodiments, the substituted ring B' is

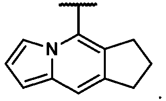

In some embodiments, the substituted ring B' is

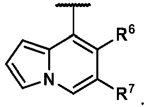

In certain of these embodiments, the substituted ring B' is

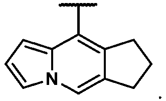

### The Ring B"

In some embodiments, B" is 4-pyridyl.

In some embodiments, B" is 4-pyridyl N-oxide.

In some embodiments, B" is one of the rings disclosed hereinbelow, substituted as disclosed hereinbelow, wherein in each case the bond that is shown as being broken by the wavy line 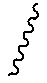 connects B" to the NHC(O)group of Formula AA.

In some embodiments, the substituted ring B" is

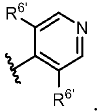

In some embodiments, the substituted ring B" is

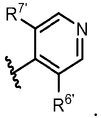

In some embodiments, the substituted ring B" is

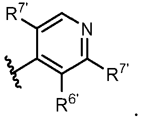

In some embodiments, the substituted ring B" is

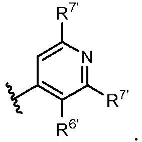

In some embodiments, the substituted ring B" is

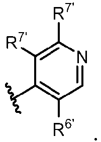

In some embodiments, the substituted ring B" is

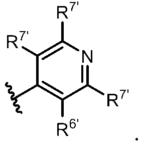

In some embodiments, the substituted ring B" is

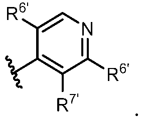

In some embodiments, the substituted ring B" is

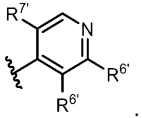

In some embodiments, the substituted ring B" is

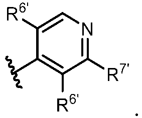

In some embodiments, the substituted ring B" is

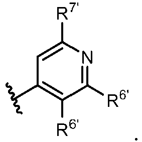

In some embodiments, the substituted ring B" is

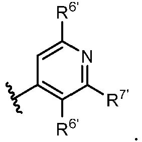

In some embodiments, the substituted ring B" is

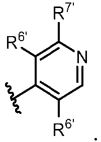

In some embodiments, the substituted ring B" is

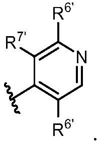

In some embodiments, the substituted ring B" is

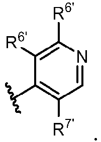

In some embodiments, the substituted ring B" is

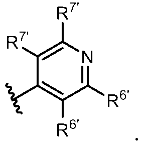

In some embodiments, the substituted ring B" is

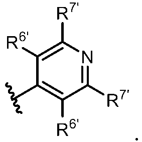

In some embodiments, the substituted ring B" is

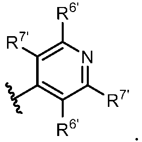

In some embodiments, the substituted ring B" is

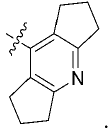

In some embodiments, the substituted ring B" is

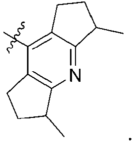

In some embodiments, the substituted ring B" is

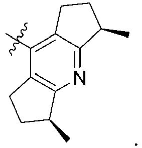

In some embodiments, the substituted ring B" is

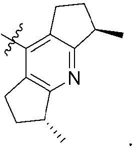

In some embodiments, the substituted ring B" is

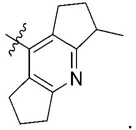

In some embodiments, the substituted ring B" is

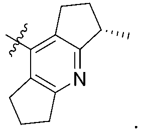

In some embodiments, the substituted ring B" is

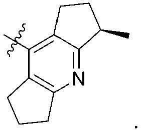

In some embodiments, the substituted ring B" is

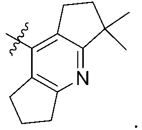

In some embodiments, the substituted ring B" is

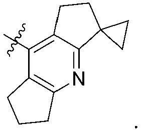

In some embodiments, the substituted ring B" is

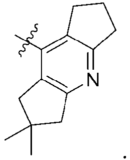

In some embodiments, the substituted ring B" is

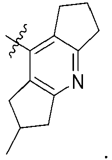

In some embodiments, the substituted ring B" is

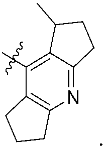

In some embodiments, the substituted ring B" is

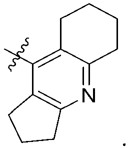

In some embodiments, the substituted ring B" is

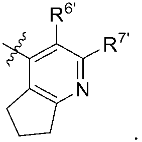

In some embodiments, the substituted ring B" is

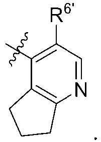

In some embodiments, the substituted ring B" is

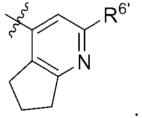

In some embodiments, the substituted ring B" is selected from the group consisting of:

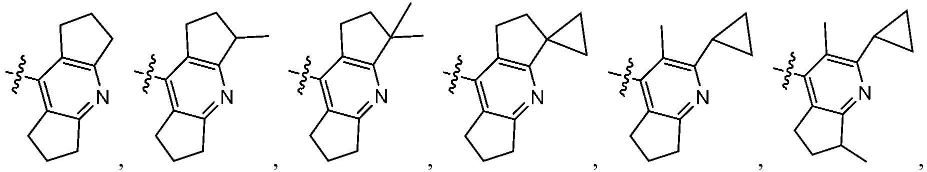

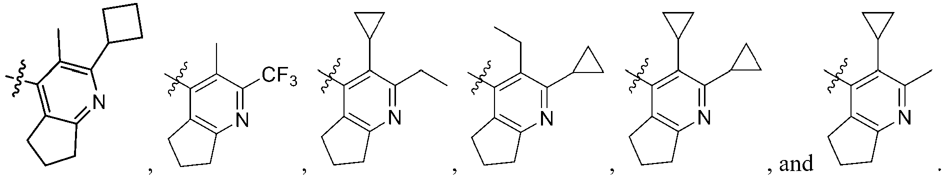

In some embodiments, the substituted ring B" is selected from the group consisting of:

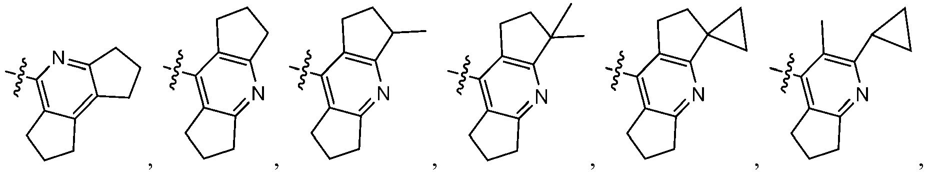

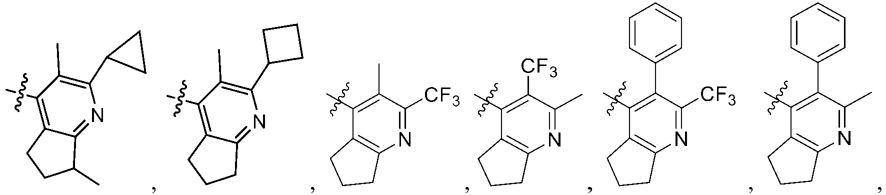

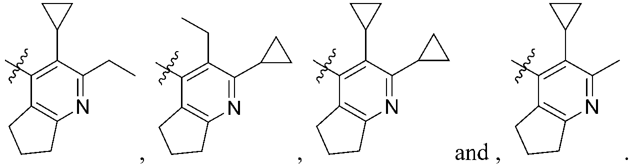

In some embodiments, the substituted ring B" is selected from the group consisting of: 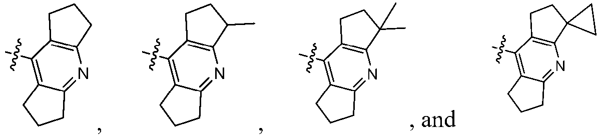 (e.g., 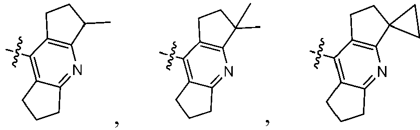 ).

In some embodiments, the substituted ring B" is selected from the group consisting of:

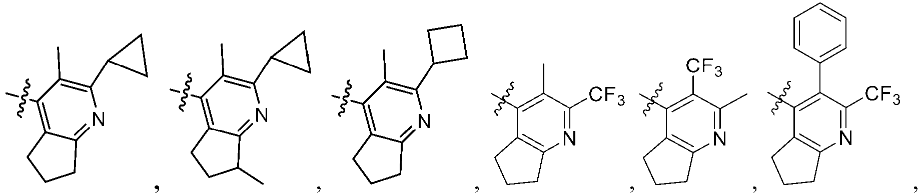

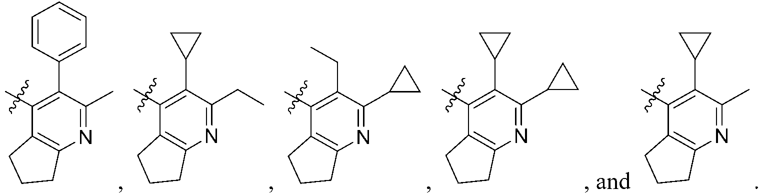

In some embodiments, the substituted ring B" is:

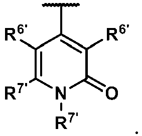

In certain of these embodiments, the substituted ring B" is:

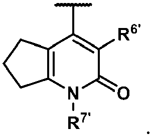

### The groups R⁶, R^{6'}, R^{6"}, R⁷, and R^{7'}

### The Groups R⁶ and R⁷

In some embodiments, R⁶ and R⁷ are each independently selected from C₁-C₆ alkyl, C₁-C₆ haloalkyl, C₁-C₆ alkoxy, C₁-C₆ haloalkoxy, halo, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,

In some embodiments, each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments, R⁶ and R⁷ are each independently selected from a C₂-C₆ alkyl, C₂-C₆ haloalkyl, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, and wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments, R⁶ and R⁷ are each independently selected from a C₂-C₆ alkyl, C₂-C₆ haloalkyl, C₂-C₆ alkoxy, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3-to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3-to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments, R⁶ and R⁷ are each independently selected from a C₂-C₆ alkyl, C₂-C₆ haloalkyl, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments, R⁶ and R⁷ are each independently selected from a C₂-C₆ alkyl, C₂-C₆ haloalkyl, C₂-C₆ alkoxy, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3-to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3-to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments, each R⁶ is independently selected from the group consisting of: C₁-C₆ alkyl, C₃-C₇ cycloalkyl, halo, and C₆-C₁₀ aryl.

In some embodiments, each R⁶ is independently selected from the group consisting of: C₁-C₆ alkyl, halo, C₃-C₇ cycloalkyl, and C₆-C₁₀ aryl.

In some embodiments, each R⁶ is independently selected from the group consisting of: C₁-C₆ alkyl and C₃-C₇ cycloalkyl.

In some embodiments, each R⁶ is independently selected from the group consisting of: methyl, isopropyl, cyclopropyl, fluoro, and phenyl. For example, each R⁶ is isopropyl.

In some embodiments, each R⁷, when present, is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4-to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments, each R⁷, when present, is independently selected from the group consisting of: C₁-C₆ alkyl, C₃-C₇ cycloalkyl, halo, and C₆-C₁₀ aryl.

In some embodiments, each R⁷, when present, is independently selected from the group consisting of: methyl, isopropyl, cyclopropyl, fluoro, and phenyl.

In some embodiments, one pair of R⁶ and R⁷ on adjacent atoms, taken together with the atoms connecting them, independently form at least one C₄-C₈ carbocyclic ring or at least one 5-to 8-membered heterocyclic ring containing 1 or 2 and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments, one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In certain embodiments (when one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹),the C₄-C₈ carbocyclic ring is a C₅ carbocyclic ring optionally substituted with one or more oxo, CH₃, or hydroxy. For example, the C₅ carbocyclic ring is substituted with one CH₃. For example, the C₅ carbocyclic ring is geminally substituted with two CH₃.

In certain embodiments (when one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹), the C₄-C₈ carbocyclic ring is a C₇ carbocyclic ring, wherein the C₇ carbocyclic ring is a bicyclic spirocycle, wherein the bicyclic spirocycle comprises a 5-membered ring and a 3-membered ring.

In some embodiments, each of R⁶ and R⁷ is independently selected from the group consisting of: C₁-C₆ alkyl, C₁-C₆ haloalkyl, C₃-C₇ cycloalkyl, halo, and C₆-C₁₀ aryl; or
one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

### The Groups R^{6"} and R⁷ when Formula AA is Formula AA-1

In some embodiments of the compound of Formula AA-1, R^{6"} is selected from C₁-C₆ alkyl, C₁-C₆ haloalkyl, C₁-C₆ alkoxy, C₁-C₆ haloalkoxy, halo, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl, 3- to 10-membered heterocycloalkyl, and C₂-C₆ alkenyl,
wherein R^{6"} is optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and
wherein the C₁-C₆ alkyl or C₁-C₆ alkoxy that R^{6"} is substituted with is optionally substituted with one or more hydroxyl, C₆-C₁₀ aryl or NR⁸R⁹, or wherein R^{6"} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;

In some embodiments of the compound of Formula AA-1, each R^{6"} is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-1, each R^{6"} is independently selected from the group consisting of: C₁-C₆ alkyl, halo, C₃-C₇ cycloalkyl, and C₆-C₁₀ aryl.

In some embodiments of the compound of Formula AA-1, each R^{6"} is independently selected from the group consisting of: C₁-C₆ alkyl and C₃-C₇ cycloalkyl.

In some embodiments of the compound of Formula AA-1, each R^{6"} is independently selected from the group consisting of: methyl, isopropyl, cyclopropyl, fluoro, and phenyl. For example, each R^{6"} is isopropyl.

In some embodiments of the compound of Formula AA-1, each R⁷, when present, is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-1, R⁷ are each independently selected from C₁-C₆ alkyl, C₁-C₆ haloalkyl, C₁-C₆ alkoxy, C₁-C₆ haloalkoxy, halo, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,

In some embodiments of the compound of Formula AA-1, each R⁷, when present, is independently selected from the group consisting of: C₁-C₆ alkyl, C₃-C₇ cycloalkyl, halo, and C₆-C₁₀ aryl.

In some embodiments of the compound of Formula AA-1, each R⁷, when present, is independently selected from the group consisting of: methyl, isopropyl, cyclopropyl, fluoro, and phenyl.

In some embodiments of the compound of Formula AA-1, one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-1, one pair of R^{6"} and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In certain embodiments of the compound of Formula AA-1 (when one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹), the C₄-C₈ carbocyclic ring is a C₅ carbocyclic ring optionally substituted with one or more oxo, CH₃, or hydroxy. For example, the C₅ carbocyclic ring is substituted with one CH₃. For example, the C₅ carbocyclic ring is geminally substituted with two CH₃.

In certain embodiments of the compound of Formula AA-1 (when one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹), the C₄-C₈ carbocyclic ring is a C₇ carbocyclic ring, wherein the C₇ carbocyclic ring is a bicyclic spirocycle, wherein the bicyclic spirocycle comprises a 5-membered ring and a 3-membered ring.

In some embodiments of the compound of Formula AA-1, R^{6"} is selected from C₁-C₆ alkyl, C₁-C₆ haloalkyl, C₁-C₆ alkoxy, C₁-C₆ haloalkoxy, halo, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl, 3- to 10-membered heterocycloalkyl, and C₂-C₆ alkenyl,
wherein R^{6"} is optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R^{6"} is substituted with is optionally substituted with one or more hydroxyl, C₆-C₁₀ aryl or NR⁸R⁹, or wherein R^{6"} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;

In some embodiments of the compound of Formula AA-1, R^{6"} is selected from C₂-C₆ alkyl, C₂-C₆ haloalkyl, C₂-C₆ alkoxy, C₁-C₆ haloalkoxy, I, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3-to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl, 3- to 10-membered heterocycloalkyl, and C₂-C₆ alkenyl,
wherein R^{6"} is optionally substituted with one or more substituents independently selected from hydroxy, Cl, Br, I, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R^{6"} is substituted with is optionally substituted with one or more hydroxyl, C₆-C₁₀ aryl or NR⁸R⁹, or wherein R^{6"} is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
R⁷ is selected from C₂-C₆ alkyl, C₂-C₆ haloalkyl, C₂-C₆ alkoxy, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5-to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl, wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-1, R^{6"} is selected from C₂-C₆ alkyl, C₂-C₆ haloalkyl, C₁-C₆ haloalkoxy, I, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl, 3- to 10-membered heterocycloalkyl, and C₂-C₆ alkenyl,
wherein R^{6"} is optionally substituted with one or more substituents independently selected from hydroxy, Cl, Br, I, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R^{6"} is substituted with is optionally substituted with one or more hydroxyl, C₆-C₁₀ aryl or NR⁸R⁹, or wherein R^{6"} is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
R⁷ is selected from C₂-C₆ alkyl, C₂-C₆ haloalkyl, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl, wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments, each of R^{6"} and R⁷ is independently selected from the group consisting of: C₁-C₆ alkyl, C₁-C₆ haloalkyl, C₃-C₇ cycloalkyl, halo, and C₆-C₁₀ aryl; or
one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

### The Groups R⁶ and R⁷ when Formula AA is Formula AA-2

In some embodiments of the compound of Formula AA-2, R⁶ and R⁷ are each independently selected from C₂-C₆ alkyl, C₂-C₆ haloalkyl, C₂-C₆ alkoxy, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-2, R⁶ and R⁷ are each independently selected from C₂-C₆ alkyl, C₂-C₆ haloalkyl, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl, wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-2, each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-2, each R⁶ is independently selected from the group consisting of: C₁-C₆ alkyl, halo, C₃-C₇ cycloalkyl, and C₆-C₁₀ aryl.

In some embodiments of the compound of Formula AA-2, each R⁷ is independently selected from the group consisting of: C₁-C₆ alkyl, C₃-C₇ cycloalkyl, halo, and C₆-C₁₀ aryl.

In some embodiments of the compound of Formula AA-2, each R⁶ is independently selected from the group consisting of: C₁-C₆ alkyl and C₃-C₇ cycloalkyl.

In some embodiments of the compound of Formula AA-2, each R⁶ is independently selected from the group consisting of: methyl, isopropyl, cyclopropyl, fluoro, and phenyl. For example, each R⁶ is isopropyl.

In some embodiments of the compound of Formula AA-2, each R⁷, when present, is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-2, each R⁷ is independently selected from the group consisting of: C₁-C₆ alkyl, C₃-C₇ cycloalkyl, halo, and C₆-C₁₀ aryl.

In some embodiments of the compound of Formula AA-2, each R⁷ is independently selected from the group consisting of: methyl, isopropyl, cyclopropyl, fluoro, and phenyl.

In some embodiments of the compound of Formula AA-2, one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-2, one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In certain embodiments of the compound of Formula AA-2 (when one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.), the C₄-C₈ carbocyclic ring is a C₅ carbocyclic ring optionally substituted with one or more oxo, CH₃, or hydroxy. For example, the C₅ carbocyclic ring is substituted with one CH₃. For example, the C₅ carbocyclic ring is geminally substituted with two CH₃.

In certain embodiments of the compound of Formula AA-2 (when one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.), the C₇ carbocyclic ring is a bicyclic spirocycle, wherein the bicyclic spirocycle comprises a 5-membered ring and a 3-membered ring.

In some embodiments, each of R⁶ and R⁷ is independently selected from the group consisting of: C₁-C₆ alkyl, C₁-C₆ haloalkyl, C₃-C₇ cycloalkyl, halo, and C₆-C₁₀ aryl; or
one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

### The Groups R⁶ and R⁷ when Formula AA is Formula AA-3

In some embodiments of the compound of Formula AA-3, R⁶ and R⁷ are each independently selected from C₂-C₆ alkyl, C₂-C₆ haloalkyl, C₂-C₆ alkoxy, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-3, R⁶ and R⁷ are each independently selected from C₂-C₆ alkyl, C₂-C₆ haloalkyl, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl, wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-3, each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-3, each R⁶ is independently selected from the group consisting of: C₁-C₆ alkyl, halo, C₃-C₇ cycloalkyl, and C₆-C₁₀ aryl.

In some embodiments of the compound of Formula AA-3, each R⁶ is independently selected from the group consisting of: C₁-C₆ alkyl and C₃-C₇ cycloalkyl.

In some embodiments of the compound of Formula AA-3, each R⁶ is independently selected from the group consisting of: methyl, isopropyl, cyclopropyl, fluoro, and phenyl. For example, each R⁶ is isopropyl.

In some embodiments of the compound of Formula AA-3, each R⁷, when present, is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-3, each R⁷, when present, is independently selected from the group consisting of: C₁-C₆ alkyl, C₃-C₇ cycloalkyl, halo, and C₆-C₁₀ aryl.

In some embodiments of the compound of Formula AA-3, each R⁷, when present, is independently selected from the group consisting of: methyl, isopropyl, cyclopropyl, fluoro, and phenyl.

In some embodiments of the compound of Formula AA-3, one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-3, one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In certain embodiments of the compound of Formula AA-3 (when one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹), the C₄-C₈ carbocyclic ring is a C₅ carbocyclic ring optionally substituted with one or more oxo, CH₃, or hydroxy. For example, the C₅ carbocyclic ring is substituted with one CH₃. For example, the C₅ carbocyclic ring is geminally substituted with two CH₃.

In certain embodiments of the compound of Formula AA-3 (when one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹)the C₄-C₈ carbocyclic ring is a C₇ carbocyclic ring, wherein the C₇ carbocyclic ring is a bicyclic spirocycle, wherein the bicyclic spirocycle comprises a 5-membered ring and a 3-membered ring.

In some embodiments, each of R⁶ and R⁷ is independently selected from the group consisting of: C₁-C₆ alkyl, C₁-C₆ haloalkyl, C₃-C₇ cycloalkyl, halo, and C₆-C₁₀ aryl; or
one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

### The Groups R⁶ and R⁷ when Formula AA is Formula AA-4

In some embodiments of the compound of Formula AA-4, R⁶ and R⁷ are each independently selected from C₂-C₆ alkyl, C₂-C₆ haloalkyl, C₂-C₆ alkoxy, C₁-C₆ haloalkoxy, I, CN, NO₂, COC₁-C₆ alkyl, CO₂C_{I1}-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-4, R⁶ and R⁷ are each independently selected from C₂-C₆ alkyl, C₂-C₆ haloalkyl, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl, wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-4, each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-4, each R⁶ is independently selected from the group consisting of: C₁-C₆ alkyl, halo, C₃-C₇ cycloalkyl, and C₆-C₁₀ aryl.

In some embodiments of the compound of Formula AA-4, each R⁶ is independently selected from the group consisting of: methyl, isopropyl, cyclopropyl, fluoro, and phenyl.

In some embodiments of the compound of Formula AA-4, each R⁷, when present, is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-4, each R⁷, when present, is independently selected from the group consisting of: C₁-C₆ alkyl, C₃-C₇ cycloalkyl, halo, and C₆-C₁₀ aryl.

In some embodiments of the compound of Formula AA-4, each R⁷, when present, is independently selected from the group consisting of: methyl, isopropyl, cyclopropyl, fluoro, and phenyl.

In some embodiments, each of R⁶ and R⁷ is independently selected from the group consisting of: C₁-C₆ alkyl, C₁-C₆ haloalkyl, C₃-C₇ cycloalkyl, halo, and C₆-C₁₀ aryl; or
one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

### The Groups R^{6'} and R^{7'} when Formula AA is Formula AA-5

In some embodiments of the compound of Formula AA-5, R^{6'} and R^{7'} are each independently selected from unbranched C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R^{6'} and R^{7'} are each optionally substituted with one or more substituents independently selected from hydroxy, halo, CN, oxo, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkoxy that R^{6'} or R^{7'} is substituted with is optionally substituted with one or more hydroxyl, C₆-C₁₀ aryl or NR⁸R⁹, or wherein R^{6'} or R^{7'} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹.

In some embodiments of the compound of Formula AA-5, R^{6'} and R^{7'} are each independently selected from unbranched C₂-C₆ alkyl, C₂-C₆ haloalkyl, C₂-C₆ alkoxy, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R^{6'} and R^{7'} are each optionally substituted with one or more substituents independently selected from hydroxy, halo, CN, oxo, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkoxy that R^{6'} or R^{7'} is substituted with is optionally substituted with one or more hydroxyl, C₆-C₁₀ aryl or NR⁸R⁹, or wherein R^{6'} or R^{7'} is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶' and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-5, R^{6'} and R^{7'} are each independently selected from unbranched C₂-C₆ alkyl, C₂-C₆ haloalkyl, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5-to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl, wherein R^{6'} and R^{7'} are each optionally substituted with one or more substituents independently selected from hydroxy, halo, CN, oxo, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkoxy that R^{6'} or R^{7'} is substituted with is optionally substituted with one or more hydroxyl, C₆-C₁₀ aryl or NR⁸R⁹, or wherein R^{6'} or R^{7'} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-5, each R^{6'} is independently selected from the group consisting of: unbranched C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the unbranched C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-5, each R^{6'} is independently selected from the group consisting of: unbranched C₁-C₆ alkyl, C₃-C₇ cycloalkyl, halo, and C₆-C₁₀ aryl.

In some embodiments of the compound of Formula AA-5, each R^{6'} is independently selected from the group consisting of: unbranched C₁-C₆ alkyl and C₃-C₇ cycloalkyl.

In some embodiments of the compound of Formula AA-5, each R^{6'} is independently selected from the group consisting of: methyl, cyclopropyl, fluoro, and phenyl.

In some embodiments of the compound of Formula AA-5, each R^{7'} is independently selected from the group consisting of: unbranched C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In some embodiments of the compound of Formula AA-5, each R^{7'} is independently selected from the group consisting of: unbranched C₁-C₆ alkyl, C₃-C₇ cycloalkyl, halo, and C₆-C₁₀ aryl.

In some embodiments of the compound of Formula AA-5, each R^{7'} is independently selected from the group consisting of: methyl, cyclopropyl, fluoro, and phenyl.

In some embodiments of the compound of Formula AA-5, one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-5, one pair of R^{6'} and R^{7'} on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In certain embodiments of the compound of Formula AA-5 (when one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹), the C₄-C₈ carbocyclic ring is a C₅ carbocyclic ring optionally substituted with one or more oxo, CH₃, or hydroxy. For example, the C₅ carbocyclic ring is substituted with one CH₃. For example, the C₅ carbocyclic ring is geminally substituted with two CH₃.

In certain embodiments of the compound of Formula AA-5 (when one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹),the C₄-C₈ carbocyclic ring is a C₇ carbocyclic ring, wherein the C₇ carbocyclic ring is a bicyclic spirocycle, wherein the bicyclic spirocycle comprises a 5-membered ring and a 3-membered ring.

In some embodiments, each of R^{6'} and R^{7'} is independently selected from the group consisting of: C₁-C₆ alkyl, C₁-C₆ haloalkyl, C₃-C₇ cycloalkyl, halo, and C₆-C₁₀ aryl; or
one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

### The group R³

In some embodiments, R³ is selected from hydrogen, C₁-C₆ alkyl, and 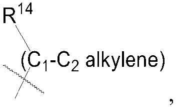 wherein the C₁-C₂ alkylene group is optionally substituted with oxo.

In some embodiments, R³ is hydrogen.

In some embodiments, R³ is other than hydrogen.

In some embodiments, R³ is cyano.

In some embodiments, R³ is hydroxy.

In some embodiments, R³ is C₁-C₆ alkoxy.

In some embodiments, R³ is C₁-C₆ alkyl.

In some embodiments, R³ is methyl.

In some embodiments, R³ is 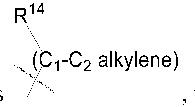 wherein the C₁-C₂ alkylene group is optionally

substituted with oxo.

In some embodiments, R³ is -CH₂R¹⁴.

In some embodiments, R³ is -C(O)R¹⁴. In certain of these embodiments, R³ is CHO. In certain other of these embodiments, R³ is C(O)C₁-C₆ alkyl.

In some embodiments, R³ is -CH₂CH₂R¹⁴.

In some embodiments, R³ is -CHR¹⁴CH₃.

In some embodiments, R³ is -CH₂C(O)R¹⁴.

In some embodiments, R³ is -C(O)CH₂R¹⁴.

In some embodiments, R³ is CO₂C₁-C₆ alkyl (e.g., CO₂*t*-Bu).

### The group R¹⁴

In some embodiments, R¹⁴ is hydrogen, C₁-C₆ alkyl, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶.

In some embodiments, R¹⁴ is hydrogen or C₁-C₆ alkyl.

In some embodiments, R¹⁴ is hydrogen, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶.

In some embodiments, R¹⁴ is hydrogen.

In some embodiments, R¹⁴ is C₁-C₆ alkyl.

In some embodiments, R¹⁴ is methyl.

In some embodiments, R¹⁴ is 5- to 10-membered monocyclic or bicyclic heteroaryl optionally independently substituted with 1 or 2 R⁶.

In some embodiments, R¹⁴ is C₆-C₁₀ monocyclic or bicyclic aryl optionally independently substituted with 1 or 2 R⁶.

### The moiety S(=O)(NHR³)=N-

In some embodiments, the sulfur in the moiety S(=O)(NHR³)=N- has (S) stereochemistry.

In some embodiments, the sulfur in the moiety S(=O)(NHR³)=N- has (R) stereochemistry.

### The group R¹⁰

In some embodiments, R¹⁰ is C₁-C₆ alkyl.

In some embodiments, R¹⁰ is methyl.

In some embodiments, R¹⁰ is ethyl.

### The groups R⁸ and R⁹

In some embodiments, each of R⁸ and R⁹ at each occurrence is independently selected from hydrogen, C₁-C₆ alkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl or 3- to 7-membered heterocycloalkyl; or R⁸ and R⁹ taken together with the nitrogen they are attached to form a 3- to 7-membered ring optionally containing one or more heteroatoms in addition to the nitrogen they are attached to.

In some embodiments, each of R⁸ and R⁹ at each occurrence is independently selected from hydrogen, C₁-C₆ alkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl or 3- to 7-membered heterocycloalkyl; or R⁸ and R⁹ taken together with the nitrogen they are attached to form a 3- to 7-membered ring optionally containing one or more heteroatoms in addition to the nitrogen they are attached to.

In some embodiments, each of R⁸ and R⁹ at each occurrence is hydrogen,

In some embodiments, each R⁸ at each occurrence is hydrogen and each R⁹ at each occurrence is C₁-C₆ alkyl.

In some embodiments, each R⁸ at each occurrence is hydrogen and each R⁹ at each occurrence is methyl.

In some embodiments, each R⁸ at each occurrence is hydrogen and each R⁹ at each occurrence is ethyl.

In some embodiments, each of R⁸ and R⁹ at each occurrence is methyl.

In some embodiments, each of R⁸ and R⁹ at each occurrence is ethyl.

In some embodiments, R⁸ and R⁹ taken together with the nitrogen they are attached to form a 3-membered ring.

In some embodiments, R⁸ and R⁹ taken together with the nitrogen they are attached to form a 4-membered ring.

In some embodiments, R⁸ and R⁹ taken together with the nitrogen they are attached to form a 5-membered ring.

In some embodiments, R⁸ and R⁹ taken together with the nitrogen they are attached to form a 6-membered ring optionally containing one or more oxygen atoms in addition to the nitrogen they are attached to.

In some embodiments, R⁸ and R⁹ taken together with the nitrogen they are attached to form a 6-membered ring optionally containing one or more nitrogen atoms in addition to the nitrogen they are attached to.

In some embodiments, R⁸ and R⁹ taken together with the nitrogen they are attached to form a 7-membered ring.

In some embodiments, one of R⁸ and R⁹ is C(O)R¹³; R¹³ is -(Z¹-Z^{Z})ₐ₁-Z³; and a1 is 0. In certain of these embodiments, the other one of R⁸ and R⁹ is hydrogen.

As a non-limiting example of the foregoing embodiments, NR⁸R⁹ is selected from the group consisting of: NHCOC₆-C₁₀ aryl, NHCO(5- to 10-membered heteroaryl), NHCO(3- to 7-membered heterocycloalkyl), and NHCOC₂-C₆ alkynyl.

In some embodiments, one of R⁸ and R⁹ is C(O)R¹³; R¹³ is C₁-C₆ alkyl.

In certain embodiments, NR⁸R⁹ is selected from the group consisting of: NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, and NH(C=NR¹³)NR¹¹R¹².

In certain embodiments, NR⁸R⁹ is selected from the group consisting of: NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, and NHCOOC₁-C₆ alkyl.

### The group R¹³

In some embodiments, R¹³ is C₁-C₆ alkyl.

In some embodiments, R¹³ is methyl.

In some embodiments, R¹³ is ethyl.

In some embodiments, R¹³ is C₆-C₁₀ aryl.

In some embodiments, R¹³ is phenyl.

In some embodiments, R¹³ is 5- to 10-membered heteroaryl.

In some embodiments, R¹³ is -(Z¹-Z²)ₐ₁-Z³.

In certain of these embodiments, a1 is 0. In certain embodiments, Z³ is C₆-C₁₀ aryl or 5- to 10-membered heteroaryl.

In some embodiments, R¹³ is C₆-C₁₀ aryl.

In some embodiments, R¹³ is phenyl.

In some embodiments, R¹³ is 5- to 10-membered heteroaryl.

In some embodiments, C(O)R¹³ is selected from COC₁-C₆ alkyl, CO-C₆-C₁₀ aryl, and CO(5- to 10-membered heteroaryl).

### The groups R¹¹ and R¹²

In some embodiments, each of R¹¹ and R¹² at each occurrence is independently selected from hydrogen and C₁-C₆ alkyl.

In some embodiments, each of R¹¹ and R¹² at each occurrence is hydrogen,

In some embodiments, each R¹¹ at each occurrence is hydrogen and each R¹² at each occurrence is C₁-C₆ alkyl.

In some embodiments, each R¹¹ at each occurrence is hydrogen and each R¹² at each occurrence is methyl.

In some embodiments, each R¹¹ at each occurrence is hydrogen and each R¹² at each occurrence is ethyl.

In some embodiments, each of R¹¹ and R¹² at each occurrence is methyl.

In some embodiments, each of R¹¹ and R¹² at each occurrence is ethyl.

### The Group R¹⁵

In some embodiments, R¹⁵ is -(Z⁴-Z⁵)ₐ₂-Z⁶.

In certain embodiments, a2 is 1-5.

In certain embodiments, the Z⁴ group directly attached to R¹ or R² is -O-.

In certain embodiments, each Z⁴ is independently -O- or -NH-, provided that the Z⁴ group directly attached to R¹ or R² is -O-.

In certain embodiments, each Z⁴ is -O-.

In certain embodiments, each Z⁵ is independently C₂-C₆ alkylene optionally substituted with one or more substituents independently selected from oxo, halo, and hydroxyl. In certain these embodiments, each Z⁵ is independently C₂-C₄ (e.g., C₂-C₃ (e.g., C₂ or C₃)) alkylene. In certain embodiments, Z⁶ is OH.

In certain embodiments, Z⁶ is NHC(O)(C₁-C₆ alkoxy).

In certain embodiments, Z⁶ is C₆-C₁₀ aryl.

In certain embodiments, Z⁶ is C₁-C₆ alkoxy.

In certain embodiments of R¹⁵, a2=1; and Z⁴ is O. In certain of these embodiments, Z⁵ is C₂-C₄ (e.g., C₂-C₃ (e.g., C₂ or C₃)) alkylene. In certain of the foregoing embodiments, Z⁶ is selected from OH, NHC(O)(C1-C6 alkoxy), and C₁-C₆ alkoxy.

As non-limiting examples, R¹⁵ is selected from: 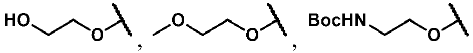 , and

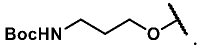

In certain embodiments of R¹⁵, a2=1; and each Z⁴ is O. In certain of these embodiments, Z⁵ is C₂-C₄ (e.g., C₂-C₃ (e.g., C₂ or C₃)) alkylene. In certain of the foregoing embodiments, Z⁶ is selected from OH, NHC(O)(C1-C6 alkoxy), and C₁-C₆ alkoxy. In certain other of the foregoing embodiments, Z⁶ is C₆-C₁₀ aryl (e.g., R¹⁵ is

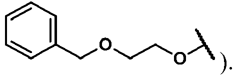

In certain embodiments of R¹⁵, a2 ≥2 (e.g., a2 is 3 or 4); each Z⁴ is O; and each Z⁵ is ethylene. In certain of these embodiments Z⁶ is OH. In certain other embodiments, Z⁶ is NHC(O)(C₁-C₆ alkoxy) (e.g., Boc). As a non-limiting example, R¹⁵ is:

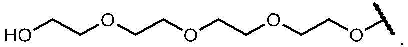

### Non-Limiting Combinations

In some embodiments, ring **A** is a 5-membered heteroaryl containing two or more heteroatoms (e.g., two) each independently selected from N, O, and S (e.g., N and S); **m is** 1; and **n** is 0 or 1. In certain of these embodiments, ring **A** is thiazolyl (e.g., thiazol-5-yl) or pyrazolyl (e.g., pyrazol-3-yl).

In certain embodiments (when ring **A** is a 5-membered heteroaryl containing two or more heteroatoms each independently selected from N, O, and S (e.g., N and S); **m is** 1; and **n** is 0 or 1), **R¹** is C₁-C₆ (e.g., C₂-C₄) alkyl optionally substituted with one or more substituents each independently selected from hydroxy, N**R⁸R⁹**, and halo (e.g., hydroxy). In certain of these embodiments, **n** is 1; and **R²** is halo (e.g., F). In certain other embodiments, **n** is 1; and **R²** is C₁-C₆ alkyl optionally substituted with one or more hydroxy.

In some embodiments, ring **A** is a 5-membered heteroaryl containing one ring sulfur atom and optionally one or more ring nitrogen atoms; **m** is 1; and **n** is 0 or 1. In certain of these embodiments ring **A** is thiophenyl or thiazolyl (e.g., thiazolyl (e.g., thiazol-5-yl)).

In certain embodiments (when ring **A** is a 5-membered heteroaryl containing one ring sulfur atom; **m** is 1; and **n** is 0 or 1), **R¹** is C₁-C₆ alkyl optionally substituted with one or more substituents each independently selected from hydroxy, N**R⁸R⁹**, and halo (e.g., hydroxy). In certain of these embodiments, **n** is 1; and **R²** is halo (e.g., F). In certain other embodiments, **n** is 1; and **R²** is C₁-C₆ alkyl optionally substituted with one or more hydroxy.

In some embodiments, ring **A** is thiazolyl (e.g., thiazol-5-yl); **m is** 1; and **n is** 0 or 1. In certain of these embodiments, **R¹** is C₁-C₆ alkyl optionally substituted with one or more hydroxy (e.g., 1, 2, or 3 (e.g., 1 or 2)). For example, **R¹** is 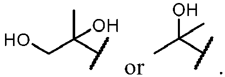 In certain of the foregoing embodiments, **n** is 1; and **R²** is C₁-C₆ alkyl optionally substituted with one or more hydroxy. In certain other embodiments, n is 0.

In some embodiments, ring **A** is pyrazolyl; **m** is 1; and **n** is 0 or 1. In certain of these embodiments, **R¹** is C₁-C₆ (e.g., C₂-C₄) alkyl optionally substituted with one or more halo (e.g., 0, 1, 2, or 3 (e.g., 0, 2 or 3)). In certain of the foregoing embodiments, **R²** is halo (e.g., F).

In some embodiments, A is thiazolyl (e.g., 2-thiazolyl or 5-thiazolyl); m is 1; n is 0 or 1; R¹ is C₁-C₆ alkyl optionally substituted with hydroxy (e.g., 2-hydroxy-2-propyl); and R², when present, is C₁-C₆ alkyl optionally substituted with hydroxyl (e.g., methyl, hydroxymethyl, or hydroxyethyl).

In some embodiments, A is pyrazolyl (e.g., 3-pyrazolyl); m is 1; n is 0; and R¹ is C₁-C₆ alkyl optionally substituted with 1-3 halo (e.g., fluoro).

In some embodiments, A is phenyl; m is 1; n is 0 or 1; and R¹ is C₁-C₆ alkyl optionally substituted with NR⁸R⁹ (e.g., dimethylamino); and R², when present, is halo (e.g., fluoro).

In some embodiments, A is thiophenyl (e.g., 2-thiophenyl); m is 1; n is 0; and R¹ is C₁-C₆ alkyl optionally substituted with hydroxyl or oxo (e.g., isopropyl, 2-hydroxy-2-propyl, or 1-propanoyl).

In some embodiments of one or more Formulae described herein, the substituted ring **B** is 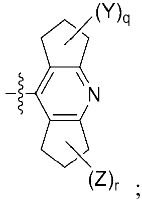 **q** is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring. In certain of these embodiments, **q** is 0; and **r is** 1 or 2.

For example, the substituted ring **B** is selected from: 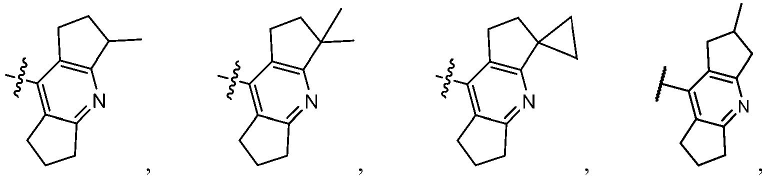 and 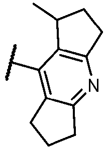 (e.g., 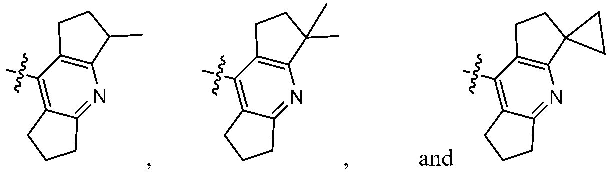 ).

In some embodiments of one or more Formulae described herein, the substituted ring **B** is 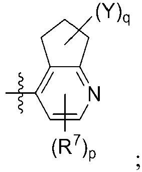 **R⁷** is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each **Y** is independently selected from C₁-C₆ alkyl (e.g., methyl) and hydroxy; or when two **Y** are attached to the same carbon, the two **Y** are taken together with the carbon they are attached to to form a cyclopropyl ring. In certain of these embodiments, **q** is 0.

In certain of the foregoing embodiments, the substituted ring B is selected from the group consisting of:

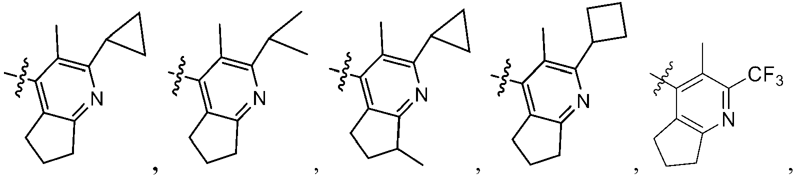

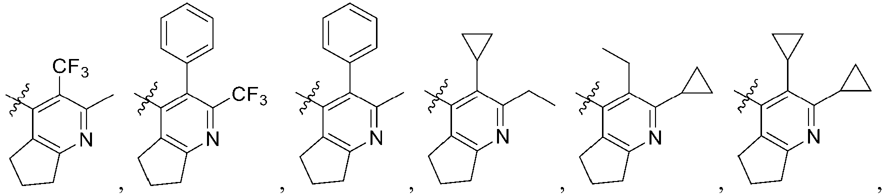

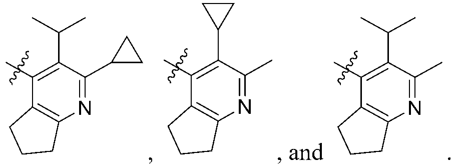

For example, the substituted ring **B** is selected from:

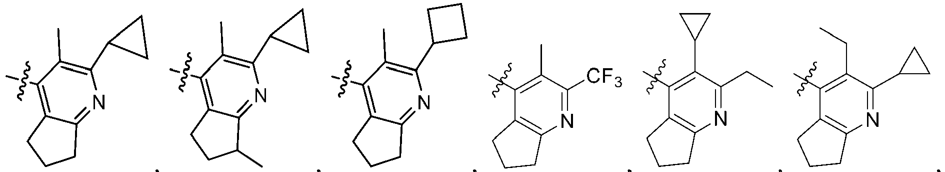

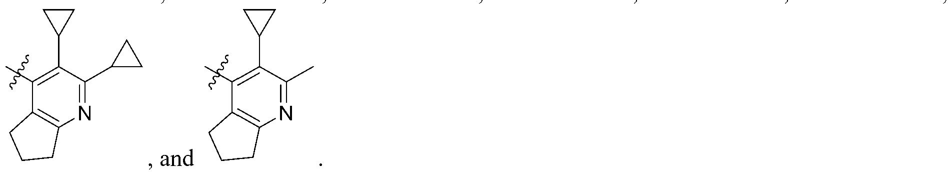

In some embodiments, the substituted ring B is 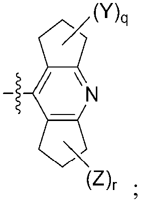 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments, the substituted ring B is 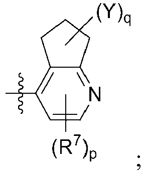 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.

In some embodiments, the substituted ring B is 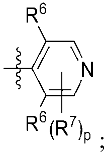 each R⁶ is independently selected from C₁-C₆ alkyl (e.g., isopropyl); each R⁷ is independently selected from halo (e.g., fluoro); p is 0 or 1.

In some embodiments, the substituted ring B is 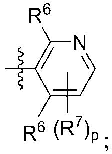 each R⁶ is independently selected from C₁-C₆ alkyl (e.g., isopropyl); each R⁷ is independently selected from halo (e.g., fluoro); p is 0 or 1.

In some embodiments, A is thiazolyl (e.g., 2-thiazolyl or 5-thiazolyl); m is 1; n is 0 or 1; R¹ is C₁-C₆ alkyl optionally substituted with hydroxy (e.g., 2-hydroxy-2-propyl); R², when present, is C₁-C₆ alkyl optionally substituted with hydroxyl (e.g., methyl, hydroxymethyl, or hydroxyethyl); the substituted ring B is 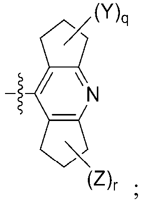 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments, A is pyrazolyl (e.g., 3-pyrazolyl); m is 1; n is 0; R¹ is C₁-C₆ alkyl optionally substituted with 1-3 halo (e.g., fluoro); the substituted ring B is 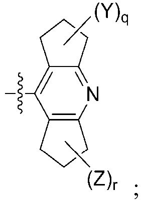 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments, A is phenyl; m is 1; n is 0 or 1; and R¹ is C₁-C₆ alkyl optionally substituted with NR⁸R⁹ (e.g., dimethylamino); R², when present, is halo (e.g., fluoro) ; the substituted ring B is 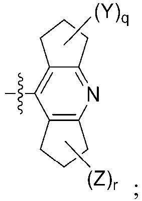 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments, the optionally substituted ring A is selected from the group consisting of a 5-membered heteroaryl comprising 2 or more heteroatoms, a 5-membered heteroaryl comprising 1 heteroatom or heteroatomic group selected from N, NH, and NR¹, and a 5-membered heteroaryl comprising 1 heteroatom selected from O and S, wherein the heteroatom is not bonded to the position of the heteroaryl that is bonded to the S(O)(NHR³)=N moiety; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is a pyrazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is an imidazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is a thiophenyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 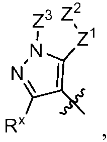 wherein R^{x} is selected from the group consisting of H and C₁-C₆ alkyl (e.g., methyl); Z¹ is selected from the group consisting of O, NH, and -CH₂- optionally substituted with 1-2 R²⁰; Z² is selected from the group consisting of NH and -CH₂- optionally substituted with 1-2 R²⁰; Z³ is selected from the group consisting of -CH₂- optionally substituted with 1-2 R²⁰, -CH₂CH₂- optionally substituted with 1-2 R²⁰, and -CH₂CH₂CH₂- optionally substituted with 1-2 R²⁰; R²⁰ is selected from the group consisting of hydroxy, halo (e.g., fluoro), oxo, C₁-C₆ alkyl (e.g., methyl or ethyl) optionally substituted with one R²¹, C₁-C₆ alkoxy (e.g., methoxy, ethoxy, or isopropoxy) optionally substituted with one R²¹, NR⁸R⁹, 3- to 10-membered heterocycloalkyl (e.g., azetidinyl or pyrrolidinyl) optionally substituted with one R²¹, or one pair of R²⁰ on the same atom, taken together with the atom connecting them, independently forms a monocyclic C₃-C₄ carbocyclic ring or a monocyclic 3- to 4-membered heterocyclic ring containing 1 O atom optionally substituted with OS(O)₂Ph; R²¹ is selected from the group consisting of halo (e.g., fluoro), NR⁸R⁹, C₂-C₆ alkynyl (e.g., ethynyl), and C₁-C₆ alkoxy (e.g., methoxy); R⁸ and R⁹ at each occurrence is independently selected from hydrogen, C₁-C₆ alkyl (e.g., methyl or ethyl), COR¹³, and CO₂R¹³; R¹³ is selected from the group consisting of: C₁-C₆ alkyl (e.g., methyl or t-butyl) and C₁-C₆ haloalkyl (e.g., trifluoromethyl).

In some embodiments, the optionally substituted ring A is 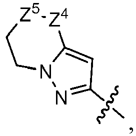 wherein Z⁴ is selected from the group consisting of -CH₂-, -C(O)-, and NH; Z⁵ is selected from the group consisting of O, NH, N-CH₃, and -CH₂-.

In some embodiments, the substituted ring B is 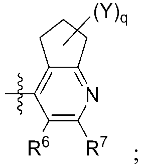 R⁶ is selected from C₁-C₆ alkyl (e.g., methyl, ethyl, or isopropyl) and C₃-C₁₀ cycloalkyl (e.g., cyclopropyl); R⁷ is selected from C₁-C₆ alkyl (e.g., methyl, ethyl, or isopropyl), C₁-C₆ haloalkyl (e.g., trifluoromethyl) and C₃-C₁₀ cycloalkyl (e.g., cyclopropyl or cyclobutyl); or R⁶ and R⁷, taken together with the atoms connecting them, independently form a C₅ carbocyclic ring optionally substituted with one or more C₁-C₆ alkyl (e.g., methyl); q is 0, 1, or 2; each Y is independently selected from C₁-C₆ alkyl (e.g., methyl); or when two Y are attached to the same carbon, the two Y are taken together with the carbon they are attached to to form a cyclopropyl ring.

In some embodiments of the compound of Formula AA-1, the substituted ring B is 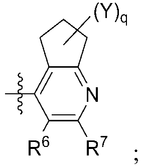 R⁶ is selected from C₁-C₆ alkyl (e.g., methyl, ethyl, or isopropyl) and C₃-C₁₀ cycloalkyl (e.g., cyclopropyl); R⁷ is selected from C₁-C₆ alkyl (e.g., methyl, ethyl, or isopropyl), C₁-C₆ haloalkyl (e.g., trifluoromethyl) and C₃-C₁₀ cycloalkyl (e.g., cyclopropyl or cyclobutyl); or R⁶ and R⁷, taken together with the atoms connecting them, independently form a C₅ carbocyclic ring optionally substituted with one or more C₁-C₆ alkyl (e.g., methyl); q is 0, 1, or 2; each Y is independently selected from C₁-C₆ alkyl (e.g., methyl); or when two Y are attached to the same carbon, the two Y are taken together with the carbon they are attached to to form a cyclopropyl ring.

In some embodiments, the optionally substituted ring A is 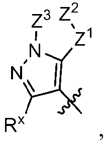 wherein R^{x} is selected from the group consisting of H and C₁-C₆ alkyl (e.g., methyl); Z¹ is selected from the group consisting of O, NH, and -CH₂- optionally substituted with 1-2 R²⁰; Z² is selected from the group consisting of NH and -CH₂- optionally substituted with 1-2 R²⁰; Z³ is selected from the group consisting of -CH₂- optionally substituted with 1-2 R²⁰, -CH₂CH₂- optionally substituted with 1-2 R²⁰, and -CH₂CH₂CH₂- optionally substituted with 1-2 R²⁰; R²⁰ is selected from the group consisting of hydroxy, halo (e.g., fluoro), oxo, C₁-C₆ alkyl (e.g., methyl or ethyl) optionally substituted with one R²¹, C₁-C₆ alkoxy (e.g., methoxy, ethoxy, or isopropoxy) optionally substituted with one R²¹, NR⁸R⁹, 3- to 10-membered heterocycloalkyl (e.g., azetidinyl or pyrrolidinyl) optionally substituted with one R²¹, or one pair of R²⁰ on the same atom, taken together with the atom connecting them, independently forms a monocyclic C₃-C₄ carbocyclic ring or a monocyclic 3- to 4-membered heterocyclic ring containing 1 O atom optionally substituted with OS(O)₂Ph; R²¹ is selected from the group consisting of halo (e.g., fluoro), NR⁸R⁹, C₂-C₆ alkynyl (e.g., ethynyl), and C₁-C₆ alkoxy (e.g., methoxy); R⁸ and R⁹ at each occurrence is independently selected from hydrogen, C₁-C₆ alkyl (e.g., methyl or ethyl), COR¹³, and CO₂R¹³; R¹³ is selected from the group consisting of: C₁-C₆ alkyl (e.g., methyl or t-butyl) and C₁-C₆ haloalkyl (e.g., trifluoromethyl); and
the substituted ring B is 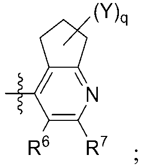 R⁶ is selected from C₁-C₆ alkyl (e.g., methyl, ethyl, or isopropyl) and C₃-C₁₀ cycloalkyl (e.g., cyclopropyl); R⁷ is selected from C₁-C₆ alkyl (e.g., methyl, ethyl, or isopropyl), C₁-C₆ haloalkyl (e.g., trifluoromethyl) and C₃-C₁₀ cycloalkyl (e.g., cyclopropyl or cyclobutyl); or R⁶ and R⁷, taken together with the atoms connecting them, independently form a C₅ carbocyclic ring optionally substituted with one or more C₁-C₆ alkyl (e.g., methyl); q is 0, 1, or 2; each Y is independently selected from C₁-C₆ alkyl (e.g., methyl); or when two Y are attached to the same carbon, the two Y are taken together with the carbon they are attached to to form a cyclopropyl ring.

In some embodiments, the optionally substituted ring A is selected from the group consisting of: 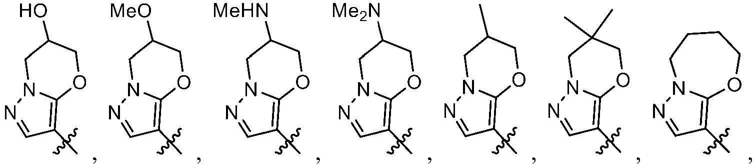 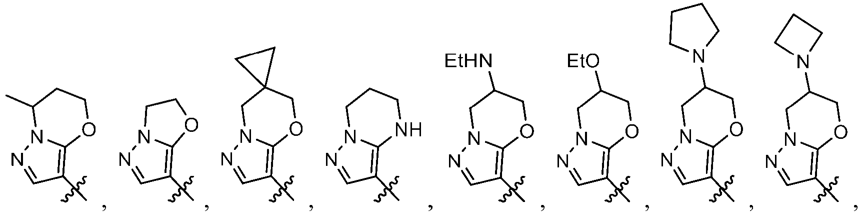 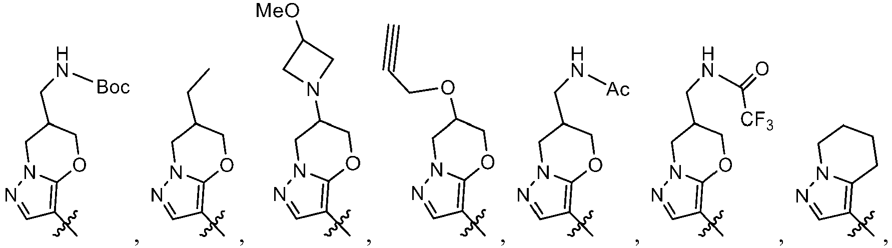 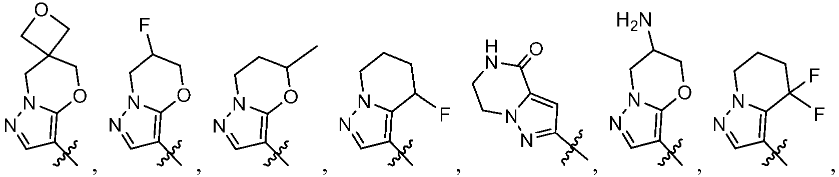 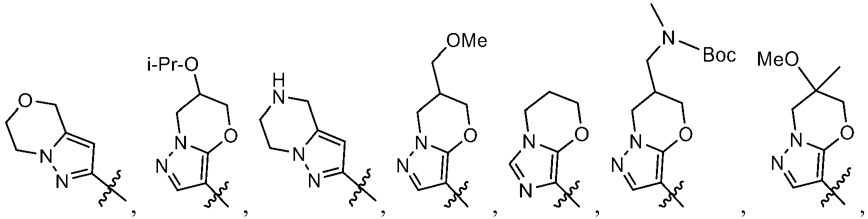 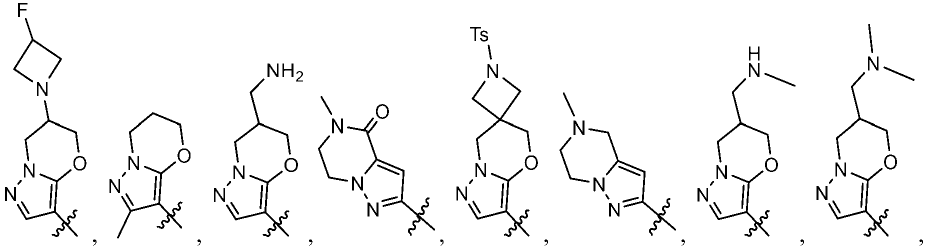 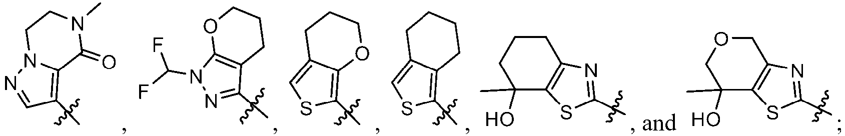 and
the substituted ring B is selected from the group consisting of:

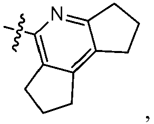

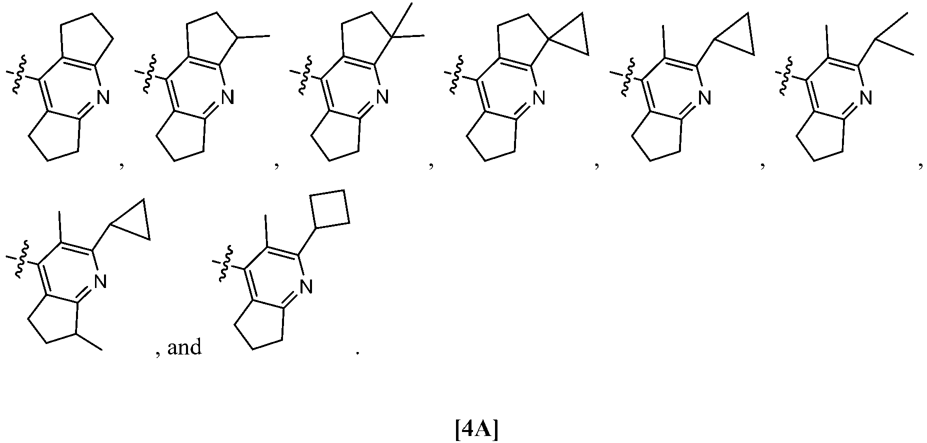

In some embodiments, ring **A** is a 5-membered heteroaryl containing two or more heteroatoms each independently selected from N, O, and S (e.g., N and S (e.g., ring **A** is pyrazolyl (e.g., pyrazol-3-yl) or thiazolyl (e.g., thiazol-5-yl));
**m is** 1; **n** is 0 or 1;
**R¹** is C₁-C₆ (e.g., C₂-C₄) alkyl optionally substituted with one or more substituents each independently selected from hydroxy and halo;
**R²** is halo or C₁-C₆ alkyl optionally substituted with one or more hydroxy;
the substituted ring B is 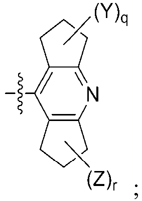 **q** is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In certain embodiments of **[4A],** ring **A** is thiazolyl (e.g., thiazol-5-yl). In certain of these embodiments, **R¹** is C₁-C₆ (e.g., C₂-C₄) alkyl optionally substituted with one or more hydroxy (e.g., 1, 2, or 3 (e.g., 1 or 2)). For example, **R¹** is 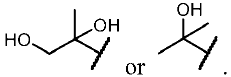 In certain of the foregoing embodiments, **n** is 1; and **R²** is C₁-C₆ alkyl optionally substituted with one or more hydroxy. In certain other embodiments, **n** is 0.

In certain embodiments of **[4A],** ring **A** is pyrazolyl. In certain of these embodiments, **R¹** is C₁-C₆ alkyl optionally substituted with one or more halo (e.g., 0, 1, 2, or 3 (e.g., 0, 2 or 3)). In certain of the foregoing embodiments, **R²** is halo (e.g., F).

In certain embodiments of **[4A], q** is 0; and r is 1 or 2.

For example, the substituted ring **B** is selected from:

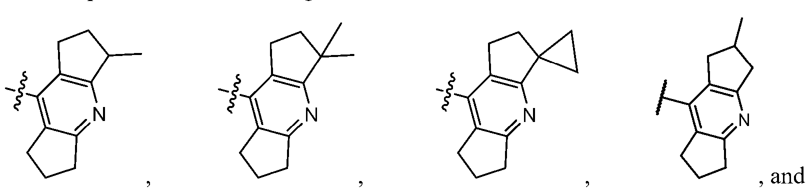

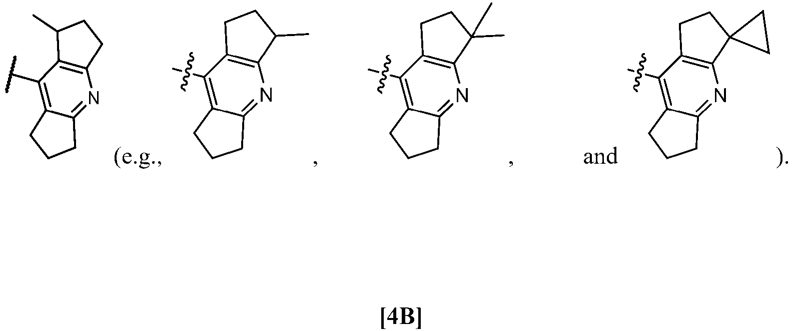

In some embodiments, ring **A** is a 5-membered heteroaryl containing two or more (e.g., two) heteroatoms each independently selected from N, O, and S (e.g., N and S (e.g., ring **A** is pyrazolyl (e.g., pyrazol-3-yl) or thiazolyl (e.g., thiazol-5-yl));
**m is** 1; **n** is 0 or 1;
**R¹** is C₁-C₆ (e.g., C₂-C₄) alkyl optionally substituted with one or more substituents each independently selected from hydroxy and halo;
**R²** is halo or C₁-C₆ alkyl optionally substituted with one or more hydroxy; and
the substituted ring **B** is 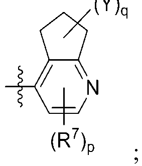 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.

In certain embodiments of **[4B],** ring **A** is thiazolyl (e.g., thiazol-5-yl). In certain of these embodiments, **R¹** is C₁-C₆ (e.g., C₂-C₄) alkyl optionally substituted with one or more hydroxy (e.g., 1, 2, or 3 (e.g., 1 or 2)). For example, **R¹** is 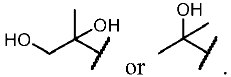 In certain of the foregoing embodiments, **n** is 1; and **R²** is C₁-C₆ alkyl optionally substituted with one or more hydroxy. In certain other embodiments, **n** is 0.

In certain embodiments of **[4B],** ring A is pyrazolyl. In certain of these embodiments, **R¹** is C₁-C₆ alkyl optionally substituted with one or more halo (e.g., 0, 1, 2, or 3 (e.g., 0, 2, or 3)). In certain of the foregoing embodiments, **R²** is halo (e.g., F).

In certain of embodiments of **[4B], p is** 1 or 2.

In certain of these embodiments, **p** is 2.

In certain other embodiments, **p** is 1; and **R⁷** is selected from C₁-C₆ alkyl (e.g., methyl, ethyl, or isopropyl), and C₃-C₁₀ cycloalkyl (e.g., cyclopropyl)

In certain embodiments of **[4B], q** is 0.

In certain embodimens of **[4B],** the substituted ring B is selected from the group consisting of:

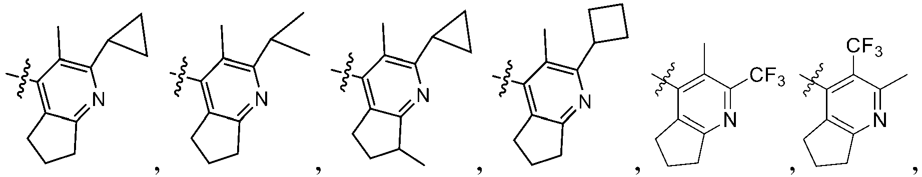

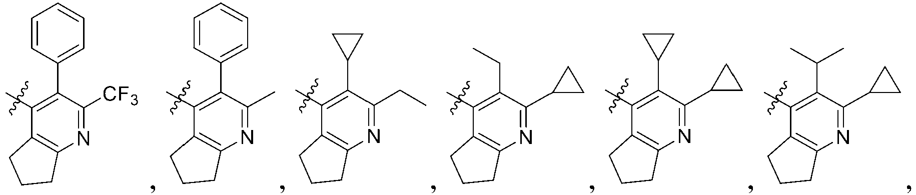

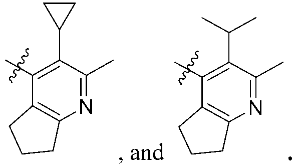

For example, the substituted ring **B** is selected from:

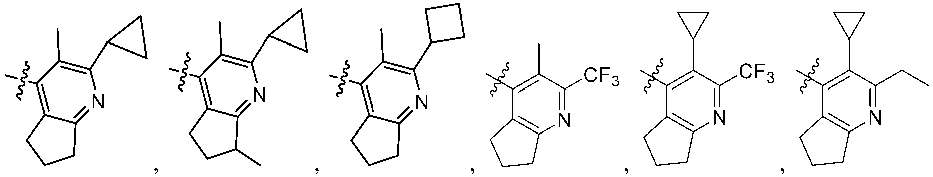

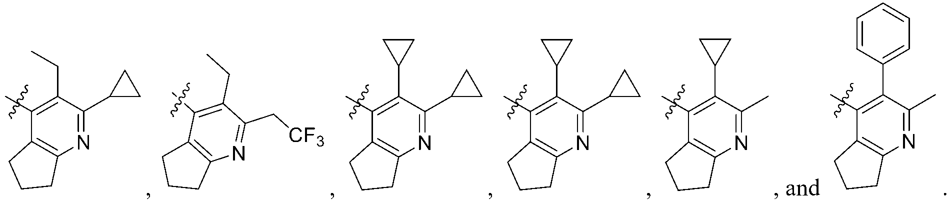

In some embodiments of Formula AA-1, ring **A'** is a 5-membered heteroaryl containing two or more heteroatoms (e.g., two) each independently selected from N, O, and S (e.g., N and S); **m is** 1; and **n** is 0 or 1. In certain of these embodiments, ring **A'** is thiazolyl (e.g., thiazol-5-yl) or pyrazolyl (e.g., pyrazol-3-yl).

In certain embodiments (when ring A' is a 5-membered heteroaryl containing two or more heteroatoms each independently selected from N, O, and S (e.g., N and S); **m is** 1; and **n** is 0 or 1), **R¹** is C₁-C₆ (e.g., C₂-C₄) alkyl optionally substituted with one or more substituents each independently selected from hydroxy, **NR⁸R⁹,** and halo (e.g., hydroxy). In certain of these embodiments, **n** is 1; and **R²** is halo (e.g., F). In certain other embodiments, **n** is 1; and **R²** is C₁-C₆ alkyl optionally substituted with one or more hydroxy.

In some embodiments of Formula AA-1, ring **A'** is a 5-membered heteroaryl containing one ring sulfur atom and optionally one or more ring nitrogen atoms; **m is** 1; and **n is** 0 or 1. In certain of these embodiments ring **A'** is thiophenyl or thiazolyl (e.g., thiazolyl (e.g., thiazol-5-yl)).

In certain embodiments (when ring **A'** is a 5-membered heteroaryl containing one ring sulfur atom; **m** is 1; and **n** is 0 or 1), **R¹** is C₁-C₆ alkyl optionally substituted with one or more substituents each independently selected from hydroxy, **NR⁸R⁹,** and halo (e.g., hydroxy). In certain of these embodiments, **n** is 1; and **R²** is halo (e.g., F). In certain other embodiments, **n** is 1; and **R²** is C₁-C₆ alkyl optionally substituted with one or more hydroxy.

In some embodiments, ring **A'** is thiazolyl (e.g., thiazol-5-yl); **m is** 1; and **n is** 0 or 1. In certain of these embodiments, **R¹** is C₁-C₆ alkyl optionally substituted with one or more hydroxy (e.g., 1, 2, or 3 (e.g., 1 or 2)). For example, **R¹** is 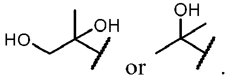 In certain of the foregoing embodiments, n is 1; and **R²** is C₁-C₆ alkyl optionally substituted with one or more hydroxy. In certain other embodiments, **n** is 0.

In some embodiments, ring **A'** is pyrazolyl; **m** is 1; and **n** is 0 or 1. In certain of these embodiments, **R¹** is C₁-C₆ (e.g., C₂-C₄) alkyl optionally substituted with one or more halo (e.g., 0, 1, 2, or 3 (e.g., 0, 2 or 3)). In certain of the foregoing embodiments, **R²** is halo (e.g., F).

In some embodiments of the compound of Formula AA-1, A' is thiazolyl (e.g., 2-thiazolyl or 5-thiazolyl); m is 1; n is 0 or 1; R¹ is C₁-C₆ alkyl optionally substituted with hydroxy (e.g., 2-hydroxy-2-propyl); and R², when present, is C₁-C₆ alkyl optionally substituted with hydroxyl (e.g., methyl, hydroxymethyl, or hydroxyethyl).

In some embodiments of the compound of Formula AA-1, A' is pyrazolyl (e.g., 3-pyrazolyl); m is 1; n is 0; and R¹ is C₁-C₆ alkyl optionally substituted with 1-3 halo (e.g., fluoro).

In some embodiments of the compound of Formula AA-1, A' is phenyl; m is 1; n is 0 or 1; and R¹ is C₁-C₆ alkyl optionally substituted with NR⁸R⁹ (e.g., dimethylamino); and R², when present, is halo (e.g., fluoro).

In some embodiments of the compound of Formula AA-1, the optionally substituted ring A' is selected from the group consisting of a 5-membered heteroaryl comprising 2 or more heteroatoms, a 5-membered heteroaryl comprising 1 heteroatom or heteroatomic group selected from N, NH, and NR¹, and a 5-membered heteroaryl comprising 1 heteroatom selected from O and S, wherein the heteroatom is not bonded to the position of the heteroaryl that is bonded to the S(O)(NHR³)=N moiety; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA-1, the optionally substituted ring A' is a pyrazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA-1, the optionally substituted ring A' is an imidazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA-1, the optionally substituted ring A' is 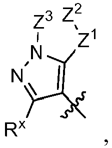 wherein R^{x} is selected from the group consisting of H and C₁-C₆ alkyl (e.g., methyl); Z¹ is selected from the group consisting of O, NH, and -CH₂- optionally substituted with 1-2 R²⁰; Z² is selected from the group consisting of NH and -CH₂- optionally substituted with 1-2 R²⁰; Z³ is selected from the group consisting of -CH₂- optionally substituted with 1-2 R²⁰, -CH₂CH₂- optionally substituted with 1-2 R²⁰, and -CH₂CH₂CH₂- optionally substituted with 1-2 R²⁰; R²⁰ is selected from the group consisting of hydroxy, halo (e.g., fluoro), oxo, C₁-C₆ alkyl (e.g., methyl or ethyl) optionally substituted with one R²¹, C₁-C₆ alkoxy (e.g., methoxy, ethoxy, or isopropoxy) optionally substituted with one R²¹, NR⁸R⁹, 3- to 10-membered heterocycloalkyl (e.g., azetidinyl or pyrrolidinyl) optionally substituted with one R²¹, or one pair of R²⁰ on the same atom, taken together with the atom connecting them, independently forms a monocyclic C₃-C₄ carbocyclic ring or a monocyclic 3- to 4-membered heterocyclic ring containing 1 O atom optionally substituted with OS(O)₂Ph; R²¹ is selected from the group consisting of halo (e.g., fluoro), NR⁸R⁹, C₂-C₆ alkynyl (e.g., ethynyl), and C₁-C₆ alkoxy (e.g., methoxy); R⁸ and R⁹ at each occurrence is independently selected from hydrogen, C₁-C₆ alkyl (e.g., methyl or ethyl), COR¹³, and CO₂R¹³; R¹³ is selected from the group consisting of: C₁-C₆ alkyl (e.g., methyl or *t-*butyl) and C₁-C₆ haloalkyl (e.g., trifluoromethyl).

In some embodiments of the compound of Formula AA-1, the optionally substituted ring A' is 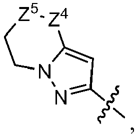 wherein Z⁴ is selected from the group consisting of -CH₂-, -C(O)-, and NH; Z⁵ is selected from the group consisting of O, NH, N-CH₃, and -CH₂-.

In some embodiments of the compound of Formula AA-1, the substituted ring B is 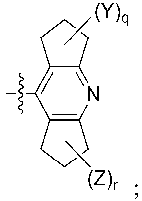 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments of the compound of Formula AA-1, the substituted ring B is 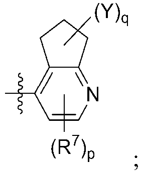 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.

In some embodiments of the compound of Formula AA-1, the substituted ring B is 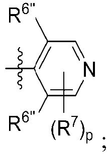 each R^{6"} is independently selected from C₁-C₆ alkyl (e.g., isopropyl); each R⁷ is independently selected from halo (e.g., fluoro); p is 0 or 1.

In some embodiments of the compound of Formula AA-1, the substituted ring B is 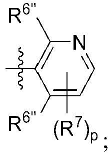 each R^{6"} is independently selected from C₁-C₆ alkyl (e.g., isopropyl); each R⁷ is independently selected from halo (e.g., fluoro); p is 0 or 1.

In some embodiments of the compound of Formula AA-1, A' is thiazolyl (e.g., 2-thiazolyl or 5-thiazolyl); m is 1; n is 0 or 1; R¹ is C₁-C₆ alkyl optionally substituted with hydroxy (e.g., 2-hydroxy-2-propyl); R², when present, is C₁-C₆ alkyl optionally substituted with hydroxyl (e.g., methyl, hydroxymethyl, or hydroxyethyl); the substituted ring B is 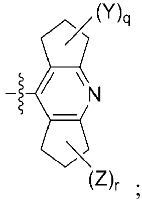 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments of the compound of Formula AA-1, A' is pyrazolyl (e.g., 3-pyrazolyl); m is 1; n is 0; R¹ is C₁-C₆ alkyl optionally substituted with 1-3 halo (e.g., fluoro) ; the substituted ring B is 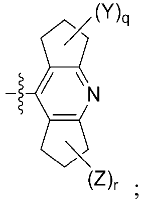 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments of the compound of Formula AA-1, A' is phenyl; m is 1; n is 0 or 1; R¹ is C₁-C₆ alkyl optionally substituted with NR⁸R⁹ (e.g., dimethylamino); and R², when present, is halo (e.g., fluoro) ; the substituted ring B is 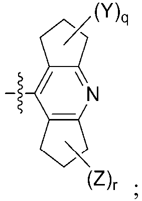 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments of the compound of Formula AA-1, the optionally substituted ring A' is selected from the group consisting of a 5-membered heteroaryl comprising 2 or more heteroatoms, a 5-membered heteroaryl comprising 1 heteroatom or heteroatomic group selected from N, NH, and NR¹, and a 5-membered heteroaryl comprising 1 heteroatom selected from O and S, wherein the heteroatom is not bonded to the position of the heteroaryl that is bonded to the S(O)(NHR³)=N moiety; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA-1, the optionally substituted ring A' is a pyrazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²),wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA-1, the optionally substituted ring A' is an imidazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/or heteroatomic groups independently selected from O, NH, NR¹³, S, S(O), and S(O)₂ (in addition to the aforementioned nitrogen atom(s) attached to R¹ and/or R²), wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA-1, the optionally substituted ring A' is a thiophenyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA-1, the optionally substituted ring A' is 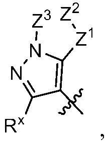 wherein R^{x} is selected from the group consisting of H and C₁-C₆ alkyl (e.g., methyl); Z¹ is selected from the group consisting of O, NH, and -CH₂- optionally substituted with 1-2 R²⁰; Z² is selected from the group consisting of NH and -CH₂- optionally substituted with 1-2 R²⁰; Z³ is selected from the group consisting of -CH₂- optionally substituted with 1-2 R²⁰, -CH₂CH₂- optionally substituted with 1-2 R²⁰, and -CH₂CH₂CH₂- optionally substituted with 1-2 R²⁰; R²⁰ is selected from the group consisting of hydroxy, halo (e.g., fluoro), oxo, C₁-C₆ alkyl (e.g., methyl or ethyl) optionally substituted with one R²¹, C₁-C₆ alkoxy (e.g., methoxy, ethoxy, or isopropoxy) optionally substituted with one R²¹, NR⁸R⁹, 3- to 10-membered heterocycloalkyl (e.g., azetidinyl or pyrrolidinyl) optionally substituted with one R²¹, or one pair of R²⁰ on the same atom, taken together with the atom connecting them, independently forms a monocyclic C₃-C₄ carbocyclic ring or a monocyclic 3- to 4-membered heterocyclic ring containing 1 O atom optionally substituted with OS(O)₂Ph; R²¹ is selected from the group consisting of halo (e.g., fluoro), NR⁸R⁹, C₂-C₆ alkynyl (e.g., ethynyl), and C₁-C₆ alkoxy (e.g., methoxy); R⁸ and R⁹ at each occurrence is independently selected from hydrogen, C₁-C₆ alkyl (e.g., methyl or ethyl), COR¹³, and CO₂R¹³; R¹³ is selected from the group consisting of: C₁-C₆ alkyl (e.g., methyl or *t-*butyl) and C₁-C₆ haloalkyl (e.g., trifluoromethyl).

In some embodiments of the compound of Formula AA-1, the optionally substituted ring A' is 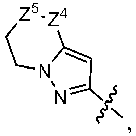 wherein Z⁴ is selected from the group consisting of -CH₂-, -C(O)-, and NH; Z⁵ is selected from the group consisting of O, NH, N-CH₃, and -CH₂-.

In some embodiments of the compound of Formula AA-1, the substituted ring B is 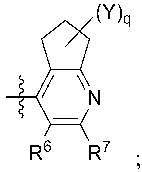 R⁶ is selected from C₁-C₆ alkyl (e.g., methyl, ethyl, or isopropyl) and C₃-C₁₀ cycloalkyl (e.g., cyclopropyl); R⁷ is selected from C₁-C₆ alkyl (e.g., methyl, ethyl, or isopropyl), C₁-C₆ haloalkyl (e.g., trifluoromethyl) and C₃-C₁₀ cycloalkyl (e.g., cyclopropyl or cyclobutyl); or R⁶ and R⁷, taken together with the atoms connecting them, independently form a C₅ carbocyclic ring optionally substituted with one or more C₁-C₆ alkyl (e.g., methyl); q is 0, 1, or 2; each Y is independently selected from C₁-C₆ alkyl (e.g., methyl); or when two Y are attached to the same carbon, the two Y are taken together with the carbon they are attached to to form a cyclopropyl ring.

In some embodiments of the compound of Formula AA-1, the optionally substituted ring A' is 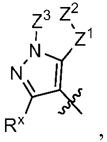 wherein R^{x} is selected from the group consisting of H and C₁-C₆ alkyl (e.g., methyl); Z¹ is selected from the group consisting of O, NH, and -CH₂- optionally substituted with 1-2 R²⁰; Z² is selected from the group consisting of NH and -CH₂- optionally substituted with 1-2 R²⁰; Z³ is selected from the group consisting of -CH₂- optionally substituted with 1-2 R²⁰, -CH₂CH₂- optionally substituted with 1-2 R²⁰, and -CH₂CH₂CH₂- optionally substituted with 1-2 R²⁰; R²⁰ is selected from the group consisting of hydroxy, halo (e.g., fluoro), oxo, C₁-C₆ alkyl (e.g., methyl or ethyl) optionally substituted with one R²¹, C₁-C₆ alkoxy (e.g., methoxy, ethoxy, or isopropoxy) optionally substituted with one R²¹, NR⁸R⁹, 3- to 10-membered heterocycloalkyl (e.g., azetidinyl or pyrrolidinyl) optionally substituted with one R²¹, or one pair of R²⁰ on the same atom, taken together with the atom connecting them, independently forms a monocyclic C₃-C₄ carbocyclic ring or a monocyclic 3- to 4-membered heterocyclic ring containing 1 O atom optionally substituted with OS(O)₂Ph; R²¹ is selected from the group consisting of halo (e.g., fluoro), NR⁸R⁹, C₂-C₆ alkynyl (e.g., ethynyl), and C₁-C₆ alkoxy (e.g., methoxy); R⁸ and R⁹ at each occurrence is independently selected from hydrogen, C₁-C₆ alkyl (e.g., methyl or ethyl), COR¹³, and CO₂R¹³; R¹³ is selected from the group consisting of: C₁-C₆ alkyl (e.g., methyl or *t-*butyl) and C₁-C₆ haloalkyl (e.g., trifluoromethyl); and
the substituted ring B is 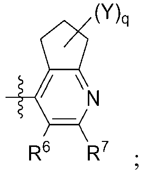 R⁶ is selected from C₁-C₆ alkyl (e.g., methyl); R⁷ is selected from C₁-C₆ alkyl (e.g., isopropyl) and C₃-C₁₀ cycloalkyl (e.g., cyclopropyl or cyclobutyl); or R⁶ and R⁷, taken together with the atoms connecting them, independently form a C₅ carbocyclic ring optionally substituted with one or more C₁-C₆ alkyl (e.g., methyl); q is 0, 1, or 2; each Y is independently selected from C₁-C₆ alkyl (e.g., methyl); or when two Y are attached to the same carbon, the two Y are taken together with the carbon they are attached to to form a cyclopropyl ring.

In some embodiments of the compound of Formula AA-1, the optionally substituted ring A' is selected from the group consisting of: 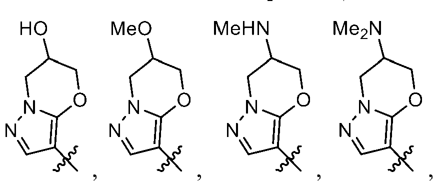 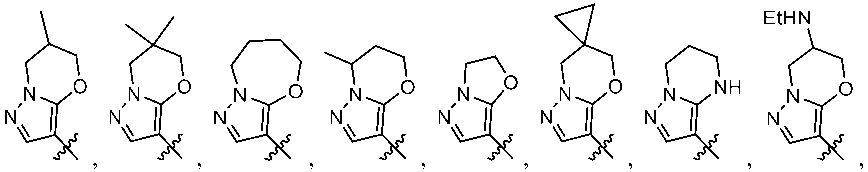 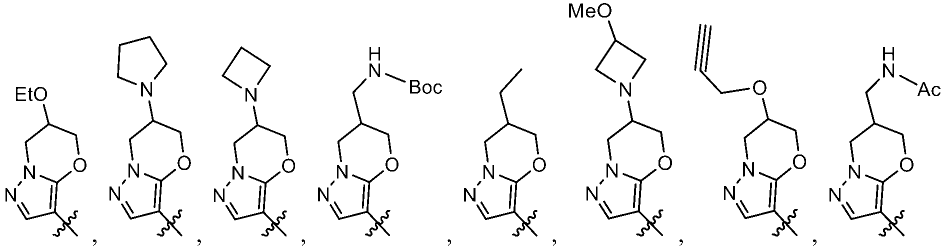 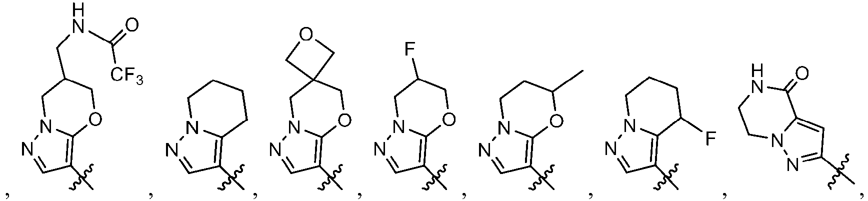 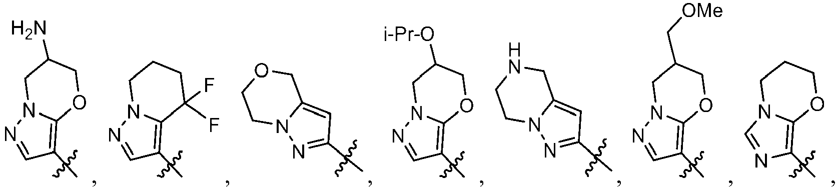 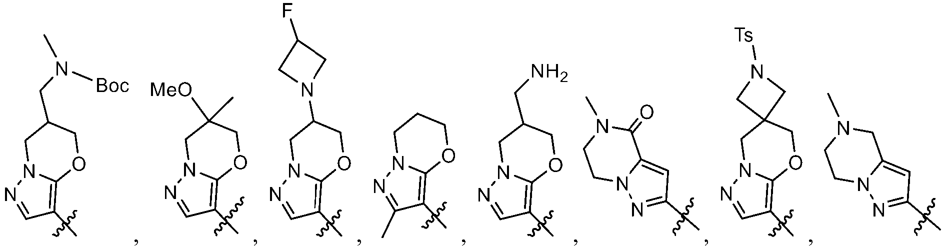 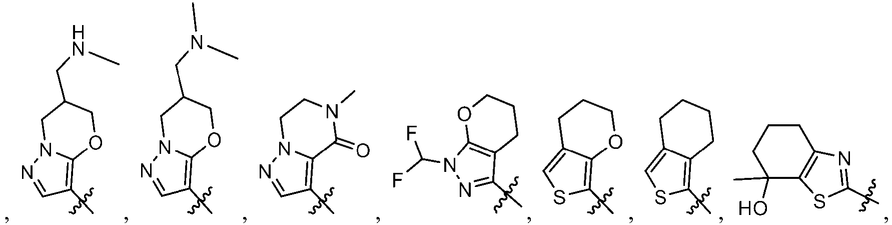 and 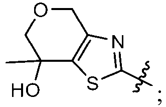 and
the substituted ring B is selected from the group consisting of:

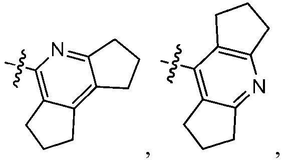

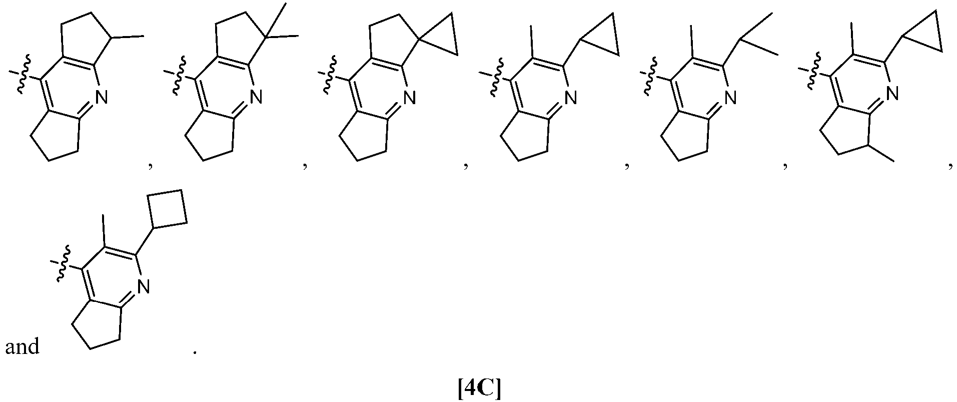

In some embodiments, ring **A'** is a 5-membered heteroaryl containing two or more heteroatoms each independently selected from N, O, and S (e.g., N and S (e.g., ring **A'** is pyrazolyl (e.g., pyrazol-3-yl) or thiazolyl (e.g., thiazol-5-yl));
**m** is 1; n is 0 or 1;
**R¹** is C₁-C₆ (e.g., C₂-C₄) alkyl optionally substituted with one or more substituents each independently selected from hydroxy and halo;
**R²** is halo or C₁-C₆ alkyl optionally substituted with one or more hydroxy;
the substituted ring **B** is 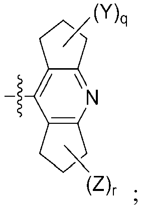 **q** is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In certain embodiments of **[4C],** ring **A'** is thiazolyl (e.g., thiazol-5-yl). In certain of these embodiments, **R¹** is C₁-C₆ (e.g., C₂-C₄) alkyl optionally substituted with one or more hydroxy (e.g., 1, 2, or 3 (e.g., 1 or 2)). For example, **R¹** is 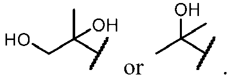 In certain of the foregoing embodiments, **n** is 1; and **R²** is C₁-C₆ alkyl optionally substituted with one or more hydroxy. In certain other embodiments, **n** is 0.

In certain embodiments of **[4C],** ring **A'** is pyrazolyl. In certain of these embodiments, **R¹** is C₁-C₆ alkyl optionally substituted with one or more halo (e.g., 0, 1, 2, or 3 (e.g., 0, 2 or 3)). In certain of the foregoing embodiments, **R²** is halo (e.g., F).

In certain embodiments of **[4C],** q is 0; and **r** is 1 or 2.

For example, the substituted ring **B** is selected from:

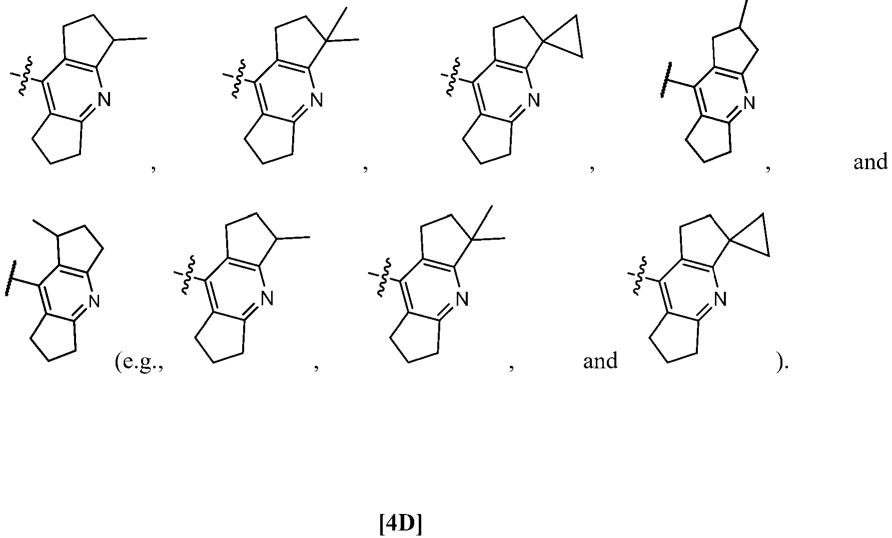

In some embodiments, ring **A'** is a 5-membered heteroaryl containing two or more (e.g., two) heteroatoms each independently selected from N, O, and S (e.g., N and S (e.g., ring **A'** is pyrazolyl (e.g., pyrazol-3-yl) or thiazolyl (e.g., thiazol-5-yl));
**m** is 1; **n** is 0 or 1;
**R¹** is C₁-C₆ (e.g., C₂-C₄) alkyl optionally substituted with one or more substituents each independently selected from hydroxy and halo;
**R²** is halo or C₁-C₆ alkyl optionally substituted with one or more hydroxy; and
the substituted ring **B** is 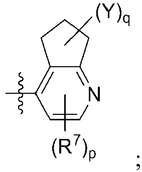 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.

In certain embodiments of **[4D],** ring **A'** is thiazolyl (e.g., thiazol-5-yl). In certain of these embodiments, **R¹** is C₁-C₆ (e.g., C₂-C₄) alkyl optionally substituted with one or more hydroxy (e.g., 1, 2, or 3 (e.g., 1 or 2)). For example, **R¹** is 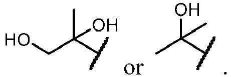 In certain of the foregoing embodiments, **n** is 1; and **R²** is C₁-C₆ alkyl optionally substituted with one or more hydroxy. In certain other embodiments, **n** is 0.

In certain embodiments of **[4D],** ring **A'** is pyrazolyl. In certain of these embodiments, **R¹** is C₁-C₆ alkyl optionally substituted with one or more halo (e.g., 0, 1, 2, or 3 (e.g., 0, 2, or 3)). In certain of the foregoing embodiments, **R²** is halo (e.g., F).

In certain embodiments of **[4D], p is** 1 or 2.

In certain of these embodiments, **p** is 2.

In certain other embodiments, **p** is 1; and **R⁷** is selected from C₁-C₆ alkyl (e.g., methyl, ethyl, or isopropyl), and C₃-C₁₀ cycloalkyl (e.g., cyclopropyl)

In certain embodiments of **[4D], q** is 0.

In certain embodimens of **[4D],** the substituted ring B is selected from the group consisting of:

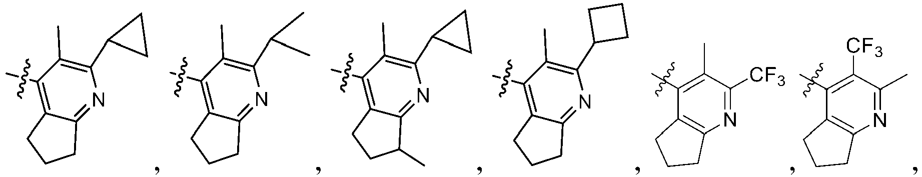

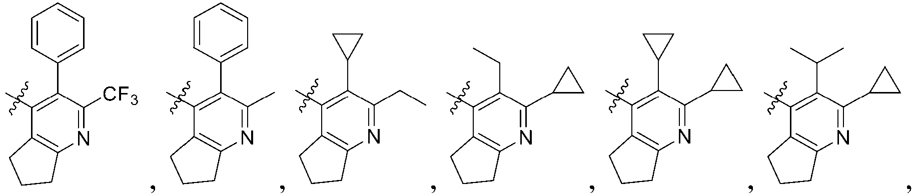

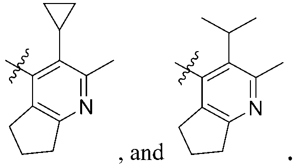

For example, the substituted ring **B** is selected from:

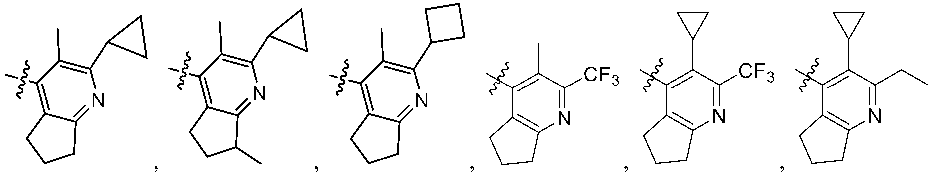

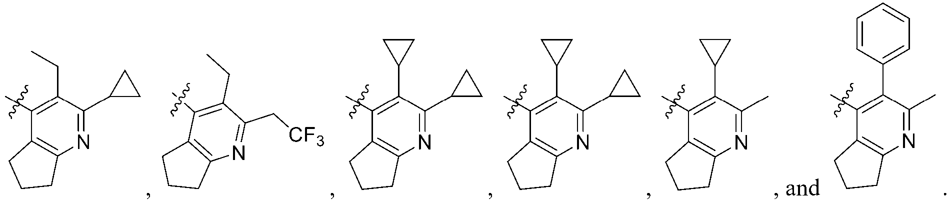

In some embodiments of the compound of Formula AA-2, A'' is thiophenyl (e.g., 2-thiophenyl); m' is 1; n' is 0; and R¹ is C₁-C₆ alkyl optionally substituted with hydroxyl or oxo (e.g., isopropyl, 2-hydroxy-2-propyl, or 1-propanoyl).

In some embodiments of the compound of Formula AA-2, the substituted ring B is 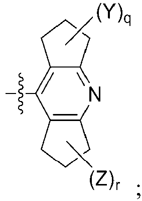 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments of the compound of Formula AA-2, the substituted ring B is 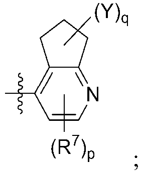 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.

In some embodiments of the compound of Formula AA-2, the substituted ring B is 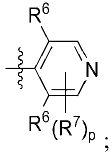 each R⁶ is independently selected from C₁-C₆ alkyl (e.g., isopropyl); each R⁷ is independently selected from halo (e.g., fluoro); p is 0 or 1.

In some embodiments of the compound of Formula AA-2, the substituted ring B is 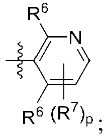 each R⁶ is independently selected from C₁-C₆ alkyl (e.g., isopropyl); each R⁷ is independently selected from halo (e.g., fluoro); p is 0 or 1.

In some embodiments of the compound of Formula AA-2, A" is thiophenyl (e.g., 2-thiophenyl); m' is 1; n' is 0; R¹ is C₁-C₆ alkyl optionally substituted with hydroxyl or oxo (e.g., isopropyl, 2-hydroxy-2-propyl, or 1-propanoyl); the substituted ring B is 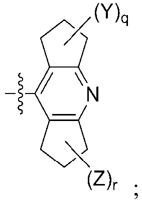 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments of the compound of Formula AA-3, A" is thiophenyl (e.g., 2-thiophenyl); m" is 1; n" is 1; R^{1'} is C₂-C₆ alkyl optionally substituted with hydroxyl or oxo (e.g., 2-hydroxy-2-propyl); and R^{2'} is C₂-C₆ alkyl optionally substituted with hydroxyl or oxo (e.g., 2-hydroxy-2-propyl).

In some embodiments of the compound of Formula AA-3, the substituted ring B is 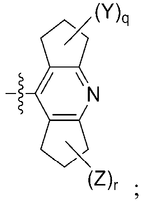 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments of the compound of Formula AA-3, the substituted ring B is 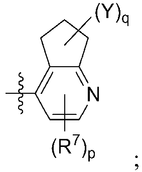 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.

In some embodiments of the compound of Formula AA-3, the substituted ring B is 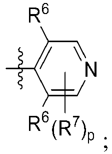 each R⁶ is independently selected from C₁-C₆ alkyl (e.g., isopropyl); each R⁷ is independently selected from halo (e.g., fluoro); p is 0 or 1.

In some embodiments of the compound of Formula AA-3, the substituted ring B is 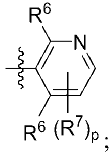 each R⁶ is independently selected from C₁-C₆ alkyl (e.g., isopropyl); each R⁷ is independently selected from halo (e.g., fluoro); p is 0 or 1.

In some embodiments of the compound of Formula AA-3, A" is thiophenyl (e.g., 2-thiophenyl); m" is 1; n" is 1; R^{1'} is C₂-C₆ alkyl optionally substituted with hydroxyl or oxo (e.g., 2-hydroxy-2-propyl); R^{2'} is C₂-C₆ alkyl optionally substituted with hydroxyl or oxo (e.g., 2-hydroxy-2-propyl); the substituted ring B is 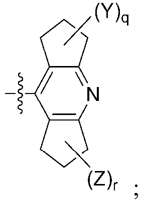 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments of the compound of Formula AA-3, the optionally substituted ring A" is a thiophenyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA-3, the substituted ring B is 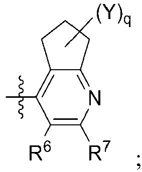 R⁶ is selected from C₁-C₆ alkyl (e.g., methyl); R⁷ is selected from C₁-C₆ alkyl (e.g., isopropyl) and C₃-C₁₀ cycloalkyl (e.g., cyclopropyl or cyclobutyl); or R⁶ and R⁷, taken together with the atoms connecting them, independently form a C₅ carbocyclic ring optionally substituted with one or more C₁-C₆ alkyl (e.g., methyl); q is 0, 1, or 2; each Y is independently selected from C₁-C₆ alkyl (e.g., methyl); or when two Y are attached to the same carbon, the two Y are taken together with the carbon they are attached to to form a cyclopropyl ring.

In some embodiments, the optionally substituted ring A" is selected from the group consisting of: 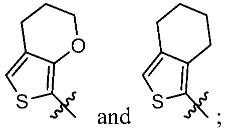 and
the substituted ring B is selected from the group consisting of:

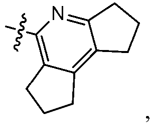

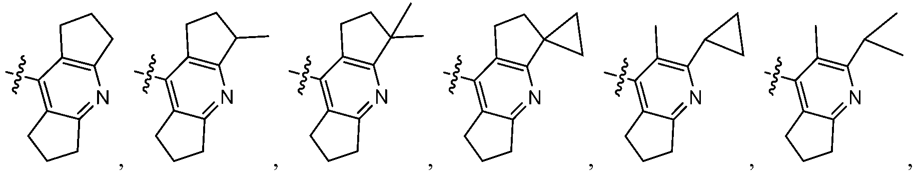

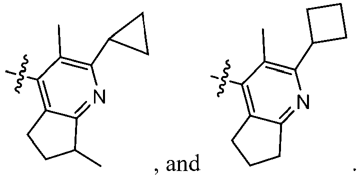

In some embodiments of the compound of Formula AA-4, A" is thiophenyl (e.g., 2-thiophenyl); R¹ is C₁-C₆ alkyl optionally substituted with hydroxyl or oxo (e.g., methyl or 2-hydroxy-2-propyl); and R^{2"} is methyl.

In some embodiments of the compound of Formula AA-4, the substituted ring B' is 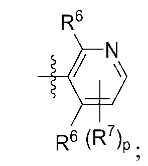 each R⁶ is independently selected from C₁-C₆ alkyl (e.g., isopropyl); each R⁷ is independently selected from halo (e.g., fluoro); p is 0 or 1.

In some embodiments of the compound of Formula AA-4, A" is thiophenyl (e.g., 2-thiophenyl); R¹ is C₁-C₆ alkyl optionally substituted with hydroxyl or oxo (e.g., methyl or 2-hydroxy-2-propyl); R^{2"} is methyl; the substituted ring B' is 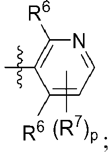 each R⁶ is independently selected from C₁-C₆ alkyl (e.g., isopropyl); each R⁷ is independently selected from halo (e.g., fluoro); p is 0 or 1.

In some embodiments of the compound of Formula AA-5, the substituted ring B" is 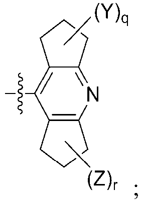 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.

In some embodiments of the compound of Formula AA-5, the substituted ring B" is 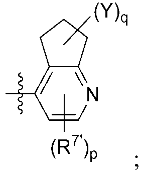 R^{7'} is selected from unbranched C₁-C₆ alkyl (e.g., methyl or ethyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.

In some embodiments, the optionally substituted ring A is 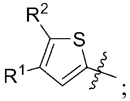 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, -NR¹⁰, COOC₁-C₆ alkyl, C₆-C₁₀ aryl, and CONR⁸R⁹.

In some embodiments, the optionally substituted ring A is 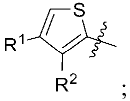 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 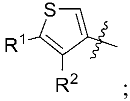 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, -NR¹⁰, COOC₁-C₆ alkyl, C₆-C₁₀ aryl, and CONR⁸R⁹.

In some embodiments, the optionally substituted ring A is 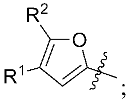 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 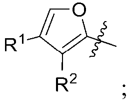 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, -NR¹⁰, COOC₁-C₆ alkyl, C₆-C₁₀ aryl, and CONR⁸R⁹.

In some embodiments, the optionally substituted ring A is 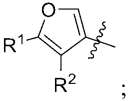 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 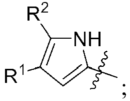 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, -NR¹⁰, COOC₁-C₆ alkyl, C₆-C₁₀ aryl, and CONR⁸R⁹.

In some embodiments, the optionally substituted ring A is 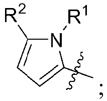 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 0-2 heteroatoms and/heteroatomic groups independently selected from O, NH, NR¹³, S, S(O), and S(O)₂ (in addition to the nitrogen atom attached to R¹), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 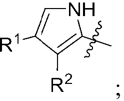 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, -NR¹⁰, COOC₁-C₆ alkyl, C₆-C₁₀ aryl, and CONR⁸R⁹.

In some embodiments, the optionally substituted ring A is 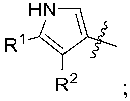 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 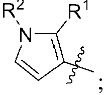 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 0-2 heteroatoms and/heteroatomic groups independently selected from O, NH, NR¹³, S, S(O), and S(O)₂ (in addition to the nitrogen atom attached to R²), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 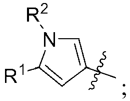 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 0-2 heteroatoms and/heteroatomic groups independently selected from O, NH, NR¹³, S, S(O), and S(O)₂ (in addition to the nitrogen attached to R²), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 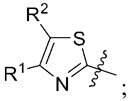 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, -NR¹⁰, COOC₁-C₆ alkyl, C₆-C₁₀ aryl, and CONR⁸R⁹.

In some embodiments, the optionally substituted ring A is 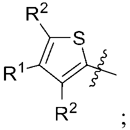 one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 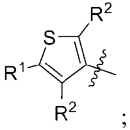 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, -NR¹⁰, COOC₁-C₆ alkyl, C₆-C₁₀ aryl, and CONR⁸R⁹.

In some embodiments, the optionally substituted ring A is 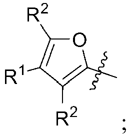 one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 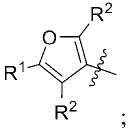 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 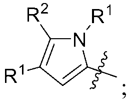 one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one of the adjacent atoms is a nitrogen atom, then the heterocyclic ring includes from 0-2 heteroatoms and/or heteroatomic groups independently selected from O, NH, NR¹³, S, S(O), and S(O)₂ (in addition to the aforementioned nitrogen atom attached to R¹), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 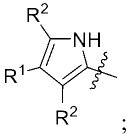 one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 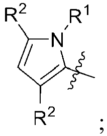 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 0-2 heteroatoms and/heteroatomic groups independently selected from O, NH, NR¹³, S, S(O), and S(O)₂ (in addition to the nitrogen atom attached to R¹), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 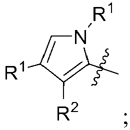 R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 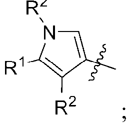 one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one of the adjacent atoms is a nitrogen atom, then the heterocyclic ring includes from 0-2 heteroatoms and/or heteroatomic groups independently selected from O, NH, NR¹³, S, S(O), and S(O)₂ (in addition to the aforementioned nitrogen atom attached to R²), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments, the optionally substituted ring A is 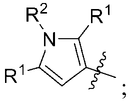 one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 0-2 heteroatoms and/heteroatomic groups independently selected from O, NH, NR¹³, S, S(O), and S(O)₂ (in addition to the nitrogen atom(s) attached to R²), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA, when ring A is phenyl, then R¹ and R² are each independently selected from C₃-C₆ alkyl, C₁-C₆ haloalkyl, C₁-C₆ alkoxy, C₁-C₆ haloalkoxy, F, I, CN, NO₂, COC₁-C₆ alkyl, CO-C₆-C₁₀ aryl, CO(5- to 10-membered heteroaryl), CO₂C₁-C₆ alkyl, CO₂C₃-C₈ cycloalkyl, OCOC₂-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ haloalkyl, C₃-C₇ cycloalkyl and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further
   optionally independently substituted with one to three hydroxy, halo, NR⁸R⁹, or oxo;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄ or C₆-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA, when ring A is pyridyl, then R¹ and R² are each independently selected from C₃-C₆ alkyl, C₁-C₆ haloalkyl, C₁-C₆ alkoxy, C₁-C₆ haloalkoxy, F, I, CN, NO₂, COC₁-C₆ alkyl, CO-C₆-C₁₀ aryl, CO(5- to 10-membered heteroaryl), CO₂C₁-C₆ alkyl, CO₂C₃-C₈ cycloalkyl, OCOC₂-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, and 3- to 7-membered heterocycloalkyl,
wherein the C₃-C₆ alkyl, C₁-C₆ haloalkyl, and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further
   optionally independently substituted with one to three hydroxy, halo, NR⁸R⁹, or oxo;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄ or C₆-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂,, and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.

In some embodiments of the compound of Formula AA, when ring A is phenyl, then R¹ and R² are each independently selected from C₃ alkyl, C₅-C₆ alkyl, C₁-C₆ haloalkyl, C₁-C₆ alkoxy, C₁-C₆ haloalkoxy, F, I, CN, NO₂, COC₂-C₆ alkyl, CO-C₆-C₁₀ aryl, CO(5- to 10-membered heteroaryl), CO₂C₁-C₆ alkyl, CO₂C₃-C₈ cycloalkyl, OCOC₂-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ haloalkyl, C₃-C₇ cycloalkyl and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further
   optionally independently substituted with one to three hydroxy, halo, NR⁸R⁹, or oxo;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄ or C₆-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
when ring A is pyridyl, then R¹ and R² are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO-C₆-C₁₀ aryl, CO(5- to 10-membered heteroaryl), CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₂-C₆ alkyl, N(C₁-C₆ alkyl)₂, NHCOC₁-C₆ alkyl, NHCOC₆-C₁₀ aryl, NHCO(5- to 10-membered heteroaryl), NHCO(3-to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl, and 3- to 7-membered heterocycloalkyl,
wherein the C₁-C₆ alkyl, C₁-C₆ haloalkyl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 5-membered heterocycloalkyl, 5-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, halo, NR⁸R⁹, or oxo;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹; and R⁶ and R⁷ are each independently selected from a C₂-C₆ alkyl, C₂-C₆ haloalkyl, C₂-C₆ alkoxy, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of the compound of Formula AA-1, when ring A' is phenyl, then R¹ and R² are each independently selected from C₃ alkyl, C₅-C₆ alkyl, C₁-C₆ haloalkyl, C₁-C₆ alkoxy, C₁-C₆ haloalkoxy, F, I, CN, NO₂, COC₂-C₆ alkyl, CO-C₆-C₁₀ aryl, CO(5- to 10-membered heteroaryl), CO₂C₁-C₆ alkyl, CO₂C₃-C₈ cycloalkyl, OCOC₂-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ haloalkyl, C₃-C₇ cycloalkyl and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further
   optionally independently substituted with one to three hydroxy, halo, NR⁸R⁹, or oxo;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄ or C₆-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂,and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
when ring A is pyridyl, then R¹ and R² are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO-C₆-C₁₀ aryl, CO(5- to 10-membered heteroaryl), CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₂-C₆ alkyl, N(C₁-C₆ alkyl)₂, NHCOC₁-C₆ alkyl, NHCOC₆-C₁₀ aryl, NHCO(5- to 10-membered heteroaryl), NHCO(3-to 7-membered heterocycloalkyl), NHCOC₂-C₆ alkynyl, NHCOOC₁-C₆ alkyl, NH-(C=NR¹³)NR¹¹R¹², CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl, and 3- to 7-membered heterocycloalkyl,
wherein the C₁-C₆ alkyl, C₁-C₆ haloalkyl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 5-membered heterocycloalkyl, 5-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), and NHCOC₂-C₆ alkynyl;
   wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, halo, NR⁸R⁹, or oxo;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂,and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3-to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹; R^{6"} is selected from C₂-C₆ alkyl, C₂-C₆ haloalkyl, C₁-C₆ haloalkoxy, I, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl, 3- to 10-membered heterocycloalkyl, and C₂-C₆ alkenyl,
wherein R^{6"} is optionally substituted with one or more substituents independently selected from hydroxy, Cl, Br, I, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R^{6"} is substituted with is optionally substituted with one or more hydroxyl, C₆-C₁₀ aryl or NR⁸R⁹, or wherein R^{6"} is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
R⁷ is selected from C₂-C₆ alkyl, C₂-C₆ haloalkyl, C₁-C₆ haloalkoxy, I, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl, wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), NHCOC₁-C₆ alkyl, NHCOC₆-C₁₀ aryl, NHCO(5- to 10-membered heteroaryl), NHCO(3- to 7-membered heterocycloalkyl), NHCOC₂-C₆ alkynyl, C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.

In some embodiments of Formula **AA, Ring A** is 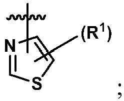 and **R¹** is C₁-C₆ alkyl substituted with one or more (e.g., from 1-2) hydroxy (e.g., 2-hydroxy-2-propyl or 1,2-dihydroxy-2-propyl).

In some embodiments of Formula AA, **Ring A** is 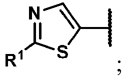 and **R¹** is C₁-C₆ alkyl substituted with one or more (e.g., from 1-2) hydroxy (e.g., 2-hydroxy-2-propyl or 1,2-dihydroxy-2-propyl).

In some embodiments of Formula AA, **Ring A** is 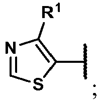 and **R¹** is C₁-C₆ alkyl substituted with one or more (e.g., from 1-2) hydroxy (e.g., 2-hydroxy-2-propyl or 1,2-dihydroxy-2-propyl).

In some embodiments of Formula AA, **Ring A** is 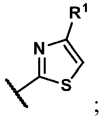 and **R¹** is C₁-C₆ alkyl substituted with one or more (e.g., from 1-2) hydroxy (e.g., 2-hydroxy-2-propyl or 1,2-dihydroxy-2-propyl).

In some embodiments of Formula AA, **Ring A** is 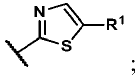 and **R¹** is C₁-C₆ alkyl substituted with one or more (e.g., from 1-2) hydroxy (e.g., 2-hydroxy-2-propyl or 1,2-dihydroxy-2-propyl).

In some embodiments of Formula AA, **Ring A** is 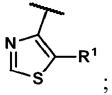 and **R¹** is C₁-C₆ alkyl substituted with one or more (e.g., from 1-2) hydroxy (e.g., 2-hydroxy-2-propyl or 1,2-dihydroxy-2-propyl).

In some embodiments of Formula AA, **Ring A** is 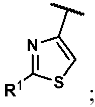 and **R¹** is C₁-C₆ alkyl substituted with one or more (e.g., from 1-2) hydroxy (e.g., 2-hydroxy-2-propyl or 1,2-dihydroxy-2-propyl).

In some embodiments of Formula AA, **Ring A** is 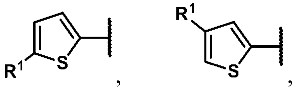 or 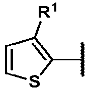 (e.g., 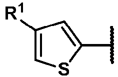 ); and **R¹** is C₁-C₆ alkyl substituted with one or more (e.g., from 1-2) hydroxy (e.g., 2-hydroxy-2-propyl or 1,2-dihydroxy-2-propyl).

In some embodiments of Formula AA, **Ring A** is 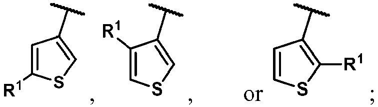 and **R¹** is C₁-C₆ alkyl substituted with one or more (e.g., from 1-2) hydroxy (e.g., 2-hydroxy-2-propyl or 1,2-dihydroxy-2-propyl).

In some embodiments of Formula AA, **Ring A** is 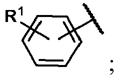 and **R¹** is C₁-C₆ alkyl substituted with one or more (e.g., one) N**R⁸R⁹.**

In some embodiments of Formula AA, **Ring A** is 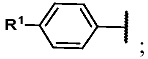 and **R¹** is C₁-C₆ alkyl substituted with one or more (e.g., one) N**R⁸R⁹.**

In some embodiments of Formula AA, **Ring A** is 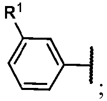 and **R¹** is C₁-C₆ alkyl substituted with one or more (e.g., one) N**R⁸R⁹.**

In some embodiments of Formula AA, **Ring A** is 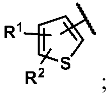 **R¹** is C₁-C₆ alkyl substituted with one or more (e.g., from 1-2) hydroxy (e.g., 2-hydroxy-2-propyl or 1,2-dihydroxy-2-propyl); and **R²** is halo.

In some embodiments of Formula AA, **Ring A** is 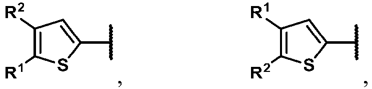 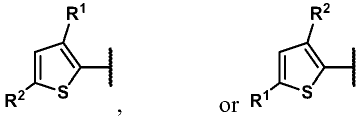 (e.g., 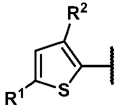 ); **R¹** is C₁-C₆ alkyl substituted with one or more (e.g., from 1-2) hydroxy (e.g., 2-hydroxy-2-propyl or 1,2-dihydroxy-2-propyl); and **R²** is halo.

In some embodiments of Formula AA, **Ring A** is 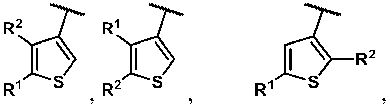 or 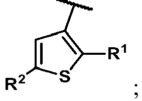 **R¹** is C₁-C₆ alkyl substituted with one or more (e.g., from 1-2) hydroxy (e.g., 2-hydroxy-2-propyl or 1,2-dihydroxy-2-propyl); and **R²** is halo.

In some embodiments of Formula AA, **Ring A** is 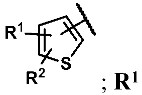 is C₁-C₆ alkyl substituted with one or more (e.g., one) N**R⁸R⁹;** and **R²** is halo.

In some embodiments of Formula AA, **Ring A** is 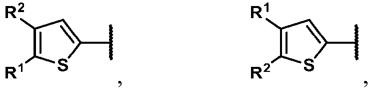 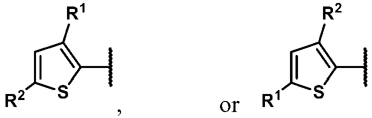 (e.g., 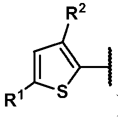 ); **R¹** is C₁-C₆ alkyl substituted with one or more (e.g., one) N**R⁸R⁹;** and **R²** is halo.

In some embodiments of Formula AA, **Ring A** is 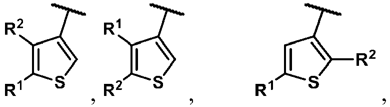 or 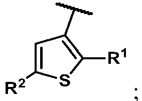 **R¹** is C₁-C₆ alkyl substituted with one or more (e.g., one) N**R⁸R⁹;** and **R²** is halo.

In some embodiments of Formula AA, **Ring A** is 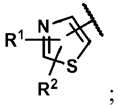 and each of **R¹** and **R²** is C₁-C₆ alkyl substituted with one or more (e.g., from 1-2) hydroxy.

In some embodiments of Formula AA, **Ring A** is 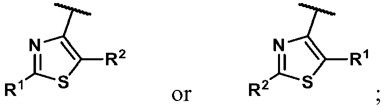 and each of **R¹** and **R²** is C₁-C₆ alkyl substituted with one or more (e.g., from 1-2) hydroxy.

In some embodiments of Formula AA, **Ring A** is 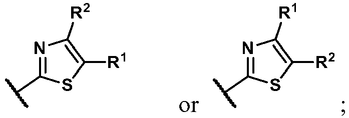 and each of **R¹** and **R²** is C₁-C₆ alkyl substituted with one or more (e.g., from 1-2) hydroxy. In certain of these embodiments, **R²** is hydroxy methyl.

In some embodiments of Formula AA, **Ring A** is 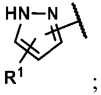 and **R¹** is C₁-C₆ alkyl optionally substituted with one or more halo (e.g., ethyl or difluoromethyl).

In some embodiments of Formula AA, **Ring A** is 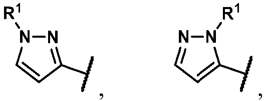 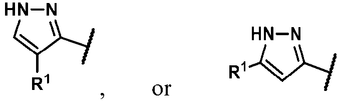 (e.g., 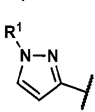 ); and **R¹** is C₁-C₆ alkyl optionally substituted with one or more halo (e.g., ethyl or difluoromethyl).

In some embodiments of Formula AA, **Ring A** is: 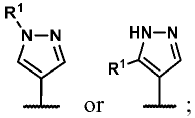 and and **R¹** is C₁-C₆ alkyl optionally substituted with one or more halo (e.g., ethyl or difluoromethyl).

In some embodiments of Formula AA, **Ring A** is 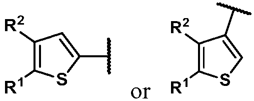 (e.g., 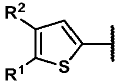 ); and **R¹** and **R²** taken together with the atoms connecting them, independently form one monocyclic or bicyclic C₄-C₁₂ carbocyclic ring (e.g., C₅ or C₆ carbocyclic ring) or one monocyclic or bicyclic 5- to-12-membered (e.g., 6-membered or 5-membered) heterocyclic ring containing 1-3 (e.g., 1-2, e.g., 1) heteroatoms independently selected from O, N, and S (e.g., N or O), wherein the carbocyclic ring or heterocyclic ring is optionally independently substituted with one or more substituents each independently selected from hydroxy, halo, oxo, C₁-C₆ alkyl (e.g., methyl), C₂-C₆ alkenyl, C₂-C₆ alkynyl, C₁-C₆ alkoxy (e.g., methoxy, ethoxy, isopropoxyl), OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl (e.g., azetidinyl or oxetanyl), and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo (e.g., fluoro), C₁-C₆ alkyl, C₂-C₆ alkenyl, C₂-C₆ alkynyl, C₃-C₁₀ cycloalkyl, C₁-C₆ alkoxy, oxo, NR⁸R⁹ (e.g., amino, methylamino, or dimethylamino), =NR¹⁰, COOC₁-C₆ alkyl, C₆-C₁₀ aryl, and CONR⁸R⁹.

In some embodiments of Formula AA, **Ring A** is 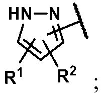 and each of **R¹** and **R²** is C₁-C₆ alkyl substituted with one or more (e.g., from 1-2) hydroxy.

In some embodiments of Formula AA, **Ring A** is 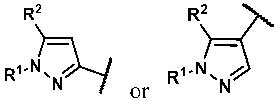 (e.g., 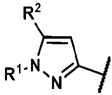 ); and each of **R¹** and **R²** is C₁-C₆ alkyl substituted with one or more (e.g., from 1-2) hydroxy.

In some embodiments of Formula AA, **Ring A** is 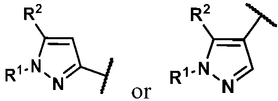 (e.g., 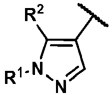 ); and **R¹** and **R²** taken together with the atoms connecting them, independently form one monocyclic or bicyclic C₄-C₁₂ carbocyclic ring (e.g., C₅ or C₆ carbocyclic ring) or one monocyclic or bicyclic 5- to-12-membered heterocyclic ring containing 1-3 (e.g., 1-2, e.g., 1) heteroatoms independently selected from O, N, and S, wherein the carbocyclic ring or heterocyclic ring is optionally independently substituted with one or more substituents each independently selected from hydroxy, halo, oxo, C₁-C₆ alkyl (e.g., methyl), C₂-C₆ alkenyl, C₂-C₆ alkynyl, C₁-C₆ alkoxy (e.g., methoxy, ethoxy, isopropoxyl), OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl (e.g., azetidinyl or oxetanyl), and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo (e.g., fluoro), C₁-C₆ alkyl, C₂-C₆ alkenyl, C₂-C₆ alkynyl, C₃-C₁₀ cycloalkyl, C₁-C₆ alkoxy, oxo, NR⁸R⁹ (e.g., amino, methylamino, or dimethylamino), =NR¹⁰, COOC₁-C₆ alkyl, C₆-C₁₀ aryl, and CONR⁸R⁹.

In some embodiments of Formula AA, the substituted ring B is

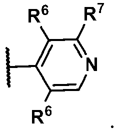

In certain of these embodiments, the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B is

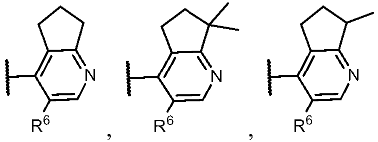

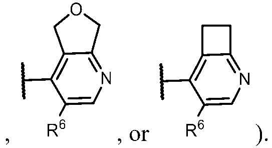

In certain embodiments (when the substituted ring B is selected from: 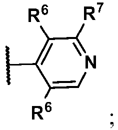 and the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from:

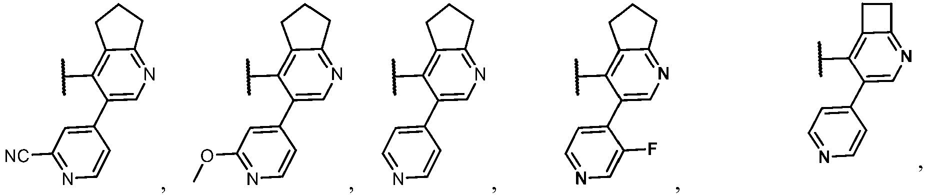

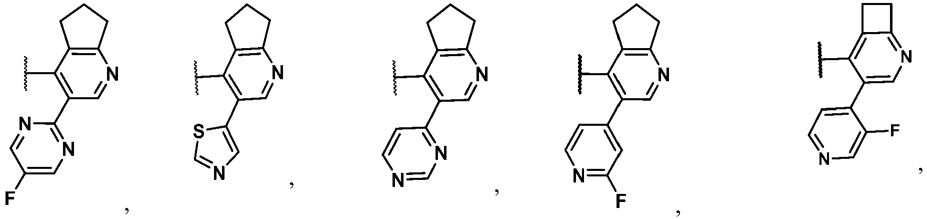

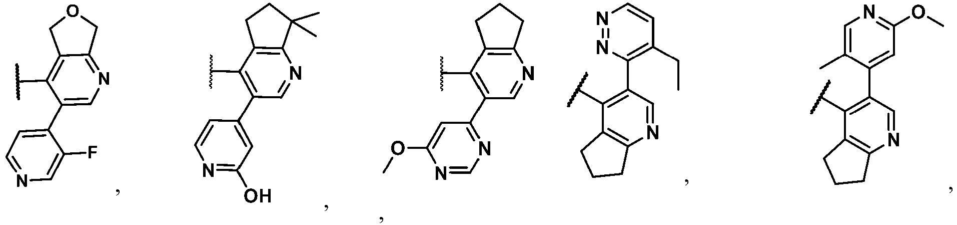

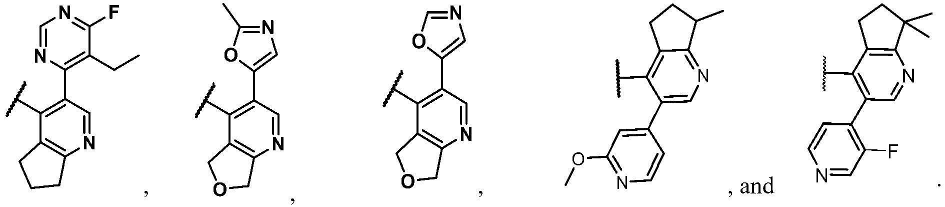

In certain embodiments (when the substituted ring B is 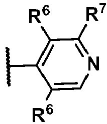 ), one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B is 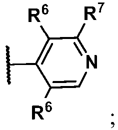 and one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R⁶ and R⁷ are independently selected from the group consisting of cyano, halo, C₁-C₆ alkyl, C₁-C₆ haloalkyl, C₁-C₆ alkoxy, and C₃-C₇ cycloalkyl.

As non-limiting examples of the foregoing embodiments, B is:

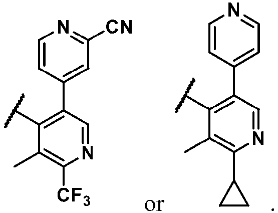

In some embodiments of Formula AA, the substituted ring B is

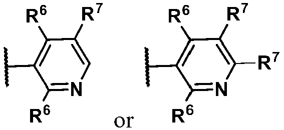

In certain of these embodiments, the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B is 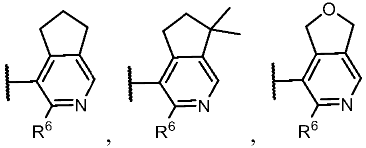 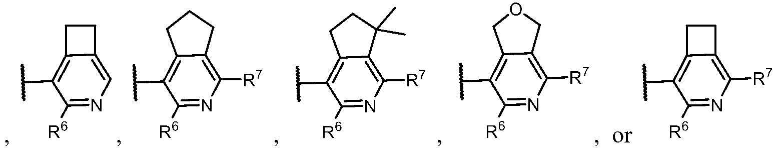 ).

In certain embodiments (when the substituted ring B is 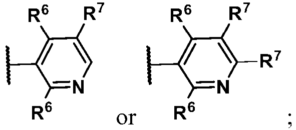 and the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from:

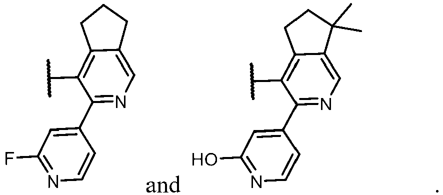

In certain embodiments (when the substituted ring B is 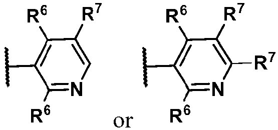 ), one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B is 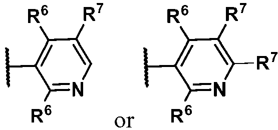 ; and one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R⁶ and each R⁷ are independently selected from the group consisting of cyano, halo, C₁-C₆ alkyl, C₁-C₆ haloalkyl, C₁-C₆ alkoxy, and C₃-C₇ cycloalkyl.

As non-limiting examples of the foregoing embodiments, B is:

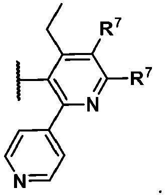

In some embodiments of Formula AA, the substituted ring B is 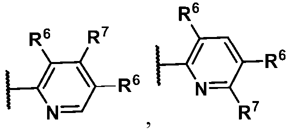 , or

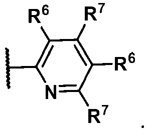

In certain of these embodiments, one pair of R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B is 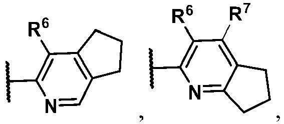 or

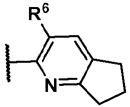

In certain embodiments (when the substituted ring B is is 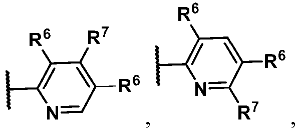 or 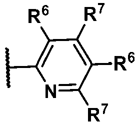 ); and one pair of R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from: 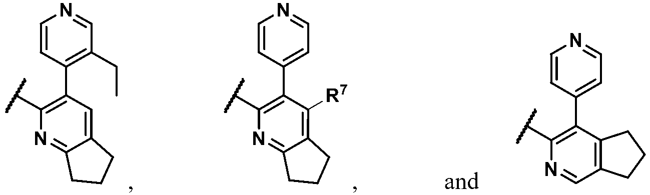 (e.g., R⁷ is cyano or halo (e.g., halo such as F)).

In some embodiments of Formula AA-1, the substituted ring B is

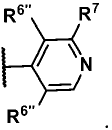

In certain of these embodiments, the R^{6"} and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R^{6"} and R⁷ on adjacent atoms, taken together with the atoms connecting them, independently form C₄-C₇ (e.g., C₅) carbocyclic ring. For example, the substituted ring B is 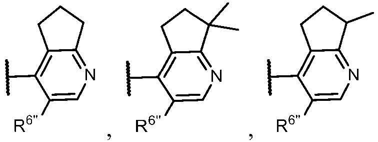 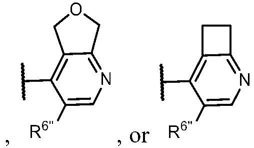 ).

In certain embodiments (when the substituted ring B is selected from: 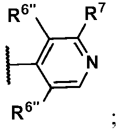 and the R^{6"} and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R^{6"} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R^{6"} is C₆-C₁₀ aryl or 5- to 10-membered heteroaryl optionally substituted with a substituent selected from halo, CN, C₁-C₆ alkyl, and C₁-C₆ alkoxy. For example, R^{6"} is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from:

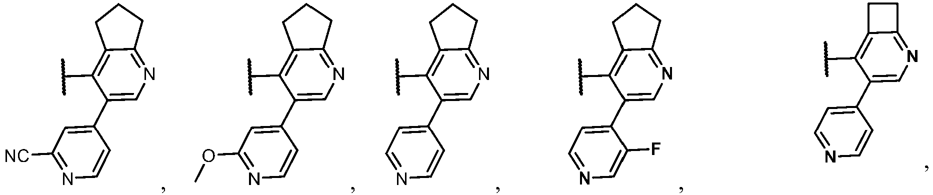

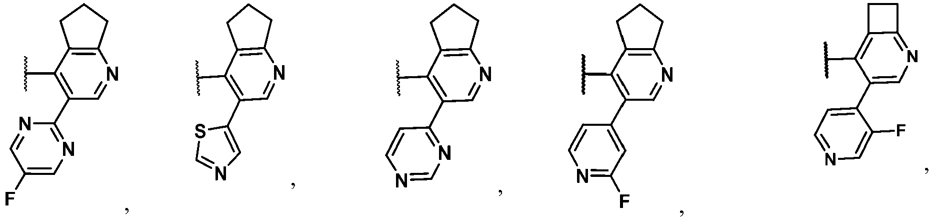

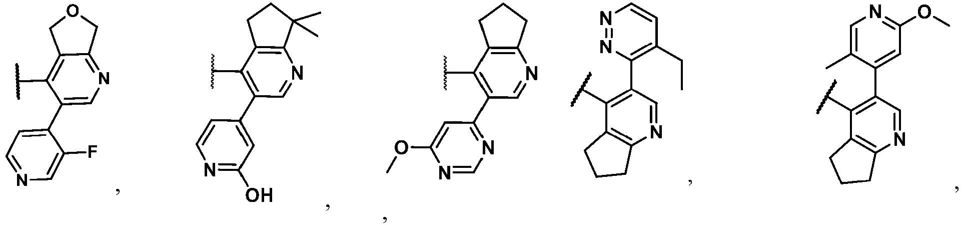

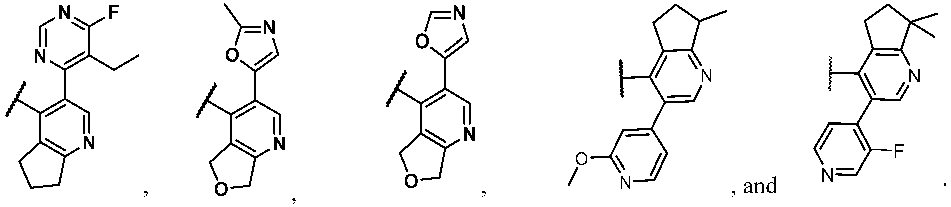

In certain embodiments (when the substituted ring B is 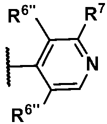 ), one R^{6"} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R^{6"} is C₆-C₁₀ aryl or 5- to 10-membered heteroaryl optionally substituted with a substituent selected from halo, CN, C₁-C₆ alkyl, and C₁-C₆ alkoxy. For example, R^{6"} is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B is 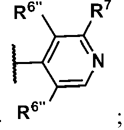 and one R^{6"} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R^{6"} and R⁷ are independently selected from the group consisting of halo, C₁-C₆ alkyl, C₁-C₆ haloalkyl, C₁-C₆ alkoxy, and C₃-C₇ cycloalkyl.

As non-limiting examples of the foregoing embodiments, B is:

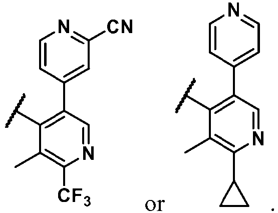

In some embodiments of Formula AA-1, the substituted ring B is 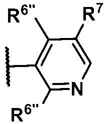 or

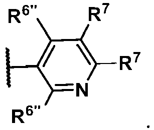

In certain of these embodiments, the R^{6"} and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R^{6"} and R⁷ on adjacent atoms, taken together with the atoms connecting them, independently form C₄-C₇ (e.g., C₅) carbocyclic ring. For example, the substituted ring B is 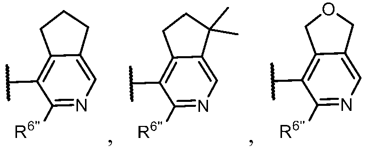 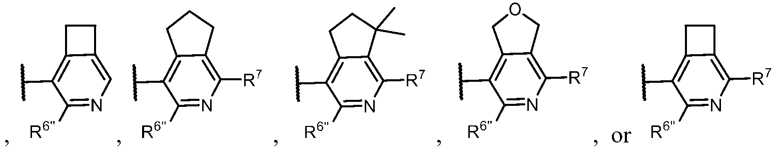 ).

In certain embodiments (when the substituted ring B is 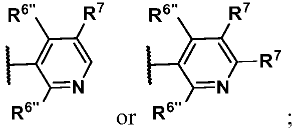 and the R^{6"} and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R^{6"} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R^{6"} is C₆-C₁₀ aryl or 5- to 10-membered heteroaryl optionally substituted with a substituent selected from halo, CN, C₁-C₆ alkyl, and C₁-C₆ alkoxy. For example, R^{6"} is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from:

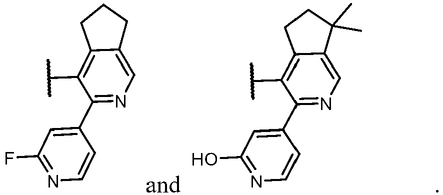

In certain embodiments (when the substituted ring B is 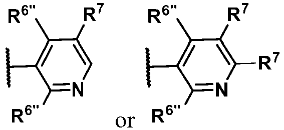 ), one R^{6"} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R^{6"} is C₆-C₁₀ aryl or 5- to 10-membered heteroaryl optionally substituted with a substituent selected from halo, CN, C₁-C₆ alkyl, and C₁-C₆ alkoxy. For example, R^{6"} is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B is 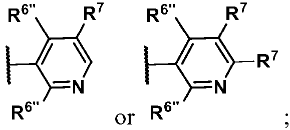 and one R^{6"} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R^{6"} and each R⁷ are independently selected from the group consisting of halo, C₁-C₆ alkyl, C₁-C₆ haloalkyl, C₁-C₆ alkoxy, and C₃-C₇ cycloalkyl.

As non-limiting examples of the foregoing embodiments, B is:

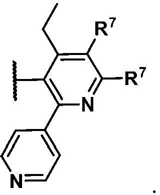

In some embodiments of Formula AA-1, the substituted ring B is

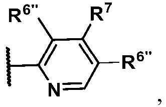

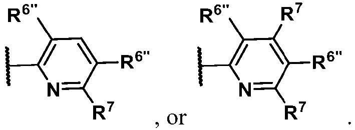

In certain of these embodiments, one pair of R^{6"} and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R^{6"} and R⁷ on adjacent atoms, taken together with the atoms connecting them, independently form C₄-C₇ (e.g., C₅) carbocyclic ring. For example, the substituted ring B is 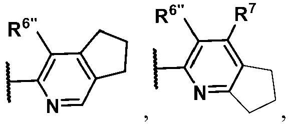 or

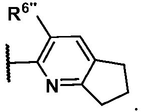

In certain embodiments (when the substituted ring B is 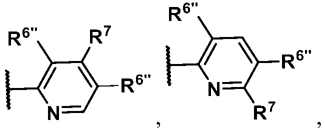 or 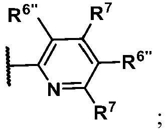 and one pair of R^{6"} and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R^{6"} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R^{6"} is C₆-C₁₀ aryl or 5- to 10-membered heteroaryl optionally substituted with a substituent selected from halo, CN, C₁-C₆ alkyl, and C₁-C₆ alkoxy. For example, R^{6"} is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from: 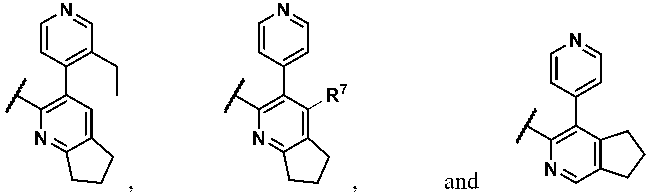 (e.g., R⁷ is cyano or halo (e.g., halo such as F)).

In some embodiments of Formula AA-2, the substituted ring B is

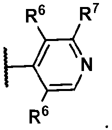

In certain of these embodiments, the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B is 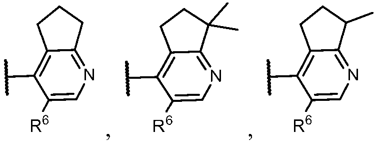 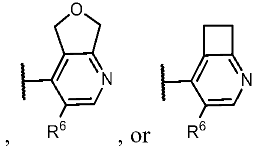 ).

In certain embodiments (when the substituted ring B is selected from: 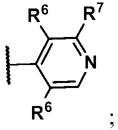 and the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from:

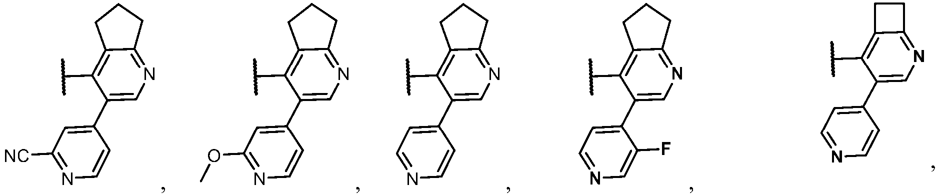

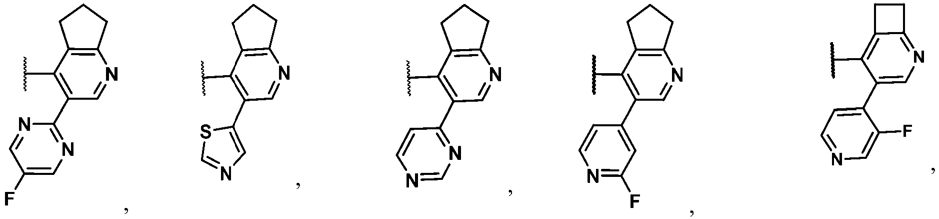

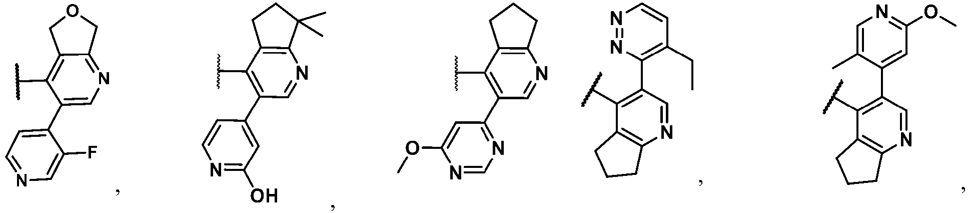

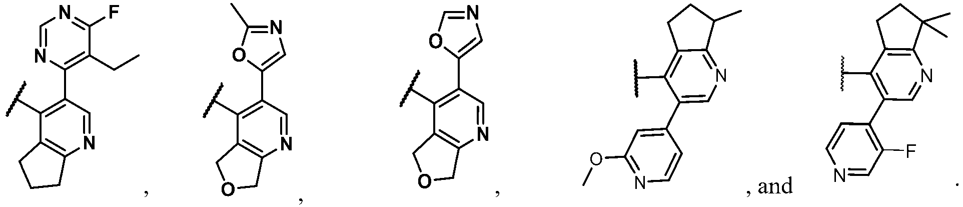

In certain embodiments (when the substituted ring B is 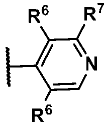 ), one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B is 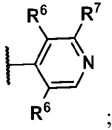 and one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R⁶ and R⁷ are independently selected from the group consisting of cyano, halo, C₁-C₆ alkyl, C₁-C₆ haloalkyl, C₁-C₆ alkoxy, and C₃-C₇ cycloalkyl.

As non-limiting examples of the foregoing embodiments, B is:

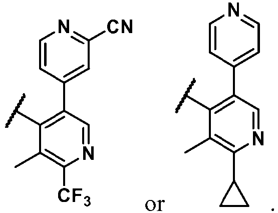

In some embodiments of Formula AA-2, the substituted ring B is 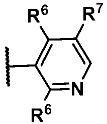 or

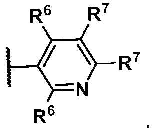

In certain of these embodiments, the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B is 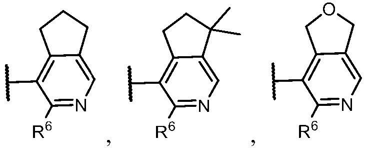 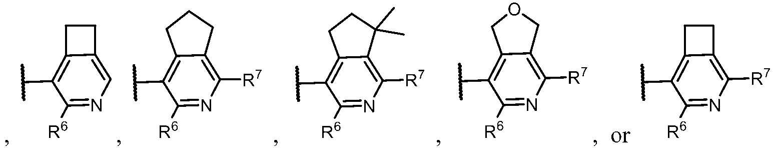 ).

In certain embodiments (when the substituted ring B is 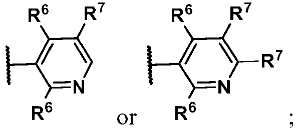 and the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from:

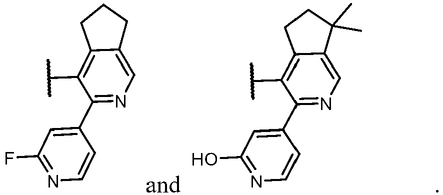

In certain embodiments (when the substituted ring B is 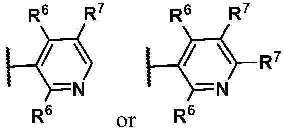 ), one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B is 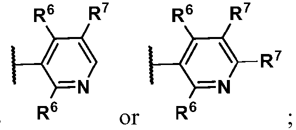 and one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R⁶ and each R⁷ are independently selected from the group consisting of cyano, halo, C₁-C₆ alkyl, C₁-C₆ haloalkyl, C₁-C₆ alkoxy, and C₃-C₇ cycloalkyl.

As non-limiting examples of the foregoing embodiments, B is:

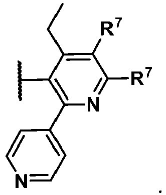

In some embodiments of Formula AA-2, the substituted ring B is

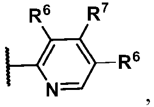

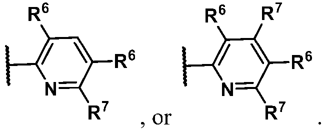

In certain of these embodiments, one pair of R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B is 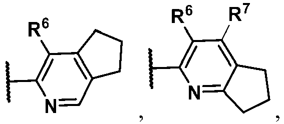 or

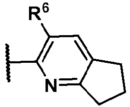

In certain embodiments (when the substituted ring B is is 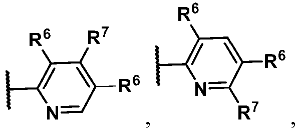 or 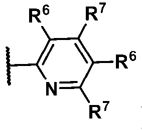 ); and one pair of R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from: 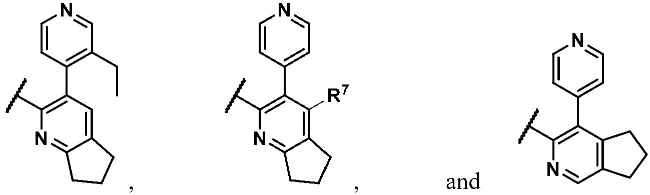 (e.g., R⁷ is cyano or halo (e.g., halo such as F)).

In some embodiments of Formula AA-3, the substituted ring B is

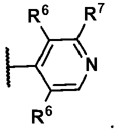

In certain of these embodiments, the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B is 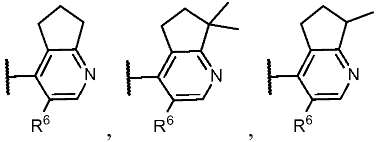 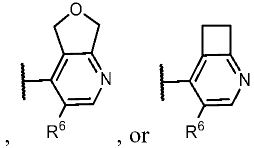 ).

In certain embodiments (when the substituted ring B is selected from: 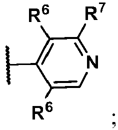 and the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from:

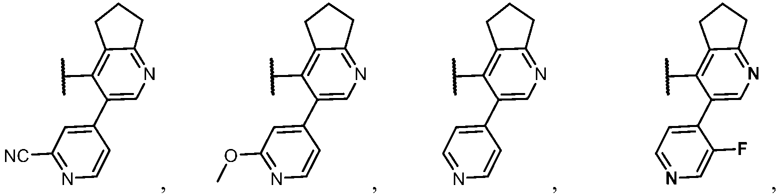

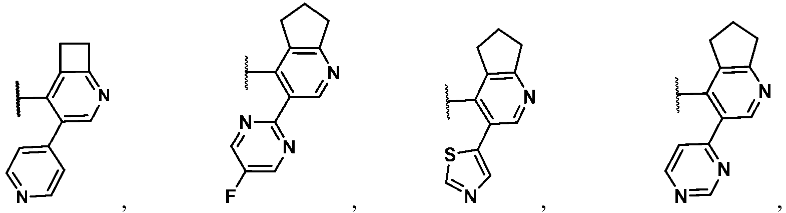

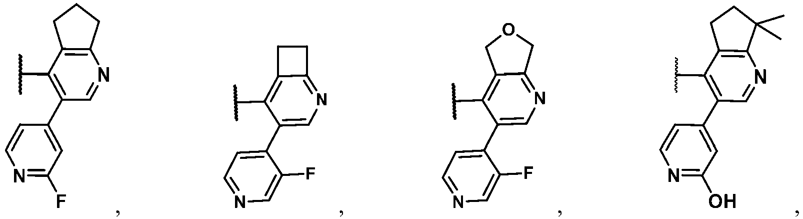

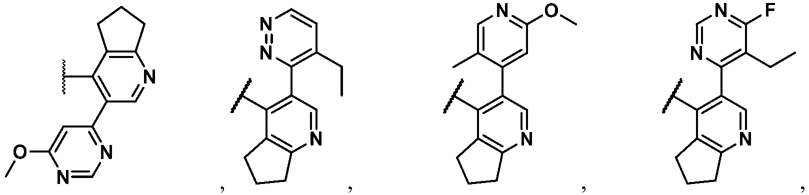

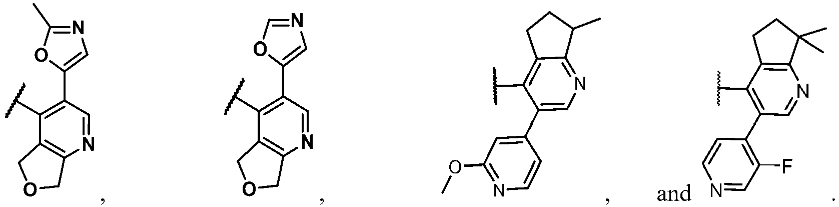

In certain embodiments (when the substituted ring B is 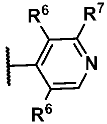 ), one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B is 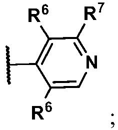 and one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R⁶ and R⁷ are independently selected from the group consisting of cyano, halo, C₁-C₆ alkyl, C₁-C₆ haloalkyl, C₁-C₆ alkoxy, and C₃-C₇ cycloalkyl.

As non-limiting examples of the foregoing embodiments, B is:

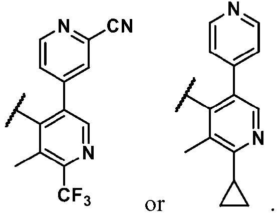

In some embodiments of Formula AA-3, the substituted ring B is 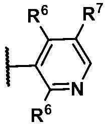 or

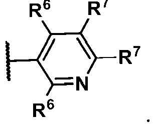

In certain of these embodiments, the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B is 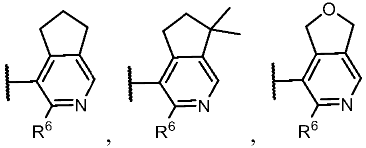 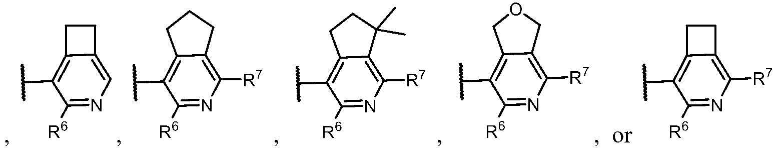 ).

In certain embodiments (when the substituted ring B is 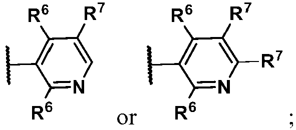 and the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from:

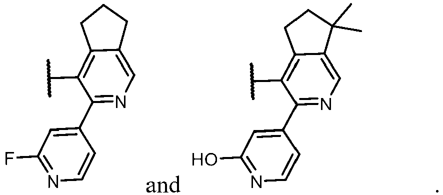

In certain embodiments (when the substituted ring B is 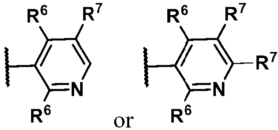 ), one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B is 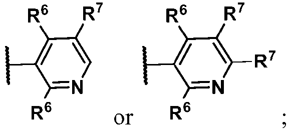 and one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R⁶ and each R⁷ are independently selected from the group consisting of cyano, halo, C₁-C₆ alkyl, C₁-C₆ haloalkyl, C₁-C₆ alkoxy, and C₃-C₇ cycloalkyl.

As non-limiting examples of the foregoing embodiments, B is:

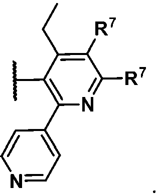

In some embodiments of Formula AA-3, the substituted ring B is

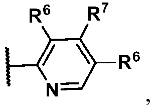

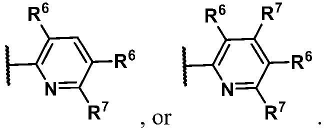

In certain of these embodiments, one pair of R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B is 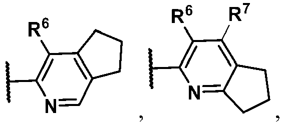 or

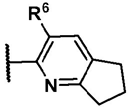

In certain embodiments (when the substituted ring B is is 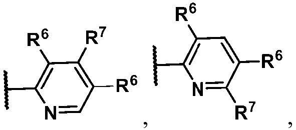 or 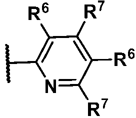 ); and one pair of R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B is selected from: 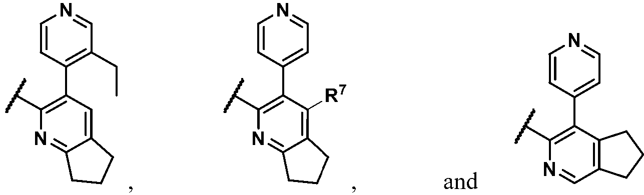 (e.g., R⁷ is cyano or halo (e.g., halo such as F)).

In some embodiments of Formula AA-4, the substituted ring B' is 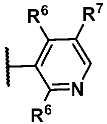 or

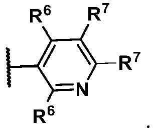

In certain of these embodiments, the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example. the substituted ring B* is 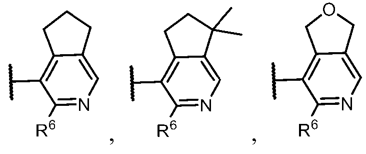 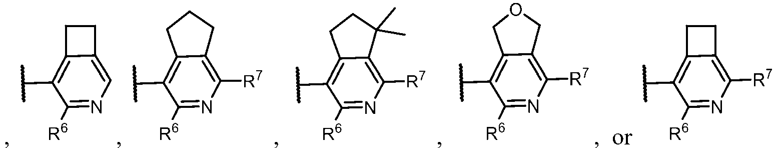 ).

In certain embodiments (when the substituted ring B' is 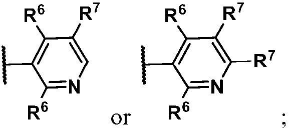 and the R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B' is selected from:

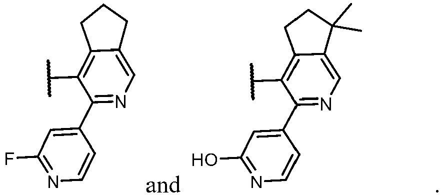

In certain embodiments (when the substituted ring B' is 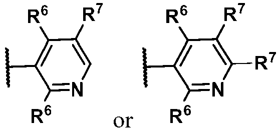 ), one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B' is 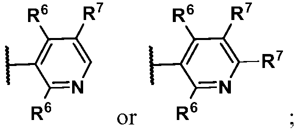 and one R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R⁶ and each R⁷ are independently selected from the group consisting of cyano, halo, C₁-C₆ alkyl, C₁-C₆ haloalkyl, C₁-C₆ alkoxy, and C₃-C₇ cycloalkyl.

As non-limiting examples of the foregoing embodiments, B is:

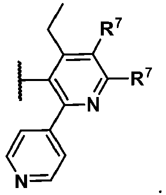

In some embodiments of Formula AA-4, the substituted ring B' is

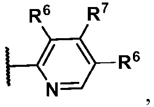

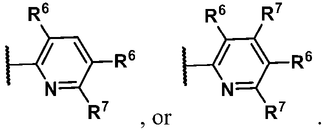

In certain of these embodiments, one pair of R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R⁶ and R⁷ on adjacent atoms, taken together with the atoms connecting them, independently form C₄-C₇ (e.g., C₅) carbocyclic ring. For example, the substituted ring B' is 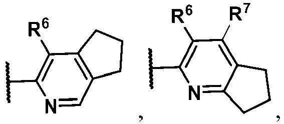 or

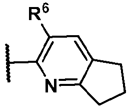

In certain embodiments (when the substituted ring B' is is 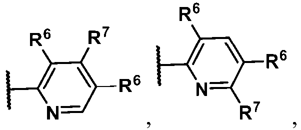 or 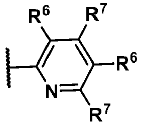 ); and one pair of R⁶ and R⁷ on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R⁶ is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R⁶ is C₆-C₁₀ aryl or 5- to 10-membered heteroaryl optionally substituted with a substituent selected from halo, CN, C₁-C₆ alkyl, and C₁-C₆ alkoxy. For example, R⁶ is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B' is selected from: 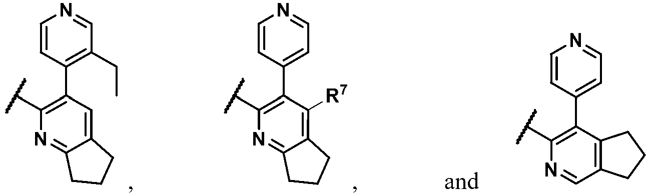 (e.g., R⁷ is cyano or halo (e.g., halo such as F)).

In some embodiments of Formula AA-5, the substituted ring B" is

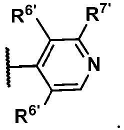

In certain of these embodiments, the R^{6'} and R^{7'} on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹. For example, the R^{6'} and R^{7'} on adjacent atoms, taken together with the atoms connecting them, independently form C₄-C₇ (e.g., C₅) carbocyclic ring. For example, the substituted ring B" is 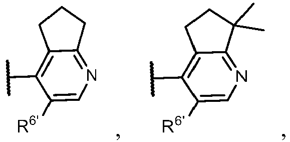 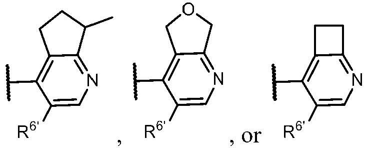 ).

In certain embodiments (when the substituted ring B" is selected from: 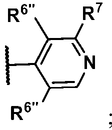 and the R^{6'} and R^{7'} on adjacent atoms, taken together with the atoms connecting them, independently form C₄-C₇ (e.g., C₄ or C₅) carbocyclic ring or 5-to-7-membered heterocyclic ring containing 1 or 2 heteroatoms independently selected from O, N, and S, wherein the carbocyclic ring or heterocyclic ring is optionally independently substituted with one or more substituents independently selected from hydroxy, halo, oxo, C₁-C₆ alkyl, C₁-C₆ alkoxy, NR⁸R⁹, =NR¹⁰, COOC₁-C₆ alkyl, C₆-C₁₀ aryl, and CONR⁸R⁹):
the remaining R^{6'} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, the remaining R^{6'} is C₆-C₁₀ aryl or 5- to 10-membered heteroaryl optionally substituted with a substituent selected from halo, CN, C₁-C₆ alkyl, and C₁-C₆ alkoxy. For example, R^{6'} is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

As a non-limiting example of the foregoing embodiments, substituted ring B" is selected from:

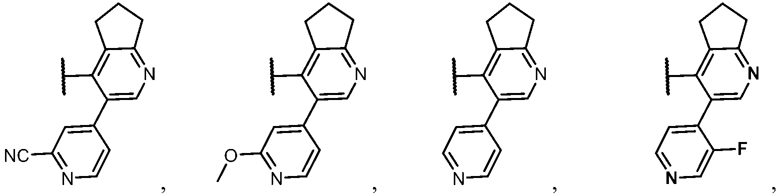

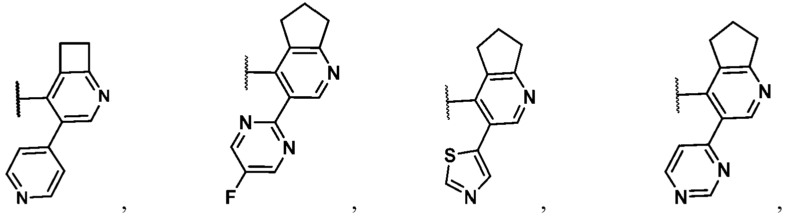

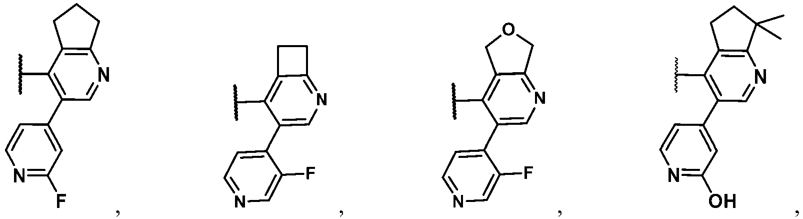

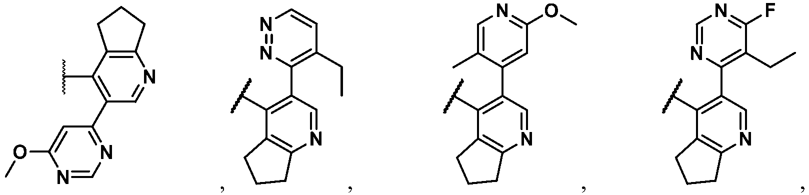

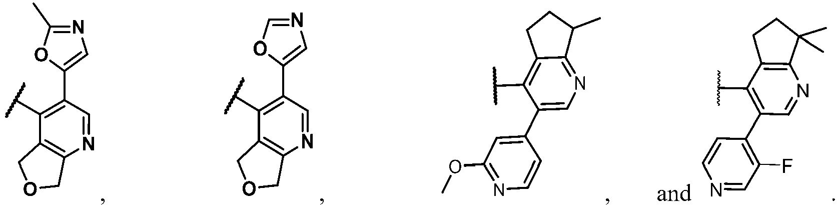

In certain embodiments (when the substituted ring B" is 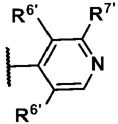 ), one R^{6'} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.

In certain of these embodiments, one R^{6'} is C₆-C₁₀ aryl or 5- to 10-membered heteroaryl optionally substituted with a substituent selected from halo, CN, C₁-C₆ alkyl, and C₁-C₆ alkoxy. For example, R^{6'} is 5-6 membered heteroaryl (e.g., pyridinyl (e.g., pyridin-4-yl), pyrimidinyl, pyridazinyl, oxazolyl, or thiazolyl) optionally substituted with a substituent selected from halo, CN, C₁-C₆ alkyl, and C₁-C₆ alkoxy.

In certain embodiments (when the substituted ring B" is 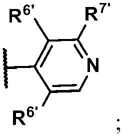 and one R^{6'} is C₆-C₁₀ aryl or 5- to 10-membered heteroaryl, each of which is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5-to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl):
the remaining R^{6'} and R^{7'} are independently selected from the group consisting of CN, halo, unbranched C₁-C₆ alkyl, C₁-C₆ haloalkyl, C₁-C₆ alkoxy, and C₃-C₇ cycloalkyl.

As non-limiting examples of the foregoing embodiments, B" is:

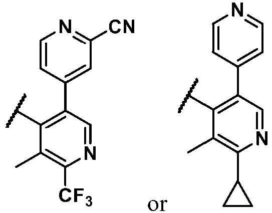

In some embodiments, the compound of Formula AA is a compound of Formula BB 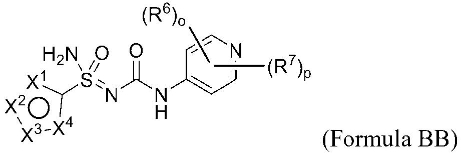 wherein
X¹ is selected from CH, CR¹, CR², N, NH, NR¹, NR², and S;
X² is selected from CH, CR¹, CR², N, NH, NR¹, NR², and S;
X³ is selected from CH, CR¹, CR², N, NH, NR¹, NR², and S;
X⁴ is selected from CH, CR¹, CR², N, NH, NR¹, NR², and S; 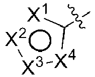
is aromatic and charge neutral;
X¹, X², X³, and X⁴ collectively comprise from 0-2 R¹ and from 0-2 R², wherein the sum of R¹ and R² is from 0-3;
o is 1 or 2 and p is 1 or 2, wherein the sum of o and p is 3 or 4; and
and wherein R¹, R², R⁶, and R⁷ are as defined previously herein.

In some embodiments of Formula BB, X¹ is CR¹ or CR².

In some embodiments of Formula BB, X¹ is CH.

In some embodiments of Formula BB, X² is N.

In some embodiments of Formula BB, X² is CH.

In some embodiments of Formula BB, X² is CR¹ or CR².

In some embodiments of Formula BB, X³ is CR¹ or CR².

In some embodiments of Formula BB, X³ is NR¹ or NR².

In some embodiments of Formula BB, X⁴ is N.

In some embodiments of Formula BB, X⁴ is S.

In some embodiments of Formula BB, X¹ is CR¹, X² is CR¹, X³ is NR², and X⁴ is N.

In some embodiments of Formula BB-1, X¹ is CR¹, X² is CH, X³ is NR², and X⁴ is N.

In some embodiments of Formula BB-1, X¹ is CH, X² is CR¹, X³ is NR², and X⁴ is N.

In some embodiments of Formula BB, X¹ is CR¹, X² is N, X³ is CR², and X⁴ is S.

In some embodiments of Formula BB, X¹ is CH, X² is N, X³ is CR², and X⁴ is S.

In some embodiments of Formula BB, X¹ is S, X² is CR¹, X³ is CR², and X⁴ is CR¹.

In some embodiments of Formula BB, X¹ is S, X² is CR¹, X³ is CH, and X⁴ is CR².

In some embodiments of Formula BB, X¹ is CR¹, X² is NR², X³ is N, and X⁴ is CR¹.

In some embodiments of Formula BB, R¹ and R² are each independently selected from C₁-C₆ alkyl, C₁-C₆ haloalkyl, halo, and C(O)R¹³ (e.g., C₁-C₆ alkyl, C₁-C₆ haloalkyl, and halo),
wherein the C₁-C₆ alkyl is optionally substituted with one or more substituents each independently selected from hydroxy or R¹⁵ (e.g., hydroxyl);
or one pair of R¹ and R² on adjacent atoms, taken together with the atoms connecting them, independently form at least one monocyclic 5- to-12-membered heterocyclic ring wherein:
   a) when each of the adjacent atoms is a carbon atom, then the heterocyclic ring includes 1 oxygen atom; and
   b) when one or both of the adjacent atoms is/are a nitrogen atom(s), then the heterocyclic ring includes from 0-1 oxygen atoms (in addition to the aforementioned nitrogen atom(s) attached to R¹ and/or R²), and
wherein the heterocyclic ring is optionally independently substituted with one or more substituents each independently selected from hydroxy, C₁-C₆ alkoxy, and NR⁸R⁹.

In some embodiments of Formula BB, R¹ and R² are each independently selected from methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, fluoro, and acetyl (e.g., methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, and fluoro),
or one pair of R¹ and R² on adjacent atoms, taken together with the atoms connecting them, independently form at least one monocyclic 6-membered heterocyclic ring wherein:
   a) when each of the adjacent atoms is a carbon atom, then the heterocyclic ring includes 1 oxygen atom; and
   b) when one or both of the adjacent atoms is/are a nitrogen atom(s), then the heterocyclic ring includes from 0-1 oxygen atoms (in addition to the aforementioned nitrogen atom(s) attached to R¹ and/or R²), and
wherein the heterocyclic ring is optionally independently substituted with one or more substituents each independently selected from hydroxy, methoxy, and methylamino.

In some embodiments of Formula BB, R¹ and R² are each independently selected from methyl, ethyl, isopropyl, 1,2-dihydroxy-2-propyl, 2-hydroxy-2-propyl, fluoromethyl, difluoromethyl, and fluoro.

In some embodiments of Formula BB, R¹ and R² are each independently selected from ethyl, 1,2-dihydroxy-2-propyl, and fluoro.

In some embodiments of Formula BB, o is 1 and p is 2.

In some embodiments of Formula BB, o is 2 and p is 1.

In some embodiments of Formula BB, o is 2 and p is 2.

In some embodiments of Formula BB, R⁶ and R⁷ are each independently selected from C₁-C₆ alkyl, C₁-C₆ haloalkyl, C₆-C₁₀ aryl, CO₂C₁-C₆ alkyl, and C₃-C₁₀ cycloalkyl,
wherein R⁶ and R⁷ are each optionally substituted with one or more substituents independently selected from halo and C₁-C₆ alkyl;
or at least one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, oxo, and C₁-C₆ alkyl.

In some embodiments of Formula BB, R⁶ and R⁷ are each independently selected from C₁-C₆ alkyl, C₁-C₆ haloalkyl, CO₂C₁-C₆ alkyl, and C₃-C₁₀ cycloalkyl,
wherein R⁶ and R⁷ are each optionally substituted with one or more substituents independently selected from halo and C₁-C₆ alkyl;
or at least one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, oxo, and C₁-C₆ alkyl.

In some embodiments of Formula BB, R⁶ and R⁷ are each independently selected from methyl, ethyl, isopropyl, trifluoromethyl, difluoromethyl, 2,2,2-trifluoroethyl, phenyl, CO₂Et, and cyclopropyl,
wherein R⁶ and R⁷ are each optionally substituted with one or more substituents independently selected from fluoro and methyl;
or at least one pair of R⁶ and R⁷ on adjacent atoms, taken together with the atoms connecting them, independently form at least one C₅ carbocyclic ring, wherein the carbocyclic ring is optionally independently substituted with one or more substituents independently selected from hydroxy, oxo, and methyl.

In some embodiments of Formula BB, R⁶ and R⁷ are each independently selected from methyl, ethyl, isopropyl, trifluoromethyl, difluoromethyl, 2,2,2-trifluoroethyl, and cyclopropyl;
or at least one pair of R⁶ and R⁷ on adjacent atoms, taken together with the atoms connecting them, independently form at least one C₅ carbocyclic ring, wherein the carbocyclic ring is optionally independently substituted with one or more methyl.

In some embodiments of Formula BB, R⁶ and R⁷ are each independently selected from methyl and trifluoromethyl;
or at least one pair of R⁶ and R⁷ on adjacent atoms, taken together with the atoms connecting them, independently form at least one C₅ carbocyclic ring, wherein the carbocyclic ring is optionally independently substituted with one or more methyl.

In some embodiments, the compound of Formula AA is a compound of Formula BB-1 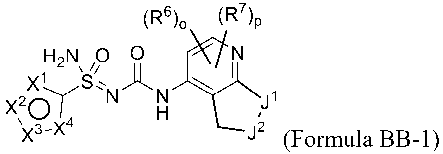 wherein
J¹ and J² are each independently selected from the group consisting of -CH₂-, 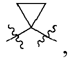 -CHR-, - C(=O)-, and -CR₂-;
each R is independently selected from hydroxy and C₁-C₆ alkyl;
o is 0 or land p is 0 or 1, wherein the sum of o and p is 1 or 2;
X¹ is selected from CH and CR¹; and
X², X³, X⁴, R⁶, and R⁷ are as defined previously herein.

In some embodiments of Formula BB-1, X¹ is CH.

In some embodiments of Formula BB-1, X¹ is CR¹.

In some embodiments of Formula BB-1, X² is N.

In some embodiments of Formula BB-1, X² is CH.

In some embodiments of Formula BB-1, X² is CR¹ or CR².

In some embodiments of Formula BB-1, X³ is CR¹ or CR².

In some embodiments of Formula BB-1, X³ is NR¹ or NR².

In some embodiments of Formula BB-1, X⁴ is N.

In some embodiments of Formula BB-1, X⁴ is S.

In some embodiments of Formula BB-1, X¹ is CR¹, X² is CR¹, X³ is NR², and X⁴ is N.

In some embodiments of Formula BB-1, X¹ is CR¹, X² is CH, X³ is NR², and X⁴ is N.

In some embodiments of Formula BB-1, X¹ is CH, X² is CR¹, X³ is NR², and X⁴ is N.

In some embodiments of Formula BB, X¹ is CR¹, X² is N, X³ is CR², and X⁴ is S.

In some embodiments of Formula BB-1, X¹ is CH, X² is N, X³ is CR², and X⁴ is S.

In some embodiments of Formula BB-1, X¹ is S, X² is CR¹, X³ is CR², and X⁴ is CR¹.

In some embodiments of Formula BB, X¹ is S, X² is CR¹, X³ is CH, and X⁴ is CR².

In some embodiments of Formula BB-1, X¹ is CR¹, X² is NR², X³ is N, and X⁴ is CR¹.

In some embodiments of Formula BB-1, R¹ and R² are each independently selected from C₁-C₆ alkyl, C₁-C₆ haloalkyl, halo, and C(O)R¹³ (e.g., C₁-C₆ alkyl, C₁-C₆ haloalkyl, and halo), wherein the C₁-C₆ alkyl is optionally substituted with one or more substituents each independently selected from hydroxyl or R¹⁵ (e.g., hydroxyl);
or one pair of R¹ and R² on adjacent atoms, taken together with the atoms connecting them, independently form at least one monocyclic 5- to-12-membered heterocyclic ring wherein:
   a) when each of the adjacent atoms is a carbon atom, then the heterocyclic ring includes 1 oxygen atom; and
   b) when one or both of the adjacent atoms is/are a nitrogen atom(s), then the heterocyclic ring includes from 0-1 oxygen atoms (in addition to the aforementioned nitrogen atom(s) attached to R¹ and/or R²), and
wherein the heterocyclic ring is optionally independently substituted with one or more substituents each independently selected from hydroxy, C₁-C₆ alkoxy, and NR⁸R⁹.

In some embodiments of Formula BB-1, R¹ and R² are each independently selected from methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, fluoro, and acetyl (e.g., methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, and fluoro),
or one pair of R¹ and R² on adjacent atoms, taken together with the atoms connecting them, independently form at least one monocyclic 6-membered heterocyclic ring wherein:
   a) when each of the adjacent atoms is a carbon atom, then the heterocyclic ring includes 1 oxygen atom; and
   b) when one or both of the adjacent atoms is/are a nitrogen atom(s), then the heterocyclic ring includes from 0-1 oxygen atoms (in addition to the aforementioned nitrogen atom(s) attached to R¹ and/or R²), and
wherein the heterocyclic ring is optionally independently substituted with one or more substituents each independently selected from hydroxy, methoxy, and methylamino.

In some embodiments of Formula BB-1, R¹ and R² are each independently selected from methyl, ethyl, isopropyl, 1,2-dihydroxy-2-propyl, 2-hydroxy-2-propyl, fluoromethyl, difluoromethyl, and fluoro.

In some embodiments of Formula BB-1, R¹ and R² are each independently selected from ethyl, 1,2-dihydroxy-2-propyl, and fluoro.

In some embodiments of Formula BB-1, o is 1 and p is 0.

In some embodiments of Formula BB-1, o is 0 and p is 1.

In some embodiments of Formula BB-1, o is 1 and p is 1.

In some embodiments of Formula BB-1, R⁶ and R⁷ are each independently selected from C₁-C₆ alkyl, C₁-C₆ haloalkyl, C₆-C₁₀ aryl, CO₂C₁-C₆ alkyl, and C₃-C₁₀ cycloalkyl,
wherein R⁶ and R⁷ are each optionally substituted with one or more substituents independently selected from halo and C₁-C₆ alkyl;
or at least one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, oxo, and C₁-C₆ alkyl.

In some embodiments of Formula BB-1, R⁶ and R⁷ are each independently selected from C₁-C₆ alkyl, C₁-C₆ haloalkyl, CO₂C₁-C₆ alkyl, and C₃-C₁₀ cycloalkyl,
wherein R⁶ and R⁷ are each optionally substituted with one or more substituents independently selected from halo and C₁-C₆ alkyl;
or at least one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, oxo, and C₁-C₆ alkyl.

In some embodiments of Formula BB-1, R⁶ and R⁷ are each independently selected from methyl, ethyl, isopropyl, trifluoromethyl, difluoromethyl, 2,2,2-trifluoroethyl, phenyl, CO₂Et, and cyclopropyl,
wherein R⁶ and R⁷ are each optionally substituted with one or more substituents independently selected from fluoro and methyl;
or at least one pair of R⁶ and R⁷ on adjacent atoms, taken together with the atoms connecting them, independently form at least one C₅ carbocyclic ring, wherein the carbocyclic ring is optionally independently substituted with one or more substituents independently selected from hydroxy, oxo, and methyl.

In some embodiments of Formula BB-1, R⁶ and R⁷ are each independently selected from methyl, ethyl, isopropyl, trifluoromethyl, difluoromethyl, 2,2,2-trifluoroethyl, and cyclopropyl;
or at least one pair of R⁶ and R⁷ on adjacent atoms, taken together with the atoms connecting them, independently form at least one C₅ carbocyclic ring, wherein the carbocyclic ring is optionally independently substituted with one or more methyl.

In some embodiments of Formula BB-1, R⁶ and R⁷ are each independently selected from methyl and trifluoromethyl;
or at least one pair of R⁶ and R⁷ on adjacent atoms, taken together with the atoms connecting them, independently form at least one C₅ carbocyclic ring, wherein the carbocyclic ring is optionally independently substituted with one or more methyl.

In some embodiments of Formula BB-1, J¹ is selected from the group consisting of -CH₂- 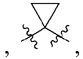 -CHR-, -C(=O)-, and -CR₂ ; and J² is -CH₂-. In any of these embodiments, R is C₁-C₆ alkyl (e.g., methyl).

In some embodiments of Formula BB-1, J¹ is selected from the group consisting of -CH₂- and -CR₂-; and J² is -CH₂-. In any of these embodiments, R is C₁-C₆ alkyl (e.g., methyl).

In some embodiments of Formula BB-1, R is C₁-C₆ alkyl (e.g., methyl).

In certain embdoiments, J¹ is -CH₂-; and J² is -CH₂-.

In some embodiments, the compound of Formula BB is a compound of Formula BB-la or Formula BB-1b 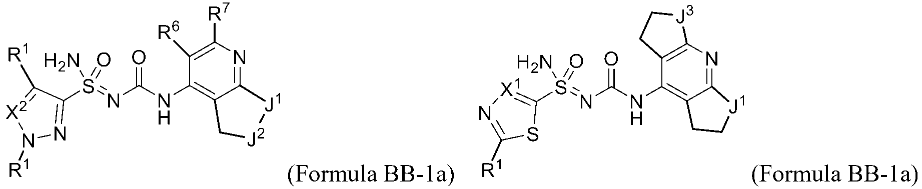
J¹, J², and J³ are each independently selected from the group consisting of -CH₂-, 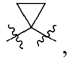 -CHR-,-CR₂-, and C(=O);
each R is independently selected from hydroxy and C₁-C₆ alkyl;
X¹ is selected from CH and CR¹;
X² is selected from N, CH, CR¹, and CR²;
   R⁶ and R⁷ are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
   wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl.

In some embodiments of Formula BB-la, X² is N.

In some embodiments of Formula BB-la, X² is CH.

In some embodiments of Formula BB-la, X² is CR¹ or CR².

In some embodiments of Formula BB-1b, X¹ is selected from CH;

In some embodiments of Formula BB-lb, X¹ is selected from CR¹;

In some embodiments of Formula BB-la and/or Formula BB-lb, each R¹ is independently selected from C₁-C₆ alkyl, C₁-C₆ haloalkyl, halo, and C(O)R¹³ (e.g., C₁-C₆ alkyl, C₁-C₆ haloalkyl, and halo),
wherein the C₁-C₆ alkyl is optionally substituted with one or more substituents each independently selected from hydroxyl or R¹⁵ (e.g., hydroxyl).

In some embodiments of Formula BB-la and/or Formula BB-1b, each R¹ is independently selected from methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, fluoro, and acetyl (e.g., methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, and fluoro).

In some embodiments of Formula BB-la and/or Formula BB-1b, each R¹ is independently selected from methyl, ethyl, isopropyl, 1,2-dihydroxy-2-propyl, 2-hydroxy-2-propyl, fluoromethyl, difluoromethyl, and fluoro.

In some embodiments of Formula BB-la and/or Formula BB-1b, each R¹ is independently selected from ethyl, 1,2-dihydroxy-2-propyl, and fluoro.

In some embodiments of Formula BB-la, the R¹ connected to carbon is halo; X² is CH; and the R¹ connected to nitrogen is C₁-C₃ alkyl. For example, the R¹ connected to carbon is fluoro; X² is CH; and the R¹ connected to nitrogen is ethyl.

In some embodiments of Formula BB-1b, X¹ is CH; and R¹ is C₁-C₃ alkyl optionally substituted with one or more hydroxyl. For example, X¹ is CH; and R¹ is 1,2-dihydroxy-2-propyl.

In some embodiments of any of the foregoing embodiments, the 1,2-dihydroxy-2-propyl has an (R) configuration at the 2-position. In other embodiments of any of the foregoing embodiments, the 1,2-dihydroxy-2-propyl has an (S) configuration at the 2-position.

In some embodiments of Formula BB-la and/or Formula BB-lb, each R² is independently selected from C₁-C₆ alkyl, C₁-C₆ haloalkyl, halo, and C(O)R¹³ (e.g., C₁-C₆ alkyl, C₁-C₆ haloalkyl, and halo),
wherein the C₁-C₆ alkyl is optionally substituted with one or more substituents each independently selected from hydroxyl or R¹⁵ (e.g., hydroxyl).

In some embodiments of Formula BB-la and/or Formula BB-lb, each R² is independently selected from methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, fluoro, and acetyl (e.g., methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, and fluoro).

In some embodiments of Formula BB-la and/or Formula BB-lb, each R² is independently selected from methyl, ethyl, isopropyl, 1,2-dihydroxy-2-propyl, 2-hydroxy-2-propyl, fluoromethyl, difluoromethyl, and fluoro.

In some embodiments of Formula BB-la and/or Formula BB-lb, each R² is independently selected from ethyl, 1,2-dihydroxy-2-propyl, and fluoro.

In some embodiments of Formula BB-la, R⁶ and R⁷ are each independently selected from C₁-C₆ alkyl, C₁-C₆ haloalkyl, C₆-C₁₀ aryl, CO₂C₁-C₆ alkyl, and C₃-C₁₀ cycloalkyl,
wherein R⁶ and R⁷ are each optionally substituted with one or more substituents independently selected from halo and C₁-C₆ alkyl.

In some embodiments of Formula BB-la, R⁶ and R⁷ are each independently selected from C₁-C₆ alkyl, C₁-C₆ haloalkyl, CO₂C₁-C₆ alkyl, and C₃-C₁₀ cycloalkyl,
wherein R⁶ and R⁷ are each optionally substituted with one or more substituents independently selected from halo and C₁-C₆ alkyl.In some embodiments of Formula BB-la, R⁶ and R⁷ are each independently selected from methyl, ethyl, isopropyl, trifluoromethyl, difluoromethyl, 2,2,2-trifluoroethyl, phenyl, CO₂Et, and cyclopropyl,
wherein R⁶ and R⁷ are each optionally substituted with one or more substituents independently selected from fluoro and methyl.

In some embodiments of Formula BB-la, R⁶ and R⁷ are each independently selected from methyl, ethyl, isopropyl, trifluoromethyl, difluoromethyl, 2,2,2-trifluoroethyl, and cyclopropyl.

In some embodiments of Formula BB-la, R⁶ and R⁷ are each independently selected from methyl and trifluoromethyl. For example, R⁶ is methyl; and R⁷ is trifluoromethyl.

In some embodiments of Formula BB-la, J¹ and J² are independently selected from -CH₂-, -CHR-, and -CR₂-.

In some embodiments of Formula BB-la, J¹ is selected from -CH₂-, -CHR-, and -CR₂-; and J² is -CH₂-.

In some embodiments of Formula BB-la, J¹ is selected from -CH₂- and -CR₂-; and J² is - CH₂-.

In some embodiments of Formula BB-1b, J¹ and J³ are independently selected from the group consisting of -CH₂-, 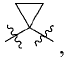 -CHR-, -CR₂-, and -C(=O)-. In any of these embodiments, R is C₁-C₆ alkyl (e.g., methyl).

In some embodiments of Formula BB-lb, J¹ and J³ are independently selected from the group consisting of -CH₂- and -CR₂-.

In some embodiments of Formula BB-lb, one or two of J¹ and J³ is other than CH₂.

In some embodiments of Formula BB-lb, J¹ is 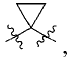 -CHR-, -CR₂-, or -C(=O)- (e.g., 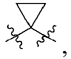 -CHR-, -CR₂-); and J³ is CH₂.

In some embodiments of Formula BB-la and/or Formula BB-1b, R is C₁-C₆ alkyl (e.g., methyl).

In some embodiments, the compound of Formula BB is a compound of Formula BB-la.

In some embodiments, the compound of Formula BB is a compound of Formula BB-lb.

In some embodiments, the compound of Formula BB is a compound of Formula BB-la-i or Formula BB-lb-i 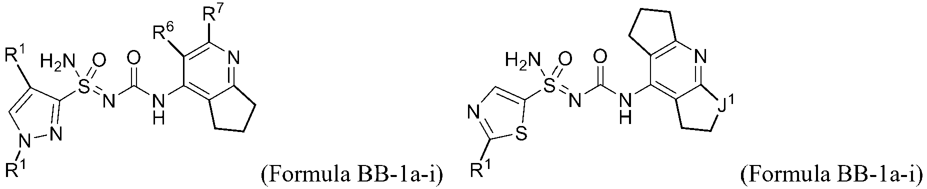
J¹ is selected from the group consisting of -CH₂-, 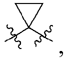 -CHR-, -CR₂-, and -C(=O)-;
each R is independently selected from hydroxy and C₁-C₆ alkyl;
   R⁶ and R⁷ are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
   wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
   wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl.

In some embodiments of Formula BB-la-i and/or Formula BB-1b-i, each R¹ is independently selected from C₁-C₆ alkyl, C₁-C₆ haloalkyl, halo, and C(O)R¹³ (e.g., C₁-C₆ alkyl, C₁-C₆ haloalkyl, and halo)
wherein the C₁-C₆ alkyl is optionally substituted with one or more substituents each independently selected from hydroxyl or R¹⁵ (e.g., hydroxyl).

In some embodiments of Formula BB-la-i and/or Formula BB-1b-i, each R¹ is independently selected from methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, fluoro, and acetyl (e.g., methyl, ethyl, isopropyl, hydroxymethyl, hydroxyethyl, 1,2-dihydroxy-2-propyl, 2-hydroxy-2-propyl, 2-hydroxyethyl, 1,2,3-trihydroxy-2-propyl, fluoromethyl, difluoromethyl, and fluoro).

In some embodiments of Formula BB-la-i and/or Formula BB-1b-i, each R¹ is independently selected from methyl, ethyl, isopropyl, 1,2-dihydroxy-2-propyl, 2-hydroxy-2-propyl, fluoromethyl, difluoromethyl, and fluoro.

In some embodiments of Formula BB-la-i and/or Formula BB-1b-i, each R¹ is independently selected from ethyl, 1,2-dihydroxy-2-propyl, and fluoro.

In some embodiments of Formula BB-la-i, the R¹ connected to carbon is halo; and the R¹ connected to nitrogen is C₁-C₃ alkyl.

In some embodiments of Formula BB-1b-i, R¹ is C₁-C₃ alkyl optionally substituted with one or more hydroxyl.

In some embodiments of Formula BB-la-i, R⁶ and R⁷ are each independently selected from C₁-C₆ alkyl, C₁-C₆ haloalkyl, C₆-C₁₀ aryl, CO₂C₁-C₆ alkyl, and C₃-C₁₀ cycloalkyl,
wherein R⁶ and R⁷ are each optionally substituted with one or more substituents independently selected from halo and C₁-C₆ alkyl.

In some embodiments of Formula BB-la-i, R⁶ and R⁷ are each independently selected from C₁-C₆ alkyl, C₁-C₆ haloalkyl, CO₂C₁-C₆ alkyl, and C₃-C₁₀ cycloalkyl,
wherein R⁶ and R⁷ are each optionally substituted with one or more substituents independently selected from halo and C₁-C₆ alkyl.

In some embodiments of Formula BB-la-i, R⁶ and R⁷ are each independently selected from methyl, ethyl, isopropyl, trifluoromethyl, difluoromethyl, 2,2,2-trifluoroethyl, phenyl, CO₂Et, and cyclopropyl,
wherein R⁶ and R⁷ are each optionally substituted with one or more substituents independently selected from fluoro and methyl.

In some embodiments of Formula BB-la-i, R⁶ and R⁷ are each independently selected from methyl, ethyl, isopropyl, trifluoromethyl, difluoromethyl, 2,2,2-trifluoroethyl, and cyclopropyl,
wherein R⁶ and R⁷ are each optionally substituted with one or more substituents independently selected from fluoro and methyl.

In some embodiments of Formula BB-la-i, R⁶ and R⁷ are each independently selected from methyl, ethyl, isopropyl, trifluoromethyl, difluoromethyl, 2,2,2-trifluoroethyl, and cyclopropyl.

In some embodiments of Formula BB-la-i, R⁶ and R⁷ are each independently selected from methyl and trifluoromethyl.

In some embodiments of Formula BB-1b, J¹ is selected from the group consisting of 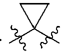 , -CHR-, and -CR₂-.

In some embodiments of Formula BB-1b, J¹ is selected from the group consisting of -CHR- and -CR₂-.

In some embodiments, the compound of Formula BB is a compound of Formula BB-la-i.

In some embodiments, the compound of Formula BB is a compound of Formula BB-lb-i.

### Additional Features of the Embodiments Herein

In some embodiments of the compound of Formula AA (e.g., Formula AA-1, Formula AA-2, Formula AA-3, Formula AA-4, or Formula AA-5), R⁶ is not CN.

In some embodiments, the compound of Formula AA is not a compound selected from the group consisting of: 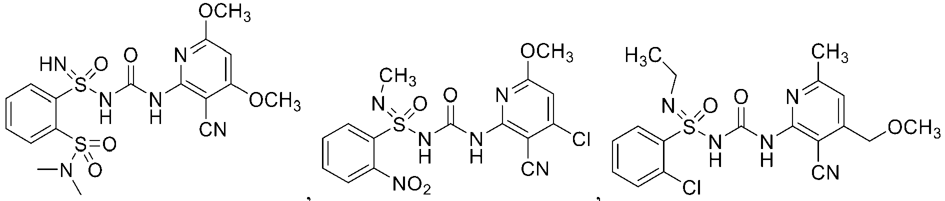 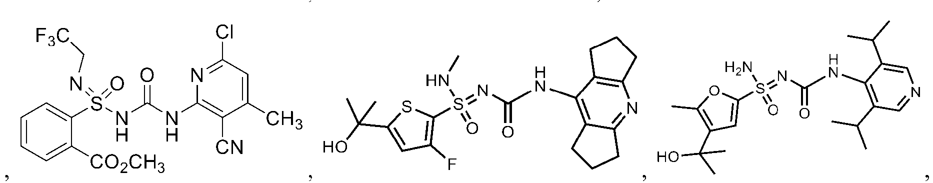 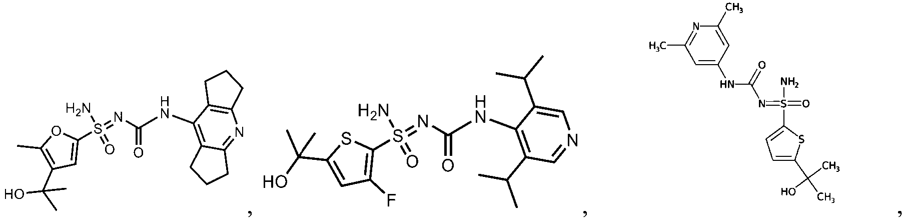 and

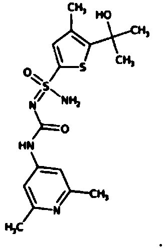

In some embodiments, the compound of Formula AA is not a compound selected from the group consisting of: 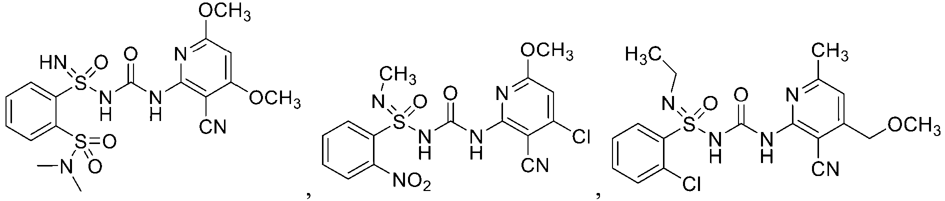 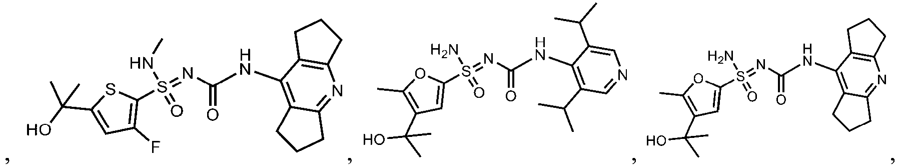 and

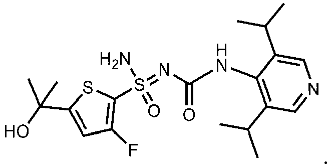

In some embodiments, the compound of Formula AA is not a compound selected from the group consisting of:

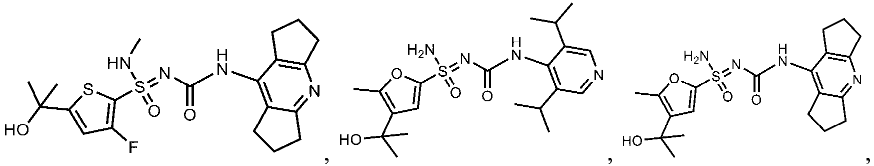

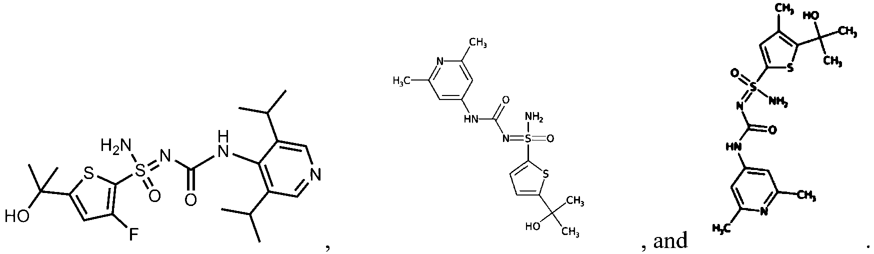

In some embodiments, the compound of Formula AA is not a compound selected from the group consisting of: 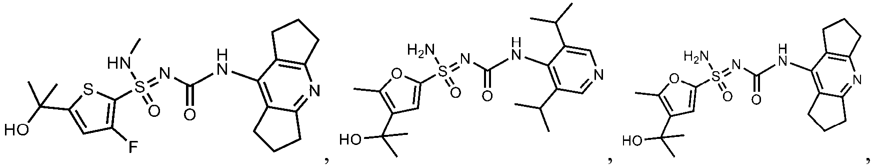 and

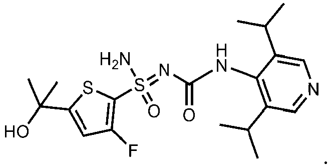

In some embodiments the compound of any of the formulae herein is not a compound disclosed in US Provisional 62/536,271, filed on July 24, 2017; and US Provisional 62/573,894, filed on October 18, 2017, each of which is incorporated herein by reference in its entirety.

In some embodiments the compound of any of the formulae herein is not a compound disclosed in EP 0173498, which is incorporated herein by reference in its entirety.

In some embodiments the compound of any of the formulae herein is not a compound disclosed in US 4666506, which is incorporated herein by reference in its entirety.

In one embodiment, provided herein is a combination of a compound of any preceding embodiemnt, for use in the treatment or the prevention of a condition mediated by TNF-α, in a patient in need thereof, wherein the compound is administered to said patient at a therapeutically effective amount. Preferably, the subject is resistant to treatment with an anti-TNFα agent. Preferably, the condition is a gut disease or disorder.

In one embodiment, provided herein is a pharmaceutical composition of comprising a compound of any preceding embodiment, and an anti-TNFα agent disclosed herein. Preferably wherein the anti-TNFα agent is Infliximab, Etanercept, Certolizumab pegol, Golimumab or Adalimumab, more preferably wherein the anti-TNFα agent is Adalimumab.

In one embodiment, provided herein is a pharmaceutical combination of a compound of any preceding embodiment, and an anti-TNFα agent Preferably wherein the anti-TNFα agent is Infliximab, Etanercept, Certolizumab pegol, Golimumab or Adalimumab, more preferably wherein the anti-TNFα agent is Adalimumab.

In one embodiment, the present invention relates to an NLRP3 antagonist for use in the treatment or the prevention of a condition mediated by TNF-α, in particular a gut disease or disorder, in a patient in need thereof, wherein the NLRP3 antagonist is administered to said patient at a therapeutically effective amount.

In one embodiment, the present invention relates to an NLRP3 antagonist for use in the treatment or the prevention of a condition, in particular a gut disease or disorder, in a patient in need thereof wherein the NLRP3 antagonist is administered to said patient at a therapeutically effective amount.

In one embodiment, the present invention relates to an NLRP3 antagonist for use in the treatment, stabilization or lessening the severity or progression of gut disease or disorder, in a patient in need thereof wherein the NLRP3 antagonist is administered to said patient at a therapeutically effective amount.

In one embodiment, the present invention relates to an NLRP3 antagonist for use in the slowing, arresting, or reducing the development of a gut disease or disorder, in a patient in need thereof wherein the NLRP3 antagonist is administered to said patient at a therapeutically effective amount.

In one embodiment, the present invention relates to an NLRP3 antagonist for use according to above listed embodiments wherein the NLRP3 antagonist is a gut-targeted NLRP3 antagonist.

In one embodiment, the present invention relates ton NLRP3 antagonist for use according to any of the above embodiments, wherein the gut disease is IBD.

In one embodiment, the present invention relates to an NLRP3 antagonist for use according to any of the above embodiments, wherein the gut disease is US or CD.

In one embodiment, the present invention relates to a method for the treatment or the prevention of a condition mediated by TNF-α, in particular a gut disease or disorder, in a patient in need thereof, comprising administering to said patient a therapeutically effective amount of a gut-targeted NLRP3 antagonist.

In one embodiment, the present invention relates to a method for the treatment or the prevention of a condition, in particular a gut disease or disorder, in a patient in need thereof, comprising administering to said patient a therapeutically effective amount of a gut-targeted NLRP3 antagonist.

In one embodiment, the present invention relates to a method for the treatment, stabilization or lessening the severity or progression of gut disease or disorder, in a patient in need thereof comprising administering to said patient a therapeutically effective amount of a gut-targeted NLRP3 antagonist.

In one embodiment, the present invention relates to a method for slowing, arresting, or reducing the development of a gut disease or disorder, in a patient in need thereof comprising administering to said patient a therapeutically effective amount of a gut-targeted NLRP3 antagonist.

In one embodiment, the present invention relates to a method according to any of the above embodiments, wherein the gut disease is IBD.

In one embodiment, the present invention relates to a method according to any of the above embodiments x to xx, wherein the gut disease is UC or CD.

In one embodiment, the present invention relates to a method for the treatment or the prevention of a condition mediated by TNF-α, in particular a gut disease or disorder, in a patient in need thereof, comprising administering to said patient a therapeutically effective amount of a gut-targeted NLRP3 antagonist.

Unless otherwise indicated, when a disclosed compound is named or depicted by a structure without specifying the stereochemistry and has one or more chiral centers, it is understood to represent all possible stereoisomers of the compound.

It is understood that the combination of variables in the formulae herein is such that the compounds are stable.

In some embodiments, provided herein is a compound that is selected from the group consisting of the compounds in Table 1A:

**Table 1A.**

| **Compound #** | **Structure** | **Compound #** | **Structure** |
|---|---|---|---|
| **101** | 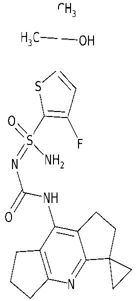 | **141ba** | 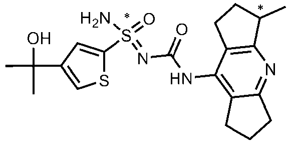 |
| **101a** | 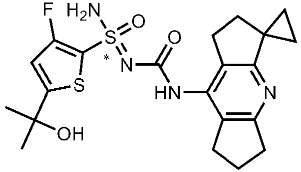 | **141bb** | 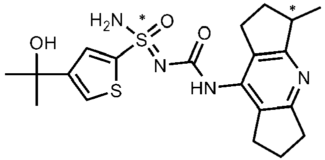 |
| **101b** | 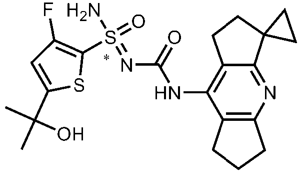 | **142** | 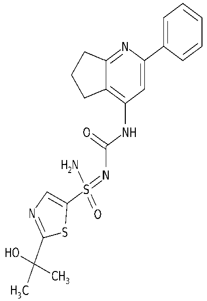 |
| **102** | 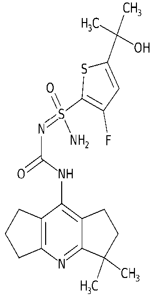 | **142a** | 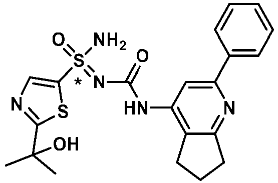 |
| **102a** | 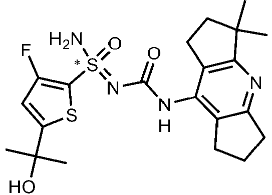 | **142b** | 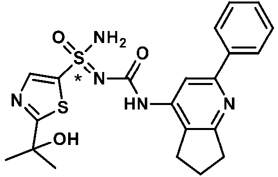 |
| **102b** | 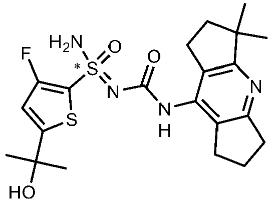 | **143** | 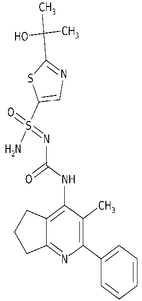 |
| **103** | 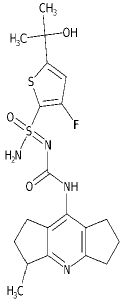 | **143a** | 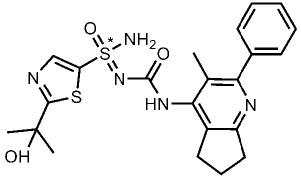 |
| **103aa** | 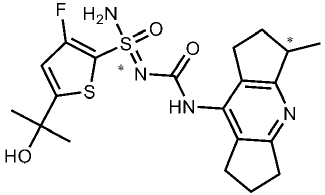 | **143b** | 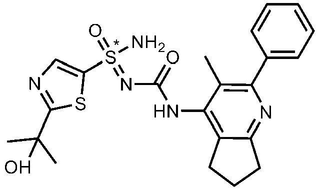 |
| **103ab** | 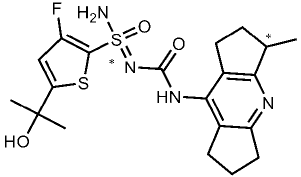 | **144** | 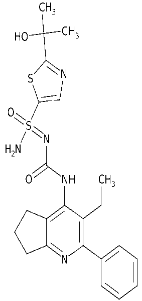 |
| **103ba** | 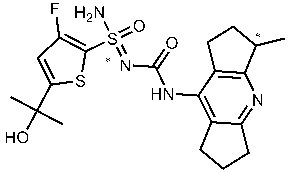 | **145** | 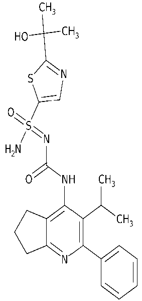 |
| **103bb** | 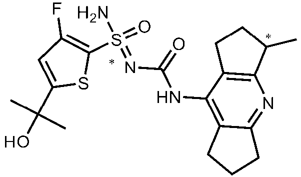 | **145a** | 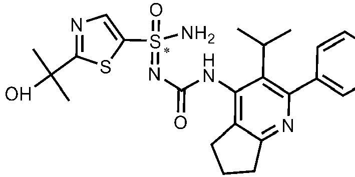 |
| **104** | 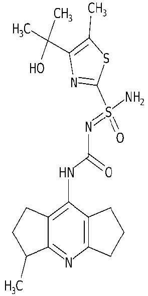 | **145b** | 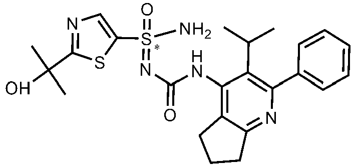 |
| **104a** | 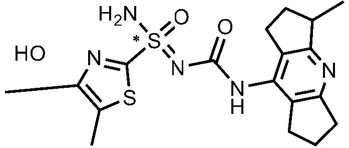 | **146** | 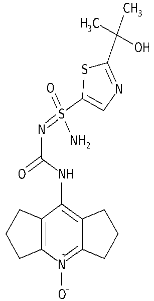 |
| **104b** | 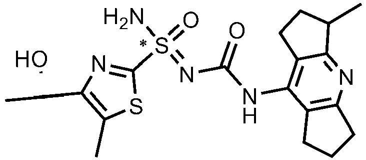 | **147** | 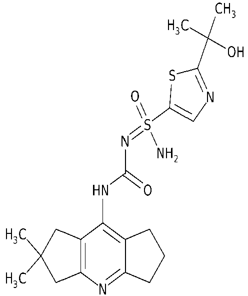 |
| **105** | 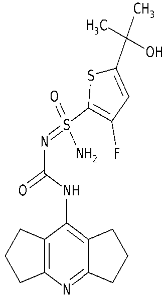 | **147a** | 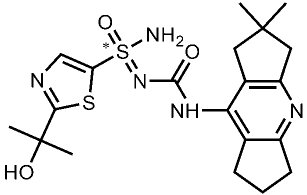 |
| **105a** | 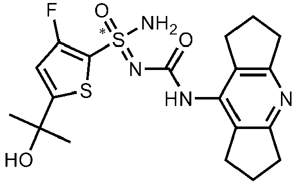 | **147b** | 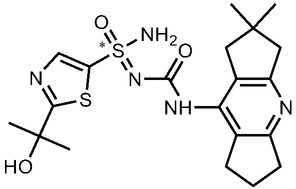 |
| **105b** | 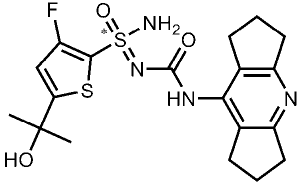 | **148** | 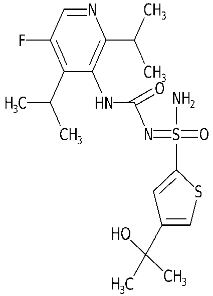 |
| **106** | 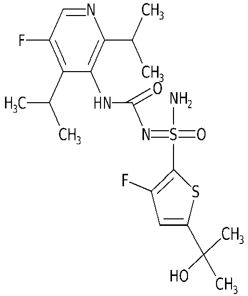 | **148a** | 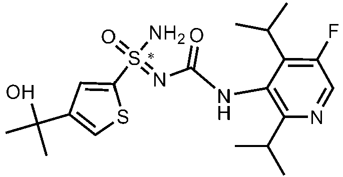 |
| **106a** | 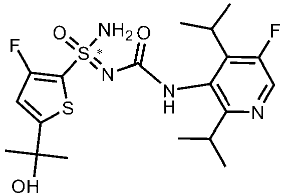 | **148b** | 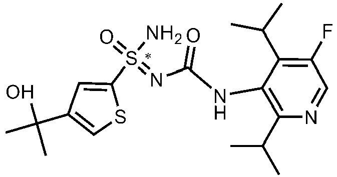 |
| **106b** | 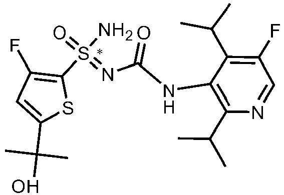 | **149** | 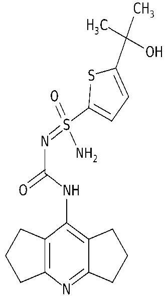 |
| **107** | 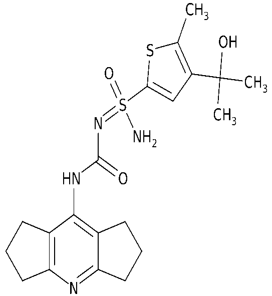 | **149a** | 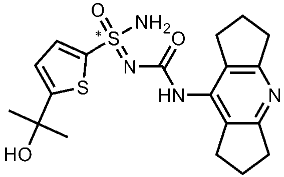 |
| **107a** | 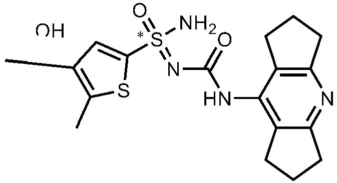 | **149b** | 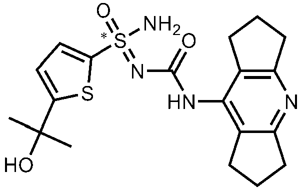 |
| **107b** | 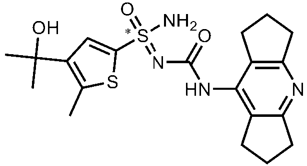 | **150** | 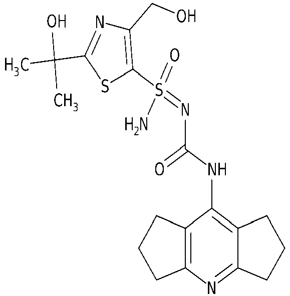 |
| **110** | 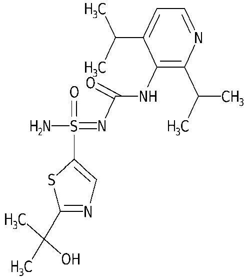 | **150a** | 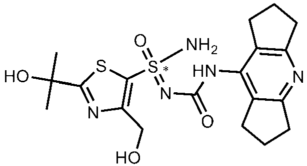 |
| **111** | 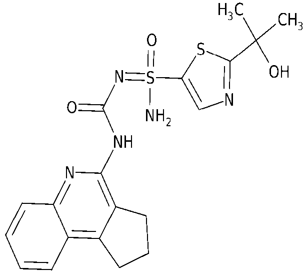 | **150b** | 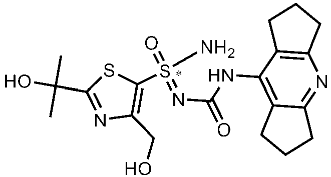 |
| **112** | 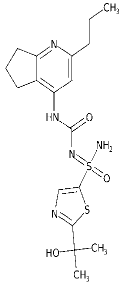 | **151** | 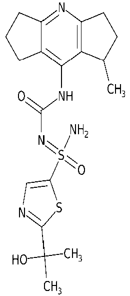 |
| **113** | 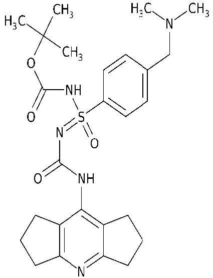 | **152** | 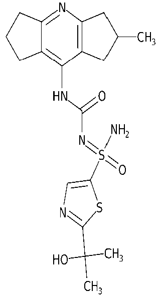 |
| **114** | 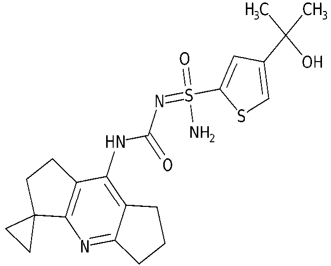 | **153** | 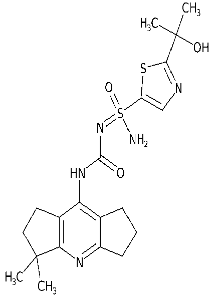 |
| **114a** | 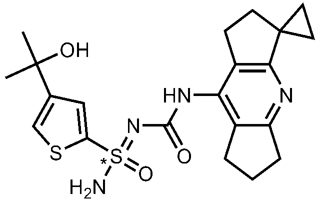 | **153a** | 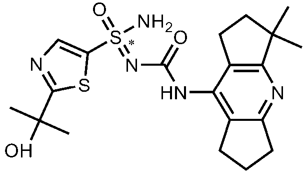 |
| **114b** | 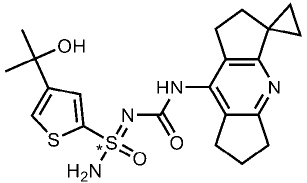 | **153b** | 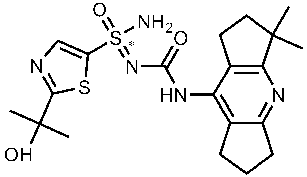 |
| **115** | 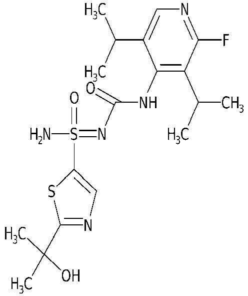 | **154** | 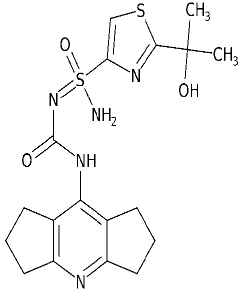 |
| **116** | 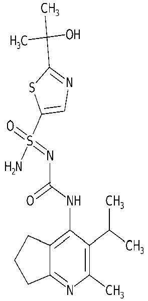 | **154a** | 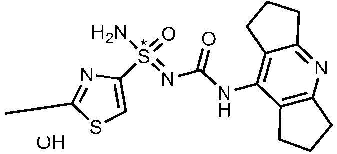 |
| **116a** | 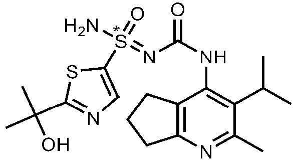 | **154b** | 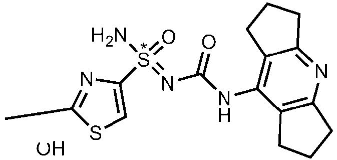 |
| **116b** | 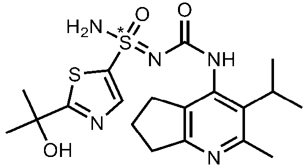 | **155** | 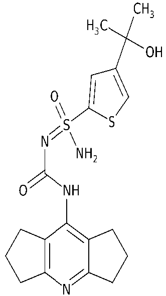 |
| **117** | 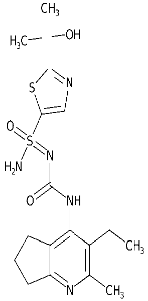 | **155a** | 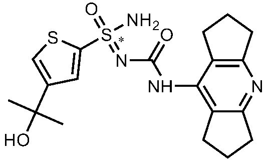 |
| **117a** | 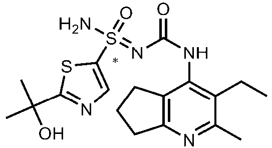 | **155b** | 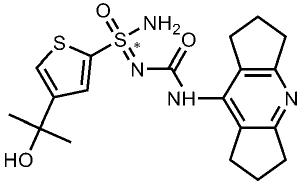 |
| **117b** | 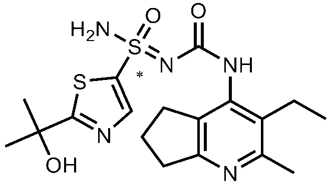 | **156** | 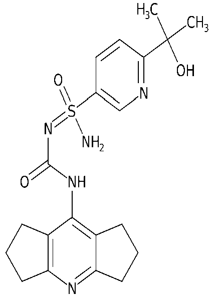 |
| **118** | 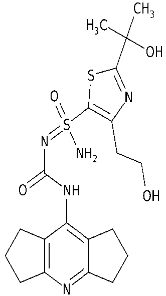 | **156a** | 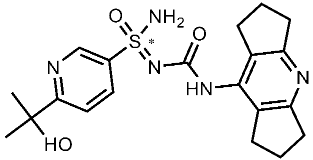 |
| **118a** | 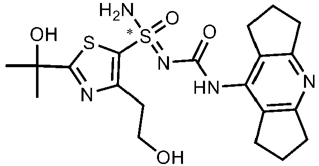 | **156b** | 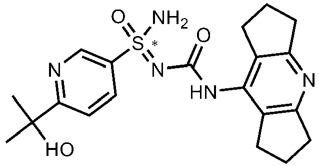 |
| **118b** | 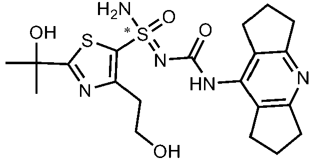 | **157** | 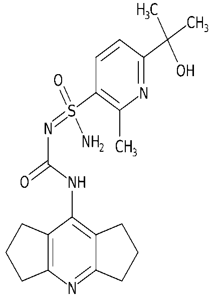 |
| **119** | 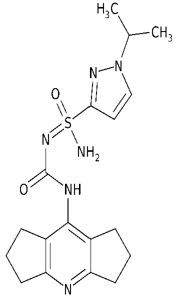 | **157a** | 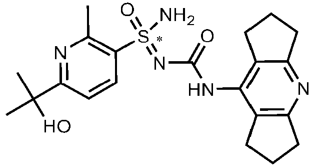 |
| **119a** | 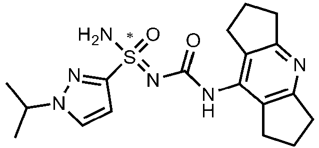 | **157b** | 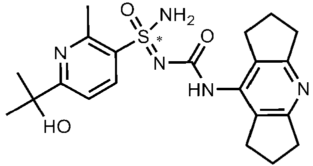 |
| **119b** | 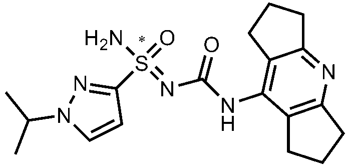 | **158** | 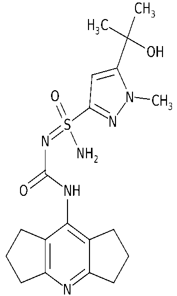 |
| **120** | 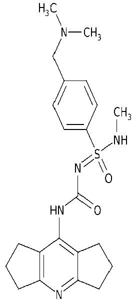 | **158a** | 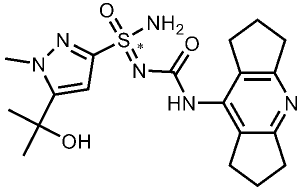 |
| **120a** | 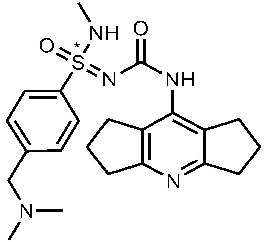 | **158b** | 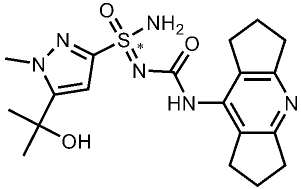 |
| **120b** | 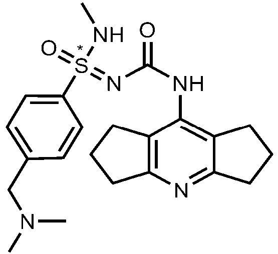 | **159** | 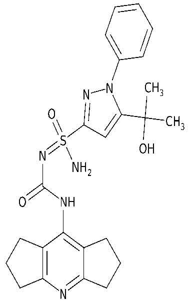 |
| **121** | 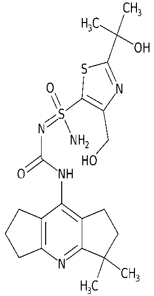 | **159a** | 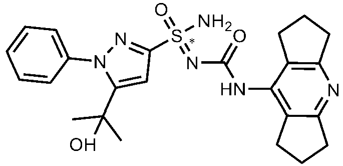 |
| **121a** | 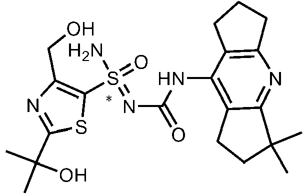 | **159b** | 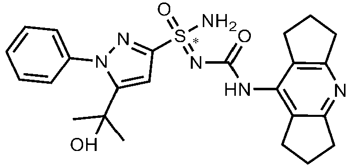 |
| **121b** | 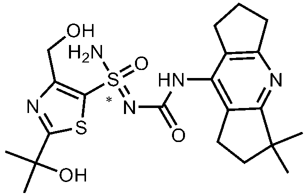 | **160** | 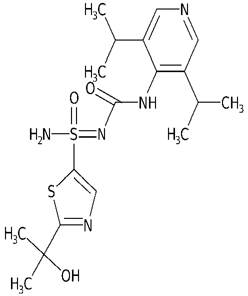 |
| **122** | 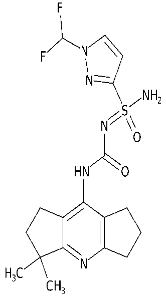 | **160a** | 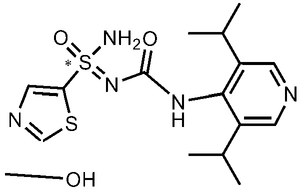 |
| **122a** | 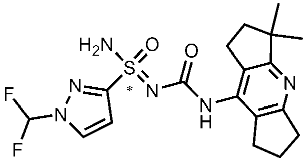 | **160b** | 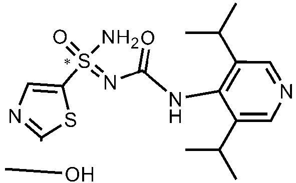 |
| **122b** | 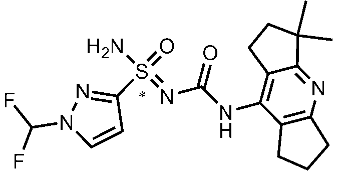 | **161** | 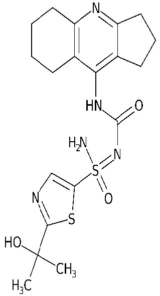 |
| **123** | 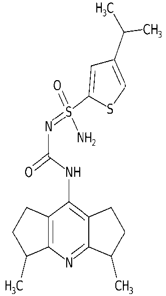 | **161a** | 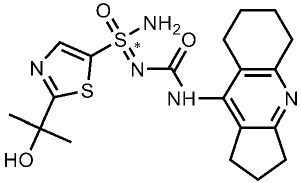 |
| **124** | 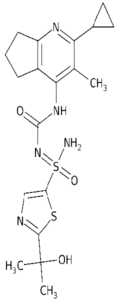 | **161b** | 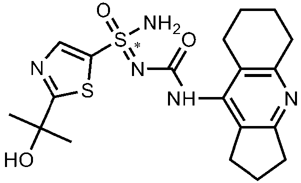 |
| **124a** | 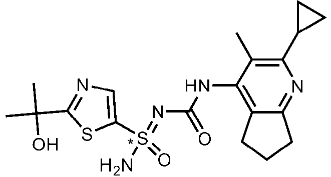 | **162** | 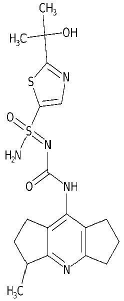 |
| **124b** | 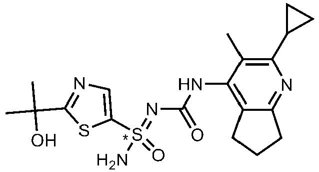 | **162aa** | 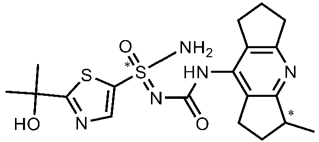 |
| **125** | 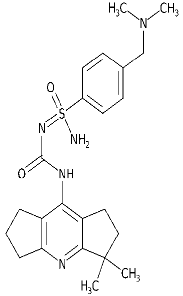 | **162ab** | 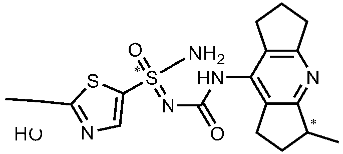 |
| **125a** | 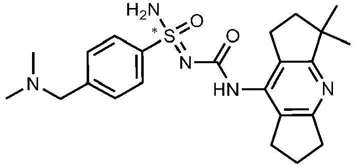 | **162ba** | 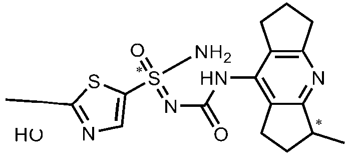 |
| **125b** | 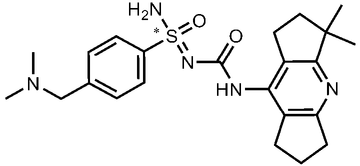 | **162bb** | 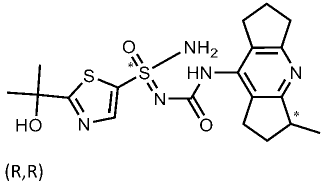 |
| **126** | 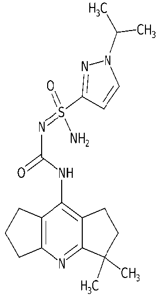 | **163** | 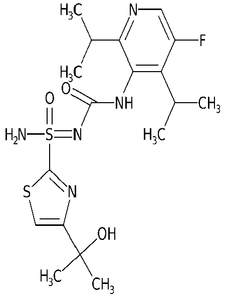 |
| **126a** | 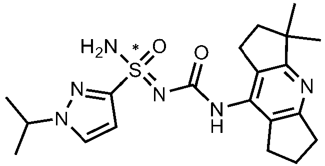 | **163a** | 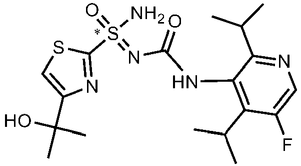 |
| **126b** | 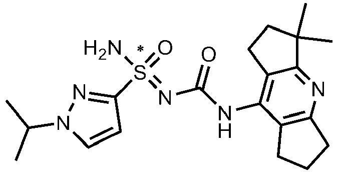 | **163b** | 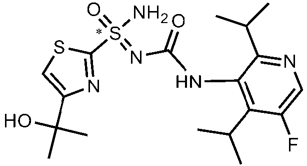 |
| **127** | 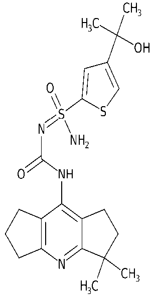 | **164** | 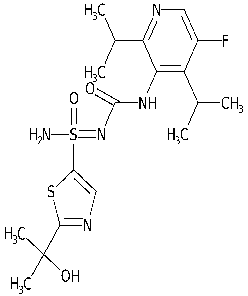 |
| **127a** | 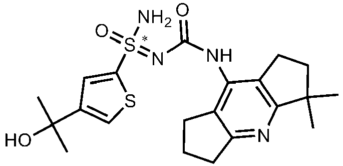 | **164a** | 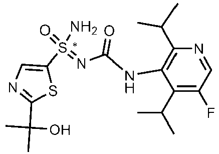 |
| **127b** | 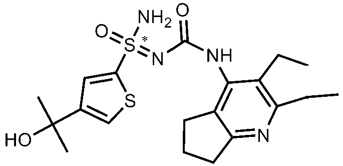 | **164b** | 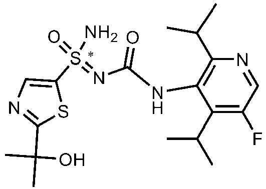 |
| **128** | 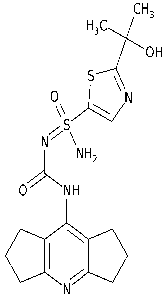 | **165** | 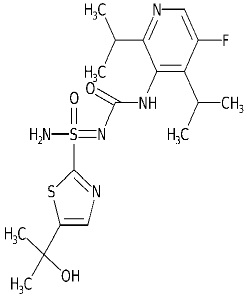 |
| **128a** | 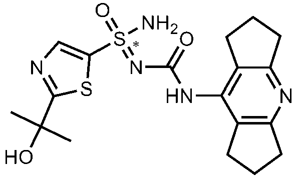 | **165a** | 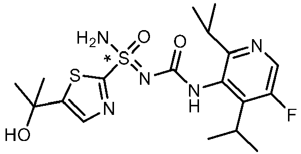 |
| **128b** | 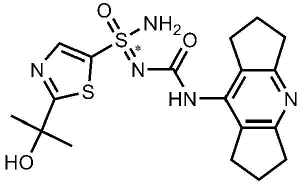 | **165b** | 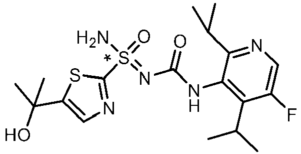 |
| **129** | 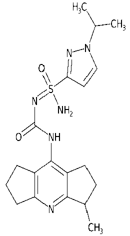 | **166** | 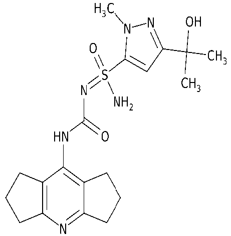 |
| **129aa** | 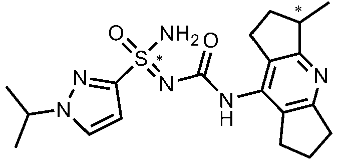 | **167** | 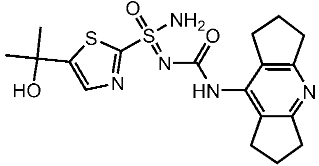 |
| **129ab** | 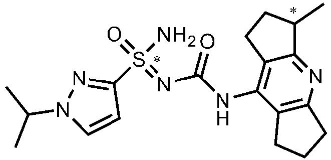 | **167a** | 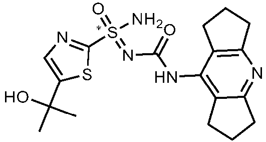 |
| **129ba** | 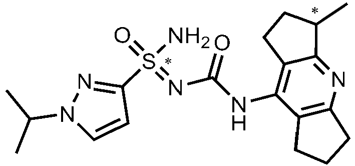 | **167b** | 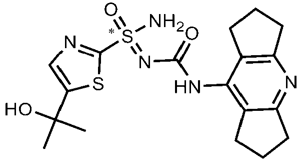 |
| **129bb** | 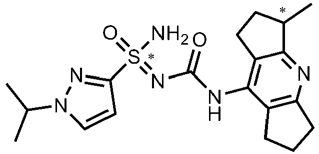 | **168** | 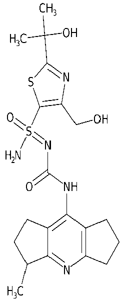 |
| **130** | 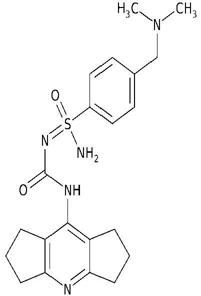 | **168a** | 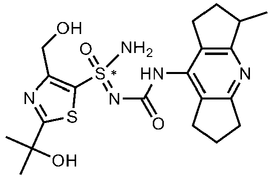 |
| **130a** | | **168aa** | |
| | 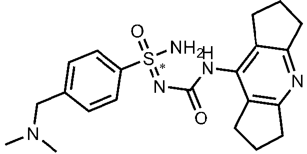 | | 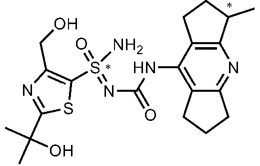 |
| **130b** | 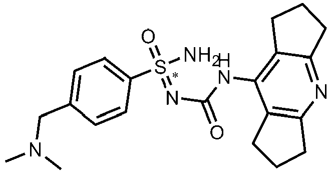 | **168ab** | 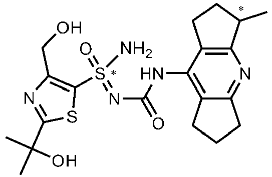 |
| **131** | 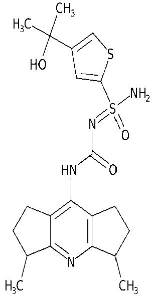 | **168b** | 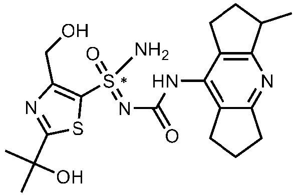 |
| **131a** | 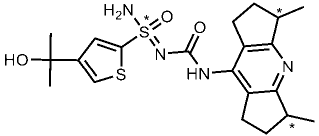 | **168ba** | 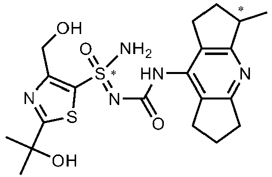 |
| **131aab** | 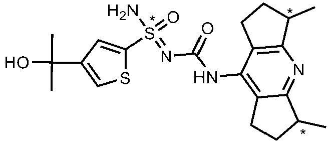 | **168bb** | 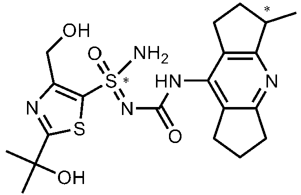 |
| **131b** | | **169a** | |
| | 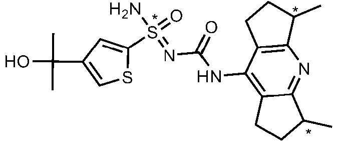 | | 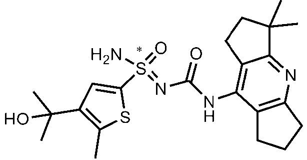 |
| **131c** | 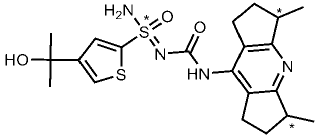 | **169b** | 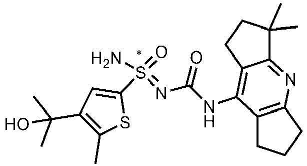 |
| **131d** | 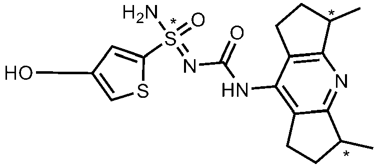 | **170** | 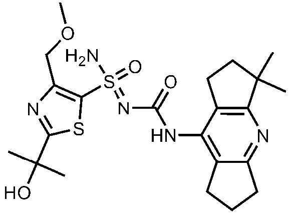 |
| **131e** | 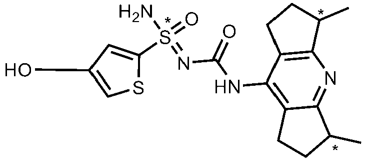 | **171** | 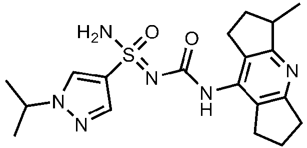 |
| **131f** | 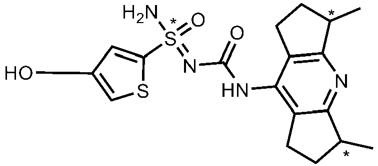 | **172** | 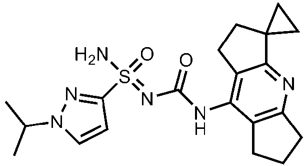 |
| **131g** | 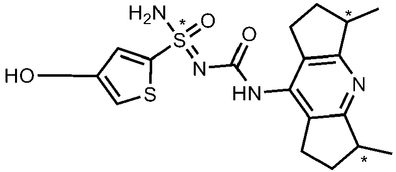 | **172a** | 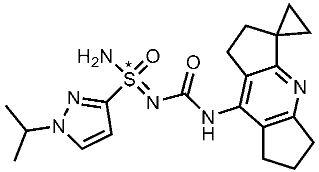 |
| **132** | 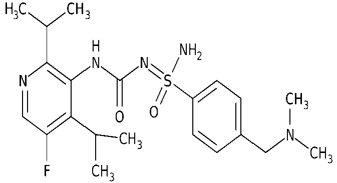 | **172b** | 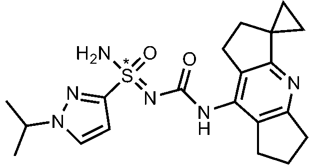 |
| **132a** | 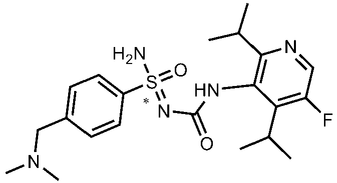 | **173** | 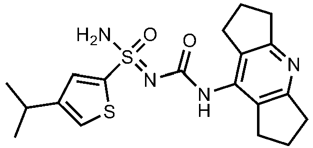 |
| **132b** | 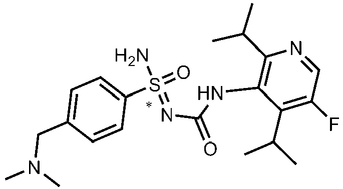 | **173a** | 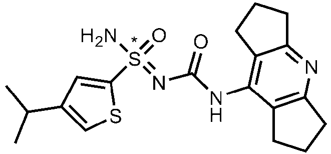 |
| **133** | 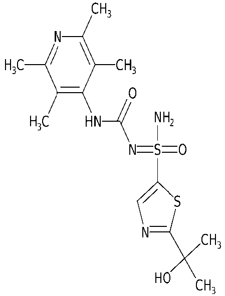 | **173b** | 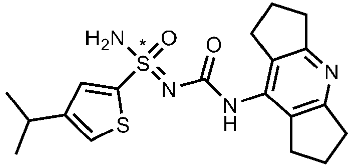 |
| **133a** | 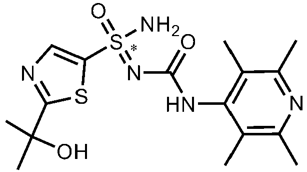 | **174** | 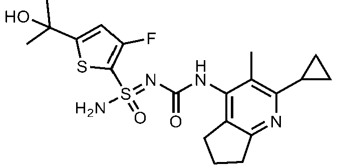 |
| **133b** | 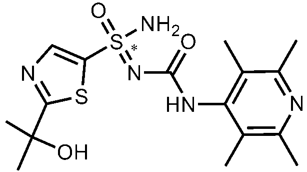 | **174a** | 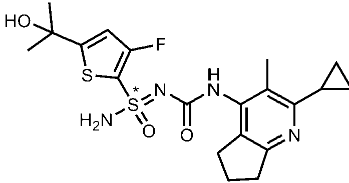 |
| **134** | 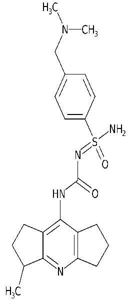 | **174b** | 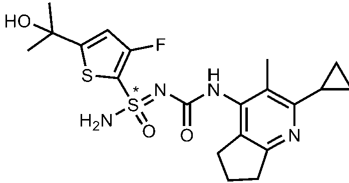 |
| **134aa** | 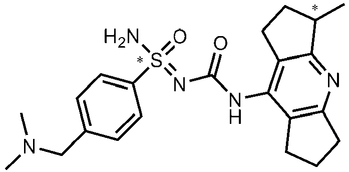 | **175** | 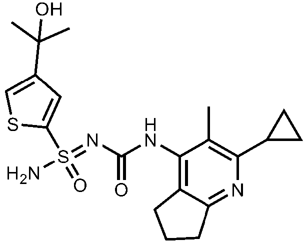 |
| **134ab** | 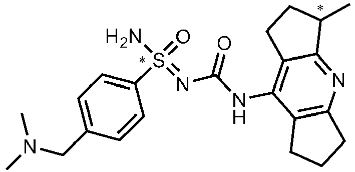 | **175a** | 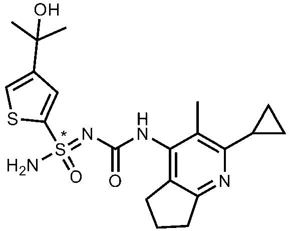 |
| **134ba** | 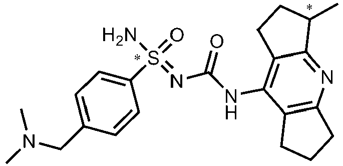 | **175b** | 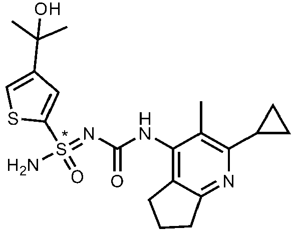 |
| **134bb** | 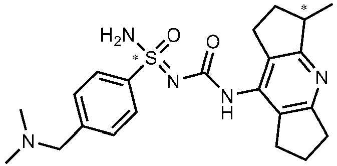 | **176** | 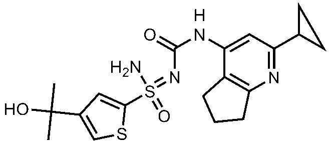 |
| **135** | 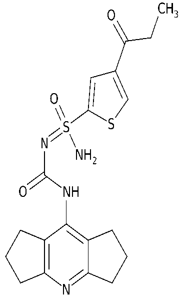 | **177** | 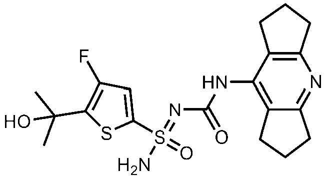 |
| **136** | 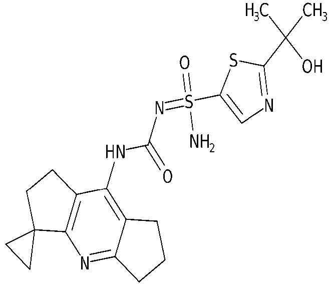 | **178** | 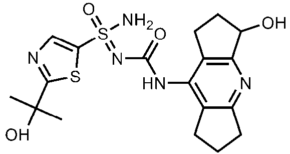 |
| **136a** | 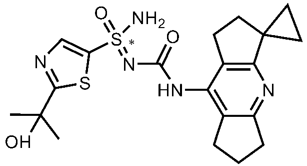 | **179** | 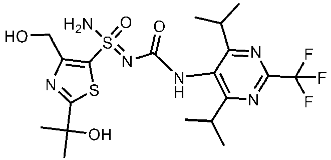 |
| **136b** | 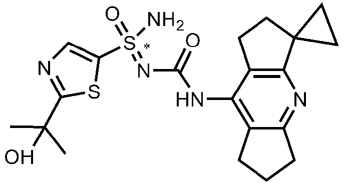 | **180** | 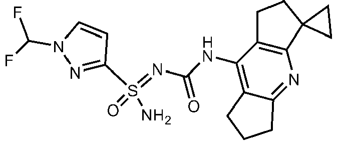 |
| **137** | 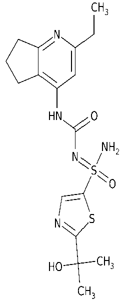 | **180a** | 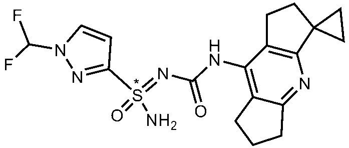 |
| **137a** | 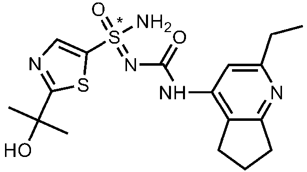 | **180b** | 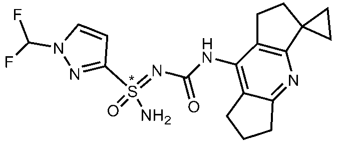 |
| **137b** | 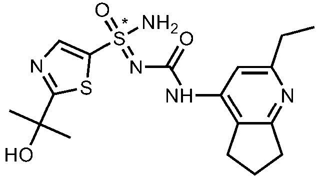 | **181** | 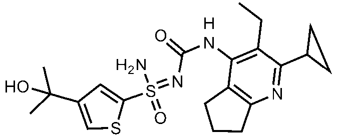 |
| **138** | 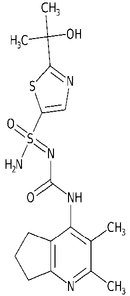 | **182** | 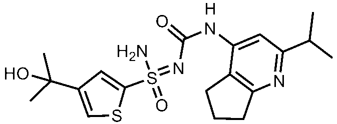 |
| **139** | 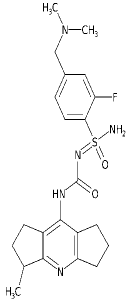 | **183** | 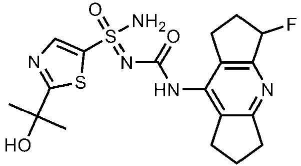 |
| **140** | 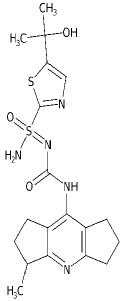 | **183a** | 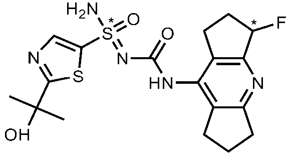 |
| **140a** | 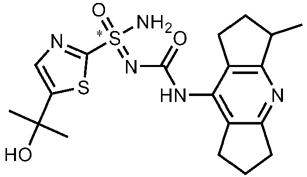 | **183b** | 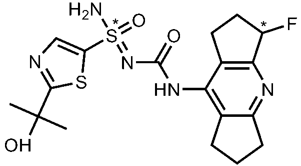 |
| **140aa** | 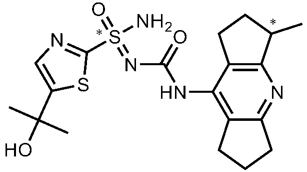 | **183c** | 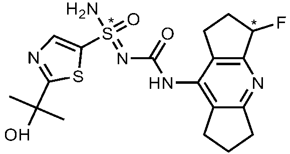 |
| **140ab** | 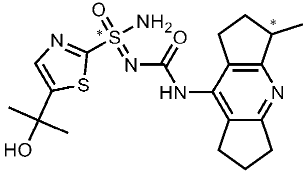 | **183d** | 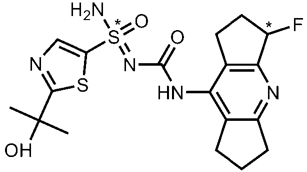 |
| **140b** | 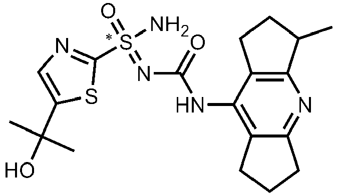 | **184a** | 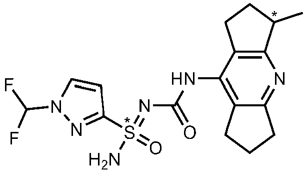 |
| **140ba** | 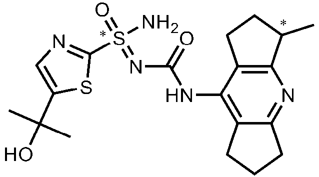 | **184b** | 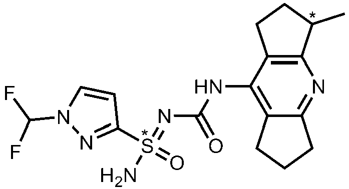 |
| **140bb** | 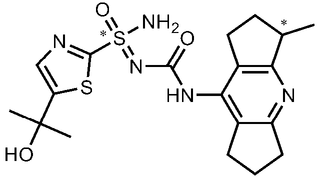 | **184c** | 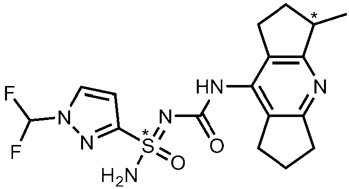 |
| **141** | 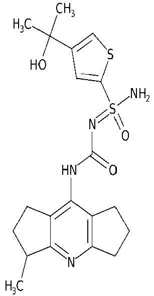 | **184d** | 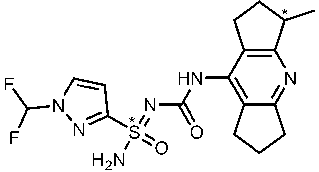 |
| **141a** | 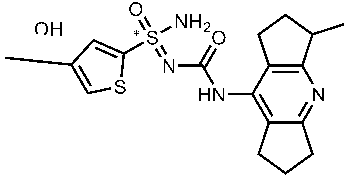 | **201** | 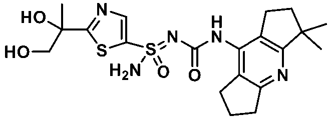 |
| **141aa** | 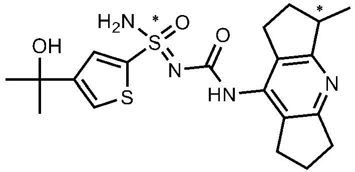 | **201a** | 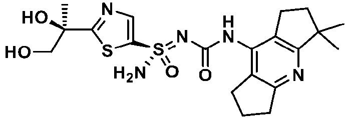 |
| **141ab** | 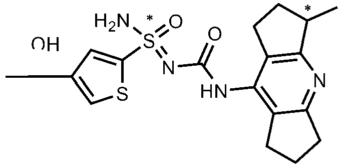 | | |
| **141b** | 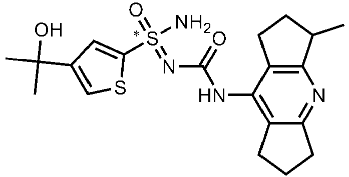 | | |
| **141ba** | 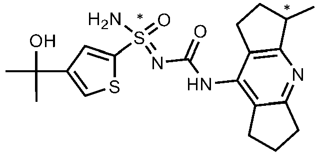 | | |

and pharmaceutically acceptable salts thereof.

In some embodiments, provided herein is a compound that is selected from the following:

| | | | |
|---|---|---|---|
| **185b** | 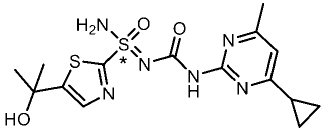 | **185** | 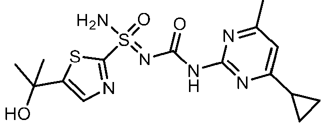 |
| **185a** | 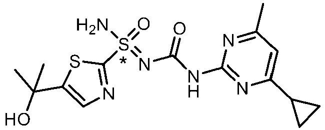 | | |

and pharmaceutically acceptable salts thereof.

In some embodiments, provided herein is a compound that is selected from the group consisting of the compounds in Table 1B:

**Table 1B.**

| | | | |
|---|---|---|---|
| **301** | 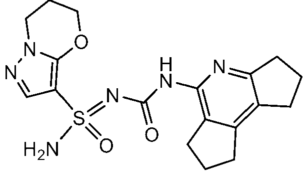 | **421** | 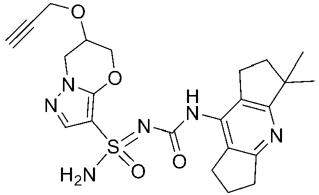 |
| **302** | 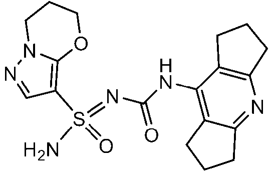 | **422** | 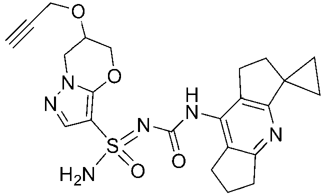 |
| **303** | 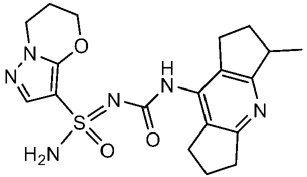 | **423** | 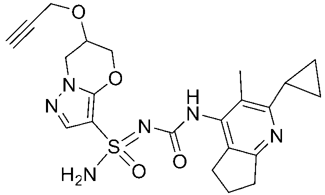 |
| **304** | 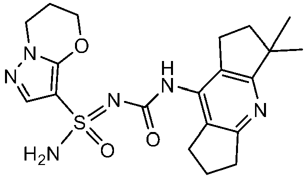 | **424** | 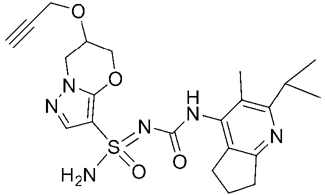 |
| **305** | 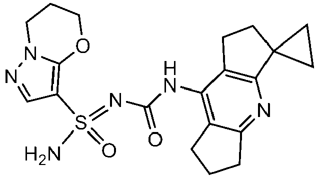 | **425** | 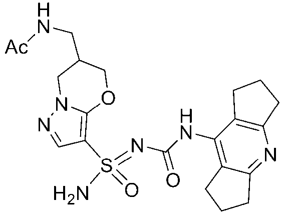 |
| **306** | 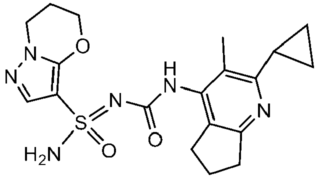 | **426** | 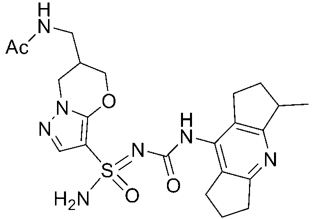 |
| **307** | 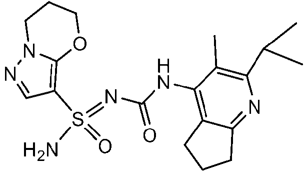 | **427** | 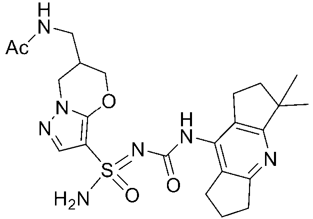 |
| **308** | 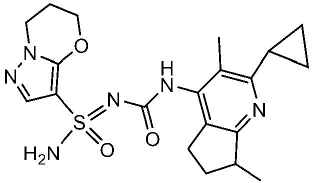 | **428** | 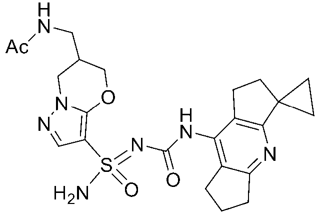 |
| **309** | 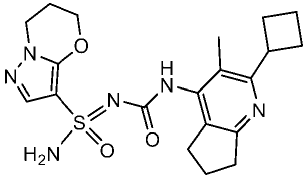 | **429** | 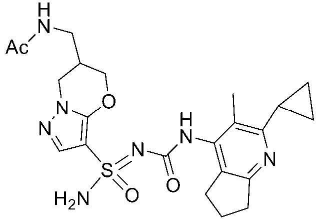 |
| **311** | 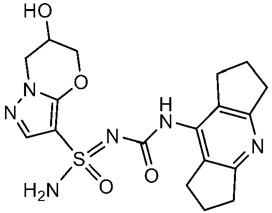 | **430** | 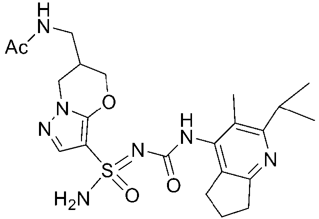 |
| **312** | 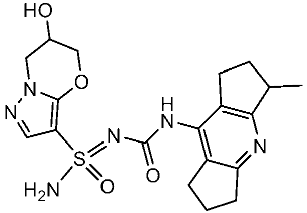 | **431** | 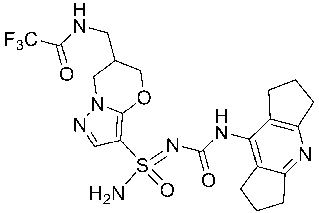 |
| **313** | 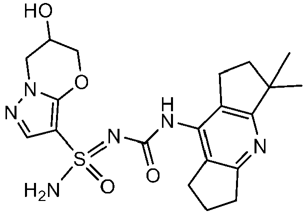 | **432** | 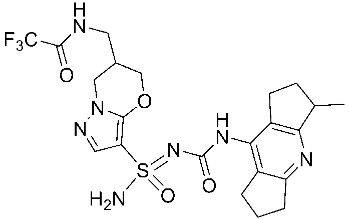 |
| **314** | 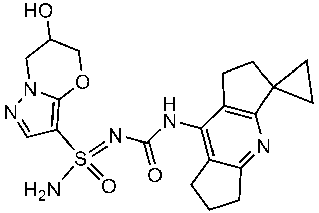 | **433** | 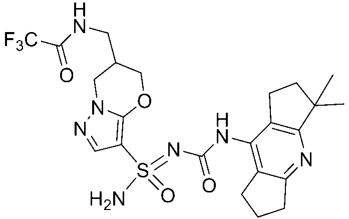 |
| **315** | 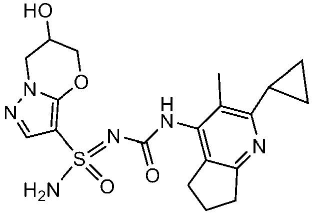 | **434** | 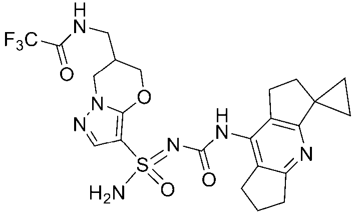 |
| **316** | 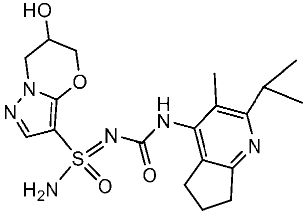 | **435** | 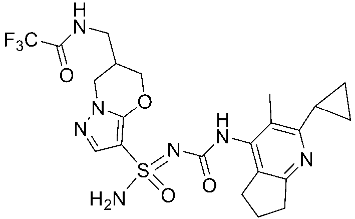 |
| **317** | 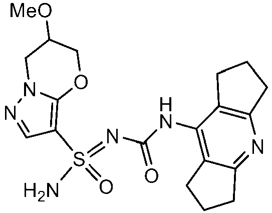 | **436** | 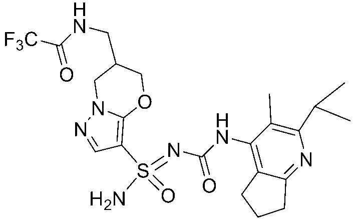 |
| **318** | 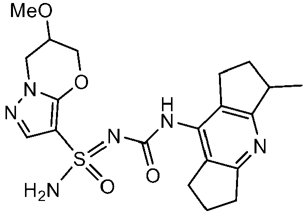 | **437** | 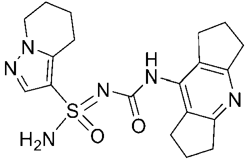 |
| **319** | 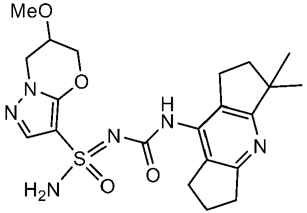 | **438** | 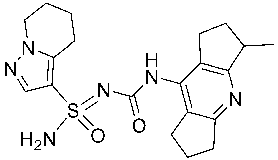 |
| **320** | 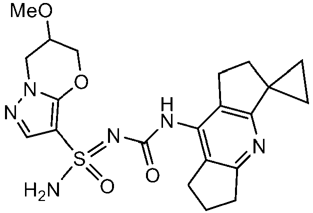 | **439** | 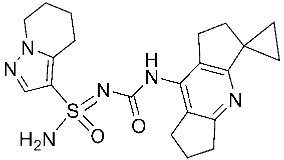 |
| **321** | 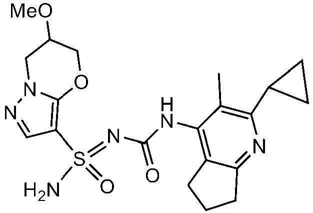 | **440** | 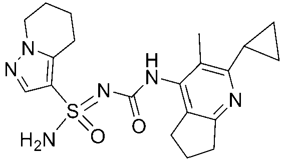 |
| **322** | 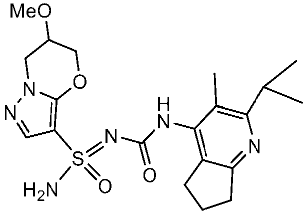 | **441** | 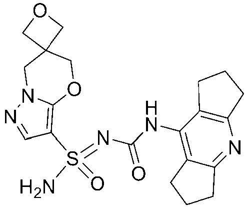 |
| **323** | 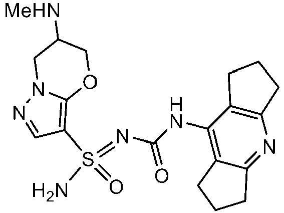 | **442** | 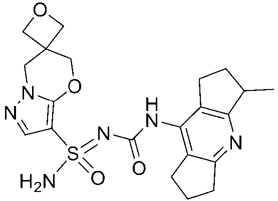 |
| **324** | 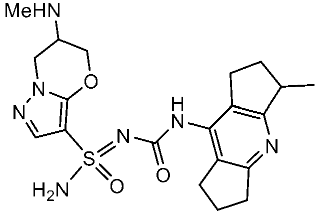 | **443** | 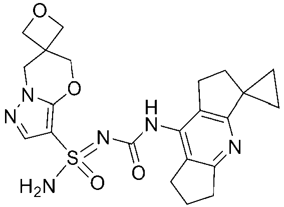 |
| **325** | 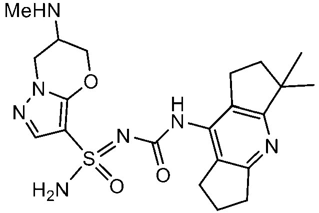 | **444** | 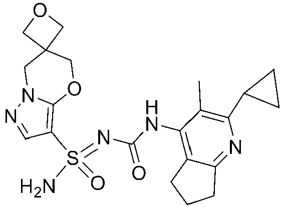 |
| **326** | 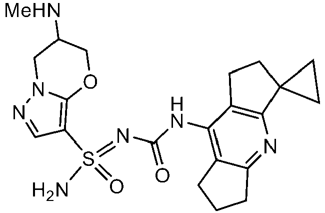 | **445** | 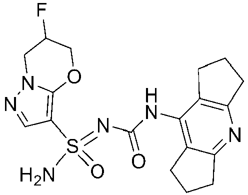 |
| **327** | 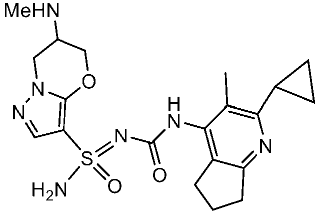 | **446** | 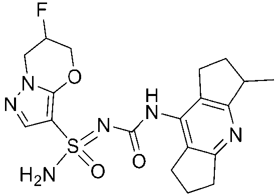 |
| **328** | 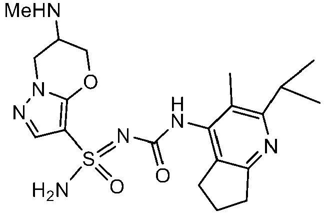 | **447** | 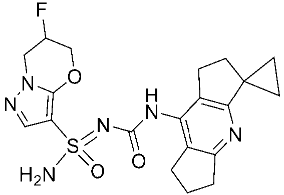 |
| **329** | 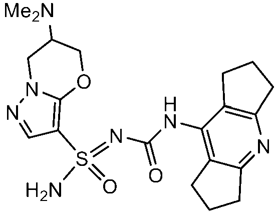 | **448** | 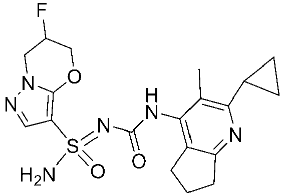 |
| **330** | 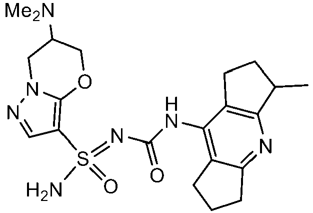 | **449** | 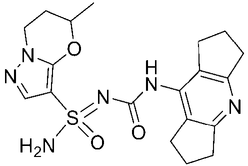 |
| **331** | 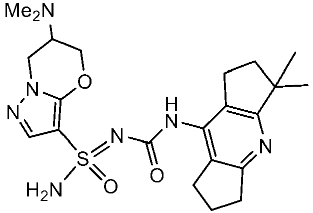 | **450** | 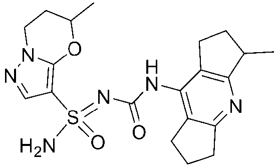 |
| **332** | 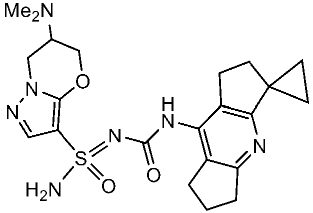 | **451** | 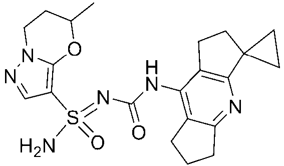 |
| **333** | 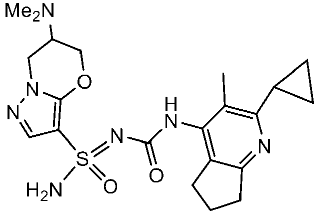 | **452** | 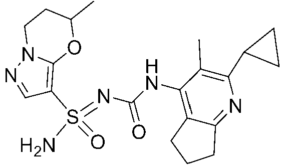 |
| **334** | 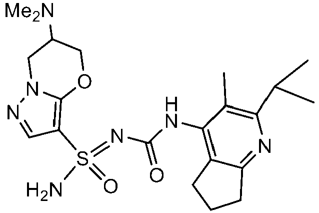 | **453** | 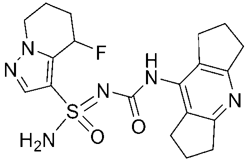 |
| **335** | 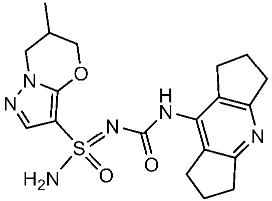 | **454** | 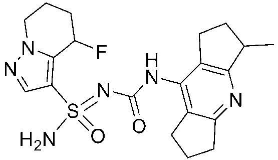 |
| **336** | 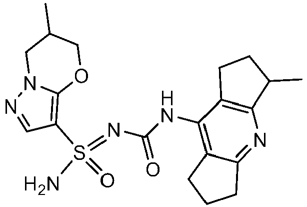 | **455** | 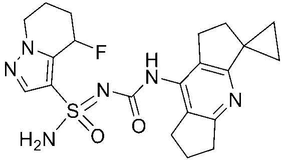 |
| **337** | 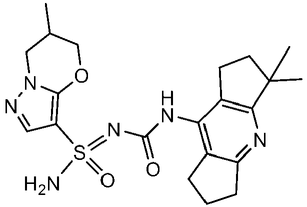 | **456** | 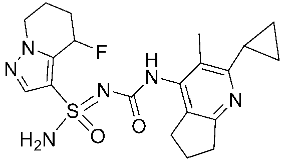 |
| **338** | 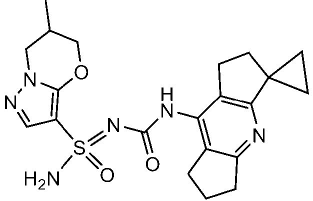 | **457** | 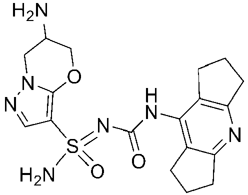 |
| **339** | 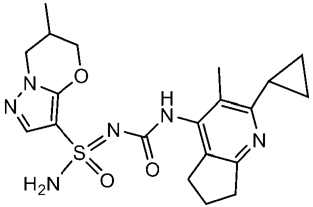 | **458** | 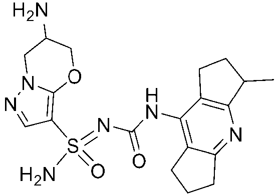 |
| **340** | 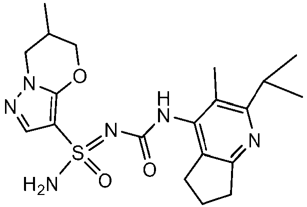 | **459** | 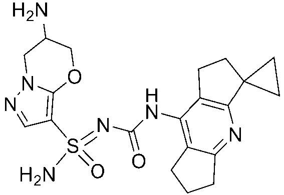 |
| **341** | 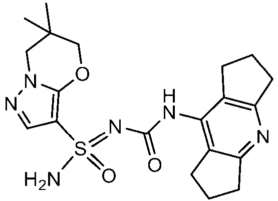 | **460** | 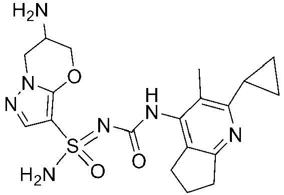 |
| **342** | 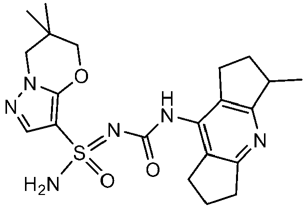 | **461** | 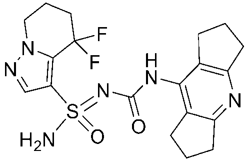 |
| **343** | 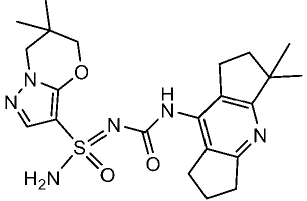 | **462** | 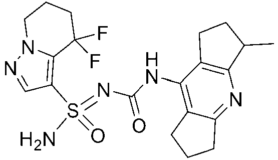 |
| **344** | 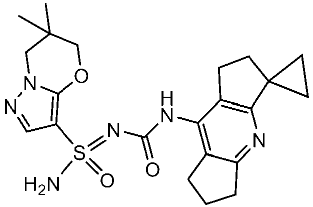 | **463** | 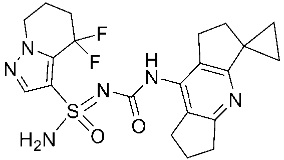 |
| **345** | 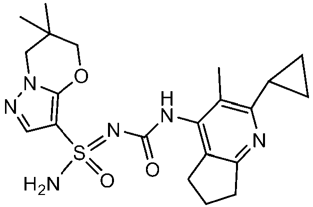 | **464** | 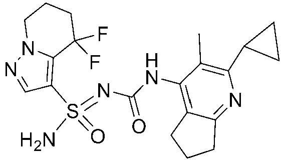 |
| **346** | 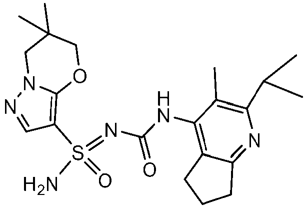 | **465** | 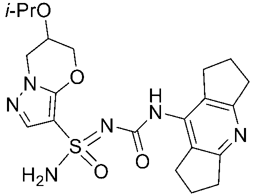 |
| **347** | 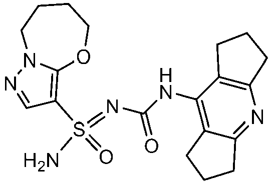 | **466** | 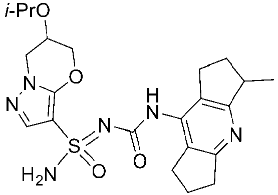 |
| **348** | 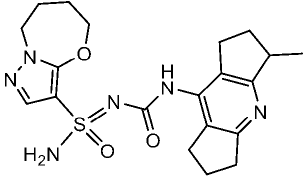 | **467** | 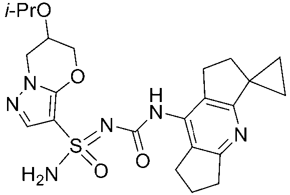 |
| **349** | 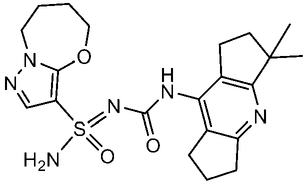 | **468** | 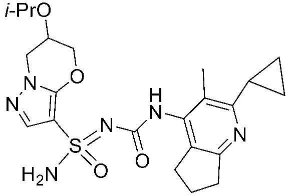 |
| **350** | 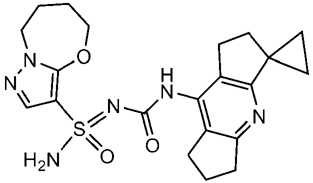 | **469** | 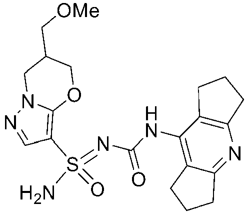 |
| **351** | 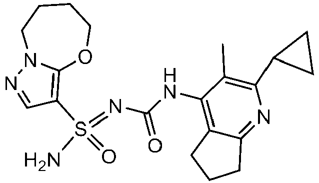 | **470** | 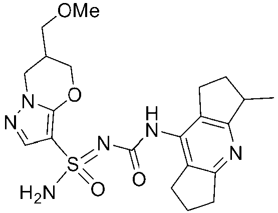 |
| **352** | 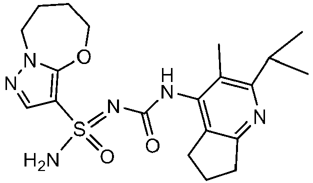 | **471** | 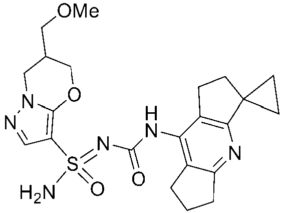 |
| **353** | 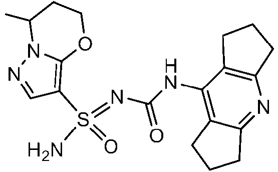 | **472** | 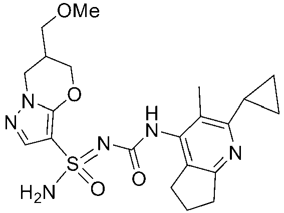 |
| **354** | 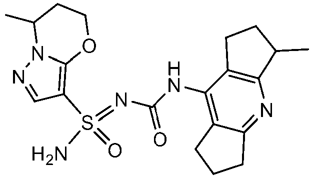 | **473** | 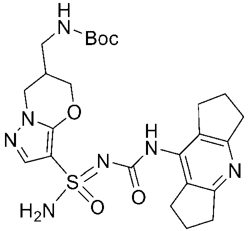 |
| **355** | 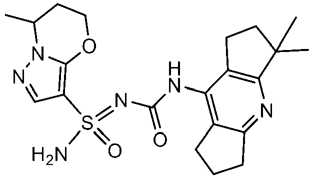 | **474** | 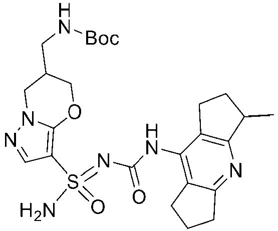 |
| **356** | 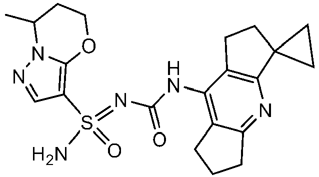 | **475** | 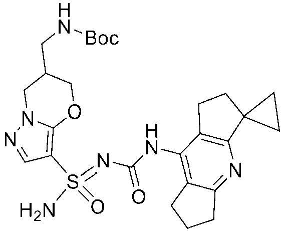 |
| **357** | 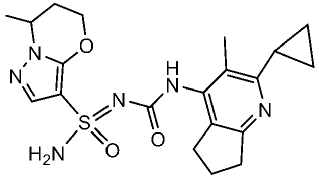 | **476** | 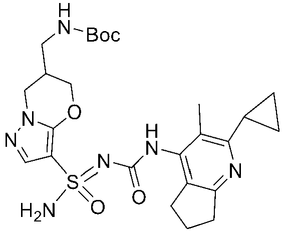 |
| **358** | 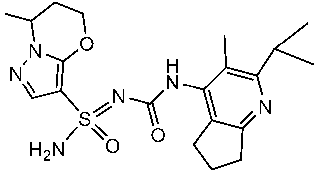 | **477** | 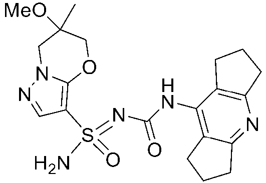 |
| **359** | 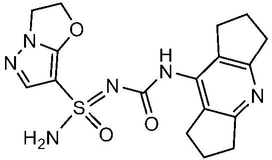 | **478** | 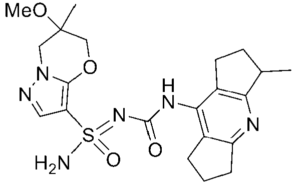 |
| **360** | 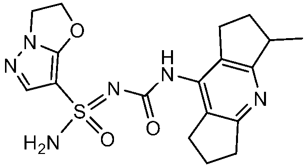 | **479** | 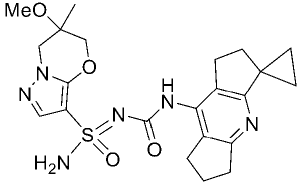 |
| **361** | 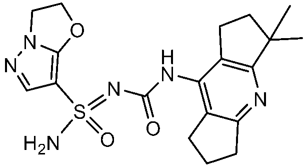 | **480** | 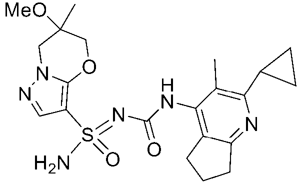 |
| **362** | 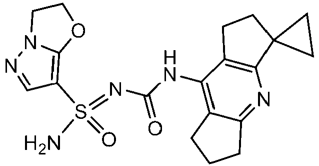 | **481** | 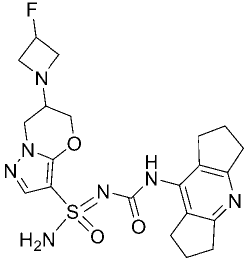 |
| **363** | 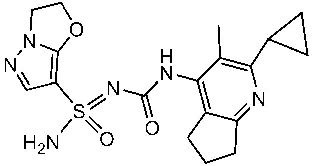 | **482** | 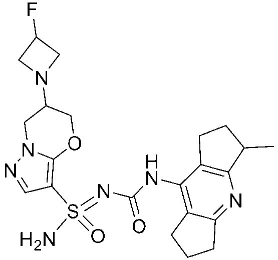 |
| **364** | 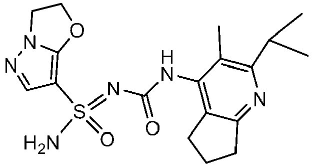 | **483** | 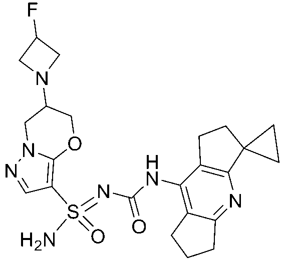 |
| **365** | 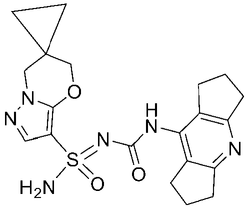 | **484** | 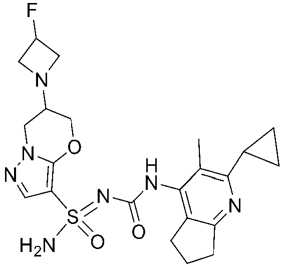 |
| **366** | 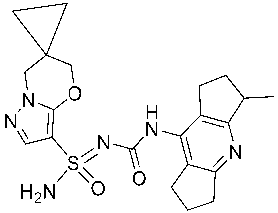 | **485** | 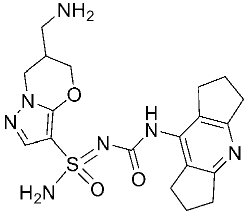 |
| **367** | 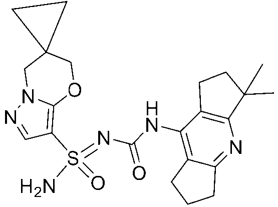 | **486** | 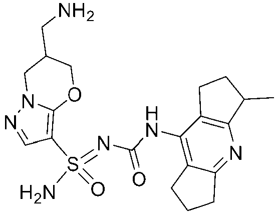 |
| **368** | 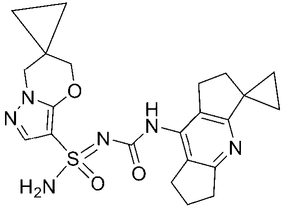 | **487** | 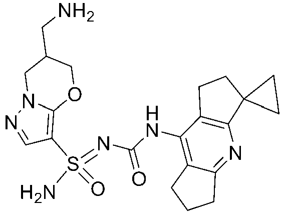 |
| **369** | 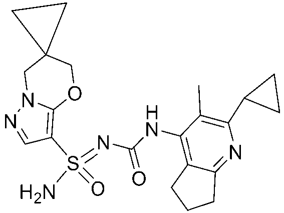 | **488** | 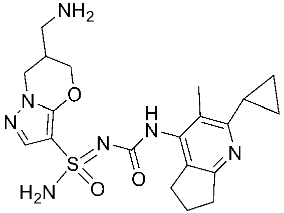 |
| **370** | 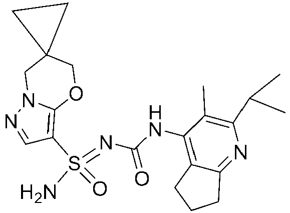 | **489** | 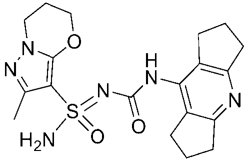 |
| **371** | 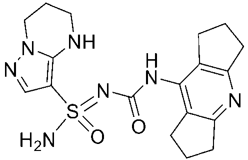 | **490** | 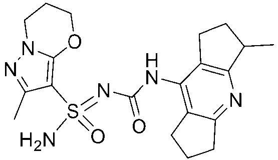 |
| **372** | 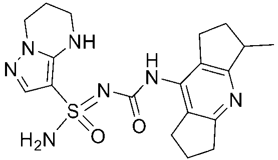 | **491** | 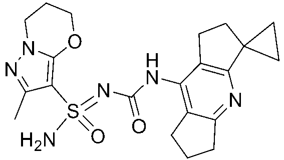 |
| **373** | 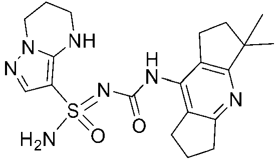 | **492** | 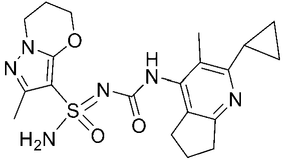 |
| **374** | 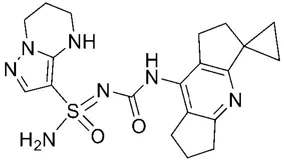 | **493** | 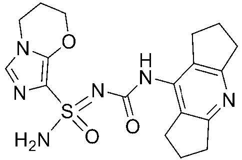 |
| **375** | 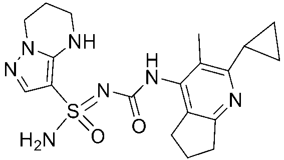 | **494** | 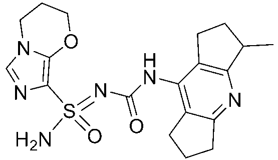 |
| **376** | 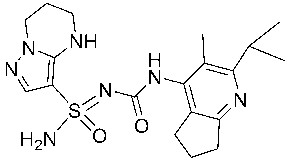 | **495** | 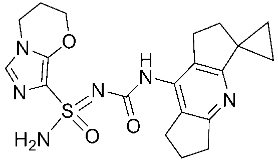 |
| **377** | 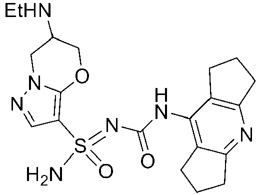 | **496** | 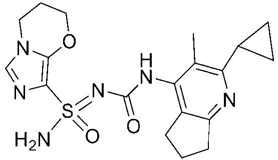 |
| **378** | 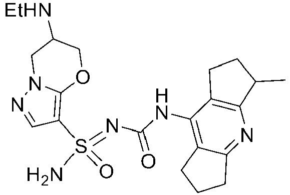 | **497** | 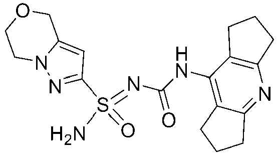 |
| **379** | 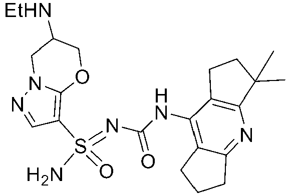 | **498** | 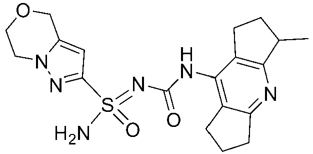 |
| **380** | 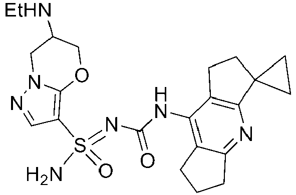 | **499** | 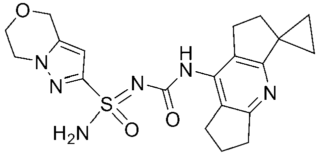 |
| **381** | 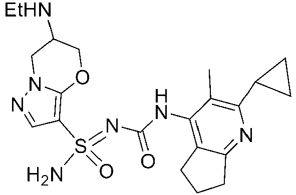 | **500** | 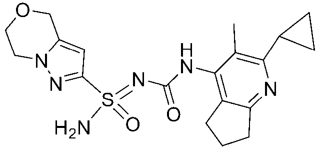 |
| **382** | 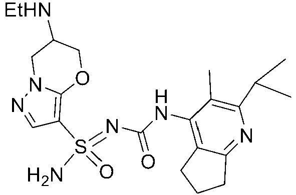 | **501** | 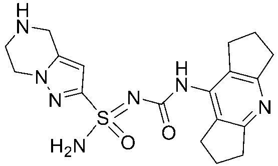 |
| **383** | 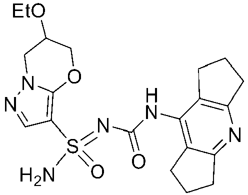 | **502** | 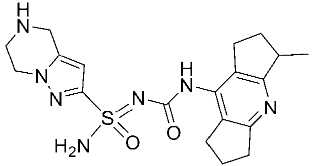 |
| **384** | 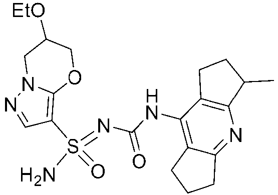 | **503** | 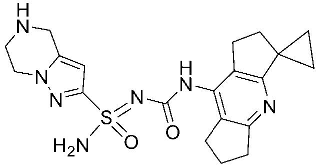 |
| **385** | 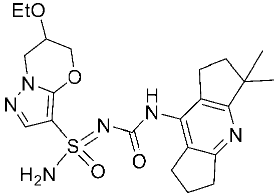 | **504** | 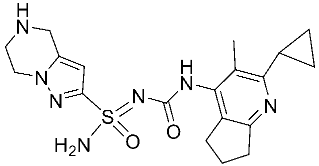 |
| **386** | 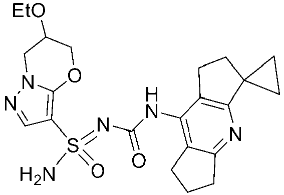 | **505** | 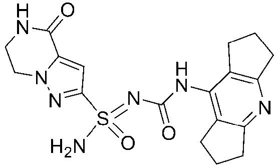 |
| **387** | 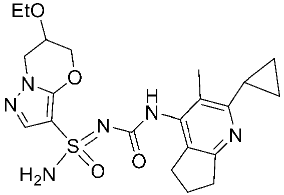 | **506** | 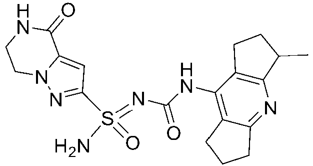 |
| **388** | 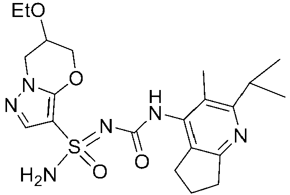 | **507** | 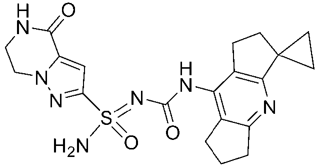 |
| **389** | 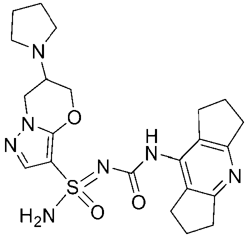 | **508** | 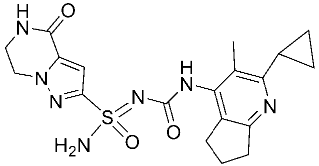 |
| **390** | 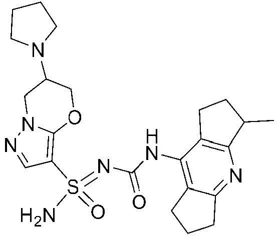 | **509** | 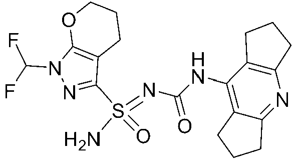 |
| **391** | 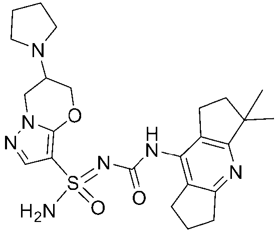 | **510** | 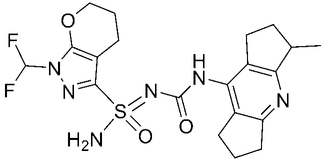 |
| **392** | 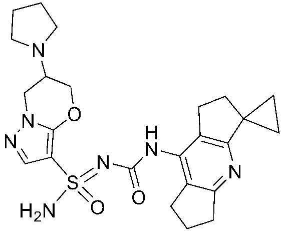 | **511** | 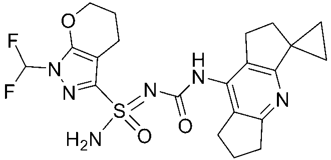 |
| **393** | 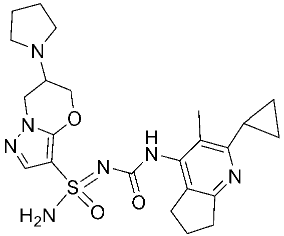 | **512** | 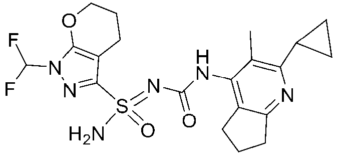 |
| **394** | 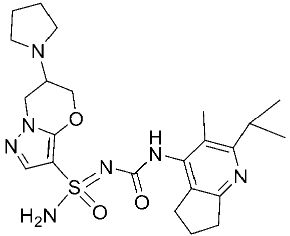 | **513** | 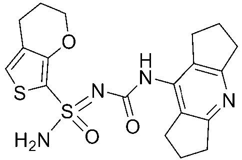 |
| **395** | 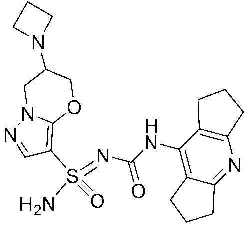 | **514** | 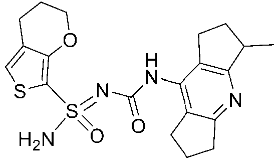 |
| **396** | 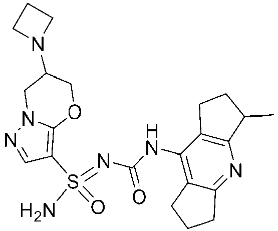 | **515** | 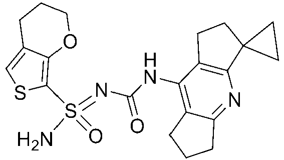 |
| **397** | 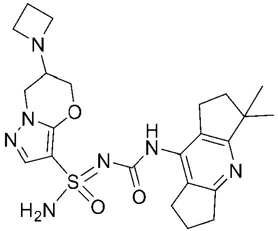 | **516** | 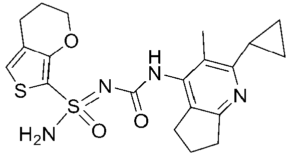 |
| **398** | 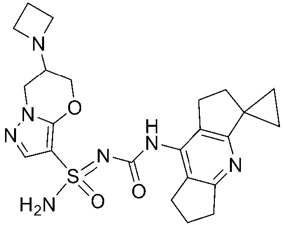 | **517** | 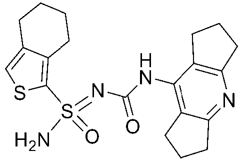 |
| **399** | 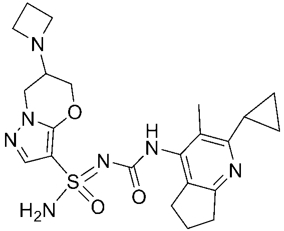 | **518** | 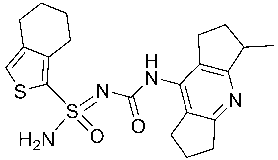 |
| **400** | 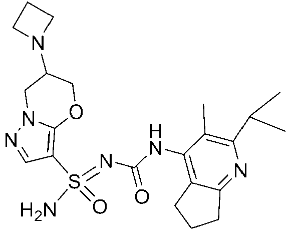 | **519** | 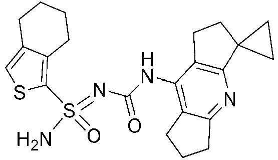 |
| **401** | 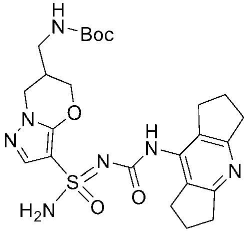 | **520** | 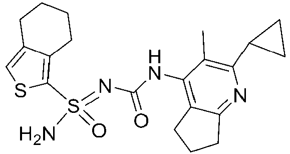 |
| **402** | 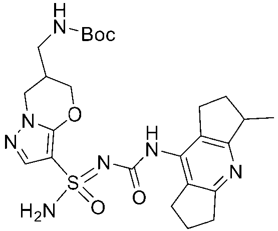 | **521** | 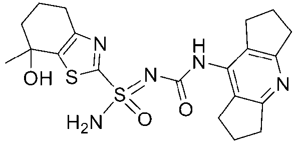 |
| **403** | 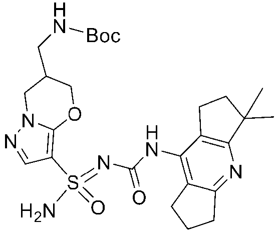 | **522** | 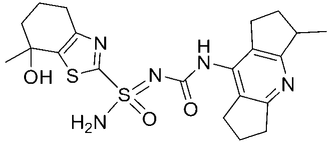 |
| **404** | 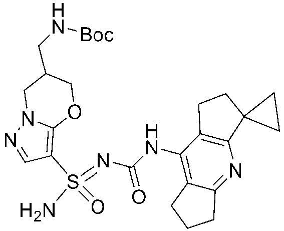 | **523** | 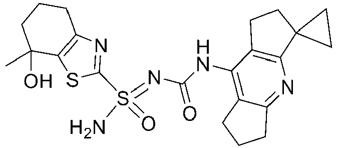 |
| **405** | 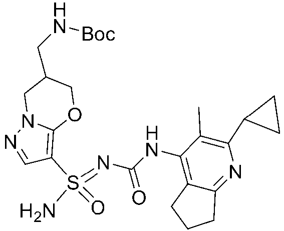 | **524** | 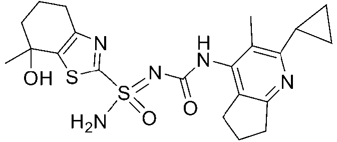 |
| **406** | 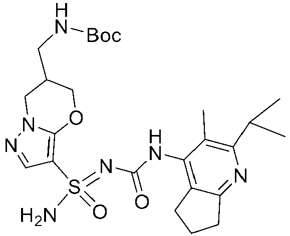 | **525** | 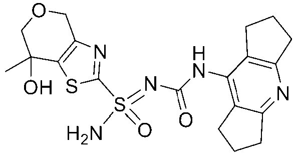 |
| **407** | 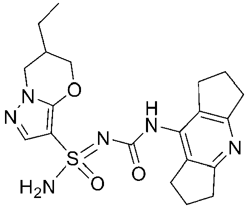 | **526** | 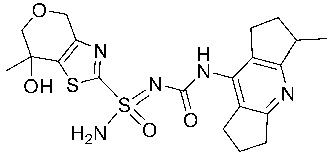 |
| **408** | 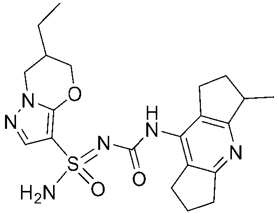 | **527** | 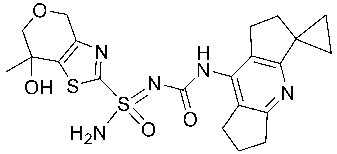 |
| **409** | 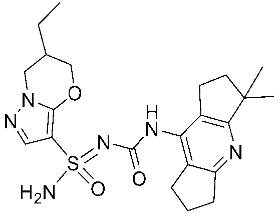 | **528** | 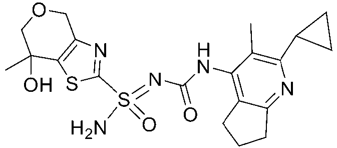 |
| **410** | 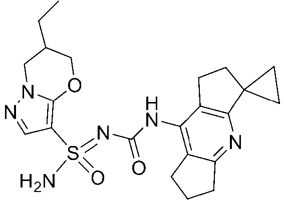 | **529** | 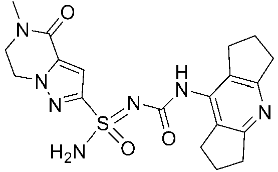 |
| **411** | 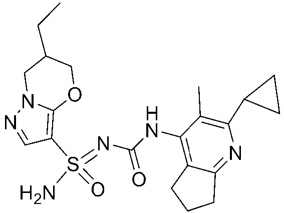 | **530** | 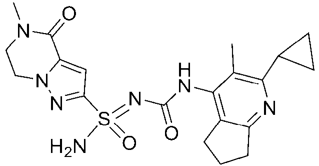 |
| **412** | 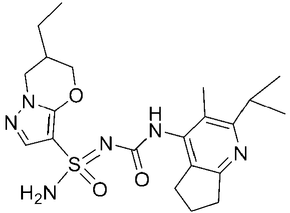 | **531** | 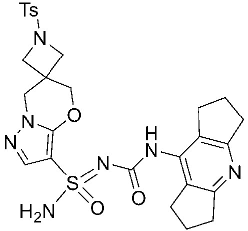 |
| **413** | 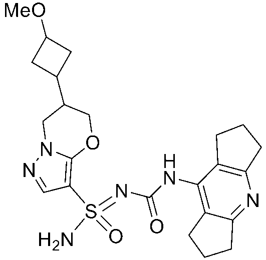 | **532** | 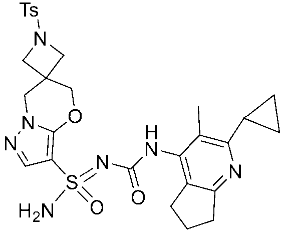 |
| **414** | 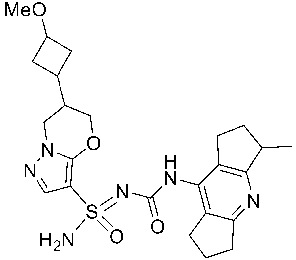 | **533** | 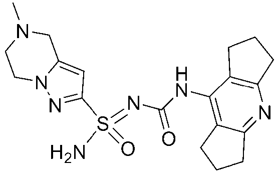 |
| **415** | 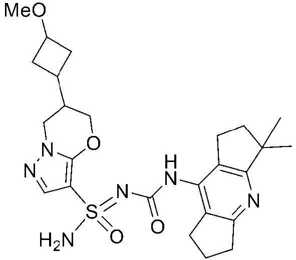 | **534** | 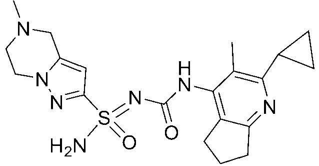 |
| **416** | 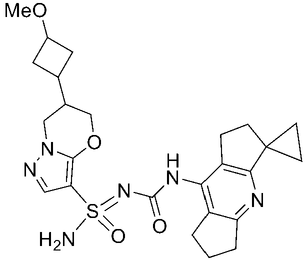 | **535** | 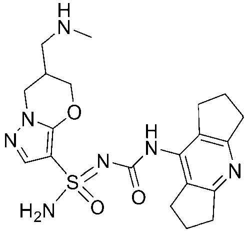 |
| **417** | 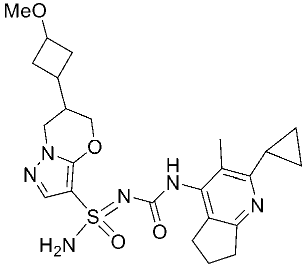 | **536** | 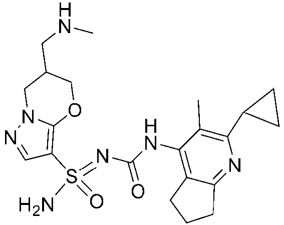 |
| **418** | 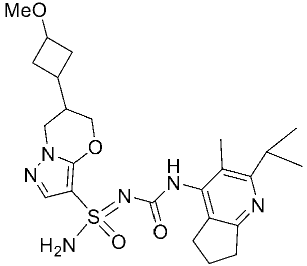 | **537** | 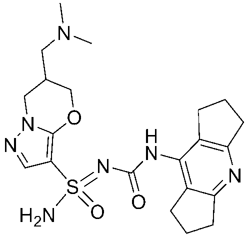 |
| **419** | 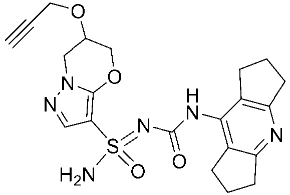 | **538** | 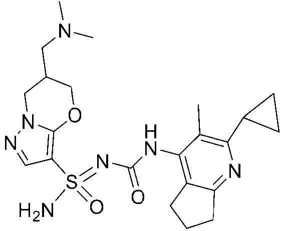 |
| **420** | 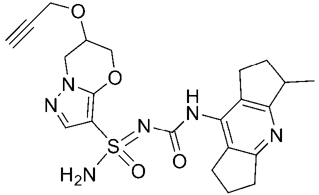 | **539** | 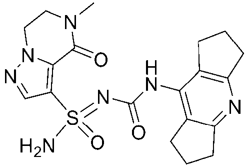 |
| | | **540** | 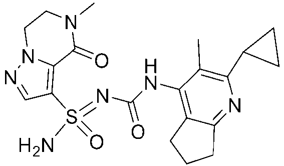 |

and pharmaceutically acceptable salts thereof.

In some embodiments, provided herein is a compound that is selected from the group consisting of the compounds in **Table 1C:**

**Table 1C**

| | | | |
|---|---|---|---|
| **115a** | 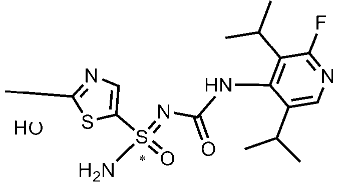 | **660** | 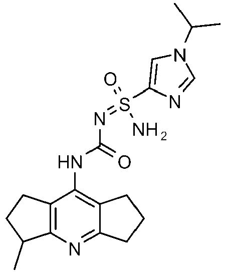 |
| **115b** | 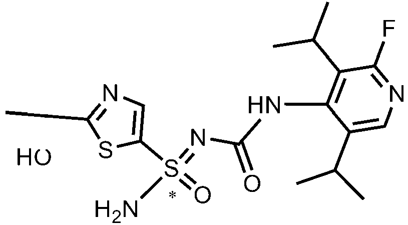 | **660a** | 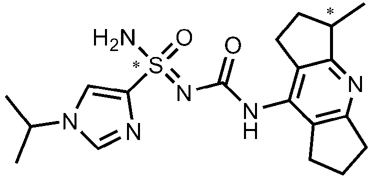 |
| **140c** | 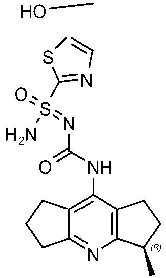 | **660b** | 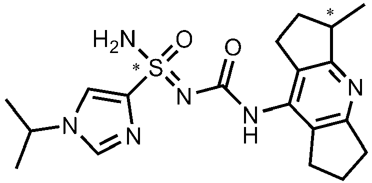 |
| **170a** | 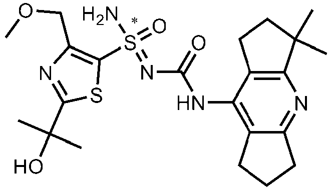 | **660c** | 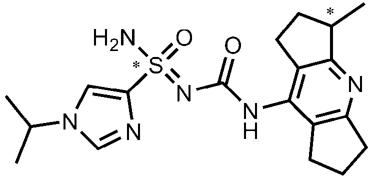 |
| **170b** | 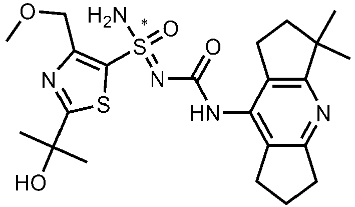 | **660d** | 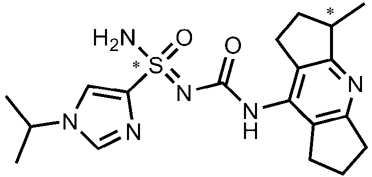 |
| **171a** | 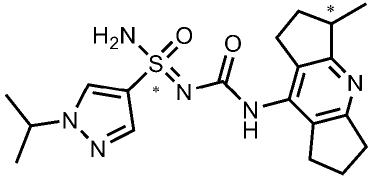 | **661** | 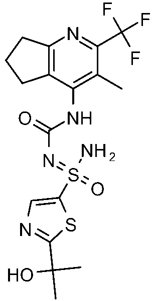 |
| **171b** | 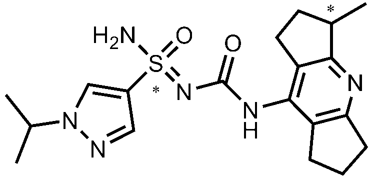 | **661a** | 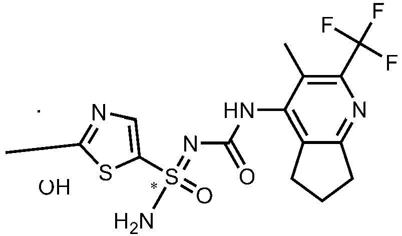 |
| **171c** | 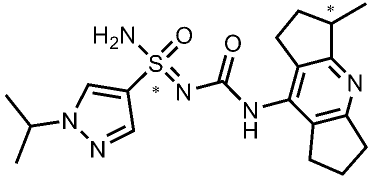 | **661b** | 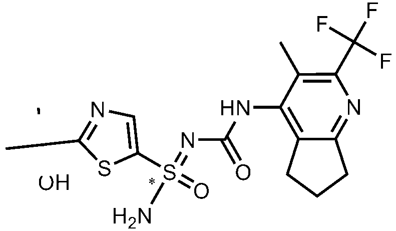 |
| **171d** | 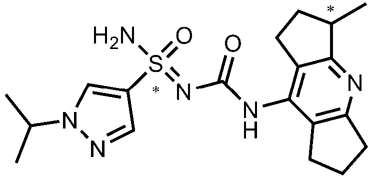 | **662** | 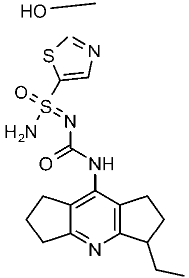 |
| **176a** | 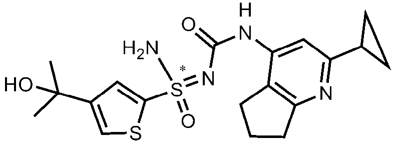 | **662a** | 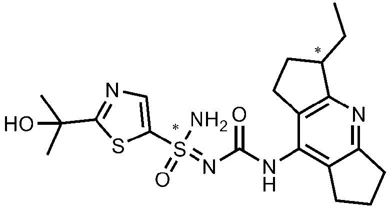 |
| **176b** | 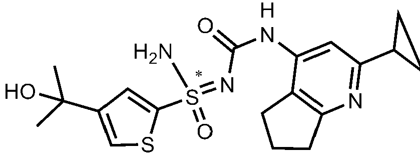 | **662b** | 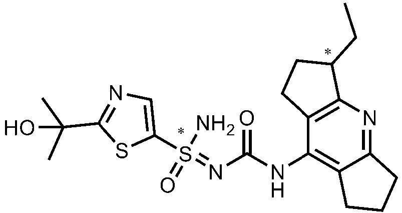 |
| **181a** | 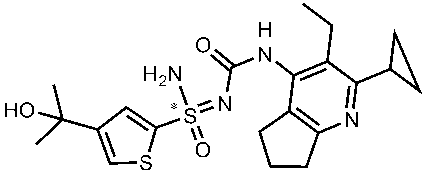 | **662c** | 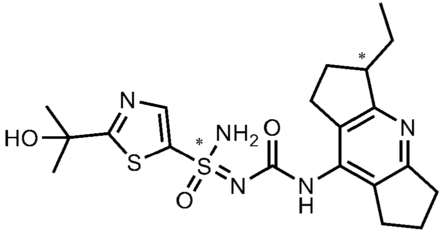 |
| **181b** | 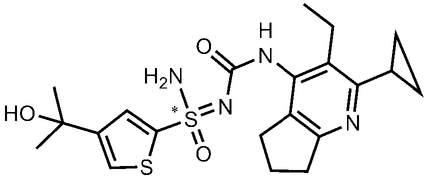 | **662d** | 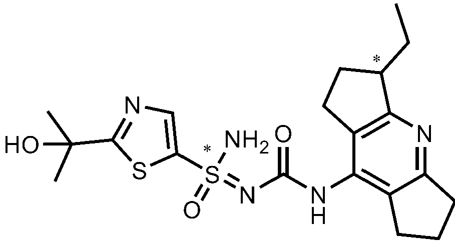 |
| **182a** | 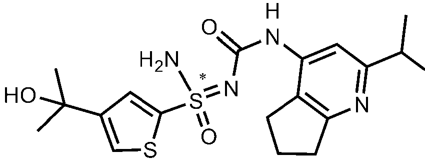 | **662e** | 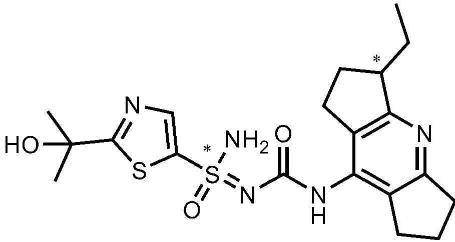 |
| **182b** | 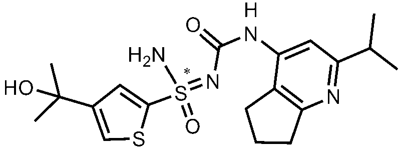 | **663** | 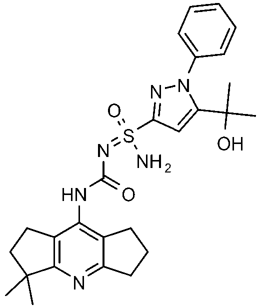 |
| **201b** | 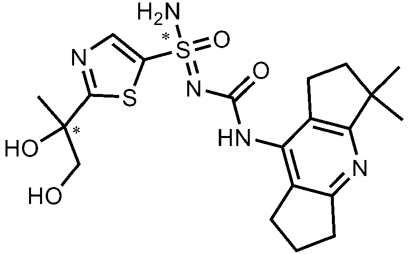 | **663a** | 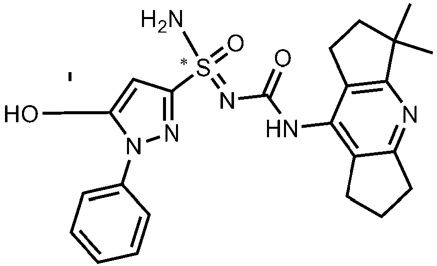 |
| **201c** | 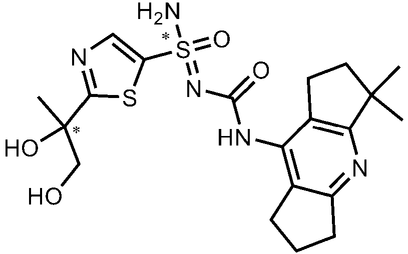 | **663b** | 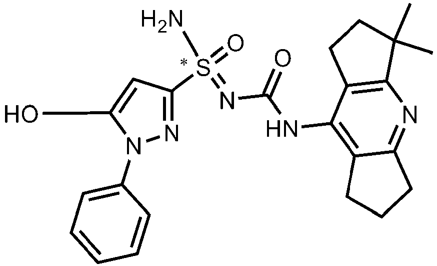 |
| **201d** | 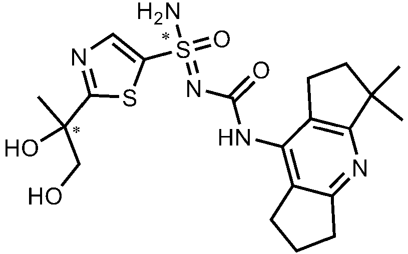 | **664** | 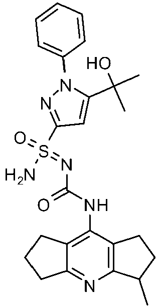 |
| **201e** | 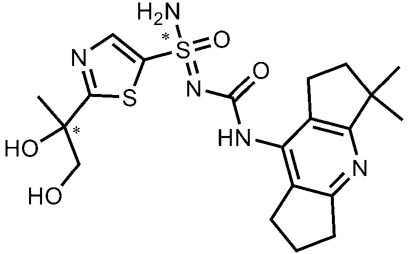 | **664a** | 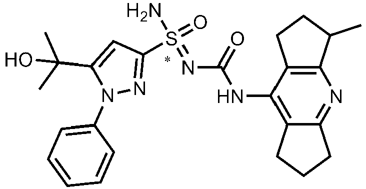 |
| **201f** | 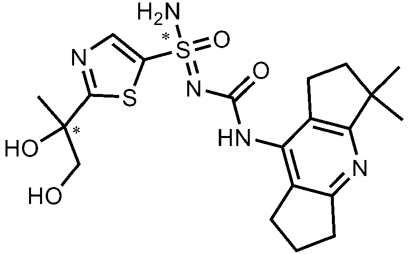 | **664b** | 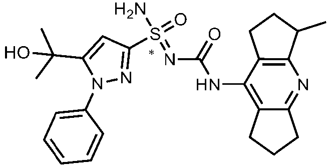 |
| **304a** | 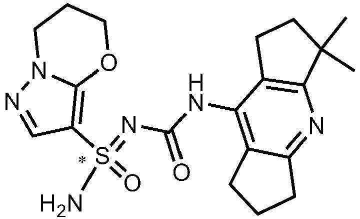 | **665** | 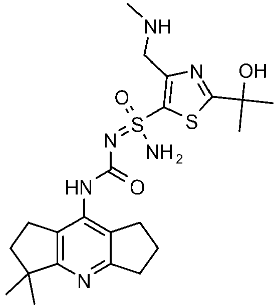 |
| **304b** | 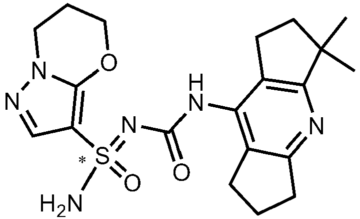 | **667** | 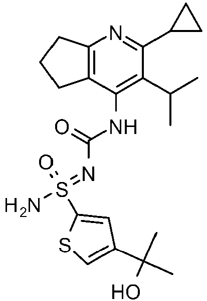 |
| **306a** | 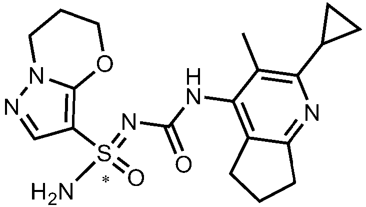 | **667a** | 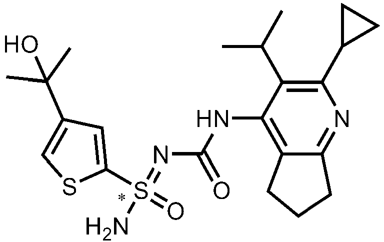 |
| **306b** | 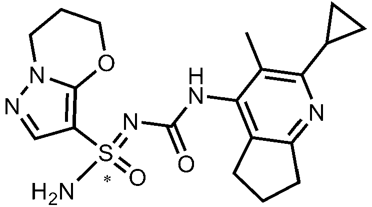 | **667b** | 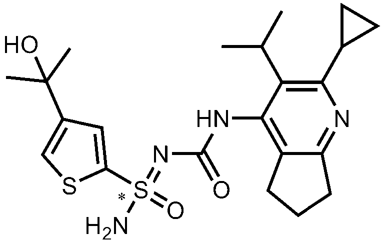 |
| **601** | 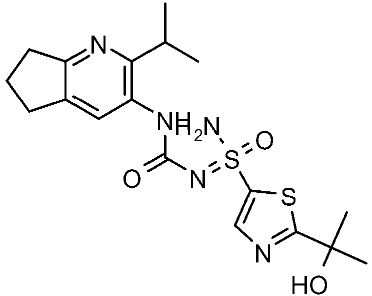 | **668** | 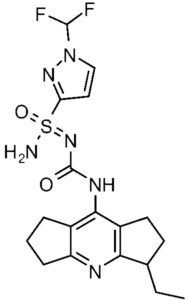 |
| **601a** | 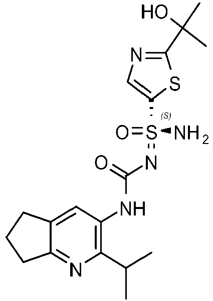 | **668a** | 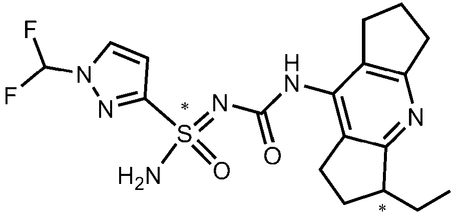 |
| **601b** | 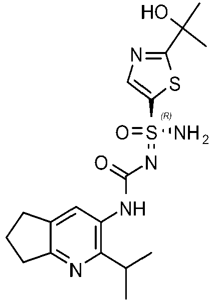 | **668b** | 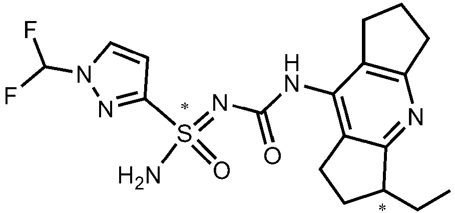 |
| **602** | 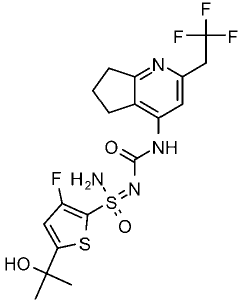 | **668c** | 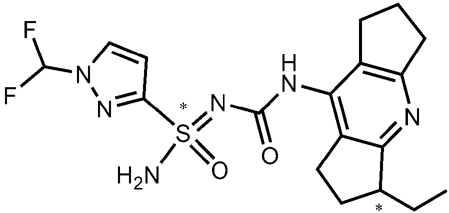 |
| **602a** | 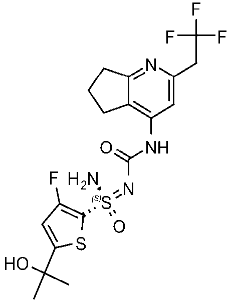 | **668d** | 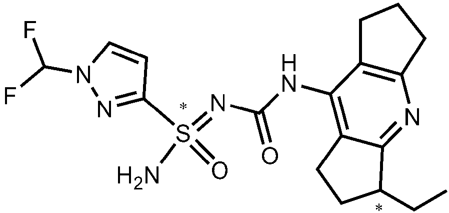 |
| **602b** | 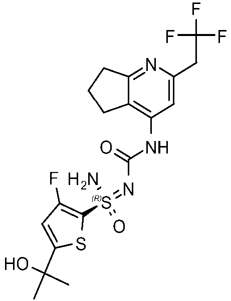 | **669** | 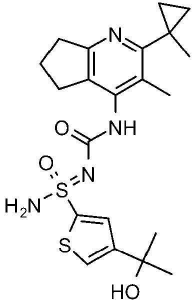 |
| **603** | 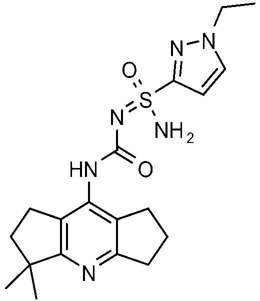 | **669a** | 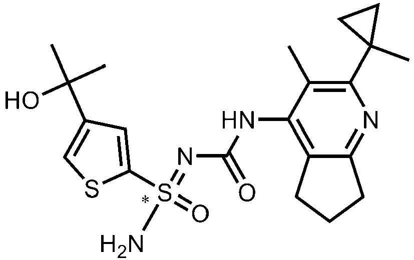 |
| **604** | 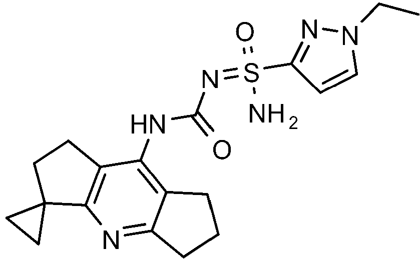 | **669b** | 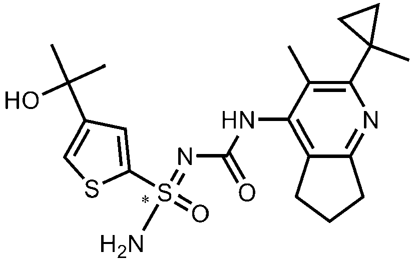 |
| **604a** | 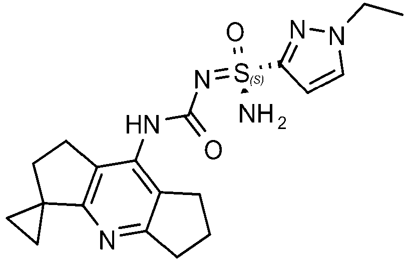 | **670** | 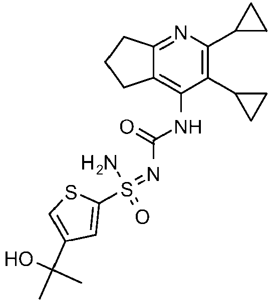 |
| **604b** | 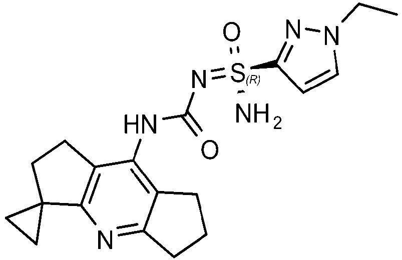 | **670a** | 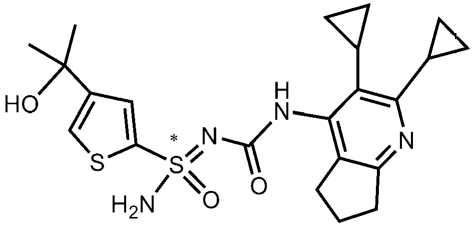 |
| **605** | 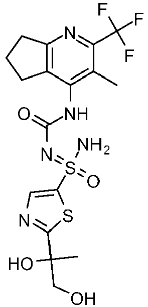 | **670b** | 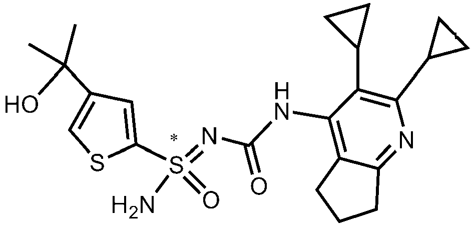 |
| **605a** | 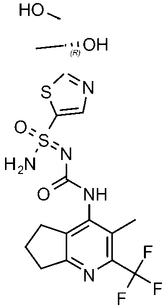 | **671** | 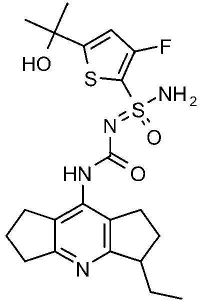 |
| **605b** | 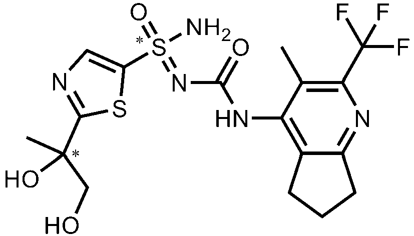 | **671a** | 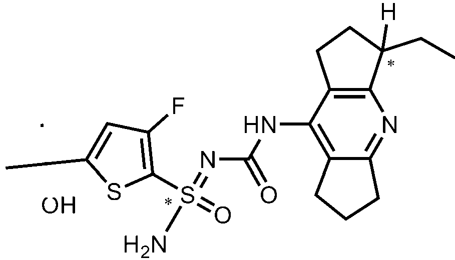 |
| **605c** | 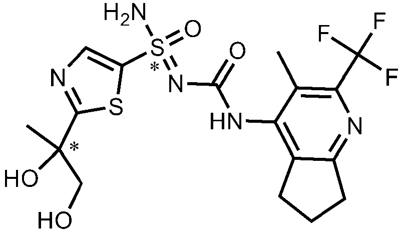 | **671b** | 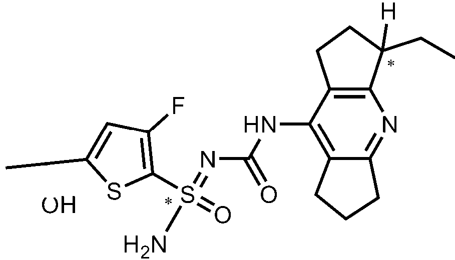 |
| **605d** | 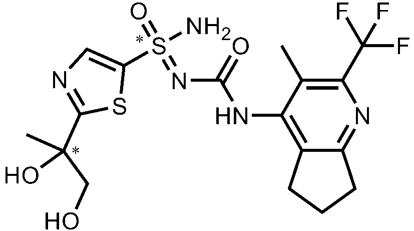 | **671c** | 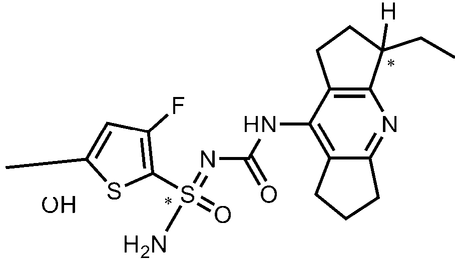 |
| **605e** | 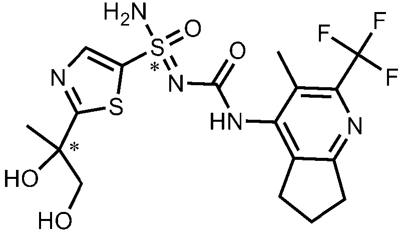 | **671d** | 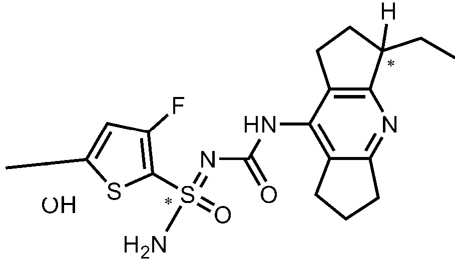 |
| **605f** | 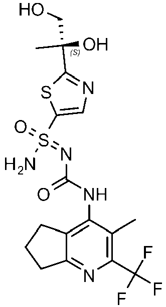 | **672** | 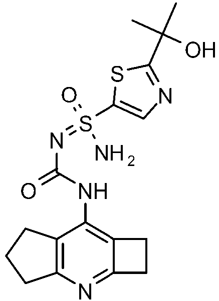 |
| **605g** | 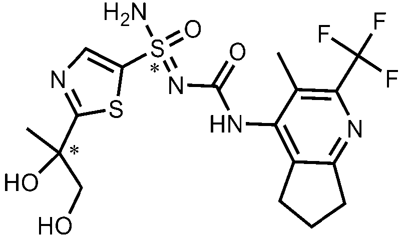 | **673** | 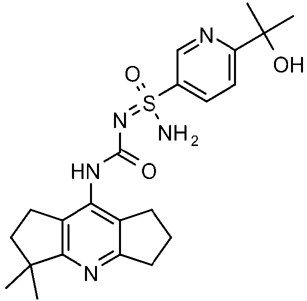 |
| **605h** | 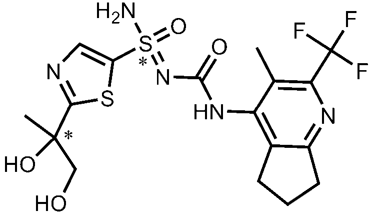 | **673a** | 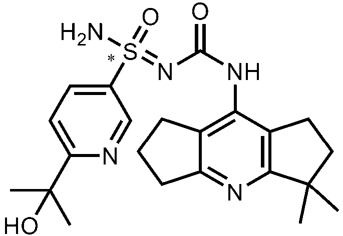 |
| **607** | 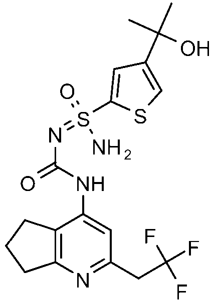 | **673b** | 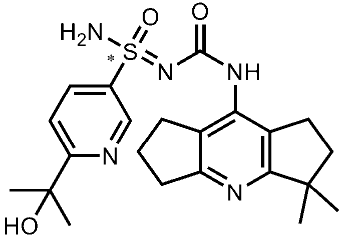 |
| **607a** | 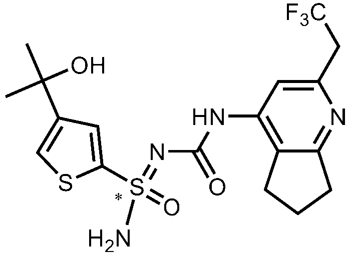 | **674** | 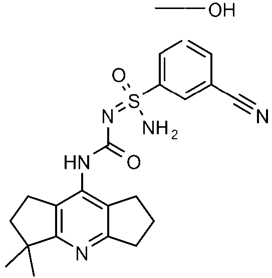 |
| **607b** | 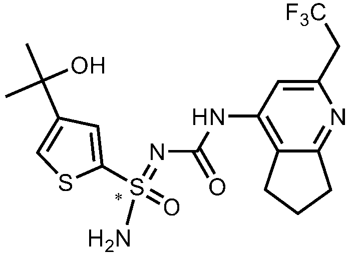 | **674a** | 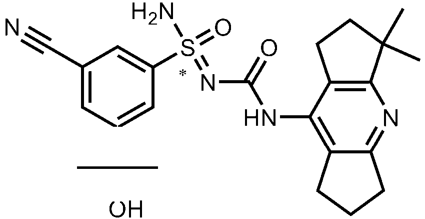 |
| **608** | 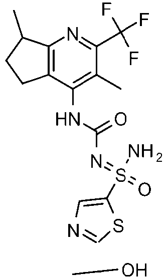 | **674b** | 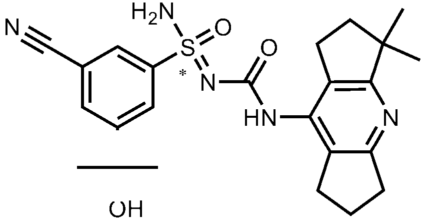 |
| **608a** | 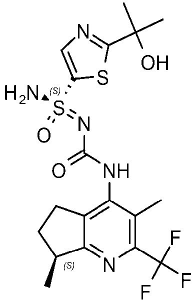 | **675** | 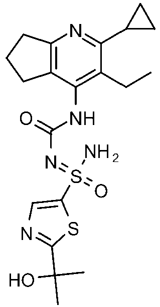 |
| **608b** | 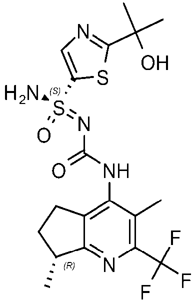 | **675a** | 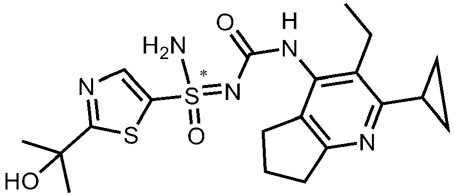 |
| **608c** | 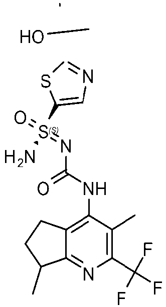 | **675b** | 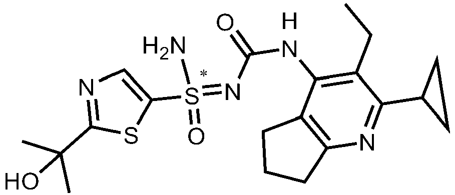 |
| **608d** | 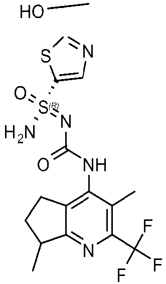 | **676** | 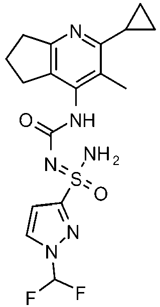 |
| **609** | 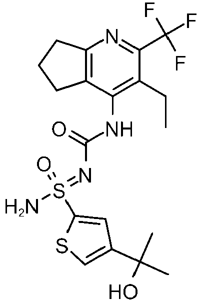 | **676a** | 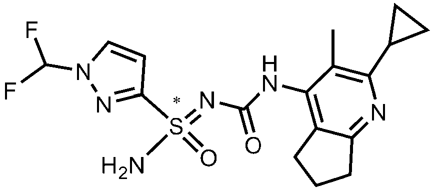 |
| **610** | 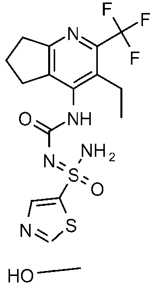 | **676b** | 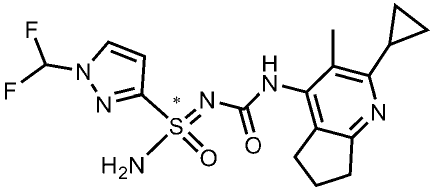 |
| **610a** | 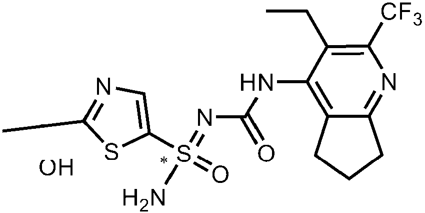 | **677** | 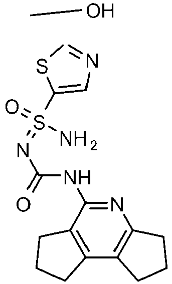 |
| **610b** | 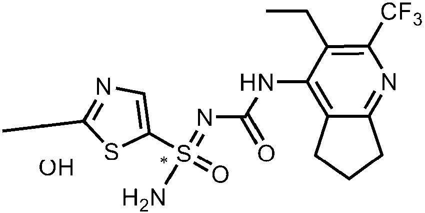 | **678** | 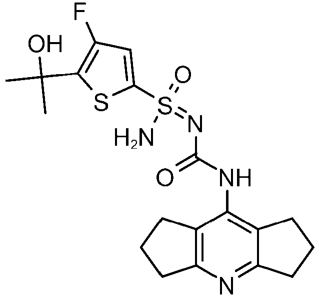 |
| **611** | 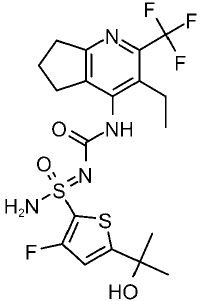 | **678a** | 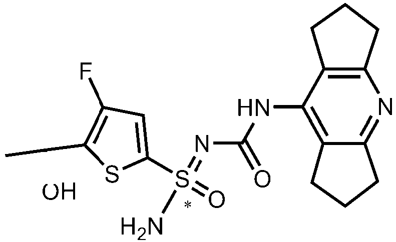 |
| **611a** | 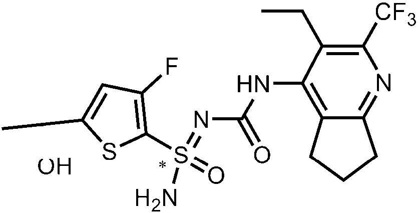 | **678b** | 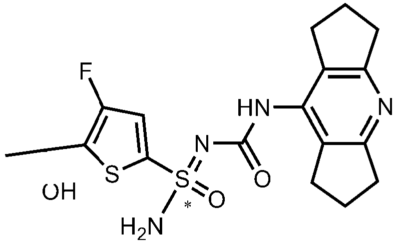 |
| **611b** | 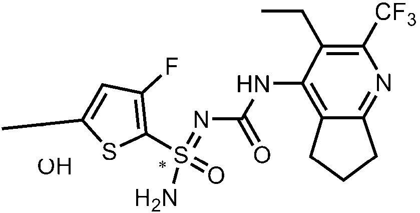 | **679** | 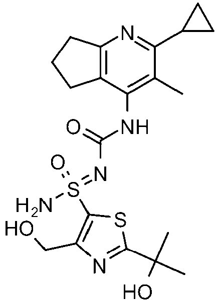 |
| **612** | 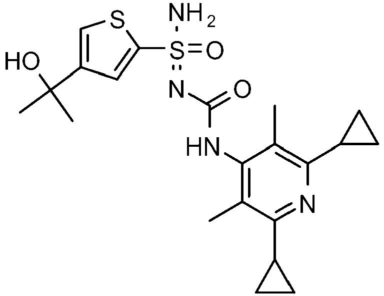 | **680** | 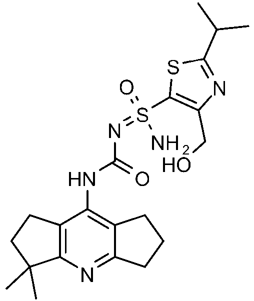 |
| **612a** | 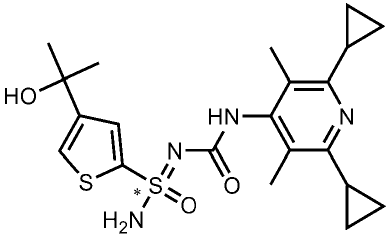 | **680a** | 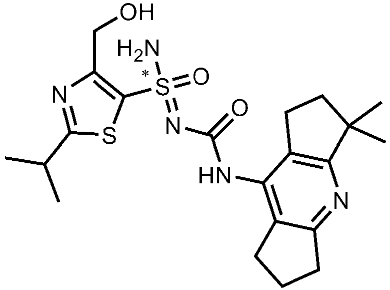 |
| **612b** | 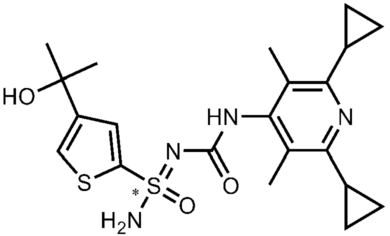 | **680b** | 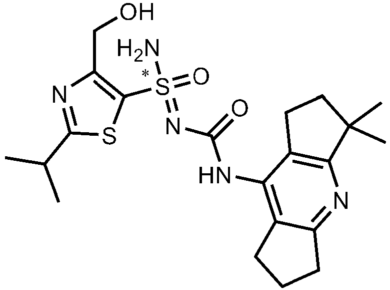 |
| **613** | 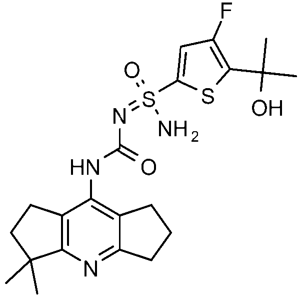 | **681** | 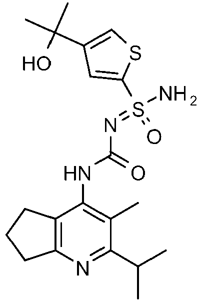 |
| **613a** | 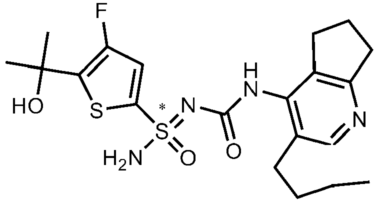 | **681a** | 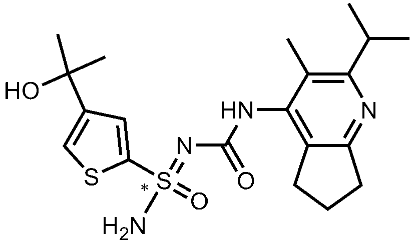 |
| **613b** | 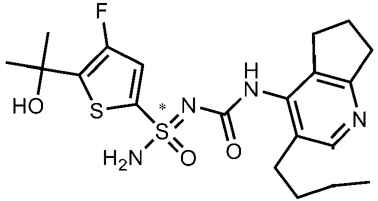 | **681b** | 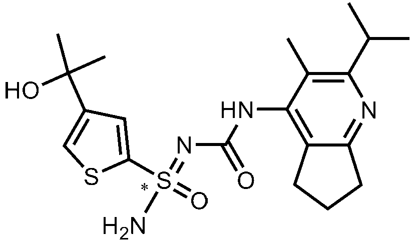 |
| **614** | 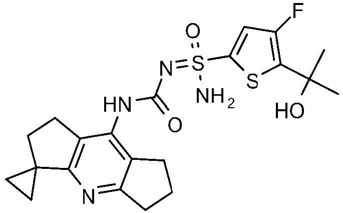 | **682** | 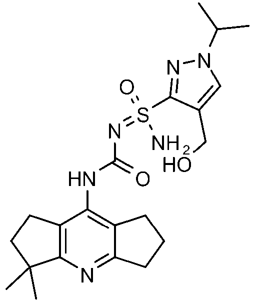 |
| **614a** | 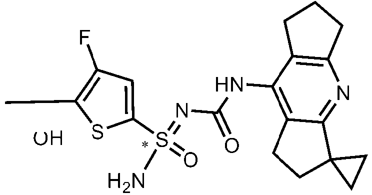 | **682a** | 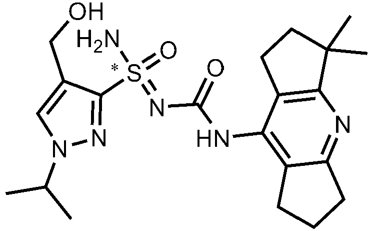 |
| **614b** | 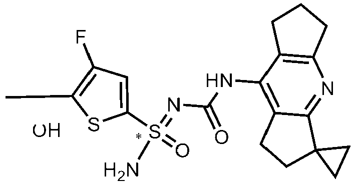 | **682b** | 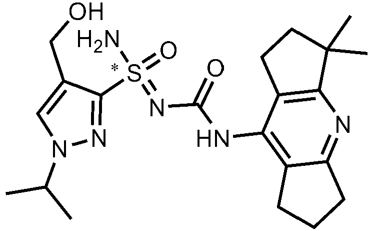 |
| **615** | 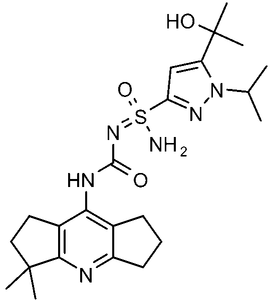 | **683** | 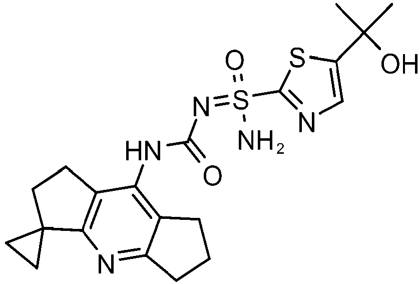 |
| **615a** | 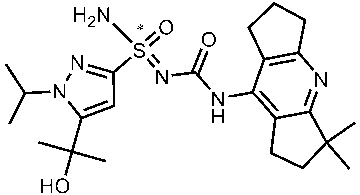 | **683a** | 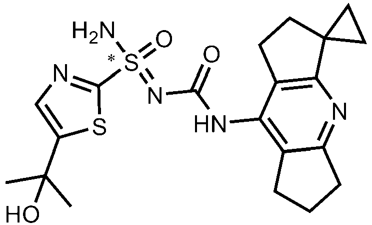 |
| **615b** | 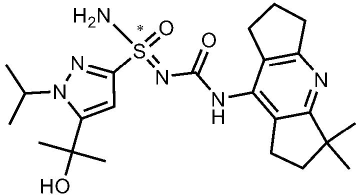 | **683b** | 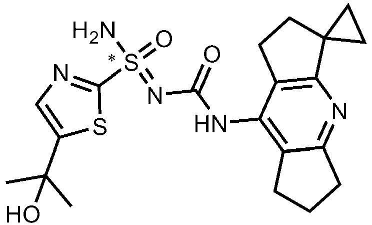 |
| **616** | 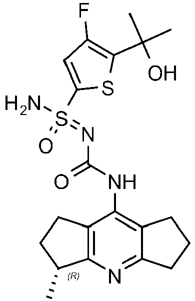 | **684** | 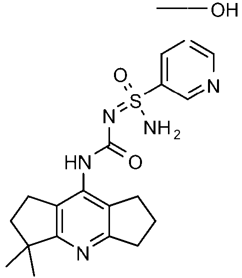 |
| **616a** | 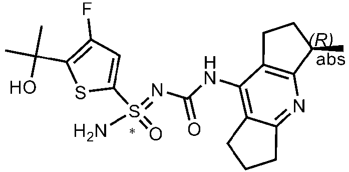 | **684a** | 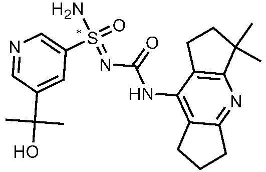 |
| **616b** | 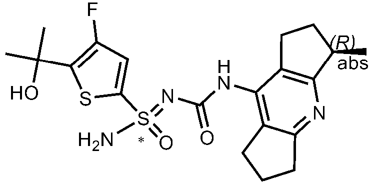 | **684b** | 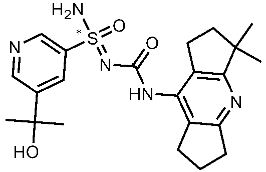 |
| **617** | 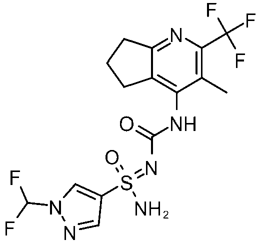 | **685** | 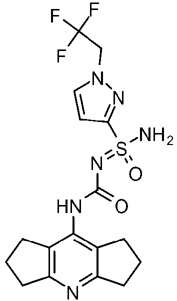 |
| **617a** | 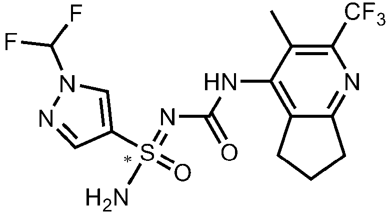 | **685a** | 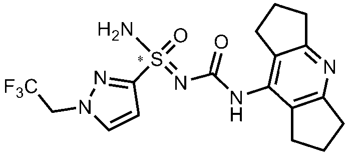 |
| **617b** | 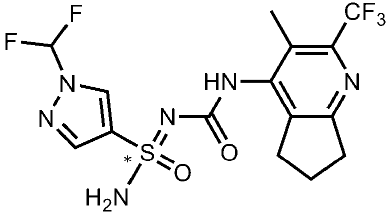 | **685b** | 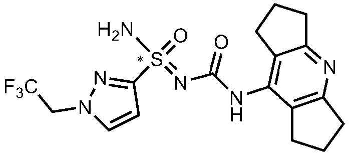 |
| **618** | 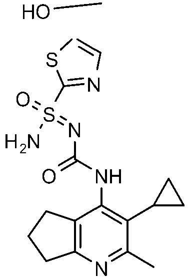 | **686** | 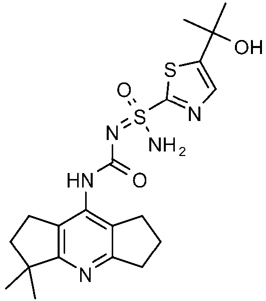 |
| **618a** | 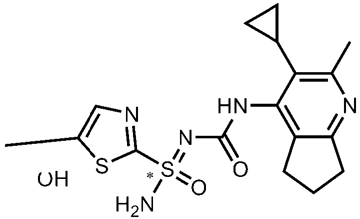 | **686a** | 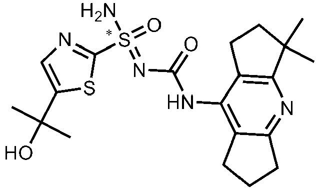 |
| **618b** | 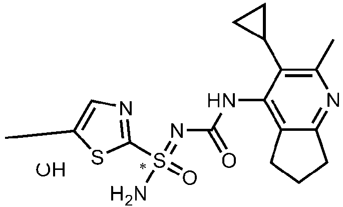 | **686b** | 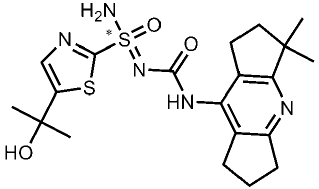 |
| **619** | 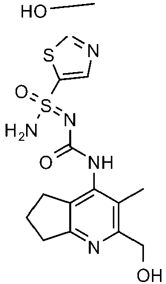 | **687** | 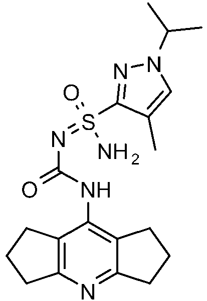 |
| **620** | 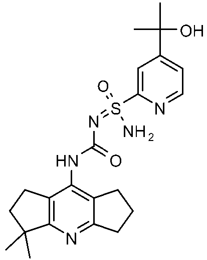 | **687a** | 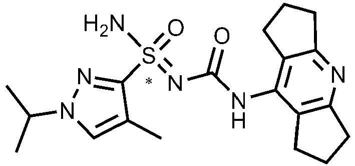 |
| **620a** | 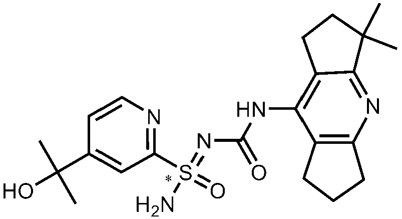 | **687b** | 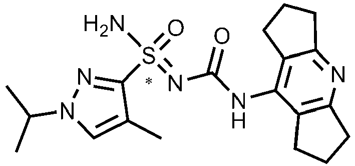 |
| **620b** | 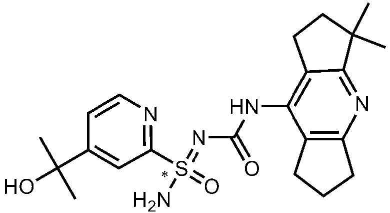 | **688** | 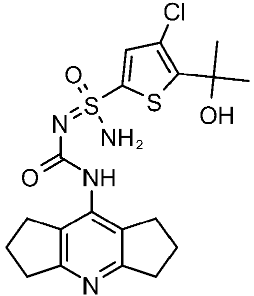 |
| **621** | 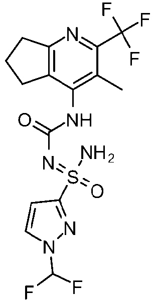 | **688a** | 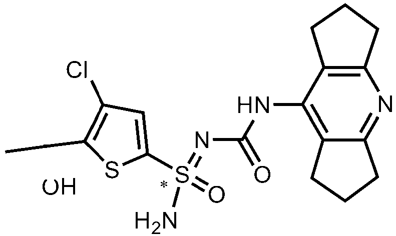 |
| **621a** | 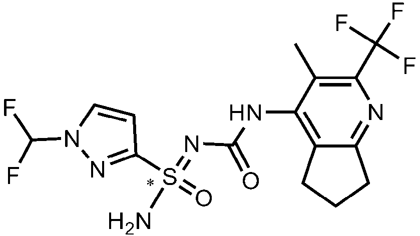 | **688b** | 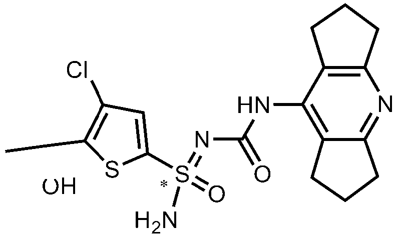 |
| **621b** | 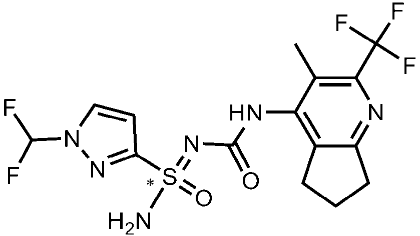 | **689** | 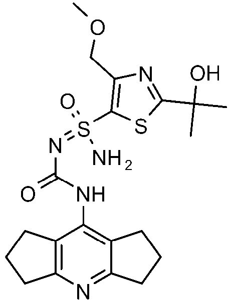 |
| **622** | 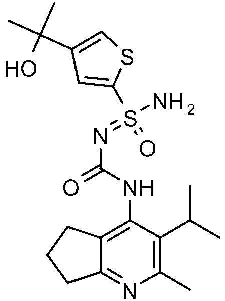 | **689a** | 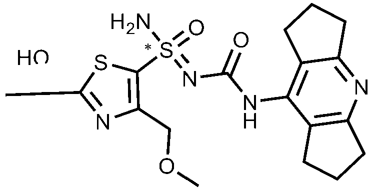 |
| **622a** | 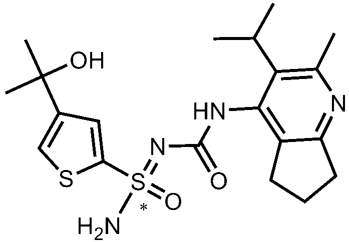 | **689b** | 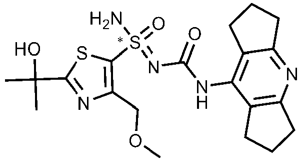 |
| **622b** | 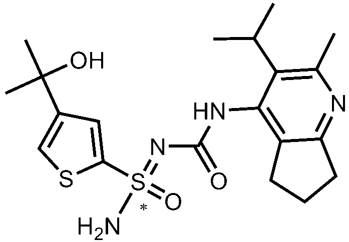 | **690** | 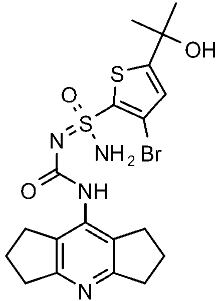 |
| **623** | 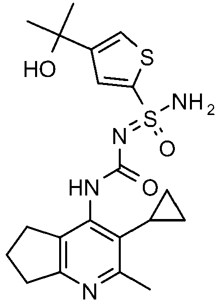 | **690a** | 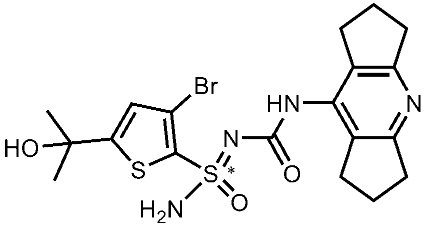 |
| **623a** | 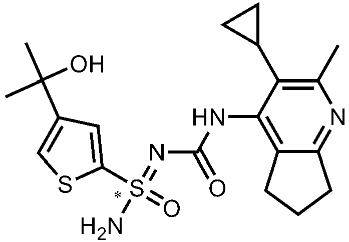 | **690b** | 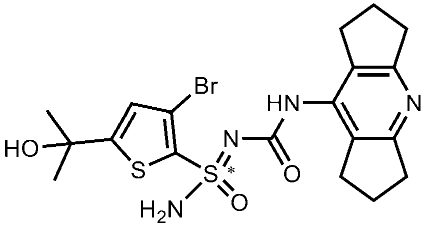 |
| **623b** | 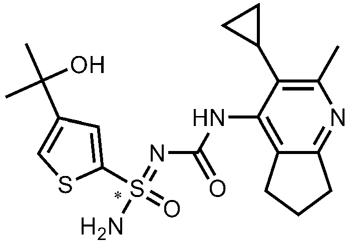 | **691** | 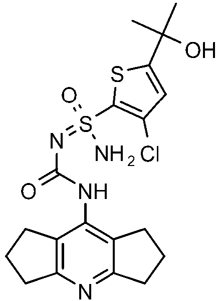 |
| **624** | 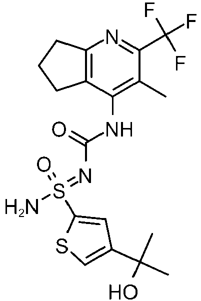 | **691a** | 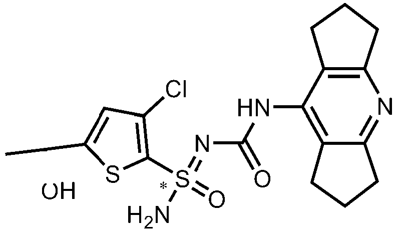 |
| **624a** | 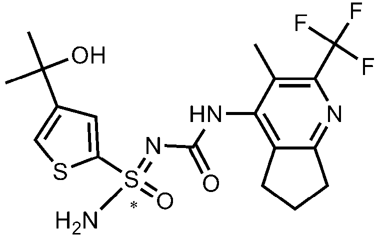 | **691b** | 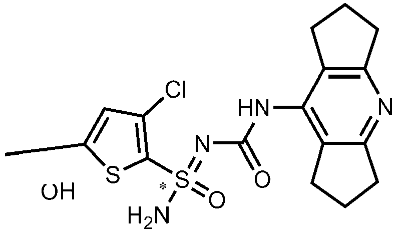 |
| **624b** | 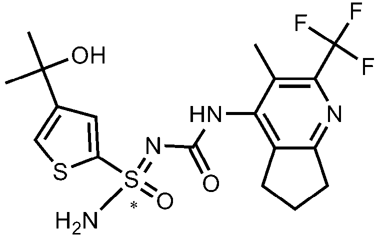 | **692** | 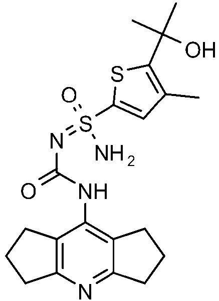 |
| **625** | 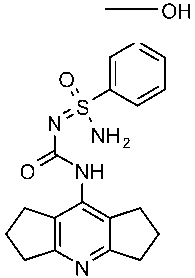 | **692a** | 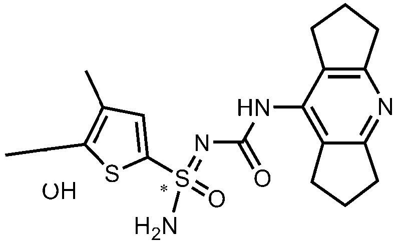 |
| **625a** | 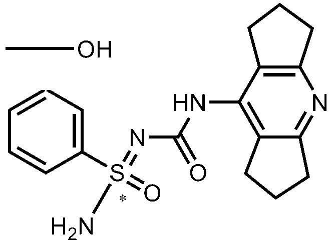 | **692b** | 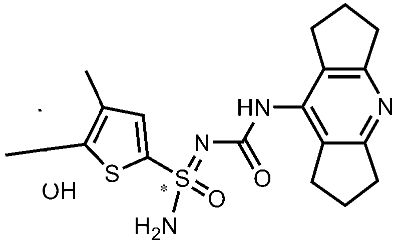 |
| **625b** | 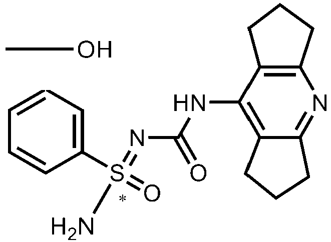 | **693** | 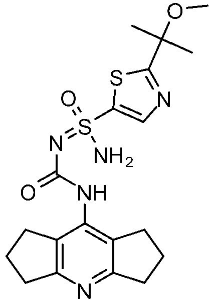 |
| **626** | 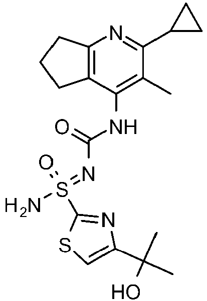 | **694** | 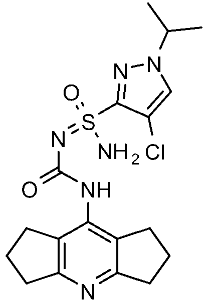 |
| **626a** | 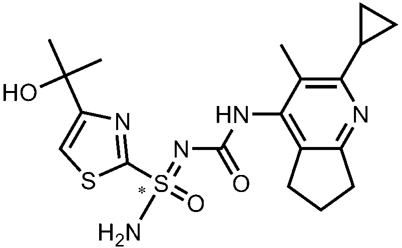 | **694a** | 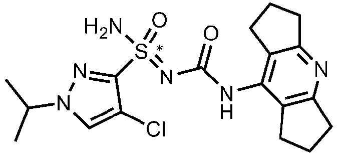 |
| **626b** | 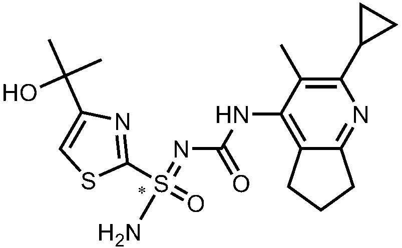 | **694b** | 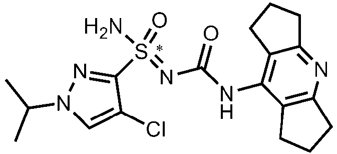 |
| **627** | 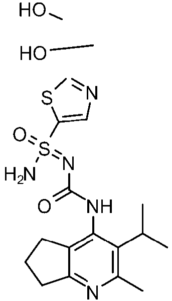 | **695** | 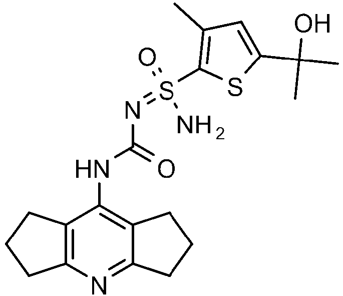 |
| **628** | 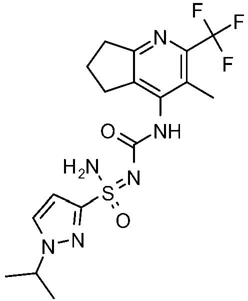 | **695a** | 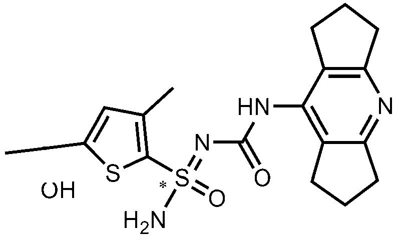 |
| **628a** | 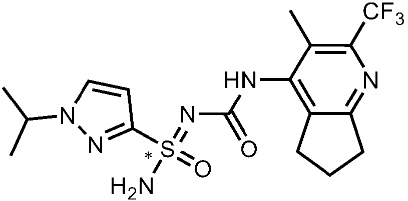 | **695b** | 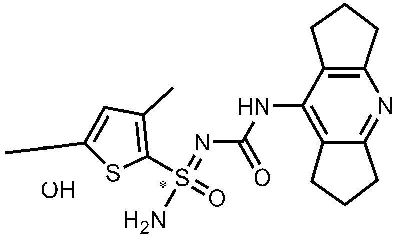 |
| **628b** | 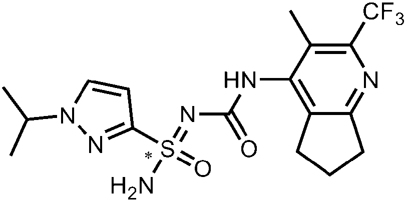 | **696** | 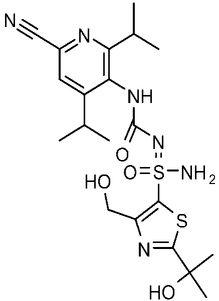 |
| **629** | 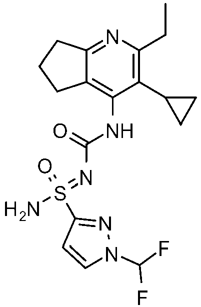 | **697a** | 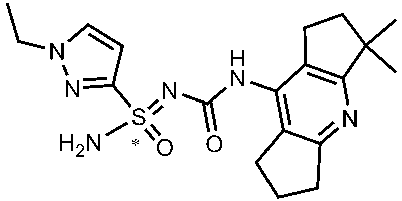 |
| **630** | 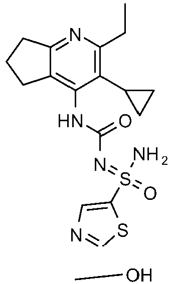 | **697b** | 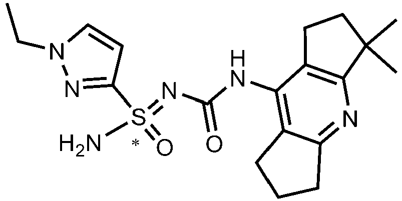 |
| **630a** | 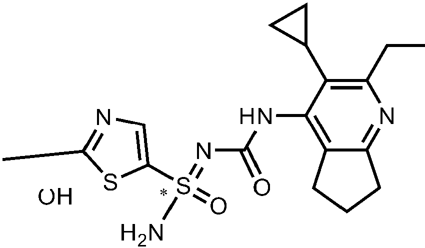 | **698a** | 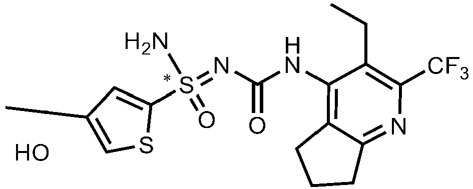 |
| **630b** | 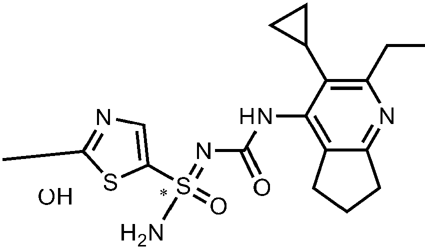 | **698b** | 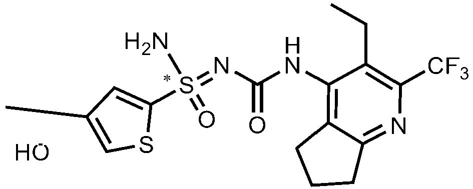 |
| **631** | 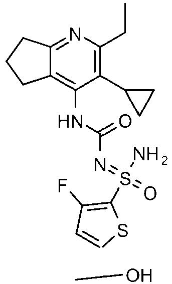 | **699a** | 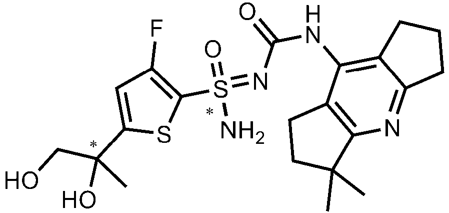 |
| **631a** | 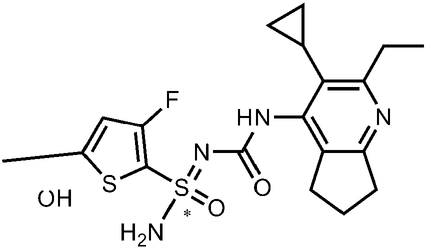 | **699b** | 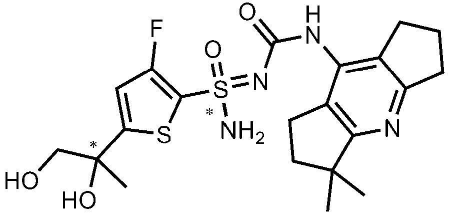 |
| **631b** | 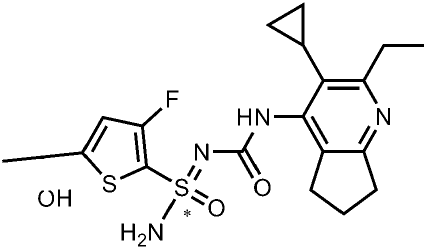 | **699c** | 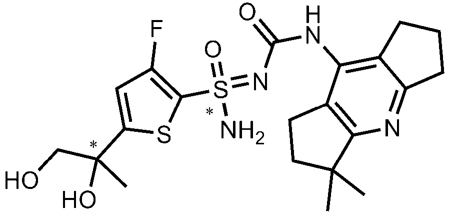 |
| **632** | 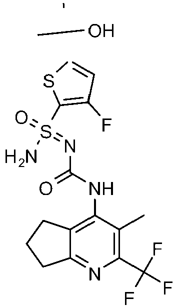 | **700a** | 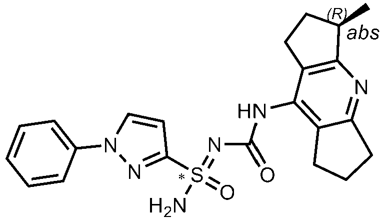 |
| **632a** | 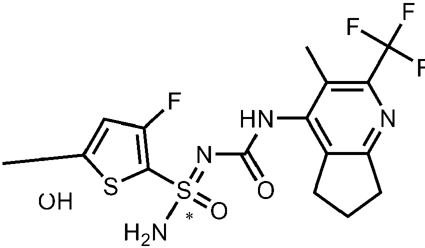 | **700b** | 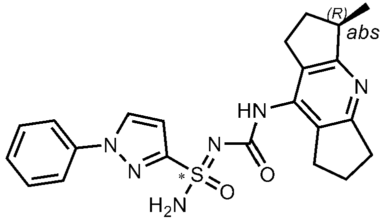 |
| **632b** | 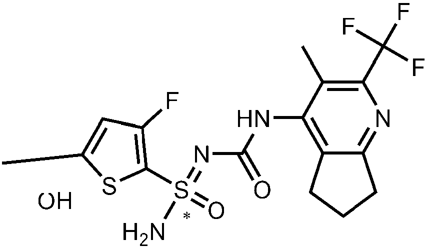 | **701a** | 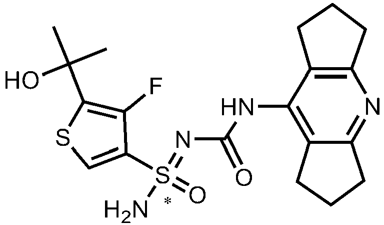 |
| **633** | 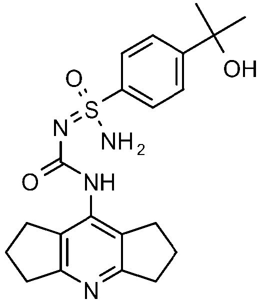 | **701b** | 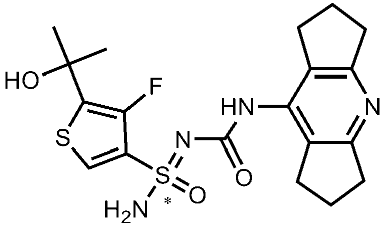 |
| **633a** | 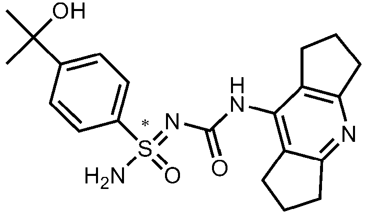 | **702** | 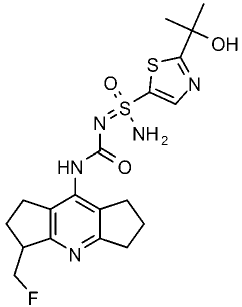 |
| **633b** | 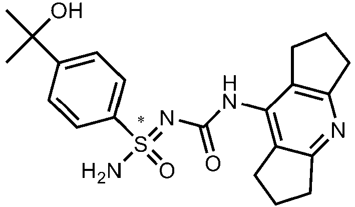 | **703** | 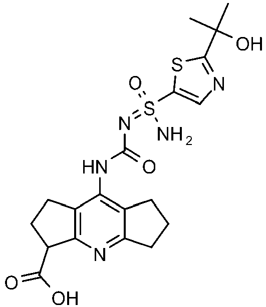 |
| **634** | 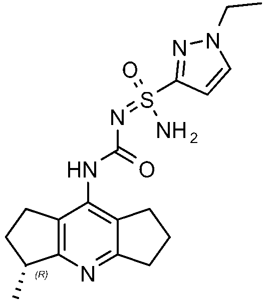 | **704** | 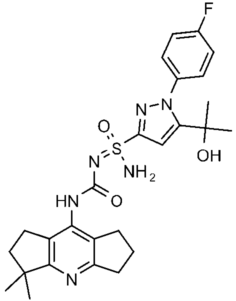 |
| **634a** | 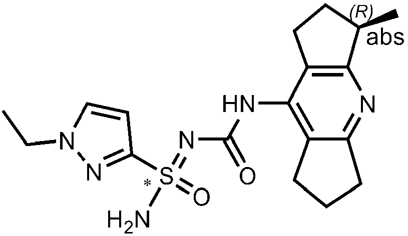 | **705a** | 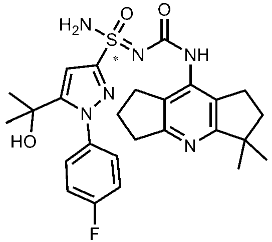 |
| **634b** | 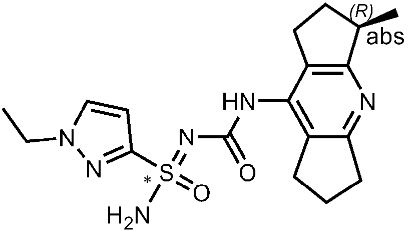 | **705b** | F |
| **635** | 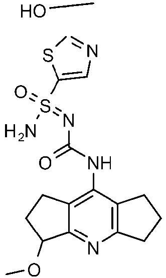 | **706** | 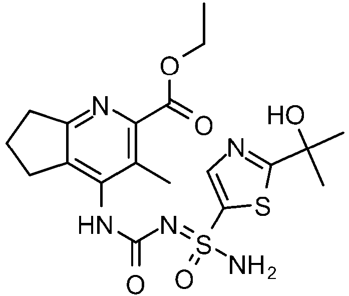 |
| **636a** | 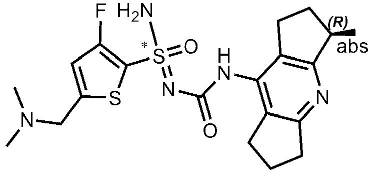 | **707** | 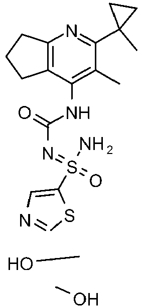 |
| **636b** | 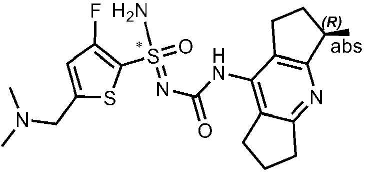 | **708** | 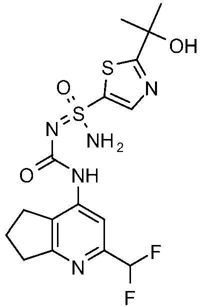 |
| **636c** | 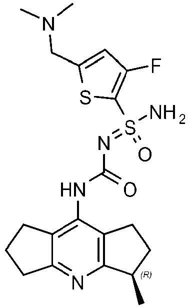 | **709** | 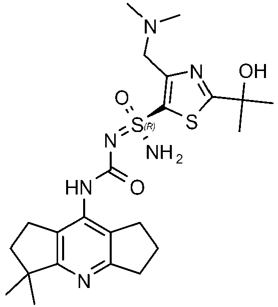 |
| **637** | 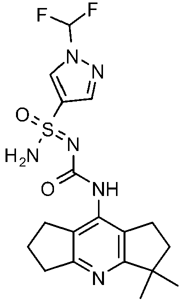 | **710** | 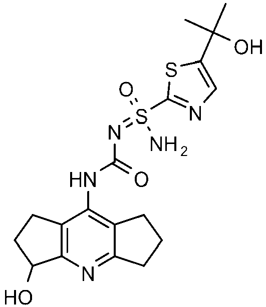 |
| **637a** | 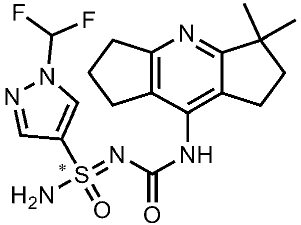 | **711** | 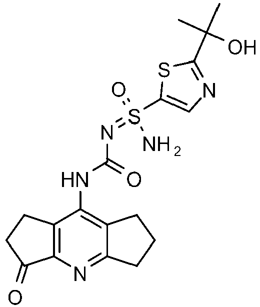 |
| **637b** | 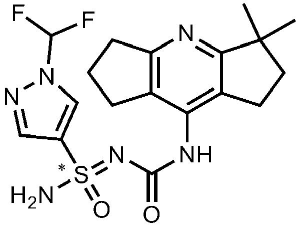 | **712** | 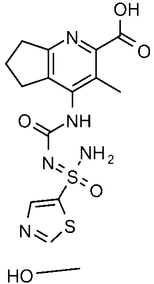 |
| **638** | 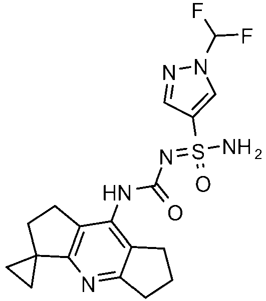 | **713** | 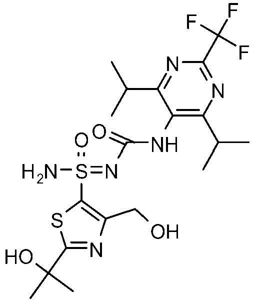 |
| **638a** | 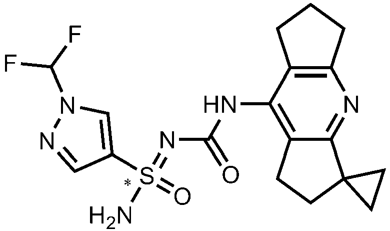 | **716a** | 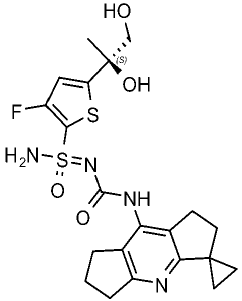 |
| **638b** | 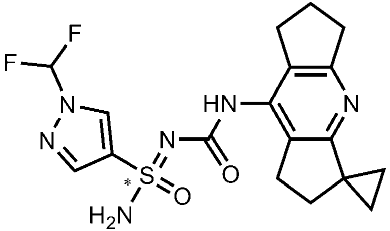 | **716b** | 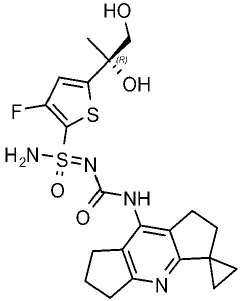 |
| **639** | 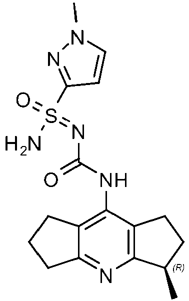 | **717** | 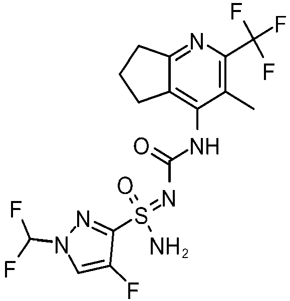 |
| **639a** | 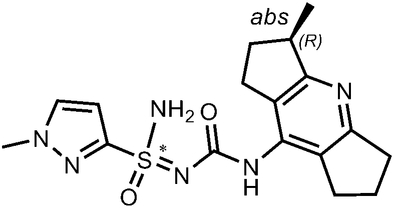 | **718a** | 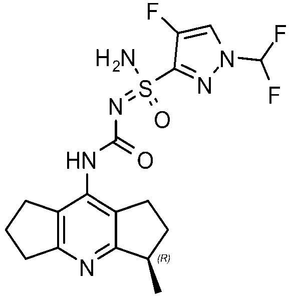 |
| **639b** | 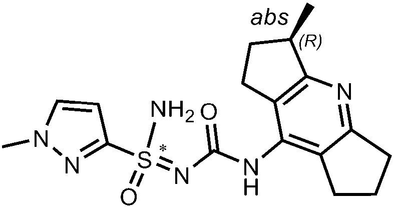 | **719** | 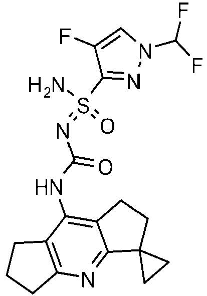 |
| **640** | 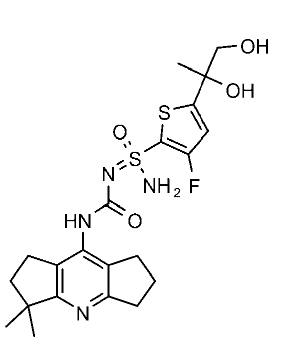 | **720a** | 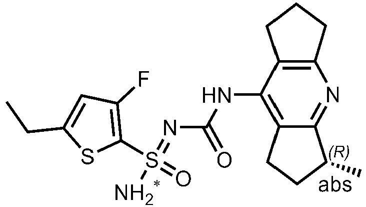 |
| **640a** | 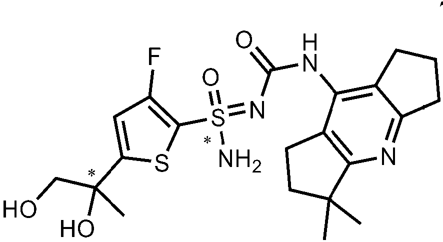 | **720b** | 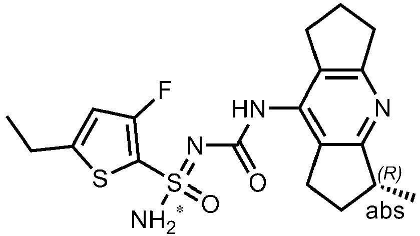 |
| **640b** | 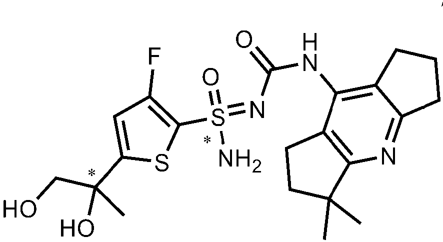 | **720c** | 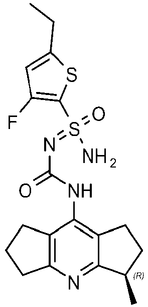 |
| **640c** | 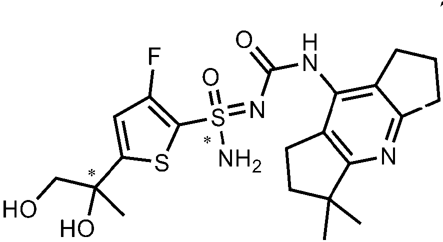 | **721a** | 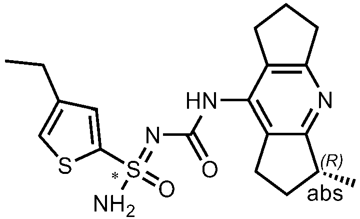 |
| **641** | 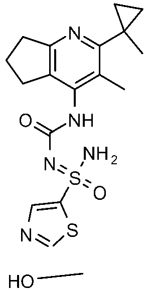 | **721b** | 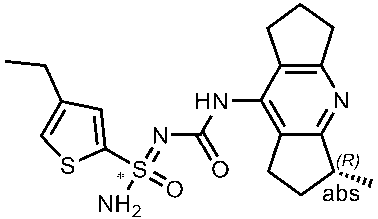 |
| **642** | 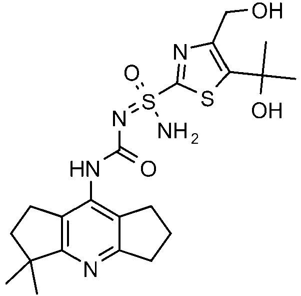 | **721c** | 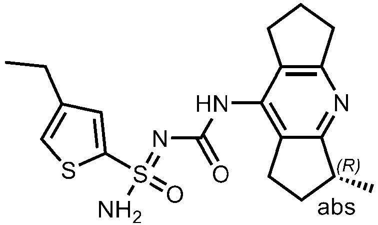 |
| **642a** | 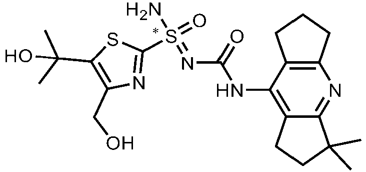 | **722** | 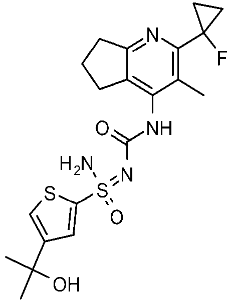 |
| **642b** | 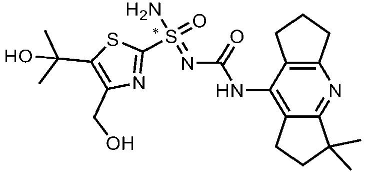 | **723a** | 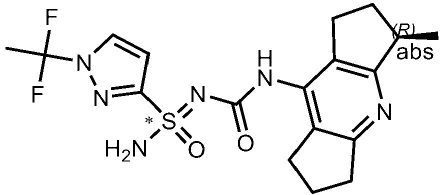 |
| **643** | 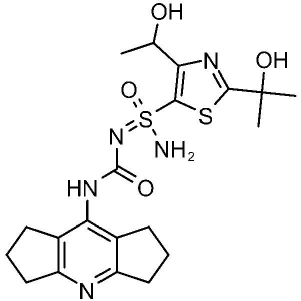 | **723b** | 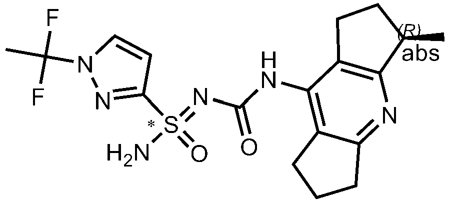 |
| **643a** | 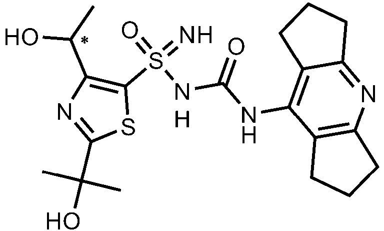 | **723c** | 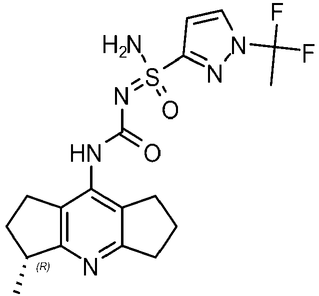 |
| **643b** | 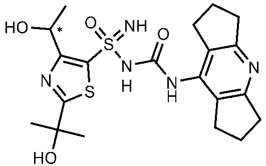 | **724** | 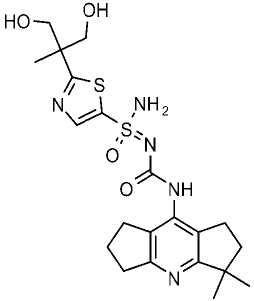 |
| **644** | 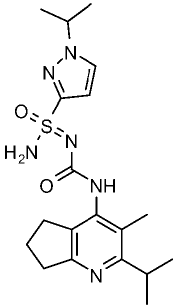 | **725a** | 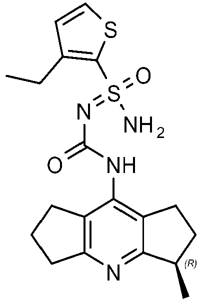 |
| **644a** | 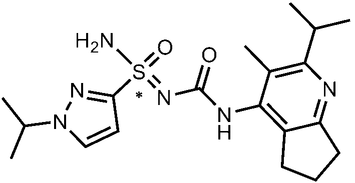 | **726** | 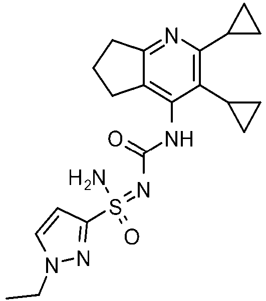 |
| **644b** | 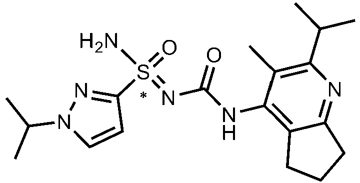 | **726a** | 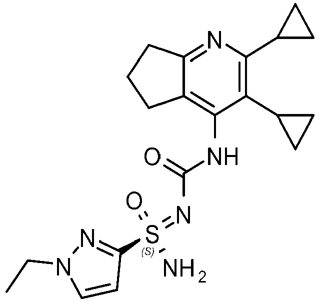 |
| **645** | 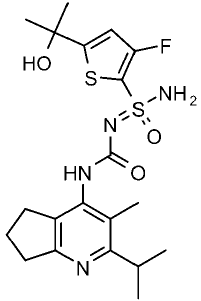 | **726b** | 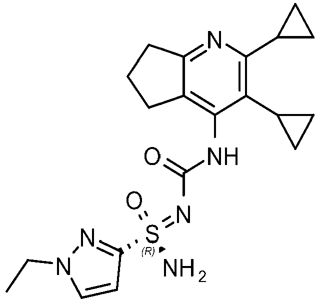 |
| **645a** | 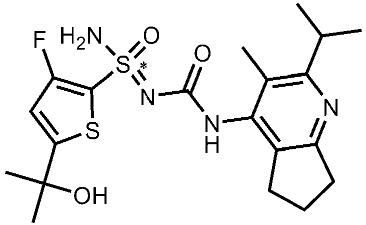 | **727** | 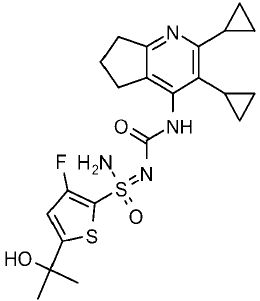 |
| **645b** | 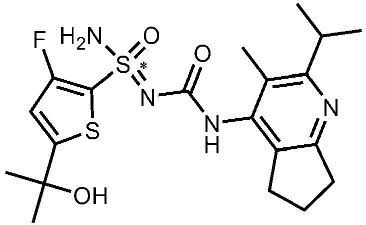 | **727a** | 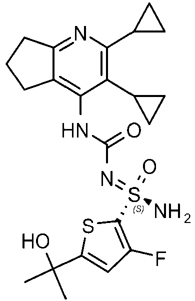 |
| **646a** | 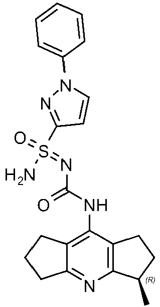 | **727b** | 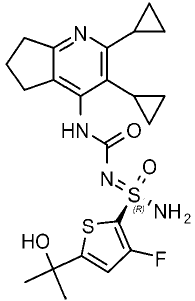 |
| **647** | 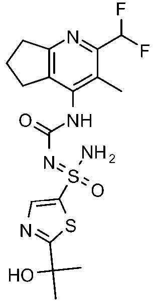 | **728** | 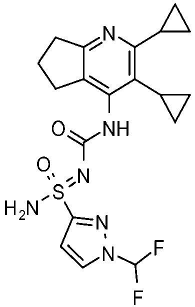 |
| **647a** | 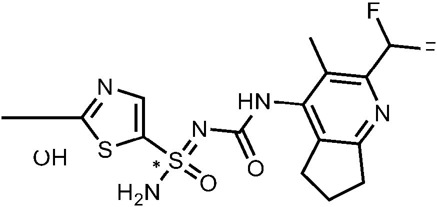 | **728a** | 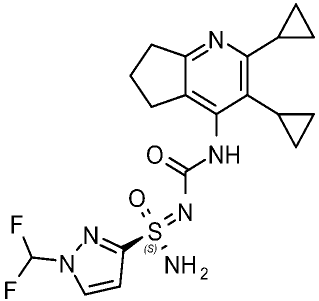 |
| **647b** | 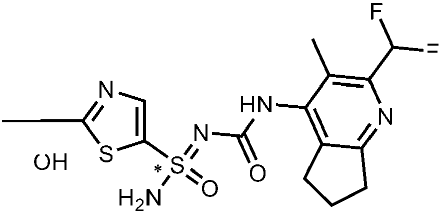 | **728b** | 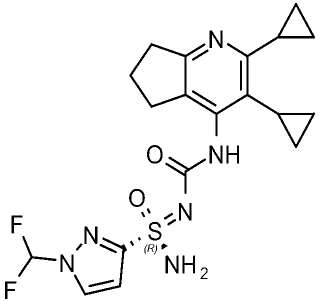 |
| **648** | 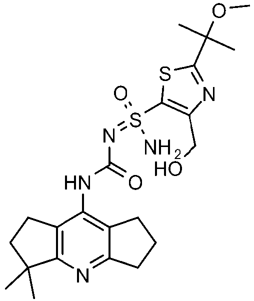 | **729** | 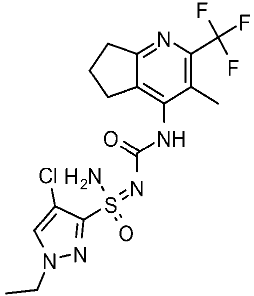 |
| **648a** | 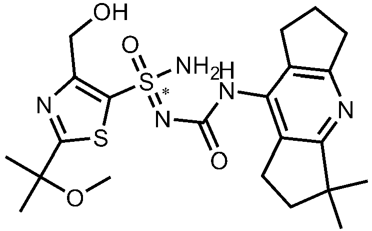 | **729a** | 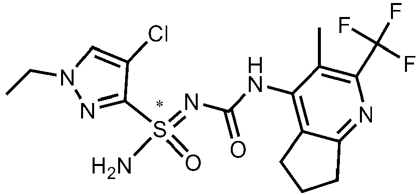 |
| **648b** | 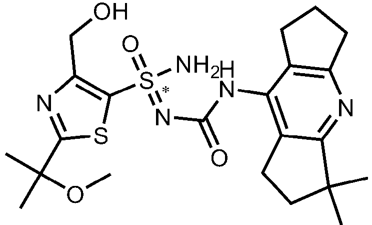 | **729b** | 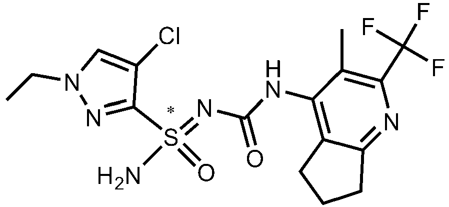 |
| **649** | 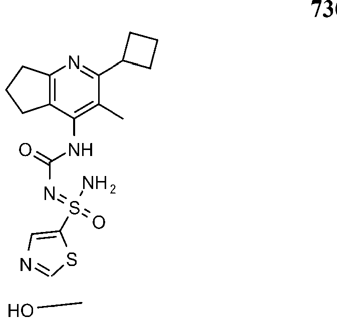 | **730** | 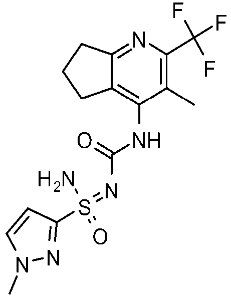 |
| **649a** | 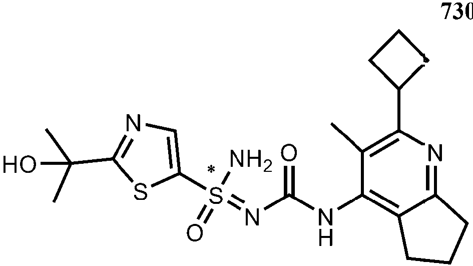 | **730a** | 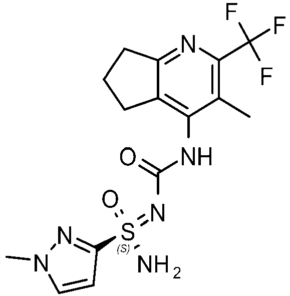 |
| **649b** | 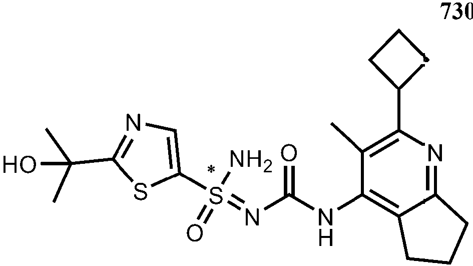 | **730b** | 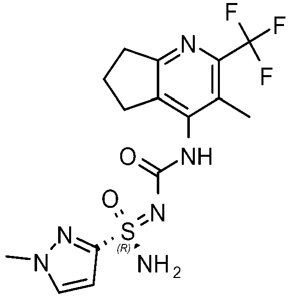 |
| **650** | 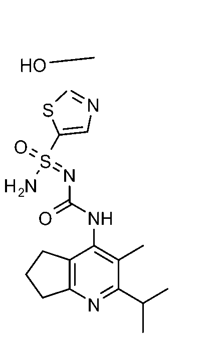 | **731** | 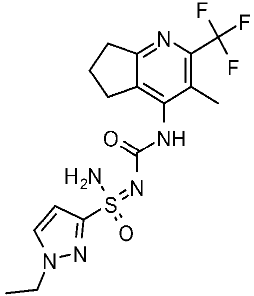 |
| **650a** | 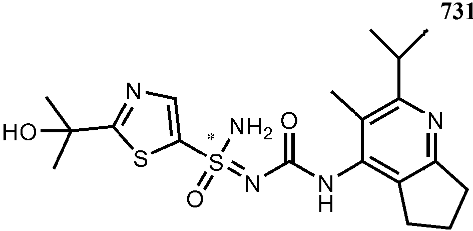 | **731** | 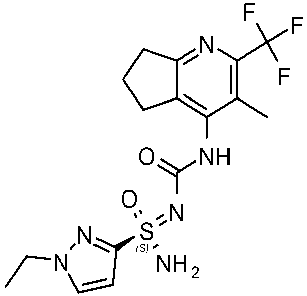 |
| **650b** | 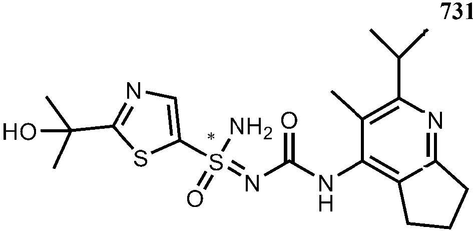 | **731** | 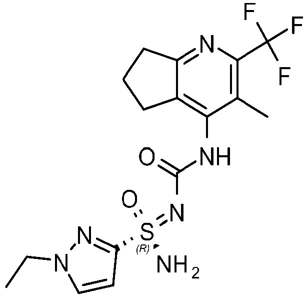 |
| **651** | 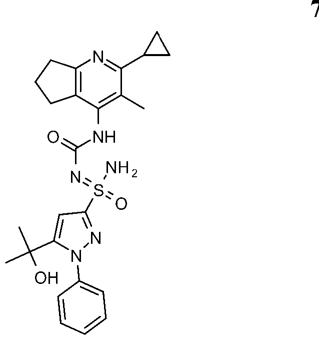 | **732a** | 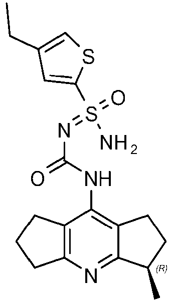 |
| **651a** | 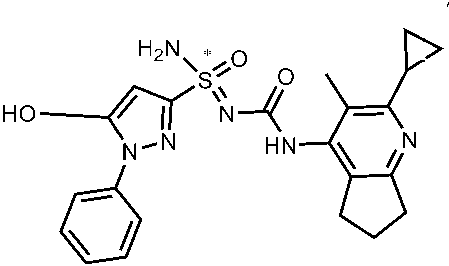 | **733** | 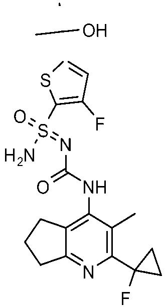 |
| **651b** | 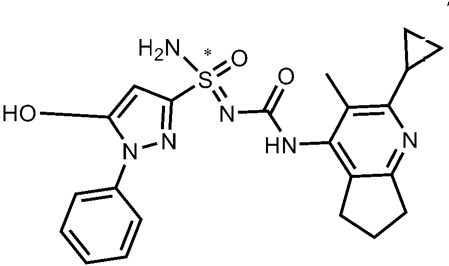 | **734** | 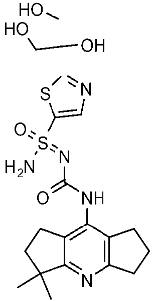 |
| **652** | 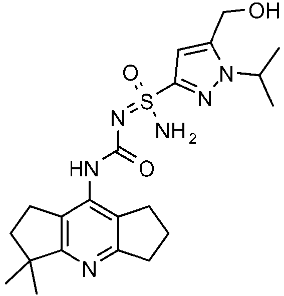 | **735** | 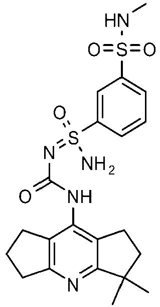 |
| **652a** | 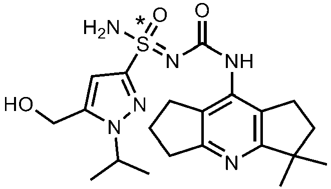 | **736** | 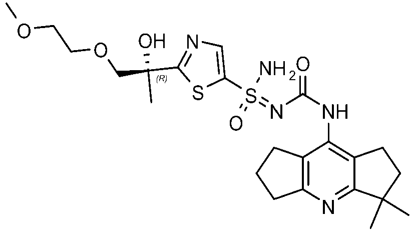 |
| **652b** | 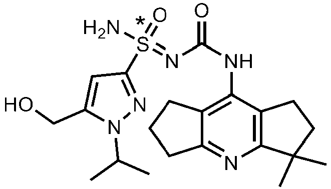 | **737** | 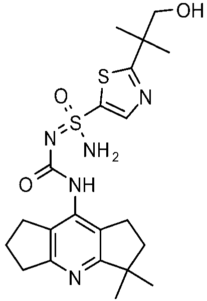 |
| **653** | 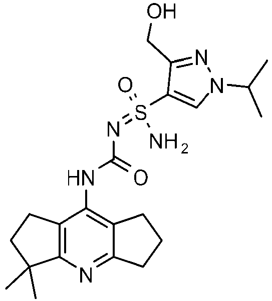 | **738** | 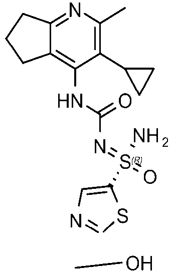 |
| **653a** | 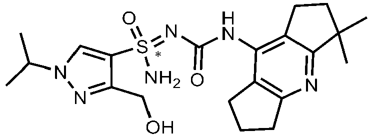 | **739** | 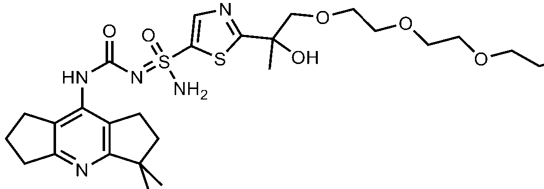 |
| **653b** | 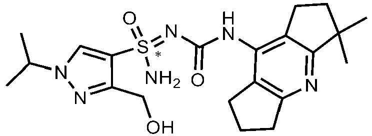 | **739a** | 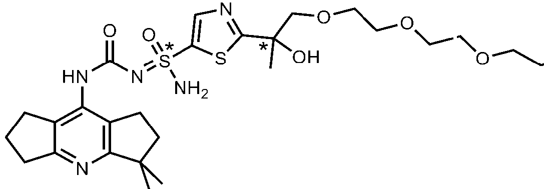 |
| **654** | 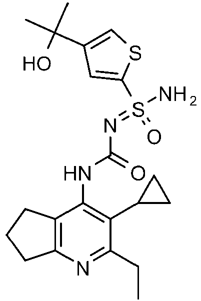 | **739b** | 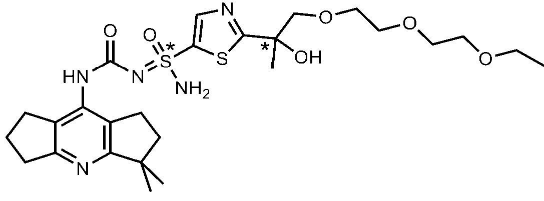 |
| **654a** | 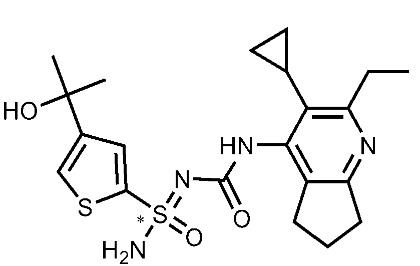 | **739c** | 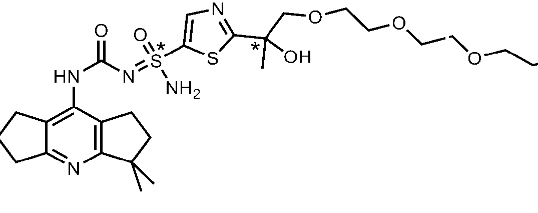 |
| **654b** | 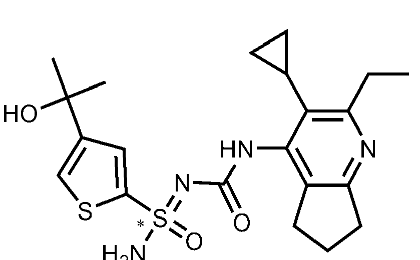 | **739d** | 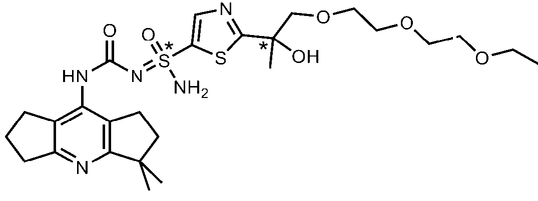 |
| **655** | 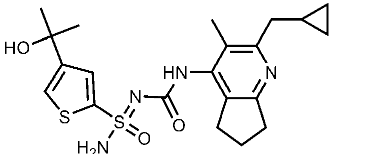 | **740** | 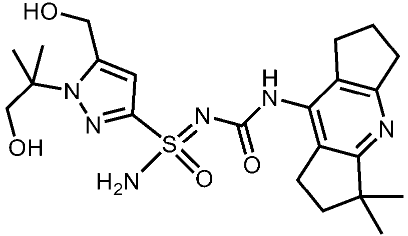 |
| **655a** | 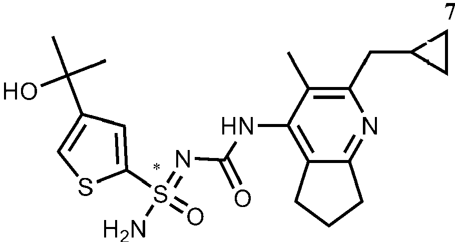 | **740a** | 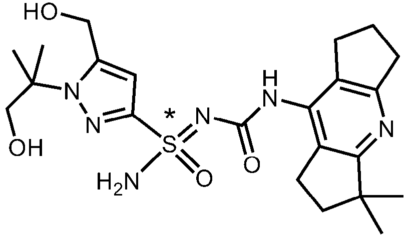 |
| **655b** | 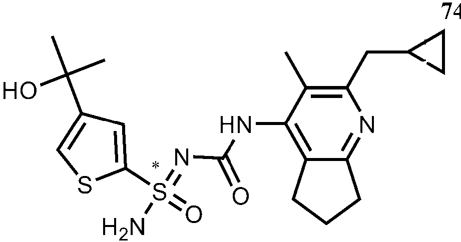 | **740b** | 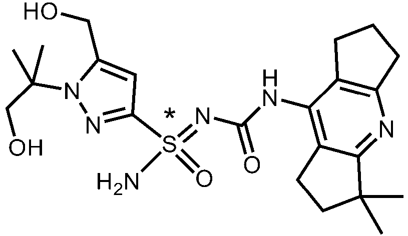 |
| **656** | 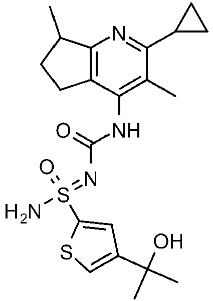 | **741** | 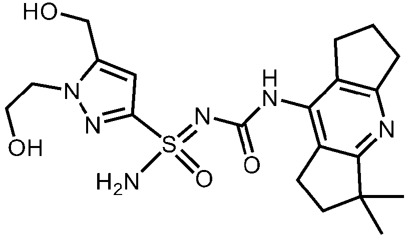 |
| **656a** | 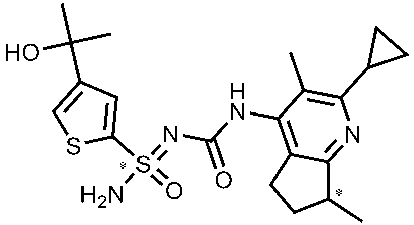 | **741a** | 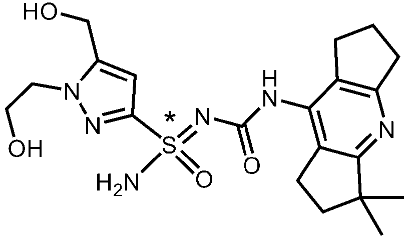 |
| **656b** | 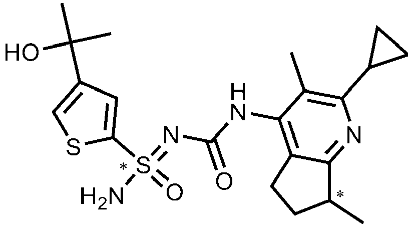 | **741b** | 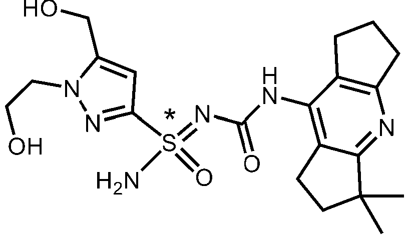 |
| **656c** | 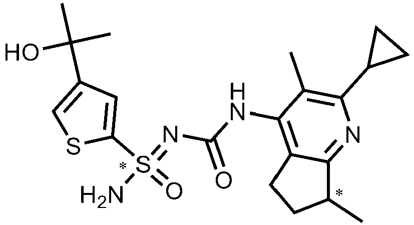 | **742** | 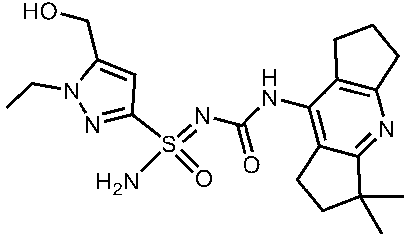 |
| **656d** | 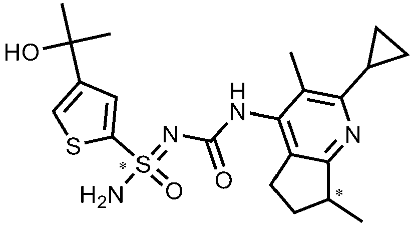 | **742a** | 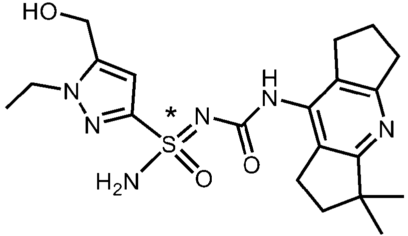 |
| **657** | 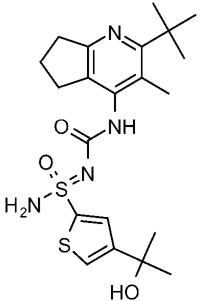 | **742b** | 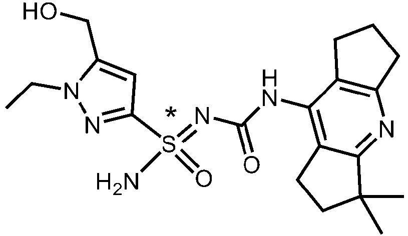 |
| **657a** | 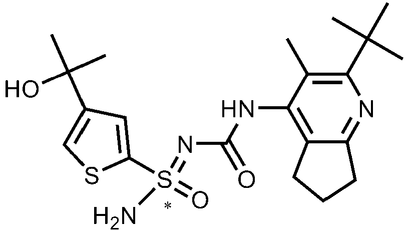 | **743** | 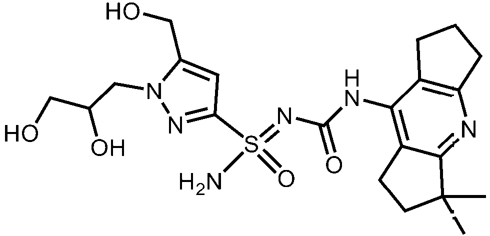 |
| **657b** | 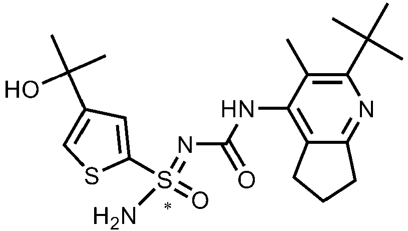 | **743a** | 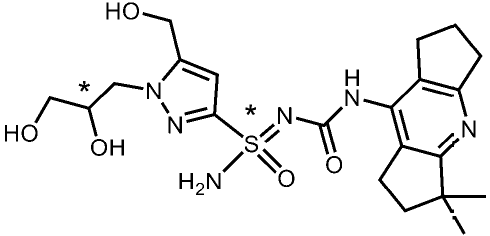 |
| **658** | 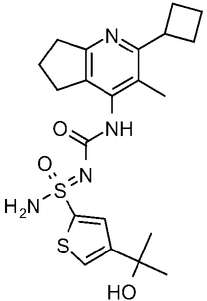 | **743b** | 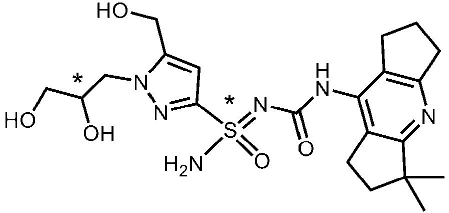 |
| **658a** | 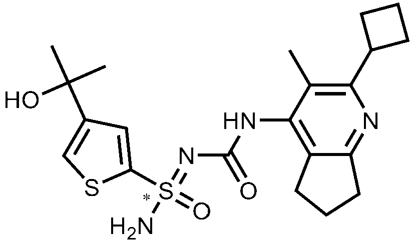 | **743c** | 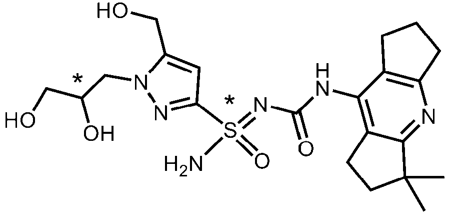 |
| **658b** | 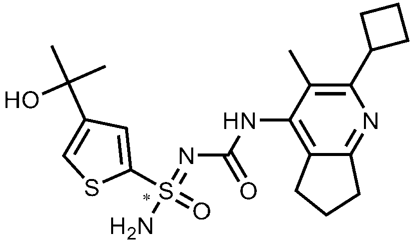 | **743d** | 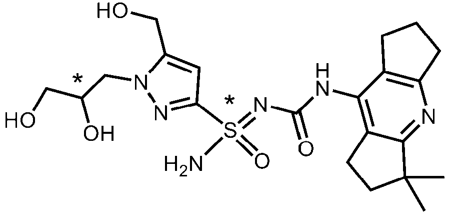 |
| **659** | 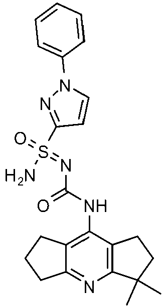 | **744** | 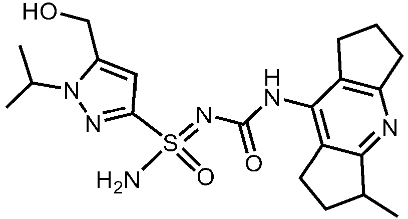 |
| **659a** | 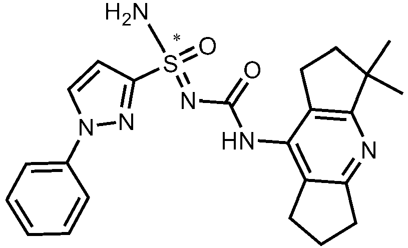 | **744a** | 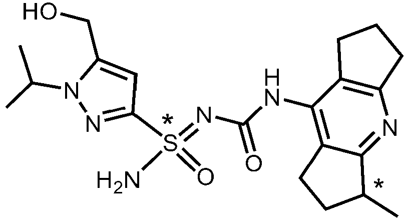 |
| **659b** | 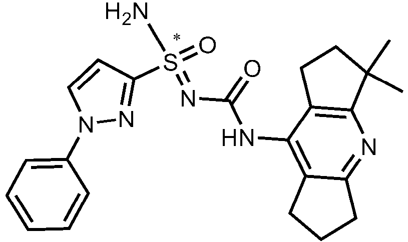 | **744b** | 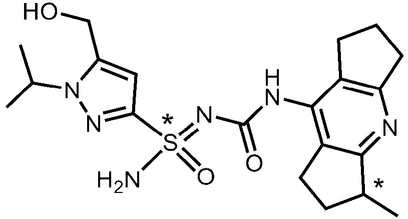 |
| | | **744c** | 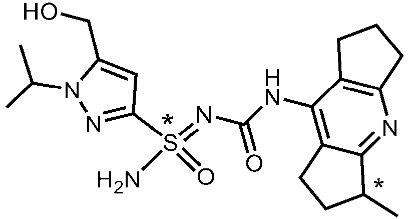 |
| | | **744d** | 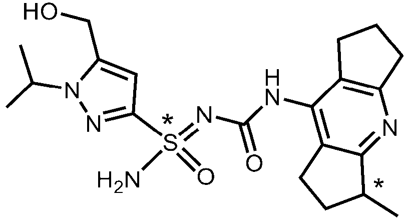 |

and pharmaceutically acceptable salts thereof.

In some embodiments, the compound is selected from the group consisting of compounds in **Table 1D** below

**Table 1D**

| **Cmpd #** | **Structure** | **Cmpd #** | **Structure** |
|---|---|---|---|
| **801** | 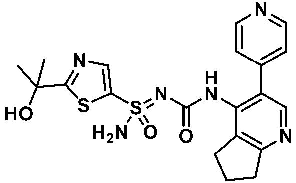 | **821a** | 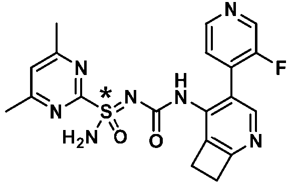 |
| **801a** | 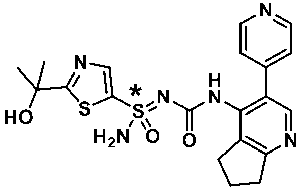 | **821b** | 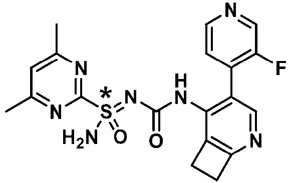 |
| **801b** | 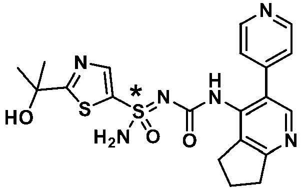 | **822** | 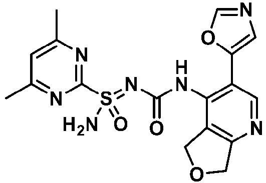 |
| **802** | 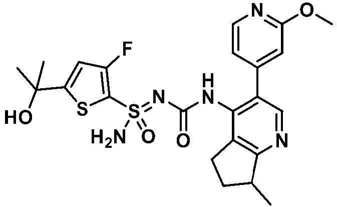 | **822a** | 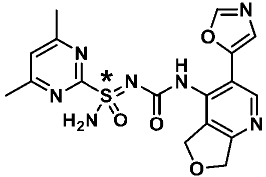 |
| **802a** | 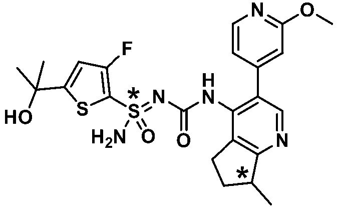 | **822b** | 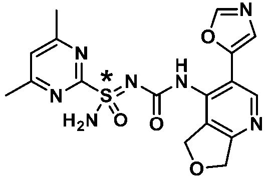 |
| **802b** | 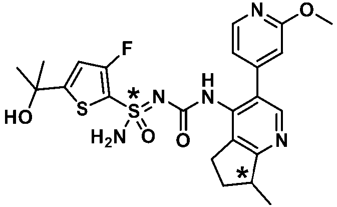 | **823** | 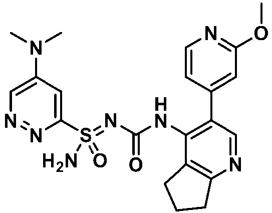 |
| **802c** | 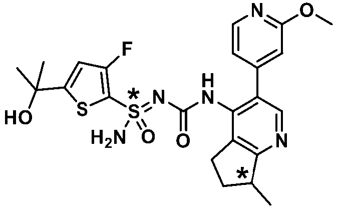 | **823a** | 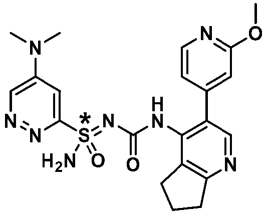 |
| **802d** | 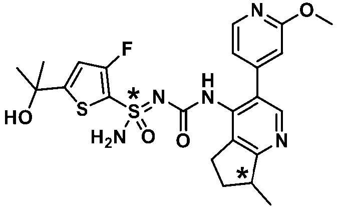 | **823b** | 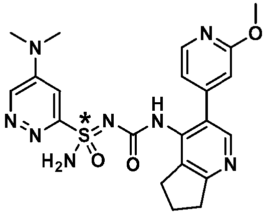 |
| **803** | 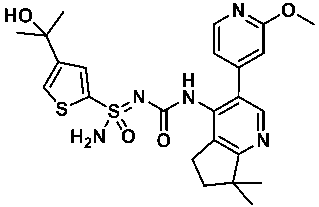 | **824** | 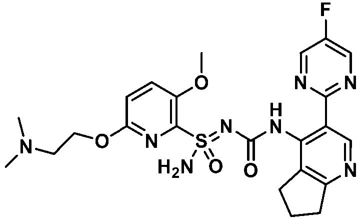 |
| **803a** | 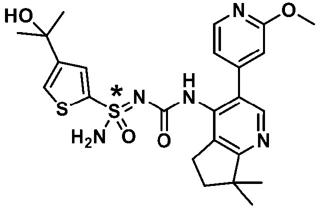 | **824a** | 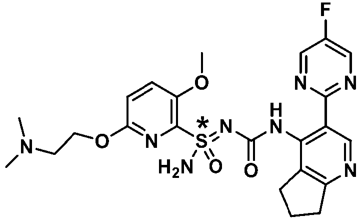 |
| **803b** | 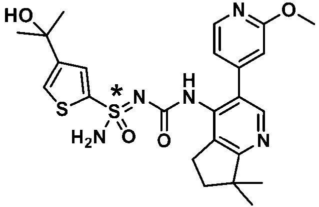 | **824b** | 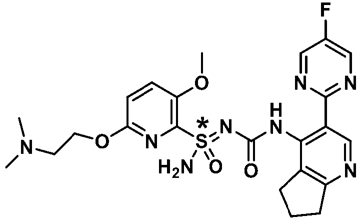 |
| **804** | 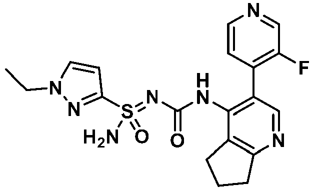 | **825** | 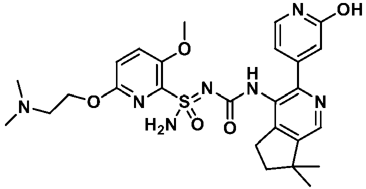 |
| **804a** | 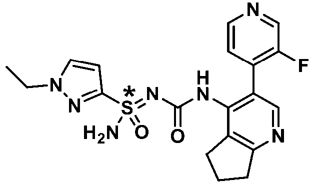 | **825a** | 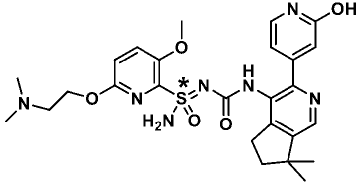 |
| **804b** | 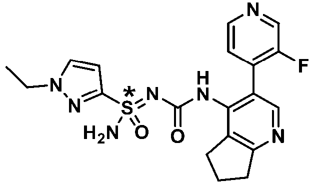 | **825b** | 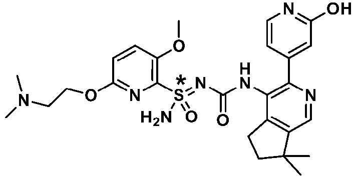 |
| **805** | 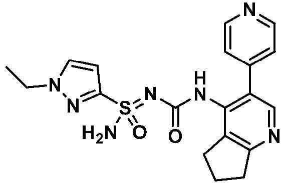 | **826** | 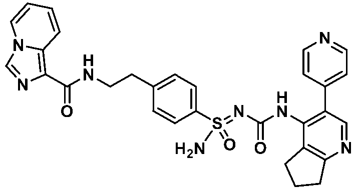 |
| **805a** | 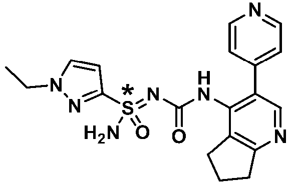 | **826a** | 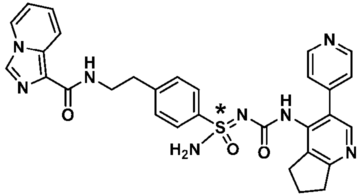 |
| **805b** | 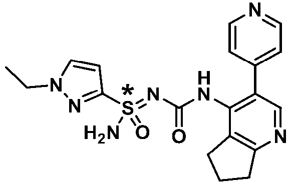 | **826b** | 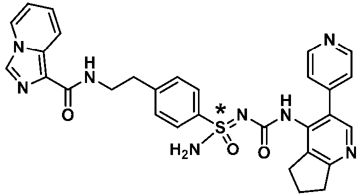 |
| **806** | 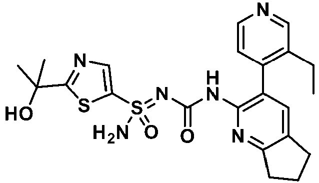 | **827** | 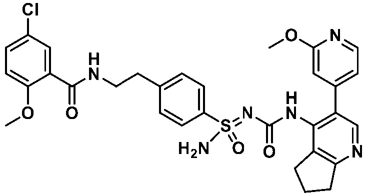 |
| **806a** | 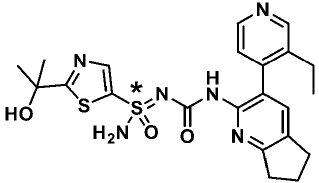 | **827a** | 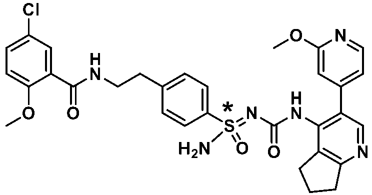 |
| **806b** | 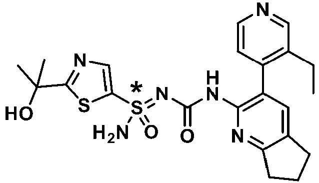 | **827b** | 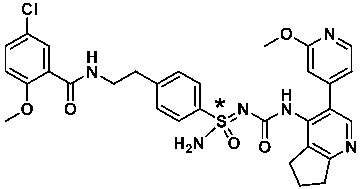 |
| **807** | 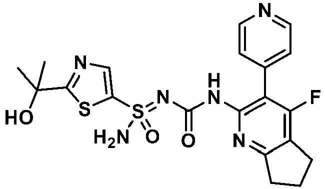 | **828** | 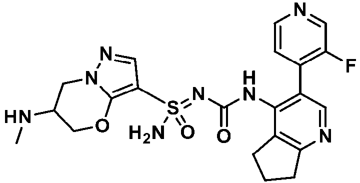 |
| **807a** | 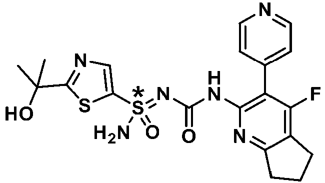 | **828a** | 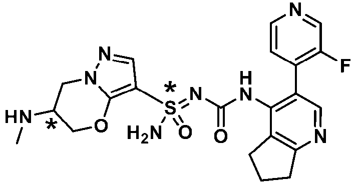 |
| **807b** | 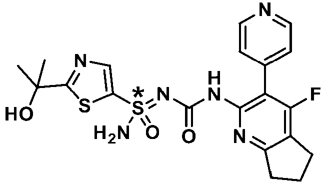 | **828b** | 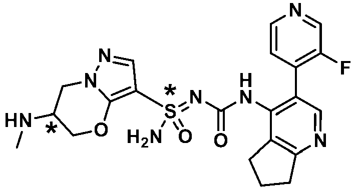 |
| **808** | 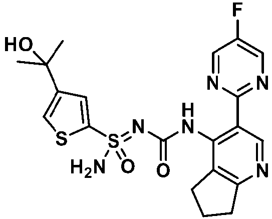 | **828c** | 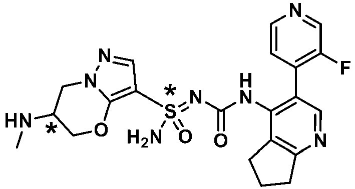 |
| **808a** | 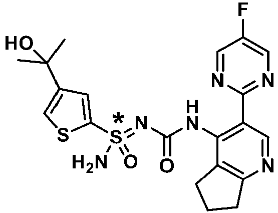 | **828d** | 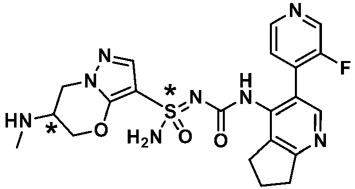 |
| **808b** | 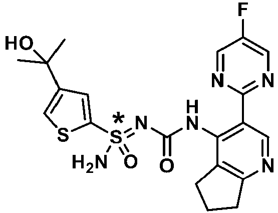 | **829** | 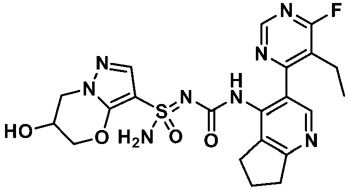 |
| **809** | 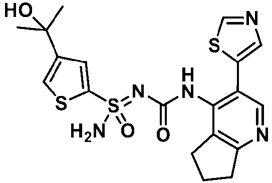 | **829a** | 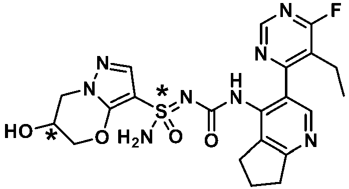 |
| **809a** | 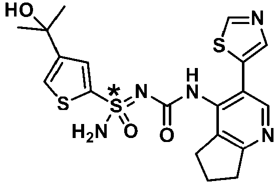 | **829b** | 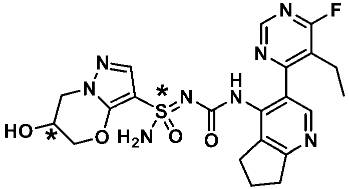 |
| **809b** | 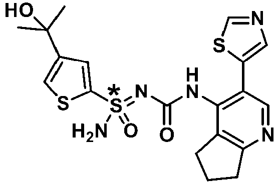 | **829c** | 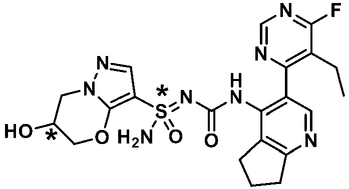 |
| **810** | 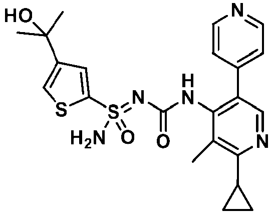 | **829d** | 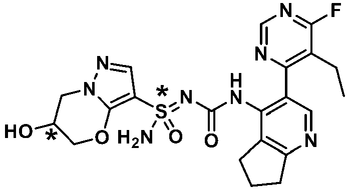 |
| **811a** | 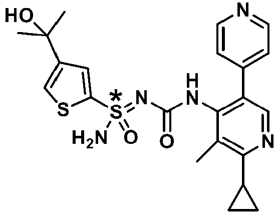 | **830** | 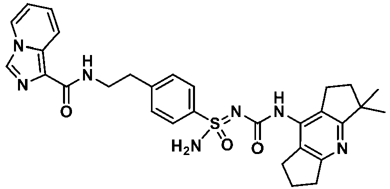 |
| **811b** | 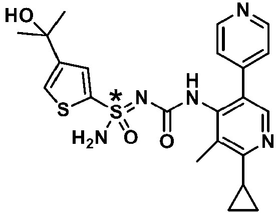 | **830a** | 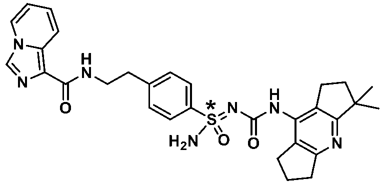 |
| **811** | 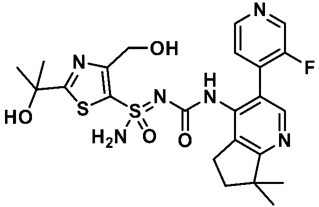 | **830b** | 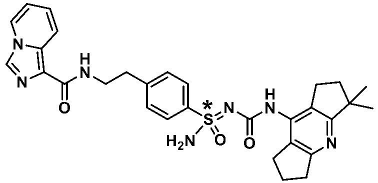 |
| **811a** | 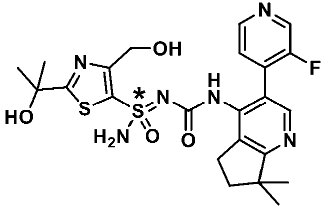 | **831** | 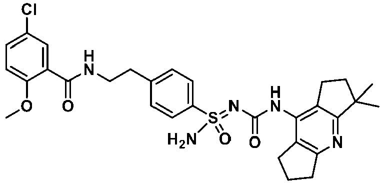 |
| **811b** | 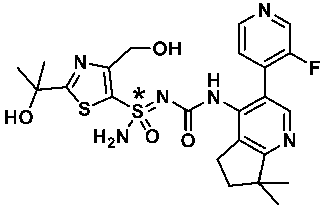 | **831a** | 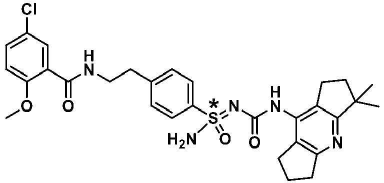 |
| **812** | 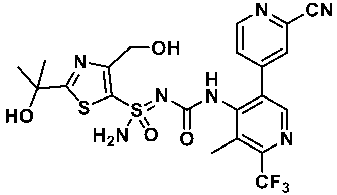 | **831b** | 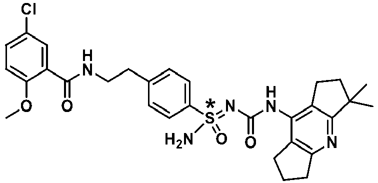 |
| **812a** | 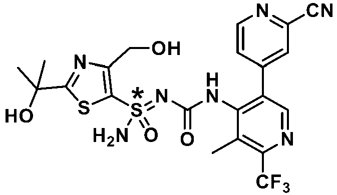 | **832** | 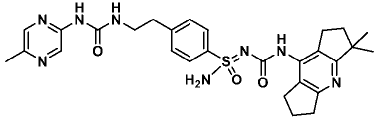 |
| **812b** | 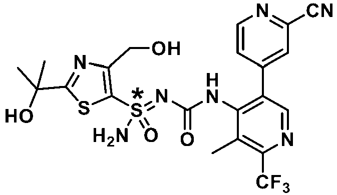 | **832a** | 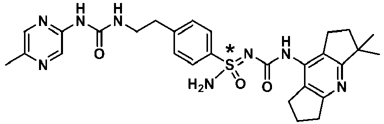 |
| **813** | 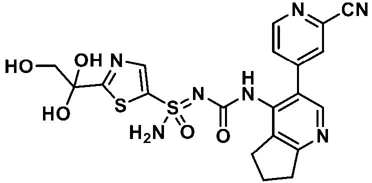 | **832b** | 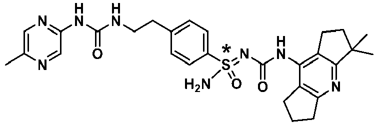 |
| **813a** | 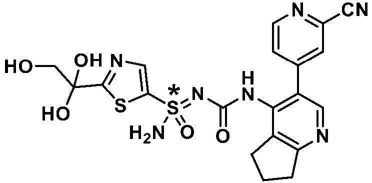 | **833** | 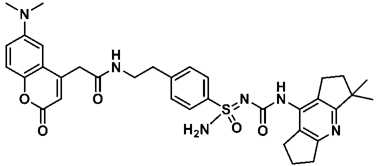 |
| **813b** | 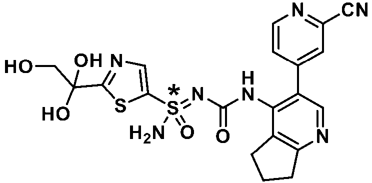 | **833a** | 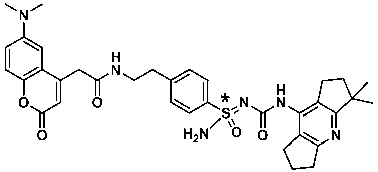 |
| **814** | 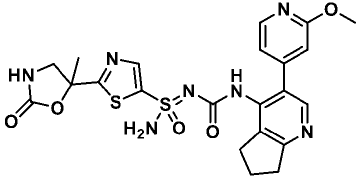 | **833b** | 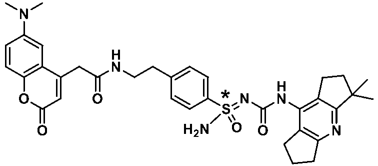 |
| **814a** | 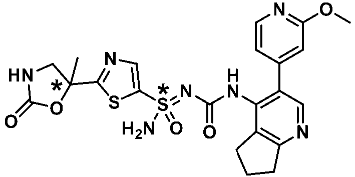 | **834** | 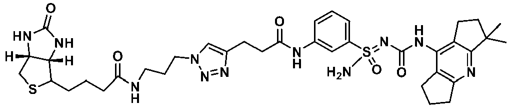 |
| **814b** | 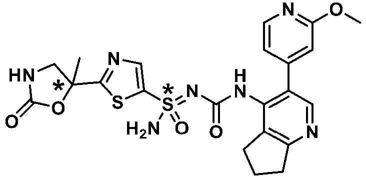 | **834a** | 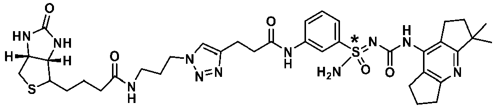 |
| **814c** | 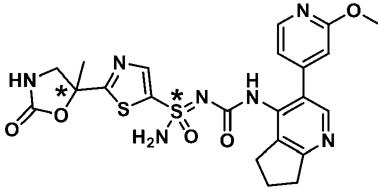 | **834b** | 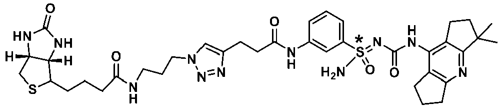 |
| **814d** | 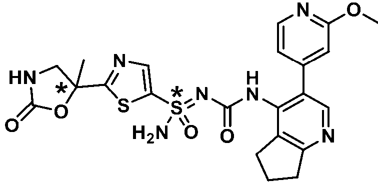 | **835** | 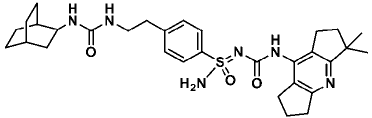 |
| **815** | 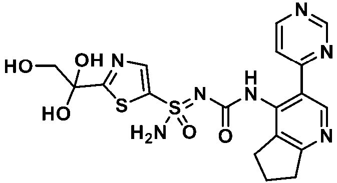 | **835a** | 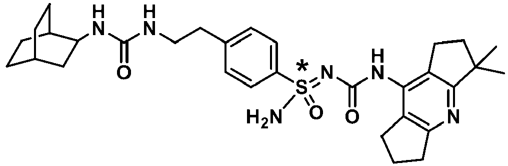 |
| **815a** | 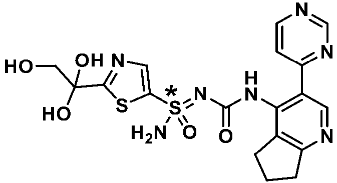 | **835b** | 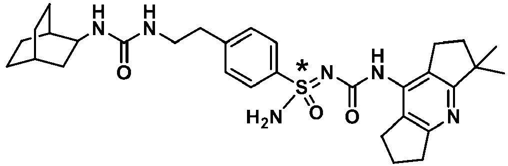 |
| **815d** | 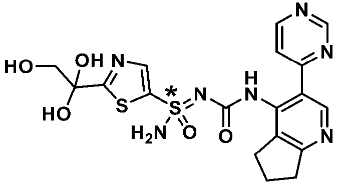 | **836** | 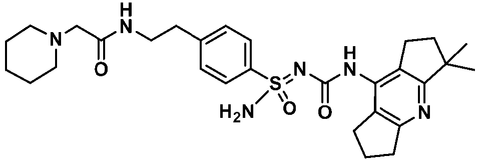 |
| **816** | 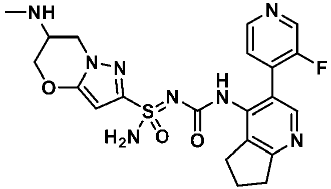 | **836a** | 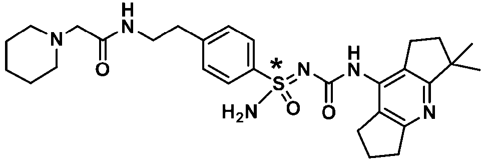 |
| **816a** | 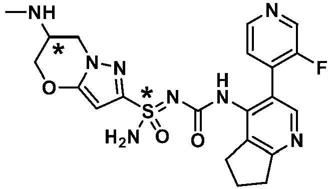 | **836b** | 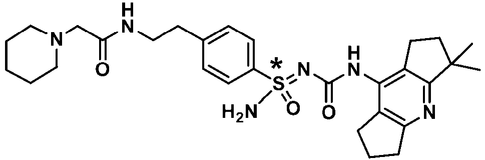 |
| **816b** | 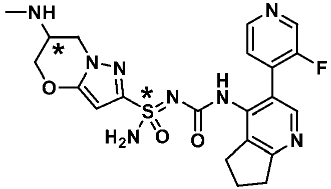 | **837** | 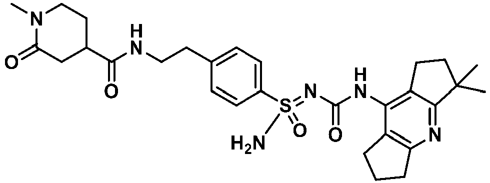 |
| **816c** | 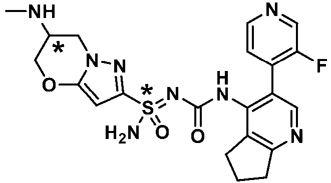 | **837a** | 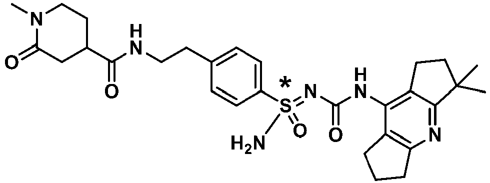 |
| **816d** | 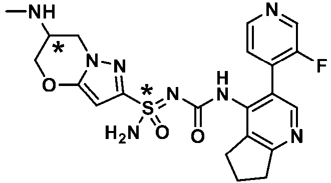 | **837b** | 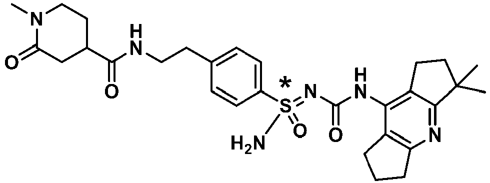 |
| **817** | 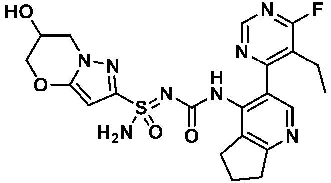 | **838** | 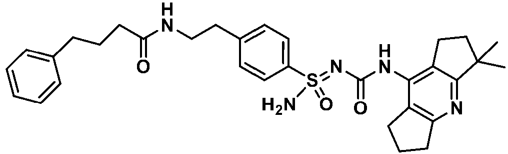 |
| **817a** | 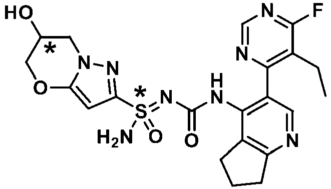 | **838a** | 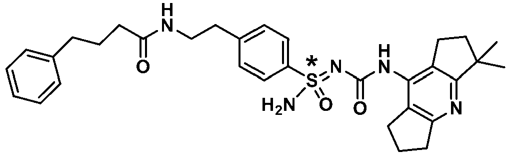 |
| **817b** | 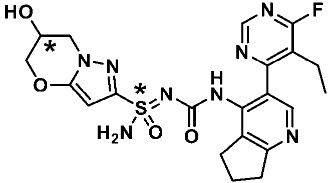 | **838b** | 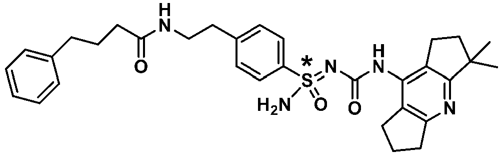 |
| **817c** | 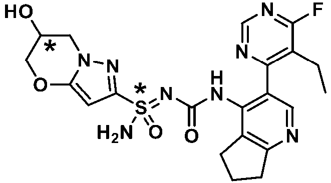 | **839** | 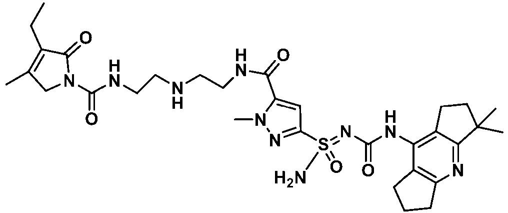 |
| **817d** | 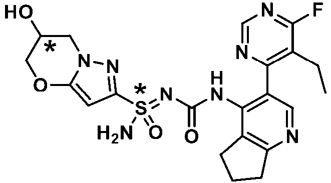 | **839a** | 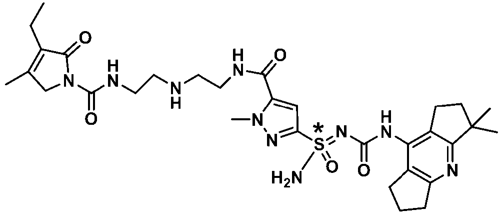 |
| **818** | 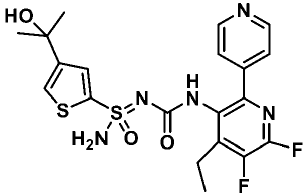 | **839b** | 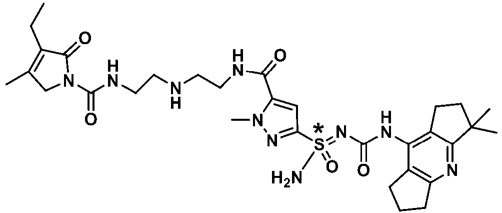 |
| **818a** | 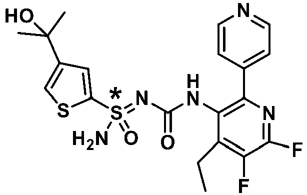 | **840** | 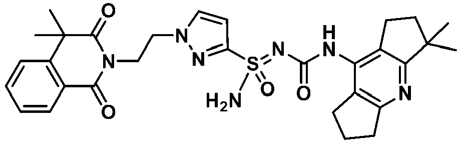 |
| **818b** | 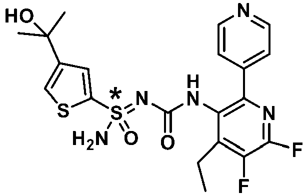 | **840a** | 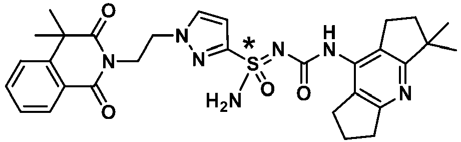 |
| **819** | 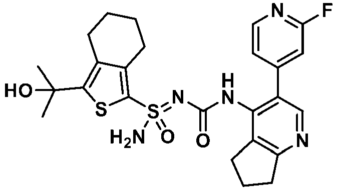 | **840b** | 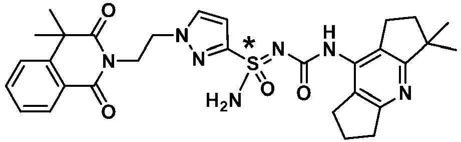 |
| **819a** | 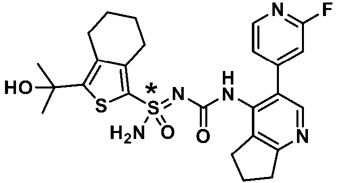 | **841** | 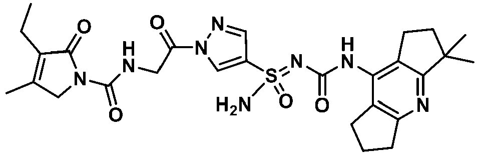 |
| **819b** | 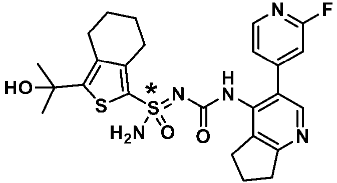 | **841a** | 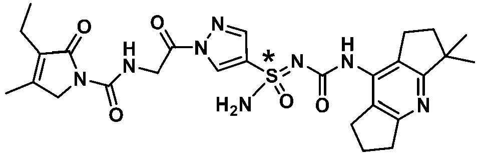 |
| **820** | 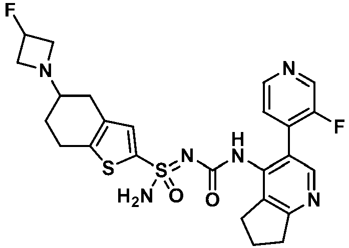 | **841b** | 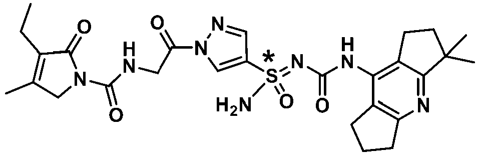 |
| **820a** | 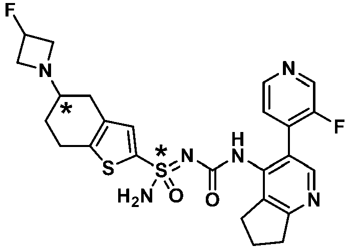 | **842** | 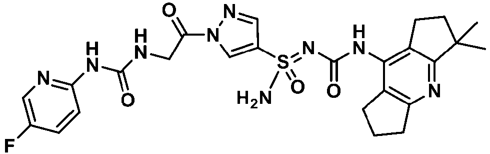 |
| **820b** | 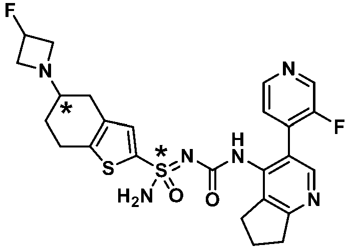 | **842a** | 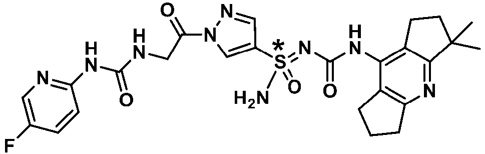 |
| **820c** | 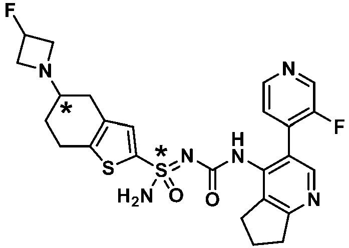 | **842b** | 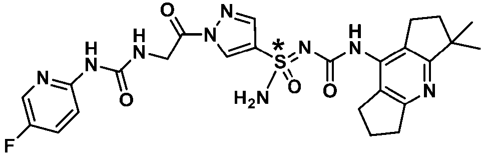 |
| **820d** | 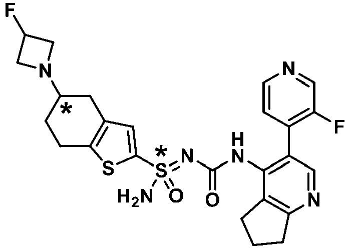 | **843** | 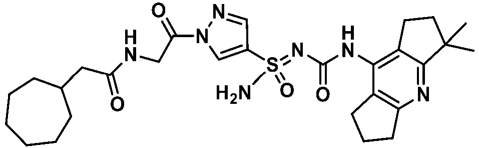 |
| **821** | 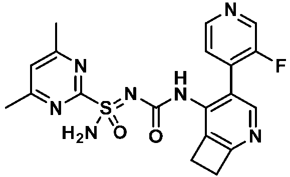 | **843a** | 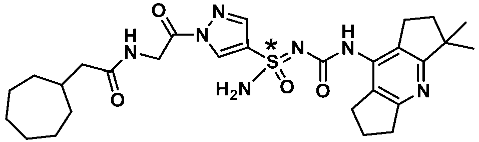 |
| | | **843b** | 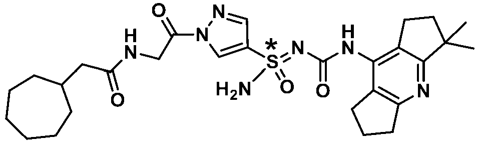 |

and pharmaceutically acceptable salts thereof.

In some embodiments, the compound is selected from the group consisting of compounds in **Table 1E** below

**Table 1E**

| **Cmpd #** | **Structure** | **Cmpd #** | **Structure** |
|---|---|---|---|
| 901 | 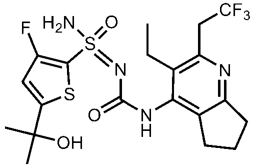 | 967b | 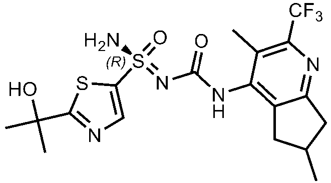 |
| 901a | 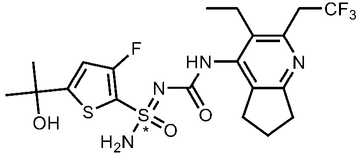 | 968 | 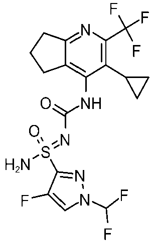 |
| 901b | 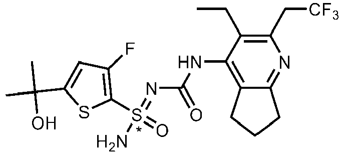 | 969 | 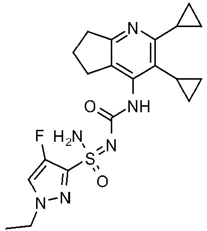 |
| 902 | 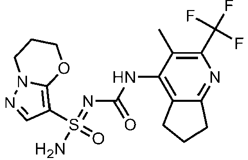 | 969a | 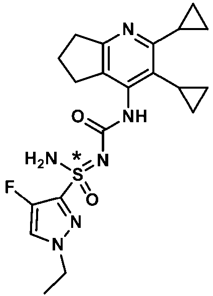 |
| 902a | 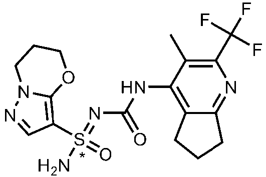 | 969b | 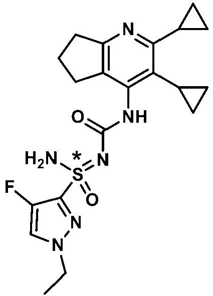 |
| 902b | 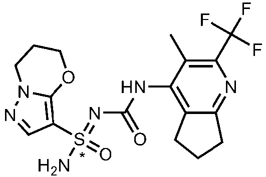 | 970 | 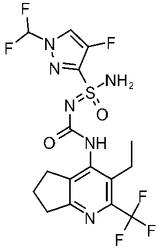 |
| 903 | 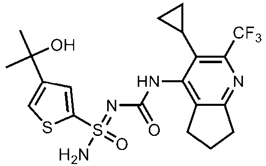 | 970a | 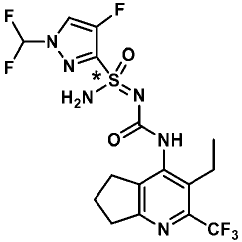 |
| 903a | 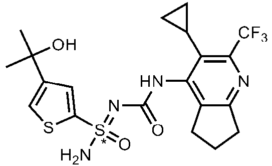 | 970b | 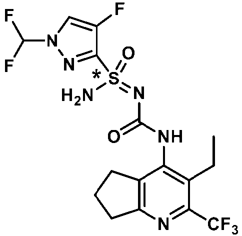 |
| 903b | 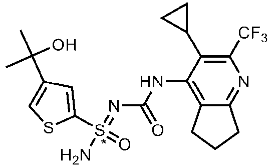 | 971 | 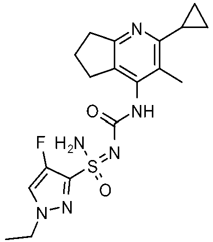 |
| 904 | 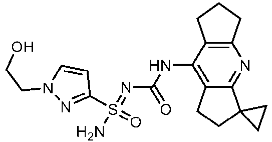 | 971a | 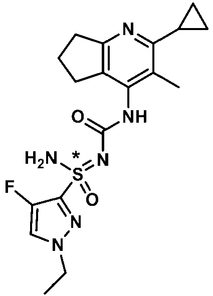 |
| 904a | 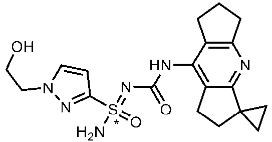 | 971b | 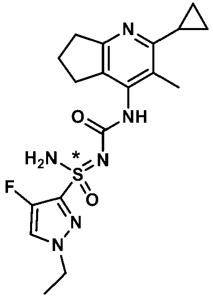 |
| 904b | 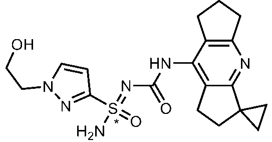 | 972 | 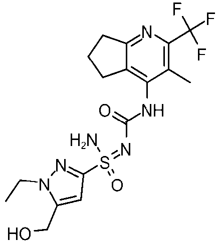 |
| 905 | 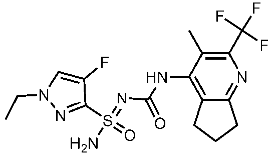 | 973 | 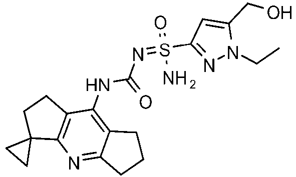 |
| 905a | 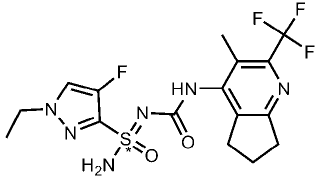 | 974 | 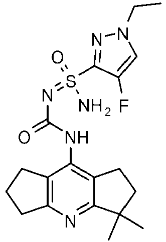 |
| 905b | 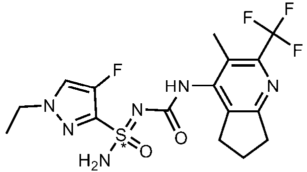 | 974a | 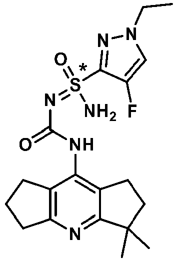 |
| 906 | 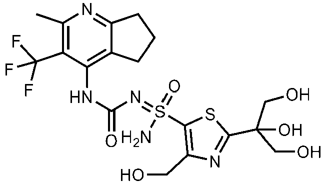 | 974b | 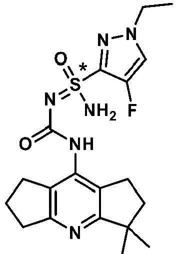 |
| 907 | 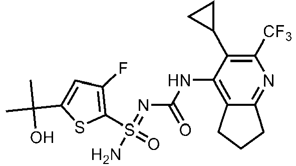 | 975 | 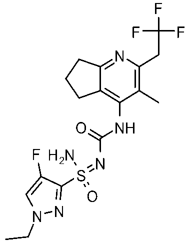 |
| 907a | 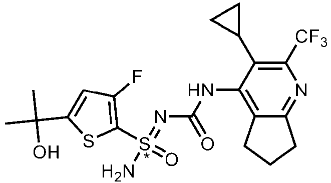 | 975a | 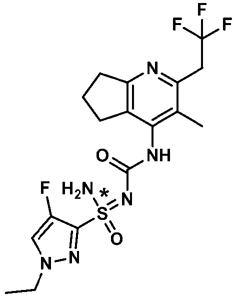 |
| 907b | 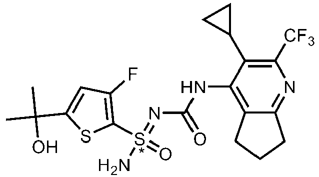 | 975b | 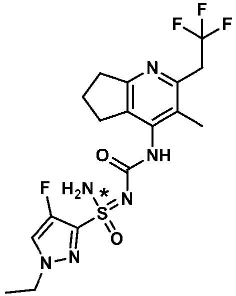 |
| 908 | 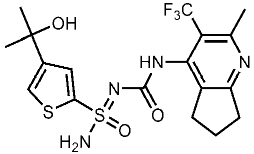 | 976 | 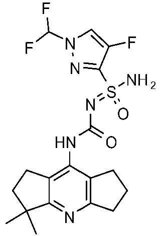 |
| 908a | 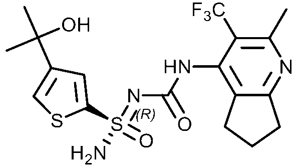 | 976a | 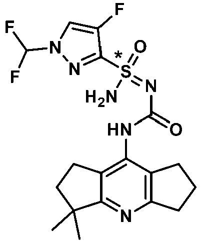 |
| 909 | 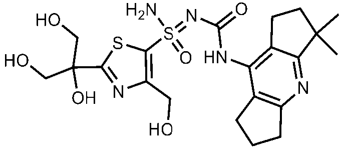 | 976b | 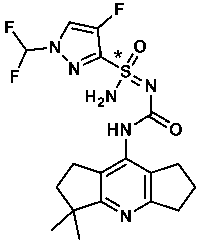 |
| 910 | 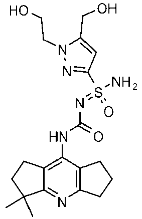 | 977 | 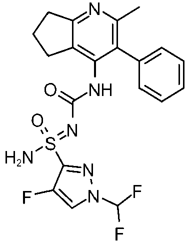 |
| 911 | 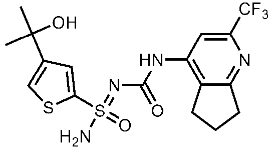 | 977a | 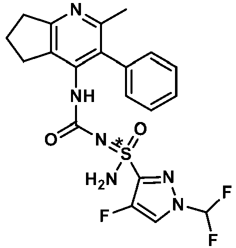 |
| 911a | 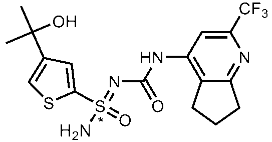 | 977b | 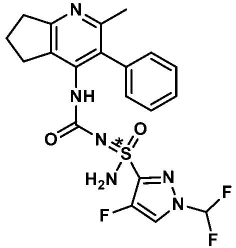 |
| 911b | 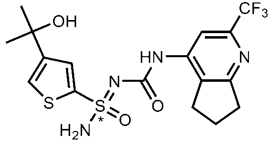 | 978 | 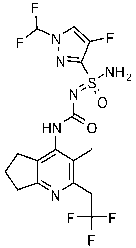 |
| 912 | 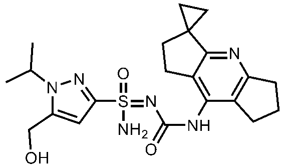 | 978a | 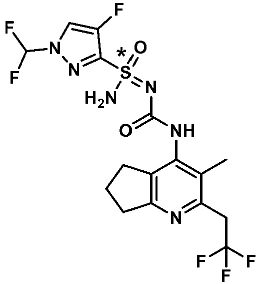 |
| 912a | 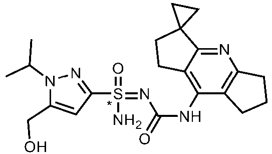 | 978b | 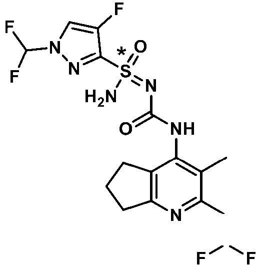 |
| 912b | 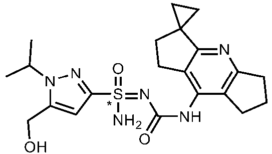 | 979 | 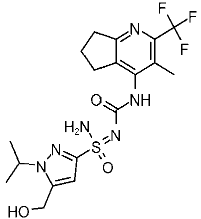 |
| 913 | 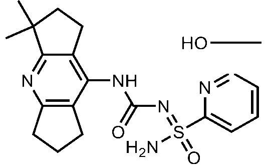 | 979a | 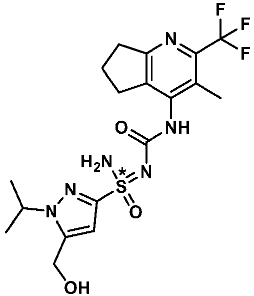 |
| 914 | 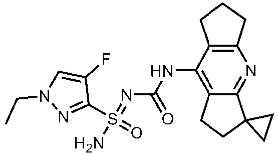 | 979b | 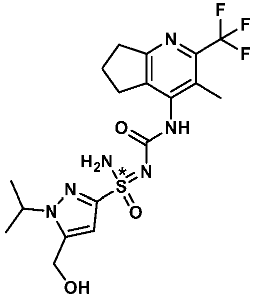 |
| 914a | 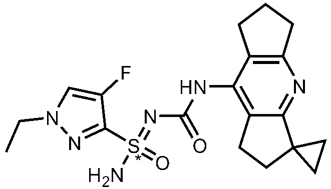 | 980 | 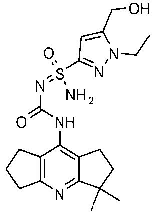 |
| 914b | 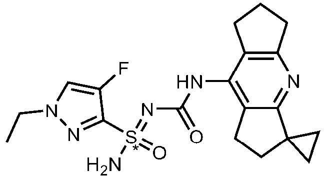 | 980a | 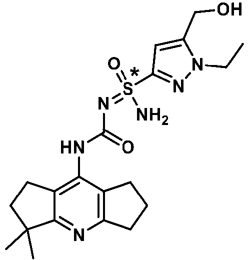 |
| 915 | 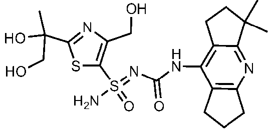 | 980b | 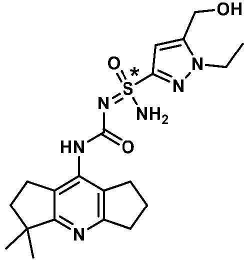 |
| 915a | 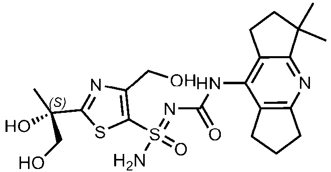 | 981a | 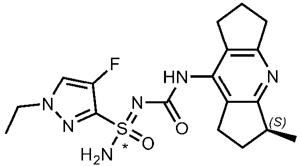 |
| 915b | 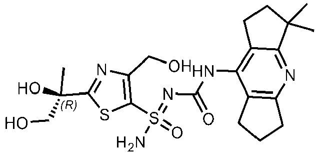 | 981b | 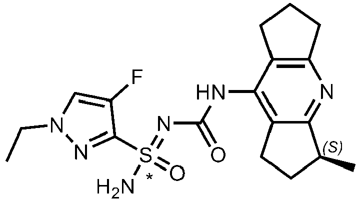 |
| 916 | 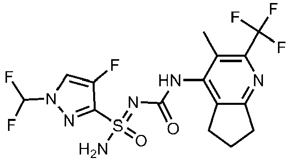 | 981c | 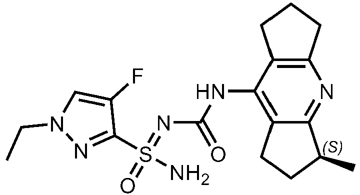 |
| 916a | 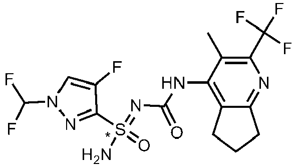 | 982a | 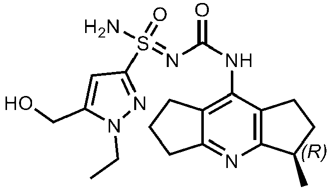 |
| 916b | 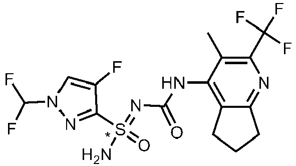 | 983 | 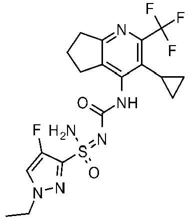 |
| 917 | 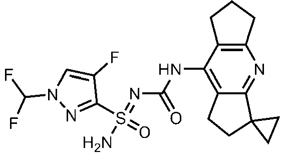 | 983a | 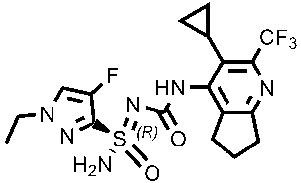 |
| 917a | 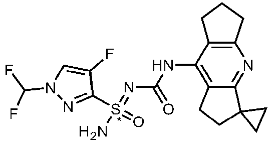 | 983b | 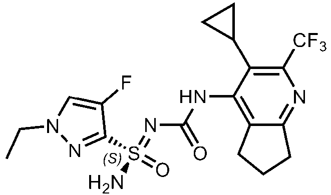 |
| 917b | 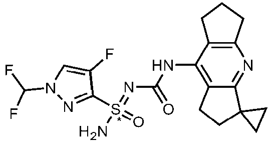 | 984 | 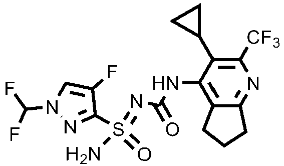 |
| 918 | 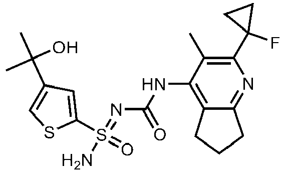 | 984a | 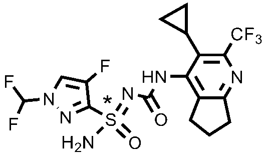 |
| 918a | 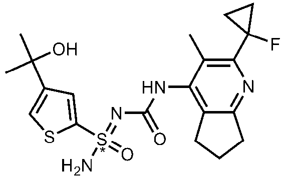 | 984b | 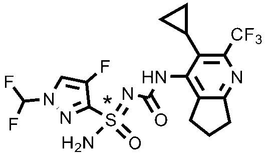 |
| 918b | 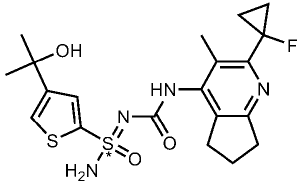 | 985 | 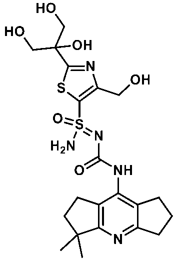 |
| 919 | 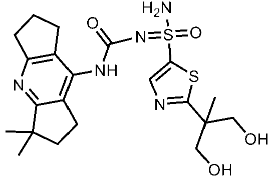 | 985a | 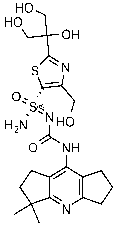 |
| 920 | 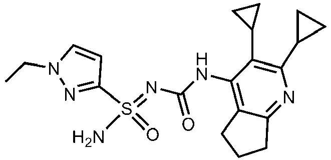 | 985b | 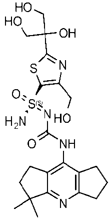 |
| 920a | 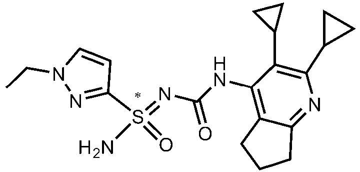 | 986 | 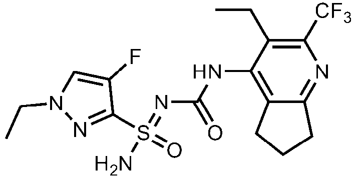 |
| 920b | 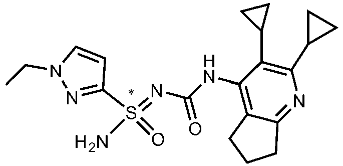 | 987 | 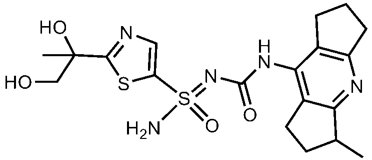 |
| 921 | 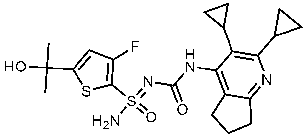 | 987a | 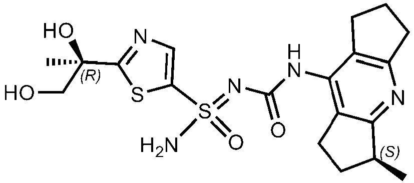 |
| 921a | 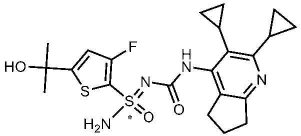 | 987b | 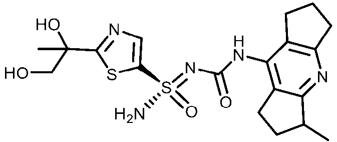 |
| 921b | 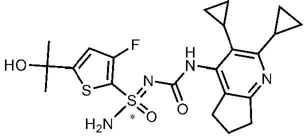 | 988 | 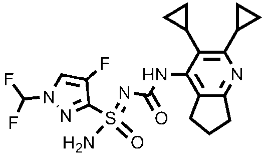 |
| 922 | 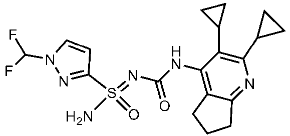 | 989 | 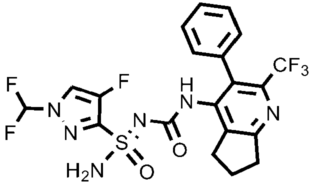 |
| 922a | 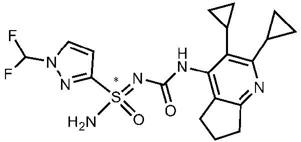 | 990 | 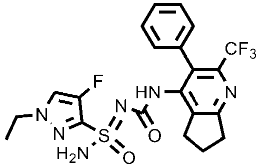 |
| 922b | 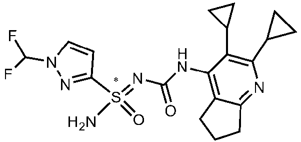 | 991 | 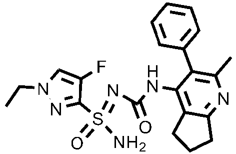 |
| 923 | 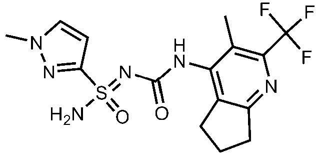 | 992 | 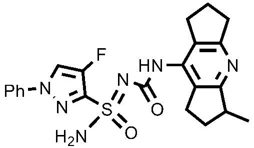 |
| 923a | 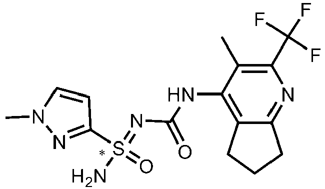 | 992a | 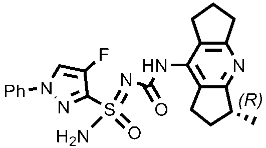 |
| 923b | 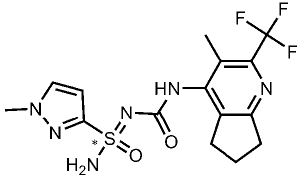 | 993 | 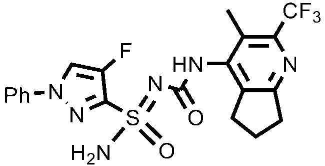 |
| 924 | 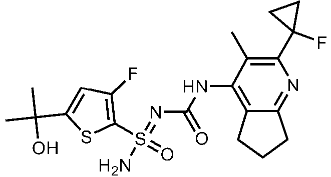 | 994 | 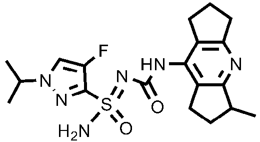 |
| 924a | 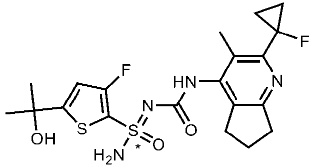 | 994a | 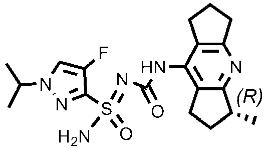 |
| 924b | 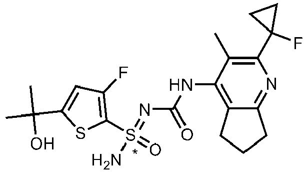 | 995 | 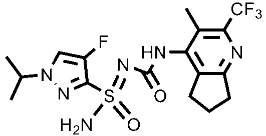 |
| 925 | 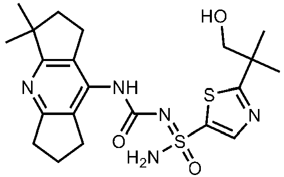 | 996 | 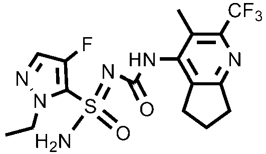 |
| 926 | 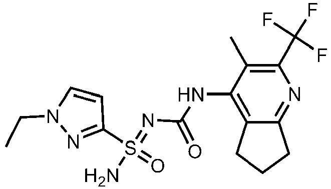 | 997 | 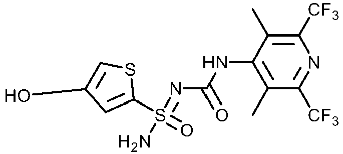 |
| 926a | 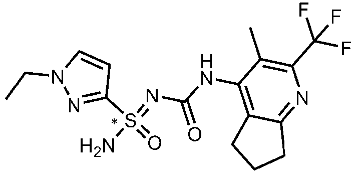 | 998 | 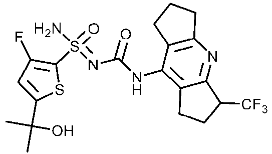 |
| 926b | 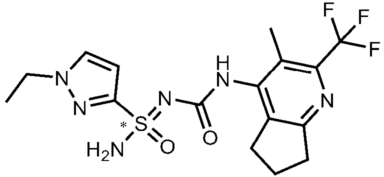 | 998a | 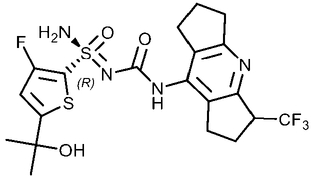 |
| 927 | 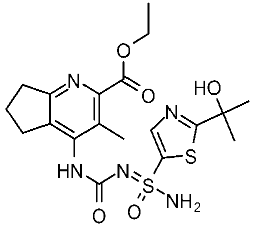 | 999 | 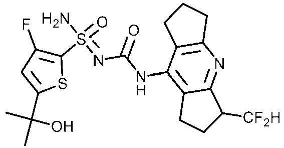 |
| 928 | 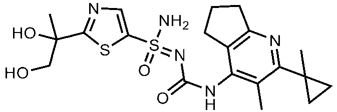 | 999a | 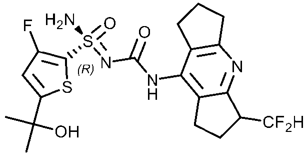 |
| 929 | 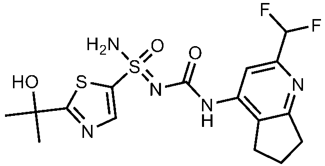 | 1000 | 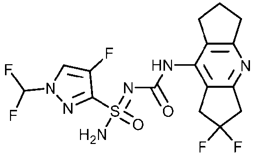 |
| 930 | 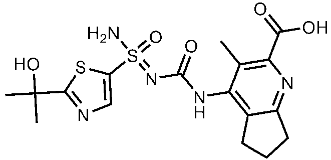 | 1000a | 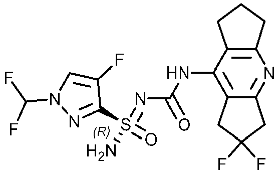 |
| 931 | 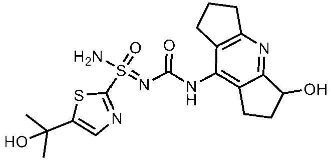 | 1001 | 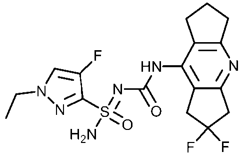 |
| 932 | 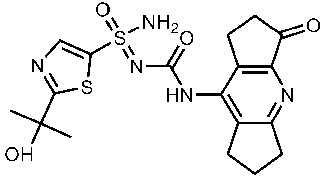 | 1001a | 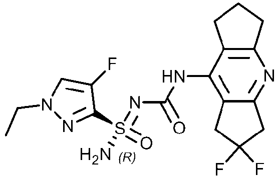 |
| 933a | 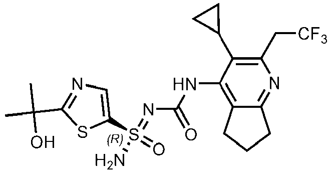 | 1002 | 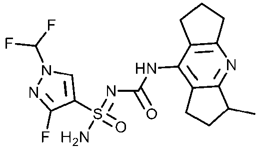 |
| 934a | 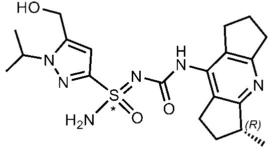 | 1002a | 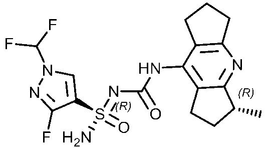 |
| 934b | 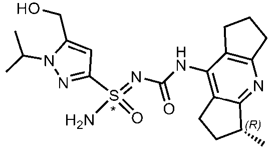 | 1003 | 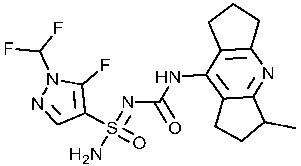 |
| 935a | 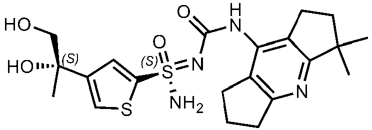 | 1003a | 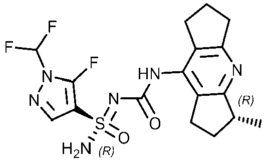 |
| 935b | 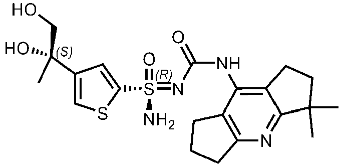 | 1004 | 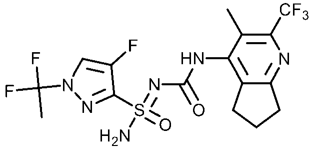 |
| 935c | 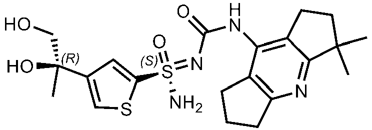 | 1004a | 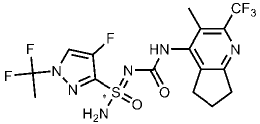 |
| 935d | 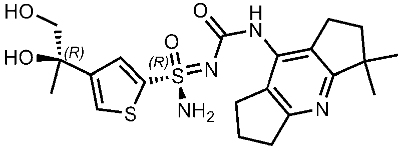 | 1005 | 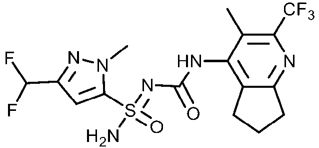 |
| 935e | 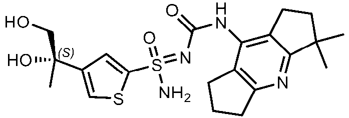 | 1005a | 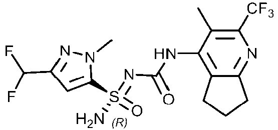 |
| 936a | 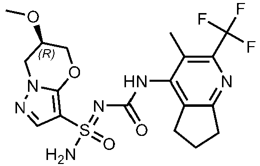 | 1006 | 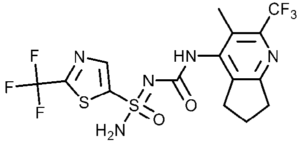 |
| 936b | 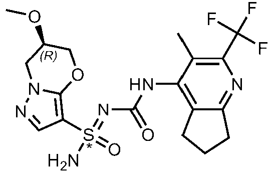 | 1006a | 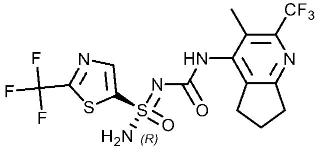 |
| 936c | 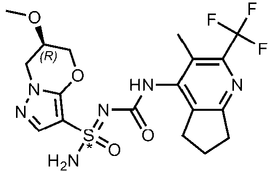 | 1007 | 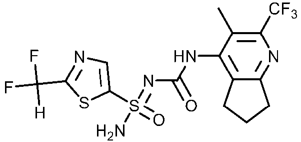 |
| 937a | 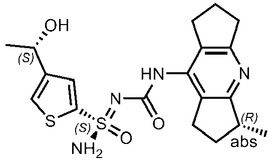 | 1007a | 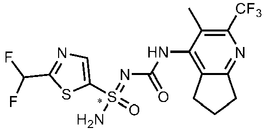 |
| 937b | 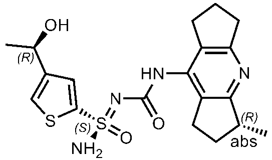 | 1008 | 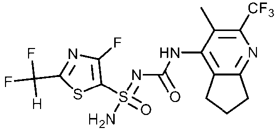 |
| 937c | 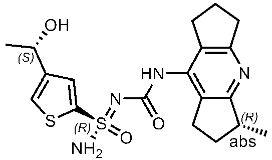 | 1008a | 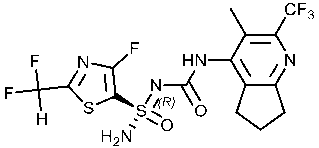 |
| 937d | 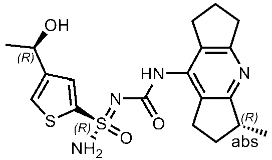 | 1009 | 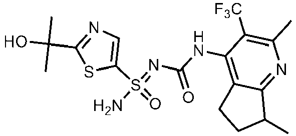 |
| 937e | 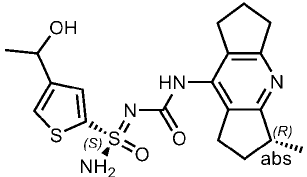 | 1009a | 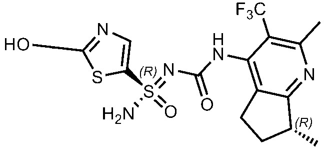 |
| 937f | 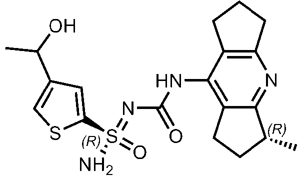 | 1010 | 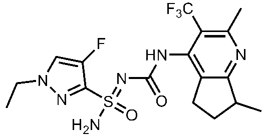 |
| 938a | 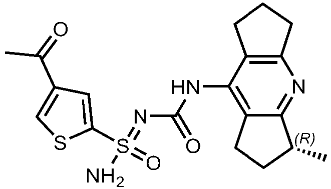 | 1010a | 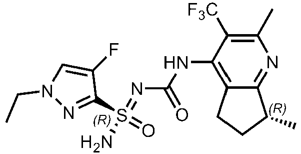 |
| 938b | 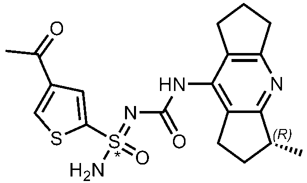 | 1011 | 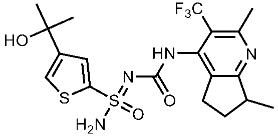 |
| 938c | 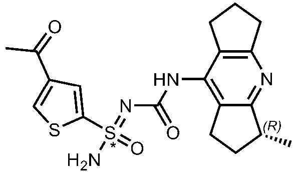 | 1011a | 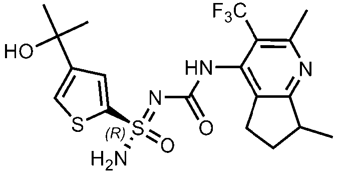 |
| 939a | 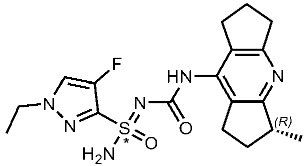 | 1012 | 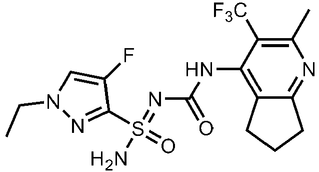 |
| 939b | 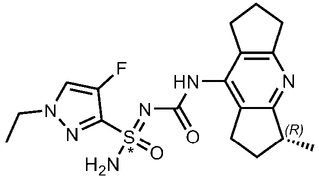 | 1013 | 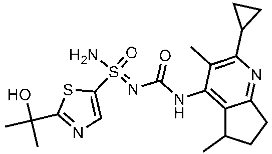 |
| 940a | 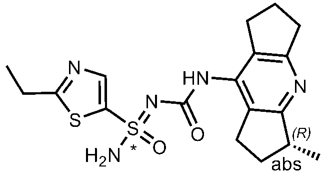 | 1013a | 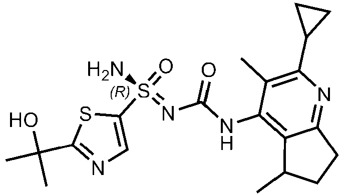 |
| 940b | 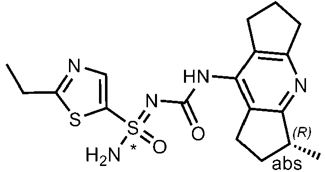 | 1014 | 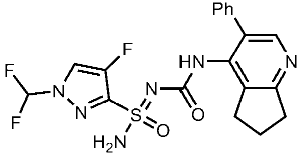 |
| 941a | 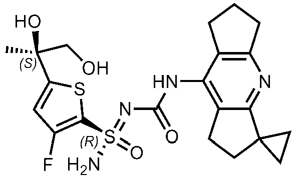 | 1014a | 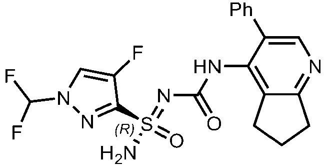 |
| 941b | 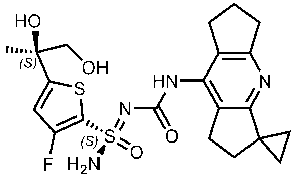 | 1015 | 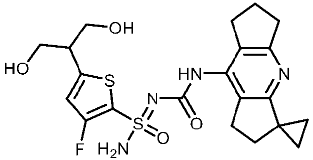 |
| 941c | 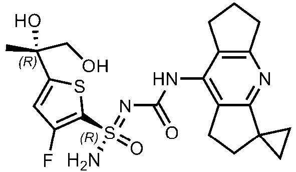 | 1015a | 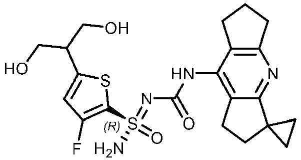 |
| 941d | 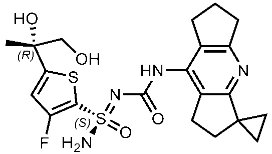 | 1016 | 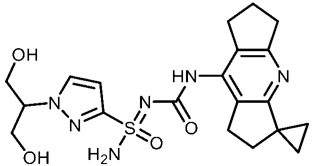 |
| 942a | 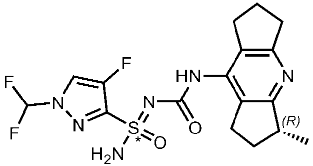 | 1016a | 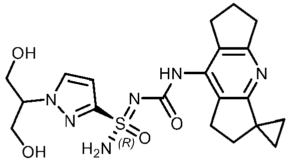 |
| 942b | 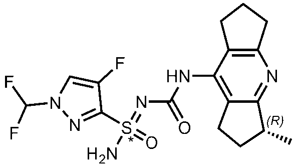 | 1017 | 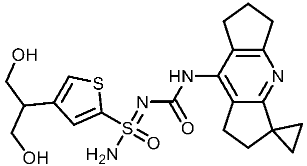 |
| 942c | 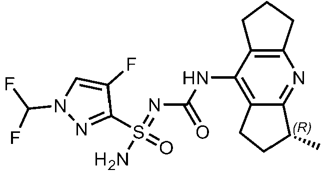 | 1017a | 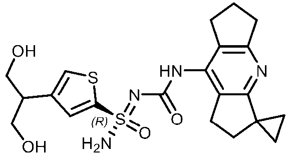 |
| 943a | 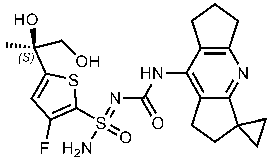 | 1018 | 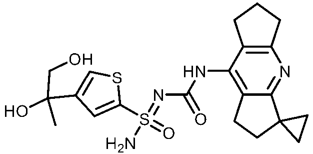 |
| 943b | 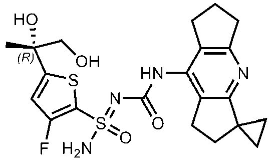 | 1018a | 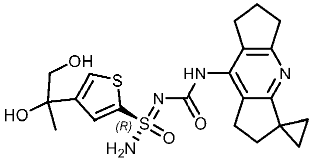 |
| 944a | 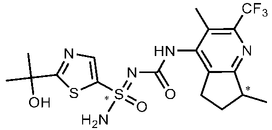 | 1019 | 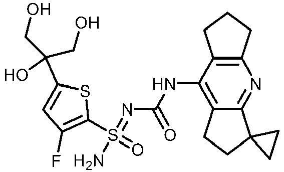 |
| 944b | 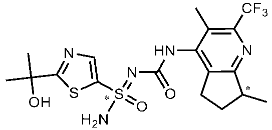 | 1019a | 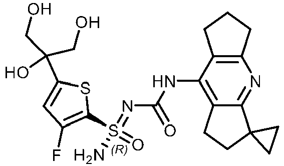 |
| 945a | 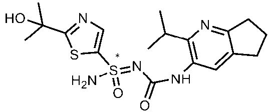 | 1020 | 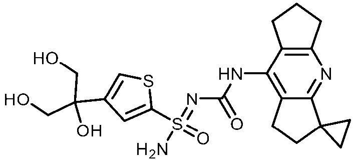 |
| 945b | 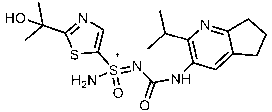 | 1020a | 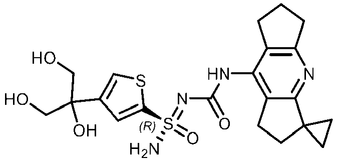 |
| 946a | 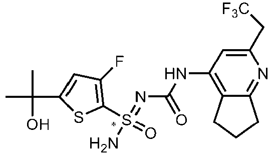 | 1021 | 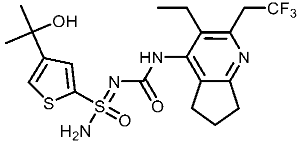 |
| 946b | 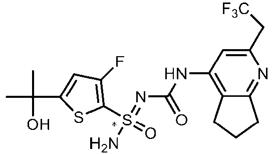 | 1022 | 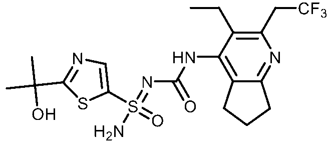 |
| 947a | 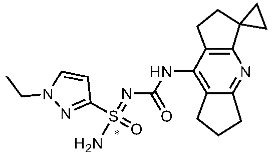 | 1022a | 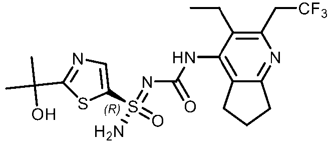 |
| 947b | 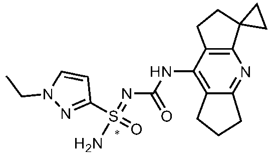 | 1023 | 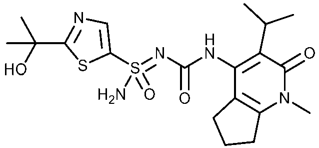 |
| 948a | 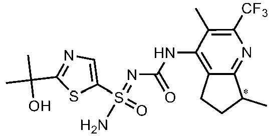 | 1024 | 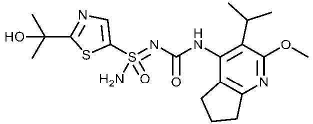 |
| 948b | 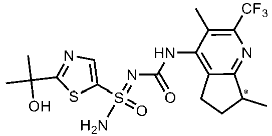 | 1025 | 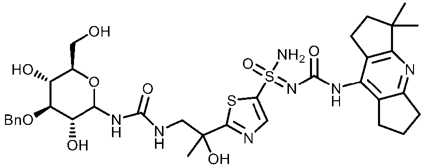 |
| 949a | 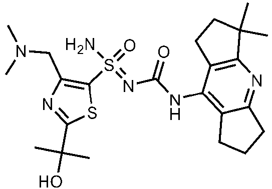 | 1025a | 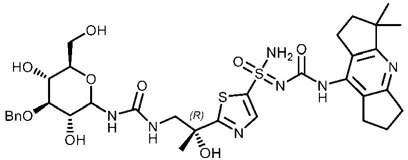 |
| 950a | 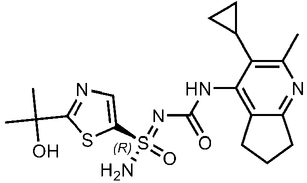 | 1026 | 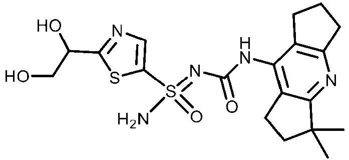 |
| 951a | 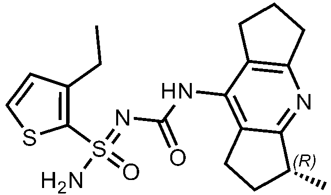 | 1026a | 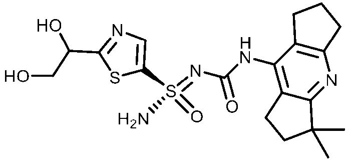 |
| 952a | 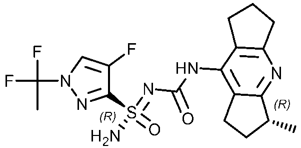 | 1027 | 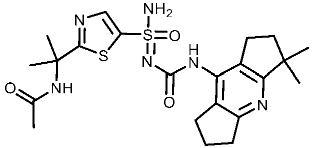 |
| 953a | 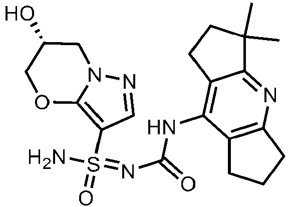 | 1028 | 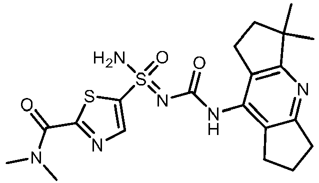 |
| 954a | 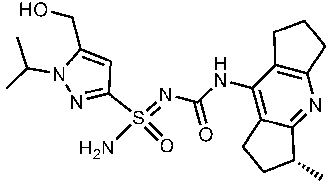 | 1029 | 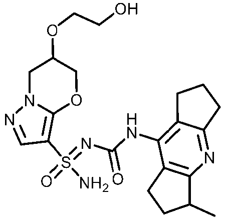 |
| 955a | 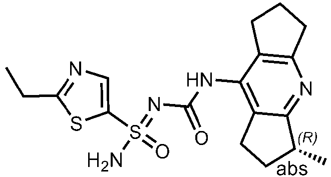 | 1029a | 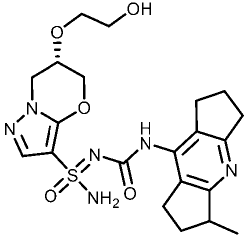 |
| 956a | 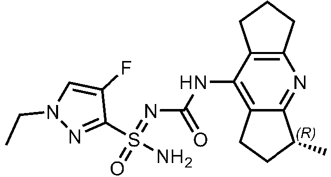 | 1030 | 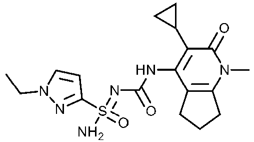 |
| 957a | 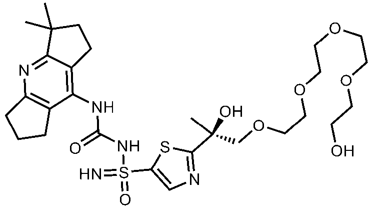 | 1031 | 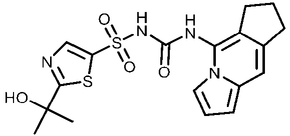 |
| 958a | 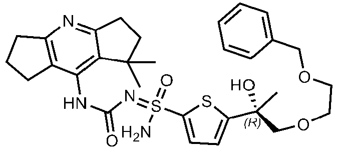 | 1032 | 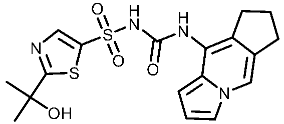 |
| 959a | 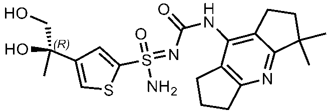 | 1033 | 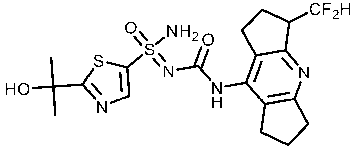 |
| 960a | 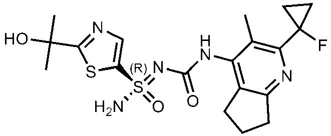 | 1034 | 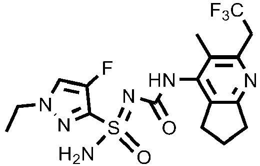 |
| 961a | 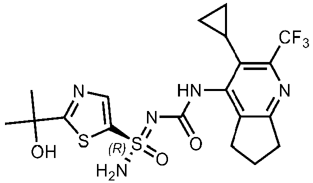 | 1034a | 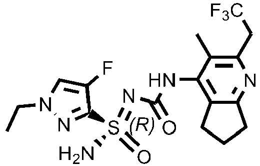 |
| 962a | 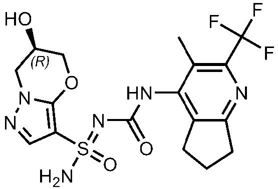 | 1035 | 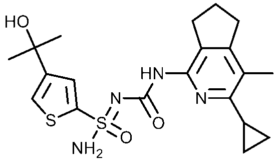 |
| 963a | 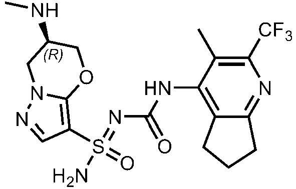 | 1036 | 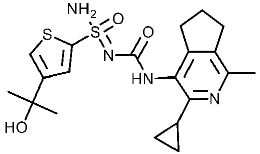 |
| 964a | 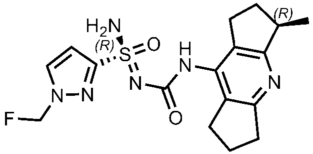 | 1037 | 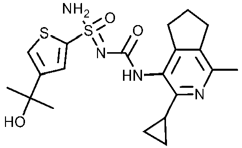 |
| 965a | 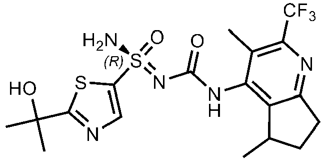 | 1038 | 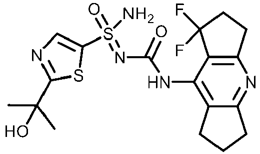 |
| 966a | 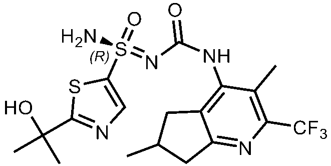 | 1039 | 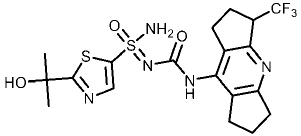 |
| 967a | 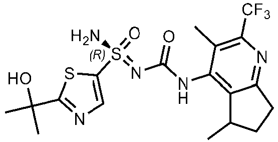 | 1040 | 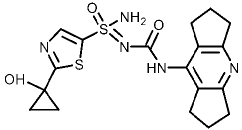 |
| 1041a | 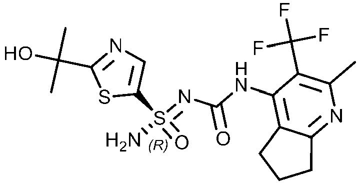 | 1041 | 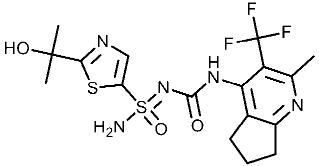 |

and pharmaceutically acceptable salts thereof.

In some embodiments, the compound is selected from the group consisting of compounds in **Table 1F** below

**Table 1F**

| **Cpd #** | **Structure** | **Cpd #** | **Structure** |
|---|---|---|---|
| **1100** | 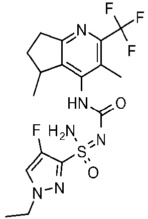 | **1101** | 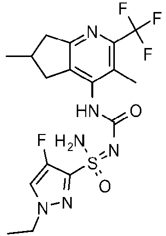 |
| **1100a** | 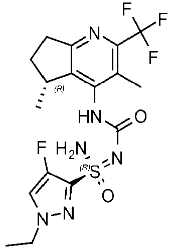 | **1101a** | 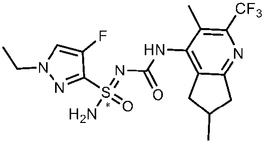 |
| **1100b** | 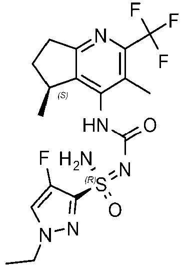 | **1101b** | 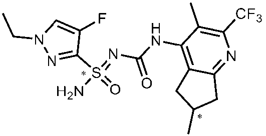 |
| **1100c** | 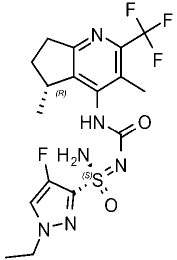 | **1101c** | 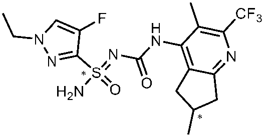 |
| **1100d** | 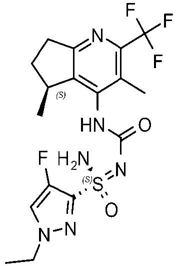 | **1101d** | 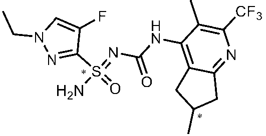 |
| | | **1101e** | 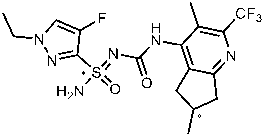 |
| **1102** | 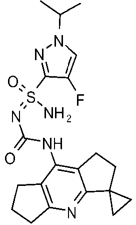 | **1103** | 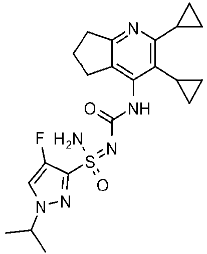 |
| **1102a** | 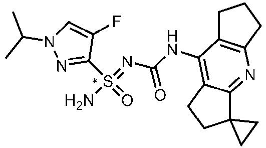 | **1103a** | 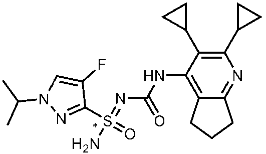 |
| **1102b** | 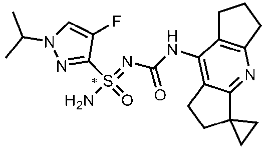 | **1103b** | 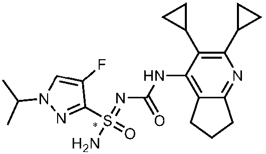 |
| **1104** | 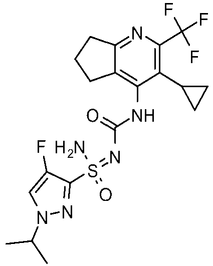 | **1105** | 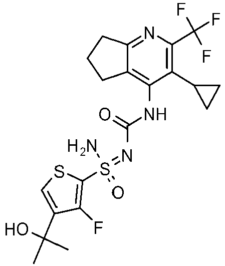 |
| **1104a** | 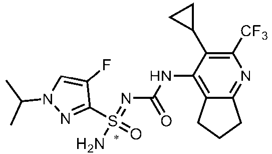 | **1105a** | 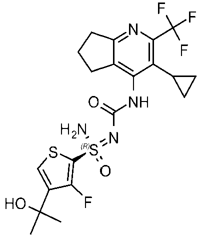 |
| **1104b** | 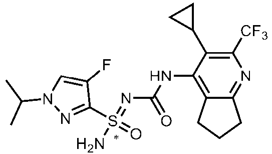 | **1105b** | 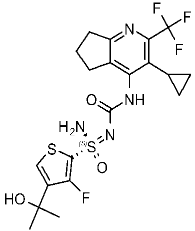 |
| **1106** | 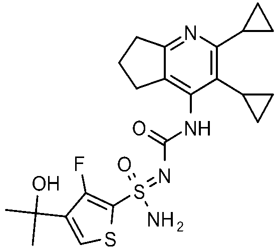 | **1107** | 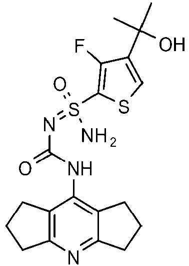 |
| **1106a** | 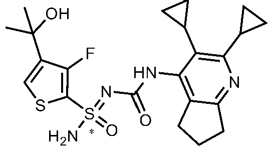 | **1107a** | 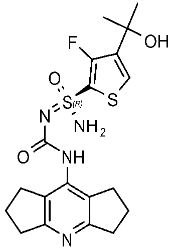 |
| **1106b** | 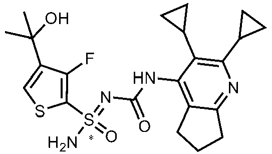 | **1107b** | 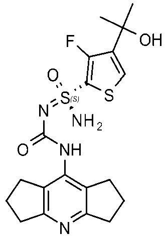 |
| **1108** | 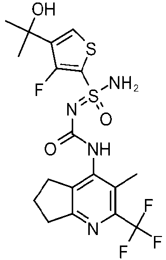 | **1109** | 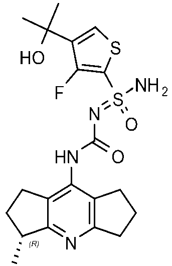 |
| **1108a** | 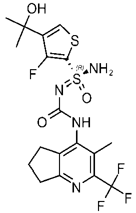 | **1109a** | 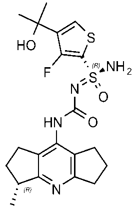 |
| **1108b** | 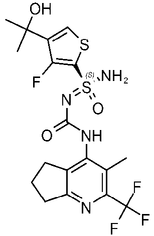 | **1109b** | 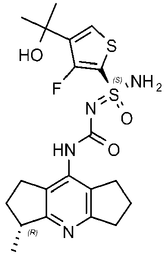 |
| **1110** | 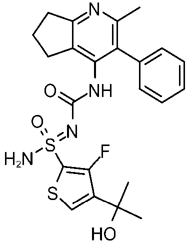 | **1111** | 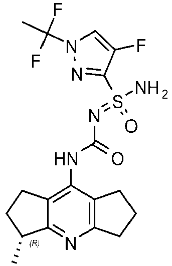 |
| **1110a** | 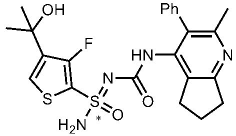 | **1110b** | 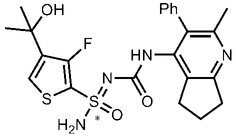 |
| **1112** | 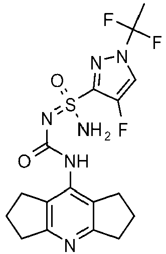 | **1113** | 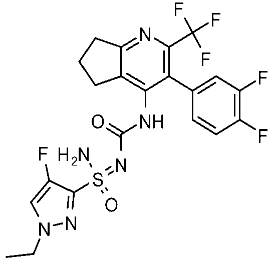 |
| **1113a** | 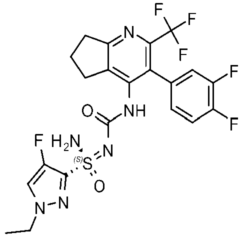 | **1113b** | 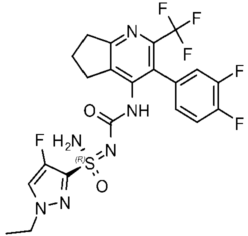 |
| **1114** | 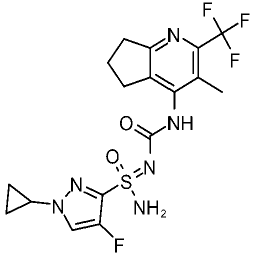 | **1115a** | 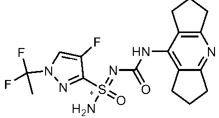 |
| **1115b** | 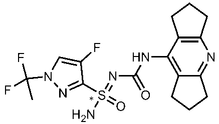 | **1116a** | 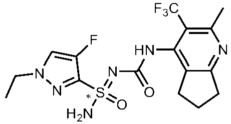 |
| **1116 b** | 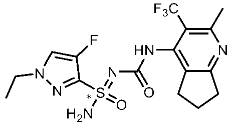 | **1117a** | 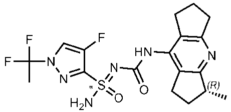 |
| **1117 b** | 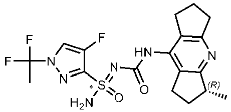 | **1118** | 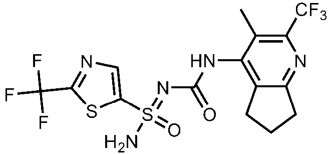 |
| **1119** | 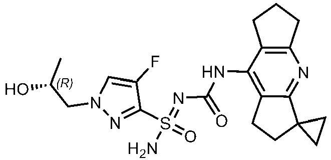 | **1004b** | 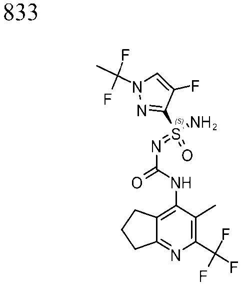 |
| **1007 b** | 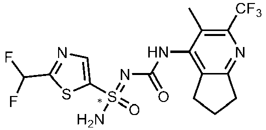 | | |

In one embodiment, provided herein is a pharmaceutical composition comprising any NLRP3 antagonist species defined here (for example, a compound or example of Tables 1A, 1E, 1F, B1, B2, B3, and B4; for example any one of examples 1 to 794) and an anti-TNFα agent disclosed herein. Preferably wherein the anti-TNFα agent is Infliximab, Etanercept, Certolizumab pegol, Golimumab or Adalimumab, more preferably wherein the anti-TNFα agent is Adalimumab.

In one embodiment, provided herein is a pharmaceutical combination of any NLRP3 antagonist species defined here (for example, a compound or example of Tables 1A, 1E, 1F, B1, B2, B3, and B4; for example any one of examples 1 to 794), and an anti-TNFα agent Preferably wherein the anti-TNFα agent is Infliximab, Etanercept, Certolizumab pegol, Golimumab or Adalimumab, more preferably wherein the anti-TNFα agent is Adalimumab.

### Anti-TNFα Agents

The term "anti-TNFα agent" refers to an agent which directly or indirectly blocks, down-regulates, impairs, inhibits, impairs, or reduces TNFα activity and/or expression. In some embodiments, an anti-TNFα agent is an antibody or an antigen-binding fragment thereof, a fusion protein, a soluble TNFα receptor (a soluble tumor necrosis factor receptor superfamily member 1A (TNFR1) or a soluble tumor necrosis factor receptor superfamily 1B (TNFR2)), an inhibitory nucleic acid, or a small molecule TNFα antagonist. In some embodiments, the inhibitory nucleic acid is a ribozyme, small hairpin RNA, a small interfering RNA, an antisense nucleic acid, or an aptamer.

Exemplary anti-TNFα agents that directly block, down-regulate, impair, inhibit, or reduce TNFα activity and/or expression can, e.g., inhibit or decrease the expression level of TNFα or a receptor of TNFα (TNFR1 or TNFR2) in a cell (e.g., a cell obtained from a subject, a mammalian cell), or inhibit or reduce binding of TNFα to its receptor (TNFR1 and/or TNFR2) and/or. Non-limiting examples of anti-TNFα agents that directly block, down-regulate, impair, inhibit, or reduce TNFα activity and/or expression include an antibody or fragment thereof, a fusion protein, a soluble TNFα receptor (e.g., a soluble TNFR1 or soluble TNFR2), inhibitory nucleic acids (e.g., any of the examples of inhibitory nucleic acids described herein), and a small molecule TNFα antagonist.

Exemplary anti-TNFα agents that can indirectly block, down-regulate, impair, inhibitreduce TNFα activity and/or expression can, e.g., inhibit or decrease the level of downstream signaling of a TNFα receptor (e.g., TNFR1 or TNFR2) in a mammalian cell (e.g., decrease the level and/or activity of one or more of the following signaling proteins: AP-1, mitogen-activated protein kinase kinase kinase 5 (ASK1), inhibitor of nuclear factor kappa B (IKK), mitogen-activated protein kinase 8 (JNK), mitogen-activated protein kinase (MAPK), MEKK 1/4, MEKK 4/7, MEKK 3/6, nuclear factor kappa B (NF-κB), mitogen-activated protein kinase kinase kinase 14 (NIK), receptor interacting serine/threonine kinase 1 (RIP), TNFRSF1A associated via death domain (TRADD), and TNF receptor associated factor 2 (TRAF2), in a cell), and/or decrease the level of TNFα-induced gene expression in a mammalian cell (e.g., decrease the transcription of genes regulated by, e.g., one or more transcription factors selected from the group of activating transcription factor 2 (ATF2), c-Jun, and NF-κB). A description of downstream signaling of a TNFα receptor is provided in Wajant et al., Cell Death Differentiation 10:45-65, 2003 (incorporated herein by reference). For example, such indirect anti-TNFα agents can be an inhibitory nucleic acid that targets (decreases the expression) a signaling component downstream of a TNFα-induced gene (e.g., any TNFα-induced gene known in the art), a TNFα receptor (e.g., any one or more of the signaling components downstream of a TNFα receptor described herein or known in the art), or a transcription factor selected from the group of NF-κB, c-Jun, and ATF2.

In other examples, such indirect anti-TNFα agents can be a small molecule inhibitor of a protein encoded by a TNFα-induced gene (e.g., any protein encoded by a TNFα-induced gene known in the art), a small molecule inhibitor of a signaling component downstream of a TNFα receptor (e.g., any of the signaling components downstream of a TNFα receptor described herein or known in the art), and a small molecule inhibitor of a transcription factor selected from the group of ATF2, c-Jun, and NF-κB.

In other embodiments, anti-TNFα agents that can indirectly block, down-regulate, impair, or reduce one or more components in a cell (e.g., acell obtained from a subject, a mammalian cell) that are involved in the signaling pathway that results in TNFα mRNA transcription, TNFα mRNA stabilization, and TNFα mRNA translation (e.g., one or more components selected from the group of CD14, c-Jun, ERK1/2, IKK, IκB, interleukin 1 receptor associated kinase 1 (IRAK), JNK, lipopolysaccharide binding protein (LBP), MEK1/2, MEK3/6, MEK4/7, MK2, MyD88, NF-κB, NIK, PKR, p38, AKT serine/threonine kinase 1 (rac), raf kinase (raf), ras, TRAF6, TTP). For example, such indirect anti-TNFα agents can be an inhibitory nucleic acid that targets (decreases the expression) of a component in a mammalian cell that is involved in the signaling pathway that results in TNFα mRNA transcription, TNFα mRNA stabilization, and TNFα mRNA translation (e.g., a component selected from the group of CD14, c-Jun, ERK1/2, IKK, IκB, IRAK, JNK, LBP, MEK1/2, MEK3/6, MEK4/7, MK2, MyD88, NF-κB, NIK, IRAK, lipopolysaccharide binding protein (LBP), PKR, p38, rac, raf, ras, TRAF6, TTP). In other examples, an indirect anti-TNFα agents is a small molecule inhibitor of a component in a mammalian cell that is involved in the signaling pathway that results in TNFα mRNA transcription, TNFα mRNA stabilization, and TNFα mRNA translation (e.g., a component selected from the group of CD14, c-Jun, ERK1/2, IKK, IκB, IRAK, JNK, lipopolysaccharide binding protein (LBP), MEK1/2, MEK3/6, MEK4/7, MK2, MyD88, NF-κB, NIK, IRAK, lipopolysaccharide binding protein (LBP), PKR, p38, rac, raf, ras, TRAF6, TTP).

### Antibodies

In some embodiments, the anti-TNFα agent is an antibody or an antigen-binding fragment thereof (e.g., a Fab or a scFv). In some embodiments, an antibody or antigen-binding fragment of an antibody described herein can bind specifically to TNFα. In some embodiments, an antibody or antigen-binding fragment described herein binds specifically to any one of TNFα, TNFR1, or TNFR2. In some embodiments, an antibody or antigen-binding fragment of an antibody described herein can bind specifically to a TNFα receptor (TNFR1 or TNFR2).

In some embodiments, the antibody can be a humanized antibody, a chimeric antibody, a multivalent antibody, or a fragment thereof. In some embodiments, an antibody can be a scFv-Fc, a VHH domain, a VNAR domain, a (scFv)2, a minibody, or a BiTE.

In some embodiments, an antibody can be a crossmab, a diabody, a scDiabody, a scDiabody-CH3, a Diabody-CH3, a DutaMab, a DT-IgG, a diabody-Fc, a scDiabody-HAS, a charge pair antibody, a Fab-arm exchange antibody, a SEEDbody, a Triomab, a LUZ-Y, a Fcab, a kλ-body, an orthogonal Fab, a DVD-IgG, an IgG(H)-scFv, a scFv-(H)IgG, an IgG(L)-scFv, a scFv-(L)-IgG, an IgG (L,H)-Fc, an IgG(H)-V, a V(H)-IgG, an IgG(L)-V, a V(L)-IgG, an KIH IgG-scFab, a 2scFv-IgG, an IgG-2scFv, a scFv4-Ig, a Zybody, a DVI-IgG, a nanobody, a nanobody-HSA, a DVD-Ig, a dual-affinity re-targeting antibody (DART), a triomab, a kih IgG with a common LC, an ortho-Fab IgG, a 2-in-1-IgG, IgG-ScFv, scFv2-Fc, a bi-nanobody, tanden antibody, a DART-Fc, a scFv-HAS-scFv, a DAF (two-in-one or four-in-one), a DNL-Fab3, knobs-in-holes common LC, knobs-in-holes assembly, a TandAb, a Triple Body, a miniantibody, a minibody, a TriBi minibody, a scFv-CH3 KIH, a Fab-scFv, a scFv-CH-CL-scFv, a F(ab')2-scFV2, a scFv-KIH, a Fab-scFv-Fc, a tetravalent HCAb, a scDiabody-Fc, a tandem scFv-Fc, an intrabody, a dock and lock bispecific antibody, an ImmTAC, a HSAbody, a tandem scFv, an IgG-IgG, a Cov-X-Body, and a scFvl-PEG-scFv2.

Non-limiting examples of an antigen-binding fragment of an antibody include an Fv fragment, a Fab fragment, a F(ab')2 fragment, and a Fab' fragment. Additional examples of an antigen-binding fragment of an antibody is an antigen-binding fragment of an antigen-binding fragment of an IgA (e.g., an antigen-binding fragment of IgA1 or IgA2) (e.g., an antigen-binding fragment of a human or humanized IgA, e.g., a human or humanized IgA1 or IgA2); an antigen-binding fragment of an IgD (e.g., an antigen-binding fragment of a human or humanized IgD); an antigen-binding fragment of an IgE (e.g., an antigen-binding fragment of a human or humanized IgE); an IgG (e.g., an antigen-binding fragment of IgG1, IgG2, IgG3, or IgG4) (e.g., an antigen-binding fragment of a human or humanized IgG, e.g., human or humanized IgG1, IgG2, IgG3, or IgG4); or an antigen-binding fragment of an IgM (e.g., an antigen-binding fragment of a human or humanized IgM).

Non-limiting examples of anti-TNFα agents that are antibodies that specifically bind to TNFα are described in Ben-Horin et al., Autoimmunity Rev. 13(1):24-30, 2014; Bongartz et al., JAMA 295(19):2275-2285, 2006; Butler et al., Eur. Cytokine Network 6(4):225-230, 1994; Cohen et al., Canadian J. Gastroenterol. Hepatol. 15(6):376-384, 2001; Elliott et al., Lancet 1994; 344: 1125-1127, 1994; Feldmann et al., Ann. Rev. Immunol. 19(1):163-196, 2001; Rankin et al., Br. J. Rheumatol. 2:334-342, 1995; Knight et al., Molecular Immunol. 30(16):1443-1453, 1993; Lorenz et al., J. Immunol. 156(4):1646-1653, 1996; Hinshaw et al., Circulatory Shock 30(3):279-292, 1990; Ordas et al., Clin. Pharmacol. Therapeutics 91(4):635-646, 2012; Feldman, Nature Reviews Immunol. 2(5):364-371, 2002; Taylor et al., Nature Reviews Rheumatol. 5(10):578-582, 2009; Garces et al., Annals Rheumatic Dis. 72(12):1947-1955, 2013; Palladino et al., Nature Rev. Drug Discovery 2(9):736-746, 2003; Sandborn et al., Inflammatory Bowel Diseases 5(2):119-133, 1999; Atzeni et al., Autoimmunity Reviews 12(7):703-708, 2013; Maini et al., Immunol. Rev. 144(1):195-223, 1995; Wanner et al., Shock 11(6):391-395, 1999; and U.S. Patent Nos. 6,090,382; 6,258,562; and 6,509,015).

In certain embodiments, the anti-TNFα agent can include or is golimumab (golimumabTM), adalimumab (Humira^{™}), infliximab (Remicade^{™}), CDP571, CDP 870, or certolizumab pegol (Cimzia^{™}). In certain embodiments, the anti-TNFα agent can be a TNFα inhibitor biosimilar. Examples of approved and late-phase TNFα inhibitor biosimilars include, but are not limited to, infliximab biosimilars such as Flixabi^{™} (SB2) from Samsung Bioepis, Inflectra^{®} (CT-P13) from Celltrion/Pfizer, GS071 from Aprogen, Remsima^{™}, PF-06438179 from Pfizer/Sandoz, NI-071 from Nichi-Iko Pharmaceutical Co., and ABP 710 from Amgen; adalimumab biosimilars such as Amgevita^{®} (ABP 501) from Amgen and Exemptia^{™} from Zydus Cadila, BMO-2 or MYL-1401-A from Biocon/Mylan, CHS-1420 from Coherus, FKB327 from Kyowa Kirin, and BI 695501 from Boehringer Ingelheim;Solymbic^{®}, SB5 from Samsung Bioepis, GP-2017 from Sandoz, ONS-3010 from Oncobiologics, M923 from Momenta, PF-06410293 from Pfizer, and etanercept biosimilars such as Erelzi^{™} from Sandoz/Novartis, Brenzys^{™} (SB4) from Samsung Bioepis, GP2015 from Sandoz, TuNEX^{®} from Mycenax, LBEC0101 from LG Life, and CHS-0214 from Coherus.

In some embodiments of any of the methods described herein, the anti-TNFα agent is selected from the group consisting of: adalimumab, certolizumab, etanercept, golimumab, infliximabm, CDP571, and CDP 870.

In some embodiments, any of the antibodies or antigen-binding fragments described herein has a dissociation constant (K_{D}) of less than 1 x 10⁻⁵ M (e.g., less than 0.5 x 10⁻⁵ M, less than 1 x 10⁻⁶ M, less than 0.5 x 10⁻⁶ M, less than 1 x 10⁻⁷ M, less than 0.5 x 10⁻⁷ M, less than 1 x 10⁻⁸ M, less than 0.5 x 10⁻⁸ M, less than 1 x 10⁻⁹ M, less than 0.5 x 10⁻⁹ M, less than 1 x 10⁻¹⁰ M, less than 0.5 x 10⁻¹⁰ M, less than 1 x 10⁻¹¹ M, less than 0.5 x 10⁻¹¹ M, or less than 1 x 10⁻¹² M), e.g., as measured in phosphate buffered saline using surface plasmon resonance (SPR).

In some embodiments, any of the antibodies or antigen-binding fragments described herein has a K_{D} of about 1 x 10⁻¹² M to about 1 x 10⁻⁵ M, about 0.5 x 10⁻⁵ M, about 1 x 10⁻⁶ M, about 0.5 x 10⁻⁶ M, about 1 x 10⁻⁷ M, about 0.5 x 10⁻⁷ M, about 1 x 10⁻⁸ M, about 0.5 x 10⁻⁸ M, about 1 x 10⁻⁹ M, about 0.5 x 10⁻⁹ M, about 1 x 10⁻¹⁰ M, about 0.5 x 10⁻¹⁰ M, about 1 x 10⁻¹¹ M, or about 0.5 x 10⁻¹¹ M (inclusive); about 0.5 x 10⁻¹¹ M to about 1 x 10⁻⁵ M, about 0.5 x 10⁻⁵ M, about 1 x 10⁻⁶ M, about 0.5 x 10⁻⁶ M, about 1 x 10⁻⁷ M, about 0.5 x 10⁻⁷ M, about 1 x 10⁻⁸ M, about 0.5 x 10⁻⁸ M, about 1 x 10⁻⁹ M, about 0.5 x 10⁻⁹ M, about 1 x 10⁻¹⁰ M, about 0.5 x 10⁻¹⁰ M, or about 1 x 10⁻¹¹ M (inclusive); about 1 x 10⁻¹¹ M to about 1 x 10⁻⁵ M, about 0.5 x 10⁻⁵ M, about 1 x 10⁻⁶ M, about 0.5 x 10⁻⁶ M, about 1 x 10⁻⁷ M, about 0.5 x 10⁻⁷ M, about 1 x 10⁻⁸ M, about 0.5 x 10⁻⁸ M, about 1 x 10⁻⁹ M, about 0.5 x 10⁻⁹ M, about 1 x 10⁻¹⁰ M, or about 0.5 x 10⁻¹⁰ M (inclusive); about 0.5 x 10⁻¹⁰ M to about 1 x 10⁻⁵ M, about 0.5 x 10⁻⁵ M, about 1 x 10⁻⁶ M, about 0.5 x 10⁻⁶ M, about 1 x 10⁻⁷ M, about 0.5 x 10⁻⁷ M, about 1 x 10⁻⁸ M, about 0.5 x 10⁻⁸ M, about 1 x 10⁻⁹ M, about 0.5 x 10⁻⁹ M, or about 1 x 10⁻¹⁰ M (inclusive); about 1 x 10⁻¹⁰ M to about 1 x 10⁻⁵ M, about 0.5 x 10⁻⁵ M, about 1 x 10⁻⁶ M, about 0.5 x 10⁻⁶ M, about 1 x 10⁻⁷ M, about 0.5 x 10⁻⁷ M, about 1 x 10⁻⁸ M, about 0.5 x 10⁻⁸ M, about 1 x 10⁻⁹ M, or about 0.5 x 10⁻⁹ M (inclusive); about 0.5 x 10⁻⁹ M to about 1 x 10⁻⁵ M, about 0.5 x 10⁻⁵ M, about 1 x 10⁻⁶ M, about 0.5 x 10⁻⁶ M, about 1 x 10⁻⁷ M, about 0.5 x 10⁻⁷ M, about 1 x 10⁻⁸ M, about 0.5 x 10⁻⁸ M, or about 1 x 10⁻⁹ M (inclusive); about 1 x 10⁻⁹ M to about 1 x 10⁻⁵ M, about 0.5 x 10⁻⁵ M, about 1 x 10⁻⁶ M, about 0.5 x 10⁻⁶ M, about 1 x 10⁻⁷ M, about 0.5 x 10⁻⁷ M, about 1 x 10⁻⁸ M, or about 0.5 x 10⁻⁸ M (inclusive); about 0.5 x 10⁻⁸ M to about 1 x 10⁻⁵ M, about 0.5 x 10⁻⁵ M, about 1 x 10⁻⁶ M, about 0.5 x 10⁻⁶ M, about 1 x 10⁻⁷ M, about 0.5 x 10⁻⁷ M, or about 1 x 10⁻⁸ M (inclusive); about 1 x 10⁻⁸ M to about 1 x 10⁻⁵ M, about 0.5 x 10⁻⁵ M, about 1 x 10⁻⁶ M, about 0.5 x 10⁻⁶ M, about 1 x 10⁻⁷ M, or about 0.5 x 10⁻⁷ M (inclusive); about 0.5 x 10⁻⁷ M to about 1 x 10⁻⁵ M, about 0.5 x 10⁻⁵ M, about 1 x 10⁻⁶ M, about 0.5 x 10⁻⁶ M, or about 1 x 10⁻⁷ M (inclusive); about 1 x 10⁻⁷ M to about 1 x 10⁻⁵ M, about 0.5 x 10⁻⁵ M, about 1 x 10⁻⁶ M, or about 0.5 x 10⁻⁶ M (inclusive); about 0.5 x 10⁻⁶ M to about 1 x 10⁻⁵ M, about 0.5 x 10⁻⁵ M, or about 1 x 10⁻⁶ M (inclusive); about 1 x 10⁻⁶ M to about 1 x 10⁻⁵ M or about 0.5 x 10⁻⁵ M (inclusive); or about 0.5 x 10⁻⁵ M to about 1 x 10⁻⁵ M (inclusive), e.g., as measured in phosphate buffered saline using surface plasmon resonance (SPR).

In some embodiments, any of the antibodies or antigen-binding fragments described herein has a K_{off} of about 1 x 10⁻⁶ s⁻¹ to about 1 x 10⁻³ s⁻¹, about 0.5 x 10⁻³ s⁻¹, about 1 x 10⁻⁴ s⁻¹, about 0.5 x 10⁻⁴ s⁻¹, about 1 x 10⁻⁵ s⁻¹, or about 0.5 x 10⁻⁵ s⁻¹ (inclusive); about 0.5 x 10⁻⁵ s⁻¹ to about 1 x 10⁻³ s⁻¹, about 0.5 x 10⁻³ s⁻¹, about 1 x 10⁻⁴ s⁻¹, about 0.5 x 10⁻⁴ s⁻¹, or about 1 x 10⁻⁵ s⁻¹ (inclusive); about 1 x 10⁻⁵ s⁻¹ to about 1 x 10⁻³ s⁻¹, about 0.5 x 10⁻³ s⁻¹, about 1 x 10⁻⁴ s⁻¹, or about 0.5 x 10⁻⁴ s⁻¹ (inclusive); about 0.5 x 10⁻⁴ s⁻¹ to about 1 x 10⁻³ s⁻¹, about 0.5 x 10⁻³ s⁻¹, or about 1 x 10⁻⁴ s⁻¹ (inclusive); about 1 x 10⁻⁴ s⁻¹ to about 1 x 10⁻³ s⁻¹, or about 0.5 x 10⁻³ s⁻¹ (inclusive); or about 0.5 x 10⁻⁵ s⁻¹ to about 1 x 10⁻³ s⁻¹ (inclusive), e.g., as measured in phosphate buffered saline using surface plasmon resonance (SPR).

In some embodiments, any of the antibodies or antigen-binding fragments described herein has a Kₒₙ of about 1 x 10² M⁻¹s⁻¹ to about 1 x 10⁶ M⁻¹s⁻¹, about 0.5 x 10⁶ M⁻¹s⁻¹, about 1 x 10⁵ M⁻¹s⁻¹, about 0.5 x 10⁵ M⁻¹s⁻¹, about 1 x 10⁴ M⁻¹s⁻¹, about 0.5 x 10⁴ M⁻¹s⁻¹, about 1 x 10³ M⁻¹s⁻¹, or about 0.5 x 10³ M⁻¹s⁻¹ (inclusive); about 0.5 x 10³ M⁻¹s⁻¹ to about 1 x 10⁶ M⁻¹s⁻¹, about 0.5 x 10⁶ M⁻¹s⁻¹, about 1 x 10⁵ M⁻¹s⁻¹, about 0.5 x 10⁵ M⁻¹s⁻¹, about 1 x 10⁴ M⁻¹s⁻¹, about 0.5 x 10⁴ M⁻¹s⁻¹, or about 1 x 10³ M⁻¹s⁻¹ (inclusive); about 1 x 10³ M⁻¹s⁻¹ to about 1 x 10⁶ M⁻¹s⁻¹, about 0.5 x 10⁶ M⁻¹s⁻¹, about 1 x 10⁵ M⁻¹s⁻¹, about 0.5 x 10⁵ M⁻¹s⁻¹, about 1 x 10⁴ M⁻¹s⁻¹, or about 0.5 x 10⁴ M⁻¹s⁻¹ (inclusive); about 0.5 x 10⁴ M⁻¹s⁻¹ to about 1 x 10⁶ M⁻¹s⁻¹, about 0.5 x 10⁶ M⁻¹s⁻¹, about 1 x 10⁵ M⁻¹s⁻¹, about 0.5 x 10⁵ M⁻¹s⁻¹, or about 1 x 10⁴ M⁻¹s⁻¹ (inclusive); about 1 x 10⁴ M⁻¹s⁻¹ to about 1 x 10⁶ M⁻¹s⁻¹, about 0.5 x 10⁶ M⁻¹s⁻¹, about 1 x 10⁵ M⁻¹s⁻¹, or about 0.5 x 10⁵ M⁻¹s⁻¹ (inclusive); about 0.5 x 10⁵ M⁻¹s⁻¹ to about 1 x 10⁶ M⁻¹s⁻¹, about 0.5 x 10⁶ M⁻¹s⁻¹, or about 1 x 10⁵ M⁻¹s⁻¹ (inclusive); about 1 x 10⁵ M⁻¹s⁻¹ to about 1 x 10⁶ M⁻¹s⁻¹, or about 0.5 x 10⁶ M⁻¹s⁻¹ (inclusive); or about 0.5 x 10⁶ M⁻¹s⁻¹ to about 1 x 10⁶ M⁻¹s⁻¹ (inclusive), e.g., as measured in phosphate buffered saline using surface plasmon resonance (SPR).

### Fusion Proteins

In some embodiments, the anti-TNFα agent is a fusion protein (e.g., an extracellular domain of a TNFR fused to a partner peptide, e.g., an Fc region of an immunoglobulin, e.g., human IgG) (see, e.g., Deeg et al., Leukemia 16(2):162, 2002; Peppel et al., J. Exp. Med. 174(6):1483-1489, 1991) or a soluble TNFR (e.g., TNFR1 or TNFR2) that binds specifically to TNFα. In some embodiments, the anti-TNFα agent includes or is a soluble TNFα receptor (e.g., Bjornberg et al., Lymphokine Cytokine Res. 13(3):203-211, 1994; Kozak et al., Am. J. Physiol. Reg. Integrative Comparative Physiol. 269(1):R23-R29, 1995; Tsao et al., Eur Respir J. 14(3):490-495, 1999; Watt et al., J Leukoc Biol. 66(6):1005-1013, 1999; Mohler et al., J. Immunol. 151(3):1548-1561, 1993; Nophar et al., EMBO J. 9(10):3269, 1990; Piguet et al., Eur. Respiratory J. 7(3):515-518, 1994; and Gray et al., Proc. Natl. Acad. Sci. U.S.A. 87(19):7380-7384, 1990). In some embodiments, the anti-TNFα agent includes or is etanercept (Enbrel^{™}) (see, e.g., WO 91/03553 and WO 09/406,476, incorporated by reference herein). In some embodiments, the anti-TNFα agent inhibitor includes or is r-TBP-I (e.g., Gradstein et al., J. Acquir. Immune Defic. Syndr. 26(2): 111-117, 2001).

### Inhibitory Nucleic Acids

Inhibitory nucleic acids that can decrease the expression of AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA expression in a mammalian cell include antisense nucleic acid molecules, i.e., nucleic acid molecules whose nucleotide sequence is fully or partially complementary to all or part of a AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA (e.g., fully or partially complementary to all or a part of any one of the sequences presented in Table E).

**Table E.**

| **Human gene** | **mRNA GenBank accession number(s)** |
|---|---|
| Tumor necrosis factor (TNF, a.k.a. TNF-alpha) | NM_000594 |
| TNF receptor superfamily member 1A (TNFRSF1A) (a.k.a. TNFR1) | NM_001065 |
| | NM_001346091 |
| | NM_001346092 |
| TNF receptor superfamily member 1B (TNFRSF1B) (a.k.a. TNFR2) | NM_001066 |
| | XM_011542060 |
| | XM_011542063 |
| | XM_017002214 |
| | XM_017002215 |
| | XM_017002211 |
| TNFRSF1A associated via death domain (TRADD) | NM_003789 |
| | NM_001323552 |
| | XM_005256213 |
| | XM_017023815 |
| TNF receptor associated factor 2 (TRAF2) | NM_021138 |
| | XM_011518976 |
| | XM_011518977 |
| | XM_011518974 |
| JunD proto-oncogene, AP-1 transcription factor subunit (JUND) | NM_001286968 |
| | NM_005354 |
| Mitogen-activated protein kinase kinase kinase 5 (MAP3K5) (a.k.a. ASK1) | NM_005923 |
| | XM_017010875 |
| | XM_017010872 |
| | XM_017010873 |
| | XM_017010877 |
| | XM_017010874 |
| | XM_017010871 |
| | XM_017010870 |
| | XM_017010876 |
| | XM_011535839 |
| CD14 | NM_000591 |
| | NM_001040021 |
| | NM_001174104 |
| | NM_001174105 |
| Mitogen-activated protein kinase 3 (MAPK3) (a.k.a. ERK1) | NM_001040056 |
| | NM_001109891 |
| | NM_002746 |
| Mitogen-activated protein kinase 1 (MAPK1) (a.k.a. ERK2) | NM_002745 |
| | NM_138957 |
| Inhibitor of nuclear factor kappa B kinase subunit beta (IKBKB) | NM_001190720 |
| | NM_001242778 |
| | NM_001556 |
| | XM_005273491 |
| | XM_005273496 |
| | XM_005273493 |
| | XM_005273498 |
| | XM_011544518 |
| | XM_005273492 |
| | XM_005273490 |
| | XM_005273494 |
| | 12XM_017013396 |
| | XM_011544521 |
| | XM_011544522 |
| | XM_005273495 |
| | XM_011544517 |
| | XM_011544520 |
| | XM_011544519 |
| NFKB inhibitor alpha (NFKBIA) | NM_020529 |
| Interleukin 1 receptor associated kinase 1 (IRAK1) | NM_001025242 |
| | NM_001025243 |
| | NM_001569 |
| | XM_005274668 |
| Mitogen-activated protein kinase 8 (MAPK8) (a.k.a. JNK) | NM_001278547 |
| | NM_001278548 |
| | NM_001323302 |
| | NM_001323320 |
| | NM_001323321 |
| | NM_001323322 |
| | NM_001323323 |
| | NM_001323324 |
| | NM_001323325 |
| | NM_001323326 |
| | NM_001323327 |
| | NM_001323328 |
| | NM_001323329 |
| | NM_001323330 |
| | NM_001323331 |
| | NM_139046 |
| | NM_139049 |
| | XM_024448079 |
| | XM_024448080 |
| Lipopolysaccharide binding protein (LBP) | NM_004139 |
| Mitogen-activated protein kinase kinase 1 (MAP2K1) (a.k.a. MEK1) | NM_002755 |
| | XM_017022411 |
| | XM_011521783 |
| | XM_017022412 |
| | XM_017022413 |
| Mitogen-activated protein kinase kinase 2 (MAP2K2) (a.k.a. MEK2) | NM_030662 |
| | XM_006722799 |
| | XM_017026990 |
| | XM_017026989 |
| | XM_017026991 |
| Mitogen-activated protein kinase kinase 3 (MAP2K3) (a.k.a. MEK3) | NM_001316332 |
| | NM_002756 |
| | NM_145109 |
| | XM_017024859 |
| | XM_005256723 |
| | XM_017024857 |
| | XM_011523959 |
| | XM_017024858 |
| | XM_011523958 |
| Mitogen-activated protein kinase kinase 6 (MAP2K6) (a.k.a. MEK6) | NM_001330450 |
| | NM_002758 |
| | XM_005257516 |
| | XM_011525027 |
| | XM_011525026 |
| | XM_006721975 |
| Mitogen-activated protein kinase kinase kinase 1 (MAP3K1) (a.k.a. MEKK1) | NM_005921 |
| | XM_017009485 |
| | XM_017009484 |
| Mitogen-activated protein kinase kinase kinase 3 (MAP3K3) (a.k.a. MEKK3) | NM_001330431 |
| | NM_001363768 |
| | NM_002401 |
| | NM_203351 |
| | XM_005257378 |
| Mitogen-activated protein kinase kinase kinase 4 (MAP3K4) (a.k.a. MEKK4) | NM_001291958 |
| | NM_001301072 |
| | NM_001363582 |
| | NM_005922 |
| | NM_006724 |
| | XM_017010869 |
| Mitogen-activated protein kinase kinase kinase 6 (MAP3K6) (a.k.a. MEKK6) | NM_001297609 |
| | NM_004672 |
| | XM_017002771 |
| | XM_017002772 |
| Mitogen-activated protein kinase kinase kinase 7 (MAP3K7) (a.k.a. MEKK7) | NM_003188 |
| | NM_145331 |
| | NM_145332 |
| | NM_145333 |
| | XM_006715553 |
| | XM_017011226 |
| MAPK activated protein kinase 2 (MAPKAPK2) (a.k.a. MK2) | NM_004759 |
| | NM_032960 |
| | XM_005273353 |
| | XM_017002810 |
| MYD88, innate immune signal transduction adaptor (MYD88) | NM_001172566 |
| | NM_001172567 |
| | NM_001172568 |
| | NM_001172569 |
| | NM_001365876 |
| | NM_001365877 |
| | NM_002468 |
| Nuclear factor kappa B subunit 1 (NFKB1) | NM_001165412 |
| | NM_001319226 |
| | NM_003998 |
| | XM_024454069 |
| | XM_024454067 |
| | XM_011532006 |
| | XM_024454068 |
| Mitogen-activated protein kinase kinase kinase 14 (MAP3K14) (a.k.a. NIK) | NM_003954 |
| | XM_011525441 |
| Mitogen-activated protein kinase 14 (MAPK14) (a.k.a. p38) | NM_001315 |
| | NM_139012 |
| | NM_139013 |
| | NM_139014 |
| | XM_011514310 |
| | XM_017010300 |
| | XM_017010299 |
| | XM_017010301 |
| | XM_017010304 |
| | XM_017010303 |
| | XM_017010302 |
| | XM_006714998 |
| Eukaryotic translation initiation factor 2 alpha kinase 2 (EIF2AK2) (a.k.a. PKR) | NM_001135651 |
| | NM_001135652 |
| | NM_002759 |
| | XM_011532987 |
| | XM_017004503 |
| AKT serine/threonine kinase 1 (AKT1) (a.k.a. RAC) | NM_001014431 |
| | NM_001014432 |
| | NM_005163 |
| Zinc fingers and homeoboxes 2 (ZHX2) (a.k.a. RAF) | NM_001362797 |
| | NM_014943 |
| | XM_011516932 |
| | XM_005250836 |
| KRAS proto-oncogene, GTPase (KRAS) | NM_001369786 |
| | NM_001369787 |
| | NM_004985 |
| | NM_033360 |
| NRAS proto-oncogene, GTPase (NRAS) | NM_002524 |
| Receptor interacting serine/threonine kinase 1 (RIPK1) (a.k.a. RIP) | NM_001317061 |
| | NM_001354930 |
| | NM_001354931 |
| | NM_001354932 |
| | NM_001354933 |
| | NM_001354934 |
| | NM_003804 |
| | XM_017011405 |
| | XM_006715237 |
| | XM_017011403 |
| | XM_017011404 |
| TNF receptor associated factor 6 (TRAF6) | NM_004620 |
| | NM_145803 |
| | XM_017018220 |
| ZFP36 ring finger protein (ZFP36) (a.k.a. TTP) | NM_003407 |

An antisense nucleic acid molecule can be fully or partially complementary to all or part of a non-coding region of the coding strand of a nucleotide sequence encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein. Non-coding regions (5' and 3' untranslated regions) are the 5' and 3' sequences that flank the coding region in a gene and are not translated into amino acids.

Based upon the sequences disclosed herein, one of skill in the art can easily choose and synthesize any of a number of appropriate antisense nucleic acids to target a nucleic acid encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein described herein. Antisense nucleic acids targeting a nucleic acid encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein can be designed using the software available at the Integrated DNA Technologies website.

An antisense nucleic acid can be, for example, about 5, 10, 15, 18, 20, 22, 24, 25, 26, 28, 30, 32, 35, 36, 38, 40, 42, 44, 45, 46, 48, or 50 nucleotides or more in length. An antisense oligonucleotide can be constructed using enzymatic ligation reactions and chemical synthesis using procedures known in the art. For example, an antisense nucleic acid can be chemically synthesized using variously modified nucleotides or naturally occurring nucleotides designed to increase the physical stability of the duplex formed between the antisense and sense nucleic acids, e.g., phosphorothioate derivatives and acridine substituted nucleotides or to increase the biological stability of the molecules.

Examples of modified nucleotides which can be used to generate an antisense nucleic acid include 1-methylguanine, 1-methylinosine, 2,2-dimethylguanine, 2-methyladenine, 2-methylguanine, 3-methylcytosine, 2-methylthio-N6-isopentenyladenine, uracil-5-oxyacetic acid (v), wybutoxosine, pseudouracil, queosine, 2-thiocytosine, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest).

The antisense nucleic acid molecules described herein can be prepared in vitro and administered to a subject, e.g., a human subject. Alternatively, they can be generated in situ such that they hybridize with or bind to cellular mRNA and/or genomic DNA encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP protein to thereby inhibit expression, e.g., by inhibiting transcription and/or translation. The hybridization can be by conventional nucleotide complementarities to form a stable duplex, or, for example, in the case of an antisense nucleic acid molecule that binds to DNA duplexes, through specific interactions in the major groove of the double helix. The antisense nucleic acid molecules can be delivered to a mammalian cell using a vector (e.g., an adenovirus vector, a lentivirus, or a retrovirus).

An antisense nucleic acid can be an α-anomeric nucleic acid molecule. An α-anomeric nucleic acid molecule forms specific double-stranded hybrids with complementary RNA in which, contrary to the usual, β-units, the strands run parallel to each other (Gaultier et al., Nucleic Acids Res. 15:6625-6641, 1987). The antisense nucleic acid can also comprise a chimeric RNA-DNA analog (Inoue et al., FEBS Lett. 215:327-330, 1987) or a 2'-O-methylribonucleotide (Inoue et al., Nucleic Acids Res. 15:6131-6148, 1987).

Another example of an inhibitory nucleic acid is a ribozyme that has specificity for a nucleic acid encoding an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA, e.g., specificity for any one of the sequences presented in Table E). Ribozymes are catalytic RNA molecules with ribonuclease activity that are capable of cleaving a single-stranded nucleic acid, such as an mRNA, to which they have a complementary region. Thus, ribozymes (e.g., hammerhead ribozymes (described in Haselhoff and Gerlach, Nature 334:585-591, 1988)) can be used to catalytically cleave mRNA transcripts to thereby inhibit translation of the protein encoded by the mRNA. An AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA can be used to select a catalytic RNA having a specific ribonuclease activity from a pool of RNA molecules. See, e.g., Bartel et al., Science 261:1411-1418, 1993.

Alternatively, a ribozyme having specificity for an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA can be designed based upon the nucleotide sequence of any of the AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA sequences disclosed herein (e.g., in Table E). For example, a derivative of a Tetrahymena L-19 IVS RNA can be constructed in which the nucleotide sequence of the active site is complementary to the nucleotide sequence to be cleaved in an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP mRNA (see, e.g., U.S. Patent. Nos. 4,987,071 and 5,116,742).

An inhibitory nucleic acid can also be a nucleic acid molecule that forms triple helical structures. For example, expression of an AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP polypeptide can be inhibited by targeting nucleotide sequences complementary to the regulatory region of the gene encoding the AP-1, ASK1, CD14, c-jun, ERK1/2, IκB, IKK, IRAK, JNK, LBP, MAPK, MEK1/2, MEKK1/4, MEKK4/7, MEKK 3/6, MK2, MyD88, NF-κB, NIK, p38, PKR, rac, ras, raf, RIP, TNFα, TNFR1, TNFR2, TRADD, TRAF2, TRAF6, or TTP polypeptide (e.g., the promoter and/or enhancer, e.g., a sequence that is at least 1 kb, 2 kb, 3 kb, 4 kb, or 5 kb upstream of the transcription initiation start state) to form triple helical structures that prevent transcription of the gene in target cells. See generally Maher, Bioassays 14(12):807-15, 1992; Helene, Anticancer Drug Des. 6(6):569-84, 1991; and Helene, Ann. N.Y. Acad. Sci. 660:27-36, 1992.

In various embodiments, inhibitory nucleic acids can be modified at the sugar moiety, the base moiety, or phosphate backbone to improve, e.g., the solubility, stability, or hybridization, of the molecule. For example, the deoxyribose phosphate backbone of the nucleic acids can be modified to generate peptide nucleic acids (see, e.g., Hyrup et al., Bioorganic Medicinal Chem. 4(1):5-23, 1996). Peptide nucleic acids (PNAs) are nucleic acid mimics, e.g., DNA mimics, in which the deoxyribose phosphate backbone is replaced by a pseudopeptide backbone and only the four natural nucleobases are retained. The neutral backbone of PNAs allows for specific hybridization to RNA and DNA under conditions of low ionic strength. PNA oligomers can be synthesized using standard solid phase peptide synthesis protocols (see, e.g., Perry-O'Keefe et al., Proc. Natl. Acad. Sci. U.S.A. 93:14670-675, 1996). PNAs can be used as antisense or antigene agents for sequence-specific modulation of gene expression by, e.g., inducing transcription or translation arrest or inhibiting replication.

### Small Molecules

In some embodiments, the anti-TNFα agent is a small molecule. In some embodiments, the small molecule is a tumor necrosis factor-converting enzyme (TACE) inhibitor (e.g., Moss et al., Nature Clinical Practice Rheumatology 4: 300-309, 2008). In some embodiments, the anti-TNFα agent is C87 (Ma et al., J. Biol. Chem. 289(18):12457-66, 2014). In some embodiments, the small molecule is LMP-420 (e.g., Haraguchi et al., AIDS Res. Ther. 3:8, 2006). In some embodiments, the TACE inhibitor is TMI-005 and BMS-561392. Additional examples of small molecule inhibitors are described in, e.g., He et al., Science 310(5750):1022-1025, 2005.

In some examples, the anti-TNFα agent is a small molecule that inhibits the activity of one of AP-1, ASK1, IKK, JNK, MAPK, MEKK 1/4, MEKK4/7, MEKK 3/6, NIK, TRADD, RIP, NF-κB, and TRADD in a cell (e.g., in a cell obtained from a subject, a mammalian cell).

In some examples, the anti-TNFα agent is a small molecule that inhibits the activity of one of CD14, MyD88 (see, e.g., Olson et al., Scientific Reports 5:14246, 2015), ras (e.g., Baker et al., Nature 497:577-578, 2013), raf (e.g., vemurafenib (PLX4032, RG7204), sorafenib tosylate, PLX-4720, dabrafenib (GSK2118436), GDC-0879, RAF265 (CHIR-265), AZ 628, NVP-BHG712, SB590885, ZM 336372, sorafenib, GW5074, TAK-632, CEP-32496, encorafenib (LGX818), CCT196969, LY3009120, RO5126766 (CH5126766), PLX7904, and MLN2480).

In some examples, the anti-TNFα agent TNFα inhibitor is a small molecule that inhibits the activity of one of MK2 (PF 3644022 and PHA 767491), JNK (e.g., AEG 3482, BI 78D3, CEP 1347, c-JUN peptide, IQ 1S, JIP-1 (153-163), SP600125, SU 3327, and TCS JNK60), c-jun (e.g., AEG 3482, BI 78D3, CEP 1347, c-JUN peptide, IQ 1S, JIP-1 (153-163), SP600125, SU 3327, and TCS JNK60), MEK3/6 (e.g., Akinleye et al., J. Hematol. Oncol. 6:27, 2013), p38 (e.g., AL 8697, AMG 548, BIRB 796, CMPD-1, DBM 1285 dihydrochloride, EO 1428, JX 401, ML 3403, Org 48762-0, PH 797804, RWJ 67657, SB 202190, SB 203580, SB 239063, SB 706504, SCIO 469, SKF 86002, SX 011, TA 01, TA 02, TAK 715, VX 702, and VX 745), PKR (e.g., 2-aminopurine or CAS 608512-97-6), TTP (e.g., CAS 329907-28-0), MEK1/2 (e.g., Facciorusso et al., Expert Review Gastroentrol. Hepatol. 9:993-1003, 2015), ERK1/2 (e.g., Mandal et al., Oncogene 35:2547-2561, 2016), NIK (e.g., Mortier et al., Bioorg. Med. Chem. Lett. 20:4515-4520, 2010), IKK (e.g., Reilly et al., Nature Med. 19:313-321, 2013), IκB (e.g., Suzuki et al., Expert. Opin. Invest. Drugs 20:395-405, 2011), NF-κB (e.g., Gupta et al., Biochim. Biophys. Acta 1799(10-12):775-787, 2010), rac (e.g., U.S. Patent No. 9,278,956), MEK4/7, IRAK (Chaudhary et al., J. Med. Chem. 58(1):96-110, 2015), LBP (see, e.g., U.S. Patent No. 5,705,398), and TRAF6 (e.g., 3-[(2,5-Dimethylphenyl)amino]-1-phenyl-2-propen-1-one).

In some embodiments of any of the methods described herein, the inhibitory nucleic acid can be about 10 nucleotides to about 50 nucleotides (e.g., about 10 nucleotides to about 45 nucleotides, about 10 nucleotides to about 40 nucleotides, about 10 nucleotides to about 35 nucleotides, about 10 nucleotides to about 30 nucleotides, about 10 nucleotides to about 28 nucleotides, about 10 nucleotides to about 26 nucleotides, about 10 nucleotides to about 25 nucleotides, about 10 nucleotides to about 24 nucleotides, about 10 nucleotides to about 22 nucleotides, about 10 nucleotides to about 20 nucleotides, about 10 nucleotides to about 18 nucleotides, about 10 nucleotides to about 16 nucleotides, about 10 nucleotides to about 14 nucleotides, about 10 nucleotides to about 12 nucleotides, about 12 nucleotides to about 50 nucleotides, about 12 nucleotides to about 45 nucleotides, about 12 nucleotides to about 40 nucleotides, about 12 nucleotides to about 35 nucleotides, about 12 nucleotides to about 30 nucleotides, about 12 nucleotides to about 28 nucleotides, about 12 nucleotides to about 26 nucleotides, about 12 nucleotides to about 25 nucleotides, about 12 nucleotides to about 24 nucleotides, about 12 nucleotides to about 22 nucleotides, about 12 nucleotides to about 20 nucleotides, about 12 nucleotides to about 18 nucleotides, about 12 nucleotides to about 16 nucleotides, about 12 nucleotides to about 14 nucleotides, about 15 nucleotides to about 50 nucleotides, about 15nucleotides to about 45 nucleotides, about 15nucleotides to about 40 nucleotides, about 15nucleotides to about 35 nucleotides, about 15 nucleotides to about 30 nucleotides, about 15nucleotides to about 28 nucleotides, about 15nucleotides to about 26 nucleotides, about 15nucleotides to about 25 nucleotides, about 15nucleotides to about 24 nucleotides, about 15nucleotides to about 22 nucleotides, about 15nucleotides to about 20 nucleotides, about 15nucleotides to about 18 nucleotides, about 15nucleotides to about 16 nucleotides, about 16 nucleotides to about 50 nucleotides, about 16 nucleotides to about 45 nucleotides, about 16 nucleotides to about 40 nucleotides, about 16 nucleotides to about 35 nucleotides, about 16 nucleotides to about 30 nucleotides, about 16 nucleotides to about 28 nucleotides, about 16 nucleotides to about 26 nucleotides, about 16 nucleotides to about 25 nucleotides, about 16 nucleotides to about 24 nucleotides, about 16 nucleotides to about 22 nucleotides, about 16 nucleotides to about 20 nucleotides, about 16 nucleotides to about 18 nucleotides, about 18 nucleotides to about 20 nucleotides, about 20 nucleotides to about 50 nucleotides, about 20 nucleotides to about 45 nucleotides, about 20 nucleotides to about 40 nucleotides, about 20 nucleotides to about 35 nucleotides, about 20 nucleotides to about 30 nucleotides, about 20 nucleotides to about 28 nucleotides, about 20 nucleotides to about 26 nucleotides, about 20 nucleotides to about 25 nucleotides, about 20 nucleotides to about 24 nucleotides, about 20 nucleotides to about 22 nucleotides, about 24 nucleotides to about 50 nucleotides, about 24 nucleotides to about 45 nucleotides, about 24 nucleotides to about 40 nucleotides, about 24 nucleotides to about 35 nucleotides, about 24 nucleotides to about 30 nucleotides, about 24 nucleotides to about 28 nucleotides, about 24 nucleotides to about 26 nucleotides, about 24 nucleotides to about 25 nucleotides, about 26 nucleotides to about 50 nucleotides, about 26 nucleotides to about 45 nucleotides, about 26 nucleotides to about 40 nucleotides, about 26 nucleotides to about 35 nucleotides, about 26 nucleotides to about 30 nucleotides, about 26 nucleotides to about 28 nucleotides, about 28 nucleotides to about 50 nucleotides, about 28 nucleotides to about 45 nucleotides, about 28 nucleotides to about 40 nucleotides, about 28 nucleotides to about 35 nucleotides, about 28 nucleotides to about 30 nucleotides, about 30 nucleotides to about 50 nucleotides, about 30 nucleotides to about 45 nucleotides, about 30 nucleotides to about 40 nucleotides, about 30 nucleotides to about 38 nucleotides, about 30 nucleotides to about 36 nucleotides, about 30 nucleotides to about 34 nucleotides, about 30 nucleotides to about 32 nucleotides, about 32 nucleotides to about 50 nucleotides, about 32 nucleotides to about 45 nucleotides, about 32 nucleotides to about 40 nucleotides, about 32 nucleotides to about 35 nucleotides, about 35 nucleotides to about 50 nucleotides, about 35 nucleotides to about 45 nucleotides, about 35 nucleotides to about 40 nucleotides, about 40 nucleotides to about 50 nucleotides, about 40 nucleotides to about 45 nucleotides, about 42 nucleotides to about 50 nucleotides, about 42 nucleotides to about 45 nucleotides, or about 45 nucleotides to about 50 nucleotides) in length. One skilled in the art will appreciate that inhibitory nucleic acids may comprises at least one modified nucleic acid at either the 5' or 3' end of DNA or RNA.

In some embodiments, the inhibitory nucleic acid can be formulated in a liposome, a micelle (e.g., a mixed micelle), a nanoemulsion, or a microemulsion, a solid nanoparticle, or a nanoparticle (e.g., a nanoparticle including one or more synthetic polymers). Additional exemplary structural features of inhibitory nucleic acids and formulations of inhibitory nucleic acids are described in US 2016/0090598.

In some embodiments, the inhibitory nucleic acid (e.g., any of the inhibitory nucleic acid described herein) can include a sterile saline solution (e.g., phosphate-buffered saline (PBS)). In some embodiments, the inhibitory nucleic acid (e.g., any of the inhibitory nucleic acid described herein) can include a tissue-specific delivery molecule (e.g., a tissue-specific antibody).

### Pharmaceutical Compositions and Administration

### General

In some embodiments, a chemical entity (e.g., a compound that modulates (e.g., antagonizes) NLRP3, or a pharmaceutically acceptable salt, and/or hydrate, and/or cocrystal, and/or drug combination thereof) is administered as a pharmaceutical composition that includes the chemical entity and one or more pharmaceutically acceptable excipients, and optionally one or more additional therapeutic agents as described herein.

In some embodiments, the chemical entities can be administered in combination with one or more conventional pharmaceutical excipients. Pharmaceutically acceptable excipients include, but are not limited to, ion exchangers, alumina, aluminum stearate, lecithin, self-emulsifying drug delivery systems (SEDDS) such as d-α-tocopherolpolyethylene glycol 1000 succinate, surfactants used in pharmaceutical dosage forms such as Tweens, poloxamers or other similar polymeric delivery matrices, serum proteins, such as human serum albumin, buffer substances such as phosphates, tris, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such as hydroxyalkylcyclodextrins, including 2- and 3-hydroxypropyl-β-cyclodextrins, or other solubilized derivatives can also be used to enhance delivery of compounds described herein. Dosage forms or compositions containing a chemical entity as described herein in the range of 0.005% to 100% with the balance made up from non-toxic excipient may be prepared. The contemplated compositions may contain 0.001%-100% of a chemical entity provided herein, in one embodiment 0.1-95%, in another embodiment 75-85%, in a further embodiment 20-80%. Actual methods of preparing such dosage forms are known, or will be apparent, to those skilled in this art; for example, see Remington: The Science and Practice of Pharmacy, 22nd Edition (Pharmaceutical Press, London, UK. 2012).

In some embodiments, an NLRP3 antagonist and/or an anti-TNFα agent disclosed herein is administered as a pharmaceutical composition that includes the NLRP3 antagonist and/or anti-TNFα agent and one or more pharmaceutically acceptable excipients, and optionally one or more additional therapeutic agents as described herein. Preferably the pharmaceutical composition that includes an NLRP3 antagonist and an anti-TNFα agent.

Preferably the above pharmaceutical composition embodiments comprise an NLRP3 antagonist disclosed herein. More preferably the above pharmaceutical composition embodiments comprise an NLRP3 antagonist and an anti-TNFα agent disclosed herein.

### Routes of Administration and Composition Components

In some embodiments, the chemical entities described herein or a pharmaceutical composition thereof can be administered to subject in need thereof by any accepted route of administration. Acceptable routes of administration include, but are not limited to, buccal, cutaneous, endocervical, endosinusial, endotracheal, enteral, epidural, interstitial, intra-abdominal, intra-arterial, intrabronchial, intrabursal, intracerebral, intracisternal, intracoronary, intradermal, intraductal, intraduodenal, intradural, intraepidermal, intraesophageal, intragastric, intragingival, intraileal, intralymphatic, intramedullary, intrameningeal, intramuscular, intraovarian, intraperitoneal, intraprostatic, intrapulmonary, intrasinal, intraspinal, intrasynovial, intratesticular, intrathecal, intratubular, intratumoral, intrauterine, intravascular, intravenous, nasal, nasogastric, oral, parenteral, percutaneous, peridural, rectal, respiratory (inhalation), subcutaneous, sublingual, submucosal, topical, transdermal, transmucosal, transtracheal, ureteral, urethral and vaginal. In certain embodiments, a preferred route of administration is parenteral (e.g., intratumoral).

Compositions can be formulated for parenteral administration, e.g., formulated for injection via the intravenous, intramuscular, sub-cutaneous, or even intraperitoneal routes. Typically, such compositions can be prepared as injectables, either as liquid solutions or suspensions; solid forms suitable for use to prepare solutions or suspensions upon the addition of a liquid prior to injection can also be prepared; and the preparations can also be emulsified. The preparation of such formulations will be known to those of skill in the art in light of the present disclosure.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The carrier also can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof.

Intratumoral injections are discussed, e.g., in Lammers, et al., "Effect of Intratumoral Injection on the Biodistribution and the Therapeutic Potential of HPMA Copolymer-Based Drug Delivery Systems" Neoplasia. 2006, 10, 788-795.

In certain embodiments, the chemical entities described herein or a pharmaceutical composition thereof are suitable for local, topical administration to the digestive or GI tract, e.g., rectal administration. Rectal compositions include, without limitation, enemas, rectal gels, rectal foams, rectal aerosols, suppositories, jelly suppositories, and enemas (e.g., retention enemas).

Pharmacologically acceptable excipients usable in the rectal composition as a gel, cream, enema, or rectal suppository, include, without limitation, any one or more of cocoa butter glycerides, synthetic polymers such as polyvinylpyrrolidone, PEG (like PEG ointments), glycerine, glycerinated gelatin, hydrogenated vegetable oils, poloxamers, mixtures of polyethylene glycols of various molecular weights and fatty acid esters of polyethylene glycol Vaseline, anhydrous lanolin, shark liver oil, sodium saccharinate, menthol, sweet almond oil, sorbitol, sodium benzoate, anoxid SBN, vanilla essential oil, aerosol, parabens in phenoxyethanol, sodium methyl p-oxybenzoate, sodium propyl p-oxybenzoate, diethylamine, carbomers, carbopol, methyloxybenzoate, macrogol cetostearyl ether, cocoyl caprylocaprate, isopropyl alcohol, propylene glycol, liquid paraffin, xanthan gum, carboxy-metabisulfite, sodium edetate, sodium benzoate, potassium metabisulfite, grapefruit seed extract, methyl sulfonyl methane (MSM) , lactic acid, glycine, vitamins, such as vitamin A and E and potassium acetate.

In certain embodiments, suppositories can be prepared by mixing the chemical entities described herein with suitable non-irritating excipients or carriers such as cocoa butter, polyethylene glycol or a suppository wax which are solid at ambient temperature but liquid at body temperature and therefore melt in the rectum and release the active compound. In other embodiments, compositions for rectal administration are in the form of an enema.

In other embodiments, the compounds described herein or a pharmaceutical composition thereof are suitable for local delivery to the digestive or GI tract by way of oral administration (e.g., solid or liquid dosage forms.).

Solid dosage forms for oral administration include capsules, tablets, pills, powders, and granules. In such solid dosage forms, the chemical entity is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

In one embodiment, the compositions will take the form of a unit dosage form such as a pill or tablet and thus the composition may contain, along with a chemical entity provided herein, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In another solid dosage form, a powder, marume, solution or suspension (*e.g*., in propylene carbonate, vegetable oils, PEG's, poloxamer 124 or triglycerides) is encapsulated in a capsule (gelatin or cellulose base capsule). Unit dosage forms in which one or more chemical entities provided herein or additional active agents are physically separated are also contemplated; *e.g*., capsules with granules (or tablets in a capsule) of each drug; two-layer tablets; two-compartment gel caps, etc. Enteric coated or delayed release oral dosage forms are also contemplated.

Other physiologically acceptable compounds include wetting agents, emulsifying agents, dispersing agents or preservatives that are particularly useful for preventing the growth or action of microorganisms. Various preservatives are well known and include, for example, phenol and ascorbic acid.

In certain embodiments the excipients are sterile and generally free of undesirable matter. These compositions can be sterilized by conventional, well-known sterilization techniques. For various oral dosage form excipients such as tablets and capsules sterility is not required. The USP/NF standard is usually sufficient.

In certain embodiments, solid oral dosage forms can further include one or more components that chemically and/or structurally predispose the composition for delivery of the chemical entity to the stomach or the lower GI; e.g., the ascending colon and/or transverse colon and/or distal colon and/or small bowel. Exemplary formulation techniques are described in, e.g., Filipski, K.J., et al., Current Topics in Medicinal Chemistry, 2013, 13, 776-802, which is incorporated herein by reference in its entirety.

Examples include upper-GI targeting techniques, e.g., Accordion Pill (Intec Pharma), floating capsules, and materials capable of adhering to mucosal walls.

Other examples include lower-GI targeting techniques. For targeting various regions in the intestinal tract, several enteric/pH-responsive coatings and excipients are available. These materials are typically polymers that are designed to dissolve or erode at specific pH ranges, selected based upon the GI region of desired drug release. These materials also function to protect acid labile drugs from gastric fluid or limit exposure in cases where the active ingredient may be irritating to the upper GI (e.g., hydroxypropyl methylcellulose phthalate series, Coateric (polyvinyl acetate phthalate), cellulose acetate phthalate, hydroxypropyl methylcellulose acetate succinate, Eudragit series (methacrylic acid-methyl methacrylate copolymers), and Marcoat). Other techniques include dosage forms that respond to local flora in the GI tract, Pressure-controlled colon delivery capsule, and Pulsincap.

Ocular compositions can include, without limitation, one or more of any of the following: viscogens (e.g., Carboxymethylcellulose, Glycerin, Polyvinylpyrrolidone, Polyethylene glycol); Stabilizers (e.g., Pluronic (triblock copolymers), Cyclodextrins); Preservatives (e.g., Benzalkonium chloride, ETDA, SofZia (boric acid, propylene glycol, sorbitol, and zinc chloride; Alcon Laboratories, Inc.), Purite (stabilized oxychloro complex; Allergan, Inc.)).

Topical compositions can include ointments and creams. Ointments are semisolid preparations that are typically based on petrolatum or other petroleum derivatives. Creams containing the selected active agent are typically viscous liquid or semisolid emulsions, often either oil-in-water or water-in-oil. Cream bases are typically water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. As with other carriers or vehicles, an ointment base should be inert, stable, nonirritating and non-sensitizing.

In any of the foregoing embodiments, pharmaceutical compositions described herein can include one or more one or more of the following: lipids, interbilayer crosslinked multilamellar vesicles, biodegradeable poly(D,L-lactic-co-glycolic acid) [PLGA]-based or poly anhydride-based nanoparticles or microparticles, and nanoporous particle-supported lipid bilayers.

### Enema Formulations

In some embodiments, enema formulations containing the chemical entities described herein are provided in "ready-to-use" form.

In some embodiments, enema formulations containing the chemical entities described herein are provided in one or more kits or packs. In certain embodiments, the kit or pack includes two or more separately contained/packaged components, e.g. two components, which when mixed together, provide the desired formulation (e.g., as a suspension). In certain of these embodiments, the two component system includes a first component and a second component, in which: **(i)** the first component (e.g., contained in a sachet) includes the chemical entity (as described anywhere herein) and optionally one or more pharmaceutically acceptable excipients (e.g., together formulated as a solid preparation, e.g., together formulated as a wet granulated solid preparation); and **(ii)** the second component (e.g., contained in a vial or bottle) includes one or more liquids and optionally one or more other pharmaceutically acceptable excipients together forming a liquid carrier. Prior to use (e.g., immediately prior to use), the contents of **(i)** and **(ii)** are combined to form the desired enema formulation, e.g., as a suspension. In other embodiments, each of component **(i)** and **(ii)** is provided in its own separate kit or pack.

In some embodiments, each of the one or more liquids is water, or a physiologically acceptable solvent, or a mixture of water and one or more physiologically acceptable solvents. Typical such solvents include, without limitation, glycerol, ethylene glycol, propylene glycol, polyethylene glycol and polypropylene glycol. In certain embodiments, each of the one or more liquids is water. In other embodiments, each of the one or more liquids is an oil, e.g. natural and/or synthetic oils that are commonly used in pharmaceutical preparations.

Further pharmaceutical excipients and carriers that may be used in the pharmaceutical products herein described are listed in various handbooks (e.g. D. E. Bugay and W. P. Findlay (Eds) Pharmaceutical excipients (Marcel Dekker, New York, 1999), E-M Hoepfner, A. Reng and P. C. Schmidt (Eds) Fiedler Encyclopedia of Excipients for Pharmaceuticals, Cosmetics and Related Areas (Edition Cantor, Munich, 2002) and H. P. Fielder (Ed) Lexikon der Hilfsstoffe für Pharmazie, Kosmetik and angrenzende Gebiete (Edition Cantor Aulendorf, 1989)).

In some embodiments, each of the one or more pharmaceutically acceptable excipients can be independently selelcted from thickeners, viscosity enhancing agents, bulking agents, mucoadhesive agents, penetration enhanceers, buffers, preservatives, diluents, binders, lubricants, glidants, disintegrants, fillers, solubilizing agents, pH modifying agents, preservatives, stabilizing agents, anti-oxidants, wetting or emulsifying agents, suspending agents, pigments, colorants, isotonic agents, chelating agents, emulsifiers, and diagnostic agents.

In certain embodiments, each of the one or more pharmaceutically acceptable excipients can be independently selelcted from thickeners, viscosity enhancing agents, mucoadhesive agents, buffers, preservatives, diluents, binders, lubricants, glidants, disintegrants, and fillers.

In certain embodiments, each of the one or more pharmaceutically acceptable excipients can be independently selelcted from thickeners, viscosity enhancing agents, bulking agents, mucoadhesive agents, buffers, preservatives, and fillers.

In certain embodiments, each of the one or more pharmaceutically acceptable excipients can be independently selelcted from diluents, binders, lubricants, glidants, and disintegrants.

Examples of thickeners, viscosity enhancing agents, and mucoadhesive agents include without limitation: gums, e.g. xanthan gum, guar gum, locust bean gum, tragacanth gums, karaya gum, ghatti gum, cholla gum, psyllium seed gum and gum arabic; poly(carboxylic acid-containing) based polymers, such as poly (acrylic, maleic, itaconic, citraconic, hydroxyethyl methacrylic or methacrylic) acid which have strong hydrogen-bonding groups, or derivatives thereof such as salts and esters; cellulose derivatives, such as methyl cellulose, ethyl cellulose, methylethyl cellulose, hydroxymethyl cellulose, hydroxyethyl cellulose, hydroxypropyl cellulose, hydroxyethyl ethyl cellulose, carboxymethyl cellulose, hydroxypropylmethyl cellulose or cellulose esters or ethers or derivatives or salts thereof; clays such as manomorillonite clays, e.g. Veegun, attapulgite clay; polysaccharides such as dextran, pectin, amylopectin, agar, mannan or polygalactonic acid or starches such as hydroxypropyl starch or carboxymethyl starch; polypeptides such as casein, gluten, gelatin, fibrin glue; chitosan, e.g. lactate or glutamate or carboxymethyl chitin; glycosaminoglycans such as hyaluronic acid; metals or water soluble salts of alginic acid such as sodium alginate or magnesium alginate; schleroglucan; adhesives containing bismuth oxide or aluminium oxide; atherocollagen; polyvinyl polymers such as carboxyvinyl polymers; polyvinylpyrrolidone (povidone); polyvinyl alcohol; polyvinyl acetates, polyvinylmethyl ethers, polyvinyl chlorides, polyvinylidenes, and/or the like; polycarboxylated vinyl polymers such as polyacrylic acid as mentioned above; polysiloxanes; polyethers; polyethylene oxides and glycols; polyalkoxys and polyacrylamides and derivatives and salts thereof. Preferred examples can include cellulose derivatives, such as methyl cellulose, ethyl cellulose, methylethyl cellulose, hydroxymethyl cellulose, hydroxyethyl cellulose, hydroxypropyl cellulose, hydroxyethyl ethyl cellulose, carboxymethyl cellulose, hydroxypropylmethyl cellulose or cellulose esters or ethers or derivatives or salts thereof (e.g., methyl cellulose); and polyvinyl polymers such as polyvinylpyrrolidone (povidone).

Examples of preservatives include without limitation: benzalkonium chloride, benzoxonium chloride, benzethonium chloride, cetrimide, sepazonium chloride, cetylpyridinium chloride, domiphen bromide (Bradosol^{®}), thiomersal, phenylmercuric nitrate, phenylmercuric acetate, phenylmercuric borate, methylparaben, propylparaben, chlorobutanol, benzyl alcohol, phenyl ethyl alcohol, chlorohexidine, polyhexamethylene biguanide, sodium perborate, imidazolidinyl urea, sorbic acid, Purite^{®}), Polyquart^{®}), and sodium perborate tetrahydrate and the like.

In certain embodiments, the preservative is a paraben, or a pharmaceutically acceptable salt thereof. In some embodiments, the paraben is an alkyl substituted 4-hydroxybenzoate, or a pharmaceutically acceptable salt or ester thereof. In certain embodiments, the alkyl is a C1-C4 alkyl. In certain embodiments, the preservative is methyl 4-hydroxybenzoate (methylparaben), or a pharmaceutically acceptable salt or ester thereof, propyl 4-hydroxybenzoate (propylparaben), or a pharmaceutically acceptable salt or ester thereof, or a combination thereof.

Examples of buffers include without limitation: phosphate buffer system (sodium dihydrogen phospahate dehydrate, disodium phosphate dodecahydrate, bibasic sodium phosphate, anhydrous monobasic sodium phosphate), bicarbonate buffer system, and bisulfate buffer system.

Examples of disintegrants include, without limitation: carmellose calcium, low substituted hydroxypropyl cellulose (L-HPC), carmellose, croscarmellose sodium, partially pregelatinized starch, dry starch, carboxymethyl starch sodium, crospovidone, polysorbate 80 (polyoxyethylenesorbitan oleate), starch, sodium starch glycolate, hydroxypropyl cellulose pregelatinized starch, clays, cellulose, alginine, gums or cross linked polymers, such as crosslinked PVP (Polyplasdone XL from GAF Chemical Corp). In certain embodiments, the disintegrant is crospovidone.

Examples of glidants and lubricants (aggregation inhibitors) include without limitation: talc, magnesium stearate, calcium stearate, colloidal silica, stearic acid, aqueous silicon dioxide, synthetic magnesium silicate, fine granulated silicon oxide, starch, sodium laurylsulfate, boric acid, magnesium oxide, waxes, hydrogenated oil, polyethylene glycol, sodium benzoate, stearic acid glycerol behenate, polyethylene glycol, and mineral oil. In certain embodiments, the glidant/lubricant is magnesium stearate, talc, and/or colloidal silica; e.g., magnesium stearate and/or talc.

Examples of diluents, also referred to as "fillers" or "bulking agents" include without limitation: dicalcium phosphate dihydrate, calcium sulfate, lactose (e.g., lactose monohydrate), sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powdered sugar. In certain embodiments, the diluent is lactose (e.g., lactose monohydrate).

Examples of binders include without limitation: starch, pregelatinized starch, gelatin, sugars (including sucrose, glucose, dxtrose, lactose and sorbitol), polyethylene glycol, waxes, natural and synthetic gums such as acacia tragacanth, sodium alginate cellulose, including hydroxypropylmethylcellulose, hydroxypropylcellulose, ethylcellulose, and veegum, and synthetic polymers such as acrylic acid and methacrylic acid copolymers, methacrylic acid copolymers, methyl methacrylate copolymers, aminoalkyl methacrylate copolymers, polyacrylic acid/polymethacrylic acid and polyvinylpyrrolidone (povidone). In certain embodiments, the binder is polyvinylpyrrolidone (povidone).

In some embodiments, enema formulations containing the chemical entities described herein include water and one or more (e.g., all) of the following excipients:
- One or more (e.g., one, two, or three) thickeners, viscosity enhancing agents, binders, and/or mucoadhesive agents (e.g., cellulose or cellulose esters or ethers or derivatives or salts thereof (e.g., methyl cellulose); and polyvinyl polymers such as polyvinylpyrrolidone (povidone);
- One or more (e.g., one or two; e.g., two) preservatives, such as a paraben, e.g., methyl 4-hydroxybenzoate (methylparaben), or a pharmaceutically acceptable salt or ester thereof, propyl 4-hydroxybenzoate (propylparaben), or a pharmaceutically acceptable salt or ester thereof, or a combination thereof;
- One or more (e.g., one or two; e.g., two) buffers, such as phosphate buffer system (e.g., sodium dihydrogen phospahate dehydrate, disodium phosphate dodecahydrate);
- One or more (e.g., one or two, e.g., two) glidants and/or lubricants, such as magnesium stearate and/or talc;
- One or more (e.g., one or two; e.g., one) disintegrants, such as crospovidone; and
- One or more (e.g., one or two; e.g., one) diluents, such as lactose (e.g., lactose monohydrate).

In certain of these embodiments, the chemical entity is a compound of Formula AA, or a pharmaceutically acceptable salt and/or hydrate and/or cocrystal thereof.

In certain embodiments, enema formulations containing the chemical entities described herein include water, methyl cellulose, povidone, methylparaben, propylparaben, sodium dihydrogen phospahate dehydrate, disodium phosphate dodecahydrate, crospovidone, lactose monohydrate, magnesium stearate, and talc. In certain of these embodiments, the chemical entity is a compound of Formula AA, or a pharmaceutically acceptable salt and/or hydrate and/or cocrystal thereof.

In certain embodiments, enema formulations containing the chemical entities described herein are provided in one or more kits or packs. In certain embodiments, the kit or pack includes two separately contained/packaged components, which when mixed together, provide the desired formulation (e.g., as a suspension). In certain of these embodiments, the two component system includes a first component and a second component, in which: **(i)** the first component (e.g., contained in a sachet) includes the chemical entity (as described anywhere herein) and one or more pharmaceutically acceptable excipients (e.g., together formulated as a solid preparation, e.g., together formulated as a wet granulated solid preparation); and **(ii)** the second component (e.g., contained in a vial or bottle) includes one or more liquids and one or more one or more other pharmaceutically acceptable excipients together forming a liquid carrier. In other embodiments, each of component **(i)** and **(ii)** is provided in its own separate kit or pack.

In certain of these embodiments, component **(i)** includes the chemical entitiy (e.g., a compound of Formula AA, or a pharmaceutically acceptable salt and/or hydrate and/or cocrystal thereof; e.g., a compound of Formula AA) and one or more (e.g., all) of the following excipients:
**(a)** One or more (e.g., one) binders (e.g., a polyvinyl polymer, such as polyvinylpyrrolidone (povidone);
**(b)** One or more (e.g., one or two, e.g., two) glidants and/or lubricants, such as magnesium stearate and/or talc;
**(c)** One or more (e.g., one or two; e.g., one) disintegrants, such as crospovidone; and
**(d)** One or more (e.g., one or two; e.g., one) diluents, such as lactose (e.g., lactose monohydrate).

In certain embodiments, component **(i)** includes from about 40 weight percent to about 80 weight percent (e.g., from about 50 weight percent to about 70 weight percent, from about 55 weight percent to about 70 weight percent; from about 60 weight percent to about 65 weight percent; e.g., about 62.1 weight percent) of the chemical entity (e.g., a compound of Formula AA, or a pharmaceutically acceptable salt and/or hydrate and/or cocrystal thereof).

In certain embodiments, component **(i)** includes from about 0.5 weight percent to about 5 weight percent (e.g., from about 1.5 weight percent to about 4.5 weight percent, from about 2 weight percent to about 3.5 weight percent; e.g., about 2.76 weight percent) of the binder (e.g., povidone).

In certain embodiments, component **(i)** includes from about 0.5 weight percent to about 5 weight percent (e.g., from about 0.5 weight percent to about 3 weight percent, from about 1 weight percent to about 3 weight percent; about 2 weight percent e.g., about 1.9 weight percent) of the disintegrant (e.g., crospovidone).

In certain embodiments, component **(i)** includes from about 10 weight percent to about 50 weight percent (e.g., from about 20 weight percent to about 40 weight percent, from about 25 weight percent to about 35 weight percent; e.g., about 31.03 weight percent) of the diluent (e.g., lactose, e.g., lactose monohydrate).

In certain embodiments, component **(i)** includes from about 0.05 weight percent to about 5 weight percent (e.g., from about 0.05 weight percent to about 3 weight percent) of the glidants and/or lubricants.

In certain embodiments (e.g., when component (i) includes one or more lubricants, such as magnesium stearate), component **(i)** includes from about 0.05 weight percent to about 1 weight percent (e.g., from about 0.05 weight percent to about 1 weight percent; from about 0.1 weight percent to about 1 weight percent; from about 0.1 weight percent to about 0.5 weight percent; e.g., about 0.27 weight percent) of the lubricant (e.g., magnesium stearate).

In certain embodiments (when component **(i)** includes one or more lubricants, such as talc), component (i) includesfrom about 0.5 weight percent to about 5 weight percent (e.g., from about 0.5 weight percent to about 3 weight percent, from about 1 weight percent to about 3 weight percent; from about 1.5 weight percent to about 2.5 weight percent; from about 1.8 weight percent to about 2.2 weight percent; about 1.93 weight percent) of the lubricant (e.g., talc).

In certain of these embodiments, each of **(a), (b), (c),** and **(d)** above is present.

In certain embodiments, component **(i)** includes the ingredients and amounts as shown in Table A.

**Table A**

| Ingredient | Weight Percent |
|---|---|
| A compound of Formula AA | 40 weight percent to about 80 weight percent (e.g., from about 50 weight percent to about 70 weight percent, from about 55 weight percent to about 70 weight percent; from about 60 weight percent to about 65 weight percent; e.g., about 62.1 weight percent) |
| Crospovidone (Kollidon CL) | 0.5 weight percent to about 5 weight percent (e.g., from about 0.5 weight percent to about 3 weight percent, from about 1 weight percent to about 3 weight percent; about 1.93 weight percent |
| lactose monohydrate (Pharmatose 200M) | about 10 weight percent to about 50 weight percent (e.g., from about 20 weight percent to about 40 weight percent, from about 25 |
| | weight percent to about 35 weight percent; e.g., about 31.03 weight percent |
| Povidone (Kollidon K30) | about 0.5 weight percent to about 5 weight percent (e.g., from about 1.5 weight percent to about 4.5 weight percent, from about 2 weight percent to about 3.5 weight percent; e.g., about 2.76 weight percent |
| talc | 0.5 weight percent to about 5 weight percent (e.g., from about 0.5 weight percent to about 3 weight percent, from about 1 weight percent to about 3 weight percent; from about 1.5 weight percent to about 2.5 weight percent; from about 1.8 weight percent to about 2.2 weight percent;e.g., about 1.93 weight percent |
| Magnesium stearate | about 0.05 weight percent to about 1 weight percent (e.g., from about 0.05 weight percent to about 1 weight percent; from about 0.1 weight percent to about 1 weight percent; from about 0.1 weight percent to about 0.5 weight percent; e.g., about 0.27 weight percent |

In certain embodiments, component **(i)** includes the ingredients and amounts as shown in Table B.

**Table B**

| Ingredient | Weight Percent |
|---|---|
| A compound of Formula AA | About 62.1 weight percent) |
| Crospovidone (Kollidon CL) | About 1.93 weight percent |
| lactose monohydrate (Pharmatose 200M) | About 31.03 weight percent |
| Povidone (Kollidon K30) | About 2.76 weight percent |
| talc | About 1.93 weight percent |
| Magnesium stearate | About 0.27 weight percent |

In certain embodiments, component **(i)** is formulated as a wet granulated solid preparation. In certain of these embodiments an internal phase of ingredients (the chemical entity, disintegrant, and diluent) are combined and mixed in a high-shear granulator. A binder (e.g., povidone) is dissolved in water to form a granulating solution. This solution is added to the Inner Phase mixture resulting in the development of granules. While not wishing to be bound by theory, granule development is believed to be facilitated by the interaction of the polymeric binder with the materials of the internal phase. Once the granulation is formed and dried, an external phase (e.g., one or more lubricants - not an intrinsic component of the dried granulation), is added to the dry granulation. It is believed that lubrication of the granulation is important to the flowability of the granulation, in particular for packaging.

In certain of the foregoing embodiments, component (ii) includes water and one or more (e.g., all) of the following excipients:
**(a')** One or more (e.g., one, two; e.g., two) thickeners, viscosity enhancing agents, binders, and/or mucoadhesive agents (e.g., cellulose or cellulose esters or ethers or derivatives or salts thereof (e.g., methyl cellulose); and polyvinyl polymers such as polyvinylpyrrolidone (povidone);
**(b')** One or more (e.g., one or two; e.g., two) preservatives, such as a paraben, e.g., methyl 4-hydroxybenzoate (methylparaben), or a pharmaceutically acceptable salt or ester thereof, propyl 4-hydroxybenzoate (propylparaben), or a pharmaceutically acceptable salt or ester thereof, or a combination thereof; and
**(c')** One or more (e.g., one or two; e.g., two) buffers, such as phosphate buffer system (e.g., sodium dihydrogen phospahate dihydrate, disodium phosphate dodecahydrate);

In certain of the foregoing embodiments, component **(ii)** includes water and one or more (e.g., all) of the following excipients:
**(a")** a first thickener, viscosity enhancing agent, binder, and/or mucoadhesive agent (e.g., a cellulose or cellulose ester or ether or derivative or salt thereof (e.g., methyl cellulose));
**(a‴)** a second thickener, viscosity enhancing agent, binder, and/or mucoadhesive agent (e.g., a polyvinyl polymer, such as polyvinylpyrrolidone (povidone));
**(b")** a first preservative, such as a paraben, e.g., propyl 4-hydroxybenzoate (propylparaben), or a pharmaceutically acceptable salt or ester thereof;
**(b")** a second preservative, such as a paraben, e.g., methyl 4-hydroxybenzoate (methylparaben), or a pharmaceutically acceptable salt or ester thereof,
**(c")** a first buffer, such as phosphate buffer system (e.g., disodium phosphate dodecahydrate);
(**c**‴) a second buffer, such as phosphate buffer system (e.g., sodium dihydrogen phospahate dehydrate),

In certain embodiments, component **(ii)** includes from about 0.05 weight percent to about 5 weight percent (e.g., from about 0.05 weight percent to about 3 weight percent, from about 0.1 weight percent to about 3 weight percent; e.g., about 1.4 weight percent) of **(a").**

In certain embodiments, component **(ii)** includes from about 0.05 weight percent to about 5 weight percent (e.g., from about 0.05 weight percent to about 3 weight percent, from about 0.1 weight percent to about 2 weight percent; e.g., about 1.0 weight percent) of **(a‴).**

In certain embodiments, component **(ii)** includes from about 0.005 weight percent to about 0.1 weight percent (e.g., from about 0.005 weight percent to about 0.05 weight percent; e.g., about 0.02 weight percent) of **(b").**

In certain embodiments, component **(ii)** includes from about 0.05 weight percent to about 1 weight percent (e.g., from about 0.05 weight percent to about 0.5 weight percent; e.g., about 0.20 weight percent) of **(b‴).**

In certain embodiments, component **(ii)** includes from about 0.05 weight percent to about 1 weight percent (e.g., from about 0.05 weight percent to about 0.5 weight percent; e.g., about 0.15 weight percent) of **(c").**

In certain embodiments, component **(ii)** includes from about 0.005 weight percent to about 0.5 weight percent (e.g., from about 0.005 weight percent to about 0.3 weight percent; e.g., about 0.15 weight percent) of **(c‴).**

In certain of these embodiments, each of **(a")** - **(c‴)** is present.

In certain embodiments, component (ii) includes water (up to 100%) and the ingredients and amounts as shown in Table C.

**Table C**

| Ingredient | Weight Percent |
|---|---|
| methyl cellulose (Methocel A15C premium) | 0.05 weight percent to about 5 weight percent (e.g., from about 0.05 weight percent to about 3 weight percent, from about 0.1 weight percent to about 3 weight percent; e.g., about 1.4 weight percent |
| Povidone (Kollidon K30) | 0.05 weight percent to about 5 weight percent (e.g., from about 0.05 weight percent to about 3 weight percent, from about 0.1 weight percent to about 2 weight percent; e.g., about 1.0 weight percent |
| propyl 4-hydroxybenzoate | about 0.005 weight percent to about 0.1 weight percent (e.g., from about 0.005 weight percent to about 0.05 weight percent; e.g., about 0.02 weight percent) |
| methyl 4-hydroxybenzoate | about 0.05 weight percent to about 1 weight percent (e.g., from about 0.05 weight percent to about 0.5 weight percent; e.g., about 0.20 weight percent) |
| disodium phosphate dodecahydrate | about 0.05 weight percent to about 1 weight percent (e.g., from about 0.05 weight percent to about 0.5 weight percent; e.g., about 0.15 weight percent) |
| sodium dihydrogen phospahate dihydrate | about 0.005 weight percent to about 0.5 weight percent (e.g., from about 0.005 weight percent to about 0.3 weight percent; e.g., about 0.15 weight percent) |

In certain embodiments, component **(ii)** includes water (up to 100%) and the ingredients and amounts as shown in Table D.

**Table D**

| Ingredient | Weight Percent |
|---|---|
| methyl cellulose (Methocel A15C premium) | about 1.4 weight percent |
| Povidone (Kollidon K30) | about 1.0 weight percent |
| propyl 4-hydroxybenzoate | about 0.02 weight percent |
| methyl 4-hydroxybenzoate | about 0.20 weight percent |
| disodium phosphate dodecahydrate | about 0.15 weight percent |
| sodium dihydrogen phospahate dihydrate | about 0.15 weight percent |

Ready-to-use" enemas are generally be provided in a "single-use" sealed disposable container of plastic or glass. Those formed of a polymeric material preferably have sufficient flexibility for ease of use by an unassisted patient. Typical plastic containers can be made of polyethylene. These containers may comprise a tip for direct introduction into the rectum. Such containers may also comprise a tube between the container and the tip. The tip is preferably provided with a protective shield which is removed before use. Optionally the tip has a lubricant to improve patient compliance.

In some embodiments, the enema formulation (e.g., suspension) is poured into a bottle for delivery after it has been prepared in a separate container. In certain embodiments, the bottle is a plastic bottle (e.g., flexible to allow for delivery by squeezing the bottle), which can be a polyethylene bottle (e.g., white in color). In some embodiments, the bottle is a single chamber bottle, which contains the suspension or solution. In other embodiments, the bottle is a multichamber bottle, where each chamber contains a separate mixture or solution. In still other embodiments, the bottle can further include a tip or rectal cannula for direct introduction into the rectum.

### Dosages

The dosages may be varied depending on the requirement of the patient, the severity of the condition being treating and the particular compound being employed. Determination of the proper dosage for a particular situation can be determined by one skilled in the medical arts. The total daily dosage may be divided and administered in portions throughout the day or by means providing continuous delivery.

In some embodiments, the compounds described herein are administered at a dosage of from about 0.001 mg/Kg to about 500 mg/Kg (e.g., from about 0.001 mg/Kg to about 200 mg/Kg; from about 0.01 mg/Kg to about 200 mg/Kg; from about 0.01 mg/Kg to about 150 mg/Kg; from about 0.01 mg/Kg to about 100 mg/Kg; from about 0.01 mg/Kg to about 50 mg/Kg; from about 0.01 mg/Kg to about 10 mg/Kg; from about 0.01 mg/Kg to about 5 mg/Kg; from about 0.01 mg/Kg to about 1 mg/Kg; from about 0.01 mg/Kg to about 0.5 mg/Kg; from about 0.01 mg/Kg to about 0.1 mg/Kg; from about 0. 1 mg/Kg to about 200 mg/Kg; from about 0. 1 mg/Kg to about 150 mg/Kg; from about 0. 1 mg/Kg to about 100 mg/Kg; from about 0.1 mg/Kg to about 50 mg/Kg; from about 0. 1 mg/Kg to about 10 mg/Kg; from about 0. 1 mg/Kg to about 5 mg/Kg; from about 0. 1 mg/Kg to about 1 mg/Kg; from about 0. 1 mg/Kg to about 0.5 mg/Kg).

In some embodiments, enema formulations include from about 0.5 mg to about 2500 mg (e.g., from about 0.5 mg to about 2000 mg, from about 0.5 mg to about 1000 mg, from about 0.5 mg to about 750 mg, from about 0.5 mg to about 600 mg, from about 0.5 mg to about 500 mg, from about 0.5 mg to about 400 mg, from about 0.5 mg to about 300 mg, from about 0.5 mg to about 200 mg; e.g., from about 5 mg to about 2500 mg, from about 5 mg to about 2000 mg, from about 5 mg to about 1000 mg; from about 5 mg to about 750 mg; from about 5 mg to about 600 mg; from about 5 mg to about 500 mg; from about 5 mg to about 400 mg; from about 5 mg to about 300 mg; from about 5 mg to about 200 mg; e.g., from about 50 mg to about 2000 mg, from about 50 mg to about 1000 mg, from about 50 mg to about 750 mg, from about 50 mg to about 600 mg, from about 50 mg to about 500 mg, from about 50 mg to about 400 mg, from about 50 mg to about 300 mg, from about 50 mg to about 200 mg; e.g., from about 100 mg to about 2500 mg, from about 100 mg to about 2000 mg, from about 100 mg to about 1000 mg, from about 100 mg to about 750 mg, from about 100 mg to about 700 mg, from about 100 mg to about 600 mg, from about 100 mg to about 500 mg, from about 100 mg to about 400 mg, from about 100 mg to about 300 mg, from about 100 mg to about 200 mg; e.g., from about 150 mg to about 2500 mg, from about 150 mg to about 2000 mg, from about 150 mg to about 1000 mg, from about 150 mg to about 750 mg, from about 150 mg to about 700 mg, from about 150 mg to about 600 mg, from about 150 mg to about 500 mg, from about 150 mg to about 400 mg, from about 150 mg to about 300 mg, from about 150 mg to about 200 mg; e.g., from about 150 mg to about 500 mg; e.g., from about 300 mg to about 2500 mg, from about 300 mg to about 2000 mg, from about 300 mg to about 1000 mg, from about 300 mg to about 750 mg, from about 300 mg to about 700 mg, from about 300 mg to about 600 mg; e.g., from about 400 mg to about 2500 mg, from about 400 mg to about 2000 mg, from about 400 mg to about 1000 mg, from about 400 mg to about 750 mg, from about 400 mg to about 700 mg, from about 400 mg to about 600 from about 400 mg to about 500 mg; e.g., 150 mg or 450 mg) of the chemical entity in from about 1 mL to about 3000 mL (e.g., from about 1 mL to about 2000 mL, from about 1 mL to about 1000 mL, from about 1 mL to about 500 mL, from about 1 mL to about 250 mL, from about 1 mL to about 100 mL, from about 10 mL to about 1000 mL, from about 10 mL to about 500 mL, from about 10 mL to about 250 mL, from about 10 mL to about 100 mL, from about 30 mL to about 90 mL, from about 40 mL to about 80 mL; from about 50 mL to about 70 mL; e.g., about 1 mL, about 5 mL, about 10 mL, about 15 mL, about 20 mL, about 25 mL, about 30 mL, about 35 mL, about 40 mL, about 45 mL,about 50 mL, about 55 mL, about 60 mL, about 65 mL, about 70 mL, about 75 mL, about 100 mL, about 250 mL, or about 500 mL, or about 1000 mL, or about 2000mL, or about 3000 mL; e.g., 60 mL) of liquid carrier.

In certain embodiments, enema formulations include from about 50 mg to about 250 mg (e.g., from about 100 mg to about 200; e.g., about 150 mg) of the chemical entity in from about 10 mL to about 100 mL (e.g., from about 20 mL to about 100 mL, from about 30 mL to about 90 mL, from about 40 mL to about 80 mL; from about 50 mL to about 70 mL) of liquid carrier. In certain embodiments, enema formulations include about 150 mg of the chemical entity in about 60 mL of the liquid carrier. In certain of these embodiments, the chemical entity is a compound of Formula AA, or a pharmaceutically acceptable salt and/or hydrate and/or cocrystal thereof. For example, enema formulations can include about 150 mg of a compound of Formula AA in about 60 mL of the liquid carrier.

In certain embodiments, enema formulations include from about 350 mg to about 550 mg (e.g., from about 400 mg to about 500; e.g., about 450 mg) of the chemical entity in from about 10 mL to about 100 mL (e.g., from about 20 mL to about 100 mL, from about 30 mL to about 90 mL, from about 40 mL to about 80 mL; from about 50 mL to about 70 mL) of liquid carrier. In certain embodiments, enema formulations include about 450 mg of the chemical entity in about 60 mL of the liquid carrier. In certain of these embodiments, the chemical entity is a compound of Formula AA, or a pharmaceutically acceptable salt and/or hydrate and/or cocrystal thereof. For example, enema formulations can include about 450 mg of a compound of Formula AA in about 60 mL of the liquid carrier.

In some embodiments, enema formulations include from about from about 0.01 mg/mL to about 50 mg/mL (e.g., from about 0.01 mg/mL to about 25 mg/mL; from about 0.01 mg/mL to about 10 mg/mL; from about 0.01 mg/mL to about 5 mg/mL; from about 0.1 mg/mL to about 50 mg/mL; from about 0.01 mg/mL to about 25 mg/mL; from about 0.1 mg/mL to about 10 mg/mL; from about 0.1 mg/mL to about 5 mg/mL; from about 1 mg/mL to about 10 mg/mL; from about 1 mg/mL to about 5 mg/mL; from about 5 mg/mL to about 10 mg/mL; e.g., about 2.5 mg/mL or about 7.5 mg/mL) of the chemical entity in liquid carrier. In certain of these embodiments, the chemical entity is a compound of Formula AA, or a pharmaceutically acceptable salt and/or hydrate and/or cocrystal thereof. For example, enema formulations can include about 2.5 mg/mL or about 7.5 mg/mL of a compound of Formula AA in liquid carrier.

### Regimens

The foregoing dosages can be administered on a daily basis (e.g., as a single dose or as two or more divided doses) or non-daily basis (e.g., every other day, every two days, every three days, once weekly, twice weeks, once every two weeks, once a month).

In some embodiments, the period of administration of a compound described herein is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 1 1 months, 12 months, or more. In a further embodiment, a period of during which administration is stopped is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 1 1 weeks, 12 weeks, 4 months, 5 months, 6 months, 7 months, 8 months, 9 months, 10 months, 1 1 months, 12 months, or more. In an embodiment, a therapeutic compound is administered to an individual for a period of time followed by a separate period of time. In another embodiment, a therapeutic compound is administered for a first period and a second period following the first period, with administration stopped during the second period, followed by a third period where administration of the therapeutic compound is started and then a fourth period following the third period where administration is stopped. In an aspect of this embodiment, the period of administration of a therapeutic compound followed by a period where administration is stopped is repeated for a determined or undetermined period of time. In a further embodiment, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

### Methods of Treatment

In some embodiments, methods for treating a subject having condition, disease or disorder in which a decrease or increase in NLRP3 activity (e.g., an increase, e.g., NLRP3 signaling) contributes to the pathology and/or symptoms and/or progression of the condition, disease or disorder are provided, comprising administering to a subject an effective amount of a chemical entity described herein (e.g., a compound described generically or specifically herein or a pharmaceutically acceptable salt thereof or compositions containing the same).

### Indications

In some embodiments, the condition, disease or disorder is selected from: inappropriate host responses to infectious diseases where active infection exists at any body site, such as septic shock, disseminated intravascular coagulation, and/or adult respiratory distress syndrome; acute or chronic inflammation due to antigen, antibody and/or complement deposition; inflammatory conditions including arthritis, cholangitis, colitis, encephalitis, endocarditis, glomerulonephritis, hepatitis, myocarditis, pancreatitis, pericarditis, reperfusion injury and vasculitis, immune-based diseases such as acute and delayed hypersensitivity, graft rejection, and graft-versus-host disease; auto-immune diseases including Type 1 diabetes mellitus and multiple sclerosis. For example, the condition, disease or disorder may be an inflammatory disorder such as rheumatoid arthritis, osteoarthritis, septic shock, COPD and periodontal disease.

In some embodiments, the condition, disease or disorder is an autoimmune diseases. Nonlimiting examples include rheumatoid arthritis, systemic lupus erythematosus, multiple sclerosis, inflammatory bowel diseases (IBDs) comprising Crohn disease (CD) and ulcerative colitis (UC), which are chronic inflammatory conditions with polygenic susceptibility. In certain embodiments, the condition is an inflammatory bowel disease. In certain embodiments, the condition is Crohn's disease, autoimmune colitis, iatrogenic autoimmune colitis, ulcerative colitis, colitis induced by one or more chemotherapeutic agents, colitis induced by treatment with adoptive cell therapy, colitis associated by one or more alloimmune diseases (such as graft-vs-host disease, e.g., acute graft vs. host disease and chronic graft vs. host disease), radiation enteritis, collagenous colitis, lymphocytic colitis, microscopic colitis, and radiation enteritis. In certain of these embodiments, the condition is alloimmune disease (such as graft-vs-host disease, e.g., acute graft vs. host disease and chronic graft vs. host disease), celiac disease, irritable bowel syndrome, rheumatoid arthritis, lupus, scleroderma, psoriasis, cutaneous T-cell lymphoma, uveitis, and mucositis (e.g., oral mucositis, esophageal mucositis or intestinal mucositis).

In some embodiments, the condition, disease or disorder is selected from major adverse cardiovascular events such as carbiovascular death, non-fatal myocardial infarction and non-fatal stroke in patients with a prior hear attack and inflammatory atherosclerosis (see for example, NCT01327846).

In some embodiments, the condition, disease or disorder is selected from metabolic disorders such as type 2 diabetes, atherosclerosis, obesity and gout, as well as diseases of the central nervous system, such as Alzheimer's disease and multiple sclerosis and Amyotrophic Lateral Sclerosis and Parkinson disease, lung disease, such as asthma and COPD and pulmonary idiopathic fibrosis, liver disease, such as NASH syndrome, viral hepatitis and cirrhosis, pancreatic disease, such as acute and chronic pancreatitis, kidney disease, such as acute and chronic kidney injury, intestinal disease such as Crohn's disease and Ulcerative Colitis, skin disease such as psoriasis, musculoskeletal disease such as scleroderma, vessel disorders, such as giant cell arteritis, disorders of the bones, such as Osteoarthritis , osteoporosis and osteopetrosis disorders eye disease, such as glaucoma and macular degeneration, diseased caused by viral infection such as HIV and AIDS, autoimmune disease such as Rheumatoid Arthritis, Systemic Lupus Erythematosus, Autoimmune Thyroiditis, Addison's disease, pernicious anemia, cancer and aging.

In some embodiments, the condition, disease or disorder is a cardiovascular indication. In some embodiments, the condition, disease or disorder is myocardial infraction. In some embodiments, the condition, disease or disorder is stroke.

In some embodiments, the condition, disease or disorder is obesity.

In some embodiments, the condition, disease or disorder is Type 2 Diabetes.

In some embodiments, the condition, disease or disorder is NASH.

In some embodiments, the condition, disease or disorder is Alzheimer's disease.

In some embodiments, the condition, disease or disorder is gout.

In some embodiments, the condition, disease or disorder is SLE.

In some embodiments, the condition, disease or disorder is rheumatoid arthritis.

In some embodiments, the condition, disease or disorder is IBD.

In some embodiments, the condition, disease or disorder is multiple sclerosis.

In some embodiments, the condition, disease or disorder is COPD.

In some embodiments, the condition, disease or disorder is asthma.

In some embodiments, the condition, disease or disorder is scleroderma.

In some embodiments, the condition, disease or disorder is pulmonary fibrosis.

In some embodiments, the condition, disease or disorder is age related macular degeneration (AMD).

In some embodiments, the condition, disease or disorder is cystic fibrosis.

In some embodiments, the condition, disease or disorder is Muckle Wells syndrome.

In some embodiments, the condition, disease or disorder is familial cold auto inflammatory syndrome (FCAS).

In some embodiments, the condition, disease or disorder is chronic neurologic cutaneous and articular syndrome.

In some embodiments, the condition, disease or disorder is selected from: myelodysplastic syndromes (MDS); non-small cell lung cancer, such as non-small cell lung cancer in patients carrying mutation or overexpression of NLRP3; acute lymphoblastic leukemia (ALL), such as ALL in patients resistant to glucocorticoids treatment; Langerhan's cell histiocytosis (LCH); multiple myeloma; promyelocytic leukemia; acute myeloid leukemia (AML) chronic myeloid leukemia (CML); gastric cancer; and lung cancer metastasis.

In some embodiments, the condition, disease or disorder is selected from: myelodysplastic syndromes (MDS); non-small cell lung cancer, such as non-small cell lung cancer in patients carrying mutation or overexpression of NLRP3; acute lymphoblastic leukemia (ALL), such as ALL in patients resistant to glucocorticoids treatment; Langerhan's cell histiocytosis (LCH); multiple myeloma; promyelocytic leukemia; gastric cancer; and lung cancer metastasis.

In some embodiments, the indication is MDS.

In some embodiments, the indication is non-small lung cancer in patients carrying mutation or overexpression of NLRP3.

In some embodiments, the indication is ALL in patients resistant to glucocorticoids treatment.

In some embodiments, the indication is LCH.

In some embodiments, the indication is multiple myeloma.

In some embodiments, the indication is promyelocytic leukemia.

In some embodiments, the indication is gastric cancer.

In some embodiments, the indication is lung cancer metastasis.

### Combination therapy

This disclosure contemplates both monotherapy regimens as well as combination therapy regimens.

In some embodiments, the methods described herein can further include administering one or more additional therapies (e.g., one or more additional therapeutic agents and/or one or more therapeutic regimens) in combination with administration of the compounds described herein.

In certain embodiments, the second therapeutic agent or regimen is administered to the subject prior to contacting with or administering the chemical entity (e.g., about one hour prior, or about 6 hours prior, or about 12 hours prior, or about 24 hours prior, or about 48 hours prior, or about 1 week prior, or about 1 month prior).

In other embodiments, the second therapeutic agent or regimen is administered to the subject at about the same time as contacting with or administering the chemical entity. By way of example, the second therapeutic agent or regimen and the chemical entity are provided to the subject simultaneously in the same dosage form. As another example, the second therapeutic agent or regimen and the chemical entity are provided to the subject concurrently in separate dosage forms.

In still other embodiments, the second therapeutic agent or regimen is administered to the subject after contacting with or administering the chemical entity (e.g., about one hour after, or about 6 hours after, or about 12 hours after, or about 24 hours after, or about 48 hours after, or about 1 week after, or about 1 month after).

### Patient Selection

In some embodiments, the methods described herein further include the step of identifying a subject (e.g., a patient) in need of treatment for an indication related to NLRP3 activity, such as an indication related to NLRP3 polymorphism.

In some embodiments, the methods described herein further include the step of identifying a subject (e.g., a patient) in need of treatment for an indication related to NLRP3 activity, such as an indication related to NLRP3 where polymorphism is a gain of function

In some embodiments, the methods described herein further include the step of identifying a subject (e.g., a patient) in need of treatment for an indication related to NLRP3 activity, such as an indication related to NLRP3 polymorphism found in CAPS syndromes.

In some embodiments, the methods described herein further include the step of identifying a subject (e.g., a patient) in need of treatment for an indication related to NLRP3 activity, such as an indication related NLRP3 polymorphism where the polymorphism is VAR_014104 (R262W)

In some embodiments, the methods described herein further include the step of identifying a subject (e.g., a patient) in need of treatment for an indication related to NLRP3 activity, such as an indication related NLRP3 polymorphism where the polymorphism is a natural variant reported in http://www.uniprot.org/uniprot/Q96P20.

In some embodiments, the methods described herein further include the step of identifying a subject (e.g., a patient) in need of treatment for an indication related to NLRP3 activity, such as an indication related to point mutation of NLRP3 signaling.

### Compound Preparation and Biological Assays

As can be appreciated by the skilled artisan, methods of synthesizing the compounds of the formulae herein will be evident to those of ordinary skill in the art. Synthetic chemistry transformations and protecting group methodologies (protection and deprotection) useful in synthesizing the compounds described herein are known in the art and include, for example, those such as described in R. Larock, Comprehensive Organic Transformations, VCH Publishers (1989); T. W. Greene and RGM. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

### Preparative Examples

The following abbreviations have the indicated meanings:
ACN = acetonitrile
Ac = acetyl
(BnS)₂ = 1,2-dibenzyldisulfane
BTC = trichloromethyl chloroformate
Boc = *t*-butyloxy carbonyl
Burgess reagent = 1-Methoxy-N-triethylammoniosulfonyl-methanimidate
CDI = N,N'-carbonyldiimidazole
m-CPBA = 3-chloroperbenzoic acid
DAST= diethylaminosulfurtrifluoride
DCM = dichloromethane
DEA = diethylamine
DMAP = N,N-dimethylpyridin-4-amine
DME = 1,2-dimethoxyethane
DMF = *N,N*-dimethylformamide
DMSO = dimethyl sulfoxide
DIEA = *N,N*-diisopropylethylamine
DPPA = diphenylphosphoryl azide
EtOH = ethanol
EtOAc = ethyl acetate
HATU = 2-(3H-[1,2,3]triazolo[4,5-b]pyridin-3-yl)-1,1,3,3-tetramethylisouronium hexafluorophosphate(V)
Hex = hexane
HPLC = high performance liquid chromatography
KHMDS = potassium bis(trimethylsilyl)amide
LC-MS = liquid chromatography - mass spectrometry
LiHMDS = lithium bis(trimethylsilyl)amide
LDA = lithium diisopropylamide
Me = methyl
MeOH = methanol
MSA = methanesulfonic acid
Mts-*S, R, R*-Aux = (2R,3aS,8aR)-3-(mesitylsulfonyl)-3,3a,8,8a-tetrahydroindeno[1,2-d][1,2,3]oxathiazole 2-oxide
NBS = *N*-bromosuccinimide
NCS = *N*-chlorosuccinimide
NMP = 1-methylpyrrolidin-2-one
NMR = nuclear magnetic resonance
Pd(dppf)Cl₂ = dichloro[1,1'-bis(diphenylphosphino)ferrocene]palladium
Pd(dtbpf)Cl₂ = [1,1'-Bis(di-tert-butylphosphino)ferrocene]dichloropalladium(II)
PE = petroleum ether
Ph = phenyl
PPh₃Cl₂ = dichlorotriphenylphosphorane
Py = pyridine
RT = room temperature
Rt = retention time
RuPhos = 2-dicyclohexylphosphino-2',6'-di-i-propoxy-1,1'-biphenyl
Sat. = saturated
SEM-Cl = (2-(chloromethoxy)ethyl)trimethylsilane
TBAF = tetrabutylammonium fluoride
TBS = tert-butyldimethylsilyl
TBSCl = tert-butyldimethylsilyl chloride
TBDPSCl = tert-butyldiphenylsilyl chloride
t-BuOK = potassium 2-methylpropan-2-olate
t-BuOLi = lithium 2-methylpropan-2-olate
TCCA = trichloroisocyanuric acid
TEA = triethylamine
TFA = trifluoroacetic acid
THF = tetrahydrofuran
TLC = thin layer chromatography
TMAD = (E)-N1,N1,N2,N2-tetramethyldiazene-1,2-dicarboxamide
TsOH = 4-methylbenzenesulfonic acid
UV = ultraviolet
Xphos Pd G₂ = chloro(2-dicyclohexylphosphino-2',4',6'-triisopropyl-1,1'-biphenyl)[2-(2'-amino-1,1'-biphenyl)]palladium(II)
DMA = dimethylacetamide
Davephos = 2-Dicyclohexylphosphino-2'-(N,N-dimethylamino)biphenyl KHMDS = potassium bis(trimethylsilyl)amide
KF = Kart Fischer
NMO = 4-Methylmorpholine N-oxide
Pd(dtbpf)Cl₂ = 1,1'-Bis(di-t-butylphosphino)ferrocene palladium dichloride
Sphos = 2-dicyclohexylphosphino-2',6'-dimethoxybiphenyl
TLC = thin-layer chromatography
T3P = 2,4,6-tripropyl-1,3,5,2,4,6-trioxatriphosphinane 2,4,6-trioxide

### General

The progress of reactions was often monitored by TLC or LC-MS. The identity of the products was often confirmed by LC-MS. The LC-MS was recorded using one of the following methods.

Method A: Shim-pack XR-ODS, C18, 3x50 mm, 2.5 um column, 1.0 uL injection, 1.5 mL/min flow rate, 90-900 amu scan range, 190-400 nm UV range, 5-100% (1.1 min), 100% (0.6 min) gradient with ACN (0.05% TFA) and water (0.05% TFA), 2-minute total run time.

Method B: Kinetex EVO, C18, 3x50 mm, 2.2 um column, 1.0 uL injection, 1.5 mL/min flow rate, 90-900 amu scan range, 190-400 nm UV range, 10-95% (1.1 min), 95% (0.6 min) gradient with ACN and water (0.5% NH₄HCO₃ ), 2 minute total run time.

Method C: Shim-pack XR-ODS, C18, 3x50 mm, 2.5 um column, 1.0 uL injection, 1.5 mL/min flow rate, 90-900 amu scan range, 190-400 nm UV range, 5-100% (2.1 min), 100% (0.6 min) gradient with ACN (0.05% TFA) and water (0.05% TFA), 3 minute total run time.

Method D: Kinetex EVO, C18, 3x50 mm, 2.2 um column, 1.0 uL injection, 1.5 mL/min flow rate, 90-900 amu scan range, 190-400 nm UV range, 10-95% (2.1 min), 95% (0.6 min) gradient with ACN and water (0.5% NH₄HCO₃), 3 minute total run time.

Method F: Phenomenex, CH0-7644, Onyx Monolithic C18, 50 x 4.6 mm, 10.0 uL injection, 1.5 mL/min flow rate, 100-1500 amu scan range, 220 and 254 nm UV detection, 5% with ACN (0.1% TFA) to 100% water (0.1% TFA) over 9.5 min, with a stay at 100% (ACN, 0.1% TFA) for 1 min, then equilibration to 5% (ACN, 0.1% TFA) over 1.5 min.

The final targets were purified by Prep-HPLC. The Prep-HPLC was carried out using the following method.

Method E: Prep-HPLC: Column, XBridge Shield RP18 OBD (19x250 mm, 10 um); mobile phase, Water (10 mM NH₄HCO₃) and ACN, UV detection 254/210 nm.

Method G: Prep-HPLC: Higgins Analytical Proto 200, C18 Column, 250 x 20 mm, 10 um; mobile phase, Water (0.1% TFA) and ACN (0.1% TFA), UV detection 254/210 nm.

NMR was recorded on BRUKER NMR 300.03 MHz, DUL-C-H, ULTRASHIELD^{™}300, AVANCE II 300 B-ACS^{™}120 or BRUKER NMR 400.13 MHz, BBFO, ULTRASHIELD^{™}400, AVANCE III 400, B-ACS^{™}120
**Scheme for final targets:** Schemes 1-7 below illustrate several conditions used for coupling of sulfonimidamide and isocyanate to afford aminocarbonyl sulfonimidamide.

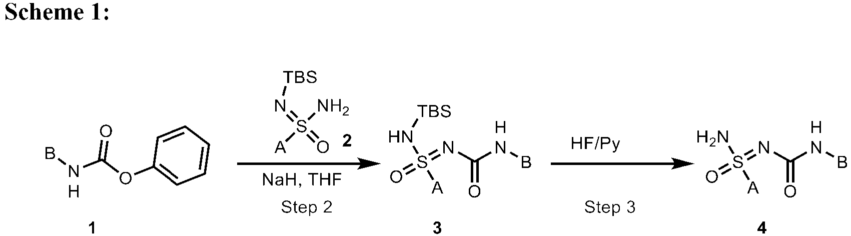

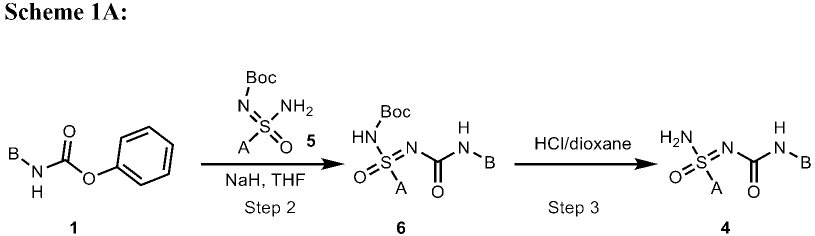

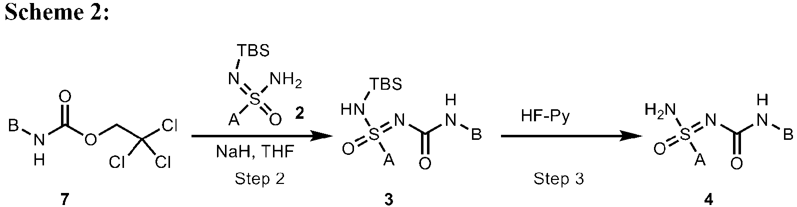

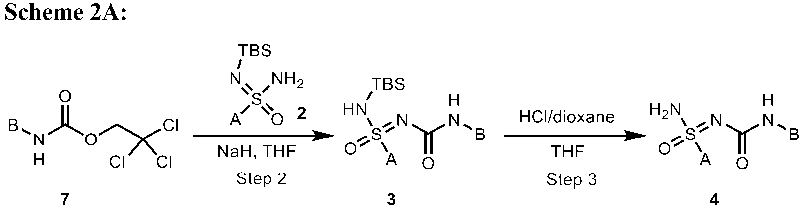

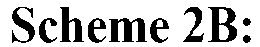

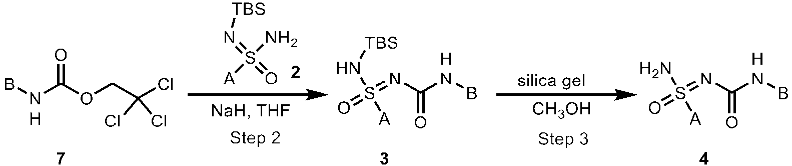

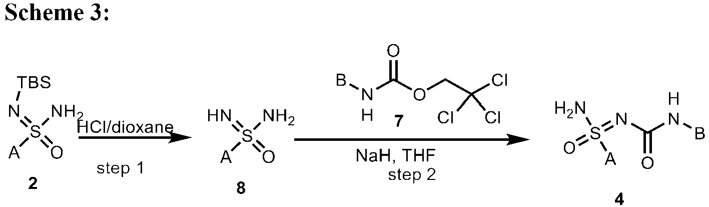

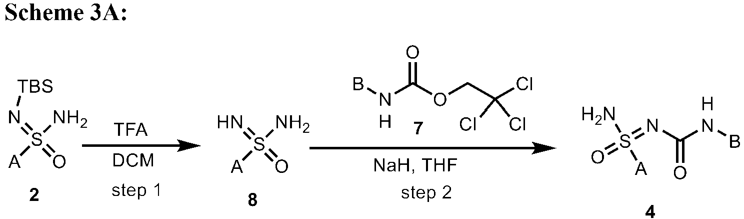

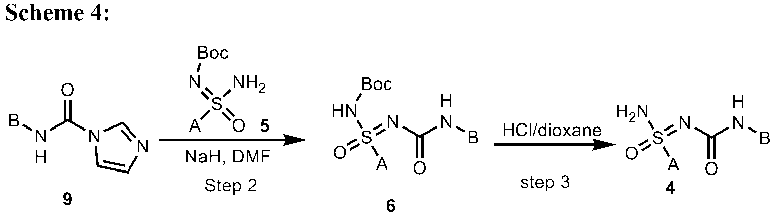

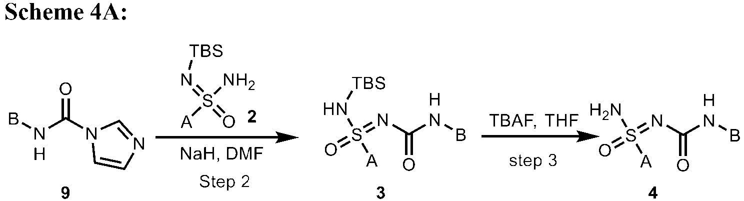

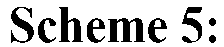

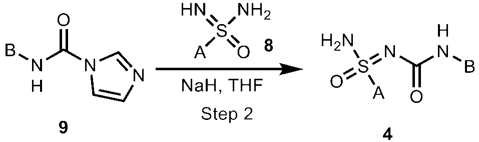

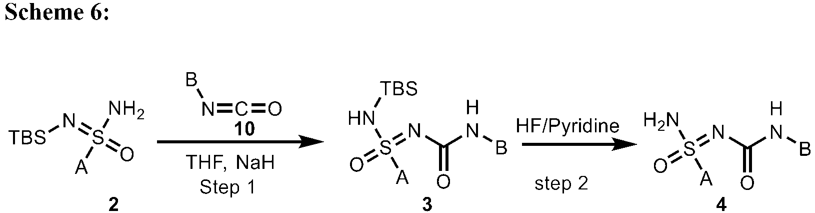

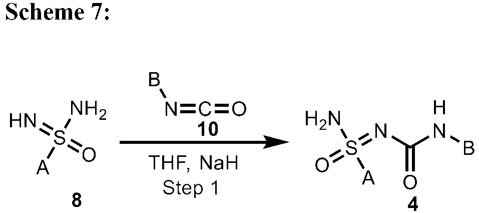

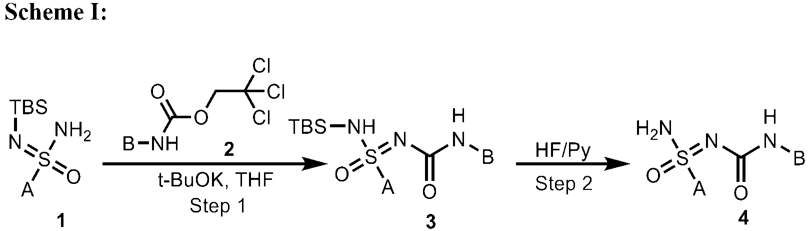

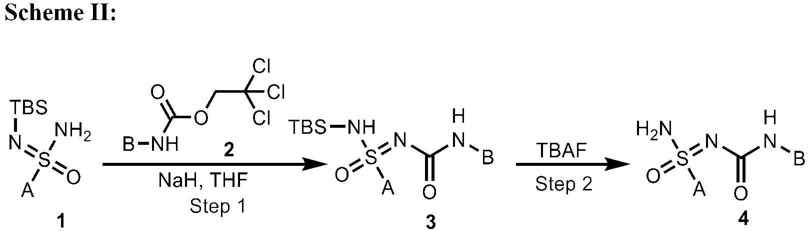

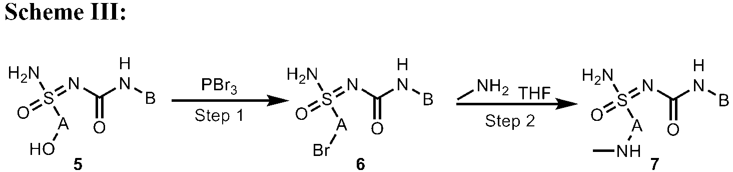

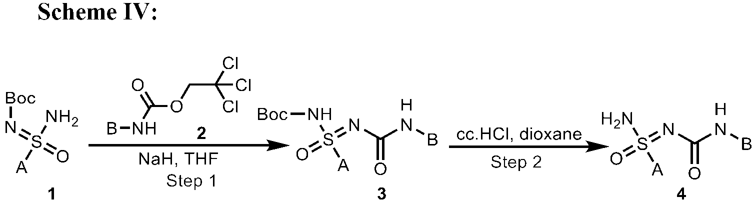

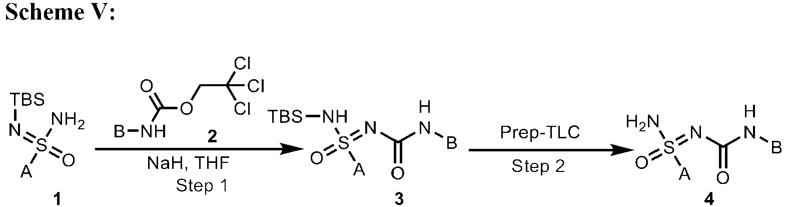

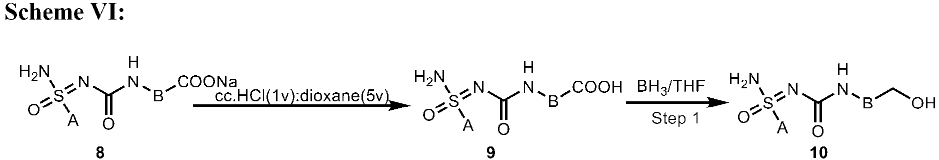

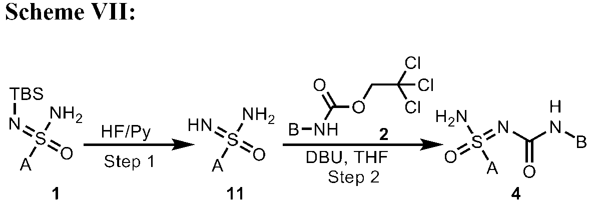

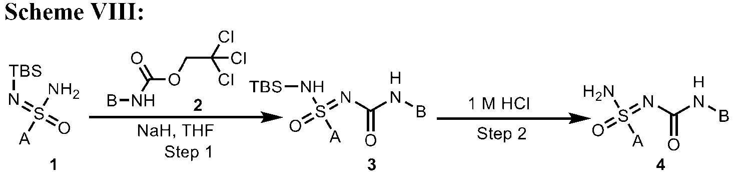

**Scheme for final targets:** Schemes IX-XVI below illustrate several conditions used for coupling of sulfonimidamide and isocyanate to afford aminocarbonyl sulfonimidamide.

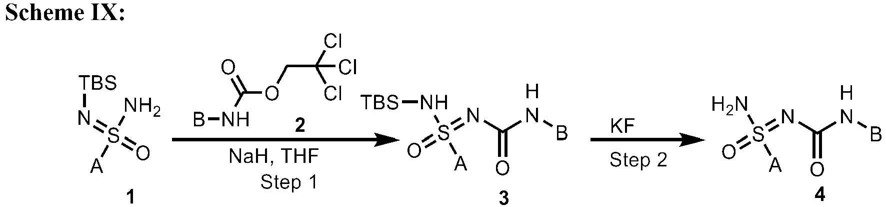

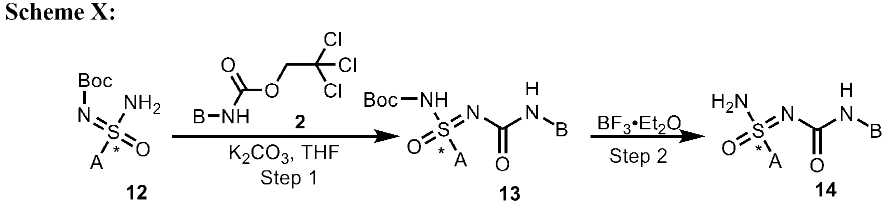

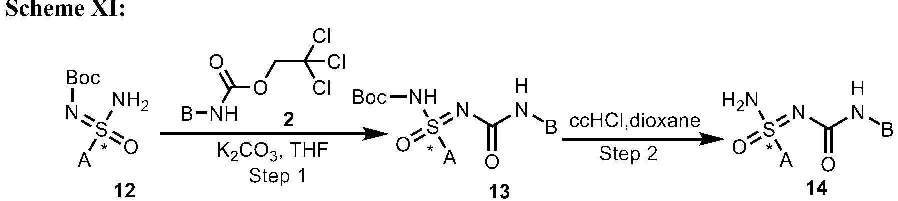

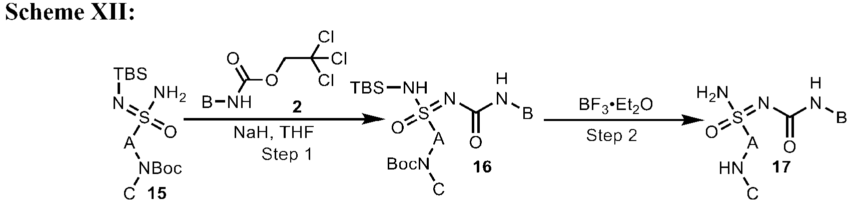

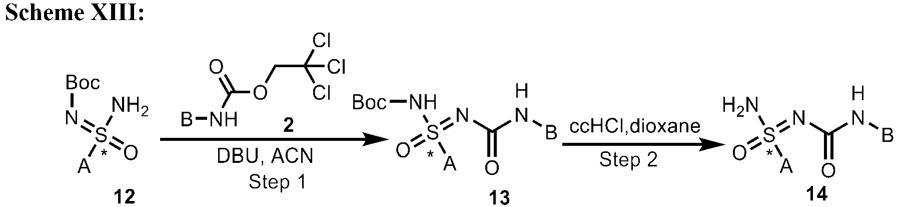

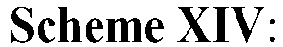

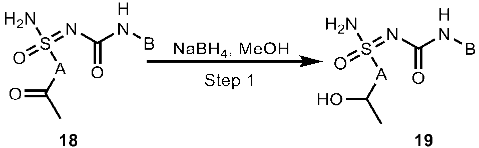

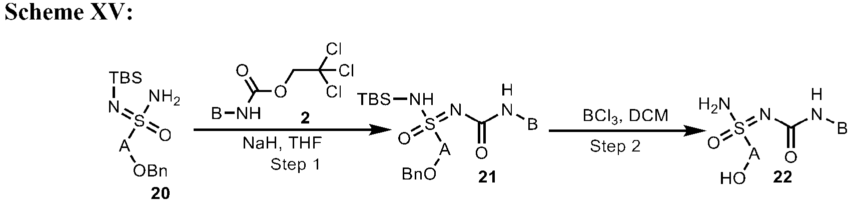

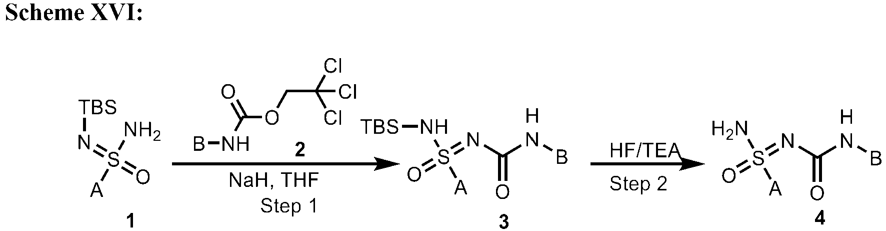

**Scheme for the preparation of Sulfonamides Intermediates:** Schemes **8A-28** below illustrate the preparation of sulfonamide intermediates.

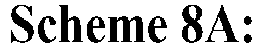

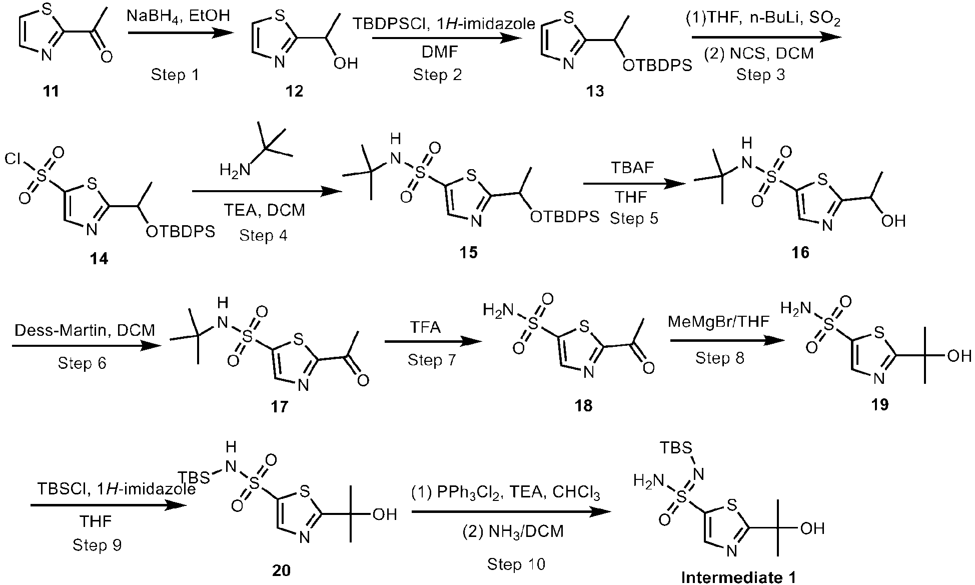

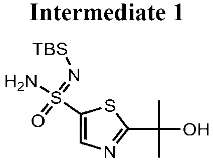

### N'-(tert-butyldimethylsilyl)-2-(2-hydroxypropan-2-yl)thiazole-5-sulfonimidamide

### Step 1: 1-(Thiazol-2-yl)ethanol

Into a 500-mL round-bottom flask was placed 1-(thiazol-2-yl)ethanone (20 g, 157 mmol) in EtOH (200 mL). This was followed by the addition of NaBH₄ (3.0 g, 81.3 mmol) in portions at 0°C. The resulting solution was stirred for 2 h at RT and then was quenched by the addition of 10 mL of NH₄Cl (sat.). The resulting solution was diluted with 200 mL of water and extracted with 2x200 mL of DCM. The organic layers were combined, dried over anhydrous Na₂SO₄, and then concentrated under vacuum. This resulted in 20 g (98%) of the title compound as light yellow oil. MS-ESI: 130 (M+1).

### Step 2: 2-(1-(Tert-butyldiphenylsilyloxy)ethyl)thiazole

Into a 500-mL round-bottom flask was placed 1-(thiazol-2-yl)ethanol (20 g, 155 mmol) in DMF (150 mL). To the stirred solution was added 1*H*-imidazole (20.5 g, 301 mmol). This was followed by the addition of TBDPSCl (46 g, 167 mmol) dropwise with stirring at 0°C· The resulting solution was stirred for 2 h at RT and then was diluted with 300 mL of water. The resulting solution was extracted with 3x200 mL of DCM. The organic layers were combined and concentrated under vacuum. The residue was eluted from silica gel with a gradient of EtOAc/PE (1:100 to 1:80). This resulted in 55 g (97%) of the title compound as colorless oil. MS-ESI: 368.1 (M+1).

### Step 3: 2-(1-(Tert-butyldiphenylsilyloxy)ethyl)thiazole-5-sulfonyl chloride

Into a 500-mL 3-necked round-bottom flask purged with and maintained under nitrogen was placed a solution of 2-(1-(*tert*-butyldiphenylsilyloxy)ethyl)thiazole (30 g, 81.6 mmol) in THF (200 mL). This was followed by the addition of n-BuLi (2.5 M in THF, 35.2 mL) dropwise with stirring at -78°C. The resulting solution was stirred for 0.5 h at -78°C, and then SO₂ was introduced into the above reaction mixture. The reaction was slowly warmed to RT, and then NCS (12.8 g, 95.9 mmol) was added. The resulting solution was stirred for 1 h at RT. The solids were filtered out. The resulting filtrate was concentrated under vacuum. This resulted in 30 g (crude, 79%) of the title compound as brown oil. The crude product was used in the next step.

### Step 4: N-tert-butyl-2-(1-(tert-butyldiphenylsilyloxy)ethyl)thiazole-5-sulfonamide

Into a 500-mL round-bottom flask was placed 2-(1-(tert-butyldiphenylsilyloxy)ethyl)thiazole-5-sulfonyl chloride (crude, 30 g, 64.4 mmol) in DCM (200 mL). To the stirred solution was added TEA (13 g, 128 mmol) dropwise. This was followed by the addition of 2-methylpropan-2-amine (5.6 g, 76.6 mmol) dropwise with stirring at 0°C. The resulting solution was stirred for 2 h at RT and then was concentrated under vacuum. The residue was eluted from silica gel with a gradient of EtOAc/PE (1:30 to 1:20). This resulted in 25 g (61% over two steps) of the title compound as brown oil. MS-ESI: 503.2 (M+1).

### Step 5: N-tert-butyl-2-(1-hydroxyethyl)thiazole-5-sulfonamide

Into a 500-mL round-bottom flask was placed N-*tert*-butyl-2-(1-(*tert-*butyldiphenylsilyloxy)ethyl)thiazole- 5-sulfonamide (25 g, 49.7 mmol) in THF (200 mL). To this stirred solution was added TBAF (30 g, 99.7 mmol) in portions. The resulting solution was stirred for 2 h at RT and then diluted with 200 mL of water. The resulting solution was extracted with 3x200 mL of DCM. The organic layers were combined and concentrated under vacuum. The residue was eluted from a silica gel with a gradient of EtOAc/PE (1:20 to 1:10). This resulted in 12 g (91%) of the title compound as light yellow oil. MS-ESI: 265.1 (M+1).

### Step 6: 2-Acetyl-N-tert-butylthiazole-5-sulfonamide

Into a 500-mL round-bottom flask was placed N-*tert*-butyl-2-(1-hydroxyethyl)thiazole-5-sulfonamide (12 g, 45.4 mmol) in DCM (200 mL). To this solution was added Dess-Martin reagent (20 g, 47.2 mmol) in portions with stirring at RT. The resulting solution was stirred for 2 h at RT and then concentrated under vacuum. The residue was eluted from silica gel with a gradient of EtOAc/PE (1:20 to 1:10). This resulted in 9.0 g (76%) of the title compound as a light yellow solid. MS-ESI: 263.0 (M+1).

### Step 7: 2-Acetylthiazole-5-sulfonamide

Into a 100-mL round-bottom flask was placed 2-acetyl-N-*tert*-butylthiazole-5-sulfonamide (7.0 g, 26.7 mmol) in TFA (20 mL). The resulting solution was stirred for 14 h at 70°C and then concentrated under vacuum. The residue was eluted from silica gel with a gradient of EtOAc/PE (1:5 to 1:3). This resulted in 5.0 g (91%) of the title compound as a yellow solid. MS-ESI: 207.0 (M+1).

### Step 8: 2-(2-Hydroxypropan-2-yl)thiazole-5-sulfonamide

Into a 500-mL 3-necked round-bottom flask purged with and maintained under nitrogen was placed 2-acetylthiazole-5-sulfonamide (5.0 g, 24.3 mmol) in THF (100 mL). This was followed by the addition of MeMgBr (3 M in THF, 8.1 mL, 24.3 mmol) dropwise with stirring at 0°C. The resulting solution was stirred for 14 h at RT and then was quenched by the addition of 100 mL of NH₄Cl (sat.). The resulting solution was extracted with 2x150 mL of DCM. The organic layers were combined and concentrated under vacuum. The residue was eluted from silica gel with a gradient of EtOAc/PE (1:5 to 1:3). This resulted in 2.9 g (54%) of the title compound as a light yellow solid. MS-ESI: 223.0 (M+1).

### Step 9: N-(tert-butyldimethylsilyl)-2-(2-hydroxypropan-2-yl)thiazole-5-sulfonamide

Into a 250-mL round-bottom flask purged with and maintained under nitrogen was placed 2-(2-hydroxypropan-2-yl)thiazole-5-sulfonamide (1.0 g, 4.5 mmol) in DCM (100 mL). To the stirred solution was added 1*H*-imidazole (613 mg, 9.01 mmol) and TBSCl (3.4 g, 22.5 mmol). The resulting solution was stirred for 14 h at RT and then diluted with 100 mL of water. The resulting mixture was extracted with 3x50 mL of DCM and the organic layers were combined and concentrated under vacuum. The residue was eluted from silica gel with a gradient of EtOAc/PE (1:10 to 1:3). This resulted in 1.4 g (93%) of the title compound as a white solid. MS-ESI: 337 (M+1).

### Step 10: N'-(tert-butyldimethylsilyl)-2-(2-hydroxypropan-2-yl)thiazole-5-sulfonimidamide

Into a 250-mL 3-necked round-bottom flask purged with and maintained under nitrogen was placed PPh₃Cl₂ (3.0 g, 10.2 mmol) in CHCl₃ (100 mL). This was followed by the addition of TEA (2.06 g, 20.4 mmol) dropwise with stirring at RT. After stirring at 0°C for 10 min, a solution of *N-*(*tert*-butyldimethylsilyl)-2-(2-hydroxypropan-2-yl)thiazole-5-sulfonamide (2.3 g, 6.8 mmol) in CHCl₃ (10 mL) was dropwise to the above with stirring at 0°C. The resulting solution was allowed to react for 30 min at 0°C. To the mixture was added a saturated solution of ammonia in DCM (10 mL) at 0°C. The resulting solution was stirred for 2 h at RT. The reaction was then quenched by the addition of 100 mL of water. The resulting solution was extracted with 3x50 mL of DCM and the organic layers combined and concentrated under vacuum. The residue was eluted from silica gel with a gradient of EtOAc/PE (1:10 to 1:3). This resulted in 1.2 g (53%) of the title compound as a light yellow solid. MS-ESI: 336 (M+1).

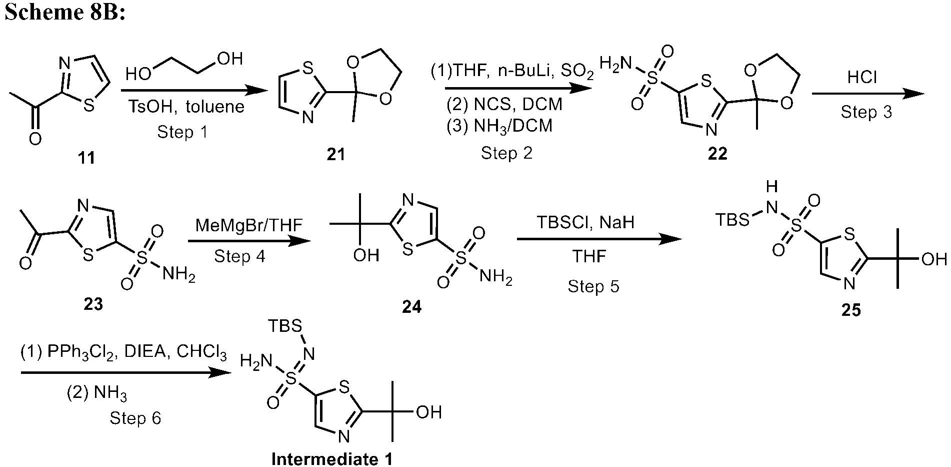

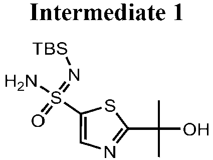

### N'-(tert-butyldimethylsilyl)-2-(2-hydroxypropan-2-yl)thiazole-5-sulfonimidamide

### Step 1: 2-(2-Methyl-1,3-dioxolan-2-yl)thiazole

Into a 500-mL round-bottom flask, was placed a solution of 1-(thiazol-2-yl)ethanone (20 g, 157 mmol) in toluene (300 mL) and ethane-1,2-diol (19.5 g, 314 mmol). To the solution was added TsOH (2.7 g, 15.7 mmol). The resulting solution was refluxed overnight, and water was separated from the solution during the reflux. The resulting solution was diluted with 200 mL of water and extracted with 2x100 mL of EtOAc. The organic layers were combined, dried over anhydrous Na₂SO₄, and then concentrated under vacuum. This resulted in 26.6 g (99%) of the title compound as light yellow oil. MS-ESI: 172.0 (M+1).

### Step 2: 2-(2-Methyl-1,3-dioxolan-2-yl)thiazole-5-sulfonamide

Into a 500-mL 3-necked round-bottom flask purged with and maintained under nitrogen was placed a solution of 2-(2-methyl-1,3-dioxolan-2-yl)thiazole (14 g, 81.6 mmol) in THF (200 mL). This was followed by the addition of n-BuLi (2.5 M in THF, 35.2 mL, 88 mmol) dropwise with stirring at -78°C. The resulting solution was stirred for 0.5 h at -78°C and then SO₂ was introduced into the above reaction mixture. The reaction was slowly warmed to RT and then NCS (12.8 g, 95.9 mmol) was added. The resulting solution was stirred for 1 h at RT. The solids were filtered out. The resulting filtrate was concentrated under vacuum and then was diluted in DCM (160 mL). To the above was added a saturated solution of ammonia in DCM (300 mL). The resulting solution was stirred for 3 h at RT and then was concentrated under vacuum. The residue was eluted from silica gel with a gradient of EtOAc/PE (1:20 to 1:5). This resulted in 12.5 g (61%) of the title compound as a yellow solid. MS-ESI: 251.0 (M+1).

### Step 3: 2-Acetylthiazole-5-sulfonamide

Into a 250-mL round-bottom flask was placed a solution of 2-(2-methyl-1,3-dioxolan-2-yl)thiazole-5- sulfonamide (12.5 g, 50 mmol) in THF (125 mL). To the above was added aq. HCl (4 N, 50.0 mL). The resulting solution was stirred for 6 h at 70°C. The resulting solution was diluted with 100 mL of water and extracted with 2x200 mL of EtOAc. The organic layers were combined, dried over anhydrous Na₂SO₄, then concentrated under vacuum. The residue was eluted from silica gel with a gradient of EtOAc/PE (1:2 to 1:1). This resulted in 9.3 g (90%) of the title compound as a yellow solid. MS-ESI: 207.0 (M+1).

### Step 4: 2-(2-hydroxypropan-2-yl)thiazole-5-sulfonamide

Into a 500-mL round-bottom flask purged with and maintained under nitrogen was placed a solution of 2-acetylthiazole-5-sulfonamide (15 g, 72.8 mmol) in THF (150 mL). This was followed by the addition of MeMgBr (3 M in THF, 97 mL, 291mmol) dropwise with stirring at 0°C. The resulting solution was stirred for 14 h at RT and then was quenched by the addition of 100 mL of NH₄Cl (sat.). The resulting solution was extracted with 3x150 mL of DCM and the organic layers combined and dried over anhydrous Na₂SO₄, then concentrated under vacuum. The residue was eluted from silica gel with EtOAc/PE (1:5 to 1:3). This resulted in 11.5 g (78%) of the title compound as a white solid. MS-ESI: 223 (M+1).

### Step 5: N-(tert-butyldimethylsilyl)-2-(2-hydroxypropan-2-yl)thiazole-5-sulfonamide

Into a 250-mL 3-necked round-bottom flask purged with and maintained under nitrogen was placed a solution of 2-(2-hydroxypropan-2-yl)thiazole-5-sulfonamide (5.0 g, 22.5 mmol) in THF (100 mL). Then to the above was added NaH (60% wt. oil dispersion, 1.8 g, 45 mmol) in portions in an ice/water bath. After stirring for 20 minutes in a water/ice bath, this was followed by the addition of a solution of TBSCl (4.1 g, 27.2 mmol) in THF (10 mL) dropwise with stirring at 0°C. The resulting solution was stirred for 4 h at RT. The reaction was quenched with sat. NH₄Cl (100 mL). The resulting solution was extracted with 3 x 100 mL of EtOAc and the combined organic layers were dried over Na₂SO₄ and concentrated under vacuum. The crude solid was washed with EtOAc/hexane (1:5) (2x100 mL). This resulted in 6.81 g (90%) of the title compound as a yellow solid. MS-ESI: 337 (M+1).

**Step 6** used the same procedures for converting compound **20** to Intermediate **1** shown in Scheme 8A to afford Intermediate **1** from compound **25.** MS-ESI: 336.1 (M+1).

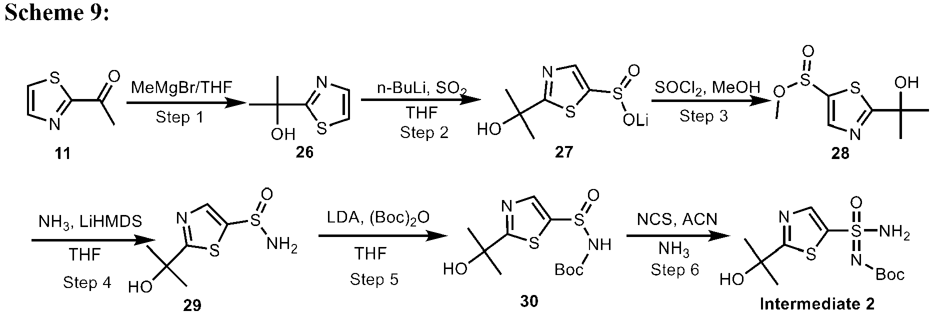

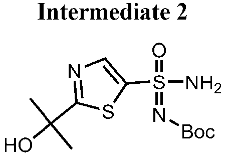

### N'-(tert-butoxycarbonyl)-2-(2-hydroxypropan-2-yl)thiazole-5-sulfonimidamide

### Step 1: 2-(Thiazol-2-yl)propan-2-ol

Into a 10-L 4-necked round-bottom flask purged with and maintained under nitrogen was placed a solution of 1-(thiazol-2-yl)ethanone (200 g, 1.6 mol) in THF (4 L). This was followed by the addition of MeMgBr (3 M in THF, 942 mL) dropwise with stirring at 0°C. The mixture was stirred at 0°C for 2 h. After warming the mixture to RT, the solution was stirred for an additional 16 h. Then the reaction was quenched by the addition of 3.0 L of NH₄Cl (sat.). The resulting solution was extracted with 3x1 L of EtOAc. The organic layers were combined, dried over anhydrous Na₂SO₄, and then concentrated under vacuum. The residue was eluted from silica gel with a gradient of EtOAc/PE (1:3 to 1:1). This resulted in 210 g (93%) of the title compound as a brown oil. MS-ESI: 144.0 (M+1). ¹H NMR (400 MHz, DMSO-*d₆*) δ 7.68 (d, *J* = 3.2 Hz, 1H), 7.54 (d, *J* = 3.2 Hz, 1H), 5.94 (s, 1H), 1.51 (s, 6H).

### Step 2: Lithium 2-(2-hydroxypropan-2-yl)thiazole-5-sulfinate

Into a 10-L 4-necked round-bottom flask purged with and maintained under nitrogen was placed a solution of 2-(thiazol-2-yl)propan-2-ol (50 g, 349 mmol) in THF (1.5 L). This was followed by the addition of n-BuLi (2.5 M in hexane, 350 mL) dropwise with stirring at -78°C. The mixture was stirred at -78°C for 1 h. Then SO₂ was bubbled into the mixture for 15 min below -30°C. The mixture was stirred for an additional 1 h at RT and then was concentrated under vacuum. This resulted in 87 g title compound as a light yellow solid (crude). The crude product was used directly in the next step.

### Step 3: Methyl 2-(2-hydroxypropan-2-yl)thiazole-5-sulfinate

Into a 2-L 3-necked round-bottom flask, lithium 2-(2-hydroxypropan-2-yl)thiazole-5-sulfinate (87 g, crude) was dissolved in anhydrous methanol (500 mL). Then SOCl₂ (43 g, 360 mmol) was added to the mixture dropwise with stirring at 0°C. The mixture was stirred overnight at RT and was then concentrated under vacuum. The residue was diluted with 500 mL of EtOAc. The resulting solution was washed with 2x200 mL of water and 2x200 mL of brine. The solution was dried over anhydrous Na₂SO₄, and then concentrated under vacuum. This resulted in 72 g crude title compound as light yellow oil. The crude product was used directly in the next step. MS-ESI: 222 (M+1). ¹H NMR (400 MHz, DMSO-*d₆*) δ 8.15 (s, 1H), 6.32 (s, 1H), 3.65 (s, 3H), 1.53 (d, *J* = 2.0 Hz, 6H).

### Step 4: 2-(2-Hydroxypropan-2-yl)thiazole-5-sulfinamide

Into a 10-L 4-necked round-bottom flask purged with and maintained under nitrogen, was placed a solution of methyl 2-(2-hydroxypropan-2-yl)thiazole-5-sulfinate (72 g, 326 mmol) in THF (500 mL). Then to the above NH₃ (0.5 M in THF, 2.0 L) was added. After cooling to -78°C, LiHMDS (1 M in THF, 2.0 L) was added to the mixture dropwise with stirring. Then the mixture was stirred at -78°C for 2 h. The reaction was quenched by the addition of 500 mL of NH₄Cl (sat.). The resulting solution was extracted with 3x300 mL of EtOAc. The organic layers were combined, dried over anhydrous Na₂SO₄, and then concentrated under vacuum. This resulted in 32 g crude title compound as brown oil. The crude product was used directly in the next step. MS-ESI: 207 (M+1). ¹H NMR (400 MHz, DMSO-*d₆*) δ 7.77 (s, 1H), 6.73 (s, 2H), 6.17 (s, 1H), 1.51 (d, *J* = 1.4 Hz, 6H).

### Step 5: Tert-butyl 2-(2-hydroxypropan-2-yl)thiazol-5-ylsulfinylcarbamate

Into a 1-L 3-necked round-bottom flask purged with and maintained under nitrogen, was placed a solution of 2-(2-hydroxypropan-2-yl)thiazole-5-sulfinamide (32 g, crude) in THF (300 mL). This was followed by the addition of LDA (2 M in THF, 116 mL) dropwise with string at 0°C. The mixture was stirred at 0°C for 1 h, then (Boc)₂O (33.8 g, 155 mmol) was added in portions at 0°C. The mixture was warmed to RT and stirred for an additional 2 h. The reaction was quenched with 200 mL of ice-water (200 mL), and the pH value of the solution was adjusted to 6 with HCO₂H. The resulting solution was extracted with 3x200 mL of EtOAc. The organic layers were combined, dried over anhydrous Na₂SO₄, and then concentrated under vacuum. The residue was eluted from silica gel with a gradient of EtOAc/PE (1:2 to 1:1). This resulted in 19 g (18%, 4 steps) of the title compound as a white solid. MS-ESI: 307 (M+1).

### Step 6: N-(tert-butyldimethylsilyl)-2-(2-hydroxypropan-2-yl)thiazole-5-sulfonimidamide

Into a 1-L 3-necked round-bottom flask purged with and maintained under nitrogen, *tert*-butyl 2-(2-hydroxypropan-2-yl)thiazol-5-ylsulfinylcarbamate (19 g, 62 mmol) was dissolved in fresh distilled ACN (200 mL). Then to the above solution was added NCS (9.8 g, 74 mmol) in portions. The mixture was stirred for 1 h at RT and then NH₃ was bubbled in the mixture for 15 min. The mixture was stirred at RT for 2 h and then concentrated under vacuum. The residue was applied onto silica gel and eluted with a gradient of EtOAc/PE (1:2 to 1:1). This resulted in 13 g (65%) of the title compound as a white solid. MS-ESI: 322 (M+1). ¹H NMR (300 MHz, DMSO-*d₆*) δ 7.99 (s, 1H), 7.72 (s, 2H), 6.29 (s, 1H), 1.49 (d, *J* = 2.0 Hz, 6H), 1.27 (s, 9H).

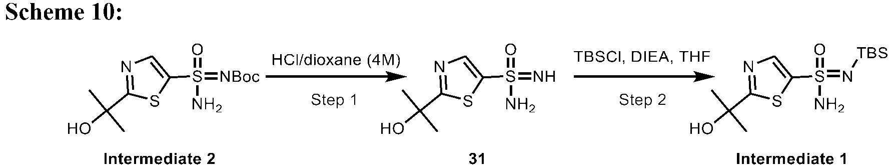

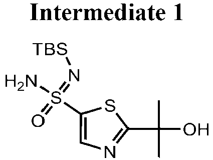

### N'-(tert-butyldimethylsilyl)-2-(2-hydroxypropan-2-yl)thiazole-5-sulfonimidamide

### Step 1: 2-(2-Hydroxypropan-2-yl)thiazole-5-sulfonimidamide

Into a 250-mL round-bottom flask, was placed a solution of tert-butyl 2-(2-hydroxypropan-2-yl)thiazole -5-sulfonimidoylcarbamate (3.21 g, 10 mmol) in HCl/dioxane (4 M, 50 mL). The resulting solution was stirred for 1 h at RT. The solution was concentrated to give the title compound (3.2 g, crude, yellow oil). MS-ESI: 222 (M+1).

### Step 2: N'-(tert-butyldimethylsilyl)-2-(2-hydroxypropan-2-yl)thiazole-5-sulfonimidamide

Into a 250-mL round-bottom flask, was placed 2-(2-hydroxypropan-2-yl)thiazole-5-sulfonimidamide (3.2 g crude, 10 mmol) in THF (100 mL), and then DIEA (3.87 g, 30 mmol) was added in the reaction solution at RT. TBSCl (3.0 g, 20 mmol) was added to the solution in portions. The resulting solution was stirred for 16 h at RT. The solution was concentrated, and the crude product was eluted from silica gel with EtOAc/ PE (1:1) to give the title compound (2.3 g, yield 70%, yellow solid). MS-ESI: 336 (M+1).

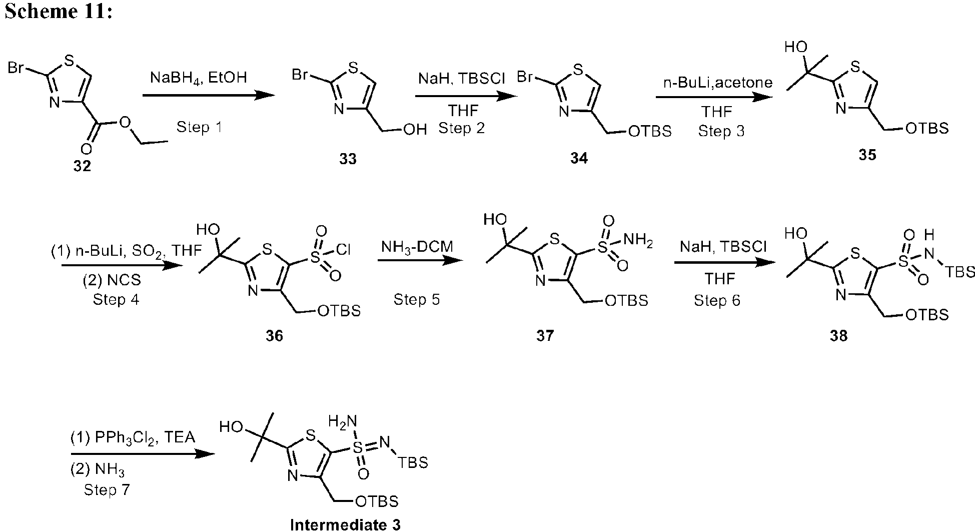

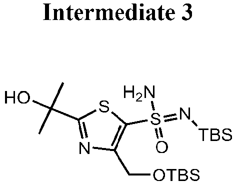

### N'-(tert-butyldimethylsilyl)-4-((tert-buiyldimethylsilyloxy)methyl)-2-(2-hydroxypropan-2-yl)thiazole-5-sulfonimidamide

### Step 1: (2-Bromothiazol-4-yl)methanol

Into a 100-mL round-bottom flask, was placed a solution of ethyl 2-bromo-1,3-thiazole-4-carboxylate (3.0 g, 12.7 mmol) in EtOH (30 mL). To the stirred solution was added NaBH₄ (1.0 g, 25.41 mmol) in portions with an ice/water bath. The resulting solution was stirred for 3 h at RT. The reaction was then quenched by the addition of 100 mL of water in an ice/water bath. The resulting solution was extracted with 3x100 ml of EtOAc and the combined organic layers were concentrated. This resulted in 2.0 g (81%) of the title compound as yellow oil. MS-ESI: 196.2/194.2 (M+1).

### Step 2: 2-Bromo-4-((tert-butyldimethylsilyloxy)methyl)thiazole

Into a 100-mL round-bottom flask, was placed a solution of (2-bromo-1,3-thiazol-4-yl)methanol (2.0 g, 10.3 mmol) in THF (20 mL). To the solution was added NaH (60% wt. oil dispersion, 1.2 g, 30.9 mmol) in portions with an ice/water bath. After stirring for 15 minutes at RT, a solution of TBSCl (4.7 g, 30.9 mmol) in THF (5 mL) was added dropwise in an ice/water bath. The resulting solution was stirred for 2 h at RT. The reaction was then quenched by the addition of 50 mL of water. The resulting solution was extracted with 3x100 ml of EtOAc, the organic layers were combined, dried over anhydrous Na₂SO₄, and concentrated under vacuum. The residue was eluted from a silica gel with EtOAc/PE (1:30). This resulted in 2.5 g (79%) of the title compound as yellow oil. MS-ESI: 310.2/308.2 (M+1).

### Step 3: 2-(4-((Tert-butyldimethylsilyloxy)methyl)thiazol-2-yl)propan-2-ol

Into a 100-mL round-bottom flask purged and maintained with an inert atmosphere of nitrogen, was placed a solution of 2-bromo-4-((tert-butyldimethylsilyloxy)methyl)thiazole (2.5 g, 8.11 mmol) in THF (30 mL). To this solution was added n-BuLi (2.5 M in hexane, 4.86 mL, 12.2 mmol) dropwise at -78°C, and the resulting mixture was stirred for 30 min at -78°C. To the above was added acetone (0.9 g, 16.2 mmol) dropwise at -78°C, then stirred for 1 h at RT. The reaction was then quenched by the addition of 100 mL of water. The resulting solution was extracted with 3x100 ml of EtOAc. The organic layers were combined, dried over anhydrous Na₂SO₄, and concentrated under vacuum. The residue was eluted from a silica gel with EtOAc/PE (1:10). This resulted in 2.0 g (86%) of the title compound as yellow oil. MS-ESI: 288.2 (M+1).

### Step 4: 4-((Tert-butyldimethylsilyloxy)methyl)-2-(2-hydroxypropan-2-yl)thiazole-5-sulfonyl chloride

Into a 250-mL round-bottom flask purged and maintained with an inert atmosphere of nitrogen, was placed a solution of 2-(4-((tert-butyldimethylsilyloxy)methyl)thiazol-2-yl)propan-2-ol (2.0 g, 6.96 mmol) in THF (20 mL). To this solution was added n-BuLi (2.5 M in hexane, 8.4 mL, 20.9 mmol) dropwise at -78°C, and the resulting solution was stirred for 30 min at -78°C. Then SO₂ was introduced in this solution for 10 minutes below -30°C and stirred for 30 min at RT. The resulting solution was concentrated under vacuum. The crude solid was dissolved in DCM (30ml), following by the addition of NCS (1.4 g, 10.4 mmol) in portions in an ice/water bath. The solution was stirred for 2 h at RT. The resulting mixture was concentrated under vacuum. This resulted in 2.5 g crude title compound as a yellow solid.

### Step 5: 4-((Tert-butyldimethylsilyloxy)methyl)-2-(2-hydroxypropan-2-yl)thiazole-5-sulfonamide

Into a 100-mL round-bottom flask was placed a solution of 4-((tert-butyldimethylsilyloxy)methyl)-2-(2-hydroxypropan-2-yl)thiazole-5-sulfonyl chloride (2.5 g, 6.48 mmol) in DCM (30 mL). To the above was added a saturated solution of ammonia in DCM (10 mL) in an ice/water bath. The resulting solution was stirred for 1 h at RT. The resulting mixture was concentrated. The residue was eluted from silica gel with EtOAc/PE (1:5). This resulted in 1.2 g (51%) of the title compound as yellow oil. MS-ESI: 367.2 (M+1).

### Step 6: N-(tert-butyldimethylsilyl)-4-((tert-butyldimethylsilyloxy)methyl)-2-(2-hydroxypropan-2-yl)thiazole-5-sulfonamide

To a solution of 4-((tert-butyldimethylsilyloxy)methyl)-2-(2-hydroxypropan-2-yl)thiazole-5-sulfonamide (1.2 g, 3.27 mmol) in THF (20 mL), NaH (60% wt. oil dispersion, 0.4 g, 9.8 mmol) was added in portions with an ice/water bath. After stirring for 15 minutes at RT, a solution of TBSCl (1.5 g, 9.82 mmol) in THF (5 mL) was added dropwise in an ice/water bath. The resulting solution was stirred for 2 h at RT. The reaction was quenched by the addition of 100 mL of water. The resulting solution was extracted with 3x100 ml of EtOAc. The organic layers were combined, dried over anhydrous Na₂SO₄ and concentrated under vacuum. This resulted in 1.3 g (83%) of the title compound as yellow oil. MS-ESI: 481.2 (M+1).

### Step 7: N'-(tert-butyldimethylsilyl)-4-((tert-butyldimethylsilyloxy)methyl)-2-(2-hydroxypropan-2-yl)thiazole-5-sulfonimidamide

Into a 100-mL round-bottom flask purged and maintained with an inert atmosphere of nitrogen was placed a solution of PPh₃Cl₂ (1.4 g, 4.06 mmol) in CHCl₃ (10 mL). TEA (0.80 g, 8.11 mmol) was then added dropwise in an ice/water bath. The solution was stirred at RT for 20 minutes. To this solution was added N-(tert-butyldimethylsilyl)-4-((tert-butyldimethylsilyloxy)methyl)-2-(2-hydroxypropan-2-yl)thiazole-5-sulfonamide (1.3 g, 2.70 mmol) in CHCl₃ (10 mL) dropwise in ice/water bath. The solution was stirred for 0.5 h at RT. The saturated solution of ammonia in DCM (20 mL) was poured into this solution at 0°C. The solution was stirred for 1 h at RT. The resulting solution was concentrated under vacuum. The residue was eluted from silica gel with EtOAc/PE (1:5). This resulted in 600 mg (46%) of the title compound as a yellow solid. MS-ESI: 480.2 (M+1).

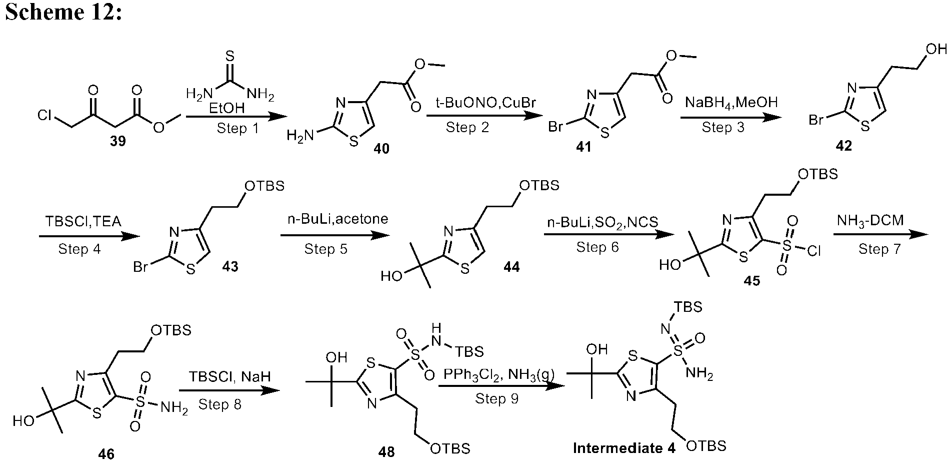

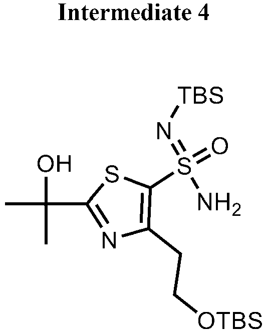

### N-(tert-butyldimethylsilyl)-4-(2-((tert-butyldimethylsilyl)oxy)ethyl)-2-(2-hydroxypropan-2-yl)thiazole-5-sulfonimidamide

### Step 1: Methyl 2-(2-aminothiazol-4-yl)acetate

Into a 1-L round-bottom flask, was placed a solution of methyl 4-chloro-3-oxobutanoate (15.0 g, 100 mmol) in EtOH (350 mL). Then thiourea (7.6 g, 100 mmol) was added to the solution. The resulting solution was stirred overnight under reflux. The resulting mixture was cooled to RT and filtered to collect the solid. The solid thus obtained was washed with Et₂O (2x200 mL) and dried in an oven at 50°C overnight to give the title compound (15.4 g, 89.5%) as a yellow solid. MS-ESI: 173 (M+1).

### Step 2: Methyl 2-(2-bromothiazol-4-yl)acetate

Into a 500 mL round-bottom flask, was placed a solution of methyl 2-(2-aminothiazol-4-yl)acetate (15.4 g, 89.5 mmol) in MeCN (250 mL). CuBr was added to the solution, followed by t-BuONO which was added dropwise at 0 °C. The resulting solution was stirred for 30 min at RT and then stirred for 2 h at 70 °C. The resulting mixture was concentrated and eluted from silica gel with EtOAc/PE (1:10) to give the title compound (12.3 g, 58.2%) as white solid. MS-ESI: 236/238 (M+1).

### Step 3: 2-(2-Bromothiazol-4-yl)ethanol

Into a 1-L round-bottom flask, was placed a solution of methyl 2-(2-bromothiazol-4-yl)acetate (12.3 g, 51.9 mmol) in EtOH (200 mL). NaBH₄ (3.9 g, 104 mmol) was added to the solution in portions at 0 °C. The resulting solution was stirred for 3 h at RT. The reaction was then quenched by the addition of 1 L of ice-water. The resulting solution was extracted with 3x500 ml of EtOAc, and the combined organic layers were dried over Na₂SO₄ and concentrated under vacuum. This resulted in 8.9 g (82.1%) of the title compound as yellow oil. MS-ESI: 208/210 (M+1).

### Step 4: 2-Bromo-4-(2-((tert-butyldimethylsilyl)oxy)ethyl)thiazole

Into a 500 mL round-bottom flask, was placed a solution of 2-(2-bromothiazol-4-yl)ethanol (8.9 g, 42.6 mmol) in THF (400 mL). NaH (60% wt. oil dispersion, 2.56 g, 63.9 mmol) was added to the mixture in portions at 0 °C. The mixture was stirred at 0 °C for another 1 h, and TBSCl (10.2 g, 68.2 mmol) was added to the mixture in portions at 0 °C. The resulting solution was stirred for 2 h at RT. The reaction was then quenched by the addition of 300 mL of ice-water. The resulting solution was extracted with 3x300 ml of EtOAc; the combined organic phase was dried over Na₂SO₄ and concentrated. The residue was eluted from silica gel with EtOAc/PE (1:30). This resulted in 7.6 g (55%) of the title compound as yellow oil. MS-ESI: 322/324 (M+1).

**Steps 5-9** used the same procedures for converting compound **34** to Intermediate **3** shown in **Scheme 11** to afford Intermediate **4** from compound **43.** MS-ESI: 494 (M+1).

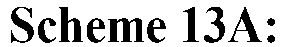

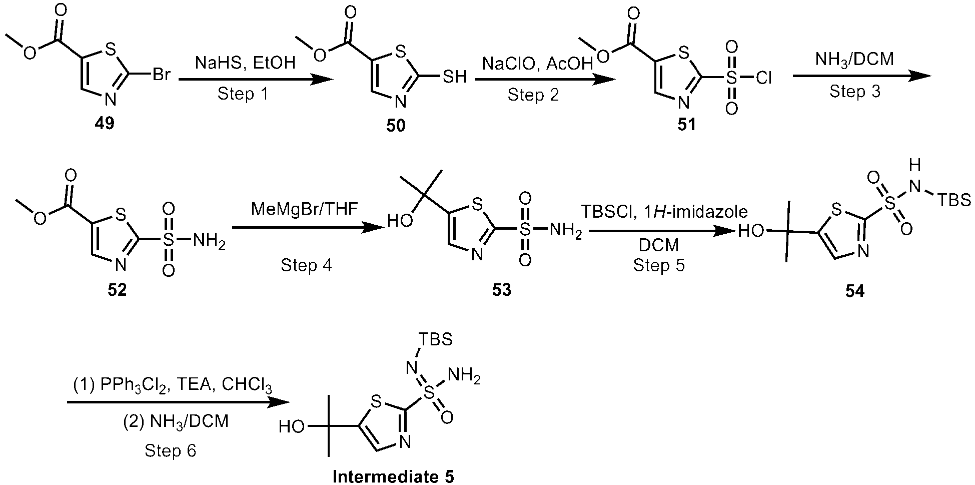

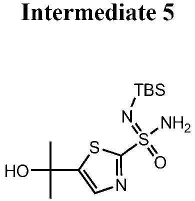

### N'-(tert-butyldimethylsilyl)-5-(2-hydroxypropan-2-yl)thiazole-2-sulfonimidamide

### Step 1: Methyl 2-mercaptothiazole-5-carboxylate

Into a 2-L round-bottom flask was placed methyl 2-bromothiazole-5-carboxylate (100 g, 450 mmol) and EtOH (1.0 L). To the stirred solution was added sodium hydrogen sulfide (50 g, 890 mmol) in portions with stirring. The resulting solution was stirred for 2 h at 80°C and then was cooled to 0°C with a water/ice bath. The pH of the solution was adjusted to 3 with hydrogen chloride (1 M). The solids were collected by filtration. This resulted in 63.2 g (80%) of the title compound as a light yellow solid. MS-ESI: 176.0 (M+1).

### Step 2: Methyl 2-(chlorosulfonyl)thiazole-5-carboxylate

Into a 1-L round-bottom flask was placed methyl 2-mercaptothiazole-5-carboxylate (30 g, 170 mmol) and acetic acid (300 mL). This was followed by the addition of sodium hypochlorite (300 mL, 8.0%-10% wt.) in portions at 0°C. The resulting solution was stirred for 2 h at RT and then was diluted with 500 mL of water. The solution was extracted with 3x300 mL of DCM, and the combined organic layers were washed with 2x300 mL of brine and dried over anhydrous Na₂SO₄. The crude product as a yellow solution in DCM was used in the next step.

### Step 3: Methyl 2-sulfamoylthiazole-5-carboxylate

Into a 2-L round-bottom flask was placed methyl 2-(chlorosulfonyl)thiazole-5-carboxylate as a crude solution in DCM (900 mL). To the solution was introduced NH₃ (g) below 0°C for 20 minutes. The resulting solution was stirred for 1 h at RT and then concentrated under vacuum. The residue was eluted from silica gel with EtOAc/PE (1:5 to 1:3). This resulted in 23 g (75%, 2 steps) of the title compound as a white solid. MS-ESI: 223.0 (M+1).

### Step 4: 5-(2-Hydroxypropan-2-yl)thiazole-2-sulfonamide

Into a 500-mL round-bottom flask purged with and maintained under nitrogen was placed a solution of methyl 2-sulfamoylthiazole-5-carboxylate (15 g, 67.5 mmol) in THF (150 mL). This was followed by the addition of MeMgBr/THF (3 M, 90 mL) dropwise with stirring at 0°C. The resulting solution was stirred for 14 h at RT and then was quenched by the addition of 100 mL of NH₄Cl (sat.). The resulting solution was extracted with 3x150 mL of DCM and the organic layers combined and dried over anhydrous Na₂SO₄, then concentrated under vacuum. The residue was eluted from silica gel with EtOAc/PE (1:5 to 1:3). This resulted in 11.5 g (78%) of the title compound as a white solid. MS-ESI: 223.0 (M+1).

**Steps 5-6** used similar procedure for converting compound **19** to Intermediate **1** shown in Scheme 8A to afford Intermediate **5** from compound **53.** MS-ESI: 336.1 (M+1).

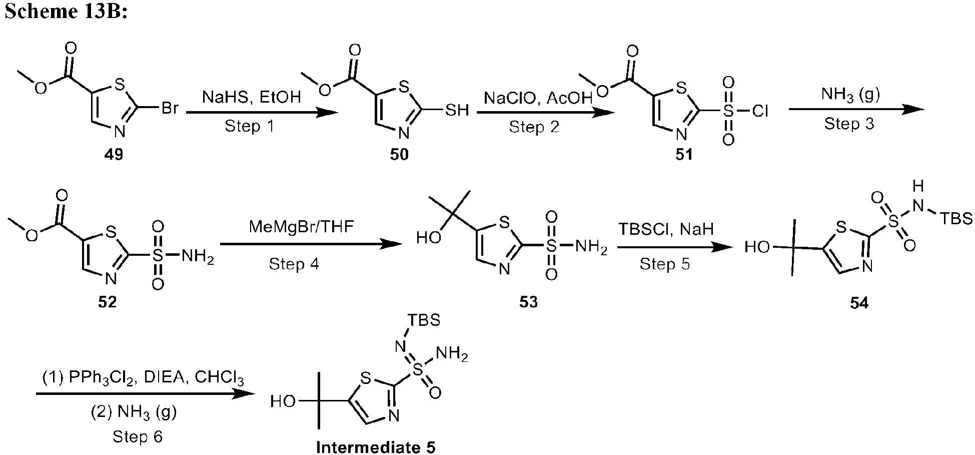

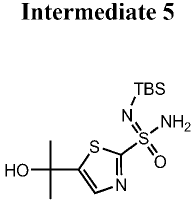

### N'-(tert-butyldimethylsilyl)-5-(2-hydroxypropan-2-yl)thiazole-2-sulfonimidamide

### Step 1: Methyl 2-mercaptothiazole-5-carboxylate

Into a 2-L round-bottom flask was placed methyl 2-bromothiazole-5-carboxylate (100 g, 450 mmol) and EtOH (1.0 L). To the stirred solution was added sodium hydrogen sulfide (50 g, 890 mmol) in portions with stirring. The resulting solution was stirred for 2 h at 80°C and then was cooled to 0°C with a water/ice bath. The pH value of the solution was adjusted to 3 with hydrogen chloride (1 M). The solids were collected by filtration. This resulted in 63.2 g (80%) of the title compound as a light yellow solid. MS-ESI: 176.0 (M+1).

### Step 2: Methyl 2-(chlorosulfonyl)thiazole-5-carboxylate

Into a 1-L round-bottom flask was placed methyl 2-mercaptothiazole-5-carboxylate (30 g, 170 mmol) and acetic acid (300 mL). This was followed by the addition of sodium hypochlorite (300 mL, 8.0%-10% wt.) in portions at 0°C. The resulting solution was stirred for 2 h at RT and then was diluted with 500 mL of water. The solution was extracted with 3x300 mL of DCM. The combined organic layers were washed with 2x300 mL of brine and dried over anhydrous Na₂SO₄. The crude product as a yellow solution in DCM was used in the next step.

### Step 3: Methyl 2-sulfamoylthiazole-5-carboxylate

Into a 2-L round-bottom flask was placed methyl 2-(chlorosulfonyl)thiazole-5-carboxylate as a crude solution in DCM (900 mL). To the solution was introduced NH₃ (g) below 0°C for 20 minutes. The resulting solution was stirred for 1 h at RT and then concentrated under vacuum. The residue was eluted from silica gel with EtOAc/PE (1:5 to 1:3). This resulted in 23 g (75%, 2 steps) of the title compound as a white solid. MS-ESI: 223.0 (M+1).

### Step 4: 5-(2-Hydroxypropan-2-yl)thiazole-2-sulfonamide

Into a 500-mL round-bottom flask purged with and maintained under nitrogen was placed a solution of methyl 2-sulfamoylthiazole-5-carboxylate (15 g, 67.5 mmol) in THF (150 mL). This was followed by the addition of MeMgBr/THF (3 M, 90 mL) dropwise with stirring at 0°C. The resulting solution was stirred for 14 h at RT and then was quenched by the addition of 100 mL of NH₄Cl (sat.). The resulting solution was extracted with 3x150 mL of DCM. The organic layers were combined, dried over anhydrous Na₂SO₄, and then concentrated under vacuum. The residue was eluted from silica gel with EtOAc/PE (1:5 to 1:3). This resulted in 11.5 g (78%) of the title compound as a white solid. MS-ESI: 223.0 (M+1).

### Step 5: N-(tert-butyldimethylsilyl)-5-(2-hydroxypropan-2-yl)thiazole-2-sulfonamide

Into a 250-mL 3-necked round-bottom flask purged with and maintained under nitrogen was placed a solution of 5-(2-hydroxypropan-2-yl)thiazole-2-sulfonamide (5.0 g, 22.5 mmol) in THF (100 mL). Then to the above was added NaH (60% wt. oil dispersion, 1.8 g, 45.0 mmol) in portions in an ice/water bath. After stirring for 20 minutes in a water/ice bath, a solution of TBSCl (4.1 g, 27.2 mmol) in THF (10 mL) was added dropwise with stirring at 0°C. The resulting solution was stirred for 4 h at RT. The reaction was quenched with sat. NH₄Cl (100 mL). The resulting solution was extracted with 3 x 100 mL of EtOAc, and the combined organic layers were dried over Na₂SO₄ and concentrated under vacuum. The crude solid was washed with EtOAc/hexane (1:5) (2x100 mL). This resulted in 6.81 g (90%) of the title compound as a yellow solid. MS-ESI: 337.1 (M+1), 335.1 (M-1) in positive and negative ion mode, respectively.

### Step 6: N'-(tert-butyldimethylsilyl)-5-(2-hydroxypropan-2-yl)thiazole-2-sulfonimidamide

Into a 100-mL 3-necked round-bottom flask purged with and maintained under nitrogen was placed a solution of PPh₃Cl₂ (3.0 g, 9.0 mmol) in CHCl₃ (100 mL). This was followed by the addition of DIEA (1.54 g, 11.9 mmol) dropwise with stirring at RT. The resulting solution was stirred for 10 min at RT. This was followed by the addition of a solution of N-(tertbutyldimethylsilyl)-5-(2-hydroxypropan-2-yl)thiazole-2-sulfonamide (2.0 g, 5.9 mmol) in CHCl₃ (30 mL) dropwise with stirring in an ice/water bath. The resulting solution was stirred for 30 min in an ice/water bath. To the above was introduced NH₃ (g) below 0°C for 15 minutes. The resulting solution was stirred for 20 minutes at RT. The solids were filtered out and the filtrate was concentrated, and the residue was dissolved in 300 mL of EtOAc. The solution was washed with brine (2x100 mL), dried over Na₂SO₄ and concentrated under vacuum. The crude solid was washed with CHCl₃ (100 mL). Then the filtrate was concentrated under vacuum and the residue was eluted from silica gel with EtOAc/PE (1:10 to 1:3). The original washed solid and solid from silica gel purification were combined. This resulted in 1.2 g (60%) of the title compound as a white solid. MS-ESI: 336.1 (M+1).

¹H-NMR (300 MHz, DMSO-*d*₆) δ 7.66 (s, 1H), 7.12 (s, 2H), 5.78 (s, 1H), 1.51 (s, 6H), 0.86 (s, 9H), 0.02 (s, 3H), 0.01 (s, 3H).

**Table 2. The Intermediates in the following Table were prepared using the similar procedures for converting compound 49 to Intermediate 5 shown in Scheme 13B from appropriate starting materials.**

| Intermediate # | Structure | IUPAC Name | Exact Mass[M+H]⁺ |
|---|---|---|---|
| **Intermediate 6** | 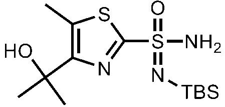 | N'-(tert-butyldimethylsilyl)-4-(2-hydroxypropan-2-yl)-5-methylthiazole-2-sulfonimidamide | 350 |

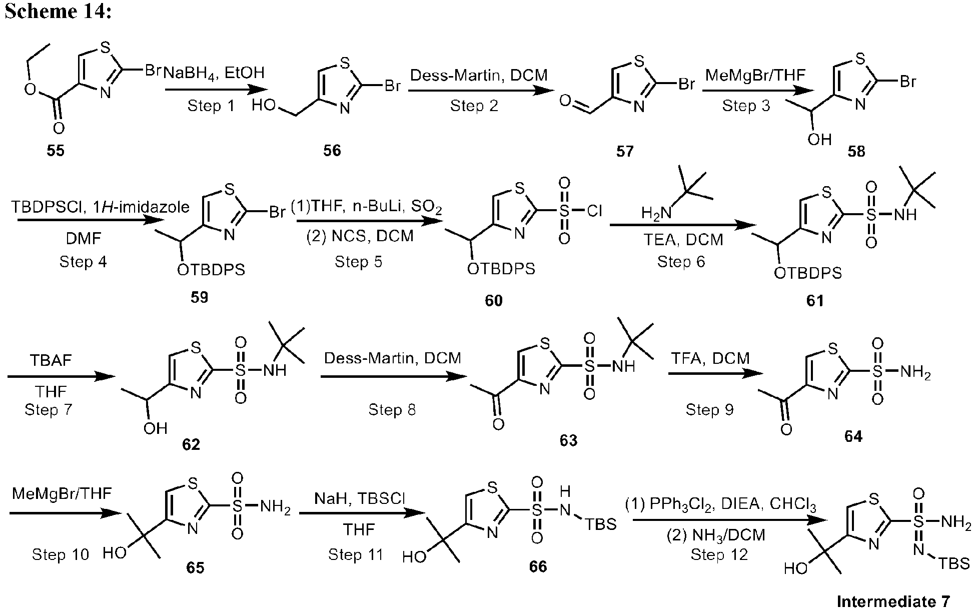

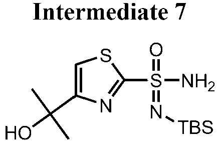

### N'-(tert-butyldimethylsilyl)-4-(2-hydroxypropan-2-yl)thiazole-2-sulfonimidamide

### Step 1: (2-Bromothiazol-4-yl)methanol

Into a 500-mL round-bottom flask was placed a solution of ethyl 2-bromothiazole-4-carboxylate (14 g, 59.3 mmol) in EtOH (200 mL). This was followed by the addition of NaBH₄ (2.3 g, 60.5 mmol) in portions at 0°C. The resulting solution was stirred for 3 h at RT and was then quenched by the addition of 100 mL of water. The resulting solution was extracted with 2x200 mL of DCM. The organic layers were combined, dried over anhydrous Na₂SO₄, and then concentrated under vacuum. This resulted in 10.0 g (87%) of the title compound as colorless oil. MS-ESI: 195.9/193.9 (M+1).

### Step 2: 2-Bromothiazole-4-carbaldehyde

Into a 250-mL round-bottom flask was placed a solution of (2-bromothiazol-4-yl)methanol (10.0 g, 51.5 mmol) in DCM (100 mL). To the solution was added Dess-Martin reagent (24.0 g, 56.6 mmol). The resulting solution was stirred for 2 h at RT and was then concentrated under vacuum. The residue was eluted from silica gel with a gradient of EtOAc/PE (1:50 to 1:20). This resulted in 8.0 g (81%) of the title compound as yellow oil. MS-ESI: 193.9/191.9 (M+1).

### Step 3: 1-(2-Bromothiazol-4-yl)ethanol

Into a 250-mL 3-necked round-bottom flask purged with and maintained under nitrogen was placed a solution of 2-bromothiazole-4-carbaldehyde (8.0 g, 41.7 mmol) in THF (100 mL). This was followed by the addition of MeMgBr (3 M in THF, 15 mL) dropwise with stirring at 0°C. The resulting solution was stirred for 2 h at RT and then was quenched by the addition of 100 mL of NH₄Cl (sat.). The resulting solution was extracted with 3x100 mL of DCM, and the combined organic layers were concentrated under vacuum. The residue was eluted from silica gel with a gradient of EtOAc/PE (1:10 to 1:5). This resulted in 6.0 g (69%) of the title compound as brown oil. MS-ESI: 209.9/207.9 (M+1).

### Step 4: 2-Bromo-4-(1-(tert-butyldiphenylsilyloxy)ethyl)thiazole

Into a 250-mL round-bottom flask was placed a solution of 1-(2-bromothiazol-4-yl)ethanol (6.0 g, 28.8 mmol) and 1*H*-imidazole (4.0 g, 58.8 mmol) in DMF (50 mL). To the solution was added TBDPSCl (8.7 g, 31.6 mmol). The resulting solution was stirred for 12 h at RT and was then diluted with 100 mL of water. The resulting solution was extracted with 3x100 mL of DCM, and the combined organic layers were concentrated under vacuum. The residue was eluted from silica gel with a gradient of EtOAc/PE (1:100 to 1:50). This resulted in 10.0 g (78%) of the title compound as light yellow oil. MS-ESI: 448.1/446.1 (M+1).

### Step 5: 4-(1-(Tert-butyldiphenylsilyloxy)ethyl)thiazole-2-sulfonyl chloride

Into a 250-mL 3-necked round-bottom flask purged with and maintained under nitrogen was placed a solution of 2-bromo-4-(1-(*tert*-butyldiphenylsilyloxy)ethyl)thiazole (10.0 g, 22.4 mmol) in THF (100 mL). This was followed by the addition of n-BuLi (2.5 M in THF, 11 mL) dropwise with stirring at -78°C. The resulting solution was stirred for 30 min at -78°C. To the above SO₂ gas was introduced. The reaction was warmed to RT and stirred for 30 min and then was concentrated under vacuum. The residue was dissolved in DCM (100 mL) and then NCS (3.6 g, 26.9 mmol) was added. The resulting solution was stirred for 30 min at RT and then was concentrated under vacuum. This resulted in 8.0 g (crude, 77%) of the title compound as a white solid. The crude product was used in the next step.

### Step 6: N-tert-butyl-4-(1-(tert-butyldiphenylsilyloxy)ethyl)thiazole-2-sulfonamide

Into a 100-mL round-bottom flask purged with and maintained under nitrogen was placed a solution of 4-(1-(*tert*-butyldiphenylsilyloxy)ethyl)thiazole-2-sulfonyl chloride (8.0 g, 17.2 mmol) in DCM (50 mL). To the solution were added TEA (3.5 g, 34.6 mmol) and 2-methylpropan-2-amine (1.9 g, 26.0 mmol). The resulting solution was stirred for 2 h at RT and was then concentrated under vacuum. The residue was eluted from silica gel with a gradient of EtOAc/PE (1:15 to 1:5). This resulted in 8.0 g (71%, 2 steps) of the title compound as brown oil. MS-ESI: 503.2 (M+1).

### Step 7: N-tert-butyl-4-(1-hydroxyethyl)thiazole-2-sulfonamide

Into a 250-mL round-bottom flask was placed a solution of N-*tert*-butyl-4-(1-(*tert-*butyldiphenylsilyloxy)ethyl)thiazole-2-sulfonamide (8.0 g, 15.9 mmol) in THF (100 mL). To the solution was added TBAF (9.6 g, 293 mmol). The resulting solution was stirred for 2 h at RT and then was diluted with 100 mL of water. The resulting solution was extracted with 3x100 mL of DCM , and the combined organic layers were concentrated under vacuum. The residue was eluted from silica gel with a gradient of EtOAc/PE (1:10 to 1:3). This resulted in 4.0 g (95%) of the title compound as light yellow oil. MS-ESI: 265.1 (M+1).

### Step 8: 4-Acetyl-N-tert-butylthiazole-2-sulfonamide

Into a 100-mL round-bottom flask, was placed a solution of N-*tert*-butyl-4-(1-hydroxyethyl)thiazole-2-sulfonamide (4.0 g, 15.1 mmol) in DCM (50 mL). To the solution was added Dess-Martin reagent (7.1 g, 16.6 mmol). The resulting solution was stirred for 2 h at RT and then was concentrated under vacuum. The residue was eluted from silica gel with a gradient of EtOAc/PE (1:10 to 1:3). This resulted in 3.5 g (88%) of the title compound as light yellow oil. MS-ESI: 363.0 (M+1).

### Step 9: 4-Acetylthiazole-2-sulfonamide

Into a 100-mL round-bottom flask was placed a solution of 4-acetyl-N-*tert*-butylthiazole-2-sulfonamide (3.5 g, 13.3 mmol) in DCM (5 mL). To the solution was added TFA (20 mL). The resulting solution was stirred for 14 h at 40°C and then was concentrated under vacuum. The residue was eluted from silica gel with a gradient of EtOAc/PE (1:10 to 1:3). This resulted in 2.5 g (91%) of the title compound as a gray solid. MS-ESI: 207.0 (M+1).

**Steps 10-12** used similar procedures for converting compound **23** to Intermediate **1** shown in Scheme 8B to afford Intermediate **7** from compound **64.** MS-ESI: 336.1 (M+1).

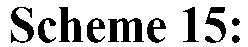

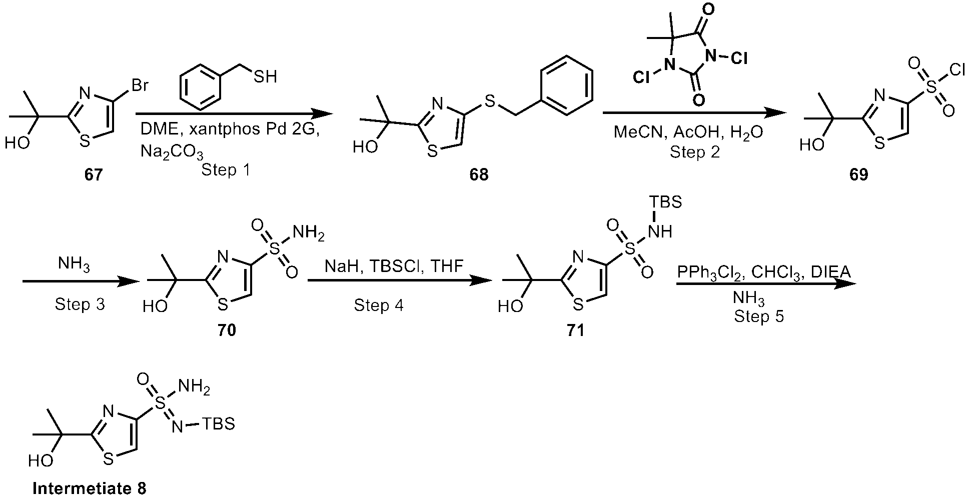

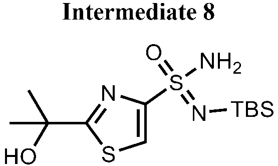

### N'-(tert-butyldimethylsilyl)-2-(2-hydroxypropan-2-yl)thiazole-4-sulfonimidamide

### Step 1: 2-(4-(Benzylthio)thiazol-2-yl)propan-2-ol

Into a 250-mL round-bottom flask, and maintained with an inert atmosphere of nitrogen, was placed a solution of 2-(4-bromo-1,3-thiazol-2-yl)propan-2-ol (2.0 g, 9.0 mmol) in DME (50 mL). This was followed by the addition of phenylmethanethiol (11.2 g, 90.0 mmol). To this was added Na₂CO₃ (1.91 g, 18.0 mmol) and Xphos Pd G₂ (2.4 g, 2.7 mmol). The resulting solution was stirred for an overnight at 80°C. The resulting mixture was diluted with 150 mL of H₂O. The resulting solution was extracted with 2x300 mL of EtOAc dried over anhydrous sodium sulfate and concentrated. The residue was eluted from silica gel with EtOAc/PE (1:1). This resulted in 800 mg (33%) of the title compound as a white solid. MS-ESI: 226.1 (M+1).

### Step 2: 2-(2-Hydroxypropan-2-yl)thiazole-4-sulfonyl chloride

Into a 100-mL round-bottom flask, was placed a solution of 2-(4-(benzylthio)thiazol-2-yl)propan-2-ol (750 mg, 2.83 mmol) in MeCN (20 mL). To the solution was added AcOH (0.75 mL) and H₂O (0.50 mL). This was followed by the addition of 1,3-dichloro-5,5-dimethylimidazolidine-2,4-dione (1.67 g, 8.48 mmol) at 0°C. The resulting solution was stirred for 2 h at RT. The resulting mixture was concentrated. The resulting mixture was diluted with 20 mL of H₂O. The resulting solution was extracted with 3x75 mL of DCM and dried over anhydrous sodium sulfate and concentrated. This resulted in 550 mg (80.5%) of the title compound as a crude solid. MS-ESI: 242 (M+1).

### Step 3: 2-(2-Hydroxypropan-2-yl)thiazole-4-sulfonamide

Into a 100-mL round-bottom flask, was placed a solution of 2-(2-hydroxypropan-2-yl)thiazole-4-sulfonyl chloride (550 mg, 2.28 mmol) in DCM (30 mL). To the above NH₃ (g) was introduced for 10 min at 0°C. The resulting solution was stirred for 20 min at RT. The resulting mixture was concentrated. The residue was eluted from a silica gel with EtOAc/PE (1:1). This resulted in 450 mg (89%) of the title compound as a yellow solid. MS-ESI: 223 (M+1).

**Steps 4-5** used similar procedures for converting compound **24** to Intermediate **1** shown in Scheme 8B to convert Intermediate **8** from compound **70.** MS-ESI: 336 (M+1).

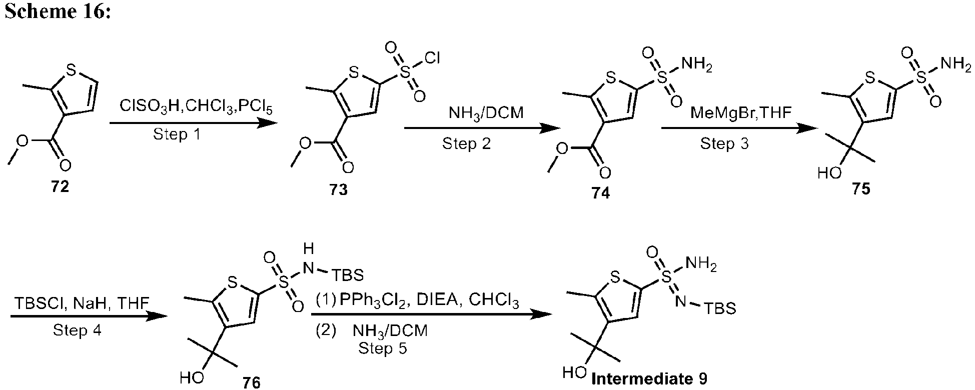

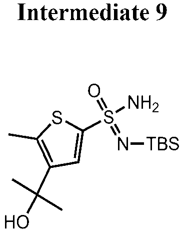

### N'-(tert-butyldimethylsilyl)-4-(2-hydroxypropan-2-yl)-5-methylthiophene-2-sulfonimidamide

### Step 1: Methyl 5-(chlorosulfonyl)-2-methylthiophene-3-carboxylate

Into a 250-mL round-bottom flask, was placed methyl 2-methylthiophene-3-carboxylate (5.0 g, 32 mmol), CHCl₃ (70 mL). This was followed by the addition of CISO₂OH (5.6 g, 48 mmol) dropwise with stirring. To this was added PCl₅ (13g, 64 mmol) with stirring. The resulting solution was stirred for 2 h at 60°C in an oil bath. The reaction was then quenched by the addition of 150 mL of water/ice. The resulting solution was extracted with 3x80 ml of DCM and dried over anhydrous sodium sulfate and concentrated. This resulted in 5.2 g (64%) of the title compound as a yellow solid.

**Steps 2-5** used similar procedures for converting compound **51** to intermediate **5** shown in Scheme 13B to
Intermediate **9** from compound **73.** MS-ESI: 349 (M+1).

**Table 3. The Intermediate in the following Table was prepared using similar procedure as shown in Scheme 16 above for converting compound 72 to Intermediate 9 starting from the appropriate materials.**

| Intermediate # | Structure | IUPAC Name | Exact Mass [M+H]⁺ |
|---|---|---|---|
| Intermediate 10 | 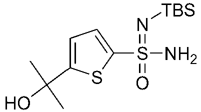 | N'-(*tert*-butyldimethylsilyl)-5-(2-hydroxypropan-2-yl)thiophene-2-sulfonimidamide | 335.1 |
| Intermediate 11 | 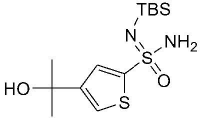 | N'-(tert-butyldimethylsilyl)-4-(2-hydroxypropan-2-yl)thiophene-2-sulfonimidamide | 335.1 |
| Intermediate 12 | 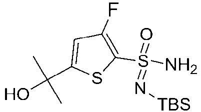 | N'-(tert-butyldimethylsilyl)-3-fluoro-5-(2-hydroxypropan-2-yl)thiophene-2-sulfonimidamide | 353.1 |

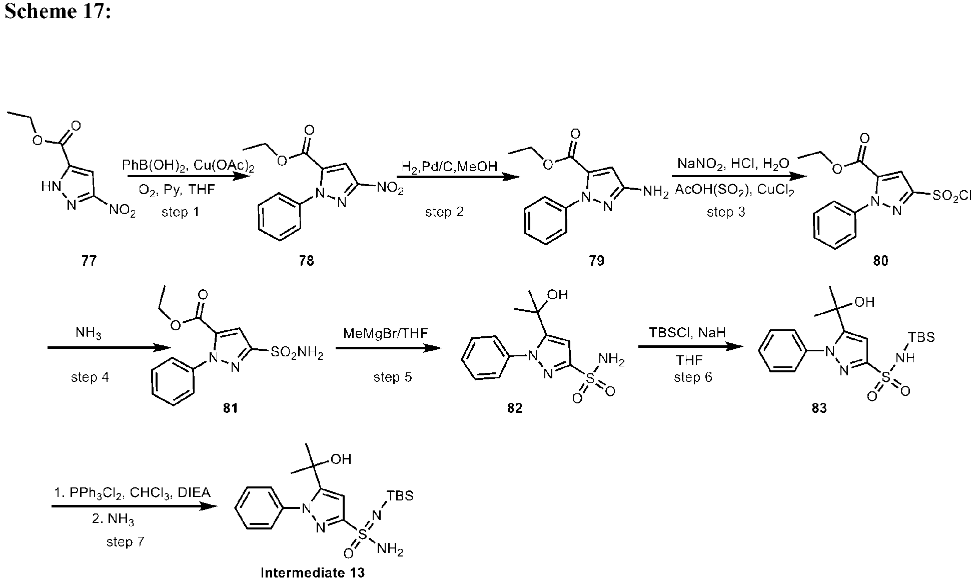

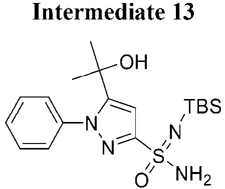

### N'-(tert-butyldimethylsilyl)-5-(2-hydroxypropan-2-yl)-1-phenyl-1H-pyrazole-3-sulfonimidamide

### Step 1: Ethyl 3-nitro-1-phenyl-1H-pyrazole-5-carboxylate

Into a 250-mL round-bottom flask purged and maintained with an inert atmosphere of oxygen, was placed ethyl 3-nitro-1H-pyrazole-5-carboxylate (5 g, 27 mmol) in tetrahydrofuran (150 mL). To the stirred solution was added phenylboronic acid (6.6 g, 54 mmol), Cu(OAc)₂ (7.38 g, 41 mmol) and pyridine (8.54 g, 108 mmol). The resulting solution was stirred overnight at RT. The resulting mixture was concentrated under vacuum. The residue was eluted from silica gel with EtOAc/PE (1:1). This resulted in 3.1 g (44%) of the title compound as an off-white solid. MS-ESI: 262 (M+1).

### Step 2: Ethyl 3-amino-1-phenyl-1H-pyrazole-5-carboxylate

Into a 100-mL round-bottom flask, was placed ethyl 3-nitro-1-phenyl-1H-pyrazole-5-carboxylate (3.92 g, 15 mmol) and methanol (50 mL). To the stirred solution was added Pd/C (wet 10%wt, 400 mg). The flask was evacuated and filled three times with hydrogen. The resulting solution was stirred overnight at RT. The solids were filtered out. The resulting mixture was concentrated under vacuum. This resulted in 2.8 g (81%) of the title compound as a light yellow solid. MS-ESI: 232 (M+1).

### Step 3: Ethyl 3-(chlorosulfonyl)-1-phenyl-1H-pyrazole-5-carboxylate

Into a 100-mL round-bottom flask, was placed ethyl 3-amino-1-phenyl-1H-pyrazole-5-carboxylate (1.8 g, 7.78 mmol) in HCl (6 M, 15 mL). This was followed by the addition of a solution of NaNO₂ (646 mg, 9.36 mmol) in water (2 mL) dropwise with stirring at -10°C. The resulting solution was stirred for 30 min at -10°C. The above mixture was added to a saturated solution of SO₂ in AcOH (20 mL) dropwise with stirring at 0°C. Then to the above was added CuCl₂ (1.05 g, 7.81 mmol). The resulting solution was stirred for 1 h at RT. The reaction was then quenched by the addition of 30 mL of water. The resulting solution was extracted with 3x30 mL of DCM. The organic layers were combined, dried over anhydrous sodium sulfate, and concentrated under vacuum. This resulted in 2.2 g (90%) of the title compound as a light yellow solid.

### Step 4: Ethyl 1-phenyl-3-sulfamoyl-1H-pyrazole-5-carboxylate

Into a 100-mL round-bottom flask, was placed a solution of ethyl 3-(chlorosulfonyl)-1-phenyl-1H-pyrazole-5-carboxylate (2.2 g, 6.99 mmol) in DCM (10 mL). Then to the above was introduced NH₃ gas bubbled at 0°C for 10 min. The resulting solution was stirred for 2 h at RT. The resulting mixture was concentrated under vacuum. The residue was applied onto silica gel with EtOAc/PE (1:1). This resulted in 1.07 g (52%) of the title compound as a light yellow solid. MS-ESI: 296 (M+1).

### Step 5: 5-(2-Hydroxypropan-2-yl)-1-phenyl-1H-pyrazole-3-sulfonamide

Into a 100-mL 3-necked round-bottom flask purged and maintained with an inert atmosphere of nitrogen, was placed a solution of ethyl 1-phenyl-3-sulfamoyl-1H-pyrazole-5-carboxylate (1.65 g, 5.59 mmol) in tetrahydrofuran (30 mL). This was followed by the addition of MeMgBr (3 M in THF, 18.6 mL) dropwise with stirring at 0°C. The resulting solution was stirred overnight at RT. The reaction was then quenched by the addition of 30 mL of NH₄Cl (sat.). The resulting solution was extracted with 3x30 mL of DCM and the organic layers combined and dried over anhydrous sodium sulfate and concentrated under vacuum. The residue was eluted from silica gel with EtOAc/PE (2:1). This resulted in 1.35 g (86%) of the title compound as a yellow solid. MS-ESI: 282 (M+1).

### Step 6: N-(tert-butyldimethylsilyl)-5-(2-hydroxypropan-2-yl)-1-phenyl-1H-pyrazole-3-sulfonamide

Into a 100-mL round-bottom flask, was placed 5-(2-hydroxypropan-2-yl)-1-phenyl-1H-pyrazole-3- sulfonamide (500 mg, 1.78 mmol) in tetrahydrofuran (10 mL). This was followed by the addition of sodium hydride (60% wt. oil dispersion, 143 mg, 3.58 mmol) in portions at 0°C. Then to the above was added TBSCl (538 mg, 3.57 mmol). The resulting solution was stirred for 2 h at RT. The reaction was then quenched by the addition of 10 mL of water. The resulting solution was extracted with 3x10 mL of DCM. The organic layers were combined, dried over anhydrous sodium sulfate, and concentrated under vacuum. The residue was eluted from silica gel with EtOAc/PE (1:2). This resulted in 660 mg (94%) of the title compound as a light yellow solid. MS-ESI: 396 (M+1).

### Step 7: N'-(tert-butyldimethylsilyl)-5-(2-hydroxypropan-2-yl)-1-phenyl-1H-pyrazole-3-sulfonimidamide

Into a 100-mL 3-necked round-bottom flask purged and maintained with an inert atmosphere of nitrogen, was placed the solution of PPh₃Cl₂ (1.67 g, 5.01 mmol) in chloroform (30 mL). This was followed by the addition of DIEA (1.29 g, 9.98 mmol) dropwise with stirring at RT. The resulting solution was stirred for 10 min at RT and the reaction system was cooled to 0°C. To this was added a solution of *N*-(*tert*-butyldimethylsilyl)-5-(2-hydroxypropan-2-yl)-1-phenyl-1H-pyrazole-3-sulfonamide (660 mg, 1.67 mmol) in chloroform (3 mL) dropwise with stirring at 0°C. The resulting solution was stirred for 30 min at 0°C. To the mixture was introduced NH₃ gas bubble for 15 min at 0°C. The resulting solution was stirred for 2 h at RT, after which it was diluted with 30 mL of water. The resulting solution was extracted with 3x30 mL of DCM. The organic layers were combined, dried over anhydrous sodium sulfate, and concentrated under vacuum. The residue was eluted from silica gel with EtOAc/PE (1:1). This resulted in 530 mg (81%) of the title compound as a light yellow solid. MS-ESI: 395 (M+1).

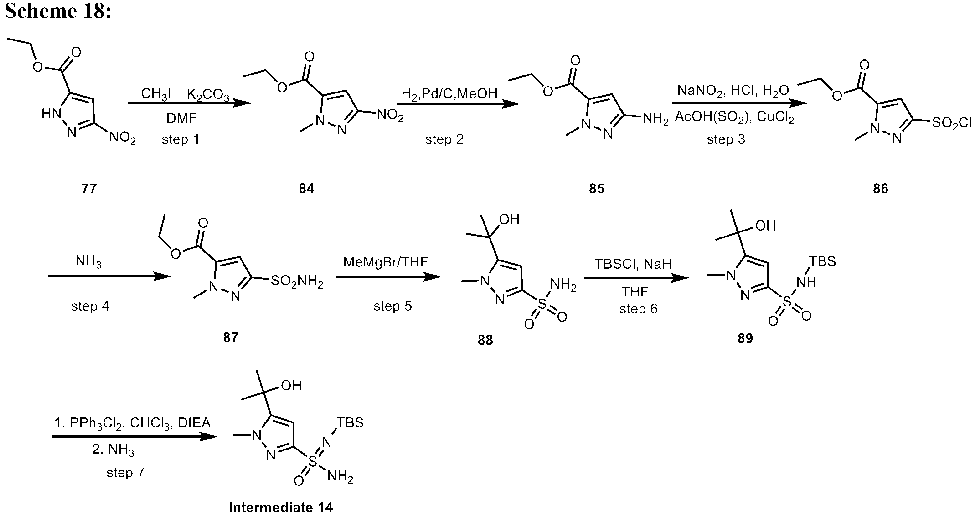

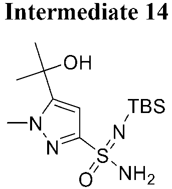

### N'-(tert-butyldimethylsilyl)-5-(2-hydroxypropan-2-yl)-1-methyl-1H-pyrazole-3-sulfonimidamide

### Step 1: Ethyl 1-methyl-3-nitro-1H-pyrazole-5-carboxylate

Into a 100-mL 3-necked round-bottom flask purged and maintained with an inert atmosphere of nitrogen, was placed the solution of ethyl 3-nitro-1H-pyrazole-5-carboxylate (1.0 g, 5.41 mmol) in DMF (20 mL). This was followed by the addition of K₂CO₃ (1.49 g, 10.8 mmol) in portions at 0°C. Then to the above was added CH₃I (922 mg, 6.49 mmol) dropwise at 0°C in an ice bath. The resulting solution was stirred for 16 h at RT. The reaction was then quenched by the addition of 10 mL of water. The resulting solution was extracted with 3x10 mL of DCM. The organic layers were combined, dried over anhydrous sodium sulfate, and concentrated under vacuum. The residue was eluted from silica gel with EtOAc/PE (1:2). This resulted in 807 mg (75%) of the title compound as a light yellow solid. MS-ESI: 200 (M+1).

**Steps 2-7** used similar procedures for converting compound **78** to intermediate **13** shown in Scheme 17 to afford intermediate **14** from compound **84.** MS-ESI: 333 (M+1).

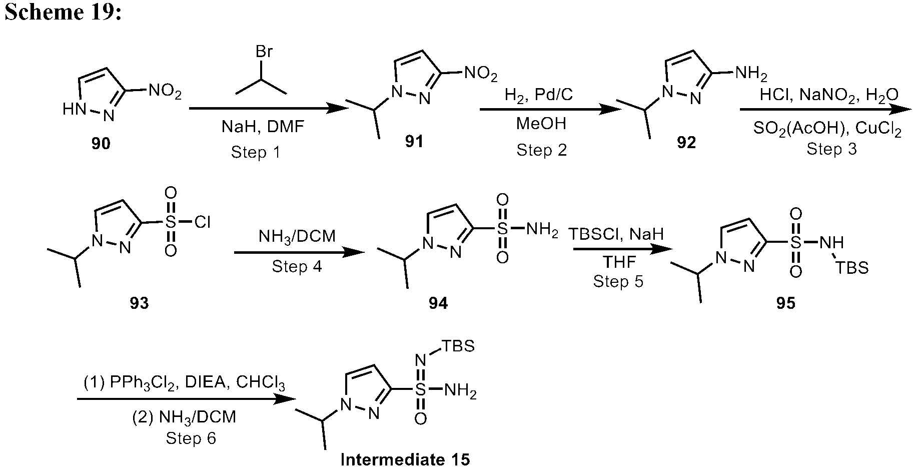

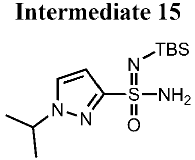

### N'-(tert-butyldimethylsilyl)-1-isopropyl-1H-pyrazole-3-sulfonimidamide

### Step 1: 1-Isopropyl-3-nitro-1H-pyrazole

Into a 250-mL round-bottom flask was placed a solution of 3-nitro-1*H*-pyrazole (10 g, 88.4 mmol) in DMF (100 mL). This was followed by the addition of NaH (60% wt., 3.9 g, 97.5 mmol) in portions at 0°C. The resulting solution was stirred for 0.5 h at 0°C. This was followed by the addition of 2-bromopropane (14.1 g, 115 mmol) dropwise with stirring at 0°C in 10 min. The resulting solution was stirred for 16 h at RT and then was quenched by the addition of 100 mL of water. The resulting solution was extracted with 3x100 mL of EtOAc. The organic layers were combined, dried over anhydrous Na₂SO₄, and then concentrated under vacuum. The residue was eluted from silica gel with a gradient of EtOAc/PE (1:5 to 1:3). This resulted in 11.8 g (86%) of the title compound as yellow oil. MS-ESI: 156.1 (M+1).

**Steps 2-6** used similar procedures for converting compound **78** to intermediate **13** shown in Scheme 17 to afford intermediate **15** from compound **91.** MS-ESI: 333 (M+1).

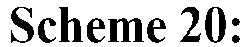

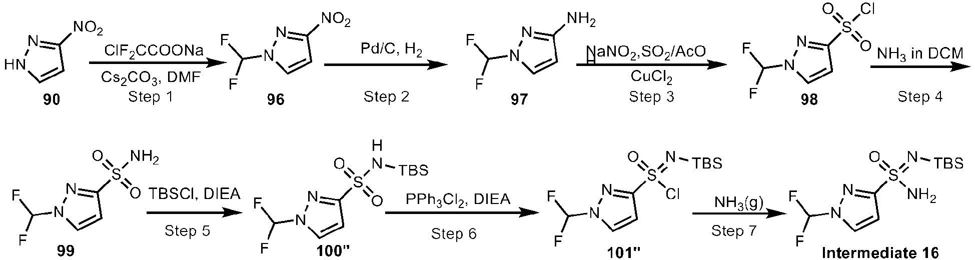

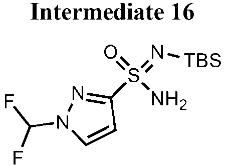

### N'-(tert-butyldimethylsilyl)-1-(difluoromethyl)-1H-pyrazole-3-sulfonimidamide

### Step 1: 1-(Difluoromethyl)-3-nitro-1H-pyrazole

Into a 250-mL round-bottom flask, was placed a solution of 3-nitro-1H-pyrazole (5 g, 44 mmol) in DMF (40 mL). To the stirred solution was added Cs₂CO₃ (14.4 g, 44 mmol) and F₂ClCCOONa (13.4 g, 88 mmol). The resulting solution was stirred for 30 min at 120°C. The reaction was then quenched by the addition of water. The resulting solution was extracted with 3x100 mL of EtOAc. The resulting mixture was washed with 3x100 mL of brine. The mixture was dried over anhydrous sodium sulfate. The solids were filtered out. The resulting mixture was concentrated under vacuum. The residue was eluted from silica gel with EtOAc/PE (1:10). This resulted in 4.3 g (59.6%) of 1-(difluoromethyl)-3-nitro-1H-pyrazole as yellow oil.

**Steps 2-7** used similar procedures for converting compound **78** to intermediate **13** shown in Scheme 17 to afford intermediate **16** from compound **96.** MS-ESI: 311 (M+1).

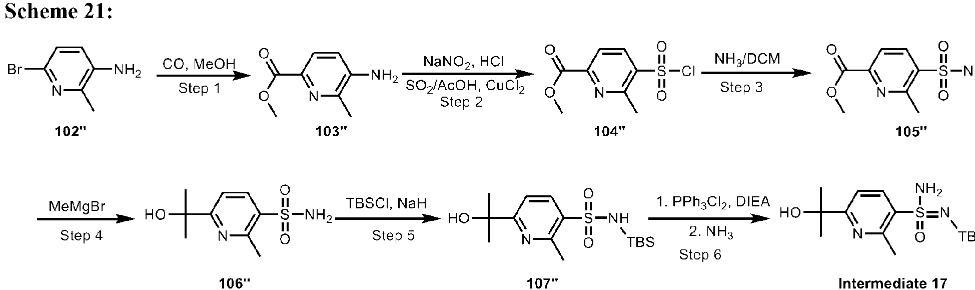

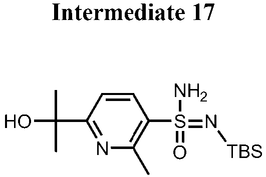

### N'-(tert-butyldimethylsilyl)-6-(2-hydroxypropan-2-yl)-2-methylpyridine-3-sulfonimidamide

### Step 1: Methyl 5-amino-6-methylpicolinate

Into a 50-mL seal tube was placed methyl 6-bromo-2-methylpyridin-3-amine (500 mg, 2.67 mmol) in MeOH (15 mL). To the stirred solution was added Pd(OAc)₂ (120 mg, 0.53 mmol), dppf (444 mg, 0.80 mmol) and TEA (809 mg, 8.01 mmol) with stirring. The seal tube was evacuated and flushed three times with CO. The resulting solution was stirred for 5 h at 100°C under 10 atm of CO. Then the solution was concentrated under vacuum. The residue was eluted from silica gel with EtOAc/PE (1:1). This resulted in 351mg (79.2 %) of the title compound as a light yellow solid. MS-ESI: 167 (M+1).

**Steps 2-6** used similar procedures for converting compound **79** to Intermediate **13** shown in Scheme 17 to
afford Intermediate **17** from compound **103".** MS-ESI: 344 (M+1).

**Table 4. The Intermediates in the following Table were prepared using the similar procedures for converting compound 102" to Intermediate 17 shown in Scheme 21 from appropriated starting materials.**

| Intermediate # | Structure | IUPAC Name | Exact Mass[M+H]⁺ |
|---|---|---|---|
| **Intermediate 18** | 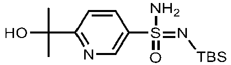 | N'-(tert-butyldimethylsilyl)-6-(2-hydroxypropan-2-yl)pyridine-3-sulfonimidamide | 330 |

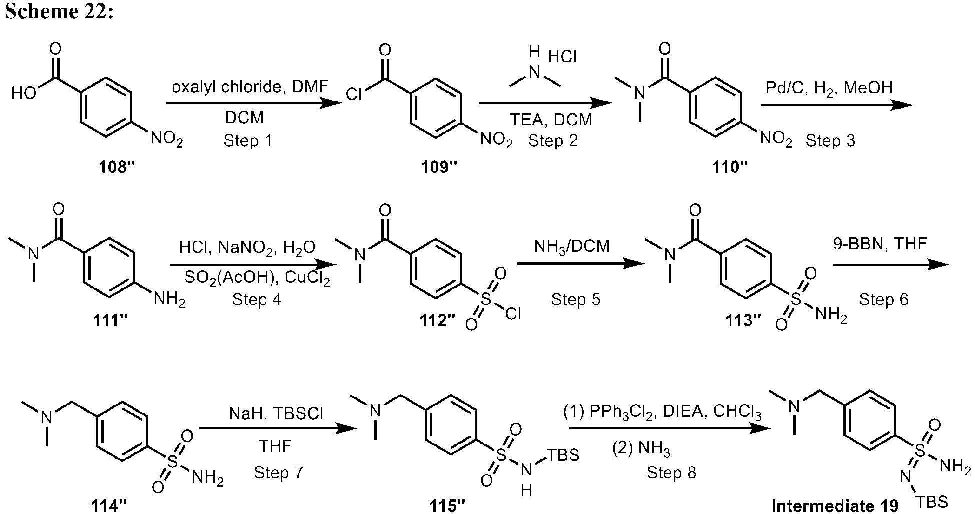

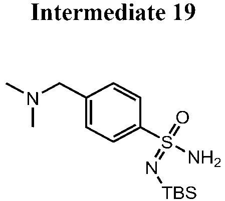

### N'-(tert-butyldimethylsilyl)-4-((dimethylamino)methyl)benzenesulfonimidamide

### Step 1: 4-Nitrobenzoyl chloride

Into a 500-mL round-bottom flask was placed 4-nitrobenzoic acid (20 g, 120 mmol) in DCM (200 mL) and DMF (0.2 mL). This was followed by the addition of oxalyl chloride (15 mL, 135 mmol) dropwise with stirring at 0°C. The resulting solution was stirred for 4 h at RT and then was concentrated under vacuum. This resulted in 22 g crude title compound as yellow oil. The crude product was used in the next step.

### Step 2: N,N-dimethyl-4-nitrobenzamide

Into a 500-mL round-bottom flask was placed dimethylamine hydrochloride (9.8 g, 120 mmol) in DCM (200 mL) and TEA (41.5 mL, 300 mmol). This was followed by the addition of 4-nitrobenzoyl chloride (22 g, crude) dropwise with stirring at 0°C. The resulting solution was stirred for 6 h at RT and then was concentrated under vacuum. The resulting mixture was washed with 2x50 mL of water. The solids were collected by filtration. This resulted in 16 g (69%, 2 steps) of the title compound as a white solid. MS-ESI: 195.1 (M+1).

### Step 3: 4-Amino-N,N-dimethylbenzamide

Into a 250-mL round-bottom flask was placed *N,N*-dimethyl-4-nitrobenzamide (16 g, 82.4 mmol) in MeOH (100 mL), to the above solution was added Pd/C (wet.10% wt., 1.0 g). The flask was evacuated and flushed three times with hydrogen. The resulting solution was stirred for 12 h at RT under an atmosphere of hydrogen. The Pd/C catalysts were filtered out, and the filtrate was concentrated under vacuum. This resulted in 13 g (96%) of the title compound as a white solid. MS-ESI: 165.1 (M+1).

### Step 4: 4-(Dimethylcarbamoyl)benzene-1-sulfonyl chloride

Into a 50-mL round-bottom flask was placed 4-amino-*N,N*-dimethylbenzamide (3.0 g, 18.3 mmol) in HCl (6 M, 12 mL). This was followed by the addition of a solution of NaNO₂ (1.5 g, 21.7 mmol) in water (3 mL) dropwise with stirring at 0°C. The resulting solution was stirred for 30 min at 0°C. The above mixture was added to a saturated solution of SO₂ in AcOH (100 mL) dropwise with stirring at 0°C. To the above was added CuCl₂ (4.8 g, 35.7 mmol). The resulting solution was stirred for 2 h at RT and then was quenched by the addition of 100 mL of water. The resulting solution was extracted with 2x100 mL of DCM. The organic layers were combined, dried over anhydrous Na₂SO₄, and then concentrated under vacuum. This resulted in 5 g crude title compound as yellow oil. The crude product was used in the next step.

### Step 5: N,N-dimethyl-4-sulfamoylbenzamide

Into a 250-mL round-bottom flask was placed 4-(dimethylcarbamoyl)benzene-1-sulfonyl chloride (5 g, 20.2 mmol) in DCM (20 mL). To the above solution was added a saturated solution of ammonia in DCM (80 mL). The resulting solution was stirred for 2 h at RT and then was concentrated under vacuum. The resulting mixture was washed with 3x100 mL of EtOAc. The solids were filtered out. The resulting filtrate was concentrated under vacuum. This resulted in 3.1 g (67%) of the title compound as a white solid. MS-ESI: 229.1 (M+1).

### Step 6: 4-((Dimethylamino)methyl)benzenesulfonamide

Into a 100-mL round-bottom flask purged with and maintained under nitrogen was placed a solution of N,N-dimethyl-4-sulfamoylbenzamide (1.8 g, 7.9 mmol) in THF (50 mL). This was followed by the addition of 9-BBN (5.8 g, 47.5 mmol) in portions at 0°C. The resulting solution was stirred for 12 h at 70°C and then was quenched by the addition of 20 mL of water/ice. The resulting solution was extracted with 3x100 mL of EtOAc and the organic layers were combined. The resulting mixture was washed with 200 mL of water and then the organic layer was concentrated under vacuum. The residue was Pd/C a silica gel with DCM/MeOH (20:1 to 15:1). This resulted in 1.0 g (59%) of the title compound as a white solid. MS-ESI: 215.1 (M+1).

**Steps 7-8** used similar procedures for converting compound **82** to intermediate **13** shown in Scheme 17 to afford Intermediate **19** from compound **114".** MS-ESI: 328 (M+1).

**Table 4. The Intermediates in the following Table were prepared using the similar procedures for converting compound 108" to Intermediate 19 shown in Scheme 22 from appropriated starting materials.**

| Intermediate # | Structure | IUPAC Name | Exact Mass[M+H]⁺ |
|---|---|---|---|
| Intermediate 20 | 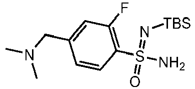 | N'-(tert-butyldimethylsilyl)-4-((dimethylamino)methyl)-2-fluorobenzenesulfonimidamide | 346.2 |

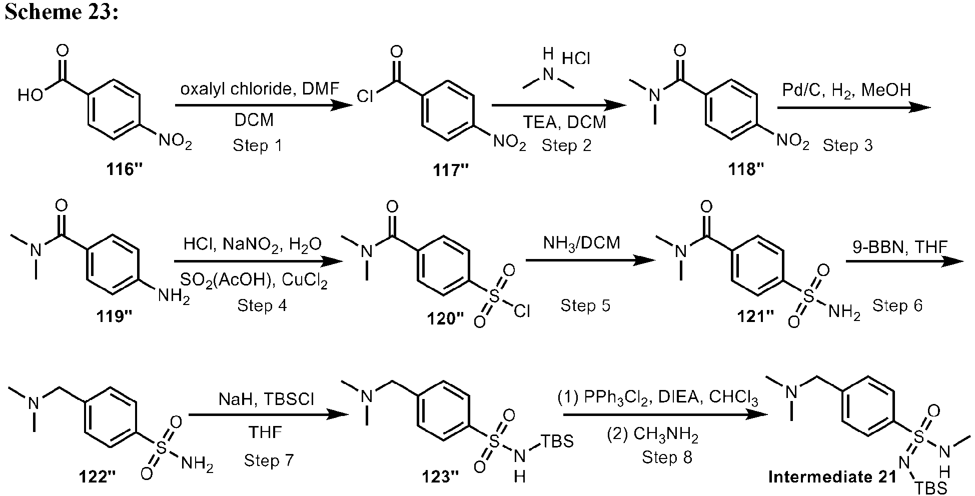

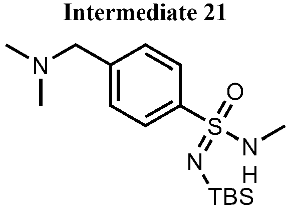

### N'-(tert-butyldimethylsilyl)-4-((dimethylamino)methyl)-N-methylbenzenesulfonimidamide

**Steps 1-7** used similar procedures for converting compound **108"** to compound **115"** shown in Scheme 22 to afford compound **123"** from compound **116".** MS-ESI: 329 (M+1).

### Step 9: N'-(tert-butyldimethylsilyl)-4-((dimethylamino)methyl)-N-methylbenzenesulfonimidamide

Into a 500-mL 3-necked round-bottom flask purged and maintained with an inert atmosphere of nitrogen, was placed the solution of PPh₃Cl₂ (13 g, 39 mmol) in chloroform (150 mL). This was followed by the addition of DIEA (10 g, 78 mmol) dropwise with stirring at RT. The resulting solution was stirred for 10 min at RT and the reaction system was cooled to 0°C. To this was added a solution of *N*-(*tert*-butyldimethylsilyl)-4-((dimethylamino)methyl)benzenesulfonamide (3.2 g, 9.76 mmol) in chloroform (30 mL) dropwise with stirring at 0°C. The resulting solution was stirred for 30 min at 0°C. To the mixture was added CH₃NH₂ (14.6 mL, 29.3 mmol, 2 M) for 15 min at 0°C. The resulting solution was stirred for 2 h at RT. The resulting solution was diluted with 100 mL of water. The resulting solution was extracted with 3x200 mL of DCM. The organic layers were combined, dried over anhydrous sodium sulfate, and concentrated under vacuum. The residue was eluted from silica gel with EtOAc/PE (36/64). This resulted in 1.43 g (43%) of the title compound as a yellow solid. MS-ESI: 342 (M+1).

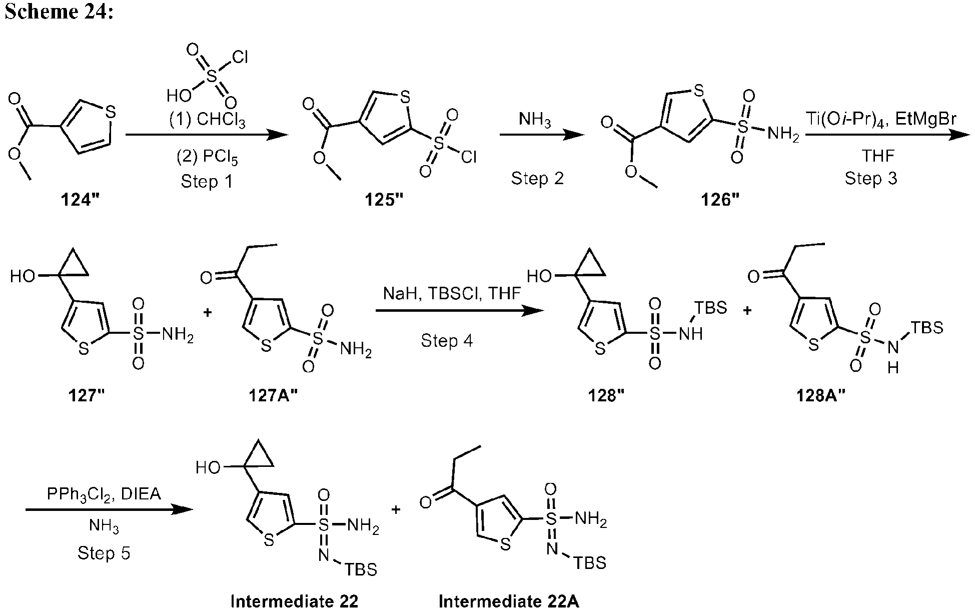

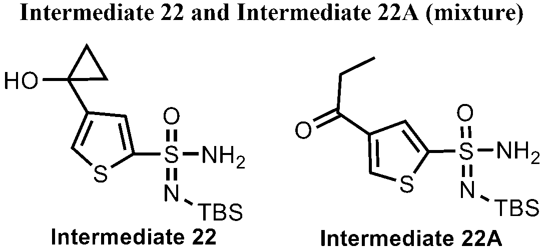

### N'-(tert-butyldimethylsilyl)-4-(1-hydroxycyclopropyl)thiophene-2-sulfonimidamide and N'-(tertbutyldimethylsilyl)-4-propionylthiophene-2-sulfonimidamide

Steps 1-2 used similar procedures for converting compound **72** to compound **74** shown in Scheme **16** to afford compound **126"** from compound 124". MS-ESI: 221 (M+1).

### Step 3: Mixture of 4-(1-hydroxycyclopropyl)thiophene-2-sulfonamide and 4-propionylthiophene-2-sulfonamide

To a solution of methyl 5-sulfamoylthiophene-3-carboxylate (5.0 g, 22.6 mmol) in THF (100 mL) under nitrogen was added Ti(O*i*-Pr)₄ (1.28 g, 4.52 mmol) dropwise at 0 °C. This was followed by the addition of EtMgBr (3 M in Et₂O, 45 mL, 135 mmol) dropwise with stirring at 0 °C. The resulting solution was stirred for 3 h at RT. The reaction was then quenched with 50 mL of sat. NH₄Cl (aq.). The resulting solution was extracted with 5x100 mL of EtOAc. The combined organic layers were combined, dried over anhydrous Na₂SO₄, and concentrated under vacuum. The residue was eluted from silica gel with DCM/MeOH (25:1). This resulted in 1.66 g (33.5%) of the title compounds (~1:1 mixture) as a light yellow solid. MS-ESI: both 220 (M+1). Steps 4-5 used similar procedures for converting compound **82** to Intermediate **13** shown in Scheme **17** to afford the mixture (~1:1) of Intermediate **22** and Intermediate **22A** from mixture of compound **127"** and **127A".** MS-ESI: both 333 (M+1).

**Steps 4-5** used similar procedures for converting compound **82** to intermediate **13** shown in Scheme 17 to afford intermediate **22** from compound 127". MS-ESI: 333 (M+1).

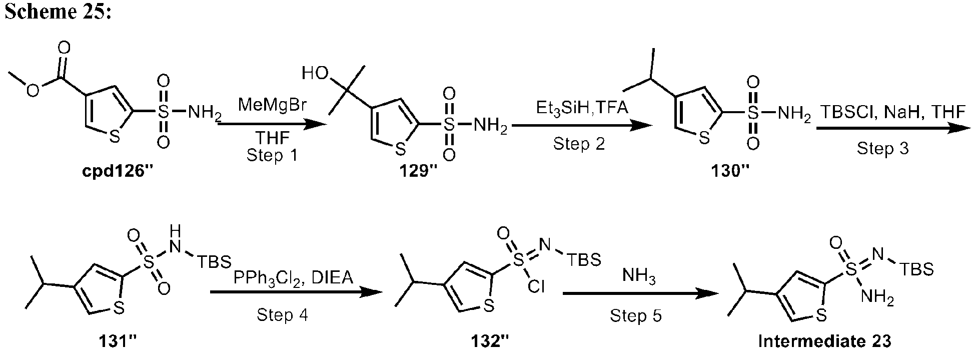

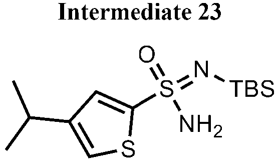

### N'-(tert-butyldimethylsilyl)-4-isopropylthiophene-2-sulfonimidamide

### Step 1: 4-(2-Hydroxypropan-2-yl)thiophene-2-sulfonamide

Into a 250-mL 3-necked round-bottom flask purged with and maintained under nitrogen was placed a solution of methyl 5-sulfamoylthiophene-3-carboxylate (4.42 g, 20 mmol) in THF (200 mL). This was followed by the addition of MeMgBr (3 M in THF, 40 mL) dropwise with stirring at 0°C. The resulting solution was stirred for 16 h at RT and was then quenched by the addition of 100 mL of NH₄Cl (sat.). The resulting solution was extracted with 3x100 mL of EtOAc. The organic layers were combined, dried over anhydrous Na₂SO₄, and then concentrated under vacuum. The residue was applied onto a silica gel and eluted with a gradient of EtOAc/PE (1:3 to 1:1). This resulted in 2.21 g (50%) of the title compound as a light yellow solid. MS-ESI: 220.2 (M-1).

### Step 2: 4-Isopropylthiophene-2-sulfonamide

Into a 250-mL round-bottom flask, was placed a solution of 4-(2-hydroxypropan-2-yl)thiophene-2-sulfonamide (1.5 g, 6.79 mmol) in DCM (20 mL). To the stirred solution was added TFA (3.9g, 34 mmol) and Et₃SiH (2.32 g, 20 mmol). The resulting solution was stirred for overnight at RT. The mixture was concentrated under vacuum. The residue was eluted from silica gel with a gradient of EtOAc/PE (1:5 to 1:3). This resulted in 1.1 g (79%) of the title compound as a light yellow solid. MS-ESI: 206 (M+1).

**Steps 3-5** used similar procedures for converting compound **82** to intermediate **13** shown in Scheme 17 to afford intermediate **23** from compound **130".** MS-ESI: 319 (M+1).

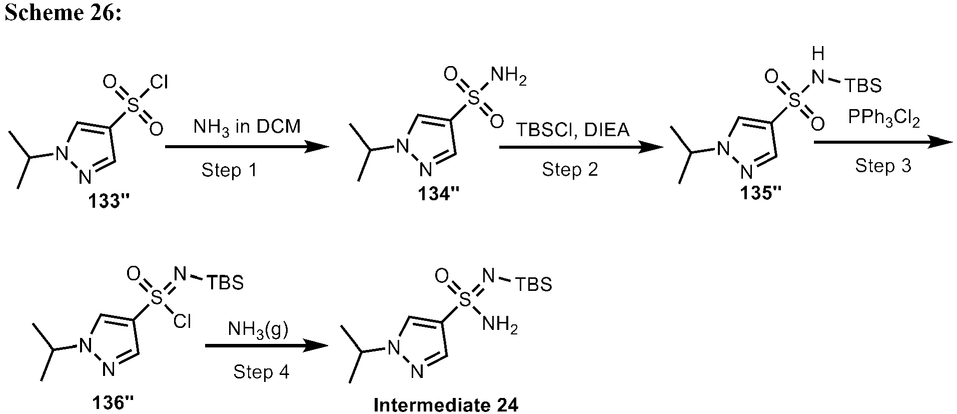

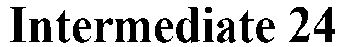

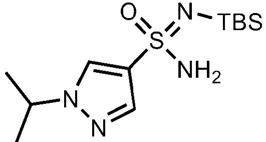

### N'-(tert-butyldimethylsilyl)-1-isopropyl-1H-pyrazole-4-sulfonimidamide

**Steps 1-4** used similar procedures for converting compound **98** to intermediate 16 shown in Scheme 20 to afford intermediate **24** from compound **133".** MS-ESI: 303 (M+1).

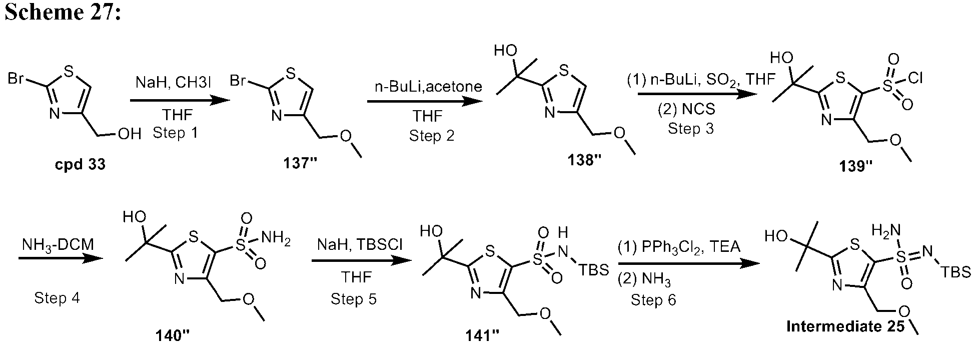

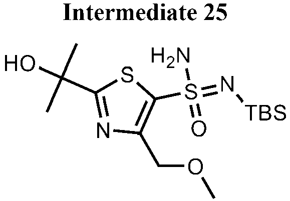

### N'-(tert-butyldimethylsilyl)-2-(2-hydroxypropan-2-yl)-4-(methoxymethyl)thiazole-5-sulfonimidamide

### Step 1: 2-Bromo-4-(methoxymethyl)thiazole

Into a 500-mL round-bottom flask, was placed a solution of (2-bromothiazol-4-yl)methanol (10 g, 51.8 mmol) in THF (200 mL). To a stirred solution was added NaH (60%wt. oil dispersion, 4.15 g, 104 mmol) in three times at 0°C in an ice/ethanol bath. To this reaction solution was added MeI (11 g, 77.7 mmol) dropwise with stirring at 0°C in an ice/ethanol bath. The resulting solution was stirred for 3 h at RT. The reaction was then quenched by the addition of water (50 mL) at 0°C. The resulting solution was extracted with 3x300 mL of EtOAc. The organic layers were combined, dried over anhydrous sodium sulfate, and concentrated under vacuum. This resulted in 8.58 g (80%) of the title compound as a white solid. MS-ESI: 208/210 (M+1).

**Steps 2-6** used similar procedures for converting compound **34** to intermediate **3** shown in Scheme 11 to afford intermediate **25** from compound **137".** MS-ESI: 380 (M+1).

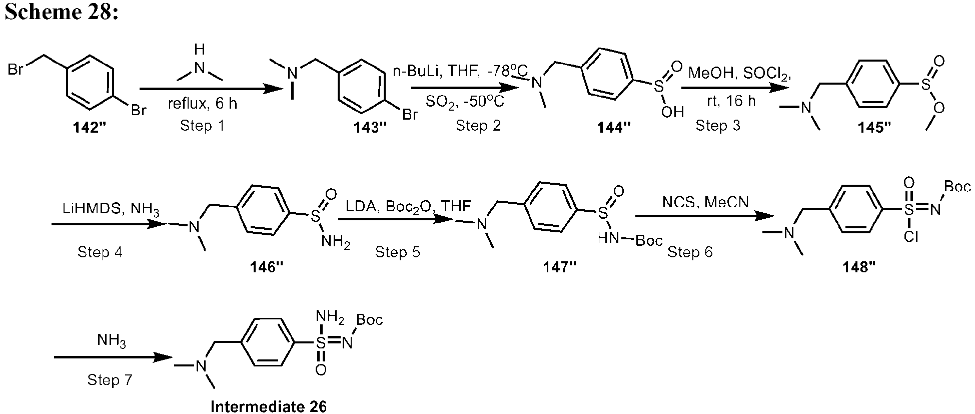

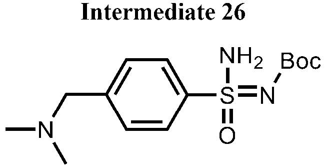

### Tert-butyl (amino(4-((dimethylamino)methyl)phenyl)(oxo)-λ6-sulfaneylidene)carbamate

### Step 1: 1-(4-Bromophenyl)-N,N-dimethylmethanamine

To a solution of 1-bromo-4-(bromomethyl)benzene (125 g, 500 mmol) in DCM (2.5 L) was added EtOH/MeNH₂ (268 mL, 33% in EtOH). The resulting mixture was heated to reflux for 6 h. The reaction was then cooled to room temperature and poured onto sat. NaHCO₃ (1.2 L), before extracting with EtOAc (4x1.0 L). The combined organic phases were dried over Na₂SO₄, filtered, and concentrated under vacuum. The crude product was purified by a silica gel with EtOAc/petrol ether (1:1). This resulted in 83 g (77%) of the title compound as a yellow solid. MS-ESI: 214/216 (M+1).

**Steps 2-7** used similar procedures for converting compound **26** to intermediate **2** shown in Scheme 9 to afford intermediate **26** from compound **143".** MS-ESI: 314 (M+1).

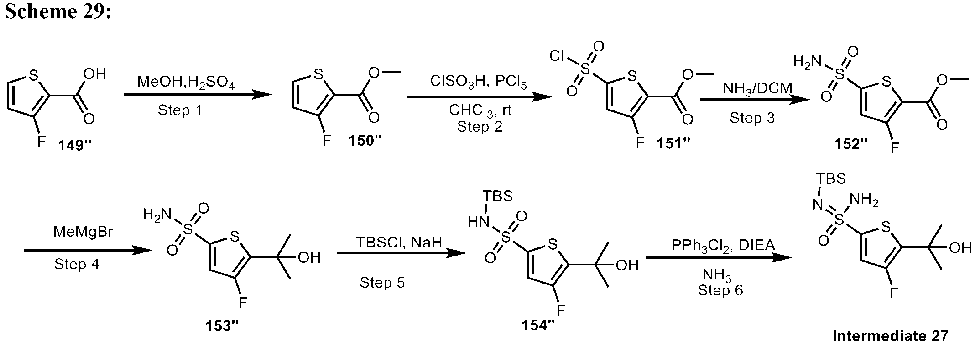

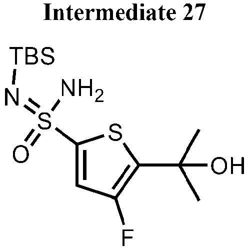

### N'-(tert-butyldimethylsilyl)-4-fluoro-5-(2-hydroxypropan-2-yl)thiophene-2-sulfonimidamide

### Step 1: Methyl 3-fluorothiophene-2-carboxylate

Into a 250-mL round-bottom flask, was placed 3-fluorothiophene-2-carboxylic acid (10 g, 68 mmol) in MeOH (10 mL) and H₂SO₄ (50 mL). The resulting solution was stirred for overnight at 60°C. The reaction was then quenched by the addition of 100 mL of water/ice. The resulting solution was extracted with 2x100 mL of ethyl acetate. The organic layers were combined, dried, and concentrated. This resulted in 9.6 g (87.60%) of methyl 3-fluorothiophene-2-carboxylate as a grey solid. MS-ESI: 161 (M+1).

**Step 2** used similar procedures for converting compound **72** to compound **73** shown in Scheme 16 to compound **151"** from compound **150".**

**Steps 3-6** used similar procedures for converting compound **51** to intermediate **5** shown in Scheme 13B to Intermediate **27** from compound **151".** MS-ESI: 353 (M+1).

**Schemes for phenylacetic acid Intermediates:** Schemes **30-36** illustrate the preparation of amino pyridines intermediates.

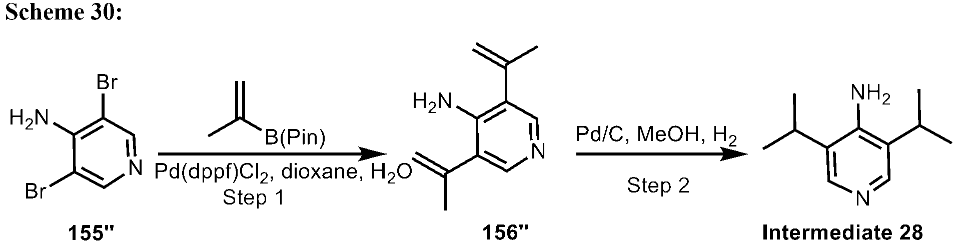

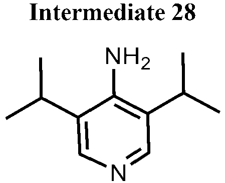

### 3,5-Diisopropylpyridin-4-amine

### Step 1: 3,5-Di(prop-1-en-2-yl)pyridin-4-amine

Into a 500-mL round-bottom flask purged and maintained with an inert atmosphere of nitrogen, was placed 3,5-dibromopyridin-4-amine (5.0 g, 20 mmol) in dioxane (150 mL) and water (15 mL). To the stirred solution was added 4,4,5,5-tetramethyl-2-(prop-1-en-2-yl)-1,3,2-dioxaborolane (10 g, 60 mmol), Cs₂CO₃ (19.6 g, 60 mmol) and Pd(dppf)Cl₂ (1.46 g, 2.0 mmol). The resulting solution was stirred for 15 h at 90°C in an oil bath. The resulting mixture was concentrated under vacuum. The residue was eluted from a silica gel with EtOAc/PE (1:3). This resulted in 3.0 g (87%) of the title compound as light yellow oil. MS-ESI: 175 (M+1).

### Step 2: 3,5-Diisopropylpyridin-4-amine

Into a 250-mL round-bottom flask, was placed 3,5-bis(prop-1-en-2-yl)pyridin-4-amine (3.0 g, 17.2 mmol) in MeOH (50 mL), and Pd/C (wet.10% wt., 300 mg). The flask was evacuated and flushed three times with hydrogen. The resulting solution was stirred for 16 h at RT under an atmosphere of hydrogen. The Pd/C catalysts were filtered out, the filtrate was concentrated under vacuum. This resulted in 2.8 g (91%) of the title compound as a light yellow solid. MS-ESI: 178 (M+1). **Table 6.** The Intermediates in the following Table were prepared using the similar procedures for converting compound **155"** to Intermediate **28** shown in Scheme 30 from appropriated starting materials.

| Intermediate # | Structure | IUPAC Name | Exact Mass[M+H]⁺ |
|---|---|---|---|
| Intermediate 29 | 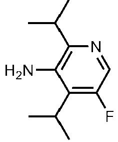 | 5-fluoro-2,4-diisopropylpyridin-3-amine | 197 |
| Intermediate 30 | 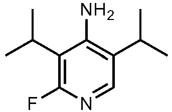 | 2-fluoro-3,5-diisopropylpyridin-4-amine | 197 |
| Intermediate 31 | 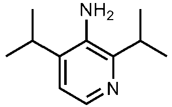 | 2,4-Diisopropylpyridin-3-amine | 179 |

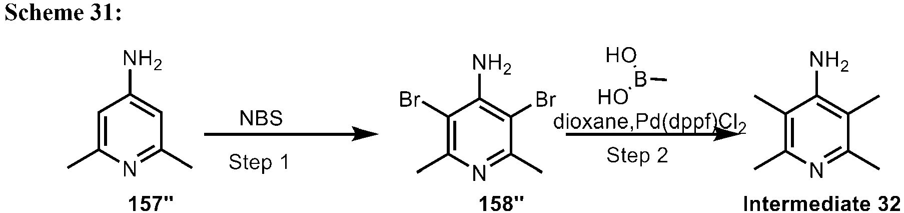

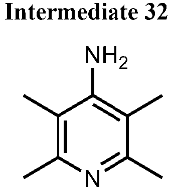

### 2,3,5,6-Tetramethylpyridin-4-amine

### Step 1: 3,5-Dibromo-2,6-dimethylpyridin-4-amine

Into a 250-mL round-bottom flask, was placed 2,6-dimethylpyridin-4-amine (5.0 g, 40.9 mmol) in ACN (100 mL). This was followed by the addition of NBS (14.6 g, 81.9 mmol) in several batches at 0°C. The resulting solution was stirred for 2 h at RT. The resulting mixture was concentrated. The residue was eluted from silica gel with EtOAc/PE (1:4). This resulted in 10.6 g (93%) of the title compound as a yellow solid. MS-ESI: 279/281/283(M+1).

### Step 2: 2,3,5,6-Tetramethylpyridin-4-amine

Into a 100-mL round-bottom flask purged and maintained with an inert atmosphere of nitrogen, was placed 3,5-dibromo-2,6-dimethylpyridin-4-amine (2.0 g, 7.1 mmol) in dioxane (30 mL) and H₂O (6.0 mL). To the above solution was added Cs₂CO₃ (4.66 g, 14.3 mmol), methylboronic acid (941 mg, 15.7 mmol) and Pd(dppf)Cl₂ (523 mg, 0.71 mmol). The resulting solution was stirred for 16 h at 100°C in an oil bath. The solids were filtered out. The resulting mixture was concentrated. The residue was eluted from silica gel with DCM/MeOH (10:1). This resulted in 220 mg (20.5%) of the title compound as a red solid. MS-ESI: 151 (M+1).

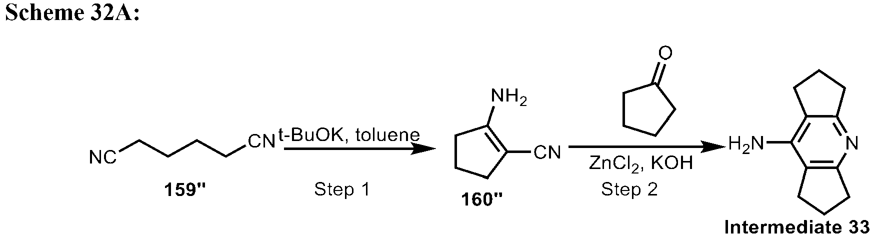

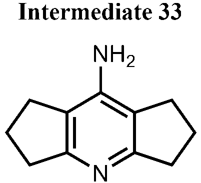

### 1,2,3,5,6,7-Hexahydrodicyclopenta[b,e]pyridin-8-amine

### Step 1: 2-Aminocyclopent-1-ene-1-carbonitrile

Into a 500-mL round-bottom flask purged and maintained with an inert atmosphere of nitrogen, was placed a mixture of adiponitrile (10.8 g, 100 mmol) in toluene (250 mL). The reaction mixture was heated to 65°C, and t-BuOK (112 g, 100 mmol) was added into the solution at 65°C in portions. The resulting solution was stirred for at 80°C for 8 h. The reaction mixture was then cooled to RT and quenched by the addition of 200 mL of water/ice. The solids were collected by filtration. The filter cake was washed with water (100mL) and hexane (200 mL), after which it was dried under an infra-red lamp. This resulted in 9.18 g (85.0%) of the title compound as an off-white solid. MS-ESI: 109 (M+1).

### Step 2: 1,2,3,5,6,7-Hexahydrodicyclopenta[b,e]pyridin-8-amine

Into a 250-mL round-bottom flask purged and maintained with an inert atmosphere of nitrogen, was placed 2-aminocyclopent-1-ene-1-carbonitrile (5.0 g, 46.2 mmol) in xylene (125 mL). To the above solution was added cyclopentanone (7.8 g, 93 mmol) and ZnCl₂ (6.9 g, 51 mmol). The resulting solution was stirred for overnight at 140°C in an oil bath. The resulting solution was diluted with 150 mL of MeOH, after which a solution of KOH (25 mL, 5.0 M) was dropped into it. The solids were filtered out. The resulting mixture was concentrated. The residue was dissolved in 250 mL of EtOAc. The solids were collected by filtration. This resulted in 4.2 g (52%) of the title compound as a brown solid. MS-ESI: 175 (M+1).

**Table 7. The Intermediate 33 and Intermediate 34 in the following Table were separated from the same reaction using the similar procedures for converting compound 160" to Intermediate 33 shown in Scheme 32A from 3-methylcyclopentanone.**

| Intermediate # | Structure | IUPAC Name | Exact Mass[M+H]⁺ |
|---|---|---|---|
| Intermediate 34 | 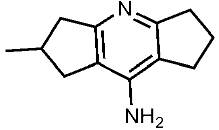 | 2-methyl-1,2,3,5,6,7-hexahydrodicyclopenta[b,e]pyridin-8-amine | 189 |
| Intermediate 35 | 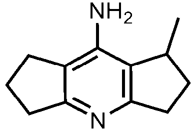 | 1-methyl-1,2,3,5,6,7-hexahydrodicyclopenta[b,e]pyridin-8-amine | 189 |

**Table 8. The Intermediates in the following Table were prepared using the similar procedures for converting compound 160" to Intermediate 33 shown in Scheme 32A from appropriated starting materials.**

| Intermediate # | Structure | IUPAC Name | Exact Mass[M+H]⁺ |
|---|---|---|---|
| Intermediate 36 | 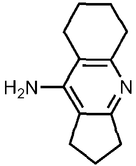 | 2,3,5,6,7,8-hexahydro-1H-cyclopenta[b]quinolin-9-amine | 188 |
| Intermediate 37 | 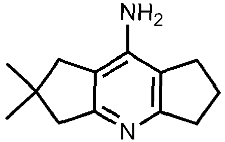 | 2,2-dimethyl-1,2,3,5,6,7-hexahydrodicyclopenta[b,e]pyridin-8-amine | 203 |
| Intermediate 38 | 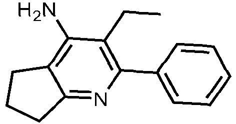 | 3-ethyl-2-phenyl-6,7-dihydro-5H-cyclopenta[b]pyridin-4-amine | 239 |
| Intermediate 39 | 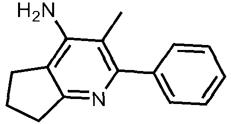 | 3-methyl-2-phenyl-6,7-dihydro-5H-cyclopenta[b]pyridin-4-amine | 225 |
| Intermediate 40 | 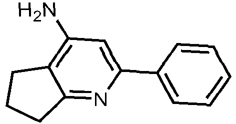 | 2-phenyl-6,7-dihydro-5H-cyclopenta[b]pyridin-4-amine | 211 |
| Intermediate 41 | 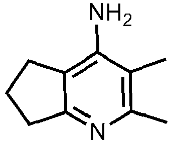 | 2,3-dimethyl-6,7-dihydro-5H-cyclopenta[b]pyridin-4-amine | 163 |
| Intermediate 42 | 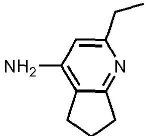 | 2-ethyl-6,7-dihydro-5H-cyclopenta[b]pyridin-4-amine | 163 |
| Intermediate 43 | 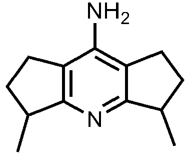 | 3,5-dimethyl-1,2,3,5,6,7-hexahydrodicyclopenta[b,e]pyridin-8-amine | 203 |
| Intermediate 44 | 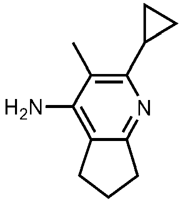 | 2-cyclopropyl-3-methyl-6,7-dihydro-5H-cyclopenta[b]pyridin-4-amine | 189 |

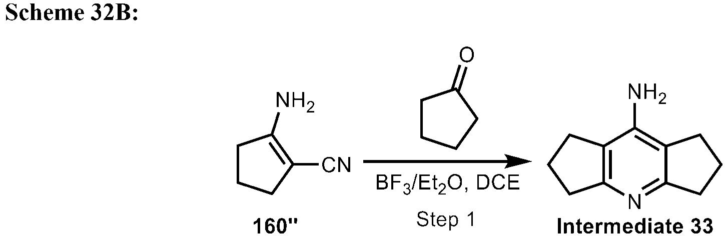

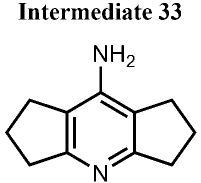

### 1,2,3,5,6,7-Hexahydrodicyclopenta[b,e]pyridin-8-amine

Into a 250-mL 3-necked round-bottom flask purged and maintained with an inert atmosphere of nitrogen, was placed a mixture of 2-aminocyclopent-1-enecarbonitrile (5.4 g, 50 mmol) in DCE (125 mL). To this solution was added cyclopentanone (8.4 g, 100 mmol). Then BF₃.Et₂O (46.5%wt., 14.5 g) was added to this solution at 0°C in an ice bath. The reaction was heated to 75°C for 6 h. The reaction cooled to RT, after which it was quenched by the addition of 100 mL of water/ice and extracted with DCM (2x50 mL). The aqueous phase was collected. The pH value was adjusted to 14 with NaOH (6 M) until the solid was precipitated. The solids were collected by filtration. The filter cake was washed with water (150 mL) then dried by infra-red drying, this resulted of the title compound (7.0 g, yield 80%, light yellow solid). MS-ESI: 175 (M+1).

**Table 9. The Intermediates in the following Table were prepared using the similar procedures for converting compound 160" to Intermediate 33 shown in Scheme 32B from appropriated starting materials.**

| Intermediate # | Structure | IUPAC Name | Exact Mass[M+H]⁺ |
|---|---|---|---|
| Intermediate 45 | 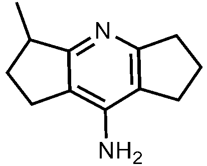 | 3-methyl-1,2,3,5,6,7-hexahydrodicyclopenta[b,e]pyridin-8-amine | 189 |
| Intermediate 46 | 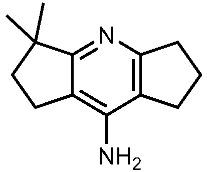 | 3,3-dimethyl-1,2,3,5,6,7-hexahydrodicyclopenta[b,e]pyridin-8-amine | 203 |
| Intermediate 47 | 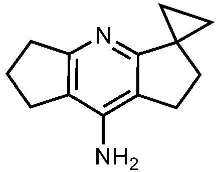 | 1',5',6',7'-tetrahydro-2'H-spiro[cyclopropane-1,3'-dicyclopenta[b,e]pyridin]-8'-amine | 201 |
| Intermediate 48 | 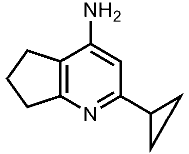 | 2-cyclopropyl-6,7-dihydro-5H-cyclopenta[b]pyridin-4-amine | 175 |
| Intermediate 49 | 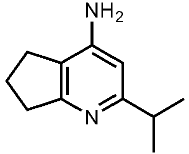 | 2-isopropyl-6,7-dihydro-5H-cyclopenta[b]pyridin-4-amine | 177 |

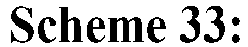

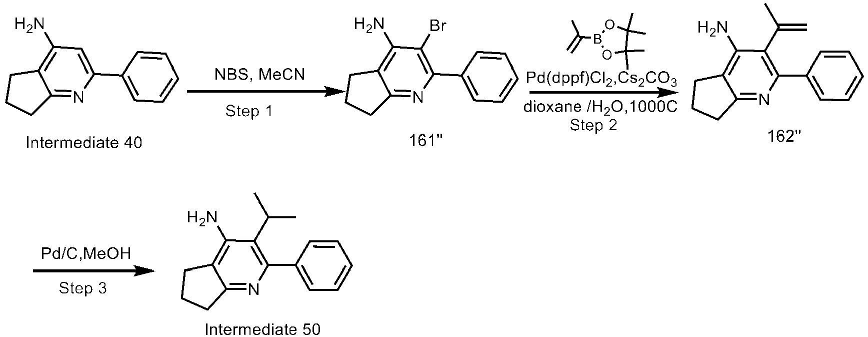

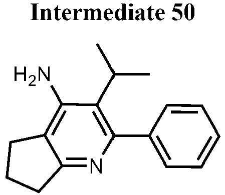

### 3-Isopropyl-2-phenyl-6,7-dihydro-5H-cyclopenta[b]pyridin-4-amine

### Step 1: 3-Bromo-2-phenyl-6,7-dihydro-5H-cyclopenta[b]pyridin-4-amine

Into a 100-mL round-bottom flask, was placed 2-phenyl-5H,6H,7H-cyclopenta[b]pyridin-4-amine (1.36 g, 6.47 mmol) in ACN (20 mL). To the stirred solution was added NBS (1.38 g, 7.76 mmol). The resulting solution was stirred for 2 h at RT. The resulting mixture was concentrated. The residue was eluted from a silica gel with EtOAc/PE (1:2). This resulted in 680 mg (36.4%) of the title compound as a yellow solid. MS-ESI: 289/291 (M+1).

### Step 2: 2-Phenyl-3-(prop-1-en-2-yl)-6,7-dihydro-5H-cyclopenta[b]pyridin-4-amine

Into a 100-mL round-bottom flask purged and maintained with an inert atmosphere of nitrogen, was placed 3-bromo-2-phenyl-6,7-dihydro-5H-cyclopenta[b]pyridin-4-amine (680 mg, 2.35 mmol) in dioxane (15 mL) and H₂O (3 mL). To the stirred solution was added Cs₂CO₃ (1.53 g, 4.7 mmol), 4,4,5,5-tetramethyl-2-(prop-1-en-2-yl)-1,3,2-dioxaborolane (474 mg, 2.8mmol), and Pd(dppf)Cl₂ (86 mg, 0.12 mmol). The resulting solution was stirred for 16 h at 100°C in an oil bath. The resulting mixture was concentrated. The residue was eluted from a silica gel with EtOAc/PE (1:2). This resulted in 350 mg (59%) of the title compound as a light yellow solid. MS-ESI: 251 (M+1).

### Step 3: 3-Isopropyl-2-phenyl-6,7-dihydro-5H-cyclopenta[b]pyridin-4-amine

Into a 100-mL round-bottom flask purged and maintained with an inert atmosphere of H₂, was placed 2-phenyl-3-(prop-1-en-2-yl)-6,7-dihydro-5H-cyclopenta[b]pyridin-4-amine (350 mg, 1.4 mmol) in MeOH (15 mL). To the stirred solution was added Pd/C (wet 10%wt., 50 mg, 0.47 mmol). The flask was evacuated and flushed three times with hydrogen. The resulting solution was stirred for 16 h at 50°C under an atmosphere of hydrogen. The Pd/C catalysts were filtered out, the filtrate was concentrated under vacuum. This resulted in 250 mg (71%) of the title compound as a yellow solid. MS-ESI: 253 (M+1).

**Table 10. The Intermediates in the following Table were prepared using the similar procedures for converting intermediate 40 to Intermediate 50 shown in Scheme 33 from appropriated starting materials.**

| Intermediate # | Structure | IUPAC Name | Exact Mass[M+H]⁺ |
|---|---|---|---|
| Intermediate 51 | 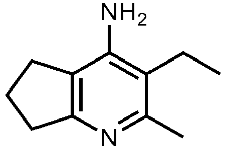 | 3-ethyl-2-methyl-6,7-dihydro-5H-cyclopenta[b]pyridin-4-amine | 177 |
| Intermediate 52 | 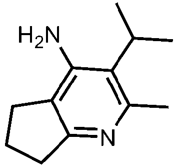 | 3-isopropyl-2-methyl-6,7-dihydro-5H-cyclopenta[b]pyridin-4-amine | 191 |
| Intermediate 53 | 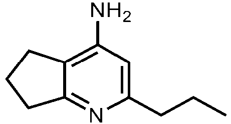 | 2-propyl-6,7-dihydro-5H-cyclopenta[b]pyridin-4-amine | 177 |
| Intermediate 54 | 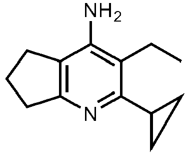 | 2-cyclopropyl-3-ethyl-6,7-dihydro-5H-cyclopenta[b]pyridin-4-amine | 203 |

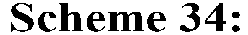

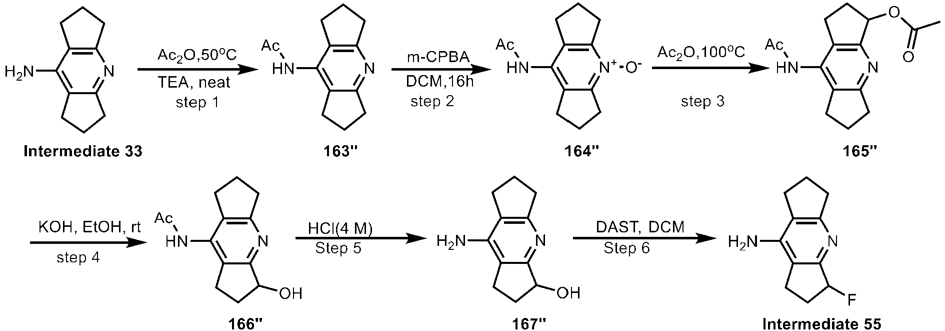

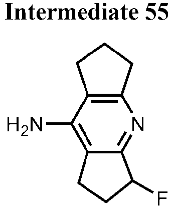

### 3-Fluoro-1,2,3,5,6,7-hexahydrodicyclopenta[b,e]pyridin-8-amine

### Step 1: N-(1,2,3,5,6,7-hexahydrodicyclopenta[b,e]pyridin-8-yl)acetamide

Into a 2-L 3-necked round-bottom flask, was placed a solution of 1,2,3,5,6,7-hexahydrodicyclopenta[b,e]pyridin-8-amine (35 g, 201 mmol) in Ac₂O (805 mL). This was followed by the addition of triethylamine (61 g, 603 mmol) dropwise with stirring at 0°C in 5 min. The resulting solution was stirred for overnight at 100°C in an oil bath. The resulting mixture was concentrated under vacuum. The pH value of the solution was adjusted to 14 with aq. KOH (20 M). The solids were collected by filtration. This resulted in 31 g (71%) of the title compound as a brown solid. MS-ESI: 217 (M+1).

### Step 2: 8-Acetamido-1,2,3,5,6,7-hexahydrodicyclopenta[b,e]pyridine 4-oxide

Into a 1-L round-bottom flask purged and maintained with an inert atmosphere of nitrogen, was placed a solution of N-(1,2,3,5,6,7-hexahydrodicyclopenta[b,e]pyridin-8-yl)acetamide (31.4 g, 145 mmol) in DCM (250 mL). This was followed by the addition of m-CPBA (75 g, 436 mmol) dropwise with stirring at 0°C in 5 min. The resulting solution was stirred for overnight at RT. The resulting mixture was concentrated. The residue was eluted from silica gel with DCM/MeOH (24: 1). This resulted in 14 g (42%) of the title compound as a white solid. MS-ESI: 233(M+1).

### Step 3: 8-Acetamido-1,2,3,5,6,7-hexahydrodicyclopenta[b,e]pyridin-3-yl acetate

Into a 250-mL round-bottom flask, was placed 8-acetamido-1,2,3,5,6,7-hexahydrodicyclopenta[b,e]pyridine 4-oxide (14.9 g, 64 mmol) in Ac₂O (80 mL). The resulting solution was stirred for 1 h at 100°C in an oil bath. The resulting mixture was concentrated. This resulted in 16 g crude title compound as a black crude solid. MS-ESI: 275 (M+1).

### Step 4: N-(3-hydroxy-1,2,3,5,6,7-hexahydrodicyclopenta[b,e]pyridin-8-yl)acetamide

Into a 250-mL round-bottom flask, was placed a solution of 8-acetamido-1,2,3,5,6,7-hexahydrodicyclopenta[b,e]pyridin-3-yl acetate (4.5 g, 16.4 mmol) in ethanol (100 mL), to the above solution was added KOH (550 mg, 9.84 mmol) with stirring. The resulting solution was stirred for overnight at RT. The resulting mixture was concentrated. The residue was eluted from silica gel with DCM/MeOH (26:1). This resulted in 2.1 g (55%) of the title compound as a light yellow solid. MS-ESI: 233 (M+1).

### Step 5: 8-Amino-1,2,3,5,6,7-hexahydrodicyclopenta[b,e]pyridin-3-ol

Into an 8-mL sealed tube, was placed a solution of N-(3-hydroxy-1,2,3,5,6,7-hexahydrodicyclopenta[b,e]pyridin-8-yl)acetamide (200 mg, 0.86 mmol) in HCl (4 M, 3 mL). The resulting solution was stirred for 40 min at 100°C. The pH value of the solution was adjusted to 7 with NaOH (1 M). The resulting mixture was concentrated. The residue was applied onto a silica gel with DCM/MeOH (3:1). This resulted in 80 mg (49%) of the title compound as a yellow solid. MS-ESI: 191 (M+1).

### Step 6: 3-Fluoro-1,2,3,5,6,7-hexahydrodicyclopenta[b,e]pyridin-8-amine

Into an 8-mL sealed tube, was placed a solution of 8-amino-1,2,3,5,6,7-hexahydrodicyclopenta[b,e]pyridin-3-ol (200 mg, 1.05 mmol) in DCM (2 mL). To the solution was added DAST (254 mg, 1.58 mmol) at 0°C. The resulting solution was stirred for 2 h at RT. The residue was eluted from a silica gel with DCM/MeOH (8:1). This resulted in 85 mg (42%) of the title compound as a yellow solid. MS-ESI: 193 (M+1).

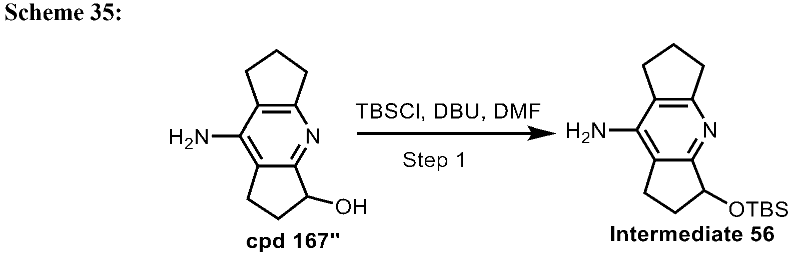

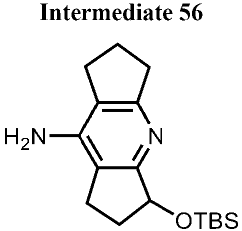

### 3-((Tert-butyldimethylsilyl)oxy)-1,2,3,5,6,7-hexahydrodicyclopenta[b,e]pyridin-8-amine

Into a 100-mL round-bottom flask, was placed 8-amino-1,2,3,5,6,7-hexahydrodicyclopenta[b,e]pyridin-3-ol (1.0 g, 5.26 mmol) in DMF (10 mL, 129 mmol). To the stirred solution was added DBU (1.6 g, 11 mmol) and TBSCl (1.58 g, 11 mmol). The resulting solution was stirred for 4 h at RT. The reaction was then quenched by the addition of 1 mL of water. The resulting solution was extracted with 3x15 mL of EtOAc. The organic layers were combined, dried over anhydrous sodium sulfate, and concentrated. The residue was eluted from silica gel with EtOAc/PE (41%). This resulted in 1.07 g (67%) of the title compound as a white solid.

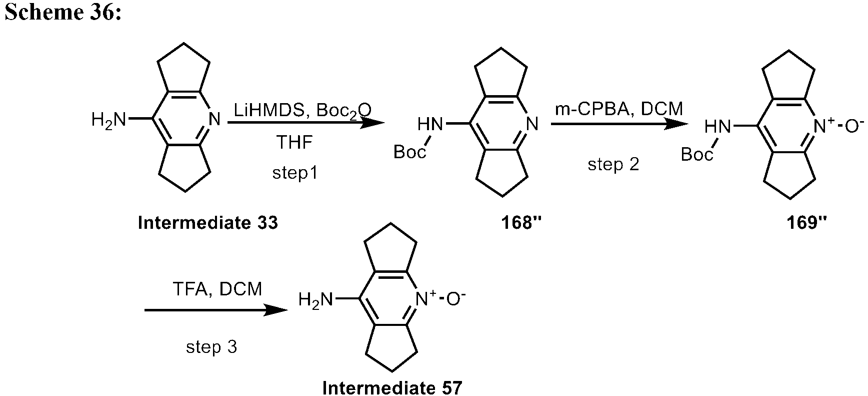

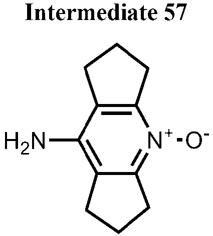

### 8-Amino-1,2,3,5,6,7-hexahydrodicyclopenta[b,e]pyridine 4-oxide

### Step 1: Tert-butyl (1,2,3,5,6,7-hexahydrodicyclopenta[b,e]pyridin-8-yl)carbamate

Into a 50-mL round-bottom flask purged and maintained with an inert atmosphere of nitrogen, was placed 1,2,3,5,6,7-hexahydrodicyclopenta[b,e]pyridin-8-amine (500 mg, 2.87 mmol) in THF (3 mL). This was followed by the addition of LiHMDS (1 M in THF, 5.7 mL, 5.7 mmol) dropwise with stirring at 80°C. The mixture was stirred for an additional 1 h. To this was added (Boc)₂O (1.88 g, 8.61 mmol) at 80°C. The resulting solution was stirred for 16 h at 80°C in an oil bath. The reaction was then quenched by the addition of water. The resulting solution was extracted with 3x30 mL of EtOAc and concentrated. The residue was eluted from a silica gel with DCM/MeOH (15:1). This resulted in 300 mg (38%) of the title compound as a dark yellow solid. MS-ESI: 275(M+1).

### Step 2: 8-((Tert-butoxycarbonyl)amino)-1,2,3,5,6,7-hexahydrodicyclopenta[b,e]pyridine 4-oxide

Into a 50-mL round-bottom flask purged and maintained with an inert atmosphere of nitrogen, was placed tert-butyl (1,2,3,5,6,7-hexahydrodicyclopenta[b,e]pyridin-8-yl)carbamate (900 mg, 3.28 mmol) in DCM (15 mL). To the above solution was added m-CPBA (1.13 g, 6.56 mmol). The resulting solution was stirred for 16 h at RT. The reaction was then quenched by the addition of water. The resulting solution was extracted with 3x30 mL of EtOAc and concentrated. The residue was eluted from a silica gel with DCM/MeOH (15:1). This resulted in 750 mg (78.7%) of the title compound as a dark yellow solid. MS-ESI: 291(M+1).

### Step 3: 8-Amino-1,2,3,5,6,7-hexahydrodicyclopenta[b,e]pyridine 4-oxide

Into a 50-mL round-bottom flask purged and maintained with an inert atmosphere of nitrogen, was placed 8-((tert-butoxycarbonyl)amino)-1,2,3,5,6,7-hexahydrodicyclopenta[b,e]pyridine 4-oxide (550 mg, 1.89 mmol) in DCM (8.0 mL) and TFA (4.0 mL). The resulting solution was stirred for 20 min at RT. The pH value of the solution was adjusted to 7 with Na₂CO₃. The resulting mixture was concentrated. The residue was eluted from silica gel with DCM/MeOH (8:1). This resulted in 184 mg (51%) of the title compound as a yellow solid. MS-ESI: 191(M+1).

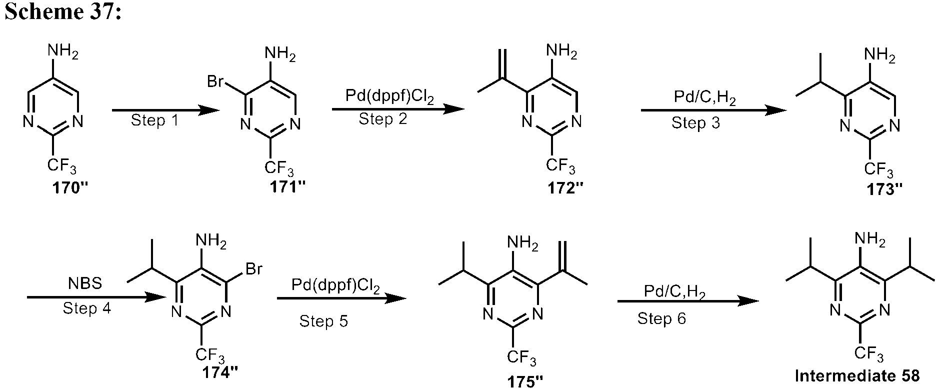

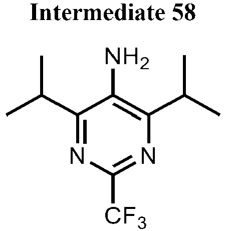

### 4,6-diisopropyl-2-(trifluoromethyl)pyrimidin-5-amine

### Step 1: 4-Bromo-2-(trifluoromethyl)pyrimidin-5-amine

Into a 100-mL round-bottom flask purged and maintained with an inert atmosphere of nitrogen, was placed 2-(trifluoromethyl)pyrimidin-5-amine (2.0 g, 12.26 mmol) in acetonitrile (20 mL). To this stirred solution was added NBS (2.62 g, 14.7 mmol). The resulting solution was stirred for 12 h at RT. The resulting solution was diluted with 40 mL of water, extracted with 2x30 mL of DCM, and concentrated. The residue was eluted from silica gel with EtOAc/PE (1:50 to 1:20). This resulted in 1.6 g (54%) of the title compound as a brown solid. MS-ESI: 242/244 [M+1]

### Step 2: 4-(Prop-1-en-2-yl)-2-(trifluoromethyl)pyrimidin-5-amine

Into a 100-mL round-bottom flask purged and maintained with an inert atmosphere of nitrogen, was placed 4-bromo-2-(trifluoromethyl)pyrimidin-5-amine (1.6 g, 6.61 mmol) in dioxane (20 mL). This was followed by the addition of 4,4,5,5-tetramethyl-2-(prop-1-en-2-yl)-1,3,2-dioxaborolane (1.44 g, 8.57 mmol), Pd(dppf)Cl₂ (242 mg, 0.33 mmol), and Cs₂CO₃ (3.23 g, 9.92 mmol). The resulting solution was stirred for 14 h at 100°C in an oil bath. The resulting solution was diluted with 40 mL of water. The resulting solution was extracted with 3x30 mL of DCM and concentrated. The residue was eluted from silica gel with EtOAc/PE (1:5). This resulted in 1.1 g (82%) of the title compound as a brown solid. MS-ESI: 204 [M+1].

### Step 3: 4-Isopropyl-2-(trifluoromethyl)pyrimidin-5-amine

Into a 100-mL round-bottom flask purged and maintained with an inert atmosphere of nitrogen, was placed 4-(prop-1-en-2-yl)-2-(trifluoromethyl)pyrimidin-5-amine (1.2 g, 5.91 mmol) in MeOH (20 mL), to the stirred solution was added Pd/C (10%wt., 200 mg). The flask was evacuated and filled three times with hydrogen. The resulting solution was stirred 16h at RT under hydrogen. The solids were filtered out. The resulting mixture was concentrated under vacuum. This resulted in 1.1 g (91%) of the title compound as a brown solid. MS-ESI: 206 [M+1].

### Step 4: 4-Bromo-6-isopropyl-2-(trifluoromethyl)pyrimidin-5-amine

Into a 50-mL round-bottom flask purged and maintained with an inert atmosphere of nitrogen, was placed 4-(propan-2-yl)-2-(trifluoromethyl)pyrimidin-5-amine (1.1 g, 5.36 mmol) in acetonitrile (20 mL), to this solution was added NBS (1.15 g, 6.46 mmol) in portions with stirring. The resulting solution was stirred for 12 h at RT. The resulting solution was diluted with 40 mL of water. The resulting solution was extracted with 2x30 mL of DCM and concentrated. The residue was eluted from silica gel with EtOAc/PE (1:40 to 1:30). This resulted in 1.0 g (66%) of the title compound as a brown solid. MS-ESI: 284/286 [M+1].

### Step 5: 4-Isopropyl-6-(prop-1-en-2-yl)-2-(trifluoromethyl)pyrimidin-5-amine

Into a 100-mL round-bottom flask purged and maintained with an inert atmosphere of nitrogen, was placed 4-bromo-6-(propan-2-yl)-2-(trifluoromethyl)pyrimidin-5-amine (1.2 g, 4.2 mmol) in dioxane (20 mL) and H₂O (4 mL). To the stirred solution was added 4,4,5,5-tetramethyl-2-(prop-1-en-2-yl)-1,3,2-dioxaborolane (0.92 g, 5.5 mmol), Pd(dppf)Cl₂ (155 mg, 0.21 mmol) and Cs₂CO₃ (2.06 g, 6.3 mmol). The resulting solution was stirred for 16 h at 100°C in an oil bath. The resulting mixture was concentrated. The residue was applied onto a silica gel with EtOAc/PE (1:3). This resulted in 870 mg (84%) of the title compound as a yellow solid.

### Step 6: 4,6-Diisopropyl-2-(trifluoromethyl)pyrimidin-5-amine

Into a 50-mL round-bottom flask purged and maintained with an inert atmosphere of H₂, was placed 4-isopropyl-6-(prop-1-en-2-yl)-2-(trifluoromethyl)pyrimidin-5-amine (870 mg, 3.5 mmol) in MeOH (20 mL). To the stirred solution was added Pd/C (10%wt., 125 mg). The flask was evacuated and filled three times with hydrogen. The resulting solution was stirred 16 h at RT under hydrogen. The solids were filtered out. The resulting mixture was concentrated under vacuum. This resulted in 830 mg (99.2%) of the title compound as yellow oil.

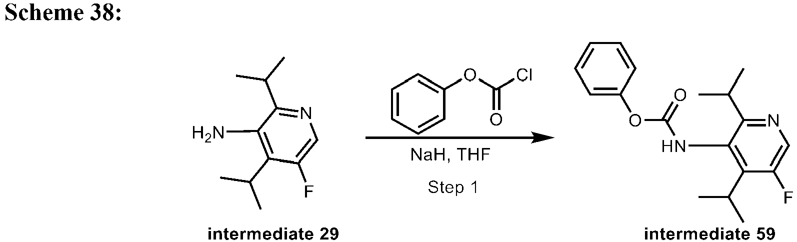

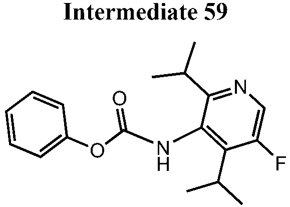

### Phenyl 5-fluoro-2,4-diisopropylpyridin-3-ylcarbamate

Into a 50-mL round-bottom flask purged and maintained with an inert atmosphere of nitrogen, was placed 5-fluoro-2,4-diisopropylpyridin-3-amine (200 mg, 1.02 mmol) in THF (10 mL). To the stirred solution was added NaH (60%wt. oil dispersion, 122 mg, 3.06 mmol) at 0°C. The resulting solution was stirred for 10 min at RT. Then phenyl chloroformate (160 mg, 1.02 mmol) was added at 0°C. The resulting solution was allowed to react, with stirring, for an additional 20 h at RT. The reaction mixture was used in the next step directly without further purification.

**Table 11. The Intermediates in the following Table were prepared using the similar procedures for converting intermediate 29 to Intermediate 59 shown in Scheme 38 from appropriated starting materials.**

| Intermediate # | Structure | IUPAC Name | Quenched with MeOH, Exact Mass[M+H]⁺ |
|---|---|---|---|
| Intermediate 60 | 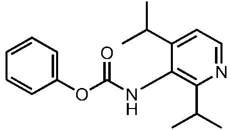 | Phenyl 2,4-diisopropylpyridin-3-ylcarbamate | 237 |
| Intermediate 61 | 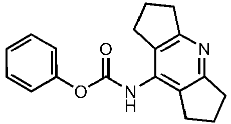 | Phenyl (1,2,3,5,6,7-hexahydrodicyclopenta[b,e]pyridin-8-yl)carbamate | 233 |

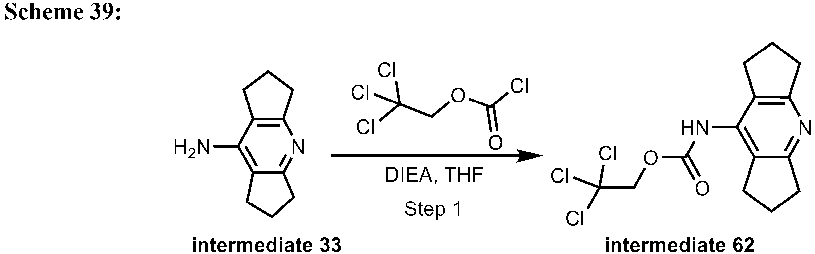

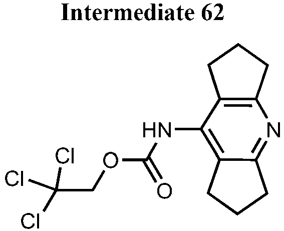

### 2,2,2-Trichloroethyl (1,2,3,5,6,7-hexahydrodicyclopenta[b,e]pyridin-8-yl)carbamate

Into a 1-L round-bottom flask purged and maintained with an inert atmosphere of nitrogen, was placed 1,2,3,5,6,7-hexahydrodicyclopenta[b,e]pyridin-8-amine (6.7 g, 38 mmol) in THF (500 mL). To the above solution was added DIEA (9.92 g, 76.9 mmol) dropwise at RT. Then 2,2,2-trichloroethyl chloroformate (16 g, 76.9 mmol) was dropped into the reaction solution at 0°C. The resulting solution was stirred for 16 h at RT. The resulting mixture was concentrated. The residue was eluted from silica gel with EtOAc/hexane (1:4). This resulted in 7.3 g (54.4%) of the title compound as a yellow solid. MS-ESI: 349/351 (M+1).

**Table 12. The Intermediates in the following Table were prepared using the similar procedures for converting intermediate 33 to Intermediate 62 shown in Scheme 39 from appropriated starting materials.**

| Intermediate # | Structure | IUPAC Name | Exact Mass[M+H]⁺ |
|---|---|---|---|
| Intermediate 63 | 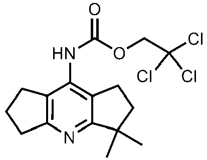 | 2,2,2-trichloroethyl (3,3-dimethyl-1,2,3,5,6,7-hexahydrodicyclopenta[b,e]pyridin-8-yl)carbamate | 377/379 |
| Intermediate 64 | 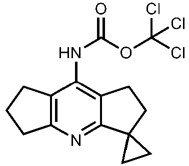 | trichloromethyl (1',5',6',7'-tetrahydro-2'H-spiro[cyclopropane-1,3'-dicyclopenta[b,e]pyridin]-8'-yl)carbamate | 361/363 |
| Intermediate 65 | 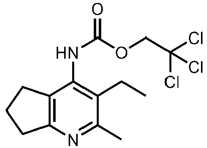 | 2,2,2-trichloroethyl (3-ethyl-2-methyl-6,7-dihydro-5H-cyclopenta[b]pyridin-4-yl)carbamate | 351/353 |
| Intermediate 66 | 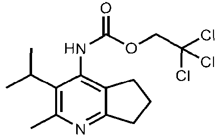 | 2,2,2-trichloroethyl (3-isopropyl-2-methyl-6,7-dihydro-5H-cyclopenta[b]pyridin-4-yl)carbamate | 365/367 |
| Intermediate 67 | 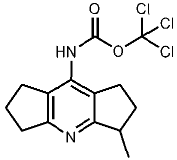 | trichloromethyl (3-methyl-1,2,3,5,6,7-hexahydrodicyclopenta[b,e]pyridin-8-yl)carbamate | 349/351 |
| Intermediate 68 | 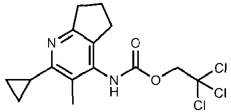 | 2,2,2-trichloroethyl (2-cyclopropyl-3-methyl-6,7-dihydro-5H-cyclopenta[b]pyridin-4-yl)carbamate | 363/365 |
| Intermediate 69 | 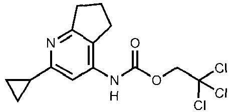 | 2,2,2-trichloroethyl (2-cyclopropyl-6,7-dihydro-5H-cyclopenta[b]pyridin-4-yl)carbamate | 349/351 |
| Intermediate 70 | 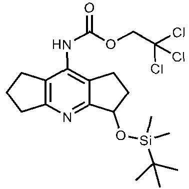 | 2,2,2-trichloroethyl (3-((tert-butyldimethylsilyl)oxy)-1,2,3,5,6,7-hexahydrodicyclopenta[b,e]pyridin-8-yl)carbamate | 479/481 |
| Intermediate 71 | 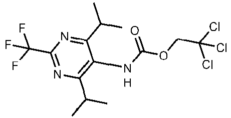 | 2,2,2-trichloroethyl (4,6-diisopropyl-2-(trifluoromethyl)pyrimidin-5-yl)carbamate | 422/424 |
| Intermediate 72 | 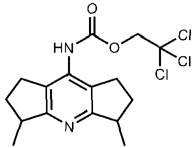 | 2,2,2-trichloroethyl (3,5-dimethyl-1,2,3,5,6,7-hexahydrodicyclopenta[b,e]pyridin-8-yl)carbamate | 377/379 |
| Intermediate 73 | 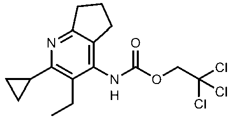 | 2,2,2-trichloroethyl (2-cyclopropyl-3-ethyl-6,7-dihydro-5H-cyclopenta[b]pyridin-4-yl)carbamate | 377/379 |
| Intermediate 74 | 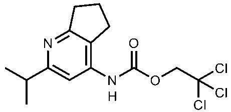 | 2,2,2-trichloroethyl (2-isopropyl-6,7-dihydro-5H-cyclopenta[b]pyridin-4-yl)carbamate | 351/353 |
| Intermediate 75 | 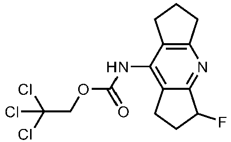 | 2,2,2-trichloroethyl (3-fluoro-1,2,3,5,6,7-hexahydrodicyclopenta[b,e]pyridin-8-yl)carbamate | 367/369 |

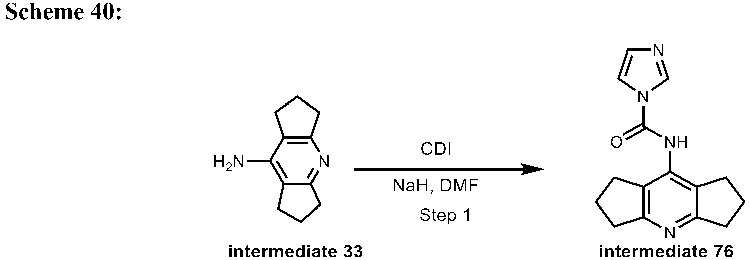

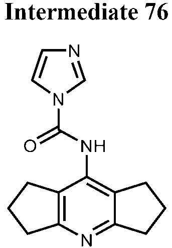

### N-(1,2,3,5,6,7-hexahydrodicyclopenta[b,e]pyridin-8-yl)-1H-imidazole-1-carboxamide

Into a 50-mL 3-necked round-bottom flask purged and maintained with an inert atmosphere of nitrogen, was placed 1,2,3,5,6,7-hexahydrodicyclopenta[b,e]pyridin-8-amine (2.0 g, 11.5 mmol) in DMF (22 mL). To the stirred solution was added NaH (60%wt. oil dispersion, 1.38 g, 34.5 mmol) at 0 °C. The resulting solution was stirred for 30 min at RT. Then CDI (2.79 g, 17.3 mmol) was added to the solution. The resulting solution was stirred for 1 h at RT. The crude product was used directly without work-up.

**Table 13. The Intermediates in the following Table were prepared using the similar procedures for converting intermediate 33 to Intermediate 76 shown in Scheme 40 from appropriated starting materials.**

| Intermediate # | Structure | IUPAC Name | Quenched with MeOH, Exact Mass[M+H]⁺ |
|---|---|---|---|
| Intermediate 77 | 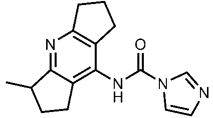 | N-(3-methyl-1,2,3,5,6,7-hexahydrodicyclopenta[b,e]pyridin-8-yl)-1H-imidazole-1-carboxamide | 265 |
| Intermediate 78 | 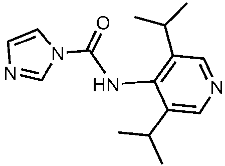 | N-(3,5-diisopropylpyridin-4-yl)-1H-imidazole-1-carboxamide | 237 |
| Intermediate 79 | 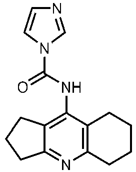 | N-(2,3,5,6,7,8-hexahydro-1H-cyclopenta[b]quinolin-9-yl)-1H-imidazole-1-carboxamide | 247 |
| Intermediate 80 | 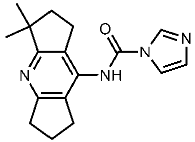 | N-(3,3-dimethyl-1,2,3,5,6,7-hexahydrodicyclopenta[b,e]pyridin-8-yl)-1H-imidazole-1-carboxamide | 261 |
| Intermediate 81 | 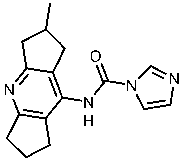 | N-(2-methyl-1,2,3,5,6,7-hexahydrodicyclopenta[b,e]pyridin-8-yl)-1H-imidazole-1-carboxamide | 247 |
| Intermediate 82 | 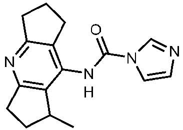 | N-(1-methyl-1,2,3,5,6,7-hexahydrodicyclopenta[b,e]pyridin-8-yl)-1H-imidazole-1-carboxamide | 247 |
| Intermediate 83 | 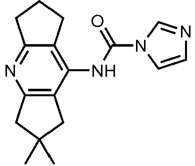 | N-(2,2-dimethyl-1,2,3,5,6,7-hexahydrodicyclopenta[b,e]pyridin-8-yl)-1H-imidazole-1-carboxamide | 261 |
| Intermediate 84 | 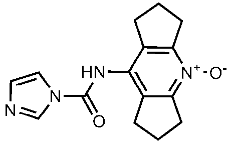 | 8-(1H-imidazole-1-carboxamido)-1,2,3,5,6,7-hexahydrodicyclopenta[b,e]pyridine 4-oxide | 249 |
| Intermediate 85 | 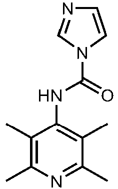 | N-(2,3,5,6-tetramethylpyridin-4-yl)-1H-imidazole-1-carboxamide | 209 |
| Intermediate 86 | 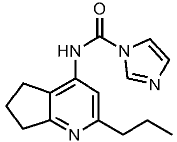 | N-(2-propyl-6,7-dihydro-5H-cyclopenta[b]pyridin-4-yl)-1H-imidazole-1-carboxamide | 235 |

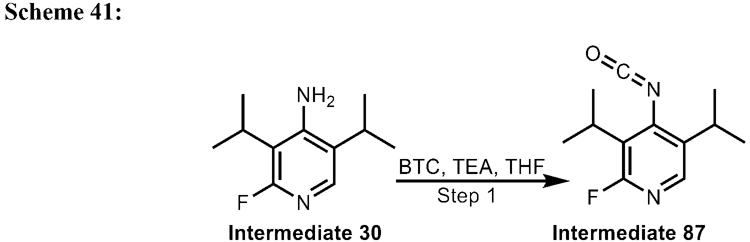

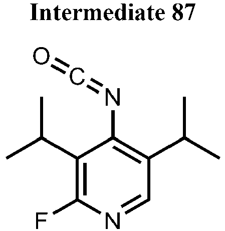

### 2-Fluoro-4-isocyanato-3,5-diisopropylpyridine

Into a 50-mL round-bottom flask purged and maintained with an inert atmosphere of nitrogen, was placed 2-fluoro-3,5-diisopropylpyridin-4-amine (100 mg, 0.51 mmol) in THF (20 mL). To the stirred solution was added TEA (155 mg, 1.53 mmol) and BTC (75 mg, 0.26 mmol). The resulting solution was stirred for 2 h at 60°C in an oil bath. The resulting mixture was concentrated. This resulted in 150 mg crude title product as a grey solid.

**Table 14. The Intermediates in the following Table were prepared using the similar procedures for converting intermediate 30 to Intermediate 87 shown in Scheme 41 from appropriated starting materials.**

| Intermediate # | Structure | IUPAC Name | Quenched with MeOH, Exact Mass[M+H]⁺ |
|---|---|---|---|
| Intermediate 88 | 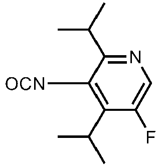 | 5-fluoro-3-isocyanato-2,4-diisopropylpyridine | 255 |
| Intermediate 89 | 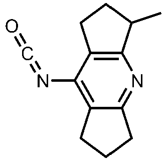 | 8-isocyanato-3-methyl-1,2,3,5,6,7-hexahydrodicyclopenta[b,e]pyridine | 247 |
| Intermediate 90 | 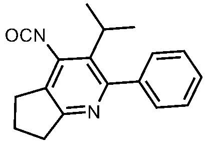 | 4-isocyanato-3-isopropyl-2-phenyl-6,7-dihydro-5H-cyclopenta[b]pyridine | 311 |
| Intermediate 91 | 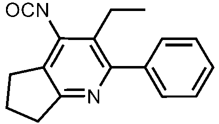 | 3-ethyl-4-isocyanato-2-phenyl-6,7-dihydro-5H-cyclopenta[b]pyridine | 297 |
| Intermediate 92 | 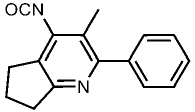 | 4-isocyanato-3-methyl-2-phenyl-6,7-dihydro-5H-cyclopenta[b]pyridine | 283 |
| Intermediate 93 | 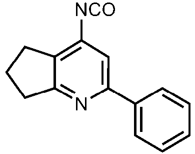 | 4-isocyanato-2-phenyl-6,7-dihydro-5H-cyclopenta[b]pyridine | 269 |
| Intermediate 94 | 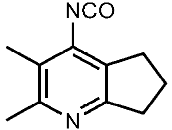 | 4-isocyanato-2,3-dimethyl-6,7-dihydro-5H-cyclopenta[b]pyridine | 221 |
| Intermediate 95 | 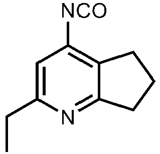 | 2-ethyl-4-isocyanato-6,7-dihydro-5H-cyclopenta[b]pyridine | 221 |
| Intermediate 96 | 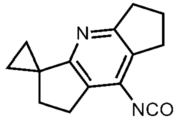 | 8'-isocyanato-1',5',6',7'-tetrahydro-2'H-spiro[cyclopropane-1,3'-dicyclopenta[b,e]pyridine] | 259 |
| Intermediate 97 | 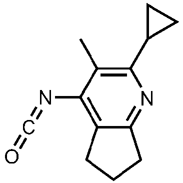 | 2-cyclopropyl-4-isocyanato-3-methyl-6,7-dihydro-5H-cyclopenta[b]pyridine | 247 |

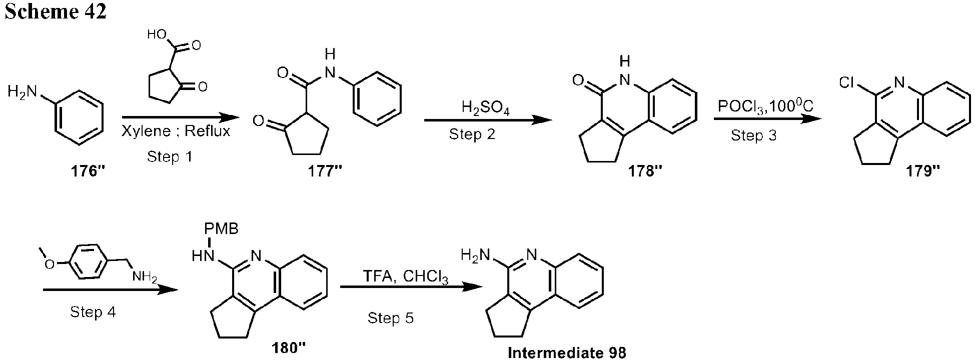

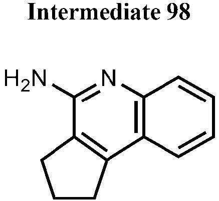

### 2,3-Dihydro-1H-cyclopenta[c]quinolin-4-amine

### Step 1: 2-Oxo-N-phenylcyclopentane-1-carboxamide

Into a 250-mL round-bottom flask purged and maintained under an inert atmosphere of nitrogen, was placed methyl 2-oxocyclopentane-1-carboxylate (10 g, 70 mmol) in xylene (100 mL), to the stirred solution was added aniline (6.6 g, 71 mmol). The resulting solution was stirred for 6 h at 140 °C in an oil bath. The resulting mixture was concentrated. This resulted in 13.4 g (93%) of the title compound as brown oil. MS-ESI: 204 (M+1).

### Step 2: 2,3-Dihydro-1H-cyclopenta[c]quinolin-4(5H)-one

Into a 250-mL round-bottom flask, was placed 2-oxo-N-phenylcyclopentane-1-carboxamide (13.4 g, 65.7 mmol) in H₂SO₄ (50 mL). The resulting solution was stirred for overnight at 30 °C in an oil bath. The resulting solution was carefully poured 1.0 L of H₂O. The pH value of the solution was adjusted to 7 with aq. NaOH (10 %wt.). The solids were filtered out. The resulting solution was extracted with 2x250 ml of ethyl acetate dried over anhydrous sodium sulfate and concentrated. This resulted in 3.7 g of the title compound as a dark yellow solid. MS-ESI: 186 (M+1).

### Step 3: 4-Chloro-2,3-dihydro-1H-cyclopenta[c]quinolone

Into a 100-mL round-bottom flask, was placed 2,3-dihydro-1H-cyclopenta[c]quinolin-4(5H)-one (950 mg, 5.13 mmol) in POCl₃ (20 mL, 215 mmol). The resulting solution was stirred overnight at 80 °C in an oil bath. The resulting mixture was concentrated. The resulting solution was diluted with 50 mL of ethyl acetate. The resulting mixture was washed with 3x25 mL of H₂O. The mixture was dried over anhydrous sodium sulfate and then concentrated. This resulted in 975 mg (93%) of the title compound as a dark yellow solid. MS-ESI: 204 (M+1).

### Step 4: N-(4-methoxybenzyl)-2,3-dihydro-1H-cyclopenta[c]quinolin-4-amine

Into a 100-mL round-bottom flask purged and maintained with an inert atmosphere of nitrogen, was placed 4-chloro-2,3-dihydro-1H-cyclopenta[c]quinoline (780 mg, 3.83 mmol) in dioxane (25 mL), to the above solution was added 1-(4-methoxyphenyl) methanamine (788 mg, 5.74 mmol), Pd₂(dba)₃ (351 mg, 0.38 mmol), DavePhos (151 mg, 0.38 mmol) and t-BuOK (1.29g, 11.5 mmol). The resulting solution was stirred for 4 h at 100 °C in an oil bath. The resulting mixture was concentrated. The residue was eluted from a silica gel column with ethyl acetate/petroleum ether (1:5). This resulted in 768 mg (66%) of the title compound as a dark yellow solid. MS-ESI: 304 (M+1).

### Step 5: 2,3-Dihydro-1H-cyclopenta[c]quinolin-4-amine

Into a 50-mL round-bottom flask, was placed N-(4-methoxybenzyl)-2,3-dihydro-1H-cyclopenta[c]quinolin-4-amine (800 mg, 2.63 mmol) in CHCl₃ (15 mL), to the above solution was added TFA (17.1 mL, 150 mmol) dropwise. The resulting solution was stirred for 4 h at 65 °C in an oil bath. The resulting mixture was concentrated. The resulting solution was diluted with ?? mL of ethyl acetate. The pH value of the solution was adjusted to 8 with the solution of Na₂CO₃ (10 %wt.). The resulting solution was extracted with 2x150 ml of ethyl acetate and the organic layers combined and concentrated. This resulted in 750 mg crude title compound as a yellow solid. MS-ESI: 185 (M+1).

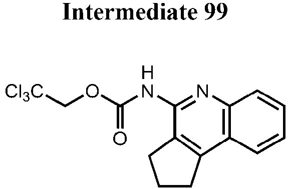

### 2,2,2-Trichloroethyl 2,3-dihydro-1H-cyclopenta[c]quinolin-4-ylcarbamate

Intermediate 99 was prepared using similar procedures for converting Intermediate 33 to Intermediate **62** shown in Scheme 39 from Intermediate 98. MS-ESI: 359/361 (M+1).

**Schemes of Sulfonimidoylamide Intermediates:** Schemes **43-67** illustrate the preparation of sulfonimidoylamide intermediates.

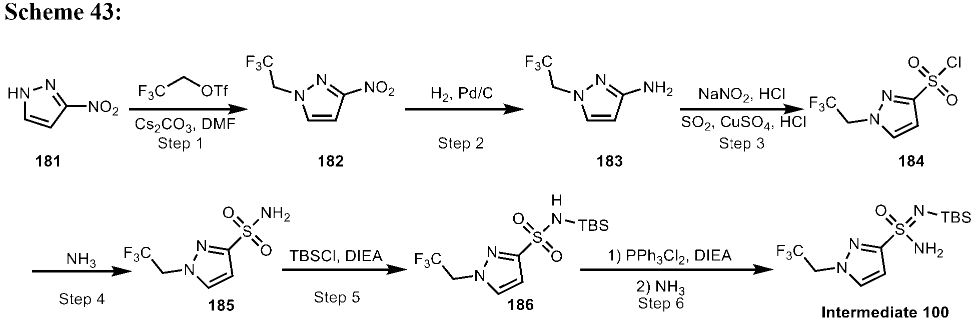

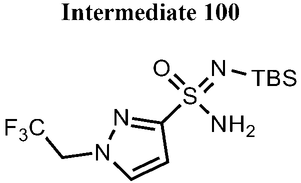

### N'-(tert-butyldimethylsilyl)-1-(2,2,2-trifluoroethyl)-1H-pyrazole-3-sulfonimidamide

### Step 1: 3-Nitro-1-(2,2,2-trifluoroethyl)-1H-pyrazole

To a stirred solution of 3-nitro-1H-pyrazole (10 g, 88.5 mmol) in DMF (100 mL) in a 250-mL round-bottom flask was added Cs₂CO₃ (57.7 g, 177 mmol) at RT, followed by the addition of 2,2,2-trifluoroethyl trifluoromethanesulfonate (20.5 g, 88.5 mmol) dropwise at 0 °C. The resulting solution was stirred for 2 h at RT. The solids were filtered out.

The resulting mixture was diluted with 200 mL of EtOAc then washed with 3x300 mL of water. The organic layer was concentrated under vacuum. The residue was eluted from silica gel with EtOAc/PE (1:10). This resulted in 10.5 g (61%) of the title compound as a yellow solid. MS-ESI: 196 (M+1).

### Step 2: 1-(2,2,2-Trifluoroethyl)-1H-pyrazol-3-amine

To a stirred solution of 3-nitro-1-(2,2,2-trifluoroethyl)-1H-pyrazole (7.2 g, 36.9 mmol) in MeOH (70 ml) in a 100-mL round-bottom flask under nitrogen was added Pd/C (10% wt., 720 mg) in portions. The flask was evacuated and refilled three times with hydrogen. The resulting solution was stirred overnight at RT under hydrogen with a balloon. The solid was filtered out. The resulting mixture was concentrated under reduced pressure. This resulted in 5.8 g (95%) of the title compound as a yellow solid. MS-ESI: 166.1 (M+1).

### Step 3: 1-(2,2,2-Trifluoroethyl)-1H-pyrazole-3-sulfonyl chloride

To a stirred solution of 1-(2,2,2-trifluoroethyl)-1H-pyrazol-3-amine (5.0 g, 30.3 mmol) in HCl (6 M, 50 mL) in a 250-mL 3-necked round-bottom flask was added NaNO₂ (2.5 g, 36.4 mmol) in H₂O (20 mL) dropwise with stirring at 0 °C over 10 min. The resulting solution was stirred for 30 min at 0 °C, this solution was assigned as solution A. Then CuSO₄ (14.5 g, 90.8 mmol) was added to a 500-mL single necked round-bottom flask with AcOH (90 mL) as the solvent. Then SO₂ (g) was bubbled to the solution with stirring at RT for 20 min, this solution was assigned as solution B. To the solution B was added solution A dropwise with stirring at 0 °C. The resulting solution was stirred for 2 h at RT. The residue was diluted with 200 mL of water and extracted with 3x200 mL of DCM. The organic layers were combined and dried over anhydrous Na₂SO₄ and concentrated under vacuum. This resulted in 5.6 g (crude) of the title compound as yellow oil.

### Step 4: 1-(2,2,2-Trifluoroethyl)-1H-pyrazole-3-sulfonamide

To a stirred solution of 1-(2,2,2-trifluoroethyl)-1H-pyrazole-3-sulfonyl chloride solution (crude; from the Step above) in DCM (100 mL) in a 250 mL round bottom flask was bubbled NH₃ (g) at 0 °C for 10 min. The resulting solution was stirred for 2 h at RT. The solution was concentrated under vacuum. The crude product was eluted from silica gel with DCM/MeOH (95:5). This resulted in 4.7 g (67% over two steps) of the title compound as a yellow solid. MS-ESI: 230 (M+1).

### Step 5: N-(tert-butyldimethylsilyl)-1-(2,2,2-trifluoroethyl)-1H-pyrazole-3-sulfonamide

To a stirred solution of 1-(2,2,2-trifluoroethyl)-1H-pyrazole-3-sulfonamide (3.2 g, 14 mmol) in DCM (100 mL) in 250 mL round-bottom flask was added DIEA (7.2 g, 55.7 mmol) at RT, then TBSCl (3.2 g, 20.9 mmol) was added in portions at RT. The resulting solution was stirred for 2 h at RT. The resulting mixture was washed with 5x100 mL of H₂O. The organic layer was dried over anhydrous Na₂SO₄ and concentrated under vacuum. This resulted in 4.0 g (83 %) of the title compound as a yellow solid. MS-ESI: 344 (M+1).

### Step 6: N'-(tert-butyldimethylsilyl)-1-(2,2,2-trifluoroethyl)-1H-pyrazole-3-sulfonimidamide

To a stirred solution of PPh₃Cl₂ (15.5 g, 46.6 mmol) in CHCl₃ (100 mL) in a 250 mL 3-necked round-bottom flask under nitrogen was added DIEA (7.51 g, 58.2 mmol) dropwise with stirring at 0 °C. After stirred for 10 min at RT, to the above was added a solution of N-(tertbutyldimethylsilyl)-1-(2,2,2-trifluoroethyl)-1H- pyrazole-3-sulfonamide (4.0 g, 11.7 mmol) in CHCl₃ (30 mL) dropwise with stirring at 0 °C. The resulting solution was allowed to react for 30 min at 0 °C. To the mixture was bubbled NH₃ (g) with stirring at 0 °C for 15 min. The resulting solution was stirred for 2 h at RT. The reaction was then quenched by the addition of 200 mL of water. The resulting solution was extracted with 3x200 mL of DCM and the organic layers were combined and concentrated under vacuum. The residue was eluted from silica gel with DCM/MeOH (95:5). This resulted in 1.8 g (45%) of the title compound as a white solid. MS-ESI: 343 (M+1).

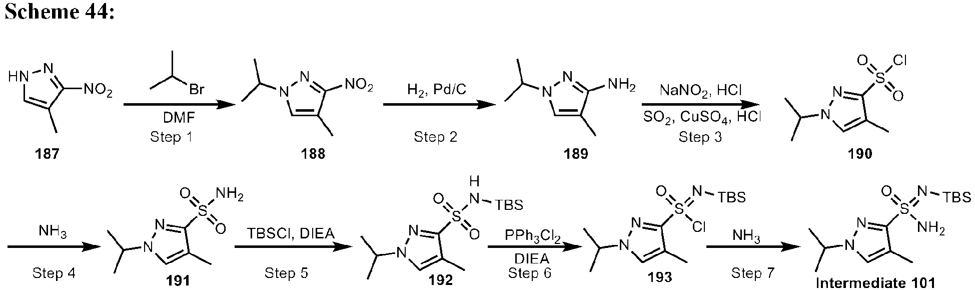

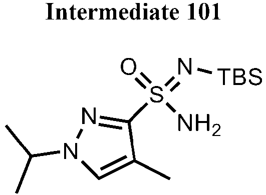

### N'-(tert-butyldimethylsilyl)-1-isopropyl-4-methyl-1H-pyrazole-3-sulfonimidamide

### Step 1: 1-Isopropyl-4-methyl-3-nitro-1H-pyrazole

To a stirred solution of 4-methyl-3-nitro-1H-pyrazole (10 g, 78.7 mmol) in DMF (100 mL) in a 250 mL round-bottom flask under nitrogen was added K₂CO₃ (32.6 g, 236 mmol) in portions, then 2-bromopropane (19.4 g, 157 mmol) was added dropwise at RT. The resulting solution was stirred for 2 days at RT. The solids were filtered out. The resulting mixture was diluted with 100 mL of EtOAc. The resulting mixture was washed with 5x200 mL of water. The organic layer was dried over anhydrous Na₂SO₄ and concentrated under vacuum. The residue was eluted from silica gel with EtOAc/PE (1:20). This resulted in 12.3 g (92%) of the title compound as yellow oil. MS-ESI: 170 (M+1).

Steps 2-7 used similar procedures for converting compound **182** to intermediate **100** shown in Scheme 43 to afford intermediate **101** from compound **188.** MS-ESI: 317 (M+1).

**Table 25. The Intermediates in the following table were prepared using similar procedures for converting compound 187 to Intermediate 101 shown in Scheme 44 from appropriated starting materials.**

| Intermediate # | Structure | IUPAC Name | Exact Mass [M-H]⁻ |
|---|---|---|---|
| **Intermediate 102** | 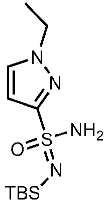 | N'-(tert-butyldimethylsilyl)-1-ethyl-1H-pyrazole-3-sulfonimidamide | 289 |
| **Intermediate 103** | 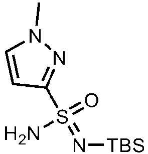 | N'-(tert-butyldimethylsilyl)-1-methyl-1H-pyrazole-3-sulfonimidamide | 275 |

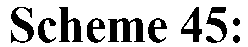

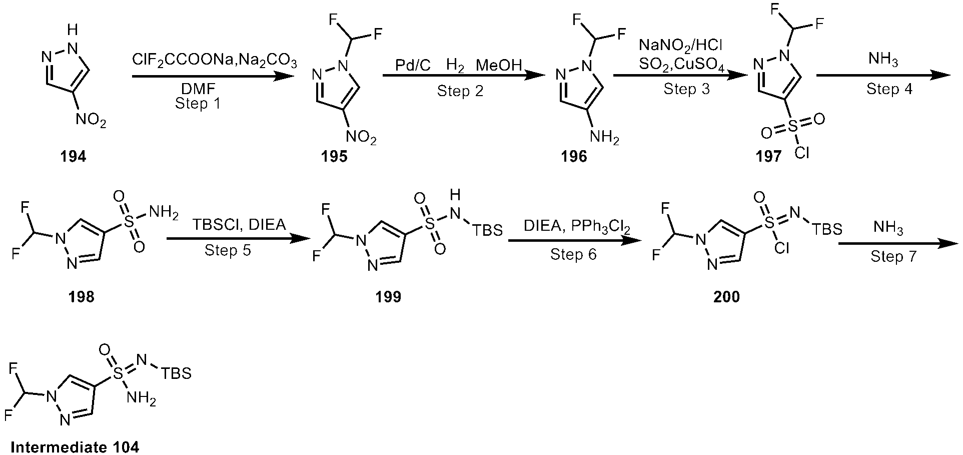

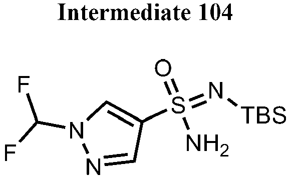

### N'-(tert-butyldimethylsilyl)-1-(difluoromethyl)-1H-pyrazole-4-sulfonimidamide

### Step 1: 1-(Difluoromethyl)-4-nitro-1H-pyrazole

To a stirred solution of 4-nitro-1H-pyrazole (15 g, 133 mmol) in DMF (150 mL) in a 500-mL round-bottom flask was added Na₂CO₃ (21.1 g, 199 mmol) and sodium 2-chloro-2,2-difluoroacetate (24.3 g, 159 mmol) at RT. The resulting solution was stirred for 3 h at 90 °C. The reaction was then quenched by the addition of 100 mL of water. The resulting solution was extracted with 2x100 mL of EtOAc and the organic layers were dried over anhydrous sodium sulfate and concentrated under vacuum. This resulted in 16 g (97%) of the title compound as red liquid. MS-ESI: 164 (M+1).

Steps 2-7 used similar procedures for converting compound **182** to intermediate **100** shown in Scheme 43 to afford intermediate **104** from compound **195.** MS-ESI: 311 (M+1).

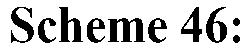

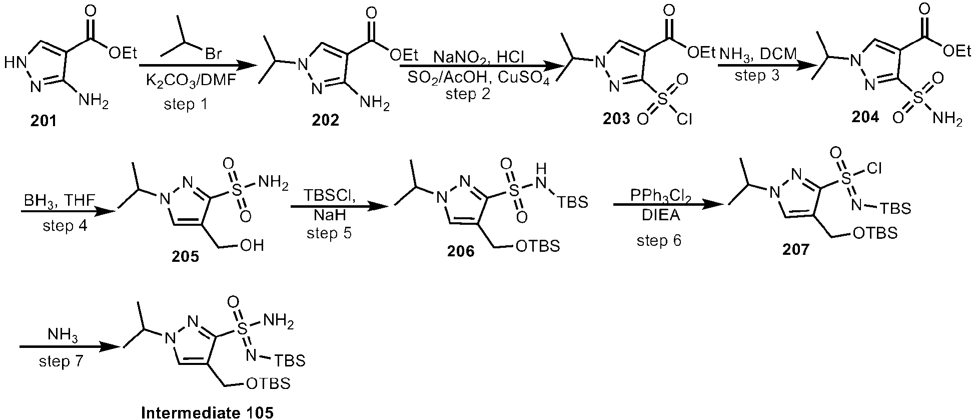

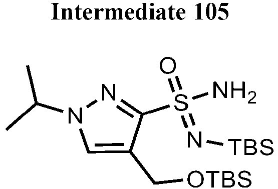

### N'-(tert-butyldimethylsilyl)-4-(((tert-butyldimethylsilyl)oxy)methyl)-1-isopropyl-1H-pyrazole-3-sulfonimidamide

Step 1 used similar procedures for converting compound **187** to compound **188** shown in Scheme 44 to afford compound **202** from compound **201.** MS-ESI: 198 (M+1).

Steps 2-3 used similar procedures for converting compound **183** to compound **185** shown in Scheme 43 to afford compound **204** from compound **202.** MS-ESI: 262 (M+1).

### Step 4: 4-(Hydroxymethyl)-1-isopropyl-1H-pyrazole-3-sulfonamide

To a stirred solution of ethyl 1-isopropyl-3-sulfamoyl-1H-pyrazole-4-carboxylate (1.5 g, 5.3 mmol) in THF (10 mL) in a 100 mL round-bottom flask under nitrogen was added BH₃-THF (1 M, 6.4 mL, 6.4 mmol) dropwise at 0 °C in a water/ice bath. The resulting solution was stirred for 2 h at RT. The resulting mixture was quenched by the addition of 50 mL of water and extracted with 2x100 mL of EtOAc, the organic layers were combined and dried over anhydrous Na₂SO₄ and concentrated under vacuum. The residue was eluted from silica gel with EtOAc/PE (1/1). This resulted in 1.1 g (94%) of the title compound as a white solid. MS-ESI: 220 (M+1).

### Step 5: N-(tert-butyldimethylsilyl)-4-((tert-butyldimethylsilyloxy)methyl)-1-isopropyl-1H-pyrazole-3- sulfonamide

To a stirred solution of 4-(hydroxymethyl)-1-isopropyl-1H-pyrazole-3-sulfonamide (1.2 g, 6.0 mmol) in THF (20 mL) in a 250 mL round-bottom flask under nitrogen was added NaH (60% wt. dispersion in mineral oil, 867 mg, 36 mmol) in portions at 0 °C, followed by the addition of TBSCl (1.8 g, 12.0 mmol) in small portions at 0 °C. The resulting solution was stirred for 3 h at RT. The resulting mixture was quenched by the addition of 50 mL of water and extracted with 2x100 mL of EtOAc and the organic layers were dried over anhydrous Na₂SO₄ and concentrated under vacuum. This resulted in 1.2 g (crude) of the title compound as a yellow solid. MS-ESI: 448 (M+1).

Steps 6-7 used similar procedures for converting compound **186** to Intermediate **100** shown in Scheme 43 to afford intermediate **105** from compound **206.** MS-ESI: 447 (M+1).

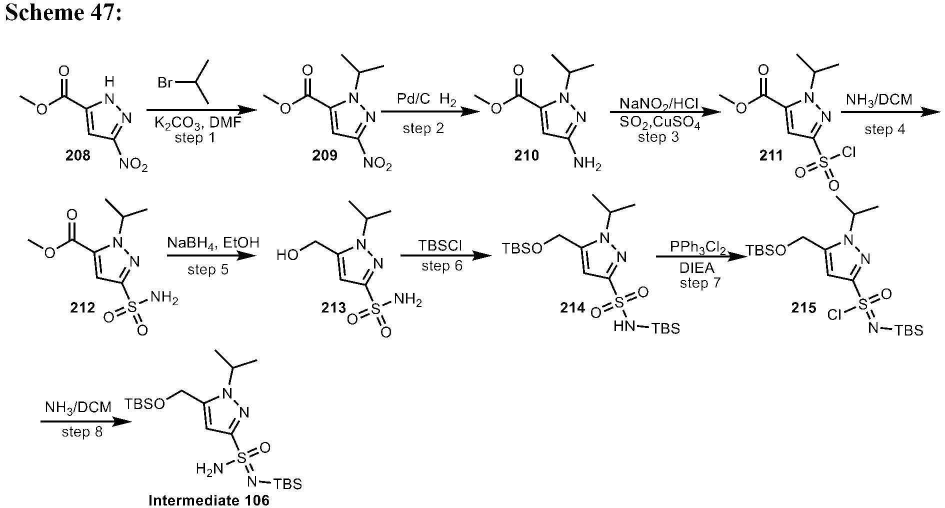

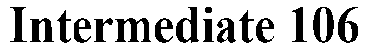

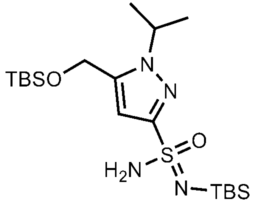

### N'-(tert-butyldimethylsilyl)-5-(((tert-butyldimethylsilyl)oxy)methyl)-1-isopropyl-1H-pyrazole-3-sulfonimidamide

Steps 1-4 used similar procedures for converting compound **187** to compound **191** shown in Scheme 44 to afford compound **212** from compound **208.** MS-ESI: 246 (M-1).

### Step 5: 5-(Hydroxymethyl)-1-isopropyl-1H-pyrazole-3-sulfonamide

To a stirred solution of methyl 1-isopropyl-3-sulfamoyl-1H-pyrazole-5-carboxylate (2.4 g, 9.7 mmol) in ethanol (150 mL) in a 500 mL round-bottom flask under nitrogen was added NaBH₄ (1.84 g, 48.5 mmol) in portions at 0 °C. The resulting solution was stirred overnight at RT. The reaction was quenched by the addition of sat. NH₄Cl (aq.) (20 mL) at 0 °C. The resulting mixture was concentrated under reduced pressure. The mixture was adjusted to pH 7 with HCl (aq.). The resulting mixture was extracted with 3x50 mL EtOAc. The combined organic layer was washed with water (3x20 mL) and dried over anhydrous Na₂SO₄. After filtration, the filtrate was concentrated under reduced pressure. The residue was eluted from silica gel with PE/EtOAc (3:1). This resulted in 1.5 g (70%) of the title compound as a light yellow solid. MS-ESI: 220 (M+1).

Steps 6-8 used similar procedures for converting compound **205** to Intermediate **105** shown in Scheme 46 to afford Intermediate **106** from compound **216.** MS-ESI: 447 (M+1).

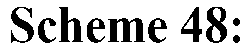

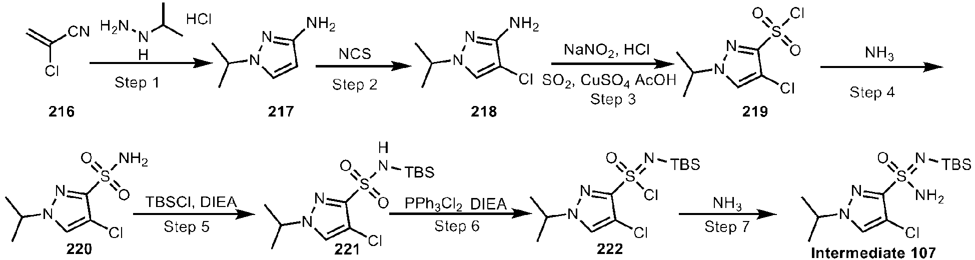

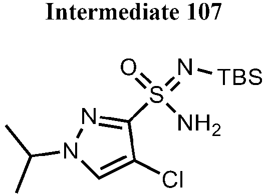

### N'-(tert-butyldimethylsilyl)-4-chloro-1-isopropyl-1H-pyrazole-3-sulfonimidamide

### Step 1: 1-Isopropyl-1H-pyrazol-3-amine

To a stirred solution of isopropylhydrazine hydrochloride (60 g, 546 mmol) in H₂O (600 mL) in a 1 L round-bottom flask was added K₂CO₃ (226 g, 1.64 mol) in portions at 0 °C. The resulting solution was stirred for 2 h at RT. Then 2-chloroacrylonitrile (28 g, 328 mmol) was added to the solution dropwise at 0 °C. The resulting solution was stirred overnight at 50 °C. The resulting solution was extracted with 3x500 mL of EtOAc. The organic layers were combined and dried over anhydrous sodium sulfate and concentrated under vacuum. This resulted in 36 g (90%) of the title compound as yellow oil. MS-ESI: 126 (M+1).

### Step 2: 4-Chloro-1-isopropyl-1H-pyrazol-3-amine

To a stirred solution of 1-isopropyl-1H-pyrazol-3-amine (30 g, 240 mmol) in ACN (300 mL) in a 500 mL round-bottom flask under nitrogen was added NCS (32 g, 240 mmol) in portions. The resulting solution was stirred for 2 h at RT. The resulting mixture was quenched by the addition of 500 mL of water and extracted with 3x500 mL of EtOAc and the organic layers were dried over anhydrous Na₂SO₄ and concentrated under vacuum. The residue was eluted from silica gel with EtOAc/PE (1:1). This resulted in 25 g (65%) of the title compound as a dark yellow solid. MS-ESI: 160 (M+1).

Steps 3-7 used similar procedures for converting compound **183** to Intermediate **100** shown in Scheme 43 to afford Intermediate **108** from compound **218.** MS-ESI: 337 (M+1).

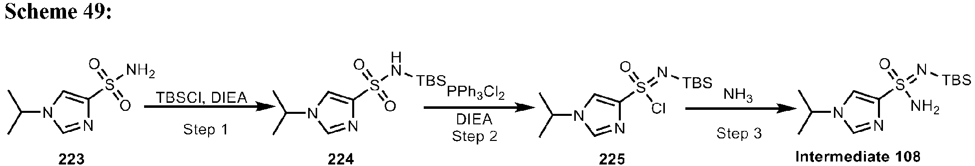

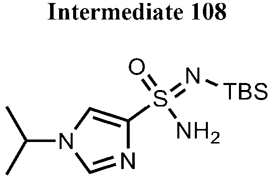

### N'-(tert-butyldimethylsilyl)-1-isopropyl-1H-imidazole-4-sulfonimidamide

Steps 1-3 used similar procedures for converting compound **185** to Intermediate **100** shown in Scheme 43 to afford Intermediate **108** from compound **223.** MS-ESI: 303 (M+1).

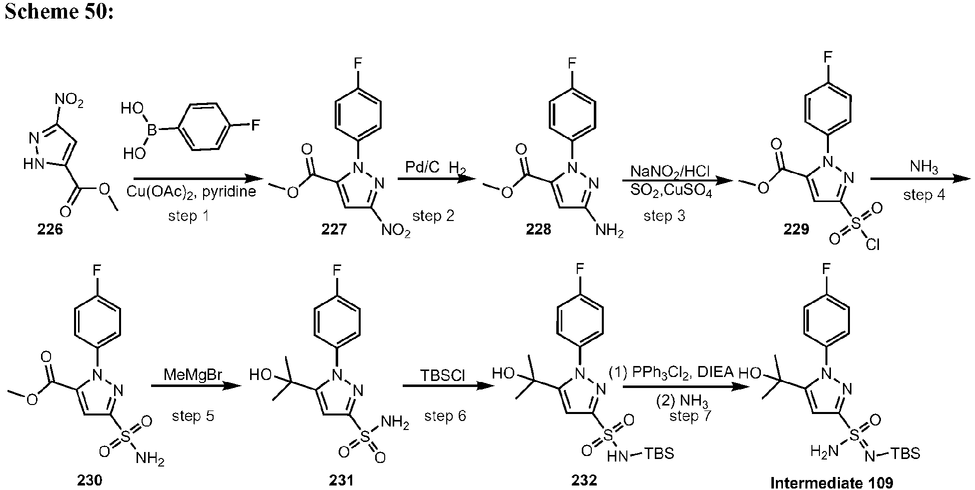

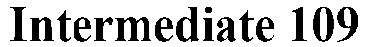

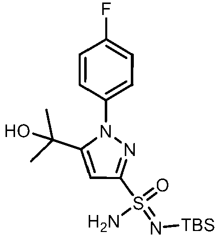

### N'-(tert-butyldimethylsilyl)-1-(4-fluorophenyl)-5-(2-hydroxypropan-2-yl)-1H-pyrazole-3-sulfonimidamide

### Step 1: Methyl 1-(4-fluorophenyl)-3-nitro-1H-pyrazole-5-carboxylate

To a stirred solution of methyl 3-nitro-1H-pyrazole-5-carboxylate (10 g, 58.4 mmol) in DCM (200 mL) in a 500 mL round-bottom flask was added (4-fluorophenyl)boronic acid (24.5 g, 175 mmol) and pyridine (9.25 g, 117 mmol), followed by the addition of Cu(OAc)₂ (15.9 g, 88 mmol) at RT. The resulting solution was stirred for 16 h at RT. The solids were filtered out. The resulting solution was concentrated under vacuum. The residue was eluted from silica gel with EtOAc/PE (1:3). This resulted in 6.4 g (41%) of the title compound as an off-white solid. MS-ESI: 266 (M+1).

Steps 2-4 used similar procedures for converting compound **182** to compound **185** shown in Scheme 43 to afford compound **230** from compound **227.** MS-ESI: 298 (M-1).

### Step 5: 1-(4-Fluorophenyl)-5-(2-hydroxypropan-2-yl)-1H-pyrazole-3-sulfonamide

To a stirred solution of methyl 1-(4-fluorophenyl)-3-sulfamoyl-1H-pyrazole-5-carboxylate (2.8 g, 9.4 mmol) in THF (100 mL) in a 250 mL round-bottom flask under nitrogen was added MeMgBr in THF (3 M, 16 mL, 48 mmol) dropwise at 0 °C in an ice bath. The resulting solution was stirred for 2 h at 0 °C. The reaction was then quenched by the addition of 20 mL of water/ice. The resulting solution was extracted with 3x100 mL of EtOAc. The organic layers combined and dried over anhydrous Na₂SO₄ and concentrated under vacuum. The residue was eluted from silica gel with EtOAc/PE (1:2). This resulted in 1.2 g (43%) of the title compound as a yellow solid. MS-ESI: 300 (M+1).

Steps 6-7 used similar procedures for converting compound **205** to Intermediate **105** shown in Scheme 46 to afford Intermediate **109** from compound **231.** MS-ESI: 432 (M+1).

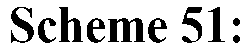

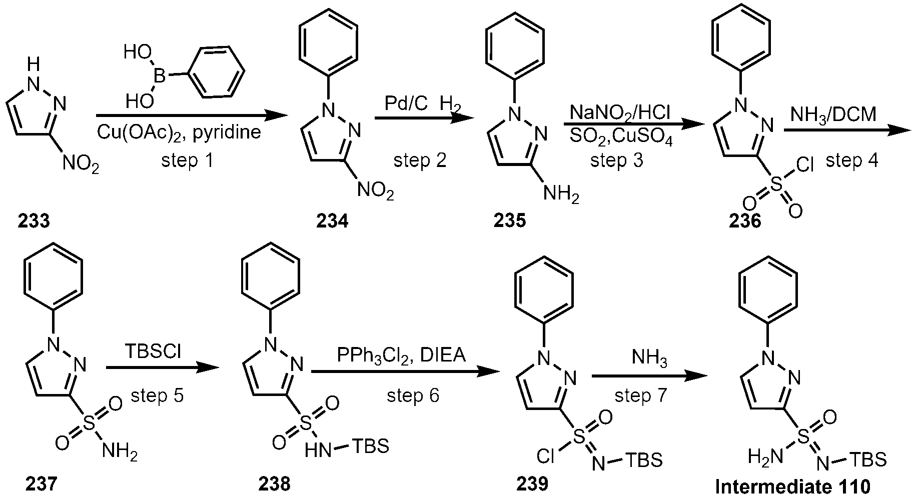

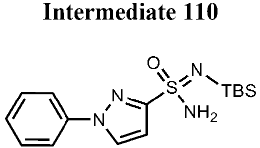

### N'-(tert-butyldimethylsilyl)-1-phenyl-1H-pyrazole-3-sulfonimidamide

Steps 1-4 used similar procedures for converting compound **226** to compound **230** shown in Scheme 50 to afford compound **237** from compound **233.** MS-ESI: 222 (M-1).

Steps 5-7 used similar procedures for converting compound **205** to Intermediate **105** shown in Scheme 46 to afford Intermediate **110** from compound **237.** MS-ESI: 337 (M+1).

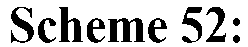

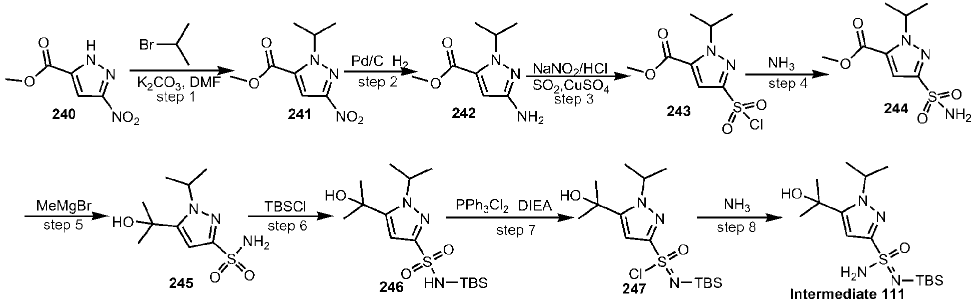

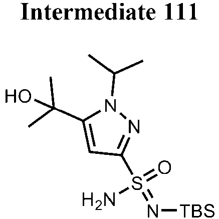

### N'-(tert-butyldimethylsilyl)-5-(2-hydroxypropan-2-yl)-1-isopropyl-1H-pyrazole-3-sulfonimidamide

Steps 1-4 used similar procedures for converting compound **187** to compound **191** shown in Scheme 44 to afford compound **244** from compound **240.** MS-ESI: 246 (M-1).

Steps 5-8 used similar procedures for converting compound **230** to Intermediate **109** shown in Scheme 50 to afford Intermediate **111** from compound **244.** MS-ESI: 361 (M+1).

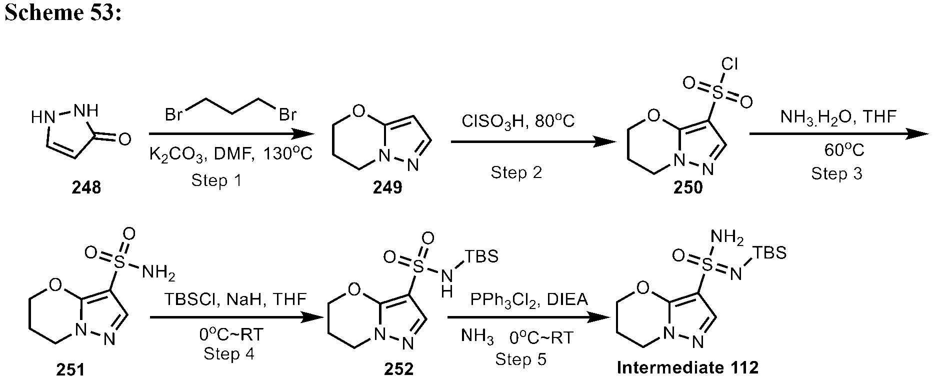

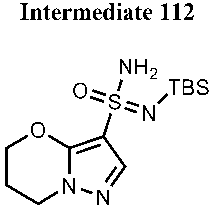

### N'-(tert-butyldimethylsilyl)-6,7-dihydro-5H-pyrazolo[5,1-b][1,3]oxazine-3-sulfonimidamide

### Step 1: 6,7-Dihydro-5H-pyrazolo[5,1-b][1,3]oxazine

To a stirred solution of 1,2-dihydro-3H-pyrazol-3-one (42 g, 500 mmol) in DMF (500 mL) in a 1 L round-bottom flask under nitrogen was added K₂CO₃ (138 g, 1.0 mol) in portions at RT. Followed by the addition of 1,3-dibromopropane (111 g, 550 mmol) dropwise at RT. The resulting mixture was stirred for 16 h at 130 °C. The insoluble matter was filtered out, the filtrate was poured into 1.5 L of water and extracted with 3x500 mL of EtOAc. The organic layers were combined and dried over anhydrous Na₂SO₄ and concentrated under vacuum. The residue was eluted from silica gel with petroleum ether/EtOAc (20:1) to afford 24.8 g (40%) the title compound as a yellow solid. MS-ESI: 125 (M+1). ¹H NMR (400 MHz, CDCl₃): δ 7.31 (d, *J =* 2.0 Hz, 1H), 5.48 (d, *J =* 2.0 Hz, 1H), 4.28 (t, *J =* 5.2 Hz, 2H), 4.18 (t, *J =* 6.2 Hz, 2H), 2.30-2.23 (m, 2H).

### Step 2: 6,7-Dihydro-5H-pyrazolo[5,1-b][1,3]oxazine-3-sulfonyl chloride

A solution of 6,7-dihydro-5H-pyrazolo[5,1-b][1,3]oxazine (24 g, 194 mmol) in chlorosulfonic acid (143 mL) in a 500-mL 3-necked round-bottom flask under nitrogen was stirred for 16 h at 80 °C. The reaction mixture was poured into 1.5 L of water/ice very slowly and extracted with 3x500 mL of EtOAc. The organic layers were combined and dried over anhydrous Na₂SO₄ and concentrated under reduced pressure. The residue was washed with 300 mL of PE. This resulted in 28.1 g (65.0%) of the title compound as a yellow solid. MS-ESI: 223/225 (M+1).

### Step 3: 6,7-Dihydro-5H-pyrazolo[5,1-b][1,3]oxazine-3-sulfonamide

To a stirred solution of ammonia (30% wt., 40 mL) in a 250-mL 3-necked round-bottom flask under nitrogen was added 6,7-dihydro-5H-pyrazolo[5,1-b][1,3]oxazine-3-sulfonyl chloride (28 g, 126 mmol) in THF (80 mL) dropwise at RT. The resulting solution was stirred for 16 h at 60 °C. The organic solvent was removed by concentrated under reduced pressure. The water layer was extracted with 3x100 mL of EtOAc. The organic layers were combined and dried over anhydrous Na₂SO₄ and concentrated under reduced pressure. The residue was eluted from a silica gel column with PE/EtOAc (1:1). This resulted in 16.9 g (66%) of the title compound as a light yellow solid. MS-ESI: 202 (M-1). ¹H NMR (300 MHZ, DMSO-*d₆*): δ 7.47 (s, 1H), 7.08 (s, 2H), 4.40 (t, *J =* 5.1 Hz, 2H), 4.10 (t, *J =* 6.0 Hz, 2H), 2.25-2.15 (m, 2H).

Steps 4-5 used similar procedures for converting compound **205** to Intermediate **105** shown in Scheme 46 to afford Intermediate **112** from compound **251.** MS-ESI: 317 (M+1).

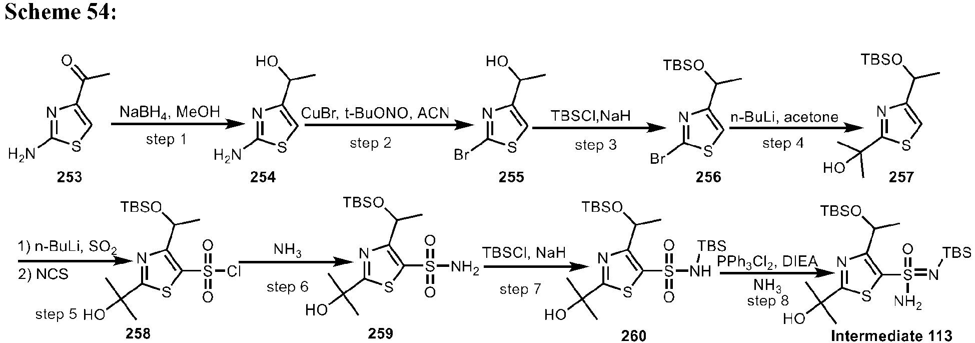

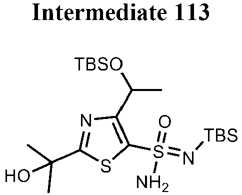

### N'-(tert-butyldimethylsilyl)-4-(1-(tert-butyldimethysilyloxy)ethyl)-2-(2-hydroxypropan-2-yl)thiazole-5-sulfonimidamide

### Step 1: 1-(2-Aminothiazol-4-yl)ethanol

To a stirred solution of 1-(2-aminothiazol-4-yl)ethan-1-one (5.6 g, 39.4 mmol) in MeOH (100 mL) in a 500 mL 3-necked round-bottom flask under nitrogen was added NaBH₄ (2.24 g, 59.2 mmol) in portions below 5 °C. The resulting solution was stirred for 2 h at RT. The reaction was then quenched by the addition of 500 mL of water/ice. The resulting solution was extracted with 3x300 mL of EtOAc. The combined organic layer was washed with 3x300 ml of brine. The mixture was dried over anhydrous Na₂SO₄ and concentrated under vacuum. This resulted in 5.68 g (99%) of the crude title compound as a light yellow solid. MS-ESI: 145 (M+1).

### Step 2: 1-(2-Bromothiazol-4-yl)ethanol

To a stirred solution of 1-(2-aminothiazol-4-yl)ethanol (5.68 g, crude) in ACN (100 mL) in a 250 mL round-bottom flask under nitrogen was added CuBr (8.48 g, 59.1 mmol) and tert-butyl nitrite (6.09 g, 59.1 mmol). The resulting solution was stirred for 2 h at 65 °C. The insoluble matter was filtered out and the filtrate was concentrated under vacuum. The residue was eluted from silica gel with EtOAc/PE (1:4). This resulted in 3.03 g (37%, over two steps) of the title compound as a light yellow solid. MS-ESI: 208 (M+1).

### Step 3: 2-Bromo-4-(1-(tert-butyldimethylsilyloxy)ethyl)thiazole

To a stirred solution of 1-(2-bromothiazol-4-yl)ethanol (4.0 g, 19.2 mmol) in THF (70 mL) in a 250 mL round-bottom flask under nitrogen was added NaH (60% wt., dispersion in mineral oil, 1.54 g, 38.4 mmol) at 0 °C. This was followed by the addition of a solution of TBSCl (5.80 g, 38.4 mmol) in THF (10 mL) dropwise at 0 °C over 3 min. The resulting solution was stirred for 10 h at RT. The resulting solution was diluted with H₂O (100 mL) and extracted with 3x100 mL of DCM. The organic layer was dried over anhydrous Na₂SO₄ and concentrated under vacuum. This resulted in 7.0 g (crude) of the title compound as light yellow oil. MS-ESI: 322 (M+1).

### Step 4: 2-(4-(1-(Tert-butyldimethylsilyloxy)ethyl)thiazol-2-yl)propan-2-ol

To a stirred solution of 2-bromo-4-(1-(tert-butyldimethylsilyloxy)ethyl)thiazole (5.2 g, 16.1 mmol) in THF (100 mL) in a 500 mL three-neck round-bottom flask under nitrogen was added n-BuLi in hexane (2.5 M, 16 mL, 40.3 mmol) dropwise at -70 °C in a liquid nitrogen/EtOH bath. The resulting solution was stirred for 50 min at -70 °C. Then propan-2-one (9.37 g, 161 mmol) was added dropwise to the above solution at -70 °C. The resulting solution was allowed to react with stirring for an additional 60 min at RT. The reaction was then quenched by the addition of 100 mL of sat. NH₄Cl. The resulting solution was extracted with 3x200 mL of EtOAc and the organic layers were dried over anhydrous Na₂SO₄ and concentrated under vacuum. The residue was eluted from silica gel with EtOAc/PE (1:30). This resulted in 3.03 g (62%) of the title compound as a white solid. MS-ESI: 302 (M+1).

### Step 5: 4-(1-(Tert-butyldimethylsilyloxy)ethyl)-2-(2-hydroxypropan-2-yl)thiazole-5-sulfonamide

To a stirred solution of 2-(4-(1-(tert-butyldimethylsilyloxy)ethyl)thiazol-2-yl)propan-2-ol (300 mg, 1.0 mmol) in THF (20 mL) in a 100 mL 3-necked round-bottom flask under nitrogen was added n-BuLi in hexane (2.5 M, 2.0 mL, 5.0 mmol) dropwise at -70 °C in a liquid nitrogen/EtOH bath. The resulting solution was stirred for 30 min at -70 °C. Then SO₂ was bubbled into the above solution at -70 °C for 30 min. Then the resulting solution was stirred for 2 h at RT. The reaction solution was concentrated under vacuum. The crude product was dissolved in DCM (15 mL). Then NCS (199 mg, 1.5 mmol) was added in small portions at RT to the above solution. The resulting solution was allowed to react, with stirring, for an additional 2 h at RT. The resulting mixture was quenched with 15 mL H₂O, the organic layer was collected and washed with 3x15 mL of water, then dried over anhydrous Na₂SO₄ and concentrated under vacuum. This resulted in 400 mg (crude) of the title compound as a light yellow solid.

### Step 6: N-(tert-butyldimethylsilyl)-4-(1-(tert-butyldimethylsilyloxy)ethyl)-2-(2-hydroxypropan-2-yl) thiazole-5-sulfonamide

To a stirred solution of 4-(1-(tert-butyldimethylsilyloxy)ethyl)-2-(2-hydroxypropan-2-yl)thiazole- 5-sulfonamide (400 mg, crude from the Step above) in DCM (20 mL) in a 100 mL round-bottom flask was bubbled NH₃ (gas) for 10 min at 0 °C. The reaction was stirred for 30 min at RT. The resulting mixture was concentrated under vacuum. The residue was eluted from silica gel with EtOAc /PE (1:1) to afford the title compound (1.49 g, 52%, over two steps) as a yellow solid. MS-ESI: 495 (M+1).

Steps 7-8 used similar procedures for converting compound **205** to Intermediate **105** shown in Scheme 46 to afford Intermediate **113** from compound **259.** MS-ESI: 494 (M+1).

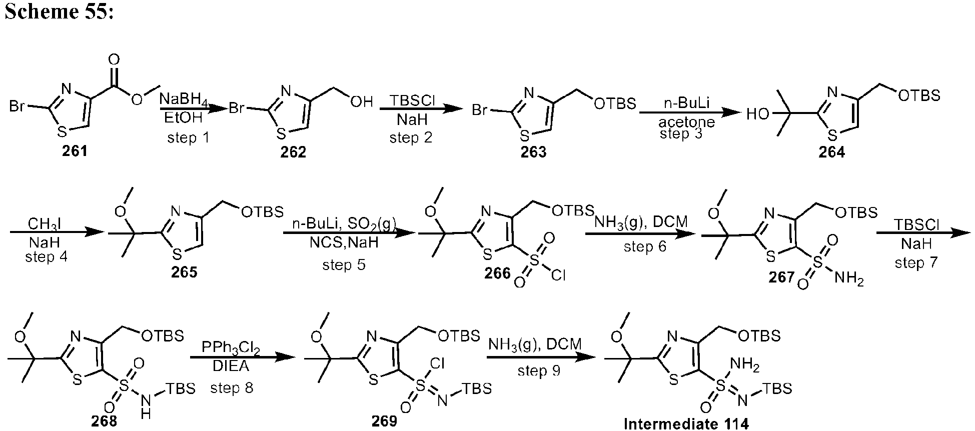

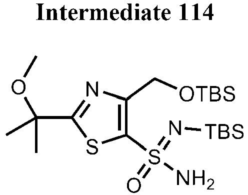

### N'-(tert-butyldimethylsilyl)-4-(((tert-butyldimethylsilyl)oxy)methyl)-2-(2-methoxypropan-2-yl)thiazole-5-sulfonimidamide

Step 1 used similar procedures for converting compound **212** to compound **213** shown in Scheme 47 to afford compound **262** from compound **261.** MS-ESI: 194/196 (M+1).

Steps 2-3 used similar procedures for converting compound **255** to compound **257** shown in Scheme 54 to afford compound **264** from compound **262.** MS-ESI: 288 (M+1).

### Step 4: 4-((Tert-butyldimethylsilyloxy)methyl)-2-(2-methoxypropan-2-yl)thiazole

To a stirred solution of 2-(4-((tert-butyldimethylsilyloxy)methyl)thiazol-2-yl)propan-2-ol (2.4 g, 8.3 mmol) in THF (30 mL) in a 50 mL 3-necked round-bottom flask under nitrogen was added NaH (60%wt. dispersion in mineral oil, 996 mg, 24.9 mmol) in portions at 0 °C followed by the addition of iodomethane (3.55 g, 25 mmol) dropwise at 0 °C in a water/ice bath. The resulting solution was stirred for 16 h at RT. The reaction was quenched with 50 mL water/ice. The resulting mixture was extracted with 3x100 mL of DCM. The organic layers were combined and dried over anhydrous Na₂SO₄ and concentrated under vacuum. The residue was eluted from silica gel with EtOAc/PE (1:20). This resulted in 1.7 g (68%) of the title compound as a yellow solid. MS-ESI: 302 (M+1).

Steps 5-9 used similar procedures for converting compound **257** to Intermediate **113** shown in Scheme 54 to afford Intermediate **114** from compound **265.** MS-ESI: 494 (M+1).

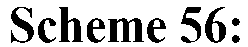

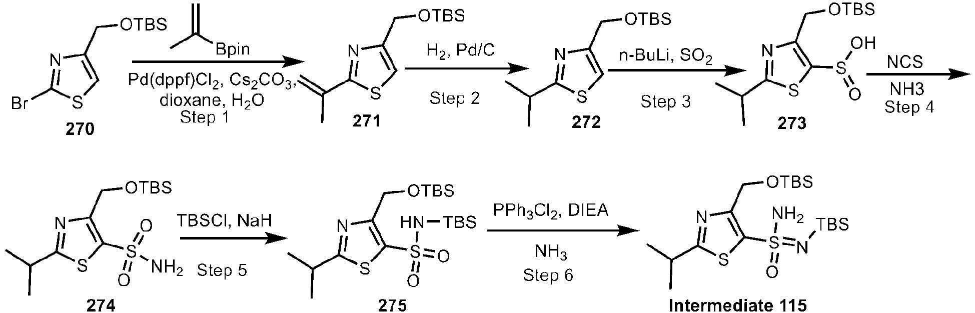

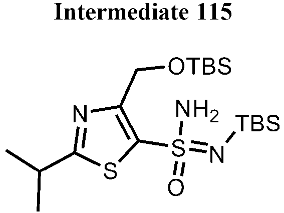

### N'-(tert-butyldimethylsilyl)-4-((tert-butyldimethylsilyloxy)methyl)-2-isopropylthiazole-5-sulfonimidamide

### Step 1: 4-((Tert-butyldimethylsilyloxy)methyl)-2-(prop-1-en-2-yl)thiazole

To a stirred solution of 4,4,5,5-tetramethyl-2-(prop-1-en-2-yl)-1,3,2-dioxaborolane (41 g, 244 mmol) in dioxane (420 mL) and water (105 mL) in a 2 L 3-necked round-bottom flask under nitrogen was added 2-bromo-4-(((tert-butyldimethylsilyl)oxy)methyl)thiazole (25 g, 81 mmol), Cs₂CO₃ (53 g, 162mmol) and Pd(dppf)Cl₂ (5.95 g, 8.1 mmol). The resulting solution was stirred overnight at 80 °C. The solids were filtered out. The resulting solution was concentrated under vacuum and diluted with 400 mL of water. The resulting solution was extracted with 3x350 mL of DCM and the organic layers combined and dried over anhydrous Na₂SO₄. The solids were filtered out. The resulting mixture was concentrated under vacuum. The residue was eluted from silica gel with EtOAc/PE (15:85). This resulted in 18 g (82%) of the title compound as yellow oil. MS-ESI: 270 (M+1).

### Step 2: 4-((Tert-butyldimethylsilyloxy)methyl)-2-isopropylthiazole

To a solution of 4-((tert-butyldimethylsilyloxy)methyl)-2-(prop-1-en-2-yl)thiazole (19 g, 71 mmol) in isopropanol (350 mL) in a 1 L round-bottom flask under nitrogen was added Pd/C (10% wt., 2.0 g) in portions. The flask was evacuated and refilled three times with hydrogen. The resulting solution was stirred overnight at RT under hydrogen with a balloon. The solids were filtered out. The resulting mixture was concentrated under vacuum. The residue was eluted from silica gel with EtOAc/PE (1:9). This resulted in 15 g (78%) of the title compound as a yellow oil. MS-ESI: 272 (M+1).

Steps 3-6 used similar procedures for converting compound **257** to Intermediate **113** shown in Scheme 54 to afford Intermediate **115** from compound **272.** MS-ESI: 464 (M+1).

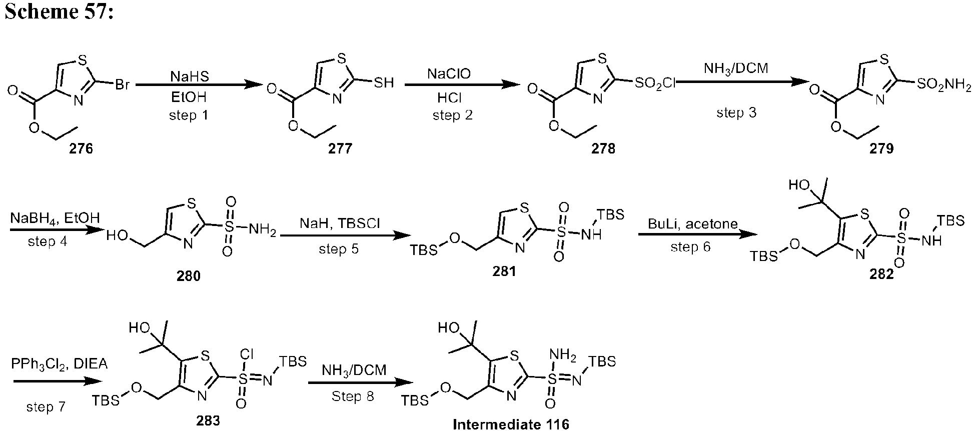

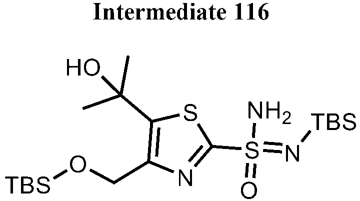

### N'-(tert-butyldimethylsilyl)-4-((tert-buiyldimethylsilyloxy)methyl)-5-(2-₋hydroxypropan-2-yl)thiazole-2-sulfonimidamide

### Step 1: Ethyl 2-mercaptothiazole-4-carboxylate

To a stirred solution of ethyl 2-bromothiazole-4-carboxylate (30 g, 127 mmol) in EtOH (200 mL) in a 1 L round-bottom flask was added NaSH (36 g, 635 mmol) in portions. The resulting solution was stirred for 3 h at 85 °C under nitrogen. The resulting mixture was concentrated under vacuum. The residue was dissolved in 200 mL of H₂O. The pH value of the solution was adjusted to 3 with HCl (1 M) below 5 °C. The solids were collected by filtration. This resulted in 22 g (91.5%) of the title compound as a yellow solid. MS-ESI: 190 (M+1).

### Step 2: Ethyl 2-(chlorosulfonyl)thiazole-4-carboxylate

To a stirred solution of ethyl 2-mercaptothiazole-4-carboxylate (16 g, 84.5 mmol) in HCl (6 M, 100 mL) in a 500 mL round-bottom flask was added NaClO (10% wt., 150 mL, 2.22 mol) dropwise at 0 °C. The resulting solution was stirred for 2 h at 0 °C. The reaction mixture was diluted with 500 mL of H₂O. The resulting solution was extracted with 3x500 mL of DCM. The organic layer was dried over Na₂SO₄ and concentrated under reduced pressure. This resulted in 10.8 g (crude) title compound as yellow oil.

### Step 3: Ethyl 2-sulfamoylthiazole-4-carboxylate

To a stirred solution of ethyl 2-(chlorosulfonyl)thiazole-4-carboxylate (10.8 g, crude) in DCM (100 mL) in a 500 mL round-bottom flask was bubbled NH₃ (g) for 20 min at 0 °C. The resulting solution was stirred for 1 h at 0 °C. The resulting mixture was concentrated under vacuum. The residue was eluted from silica gel with DCM/MeOH (10:1). This resulted in 18 g (65.5% over two steps) of the title compound as a light yellow solid. MS-ESI: 237 (M+1).

### Step 4: 4-(Hydroxymethyl)thiazole-2-sulfonamide

To a stirred solution of ethyl 2-sulfamoylthiazole-4-carboxylate (18 g, 76 mmol) in EtOH (200 mL) in a 500 mL round-bottom flask under nitrogen was added NaBH₄ (8.65 g, 229 mmol) in portions at 0 °C. The resulting solution was stirred for 3 h at RT. The reaction was then quenched by the addition of 300 mL of water and extracted with 2x300 mL of EtOAc. The organic layer was dried over anhydrous Na₂SO₄ and concentrated under vacuum. This resulted in 7.6 g (51.4%) of the title compound as a white solid. MS-ESI: 195 (M+1).

### Step 5: N-(tert-butyldimethylsilyl)-4-((tert-butyldimethylsilyloxy)methyl)thiazole-2-sulfonamide

To a stirred solution of 4-(hydroxymethyl)thiazole-2-sulfonamide (3.1 g, 16 mmol) in THF (25 mL) in a 50 mL round-bottom flask under nitrogen was added NaH (60%wt. dispersion in mineral oil, 3.06 g, 128 mmol) in several batches at 0 °C in a water/ice bath. The resulting solution was stirred for 10 min at 0 °C. To the above was added TBSCl (7.22 g, 47.9 mmol) in portions at 0 °C. The resulting solution was stirred for 1 h at RT. The reaction was then quenched with 50 mL water and extracted with 3x100 mL EtOAc. The organic layer was dried over anhydrous Na₂SO₄ and concentrated under vacuum. The residue was eluted from silica gel with PE/EtOAc (5:1). This resulted in 3.06 g (45%) of the title compound as yellow oil. MS-ESI: 423 (M+1).

### Step 6: N-(tert-butyldimethylsilyl)-4-((tert-butyldimethylsilyloxy)methyl)-5-(2-hydroxypropan-2-yl) thiazole-2-sulfonamide

To a stirred solution of N-(tert-butyldimethylsilyl)-4-((tert-butyldimethylsilyloxy)methyl)thiazole-2- sulfonamide (3.0 g, 7.1 mmol) in THF (20 mL) in a 100-mL 3-necked round-bottom flask under nitrogen was added n-BuLi in hexane (2.5 M, 4.0 mL, 10 mmol) dropwise at -78 °C in a liquid nitrogen/EtOH bath. The resulting solution was stirred for 1 h at -78 °C. This was followed by the addition of propan-2-one (4.12 g, 71 mmol) dropwise at -78 °C. The resulting solution was stirred for 1 h at RT. The reaction was then quenched by the addition of 20 mL of water/ice and extracted with 3x50 mL of EtOAc. The organic layer was dried over anhydrous Na₂SO₄ and concentrated under vacuum. The residue was eluted from silica gel with EtOAc/PE (1:10). This resulted in 1.5 g (44%) of the title compound as yellow oil. MS-ESI: 481 (M+1).

Steps 7-8 used similar procedures for converting compound **186** to Intermediate **100** shown in Scheme 43 to afford Intermediate **116** from compound **282.** MS-ESI: 480 (M+1).

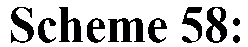

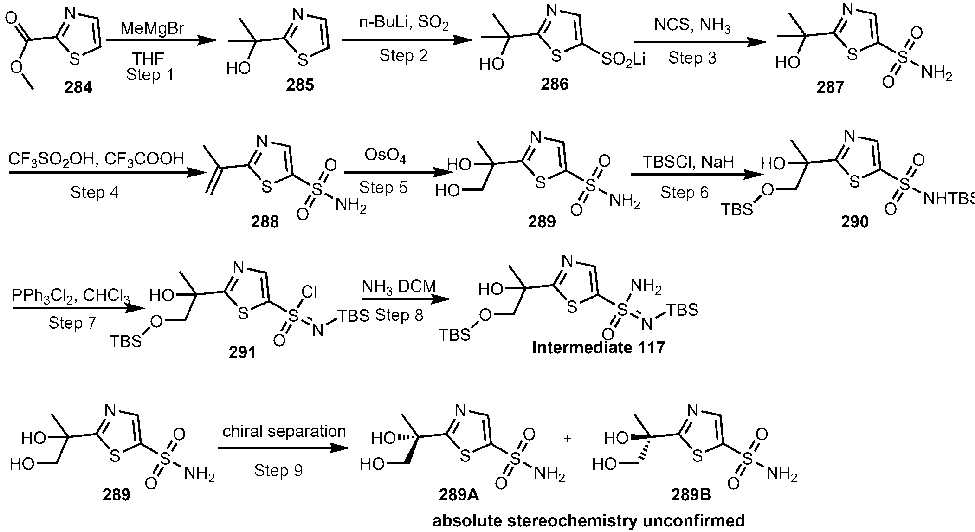

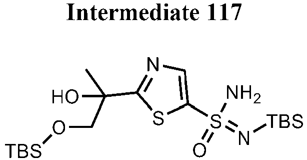

### N'-(tert-butyldimethylsilyl)-2-(1-((tert-butyldimethylsilyl)oxy)-2-hydroxypropan-2-yl)thiazole-5-sulfonimidamide

### Step 1: 2-(Thiazol-2-yl)propan-2-ol

To a stirred solution of 1-(thiazol-2-yl)ethanone (200 g, 1.6 mol) in THF (4 L) in a 10-L 4-necked round-bottom flask under nitrogen was added MeMgBr in THF (3 M, 942 mL, 2.83 mol) dropwise at 0 °C. The resulting solution was stirred for 2 h at 0 °C. the solution was stirred for 16 h at RT. Then the reaction was quenched by the addition of 3 L of sat.NH₄Cl. The resulting solution was extracted with 3x1.0 L of EtOAc. The organic layers were combined and dried over anhydrous Na₂SO₄ and concentrated under vacuum. The residue was eluted from silica gel with a gradient of EtOAc/PE (1:3 to 1:1). This resulted in 210 g (93%) of the title compound as a brown oil. MS-ESI: 144.0 (M+1).

### Step 2: Lithium 2-(2-hydroxypropan-2-yl)thiazole-5-sulfinate

To a stirred solution of 2-(thiazol-2-yl)propan-2-ol (20 g, 140 mmol) in THF (400 mL) in a 1-L 3-necked round-bottom flask under nitrogen was added n-BuLi in hexane (2.50 M, 140 mL, 350 mmol) dropwise at -78 °C. Then the resulting solution was stirred for 1 h at -78 °C. Then SO₂ (g) was bubbled to the solution at -50 °C for 20 min. The resulting solution was allowed to react, with stirring, for an additional 2 h at RT. The resulting mixture was concentrated directly under vacuum. This resulted in 20 g (crude) of the title compound as a yellow crude solid. MS-ESI: 206 (M-1).

### Step 3: 2-(2-Hydroxypropan-2-yl)thiazole-5-sulfonamide

To a stirred solution of lithium 2-(2-hydroxypropan-2-yl)-1,3-thiazole-5-sulfinate (20 g, crude) in DCM (400 mL) in a 1-L round-bottom flask was added NCS (18.8 g, 141 mmol) in portions at 0 °C. The resulting solution was stirred for 2 h at RT. The reaction was quenched with 500 ml of water, then extracted with 3x500 mL of DCM and the organic layers were combined and dried over anhydrous Na₂SO₄. Then NH₃ (g) was bubbled to the DCM mixture for 30 min at 0 °C. The resulting solution was stirred for 2 h at RT and concentrated under vacuum. The residue was eluted from silica gel with EtOAc/PE (1/1). This resulted in 2.0 g (6.43% over two steps) of the title compound as a brown solid. MS-ESI: 223 (M+1).

### Step 4: 2-(Prop-1-en-2-yl)thiazole-5-sulfonamide

To a stirred solution of 2-(2-hydroxypropan-2-yl)thiazole-5-sulfonamide (50 g, 225 mmol,) in CF₃SO₃H (60 mL) in a 500 mL round-bottom flask was added TFA (60 mL) dropwise at RT. The resulting solution was stirred for 16 h at 50 °C in an oil bath. The reaction mixture was concentrated under reduced pressure. The pH value of the residue was adjusted to 8 with aq. NaOH (3%wt.). The resulting solution was extracted with 3x300 mL of DCM and the organic layers were combined and concentrated under vacuum. The residue was eluted from silica gel with EtOAc/PE (1:2). This resulted in 10 g (21%) of the title compound as an off-white solid. MS-ESI: 205 (M+1).

### Step 5: 2-(1,2-Dihydroxypropan-2-yl)thiazole-5-sulfonamide

To a stirred solution of 2-(prop-1-en-2-yl)thiazole-5-sulfonamide (10 g, 49 mmol) in t-BuOH (40 mL) and acetone (40 mL) in a 250-mL round-bottom flask was added NMO (11.5 g, 97.9 mmol) and the resulting solution was stirred for 15 min at RT. Then to this was added a solution of OsO₄ (1.24 g, 4.9 mmol) in H₂O (60 mL) dropwise at RT. The resulting solution was stirred overnight at RT. The reaction was then quenched by the addition of saturated aq. Na₂S₂O₃ (50 mL). The resulting solution was extracted with 3x200 mL EtOAc. The organic layers were combined and dried over anhydrous Na₂SO₄ and concentrated under vacuum. The crude product was eluted from silica gel with MeOH/DCM (7:100). This resulted in 5.0 g (43%) of the title compound as yellow oil. MS-ESI: 239 (M+1).

Steps 6-8 used similar procedures for converting compound **205** to Intermediate **105** shown in Scheme 46 to afford Intermediate **117** from compound **289.** MS-ESI: 466 (M+1).

### Step 9: (R) and (S) 2-(1,2-dihydroxypropan-2-yl)thiazole-5-sulfonamide

Compound **289** (5 g) was resolved by Prep-Chiral-HPLC with the following conditions: CHIRALPAK AD, 5*25 cm, 5 um; Mobile Phase A: CO₂, Mobile Phase B: MeOH (2 mM NH₃-MeOH); Flow rate: 200 mL/min; Gradient: 40% B; UV 220 nm; Rt₁: 3.5 min (Compound **289A**); Rt₂: 5.6 min (Compound **289B**). This resulted in 2.0 g (99%ee) of Compound **289A** and 2.1 g (98%ee) of Compound **289B,** both as white solids. MS-ESI: 237 (M-1).

**Table 26A. The Intermediate 117A and 117B in the following Table were prepared using the similar procedures for converting compound 289 to Intermediate 117 shown in Scheme 58 using Compound 289A and Compound 289B.**

| Intermediate # | Structure | IUPAC Name | Exact Mass [M+H]⁻ |
|---|---|---|---|
| **Intermediate 117A** | 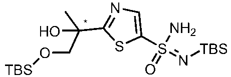 | (S) or (R) N'-(tert-butyldimethylsilyl)-2-(1-(tert-butyldimethylsilyloxy)-2-hydroxypropan-2-yl)thiazole-5-sulfonimidamide | 466 |
| **Intermediate 117B** | 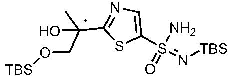 | (R) or (S) N'-(tert-butyldimethylsilyl)-2-(1-(tert-butyldimethylsilyloxy)-2-hydroxypropan-2-yl)thiazole-5-sulfonimidamide | 466 |

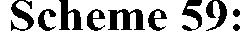

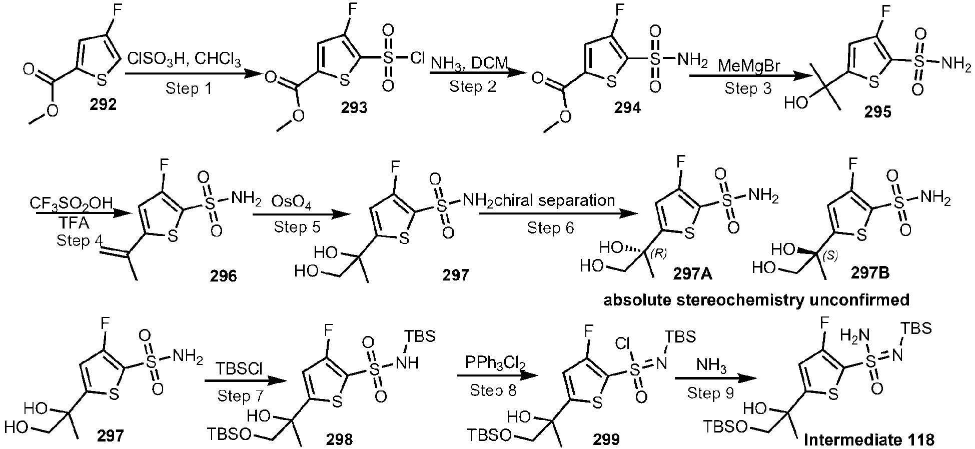

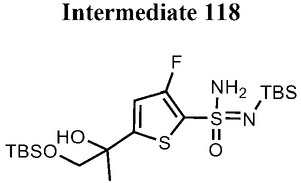

### N'-(tert-butyldimethylsilyl)-5-(1-((tert-butyldimethylsilyl)oxy)-2-hydroxypropan-2-yl)-3-fluorothiophene-2-sulfonimidamide

### Step 1: Methyl 5-(chlorosulfonyl)-4-fluorothiophene-2-carboxylate

To a stirred solution of methyl 4-fluorothiophene-2-carboxylate (10 g, 62.4 mmol) in CHCl₃ (100 mL) in a 500-mL round-bottom flask was added ClSO₃H (21.8 g, 187 mmol) dropwise at 0 °C. The resulting solution was stirred for 12 h at RT. Then to the above was added PCl₅ (65 g, 312 mmol) at 0 °C in an ice bath. The resulting solution was stirred for 2 h at 50 °C. The reaction solution was poured into 500 mL of water/ice very slowly. The resulting solution was extracted with 3x500 mL of DCM and the organic layers were combined and dried over anhydrous Na₂SO₄. This resulted in a solution of the title compound in DCM (1.5 L) and used for next step directly without further purification.

### Step 2: Methyl 4-fluoro-5-sulfamoylthiophene-2-carboxylate

To the DCM solution prepared in step above was bubbled NH₃ (gas) at 0 °C for 15 minutes. The reaction solution was stirred for 3 h at RT and concentrated under reduced vacuum. The crude product was eluted from silica gel with EtOAc/PE (1:3). This resulted in 7.3 g (49%, over two steps) of the title compound as a yellow solid. MS-ESI: 240 (M+1).

### Step 3: 3-Fluoro-5-(2-hydroxypropan-2-yl)thiophene-2-sulfonamide

To a stirred solution of methyl 4-fluoro-5-sulfamoylthiophene-2-carboxylate (7.3 g, 30.5 mmol.) in THF (200 mL) in a 1 L 3-necked round-bottom flask under nitrogen was added MeMgBr in THF (3 M, 51 mL, 153 mmol) dropwise at 0 °C. The resulting solution was stirred for 14 h at RT and then was quenched by the addition of 100 mL of sat. NH₄Cl. The resulting solution was extracted with 3x150 mL of EtOAc and the organic layers were combined, dried over anhydrous Na₂SO₄ and concentrated under vacuum. The residue was eluted from silica gel with EtOAc/PE (1:5 to 1:1). This resulted in 5.5 g (75%) of the title compound as a white solid. MS-ESI: 240 (M+1).

Steps 4-5 used similar procedures for converting compound **287** to compound **289** shown in Scheme 58 to afford compound **297** from compound **295.** MS-ESI: 254 (M-1).

### Step 6: (R) and (S)-5-(1,2-Dihydroxypropan-2-yl)-3-fluorothiophene-2-sulfonamide

Compound **297** (3.0 g) was resolved by Prep-Chiral-HPLC with the following conditions: Lux^{®} 5µm Amylose-1, 5*25 cm, 5 µm; Mobile Phase A: CO₂, Mobile Phase B: MeOH (2 mM NH₃); Flow rate: 200 mL/min; Gradient: 50% B; UV 220 nm; Rt1: 3 min (297A); Rt2: 6.8 min (297B); This resulted in 1.1 g of Compound **297A** (99% ee) and 1.0 g of Compound **297B** (99%ee). MS-ESI: 254 (M-1).

Steps 7-9 used similar procedures for converting compound **205** to Intermediate **105** shown in Scheme 46 to afford Intermediate **118** from compound **297.** MS-ESI: 483 (M+1).

**Table 26. The Intermediates in the following Table were prepared using the similar procedures for converting compound 297 to Intermediate 118 shown in Scheme 59 using Compound 297A and Compound 297B.**

| Intermediate # | Structure | IUPAC Name | Exact Mass [M-H]⁻ |
|---|---|---|---|
| **Intermediate 119** | 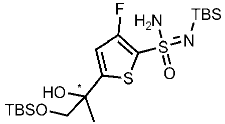 | (R, RS) or (S, RS) N'-(tertbutyldimethylsilyl)-5-(1-((tertbutyldimethylsilyl)oxy)-2-hydroxypropan-2-yl)-3-fluorothiophene-2-sulfonimidamide | 483 |
| **Intermediate 120** | 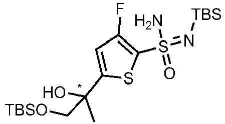 | (S, RS) or (R, RS) N'-(tertbutyldimethylsilyl)-5-(1-((tertbutyldimethylsilyl)oxy)-2-hydroxypropan-2-yl)-3-fluorothiophene-2-sulfonimidamide | 483 |

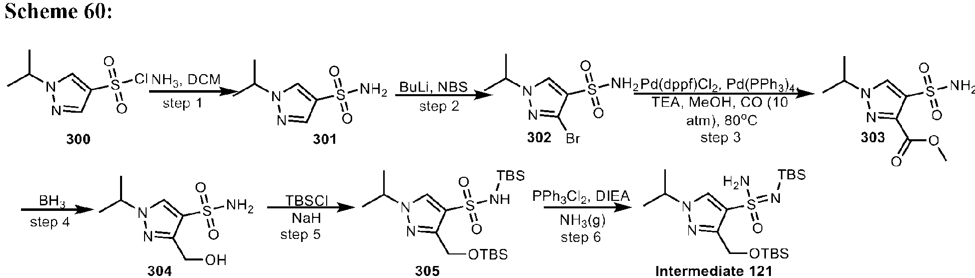

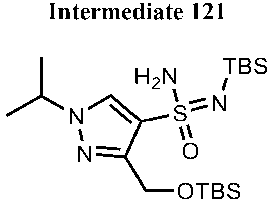

### N'-(tert-butyldimethylsilyl)-3-((tert-butyldimethylsilyloxy)methyl)-1-isopropyl-1H-pyrazole-4-sulfonimidamide

### Step 1: 1-Isopropyl-1H-pyrazole-4-sulfonamide

To a stirred solution of 1-isopropyl-1H-pyrazole-4-sulfonyl chloride (6.0 g, 28.8 mmol) and in DCM (60 mL) in a 250 mL round bottom flask was bubbled NH₃ (g) at 0 °C for 10 min. The reaction solution was stirred for 1 h at RT and concentrated under reduced pressure. The residue was eluted from silica gel with PE/EtOAc (1:1). This resulted in 3.2 g (59%) of the title compound as a yellow solid. MS-ESI: 190 (M+1).

### Step 2: 3-Bromo-1-isopropyl-1H-pyrazole-4-sulfonamide

To a stirred solution of 1-isopropyl-1H-pyrazole-4-sulfonamide (6.4 g, 33.8 mmol) in THF (100 mL) in a 500-mL 3-necked round-bottom flask under nitrogen was added n-BuLi in hexane (2.5 M, 30 mL, 74.4 mmol) dropwise at -78 °C in a liquid nitrogen/EtOH bath. Then the reaction was stirred for 1 h at -78 °C. To the above mixture was added NBS (7.22 g, 40.6 mmol) in THF (20 mL) dropwise at -78 °C. The resulting mixture was stirred for additional 2 h at RT. The reaction mixture was quenched by the addition of water/ice (50 mL) at 0 °C. The resulting mixture was extracted with EtOAc (3x100 mL). The combined organic layers were dried over anhydrous Na₂SO₄. After filtration, the filtrate was concentrated under reduced pressure. The residue was purified by reverse flash chromatography with the following conditions: C18 silica gel; mobile phase, MeCN in water, 10% to 50% gradient in 10 min; detector, UV 254 nm. This resulted in 2.5g (28%) of the title compound as a yellow solid. MS-ESI: 268 (M+1).

### Step 3: Methyl 1-isopropyl-4-sulfamoyl-1H-pyrazole-3-carboxylate

To a stirred solution of 3-bromo-1-isopropyl-1H-pyrazole-4-sulfonamide (2.0 g, 7.46 mmol) in MeOH (100 mL) in a 250-mL round-bottom flask under nitrogen was added TEA (3.77 g, 37.3 mmol), Pd(PPh₃)₄ (862 mg, 0.75 mmol) and Pd(dppf)Cl₂ (546 mg, 0.75 mmol). The resulting solution was stirred overnight at 80 °C under CO atmosphere (10 atm). The insoluble matter was filtered out and the filtrate was concentrated under vacuum. The residue was eluted from silica gel with PE/EtOAc (1:1). This resulted in 920 mg (50%) of the title compound as a yellow solid. MS-ESI: 248 (M+1).

### Step 4: 3-(Hydroxymethyl)-1-isopropyl-1H-pyrazole-4-sulfonamide

To a stirred solution of methyl 1-isopropyl-4-sulfamoyl-1H-pyrazole-3-carboxylate (900 mg, 3.64 mmol) in THF (30 mL) in a 100-mL round-bottom flask under nitrogen was added BH₃ in THF (1 M, 36 mg, 36 mmol) dropwise at 0 °C in an ice bath. The reaction solution was stirred overnight at 50 °C. The reaction was quenched with MeOH (30 mL) at 0 °C. The mixture was adjusted to pH 5~6 with HCl (6 M). The resulting mixture was concentrated under vacuum. The residue was eluted from silica gel with PE/EtOAc (1:1). This resulted in 600 mg (75%) of the title compound as a yellow solid. MS-ESI: 220 (M+1).

Steps 5-6 used similar procedures for converting compound **205** to Intermediate **105** shown in Scheme 46 to afford Intermediate **121** from compound **304.** MS-ESI: 447 (M+1).

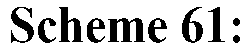

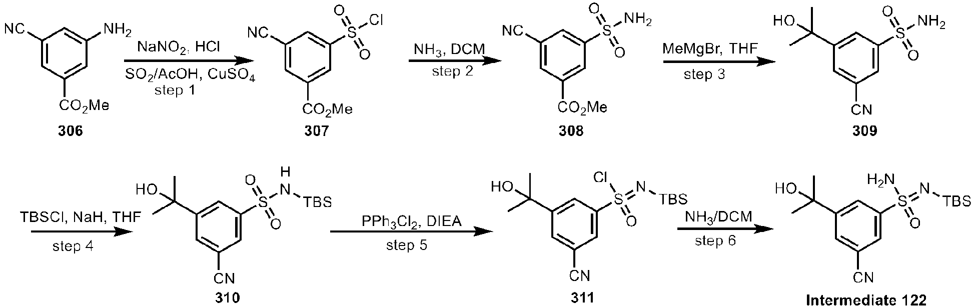

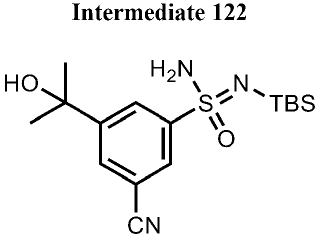

### N'-(tert-butyldimethylsilyl)-3-(2-hydroxypropan-2-yl)-5-isocyanobenzenesulfonimidamide

Steps 1-2 used similar procedures for converting compound **183** to compound **185** shown in Scheme **43** to afford compound **308** from compound **306.** MS-ESI: 241 (M+1).

Steps 3-6 used similar procedures for converting compound **230** to Intermediate **109** shown in Scheme 50 to afford Intermediate **122** from compound **308.** MS-ESI: 354 (M+1).

**Table 27. The Intermediates in the following Table were prepared using similar procedures for converting compound 306 to Intermediate 122 shown in Scheme 61 from appropriated starting materials.**

| Intermediate # | Structure | IUPAC Name | Exact Mass [M-H]⁻ |
|---|---|---|---|
| **Intermediate 123** | 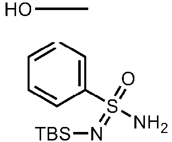 | N'-(tert-butyldimethylsilyl)-3-(2-hydroxypropan-2-yl)benzenesulfonimidamide | 329 |

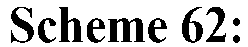

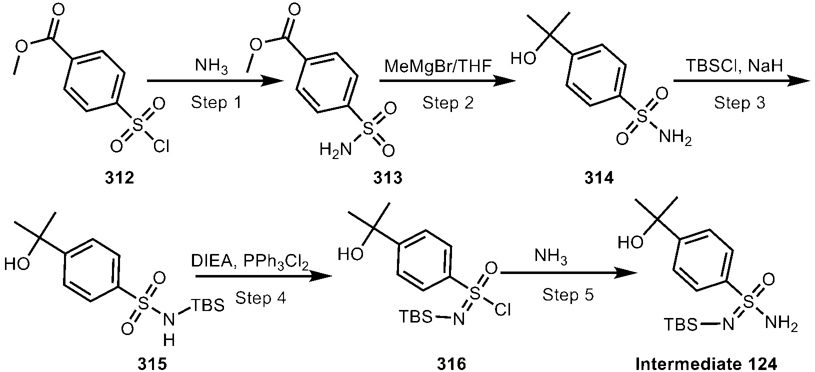

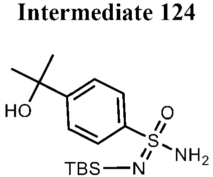

### N'-(tert-butyldimethylsilyl)-4-(2-hydroxypropan-2-yl)benzenesulfonimidamide

Step 1 used similar procedures for converting compound **300** to compound **301** shown in Scheme 60 to afford compound **313** from compound **312.** MS-ESI: 214 (M-1).

Steps 2-5 used similar procedures for converting compound **230** to Intermediate **109** shown in Scheme 50 to afford Intermediate **124** from compound **313.** MS-ESI: 329 (M+1).

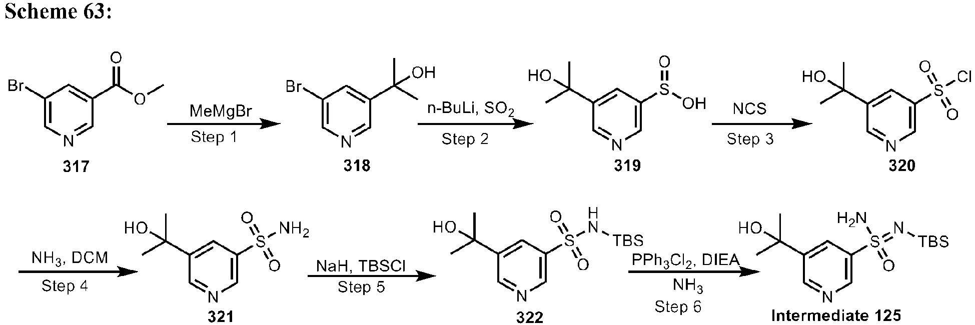

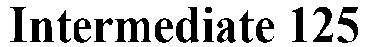

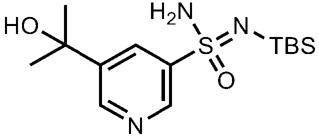

### N'-(tert-butyldimethylsilyl)-5-(2-hydroxypropan-2-yl)pyridine-3-sulfonimidamide

Steps 1-4 used similar procedures for converting compound **284** to compound **287** shown in Scheme 58 to afford compound **321** from compound **317.** MS-ESI: 215 (M+1).

Steps 5-6 used similar procedures for converting compound **205** to Intermediate **105** shown in Scheme 46 to afford Intermediate **125** from compound **321.** MS-ESI: 330 (M+1).

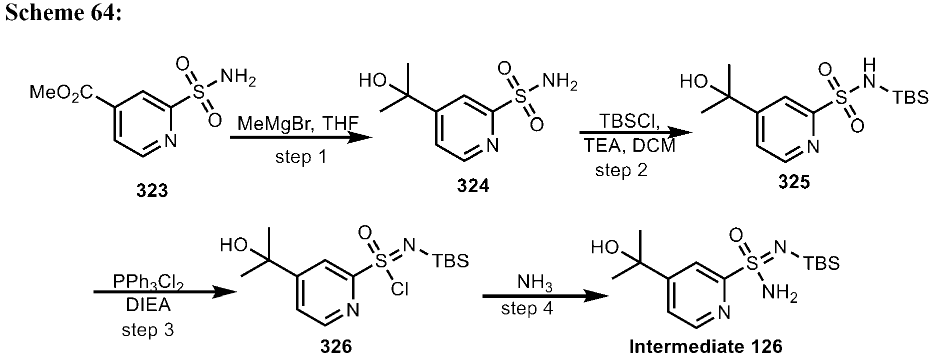

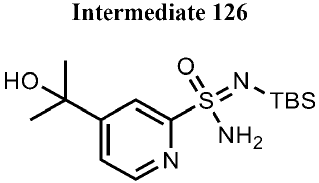

### N'-(tert-butyldimethylsilyl)-4-(2-hydroxypropan-2-yl)pyridine-2-sulfonimidamide

Step 1 used similar procedures for converting compound **284** to compound **285** shown in Scheme 58 to afford compound **324** from compound **323.** MS-ESI: 215 (M-1).

Steps 2-4 used similar procedures for converting compound **205** to Intermediate **105** shown in Scheme 46 to afford Intermediate **126** from compound **324.** MS-ESI: 330 (M+1).

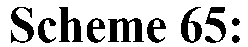

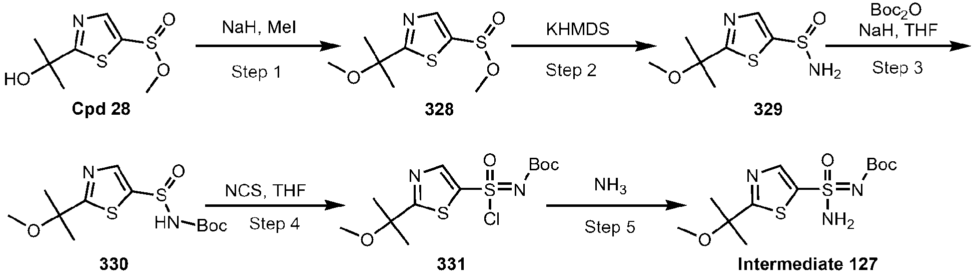

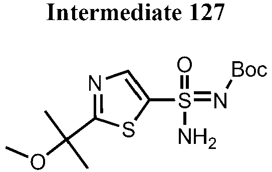

### Tert-butyl (amino(2-(2-methoxypropan-2-yl)thiazol-5-yl)(oxo)-λ⁶-sulfaneylidene)carbamate

### Step 1: Methyl 2-(2-methoxypropan-2-yl)thiazole-5-sulfinate

To a stirred solution of methyl 2-(2-hydroxypropan-2-yl)-1,3-thiazole-5-sulfinate (40 g, 181 mmol) in THF (500 mL) in a 1-L round-bottom flask under nitrogen was added NaH (60%wt. dispersion in mineral oil, 7.95 g, 199 mmol) in portions at 0 °C in an ice/ethanol bath. To this reaction solution was added MeI (51.3 g, 362 mmol) dropwise at 0 °C. The resulting solution was stirred for 3 h at RT. The reaction was then quenched by the addition of water (50 mL) at 0 °C. The resulting solution was extracted with 3x300 mL of EtOAc and the organic layers were combined, dried over anhydrous Na₂SO₄ and concentrated under vacuum. This resulted in 32 g (75.3%) of the title compound as a white solid. MS-ESI: 236 (M+1).

### Step 2: 2-(2-Methoxypropan-2-yl)thiazole-5-sulfinamide

To a stirred solution of methyl 2-(2-methoxypropan-2-yl)-1,3-thiazole-5-sulfinate (20 g, 85 mmol) in THF (500 mL) in a 1-L 3-necked round-bottom flask under nitrogen was added KHMDS in THF (1 M, 1.0 L, 1.0 mol) dropwise at -78 °C in a liquid nitrogen/ethanol bath. The resulting solution was stirred for 3 h at -78 °C in a liquid nitrogen/ethanol bath. The reaction was quenched by the addition of water (50 mL). The resulting solution was extracted with 3x300 mL of EtOAc. The organic layer was dried over anhydrous Na₂SO₄ and concentrated under vacuum.

The residue was eluted from a silica gel with EtOAc/PE (1:3). This resulted in 14 g (74.8%) of the title compound as a white solid. MS-ESI: 221.0 (M+1).

### Step 3: Tert-butyl ((2-(2-methoxypropan-2-yl)thiazol-5-yl)sulfinyl)carbamate

To a stirred solution of 2-(2-methoxypropan-2-yl)-1,3-thiazole-5-sulfinamide (10 g, 45.4 mmol) in THF (250 mL) in a 500-mL round-bottom flask under nitrogen was added NaH (60%wt. dispersion in mineral oil, 3.63 g, 90.8 mmol) in portions at 0 °C in an ice/ethanol bath. To this solution was added Boc₂O (9.91 g, 45.4 mmol) in portions at 0 °C in an ice/ethanol bath. The resulting solution was stirred for 3 h at RT. The reaction was then quenched by the addition of water (50 mL). The resulting solution was extracted with 3x300 mL of EtOAc. The organic layer was dried over anhydrous Na₂SO₄ and concentrated under vacuum. This resulted in 12 g (82.5%) of the title compound as a white solid. MS-ESI: 321.1 (M+1).

### Step 4: Tert-butyl (chloro(2-(2-methoxypropan-2-yl)thiazol-5-yl)(oxo)-λ₆-sulfaneylidene)carbamate

To a stirred solution of tert-butyl N-[[2-(2-methoxypropan-2-yl)-1,3-thiazol-5-yl]sulfinyl]carbamate (11 g, 34.3 mmol) in THF (200 mL) in a 500-mL round-bottom flask under nitrogen was added NCS (13.8 g, 103 mmol) in small portions at 0 °C. The resulting solution was stirred for 3 h at RT. This reaction solution was used to the next step directly without further purification.

### Step 5: Tert-butyl (amino(2-(2-methoxypropan-2-yl)thiazol-5-yl)(oxo)-λ⁶-sulfaneylidene)carbamate

NH₃ gas was bubbled into a stirred solution of tert-butyl (chloro(2-(2-methoxypropan-2-yl)thiazol-5- yl)(oxo)-λ⁶-sulfaneylidene) carbamate (prepared from last step) in THF (200 mL) for 15 min at 0 °C. The resulting solution was stirred for 1 h at RT and concentrated under vacuum. The residue was eluted from silica gel with EtOAc/PE(1:1). This resulted in 7.0 g (60.8% over two steps) of the title compound as a white solid. MS-ESI: 336.1 (M+1).

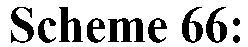

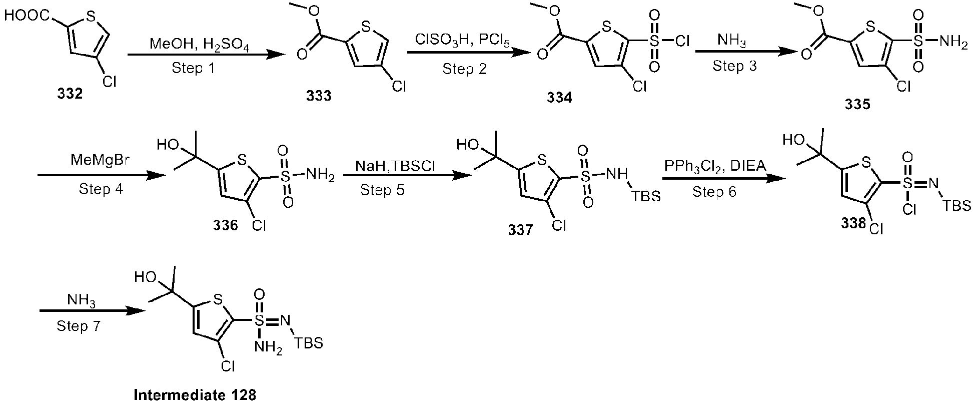

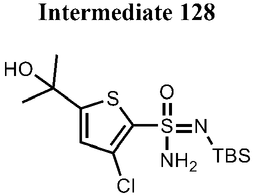

### N'-(tert-butyldimethylsilyl)-3-chloro-5-(2-hydroxypropan-2-yl)thiophene-2-sulfonimidamide

### Step 1: Methyl 4-chlorothiophene-2-carboxylate

To a stirred solution 4-chlorothiophene-2-carboxylic acid (9.0 g, 55 mmol) in MeOH (100 mL) in a 100-mL round-bottom flask was added H₂SO₄ (8.0 mL) dropwise at 0°C in an ice bath. The resulting solution was stirred for 16 h at 70°C in an oil bath. The reaction solution was poured into 80 mL of water/ice slowly. The pH value of the solution was adjusted to 10 with KOH (sat.). The resulting solution was extracted with 3x80 mL of DCM. The organic layer was dried over anhydrous Na₂SO₄ and concentrated under vacuum. This resulted in 8.0 g (81.8%) of methyl 4-chlorothiophene-2-carboxylate as a yellow oil. MS-ESI: 177/179 (M+1).

Steps 2-4 used similar procedures for converting compound **292** to compound **295** shown in Scheme 59 to afford compound **336** from compound **333.** MS-ESI: 254/256 (M-1).

Steps 5-7 used similar procedures for converting compound **205** to Intermediate **105** shown in Scheme 46 to afford Intermediate **128** from compound **336.** MS-ESI: 369/371 (M+1).

**Table 28. The Intermediates in the following Table were prepared using the similar procedures for converting compound 332 to Intermediate 128 shown in Scheme 66 from appropriated starting materials.**

| Intermediate # | Structure | IUPAC Name | Exact Mass [M-H]⁻ |
|---|---|---|---|
| **Intermediate 129** | 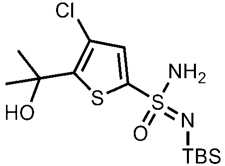 | N'-(tert-butyldimethylsilyl)-4-chloro-5-(2-hydroxypropan-2-yl)thiophene-2-sulfonimidamide | 369/371 |
| **Intermediate 130** | 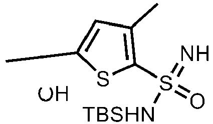 | N-(tert-butyldimethylsilyl)-5-(2-hydroxypropan-2-yl)-3-methylthiophene-2-sulfonimidamide | 349 |
| **Intermediate 131** | 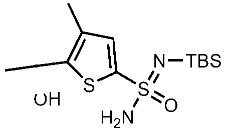 | N'-(tert-butyldimethylsilyl)-5-(2-hydroxypropan-2-yl)-4-methylthiophene-2-sulfonimidamide | 349 |
| **Intermediate 132** | 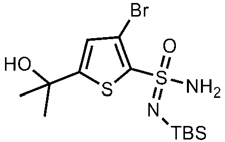 | 3-Bromo-N'-(tert-butyldimethylsilyl)-5-(2-hydroxypropan-2-yl)thiophene-2-sulfonimidamide | 413/415 |

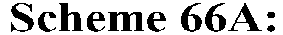

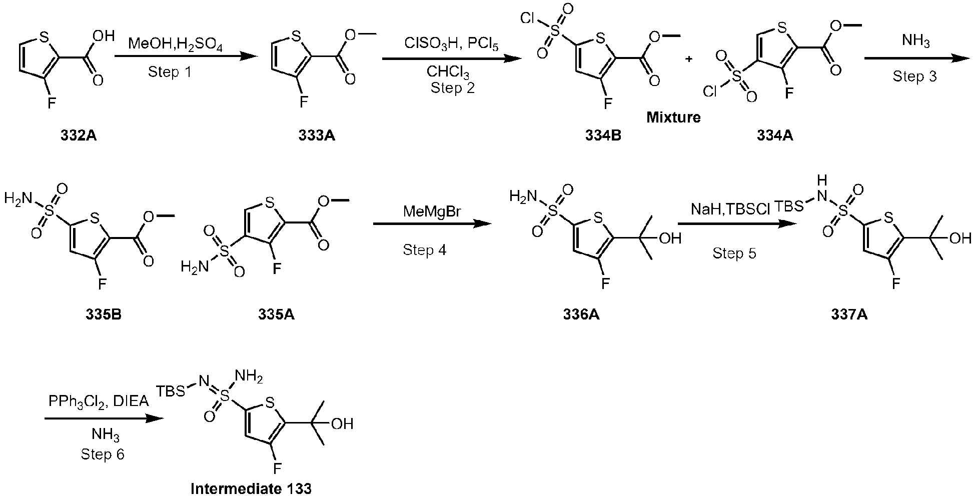

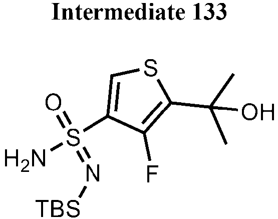

### N'-(tert-butyldimethylsilyl)-4-fluoro-5-(2-hydroxypropan-2-yl)thiophene-3-sulfonimidamide

Steps 1 used similar procedures for converting compound **332** to compound **333** shown in Scheme 66 to afford compound **333A** from compound **332A.** MS-ESI: 161 (M+1).

### Step 2: Mixture of Methyl 5-(chlorosulfonyl)-3-fluorothiophene-2-carboxylate (Major) and methyl 4-(chlorosulfonyl)-3-fluorothiophene-2-carboxylate (Minor)

To a stirred solution of methyl 3-fluorothiophene-2-carboxylate (10 g, 62.5 mmol) in CHCl₃ (100 mL) in a 500-mL round-bottom flask was added ClSO₃H (21.8 g, 187 mmol) dropwise at 0 °C. The resulting solution was stirred for 12 h at RT. Then to the above was added PCl₅ (65 g, 312 mmol) at 0 °C in an ice bath. The resulting solution was stirred for 2 h at 60 °C. The reaction solution was poured into 500 mL of water/ice very slowly. The resulting solution was extracted with 3x500 mL of DCM and the organic layers were combined and dried over anhydrous Na₂SO₄. This resulted in a mixture solution of the title compounds in DCM (1.5 L) and used for next step directly without further purification.

### Step 3: Methyl 3-fluoro-5-sulfamoylthiophene-2-carboxylate (Major) and methyl 3-fluoro-4-sulfamoylthiophene-2-carboxylate (Minor)

To the DCM solution prepared in last step was bubbled NH₃ (gas) at 0 °C for 15 minutes. The reaction solution was stirred for 3 h at RT, then concentrated under reduced vacuum. The crude product was eluted from silica gel with EtOAc/PE (1:3). This resulted in 5.4 g (36% over two steps) of methyl 3-fluoro-5-sulfamoylthiophene-2-carboxylate (Compound **335B, major**) and 1.8 g (12% over two steps) of methyl 3-fluoro-4-sulfamoylthiophene-2-carboxylate (Compound **335A, minor**) both as a yellow solid. MS-ESI: 238 (M-1). Compound **335B:** ¹H NMR (300 MHz, DMSO-*d₆*) δ 8.07 (br s, 2H), 7.63 (s, 1H), 3.86 (s, 3H). ¹⁹F NMR (300 MHz, DMSO-*d₆*) δ -110.93. Compound **335A:** ¹H NMR (300 MHz, DMSO-*d₆*) δ 8.38 (d, *J =* 4.5 Hz, 1H), 7.87 (br s, 2H), 3.85 (s, 3H). ¹⁹F NMR (300 MHz, DMSO-*d₆*) δ -113.16.

Steps 4-6 used similar procedures for converting compound **335** to Intermediate **128** shown in Scheme 66 to afford Intermediate **133** from compound **335A.** MS-ESI: 353 (M+1).

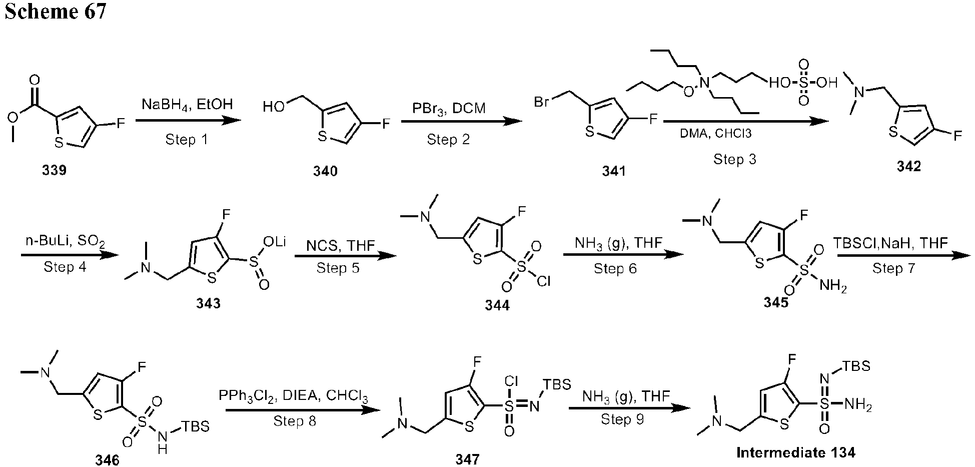

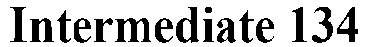

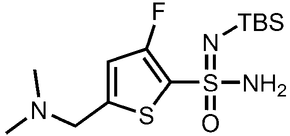

### N'-(tert-butyldimethylsilyl)-5-((dimethylamino)methyl)-3-fluorothiophene-2-sulfonimidamide

### Step 1: (4-Fluorothiophen-2-yl)methanol

To a stirred solution of methyl 4-fluorothiophene-2-carboxylate (10 g, 62.4 mmol) in ethanol (300 mL) in a 1 L round-bottom flask under nitrogen was added NaBH₄ (4.62 g, 125 mmol) in portions at 0°C in an ice/ethanol bath. The reaction solution was stirred for 16 h at RT. The reaction was then quenched by the addition of 50 mL of water. Then the mixture was concentrated and extracted with 3x100mL of EtOAc. The organic layer was combined and dried over anhydrous Na₂SO₄ and concentrated under vacuum. This resulted in 6.4 g (77.6%) of the title compound as a yellow oil. MS-ESI: 133 (M+1).

### Step 2: 2-(Bromomethyl)-4-fluorothiophene

To a stirred solution of (4-fluorothiophen-2-yl)methanol (8.5 g, 64.3 mmol) in DCM (70 mL) in a 250-mL round-bottom flask was added PBr₃ (19.2 g, 70.8 mmol) dropwise at 0 °C in an ice/ethanol bath. The resulting solution was stirred for 30 min at 0 °C. The resulting solution was allowed to react for an additional 12 h at RT. The reaction was then quenched by the addition of 50 mL of water. Then the mixture was concentrated and extracted with 3x100 mL of EtOAc. The organic layers were combined and dried over anhydrous Na₂SO₄ and concentrated under vacuum. The residue was eluted from a silica gel with EtOAc/PE (15/85). This resulted in 7.0 g (55.8%) of the title compound as yellow oil. MS-ESI: 194/196 (M+1).

### Step 3: 1-(4-Fluorothiophen-2-yl)-N,N-dimethylmethanamine

To a stirred solution of 2-(bromomethyl)-4-fluorothiophene (7.4 g, 37.9 mmol) in CHCl₃ (50 mL) in a 250-mL round-bottom flask was added butoxytributyl-λ⁴-azane sulfate (6.76 g, 19 mmol) and DMA (37 mL, 425 mmol) at RT. The resulting solution was stirred for 2 h at 60 °C. The reaction was then quenched by the addition of 50 mL of water. Then organic solvent was removed and the residue was extracted with 3x100 mL of EtOAc. The organic layers were combined, dried over anhydrous Na₂SO₄ and concentrated under vacuum. The residue was eluted from silica gel with EtOAc/PE (17/83). This resulted in 5.1 g (85%) of the title compound as a yellow solid. MS-ESI: 160 (M+1).

Steps 4-9 used similar procedures for converting compound **257** to Intermediate **113** shown in Scheme 54 to afford Intermediate **134** from compound **342.** MS-ESI: 352 (M+1).

**Schemes for amino pyridines Intermediates:** Schemes **68-83** illustrate the preparation of amino pyridines intermediates.

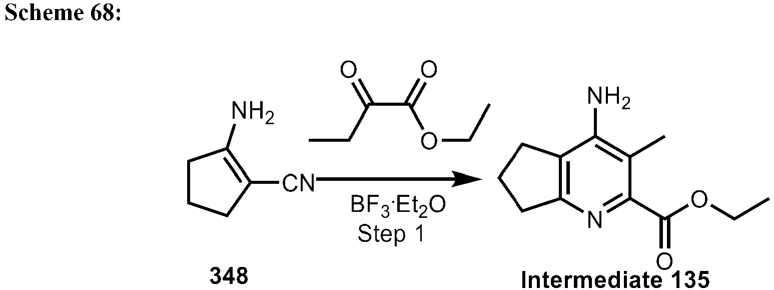

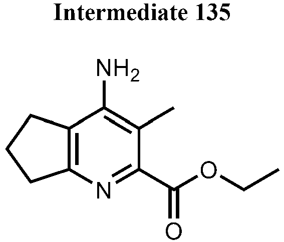

### Ethyl 4-amino-3-methyl-6,7-dihydro-5H-cyclopenta[b]pyridine-2-carboxylate

### Step 1: Ethyl 4-amino-3-methyl-6,7-dihydro-5H-cyclopenta[b]pyridine-2-carboxylate

To a stirred solution of 2-aminocyclopent-1-ene-1-carbonitrile (20 g, 185 mmol) in DCE (400 mL) in a 1 L round-bottom flask under nitrogen was added ethyl 2-oxobutanoate (24.1 g, 185 mmol) and BF₃·Et₂O (47% wt., 25 g, 370 mmol) dropwise at 0 °C. The resulting solution was stirred for 4 h at 80 °C in an oil bath. The resulting mixture was concentrated under vacuum. The resulting solution was diluted with 500 mL of water. The pH value of the solution was adjusted to 8 with K₂CO₃ (sat.). The resulting solution was extracted with 3x500 mL of EtOAc. The organic layers were dried over anhydrous Na₂SO₄. The resulting mixture was concentrated under vacuum. The residue was eluted from silica gel with EtOAc/PE (1:1). This resulted in 900 mg (2.2%) of the title compound as a yellow solid. MS-ESI: 221 (M+1).

**Table 29. The Intermediates in the following Table were prepared using the similar procedures for converting compound 348 to Intermediate 135 shown in Scheme 68 from appropriated starting materials.**

| Intermediate # | Structure | IUPAC Name | Exact Mass [M-H]⁻ |
|---|---|---|---|
| **Intermediate 136** | 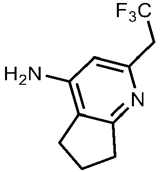 | 2-(2,2,2-Trifluoroethyl)-6,7-dihydro-5H-cyclopenta[b]pyridin-4-amine | 217 |
| **Intermediate 137** | 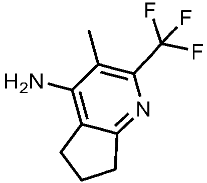 | 3-Methyl-2-(trifluoromethyl)-6,7-dihydro-5H-cyclopenta[b]pyridin-4-amine | 217 |
| **Intermediate 138** | 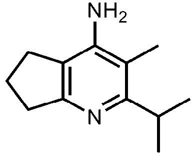 | 2-Isopropyl-3-methyl-6,7-dihydro-5H-cyclopenta[b]pyridin-4-amine | 191 |
| **Intermediate 139** | 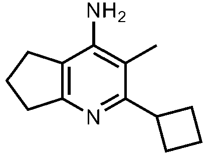 | 2-Cyclobutyl-3-methyl-6,7-dihydro-5H-cyclopenta[b]pyridin-4-amine | 203 |
| **Intermediate 140** | 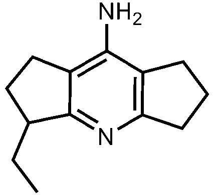 | 3-Ethyl-1,2,3,5,6,7-hexahydrodicyclopenta[b,e]pyridin-8-amine | 203 |
| **Intermediate 141** | 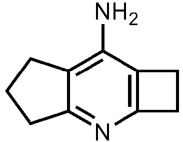 | 2,4,5,6-Tetrahydro-1H-cyclobuta[b]cyclopenta[e]pyridin-7-amine | 161 |

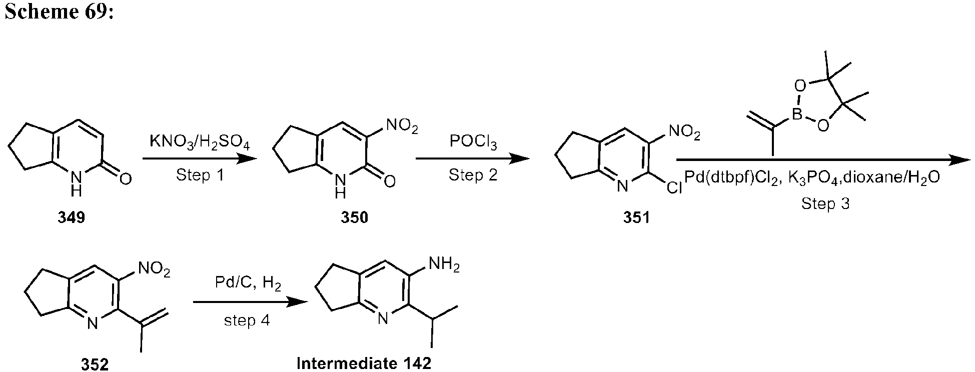

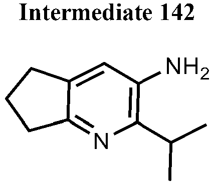

### 2-Isopropyl-6,7-dihydro-5H-cyclopenta[b]pyridin-3-amine

### Step 1: 3-Nitro-1,5,6,7-tetrahydro-2H-cyclopenta[b]pyridin-2-one

To a stirred solution of 1,5,6,7-tetrahydro-2H-cyclopenta[b]pyridin-2-one (5.0 g, 37 mmol) in DCM/conc. H₂SO₄ (10:1) (55 mL) in a 250 mL round-bottom flask was added KNO₃ (4.11 g, 40.7 mmol) in portions at 0 °C in an ice bath. The resulting solution was stirred for 4 h at RT. The resulting solution was poured into 200 mL ice/water. The mixture was extracted with 3x200 mL DCM. The organic layer was dried over anhydrous Na₂SO₄ and concentrated under vacuum. The residue was eluted from silica gel with EtOAc/PE (1:1). This resulted in 621 mg (9.3%) of the title compound as a yellow solid. MS-ESI: 181 (M+1).

### Step 2: 2-Chloro-3-nitro-6,7-dihydro-5H-cyclopenta[b]pyridine

To a stirred solution of 3-nitro-1,5,6,7-tetrahydro-2H-cyclopenta[b]pyridin-2-one (621 mg, 3.4 mmol) in MeCN (30 mL) in a 100 mL round-bottom flask was added POCl₃ (2.61 g, 17 mmol) dropwise at 0 °C. The resulting solution was stirred for 6 h at 60 °C and concentrated under vacuum. The mixture was dissolved in DCM. Then the solution was poured into 10 mL water/ice and extracted with 3x30 mL DCM. The organic layer was dried over anhydrous Na₂SO₄ and concentrated under vacuum. The residue was eluted from silica gel with EtOAc/PE (1:5). This resulted in 417 mg (61%) of the title compound as a yellow solid. MS-ESI: 199 (M+1).

### Step 3: 3-Nitro-2-(prop-1-en-2-yl)-6,7-dihydro-5H-cyclopenta[b]pyridine

To a stirred solution of 2-chloro-3-nitro-6,7-dihydro-5H-cyclopenta[b]pyridine (410 mg, 2.1 mmol) in 1,4-dioxane/H₂O (5:1) (24 mL) in a 100 mL round-bottom flask under nitrogen was added 4,4,5,5-tetramethyl-2-(prop-1-en-2-yl)-1,3,2-dioxaborolane (521 mg, 3.1 mmol), K₃PO₄ (890 mg, 4.2 mmol) and Pd(dtbpf)Cl₂ (135 mg, 0.21 mmol). The resulting solution was stirred for 6 h at 60 °C. The reaction mixture was diluted with 20 mL H₂O and extracted with EtOAc. The organic layer was dried over anhydrous Na₂SO₄ and concentrated under vacuum. The residue was eluted from silica gel with EtOAc/PE (1:3). This resulted in 235 mg (55.7%) of the title compound as a brown solid. MS-ESI: 205 (M+1).

### Step 4: 2-Isopropyl-6,7-dihydro-5H-cyclopenta[b]pyridin-3-amine

To a stirred solution of 3-nitro-2-(prop-1-en-2-yl)-6,7-dihydro-5H-cyclopenta[b]pyridine (235 mg, 1.15 mmol) in MeOH (10 mL) in a 50 mL round-bottom flask under nitrogen was added Pd/C (10% wt., 24 mg). The flask was evacuated and refilled three times with hydrogen. The resulting solution was stirred for 2 h at RT under an atmosphere of hydrogen with a balloon. The solids were filtered out. The resulting mixture was concentrated under vacuum. The residue was eluted from silica gel with EtOAc/PE (1:4). This resulted in 197 mg (97%) of the title compound as a white solid. MS-ESI: 177 (M+1).

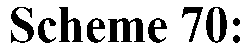

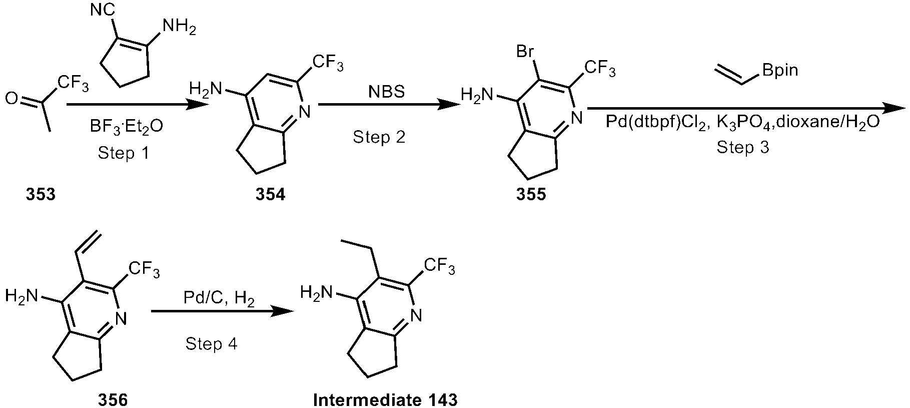

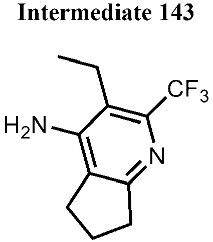

### 3-Ethyl-2-(trifluoromethyl)-6,7-dihydro-5H-cyclopenta[b]pyridin-4-amine

Step 1 used similar procedures for converting compound **348** to intermediate **135** shown in Scheme 68 to afford compound **354** from compound **353.** MS-ESI: 203 (M+1).

### Step 2: 3-Bromo-2-(trifluoromethyl)-6,7-dihydro-5H-cyclopenta[b]pyridin-4-amine

To a stirred solution of 2-(trifluoromethyl)-6,7-dihydro-5H-cyclopenta[b]pyridin-4-amine (1.5 g, 7.42 mmol) in MeCN (200 mL) in a 500-mL round-bottom flask under nitrogen was added NBS (10.6 g, 59.4 mmol). The resulting solution was stirred overnight at RT. The reaction was then quenched by the addition of 50 mL of sat. Na₂S₂O₃ (aq). The mixture was extracted with 3x100 mL of EtOAc. The organic layer was dried over anhydrous Na₂SO₄ and concentrated under vacuum. The residue was eluted from silica gel with EtOAc/PE (1/3). This resulted in 1.1 g (53%) of the title compound as a dark yellow solid. MS-ESI: 281/283 (M+1).

Steps 3-4 used similar procedures for converting compound **351** to Intermediate **142** shown in Scheme 69 to afford Intermediate **143** from compound **355.** MS-ESI: 231 (M+1).

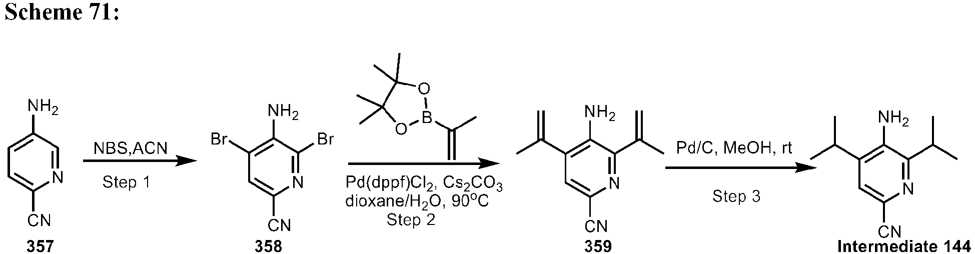

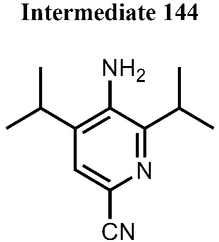

### 5-Amino-4,6-diisopropylpicolinonitrile

Step 1 used similar procedures for converting compound **354** to compound **355** shown in Scheme 70 to afford compound **358** from compound **357.** MS-ESI: 276/278 (M+1).

### Step 2: 5-Amino-4,6-di(prop-1-en-2-yl)picolinonitrile

To a stirred solution of 5-amino-4,6-dibromopicolinonitrile (3.3 g, 12 mmol) in dioxane (125 mL) and H₂O (17 mL) in a 500 mL round-bottom flask under nitrogen was added 4,4,5,5-tetramethyl-2-(prop-1-en-2-yl)- 1,3,2-dioxaborolane (5.0 g, 29.8 mmol), Cs₂CO₃ (11.7 g, 35.8 mmol) and Pd(dppf)Cl₂ (1.74 g, 2.4 mmol). The resulting solution was stirred overnight at 90 °C in an oil bath. The resulting solution was concentrated under vacuum. The reaction solution was diluted with 200 mL H₂O. The mixture was extracted with 3x200 mL EtOAc and the organic layers was dried over anhydrous Na₂SO₄ and concentrated under vacuum. The residue was eluted from silica gel with EtOAc/PE (1:3). This resulted in 1.96 g (82.5%) of the title compound as yellow oil. MS-ESI: 200 (M+1).

Step 3 used similar procedures for converting compound **352** to Intermediate **142** shown in Scheme 69 to afford Intermediate **144** from compound **359.** MS-ESI: 204 (M+1).

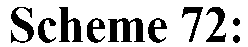

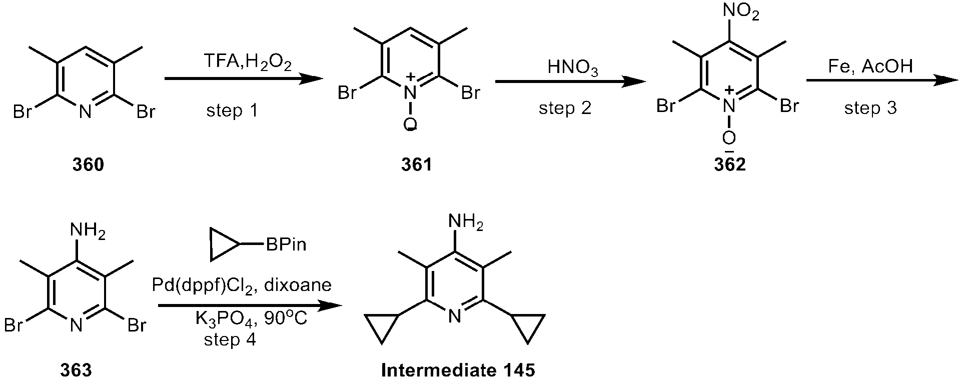

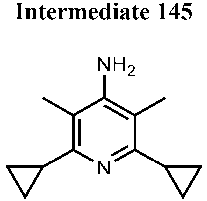

### 2,6-Dicyclopropyl-3,5-dimethylpyridin-4-amine

### Step 1: 2,6-Dibromo-3,5-dimethylpyridine 1-oxide

To a stirred solution of 2,6-dibromo-3,5-dimethylpyridine (2.0 g, 7.55 mmol) in TFA (20 mL) in a 100-mL round-bottom flask was added H₂O₂ (30% wt., 4.0 mL) dropwise at 0 °C. The resulting solution was stirred for 12 h at 80 °C in an oil bath. The resulting solution was diluted with 100 mL of water. The mixture was extracted with 3x100 mL of DCM. The organic layer was dried over anhydrous Na₂SO₄ and concentrated under vacuum. This resulted in 1.5 g (71%) of the title compound as a brown solid. MS-ESI: 280/282/284 (M+1).

### Step 2: 2,6-Dibromo-3,5-dimethyl-4-nitropyridine 1-oxide

To a stirred solution of 2,6-dibromo-3,5-dimethylpyridine 1-oxide (1.50 g, 5.34 mmol) in conc. H₂SO₄ (20 mL) in a 100-mL round-bottom flask was added HNO₃ (4.0 mL) dropwise at 0 °C in an ice bath. The resulting solution was stirred for 4 h at 60 °C in an oil bath. The reaction was poured into 100 mL of water/ice slowly. The resulting solution was extracted with 3x100 mL of DCM. The organic layer was dried over anhydrous Na₂SO₄ and concentrated under vacuum.

This resulted in 1.3 g (74.7%) of the title compound as a brown solid. MS-ESI: 325/327/329 (M+1).

### Step 3: 2,6-Dibromo-3,5-dimethylpyridin-4-amine

To a stirred solution of 2,6-dibromo-3,5-dimethyl-4-nitropyridine 1-oxide (1.3 g, 3.99 mmol) in AcOH (20 mL) in a 100-mL round-bottom flask under nitrogen was added Fe powder (1.11 g, 0.020 mmol). The resulting mixture was stirred for 14 h at 35 °C. The insoluble matter was filtered out and the filtrate was diluted with 50 mL of water. The resulting solution was extracted with 3x50 mL DCM. The organic layer was dried over anhydrous Na₂SO₄ and concentrated under vacuum. The residue was eluted from silica gel with EtOAc/PE (1:10 to 1:3). This resulted in 900 mg (80.6%) of the title compound as a brown solid. MS-ESI: 278/280/282 (M+1).

Step 4 used similar procedures for converting compound **358** to Intermediate **359** shown in Scheme 71 to afford Intermediate **145** from compound **363.** MS-ESI: 203 (M+1).

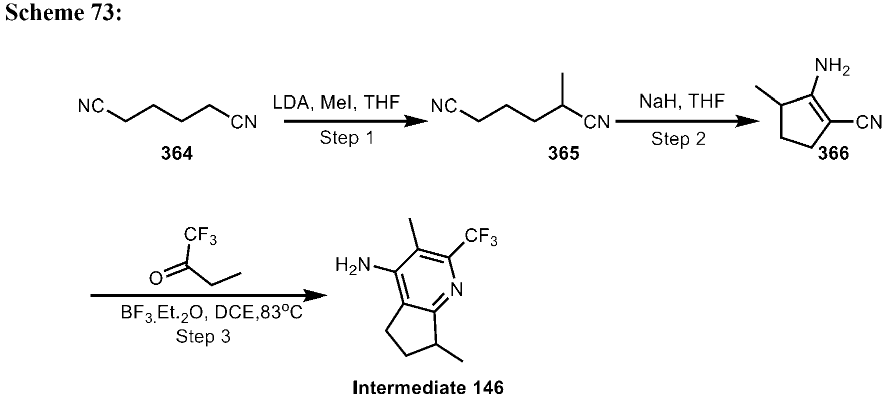

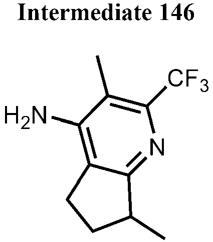

### 3,7-Dimethyl-2-(trifluoromethyl)-6,7-dihydro-5H-cyclopenta[b]pyridin-4-amine

### Step 1: 2-Methylhexanedinitrile

To a stirred of solution of LDA (76.4 mL, 153 mmol) in 200 mL THF in a 1-L 3-necked round-bottom flask under nitrogen was added a cold solution of adiponitrile (15 g, 139 mmol) in THF

(50 mL) dropwise at -78 °C in a liquid nitrogen/EtOH bath. The resulting solution was stirred for 1 h at -78 °C. To the reaction was added CH₃I (21.7 g, 153 mmol) in THF (50 mL) dropwise at - 78 °C. Then the reaction was stirred for 1 h at -10 °C. The reaction was quenched with sat. NH₄Cl (100 mL). The THF was removed and the mixture was extracted with 3x200 mL EtOAc and the organic layers were dried over anhydrous Na₂SO₄ and concentrated under vacuum. This resulted in 15 g (crude) title compound as yellow oil. MS-ESI: 123 (M+1).

### Step 2: 2-Amino-3-methylcyclopent-1-ene-1-carbonitrile

To a stirred solution of 2-methylhexanedinitrile (15 g, 123 mmol) in THF (450 mL) in a 1-L 3-necked round bottom flask under nitrogen was added NaH ( 60% wt. dispersion in mineral oil, 9.84 g, 246 mmol) in portions at 0 °C in an ice/EtOH bath. The reaction was stirred for 30 min at 0 °C and then overnight at 80 °C. The reaction was quenched with 200 mL H₂O. The mixture was extracted with 3x300 mL EtOAc. The combined organic layer was dried over anhydrous Na₂SO₄. The crude product was eluted from silica gel with EtOAc/PE (1:3). This resulted in 4.8 g (28%, over two steps) of the title compound as a yellow solid. MS-ESI: 123 (M+1).

Step 3 used similar procedures for converting compound **348** to Intermediate **135** shown in Scheme 68 to afford Intermediate **146** from compound **366.** MS-ESI: 231 (M+1).

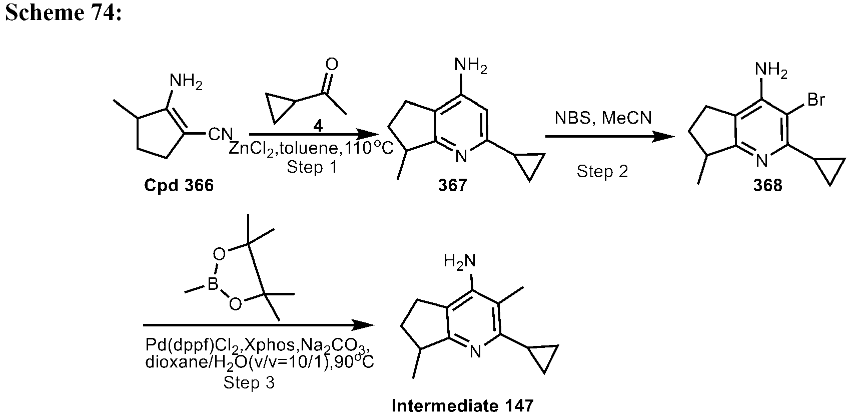

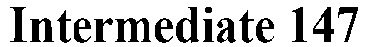

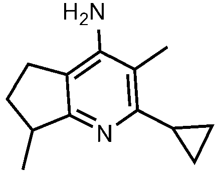

### 2-Cyclopropyl-3,7-dimethyl-6,7-dihydro-5H-cyclopenta[b]pyridin-4-amine

### Step 1: 2-Cyclopropyl-7-methyl-6,7-dihydro-5H-cyclopenta[b]pyridin-4-amine

To a stirred solution of 2-amino-3-methylcyclopent-1-ene-1-carbonitrile (25.4 g, 208 mmol) in toluene (150 mL) in a 500-mL round-bottom flask under nitrogen was added 1-cyclopropylethan-1-one (35 g, 416 mmol) and ZnCl₂ (31 g, 229 mmol) in portions. The resulting solution was stirred for 16 h at 110 °C in an oil bath. The reaction was then quenched by the addition of 200 mL of water. The pH value of the solution was adjusted to 14 with NaOH (3 M). The resulting solution was extracted with 3x300 mL of EtOAc. The organic layer was dried over anhydrous Na₂SO₄ and concentrated under vacuum. The residue was eluted from silica gel with EtOAc/PE (1:5). The crude product was further purified by Prep-HPLC with the following conditions: XBridge Prep C18 OBD Column 19×150 mm 5 um; Mobile Phase A: water (10 mM NH₄HCO₃+0.1%NH₃·H₂O), Mobile Phase B: ACN; Flow rate: 25 mL/min; Gradient: 17% B to 18% B over 10 min; 254/210 nm; Rt: 9.30 min This resulted in 1.5 g (3.8%) of the title compound as light yellow oil. MS-ESI: 189 (M+1).

Step 2 used similar procedures for converting compound **354** to compound **355** shown in Scheme 70 to afford compound **368** from compound **367.** MS-ESI: 267/269 (M+1).

### Step 3: 2-Cyclopropyl-3,7-dimethyl-6,7-dihydro-5H-cyclopenta[b]pyridin-4-amine

To a stirred solution of 3-bromo-2-cyclopropyl-7-methyl-6,7-dihydro-5H-cyclopenta[b]pyridin-4-amine (1.5 g, 5.61 mmol) in dioxane (25 mL) and H₂O (2.5 mL) in a 100-mL round-bottom flask under nitrogen was added Cs₂CO₃ (4.57 g, 14 mmol), Pd(dppf)Cl₂·CH₂Cl₂ (0.46 g, 0.56 mmol) and 2,4,4,5,5-pentamethyl-1,3,2- dioxaborolane (1.99 g, 14 mmol). The resulting solution was stirred for 16 h at 90 °C in an oil bath. The reaction was then quenched by the addition of 50 mL water. The resulting solution was extracted with 3x50 mL of DCM and the organic layers was dried over anhydrous Na₂SO₄ and concentrated under vacuum. The residue was eluted from silica gel with DCM/MeOH (10:1). This resulted in 0.60 g (53%) of the title compound as light yellow oil. MS-ESI: 203 (M+1).

**Table 30. The Intermediates in the following Table were prepared using the similar procedures for converting compound 366 to Intermediate 147 shown in Scheme 74 from appropriated starting materials.**

| Intermediate # | Structure | IUPAC Name | Exact Mass [M-H]⁻ |
|---|---|---|---|
| **Intermediate 148** | 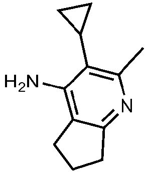 | 3-Cyclopropyl-2-methyl-6,7-dihydro-5H-cyclopenta[b]pyridin-4-amine | 189 |

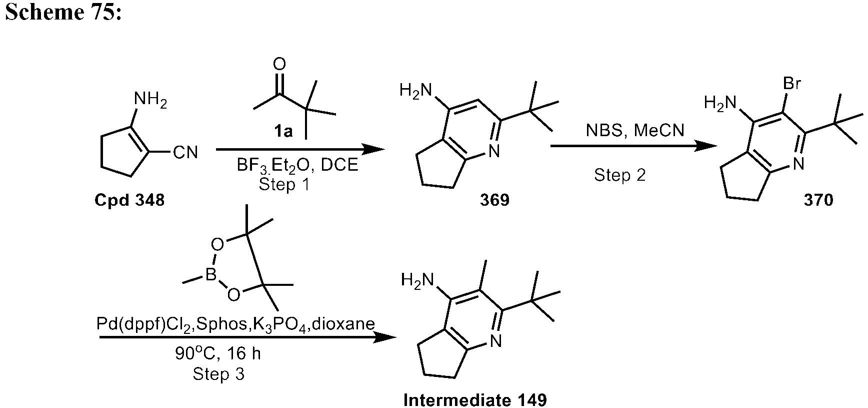

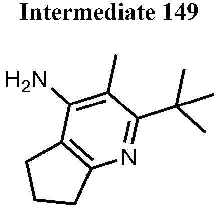

### 2-(Tert-butyl)-3-methyl-6,7-dihydro-5H-cyclopenta[b]pyridin-4-amine

Step 1 used similar procedures for converting compound **348** to Intermediate **135** shown in Scheme 68 to afford compound **369** from compound **348.** MS-ESI: 191 (M+1).

Step 2 used similar procedures for converting compound **354** to compound **355** shown in Scheme 70 to afford compound **370** from compound **369.** MS-ESI: 269/271 (M+1).

### Step 3: 2-(Tert-butyl)-3-methyl-6,7-dihydro-5H-cyclopenta[b]pyridin-4-amine

To a stirred solution of 3-bromo-2-(tert-butyl)-6,7-dihydro-5H-cyclopenta[b]pyridin-4-amine (550 mg, 2.04 mmol) in a 100-mL round-bottom flask under nitrogen was added Cs₂CO₃ (1.33 g, 4.09 mmol), 2,4,4,5,5- pentamethyl-1,3,2-dioxaborolane (435 mg, 3.06 mmol), Pd(dppf)Cl₂ (145 mg, 0.20 mmol) and SPhos (84 mg, 0.20 mmol). The resulting solution was stirred overnight at 80°C. The reaction solution was diluted with 20 mL H₂O. The resulting mixture was extracted with 3x50 mL EtOAc. The organic layer was dried over anhydrous Na₂SO₄ and concentrated under vacuum. The residue was eluted from silica gel with DCM/MeOH (50:1). This resulted in 243 mg (58%) of the title compound as a brown solid. MS-ESI: 205 (M+1).

**Table 31. The Intermediates in the following Table were prepared using the similar procedures for converting compound 348 to Intermediate 149 shown in Scheme 75 from appropriated starting materials.**

| Intermediate # | Structure | IUPAC Name | Exact Mass [M-H]⁻ |
|---|---|---|---|
| **Intermediate 150** | 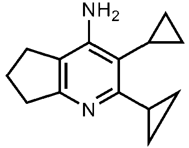 | 2,3-Dicyclopropyl-6,7-dihydro-5H-cyclopenta[b]pyridin-4-amine | 215 |
| **Intermediate 151** | 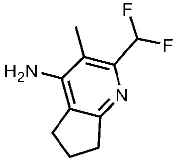 | 2-(Difluoromethyl)-3-methyl-6,7-dihydro-5H-cyclopenta[b]pyridin-4-amine | 199 |
| **Intermediate 152** | 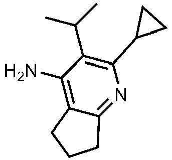 | 2-Cyclopropyl-3-isopropyl-6,7-dihydro-5H-cyclopenta[b]pyridin-4-amine | 217 |

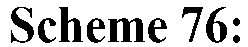

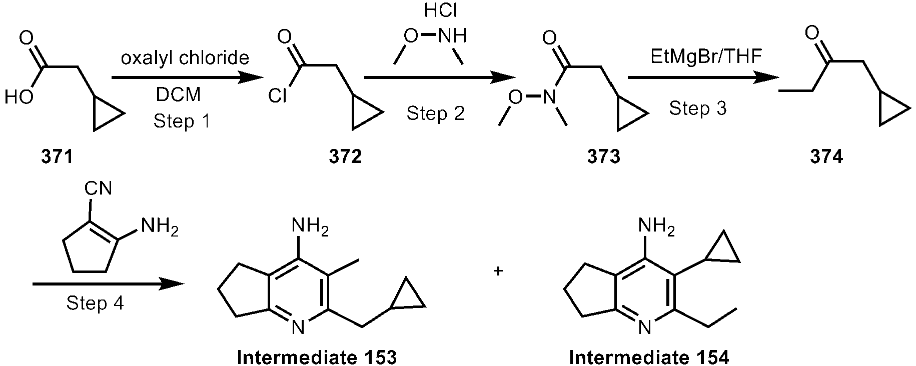

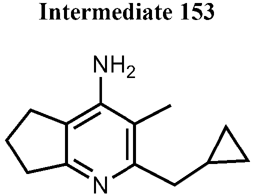

### 2-(Cyclopropylmethyl)-3-methyl-6,7-dihydro-5H-cyclopenta[b]pyridin-4-amine

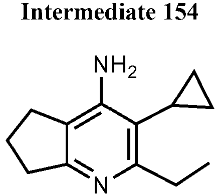

### 3-Cyclopropyl-2-ethyl-6,7-dihydro-5H-cyclopenta[b]pyridin-4-amine

### Step 1: 2-Cyclopropylacetyl chloride

To a stirred solution of 2-cyclopropylacetic acid (10 g, 99.9 mmol) in DCM (200 mL) in a 500-mL round-bottom flask under nitrogen was added oxalyl chloride (14 g, 110 mmol) dropwise at 0 °C in ice bath. The resulting solution was stirred for 2 h at RT. The resulting mixture was concentrated under vacuum. This resulted in 10 g (crude) of the title compound as colorless oil.

### Step 2: 2-Cyclopropyl-N-methoxy-N-methylacetamide

To a stirred solution of 2-cyclopropylacetyl chloride (10 g, crude) in DCM (200 mL) in a 500-mL round-bottom flask was added TEA (25.7 g, 254 mmol), followed by the addition of N,O-dimethylhydroxylamine hydrochloride (16.4 g, 169 mmol) in DCM (50 mL) dropwise at 0 °C in an ice bath. The resulting solution was stirred for 2 h at RT. The resulting solution was diluted with 200 mL of water. The mixture was extracted with 3x200 mL of DCM and the organic layers were dried over anhydrous Na₂SO₄ and concentrated under vacuum. This resulted in 22 g (91%) of the title compound as colorless oil. MS-ESI: 144 (M+1).

### Step 3: 1-Cyclopropylbutan-2-one

To a stirred solution of 2-cyclopropyl-N-methoxy-N-methylacetamide (20 g, 140 mmol) in THF (200 mL) in a 500-mL round-bottom flask under nitrogen was added EtMgBr in Et₂O (2 M, 91 mL, 182 mmol) dropwise at 0 °C in an ice bath. The resulting solution was stirred for 14 h at RT. The reaction was then quenched by the addition of 200 mL water. The mixture was extracted with 3x200 mL of DCM and the organic layers were dried over anhydrous Na₂SO₄ and concentrated under vacuum. This resulted in 13 g (83%) of the title compound as brown liquid. MS-ESI: 113 (M+1).

### Step 4: 2-(Cyclopropylmethyl)-3-methyl-6,7-dihydro-5H-cyclopenta[b]pyridin-4-amine and 3-Cyclopropyl-2-ethyl-6,7-dihydro-5H-cyclopenta[b]pyridin-4-amine

To a stirred solution of 1-cyclopropylbutan-2-one (9.64 g, 89.1 mmol) in DCE (100 mL) in a 250-mL round-bottom flask under nitrogen was added 1-cyclopropylbutan-2-one (10 g, 89 mmol) and BF₃·Et₂O (47% wt., 19 g, 134 mmol) dropwise at 0 °C in an ice bath. The resulting solution was stirred for 14 h at 80 °C in an oil bath. The resulting solution was diluted with 100 mL of water. The pH value of the solution was adjusted to 9 with NaOH (3 M). The resulting solution was extracted with 3x300 mL of DCM. The organic layer was dried over anhydrous Na₂SO₄ and concentrated under vacuum. The residue was eluted from silica gel with DCM/MeOH (100:1 to 10:1) and resulted in a mixture. Then the mixture was separated by SFC with the following conditions: Ultimate XB-NH2, 21.2*250 mm; 5um ; Mobile Phase A: CO₂, Mobile Phase B:EtOH (8 mM NH₃·MeOH); Flow rate: 60 mL/min; Gradient: 35% B over 9 min; UV 220 nm; Rt1: 6.25 min **Intermediate 153;** Rt2: 7.40 min **Intermediate 154;** Injection Volumn:1.1 ml; Number of runs:100; this resulted in **Intermediate 153 (4.0 g)** and **Intermediate 154 (2.0 g).** MS-ESI: 203 (M+1).

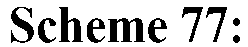

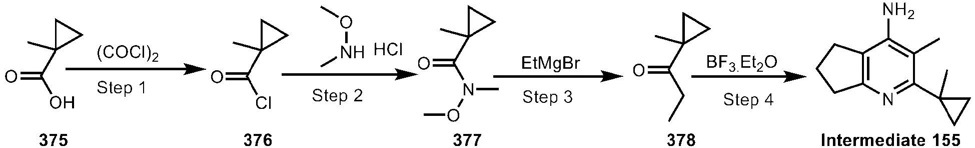

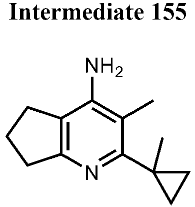

### 3-Methyl-2-(1-methylcyclopropyl)-6,7-dihydro-5H-cyclopenta[b]pyridin-4-amine

Steps 1-4 used similar procedures for converting compound **371** to Intermediate **153** shown in Scheme 76 to afford Intermediate **155** from compound **375.** MS-ESI: 203 (M+1).

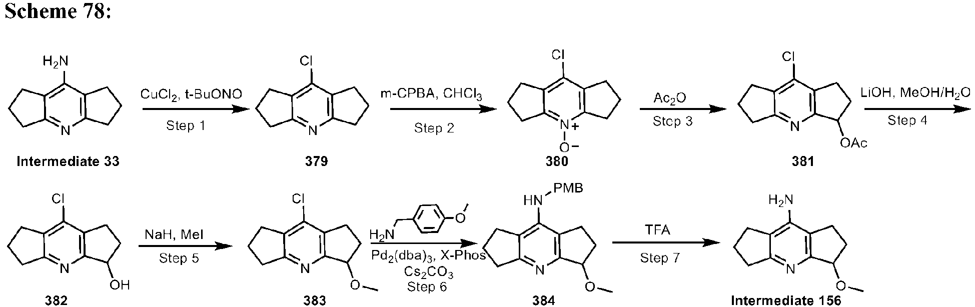

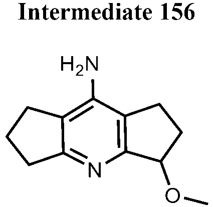

### 3-Methoxy-1,2,3,5,6,7-hexahydrodicyclopenta[b,e]pyridin-8-amine

### Step 1: 8-Chloro-1,2,3,5,6,7-hexahydrodicyclopenta[b,e]pyridine

To a stirred solution of 1,2,3,5,6,7-hexahydrodicyclopenta[b,e]pyridin-8-amine (1.75 g, 10 mmol) in CHCl₃ (80 mL) in a 500-mL round-bottom flask under nitrogen was added tert-butyl nitrite (2.06 g, 20 mmol) and CuCl₂ (2.70 g, 20 mmol) in portions at RT. The resulting mixture was stirred overnight at RT. The resulting mixture was washed with 3x50 mL of water and dried over anhydrous Na₂SO₄. The resulting mixture was filtered. The filtrate was concentrated under reduced pressure. The residue was eluted from silica gel with PE/EtOAc (10:1). This resulted in 1.1 g (57%) of the title compound as yellow oil. MS-ESI: 194/196 (M+1).

### Step 2: 8-Chloro-1,2,3,5,6,7-hexahydrodicyclopenta[b,e]pyridine 4-oxide

To a stirred solution of 8-chloro-1,2,3,5,6,7-hexahydrodicyclopenta[b,e]pyridine (1.10 g, 5.7 mmol) in CHCl₃ (60 mL) in a 250-mL round-bottom flask was added m-CPBA (1.96 g, 11.4 mmol) in portions at 0 °C. The resulting mixture was stirred for 3h at RT. The reaction was quenched with sat.Na₂SO₃ at 0 °C. The resulting mixture was washed with 3x15 mL of water and dried with anhydrous Na₂SO₄. The resulting mixture was filtered. The filtrate was concentrated under reduced pressure. The residue was eluted from silica gel with PE/EtOAc (3:1). This resulted in 1.03 g (86.5%) of the title compound as yellow oil. MS-ESI: 210/212 (M+1).

### Step 3: 8-Chloro-1,2,3,5,6,7-hexahydrodicyclopenta[b,e]pyridin-3-yl acetate

A solution of 8-chloro-1,2,3,5,6,7-hexahydrodicyclopenta[b,e]pyridine 4-oxide (1.0 g, 4.8 mmol) in Ac₂O (50 mL) in a 100-mL round-bottom flask was stirred overnight at RT. The resulting mixture was concentrated under reduced pressure. This resulted in 550 mg (crude) of the title compound as yellow oil. MS-ESI: 252/254 (M+1).

### Step 4: 8-Chloro-1,2,3,5,6,7-hexahydrodicyclopenta[b,e]pyridin-3-ol

To a stirred solution of 8-chloro-1,2,3,5,6,7-hexahydrodicyclopenta[b,e]pyridin-3-yl acetate (550 mg, crude) in MeOH (25 mL) and H₂O (5 mL) in a 100-mL round-bottom flask was added NaOH (224 mg, 5.6 mmol) in portions at 0 °C. The reaction solution was stirred for 6 h at 60 °C. The mixture was cooled to RT and concentrated under vacuum. The residue was eluted from silica gel with DCM/MeOH (10:1). This resulted in 550 mg (55%, over two steps) of the title compound as colorless oil. MS-ESI: 210/212 (M+1).

### Step 5: 8-Chloro-3-methoxy-1,2,3,5,6,7-hexahydrodicyclopenta[b,e]pyridine

To a stirred solution of 8-chloro-1,2,3,5,6,7-hexahydrodicyclopenta[b,e]pyridin-3-ol (550 mg, 2.6 mmol) in THF (25 mL) in a 100-mL 3-necked round-bottom flask under nitrogen was added NaH (60% wt. dispersion in mineral oil, 208 mg, 5.2 mmol) in portions at 0 °C in an ice bath. The resulting mixture was stirred for 30 min at 0 °C. To the above solution was added CH₃I (745 mg, 5.2 mmol) at 0 °C. The resulting mixture was stirred for 30 min at 0 °C. The reaction was quenched with water/ice (5 mL) at 0 °C. The mixture was extracted with 3x15 mL EtOAc. The organic layers were dried over anhydrous Na₂SO₄ and concentrated under vacuum. The residue was eluted from silica gel with PE/EtOAc (10:1). This resulted in (500 mg, 85 %) of the title compound as yellow oil. MS-ESI: 224/226 (M+1).

### Step 6: 3-Methoxy-N-(4-methoxybenzyl)-1,2,3,5,6,7-hexahydrodicyclopenta[b,e]pyridin-8-amine

To a stirred solution of 8-chloro-3-methoxy-1,2,3,5,6,7-hexahydrodicyclopenta[b,e]pyridine (500 mg, 2.2 mmol) in 1,4-dioxane (15 mL) and H₂O (1 mL) in a 100-mL round-bottom flask under nitrogen was added (4-methoxyphenyl)methanamine (613 mg, 4.4 mmol) and Cs₂CO₃ (1.45 g, 4.4 mmol) and Pd₂(dba)₃ (20.5 mg, 0.022 mmol) and X-Phos (21.31 mg, 0.045 mmol). The resulting solution was stirred overnight at 100 °C. The resulting mixture was concentrated under reduced pressure. The resulted mixture was diluted with H₂O (10 mL) and extracted with 3x15 mL EtOAc. The organic layer was dried over anhydrous Na₂SO₄ and concentrated under vacuum. The residue was eluted from silica gel with PE/EtOAc (1:1). This resulted in 370 mg (51%) of the title compound as a yellow solid. MS-ESI: 325 (M+1).

### Step 7: 3-Methoxy-1,2,3,5,6,7-hexahydrodicyclopenta[b,e]pyridin-8-amine

A solution of 3-methoxy-N-(4-methoxybenzyl)-1,2,3,5,6,7-hexahydrodicyclopenta[b,e]pyridin-8-amine (370 mg, 1.14 mmol) in TFA (10 mL) was stirred for 3 h at 80 °C. The resulting mixture was concentrated under vacuum. This resulted in 450 mg (crude) of the title compound as a yellow solid. MS-ESI: 205 (M+1).

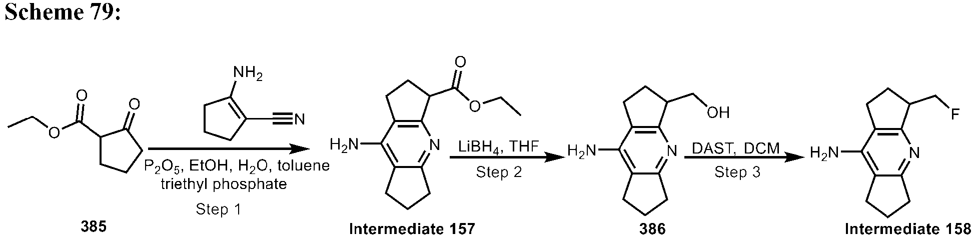

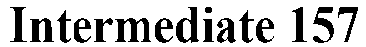

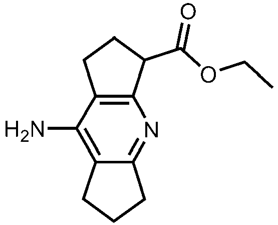

### Ethyl 8-amino-1,2,3,5,6,7-hexahydrodicyclopenta[b,e]pyridine-3-carboxylate

### Step 1: Ethyl 8-amino-1,2,3,5,6,7-hexahydrodicyclopenta[b,e]pyridine-3-carboxylate

To a stirred solution of P₂O₅ (27.3 g, 192 mmol) in toluene (100 mL) in a 250-mL round-bottom flask under nitrogen was added triethyl phosphate (22.5 g , 123 mmol) dropwise at 55~60 °C, followed by the addition of ethanol (2.20 g, 9.55 mmol) dropwise at 55~60 °C. The reaction was stirred for 30 min. The solution was cooled to RT, the mixture of 2-aminocyclopent-1-ene-1-carbonitrile (3.81 g, 35 mmol) and ethyl 2-oxocyclopentane-1-carboxylate (5.0 g, 32 mmol) in toluene (20 mL) at RT. Then the solution was stirred for 3.5 h at 55 °C. The solution was cooled to RT, water (100 mL) was added dropwise below 40 °C in an ice bath. Then the solution was stirred at 55 °C for 30 min. The aqueous phase collected and adjusted the pH value to 8 with sat. Na₂CO₃ solution. The resulting solution was extracted with 3x150 mL of EtOAc, the combined organic layer was dried over anhydrous Na₂SO₄ and concentrated under vacuum. The residue was eluted from silica gel with DCM/MeOH (17:1). This resulted in 1.1 g (15%) of the title compound as a brown solid. MS-ESI: 247 (M+1).

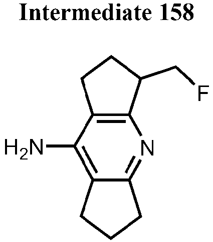

### 3-(Fluoromethyl)-1,2,3,5,6,7-hexahydrodicyclopenta[b,e]pyridin-8-amine

### Step 2: (8-Amino-1,2,3,5,6,7-hexahydrodicyclopenta[b,e]pyridin-3-yl)methanol

To a stirred solution of ethyl 8-amino-1,2,3,5,6,7-hexahydrodicyclopenta[b,e]pyridine-3-carboxylate (680 mg, 2.76 mmol) in THF (10 mL) in a 50-mL round-bottom flask under nitrogen was added LiBH₄ (180 mg, 8.28 mmol) in portions at 0 °C in an ice bath. The resulting solution was stirred for 3 h at RT. The reaction was then quenched by the addition of 5 mL water. The resulting mixture was concentrated under vacuum. The residue was eluted from silica gel with DCM/MeOH (8:1). This resulted in 220 mg (39%) of the title compound as an off-white solid. MS-ESI: 205 (M+1).

### Step 3: 3-(Fluoromethyl)-1,2,3,5,6,7-hexahydrodicyclopenta[b,e]pyridin-8-amine

To a stirred solution of (8-amino-1,2,3,5,6,7-hexahydrodicyclopenta[b,e]pyridin-3-yl)methanol (50 mg, 0.25 mmol) in DCM (10 mL) in a 50-mL 3-necked round-bottom flask under nitrogen was added DAST (197 mg, 1.22 mmol) dropwise 0 °C in an ice bath. The resulting solution was stirred for 2 h at RT. The reaction was then quenched by the addition of 5 mL MeOH. The resulting mixture was concentrated under vacuum. The residue was eluted from silica gel with DCM/MeOH (10:1). The crude product was purified by Prep-HPLC under the following conditions: XBridge Prep OBD C18 Column 30×150 mm 5 um; Mobile Phase A: water (10 mM NH₄HCO₃), Mobile Phase B: ACN; Flow rate: 60 mL/min; Gradient: 25% B to 42% B in 7 min; 254/210 nm; Rt: 6.83 min. This resulted in 75 mg (99%) of the title compound as a light yellow oil. MS-ESI: 247 (M+1).

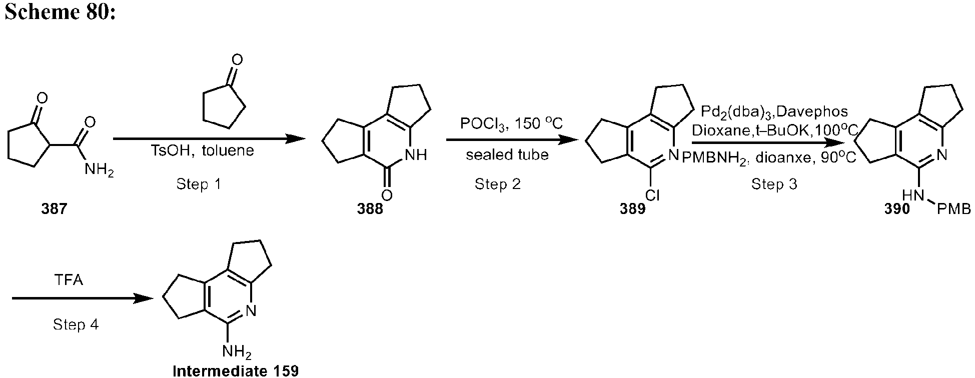

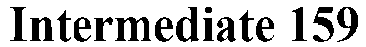

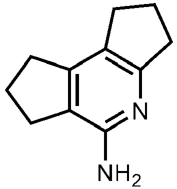

### 1,2,3,6,7,8-Hexahydrodicyclopenta[b,d]pyridin-5-amine

### Step 1: 2,3,4,6,7,8-Hexahydrodicyclopenta[b,d]pyridin-5(1H)-one

To a stirred solution of 2-oxocyclopentane-1-carboxamide (2.0 g, 15.7 mmol) in toluene (80 mL) in a 250-mL 3-necked round-bottom flask under nitrogen was added cyclopentanone (1.32 g, 15.7 mmol) and TsOH (1.63 g, 9.44 mmol). The resulting solution was stirred for 4 h at 120 °C in an oil bath. The mixture was concentrated under reduced pressure. The residue was eluted from silica gel with DCM/MeOH (10:1). This resulted in 301 mg (11%) of the title compound as a yellow solid. MS-ESI: 176 (M+1).

### Step 2: 5-Chloro-1,2,3,6,7,8-hexahydrodicyclopenta[b,d]pyridine

A stirred solution of 2,3,4,6,7,8-hexahydrodicyclopenta[b,d]pyridin-5(1H)-one (301 mg, 1.72 mmol) in phosphoryl trichloride (25 mL) in a 100-mL round-bottom flask was stirred for 16 h at 150 °C in an oil bath. The reaction was then quenched by the addition of H₂O (200 mL). The pH value of the solution was adjusted to 7 with NaOH (3 M). The resulting solution was extracted with 3x150 mL of EtOAc. The organic layer was dried over anhydrous Na₂SO₄ and concentrated under vacuum. The crude product was purified by Prep-HPLC under the following conditions: XBridge Prep C18 OBD Column 19×150 mm 5 um; Mobile Phase A: water (10 mM NH₄HCO₃+0.1%NH₃·H₂O), Mobile Phase B: ACN; Flow rate: 25 mL/min; Gradient: 35% B to 80% B in 8 min; 254 /210 nm; Rt: 7.00 min. This resulted in 152 mg (46%) of the title compound as a white solid. MS-ESI: 194/196 (M+1).

### Step 3: N-(4-methoxybenzyl)-1,2,3,6,7,8-hexahydrodicyclopenta[b,d]pyridin-5-amine

To a stirred solution of 5-chloro-1,2,3,6,7,8-hexahydrodicyclopenta[b,d]pyridine (156 mg, 0.81 mmol) in dioxane (25 mL) in a 100-mL 3-necked round-bottom flask under nitrogen was added 1-(4-methoxyphenyl)methanamine (221 mg, 1.62 mmol), t-BuOK (272 mg, 2.43 mmol), Pd₂(dba)₃ (74 mg, 0.081 mmol), and Davephos (32 mg, 0.081 mmol). The resulting solution was stirred for 4 h at 100 °C in an oil bath. The reaction mixture was diluted with H₂O (20 mL) and extracted with 3x50 mL EtOAc. The organic layer was dried over anhydrous Na₂SO₄ and concentrated under vacuum. The residue was eluted from silica gel with DCM/MeOH (10: 1). This resulted in 168 mg (71%) of the title compound as yellow oil. MS-ESI: 295 (M+1).

### Step 4: 1,2,3,6,7,8-Hexahydrodicyclopenta[b,d]pyridin-5-amine

To a stirred solution of N-(4-methoxybenzyl)-1,2,3,6,7,8-hexahydrodicyclopenta[b,d]pyridin-5-amine (100 mg, 0.34 mmol) in CHCl₃ (5 mL) in a 50-mL round-bottom flask was added TFA (5 mL) dropwise at 0 °C. The resulting solution was stirred for 2 h at 65 °C in an oil bath. The solution was concentrated under vacuum. The residue was dissolved in DCM (10 mL). The pH value of the solution was adjusted to 10 with NaOH (3 M). The resulting mixture was extracted with 3x20 mL of EtOAc. The organic layer was dried over anhydrous Na₂SO₄ and concentrated under vacuum. The residue was eluted from silica gel with DCM/MeOH (10:1). This resulted in 50 mg (84%) of the title compound as a white solid. MS-ESI: 175 (M+1).

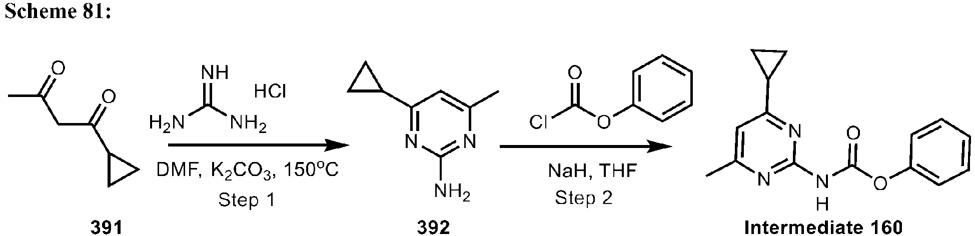

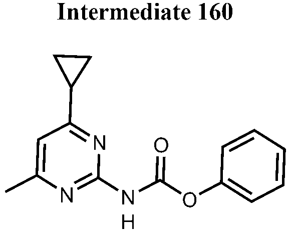

### Phenyl (4-cyclopropyl-6-methylpyrimidin-2-yl)carbamate

### Step 1: 4-Cyclopropyl-6-methylpyrimidin-2-amine

To a stirred solution of 1-cyclopropylbutane-1,3-dione (2.0 g, 16 mmol) in DMF (20 mL) in a 100-mL sealed tube was added K₂CO₃ (4.42 g, 32 mmol) and guanidine hydrochloride (3.02 g, 32 mmol). The resulting solution was stirred for 4 h at 150 °C in an oil bath. The resulting mixture was concentrated under vacuum. The mixture was dissolved into 20 mL EtOAc. The resulting mixture was washed with 2x50 mL of H₂O. The organic layer was dried over anhydrous Na₂SO₄ and concentrated under vacuum. This resulted in 1.6 g (68%) of the title compound as a yellow solid. MS-ESI: 150 (M+1).

### Step 2: Phenyl (4-cyclopropyl-6-methylpyrimidin-2-yl)carbamate

To a stirred solution of 4-cyclopropyl-6-methylpyrimidin-2-amine (100 mg, 0.67 mmol) in THF (20 mL) in a 50-mL round-bottom flask under nitrogen was added NaH (60% wt. dispersion in mineral oil, 53.6 mg, 1.34 mmol) and phenyl chloroformate (115 mg, 0.74 mmol). The resulting solution was stirred for 24 h at RT. The reaction solution was quenched with H₂O (10 mL). The mixture was extracted with 3x20 mL EtOAc. The organic layer was dried over anhydrous Na₂SO₄ and concentrated under vacuum. This resulted in 150 mg (83%) of the title compound as a white solid. MS-ESI: 270 (M+1).

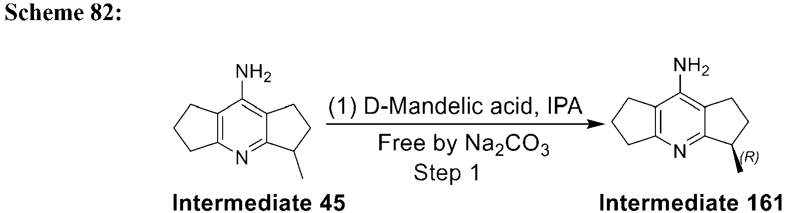

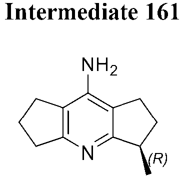

### (R)-3-methyl-1,2,3,5,6,7-hexahydrodicyclopenta[b,e]pyridin-8-amine

### Step 1: (R)-3-Methyl-1,2,3,5,6,7-hexahydrodicyclopenta[b,e]pyridin-8-amine

A stirred solution of 3-methyl-1,2,3,5,6,7-hexahydrodicyclopenta[b,e]pyridin-8-amine (47 g, 250 mmol) in isopropyl alcohol (423 mL) was heated to 80 °C in a 1 L 3-neck flask with condenser and stirring bar. The solution was kept stirring for 0.5 h. The solution was cooled to about 50 °C. The solution was filtered at that temperature though a Buchner funnel to remove insoluble impurities. The solid was washed with isopropyl alcohol (3x10 mL). The filtrate was transfer to a round bottle and concentrated to 350 g. The solution was transferred to a 2 L 3-necked round-bottom flask equipped with a mechanical stir and reflux condenser in an oil bath. The round bottle was washed with 27 g isopropyl alcohol to transfer all the material to the 2 L 3-necks flask. The resulting solution was heated to 80 °C and a solution of (R)-(-)-Mandelic acid (38 g, 250 mmol) in isopropyl alcohol (188 mL) was added dropwise at that temperature. The resulting mixture was stirred at 80 °C for 5 min. The system was cooled in 5 °C step and a small amount of the seed crystals of the product was added. If the seed dissolved, repeat the above operation. When the system was cooled to 65 °C, the seed was undissolved and crystals started to grow, the system turned to cloudy slowly, the solution was stirred for 30 minute at this temperature. From then on, the solution was stirred and maintained for 30 minute while the temperature was cooled in 5 °C steps until the system temperature was 40 °C. Turn off the heating switch of oil bath and the mixture was slowly cooled to 28 °C. After 16 h, the ee value of solid was monitored (96%ee). The solid was collected by filtered. The resulting solid was slurry in isopropyl alcohol (180 mL) for 1h and filtrated again. The filtrate was decanted, and the precipitate was washed with chromatographic isopropyl alcohol (30 mL) to give the title compound as a white solid (35 g, 40.5% yield, 98%ee, which contain 13.8% IPA). The solid was dissolved in water (420 mL) and concentrated to afford 29.2 g white solid (KF=5.1%). The mother liquid was combined and concentrated to afford 56.7 g S-isomer (75%ee, contain 17.8% IPA) as a foam. Then 26.9 g (R) salt was freed by aq. Na₂CO₃ (4 M, 500 mL) and exacted with 3x300 mL EA, then the organic phase was washed with 500 mL of brine and dried over anhydrous Na₂SO₄ and concentrated under vacuum. This resulted in 14.3g (38% yield, 98%ee, LCAP=96%) of the title compound as a white solid. MS-ESI: 189 (M+1). Chiral analysis method: Column, CHIRALPAK IC 4.6*150 mm, 3 µm. Mobile phase: Hex(0.1%DEA):IPA=85:15. Column Temperature: 30°C. Flow Rate: 1.0 mL/min. Monitor: UV 254 nm. Intermediate **161** is the first peak with a retention time of 5.8 min. The S enantiomer is the second peak with a retention time of 7.0 min.

In a separate experiment, crystals were obtained from Intermediate 45 and (S)-(+)-Mandelic acid using similar procedures. The structure of this salt was solved by single crystal X-ray crystallography to be the (S) enantiomer of Intermediate 45. Therefore, Intermediate 161 has the (R) configuration.

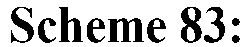

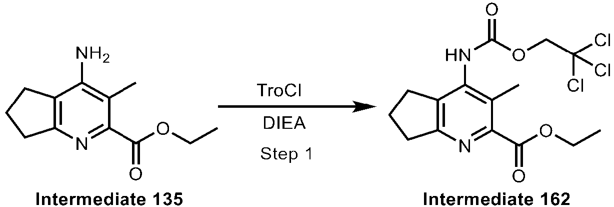

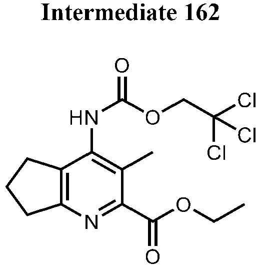

### Ethyl 3-methyl-4-(((2,2,2-trichloroethoxy)carbonyl)amino)-6,7-dihydro-5H-cyclopenta[b]pyridine-2-carboxylate

### Step 1: Ethyl 3-methyl-4-(((2,2,2-trichloroethoxy)carbonyl)amino)-6,7-dihydro-5H-cyclopenta[b] pyridine-2-carboxylate

To a stirred solution of ethyl 4-amino-3-methyl-5H,6H,7H-cyclopenta[b]pyridine-2-carboxylate (500 mg, 2.27 mmol) in THF (30 mL) in a 100-mL round-bottom flask under nitrogen was added DIEA (587 mg, 4.54 mmol) at RT, followed by the addition of 2,2,2-trichloroethyl chloroformate (962 mg, 4.54 mmol) dropwise at RT. The resulting solution was stirred overnight at RT. The reaction solution was quenched with H₂O (10 mL). Th mixture was extracted with 3x50 mL EtOAc. The organic layer was dried over anhydrous Na₂SO₄ and concentrated under vacuum. The residue was eluted from silica gel with EtOAc/PE(1:1). This resulted in 610 mg (68%) of the title compound as a brown yellow solid. MS-ESI: 395/397/399 (M+1).

**Table 32. The Intermediates in the following Table were prepared using the similar procedures for converting Intermediate 135 to Intermediate 162 shown in Scheme 83 from appropriated starting materials.**

| Intermediate # | Structure | IUPAC Name | Exact Mass [M-H]⁻ |
|---|---|---|---|
| **Intermediate 163** | 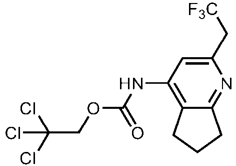 | 2,2,2-trichloroethyl (2-(2,2,2-trifluoroethyl)-6,7-dihydro-5H-cyclopenta[b]pyridin-4-yl)carbamate | 391/393/395 |
| **Intermediate 164** | 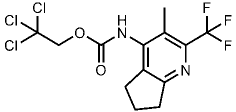 | 2,2,2-trichloroethyl (3-methyl-2-(trifluoromethyl)-6,7-dihydro-5H-cyclopenta[b]pyridin-4-yl)carbamate | 391/393/395 |
| **Intermediate 165** | 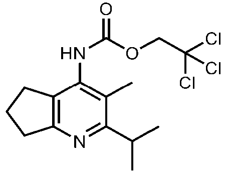 | 2,2,2-trichloroethyl (2-isopropyl-3-methyl-6,7-dihydro-5H-cyclopenta[b]pyridin-4-yl)carbamate | 365/367/369 |
| **Intermediate 166** | 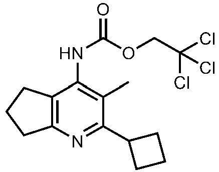 | 2,2,2-trichloroethyl (2-cyclobutyl-3-methyl-6,7-dihydro-5H-cyclopenta[b]pyridin-4-yl)carbamate | 377/379/381 |
| **Intermediate 167** | 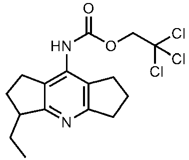 | 2,2,2-trichloroethyl (3-ethyl-1,2,3,5,6,7-hexahydrodicyclopenta[b,e]pyridin-8-yl)carbamate | 377/379/281 |
| **Intermediate 168** | 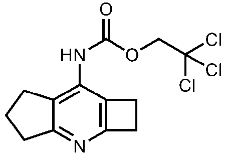 | 2,2,2-trichloroethyl (2,4,5,6-tetrahydro-1H-cyclobuta[b]cyclopenta[e]pyridin-7-yl)carbamate | 335/337/339 |
| **Intermediate 169** | 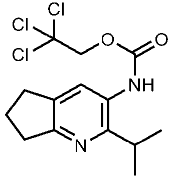 | 2,2,2-trichloroethyl (2-isopropyl-6,7-dihydro-5H-cyclopenta[b]pyridin-3-yl)carbamate | 351/353/355 |
| **Intermediate 170** | 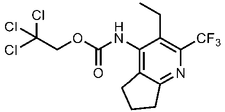 | 2,2,2-trichloroethyl (3-ethyl-2-(trifluoromethyl)-6,7-dihydro-5H-cyclopenta[b]pyridin-4-yl)carbamate | 405/407/409 |
| **Intermediate 171** | 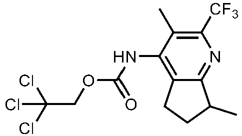 | 2,2,2-trichloroethyl (3,7-dimethyl-2-(trifluoromethyl)-6,7-dihydro-5H-cyclopenta[b]pyridin-4-yl)carbamate | 405/407/409 |
| **Intermediate 172** | 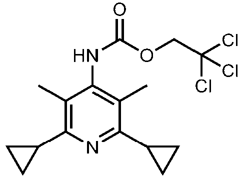 | 2,2,2-trichloroethyl (2,6-dicyclopropyl-3,5-dimethylpyridin-4-yl)carbamate | 377/379/381 |
| **Intermediate 173** | 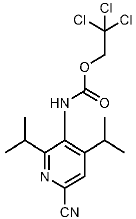 | 2,2,2-trichloroethyl (6-cyano-2,4-diisopropylpyridin-3-yl)carbamate | 378/380/382 |
| **Intermediate 174** | 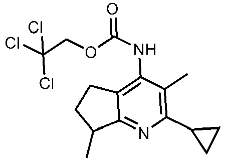 | 2,2,2-trichloroethyl (2-cyclopropyl-3,7-dimethyl-6,7-dihydro-5H-cyclopenta[b]pyridin-4-yl)carbamate | 377/379/381 |
| **Intermediate 175** | 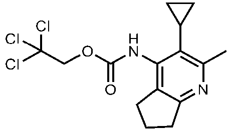 | 2,2,2-trichloroethyl (3-cyclopropyl-2-methyl-6,7-dihydro-5H-cyclopenta[b]pyridin-4-yl)carbamate | 363/365/367 |
| **Intermediate 176** | 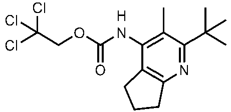 | 2,2,2-trichloroethyl (2-(tert-butyl)-3-methyl-6,7-dihydro-5H-cyclopenta[b]pyridin-4-yl)carbamate | 379/381/383 |
| **Intermediate 177** | 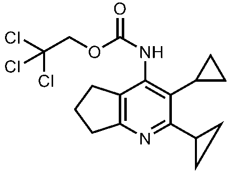 | 2,2,2-trichloroethyl (2,3-dicyclopropyl-6,7-dihydro-5H-cyclopenta[b]pyridin-4-yl)carbamate | 389/391/393 |
| **Intermediate 178** | 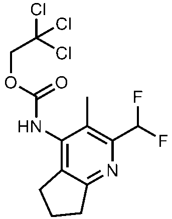 | 2,2,2-trichloroethyl (2-(difluoromethyl)-3-methyl-6,7-dihydro-5H-cyclopenta[b]pyridin-4-yl)carbamate | 373/375/377 |
| **Intermediate 179** | 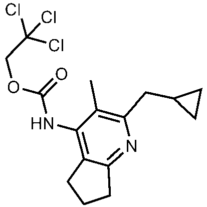 | 2,2,2-trichloroethyl (2-(cyclopropylmethyl)-3-methyl-6,7-dihydro-5H-cyclopenta[b]pyridin-4-yl)carbamate | 377/379/381 |
| **Intermediate 180** | 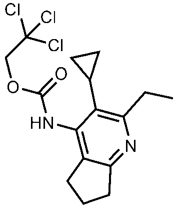 | 2,2,2-trichloroethyl (3-cyclopropyl-2-ethyl-6,7-dihydro-5H-cyclopenta[b]pyridin-4-yl)carbamate | 377/379/381 |
| **Intermediate 181** | 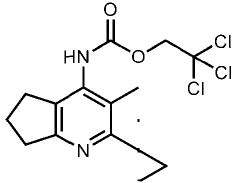 | 2,2,2-trichloroethyl (3-methyl-2-(1-methylcyclopropyl)-6,7-dihydro-5H-cyclopenta[b]pyridin-4-yl)carbamate | 377/379/381 |
| **Intermediate 182** | 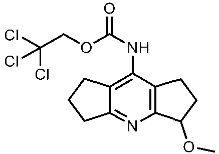 | 2,2,2-trichloroethyl (3-methoxy-1,2,3,5,6,7-hexahydrodicyclopenta[b,e]pyridin-8-yl)carbamate | 379/381/383 |
| **Intermediate 183** | 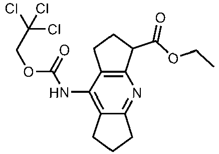 | ethyl 8-(((2,2,2-trichloroethoxy)carbonyl)amino)-1,2,3,5,6,7-hexahydrodicyclopenta[b,e]pyridine-3-carboxylate | 421/423/425 |
| **Intermediate 184** | 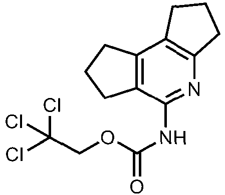 | 2,2,2-trichloroethyl (1,2,3,6,7,8-hexahydrodicyclopenta[b,d]pyridin-5-yl)carbamate | 349/351/353 |
| **Intermediate 185** | 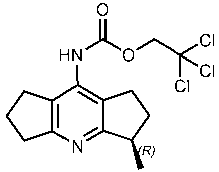 | 2,2,2-trichloroethyl (R)-(3-methyl-1,2,3,5,6,7-hexahydrodicyclopenta[b,e]pyridin-8-yl)carbamate | 363/365/367 |
| **Intermediate 186** | 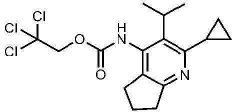 | 2,2,2-trichloroethyl (2-cyclopropyl-3-isopropyl-6,7-dihydro-5H-cyclopenta[b]pyridin-4-yl)carbamate | 391/393/395 |
| **Intermediate 187** | 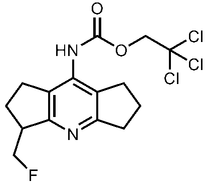 | 2,2,2-trichloroethyl (3-(fluoromethyl)-1,2,3,5,6,7-hexahydrodicyclopenta[b,e]pyridin-8-yl)carbamate | 381/383/385 |

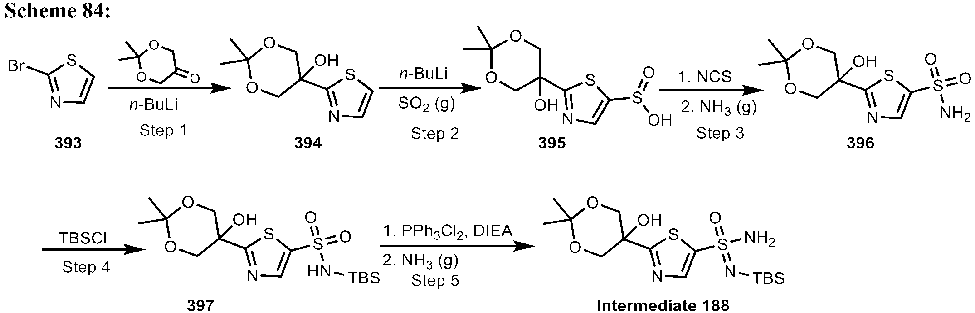

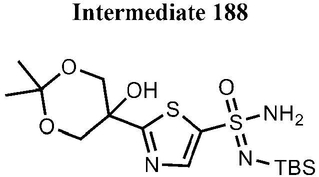

### N'-(tert-butyldimethylsilyl)-2-(5-hydroxy-2,2-dimethyl-1,3-dioxan-5-yl)thiazole-5-sulfonimidamide

### Step 1: 2,2-Dimethyl-5-(thiazol-2-yl)-1,3-dioxan-5-ol

To a stirred solution of 2-bromothiazole (4.89 g, 30.0 mmol) in THF (200 mL) in a 500-mL 3-necked round-bottom flask under nitrogen was added n-BuLi in hexane (2.5 M, 12.0 mL, 30 mmol) dropwise at -70 °C in a liquid nitrogen/ethanol. The resulting solution was stirred for 1 h at -70 °C. Then 2,2-dimethyl-1,3- dioxan-5-one (3.9 g, 30 mmol) in THF (10 mL) was added dropwise at -70 °C. The resulting solution was stirred for an additional 30 min at RT. The reaction solution was quenched with H₂O (200 mL). The resulting mixture was extracted with EtOAc (3x200 mL). The organic layer was dried over anhydrous Na₂SO₄ and concentrated under vacuum. The residue was eluted from silica gel with EtOAc/PE (1/10). This resulted in 3.2 g (50%) of the title compound as a yellow solid. MS-ESI: 216 (M+1). Steps 2-5 used similar procedures for converting compound **257** to Intermediate **113** shown in Scheme 54 to afford Intermediate **188** from compound **394.** MS-ESI: 408 (M+1).

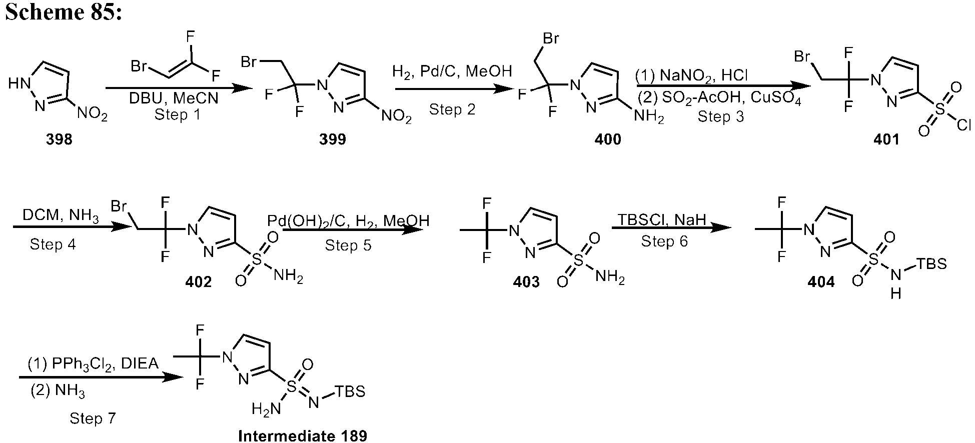

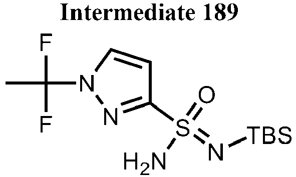

### N'-(tert-butyldimethylsilyl)-1-(1,1-difluoroethyl)-1H-pyrazole-3-sulfonimidamide

### Step 1: 1-(2-Bromo-1,1-difluoroethyl)-3-nitro-1H-pyrazole

To a stirred solution of 2-bromo-1,1-difluoroethene (8.5 g, 59 mmol) in MeCN (30 mL) in a 100-mL round-bottom flask under nitrogen was added 3-nitro-1H-pyrazole (6.7 g, 59 mmol) in small portions at -30 °C followed by the addition of DBU (18.1 g, 119 mmol) dropwise at -30 °C. The resulting solution was stirred for 4 h at -20 °C. The reaction solution was concentrated under vacuum. The resulted mixture was diluted with water (100 mL). The resulting mixture was extracted with 3x100 mL of DCM. The organic layer was dried with anhydrous Na₂SO₄ and concentrated under vacuum. The residue was eluted from silica gel with EtOAc/PE (1:5). This resulted in 4.2 g (28%) of the title compound as yellow oil. MS-ESI: 256/258 (M+1).

### Step 2: 1-(2-Bromo-1,1-difluoroethyl)-1H-pyrazol-3-amine

To a stirred solution of 1-(2-bromo-1,1-difluoroethyl)-3-nitro-1H-pyrazole (2.0 g, 7.81 mmol) in MeOH (20 mL) in a 100-mL round-bottom flask under nitrogen was added Pd(OH)₂/C (20% wt., 395 mg) in portions at RT. The flask was evacuated and refilled three times with hydrogen. The resulting solution was stirred for 2 days at RT under atmosphere of hydrogen with a balloon. The solids were filtered out. The resulting mixture was concentrated under vacuum. The residue was eluted from silica gel with EtOAc/PE (1:1). This resulted in 200 mg (11%) of the title compound as brown oil. MS-ESI: 226/228 (M+1).

Steps 3-4 used similar procedures for converting compound **183** to compound **185** shown in Scheme 43 to afford compound **402** from compound **400.** MS-ESI: 290/292 (M+1).

### Step 5: 1-(1,1-Difluoroethyl)-1H-pyrazole-3-sulfonamide

To a stirred solution of 1-(2-bromo-1,1-difluoroethyl)-1H-pyrazole-3-sulfonamide (1.0 g, 3.45 mmol) in MeOH (40 mL) in a 100-mL pressure tank reactor under nitrogen was added Pd(OH)₂/C (20% wt., 200 mg) in portions at 0 °C. The resulting solution was stirred for 24 h at 70 °C under the atmosphere of hydrogen (10 atm). The solids were filtered out. The resulting mixture was concentrated under vacuum. The residue was eluted from silica gel with EtOAc/PE (1:1). This resulted in 620 mg (85%) of the title compound as a white solid. MS-ESI: 210 (M-1). Steps 6-7 used similar procedures for converting compound **205** to Intermediate **105** shown in Scheme 46 to afford Intermediate **189** from compound **403.** MS-ESI: 325 (M+1).

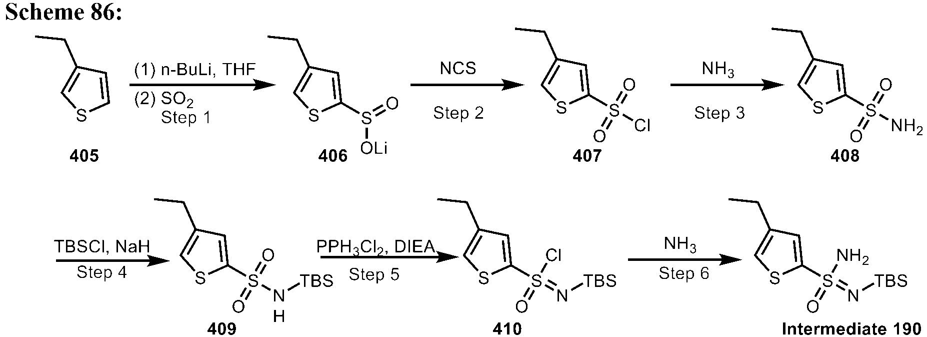

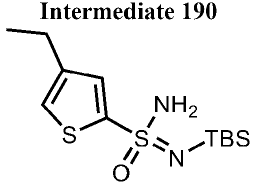

### N'-(tert-butyldimethylsilyl)-4-ethylthiophene-2-sulfonimidamide

Steps 1-6 used similar procedures for converting compound **257** to Intermediate **133** shown in Scheme 54 to afford Intermediate **190** from compound **405.** MS-ESI: 305 (M+1).

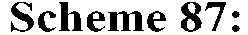

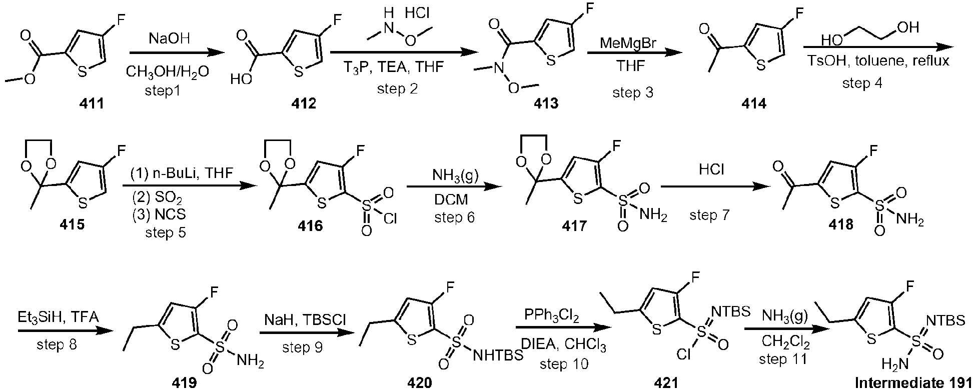

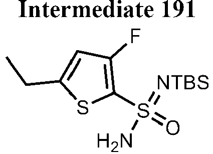

### N'-(tert-butyldimethylsilyl)-5-ethyl-3-fluorothiophene-2-sulfonimidamide

### Step 1: 4-Fluorothiophene-2-carboxylic acid

To a stirred solution of methyl 4-fluorothiophene-2-carboxylate (300 mg, 1.87 mmol) in MeOH (10 mL) in a 50-mL round-bottom flask was added a solution of NaOH (300 mg, 7.49 mmol) in H₂O (10 mL) dropwise at 0 °C. The resulting solution was stirred for 2 h at RT. The pH value of the solution was adjusted to 4 with HCl (6 M). The resulting solution was extracted with 3x20 mL of EtOAc and dried over anhydrous Na₂SO₄ and concentrated under vacuum. This resulted in 200 mg (73%) of the title compound as a white solid. MS-ESI: 145 (M-1).

### Step 2: 4-Fluoro-N-methoxy-N-methylthiophene-2-carboxamide

To a stirred solution of 4-fluorothiophene-2-carboxylic acid (200 mg, 1.37 mmol) in THF (15 mL) in a 50-mL round-bottom flask under nitrogen was added TEA (346 mg, 3.42 mmol) and N,O-dimethyl- hydroxylamine hydrochloride (202 mg, 2.06 mmol) at RT. To the stirred solution was added T3P in EtOAc (50% wt., 1.74 g, 2.74 mmol) dropwise at 0 °C. The resulting solution was stirred for 4 h at RT. The reaction solution was quenched with 20 mL water. The resulting solution was extracted with 3x20 mL of EtOAc, dried over anhydrous Na₂SO₄ and concentrated under vacuum. The residue was eluted from silica gel with EtOAc/PE (1:3). This resulted in 200 mg (77%) of the title compound as yellow oil. MS-ESI: 190 (M+1).

### Sep 3: 1-(4-Fluorothiophen-2-yl)ethan-1-one

To a stirred solution of 4-fluoro-N-methoxy-N-methylthiophene-2-carboxamide (200 mg, 1.06 mmol) in THF (10 mL) in a 50-mL 3-necked round-bottom flask under nitrogen was added MeMgBr in THF (3.0 M, 0.53 mL, 1.59 mmol) dropwise with stirring at 0 °C in 5 min. The resulting solution was stirred for 1 h at RT. The reaction was then quenched by the addition of 5.0 mL of NH₄Cl (aq). The resulting solution was extracted with 3x10 mL of DCM. The combine extract was dried over anhydrous Na₂SO₄ and concentrated under vacuum. This resulted in 120 mg (79%) of the title compound as yellow oil. MS-ESI: 145 (M+1).

### Step 4: 2-(4-Fluorothiophen-2-yl)-2-methyl-1,3-dioxolane

To a stirred solution of 1-(4-fluorothiophen-2-yl)ethan-1-one (120 mg, 0.83 mmol) in toluene (10 mL) in a 100-mL round-bottom flask was added ethane-1,2-diol (103 mg, 1.66 mmol) and 4-methylbenzenesulfonic acid (14 mg, 0.083 mmol). The resulting solution was stirred for 16 h at 110 °C. The resulting mixture was concentrated under vacuum. The residue was eluted from silica gel with EtOAc/PE (1:3). This resulted in 121 mg (77%) of the title compound as yellow oil. MS-ESI: 189 (M+1).

Steps 5-6 used similar procedures for converting compound **257** to compound **259** shown in Scheme 54 to afford compound **417** from compound **415.** MS-ESI: 268 (M+1).

### Step 7: 5-Acetyl-3-fluorothiophene-2-sulfonamide

To a stirred solution of 3-fluoro-5-(2-methyl-1,3-dioxolan-2-yl)thiophene-2-sulfonamide (360 mg, 1.35 mmol) in THF (10 mL) in a 50-mL round-bottom flask was added HCl (4 M, 10 mL, 40 mmol) dropwise at 0 °C. The resulting solution was stirred for 3 h at 60 °C in an oil bath. The resulting mixture was concentrated under vacuum. The pH value of the solution was adjusted to 8 with sat. Na₂CO₃. The resulting mixture was extracted with EtOAc (3x30 mL). The organic layer was dried over anhydrous Na₂SO₄ and concentrated under vacuum. The residue was eluted from silica gel with EtOAc/PE (1:2). This resulted in 300 mg (99%) of the title compound as yellow oil. MS-ESI: 224 (M+1).

### Step 8: 5-Ethyl-3-fluorothiophene-2-sulfonamide

To a stirred solution of 5-acetyl-3-fluorothiophene-2-sulfonamide (300 mg, 1.34 mmol) in TFA (1.53 g, 13.4 mmol) in a 50-mL round-bottom flask under nitrogen was added triethylsilane (1.56 g, 13.4 mmol) dropwise at RT. The resulting solution was stirred for 16 h at 30 °C in an oil bath.

The resulting mixture was concentrated under vacuum. The pH value of the solution was adjusted to 8 with sat. Na₂CO₃. The resulting mixture was extracted with EtOAc (3x30 mL). The organic layer was dried over anhydrous Na₂SO₄ and concentrated under vacuum. The residue was eluted from silica gel with EtOAc/PE (1:1). This resulted in 240 mg (85%) of the title compound as yellow oil. MS-ESI: 210 (M+1).

Steps 9-11 used similar procedures for converting compound **205** to Intermediate **105** shown in Scheme 46 to afford Intermediate **191** from compound **419.** MS-ESI: 323 (M+1).

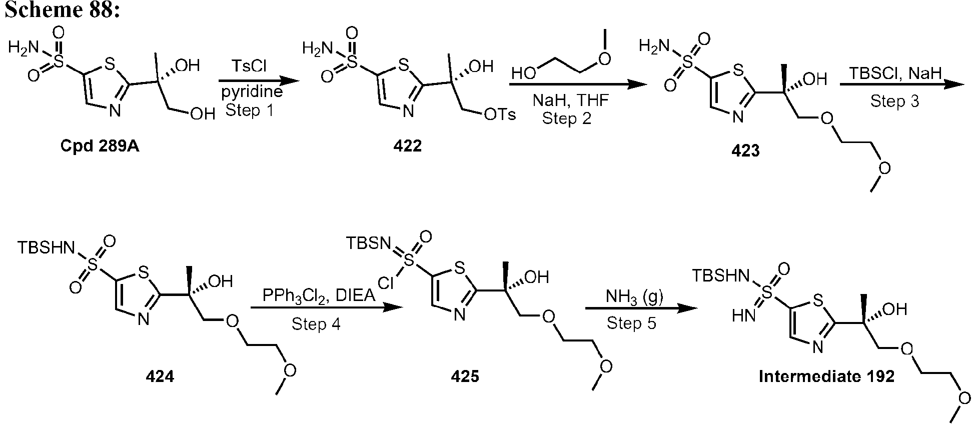

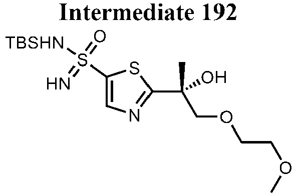

### N-(tert-butyldimethylsilyl)-2-((R)-2-hydroxy-1-(2-methoxyethoxy)propan-2-yl)thiazole-5-sulfonimidamide

### Step 1: (R)-2-hydroxy-2-(5-sulfamoylthiazol-2-yl)propyl 4-methylbenzenesulfonate

To a stirred solution of (R)-2-(1,2-dihydroxypropan-2-yl)thiazole-5-sulfonamide (Compound **289A,** 4.0 g, 17 mmol) in pyridine (40 mL) in a 250-mL round-bottom flask under nitrogen was added 4-methylbenzenesulfonyl chloride (4.8 g, 25 mmol) in portions at 0 °C. The resulting solution was stirred overnight at RT and then diluted with HCl (1 M, 40 mL) and extracted with 3x40 mL EtOAc. The combined organic layers were dried over anhydrous Na₂SO₄. After filtration, the filtrate was concentrated under vacuum. The residue was eluted from silica gel with PE/EtOAc (1:1). This resulted in 5.2 g (79%) of the title compound as a yellow solid. MS-ESI: 391 (M-1).

### Step 2: (R)-2-(2-hydroxy-1-(2-methoxyethoxy)propan-2-yl)thiazole-5-sulfonamide

To a stirred solution of 2-methoxyethan-1-ol (1.94 g, 25 mmol) in THF (30 mL) in a 100-mL round-bottom flask under nitrogen was added NaH (60% wt. dispersion in mineral oil, 2.04 g, 51 mmol) in portions at 0 °C. The resulting mixture was stirred for 30 min at 0 °C. To the solution was added (R)-2-hydroxy-2-(5- sulfamoylthiazol-2-yl)propyl 4-methylbenzenesulfonate (2.0 g, 5.1 mmol) in portions at 0 °C. The resulting mixture was stirred for 32 h at 35 °C. The reaction solution was quenched with water (20 mL) at 0 °C. The resulting mixture was extracted with EtOAc (3 x 200 mL). The combined organic layers were washed with brine (2x200 mL) and dried over anhydrous Na₂SO₄. After filtration, the filtrate was concentrated under reduced pressure. The residue was eluted from silica gel with PE/EtOAc (1:3). This resulted in 1.18 g (78%) of the title compound as yellow oil. MS-ESI: 295 (M+1). Steps 3-5 used similar procedures for converting compound **205** to Intermediate **105** shown in Scheme 46 to afford Intermediate **192** from compound **423.** MS-ESI: 410 (M+1).

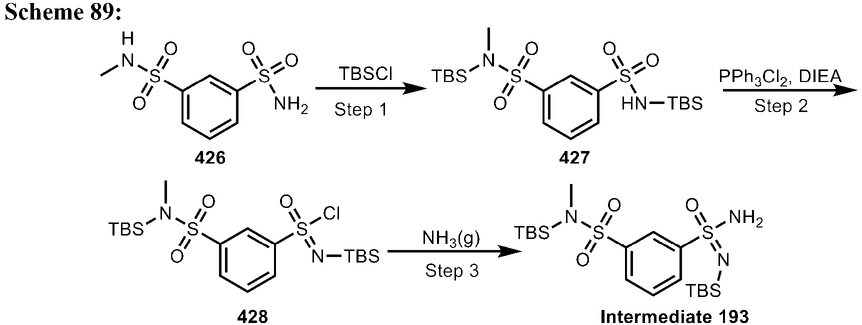

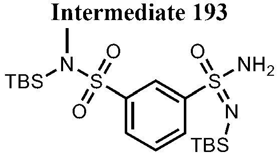

### N-(tert-butyldimethylsilyl)-3-(N'-(tert-butyldimethylsilyl)sulfamidimidoyl)-N-methylbenzenesulfonamide

### Step 1: N₁,N₃-bis(tert-butyldimethylsilyl)-N1-methylbenzene-1,3-disulfonamide

To a stirred solution of N-methylbenzene-1,3-disulfonamide (220 mg, 0.88 mmol) in THF (6.0 mL) in a 25-mL round-bottom flask under nitrogen was added NaH (60% wt. dispersion in mineral oil, 211 mg, 5.28 mmol) in portions at 0 °C. The resulting solution was stirred for 20 min at RT. To the solution was added TBSCl (397 mg, 2.64 mmol) in portions at 0 °C. The resulting solution was stirred overnight at RT. The reaction solution was quenched with 10 mL water. The mixture was extracted with 3x20 mL EtOAc. The organic layer was dried with anhydrous Na₂SO₄ and concentrated under vacuum. The residue was eluted from silica gel with PE/EtOAc (1:2). This resulted in 400 mg (95%) of the title compound as yellow oil. MS-ESI: 479 (M+1). Steps 2-4 used similar procedures for converting compound **186** to Intermediate **100** shown in Scheme 43 to afford Intermediate **193** from compound 427. MS-ESI: 478 (M+1).

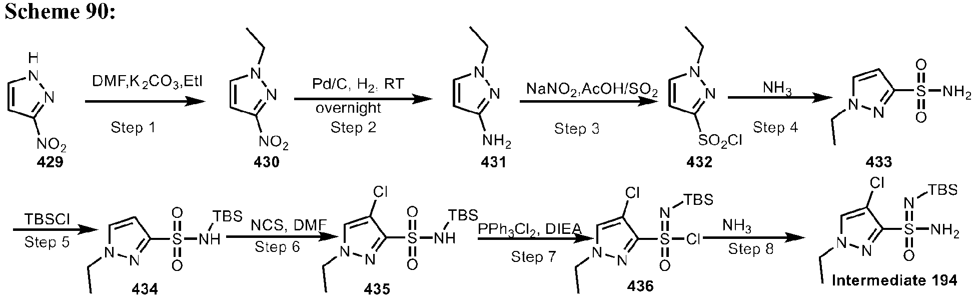

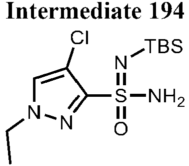

### N'-(tert-butyldimethylsilyl)-4-chloro-1-ethyl-1H-pyrazole-3-sulfonimidamide

### Step 1: 1-Ethyl-3-nitro-1H-pyrazole

To a stirred solution of 3-nitro-1H-pyrazole (30 g, 265 mmol) in DMF (150 mL) in a 500-mL round-bottom flask was added K₂CO₃ (73.3 g, 530 mmol) in portions at RT. Iodoethane (82.8 g, 530 mmol) was added dropwise at RT. The resulting solution was stirred for 1 h at 80 °C. The reaction was then quenched by the addition of 500 mL of water. The resulting mixture was extracted with 4x200 mL of EtOAc and dried over anhydrous Na₂SO₄. The solids were filtered out. The resulting mixture was concentrated under vacuum. The residue was eluted from silica gel with EtOAc/PE (1:4). This resulted in 36 g (96%) of the title compound as yellow oil. MS-ESI: 142 (M+1).

### Step 2: 1-Ethyl-1H-pyrazol-3-amine

To a stirred solution of 1-ethyl-3-nitro-1H-pyrazole (10 g, 71 mmol) in MeOH (100 mL) in a 250-mL round-bottom flask under nitrogen was added Pd/C (10% wt., 1.13 g) in portions at 0 °C. The flask was evacuated and refilled three times with hydrogen. The resulting solution was stirred for 16 h at RT under hydrogen using a balloon. The solids were filtered out. The filtrate was concentrated under vacuum. The residue was eluted from silica gel with DCM/MeOH (15:1). This resulted in 5.7 g (72%) of the title compound as yellow oil. MS-ESI: 112 (M+1).

Steps 3-5 used similar procedures for converting compound **218** to compound **221** shown in Scheme 48 to afford compound **434** from compound **431.** MS-ESI: 290 (M+1).

### Step 6: N-(tert-butyldimethylsilyl)-4-chloro-1-ethyl-1H-pyrazole-3-sulfonamide

To a stirred solution of N-(tert-butyldimethylsilyl)-1-ethyl-1H-pyrazole-3-sulfonamide (100 mg, 0.35 mmol) in DMF (10 mL) in a 25-mL round-bottom flask under nitrogen was added NCS (50.7 mg, 0.38 mmol) in portions at 0 °C. The resulting solution was stirred overnight at RT. The reaction solution was diluted with 10 mL H₂O. The resulting mixture was extracted with 3x20 mL of EtOAc, the combined organic layer was dried over anhydrous magnesium sulfate. The organic layer was concentrated under vacuum. The residue was eluted from silica gel with EtOAc/PE (1:3). This resulted in 80 mg (71.5%) of the title compound as a yellow solid. MS-ESI: 324/326 (M+1).

Steps 7-8 used similar procedures for converting compound **186** to Intermediate **100** shown in Scheme 43 to afford Intermediate **194** from compound **435.** MS-ESI: 323 (M+1).

**Schemes of Sulfonimidamide Intermediates:** Schemes **91-106** illustrate the preparation of sulfonimidamide intermediates.

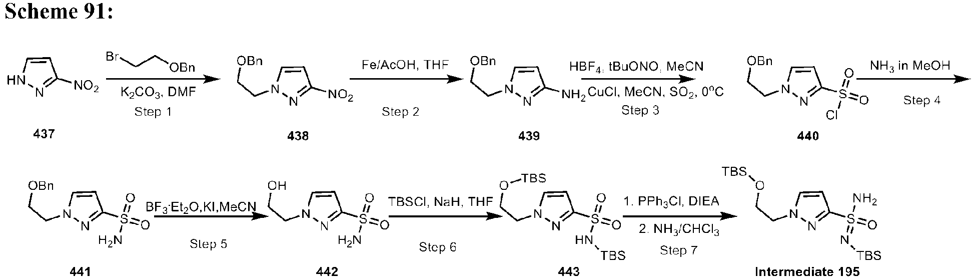

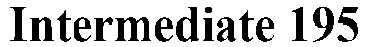

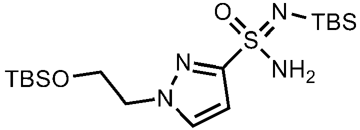

### N'-(tert-butyldimethylsilyl)-1-(2-((tert-butyldimethylsilyl)oxy)ethyl)-1H-pyrazole-3-sulfonimidamide

### Step 1: 1-(2-(Benzyloxy)ethyl)-3-nitro-1H-pyrazole

To a stirred solution of 3-nitro-1H-pyrazole (10 g, 88 mmol) in DMF (120 mL) in a 500-mL 3-necked round-bottom flask was added K₂CO₃ (18 g, 133 mmol) and ((2-bromoethoxy)methyl)benzene (20 g, 93 mmol) in portions at RT. The resulting solution was stirred for 16 h at 60 °C in an oil bath. The reaction was then quenched by the addition of 150 mL of water. The resulting solution was extracted with 3x200 mL of EtOAc. The organic layers were combined and dried over anhydrous Na₂SO₄ and concentrated under vacuum. The crude product was eluted from silica gel with EtOAc/PE (1:3). This resulted in 21.1 g (96.7%) of the title compound as yellow oil. MS-ESI: 248 (M+1).

### Step 2: 1-(2-(Benzyloxy)ethyl)-1H-pyrazol-3-amine

To a stirred solution of 1-(2-(benzyloxy)ethyl)-3-nitro-1H-pyrazole (20 g, 81 mmol) in THF (200 mL) and AcOH (50 mL) in a 1-L round-bottom flask under nitrogen was added Fe powder (45 g, 810 mmol). The resulting mixture was stirred for 4 h at RT. The solids were filtered out, to the filtrate was added 400 mL of water. The resulting solution was extracted with 3x300 mL of EtOAc, the organic layers were combined and dried over anhydrous Na₂SO₄. The residue was eluted from silica gel with EtOAc/PE (3:1). This resulted in 14.8 g (84%) of the title compound as pink oil. MS-ESI: 218 (M+1).

### Step 3: 1-(2-(Benzyloxy)ethyl)-1H-pyrazole-3-sulfonyl chloride

To a stirred solution of 1-(2-(benzyloxy)ethyl)-1H-pyrazol-3-amine (10 g, 46 mmol) in MeCN (100 ml) in a 500-mL round-bottom flask was added HBF₄ (aq., 40%wt., 15 g, 69.1 mmol) at RT, followed by the addition of tert-butyl nitrite (7.12 g, 69 mmol) dropwise below 5 °C in an ice/water bath. The resulting solution was stirred for 1.5 h at 0~5 °C, this solution was assigned as solution A. Then CuCl (13.7 g, 138 mmol) was added to a 500-mL single necked round-bottom flask with MeCN (200 mL) as the solvent. Then SO₂ (g) was bubbled to the solution with stirring at RT for 20 min, this solution was assigned as solution B. To the solution B was added solution A dropwise with stirring at 0 °C. The resulting solution was stirred for additional 3 h at RT. The reaction was then quenched by the addition of 500 mL of water. The resulting solution was extracted with 3x300 mL of EtOAc. The organic layers were combined and washed with 3x300 mL of H₂O. The mixture was dried over anhydrous sodium sulfate and concentrated. This resulted in 12.8 g (crude) of the title compound as brown yellow oil. MS-ESI: 301 (M+1).

### Step 4: 1-(2-(Benzyloxy)ethyl)-1H-pyrazole-3-sulfonamide

A solution of 1-(2-(benzyloxy)ethyl)-1H-pyrazole-3-sulfonyl chloride (crude from last step, 12.8 g) in MeOH/NH₃ (7 M, 300 mL) in a 500-mL round-bottom flask was stirred for 16 h at RT. The resulting solution was concentrated under vacuum. The residue was eluted from silica gel with DCM/MeOH (20:1). This resulted in 5.4 g (42%, over two steps) of the title compound as brown yellow oil. MS-ESI: 282 (M+1).

### Step 5: 1-(2-Hydroxyethyl)-1H-pyrazole-3-sulfonamide

To a stirred solution of 1-(2-(benzyloxy)ethyl)-1H-pyrazole-3-sulfonamide (5.4 g, 19.2 mmol) in MeCN (100 mL) in a 250-mL round-bottom flask under nitrogen was added KI (6.37 g, 38 mmol) in portions at RT. To the stirred solution was added BF₃·Et₂O (47%wt., 13 g, 192 mmol) dropwise at RT. The resulting solution was stirred for 4 h at RT. The reaction was then quenched by the addition of 5.0 mL of water. The solids were filtered out. The resulting mixture was concentrated under vacuum. The residue was eluted from silica gel with DCM/MeOH (15:1). This resulted in 4.6 g (93%) of the title compound as a yellow solid. MS-ESI: 192 (M+1).

### Step 6: N-(tert-butyldimethylsilyl)-1-(2-((tert-butyldimethylsilyl)oxy)ethyl)-1H-pyrazole-3-sulfonamide

To a stirred solution of 1-(2-hydroxyethyl)-1H-pyrazole-3-sulfonamide (4.1 g, 21 mmol) in THF (60 mL) in a 250-mL round-bottom flask under nitrogen was added NaH (60% wt. dispersion in mineral oil, 3.86 g, 96.5 mmol) at 0 °C in a water/ice bath. The resulting mixture was stirred for 20 min at RT. To the stirred mixture was added TBSCl (13.6 g, 90 mmol) at 0 °C. The resulting mixture was stirred for 5 h at RT. The reaction was then quenched by the addition of 300 mL of water. The resulting mixture was extracted with 3x150 mL of EtOAc. The combined organic layers were dried over anhydrous Na₂SO₄ and concentrated under vacuum. The residue was eluted from silica gel with EtOAc/PE (1:5). This resulted in 6.2 g (69%) of the title compound as an off-white solid. MS-ESI: 420 (M+1).

### Step 7: N'-(tert-butyldimethylsilyl)-1-(2-((tert-butyldimethylsilyl)oxy)ethyl)-1H-pyrazole-3-sulfonimidamide

To a stirred solution of PPh₃Cl₂ (1.51 g, 3.57 mmol) in CHCl₃ (15 ml) in a 50-mL 3-necked round-bottom flask under nitrogen was added DIEA (924 mg, 7.15 mmol) dropwise at 0 °C. The resulting solution was stirred for 20 min at 0 °C. To the stirred solution was added N-(tertbutyldimethylsilyl) -1-(2-((tert-butyldimethylsilyl)oxy)ethyl)-1H-pyrazole-3-sulfonamide (600 mg, 1.43 mmol) in CHCl₃ (5.0 ml) dropwise at 0 °C. The resulting solution was stirred for 2 h at 0 °C. NH₃(g) was bubbled into the reaction solution for 15 min at 0 °C. Then the solution was stirred for another 2 h at RT. The reaction was then quenched by the addition of 20 mL of H₂O. The resulting solution was extracted with 3x20 mL of DCM. The organic layers were dried over anhydrous Na₂SO₄ and concentrated under vacuum. The residue was eluted from silica gel with EtOAc/PE(1:3). This resulted in 320 mg (53 %) of the title compound as a yellow solid. MS-ESI: 419 (M+1).

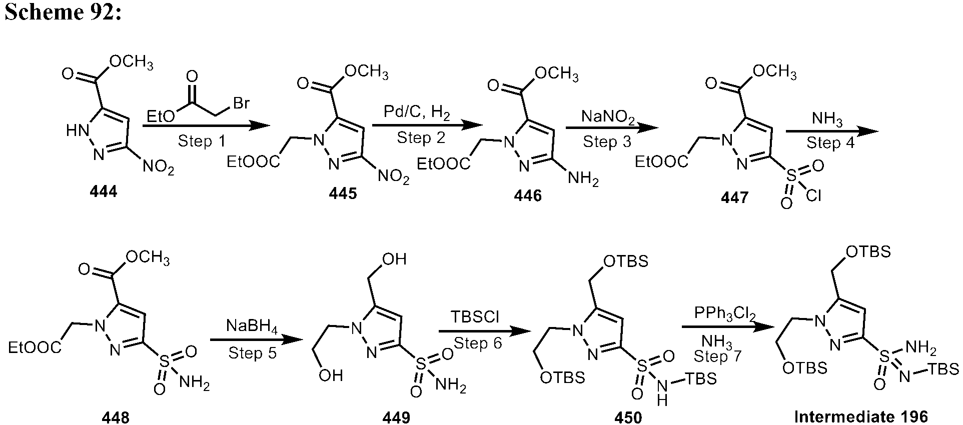

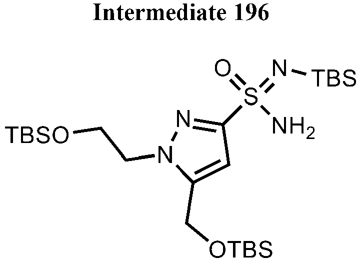

### N'-(tert-butyldimethylsilyl)-1-(2-((tert-butyldimethylsilyl)oxy)ethyl)-5-(((tertbutyldimethylsilyl)oxy)methyl)-1H-pyrazole-3-sulfonimidamide

### Step 1: Methyl 1-(2-ethoxy-2-oxoethyl)-3-nitro-1H-pyrazole-5-carboxylate

To a stirred solution of methyl 3-nitro-1H-pyrazole-5-carboxylate (5.0 g, 29 mmol) in MeCN (50 mL) in a 250-mL round-bottom flask was added Cs₂CO₃ (19 g, 58 mmol) at RT. To the stirred solution was added ethyl 2-bromoacetate (5.36 g, 35 mmol) dropwise at RT. The resulting solution was stirred for 0.5 h at 50 °C. The mixture was diluted with 100 mL of H₂O. The resulting solution was extracted with 3x50 mL of EtOAc. The combined organic layer was dried with anhydrous Na₂SO₄ and concentrated. The residue was eluted from silica gel with EtOAc/PE (1:5). This resulted in 3.8 g (50%) of the title compound as yellow oil. MS-ESI: 258 (M+1).

### Step 2: Methyl 3-amino-1-(2-ethoxy-2-oxoethyl)-1H-pyrazole-5-carboxylate

To a stirred solution of methyl 1-(2-ethoxy-2-oxoethyl)-3-nitro-1H-pyrazole-5-carboxylate (1.4 g, 5.43 mmol) in MeOH (30 mL) in a 250 round-bottom flask was added Pd/C (10%wt., 300 mg) under nitrogen in portions. The flask was evacuated and refilled three times with hydrogen. The resulting solution was stirred overnight at RT under hydrogen with a balloon. The solid was filtered out. The resulting solution was concentrated under vacuum. This resulted in 1.18 g (95%) of the title compound as a white solid. MS-ESI: 228 (M+1). ¹H NMR (400 MHz, DMSO-*d*₆) δ 6.94 (br s, 2H), 6.29 (s, 1H), 5.11 (s, 2H), 4.14 (q, *J =* 7.1 Hz, 2H), 3.79 (s, 3H), 1.19 (t, *J =* 7.1 Hz, 3H). The structure of compound 446 was confirmed by NOESY.

### Step 3: Methyl 3-(chlorosulfonyl)-1-(2-ethoxy-2-oxoethyl)-1H-pyrazole-5-carboxylate

To a stirred solution of methyl 3-amino-1-(2-ethoxy-2-oxoethyl)-1H-pyrazole-5-carboxylate (1.18 g, 5.17 mmol) in HCl (6 M, 30 mL) in a 100 mL 3-necked round-bottom flask was added NaNO₂ (1.07 g, 15.5 mmol) in H₂O (1.0 mL) dropwise at -10 °C. The resulting mixture was stirred for 1 h at -10 °C, this solution was assigned as solution A. Then CuCl₂ (348 mg, 2.59 mmol) was added to a 250-mL single necked round-bottom flask with AcOH (30 mL) as the solvent, SO₂ (g) was bubbled to the solution with stirring at RT for 20 min, this solution was assigned as solution B. To the solution B was added solution A dropwise with stirring at -10 °C. The resulting solution was stirred for additional 1 h at 0 °C. The resulting mixture was extracted with DCM (3 x 30 mL). The combined organic layers were washed with water (3x30 mL) and dried over anhydrous Na₂SO₄. After filtration, the filtrate was concentrated under vacuum. This resulted in 1.7 g (crude) of the title compound as a yellow solid.

### Step 4: Methyl 1-(2-ethoxy-2-oxoethyl)-3-sulfamoyl-1H-pyrazole-5-carboxylate

To the solution of methyl 3-(chlorosulfonyl)-1-(2-ethoxy-2-oxoethyl)-1H-pyrazole-5-carboxylate (1.7 g, crude) in DCM (50 mL) in a 250 mL round-bottom flask was introduced NH₃ (g) bubbled for 15 min at 0 °C. The reaction was stirred for 2 h at RT. The resulting mixture was concentrated under vacuum. The residue was eluted from silica gel with PE/EtOAc (3:1). This resulted in 1.33 g (88%) of the title compound as a yellow solid. MS-ESI: 290 (M-1).

### Step 5: 1-(2-Hydroxyethyl)-5-(hydroxymethyl)-1H-pyrazole-3-sulfonamide

To a stirred solution of methyl 1-(2-ethoxy-2-oxoethyl)-3-sulfamoyl-1H-pyrazole-5-carboxylate (1.33 g, 4.55 mmol) in EtOH (30 mL) in a 100 mL round-bottom flask was added NaBH₄ (865 mg, 22.8 mmol) in portions at 0 °C. The mixture was stirred overnight at RT. The reaction was quenched by the addition of sat. NH₄Cl (aq.) (20 mL) at 0 °C. The EtOH solvent was removed under vacuum. The resulting mixture was extracted with EtOAc (3 x 50 mL). The combined organic layers were washed with H₂O (3x20 mL) and dried over anhydrous Na₂SO₄ and concentrated under vacuum. The residue was eluted from silica gel with PE/EtOAc (3:1).This resulted in 889 mg (88%) of the title compound as a light yellow solid. MS-ESI: 222 (M+1).

Steps 6-7 used similar procedures for converting compound **442** to Intermediate **195** shown in Scheme 91 to afford Intermediate **196** from compound **449.** MS-ESI: 564 (M+1).

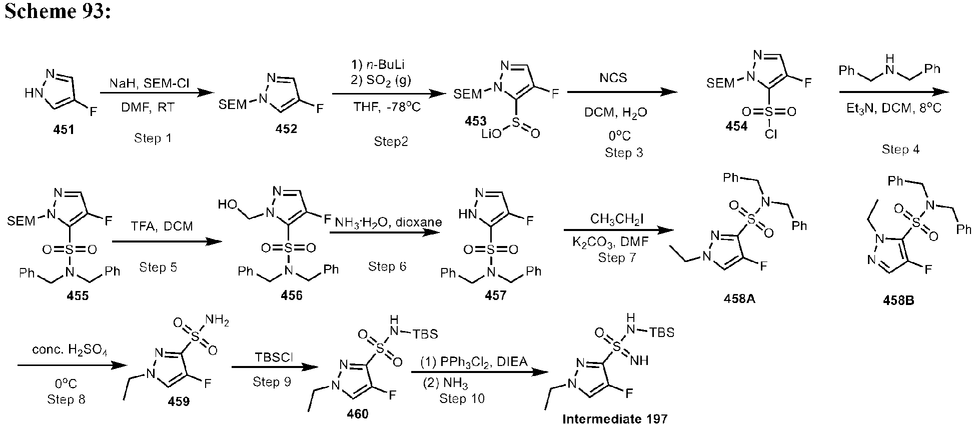

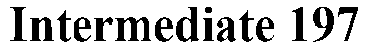

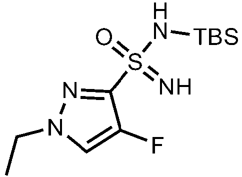

### N-(tert-butyldimethylsilyl)-1-ethyl-4-fluoro-1H-pyrazole-3-sulfonimidamide

### Step 1: 4-Fluoro-1-((2-(trimethylsilyl)ethoxy)methyl)-1H-pyrazole

To a stirred solution of 4-fluoro-1H-pyrazole (5.0 g, 58 mmol) in DMF (53 mL) in a 250-mL 3-necked round-bottom flask under nitrogen was added NaH (60% wt. dispersion in mineral oil, 5.36 g, 134 mmol) in portions at 0 °C in an ice/water bath over 10 min. The resulting solution was stirred for 30 min at 10 °C. To this was added SEM-Cl (22 g, 134 mmol) dropwise with stirring at 0 °C over 10 min. The resulting solution was stirred overnight at RT. The reaction was then quenched with 60 mL of water. The resulting solution was extracted with 60 mL of EtOAc. The combined organic layer was washed with 5x60 ml of sat. NaCl solution. The resulting mixture was concentrated. The residue was eluted from silica gel column EtOAc/PE (1:100). This resulted in 13.7 g (crude) of the title compound as a light yellow liquid. MS-ESI: 217 (M+1).

### Step 2: Lithium 4-fluoro-1-((2-(trimethylsilyl)ethoxy)methyl)-1H-pyrazole-5-sulfinate

To a stirred solution of 4-fluoro-1-((2-(trimethylsilyl)ethoxy)methyl)-1H-pyrazole (13.7 g, 63 mmol) in THF (150 mL) in a 500-mL 3-necked round-bottom flask under nitrogen was added n-BuLi in hexane (2.5 M, 28 mL, 70 mmol) dropwise at -78 °C in a liquid nitrogen/EtOH over 15 min. The resulting solution was stirred for 1 h at -78 °C. Then to the mixture was introduced SO₂ (g) bubble for 20 min -78 °C. The resulting solution was stirred for 1 h at RT. The resulting mixture was concentrated. This resulted in 20.4 g (crude) of the title compound as a white solid.

### Step 3: 4-Fluoro-1-((2-(trimethylsilyl)ethoxy)methyl)-1H-pyrazole-5-sulfonyl chloride

To a stirred solution of lithium 4-fluoro-1-((2-(trimethylsilyl)ethoxy)methyl)-1H-pyrazole-5-sulfinate (20.4 g, crude) in DCM (396 mL) and H₂O (198 mL) in a 1-L 3-necked round-bottom flask was added NCS (10 g, 78 mmol) in portions at 0 °C. The resulting solution was stirred for 1 h at 10 °C. The crude product was used directly without work-up.

### Step 4: N,N-dibenzyl-4-fluoro-1-((2-(trimethylsilyl)ethoxy)methyl)-1H-pyrazole-5-sulfonamide

To the stirred solution of 4-fluoro-1-((2-(trimethylsilyl)ethoxy)methyl)-1H-pyrazole-5-sulfonyl chloride in DCM (396 mL) and H₂O (198 mL) from last step was added Et₃N (8.85 g, 87 mmol) and dibenzylamine (17 g, 84 mmol) dropwise at 0 °C. The resulting solution was stirred for 1 h at 8 °C. The reaction was then quenched by the addition of 300 mL of water. The resulting solution was extracted with 3x300 mL of DCM. The organic layers were dried over anhydrous Na₂SO₄ and concentrated. The residue was eluted from silica gel with EtOAc/PE (1:19). This resulted in 22.5 g (81% over four steps) of the title compound as light yellow oil. MS-ESI: 476 (M+1).

### Step 5: N,N-dibenzyl-4-fluoro-1-(hydroxymethyl)-1H-pyrazole-5-sulfonamide

To a stirred solution of N,N-dibenzyl-4-fluoro-1-((2-(trimethylsilyl)ethoxy)methyl)-1H-pyrazole-5- sulfonamide (22.5 g, 47 mmol) in DCM (25 mL) in a 250-mL round-bottom flask was added TFA (25 mL). The resulting solution was stirred overnight at RT. The resulting mixture was concentrated. The residue was eluted from silica gel with EtOAc/PE (1:4). This resulted in 15 g (85%) of the title compound as yellow oil. MS-ESI: 376 (M+1).

### Step 6: N,N-dibenzyl-4-fluoro-1H-pyrazole-5-sulfonamide

To a stirred solution of N,N-dibenzyl-4-fluoro-1-(hydroxymethyl)-1H-pyrazole-5-sulfonamide (15 g, 40 mmol) in dioxane (50 mL) in a 500-mL round-bottom flask was added NH₃.H₂O (30%wt., 50 mL) dropwise at 0 °C in an ice/water bath. The resulting solution was stirred for 3 h at RT. The resulting mixture was concentrated. The residue was eluted from silica gel with EtOAc/PE (1:1). This resulted in 12 g (87%) of the title compound as a white solid. MS-ESI: 346 (M+1).

### Step 7: N,N-dibenzyl-1-ethyl-4-fluoro-1H-pyrazole-3-sulfonamide and N,N-dibenzyl-1-ethyl-4-fluoro -1H-pyrazole-5-sulfonamide

To a stirred solution of N,N-dibenzyl-4-fluoro-1H-pyrazole-5-sulfonamide (1.1 g, 3.2 mmol) in DMF (20 mL) in a 100-mL 3-necked round-bottom flask under nitrogen was added K₂CO₃ (0.88 g, 6.4 mmol) in portions at RT and ethyl iodide (0.99 g, 6.4 mmol) dropwise at RT. The resulting solution was stirred for 3 h at 110 °C. The reaction was then quenched by the addition of 20 mL of water. The resulting solution was extracted with 2x20 mL of EtOAc. The organic layers were dried over anhydrous Na₂SO₄ and concentrated. The residue was eluted from silica gel with EtOAc/PE (3:17). This resulted in 764 mg (64%) of **458A** and 218 mg (18%) of **458B** both as a light yellow solid. MS-ESI: 374 (M+1).

### Step 8: 1-Ethyl-4-fluoro-1H-pyrazole-3-sulfonamide

To a stirred solution of N,N-dibenzyl-1-ethyl-4-fluoro-1H-pyrazole-3-sulfonamide (764 mg, 2.0 mmol) in DCM (1.5 mL) in a 25-mL round-bottom flask was added H₂SO₄ (98% wt., 3.00 mL) dropwise at 0 °C in an ice/water bath. The resulting solution was stirred for 1 h at RT. The reaction was then quenched by the addition of 5.0 mL of water/ice. The mixture was extracted with EtOAc (3x50 mL). The organic layer was dried over anhydrous Na₂SO₄ and concentrated. The residue was eluted from silica gel with DCM/MeOH(93:7). This resulted in 317 mg (80%) of the title compound as a white solid. MS-ESI: 194 (M+1). ¹H NMR (300 MHz, DMSO-*d*₆) δ 8.08 (d, *J =* 4.7 Hz, 1H), 7.77 (s, 2H), 4.14 (q, *J* = 7.3 Hz, 2H), 1.39 (t, *J* = 7.3 Hz, 3H). The structure of compound 459 was confirmed by NOESY.

Steps 9-10 used similar procedures for converting compound **442** to Intermediate **195** shown in Scheme 91 to afford Intermediate **197** from compound **459.** MS-ESI: 307 (M+1)

**Table 44. The Intermediates in the following table were prepared using the similar procedures for converting compound 451 to Intermediate 197 shown in Scheme 93.**

| Intermediate # | Structure | IUPAC Name | Exact Mass [M-H]⁻ |
|---|---|---|---|
| **Intermediate 198** | 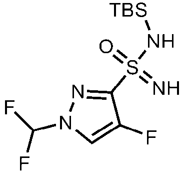 | N-(tert-butyldimethylsilyl)-1-(difluoromethyl)-4-fluoro-1H-pyrazole-3-sulfonimidamide | 329 |

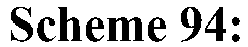

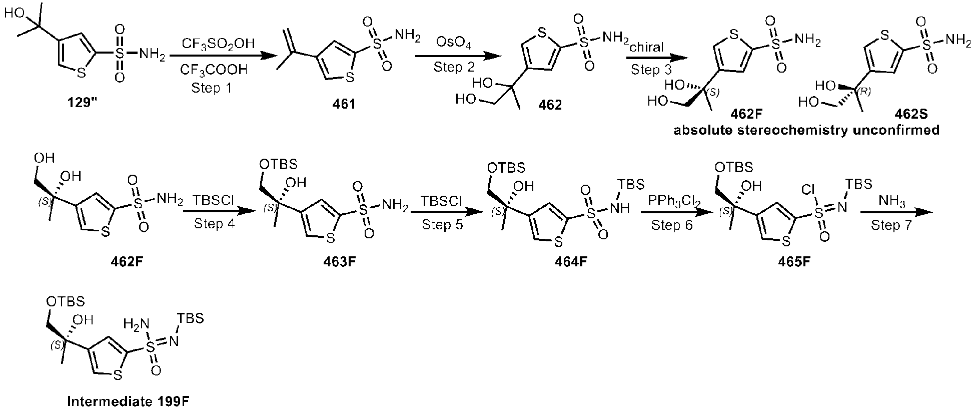

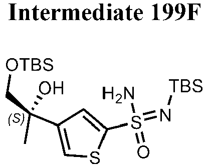

### N'-(tert-butyldimethylsilyl)-4-((S)-1-((tert-butyldimethylsilyl)oxy)-2-hydroxypropan-2-yl)thiophene-2-sulfonimidamide

### Step 1: 4-(Prop-1-en-2-yl)thiophene-2-sulfonamide

To a stirred solution of 4-(2-hydroxypropan-2-yl)thiophene-2-sulfonamide (50 g, 226 mmol) in CF₃SO₃H (60 mL) in a 500 mL round-bottom flask was added TFA (60 mL) dropwise at RT. The resulting solution was stirred for 16 h at 50 °C in an oil bath. The reaction mixture was concentrated under reduced pressure. The pH value of the residue was adjusted to 8 with aq. NaOH (3%wt.). The resulting solution was extracted with 3x300 mL of DCM and the organic layers were combined and concentrated under vacuum. The residue was eluted from silica gel with EtOAc/PE (1:2). This resulted in 10 g (22%) of the title compound as an off-white solid. MS-ESI: 202 (M-1).

### Step 2: 4-(1,2-Dihydroxypropan-2-yl)thiophene-2-sulfonamide

To a stirred solution of 4-(prop-1-en-2-yl)thiophene-2-sulfonamide (10 g, 49 mmol) in t-BuOH (40 mL) and acetone (40 mL) in a 250-mL round-bottom flask was added NMO (11.5 g, 98 mmol) and the resulting solution was stirred for 15 min at RT. Then to this was added a solution of OsO₄ (1.24 g, 4.9 mmol) in H₂O (60 mL) dropwise at RT. The resulting solution was stirred overnight at RT. The reaction was then quenched by the addition of saturated aq. Na₂S₂O₃ (50 mL). The resulting solution was extracted with 3x200 mL EtOAc. The organic layers were combined and dried over anhydrous Na₂SO₄ and concentrated under vacuum. The crude product was eluted from silica gel with MeOH/DCM (7:100). This resulted in 5.0 g (43%) of the title compound as yellow oil. MS-ESI: 236 (M-1)

### Step 3: (S) and (R) 4-(1,2-Dihydroxypropan-2-yl)thiophene-2-sulfonamide

The product from the Step above **(462,** 5.0 g) was resolved by prep-chiral HPLC using the following conditions: CHIRALPAK IG, 5*25 cm, 5 um; Mobile Phase A: CO₂, Mobile Phase B: MeOH:ACN=1:1 (2 mM NH₃-MeOH); Flow rate:150 mL/min; Gradient: 50% B; UV 220 nm; Rt₁: 4.1 min (462F); Rt₂: 7.4 min (462S). This resulted in 2.1 g (99% ee) of **462F** and 2.2 g (98% ee) of **462S,** both as white solids. MS-ESI: 236 (M-1).

### Step 4: (S)-4-(1-((tert-butyldimethylsilyl)oxy)-2-hydroxypropan-2-yl)thiophene-2-sulfonamide

To a stirred solution of (S)-4-(1,2-dihydroxypropan-2-yl)thiophene-2-sulfonamide (2.1 g, 8.8 mmol) in THF (50 mL) in a 250-mL round-bottom flask under nitrogen was added NaH (60% wt. dispersion in mineral oil, 704 mg, 17.6 mmol) at 0 °C in a water/ice bath. The resulting solution was stirred for 20 min at RT. To the stirred solution was added TBSCl (2.64 g, 17.6 mmol) at 0 °C. The resulting solution was stirred overnight at RT. The reaction was then quenched by the addition of 30 mL of water. The resulting solution was extracted with 3x30 mL of EtOAc. The combined organic layers were dried over anhydrous Na₂SO₄ and concentrated under vacuum. The residue was eluted from silica gel with EtOAc/PE (1:5). This resulted in 2.17 g (70%) of the title compound as an off-white solid. MS-ESI: 352 (M+1).

Steps 5-7 used similar procedures for converting compound **442** to Intermediate **195** shown in Scheme 91 to afford Intermediate **199F** from compound **463F.** MS-ESI: 465 (M+1).

**Table 45. The Intermediates in the following Table were prepared using the similar procedures for converting compound 462F to Intermediate 199F shown in Scheme 94 using 462S.**

| Intermediate # | Structure | IUPAC Name | Exact Mass [M-H]⁻ |
|---|---|---|---|
| **Intermediate 199S** | 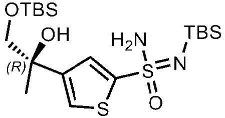 | N'-(tert-butyldimethylsilyl)-4-((R)-1-((tertbutyldimethylsilyl)oxy)-2-hydroxypropan-2-yl)thiophene-2-sulfonimidamide | 465 |

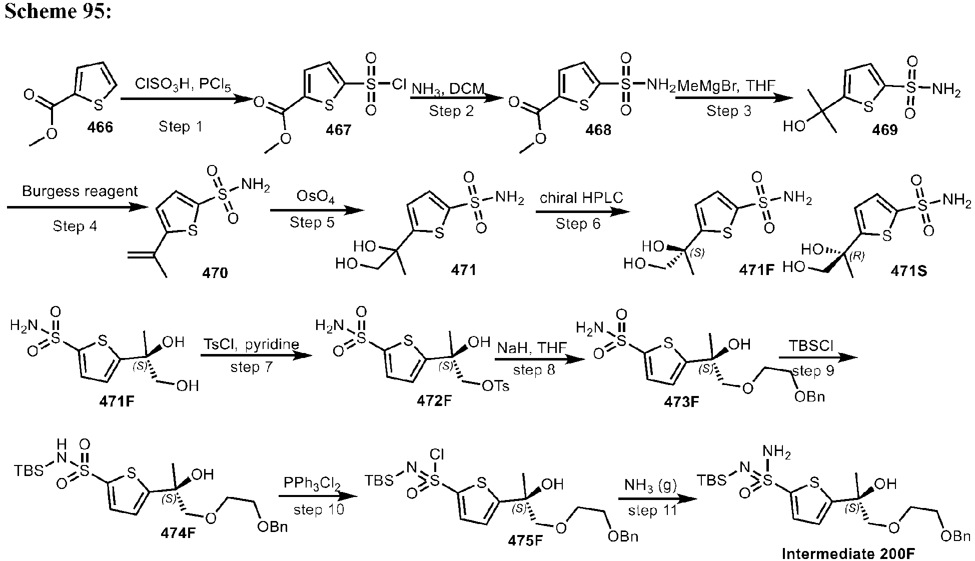

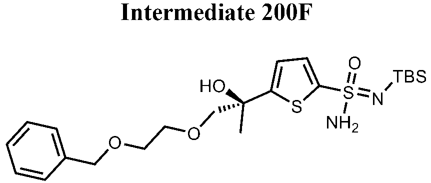

### 5-((S)-1-(2-(benzyloxy)ethoxy)-2-hydroxypropan-2-yl)-N'-(tert-butyldimethylsilyl)thiophene-2-sulfonimidamide

### Step 1: Methyl 5-(chlorosulfonyl)thiophene-2-carboxylate

To a stirred solution of methyl thiophene-2-carboxylate (2.13 g, 15 mmol) in CHCl₃ (100 mL) in a 250-mL round-bottom flask was added ClSO₃H (3.5 g, 30 mmol) dropwise at 0 °C in an ice/water bath. The resulting solution was stirred for 1 h at 0 °C. This was followed by addition of PCl₅ (6.25 g, 30 mmol) in portions at 0 °C. The resulting solution was stirred overnight at 50 °C. The reaction was quenched by the addition of ice/water (100 mL). The mixture was extracted with DCM (3x100 mL). The organic layer was dried over anhydrous Na₂SO₄ and concentrated under vacuum. This resulted in 4.0 g (crude) of the title compound as yellow oil. MS-ESI: 241/243 (M+1)

### Step 2: Methyl 5-sulfamoylthiophene-2-carboxylate

To a stirred solution of methyl 5-(chlorosulfonyl)thiophene-2-carboxylate (4.0 g, crude) in DCM (20 mL) in a 250-mL round-bottom flask was bubbled NH₃ at 0 °C for 15 min. The resulting solution was stirred overnight at RT. The resulting mixture was washed with H₂O (3x100 mL). The organic layer was dried over anhydrous Na₂SO₄ and concentrated under vacuum. This resulted in 1.99 g (60%, over two steps) of the title compound as a yellow solid. MS-ESI: 222 (M+1).

### Step 3: 5-(2-Hydroxypropan-2-yl)thiophene-2-sulfonamide

To a stirred solution of methyl 5-sulfamoylthiophene-2-carboxylate (1.0 g, 4.52 mmol) in THF (40 mL) in a 100-mL 3-necked round-bottom flask under nitrogen was added CH₃MgBr in THF (3.0 M, 7.5 mL, 22.6 mmol) dropwise at 0 °C in an ice/water bath. The resulting solution was stirred overnight at RT. The reaction solution was then quenched by adding sat. NH₄Cl (40 mL) at 0 °C. The resulting mixture was extracted with EtOAc (3x40 mL). The organic layer was dried over anhydrous Na₂SO₄ and concentrated under vacuum. The residue was eluted from silica gel with EtOAc/PE (1:2). This resulted in 500 mg (50%) of the title compound as a yellow solid. MS-ESI: 222 (M+1).

### Step 4: 5-(Prop-1-en-2-yl)thiophene-2-sulfonamide

To a stirred solution of 5-(2-hydroxypropan-2-yl)thiophene-2-sulfonamide (500 mg, 2.25 mmol) in THF(30 mL) in a 100-mL round-bottom flask under nitrogen was added Burgess reagent (1.15 g, 4.5 mmol) in portions at RT. The resulting mixture was stirred overnight at RT. The resulting mixture was quenched by addition of H₂O (10 mL) and extracted with EtOAc (3x20 mL). The organic layer was dried over anhydrous Na₂SO₄ and concentrated under vacuum. The residue was purified by Prep-TLC (PE/EtOAc=1:2). This resulted in 420 mg (91.5%) of the title compound as a white solid. MS-ESI: 204 (M+1).

### Step 5: 5-(1,2-Dihydroxypropan-2-yl)thiophene-2-sulfonamide

To a stirred solution of 5-(prop-1-en-2-yl)thiophene-2-sulfonamide (420 mg, 2.06 mmol) in t-BuOH (4.0 mL) and acetone (4.0 mL) in a 50-mL round-bottom flask was added NMO (483 mg, 4.12 mmol) and the resulting solution was stirred for 15 min at RT. Then to this was added a solution of OsO₄ (53 mg, 0.21 mmol) in H₂O (3.0 mL) dropwise at RT. The resulting solution was stirred overnight at RT. The reaction was then quenched by the addition of saturated aq. Na₂S₂O₃ (5.0 mL). The resulting solution was extracted with 3x10 mL EtOAc. The organic layers were combined and dried over anhydrous Na₂SO₄ and concentrated under vacuum. The crude product was eluted from silica gel with MeOH/DCM (7:100). This resulted in 245 mg (50%) of the title compound as yellow oil. MS-ESI: 238 (M+1).

### Step 6: (S) and (R)-5-(1,2-dihydroxypropan-2-yl)thiophene-2-sulfonamide

The product from Step 5 above **(471,** 245 mg) was resolved by prep-chiral HPLC using the following conditions: CHIRALPAK AD-H SFC, 5*25 cm, 5 um; Mobile Phase A: CO₂, Mobile Phase B: MeOH:ACN=1:1(2 mM NH₃-MeOH); Flow rate:150 mL/min; Gradient: 50% B; 220 nm; Rt₁: 4.1 min **(471F);** Rt₂: 7.4 min **(471S).** This resulted in 100 mg (99% ee) of **471F** and 110 mg (98% ee) of **471S,** both as yellow oil. MS-ESI: 236 (M-1).

### Step 7: (S)-2-hydroxy-2-(5-sulfamoylthiophen-2-yl)propyl 4-methylbenzenesulfonate

To a stirred solution of (S)-5-(1,2-dihydroxypropan-2-yl)thiophene-2-sulfonamide (100 mg, 0.42 mmol) in pyridine (8.0 mL) in a 50-mL round-bottom flask was added TsCl (239 mg, 1.26 mmol). The resulting solution was stirred for 16 h at RT. The resulting solution was diluted with 20 mL of H₂O. The resulting solution was extracted with 3x20 mL of EtOAc and the organic layers were combined and concentrated under vacuum. The residue was eluted from silica gel with EtOAc/PE (1:10). This resulted in 125 mg (76%) of the title compound as yellow oil. MS-ESI: 392 (M+1).

### Step 8: (S)-5-(1-(2-(benzyloxy)ethoxy)-2-hydroxypropan-2-yl)thiophene-2-sulfonamide

To a stirred solution of 2-(benzyloxy)ethan-1-ol (243 mg, 1.6 mmol) in THF(10 mL) in a 50-mL round-bottom flask under nitrogen was added NaH (60% wt. dispersion in mineral oil, 128 mg, 3.2 mmol) at 0 °C in an ice/water bath. The resulting mixture was stirred for 30 min at 0 °C. To the above mixture was added (S)-2-hydroxy-2-(5-sulfamoylthiophen-2-yl)propyl 4-methylbenzenesulfonate (125 mg, 0.32 mmol) at 0 °C. The resulting mixture was stirred for 32 h at 35 °C. The reaction was quenched with H₂O (10 mL) at 0 °C. The resulting mixture was extracted with EtOAc (3 x 10 mL). The combined organic layers were washed with brine (2x10 mL), dried over anhydrous Na₂SO₄ and concentrated under vacuum. The residue was eluted from silica gel with PE/EtOAc (1:2). This resulted in 113 mg (95%) as yellow oil. MS-ESI: 372 (M+1).

Steps 9-11 used similar procedures for converting compound **442** to Intermediate **195** shown in Scheme 91 to afford Intermediate **200F** from compound **473F.** MS-ESI: 485 (M+1).

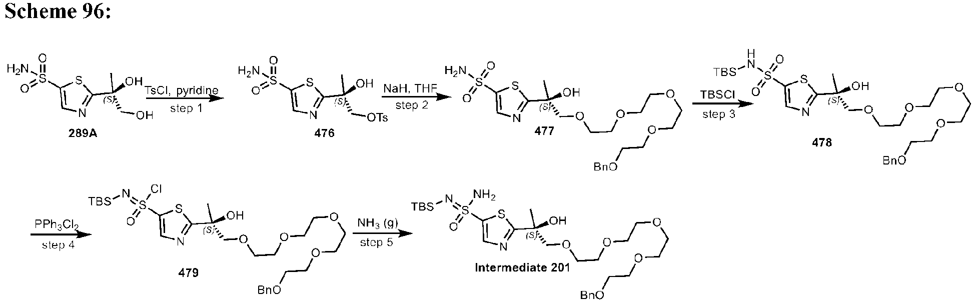

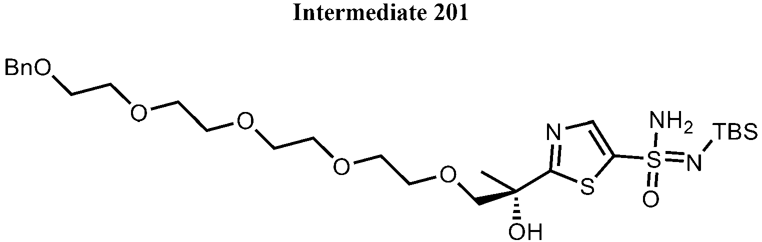

### N'-(tert-butyldimethylsilyl)-2-((S)-16-hydroxy-1-phenyl-2,5,8,11,14-pentaoxaheptadecan-16-yl)thiazole-5-sulfonimidamide

Steps 1-6 used similar procedures for converting compound **471F** to Intermediate **200F** shown in Scheme 95 to afford Intermediate **201** from compound **289A.** MS-ESI: 618 (M+1)

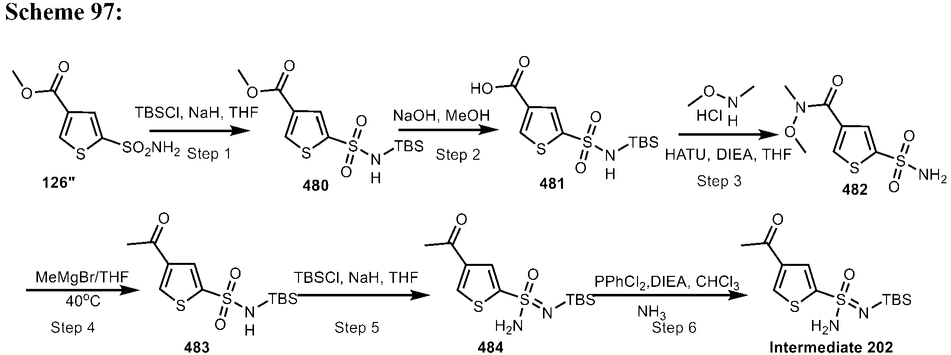

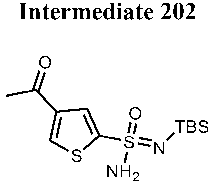

### 4-Acetyl-N'-(tert-butyldimethylsilyl)thiophene-2-sulfonimidamide

### Step 1: Methyl 5-(N-(tert-butyldimethylsilyl)sulfamoyl)thiophene-3-carboxylate

To a stirred solution of methyl 5-sulfamoylthiophene-3-carboxylate (10 g, 45 mmol) in THF (120 mL) in a 250-mL round-bottom flask under nitrogen was added NaH (60% wt. dispersion in mineral oil, 5.42 g, 135 mmol) in portions at 0 °C in an ice/water bath. To the stirred solution was added TBSCl (8.17 g, 54 mmol). The resulting solution was stirred overnight at RT. The reaction was quenched by the addition of 100 mL of water. The resulting solution was extracted with 2x200 mL of EtOAc and dried over anhydrous Na₂SO₄ and concentrated. The resulting mixture was washed with 3 x100 ml of PE. This resulted in 9.9 g (65%) of the title compound as an off-white solid. MS-ESI: 336 (M+1).

### Step 2: 5-(N-(tert-butyldimethylsilyl)sulfamoyl)thiophene-3-carboxylic acid

To a stirred solution of methyl 5-(N-(tert-butyldimethylsilyl)sulfamoyl)thiophene-3-carboxylate (5.0 g, 14.9 mmol) in MeOH (100 mL) and H₂O (5.0 mL) in a 250-mL round-bottom flask was added NaOH (1.19 g, 30 mmol) in portions at 0 °C in an ice/water bath. The resulting solution was stirred overnight at RT. The resulting mixture was concentrated under vacuum. The resulting solution was diluted with 100 mL of H₂O. The pH value was adjusted to 3 with HCl (6 M). The resulting solution was extracted with 2x150 mL of EtOAc and dried over anhydrous Na₂SO₄ and concentrated. This resulted in 4.7 g (98%) of the title compound as a yellow solid. MS-ESI: 322 (M+1).

### Step 3: N-methoxy-N-methyl-5-sulfamoylthiophene-3-carboxamide

To a stirred solution of 5-(N-(tert-butyldimethylsilyl)sulfamoyl)thiophene-3-carboxylic acid (4.7 g, 14.6 mmol) in THF (125 mL) in a 250-mL round-bottom flask was added N,O-dimethylhydroxylamine (1.34 g, 22 mmol), DIEA (5.65 g, 43.8 mmol) and HATU (11.1 g, 29.2 mmol) at RT. The resulting solution was stirred overnight at RT. The reaction solution was diluted with H₂O (100 mL). The mixture was extracted with EtOAc (3x100 mL). The organic layer was dried with anhydrous Na₂SO₄ and concentrated. The residue was eluted from silica gel with EtOAc/PE (1:3). This resulted in 1.65 g (45%) of the title compound as a light yellow solid. MS-ESI: 251 (M+1).

### Step 4: 4-Acetyl-N-(tert-butyldimethylsilyl)thiophene-2-sulfonamide

To a stirred solution of N-methoxy-N-methyl-5-sulfamoylthiophene-3-carboxamide (1.65 g, 6.57 mmol) in THF (60 mL) in a 250-mL 3-necked round-bottom flask under nitrogen was added MeMgBr in THF (3 M, 8.76 mL, 26.3 mmol) dropwise at 0 °C in an ice/water bath. The resulting solution was stirred for 2 h at 40 °C in an oil bath. The reaction was then quenched by the addition of 50 mL of ice/salt. The resulting solution was extracted with 3x100 mL of EtOAc and dried over anhydrous Na₂SO₄ and concentrated. The residue was eluted from silica gel with EtOAc/PE (1:5). This resulted in 1.93 g (92%) of the title compound as a light yellow solid. MS-ESI: 320 (M+1).

Steps 5-6 used similar procedures for converting compound **442** to Intermediate **195** shown in Scheme 91 to afford Intermediate **202** from compound **483.** MS-ESI: 319 (M+1).

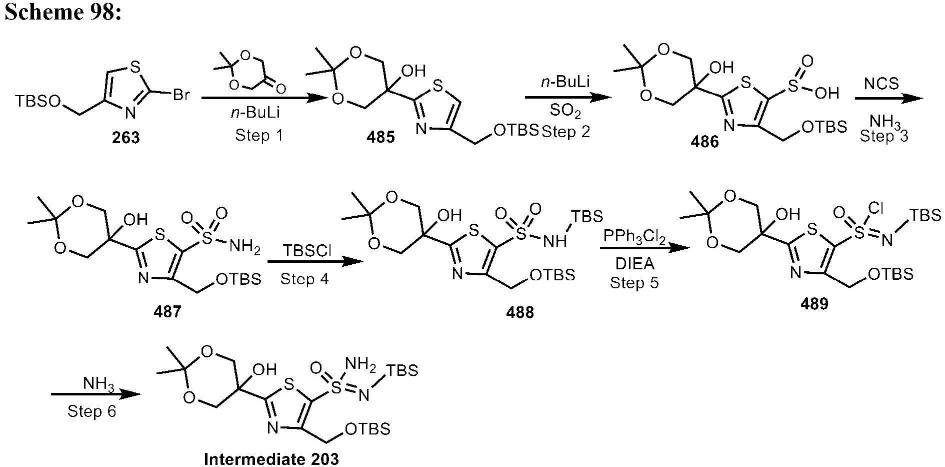

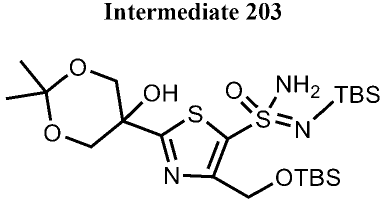

### N'-(tert-butyldimethylsilyl)-4-(((tert-butyldimethylsilyl)oxy)methyl)-2-(5-hydroxy-2,2-dimethyl-1,3-dioxan-5-yl)thiazole-5-sulfonimidamide

### Step 1: 5-(4-(((Tert-butyldimethylsilyl)oxy)methyl)thiazol-2-yl)-2,2-dimethyl-1,3-dioxan-5-ol

To a stirred solution of 2-bromo-4-(((tert-butyldimethylsilyl)oxy)methyl)thiazole (1.40 g, 4.55 mmol) in THF (30 mL) in a 100-mL 3-necked round-bottom flask under nitrogen was added n-BuLi in hexane (2.5 M, 1.8 mL, 4.55 mmol) dropwise at -78 °C in a liquid nitrogen/EtOH bath. The resulting solution was stirred for 30 min at -78 °C. Then to the mixture was added 2,2-dimethyl-1,3-dioxan-5-one (621 mg, 4.78 mmol) in portions at -78 °C. The resulting mixture was stirred for 1 h at -78 °C. The reaction was quenched by the addition of H₂O (20 mL) at 0 °C. The resulting mixture was extracted with EtOAc (3x30 mL). The combined organic layers were washed with brine, dried over anhydrous Na₂SO₄ and concentrated under vacuum. The residue was purified by Prep-TLC (PE/EtOAc 10:1). This resulted in 1.1 g (67%) of the title compound as brown oil. MS-ESI: 360 (M+1).

### Step 2: 4-(((Tert-butyldimethylsilyl)oxy)methyl)-2-(5-hydroxy-2,2-dimethyl-1,3-dioxan-5-yl)thiazole-5-

### sulfinic acid

To a stirred solution of 5-(4-(((tert-butyldimethylsilyl)oxy)methyl)thiazol-2-yl)-2,2-dimethyl-1,3-dioxan-5-ol (1.1 g, 3.06 mmol) in THF (30 mL) in a 100-mL 3-necked round-bottom flask under nitrogen was added n-BuLi in hexane (2.5 M, 2.7 mL, 6.73 mmol) dropwise at -78 °C in a liquid nitrogen/EtOH bath. The reaction solution was stirred for 30 min at -78 °C. Then SO₂ (g) was bubbled to the solution at -50 °C for 20 min. The resulting solution was allowed to react, with stirring, for an additional 2 h at RT. The resulting mixture was concentrated directly under vacuum. This resulted in 1.5 g (crude) of the title compound as a yellow solid. MS-ESI: 422 (M-1).

### Step 3: 4-(((Tert-butyldimethylsilyl)oxy)methyl)-2-(5-hydroxy-2,2-dimethyl-1,3-dioxan-5-yl)thiazole-5- sulfonyl chloride

To a stirred solution of 4-(((tert-butyldimethylsilyl)oxy)methyl)-2-(5-hydroxy-2,2-dimethyl-1,3-dioxan-5-yl) thiazole-5-sulfinic acid (1.5 g, crude) in THF (20 mL) in a 100-mL round-bottom flask was added NCS (610 mg, 4.59 mmol) in portions at 0 °C. The resulting mixture was stirred for 2 h at 0 °C. To the reaction solution was added NH₃ in THF (0.5 M, 50 mL) in portions at 0 °C. The resulting mixture was stirred for 2 h at 0 °C. The resulting mixture was concentrated under vacuum. The residue was purified by Prep-TLC (PE/EtOAc = 7:1). This resulted in 685 mg (51%, over two steps) of the title compound as a brown solid. MS-ESI: 437 (M-1).

Steps 4-6 used similar procedures for converting compound **442** to Intermediate **195** shown in Scheme 91 to afford Intermediate **203** from compound **487.** MS-ESI: 552 (M+1).

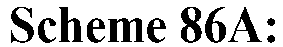

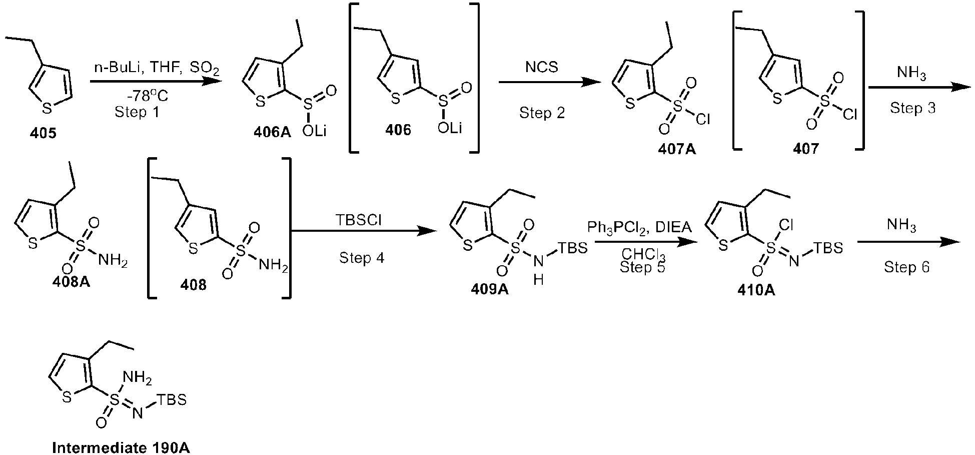

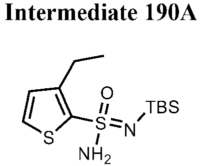

### N'-(tert-butyldimethylsilyl)-3-ethylthiophene-2-sulfonimidamide

Steps 1-2 used similar procedures for converting compound **485** to compound **487** shown in Scheme 98 to afford compound **407A** from compound **405.**

### Step 3: 3-Ethylthiophene-2-sulfonamide and 4-Ethylthiophene-2-sulfonamide

To the mixture of 3-ethylthiophene-2-sulfonyl chloride and 4-ethylthiophene-2-sulfonyl chloride from last step in DCM (200 mL) was bubbled NH₃ at 0 °C for 15 min. The solution was washed with brine (2 x 100 mL), dried over Na₂SO₄ and concentrated under vacuum. The residue was eluted from silica gel with EtOAc/PE (1:20). This resulted in 5.0 g (29%) of **408A** and 5.1 g (30%) of **408** both as a light yellow solid. MS-ESI: 190 (M-1).

Steps 4-6 used similar procedures for converting compound **442** to Intermediate **195** shown in Scheme 91 to afford Intermediate **190A** from compound **408A.** MS-ESI: 305 (M+1).

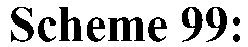

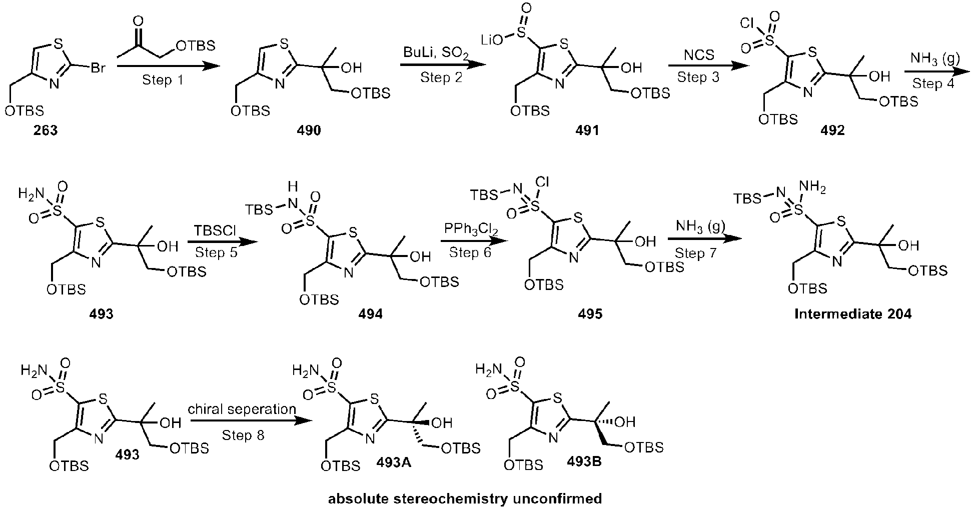

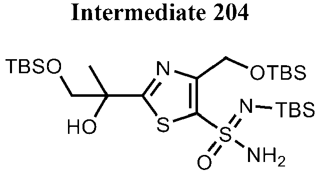

### Step 1: 1-((Tert-butyldimethylsilyl)oxy)-2-(4-(((tert-butyldimethylsilyl)oxy)methyl)thiazol-2-yl)propan -2-ol

To a stirred solution of 2-bromo-4-(((tert-butyldimethylsilyl)oxy)methyl)thiazole (3.0 g, 9.7 mmol) in THF (100 mL) in a 250-mL 3-necked round-bottom flask under nitrogen was added n-BuLi in hexane (2.5 M, 4.0 mL, 9.7 mmol) dropwise at -78 °C in a liquid nitrogen/EtOH bath. The resulting solution was stirred for 30 min at -78 °C. To the mixture was added 1-((tertbutyldimethylsilyl)oxy)propan-2-one (1.83 g, 9.7 mmol) at -78 °C in 10 min. The resulting solution was stirred for 12 h at RT. The reaction was then quenched by the addition of 10 mL of H₂O (100 mL). The resulting mixture was extracted with 3x200 mL of DCM and the organic layers were combined and concentrated under vacuum. The residue was eluted from silica gel with EtOAc/PE (1:10). This resulted in 2.5 g (62%) of the title compound as yellow oil. MS-ESI: 418 (M+1).

Steps 2-7 used similar procedures for converting compound **485** to Intermediate **203** shown in Scheme 98 to afford Intermediate **204** from compound **490.** MS-ESI: 610 (M+1).

### Step 8: (S) and (R)-2-(1-((tert-butyldimethylsilyl)oxy)-2-hydroxypropan-2-yl)-4-(((tertbutyldimethylsilyl)oxy) methyl)thiazole-5-sulfonamide

The product from the Step above **(493,** 2.4 g) was resolved by prep-chiral-HPLC using the following conditions: CHIRALPAK AD-H SFC, 5*25 cm, 5 um; Mobile Phase A: CO₂, Mobile Phase B: MeOH (2 mM NH₃); Flow rate: 200 mL/min; Gradient: 50% B; UV 220 nm; Rt₁: 4.3 min (493A); Rt₂: 6.9 min (493B); This resulted in 1.1 g of **493A** (99% ee) and 1.0 g of **493B** (99% ee). MS-ESI: 495 (M-1)

**Table 46. The Intermediates in the following Table were prepared using the similar procedures for converting compound 493 to Intermediate 204 shown in Scheme 99 using 493A and 493B.**

| Intermediate # | Structure | IUPAC Name | Exact Mass [M-H]⁻ |
|---|---|---|---|
| **Intermediate 204A** | 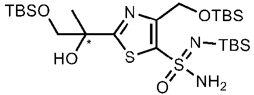 | (R) or (S) N'-(tert-butyldimethylsilyl)-2-(1-((tert-butyldimethylsilyl)oxy)-2-hydroxypropan-2-yl)-4-(((tert-butyldimethylsilyl)oxy)methyl)thiazol e-5-sulfonimidamide | 610 |
| **Intermediate 204B** | 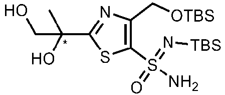 | (S) or (R) N'-(tert-butyldimethylsilyl)-2-(1-((tert-butyldimethylsilyl)oxy)-2-hydroxypropan-2-yl)-4-(((tert-butyldimethylsilyl)oxy)methyl)thiazol e-5-sulfonimidamide | 610 |

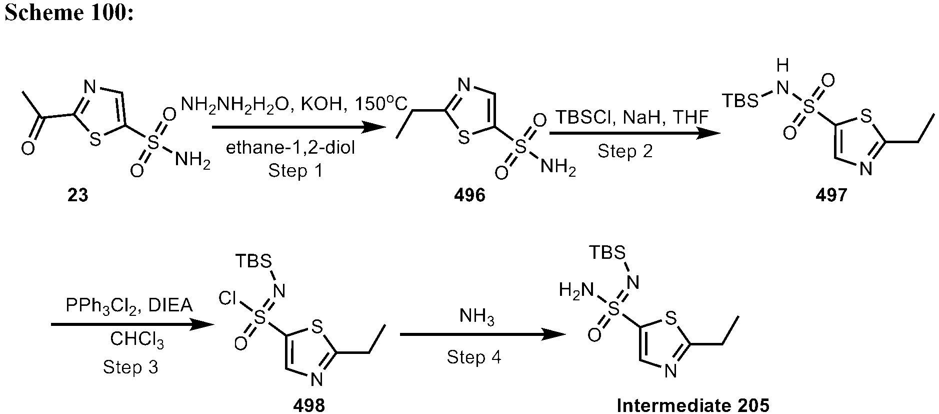

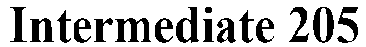

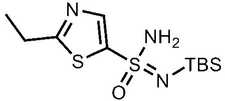

### N'-(tert-butyldimethylsilyl)-2-ethylthiazole-5-sulfonimidamide

### Step 1: 2-Ethylthiazole-5-sulfonamide

To a stirred solution of 2-acetylthiazole-5-sulfonamide (1.0 g, 4.8 mmol) in ethylene glycol (20 mL) in a 50 mL round-bottom flask was added NH₂NH₂·H₂O (1.2 g, 24 mmol) dropwise at RT. The resulting mixture was stirred for 2 h at 150 °C under nitrogen. The mixture was allowed to cool down to RT. To the above mixture was added KOH (544 mg, 9.6 mmol) at RT. The resulting mixture was stirred for additional 1.5 h at 150 °C. The residue was eluted from silica gel column with DCM/MeOH (15:1). This resulted in 430 mg (46%) of the title compound as a yellow solid. MS-ESI: 191 (M-1).

Steps 2-4 used similar procedures for converting compound **442** to Intermediate **195** shown in Scheme 91 to afford Intermediate **205** from compound **496.** MS-ESI: 306 (M+1).

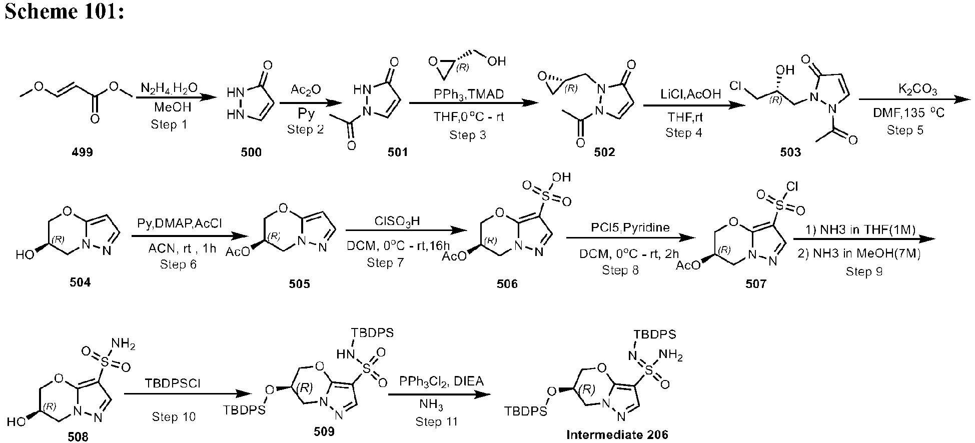

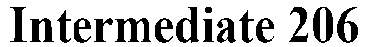

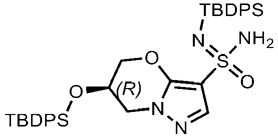

### (6R)-N'-(tert-butyldiphenylsilyl)-6-((tert-butyldiphenylsilyl)oxy)-6,7-dihydro-5H-pyrazolo[5,1-b][1,3]oxazine-3-sulfonimidamide

### Step 1: 1,2-Dihydropyrazol-5-one

To a 5 L 4-neck flask containing a solution of methyl (E)-3-methoxyacrylate (2000 g, 17.2 mol) in MeOH (2.0 L) was added hydrazine hydrate (921 g, 18.4 mol) dropwise at RT under nitrogen. The resulting mixture was stirred for 90 min at 60 °C under nitrogen. The resulting mixture was concentrated under reduced pressure. This resulted in the title compound (1467 g, 68%wt, yield 69%) as an off-white solid. MS-ESI: 85 (M+1).

### Step 2: 2-Acetyl-1,2-dihydropyrazol-5-one

To a 10 L 4-neck flask containing a solution of 1,2-dihydropyrazol-5-one (1467 g, 68%wt, 11.9 mol) in pyridine (6.0 L) was added Ac₂O (1214 g, 11.9 mol) at RT under nitrogen. The resulting mixture was stirred for 1.5 h at 95 °C under nitrogen. The resulting mixture was concentrated under reduced pressure. The residue was slurry with MeOH (1x3000 mL). The resulting mixture was filtered, the filter cake was washed with MeOH (1x500 mL). The filter cake was dried under reduced pressure. This resulted in the title compound (1630 g, 78%wt, 85%) as an off-white solid. MS-ESI: 127 (M+1). ¹H NMR (400 MHz, DMSO-*d*₆) δ 8.12 (d, J = 3.0 Hz, 1H), 6.00 (d, J = 3.0 Hz, 1H), 2.48 (s, 3H), 2.44 (s, 1H).

### Step 3: (R)-2-acetyl-1-(oxiran-2-ylmethyl)-1,2-dihydropyrazol-5-one

To a 10 L 4-neck flask containing a solution of 2-acetyl-1,2-dihydropyrazol-5-one (400 g, 78%wt, 3.17 mol) and R-glycidol (246 g, 3.33 mol) in THF (4.0 L), to the stirred solution was added PPh₃ (915 g, 3.49 mol). To the above mixture was added TMAD (705 g, 3488 mmol) in portions at 0 °C. The resulting mixture was stirred for additional 1 h at RT. The resulting mixture was quenched with 1x4.0 L of water. The aqueous layer was extracted with EtOAc (3x1.0 L). The organic layers were combined and washes with brine (1 L), dried over Na₂SO₄ and concentrated under reduced pressure. The crude was slurry in PE/EtOAc (10:1) (16 L) for 4 h. The resulting mixture was filtered. The filter cake was washed with PE/EtOAc (10:1) (1x1000 mL). The filtrate was concentrated under reduced pressure. This resulted in the title compound (470 g, 84%wt, 87%) as an off-white solid. MS-ESI: 183 (M+1). ¹H NMR (400 MHz, DMSO-*d*₆) δ 8.27 (d, J = 3.0 Hz, 1H), 6.27 (d, J = 3.0 Hz, 1H), 4.56 (dd, J = 11.8, 2.7 Hz, 1H), 4.02 (dd, J = 11.8, 6.8 Hz, 1H), 3.40-3.34 (m, 1H), 2.86 (dd, J = 5.1, 4.3 Hz, 1H), 2.74 (dd, J = 5.1, 2.6 Hz, 1H), 2.54 (s, 3H).

### Step 4: (R)-2-acetyl-1-(3-chloro-2-hydroxypropyl)-1,2-dihydropyrazol-5-one

To a 10 L 3-neck flask was placed a solution of (R)-2-acetyl-1-(oxiran-2-ylmethyl)-1,2-dihydropyrazol -5-one (500 g, 84%wt, 2.74 mol) in THF (2.5 L), to the stirred solution was added AcOH (494 g, 8233 mmol) dropwise and LiCl (186 g, 4391 mmol) in portions at 0 °C under nitrogen. The resulting mixture was stirred for 16 h at RT under nitrogen. The reaction was quenched with water at RT. The aqueous layer was extracted with EtOAc (3x3.0 L). The organic layers were combined and washed with 2x3.0 L of sat. NaHCO₃ and 5.0 L of brine. The organic layer was dried over Na₂SO₄ and concentrated under reduced pressure. This resulted in the title compound (552 g, 82%wt, yield 90%) as an off-white solid. MS-ESI: 219 (M+1). ¹H NMR (400 MHz, CDCl₃) δ 8.10 (d, *J =* 3.0 Hz, 1H), 6.02 (d, *J* = 3.0 Hz, 1H), 4.44 (d, *J =* 5.0 Hz, 2H), 4.29-4.23 (m, 1H), 3.81- 3.67 (m, 2H), 2.61 (s, 3H).

### Step 5: (R)-6,7-dihydro-5H-pyrazolo[5,1-b][1,3]oxazin-6-ol

To a 10 L 3-neck flask was placed a solution of (R)-2-acetyl-1-(3-chloro-2-hydroxypropyl)-1,2-dihydropyrazol-5-one (500 g, 82%wt, 2.29 mol) in DMF (5.0 L), to the stirred solution was added K₂CO₃ (948 g, 6.86 mol) under nitrogen. The resulting mixture was stirred for 16 h at 135 °C under nitrogen and concentrated under reduced pressure. The residue was purified by silica gel column chromatography, eluted with DCM/MeOH (20:1) to afford (152 g, yield 58%) of the title compound as an off-white solid. MS-ESI: 141 (M+1). ¹H NMR (400 MHz, MeOH-*d*₄) δ 7.11 (d, *J =* 2.1 Hz, 1H), 5.31 (d, *J =* 2.1 Hz, 1H), 4.19 - 4.12 (m, 1H), 4.12 - 3.98 (m, 3H), 3.90 - 3.81 (m, 1H).

### Step 6: (R)-6,7-dihydro-5H-pyrazolo[5,1-b][1,3]oxazin-6-yl acetate

To a stirred solution of (R)-6,7-dihydro-5H-pyrazolo[5,1-b][1,3]oxazin-6-ol (55.0 g, 392 mmol) in MeCN (825 mL) in a 2-L 3-necked round-bottom flask under nitrogen was added pyridine (93.1 g, 1.18 mol) and DMAP (4.79 g, 39.2 mmol). This was followed by the addition of acetyl chloride (43.1 g, 549 mmol) dropwise with stirring at 0 °C. The resulting solution was stirred for 2 h at RT. LCMS showed reaction was completed. The resulting mixture was concentrated directly. The residue was eluted from silica gel with EtOAc/PE (1:4). This resulted in 58 g (81%) of the title compound as a light yellow solid. MS-ESI: 183 (M+1). ¹H NMR (400 MHz, CDCl₃) δ 7.39 (d, *J* = 2.0 Hz, 1H), 5.57 (d, *J =* 2.0 Hz, 1H), 5.45-5.36 (m, 1H), 4.49-4.41 (m, 1H), 4.40-4.27 (m, 2H), 4.24 (dd, *J =* 12.1, 1.5 Hz, 1H), 2.14 (s, 3H).

### Step 7: (R)-6-acetoxy-6,7-dihydro-5H-pyrazolo[5,1-b][1,3]oxazine-3-sulfonic acid

To a stirred solution of (R)-6,7-dihydro-5H-pyrazolo[5,1-b][1,3]oxazin-6-yl acetate (58.0 g, 318 mmol) in DCM (120 mL) in a 2-L 3-necked round-bottom flask under nitrogen was added chlorosulfonic acid (81.3 g, 700 mmol) dropwise at 0 °C. The resulting solution was stirred for 12 h at RT. LCMS showed the conversion was completed. The reaction mixture was used directly in the next step. MS-ESI: 263 (M+1).

### Step 8: (R)-3-(chlorosulfonyl)-6,7-dihydro-5H-pyrazolo[5,1-b][1,3]oxazin-6-yl acetate

To a stirred solution of (R)-6-acetoxy-6,7-dihydro-5H-pyrazolo[5,1-b][1,3]oxazine-3-sulfonic acid in DCM (crude from step 7) in a 2-L 3-necked round-bottom flask under nitrogen was added pyridine (55.1 g, 696 mmol) dropwise at 0 °C. To this was added PCl₅ (144 g, 696 mmol) in portions at 0 °C. The resulting solution was stirred for 2 h at RT. The reaction was then quenched by the addition of 1 L of water/ice. The resulting solution was extracted with 3x300 mL of EtOAc. The resulting mixture was washed with 2 x500 ml of NaHCO₃ and 1 x500 mL of H₂O. The resulting mixture was washed with 1x500 mL of NaCl (aq.). The mixture was dried over anhydrous Na₂SO₄ and concentrated under vacuum. This resulted in 76 g (crude) of the title compound as a light yellow solid. MS-ESI: 281/283 (M+1).

### Step 9: (R)-6-hydroxy-6,7-dihydro-5H-pyrazolo[5,1-b][1,3]oxazine-3-sulfonamide

To a stirred solution of NH₃ in THF (1 M, 730 ml) in a 3-L round-bottom flask was added (R)-3-(chloro- sulfonyl)-6,7-dihydro-5H-pyrazolo[5,1-b][1,3] oxazin-6-yl acetate (73.0 g, 260 mmol) in several batches. The flask was then filled with NH₃ (balloon). The resulting solution was stirred overnight at 40 °C in an oil bath. After reaction completed, the solids were filtered out. The filtrate was concentrated. The residue was diluted with NH₃ (7 M in MeOH, 730 mL). The resulting solution was stirred for 2h at RT. LC showed the reaction was complete. The MeOH solution was concentrated under reduced pressure to a final volume of about 100 mL. Et₂O (360 ml) was charged to the resulting solution and kept stirring for 30 min. The mixture was filtered, and the cake washed with Et₂O (100 mL). The white solid was dried under vacuum. This resulted in 37 g (53% for 3 steps) of the title compound as a white solid. MS-ESI: 220 (M+1). ¹H NMR (300 MHz, DMSO-*d*₆) δ 7.48 (s, 1H), 7.06 (s, 2H), 5.64 (d, *J =* 2.3 Hz, 1H), 4.30 (s, 3H), 4.30 - 4.18 (m, 1H), 4.00 - 3.90 (m, 1H).

### Step 10: (R)-N-(tert-butyldiphenylsilyl)-6-((tert-butyldiphenylsilyl)oxy)-6,7-dihydro-5H-pyrazolo[5,1-b] -[1,3]oxazine-3-sulfonamide

To a stirred solution of (R)-6-hydroxy-6,7-dihydro-5H-pyrazolo[5,1-b][1,3] oxazine-3-sulfonamide (500 mg, 2.28 mmol) in DMF (20 mL) in a 50-mL round-bottom flask was added DBU (2.08 g, 13.7 mmol) and TBDPSCl (5.02 g, 18.2 mmol) at 0 °C. The resulting solution was stirred for 2 h at RT and then diluted with 30 mL of H₂O. The resulting solution was extracted with 3x100 mL of EtOAc. The combined organic layers were dried over anhydrous Na₂SO₄ and concentrated under reduced pressure. The residue was eluted from silica gel with EtOAc/PE (1:1). This resulted in 530 mg (33.3%) of the title compound as a solid. MS-ESI: 696 (M+1).

### Step 11: (6R)-N'-(tert-butyldiphenylsilyl)-6-((tert-butyldiphenylsilyl)oxy)-6,7-dihydro-5H-pyrazolo [5,1-b][1,3]oxazine-3-sulfonimidamide

To a stirred solution of PPh₃Cl₂ (759 mg, 2.28 mmol) in DCE (30 mL) in a 100-mL 3-necked round-bottom flask under nitrogen was added DIEA (492 mg, 3.81 mmol) at 0 °C. The resulting solution was stirred for 10 min at RT. Then (R)-N-(tert-butyldiphenylsilyl)-6-((tert-butyldiphenylsilyl)oxy)-6,7-dihydro-5H- pyrazolo[5,1-b][1,3] oxazine-3-sulfonamide (530 mg, 0.76 mmol) in CHCl₃ (10 mL) was added at 0 °C. The resulting solution was stirred for 30 min at 0 °C. Then NH₃ (g) was bubbled to the reaction mixture for 15 min at 0 °C. The resulting solution was stirred for 2 h at RT. The solids were filtered out. The resulting mixture was concentrated under vacuum. The residue was eluted from silica gel with EtOAc/PE (1:1). This resulted in 400 mg (75.6%) of the title compound as a white solid. MS-ESI: 695 (M+1).

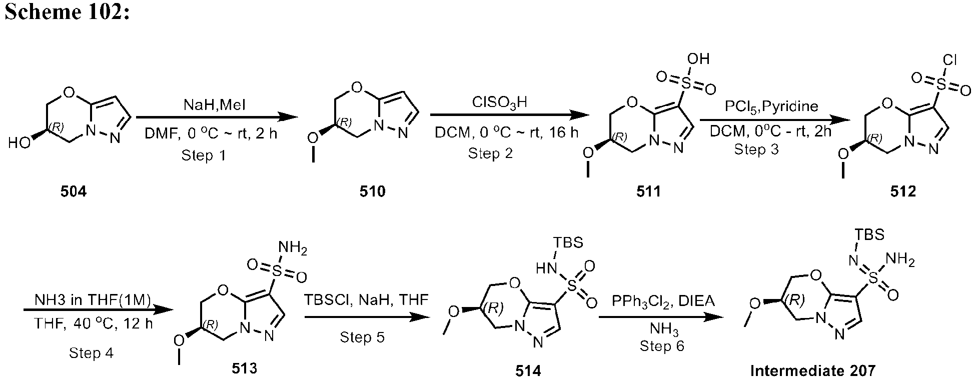

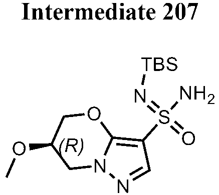

### (6R)-N'-(tert-butyldimethylsilyl)-6-methoxy-6,7-dihydro-5H-pyrazolo[5,1-b][1,3]oxazine-3-sulfonimidamide

### Step 1: (R)-6-methoxy-6,7-dihydro-5H-pyrazolo[5,1-b][1,3]oxazine

To a stirred solution of (R)-6,7-dihydro-5H-pyrazolo[5,1-b][1,3]oxazin-6-ol (40 g, 285 mmol) in DMF (400 mL) in a 1 L 4-neck round-bottom flask was added NaH (60% oil dispersion, 13.7 g, 342 mmol) in portions at 0 °C. The resulting mixture was stirred for 1 h at RT. To the above mixture was added MeI (48.7 g, 343 mmol) dropwise at RT. The resulting mixture was stirred for additional 2 h at RT. The reaction was quenched with AcOH (3.66 g, 57.1 mmol) at 0 °C and concentrated under reduced pressure. The residue was eluted from silica gel with PE/EtOAc (1:2) to afford the title compound (34.6 g, 79%) as a light yellow solid. MS-ESI: 155 (M+1).

Steps 2-4 used similar procedures for converting compound **505** to compound **508** shown in Scheme 101 to afford compound **513** from compound **510.** MS-ESI: 232 (M-1).

Steps 5-6 used similar procedures for converting compound **442** to intermediate **195** shown in Scheme 91 to afford intermediate **207** from compound **513.** MS-ESI: 347 (M+1).

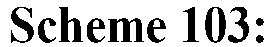

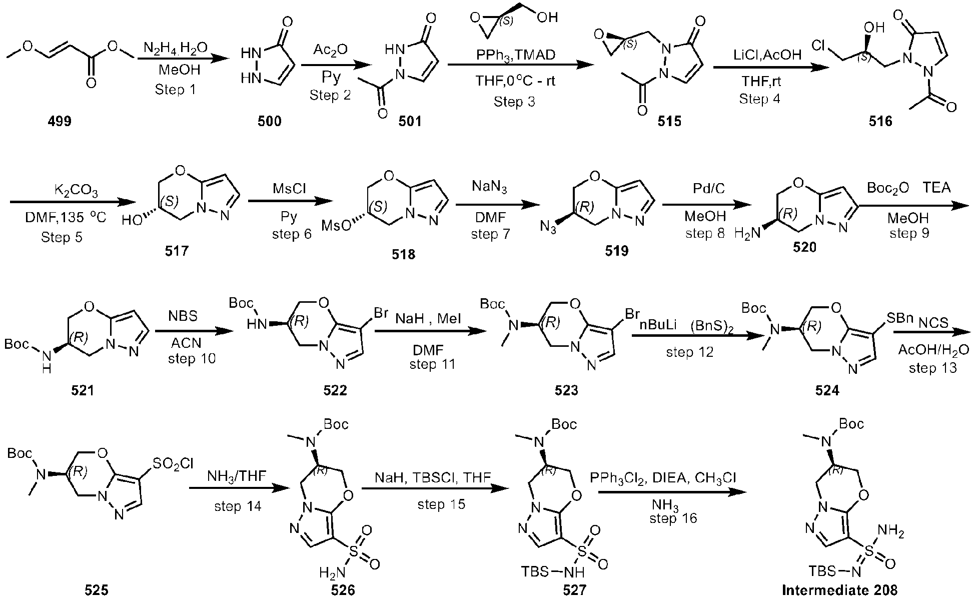

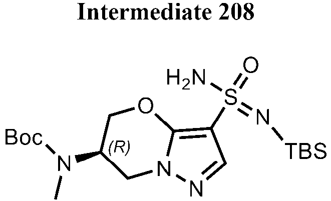

### Tert-butyl ((6R)-3-(N'-(tert-butyldimethylsilyl)sulfamidimidoyl)-6,7-dihydro-5H-pyrazolo[5,1-b][1,3]oxazin-6-yl)(methyl)carbamate

Steps 1-5 used similar procedures for converting compound **499** to compound **504** shown in Scheme 101 to afford compound **517** from compound **499.** MS-ESI: 141 (M+1).

### Step 6: (S)-6,7-dihydro-5H-pyrazolo[5,1-b][1,3]oxazin-6-yl methanesulfonate

To a stirred solution of (*S*)-6,7-dihydro-5H-pyrazolo[5,1-b][1,3]oxazin-6-ol (40 g, 285 mmol) in pyridine (280 mL) in a 500 mL 3-neck flask under nitrogen was added MsCl (39 g, 343 mmol) dropwise at RT. The resulting mixture was stirred for 30 min at RT. The resulting mixture was concentrated under reduced pressure. The crude was diluted with 500 mL of water. The resulting mixture was extracted with EtOAc (3 x 300 mL). The combined organic layers were washed with water (3x200 mL) and brine 1x100 mL, dried over anhydrous Na₂SO₄. After filtration, the filtrate was concentrated under reduced pressure. This resulted in the title compound (57 g, 92%) as an off-white solid. MS-ESI: 219 (M+1).

### Step 7: (R)-6-azido-6,7-dihydro-5H-pyrazolo[5,1-b][1,3]oxazine

To a stirred solution of (*S*)-6,7-dihydro-5H-pyrazolo[5,1-b][1,3]oxazin-6-yl methanesulfonate (46 g, 211 mmol) in DMF (313 mL) in a 1 L 3-neck flask under nitrogen was added NaN₃ (21 g, 316 mmol) at RT. The resulting mixture was stirred for 6 h at 80 °C. LCMS showed reaction was complete. This was used directly in the next step. MS-ESI: 166 (M+1).

### Step 8: (R)-6,7-dihydro-5H-pyrazolo[5,1-b][1,3]oxazin-6-amine

To a stirred solution of (*R*)-6-azido-6,7-dihydro-5H-pyrazolo[5,1-b][1,3]oxazine in DMF from the last step was added MeOH (313 mL) followed by Pd/C (10%wt., 10 g) under nitrogen. The mixture was hydrogenated at RT for 5 h under hydrogen atmosphere using a hydrogen balloon. LCMS showed reaction was complete. The mixture was filtered through a pad of Celite and concentrated under reduced pressure to remove low boiling solvent. This resulted in the title compound in DMF solution which was used directly in the next step. MS-ESI: 140 (M+1).

### Step 9: Tert-butyl (R)-(6,7-dihydro-5H-pyrazolo[5,1-b][1,3]oxazin-6-yl)carbamate

To a stirred solution of (*R*)-6,7-dihydro-5H-pyrazolo[5,1-b][1,3]oxazin-6-amine in DMF from the last step was added MeOH (313 mL) followed by TEA (50 g, 496 mmol) and di-tert-butyl dicarbonate (79 g, 364 mmol) at RT under nitrogen. The resulting mixture was stirred for 16 h at RT. The resulting mixture was quenched with 500 mL of water. The resulting mixture was extracted with EtOAc (3 x 300 mL). The combined organic layers were washed with H₂O (2x600 mL) and brine (1x600 mL), dried over anhydrous Na₂SO₄. After filtration, the filtrate was concentrated under reduced pressure. This resulted in the title compound (45.9 g, 91%, for 3 steps) as an off-white solid. MS-ESI: 240 (M+1).

### Step 10: Tert-butyl (R)-(3-bromo-6,7-dihydro-5H-pyrazolo[5,1-b][1,3]oxazin-6-yl)carbamate

To a stirred solution of *tert*-butyl (R)-(6,7-dihydro-5H-pyrazolo[5,1-b][1,3] oxazin-6-yl)carbamate (140 g, 585 mmol) in MeCN (2.1 L) in a 3 L 3-neck flask under nitrogen was added NBS (115 g, 644 mmol) at 0 °C. The resulting mixture was stirred for 2 h at RT. The resulting mixture was diluted with 2000 mL of water. The resulting mixture was extracted with EtOAc (3 x 800 mL). The combined organic layers were washed with brine (1x800 mL), dried over anhydrous Na₂SO₄. After filtration, the filtrate was concentrated under reduced pressure. This resulted in the title compound (167 g, 90%) as an off-white solid. MS-ESI: 318/320 (M+1).

### Step 11: Tert-butyl (R)-(3-bromo-6,7-dihydro-5H-pyrazolo[5,1-b][1,3]oxazin-6-yl)(methyl)carbamate

To a stirred solution of *tert*-butyl(*R*)-(3-bromo-6,7-dihydro-5H-pyrazolo -[5,1-b][1,3]oxazin-6-yl)carbamate (170 g, 534 mmol) in DMF (1.19 L) in a 3 L 3-neck flask under nitrogen was added sodium hydride (60% oil dispersion, 26 g, 650 mmol) in portions at 0 °C. The mixture was stirred for 1 h at 0 °C. Then MeI (379 g, 2.67 mol) was added and the mixture was allowed to warm to RT and stirred for 2 h. The reaction mixture was quenched by water and extracted with EtOAc (3*800 mL), the organic layers were washed with H₂O (3x500 mL) and brine (1x800 mL), dried over anhydrous Na₂SO₄. After filtration, the filtrate was concentrated under reduced pressure. The residue was purified by silica gel column chromatography, eluted with PE/EtOAc (8:1) to afford the title compound (153 g, 86%) as an off-white solid. MS-ESI: 332/334 (M+1).

### Step 12: Tert-butyl(R)-(3-(benzylthio)-6,7-dihydro-5H-pyrazolo[5,1-b][1,3]oxazin-6-yl)(methyl) carbamate

To a stirred solution of *tert*-butyl(*R*)-(3-bromo-6,7-dihydro-5H-pyrazolo-[5,1-b][1,3] oxazin-6-yl)(methyl) carbamate (130 g, 391 mmol) in THF (1.3 L) in a 3 L 3-neck flask under nitrogen was added n-BuLi (188 mL, 470 mmol, 2.5 mol/L) dropwise at -78 °C. The resulting mixture was stirred for 1 h at -78 °C. To the above mixture was added bis(phenylmethyl) disulfide (145 g, 587 mmol) in THF (300 mL) dropwise at -78 °C. The resulting mixture was stirred for additional 2 h at RT. The reaction was quenched by the addition of sat. NH₄Cl (aq.) (500 mL) at RT. The resulting mixture was extracted with EtOAc (3 x 500 mL). The combined organic layers were washed with brine (1x800 mL), dried over anhydrous Na₂SO₄. After filtration, the filtrate was concentrated under reduced pressure. The residue was purified by silica gel column chromatography, eluted with PE/EtOAc (6:1) to afford the title compound (106 g, 72%) as an off-white solid. MS-ESI: 376 (M+1).

### Step 13: Tert-butyl (R)-(3-(chlorosulfonyl)-6,7-dihydro-5H-pyrazolo[5,1-b][1,3]oxazin-6-yl)(methyl) carbamate

To a stirred solution of tert-butyl (R)-(3-(benzylthio)-6,7-dihydro-5H-pyrazolo[5,1-b][1,3]oxazin-6-yl)- (methyl) carbamate (110 g, 293 mmol) in AcOH (3.67 L)/H₂O (1.83 L) in a 10 L 3-neck flask under nitrogen was added NCS (155 g, 1.17 mol) in portions at 0 °C. The resulting mixture was stirred for 1 h at RT. The reaction was quenched with water/ice at RT. The resulting mixture was extracted with MTBE (3 x 1 L). The combined organic layers were washed with water (2 x 1.0 L), NaHCO₃ (2 x 1.0 L) and brine (1x1 L), dried over anhydrous Na₂SO₄. After filtration, the filtrate was concentrated under reduced pressure. This resulted in the title compound (80 g, crude ) as a yellow solid. MS-ESI: 374 (M+Na).

### Step 14: Tert-butyl (R)-methyl(3-sulfamoyl-6,7-dihydro-5H-pyrazolo[5,1-b][1,3]oxazin-6-yl)carbamate

To a stirred solution of tert-butyl (R)-(3-(chlorosulfonyl)-6,7-dihydro-5H-pyrazolo[5,1-b][1,3]oxazin-6-yl)(methyl)carbamate (80 g, 227 mmol) in NH₃ in THF (1 M, 800 mL, 800 mmol) in a 2 L 3-neck flask under nitrogen was filled with (balloon). The resulting mixture was stirred overnight at 60 °C. The reaction mixture was quenched by adding water and extracted with MTBE (3*800 mL), the organic layers were washed with H₂O (3x500 mL) and brine (1x800 mL), dried over anhydrous Na₂SO₄. After filtration, the filtrate was concentrated under reduced pressure. The precipitated solid was slurry with MTBE (1x100 mL). This resulted in the title compound (36 g, for 2 steps) as an off-white solid. MS-ESI: 333 (M+1).

Steps 15-16 used similar procedures for converting compound **442** to intermediate **195** shown in Scheme 91 to afford intermediate **208** from compound 526. MS-ESI: 446 (M+1).

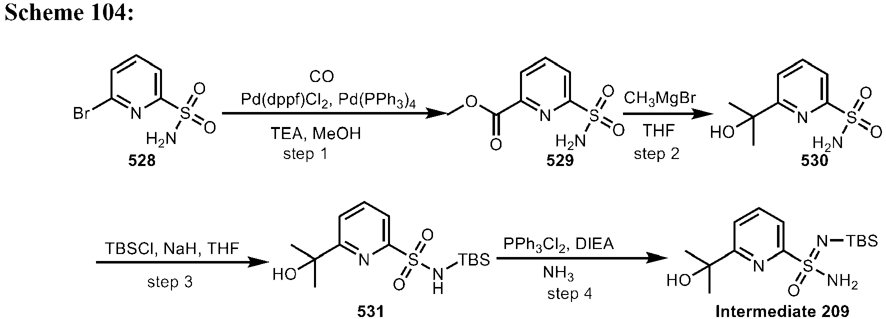

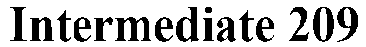

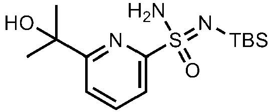

### N'-(tert-butyldimethylsilyl)-6-(2-hydroxypropan-2-yl)pyridine-2-sulfonimidamide

### Step 1: Methyl 6-sulfamoylpicolinate

To a stirred solution of 6-bromopyridine-2-sulfonamide (5.0 g, 21 mmol) in MeOH (200 mL) in a 100 mL of pressure tank reactor was added Pd(dppf)Cl₂ (1.5 g, 2.1 mmol), Pd(PPh₃)₄ (2.4 g, 2.1 mmol) and TEA (10.7 g, 105 mmol) under nitrogen. The pressure tank reactor was evacuated and refilled three times with carbon monoxide. The reaction mixture was stirred overnight at 80 °C under carbon monoxide atmosphere (10 atm). The resulting mixture was concentrated under reduced pressure. The residue was eluted from silica gel column with PE/EtOAc (1:2). This resulted 1.8 g (39%) of the title compound as a light yellow solid. MS-ESI: 215 (M-1).

### Step 2: 6-(2-Hydroxypropan-2-yl)pyridine-2-sulfonamide

To a stirred solution of methyl 6-sulfamoylpyridine-2-carboxylate (1.8 g, 8.3 mmol) in THF (180 mL) under nitrogen was added MeMgBr (3 M in THF, 28 mL, 83 mmol) dropwise at 0 °C. The solution was stirred overnight at RT under nitrogen. The reaction was quenched by adding 50 mL of water, then acidified to pH 6 with conc. HCl. The resulting mixture was extracted with EtOAc (3x200 mL). The combined organic layers were washed with brine (3x100 mL), dried over anhydrous Na₂SO₄. After filtration, the filtrate was concentrated under reduced pressure. The residue was eluted from silica gel with PE/EtOAc (5:1). This resulted in the title compound 0.90 g (50%) as an off-white solid. MS-ESI: 215 (M-1).

Steps 3-4 used similar procedures for converting compound **442** to intermediate **195** shown in Scheme 91 to afford intermediate **209** from compound **530.** MS-ESI: 330 (M+1).

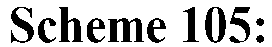

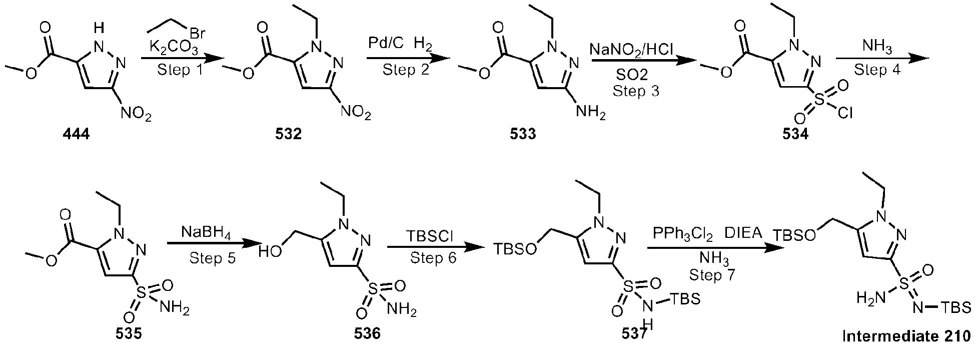

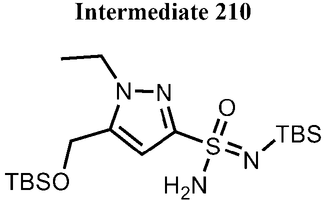

### N'-(tert-butyldimethylsilyl)-5-(((tert-butyldimethylsilyl)oxy)methyl)-1-ethyl-1H-pyrazole-3-sulfonimidamide

### Step 1: Methyl 1-ethyl-3-nitro-1H-pyrazole-5-carboxylate

To a stirred solution of methyl 3-nitro-1H-pyrazole-5-carboxylate (300 mg, 1.75 mmol) in DMF (10 mL) in a 50-mL round-bottom flask were added K₂CO₃ (485 mg, 3.5 mmol) and bromoethane (382 mg, 3.5 mmol) in portions at RT. The resulting solution was stirred for 4 h at 30 °C. The resulting solution was diluted with 10 mL of H₂O. The resulting solution was extracted with 4x20 mL of EtOAc and the organic layers were combined and dried over anhydrous Na₂SO₄ and concentrated under vacuum. The residue was eluted from silica gel with EtOAc/PE (1:2). This resulted in 305 mg (87%) of the title compound as a light yellow solid. MS-ESI: 200 (M+1).

Steps 2-7 used similar procedures for converting compound **445** to intermediate **196** shown in Scheme 92 to afford intermediate **210** from compound **532.** MS-ESI: 433 (M+1).

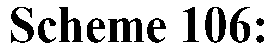

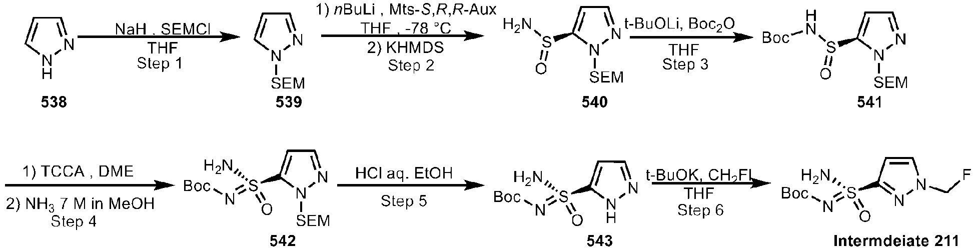

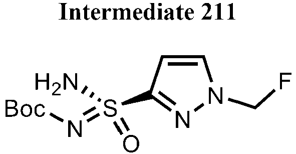

### Tert-butyl (S)-(amino(1-(fluoromethyl)-1H-pyrazol-3-yl)(oxo)-λ⁶-sulfaneylidene)carbamate

### Step 1: 1-((2-(Trimethylsilyl)ethoxy)methyl)-1H-pyrazole

To a stirred solution of 1H-pyrazole (10 g, 146.9 mmol) in dry THF (400 mL) under nitrogen was added NaH (60% wt., dispersion in mineral oil, 6.17 g, 154 mmol) in portions at 0 °C. The reaction mixture was stirred for 15 min at RT. To the above mixture was added SEMCl (26.5 mL, 150 mmol) dropwise at 0 °C and the reaction mixture was stirred for 2 h at RT. The reaction was quenched with water (200 mL) and extracted with EtOAc (3x400 mL) and the organic layers were combined, washed with water (400 mL) and brine (400 mL), dried over MgSO₄, filtered and concentrated under vacuum. The residue was eluted from silica gel with 5~50% EtOAc in hexane. This resulted in 23.5 g (81%) of the title compound as clear oil. MS-ESI: 199 (M+1).

### Step 2: (S)-1-((2-(trimethylsilyl)ethoxy)methyl)-1H-pyrazole-5-sulfinamide

To a solution of 1-((2-(trimethylsilyl)ethoxy)methyl)-1H-pyrazole (7.5 g, 37.8 mmol) in dry THF (200 mL) under nitrogen was added n-BuLi in hexane (2.5 M, 16 mL, 40 mmol) dropwise at -78 °C and the reaction mixture was stirred for 2 h at -78 °C. To above solution was cannulated a solution of Mts-*S,R,R*-Aux (15 g, 39.7 mmol) in dry THF (200 mL) maintained at -78 °C. The mixture was stirred for 3 h at -78 °C. To above mixture was added KHMDS in THF (1 M, 75.6 mL, 75.6 mmol) dropwise at -78 °C. The reaction was warmed to 0 °C. The reaction mixture was quenched with formic acid in THF (1 M, 113 mL, 113 mmol) dropwise at 0 °C. The mixture was concentrated under vacuum and the residue was dissolved in DCM and washed with brine. The organic layer was dried over Na₂SO₄, filtered and concentrated under vacuum. The residue was eluted from silica gel with EtOAc/hexane (10%~100%). This resulted in 5.6 g (57%, 99%ee Chiralpak AD, hexane/EtOH 70:30 + 0.1% DEA) of the title compound as a beige waxy solid. MS-ESI: 262 (M+1).

### Step 3: Tert-butyl (S)-((1-((2-(trimethylsilyl)ethoxy)methyl)-1H-pyrazol-5-yl)sulfinyl)carbamate

To a solution of (S)-1-((2-(trimethylsilyl)ethoxy)methyl)-1H-pyrazole-5-sulfinamide (5.5 g, 21 mmol) in dry THF (200 mL) under nitrogen was added t-BuOLi in THF (1 M, 43.1 mL, 43.1 mmol) dropwise at 0 °C. The reaction mixture was stirred for 20 min at 0 °C. To above mixture was added Boc₂O (4.59 g, 21 mmol) in portions and the reaction was stirred for 2 h at 0 °C. The reaction was quenched with formic acid in THF (1 M, 44 mL, 44 mmol) at 0 °C. The mixture was concentrated under vacuum. The residue was eluted from silica gel with 5~50% EtOAc in hexane. This resulted in 6.0 g (79%, 99%ee from Chiralpak IG, hexane/EtOH 70:30 + 0.1% DEA) of the title compound as a colorless oil. MS-ESI: 362 (M+1)

### Step 4: Tert-butyl (S)-(amino(oxo)(1-((2-(trimethylsilyl)ethoxy)methyl)-1H-pyrazol-5-yl)-λ⁶-sulfaneylidene)carbamate

To a solution of tert-butyl (S)-((1-((2-(trimethylsilyl)ethoxy)methyl)-1H-pyrazol-5-yl)sulfinyl)carbamate (5.75 g, 15.9 mmol) in dry DME (100 mL) was added TCCA (1.85 g, 7.95 mmol) in portions at 0 °C. The mixture was stirred for 1 h at 0 °C. This reaction solution was cannulated into a flask containing NH₃ (7 M in MeOH, 22.7 mL, 160 mmol) at 0 °C and the mixture was stirred for 4 h at the same temperature. The reaction mixture was diluted with DCM, filtered through a silica plug washed with EtOAc and concentrated. The residue was eluted from silica gel with 10~70% EtOAc in hexane. This resulted in 3.0 g (50%) of the title compound as a white solid. MS-ESI: 377 (M+1).

### Step 5: Tert-butyl (S)-(amino(oxo)(1H-pyrazol-5-yl)-λ⁶-sulfaneylidene)carbamate

To a solution of tert-butyl (S)-(amino(oxo)(1-((2-(trimethylsilyl)ethoxy)methyl)-1H-pyrazol-5-yl)-λ⁶- sulfaneylidene)carbamate (2.8 g, 7.44 mmol) in EtOH (50 mL) was added aq. HCl (3 M, 24.8 mL, 74.4 mmol) dropwise at RT. The mixture was stirred for 2 h at RT. The reaction was cooled to 0 °C and quenched with sat. NaHCO₃. The mixture was extracted with EtOAc (3x100 mL) and the organic layers were combined. The organic layer was washed with brine, dried over Na₂SO₄, filtered and concentrated under vacuum. The residue was eluted from silica gel with 0-20% MeOH in DCM. This resulted in 755 mg (41%, 97.5%ee from Chiralpak IG, hexane/EtOH 85:15 + 0.1% DEA) of the title compound as a white foam. MS-ESI: 247 (M+1).

### Step 6: Tert-butyl (S)-(amino(1-(fluoromethyl)-1H-pyrazol-3-yl)(oxo)-λ⁶-sulfaneylidene)carbamate

To a solution of tert-butyl (S)-(amino(oxo)(1H-pyrazol-5-yl)-λ⁶-sulfaneylidene)carbamate (45 mg, 0.18 mmol) in dry THF (2 mL) under nitrogen was added t-BuOK in THF (1 M, 384 uL, 0.38 mmol) at RT. The mixture was stirred for 30 min at RT. To above mixture was added CH₂FI (14 uL, 0.20 mmol). The mixture was stirred over-weekend. The reaction was quenched with formic acid in THF (1 M, 385 uL) and the mixture was concentrated under vacuum. The residue was eluted from silica gel with 0-25% MeOH in DCM. This resulted in 27 mg (54%, 98%ee from Chiralpak IG, hexane/EtOH 80:20 + 0.1% DEA) of the title compound as a white solid. MS-ESI: 279 (M+1).

**Schemes for amino pyridines Intermediates:** Schemes **107-115** illustrate the preparation of amino pyridines intermediates.

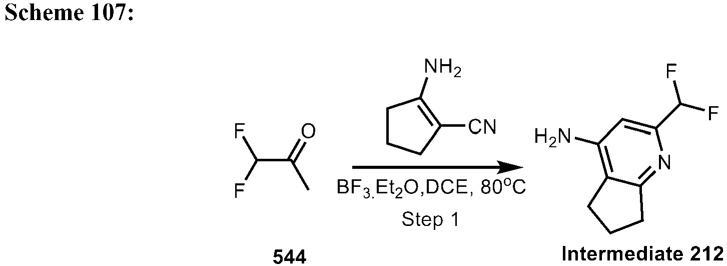

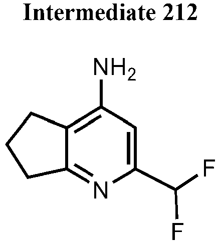

### 2-(Difluoromethyl)-6,7-dihydro-5H-cyclopenta[b]pyridin-4-amine

### Step 1: 2-(Difluoromethyl)-6,7-dihydro-5H-cyclopenta[b]pyridin-4-amine

To a stirred solution of 2-aminocyclopent-1-ene-1-carbonitrile (20 g, 185 mmol) in DCE (400 mL) in a 1 L round-bottom flask under nitrogen was added 1,1-difluoropropan-2-one (17.4 g, 185 mmol) and BF₃·Et₂O (47% wt., 25 g, 370 mmol) dropwise at 0 °C. The resulting solution was stirred for 4 h at 80 °C in an oil bath. The resulting mixture was concentrated under vacuum. The resulting solution was diluted with 500 mL of water. The pH value of the solution was adjusted to 8 with K₂CO₃ (sat.). The resulting solution was extracted with 3x500 mL of EtOAc. The organic layers were dried over anhydrous Na₂SO₄. The resulting mixture was concentrated under vacuum. The residue was eluted from silica gel with EtOAc/PE (1:1). This resulted in 1.71 g (5.0%) of the title compound as a yellow solid. MS-ESI: 185 (M+1).

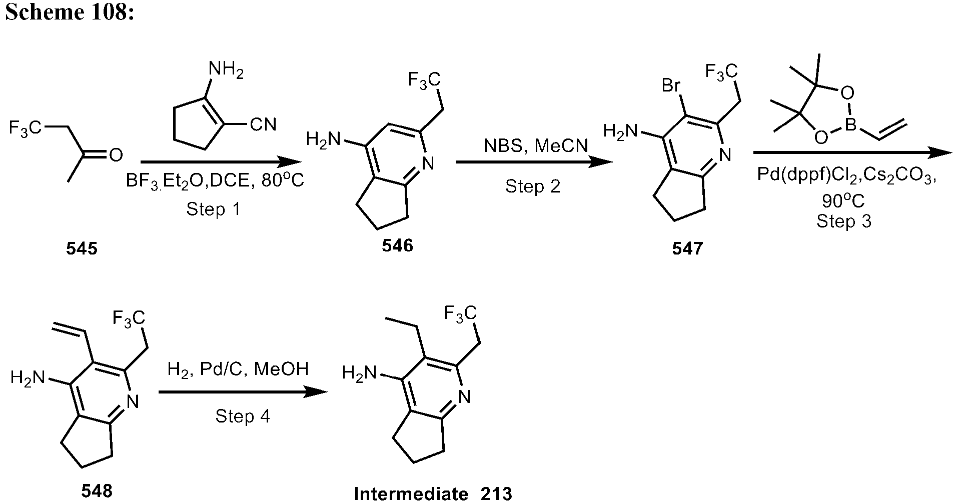

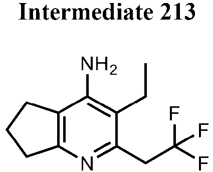

### 3-Ethyl-2-(2,2,2-trifluoroethyl)-6,7-dihydro-5H-cyclopenta[b]pyridin-4-amine

Step 1 used similar procedures for converting compound **544** to intermediate **212** shown in Scheme 107 to afford compound **546** from compound **545.** MS-ESI: 217 (M+1).

### Step 2: 3-Bromo-2-(2,2,2-trifluoroethyl)-6,7-dihydro-5H-cyclopenta[b]pyridin-4-amine

To a stirred solution of 2-(2,2,2-trifluoroethyl)-6,7-dihydro-5H-cyclopenta[b]pyridin-4-amine (950 mg, 4.4 mmol) in MeCN (20 mL) was added NBS (1.56 g, 8.8 mmol). The resulting solution was stirred for 1 h at RT under nitrogen. The reaction was then quenched by the addition of 20 mL of sat. Na₂S₂O₃ (aq). The resulting solution was extracted with 3x50 mL of EtOAc. The combined organic phase was dried over anhydrous Na₂SO₄ and then concentrated under vacuum. The residue was eluted from silica gel with EtOAc/PE (1/5). This resulted in 1.8 g (83%) of the title compound as a yellow solid. MS-ESI: 295, 297 (M+1).

### Step 3: 2-(2,2,2-Trifluoroethyl)-3-vinyl-6,7-dihydro-5H-cyclopenta[b]pyridin-4-amine

To a stirred solution of 3-bromo-2-(2,2,2-trifluoroethyl)-6,7-dihydro-5H-cyclopenta[b]pyridin-4-amine (1.0 mg, 3.4 mmol) in dioxane (100 mL) and water (10 mL) were added Cs₂CO₃ (2.2 g, 6.8 mmol), 4,4,5,5-tetramethyl-2-vinyl-1,3,2-dioxaborolane (1.0 g, 6.8 mmol) and Pd(dppf)Cl₂ (250 mg, 0.34 mmol) under nitrogen. The resulting solution was stirred overnight at 90 °C under nitrogen. The reaction mixture was then diluted with 100 mL of water. The solids were filtered out. The filtrate was extracted with 3x200 mL of EtOAc, the combined organic layers were dried over Na₂SO₄ and concentrated under vacuum. The residue was eluted from silica gel with EtOAc/PE (1/1). This resulted in 1.0 g (73%) of the title compound as a yellow solid. MS-ESI: 243 (M+1).

### Step 4: 3-Ethyl-2-(2,2,2-trifluoroethyl)-6,7-dihydro-5H-cyclopenta[b]pyridin-4-amine

To a stirred solution of 2-(2,2,2-trifluoroethyl)-3-vinyl-6,7-dihydro-5H-cyclopenta[b]pyridin-4-amine (500 mg, 2.06 mmol) in MeOH (50 mL) was added Pd/C (10%wt., 50 mg) under nitrogen in portions. The flask was evacuated and refilled three times with hydrogen. The resulting solution was stirred overnight at RT under hydrogen with a balloon. The solids were filtered out. The resulting solution was concentrated under vacuum. The residue was eluted from silica gel with EtOAc/PE (1/1). This resulted in 400 mg (80%) of the title compound as a yellow solid. MS-ESI: 245 (M+1).

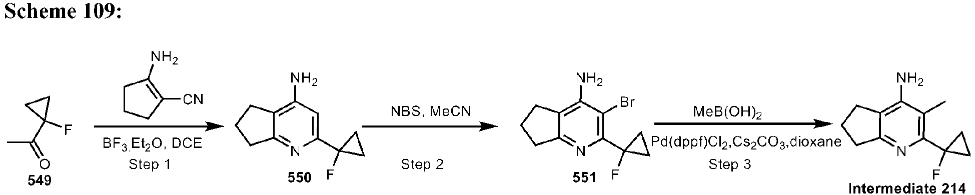

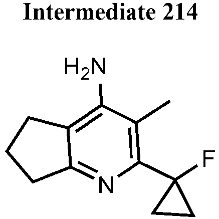

### 2-(1-Fluorocyclopropyl)-3-methyl-6,7-dihydro-5H-cyclopenta[b]pyridin-4-amine

Step 1-3 used similar procedures for converting compound **545** to compound **548** shown in Scheme 108 to afford intermediate **214** from compound **549.** MS-ESI: 207 (M+1).

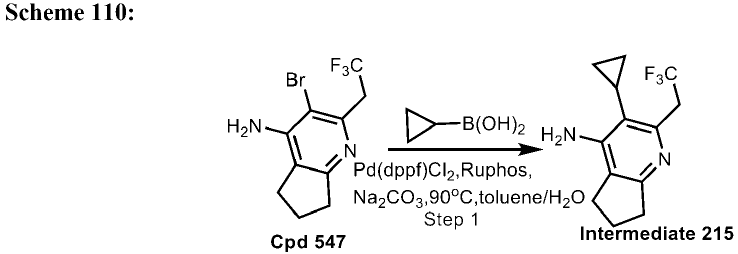

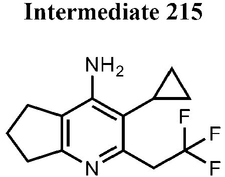

### 3-Cyclopropyl-2-(2,2,2-trifluoroethyl)-6,7-dihydro-5H-cyclopenta[b]pyridin-4-amine

### Step 1: 3-Cyclopropyl-2-(2,2,2-trifluoroethyl)-6,7-dihydro-5H-cyclopenta[b]pyridin-4-amine

To a stirred solution of 3-bromo-2-(2,2,2-trifluoroethyl)-6,7-dihydro-5H-cyclopenta[b]pyridin-4-amine (1.0 g, 3.4 mmol) in toluene (20 mL) and water (2.0 mL) were added cyclopropylboronic acid (1.2 g, 13.6 mmol) and Na₂CO₃ (1.1 g, 10.2 mmol) in portions at RT. To the above solution was added Pd(dppf)Cl₂ (20 mg, 0.03 mmol) and Ruphos (20 mg, 0.03 mmol) under nitrogen. The mixture was stirred overnight at 90 °C under nitrogen; LCMS showed the starting material was consumed. The resulting mixture was concentrated under reduced pressure. The residue was eluted from silica gel with PE/EA (6:1). This resulted in 600 mg (69%) of the title compound as a yellow solid. MS-ESI: 257 (M+1).

Table 47. The Intermediates in the following Table were prepared using the similar procedures for converting compound 547 to Intermediate 215 shown in Scheme 110 from appropriated starting materials.

| Intermediate # | Structure | IUPAC Name | Exact Mass [M-H]⁻ |
|---|---|---|---|
| **Intermediate 216** | 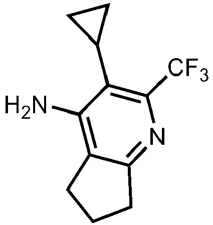 | 3-Cyclopropyl-2-(trifluoromethyl)-6,7-dihydro-5H-cyclopenta[b]pyridin-4-amine | 243 |
| **Intermediate 217 (from 558)** | 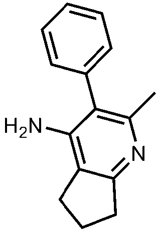 | 2-Methyl-3-phenyl-6,7-dihydro-5H-cyclopenta[b]pyridin-4-amine | 225 |

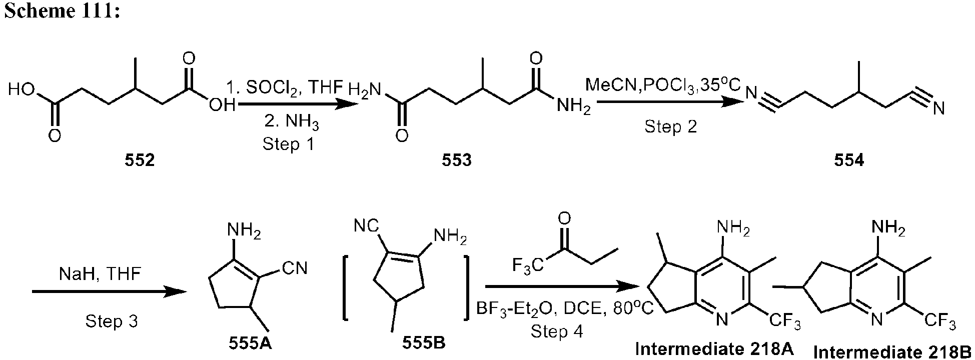

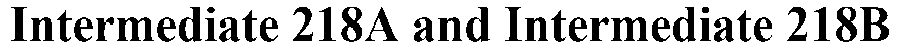

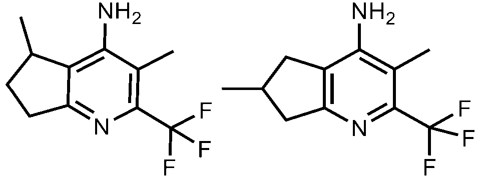

### 3,5-Dimethyl-2-(trifluoromethyl)-6,7-dihydro-5H-cyclopenta[b]pyridin-4-amine and 3,6-dimethyl-2-(trifluoromethyl)-6,7-dihydro-5H-cyclopenta[b]pyridin-4-amine

### Step 1: 3-Methylhexanediamide

To a stirred solution of 3-methyladipic acid (10 g, 62.4 mmol) in DCM (300 mL) was added SOCl₂ (100 mL, 841 mmol) dropwise with stirring at 0 °C under nitrogen. The resulting solution was stirred for 4 h at RT. The resulting mixture was concentrated under vacuum. This resulted in 11 g (crude) of 3-methylhexanedioyl dichloride as a brown oil and it was dissolved in THF (50 mL). This solution was assigned as "A". To stirred solution of NH₃ in THF (0.5 M, 300 mL) was added solution A (50 mL prepared above) dropwise at 0 °C. The resulting solution was stirred for 3 h at RT. The reaction was then quenched by the addition of 20 mL of MeOH. The resulting mixture was concentrated under vacuum. The residue was eluted from silica gel with DCM/MeOH (10:1). This resulted in 9.72 g (98%) of the title compound as a white solid. MS-ESI: 159 (M+1).

### Step 2: 3-Methylhexanedinitrile

To a stirred solution of 3-methylhexanediamide (10 g, 63.2 mmol) in MeCN (200 mL) was added POCl₃ (48.5 g, 316 mmol) dropwise at 0 °C. The resulting solution was stirred overnight at RT. The reaction was then quenched by the addition of 20 mL of water/ice. The resulting solution was extracted with 3x200 mL of DCM. The combined organic layers were dried over anhydrous sodium sulfate and concentrated under vacuum. This resulted in 3.58 g (46%) of the title compound as a yellow oil. MS-ESI: 121 (M-1).

### Step 3: Mixture of 2-amino-5-methylcyclopent-1-ene-1-carbonitrile and 2-amino-4-methylcyclopent-1-ene-1-carbonitrile

To a stirred solution of 3-methylhexanedinitrile (3 g, 24.6 mmol) in THF (100 mL) was added NaH (60% wt. dispersion in mineral oil, 2.95 g, 74 mmol) in portions at 0 °C under nitrogen. The resulting solution was stirred overnight at 80 °C. The reaction was then quenched by the addition of 50 mL of water. The resulting solution was extracted with 3x200 mL of EtOAc, the combined organic layers were dried over anhydrous sodium sulfate and concentrated under vacuum. This resulted in 1.65 g (crude) mixture (1:1) of the title compound as a brown yellow solid. MS-ESI: 121 (M-1) for both.

Step 4 used similar procedures for converting compound **544** to intermediate **212** shown in Scheme 107 to afford intermediate **218A and 218B** from compound **555A and 555B.** MS-ESI: 231 (M+1) for both.

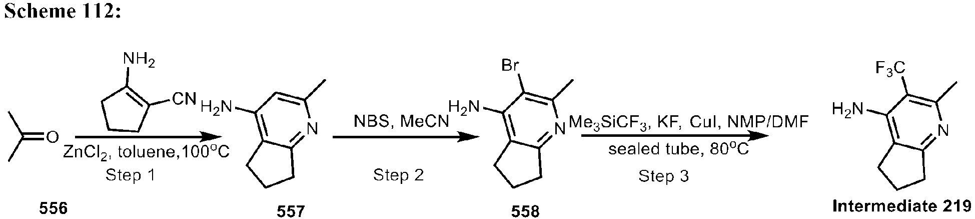

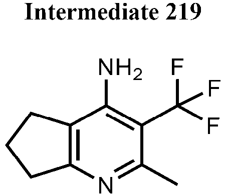

### 2-Methyl-3-(trifluoromethyl)-6,7-dihydro-5H-cyclopenta[b]pyridin-4-amine

### Step 1: 2-Methyl-6,7-dihydro-5H-cyclopenta[b]pyridin-4-amine

To a stirred solution of acetone (34.8 g, 6.0 mol) in toluene (50 mL) was added 2-aminocyclopent-1-ene-1-carbonitrile (10.8 g, 100 mmol) and ZnCl₂ (1.5 g, 110 mmol) at RT. The resulting solution was stirred for 16 h at 100 °C under nitrogen. The resulting mixture was concentrated under vacuum. The residue was eluted from silica gel with DCM/MeOH (10:1). This resulted in 2.2 g (14.9%) of the title compound as a yellow solid. MS-ESI: 149 (M+1).

### Step 2: 3-Bromo-2-methyl-6,7-dihydro-5H-cyclopenta[b]pyridin-4-amine

To a stirred solution of 2-methyl-6,7-dihydro-5H-cyclopenta[b]pyridin-4-amine (2.2 g, 14.8 mmol) in MeCN (30 mL) was added NBS (3.17 g, 18 mmol). The resulting solution was stirred for 2 h at RT. The resulting mixture was quenched with sat. Na₂SO₃ (aq., 5.0 mL), then diluted with water (50 mL), extracted with DCM (3x100 mL). The combined organic layers were dried over Na₂SO₄ and concentrated. The residue was eluted from silica gel with EtOAc/PE (1:1). This resulted in 1.3 g (39%) of the title compound as a dark brown solid. MS-ESI: 227/229 (M+1).

### Step 3: 2-Methyl-3-(trifluoromethyl)-6,7-dihydro-5H-cyclopenta[b]pyridin-4-amine

To a stirred solution of 3-bromo-2-methyl-6,7-dihydro-5H-cyclopenta[b]pyridin-4-amine (100 mg, 0.44 mmol) in DMF (2.0 mL)/NMP (2.0 mL) in a sealed tube under nitrogen was added trimethyl-(trifluoro- methyl)silane (170 mg, 1.3 mmol), CuI (168 mg, 0.88 mmol) and KF (51.2 mg, 0.88 mmol). The resulting solution was stirred overnight at 80 °C. The residue was eluted from a silica gel column with EtOAc/PE (1:1) and further purified by prep-TLC. This resulted in 3.0 mg (3.0%) of the title compound as an off-white solid. MS-ESI: 217 (M+1).

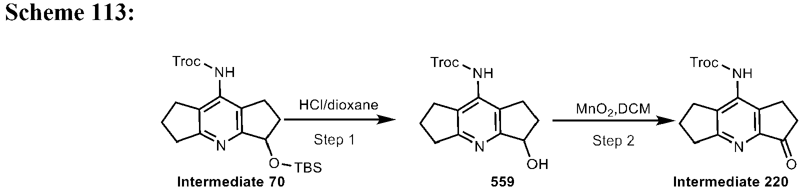

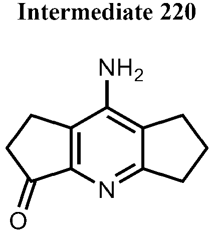

### 8-Amino-1,5,6,7-tetrahydrodicyclopenta[b,e]pyridin-3(2H)-one

### Step 1: 2,2,2-Trichloroethyl (3-hydroxy-1,2,3,5,6,7-hexahydrodicyclopenta[b,e]pyridin-8-yl)carbamate

To a stirred solution of HCl in 1,4-dioxane (4 M, 40 mL) was added 2,2,2-trichloroethyl (3-((tert-butyl- dimethylsilyl)oxy)-1,2,3,5,6,7-hexahydrodicyclopenta[b,e]pyridin-8-yl)carbamate (4.3 g, 9.0 mmol) in portions at RT. The resulting solution was stirred for 1 h at RT. The resulting mixture was concentrated. The residue was eluted from silica gel with EtOAc/PE (1:2). This resulted in 2.45 g (75%) of the title compound as an off-white solid. MS-ESI: 365/367/369 (M+1).

### Step 2: 2,2,2-Trichloroethyl (3-oxo-1,2,3,5,6,7-hexahydrodicyclopenta[b,e]pyridin-8-yl)carbamate

To a stirred solution of 2,2,2-trichloroethyl (3-hydroxy-1,2,3,5,6,7-hexahydrodicyclopenta[b,e]pyridin-8-yl) carbamate (643 mg, 1.76 mmol) in DCM (25 mL) was added MnO₂ (764 mg, 8.79 mmol) in portions at RT. The resulting solution was stirred for 2 h at RT. The solids were filtered out. The resulting mixture was concentrated. This resulted in 630 mg (crude) of the title compound as a light yellow solid. MS-ESI: 363/365/367 (M+1).

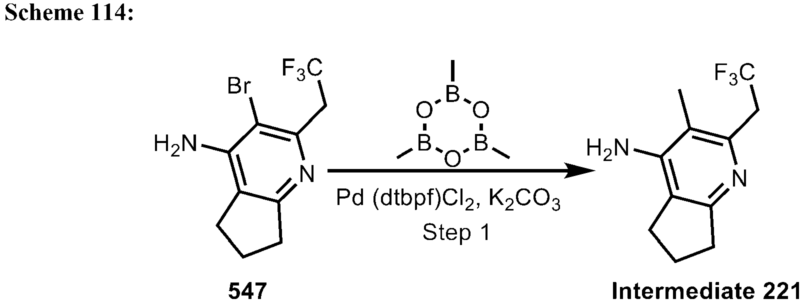

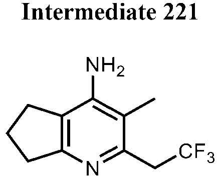

### 3-Methyl-2-(2,2,2-trifluoroethyl)-6,7-dihydro-5H-cyclopenta[b]pyridin-4-amine

### Step 1: 3-Methyl-2-(2,2,2-trifluoroethyl)-6,7-dihydro-5H-cyclopenta[b]pyridin-4-amine

To a stirred solution of 3-bromo-2-(2,2,2-trifluoroethyl)-6,7-dihydro-5H-cyclopenta[b]pyridin-4-amine (500 mg, 1.69 mmol) in dioxane (15 mL) in a 40-mL sealed tube under nitrogen were added 2,4,6-trimethyl-1,3,5,2,4,6-trioxatriborinane (1.06 g, 8.47 mmol), K₂CO₃ (706 mg, 5.08 mmol) and Pd(dtbpf)Cl₂ (221 mg, 0.034 mmol). The reaction mixture was stirred overnight at 85 °C. The reaction was quenched with H₂O (15 mL). The mixture was extracted with 3x30 mL of EtOAc and the organic layers were combined. The organic layer was dried with anhydrous Na₂SO₄ and concentrated under vacuum. The residue was eluted from silica gel with EtOAc/PE (2/1). This resulted in 200 mg (51%) of the title compound as a brown solid. MS-ESI: 231 (M+1).

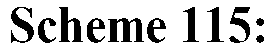

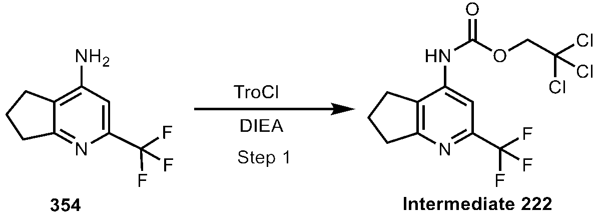

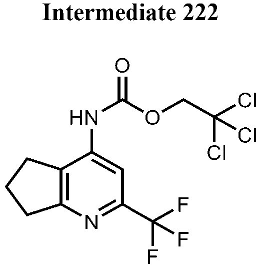

### 2,2,2-Trichloroethyl(2-(trifluoromethyl)-6,7-dihydro-5H-cyclopenta[b]pyridin-4-yl)carbamate

### Step 1: 2,2,2-Trichloroethyl (2-(trifluoromethyl)-6,7-dihydro-5H-cyclopenta[b]pyridin-4-yl)carbamate

To a stirred solution of 2-(trifluoromethyl)-6,7-dihydro-5H-cyclopenta[b]pyridin-4-amine (500 mg, 2.48 mmol) in THF (30 mL) in a 100-mL round-bottom flask under nitrogen was added DIEA (639 mg, 4.96 mmol) at RT, followed by the addition of 2,2,2-trichloroethyl chloroformate (1.05 g, 4.96 mmol) dropwise at RT. The resulting solution was stirred overnight at RT. The reaction solution was quenched with H₂O (10 mL). The mixture was extracted with 3x50 mL EtOAc. The organic layer was dried over anhydrous Na₂SO₄ and concentrated under vacuum. The residue was eluted from silica gel with EtOAc/PE(1:1). This resulted in 633 mg (68%) of the title compound as a brown yellow solid. MS-ESI: 376/378/380 (M+1).

**Table 48. The Intermediates in the following Table were prepared using the similar procedures for converting compound 354 to Intermediate 222 shown in Scheme 115 from appropriated starting materials.**

| Intermediate # | Structure | IUPAC Name | Exact Mass [M-H]⁻ |
|---|---|---|---|
| **Intermediate 223** | 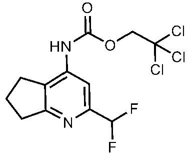 | 2,2,2-Trichloroethyl (2-(difluoromethyl)-6,7-dihydro-5H-cyclopenta[b]pyridin-4-yl)carbamate | 358/360/362 |
| **Intermediate 224** | 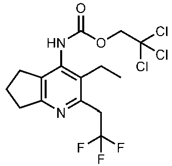 | 2,2,2-Trichloroethyl (3-ethyl-2-(2,2,2-trifluoroethyl)-6,7-dihydro-5H-cyclopenta[b]pyridin-4-yl)carbamate | 419/421/423 |
| **Intermediate 225** | 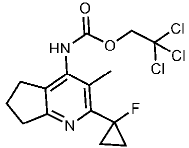 | 2,2,2-Trichloroethyl (2-(1-fluorocyclopropyl)-3-methyl-6,7-dihydro-SH-cyclopenta[b]pyridin-4-yl)carbamate | 381/383/385 |
| **Intermediate 226** | 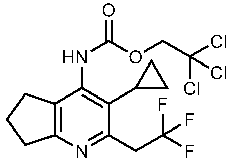 | 2,2,2-Trichloroethyl (3-cyclopropyl-2-(2,2,2-trifluoroethyl)-6,7-dihydro-5H-cyclopenta[b]pyridin-4-yl)carbamate | 431/433/435 |
| **Intermediate 227** | 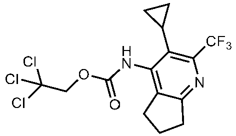 | 2,2,2-Trichloroethyl (3-cyclopropyl-2-(trifluoromethyl)-6,7-dihydro-5H-cyclopenta[b]pyridin-4-yl)carbamate | 417/419/421 |
| **Intermediate 228** | 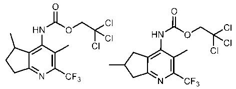 | 2,2,2-Trichloroethyl (3,5-dimethyl-2-(trifluoromethyl)-6,7-dihydro-5H-cyclopenta[b]pyridin-4-yl)carbamate and 2,2,2-trichloroethyl (3,6-dimethyl-2-(trifluoromethyl)-6,7-dihydro-5H-cyclopenta[b]pyridin-4-yl)carbamate | 405/407/409 |
| **Intermediate 229** | 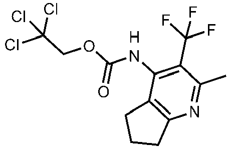 | 2,2,2-Trichloroethyl (2-methyl-3-(trifluoromethyl)-6,7-dihydro-5H-cyclopenta[b]pyridin-4-yl)carbamate | 391/393/395 |
| **Intermediate 230** | 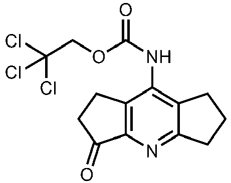 | 2,2,2-Trichloroethyl (3-oxo-1,2,3,5,6,7-hexahydrodicyclopenta[b,e]pyridin-8-yl)carbamate | 363/365/367 |
| **Intermediate 231** | 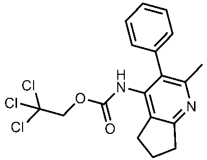 | 2,2,2-Trichloroethyl (2-methyl-3-phenyl-6,7-dihydro-5H-cyclopenta[b]pyridin-4-yl)carbamate | 399/401/403 |
| **Intermediate 232** | 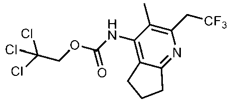 | 2,2,2-Trichloroethyl (3-methyl-2-(2,2,2-trifluoroethyl)-6,7-dihydro-5H-cyclopenta[b]pyridin-4-yl)carbamate | 405/407/409 |

**Schemes of Sulfonimidamide and amino pyridines Intermediates: Schemes 116-122** illustrate the preparation of sulfonimidamide and amino pyridines intermediates.

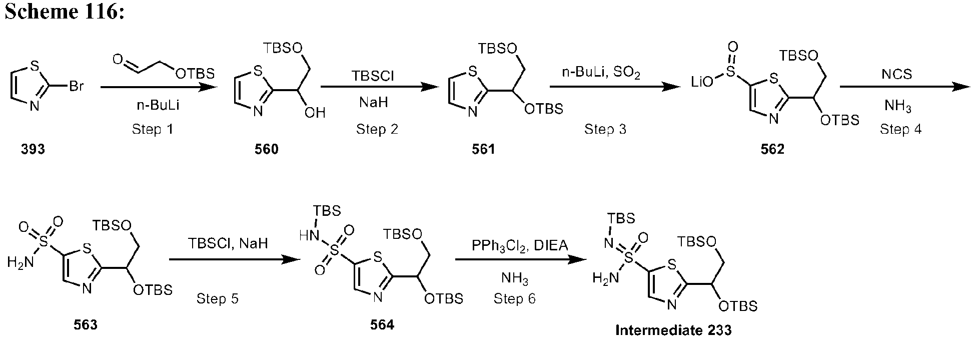

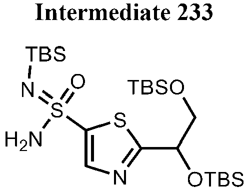

### N'-(tert-butyldimethylsilyl)-2-(2,2,3,3,8,8,9,9-octamethyl-4,7-dioxa-3,8-disiladecan-5-yl)thiazole-5-sulfonimidamide

### Step 1: 2-((Tert-butyldimethylsilyl)oxy)-1-(thiazol-2-yl)ethan-1-ol

To a stirred solution of 2-bromothiazole (10.0 g, 61.3 mmol) in THF (120 mL) under nitrogen was added n-BuLi (2.5 M in hexane, 36.8 mL, 92.0 mmol) dropwise at -78 °C. The resulting solution was stirred at -78 °C for 20 min, then to the above solution was added 2-((tertbutyldimethylsilyl)oxy)acetaldehyde (16.0 g, 92.0 mmol) in THF (10 mL) dropwise at -78 °C. The resulting solution was stirred for 2 h at -50 °C. The reaction was quenched with water (50 mL). The resulting mixture was concentrated to remove THF under vacuum. The aqueous phase was extracted with EtOAc (3x100 mL). The organic layers were combined, dried over anhydrous Na₂SO₄, and concentrated under vacuum. The residue was eluted from a silica gel column with EtOAc/PE (1:9). This resulted in 8.1 g (51.0%) of the title compound as yellow oil. MS-ESI: 260 (M+1).

### Step 2: 2-(2,2,3,3,8,8,9,9-Octamethyl-4,7-dioxa-3,8-disiladecan-5-yl)thiazole

To a stirred solution of 2-((tert-butyldimethylsilyl)oxy)-1-(thiazol-2-yl)ethan-1-ol (8.0 g, 30.9 mmol) in THF (150 mL) under nitrogen was added NaH (60%wt, 3.09 g, 77.3 mmol) in portions at 0 °C. The resulting solution was stirred for 10 min at 0 °C. Then to the above solution was added TBSCl (18.6 g, 124 mmol) in THF (15 mL) dropwise at 0 °C. The resulting solution was stirred for 16 h at RT. The reaction was quenched with water (100 mL), the resulting mixture was concentrated to remove THF under vacuum. The aqueous phase was extracted with EtOAc (3x100 mL). The organic layers were combined, dried over anhydrous Na₂SO₄, and concentrated under vacuum. The residue was eluted from a silica gel column with EtOAc/PE (4:96). This resulted in 8 g (69.3%) of the title compound as yellow oil. MS-ESI: 374 (M+1).

Steps 3-6 used similar procedures for converting compound **485** to Intermediate **203** shown in Scheme 98 to afford Intermediate **233** from compound **561.** MS-ESI: 566 (M+1).

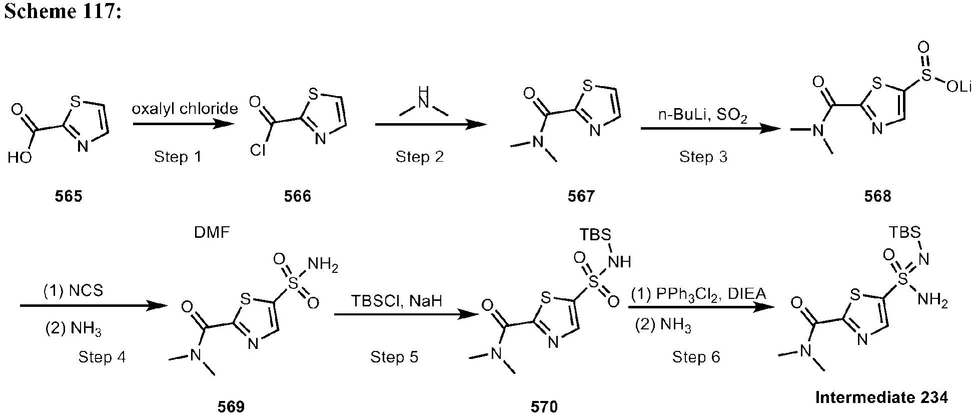

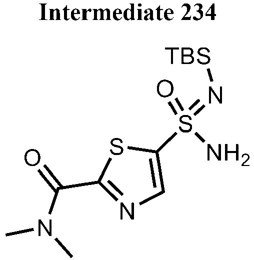

### 5-(N'-(tert-butyldimethylsilyl)sulfamidimidoyl)-N,N-dimethylthiazole-2-carboxamide

### Step 1: Thiazole-2-carbonyl chloride

To a stirred solution of thiazole-2-carboxylic acid (2.0 g, 15.5 mmol) in DCM (100 mL) was added DMF (0.095 mL, 1.24 mmol) followed by oxalyl chloride (5.91 g, 46.5 mmol) dropwise at 0 °C. The reaction mixture was stirred for 1 h at RT. The resulting mixture was concentrated under vacuum. This resulted in 2.4 g (crude) of the title compound as a yellow solid which was used for next step without further purification.

### Step 2: N,N-dimethylthiazole-2-carboxamide

To a stirred solution of dimethylamine (2 M in THF, 38.8 mL, 77.5 mmol) was added thiazole-2-carbonyl chloride (2.30 g, crude from last step) in THF (60 mL) dropwise at 0 °C. The resulting mixture was stirred for 16 h at RT. The reaction was quenched with water (100 mL) and extracted with EtOAc (3 x 100 mL). The organic layers were combined, dried over anhydrous Na₂SO₄, and concentrated under vacuum. This resulted in 2.18 g (90% over 2 steps) of the title compound as yellow oil. MS-ESI: 157 (M+1).

Steps 3-6 used similar procedures for converting compound **485** to Intermediate **203** shown in Scheme 98 to afford Intermediate **234** from compound **567.** MS-ESI: 349 (M+1).

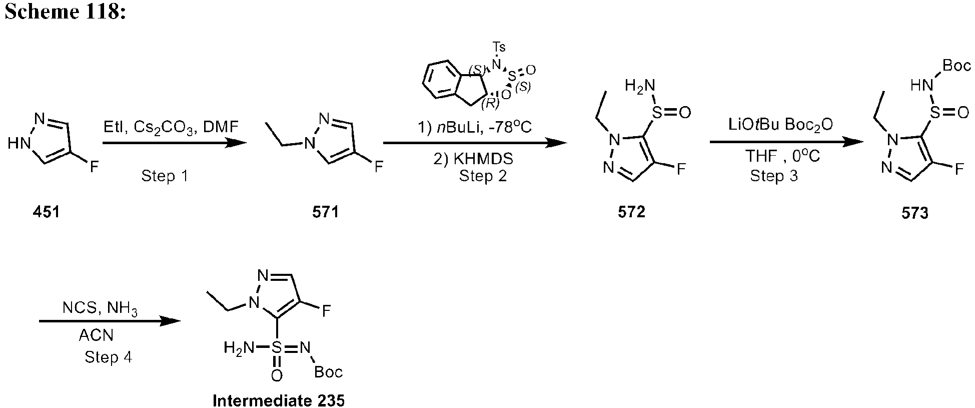

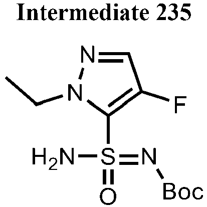

### Tert-butyl(amino(1-ethyl-4-fluoro-1H-pyrazol-5-yl)(oxo)-16-sulfaneylidene)carbamate

### Step 1: 1-Ethyl-4-fluoro-1H-pyrazole

To a stirred solution of 4-fluoro-1H-pyrazole (1.72 g, 20.0 mmol) in DMF (15 mL) under nitrogen was added Cs₂CO₃ (13.0 g, 40.0 mmol) and ethyl iodide (9.36 g, 60.0 mmol) at RT. The resulting mixture was stirred for 16 h at RT. The reaction was quenched with water (20 mL) and extracted with ether (2 x 50 mL). The organic layers were combined, dried over anhydrous Na₂SO₄, and concentrated under vacuum. This resulted in 2.0 g (87.7%) of the title compound as brown oil. MS-ESI: 115 (M+1).

### Step 2: 1-Ethyl-4-fluoro-1H-pyrazole-5-sulfinamide

To a stirred solution of 1-ethyl-4-fluoro-1H-pyrazole (1.5 g, 13.2 mmol) in THF (50 mL) under nitrogen was added n-BuLi (2.5 M in hexane, 7.92 mL, 19.8 mmol) dropwise with stirring at -78 °C. The resulting solution was stirred for 1.5 h at -78 °C, then to the above solution was added (2S,3aS,8aR)-3-tosyl-3,3a,8,8a-tetrahydroindeno[1,2-d][1,2,3]oxathiazole 2-oxide (4.82 g, 13.8 mmol) in THF (15 mL) dropwise at -78 °C. The resulting solution was stirred for 3 h at -78 °C. To the above mixture was added KHMDS (1 M in THF, 26.3 mL, 26.3 mmol) dropwise at -78 °C. The resulting solution was stirred for 1 h at -78 °C. The solution was slowly warmed to RT and stirred for an additional 1 h at RT. The reaction mixture was quenched with MeOH (50 mL) and concentrated under vacuum. The residue was diluted with water (100 mL) and extracted with EtOAc (2 x 100 mL). The organic layers were combined, dried over anhydrous Na₂SO₄, and concentrated under vacuum. The residue was eluted from a silica gel column with PE/EtOAc (1:1). This resulted in 790 mg (ee value = 0) (33.8%) of the title compound as an orange solid. MS-ESI: 178 (M+1). The regiochemistry of compound 572 was confirmed by NOESY. ¹H NMR (400 MHz, DMSO-*d*₆) δ 7.59 (d, *J =* 4.4 Hz, 1H), 6.91 (s, 2H), 4.35-4.16 (m, 2H), 1.33 (t, *J =* 7.2, Hz, 3H). NOESY: NH₂ at 6.91 (s, 2H) has correlations with N-Et's CH₂ at 4.35-4.16 (m, 2H) and CH₃ at 1.33 (t, *J =* 7.2, Hz, 3H).

### Step 3: Tert-butyl ((1-ethyl-4-fluoro-1H-pyrazol-5-yl)sulfinyl)carbamate

To a stirred solution of 1-ethyl-4-fluoro-1H-pyrazole-5-sulfinamide (696 mg, 3.93 mmol) in THF (25 mL) under nitrogen was added *t*-BuOLi (1 M in THF, 7.86 mL, 7.86 mmol) dropwise at 0 °C. The resulting mixture was stirred for 1 h at 0 °C. To the above mixture was added di-tert-butyl dicarbonate (3.42 g, 15.7 mmol) in THF (10 mL) dropwise at 0 °C. The resulting mixture was stirred for 3 h at 0 °C and then quenched with MeOH (10 mL) and concentrated under vacuum. The residue was diluted with water (30 mL) and extracted with EtOAc (2 x 30 mL). The organic layers were combined, dried over anhydrous Na₂SO₄, and concentrated under vacuum. The residue was eluted from a silica gel column with PE/EtOAc (5:1). This resulted in 717 mg (65.8%) of the title compound as light yellow oil. MS-ESI: 278 (M+1).

### Step 4: Tert-butyl (amino(1-ethyl-4-fluoro-1H-pyrazol-5-yl)(oxo)-16-sulfaneylidene)carbamate

To a stirred solution of tert-butyl ((1-ethyl-4-fluoro-1H-pyrazol-5-yl)sulfinyl)carbamate (717 mg, 2.59 mmol) in ACN (25 mL) was introduced NH₃ (g) bubble at 0 °C for 10 min. Then to the above solution was added NCS (3.45 g, 25.9 mmol) in portions at 0 °C. The resulting solution was stirred for 3 h at 35 °C in an oil bath. The residue was quenched with water (100 mL) and extracted with EtOAc (3 x 150 mL). The organic layers were combined, dried over anhydrous Na₂SO₄, and concentrated under vacuum. The residue was eluted from a silica gel column with PE/EtOAc (3:2). The product was further purified by Prep-HPLC with the following conditions: Column XSelect CSH Prep C₁₈ OBD, 19*250 mm, 5 um Mobile Phase A: Water (0.1%FA), Mobile Phase B: ACN; Flow rate: 25 mL/min; Gradient: 30% B to 53% B over 7 min; 254/210 nm. This resulted in 163 mg (21.6%) of the title compound as a white solid. MS-ESI: 293 (M+1).

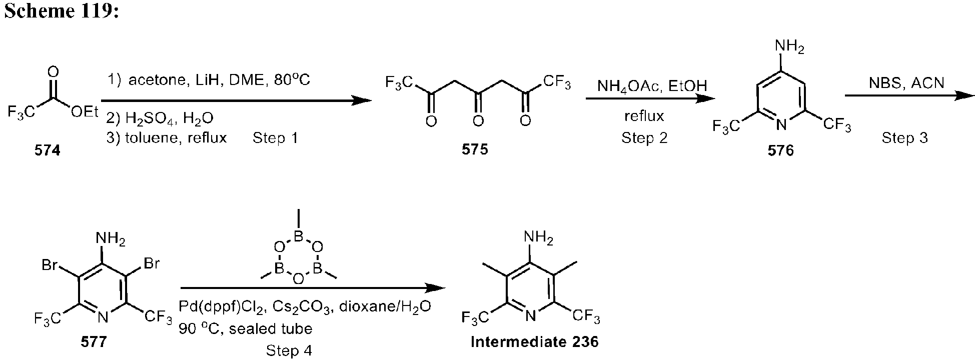

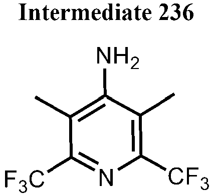

### 3,5-Dimethyl-2,6-bis(trifluoromethyl)pyridin-4-amine

### Step 1: 1,1,1,7,7,7-Hexafluoroheptane-2,4,6-trione

To a stirred mixture of LiH (1.51 g, 189 mmol) in DME (100 mL) under nitrogen were added ethyl 2,2,2-trifluoroacetate (26.9 g, 189 mmol) and acetone (5.0 g, 86.2 mmol) dropwise at 0 °C. The resulting mixture was stirred for 16 h at 80 °C under nitrogen. The mixture was allowed to cool down to RT and 50 mL of DME solvent was evaporated under vacuum. The residue was quenched with H₂SO₄ (15%wt. in water, 50 mL) dropwise at 0 °C. The aqueous layer was extracted with DCM (3x50 mL). The organic layers were combined, dried over anhydrous Na₂SO₄, and concentrated under vacuum. The residue was dissolved in toluene (50 mL) and heated to reflux with Dean-Stark trap for 4 h. After all water was separated, the resulting mixture was cooled and evaporated under vacuum to afford the title compound (26 g, crude) as a red solid. MS-ESI: 249 (M-1)

### Step 2: 2,6-Bis(trifluoromethyl)pyridin-4-amine

To a stirred mixture of 1,1,1,7,7,7-hexafluoroheptane-2,4,6-trione (14.7 g crude from last step) in EtOH (300 mL) was added NH₄OAc (36.3 g, 471 mmol) in portions at RT. The resulting mixture was heated to reflux with stirring for 16 h under nitrogen. The mixture was allowed to cool down to RT. The reaction was quenched with water (50 mL) and concentrated to remove EtOH under vacuum. The aqueous layer was extracted with EtOAc (3x30 mL). The organic layers were combined, dried over anhydrous Na₂SO₄, and concentrated under vacuum. The residue was eluted from silica gel column with PE/EtOAc (10:1) to afford the title compound (9.63 g, 85.9% over two steps) as a yellow solid. MS-ESI: 229 (M-1)

### Step 3: 3,5-Dibromo-2,6-bis(trifluoromethyl)pyridin-4-amine

To a stirred solution of 2,6-bis(trifluoromethyl)pyridin-4-amine (4.80 g, 20.9 mmol) in ACN (150 mL) under nitrogen was added NBS (18.7 g, 105 mmol) in portions at 0 °C. The resulting mixture was stirred for 16 h at 60 °C. The reaction was quenched with sat. aq. Na₂S₂O₃ (50 mL). The aqueous layer was extracted with EtOAc (3x50 mL). The organic layers were combined, dried over anhydrous Na₂SO₄, and concentrated under vacuum. The residue was eluted from silica gel column with PE/EtOAc (20:1) to afford the title compound (5.69 g, 70.2%) as a yellow solid. MS-ESI: 385/387/389 (M-1).

### Step 4: 3,5-Dimethyl-2,6-bis(trifluoromethyl)pyridin-4-amine

To a stirred mixture of 3,5-dibromo-2,6-bis(trifluoromethyl)pyridin-4-amine (5.50 g, 14.2 mmol) in dioxane (125 mL) and H₂O (12.5 mL) in a sealed tube under nitrogen were added 2,4,6-trimethyl-1,3,5,2,4,6-trioxatriborinane (50%wt. in THF, 17.9 g, 71.0 mmol), Pd(dppf)Cl₂ (1.04 g, 1.42 mmol) and Cs₂CO₃ (13.9 g, 42.6 mmol) in portions at RT. The resulting mixture was stirred for 16 h at 90 °C. The mixture was allowed to cool down to RT and then diluted with sat. aq. NaCl (100 mL) and extracted with EtOAc (3x100 mL). The organic layers were combined, dried over anhydrous Na₂SO₄, and concentrated under vacuum. The residue was eluted from silica gel column with PE/EtOAc (10:1) to afford the title compound (3.35 g, 91.3%) as a light yellow solid. MS-ESI: 259 (M+1).

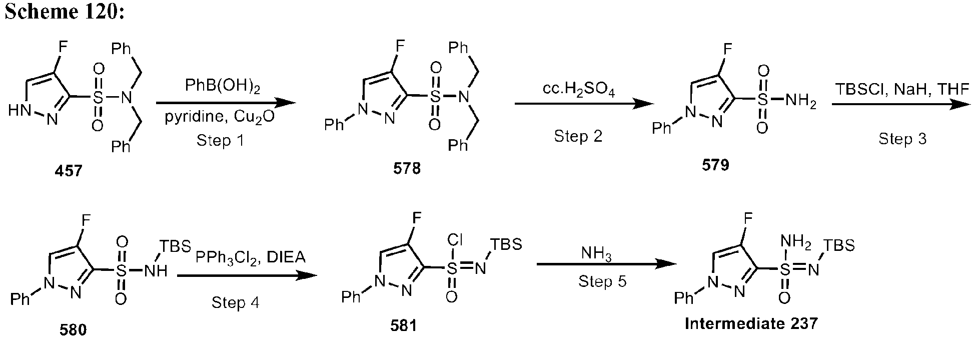

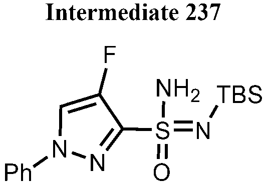

### N'-(tert-butyldimethylsilyl)-4-fluoro-1-phenyl-1H-pyrazole-3-sulfonimidamide

### Step 1: N,N-dibenzyl-4-fluoro-1-phenyl-1H-pyrazole-3-sulfonamide

To a stirred solution of N,N-dibenzyl-4-fluoro-1H-pyrazole-3-sulfonamide (4.0 g, 11.6 mmol) in DMF (120 mL) was added phenylboronic acid (4.24 g, 34.8 mmol), pyridine (2.75 g, 34.7 mmol) and Cu₂O (1.66 g, 44.6 mmol) in portions at RT. The resulting mixture was stirred for 16 h at 70 °C under air. The solids were filtered out. The resulting solution was diluted with 250 mL of water and extracted with 4x150 mL of EtOAc. The organic layers were combined, dried over anhydrous Na₂SO₄, and concentrated under vacuum. The residue was eluted from a silica gel with EtOAc/PE (1:15). This resulted in 3.31 (67.8%) of the title compound as a white solid. LCMS-ESI: 422 (M+1).

Steps 2-5 used similar procedures for converting compound **458A** to Intermediate **197** shown in Scheme 93 to afford Intermediate **237** from compound **578.** MS-ESI: 355 (M+1).

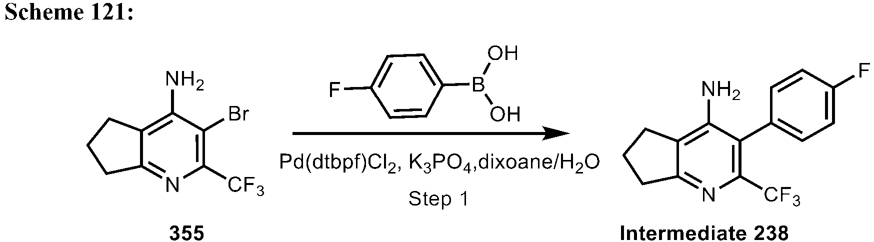

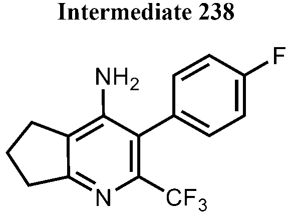

### 3-(4-Fluorophenyl)-2-(trifluoromethyl)-6,7-dihydro-5H-cyclopenta[b]pyridin-4-amine

### Step 1: 3-(4-Fluorophenyl)-2-(trifluoromethyl)-6,7-dihydro-5H-cyclopenta[b]pyridin-4-amine

To a stirred solution of 3-bromo-2-(trifluoromethyl)-6,7-dihydro-5H-cyclopenta[b]pyridin-4-amine (500 mg, 1.78 mmol) in dioxane (10 mL) and H₂O (1.0 mL) under nitrogen were added (4-fluorophenyl)boronic acid (498 mg, 3.56 mmol), K₃PO₄ (1132 mg, 5.34 mmol) and Pd(dtbpf)Cl₂ (116 mg, 0.178 mmol) in portions at RT. The reaction mixture was stirred for 16 h at 100 °C. The reaction was diluted with 10 mL of water and extracted with 3x10 mL of EtOAc. The organic layers were combined, dried over anhydrous Na₂SO₄, and concentrated under vacuum. The residue was eluted from silica gel column with EtOAc/PE (1:2). This resulted in 230 mg (43.5%) of the title compound as a yellow solid. MS-ESI: 297 (M+1)

**Table 60 The Intermediates in the following Table were prepared using the similar procedures for converting compound 355 to Intermediate 238 shown in Scheme 121 from appropriate reagents.**

| Intermediate # | Structure | IUPAC Name | Exact Mass [M+H]⁺ |
|---|---|---|---|
| **Intermediate 239** | 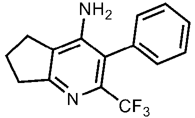 | 3-Phenyl-2-(trifluoromethyl)-6,7-dihydro-5H-cyclopenta[b]pyridin-4-amine | 279 |
| **Intermediate 240** | 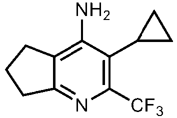 | 3-Cyclopropyl-2-(trifluoromethyl)-6,7-dihydro-5H-cyclopenta[b]pyridin-4-amine | 243 |
| **Intermediate 241** | 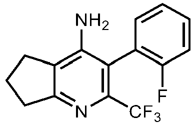 | 3-(2-Fluorophenyl)-2-(trifluoromethyl)-6,7-dihydro-5H-cyclopenta[b]pyridin-4-amine | 297 |
| **Intermediate 242** | 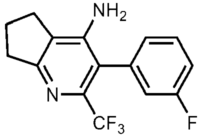 | 3-(3-Fluorophenyl)-2-(trifluoromethyl)-6,7-dihydro-5H-cyclopenta[b]pyridin-4-amine | 297 |

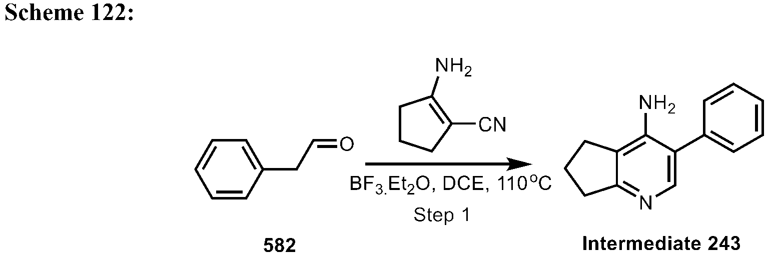

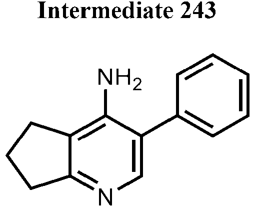

### 3-Phenyl-6,7-dihydro-5H-cyclopenta[b]pyridin-4-amine

To a stirred solution of 2-aminocyclopent-1-ene-1-carbonitrile (20 g, 185 mmol) in DCE (400 mL) under nitrogen was added 2-phenylacetaldehyde (44.4 g, 370 mmol) and BF₃·Et₂O (47% wt., 25 g, 370 mmol) dropwise at 0 °C. The resulting solution was stirred for 16 h at 110 °C. The resulting mixture was concentrated under vacuum. The resulting residue was diluted with 500 mL of water then extracted with 3x500 mL of EtOAc. The organic layers were combined, dried over anhydrous Na₂SO₄, and concentrated under vacuum. The residue was eluted from silica gel with EtOAc/PE (1:3). This resulted in 250 mg (0.64%) of the title compound as a yellow solid. MS-ESI: 211 (M+1).

**Table 61. The Intermediate in the following table were prepared using the similar procedures for converting compound 451 to Intermediate 197 shown in Scheme 93.**

| Intermediate # | Structure | IUPAC Name | Exact Mass [M-H]⁻ |
|---|---|---|---|
| **Intermediate 244** | 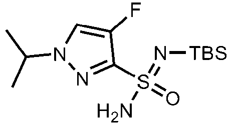 | N'-(tert-butyldimethylsilyl)-4-fluoro-1-isopropyl-1H-pyrazole-3-sulfonimidamide | 321 |

**Table 62 The Intermediate in the following table were prepared using the similar procedures for converting Intermediate 45 to Intermediate 161 shown in Scheme 82 from Intermediate 45 and (S)-(+)-Mandelic acid.**

| Intermediate # | Structure | IUPAC Name | Exact Mass [M+H]⁺ |
|---|---|---|---|
| **Intermediate 245** | 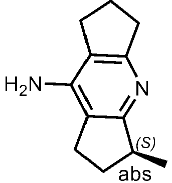 | (S)-3-Methyl-1,2,3,5,6,7-hexahydrodicyclopenta[b,e]pyridin-8-amine | 189 |

**Table 63 The Intermediates in the following Table were prepared using the similar procedures for converting compound 354 to Intermediate 222 shown in Scheme 115 from appropriated starting materials.**

| Intermediate # | Structure | IUPAC Name | Exact Mass [M-H]⁻ |
|---|---|---|---|
| **Intermediate 246** | 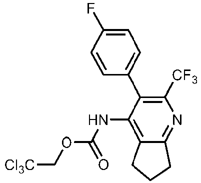 | 2,2,2-Trichloroethyl (3-(4-fluorophenyl)-2-(trifluoromethyl)-6,7-dihydro-5H-cyclopenta[b]pyridin-4-yl)carbamate | 471/473/475 |
| **Intermediate 247** | 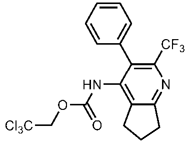 | 2,2,2-Trichloroethyl (3-phenyl-2-(trifluoromethyl)-6,7-dihydro-5H-cyclopenta[b]pyridin-4-yl)carbamate | 453/455/457 |
| **Intermediate 248** | 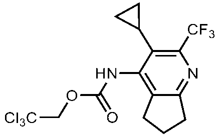 | 2,2,2-Trichloroethyl (3-cyclopropyl-2-(trifluoromethyl)-6,7-dihydro-5H-cyclopenta[b]pyridin-4-yl)carbamate | 417/419/421 |
| **Intermediate 249** | 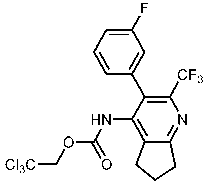 | 2,2,2-Trichloroethyl (3-(3-fluorophenyl)-2-(trifluoromethyl)-6,7-dihydro-5H-cyclopenta[b]pyridin-4-yl)carbamate | 471/473/475 |
| **Intermediate 250** | 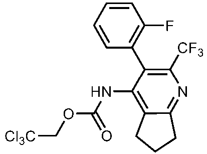 | 2,2,2-Trichloroethyl (3-(2-fluorophenyl)-2-(trifluoromethyl)-6,7-dihydro-5H-cyclopenta[b]pyridin-4-yl)carbamate | 471/473/475 |
| **Intermediate 251** | 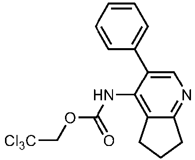 | 2,2,2-Trichloroethyl (3-phenyl-6,7-dihydro-5H-cyclopenta[b]pyridin-4-yl)carbamate | 385/387/389 |
| **Intermediate 252** | 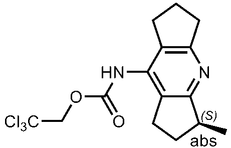 | 2,2,2-Trichloroethyl (S)-(3-methyl-1,2,3,5,6,7-hexahydrodicyclopenta[b,e]pyridin-8-yl)carbamate | 363/365/367 |

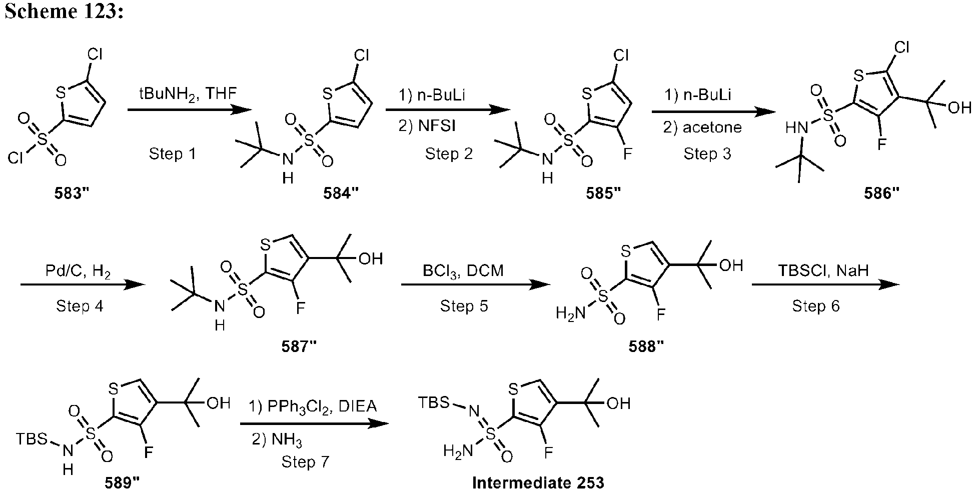

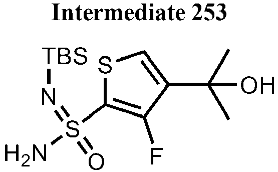

### N'-(tert-butyldimethylsilyl)-3-fluoro-4-(2-hydroxypropan-2-yl)thiophene-2-sulfonimidamide

### Step 1: N-(tert-butyl)-5-chlorothiophene-2-sulfonamide

To a stirred solution of tBuNH₂ (20.2 g, 276 mmol) in DCM (180 mL) under nitrogen was added TEA (42.0 g, 415 mmol), followed by the addition of 5-chlorothiophene-2-sulfonyl chloride (10.0 g, 46.3 mmol) in DCM (20 mL) dropwise at 0 °C. The resulting solution was stirred for 16 h at 35 °C. The reaction was quenched with water/ice (200 mL) and extracted with DCM (2x200 mL). The combined organic layers were dried over anhydrous Na₂SO₄ and concentrated under vacuum. The residue was eluted from a silica gel column with EtOAc/PE (1:10). This resulted in 11.0 g (93.9%) of the title compound as a yellow solid. MS-ESI: 252/254 (M-1). ¹H NMR (400 MHz, DMSO-*d*₆) δ 7.90 (s, 1H), 7.46 (d, *J* = 4.0 Hz, 1H), 7.21 (d, *J* = 4.1 Hz, 1H), 1.17 (s, 9H).

### Step 2: N-(tert-butyl)-5-chloro-3-fluorothiophene-2-sulfonamide

To a stirred solution of N-(tert-butyl)-5-chlorothiophene-2-sulfonamide (11.0 g, 43.5 mmol) in THF (200 mL) under nitrogen was added n-BuLi (2.5 M in hexane, 52.4 mL, 131 mmol) dropwise at -78 °C. The resulting solution was stirred at -78 °C for 1 h. To the above solution was added NFSI (41.6 g, 131 mmol) in THF (100 mL) dropwise at -78 °C. The resulting solution was slowly warmed to RT, and stirred at RT for another 1 h. The reaction mixture was then quenched with water/ice (250 mL) and extracted with EtOAc (3x300 mL). The combined organic layers were anhydrous Na₂SO₄ and concentrated under vacuum. The residue was eluted from a silica gel column with EtOAc/PE (1:20). This resulted in 6.2 g (43.3%) of the title compound as a yellow solid. MS-ESI: 270/272 (M-1).

### Step 3: N-(tert-butyl)-5-chloro-3-fluoro-4-(2-hydroxypropan-2-yl)thiophene-2-sulfonamide

To a stirred solution of N-(tert-butyl)-5-chloro-3-fluorothiophene-2-sulfonamide (6.2 g, 22.8 mmol) in THF (200 mL) under nitrogen was added n-BuLi (2.5 M in hexane, 91.2 mL, 228 mmol) dropwise at -78 °C. The resulting solution was stirred for 50 min at -78 °C. To this was added acetone (53.0 g, 913 mmol) dropwise with stirring at -78 °C. The resulting solution was stirred for additional 1.5 h at -60 °C. The reaction was then quenched with water/ice (200 mL) and extracted with EtOAc (2x300 mL). The combined organic layers were dried over anhydrous Na₂SO₄ and concentrated under vacuum. The residue was eluted from a silica gel column with EtOAc/PE (1:5). This resulted in 4.4 g (65.4 %) of the title compound as yellow oil. MS-ESI: 328/330 (M-1).

### Step 4: N-(tert-butyl)-3-fluoro-4-(2-hydroxypropan-2-yl)thiophene-2-sulfonamide

To a stirred solution of N-(tert-butyl)-5-chloro-3-fluoro-4-(2-hydroxypropan-2-yl)thiophene-2-sulfonamide (4.3 g, 13.1 mmol) in MeOH (80 mL) under nitrogen was added Pd/C (10% wt, 470 mg) in portions at RT. The flask was evacuated and refilled with hydrogen three times. The resulting mixture was stirred for 16 h at RT under hydrogen with a balloon. The solids were filtered out and the filtrate was concentrated under vacuum. This resulted in 3.88 g (crude) of the title compound as a white solid. MS-ESI: 294 (M-1).

### Step 5: 3-Fluoro-4-(2-hydroxypropan-2-yl)thiophene-2-sulfonamide

To a stirred solution of N-(tert-butyl)-3-fluoro-4-(2-hydroxypropan-2-yl)thiophene-2-sulfonamide (3.88 g, 13.1 mmol) in DCM (78 mL) under nitrogen was added BCl₃ (1 M in DCM, 39 mL, 39 mmol) dropwise at 0 °C. The resulting solution was stirred for 3 h at RT. The reaction was then quenched with water/ice (80 mL) and extracted with EtOAc (2x100 mL). The combined organic layers were dried over anhydrous Na₂SO₄ and concentrated under vacuum. The residue was eluted from a silica gel column with EtOAc/PE (2:3). This resulted in 1.9 g (60.7% over 2 steps) of the title compound as yellow oil. MS-ESI: 238 (M-1).

### Step 6: N-(tert-butyldimethylsilyl)-3-fluoro-4-(2-hydroxypropan-2-yl)thiophene-2-sulfonamide

To a stirred solution of 3-fluoro-4-(2-hydroxypropan-2-yl)thiophene-2-sulfonamide (1.89 g, 7.91 mmol) in THF (40 mL) under nitrogen was added NaH (60% wt., 630 mg, 15.9 mmol) in portions at 0 °C, followed by the addition of TBSCl (2.39 g, 15.9 mmol) in THF (5 mL) dropwise with stirring at 0 °C. The resulting solution was stirred for 2 h at RT. The reaction was quenched with water/ice (50 mL). The resulting solution was extracted with EtOAc (2x50 mL). The organic layers were combined and dried over anhydrous Na₂SO₄. The resulting mixture was concentrated under vacuum. The residue was eluted from a silica gel column with ethyl EtOAc/PE (1:3). This resulted in 2.1 g (75.2%) of the title compound as a white solid. MS-ESI: 352 (M-1). ¹H NMR (400 MHz, DMSO-*d*₆) δ 8.13 (s, 1H), 7.56 (d, *J =* 5.1 Hz, 1H), 5.38 (s, 1H), 1.43 (s, 6H), 0.89 (s, 9H), 0.16 (s, 6H).

### Step 7: N'-(tert-butyldimethylsilyl)-3-fluoro-4-(2-hydroxypropan-2-yl)thiophene-2-sulfonimidamide

To a stirred solution of PPh₃Cl₂ (1.41 g, 4.24 mmol) in CHCl₃ (15 mL) under nitrogen was added DIEA (1.83 g, 14.1 mmol) dropwise at 0 °C. The resulting solution was stirred for 15 min at 0 °C. To the above solution was added N-(tert-butyldimethylsilyl)-2-(difluoromethyl)thiazole-5-sulfonamide (1.0 g, 2.83 mmol) in CHCl₃ (10 mL) dropwise at 0 °C. The resulting solution was stirred for 2 h at 0 °C. NH₃ (g) was bubbled into the reaction solution for 10 min at 0 °C. Then the solution was stirred for another 30 min at RT. The solids were filtered out, the filtrate was diluted with water (20 mL). The resulting solution was extracted with DCM (3x20 mL). The organic layers were dried over anhydrous Na₂SO₄ and concentrated under vacuum. The residue was eluted from a silica gel column with EtOAc/PE (1:1). This resulted in 800 mg (80.1%) of the title compound as an off-white solid. MS-ESI: 351 (M-1).

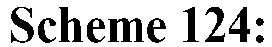

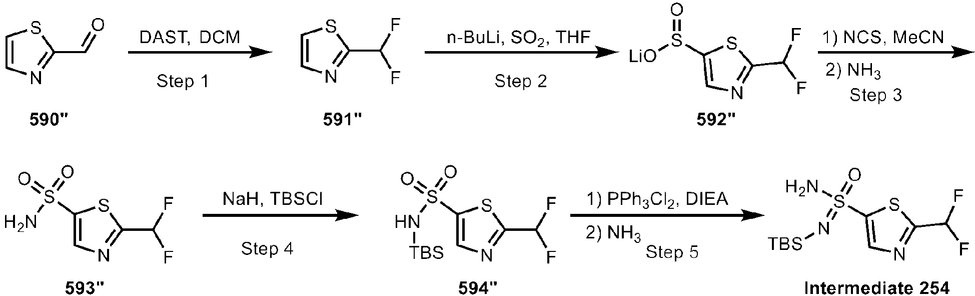

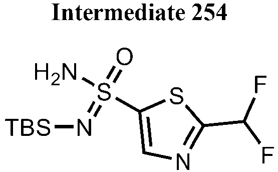

### N'-(tert-butyldimethylsilyl)-2-(difluoromethyl)thiazole-5-sulfonimidamide

### Step 1: 2-(Difluoromethyl)thiazole

To a stirred solution of thiazole-2-carbaldehyde (10 g, 88.5 mmol) in DCM (150 mL) under nitrogen atmosphere was added DAST (28.6 g, 178 mmol) dropwise at -78 °C. The resulting mixture was stirred for 16 h at RT. The reaction was quenched with 100 mL of water/ice at 0 °C. The mixture was neutralized to pH=8 with sat. NaHCO₃ (aq.). The resulting mixture was extracted with DCM (2x100 mL). The combined organic layers were dried over anhydrous Na₂SO₄ and concentrated under vacuum below 5 °C. This resulted in the title compound (5.8 g, 48.5%) as a yellow oil which was used for next step without further purification. ¹H NMR (400 MHz, CDCl₃) δ 7.94 (dt, *J* = 3.0, 1.4 Hz, 1H), 7.58 (d, *J* = 3.1 Hz, 1H), 6.91 (t, *J =* 54.8 Hz, 1H).

### Step 2: Lithium 2-(difluoromethyl)thiazole-5-sulfinate

To a stirred solution of 2-(difluoromethyl)thiazole (5.8 g, 43.0 mmol) in THF (50 mL) under nitrogen was added n-BuLi (2.5 M in hexane, 21.0 mL, 52.5 mmol) dropwise at -78 °C. The resulting solution was stirred for 20 min at -78 °C. To the above solution was introduced SO₂ bubbled at -78 °C for 5 min. The resulting mixture was stirred for 2 h at RT and concentrated under vacuum. This resulted in 16.5 g (crude) title compound as a dark yellow solid which was used for next step without further purification. MS-ESI: 198 (M-1).

### Step 3: 2-(Difluoromethyl)thiazole-5-sulfonamide

To a stirred solution of lithium 2-(difluoromethyl)thiazole-5-sulfinate (16.5 g, crude from last step) in ACN (50 mL) was added NCS (17.1 g, 128 mmol) in portions at 0 °C. The resulting solution was stirred for 2 h at RT. To the above solution was introduced NH₃ bubbled at 0 °C for 10 min. The resulting solution was stirred for 2 h at RT. The resulting mixture was concentrated under vacuum. The residue was eluted from a silica gel column with EtOAc/PE (1:1). This resulted in 2.87 g (15.2% over two steps) of the title compound as a yellow solid. MS-ESI: 213 (M-1). ¹H NMR (400 MHz, CDCl₃) δ 8.33 (t, *J =* 1.5 Hz, 1H), 6.87 (t, *J =* 54.3 Hz, 1H), 5.55 (s, 2H).

### Step 4: N-(tert-butyldimethylsilyl)-2-(difluoromethyl)thiazole-5-sulfonamide

To a stirred solution of 2-(difluoromethyl)thiazole-5-sulfonamide (2.87 g, 13.4 mmol) in THF (50 mL) under nitrogen was added NaH (60%wt, 2.05 g, 51.2 mmol) in portions at 0 °C, followed by the addition of TBSCl (4.59 g, 30.6 mmol) in THF (5 mL) dropwise with stirring at 0 °C. The resulting solution was stirred for 2 h at RT. The reaction was quenched with 50 mL of water/ice. The resulting solution was extracted with EtOAc (2x50 mL). The organic layers were combined and dried over anhydrous Na₂SO₄. The resulting mixture was concentrated under vacuum. The residue was eluted from a silica gel column with ethyl EtOAc/PE (1:4). This resulted in 2.48 g (56.4%) of the title compound as a yellow solid. MS-ESI: 327 (M-1).

### Step 5: N'-(tert-butyldimethylsilyl)-2-(difluoromethyl)thiazole-5-sulfonimidamide

To a stirred solution of PPh₃Cl₂ (2.43 g, 7.31 mmol) in CHCl₃ (30 mL) under nitrogen was added DIEA (2.40 g, 18.6 mmol) dropwise at 0 °C. The resulting solution was stirred for 15 min at 0 °C. To the above solution was added N-(tert-butyldimethylsilyl)-2-(difluoromethyl)thiazole-5-sulfonamide (1.20 g, 3.66 mmol) in CHCl₃ (5 mL) dropwise at 0 °C. The resulting solution was stirred for 2 h at 0 °C. NH₃ (g) was bubbled into the reaction solution for 15 min at 0 °C. Then the solution was stirred for another 2 h at RT. The solids were filtered out, the filtrate was diluted with water (20 mL). The resulting solution was extracted with DCM (3x20 mL). The organic layers were combined, dried over anhydrous Na₂SO₄, and concentrated under vacuum. The residue was eluted from a silica gel column with EtOAc/PE (1:1). This resulted in 670 mg (56.0%) of the title compound as yellow oil. MS-ESI: 328 (M+1).

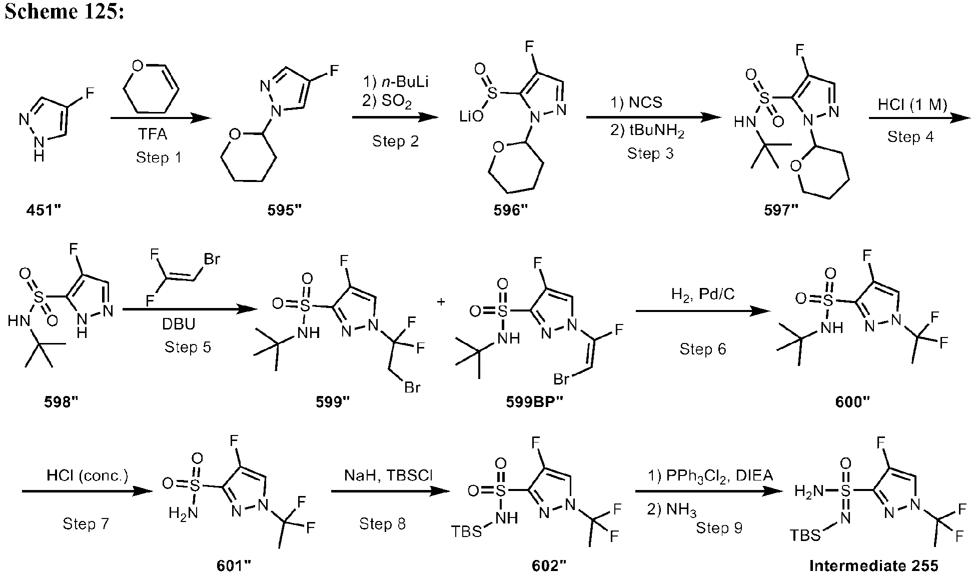

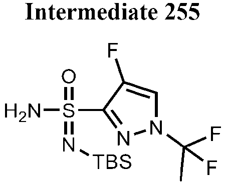

### N'-(tert-butyldimethylsilyl)-1-(1,1-difluoroethyl)-4-fluoro-1H-pyrazole-3-sulfonimidamide

### Step 1: 4-Fluoro-1-(tetrahydro-2H-pyran-2-yl)-1H-pyrazole

To a stirred solution of 4-fluoro-1H-pyrazole (5.00 g, 58.1 mmol) in 3,4-dihydro-2H-pyran (9.77 g, 116 mmol) under nitrogen was added a catalytic amount of TFA (260 mg, 2.32 mmol). The resulting solution was stirred for 16 h at 100 °C. The reaction was then quenched with 200 mg of NaH (60%wt.) at 0 °C. The resulting solution was diluted with water (100 mL). The resulting solution was extracted with EtOAc (3x100 mL). The organic layers were combined and dried over anhydrous Na₂SO₄. The solids were filtered out. The resulting mixture was concentrated under vacuum. This resulted in 12.0 g (crude) of the title compound as a light brown oil. MS-ESI: 171 (M+1).

### Step 2: Lithium 4-fluoro-1-(tetrahydro-2H-pyran-2-yl)-1H-pyrazole-5-sulfinate

To a stirred solution of 4-fluoro-1-(oxan-2-yl)pyrazole (12.0 g, crude from last step) in THF (250 mL) was added n-BuLi (2.5 M in hexane, 20.0 mL, 50 mmol) dropwise at -78 °C. The resulting solution was stirred for 40 min at -78 °C. To the above solution was introduced SO₂ (g) bubbled at -78 °C for 5 min. Then, the temperature was warmed to RT. The resulting solution was stirred for an additional 1 h at RT. The resulting mixture was concentrated under vacuum. This resulted in 28.0 g (crude) of the title compound as a light yellow semi-solid. MS-ESI: 233 (M-1).

### Step 3: N-(tert-butyl)-4-fluoro-1-(tetrahydro-2H-pyran-2-yl)-1H-pyrazole-5-sulfonamide

To a solution of lithium 4-fluoro-1-(tetrahydro-2H-pyran-2-yl)-1H-pyrazole-5-sulfinate (28.0 g, crude from last step) in ACN (200 mL) was added NCS (19.9 g, 149 mmol) in ACN (50 mL) dropwise at 0 °C. The resulting solution was stirred for 1 h at RT. To the reaction mixture above was added tBuNH₂ (49.8 g, 683 mmol) dropwise with stirring at 0 °C. The resulting solution was stirred for an additional 30 min at RT. The resulting mixture was concentrated under vacuum and diluted with 200 mL of water. The resulting solution was extracted with 3x150 mL of EtOAc, and the organic layers were combined. The residue after evaporation was purified by Prep-TLC with EtOAc/PE (1:4). This resulted in 13.3 g (75.0% over three steps) of the title compound as a yellow solid. MS-ESI: 304 (M-1). ¹H NMR (400 MHz, CDCl₃) δ 7.42 (d, *J =* 4.6 Hz, 1H), 5.90 (dd, *J =* 8.6, 2.9 Hz, 1H), 5.17 (s, 1H), 3.98-3.86 (m, 1H), 3.80-3.68 (m, 1H), 2.48-2.32 (m, 1H), 2.21-2.02 (m, 2H), 1.85-1.55 (m, 3H), 1.31 (s, 9H).

### Step 4: N-(tert-butyl)-4-fluoro-1H-pyrazole-5-sulfonamide

To a solution of N-(tert-butyl)-4-fluoro-1-(tetrahydro-2H-pyran-2-yl)-1H-pyrazole-5-sulfonamide (13.3 g, 43.6 mmol) in MeOH (220 mL) was added HCl (conc., 20 mL) dropwise at 0 °C. The resulting solution was stirred for 30 min at RT. The resulting mixture was concentrated under vacuum. This resulted in 9.7 g (crude) of the title compound as a yellow green solid. MS-ESI: 222 (M+1).

### Step 5: 1-(2-Bromo-1,1-difluoroethyl)-N-(tert-butyl)-4-fluoro-1H-pyrazole-3-sulfonamide

To a solution of N-(tert-butyl)-4-fluoro-1H-pyrazole-5-sulfonamide (9.70 g, 43.8 mmol) in THF (200 mL) was added DBU (33.4 g, 219 mmol) at -20 °C. The resulting solution was stirred for 5 min at -20 °C. Then 2-bromo-1,1-difluoroethene was bubbled to this reaction mixture at -20 °C for 10 min. The resulting solution was stirred for 1 h at -20 °C. The resulting mixture was concentrated and diluted with 150 mL of water. The resulting solution was extracted with EtOAc (3x100 mL). The organic layers were combined and dried over anhydrous Na₂SO₄. The residue was purified by Prep-TLC with EtOAc /PE (1:2). This resulted in 8.5 g (53.7% over 2 steps) of the title compound as a yellow solid. MS-ESI: 364/366 (M+1). ¹H NMR (400 MHz, DMSO-*d*₆) δ 8.80 (d, *J* = 4.5 Hz, 1H), 8.21 (s, 1H), 4.58 (t, *J =* 12.1 Hz, 2H), 1.19 (s, 9H). NOESY: Ar-H at 8.80 (d, *J* = 4.5 Hz, 1H) correlated with CH₂ at 4.58 (t, *J =* 12.1 Hz, 2H).

This also resulted in 2.0 g (13.4%) of by product (E)-1-(2-bromo-1-fluorovinyl)-N-(tert-butyl)-4-fluoro- 1H-pyrazole-3-sulfonamide (**599BP"**) as a yellow solid which eluted after **599".** MS-ESI: 344/346 (M+1). ¹H NMR (400 MHz, CDCl₃) δ 7.79 (d, *J =* 5.0 Hz, 1H), 6.27 (d, *J* = 5.3 Hz, 1H), 5.00 (br s, 1H), 1.36 (s, 9H).

### Step 6: N-(tert-butyl)-1-(1,1-difluoroethyl)-4-fluoro-1H-pyrazole-3-sulfonamide

To a stirred solution of 1-(2-bromo-1,1-difluoroethyl)-N-tert-butyl-4-fluoropyrazole-3-sulfonamide (8.0 g, 22.0 mmol) in EtOH (250 mL) in a stainless steel pressure reactor under nitrogen was added Pd/C (10%wt., 3.50 g) and AcOH (0.5 mL). The mixture was stirred at 100 °C for 16 h under hydrogen (10 atm), filtered through a Celite pad and concentrated under vacuum. The residue was purified by Prep-TLC (EA/PE 1:1) to afford 5.0 g of the title compound (79.7%) as a light yellow solid. MS-ESI: 286 (M+1).

### Step 7: 1-(1,1-Difluoroethyl)-4-fluoro-1H-pyrazole-3-sulfonamide

N-tert-butyl-1-(1,1-difluoroethyl)-4-fluoropyrazole-3-sulfonamide (5.0 g, 17.5 mmol) was added HCl (conc. 50 mL) in portions with stirring at 0 °C. The resulting solution was stirred for 1 h at RT. The resulting mixture was concentrated under vacuum. The residue was eluted from a silica gel column with EtOAc/PE (1:1). This resulted in 3.90 g (97.3%) of the title compound as a yellow solid. MS-ESI: 230 (M+1).

Steps 8-9 used similar procedures for converting compound **588"** to Intermediate **253** shown in Scheme **123** to afford Intermediate **255** from compound **601".** MS-ESI: 343 (M+1).

**Table 63A. The Intermediate in the following Table were prepared using similar procedures for converting compound 355" to Intermediate 238 shown in Scheme 121 from appropriate reagents.**

| Intermediate # | Structure | IUPAC Name | Exact Mass [M+H]⁺ |
|---|---|---|---|
| **Intermediate 256** | 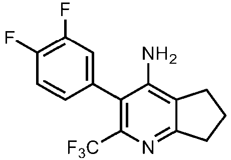 | 3-(3,4-Difluorophenyl)-2-(trifluoromethyl)-6,7-dihydro-5H-cyclopenta[b]pyridin-4-amine | 315 |

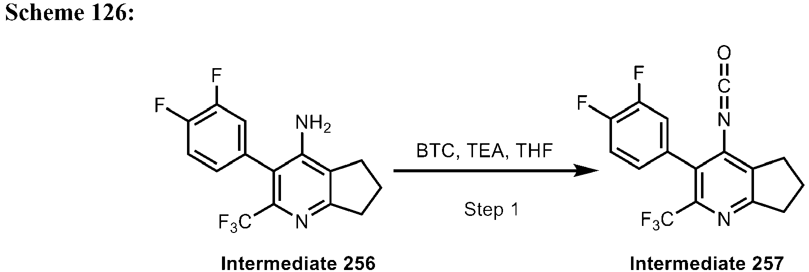

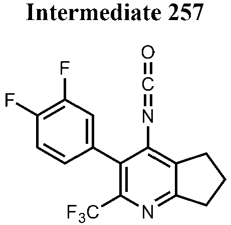

### 3-(3,4-Difluorophenyl)-4-isocyanato-2-(trifluoromethyl)-6,7-dihydro-5H-cyclopenta[b]pyridine

To a solution of 3-(3,4-difluorophenyl)-2-(trifluoromethyl)-6,7-dihydro-5H-cyclopenta[b]pyridin-4-amine (200 mg, 0.637 mmol) in THF (4 mL) under nitrogen was added TEA (129 mg, 1.28 mmol) and BTC (151 mg, 0.508 mmol) at 0 °C. The resulting solution was stirred for 2 h at 70 °C. This resulted in the title compound in a brown solution which was used for next step directly.

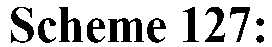

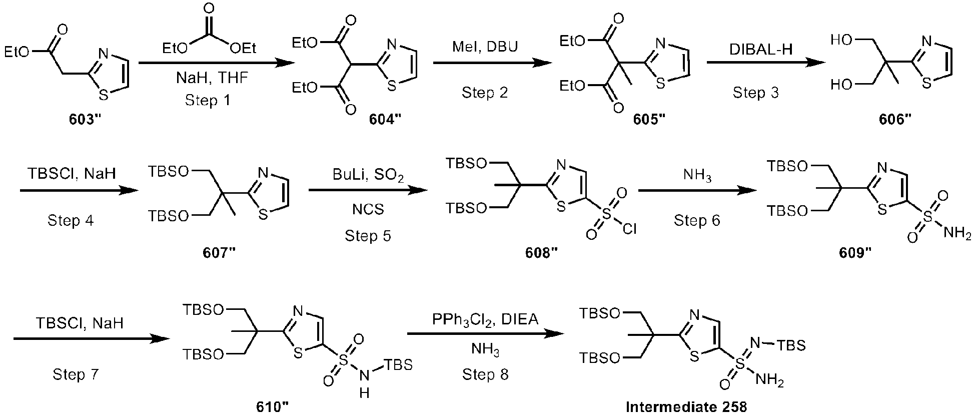

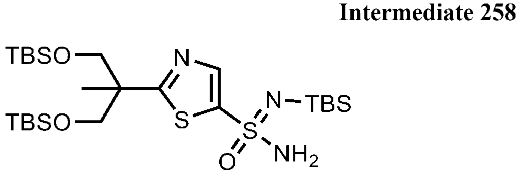

### N'-(tert-butyldimethylsilyl)-2-(2,2,3,3,6,9,9,10,10-nonamethyl-4,8-dioxa-3,9-disilaundecan-6-yl)thiazole-5-sulfonimidamide

### Step 1: Diethyl 2-(thiazol-2-yl)malonate

To a stirred solution of ethyl 2-(thiazol-2-yl)acetate (2.0 g, 11.7 mmol) in THF (20 mL) under nitrogen was added NaH (60%wt. dispersion in mineral oil, 1.0 g, 25.0 mmol) in portions at 0 °C. The resulting solution was stirred for 30 min at 0 °C. Then diethyl carbonate (23.0 g, 195 mmol) was added dropwise to the above solution. The resulting solution was stirred for 8 h at 65 °C. The reaction was then quenched with 30 mL of water, then extracted with 3x50 mL of EtOAc. The organic layers were combined and dried over anhydrous Na₂SO₄ and concentrated under vacuum. The crude product was eluted from silica gel with EtOAc/PE (1/2). This resulted in 2.44 g (85.8%) of the title compound as a light yellow solid. MS-ESI: 242 (M-1).

### Step 2: Diethyl 2-methyl-2-(thiazol-2-yl)malonate

To a stirred solution of diethyl 2-(thiazol-2-yl)malonate (2.0 g, 8.22 mmol) in THF (30 mL) was added DBU (2.50 g, 16.4 mmol) at 0 °C. Then MeI (4.67 g, 32.9 mmol) was added dropwise to the above solution. The resulting solution was stirred for 2 h at RT. The reaction was then quenched with 20 mL of water/ice, and then extracted with 3x50 mL of EtOAc. The organic layers were combined and dried over anhydrous Na₂SO₄ and concentrated under vacuum. The crude product was purified by Prep-TLC with EtOAc/PE (1/2). This resulted in 1.3 g (61.5%) of the title compound as light yellow oil. MS-ESI: 258 (M+1).

### Step 3: 2-Methyl-2-(thiazol-2-yl)propane-1,3-diol

To a stirred solution of diethyl 2-methyl-2-(thiazol-2-yl)malonate (1.0 g, 3.89 mmol) in THF (30 mL) was added DIBAL-H (1 M in hexane, 7.82 mL, 7.82 mmol) dropwise at 0 °C over 5 min. The resulting solution was stirred for 24 h at RT. The reaction was then quenched with 10 mL of MeOH. The resulting mixture was concentrated under vacuum. The crude product was purified by reverse phase column with ACN/water (15/75). This resulted in 290 mg (43.1%) of the title compound as light yellow oil. MS-ESI: 174 (M+1).

### Step 4: 2-(2,2,3,3,6,9,9,10,10-Nonamethyl-4,8-dioxa-3,9-disilaundecan-6-yl)thiazole

To a stirred solution of 2-methyl-2-(thiazol-2-yl)propane-1,3-diol (72.0 mg, 0.416 mmol) in THF (10 mL) under nitrogen was added NaH (60%wt., 66.6 mg, 1.66 mmol) in portions at 0 °C. The resulting solution was stirred for 30 min at 0 °C. Then TBSCl (190 mg, 1.25 mmol) was added to the above solution at 0 °C. The resulting solution was stirred for 2 h at RT. The reaction was then quenched with 10 mL of water/ice, and extracted with 3x30 mL of EtOAc. The organic layers were combined, dried over anhydrous Na₂SO₄, and concentrated. This resulted in 300 mg (crude) of the title compound as light yellow crude oil. MS-ESI: 402 (M+1).

Steps 5-8 used similar procedures for converting compound **485"** to intermediate **203** shown in Scheme **98** to afford Intermediate **258** from compound **607".** MS-ESI: 594 (M+1)

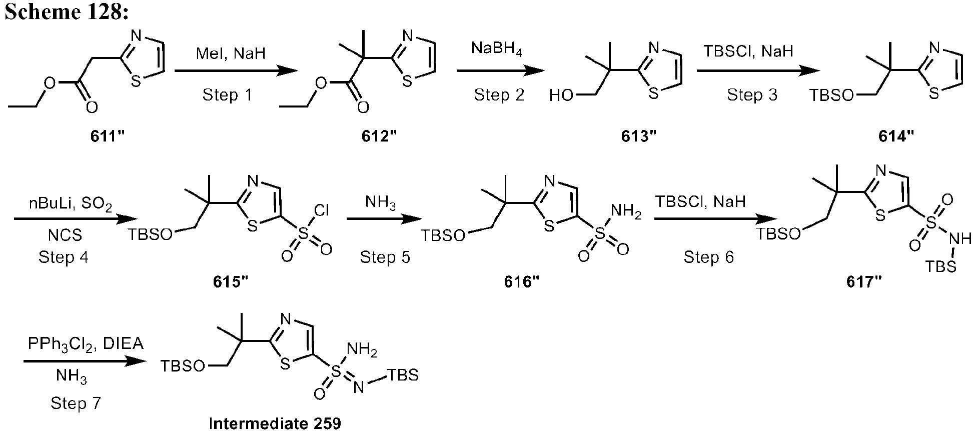

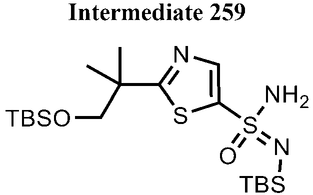

### N'-(tert-butyldimethylsilyl)-2-(1-((tert-butyldimethylsily)oxy)-2-methylpropan-2-yl)thiazole-5-sulfonimidamide

### Step 1: Ethyl 2-methyl-2-(thiazol-2-yl)propanoate

To a stirred solution of ethyl 2-(thiazol-2-yl)acetate (2.50 g, 14.6 mmol) in THF (30 mL) under nitrogen was added NaH (60%wt., 1.75 g, 43.8 mmol) in portions at 0 °C, followed the dropwise addition of methyl iodide (20.7 g, 146 mmol) at 0 °C. The resulting solution was stirred for 16 h at RT. The reaction was quenched with water/ice (30 mL). The resulting mixture was extracted with EtOAc (3x100 mL). The combined organic layers were washed with brine (2x30 mL), dried over anhydrous Na₂SO₄, and concentrated under vacuum. The residue was eluted from a silica gel column with EtOAc/PE (10:1). This resulted in 1.4 g (48.2%) of the title compound as a yellow solid. MS-ESI: 200 (M+1).

### Step 2: 2-Methyl-2-(thiazol-2-yl)propan-1-ol

To a stirred solution of ethyl 2-methyl-2-(thiazol-2-yl)propanoate (1.30 g, 6.53 mmol) in THF (50 mL) was added NaBH₄ (1.44 g, 39.1 mmol) in portions at 0 °C. The resulting solution was stirred for 2 h at RT. The reaction was quenched with water/ice (15 mL). The resulting solution was extracted with 3x50 mL of EtOAc. The combined organic layers were combined, dried over anhydrous Na₂SO₄, and concentrated under vacuum. The residue was eluted from a silica gel column with EtOAc/PE (1:3). This resulted in 600 mg (58.5%) of the title compound as a yellow solid. MS-ESI: 158 (M+1).

Steps 3-7 used similar procedures for converting compound **606"** to Intermediate **258** shown in Scheme **127** to afford Intermediate **259** from compound **613".** MS-ESI: 464 (M+1).

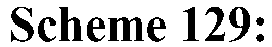

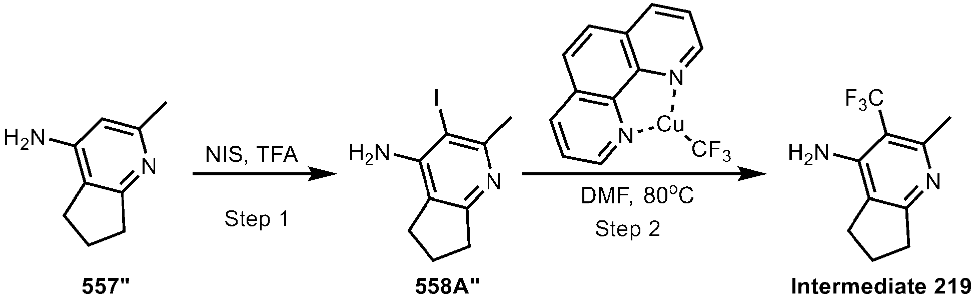

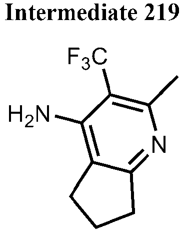

### 2-Methyl-3-(trifluoromethyl)-6,7-dihydro-5H-cyclopenta[b]pyridin-4-amine

### Step 1: 3-Iodo-2-methyl-6,7-dihydro-5H-cyclopenta[b]pyridin-4-amine

To a stirred solution of 2-methyl-6,7-dihydro-5H-cyclopenta[b]pyridin-4-amine (2.50 g, 16.9 mmol) in TFA (100 mL) at 0 °C was added NIS (7.61 g, 33.8 mmol) in portions at 0 °C. The resulting solution was stirred for 1 h at RT. The resulting mixture was concentrated under vacuum and then diluted with 250 mL of water. The resulting solution was extracted with 2x300 mL of EtOAc. The organic layers were combined, dried over anhydrous Na₂SO₄, and concentrated under vacuum. The residue was eluted from a silica gel column with EtOAc/PE (9:1). This resulted in 1.70 g (36.7%) of the title compound as a white solid. MS-ESI: 275 (M+1)

### Step 2: 2-Methyl-3-(trifluoromethyl)-6,7-dihydro-5H-cyclopenta[b]pyridin-4-amine

To a stirred solution of 3-iodo-2-methyl-6,7-dihydro-5H-cyclopenta[b]pyridin-4-amine (500 mg, 1.82 mmol) in DMF (10 mL) in a sealed tube under nitrogen was added (1,10-phenanthroline)(trifluoromethyl)copper(I) (2.27 g, 7.28 mmol). The resulting solution was stirred for 16 h at 80 °C. The solids were filtered out. The filtrate was purified by Prep-HPLC using the following conditions: Column: XBridge Shield RP18 OBD Column, 30*150 mm, 5 um; Mobile Phase A: water (10 mM NH₄HCO₃ + 0.1%NH₃-H₂O), Mobile Phase B: ACN; Flow rate: 60 mL/min; Gradient: 21% B to 35% B over 10 min; 254 nm; Rt: 9.27 min. This resulted in 140 mg (35.5%) of the title compound as a white solid. MS-ESI: 217 (M+1)

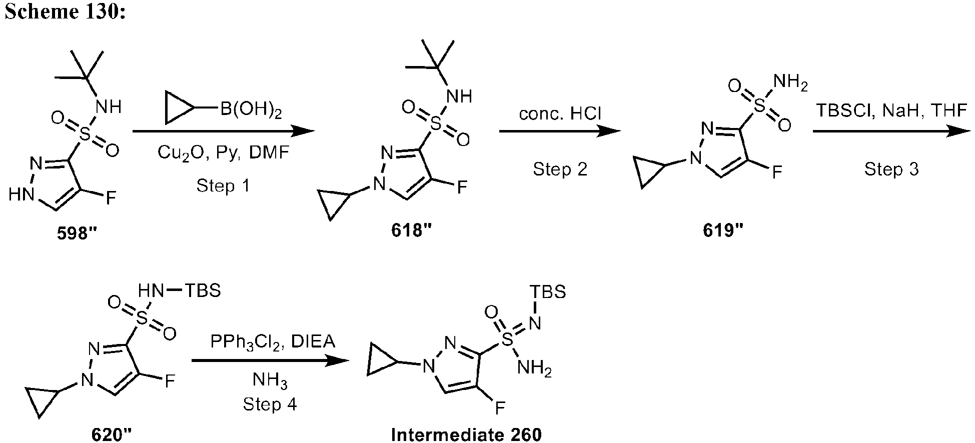

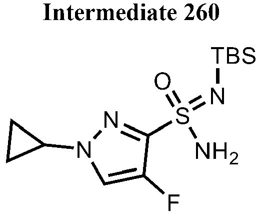

### N'-(tert-butyldimethylsilyl)-1-cyclopropyl-4-fluoro-1H-pyrazole-3-sulfonimidamide

### Step 1: N-(tert-butyl)-1-cyclopropyl-4-fluoro-1H-pyrazole-3-sulfonamide

To a stirred solution of N-(tert-butyl)-4-fluoro-1H-pyrazole-3-sulfonamide (3.0 g, 13.6 mmol) in DMF (100 mL) was added cyclopropylboronic acid (3.51 g, 40.8 mmol) and Cu₂O (1.93 g, 13.6 mmol) in portions at RT. To the above mixture was added pyridine (3.22 g, 40.8 mmol) dropwise at RT. The resulting mixture was stirred for 16 h at 70 °C under air. The resulting solution was filtered, the filter cake was washed with EtOAc (50 mL).The wash and filtrate was diluted with water (100 mL), and then extracted with EtOAc (3x100 mL). The organic layers were combined, dried over anhydrous Na₂SO₄, and concentrated under vacuum. The residue was eluted from silica gel column with EtOAc/PE (1:6). This resulted in the title compound (1.4 g, 39.5%) as a yellow solid. MS-ESI: 262 (M+1). ¹H NMR (400 MHz, DMSO-*d*₆) δ 8.14 (d, *J* = 4.7 Hz, 1H), 7.77 (s, 1H), 3.86-3.76 (m, 1H), 1.15 (s, 9H), 1.09-0.96 (m, 4H). Ar-H at 8.14 (d, *J* = 4.7 Hz, 1H) has correlations with cyclopropyl's CH₂ at 1.09-0.96 (m, 4H) and CH at 3.86-3.76 (m, 1H) in NOESY.

### Step 2: 1-Cyclopropyl-4-fluoro-1H-pyrazole-3-sulfonamide

To HCl (30 mL, conc.) was added N-(tert-butyl)-1-cyclopropyl-4-fluoro-1H-pyrazole-3-sulfonamide (1.40 g, 5.36 mmol) in portions at RT. The resulting solution was stirred for 1 h at RT. The resulting solution was concentrated under vacuum. This resulted in the title comopound (1.1 g, crude) as a brown solid. MS-ESI: 206 (M+1).

Steps 3-4 used similar procedures for converting compound **588"** to Intermediate **253** shown in Scheme **123** to afford Intermediate **260** from compound **619".** MS-ESI: 319 (M+1).

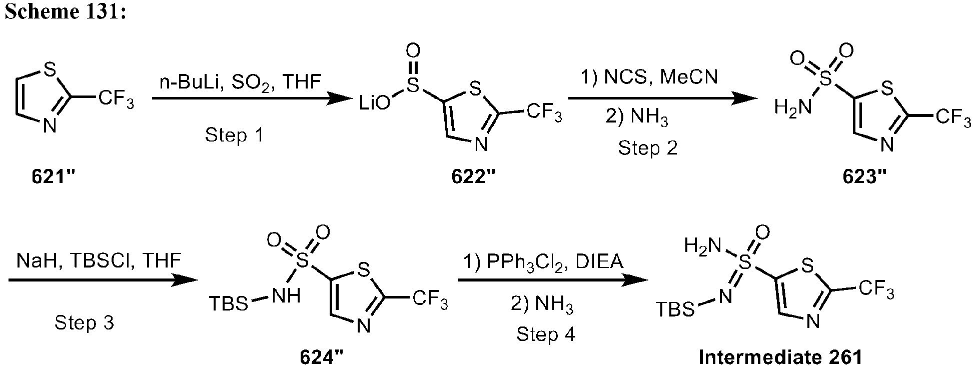

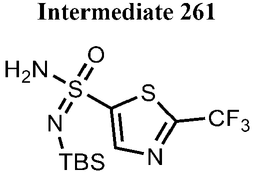

### N'-(tert-butyldimethylsilyl)-2-(trifluoromethyl)thiazole-5-sulfonimidamide

### Step 1: Lithium 2-(trifluoromethyl)thiazole-5-sulfinate

To a stirred solution of 2-(trifluoromethyl)thiazole (500 mg, 3.27 mmol) in THF (10 mL) under nitrogen was added n-BuLi (2.5 M in hexane, 3.3 mL, 8.17 mmol) dropwise at -78 °C. The resulting solution was stirred for 20 min at -78 °C. To the above solution was introduced SO₂ (g) bubbled at -78 °C for 5 min. The resulting mixture was stirred for 2 h at RT and concentrated under vacuum. This resulted in 1.5 g (crude) of the title compound as a yellow solid which was used for next step without further purification. MS-ESI: 216 (M-1).

### Step 2: 2-(Trifluoromethyl)thiazole-5-sulfonamide

To a stirred solution of lithium 2-(trifluoromethyl)thiazole-5-sulfinate (1.5 g, crude from last step) in ACN (10 mL) was added NCS (4.38 g, 32.7 mmol) in portions at 0 °C. The resulting solution was stirred for 2 h at RT. To the above solution was introduced NH₃ (g) bubbled at 0 °C for 5 min. The resulting solution was stirred for 2 h at RT. The resulting mixture was concentrated under vacuum. The mixture was dissolved EtOAc (200 mL) then washed with water (2x50 mL). The organic layer was dried over anhydrous Na₂SO₄ and concentrated under vacuum. The residue was purified by Prep-TLC with EtOAc/PE (1:1). This resulted in 160 mg (21.1% over two steps) of the title compound as a light yellow solid. MS-ESI: 231 (M-1). ¹H NMR (400 MHz, CDCl₃) δ 8.40 (s, 1H), 5.32 (s, 2H).

Steps 3-4 used similar procedures for converting compound **593"** to Intermediate **254** shown in Scheme **124** to afford Intermediate **261** from compound **623".** MS-ESI: 346 (M+1).

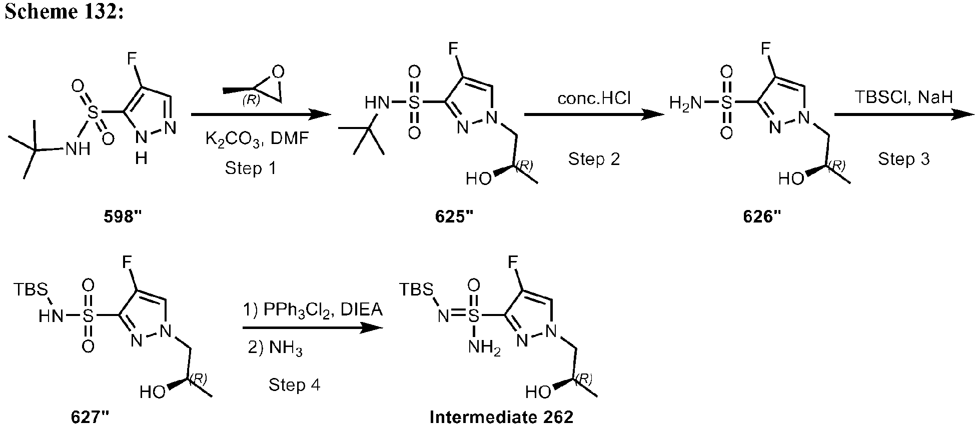

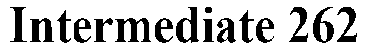

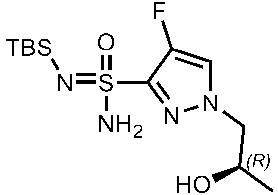

### N'-(tert-butyldimethylsilyl)-4-fluoro-1-((R)-2-hydroxypropyl)-1H-pyrazole-3-sulfonimidamide

### Step 1: (R)-N-(tert-butyl)-4-fluoro-1-(2-hydroxypropyl)-1H-pyrazole-3-sulfonamide

To a stirred solution of N-(tert-butyl)-4-fluoro-1H-pyrazole-5-sulfonamide (1.0 g, 4.52 mmol) in DMF (20 mL) under nitrogen was added K₂CO₃ (1.87 g, 13.56 mmol) and (R)-2-methyloxirane (524 mg, 9.04 mmol). The resulting solution was stirred for 16 h at 100 °C. The reaction was quenched with 25 mL of water, and extracted with 3x30 mL of EtOAc. The organic layers were combined, dried over anhydrous Na₂SO₄ and concentrated under vacuum. The residue was eluted from a silica gel column with EtOAc/PE (1:3). This resulted in 820 mg (65.0%) of the title compound as a yellow solid. MS-ESI: 280 (M+1).

### Step 2: (R)-4-fluoro-1-(2-hydroxypropyl)-1H-pyrazole-3-sulfonamide

To stirred concentrated HCl (10 mL) was added (R)-N-(tert-butyl)-4-fluoro-1-(2-hydroxypropyl)-1H- pyrazole-3-sulfonamide (820 mg, 2.94 mmol) in portions at 0 °C. The resulting solution was stirred for 2 h at RT and then concentrated under vacuum. The residue was eluted from a silica gel column with EtOAc (100%). This resulted in 600 mg (91.5%) of the title compound as yellow oil. MS-ESI: 224 (M+1). ¹H NMR (400 MHz, DMSO-*d*₆) δ 7.98 (d, *J =* 4.7 Hz, 1H), 7.68 (s, 2H), 5.03 (d, *J* = 4.3 Hz, 1H), 4.09-3.90 (m, 3H), 1.07 (d, *J =* 5.8 Hz, 3H). Ar-H at 7.98 (d, *J* = 4.7 Hz, 1H) has correlation with CH₂ at 4.09-3.90 (m, 3H) in NOESY.

Steps 3-4 used similar procedures for converting compound **588"** to Intermediate **253** shown in Scheme **123** to afford Intermediate **262** from compound **626".** MS-ESI: 337 (M+1). ¹H NMR (400 MHz, DMSO-*d*₆) δ 7.87 (d, *J =* 4.8 Hz, 1H), 6.86 (s, 2H), 5.00 (br s, 1H), 4.07-3.89 (m, 3H), 1.10-1.02 (m, 3H), 0.86 (s, 9H), 0.04 (s, 6H).

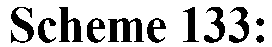

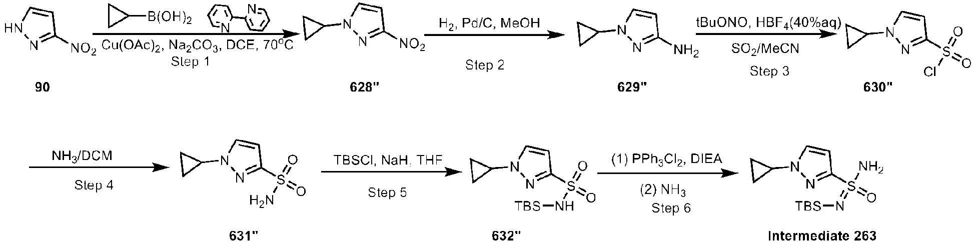

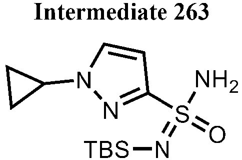

### N'-(tert-butyldimethylsilyl)-1-cyclopropyl-1H-pyrazole-3-sulfonimidamide

### Step 1: 1-Cyclopropyl-3-nitro-1H-pyrazole

To a stirred solution of 3-nitro-1H-pyrazole (20.0 g, 177 mmol) and cyclopropylboronic acid (30.4 g, 353 mmol) in DCE (500 mL) was added 2,2'-bipyridine (27.6 g, 177 mmol) dropwise at RT. To the above solution were added Na₂CO₃ (18.8 g, 177 mmol) and Cu(OAc)₂ (32.1 g, 177 mmol) in portions at RT. The resulting mixture was stirred for 16 h at 70 °C under air. The mixture was allowed to cool down to RT then filtered. The filter cake was washed with EtOAc (100 mL). The filtrate and wash were combined and concentrated under vacuum. The residue was diluted with water (500 mL), extracted with EtOAc (3x500 mL). The combined organic layers were dried over anhydrous Na₂SO₄. After filtration, the filtrate was concentrated under vacuum. The residue was eluted from silica gel column with EtOAc/PE (1:10). This resulted in the title compound (12.0 g, 44.3%) as yellow oil. ¹H NMR (400 MHz, DMSO-*d*₆) δ 8.13 (d, *J =* 2.5 Hz, 1H), 7.05 (d, *J* = 2.6 Hz, 1H), 3.96 (tt, *J* = 7.5, 3.9 Hz, 1H), 1.21-1.13 (m, 2H), 1.13-1.03 (m, 2H). Cyclopropane's CH at 3.96 (tt, *J =* 7.5, 3.9 Hz, 1H) has correlation with Ar-H at 8.13 (d, *J =* 2.5 Hz, 1H) in NOESY.

### Step 2: 1-Cyclopropyl-1H-pyrazol-3-amine

To a stirred solution of 1-cyclopropyl-3-nitro-1H-pyrazole (12.0 g, 78.4 mmol) in MeOH (50 mL) was added Pd/C (10% wt., 120 mg) in portions at RT under nitrogen. The mixture was stirred for 16 h at RT under hydrogen with a hydrogen balloon, then filtered through a Celite pad and concentrated under vacuum. This resulted in the title compound (8.68 g, 90%) as a light yellow solid. MS-ESI: 124 (M+1).

### Step 3: 1-Cyclopropyl-1H-pyrazole-3-sulfonyl chloride

To a stirred solution of 1-cyclopropyl-1H-pyrazol-3-amine (1.0 g, 8.13 mmol) in ACN (10 mL) under nitrogen was added HBF₄ (40%wt. in water, 2.68 g, 12.2 mmol) dropwise at 0 °C. To the above solution was added tert-butyl nitrite (1.26 g, 12.2 mmol) dropwise with stirring at 0 °C. The resulting solution was stirred for 1 h at 0 °C. This solution was assigned as solution A. To a stirred solution of CuCl (2.41 g, 24.4 mmol) in ACN (5 mL) was introduced SO₂ (g) bubbled for 10 min at 0 °C, this solution was assigned as solution B. To solution B was added solution A dropwise with stirring at 0 °C over 5 min. The resulting solution was stirred for 10 min at 0 °C, then allowed to react, with stirring, for an additional 3 h at RT. The reaction was quenched with water/ice (10 mL), and then extracted with DCM (2x30 mL). The organic layers were combined and washed with brine (30 mL). The organic layer was dried over anhydrous Na₂SO₄ and concentrated under vacuum. This resulted in the title compound (1.5 g, crude) as yellow oil which was used in next step without further purification.

### Step 4: 1-Cyclopropyl-1H-pyrazole-3-sulfonamide

To a stirred DCM (25 mL) was bubbled NH₃ (g) for 15 min at 0 °C, this was followed by the addition of 1-cyclopropyl-1H-pyrazole-3-sulfonyl chloride (1.50 g, crude from last step) in DCM (10 mL) dropwise at 0 °C. The resulting solution was stirred for 1 h at RT and then concentrated under vacuum. The residue was eluted from silica gel column with EtOAc/PE (1:1). This resulted in the title compound (250 mg, 16.4% over two steps) as a white solid. MS-ESI: 186 (M-1).

Steps 5-6 used similar procedures for converting compound **588"** to Intermediate **253** shown in Scheme **123** to afford Intermediate **263** from compound **631".** MS-ESI: 301 (M+1).

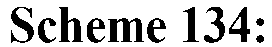

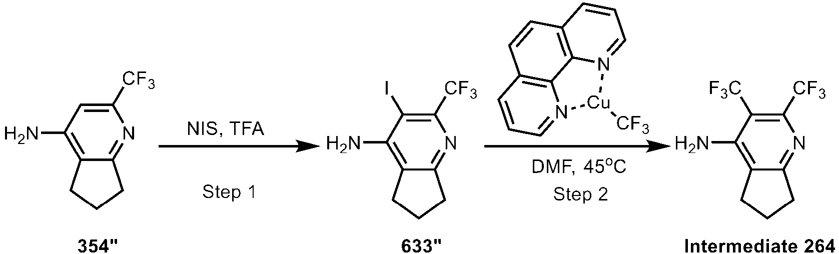

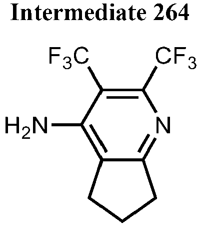

### 2,3-Bis(trifluoromethyl)-6,7-dihydro-5H-cyclopenta[b]pyridin-4-amine

### Step 1: 3-Iodo-2-(trifluoromethyl)-6,7-dihydro-5H-cyclopenta[b]pyridin-4-amine

To a stirred solution of 2-(trifluoromethyl)-6,7-dihydro-5H-cyclopenta[b]pyridin-4-amine (2.0 g, 9.89 mmol) in TFA (20 mL) under nitrogen was added NIS (3.34 g, 14.8 mmol) in portions at 0 °C. The resulting solution was stirred for 16 h at RT. The resulting mixture was concentrated under vacuum. The residue was diluted with 50 mL of water, and then extracted with 3x80 mL of EtOAc. The combined organic layers were dried over anhydrous sodium sulfate and concentrated under vacuum. The residue was eluted from silica gel column with EtOAc/PE (9:1). This resulted in 2 g (61.6%) of the title compound as a brown solid. MS-ESI: 329 (M+1).

### Step 2: 2,3-Bis(trifluoromethyl)-6,7-dihydro-5H-cyclopenta[b]pyridin-4-amine

To a stirred solution of 3-iodo-2-(trifluoromethyl)-6,7-dihydro-5H-cyclopenta[b]pyridin-4-amine (450 mg, 1.37 mmol) in DMF (5 mL) in a sealed tube under nitrogen was added (1,10-phenanthroline) (trifluoromethyl)copper(I) (1.72 g, 5.48 mmol). The resulting solution was stirred for 16 h at 45 °C. The solids were filtered out. The filtrate was purified by Prep-HPLC using the following conditions: Column: XBridge Shield RP18 OBD Column, 30*150 mm, 5 um; Mobile Phase A: Water (10 mM NH₄HCO₃ + 0.1% NH₃·H₂O), Mobile Phase B: ACN; Flow rate: 60 mL/min; Gradient: 20% B to 35% B over 10 min; UV 254 nm; RT: 9.14 min. This resulted in 150 mg (40.4%) of the title compound as a dark yellow solid. MS-ESI: 271 (M+1).

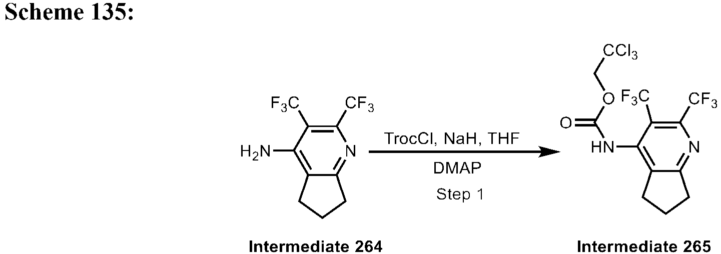

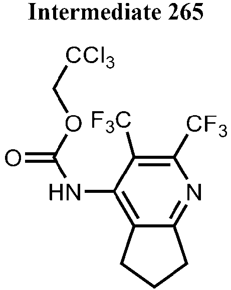

### (2,3-Bis(trifluoromethyl)-6,7-dihydro-5H-cyclopenta[b]pyridin-4-yl)carbamate

To a stirred solution of 2,3-bis(trifluoromethyl)-6,7-dihydro-5H-cyclopenta[b]pyridin-4-amine (125 mg, 0.46 mmol) in THF (15 mL) under nitrogen was added NaH (60%wt., 56 mg, 1.39 mmol) in portions at 0 °C. To this was added DMAP (28 mg, 0.23 mmol) at 0 °C. The resulting solution was stirred for 20 min at 35 °C. To the above solution was added 2,2,2-trichloroethyl carbonochloridate (1.96 g, 9.25 mmol) dropwise with stirring at 0 °C. The resulting solution was stirred for 3 days at 35 °C. The reaction was then quenched with 30 mL of water/ice. The resulting solution was extracted with 3x40 mL of EtOAc. The combined organic layers were dried over anhydrous sodium sulfate and concentrated under vacuum. The residue was eluted from a silica gel column with EtOAc/PE (1:9). This resulted in 13 mg (6.2%) of the title compound as a yellow solid. MS-ESI: 445/447/449 (M+1).

### Example 1 (Compound 165)

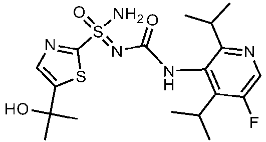

### N'-((5-fluoro-2,4-diisopropylpyridin-3-yl)carbamoyl)-5-(2-hydroxypropan-2-yl)thiazole-2-sulfonimidamide (Scheme 1)

### Examples 2 and 3 (Compounds 165a and 165b)

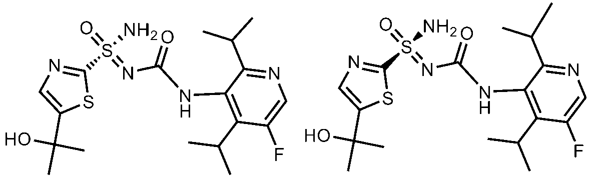

### Examples 2 and 3 (stereochemistry arbitrarily assigned)

### (R)- and (S)- N'-((5-fluoro-2,4-diisopropylpyridin-3-yl)carbamoyl)-5-(2-hydroxypropan-2-yl)thiazole-2-sulfonimidamide

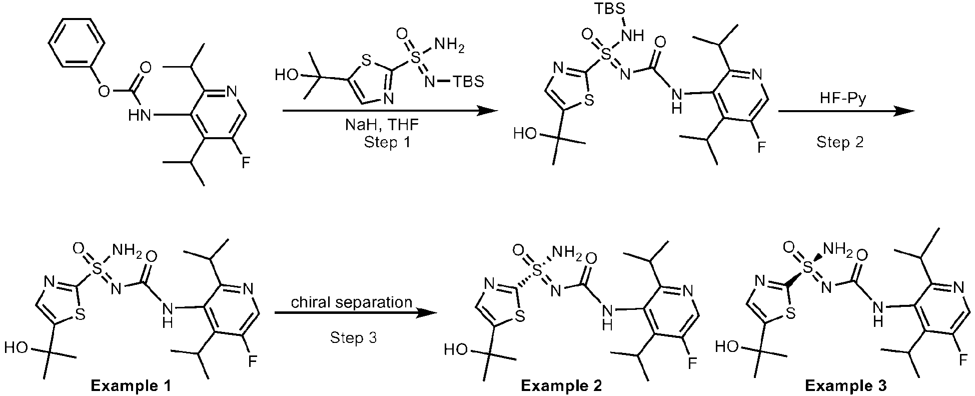

### Step 1: N-(tert-butyldimethylsilyl)-N'-((5-fluoro-2,4-diisopropylpyridin-3-yl)carbamoyl)-5-(2-hydroxy- propan-2-yl)thiazole-2-sulfonimidamide

Into a 50-mL round-bottom flask purged and maintained with an inert atmosphere of nitrogen, was placed N'-(tert-butyldimethylsilyl)-5-(2-hydroxypropan-2-yl)thiazole-2-sulfonimidamide (206 mg, 0.61 mmol) in THF (10 mL). To the above solution was added NaH (60% wt. oil dispersion, 73.6 mg, 1.84 mmol). The resulting solution was stirred for 10 min at RT. Then a solution of phenyl (5-fluoro-2,4-diisopropylpyridin-3-yl)carbamate (194 mg, 0.61 mmol) in THF (10 mL) was added to the above solution. The resulting solution was allowed to react, with stirring, for an additional 2 h at RT. The reaction was then quenched by the addition of 10 mL of water. The resulting solution was extracted with 5x20 ml of EtOAc. The organic phases were combined and concentrated. This resulted in 360 mg crude title compound as light yellow oil. MS-ESI: 558(M+1).

### Step 2: N'-((5-fluoro-2,4-diisopropylpyridin-3-yl)carbamoyl)-5-(2-hydroxypropan-2-yl)thiazole-2- sulfonimidamide

Into a 50-mL round-bottom flask, was placed N-(tert-butyldimethylsilyl)-N'-((5-fluoro-2,4-diisopropyl- pyridin-3-yl)carbamoyl)-5-(2-hydroxypropan-2-yl)thiazole-2-sulfonimidamide (360 mg, crude) in THF (5 mL) and HF pyridine (0.10 mL). The resulting solution was stirred for 2 h at RT. The resulting mixture was concentrated. The crude product was purified by Prep-HPLC with the following conditions: Column, XBridge BEH130 Prep C18 OBD, 19*150 mm 5 um; mobile phase, water (10 mM NH₄HCO₃) and ACN (30% to 60% gradient over 7 min); Detector, 254/210 nm. This resulted in 70 mg of **Example 1** as an off-white solid. MS-ESI: 444 (M+1). ¹H NMR (400 MHz, DMSO-*d*₆) δ 8.59 (s, 1H), 8.27 (s, 1H), 7.76 (br s, 3H), 5.82 (s, 1H), 3.25-3.05 (m, 2H), 1.53 (s, 6H), 1.18 (d, *J* = 6.9 Hz, 6H), 1.08 (d, *J =* 6.8 Hz, 6H).

### Step 3: Chiral resolution

The product (100 mg, Example 1) was separated with the followed condition: Column: CHIRALPAK ID, 2*25 cm (5 um); Mobile Phase A: HPLC grade Hex (0.1% FA), Phase B: HPLC grade EtOH; Flow rate: 20 mL/min; Gradient: 15% B to 15% B in 18 min; 220/254 nm; Rt1: 9.555 min; Rt2: 14.358 min. This resulted in 24.6 mg (97.9%ee, 49.2%) of **Example 2** (fast-eluting) as a white solid and 22.8 mg (98.9%ee, 45.6%) of **Example 3** as a white solid. The absolute stereochemistry was unconfirmed.

**Example 2:** MS-ESI: 444 (M+1). ¹H NMR (400 MHz, DMSO-*d*₆) δ 8.59 (s, 1H), 8.27 (s, 1H), 7.76 (br s, 3H), 5.82 (s, 1H), 3.25-3.05 (m, 2H), 1.53 (s, 6H), 1.18 (d, *J* = 6.9 Hz, 6H), 1.08 (d, *J* = 6.8 Hz, 6H).

**Example 3:** MS-ESI: 444 (M+1). ¹H NMR (400 MHz, DMSO-*d*₆) δ 8.59 (s, 1H), 8.27 (s, 1H), 7.76 (br s, 3H), 5.82 (s, 1H), 3.25-3.05 (m, 2H), 1.53 (s, 6H), 1.18 (d, *J* = 6.9 Hz, 6H), 1.08 (d, *J* = 6.8 Hz, 6H).

**Table 15. Examples in the following table were prepared using similar conditions as described in Example 1 and Scheme 1 from appropriate starting materials.**

| Example # | Compound Number | Structure | IUPAC Name | Exact Mass [M+H]⁺ |
|---|---|---|---|---|
| 4 | 163 | 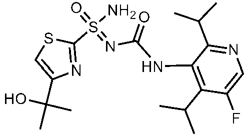 | N'-((5-fluoro-2,4-diisopropylpyridin-3-yl)carbamoyl)-4-(2-hydroxypropan-2-yl)thiazole-2-sulfonimidamide | 444 |
| 5 | 128 | 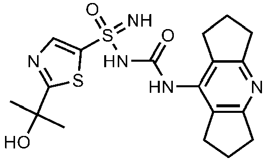 | N-((1,2,3,5,6,7-hexahydrodicyclopenta[b,e]py ridin-8-yl)carbamoyl)-2-(2-hydroxypropan-2-yl)thiazole-5-sulfonimidamide | 422 |
| 6 | 110 | 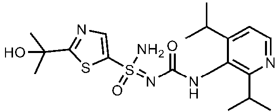 | N'-((2,4-diisopropylpyridin-3-yl)carbamoyl)-2-(2-hydroxypropan-2-yl)thiazole-5-sulfonimidamide | 426 |

### Example 7 (Compound 164)

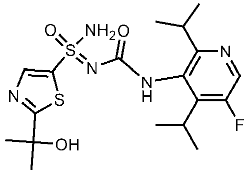

### N'-((5-fluoro-2,4-diisopropylpyridin-3-yl)carbamoyl)-2-(2-hydroxypropan-2-yl)thiazole-5-sulfonimidamide (Scheme 1A)

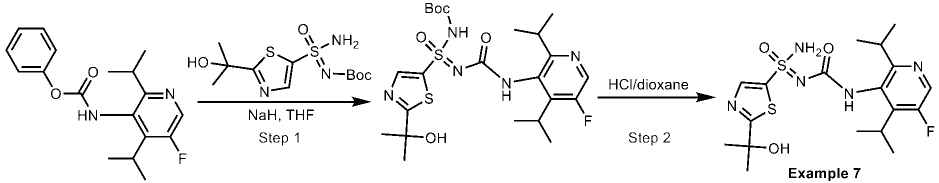

### Step 1: Tert-butyl (N-((5-fluoro-2,4-diisopropylpyridin-3-yl) carbamoyl)-2-(2-hydroxypropan-2-yl) thiazole-5-sulfonimidoyl)carbamate

Into a 50-mL round-bottom flask purged and maintained with an inert atmosphere of nitrogen, was placed tert-butyl (amino(2-(2-hydroxypropan-2-yl)thiazol-5-yl)(oxo)-λ6-sulfaneylidene)carbamate (327 mg, 1.02 mmol) in THF (10 mL). To the stirred solution was added NaH (60% wt. oil dispersion, 122 mg, 3.05 mmol). The resulting solution was stirred for 10 min at RT. Then a solution of phenyl N-[5-fluoro-2,4-bis(propan-2-yl)pyridin-3-yl]carbamate (322 mg, 1.02 mmol) in THF (10 mL) was added. The resulting solution was allowed to react, with stirring, for an additional 2 h at RT. The reaction was then quenched by the addition of 3 mL of water. The resulting mixture was concentrated. This resulted in 500 mg crude title compound as yellow oil. MS-ESI: 544 (M+1).

### Step 2: N'-((5-fluoro-2,4-diisopropylpyridin-3-yl)carbamoyl)-2-(2-hydroxypropan-2-yl)thiazole-5- sulfonimidamide

Into a 50-mL round-bottom flask, was placed tert-butyl (N-((5-fluoro-2,4-diisopropylpyridin-3-yl)-carba- moyl)-2-(2-hydroxypropan-2-yl)thiazole-5-sulfonimidoyl)carbamate (500 mg crude) in HCl(4M)/dioxane (20 mL). The resulting solution was stirred for 2 h at RT. The resulting mixture was concentrated. The crude product was purified by Prep-HPLC with the following conditions: Column, XBridge Prep C18 OBD, 5 um, 19*150 mm; mobile phase, water (10 mM NH₄HCO₃) and ACN (5% to 41% gradient in 6 min); Detector, 254/210 nm. This resulted in 90 mg of **Example 7** as an off-white solid. MS-ESI: 444 (M+1). ¹H NMR (400 MHz, DMSO-*d*₆) δ 8.60 (s, 1H), 8.29 (s, 1H), 8.06 (s, 1H), 7.86 (s, 2H), 6.26 (s, 1H), 3.29-3.05 (m, 2H), 1.49 (s, 6H), 1.20-0.99 (m, 12H).

**Table 16. Examples in the following table were prepared using similar conditions as described in Example 7 and Scheme 1A from appropriate starting materials.**

| Example # | Final Target Number | Structure | IUPAC Name | Exact Mass [M+H]⁺ |
|---|---|---|---|---|
| 8 | 167 | 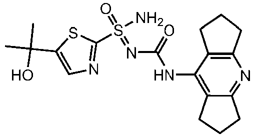 | N'-((1,2,3,5,6,7-hexahydrodicyclopenta[b, e]pyridin-8-yl)carbamoyl)-5-(2-hydroxypropan-2-yl)thiazole-2-sulfonimidamide | 422 |

### Example 9 (Compound 159)

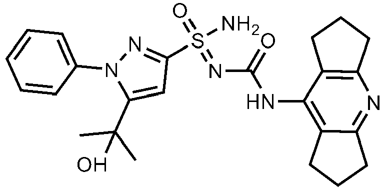

### N'-((1,2,3,5,6,7-hexahydrodicyclopenta[b,e]pyridin-8-yl)carbamoyl)-5-(2-hydroxypropan-2-yl)-1-phenyl-1H-pyrazole-3-sulfonimidamide (scheme 2)

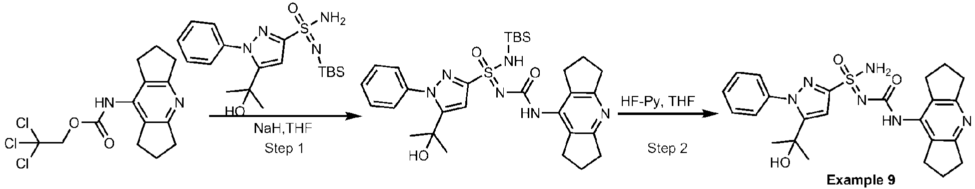

### Step 1: N-(tert-butyldimethylsilyl)-N'-((1,2,3,5,6,7-hexahydrodicyclopenta[b,e]pyridin-8-yl)carbamoyl)-5-(2-hydroxypropan-2-yl)-1-phenyl-1H-pyrazole-3-sulfonimidamide

Into a 50-mL round-bottom flask purged and maintained with an inert atmosphere of nitrogen, was placed *N*-(*tert*-butyldimethylsilyl)-5-(2-hydroxypropan-2-yl)-1-phenyl-1H-pyrazole-3-sulfonoimidamide (160 mg, 0.41 mmol) in THF (10 mL). To the stirred solution was added NaH (60% wt. oil dispersion, 48.7 mg, 1.22 mmol) at 0°C. The resulting solution was stirred for 10 min at RT. Then 2,2,2-trichloroethyl (1,2,3,5,6,7-hexahydrodicyclopenta[b,e]pyridin-8-yl)carbamate (142 mg, 0.41 mmol) was added to the reaction solution. The resulting solution was allowed to react with stirring for an additional 2 h while the temperature was maintained at 40°C in an oil bath. The reaction was then quenched by the addition of 10 mL of H₂O. The resulting solution was extracted with 5x20 mL of EtOAc and the organic layers was combined and concentrated. The residue was purified using TLC with DCM/MeOH = 10:1. This resulted in 230 mg (95.4%) of the title compound as a light yellow solid. MS-ESI: 595 (M+1).

### Step 2: N'-((1,2,3,5,6,7-hexahydrodicyclopenta[b,e]pyridin-8-yl)carbamoyl)-5-(2-hydroxypropan-2-yl)-1-phenyl-1H-pyrazole-3-sulfonimidamide

Into a 50-mL round-bottom flask, was placed N-(tert-butyldimethylsilyl)-N'- ((1,2,3,5,6,7-hexahydrodicyclopenta[b,e]pyridin-8-yl)carbamoyl)-5-(2-hydroxypropan-2-yl)-1-phenyl-1H-pyrazole-3-sulfonimidamide (230 mg, 0.39 mmol) in THF (8 mL). To the above solution was added HF-Pyridine (0.1 mL) dropwise. The resulting solution was stirred for 30 min at RT. The resulting mixture was concentrated. The crude product was purified by Prep-HPLC with the following conditions: Column, XBridge Prep C18 OBD, 19*150 mm 5 um; mobile phase, water (10 mM NH₄HCO₃+0.1%NH₃·H₂O) and ACN (10% to 54% gradient over 6 min); Detector, UV, 210/254 nm. This resulted in 102 mg (53.8%) of **Example 9** as an off-white solid. **MS-ESI:** 481 (M+1). ¹H NMR (400 MHz, DMSO-*d*₆) δ 8.74 (s, 1H), 7.58 (s, 2H), 7.52 (s, 5H), 6.75 (s, 1H), 5.43 (s, 1H), 2.79 (t, *J* = 7.6 Hz, 4H), 2.71 (t, *J* = 7.5 Hz, 4H), 2.00-1.80 (m, 4H), 1.34 (s, 6H).

**Table 17. Examples in the following table were prepared using similar conditions as described in Example 9 and Scheme 2 from appropriate starting materials.**

| Example # | Compound Number | Structure | IUPAC Name | Exact Mass [M+H]⁺ |
|---|---|---|---|---|
| 10 | 135 | 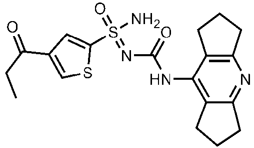 | N'-((1,2,3,5,6,7-hexahydrodicyclopenta[b,e]pyridin -8-yl)carbamoyl)-4-propionylthiophene-2-sulfonimidamide | 419 |
| 11 | 119 | 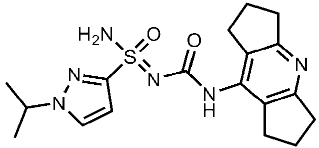 | N'-((1,2,3,5,6,7-hexahydrodicyclopenta[b,e]pyridin -8-yl)carbamoyl)-1-isopropyl-1H-pyrazole-3-sulfonimidamide | 389 |
| 12 | 122 | 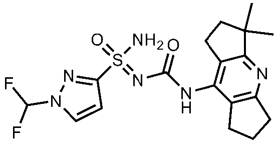 | 1-(difluoromethyl)-N'-((3,3-dimethyl-1,2,3,5,6,7-hexahydrodicyclopenta[b,e]pyridin -8-yl)carbamoyl)-1H-pyrazole-3-sulfonimidamide | 425 |
| 13 | 125 | 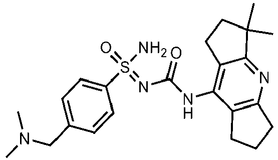 | N'-((3,3-dimethyl-1,2,3,5,6,7-hexahydrodicyclopenta[b,e]pyridin -8-yl)carbamoyl)-4-((dimethylamino)methyl)benzenes ulfonimidamide | 442 |
| 14 | 126 | 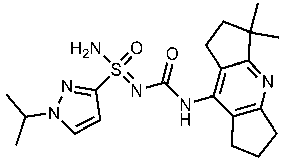 | N'-((3,3-dimethyl-1,2,3,5,6,7-hexahydrodicyclopenta[b,e]pyridin -8-yl)carbamoyl)-1-isopropyl-1H-pyrazole-3-sulfonimidamide | 417 |
| 15 | 101 | 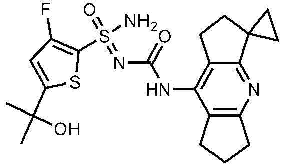 | 3-fluoro-5-(2-hydroxypropan-2-yl)-N'-((1',5',6',7'-tetrahydro-2'H-spiro [cyclopropane- 1,3'-dicyclopenta[b,e]pyridin]-8'-yl)carbamoyl)thiophene-2-sulfonimidamide | 465 |
| 16 | 114 | 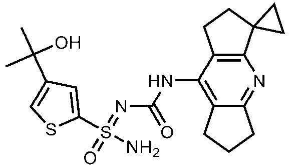 | 4-(2-hydroxypropan-2-yl)-N'-((1',5',6',7'-tetrahydro-2'H-spiro [cyclopropane- 1,3'-dicyclopenta[b,e]pyridin]-8'-yl)carbamoyl)thiophene-2-sulfonimidamide | 447 |
| 17 | 117 | 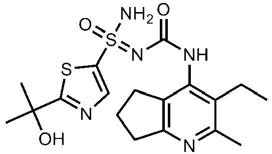 | N'-((3-ethyl-2-methyl-6,7-dihydro-5H-cyclopenta[b]pyridin-4-yl)carbamoyl)-2-(2-hydroxypropan-2-yl)thiazole-5-sulfonimidamide | 424 |
| 18 | 116 | 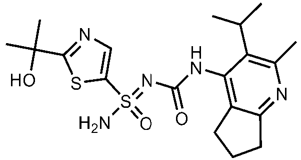 | 2-(2-hydroxypropan-2-yl)-N'-((3-isopropyl-2-methyl-6,7-dihydro-SH-cyclopenta[b]pyridin-4-yl)carbamoyl)thiazole-5-sulfonimidamide | 438 |
| 19 | 130 | 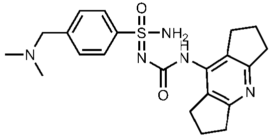 | 4-((dimethylamino)methyl)-N'-((1,2,3,5,6,7-hexahydrodicyclopenta[b,e]pyridin -8-yl)carbamoyl)benzenesulfonimida mide | 414 |
| 20 | 170 | 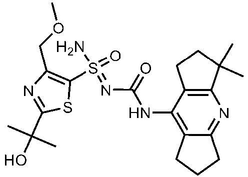 | N'-((3,3-dimethyl-1,2,3,5,6,7-hexahydrodicyclopenta[b,e]pyridin -8-yl)carbamoyl)-2-(2-hydroxypropan-2-yl)-4-(methoxymethyl)thiazole-5-sulfonimidamide | 494 |
| 21 | 171 | 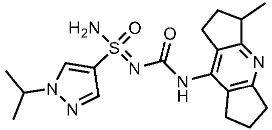 | 1-isopropyl-N'-((3-methyl-1,2,3,5,6,7-hexahydrodicyclopenta[b,e]pyridin -8-yl)carbamoyl)-1H-pyrazole-4-sulfonimidamide | 403 |
| 22 | 172 | 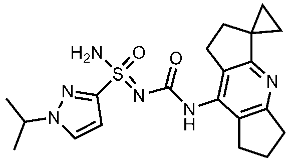 | 1-isopropyl-N'-((1',5',6',7'-tetrahydro-2'H-spiro [cyclopropane- 1,3'-dicyclopenta[b,e]pyridin]-8'-yl)carbamoyl)-1H-pyrazole-3-sulfonimidamide | 415 |
| 23 | 173 | 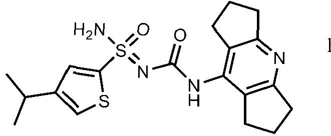 | N'-((1,2,3,5,6,7-hexahydrodicyclopenta[b,e]pyridin -8-yl)carbamoyl)-4-isopropylthiophene-2-sulfonimidamide | 405 |
| 24 | 174 | 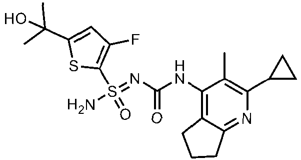 | N'-((2-cyclopropyl-3-methyl-6,7-dihydro-5H-cyclopenta[b]pyridin-4-yl)carbamoyl)-3-fluoro-5-(2-hydroxypropan-2-yl)thiophene-2-sulfonimidamide | 453 |
| 25 | 175 | 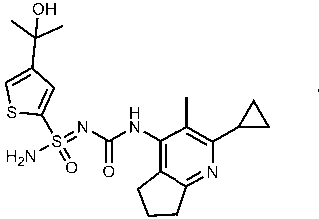 | N'-((2-cyclopropyl-3-methyl-6,7-dihydro-5H-cyclopenta[b]pyridin-4-yl)carbamoyl)-4-(2-hydroxypropan-2-yl)thiophene-2-sulfonimidamide | 435 |
| 26 | 176 | 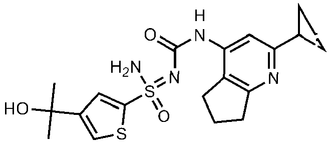 | N'-((2-cyclopropyl-6,7-dihydro-5H-cyclopenta[b]pyridin-4-yl)carbamoyl)-4-(2-hydroxypropan-2-yl)thiophene-2-sulfonimidamide | 421 |
| 27 | 177 | 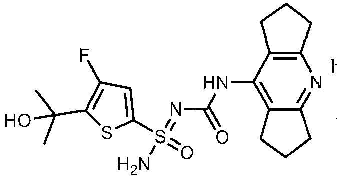 | 4-fluoro-N'-((1,2,3,5,6,7-hexahydrodicyclopenta[b,e]pyridin -8-yl)carbamoyl)-5-(2-hydroxypropan-2-yl)thiophene-2-sulfonimidamide | 439 |
| 33 | 111 | 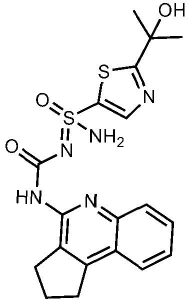 | N'-((2,3-dihydro-1H-cyclopenta[c]quinolin-4-yl)carbamoyl)-2-(2-hydroxypropan-2-yl)thiazole-5-sulfonimidamide | 432 |

### Example 28 (Compound 118)

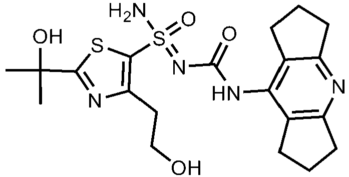

### N'-((1,2,3,5,6,7-hexahydrodicyclopenta[b,e]pyridin-8-yl)carbamoyl)-4-(2-hydroxyethyl)-2-(2-hydroxypropan-2-yl)thiazole-5-sulfonimidamide (scheme 2A)

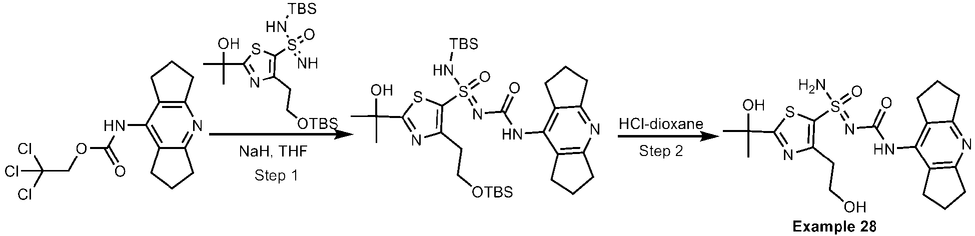

### Step 1: N-(tert-butyldimethylsilyl)-4-(2-((tert-butyldimethylsilyl)oxy)ethyl)-N'-((1,2,3,5,6,7-hexahy drodicyclopenta[b,e]pyridin-8-yl)carbamoyl)-2-(2-hydroxypropan-2-yl)thiazole-5-sulfonimidamide

Into a 100-mL round-bottom flask, was placed N-(tert-butyldimethylsilyl)-4-(2-((tert-butyldimethyl- silyl)oxy)ethyl)-2-(2-hydroxypropan-2-yl)thiazole-5-sulfonimidamide (200 mg, 0.41 mmol) in THF (15 mL). To the above solution was added NaH (60%wt. oil dispersion, 32.8 mg, 0.82 mmol), then 2,2,2-trichloroethyl (1,2,3,5,6,7-hexahydrodicyclopenta[b,e]pyridin-8-yl)carbamate (142 mg, 0.41 mmol) was added into the reaction solution. The resulting solution was stirred for 30 min at RT. The reaction was then quenched by the addition of 5.0 mL of water. The resulting solution was extracted with 3x15 mL of EtOAc, and the organic layers were combined and dried over anhydrous sodium sulfate and concentrated. The residue was eluted from silica gel with DCM/MeOH (10:1). This resulted in 210 mg (75%) of the title compound as yellow oil. MS-ESI: 694 (M+1).

### Step 2: N'-((1,2,3,5,6,7-hexahydrodicyclopenta[b,e]pyridin-8-yl)carbamoyl)-4-(2-hydroxyethyl)-2-(2-hydroxypropan-2-yl)thiazole-5-sulfonimidamide

Into a 100-mL round-bottom flask, was placed N-(tert-butyldimethylsilyl)-4-(2-((tert-butyldimethyl- silyl)oxy)ethyl)-N'-((1,2,3,5,6,7-hexahydrodicyclopenta[b,e]pyridin-8-yl)carbamoyl)-2-(2-hydroxypropan-2-yl)thiazole-5-sulfonimidamide (200 mg, 0.29 mmol) in HCl in dioxane (10 mL, 4 M). The resulting solution was stirred for 30 min at RT. The resulting mixture was concentrated. The crude product was purified by Prep-HPLC with the following conditions: Column, XBridge Prep OBD C18, 30*150 mm 5 um; mobile phase, water (10 mM NH₄HCO₃) and ACN (4% to 28% gradient in 5 min); Detector, UV 254/220nm. This resulted in 90 mg (67%) of **Example 28** as a white solid. MS-ESI: 466 (M+1). ¹H NMR (400 MHz, DMSO-*d*₆) δ 8.73 (s, 1H), 7.76 (br s, 2H), 6.19 (s, 1H), 4.90-4.70 (br s, 1H), 3.80-3.60 (m, 2H), 3.20-3.00 (m, 2H), 2.90-2.60 (m, 8H), 2.10-1.80 (m, 4H), 1.48 (d, J = 6.0 Hz, 6H).

**Table 18. Examples in the following table were prepared using similar conditions as described in Example 28 and Scheme 2A from appropriate starting materials.**

| Example # | Compound Number | Structure | IUPAC Name | Exact Mass [M+H]⁺ |
|---|---|---|---|---|
| 29 | 150 | 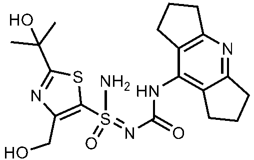 | N'-((1,2,3,5,6,7-hexahydrodicyclopenta[b,e]pyridin-8-yl)carbamoyl)-4-(hydroxymethyl)-2-(2-hydroxypropan-2-yl)thiazole-5-sulfonimidamide | 452 |
| 30 | 121 | 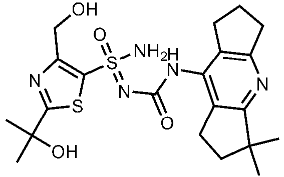 | N'-((3,3-dimethyl-1,2,3,5,6,7-hexahydrodicyclopenta[b,e]pyridin-8-yl)carbamoyl)-4-(hydroxymethyl)-2-(2-hydroxypropan-2-yl)thiazole-5-sulfonimidamide | 480 |
| 31 | 178 | 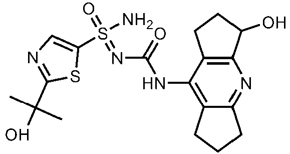 | N'-((3-hydroxy-1,2,3,5,6,7-hexahydrodicyclopenta[b,e]pyridin-8-yl)carbamoyl)-2-(2-hydroxypropan-2-yl)thiazole-5-sulfonimidamide | 438 |
| 32 | 179 | 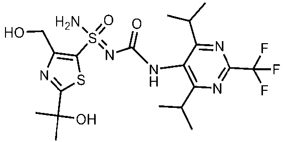 | N'-((4,6-diisopropyl-2-(trifluoromethyl)pyrimidin-5-yl)carbamoyl)-4-(hydroxymethyl)-2-(2-hydroxypropan-2-yl)thiazole-5-sulfonimidamide | 525 |

### Example 34 (Compound 107)

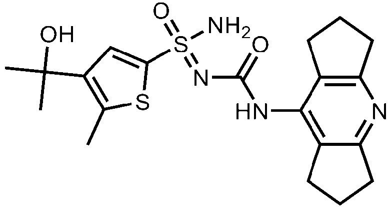

### N'-((1,2,3,5,6,7-hexahydrodicyclopenta[b,e]pyridin-8-yl)carbamoyl)-4-(2-hydroxypropan-2-yl)-5-methylthiophene-2-sulfonimidamide (scheme 2B)

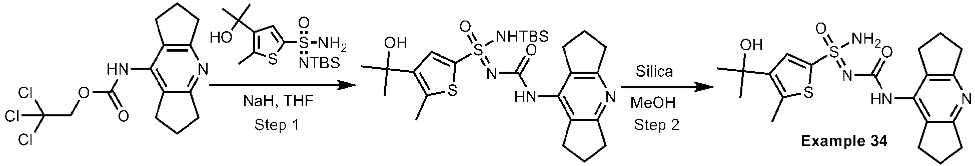

### Step 1: N-(tert-butyldimethylsilyl)-N'-((1,2,3,5,6,7-hexahydrodicyclopenta[b,e]pyridin-8-yl)carbamoyl)-4-(2-hydroxypropan-2-yl)-5-methylthiophene-2-sulfonimidamide

*N*-(tert-butyldimethylsilyl)-4-(2-hydroxypropan-2-yl)-5- methylthiophene-2-sulfonoimidamide (250 mg, 0.72 mmol) in THF (10 mL). To the above solution was added NaH (60%wt. oil dispersion, 57.2 mg, 1.43 mmol). The resulting solution was stirred for 10 min at RT. Then 2,2,2-trichloroethyl (1,2,3,5,6,7-hexahydrodicyclopenta[b,e]pyridin-8-yl)carbamate (251 mg, 0.72 mmol) was added to the reaction solution. The resulting solution was allowed to react, with stirring, for an additional 16 h at 30°C. The reaction was then quenched by the addition of 10 mL of water. The resulting mixture was concentrated. The residue was eluted from silica gel with DCM/MeOH (10:1). This resulted in 270 mg (68.6%) of the title compound as a white solid. MS-ESI: 549 (M+1).

### Step 2: N'-((1,2,3,5,6,7-hexahydrodicyclopenta[b,e]pyridin-8-yl)carbamoyl)-4-(2-hydroxypropan-2-yl) -5-methylthiophene-2-sulfonimidamide

Into a 50-mL round-bottom flask, was placed N-(tert-butyldimethylsilyl)-N'-((1,2,3,5,6,7-hexahydrodicyclo- penta[b,e]pyridin-8-yl)carbamoyl)-4-(2-hydroxypropan-2-yl)-5-methylthiophene-2-sulfonimidamide (270 mg, 0.49 mmol) in CH₃OH (10 mL). To the above solution was added silica gel (5.0 g). The resulting mixture was stirred for 2 h at RT. The resulting mixture was concentrated. The residue was eluted from silica gel with DCM/MeOH (8:1). The crude product was purified by Prep-HPLC with the following conditions: Column, Sunfire Prep C18 OBD, 10 um, 19*250 mm; mobile phase, water (0.1% FA) and ACN (10% to 40% gradient over 7 min); Detector, UV. This resulted in 100 mg (46.78%) of **Example 34** as a white solid. MS-ESI: 435 (M+1). ¹H NMR (400 MHz, DMSO-*d*₆) δ: 8.72 (s, 1H), 8.14 (s, 1H), 7.64 (br s, 2H), 7.48 (s, 1H), 5.11 (br s, 1H), 2.81-2.74 (m, 4H), 2.73-2.71 (m, 4H), 2.54 (s, 3H), 1.99-1.92 (m, 4H), 1.44 (s, 6H).

**Table 19. Examples in the following table were prepared using similar conditions as described in Example 34 and Scheme 2B from appropriate starting materials.**

| Example # | Compound Number | Structure | IUPAC Name | Exact Mass [M+H]⁺ |
|---|---|---|---|---|
| 35 | 155 | 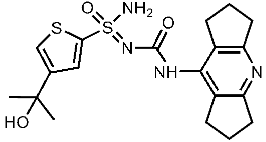 | N'-((1,2,3,5,6,7-hexahydrodicyclopenta[b,e]pyridin-8-yl)carbamoyl)-4-(2-hydroxypropan-2-yl)thiophene-2-sulfonimidamide | 421 |
| 36 | 166 | 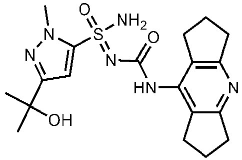 | N'-((1,2,3,5,6,7-hexahydrodicyclopenta[b,e]pyridin-8-yl)carbamoyl)-3-(2-hydroxypropan-2-yl)-1-methyl-1 H-pyrazole-5-sulfonimidamide | 419 |
| 37 | 149 | 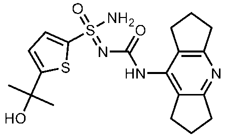 | N'-((1,2,3,5,6,7-hexahydrodicyclopenta[b,e]pyridin-8-yl)carbamoyl)-5-(2-hydroxypropan-2-yl)thiophene-2-sulfonimidamide | 421 |
| 38 | 105 | 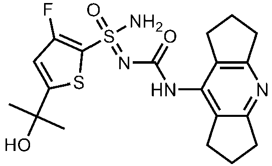 | 3-fluoro-N'-((1,2,3,5,6,7-hexahydrodicyclopenta[b,e]pyridin-8-yl)carbamoyl)-5-(2-hydroxypropan-2-yl)thiophene-2-sulfonimidamide | 439 |
| 39 | 141 | 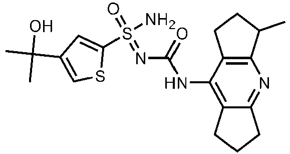 | 4-(2-hydroxypropan-2-yl)-N'-((3-methyl-1,2,3,5,6,7-hexahydrodicyclopenta[b,e]pyridin-8-yl)carbamoyl)thiophene-2-sulfonimidamide | 435 |
| 40 | 103 | 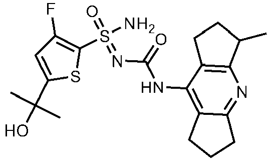 | 3-fluoro-5-(2-hydroxypropan-2-yl)-N'-((3-methyl-1,2,3,5,6,7-hexahydrodicyclopenta[b,e]pyridin-8-yl)carbamoyl)thiophene-2-sulfonimidamide | 453 |
| 41 | 127 | 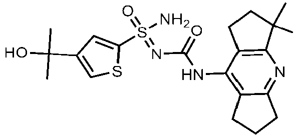 | N'-((3,3-dimethyl-1,2,3,5,6,7-hexahydrodicyclopenta[b,e]pyridin-8-yl)carbamoyl)-4-(2-hydroxypropan-2-yl)thiophene-2-sulfonimidamide | 449 |
| 42 | 102 | 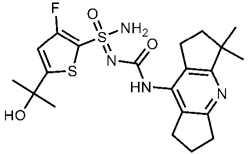 | N'-((3,3-dimethyl-1,2,3,5,6,7-hexahydrodicyclopenta[b,e]pyridin-8-yl)carbamoyl)-3-fluoro-5-(2-hydroxypropan-2-yl)thiophene-2-sulfonimidamide | 467 |
| 43 | 131 | 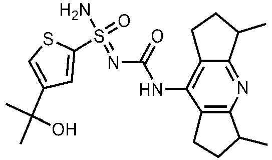 | N'-((3,5-dimethyl-1,2,3,5,6,7-hexahydrodicyclopenta[b,e]pyridin-8-yl)carbamoyl)-4-(2-hydroxypropan-2-yl)thiophene-2-sulfonimidamide | 449 |
| 44 | 123 | 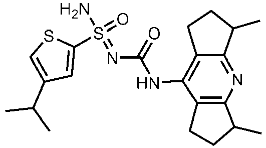 | N'-((3,5-dimethyl-1,2,3,5,6,7-hexahydrodicyclopenta[b,e]pyridin-8-yl)carbamoyl)-4-isopropylthiophene-2-sulfonimidamide | 433 |
| 45 | 180 | 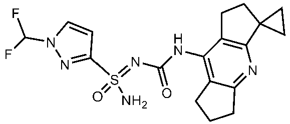 | 1-(difluoromethyl)-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 423 |
| 46 | 113 | 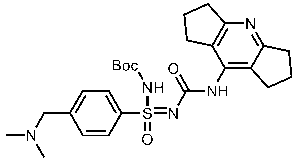 | tert-butyl (4-((dimethylamino)methyl)-N-((1,2,3,5,6,7-hexahydrodicyclopenta[b,e]pyridin-8-yl)carbamoyl)phenylsulfonimidoyl) carbamate | 514 |
| 47 | 181 | 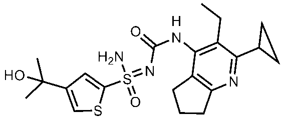 | N'-((2-cyclopropyl-3-ethyl-6,7-dihydro-5H-cyclopenta[b]pyridin-4-yl)carbamoyl)-4-(2-hydroxypropan-2-yl)thiophene-2-sulfonimidamide | 449 |
| 48 | 182 | 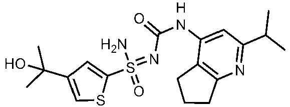 | 4-(2-hydroxypropan-2-yl)-N'-((2-isopropyl-6,7-dihydro-5H-cyclopenta[b]pyridin-4-yl)carbamoyl)thiophene-2-sulfonimidamide | 423 |

### Example 49 (Compound 183)

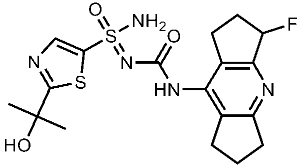

### N'-((3-fluoro-1,2,3,5,6,7-hexahydrodicyclopenta[b,e]pyridin-8-yl)carbamoyl)-2-(2-hydroxypropan-2-yl)thiazole-5-sulfonimidamide (scheme 3)

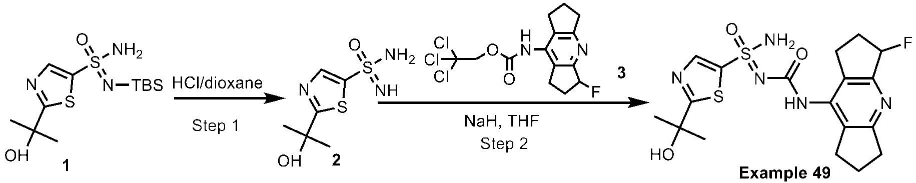

### Step 1: 2-(2-Hydroxypropan-2-yl)thiazole-5-sulfonimidamide

Into a 100-mL round-bottom flask, was placed N-(tert-butyldimethylsilyl)-2-(2-hydroxypropan-2-yl)- 1,3-thiazole-5-sulfonoimidamide (2.0 g, 5.96 mmol) in dioxane (50 mL) and aq. HCl (10 mL, 12 M). The resulting solution was stirred for 2 h at RT. The resulting mixture was concentrated. This resulted in 2.0 g crude title compound as a white solid. MS-ESI: 222 (M+1).

### Step 2: N'-((3-fluoro-1,2,3,5,6,7-hexahydrodicyclopenta[b,e]pyridin-8-yl)carbamoyl)-2-(2-hydroxy- propan-2-yl)thiazole-5-sulfonimidamide

Into a 25-mL round-bottom flask, was placed a solution of 2,2,2-trichloroethyl (3-fluoro-1,2,3,5,6,7-hexa- hydrodicyclopenta[b,e]pyridin-8-yl)carbamate (66 mg, 0.18 mmol) in THF (2.0 mL). This was followed by the addition of NaH (60% wt. oil dispersion, 14.4 mg, 0.36 mmol) at 0°C. The resulting solution was stirred for 10 min at RT. To this was added 2-(2-hydroxypropan-2-yl)-1,3-thiazole-5-sulfonoimidamide (40 mg, 0.18 mmol). The resulting solution was stirred overnight at 45°C. The reaction was then quenched by the addition of 0.5 mL of water. The resulting mixture was concentrated. The crude product was purified by Prep-HPLC with the following conditions: Column, Sunfire Prep C18 OBD, 10 um, 19*250 mm; mobile phase, water (0.05% FA) and ACN (5% to 30% gradient over 7 min); Detector, UV. This resulted in 5.4 mg (6.8%) of **Example 49** as a white solid. MS-ESI: 440 (M+1). ¹H NMR (400 MHz, DMSO-*d*₆) δ 9.04 (s, 1H), 8.10 (s, 1H), 5.99-5.60 (m, 1H), 3.00-2.60 (m, 8H), 2.10-1.90 (m, 2H), 1.51 (s, 6H).

**Table 20. Examples in the following table were prepared using similar conditions as described in Example 49 and Scheme 3 from appropriate starting materials.**

| Example # | Compound Number | Structure | IUPAC Name | Exact Mass [M+H]⁺ |
|---|---|---|---|---|
| 50 | 154 | 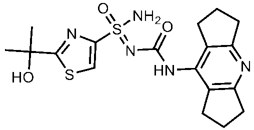 | N'-((1,2,3,5,6,7-hexahydrodicyclopenta[b,e]pyridin-8-yl)carbamoyl)-2-(2-hydroxypropan-2-yl)thiazole-4-sulfonimidamide | 422 |

### Example 51 (Compound 120)

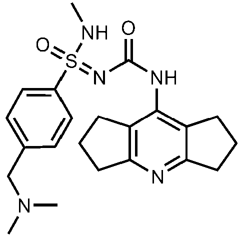

### 4-((Dimethylamino)methyl)-N'-((1,2,3,5,6,7-hexahydrodicyclopenta[b,e]pyridin-8-yl)carbamoyl)-N-methylbenzenesulfonimidamide (scheme 3A)

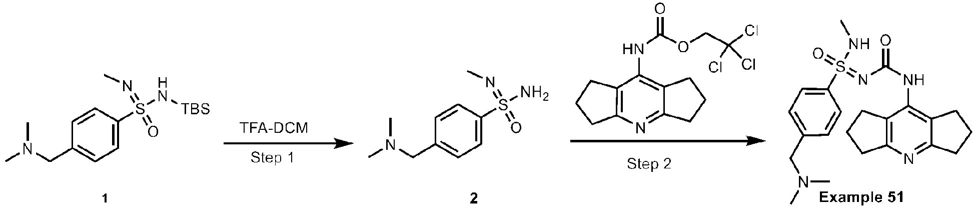

### Step 1: 4-((Dimethylamino)methyl)-N'-methylbenzenesulfonimidamide

Into a 50 mL round-bottom flask was added N-(tert-butyldimethylsilyl)-4-((dimethylamino)methyl)-N'-methylbenzenesulfonimidamide (600 mg, 1.76 mmol) in DCM (3.0 mL) at RT. To this stirred solution was added TFA (3 mL, 40 mmol) dropwise. The resulting mixture was stirred for 30 min and concentrated under reduced pressure. The crude product (500 mg) was purified by Prep-HPLC with the following conditions (Column: XBridge Prep OBD C18, 30x150 mm, 5 um; Mobile phase A: water (10 mM, NH₄HCO₃), Mobile Phase B: ACN; Flow rate: 60 mL/min; Gradient: 5% B to 45% B in 8 min; 254/210 nm; Rt: 6.13 min) to afford the title compound (250 mg, 62.6%) as a white solid. MS-ESI: 228 (M+1).

### Step 2: 4-((Dimethylamino)methyl)-N'-((1,2,3,5,6,7-hexahydrodicyclopenta[b,e]pyridin-8-yl)carbamoyl) -N-methylbenzenesulfonimidamide

Into a 50-mL round-bottom flask purged and maintained with an inert atmosphere of nitrogen, were placed 4-((dimethylamino)methyl)-N'-methylbenzenesulfonimidamide (180 mg, 0.79 mmol) in THF (10 mL) at 0°C. To a stirred solution was added NaH (60% wt. oil dispersion, 63.2 mg, 1.58 mmol) in portions at 0°C. The resulting mixture was stirred for 15min at 0°C. To the above mixture was added 2,2,2-trichloroethyl (1,2,3,5,6,7-hexahydrodicyclopenta[b,e]pyridin-8-yl)carbamate (277 mg, 0.79 mmol) in THF (3 mL) dropwise at 0°C. The resulting mixture was stirred for additional 2h at RT. The reaction was quenched by the addition of water/ice (0.5 mL) at 0°C. The resulting mixture was concentrated under reduced pressure. The crude product (300 mg) was purified by Prep-HPLC with the following conditions (Column: XBridge Prep OBD C18 30x150 mm 5 um; Mobile phase A: water (10 mM NH₄HCO₃), Mobile Phase B: ACN (5% to 50% gradient over 7 min), Flow rate: 60 mL/min; 254/210 nm UV detector; Rt 6.37 min) to afford **Example 51** as a white solid (80 mg, 23%). MS-ESI: 428(M+1). ¹H NMR (400 MHz, DMSO-*d*₆) δ 8.84 (s, 1H), 7.80 (d, J = 8.4 Hz, 2H), 7.54 (d, J = 8.4 Hz, 2H), 3.48 (s, 2H), 2.85-2.73 (m, 4H), 2.73-2.65 (m, 4H), 2.44 (s, 3H), 2.17 (s, 6H), 2.00-1.80 (m, 4H).

### Example 52 (Compound 162)

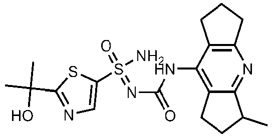

### 2-(2-hydroxypropan-2-yl)-N'-((3-methyl-1,2,3,5,6,7-hexahydrodicyclopenta[b,e]pyridin-8-yl)carbamoyl)thiazole-5-sulfonimidamide (scheme 4)

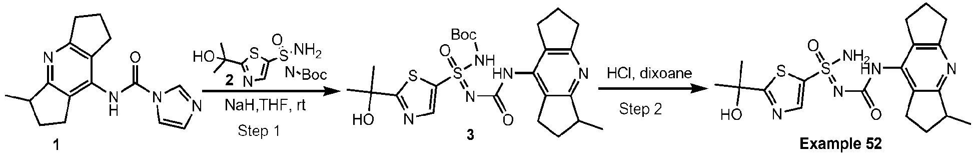

### Step 1: Tert-butyl (2-(2-hydroxypropan-2-yl)-N-((3-methyl-1,2,3,5,6,7-hexahydrodicyclopenta[b,e]- pyridin-8-yl)carbamoyl)thiazole-5-sulfonimidoyl)carbamate

Into a 1-L round-bottom flask purged and maintained with an inert atmosphere of nitrogen, was placed a solution of tert-butyl (amino(2-(2-hydroxypropan-2-yl)thiazol-5-yl)(oxo)-λ6-sulfaneylidene)carbamate (12.8 g, 40 mmol) in THF (300 mL). This was followed by the addition of NaH (60% wt. oil dispersion, 2.66 g, 66.4 mmol) in several batches at 0°C. The resulting solution was stirred for 20 min at RT. To this was added a solution of N-(3-methyl-1,2,3,5,6,7-hexahydrodicyclopenta[b,e]pyridin-8-yl)-1H-imidazole-1-carboxamide (7.5 g, 27 mmol) in DMF (50 mL) dropwise with stirring at 0°C. The resulting solution was stirred for 2 h at RT. The resulting mixture was quenched by the addition of 10 mL of H₂O. The resulting solution was extracted with 2x300 mL of EtOAc. The combined organic layer was washed with 3x200 mL H₂O, dried over anhydrous sodium sulfate and concentrated. The residue was eluted from silica gel with DCM/MeOH (10:1). This resulted in 3.5 g (24.6%) of the title compound as a white solid. MS-ESI: 536 (M+1).

### Step 2: 2-(2-hydroxypropan-2-yl)-N'-((3-methyl-1,2,3,5,6,7-hexahydrodicyclopenta[b,e]pyridin-8-yl)- carbamoyl)thiazole-5-sulfonimidamide

Into a 500-mL round-bottom flask, was placed a solution of tert-butyl (2-(2-hydroxypropan-2-yl)-N-((3- methyl-1,2,3,5,6,7-hexahydrodicyclopenta[b,e]pyridin-8-yl)carbamoyl)thiazole-5-sulfonimidoyl)carbamate (3.5 g, 6.53 mmol) in dioxane (210 mL). This was followed by the addition of HCl (52.5 mL, 12 M) dropwise with stirring at 0°C. The resulting solution was stirred for 2 h at RT. The pH value of the solution was adjusted to 7-8 with Na₂CO₃ (1.6 M). The resulting solution was extracted with 4x500 mL of EtOAc. The combined organic phase was dried over anhydrous sodium sulfate and concentrated. The crude product was purified by Prep-HPLC with the following conditions: Column, XBridge Prep C18 OBD, 19*150 mm 5 um; mobile phase, water (10 mM NH₄HCO₃) and ACN (4% to 22% gradient over 6 min); Detector, 220/254 nm UV. This resulted in 2 g (71%) of **Example 52** as a white solid. MS-ESI: 436 (M+1). ¹H NMR (400 MHz, DMSO-*d*₆) δ 8.80 (s, 1H), 8.08 (s, 1H), 7.89 (br s, 2H), 6.28 (s, 1H), 3.03-2.98 (m, 1H), 2.83-2.79 (t, *J* = 7.2 Hz, 2H), 2.72-2.61 (m, 4H), 2.27-2.20 (m, 1H), 2.07-1.92 (m, 2H), 1.51-1.45 (m, 7H), 1.21-1.19 (d, *J =* 8.0 Hz, 3H).

### Example 53 (Compound 104)

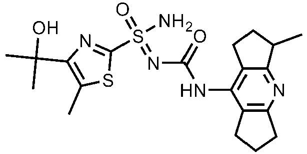

### 4-(2-Hydroxypropan-2-yl)-5-methyl-N'-((3-methyl-1,2,3,5,6,7-hexahydrodicyclopenta[b,e]pyridin-8-yl)carbamoyl)thiazole-2-sulfonimidamide (scheme 4A)

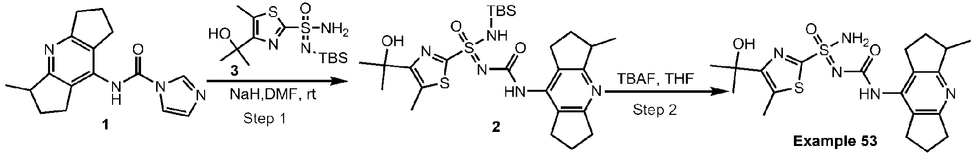

### Step 1: N-(tert-butyldimethylsilyl)-4-(2-hydroxypropan-2-yl)-5-methyl-N'-((3-methyl-1,2,3,5,6,7-hexa- hydrodicyclopenta[b,e]pyridin-8-yl)carbamoyl)thiazole-2-sulfonimidamide

Into a 100-mL round-bottom flask, was placed N-(3-methyl-1,2,3,5,6,7-hexahydrodicyclopenta[b,e]pyridin- 8-yl)-1H-imidazole-1-carboxamide (150 mg, 0.43 mmol) in DMF (15 mL). To the stirred solution was added NaH (60% wt. oil dispersion, 56.1 mg, 1.29 mmol). This was followed by the addition of the solution of N'-(tert-butyldimethylsilyl)-4-(2-hydroxypropan-2-yl)-5-methylthiazole-2-sulfonimidamide (120 mg, 0.43 mmol) in DMF (5.0 mL). The resulting solution was stirred for 16 h at 30°C in an oil bath. The reaction was then quenched by the addition of 15 mL of water. The resulting solution was extracted with 2x30 mL of EtOAc. The combined extracts were dried over anhydrous sodium sulfate and concentrated. This resulted in 180 mg (74.4%) of the title compound as a yellow solid. MS-ESI: 564 (M+1).

### 2: 4-(2-Hydroxypropan-2-yl)-5-methyl-N'-((3-methyl-1,2,3,5,6,7-hexahydrodicyclopenta[b,e]pyridin-8-yl)carbamoyl)thiazole-2-sulfonimidamide

Into a 50-mL round-bottom flask, was placed N-(tert-butyldimethylsilyl)-4-(2-hydroxypropan-2-yl)-5- methyl-N'-((3-methyl-1,2,3,5,6,7-hexahydrodicyclopenta[b,e]pyridin-8-yl)carbamoyl)thiazole-2-sulfonimidamide (180 mg, 0.32 mmol) in THF (15 mL), to the stirred solution was added TBAF (250 mg, 0.96 mmol). The resulting solution was stirred for 5 h at RT. The resulting mixture was concentrated. The residue was eluted from silica gel with DCM/MeOH (10:1). The crude product was purified by Prep-HPLC with the following conditions: Column, SunFire Prep C18 OBD, 19*150 mm 5 um; mobile phase, water (0.1% FA) and ACN (12% to 22% gradient over 10 min); Detector, UV 254 nm. This resulted in 40 mg (27.87%) of Example 53 as a white solid. MS-ESI: 450 (M+1). ¹H NMR (400 MHz, DMSO-*d*₆) δ 8.84 (s, 1H), 5.96 (s, 1H), 2.91-3.04 (m, 1H), 2.85-2.80 (m, 2H), 2.75-2.62 (m, 4H), 2.44 (s, 3H), 2.25-2.15 (m, 1H), 2.00-1.90 (m, 2H), 1.58(s, 6H), 1.42-1.40 (m, 1H), 1.20(d, J = 7.8Hz, 3H)

**Table 21. Examples in the following table were prepared using similar conditions as described in Example 53 and Scheme 4A from appropriate starting materials.**

| Example # | Final Target Number | Structure | IUPAC Name | Exact Mass [M+H]⁺ |
|---|---|---|---|---|
| 54 | 168 | 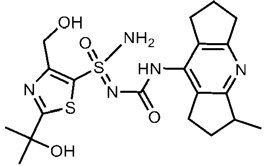 | 4-(hydroxymethyl)-2-(2-hydroxypropan-2-yl)-N'-((3-methyl-1,2,3,5,6,7-hexahydrodicyclopenta[b,e]pyridin-8-yl)carbamoyl)thiazole-5-sulfonimidamide | 466 |

### Example 55 (Compound 158)

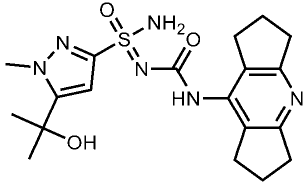

### N'-((1,2,3,5,6,7-hexahydrodicyclopenta[b,e]pyridin-8-yl)carbamoyl)-5-(2-hydroxypropan-2-yl)-1-methyl-1H-pyrazole-3-sulfonimidamide (scheme 5)

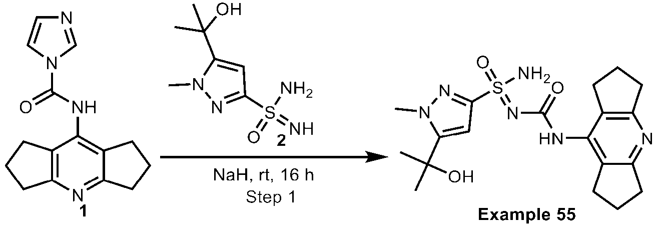

Into a 50-mL round-bottom flask purged and maintained with an inert atmosphere of nitrogen, was placed N-(1,2,3,5,6,7-hexahydrodicyclopenta[b,e]pyridin-8-yl)-1H-imidazole-1-carboxamide (246 mg, 0.92 mmol) in DMF (5 mL). To the above solution was added 5-(2-hydroxypropan-2-yl)-1-methyl-1H-pyrazole-3- sulfonimidamide (200 mg, 0.92 mmol) and NaH (60%wt. oil dispersion, 73.6 mg, 1.84 mmol). The resulting solution was stirred for 1 h at RT. The resulting mixture was quenched with 3 mL of H₂O and concentrated. The crude product was purified by Prep-HPLC with the following conditions: Column, XBridge Prep C18 OBD, 19*150 mm, 5 um; mobile phase, water (10 mM NH₄HCO₃+0.1%NH₃.H₂O) and ACN (5% to 38% gradient in 6 min); Detector, UV 210/254 nm. This resulted in 8.2 mg (2.14%) of Example 55 as a white solid. MS-ESI: 419 (M+1). ¹H NMR (400 MHz, DMSO-*d*₆) 8.70 (s, 1H), 7.47 (s, 2H), 6.49 (s, 1H), 5.51 (s, 1H), 4.04 (s, 3H), 2.90-2.60 (m, 8H), 2.00-1.80 (m, 4H), 1.48 (s, 6H).

**Table 22. Examples in the following table were prepared using similar conditions as described in Example 55 and Scheme 5 from appropriate starting materials.**

| Example # | Final Target Number | Structure | IUPAC Name | Exact Mass [M+H]⁺ |
|---|---|---|---|---|
| 56 | 157 | 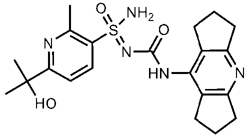 | N'-((1,2,3,5,6,7-hexahydrodicyclopenta[b,e]pyridin-8-yl)carbamoyl)-6-(2-hydroxypropan-2-yl)-2-methylpyridine-3-sulfonimidamide | 430 |
| 57 | 156 | 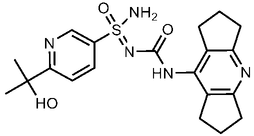 | N'-((1,2,3,5,6,7-hexahydrodicyclopenta[b,e]pyridin-8-yl)carbamoyl)-6-(2-hydroxypropan-2-yl)pyridine-3-sulfonimidamide | 416 |
| 58 | 160 | 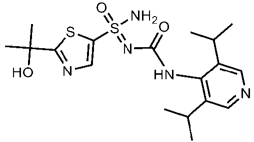 | N'-((3,5-diisopropylpyridin-4-yl)carbamoyl)-2-(2-hydroxypropan-2-yl)thiazole-5-sulfonimidamide | 426 |
| 59 | 129 | 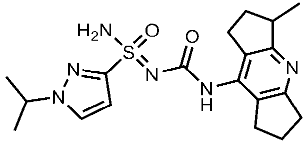 | 1-isopropyl-N'-((3-methyl-1,2,3,5,6,7-hexahydrodicyclopenta[b,e]pyridin-8-yl)carbamoyl)-1H-pyrazole-3-sulfonimidamide | 403 |
| 60 | 161 | 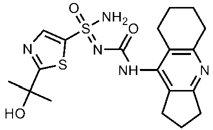 | N'-((2,3,5,6,7,8-hexahydro-1H-cyclopenta[b]quinolin-9-yl)carbamoyl)-2-(2-hydroxypropan-2-yl)thiazole-5-sulfonimidamide | 436 |
| 61 | 153 | 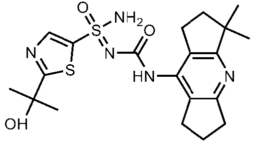 | N'-((3,3-dimethyl-1,2,3,5,6,7-hexahydrodicyclopenta[b,e]pyridin-8-yl)carbamoyl)-2-(2-hydroxypropan-2-yl)thiazole-5-sulfonimidamide | 450 |
| 62 | 152 | 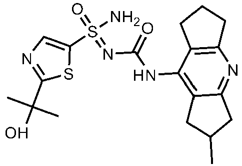 | 2-(2-hydroxypropan-2-yl)-N'-((2-methyl-1,2,3,5,6,7-hexahydrodicyclopenta[b,e]pyridin-8-yl)carbamoyl)thiazole-5-sulfonimidamide | 436 |
| 63 | 151 | 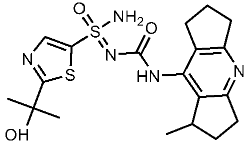 | 2-(2-hydroxypropan-2-yl)-N'-((1-methyl-1,2,3,5,6,7-hexahydrodicyclopenta[b,e]pyridin-8-yl)carbamoyl)thiazole-5-sulfonimidamide | 436 |
| 64 | 147 | 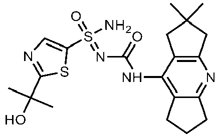 | N'-((2,2-dimethyl-1,2,3,5,6,7-hexahydrodicyclopenta[b,e]pyridin-8-yl)carbamoyl)-2-(2-hydroxypropan-2-yl)thiazole-5-sulfonimidamide | 450 |
| 65 | 146 | 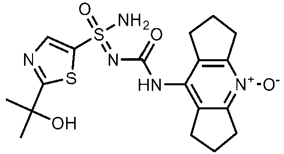 | 8-(3-(amino(2-(2-hydroxypropan-2-yl)thiazol-5-yl)(oxo)-λ6-sulfaneylidene)ureido)-1,2,3,5,6,7-hexahydrodicyclopenta[b,e]pyridine 4-oxide | 438 |
| 66 | 133 | 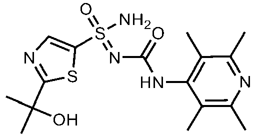 | 2-(2-hydroxypropan-2-yl)-N'-((2,3,5,6-tetramethylpyridin-4-yl)carbamoyl)thiazole-5-sulfonimidamide | 398 |
| 67 | 112 | 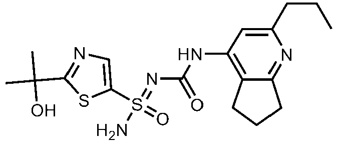 | 2-(2-hydroxypropan-2-yl)-N'-((2-propyl-6,7-dihydro-5H-cyclopenta[b]pyridin-4-yl)carbamoyl)thiazole-5-sulfonimidamide | 424 |

### Example 68 (Compound 115)

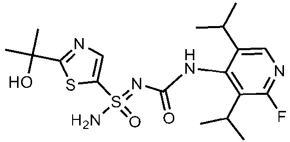

### N'-((2-fluoro-3,5-diisopropylpyridin-4-yl)carbamoyl)-2-(2-hydroxypropan-2-yl)thiazole-5-sulfonimidamide (scheme 6)

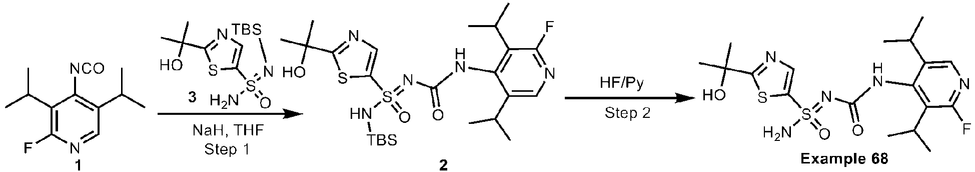

### Step 1: N-(tert-butyldimethylsilyl)-N'-((2-fluoro-3,5-diisopropylpyridin-4-yl)carbamoyl)-2-(2- hydroxypropan-2-yl)thiazole-5-sulfonimidamide

Into a 100-mL round-bottom flask, was placed 2-fluoro-4-isocyanato-3,5-diisopropylpyridine (240 mg, 1.22 mmol) in THF (10 mL), to the stirred solution was added NaH (60%wt. oil dispersion, 59 mg, 2.45 mmol) and N'-(tert-butyldimethylsilyl)-2-(2-hydroxypropan-2-yl)thiazole-5-sulfonimidamide (410 mg, 1.22 mmol). The resulting solution was stirred for 2 h at 25°C. The reaction was then quenched by the addition of 20 mL of water. The resulting solution was extracted with 2x20 mL of EtOAc and the organic layers combined and dried and concentrated. This resulted in 400 mg (59%) of the title compound as a yellow solid. MS-ESI: 558 (M+1).

### Step 2: N'-((2-fluoro-3,5-diisopropylpyridin-4-yl)carbamoyl)-2-(2-hydroxypropan-2-yl)thiazole-5- sulfonimidamide

Into a 100-mL round-bottom flask, was placed N-(tert-butyldimethylsilyl)-N'-((2-fluoro-3,5-diisopropyl- pyridin-4-yl)carbamoyl)-2-(2-hydroxypropan-2-yl)thiazole-5-sulfonimidamide (400 mg, 0.72 mmol) in THF (10 mL) and HF-pyridine (31 mg, 1.08 mmol, 70%wt.). The resulting solution was stirred for 2 h at 25°C. The resulting mixture was concentrated. The crude product was purified by Prep-HPLC with the following conditions: Column, XBridge Prep OBD C18, 30*150 mm 5 um; mobile phase, water (10 mM NH₄HCO₃) and ACN (5% to 35% gradient over 6 min); Detector, UV 220/254 nm. This resulted in 200 mg (63%) of **Example 68** as a white solid. MS-ESI: 443 (M+1). ¹H NMR (400 MHz, CD₃OD-d*₄*) δ 8.14 (s, 1H), 7.92 (s, 1H), 3.30-3.00 (m, 2H), 1.59 (s, 6H), 1.50-1.10 (m, 12H).

### Example 69 (Compound 106)

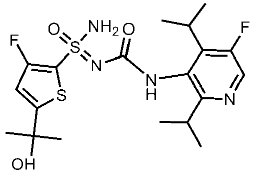

### 3-Fluoro-N'-((5-fluoro-2,4-diisopropylpyridin-3-yl)carbamoyl)-5-(2-hydroxypropan-2-yl)thiophene-2-sulfonimidamide (scheme 7)

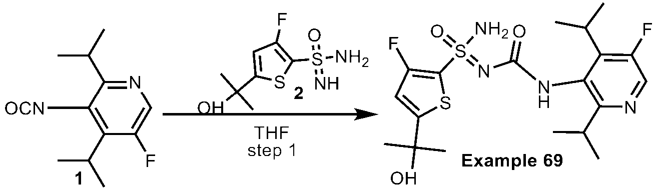

Into a 50-mL round-bottom flask, was placed 5-fluoro-3-isocyanato-2,4-diisopropylpyridine (120 mg, 0.50 mmol) in THF (15 mL). To the stirred solution was added NaH (60%wt. oil dispersion, 60.4 mg, 1.51 mmol). The resulting solution was stirred for 10 min at RT. Then 3-fluoro-5-(2-hydroxypropan-2-yl)thiophene-2-sulfonimidamide (150 mg, crude) was added . The resulting solution was allowed to react, with stirring, for an additional 1 h at RT. The reaction was then quenched by the addition of 20 mL of water. The resulting solution was extracted with 5x30 mL of EtOAc and the combined extract was concentrated. The crude product was purified by Prep-HPLC with the following conditions: Column, XBridge Prep C18 OBD, 5 um,19*150 mm ; mobile phase, water (10 mM NH₄HCO₃) and ACN (5% to 50% gradient over 6 min); Detector, UV (254/210 nm). This resulted in 75 mg (32%) of **Example 69** as an off-white solid. MS-ESI: 461 (M+1). ¹H NMR (300 MHz, DMSO-*d*₆) δ 8.54 (s, 1H), 8.29 (s, 1H), 7.68 (br s, 2H), 6.96 (s, 1H), 5.83 (s, 1H), 3.38-3.17 (m, 2H), 1.45 (s, 6H), 1.22 (d, *J* = 7.1 Hz, 6H), 1.10 (d, *J* = 6.7 Hz, 6H).

**Table 23. Examples in the following table were prepared using similar conditions as described in Example 69 and Scheme 7 from appropriate starting materials.**

| Example # | Final Target Number | Structure | IUPAC Name | Exact Mass [M+H]⁺ |
|---|---|---|---|---|
| 70 | 148 | 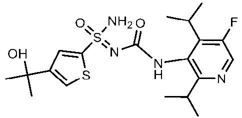 | N'-((5-fluoro-2,4-diisopropylpyridin-3-yl)carbamoyl)-4-(2-hydroxypropan-2-yl)thiophene-2-sulfonimidamide | 443 |
| 71 | 132 | 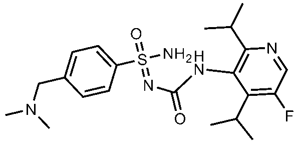 | 4-((dimethylamino)methyl)-N'-((5-fluoro-2,4-diisopropylpyridin-3-yl)carbamoyl)benzenesulfon imidamide | 436 |
| 72 | 140 | 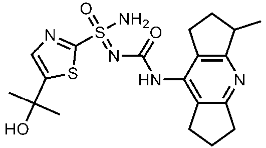 | 5-(2-hydroxypropan-2-yl)-N'-((3-methyl-1,2,3,5,6,7-hexahydrodicyclopenta[b,e] pyridin-8-yl)carbamoyl)thiazole-2-sulfonimidamide | 436 |
| 73 | 139 | 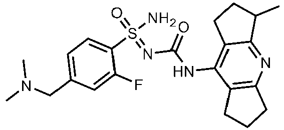 | 4-((dimethylamino)methyl)-2-fluoro-N'-((3-methyl-1,2,3,5,6,7-hexahydrodicyclopenta[b,e] pyridin-8-yl)carbamoyl)benzenesulfon imidamide | 446 |
| 74 | 134 | 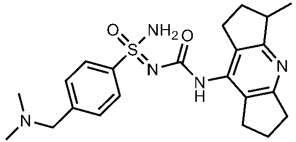 | 4-((dimethylamino)methyl)-N'-((3-methyl-1,2,3,5,6,7-hexahydrodicyclopenta[b,e] pyridin-8-yl)carbamoyl)benzenesulfon imidamide | 428 |
| 75 | 145 | 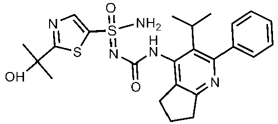 | 2-(2-hydroxypropan-2-yl)-N'-((3-isopropyl-2-phenyl-6,7-dihydro-5H-cyclopenta[b]pyridin-4-yl)carbamoyl)thiazole-5-sulfonimidamide | *500* |
| 76 | 144 | 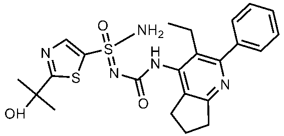 | N'-((3-ethyl-2-phenyl-6,7-dihydro-5H-cyclopenta[b]pyridin-4-yl)carbamoyl)-2-(2-hydroxypropan-2-yl)thiazole-5-sulfonimidamide | 486 |
| 77 | 143 | 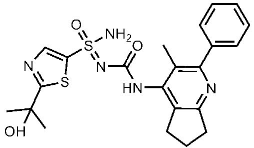 | 2-(2-hydroxypropan-2-yl)-N'-((3-methyl-2-phenyl-6,7-dihydro-5H-cyclopenta[b]pyridin-4-yl)carbamoyl)thiazole-5-sulfonimidamide | 472 |
| 78 | 142 | 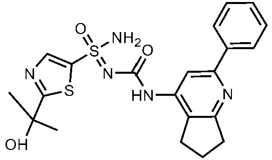 | 2-(2-hydroxypropan-2-yl)-N'-((2-phenyl-6,7-dihydro-5H-cyclopenta[b]pyridin-4-yl)carbamoyl)thiazole-5-sulfonimidamide | 458 |
| 79 | 138 | 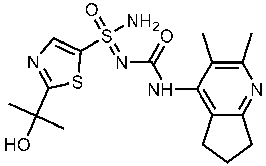 | N'-((2,3-dimethyl-6,7-dihydro-5H-cyclopenta[b]pyridin-4-yl)carbamoyl)-2-(2-hydroxypropan-2-yl)thiazole-5-sulfonimidamide | 410 |
| 80 | 137 | 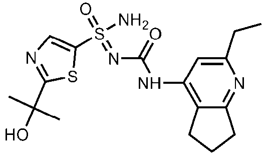 | N'-((2-ethyl-6,7-dihydro-5H-cyclopenta[b]pyridin-4-yl)carbamoyl)-2-(2-hydroxypropan-2-yl)thiazole-5-sulfonimidamide | 410 |
| 81 | 136 | 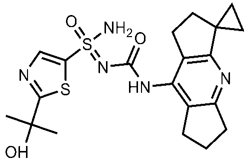 | 2-(2-hydroxypropan-2-yl)-N'-((1',5',6',7'-tetrahydro-2'H-spiro[cyclopropane-1,3'-dicyclopenta[b,e]pyridin]-8'-yl)carbamoyl)thiazole-5-sulfonimidamide | 448 |
| 82 | 124 | 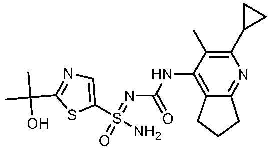 | N'-((2-cyclopropyl-3-methyl-6,7-dihydro-5H-cyclopenta[b]pyridin-4-yl)carbamoyl)-2-(2-hydroxypropan-2-yl)thiazole-5-sulfonimidamide | 436 |

**Table 24. Examples in the following table were obtained from chiral HPLC resolutions of racemic examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. For mixtures contained two chiral centers and if two columns are used for separating the four diastereomers, the individual isomers are listed in the order of faster column 1/faster column 2; faster column 1/slower column 2; slower column 1/faster column 2; followed by slower column 1/slower column 2.**

| Example # | Compound Number | Structure | IUPAC Name | Column | Eluents | LC-MS [M+H]⁺ |
|---|---|---|---|---|---|---|
| 83 | 128a | 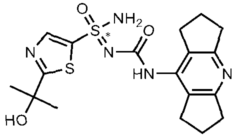 | (R) or (S)-N'-((1,2,3,5,6,7-hexahydrodicyclopenta[b,e]pyridi n-8-yl)carbamoyl)-2-(2-hydroxypropan-2-yl)thiazole-5-sulfonimidamide | CHIRALPAK IG, 2*25 cm (5 um) | EtOH in Hex (0.1% FA) | 422 |
| 84 | 128b | 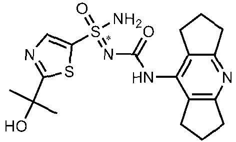 | (S) or (R)-N'-((1,2,3,5,6,7-hexahydrodicyclopenta[b,e]pyridi n-8-yl)carbamoyl)-2-(2-hydroxypropan-2-yl)thiazole-5-sulfonimidamide | CHIRALPAK IG, 2*25 cm (5 um) | EtOH in Hex (0.1% FA) | 422 |
| *85* | 150a | 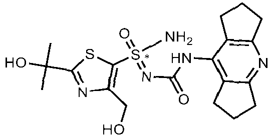 | (S) or (R)-N'-((1,2,3,5,6,7-hexahydrodicyclopenta[b,e]pyridi n-8-yl)carbamoyl)-4-(hydroxymethyl)-2-(2-hydroxypropan-2-yl)thiazole-5-sulfonimidamide | CHIRAL ART Cellulose-SB, 2*25 cm, 5 um | EtOH in Hex (0.1% FA) | 452 |
| 86 | 150b | 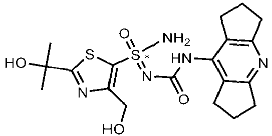 | (R) or (S)-N'-((1,2,3,5,6,7-hexahydrodicyclopenta[b,e]pyridi n-8-yl)carbamoyl)-4-(hydroxymethyl)-2-(2-hydroxypropan-2-yl)thiazole-5-sulfonimidamide | CHIRAL ART Cellulose-SB, 2*25 cm, 5 um | EtOH in Hex (0.1% FA) | 452 |
| 87 | 163a | 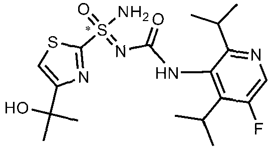 | (R) or (S)-N'-((5-fluoro-2,4-diisopropylpyridin-3-yl)carbamoyl)-4-(2-hydroxypropan-2-yl)thiazole-2-sulfonimidamide | CHIRALPAK ID, 2*25 cm (5 um) | EtOH in Hex (0.1% FA) | 444 |
| 88 | 163b | 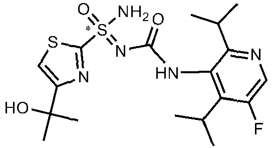 | (S) or (R)-N'-((5-fluoro-2,4-diisopropylpyridin-3-yl)carbamoyl)-4-(2-hydroxypropan-2-yl)thiazole-2-sulfonimidamide | CHIRALPAK ID, 2*25 cm (5 um) | EtOH in Hex (0.1% FA) | 444 |
| 91 | 167a | 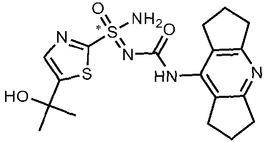 | (S) or (R)-N'-((1,2,3,5,6,7-hexahydrodicyclopenta[b,e]pyridi n-8-yl)carbamoyl)-5-(2-hydroxypropan-2-yl)thiazole-2-sulfonimidamide | Chiralpak AD, 2*25 cm (5 um) | 30% MeOH in CO₂ | 422 |
| 92 | 167b | 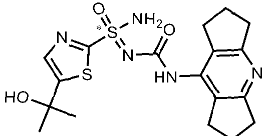 | (R) or (S)-N'-((1,2,3,5,6,7-hexahydrodicyclopenta[b,e]pyridi n-8-yl)carbamoyl)-5-(2-hydroxypropan-2-yl)thiazole-2-sulfonimidamide | Chiralpak AD, 2*25 cm (5 um) | 30% MeOH in CO₂ | 422 |
| 93 | 164b | 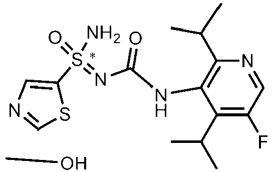 | (S) or (R)-N'-((5-fluoro-2,4-diisopropylpyridin-3-yl)carbamoyl)-2-(2-hydroxypropan-2-yl)thiazole-5-sulfonimidamide | CHIRALPAK ID, 2*25 cm (5 um) | EtOH in Hex (0.1% FA) | 444 |
| 94 | 164a | 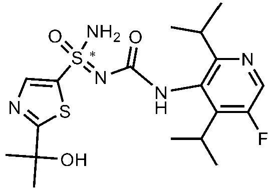 | (R) or (S)-N'-((5-fluoro-2,4-diisopropylpyridin-3-yl)carbamoyl)-2-(2-hydroxypropan-2-yl)thiazole-5-sulfonimidamide | CHIRALPAK ID, 2*25 cm (5 um) | EtOH in Hex (0.1% FA) | 444 |
| 95 | 161a | 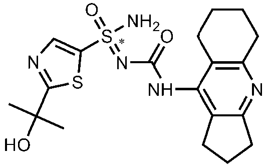 | (S) or (R)-N'-((2,3,5,6,7,8-hexahydro-1H-cyclopenta[b]quinolin-9-yl)carbamoyl)-2-(2-hydroxypropan-2-yl)thiazole-5-sulfonimidamide | CHIRAL ART Cellulose-SB S-5um, 250*20 mm | EtOH in Hex (0.1% FA) | 436 |
| 96 | 161b | 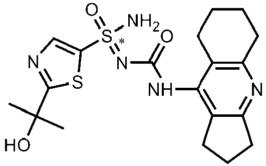 | (R) or (S)-N'-((2,3,5,6,7,8-hexahydro-1H-cyclopenta[b]quinolin-9-yl)carbamoyl)-2-(2-hydroxypropan-2-yl)thiazole-5-sulfonimidamide | CHIRAL ART Cellulose-SB S-5um, 250*20 mm | EtOH in Hex (0.1% FA) | 436 |
| 97 | 159a | 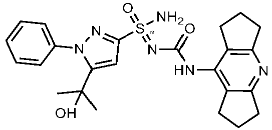 | (R) or (S)-N'-((1,2,3,5,6,7-hexahydrodicyclopenta[b,e]pyridi n-8-yl)carbamoyl)-5-(2-hydroxypropan-2-yl)-1-phenyl-1H-pyrazole-3-sulfonimidamide | CHIRALPAK IC, 2*25 cm, 5 um | EtOH in Hex (0.1% FA) | 481 |
| 98 | 159b | 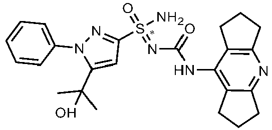 | (S) or (R)-N'-((1,2,3,5,6,7-hexahydrodicyclopenta[b,e]pyridi n-8-yl)carbamoyl)-5-(2-hydroxypropan-2-yl)-1-phenyl-1H-pyrazole-3-sulfonimidamide | CHIRALPAK IC, 2*25 cm, 5 um | EtOH in Hex (0.1% FA) | 481 |
| 99 | 158a | 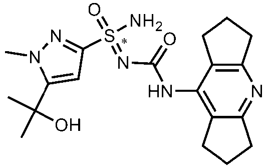 | (R) or (S)-N'-((1,2,3,5,6,7-hexahydrodicyclopenta[b,e]pyridi n-8-yl)carbamoyl)-5-(2-hydroxypropan-2-yl)-1-methyl-1H-pyrazole-3-sulfonimidamide | CHIRALPAK IC, 2*25 cm, 5 um | EtOH in Hex (8 mM NH₃.MeOH) # | 419 |
| 100 | 158b | 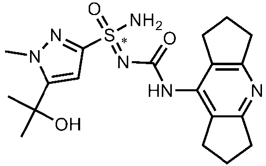 | (S) or (R)-N'-((1,2,3,5,6,7-hexahydrodicyclopenta[b,e]pyridi n-8-yl)carbamoyl)-5-(2-hydroxypropan-2-yl)-1-methyl-1H-pyrazole-3-sulfonimidamide | CHIRALPAK IC, 2*25 cm, 5 um | EtOH in Hex (8 mM NH₃.MeOH) | 419 |
| 101 | 157a | 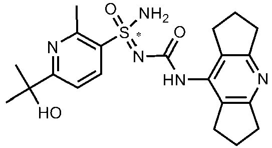 | (S) or (R)-N'-((1,2,3,5,6,7-hexahydrodicyclopenta[b,e]pyridi n-8-yl)carbamoyl)-6-(2-hydroxypropan-2-yl)-2-methylpyridine-3-sulfonimidamide | CHIRAL ART Cellulose-SB S-5um, 2*25 cm | EtOH in Hex (8 mM NH₃.MeOH) | 430 |
| 102 | 157b | 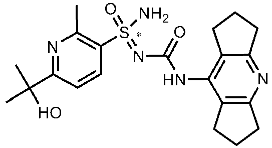 | (R) or (S)-N'-((1,2,3,5,6,7-hexahydrodicyclopenta[b,e]pyridi n-8-yl)carbamoyl)-6-(2-hydroxypropan-2-yl)-2-methylpyridine-3-sulfonimidamide | CHIRAL ART Cellulose-SB S-5um, 2*25 cm | EtOH in Hex (8 mM NH₃.MeOH) | 430 |
| 103 | 160a | 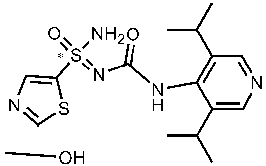 | (R) or (S)-N'-((3,5-diisopropylpyridin-4-yl)carbamoyl)-2-(2-hydroxypropan-2-yl)thiazole-5-sulfonimidamide | Chiralpak ID, 2*25 cm, 5 um | EtOH in Hex (8 mM NH₃.MeOH) | 426 |
| 104 | 160b | 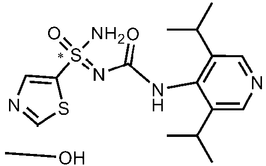 | (S) or (R)-N'-((3,5-diisopropylpyridin-4-yl)carbamoyl)-2-(2-hydroxypropan-2-yl)thiazole-5-sulfonimidamide | Chiralpak ID, 2*25 cm, 5 um | EtOH in Hex (8 mM NH₃.MeOH) | 426 |
| 105 | 162ab | 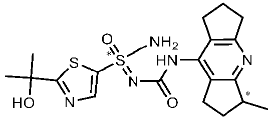 | (S, R) or (S, S)-2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | These two isomers are faster-eluting on CHIRAL ART Cellulose-SB, 2*25 cm, 5 um, EtOH in Hex (8 mM NH₃.MeOH); separated to single isomer on CHIRALPAK IG, 20*250 mm, 5 um EtOH in MTBE (10 mM NH₃-MeOH) | | 436 |
| 106 | 162aa | 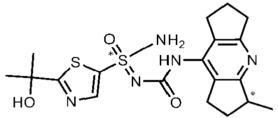 | (S, S) or (S, R)-2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | | | 436 |
| 107 | 162bb | 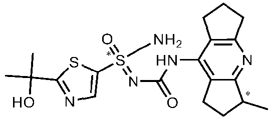 | (R, R) -2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | These two isomers are slower-eluting on CHIRAL ART Cellulose-SB, 2*25 cm, 5 um, EtOH in Hex (8 mM NH₃.MeOH); separated to single isomer on CHIRALPAK IG, 20*250 mm, 5 um EtOH in MTBE (10 mM NH₃-MeOH) | | 436 |
| 108 | 162ba | 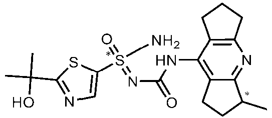 | (R, S)-2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | | | 436 |
| 109 | 156a | 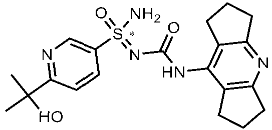 | (S) or (R)-N'-((1,2,3,5,6,7-hexahydrodicyclopenta[b,e]pyridi n-8-yl)carbamoyl)-6-(2-hydroxypropan-2-yl)pyridine-3-sulfonimidamide | CHIRAL ART Cellulose-SB S, 2*25 cm, 5 um | EtOH in MTBE (10 mM NH₃-MeOH) | 416 |
| 110 | 156b | 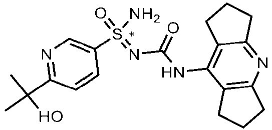 | (R) or (S)-N'-((1,2,3,5,6,7-hexahydrodicyclopenta[b,e]pyridi n-8-yl)carbamoyl)-6-(2-hydroxypropan-2-yl)pyridine-3-sulfonimidamide | CHIRAL ART Cellulose-SB S, 2*25 cm, 5 um | EtOH in MTBE (10 mM NH₃-MeOH) | 416 |
| 111 | 155a | 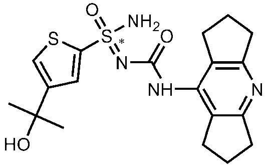 | (R) or (S)-N'-((1,2,3,5,6,7-hexahydrodicyclopenta[b,e]pyridi n-8-yl)carbamoyl)-4-(2-hydroxypropan-2-yl)thiophene-2-sulfonimidamide | CHIRALPAK IG, 2*25 cm (5 um) | EtOH in Hex (8 mM NH₃.MeOH) | 421 |
| 112 | 155b | 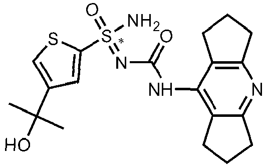 | (S) or (R)-N'-((1,2,3,5,6,7-hexahydrodicyclopenta[b,e]pyridi n-8-yl)carbamoyl)-4-(2-hydroxypropan-2-yl)thiophene-2-sulfonimidamide | CHIRALPAK IG, 2*25 cm (5 um) | EtOH in Hex (8 NH₃.MeOH) | 421 |
| 113 | 149a | 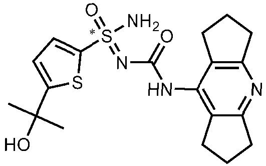 | (R) or (S)-N'-((1,2,3,5,6,7-hexahydrodicyclopenta[b,e]pyridi n-8-yl)carbamoyl)-5-(2-hydroxypropan-2-yl)thiophene-2-sulfonimidamide | CHIRALPAK IG, 2*25 cm (5 um) | EtOH in Hex (8 mM NH₃.MeOH) | 421 |
| 114 | 149b | 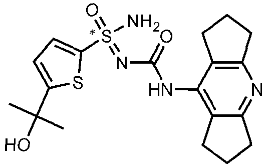 | (S) or (R)-N'-((1,2,3,5,6,7-hexahydrodicyclopenta[b,e]pyridi n-8-yl)carbamoyl)-5-(2-hydroxypropan-2-yl)thiophene-2-sulfonimidamide | CHIRALPAK IG, 2*25 cm (5 um) | EtOH in Hex (8 mM NH₃.MeOH) | 421 |
| 115 | 106a | 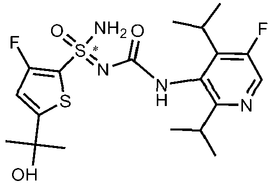 | (R) or (S)-3-fluoro-N'-((5-fluoro-2,4-diisopropylpyridin-3-yl)carbamoyl)-5-(2-hydroxypropan-2-yl)thiophene-2-sulfonimidamide | CHIRALPAK ID, 2*25 cm (5 um) | EtOH in Hex (8 mM NH₃.MeOH) | 461 |
| 116 | 106b | 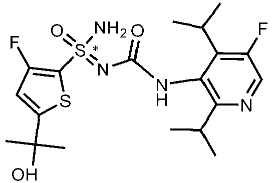 | (S) or (R)-3-fluoro-N'-((5-fluoro-2,4-diisopropylpyridin-3-yl)carbamoyl)-5-(2-hydroxypropan-2-yl)thiophene-2-sulfonimidamide | CHIRALPAK ID, 2*25 cm (5 um) | EtOH in Hex (8 mM NH₃.MeOH) | 461 |
| 117 | 148a | 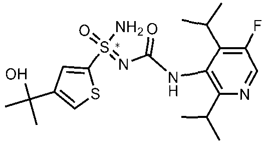 | (R) or (S)-N'-((5-fluoro-2,4-diisopropylpyridin-3-yl)carbamoyl)-4-(2-hydroxypropan-2-yl)thiophene-2-sulfonimidamide | CHIRALPAK IF, 2*25 cm, 5 um | EtOH in Hex (0.1% FA) | 443 |
| 118 | 148b | 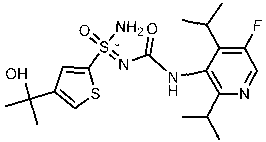 | (S) or (R)-N'-((5-fluoro-2,4-diisopropylpyridin-3-yl)carbamoyl)-4-(2-hydroxypropan-2-yl)thiophene-2-sulfonimidamide | CHIRALPAK IF, 2*25 cm, 5 um | EtOH in Hex (0.1% FA) | 443 |
| 119 | 147a | 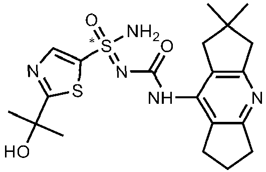 | (S) or (R)-N'-((2,2-dimethyl-1,2,3,5,6,7-hexahydrodicyclopenta[b,e]pyridi n-8-yl)carbamoyl)-2-(2-hydroxypropan-2-yl)thiazole-5-sulfonimidamide | CHIRAL ART Cellulose-SB, 2*25 cm, 5 um | EtOH in Hex (8 mM NH₃.MeOH) | 450 |
| 120 | 147b | 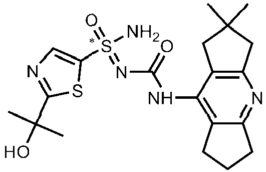 | (R) or (S)-N'-((2,2-dimethyl-1,2,3,5,6,7-hexahydrodicyclopenta[b,e]pyridi n-8-yl)carbamoyl)-2-(2-hydroxypropan-2-yl)thiazole-5-sulfonimidamide | CHIRAL ART Cellulose-SB, 2*25 cm, 5 um | EtOH in Hex (8 mM NH₃.MeOH) | 450 |
| 121 | 107a | 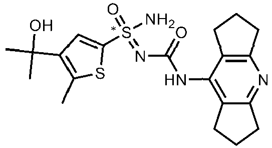 | (R) or (S)-N'-((1,2,3,5,6,7-hexahydrodicyclopenta[b,e]pyridi n-8-yl)carbamoyl)-4-(2-hydroxypropan-2-yl)-5-methylthiophene-2-sulfonimidamide | CHIRALPAK IC, 2*25 cm, 5 um | EtOH in Hex (8 mM NH₃.MeOH) | 435 |
| 122 | 107b | 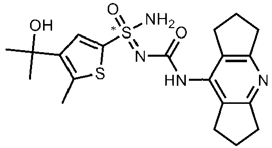 | (S) or (R)-N'-((1,2,3,5,6,7-hexahydrodicyclopenta[b,e]pyridi n-8-yl)carbamoyl)-4-(2-hydroxypropan-2-yl)-5-methylthiophene-2-sulfonimidamide | CHIRALPAK IC, 2*25 cm, 5 um | EtOH in Hex (8 mM NH₃.MeOH) | 435 |
| 123 | 145a | 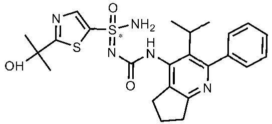 | (R) or (S)-2-(2-hydroxypropan-2-yl)-N'-((3-isopropyl-2-phenyl-6,7-dihydro-5H-cyclopenta[b]pyridin-4-yl)carbamoyl)thiazole-5-sulfonimidamide | CHIRALPAK IC, 2*25 cm, 5 um | EtOH in Hex (0.1% FA) | 500 |
| 124 | 145b | 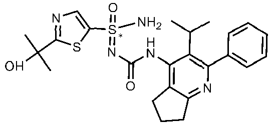 | (S) or (R)-2-(2-hydroxypropan-2-yl)-N'-((3-isopropyl-2-phenyl-6,7-dihydro-5H-cyclopenta[b]pyridin-4-yl)carbamoyl)thiazole-5-sulfonimidamide | CHIRALPAK IC, 2*25 cm, 5 um | EtOH in Hex (0.1% FA) | *500* |
| 125 | 105a | 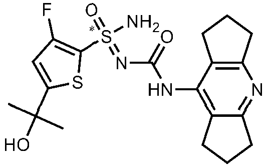 | (R) or (S)-3-fluoro-N'-((1,2,3,5,6,7-hexahydrodicyclopenta[b,e]pyridi n-8-yl)carbamoyl)-5-(2-hydroxypropan-2-yl)thiophene-2-sulfonimidamide | CHIRALPAK IG, 20*250 mm, 5 um | EtOH in Hex (8 mM NH₃.MeOH) | 439 |
| 126 | 105b | 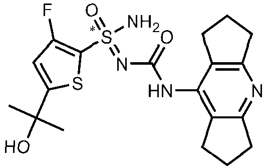 | (S) or (R)-3-fluoro-N'-((1,2,3,5,6,7-hexahydrodicyclopenta[b,e]pyridi n-8-yl)carbamoyl)-5-(2-hydroxypropan-2-yl)thiophene-2-sulfonimidamide | CHIRALPAK IG, 20*250 mm, 5 um | EtOH in Hex (8 mM NH₃.MeOH) | 439 |
| 127 | 153a | 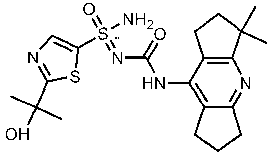 | (S) or (R)-N'-((3,3-dimethyl-1,2,3,5,6,7-hexahydrodicyclopenta[b,e]pyridi n-8-yl)carbamoyl)-2-(2-hydroxypropan-2-yl)thiazole-5-sulfonimidamide | CHIRAL ART Cellulose-SB, 2*25 cm, 5 um | EtOH in Hex (8 mM NH₃.MeOH) | 450 |
| 128 | 153b | 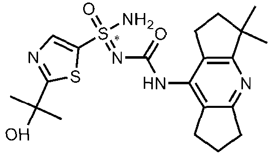 | (R) or (S)-N'-((3,3-dimethyl-1,2,3,5,6,7-hexahydrodicyclopenta[b,e]pyridi n-8-yl)carbamoyl)-2-(2-hydroxypropan-2-yl)thiazole-5-sulfonimidamide | CHIRAL ART Cellulose-SB, 2*25 cm, 5 um | EtOH in Hex (8 mM NH₃.MeOH) | 450 |
| 129 | 133a | 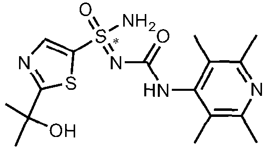 | (R) or (S)-2-(2-hydroxypropan-2-yl)-N'-((2,3,5,6-tetramethylpyridin-4-yl)carbamoyl)thiazole-5-sulfonimidamide | CHIRAL ART Cellulose-SB S-5 um, 2*25 cm, 5 um | EtOH in Hex (0.1% FA) | 398 |
| 130 | 133b | 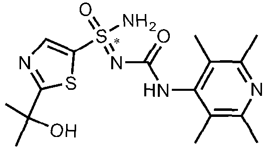 | (S) or (R)-2-(2-hydroxypropan-2-yl)-N'-((2,3,5,6-tetramethylpyridin-4-yl)carbamoyl)thiazole-5-sulfonimidamide | CHIRAL ART Cellulose-SB S-5 um, 2*25 cm, 5 um | EtOH in Hex (0.1% FA) | 398 |
| 131 | 137a | 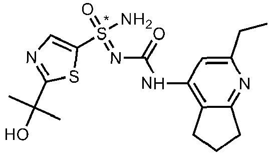 | (R) or (S)-N'-((2-ethyl-6,7-dihydro-5H-cyclopenta[b]pyridin-4-yl)carbamoyl)-2-(2-hydroxypropan-2-yl)thiazole-5-sulfonimidamide | CHIRALPAK IG, 20*250 mm, 5 um | IPA in Hex:DM=5:1 (0.1% FA) | 410 |
| 132 | 137b | 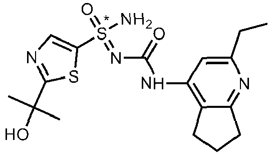 | (S) or (R)-N'-((2-ethyl-6,7-dihydro-5H-cyclopenta[b]pyridin-4-yl)carbamoyl)-2-(2-hydroxypropan-2-yl)thiazole-5-sulfonimidamide | CHIRALPAK IG, 20*250 mm, 5 um | IPA in Hex:DCM=5 :1 (0.1% FA) | 410 |
| 133 | 136a | 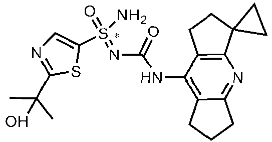 | (S) or (R)-2-(2-hydroxypropan-2-yl)-N'-((1',5',6',7'-tetrahydro-2'H-spiro[cyclopropane-1,3'-dicyclopenta[b,e]pyridin]-8'-yl)carbamoyl)thiazole-5-sulfonimidamide | CHIRAL ART Cellulose-SB, 2*25 cm, 5 um | EtOH in Hex (8 mM NH₃.MeOH) | 448 |
| 134 | 136b | 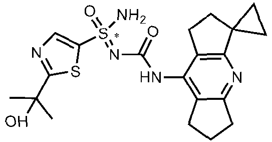 | (R) or (S)-2-(2-hydroxypropan-2-yl)-N'-((1',5',6',7'-tetrahydro-2'H-spiro[cyclopropane-1,3'-dicyclopenta[b,e]pyridin]-8'-yl)carbamoyl)thiazole-5-sulfonimidamide | CHIRAL ART Cellulose-SB, 2*25 cm, 5 um | EtOH in Hex (8 mM NH₃.MeOH) | 448 |
| 135 | 140a | 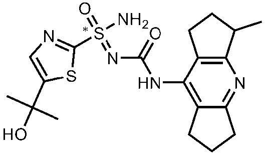 | (R, R/S) or (S, R/S)-5-(2-hydroxypropan-2-yl)-N'-((3-methyl-1,2,3,5,6,7-hexahydrodicyclopenta[b,e]pyridi n-8-yl)carbamoyl)thiazole-2-sulfonimidamide | CHIRALPAK IC, 2*25 cm, 5 um | EtOH in Hex (0.1% FA) | 436 |
| 136 | 140b | 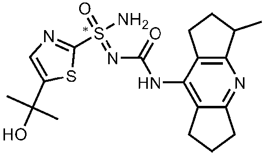 | (S, R/S) or (R, R/S)-5-(2-hydroxypropan-2-yl)-N'-((3-methyl-1,2,3,5,6,7-hexahydrodicyclopenta[b,e]pyridi n-8-yl)carbamoyl)thiazole-2-sulfonimidamide | CHIRALPAK IC, 2*25 cm, 5 um | EtOH in Hex (0.1% FA) | 436 |
| 137 | 140aa | 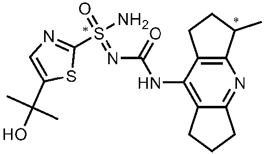 | (R, R) or (R, S)-5-(2-hydroxypropan-2-yl)-N'-((3-methyl-1,2,3,5,6,7-hexahydrodicyclopenta[b,e]pyridi n-8-yl)carbamoyl)thiazole-2-sulfonimidamide | Chiralpak AD-H, 2*25 cm (5 um); from Ex. 135 | 40% MeOH (8 mM NH₃.MeOH) in CO₂ | 436 |
| 138 | 140ab | 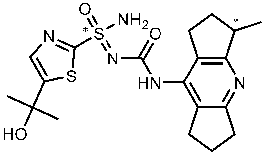 | (R, S) or (R, R)-5-(2-hydroxypropan-2-yl)-N'-((3-methyl-1,2,3,5,6,7-hexahydrodicyclopenta[b,e]pyridi n-8-yl)carbamoyl)thiazole-2-sulfonimidamide | Chiralpak AD-H, 2*25 cm (5 um); from Ex. 135 | 40% MeOH (8 mM NH₃.MeOH) in CO₂ | 436 |
| 139 | 140ba | 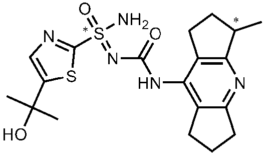 | (S, S) or (S, R)-5-(2-hydroxypropan-2-yl)-N'-((3-methyl-1,2,3,5,6,7-hexahydrodicyclopenta[b,e]pyridi n-8-yl)carbamoyl)thiazole-2-sulfonimidamide | Chiralpak AD-H, 2*25 cm (5 um); from Ex. 136 | 40% MeOH (8 mM NH₃.MeOH) in CO₂ | 436 |
| 140 | 140bb | 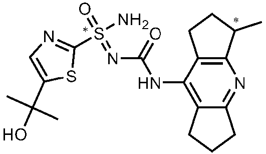 | (S, R) or (S, S)-5-(2-hydroxypropan-2-yl)-N'-((3-methyl-1,2,3,5,6,7-hexahydrodicyclopenta[b,e]pyridi n-8-yl)carbamoyl)thiazole-2-sulfonimidamide | Chiralpak AD-H, 2*25 cm (5 um); from Ex. 136 | 40% MeOH (8 mM NH₃.MeOH) in CO₂ | 436 |
| 141 | 129aa | 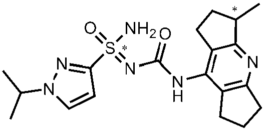 | (R, R) or (R, S)-1-isopropyl-N'-((3-methyl-1,2,3,5,6,7-hexahydrodicyclopenta[b,e]pyridi n-8-yl)carbamoyl)-1 H-pyrazole-3-sulfonimidamide | CHIRALPAK IG, 2*25 cm (5 um) | EtOH in MTBE (10 mM NH₃-MeOH) | 403 |
| 142 | 129ab | 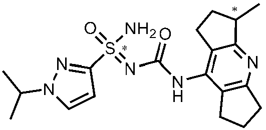 | (S, S) or (S, R)-1-isopropyl-N'-((3-methyl-1,2,3,5,6,7-hexahydrodicyclopenta[b,e]pyridi n-8-yl)carbamoyl)-1H-pyrazole-3-sulfonimidamide | CHIRALPAK IG, 2*25 cm (5 um) | EtOH in MTBE (10 mM NH₃-MeOH) | 403 |
| 143 | 129ba | 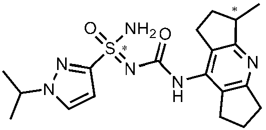 | (R, S) or (R, R)-1-isopropyl-N'-((3-methyl-1,2,3,5,6,7-hexahydrodicyclopenta[b,e]pyridi n-8-yl)carbamoyl)-1H-pyrazole-3-sulfonimidamide | CHIRALPAK IG, 2*25 cm (5 um) | EtOH in MTBE (10 mM NH₃-MEOH) | 403 |
| 144 | 129bb | 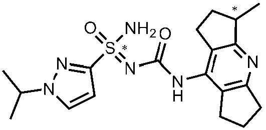 | (S, R) or (S, S)-1-isopropyl-N'-((3-methyl-1,2,3,5,6,7-hexahydrodicyclopenta[b,e]pyridi n-8-yl)carbamoyl)-1H-pyrazole-3-sulfonimidamide | CHIRALPAK IG, 2*25 cm (5 um) | EtOH in MTBE (10 mM NH₃-MEOH) | 403 |
| 145 | 127a | 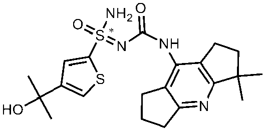 | (S) or (R)-N'-((3,3-dimethyl-1,2,3,5,6,7-hexahydrodicyclopenta[b,e]pyridi n-8-yl)carbamoyl)-4-(2-hydroxypropan-2-yl)thiophene-2-sulfonimidamide | CHIRAL ART Cellulose-SB, 2*25 cm, 5 um | EtOH in Hex (8 mM NH₃.MeOH) | 449 |
| 146 | 127b | 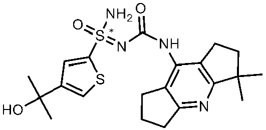 | (R) or (S)-N'-((3,3-dimethyl-1,2,3,5,6,7-hexahydrodicyclopenta[b,e]pyridi n-8-yl)carbamoyl)-4-(2-hydroxypropan-2-yl)thiophene-2-sulfonimidamide | CHIRAL ART Cellulose-SB, 2*25 cm, 5 um | EtOH in Hex (8 mM NH₃.MeOH) | 449 |
| 147 | 130a | 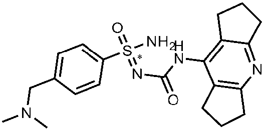 | (S) or (R)-4-((dimethylamino)methyl)-N'-((1,2,3,5,6,7-hexahydrodicyclopenta[b,e]pyridi n-8-yl)carbamoyl)benzenesulfonimid amide | CHIRAL ART Cellulose-SB, 2*25 cm, 5 um | EtOH in Hex (8 mM NH₃.MeOH) | 414 |
| 148 | 130b | 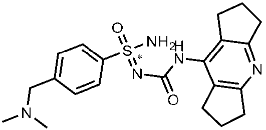 | (R) or (S)-4-((dimethylamino)methyl)-N'-((1,2,3,5,6,7-hexahydrodicyclopenta[b,e]pyridi n-8-yl)carbamoyl)benzenesulfonimid amide | CHIRAL ART Cellulose-SB, 2*25 cm, 5 um | EtOH in Hex (8 mM NH₃.MeOH) | 414 |
| 149 | 126a | 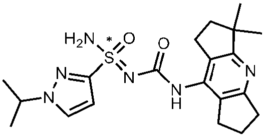 | (R) or (S)-N'-((3,3-dimethyl-1,2,3,5,6,7-hexahydrodicyclopenta[b,e]pyridi n-8-yl)carbamoyl)-1-isopropyl-1H-pyrazole-3-sulfonimidamide | CHIRALPAK IG, 20*250 mm, 5 um | EtOH in Hex (8 mM NH₃.MeOH) | 417 |
| 150 | 126b | 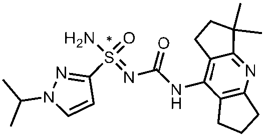 | (S) or (R)-N'-((3,3-dimethyl-1,2,3,5,6,7-hexahydrodicyclopenta[b,e]pyridi n-8-yl)carbamoyl)-1-isopropyl-1H-pyrazole-3-sulfonimidamide | CHIRALPAK IG, 20*250 mm, 5 um | EtOH in Hex (8 mM NH₃.MeOH) | 417 |
| 151 | 168a | 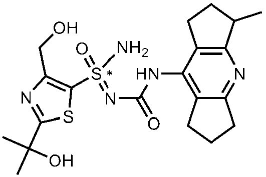 | (S, R/S) or (R, R/S)-4-(hydroxymethyl)-2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | CHIRAL ART Cellulose-SB, 2*25 cm, 5 um | EtOH in Hex (8 mM NH₃.MeOH) | 466 |
| 152 | 168b | 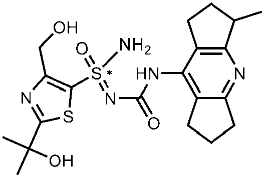 | (R, R/S) or (S, R/S)-4-(hydroxymethyl)-2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | CHIRAL ART Cellulose-SB, 2*25 cm, 5 um | EtOH in Hex (8 mM NH₃.MeOH) | 466 |
| 153 | 141b | 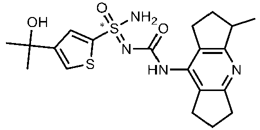 | (R, R/S) or (S, R/S) -4-(2-hydroxypropan-2-yl)-N'-((3-methyl-1,2,3,5,6,7-hexahydrodicyclopenta[b,e]pyridi n-8-yl)carbamoyl)thiophene-2-sulfonimidamide | Chiralpak IC, 2*25 cm, 5 um | EtOH in Hex (0.1% FA) | 435 |
| 154 | 141a | 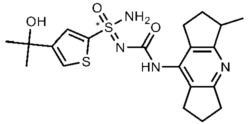 | (S, R/S) or (R, R/S) -4-(2-hydroxypropan-2-yl)-N'-((3-methyl-1,2,3,5,6,7-hexahydrodicyclopenta[b,e]pyridi n-8-yl)carbamoyl)thiophene-2-sulfonimidamide | Chiralpak IC, 2*25 cm, 5 um | EtOH in Hex (0.1% FA) | 435 |
| 155 | 141aa | 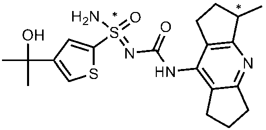 | (R, R) or (R, S)-4-(2-hydroxypropan-2-yl)-N'-((3-methyl-1,2,3,5,6,7-hexahydrodicyclopenta[b,e]pyridi n-8-yl)carbamoyl)thiophene-2-sulfonimidamide | CHIRALPAK IG, 2*25 cm (5 um); from Ex. 153 | EtOH in MTBE (10 mM NH₃-MeOH) | 435 |
| 156 | 141ab | 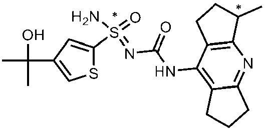 | (R, S) or (R, R)-4-(2-hydoxypropan-2-yl)-N'-((3-methyl-1,2,3,5,6,7-hexahydrodicyclopenta[b,e]pyridi n-8-yl)carbamoyl)thiophene-2-sulfonimidamide | CHIRALPAK IG, 2*25 cm (5 um); from Ex. 153 | EtOH in MTBE (10 mM NH₃-MeOH) | 435 |
| 157 | 141ba | 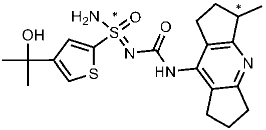 | (S, S) or (S, R)-4-(2-hydroxypropan-2-yl)-N'-((3-methyl-1,2,3,5,6,7-hexahydrodicyclopenta[b,e]pyridi n-8-yl)carbamoyl)thiophene-2-sulfonimidamide | CHIRALPAK IG, 2*25 cm (5 um); from Ex. 154 | EtOH in MTBE (10 mM NH₃-MeOH) | 435 |
| 158 | 141bb | 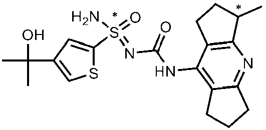 | (S, R) or (S, S)-4-(2-hydroxypropan-2-yl)-N'-((3-methyl-1,2,3,5,6,7-hexahydrodicyclopenta[b,e]pyridi n-8-yl)carbamoyl)thiophene-2-sulfonimidamide | CHIRALPAK IG, 2*25 cm (5 um); from Ex. 154 | EtOH in MTBE (10 mM NH₃-MeOH) | 435 |
| 159 | 104a | 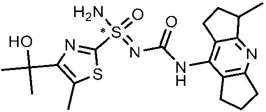 | (R, R/S) or (S, R/S)-4-(2-hydroxypropan-2-yl)-5-methyl-N'-((3-methyl-1,2,3,5,6,7-hexahydrodicyclopenta[b,e]pyridi n-8-yl)carbamoyl)thiazole-2-sulfonimidamide | CHIRALPAK IG, 20*250 mm, 5 um | EtOH in Hex (0.1% FA) | 450 |
| 160 | 104b | 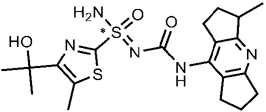 | (S, R/S) or (R, R/S)-4-(2-hydroxypropan-2-yl)-5-methyl-N'-((3-methyl-1,2,3,5,6,7-hexahydrodicyclopenta[b,e]pyridi n-8-yl)carbamoyl)thiazole-2-sulfonimidamide | CHIRALPAK IG, 20*250 mm, 5 um | EtOH in Hex (0.1% FA) | 450 |
| 161 | 168aa | 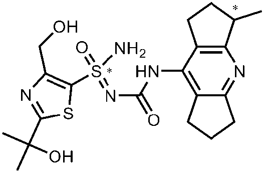 | (S, S) or (S, R)-4-(hydroxymethyl)-2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | CHIRALPAK IG, 2*25 cm (5 um) | EtOH in MTBE (10 mM NH₃-MeOH) | 466 |
| 162 | 168ab | 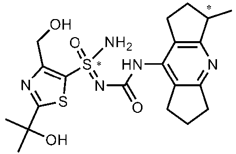 | (S, R) or (S, S)-4-(hydroxymethyl)-2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | CHIRALPAK IG, 2*25 cm (5 um) | EtOH in MTBE (10 mM NH₃-MeOH) | 466 |
| 163 | 168ab | 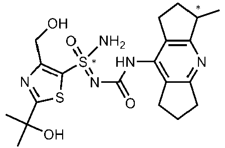 | (R, S) or (R, R)-4-(hydroxymethyl)-2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | CHIRALPAK IG, 2*25 cm (5 um) | EtOH in MTBE (10 mM NH₃-MeOH) | 466 |
| 164 | 168bb | 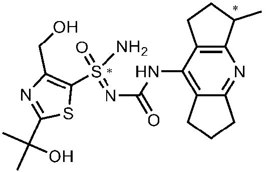 | (R, R) or (R, S)-4-(hydroxymethyl)-2-(2-hydroxypropan-2-yl)-N'-((3-methyl-1,2,3,5,6,7-hexahydrodicyclopenta[b,e]pyridi n-8-yl)carbamoyl)thiazole-5-sulfonimidamide | CHIRALPAK IG, 2*25 cm (5 um) | EtOH in MTBE (10 mM NH₃-MeOH) | 466 |
| 165 | 122a | 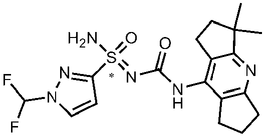 | (R) or (S)-1-(difluoromethyl)-N'-((3,3-dimethyl-1,2,3,5,6,7-hexahydrodicyclopenta[b,e]pyridi n-8-yl)carbamoyl)-1H-pyrazole-3-sulfonimidamide | CHIRALPAK IC, 2*25 cm, 5 um | EtOH in Hex (8 mM NH₃.MeOH) | 425 |
| 166 | 122b | 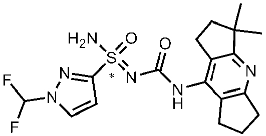 | (S) or (R)-1-(difluoromethyl)-N'-((3,3-dimethyl-1,2,3,5,6,7-hexahydrodicyclopenta[b,e]pyridi n-8-yl)carbamoyl)-1H-pyrazole-3-sulfonimidamide | CHIRALPAK IC, 2*25 cm, 5 um | EtOH in Hex (8 mM NH₃.MeOH) | 425 |
| 167 | 103aa | 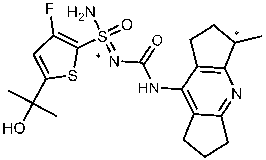 | (S, R) or (S, S)-3-fluoro-5-(2-hydroxypropan-2-yl)-N'-((3-methyl-1,2,3,5,6,7-hexahydrodicyclopenta[b,e]pyridi n-8-yl)carbamoyl)thiophene-2-sulfonimidamide | 1^{st} and 2^{nd} peaks on CHIRAL ART Cellulose-SB S, 2*25 cm, 5 um, EtOH in Hex (8 mM NH₃.MeOH); separated to individual isomers on CHIRALPAK IG, 2*25 cm, 5 um, EtOH in MTBE (10 mM NH₃-MeOH) | | 453 |
| 168 | 103ab | 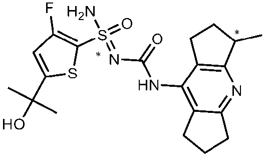 | (S, S) or (S, R)-3-fluoro-5-(2-hydroxypropan-2-yl)-N'-((3-methyl-1,2,3,5,6,7-hexahydrodicyclopenta[b,e]pyridi n-8-yl)carbamoyl)thiophene-2-sulfonimidamide | | | 453 |
| 169 | 103ba | 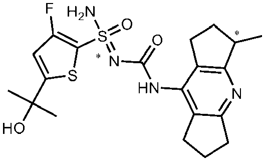 | (R, R) or (R, S)-3-fluoro-5-(2-hydroxypropan-2-yl)-N'-((3-methyl-1,2,3,5,6,7-hexahydrodicyclopenta[b,e]pyridi n-8-yl)carbamoyl)thiophene-2-sulfonimidamide | 3^{cd} and 4^{th} peaks on CHIRAL ART Cellulose-SB S, 2*25 cm, 5 um, EtOH in Hex (8 mM NH₃.MeOH); separated to individual isomers on CHIRALPAK IG, 2*25 cm, 5 um, EtOH in MTBE (10 mM NH₃-MeOH) | | 453 |
| 170 | 103bb | 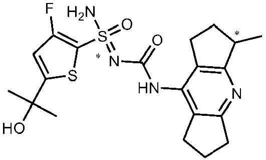 | (R, S) or (R, R)-3-fluoro-5-(2-hydroxypropan-2-yl)-N'-((3-methyl-1,2,3,5,6,7-hexahydrodicyclopenta[b,e]pyridi n-8-yl)carbamoyl)thiophene-2-sulfonimidamide | | | 453 |
| 171 | 102a | 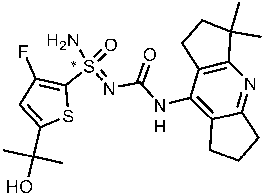 | (S) or (R)-N'-((3,3-dimethyl-1,2,3,5,6,7-hexahydrodicyclopenta[b,e]pyridi n-8-yl)carbamoyl)-3-fluoro-5-(2-hydroxypropan-2-yl)thiophene-2-sulfonimidamide | CHIRAL ART Cellulose-SB, 2*25 cm, 5 um | EtOH in Hex (8 mM NH₃.MeOH) | 467 |
| 172 | 102b | 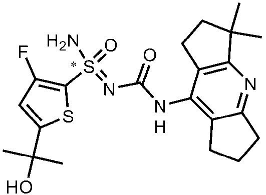 | (R) or (S)-N'-((3,3-dimethyl-1,2,3,5,6,7-hexahydrodicyclopenta[b,e]pyridi n-8-yl)carbamoyl)-3-fluoro-5-(2-hydroxypropan-2-yl)thiophene-2-sulfonimidamide | CHIRAL ART Cellulose-SB, 2*25 cm, 5 um | EtOH in Hex (8 mM NH₃.MeOH) | 467 |
| 173 | 101a | 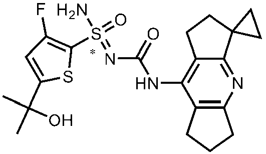 | (S) or (R)-3-fluoro-5-(2-hydroxypropan-2-yl)-N'-((1',5',6',7'-tetrahydro-2'H-spiro[cyclopropane-1,3'-dicyclopenta[b,e]pyridin]-8'-yl)carbamoyl)thiophene-2-sulfonimidamide | CHIRAL ART Cellulose-SB, 2*25 cm, 5 um | EtOH in Hex (8 mM NH₃.MeOH) | 465 |
| 174 | 101b | 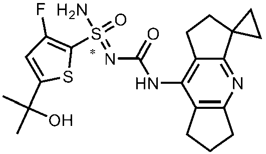 | (R) or (S)-3-fluoro-5-(2-hydroxypropan-2-yl)-N'-((1',5',6',7'-tetrahydro-2'H-spiro[cyclopropane-1,3'-dicyclopenta[b,e]pyridin]-8'-yl)carbamoyl)thiophene-2-sulfonimidamide | CHIRAL ART Cellulose-SB, 2*25 cm, 5 um | EtOH in Hex (8 mM NH₃.MeOH) | 465 |
| 175 | 125a | 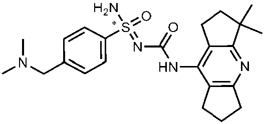 | (S) or (R)-N'-((3,3-dimethyl-1,2,3,5,6,7-hexahydrodicyclopenta[b,e]pyridi n-8-yl)carbamoyl)-4-((dimethylamino)methyl)benzene sulfonimidamide | CHIRAL ART Cellulose-SB, 2*25 cm, 5 um | EtOH in Hex (8 mM NH₃.MeOH) | 442 |
| 176 | 125b | 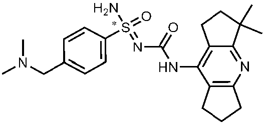 | (R) or (S)-N'-((3,3-dimethyl-1,2,3,5,6,7-hexahydrodicyclopenta[b,e]pyridi n-8-yl)carbamoyl)-4-((dimethylamino)methyl)benzene | CHIRAL ART Cellulose-SB, 2*25 cm, 5 um | EtOH in Hex (8 mM NH₃.MeOH) | 442 |
| | | | sulfonimidamide | | | |
| 177 | 132a | 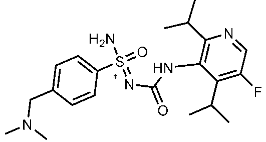 | (R) or (S)-4-((dimethylamino)methyl)-N'-((5-fluoro-2,4-diisopropylpyridin-3-yl)carbamoyl)benzenesulfonimid amide | CHIRALPAK IG, 20*250 mm, 5 um | Hex (0.1% DEA):EtOH =70:30 | 436 |
| 178 | 132b | 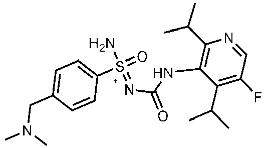 | (S) or (R)-4-((dimethylamino)methyl)-N'-((5-fluoro-2,4-diisopropylpyridin-3-yl)carbamoyl)benzenesulfonimid amide | CHIRALPAK IG, 20*250 mm, 5 um | Hex (0.1% DEA):EtOH =70:30 | 436 |
| 179 | 131b | 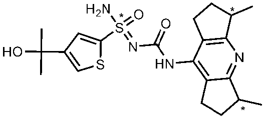 | (S) and (S, R, R) and (S, S, S) - N'-((3,5-dimethyl-1,2,3,5,6,7-hexahydrodicyclopenta[b,e]pyridi n-8-yl)carbamoyl)-4-(2-hydroxypropan-2-yl)thiophene-2-sulfonimidamide | 1^{st}, 2^{nd}, 3^{cd} peak ( three isomers) CHIRAL ART Cellulose-SB, 2*25 cm, 5 um | EtOH in Hex (8 mM NH₃.MeOH) | 449 |
| 180 | 131a | 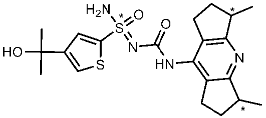 | (R, S, S) and (R, R, R) -N'-((3,5-dimethyl-1,2,3,5,6,7-hexahydrodicyclopenta[b,e]pyridi n-8-yl)carbamoyl)-4-(2-hydroxypropan-2-yl)thiophene-2-sulfonimidamide | 4^{th}, 5^{th} peaks ( two isomers) CHIRAL ART Cellulose-SB, 2*25 cm, 5 um | EtOH in Hex (8 mM NH₃.MeOH) | 449 |
| 181 | 131aab | 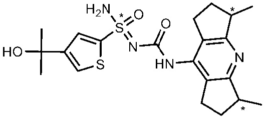 | (R, R, S) and (R, S, R)-N'-((3,5-dimethyl-1,2,3,5,6,7-hexahydrodicyclopenta[b,e]pyridi n-8-yl)carbamoyl)-4-(2-hydroxypropan-2-yl)thiophene-2-sulfonimidamide | 6^{th} peak CHIRAL ART Cellulose-SB, 2*25 cm, 5 um | EtOH in Hex (8 mM NH₃.MeOH) | 449 |
| 182 | 131c | 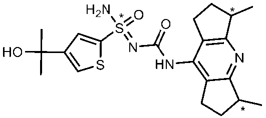 | (S, R, S) and (S, S, R) or (S, R, R) or (S, S, S) -N'-((3,5-dimethyl-1,2,3,5,6,7-hexahydrodicyclopenta[b,e]pyridi n-8-yl)carbamoyl)-4-(2-hydroxypropan-2-yl)thiophene-2-sulfonimidamide | 1^{st} perk from Ex. 179; CHIRALPAK IE, 2*25 cm, 5 um | EtOH in Hex (8 mM NH₃.MeOH) | 449 |
| 183 | 131d | 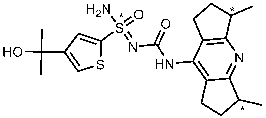 | (S, R, R) or (S, S, S) or (S, R, S) and (S, S, R)-N'-((3,5-dimethyl-1,2,3,5,6,7-hexahydrodicyclopenta[b,e]pyridi n-8-yl)carbamoyl)-4-(2-hydroxypropan-2-yl)thiophene-2-sulfonimidamide | 2^{nd} perk from Ex. 179; CHIRALPAK IE, 2*25 cm, 5 um | EtOH in Hex (8 mM NH₃.MeOH) | 449 |
| 184 | 131e | 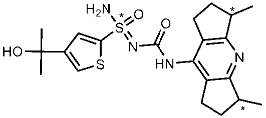 | (S, S, S) or (S, R, S) and (S, S, R) or (S, R, R)-N'-((3,5-dimethyl-1,2,3,5,6,7-hexahydrodicyclopenta[b,e]pyridi n-8-yl)carbamoyl)-4-(2-hydroxypropan-2-yl)thiophene-2-sulfonimidamide | 3^{cd} perk from Ex. 179; CHIRALPAK IE, 2*25 cm, 5 um | EtOH in Hex (8 mM NH₃.MeOH) | 449 |
| 185 | 131f | 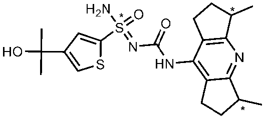 | (R, S, S) or (R, R, R)-N'-((3,5-dimethyl-1,2,3,5,6,7-hexahydrodicyclopenta[b,e]pyridi n-8-yl)carbamoyl)-4-(2-hydroxypropan-2-yl)thiophene-2-sulfonimidamide | 1^{st} perk from Ex. 180; CHIRALPAK AD-H-TC001 SFC, 2*25 cm, 5 um | MeOH (2 mM NH₃-MeOH) in CO₂ | 449 |
| 186 | 131g | 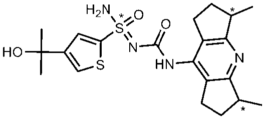 | (R, R, R) or (R, S, S)-N'-((3,5-dimethyl-1,2,3,5,6,7-hexahydrodicyclopenta[b,e]pyridi n-8-yl)carbamoyl)-4-(2-hydroxypropan-2-yl)thiophene-2-sulfonimidamide | 2^{nd} perk resulted from Ex. 180 CHIRALPAK AD-H-TC001 SFC, 2*25 cm, 5 um | MeOH (2 mM NH₃-MeOH) in CO₂ | 449 |
| 187 | 121a | 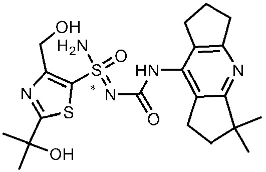 | (R) or (S)-N'-((3,3-dimethyl-1,2,3,5,6,7-hexahydrodicyclopenta[b,e]pyridi n-8-yl)carbamoyl)-4-(hydroxymethyl)-2-(2-hydroxypropan-2-yl)thiazole-5-sulfonimidamide | CHIRALPAK IG, 20*250 mm, 5 um | 30% MeOH and 70% CO₂ | 480 |
| 188 | 121b | 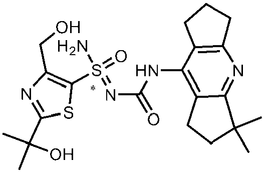 | (S) or (R)-N'-((3,3-dimethyl-1,2,3,5,6,7-hexahydrodicyclopenta[b,e]pyridi n-8-yl)carbamoyl)-4-(hydroxymethyl)-2-(2-hydroxypropan-2-yl)thiazole-5-sulfonimidamide | CHIRALPAK IG, 20*250 mm, 5 um | 30% MeOH and 70% CO₂ | 480 |
| 189 | 169a | 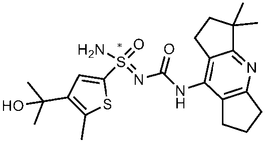 | (S) or (R)-N'-((3,3-dimethyl-1,2,3,5,6,7-hexahydrodicyclopenta[b,e]pyridi n-8-yl)carbamoyl)-4-(2-hydroxypropan-2-yl)-5-methylthiophene-2-sulfonimidamide | CHIRAL ART Cellulose-SB, 2*25 cm, 5 um | EtOH in Hex (8 mM NH₃.MeOH) | 463 |
| 190 | 169b | 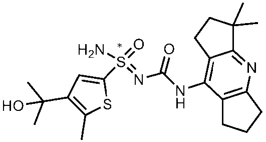 | (R) or (S)-N'-((3,3-dimethyl-1,2,3,5,6,7-hexahydrodicyclopenta[b,e]pyridi n-8-yl)carbamoyl)-4-(2-hydroxypropan-2-yl)-5-methylthiophene-2-sulfonimidamide | CHIRAL ART Cellulose-SB, 2*25 cm, 5 um | EtOH in Hex (8 mM NH₃.MeOH) | 463 |
| 191 | 119a | 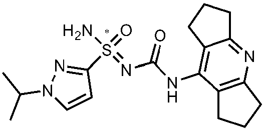 | (R) or (S)-N'-((1,2,3,5,6,7-hexahydrodicyclopenta[b,e]pyridi n-8-yl)carbamoyl)-1-isopropyl-1H-pyrazole-3-sulfonimidamide | CHIRALPAK IG, 20*250mm,5 um | EtOH in Hex (8 mM NH₃.MeOH) | 389 |
| 192 | 119b | 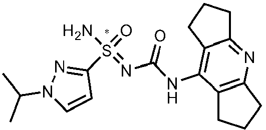 | (S) or (R)-N'-((1,2,3,5,6,7-hexahydrodicyclopenta[b,e]pyridi n-8-yl)carbamoyl)-1-isopropyl-1H-pyrazole-3-sulfonimidamide | CHIRALPAK IG, 20*250 mm, 5 um | EtOH in Hex (8 mM NH₃.MeOH) | 389 |
| 193 | 118a | 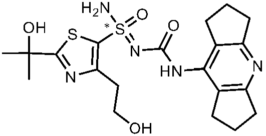 | (R) or (S)-N'-((1,2,3,5,6,7-hexahydrodicyclopenta[b,e]pyridi n-8-yl)carbamoyl)-4-(2-hydroxyethyl)-2-(2-hydroxypropan-2-yl)thiazole-5-sulfonimidamide | CHIRALPAK IF, 2*25 cm, 5 um | EtOH in Hex (8 mM NH₃.MeOH) | 466 |
| 194 | 118b | 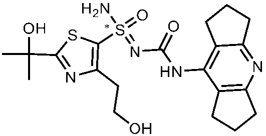 | (S) or (R)-N'-((1,2,3,5,6,7-hexahydrodicyclopenta[b,e]pyridi n-8-yl)carbamoyl)-4-(2-hydroxyethyl)-2-(2-hydroxypropan-2-yl)thiazole-5-sulfonimidamide | CHIRALPAK IF, 2*25 cm, 5 um | EtOH in Hex (8 mM NH₃.MeOH) | 466 |
| 195 | 134aa | 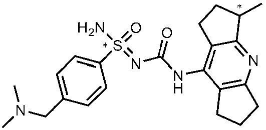 | (S, S) or (S, R)-4-((dimethylamino)methyl)-N'-((3-methyl-1,2,3,5,6,7-hexahydrodicyclopenta[b,e]pyridi n-8-yl)carbamoyl)benzenesulfonimid amide | CHIRALPAK IG, 2*25 cm (5 um) | EtOH in Hex (8 mM NH₃.MeOH) | 428 |
| 196 | 134ab | 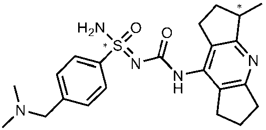 | (S, R) or (S, S)-4-((dimethylamino)methyl)-N'-((3-methyl-1,2,3,5,6,7-hexahydrodicyclopenta[b,e]pyridi n-8-yl)carbamoyl)benzenesulfonimid amide | CHIRALPAK IG, 2*25 cm (5 um) | EtOH in Hex (8 mM NH₃.MeOH) | 428 |
| 197 | 134ba | 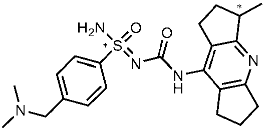 | (R, R) or (R, S)-4-((dimethylamino)methyl)-N'-((3-methyl-1,2,3,5,6,7-hexahydrodicyclopenta[b,e]pyridi n-8-yl)carbamoyl)benzenesulfonimid amide | CHIRAL ART Cellulose-SB, 2*25 cm, 5 um | EtOH in Hex (8 mM NH₃.MeOH) | 428 |
| 198 | 134bb | 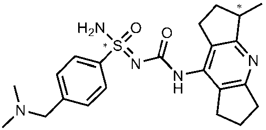 | (R, S) or (R, R)-4-((dimethylamino)methyl)-N'-((3-methyl-1,2,3,5,6,7-hexahydrodicyclopenta[b,e]pyridi n-8-yl)carbamoyl)benzenesulfonimid amide | CHIRAL ART Cellulose-SB, 2*25 cm, 5 um | EtOH in Hex (8 mM NH₃.MeOH) | 428 |
| 199 | 117a | 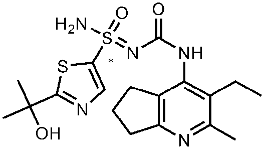 | (S) or (R)-N'-((3-ethyl-2-methyl-6,7-dihydro-5H-cyclopenta[b]pyridin-4-yl)carbamoyl)-2-(2-hydroxypropan-2-yl)thiazole-5-sulfonimidamide | CHIRAL ART Cellulose-SB, 2*25 cm, 5 um | EtOH in Hex (8 mM NH₃.MeOH) | 424 |
| 200 | 117b | 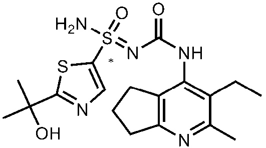 | (R) or (S)-N'-((3-ethyl-2-methyl-6,7-dihydro-5H-cyclopenta[b]pyridin-4-yl)carbamoyl)-2-(2-hydroxypropan-2-yl)thiazole-5-sulfonimidamide | CHIRAL ART Cellulose-SB, 2*25 cm, 5 um | EtOH in Hex (8 mM NH₃.MeOH) | 424 |
| 201 | 172a | 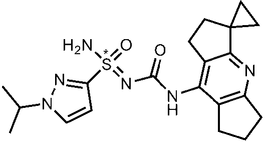 | (S) or (R)-1-Isopropyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPAK AS-H, 2*25 cm (5 um) | EtOH (2 mM NH₃-MeOH) in CO₂ | 415 |
| 202 | 172b | 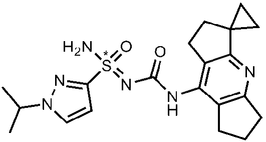 | (R) or (S)-1-Isopropyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPAK AS-H, 2*25 cm (5 um) | EtOH (2 mM NH₃-MeOH) in CO₂ | 415 |
| 203 | 173a | 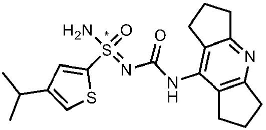 | (R) or (S)-N'-((1,2,3,5,6,7-hexahydrodicyclopenta[b,e]pyridi n-8-yl)carbamoyl)-4-isopropylthiophene-2-sulfonimidamide | CHIRALPAK IG, 2*25 cm (5 um) | EtOH in Hex (8 mM NH₃.MeOH) | 405 |
| 204 | 173b | 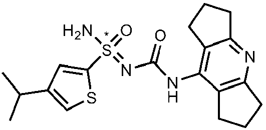 | (S) or (R)-N'-((1,2,3,5,6,7-hexahydrodicyclopenta[b,e]pyridi n-8-yl)carbamoyl)-4-isopropylthiophene-2-sulfonimidamide | CHIRALPAK IG, 2*25 cm (5 um) | EtOH in Hex (8 mM NH₃.MeOH) | 405 |
| 205 | 183a | 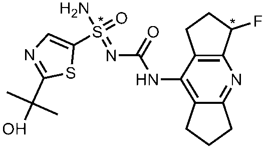 | (R, R) or (R, S)-N'-((3-fluoro-1,2,3,5,6,7-hexahydrodicyclopenta[b,e]pyridi n-8-yl)carbamoyl)-2-(2-hydroxypropan-2-yl)thiazole-5-sulfonimidamide | CHIRALPAK IC, 2*25 cm, 5 um | EtOH in Hex (0.1% FA) | 440 |
| 206 | 183b | 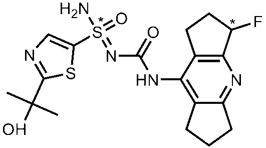 | (S, S) or (S, R)-N'-((3-fluoro-1,2,3,5,6,7-hexahydrodicyclopenta[b,e]pyridi n-8-yl)carbamoyl)-2-(2-hydroxypropan-2-yl)thiazole-5-sulfonimidamide | CHIRALPAK IC, 2*25 cm, 5 um | EtOH in Hex (0.1% FA) | 440 |
| 207 | 183c | 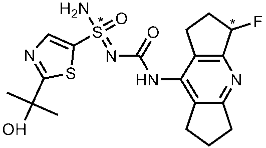 | (R, S) or (R, R)-N'-((3-fluoro-1,2,3,5,6,7-hexahydrodicyclopenta[b,e]pyridi n-8-yl)carbamoyl)-2-(2-hydroxypropan-2-yl)thiazole-5-sulfonimidamide | CHIRALPAK IC, 2*25 cm, 5 um | EtOH in Hex (0.1% FA) | 440 |
| 208 | 183d | 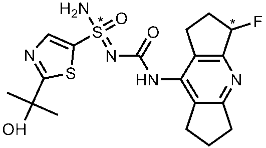 | (S, R) or (S, S)-N'-((3-fluoro-1,2,3,5,6,7-hexahydrodicyclopenta[b,e]pyridi n-8-yl)carbamoyl)-2-(2-hydroxypropan-2-yl)thiazole-5-sulfonimidamide | CHIRALPAK IC, 2*25 cm, 5 um | EtOH in Hex (0.1% FA) | 440 |
| 209 | 116a | 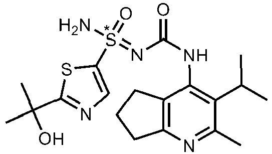 | (S) or (R)-2-(2-hydroxypropan-2-yl)-N'-((3-isopropyl-2-methyl-6,7-dihydro-5H-cyclopenta[b]pyridin-4-yl)carbamoyl)thiazole-5-sulfonimidamide | CHIRALPAK IE, 2*25 cm, 5 um | EtOH in MTBE (0.1% FA) | 438 |
| 210 | 116b | 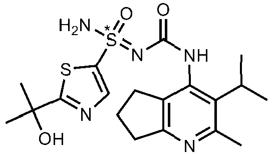 | (R) or (S)-2-(2-hydroxypropan-2-yl)-N'-((3-isopropyl-2-methyl-6,7-dihydro-5H-cyclopenta[b]pyridin-4-yl)carbamoyl)thiazole-5-sulfonimidamide | CHIRALPAK IE, 2*25 cm, 5 um | EtOH in MTBE (0.1% FA) | 438 |
| 211 | 114a | 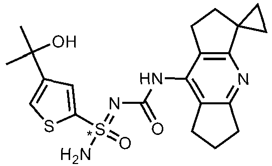 | (R) or (S)-4-(2-hydroxypropan-2-yl)-N'-((1',5',6',7'-tetrahydro-2'H-spiro[cyclopropane-1,3'-dicyclopenta[b,e]pyridin]-8'-yl)carbamoyl)thiophene-2-sulfonimidamide | CHIRALPAK IG, 2*25 cm (5 um) | EtOH in Hex (8 mM NH₃.MeOH) | 447 |
| 212 | 114b | 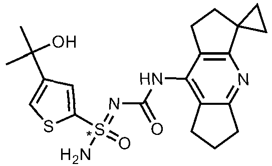 | (S) or (R)-4-(2-hydroxypropan-2-yl)-N'-((1',5',6',7'-tetrahydro-2'H-spiro[cyclopropane-1,3'-dicyclopenta[b,e]pyridin]-8'-yl)carbamoyl)thiophene-2-sulfonimidamide | CHIRALPAK IG, 2*25 cm (5 um) | EtOH in Hex (8 mM NH₃.MeOH) | 447 |
| 213 | 124a | 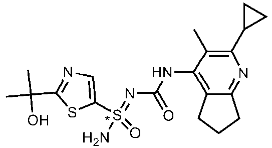 | (S) or (R)-N'-((2-cyclopropyl-3-methyl-6,7-dihydro-5H-cyclopenta[b]pyridin-4-yl)carbamoyl)-2-(2-hydroxypropan-2-yl)thiazole-5-sulfonimidamide | CHIRAL ART Cellulose-SB, 2*25 cm, 5 um | EtOH in Hex (8 mM NH₃.MeOH) | 436 |
| 214 | 124b | 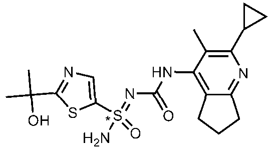 | (R) or (S)-N'-((2-cyclopropyl-3-methyl-6,7-dihydro-5H-cyclopenta[b]pyridin-4-yl)carbamoyl)-2-(2-hydroxypropan-2-yl)thiazole-5-sulfonimidamide | CHIRAL ART Cellulose-SB, 2*25 cm, 5 um | EtOH in Hex (8 mM NH₃.MeOH) | 436 |
| 215 | 154a | 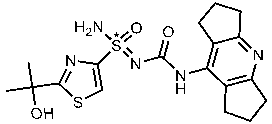 | (R) or (S)-N'-((1,2,3,5,6,7-hexahydrodicyclopenta[b,e]pyridi n-8-yl)carbamoyl)-2-(2-hydroxypropan-2-yl)thiazole-4-sulfonimidamide | CHIRALPAK IC, 2*25 cm, 5 um | EtOH in Hex (8 mM NH₃.MeOH) | 422 |
| 216 | 154b | 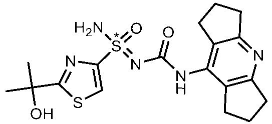 | (S) or (R)-N'-((1,2,3,5,6,7-hexahydrodicyclopenta[b,e]pyridi n-8-yl)carbamoyl)-2-(2-hydroxypropan-2-yl)thiazole-4-sulfonimidamide | CHIRALPAK IC, 2*25 cm, 5 um | EtOH in Hex (8 mM NH₃.MeOH) | 422 |
| 217 | 120a | 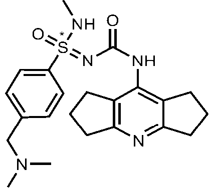 | (R) or (S)-4-((dimethylamino)methyl)-N'-((1,2,3,5,6,7-hexahydrodicyclopenta[b,e]pyridi n-8-yl)carbamoyl)-N-methylbenzenesulfonimidamide | CHIRALPAK IE, 2*25 cm, 5 um | EtOH in MTBE (10 mM NH₃-MeOH) | 428 |
| 218 | 120b | 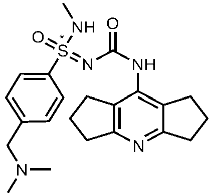 | (S) or (R)-4-((dimethylamino)methyl)-N'-((1,2,3,5,6,7-hexahydrodicyclopenta[b,e]pyridi n-8-yl)carbamoyl)-N-methylbenzenesulfonimidamide | CHIRALPAK IE, 2*25 cm, 5 um | EtOH in MTBE (10 mM NH₃-MeOH) | 428 |
| 219 | 142a | 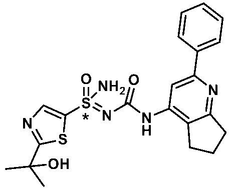 | (R) or (S)-2-(2-hydroxypropan-2-yl)-N'-((2-phenyl-6,7-dihydro-SH-cyclopenta[b]pyridin-4-yl)carbamoyl)thiazole-5-sulfonimidamide | CHIRALPAK IF, 2*25 cm, 5 um | EtOH in Hex (0.1% FA) | 458 |
| 220 | 142b | 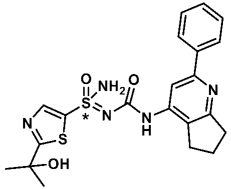 | (S) or (R)-2-(2-hydroxypropan-2-yl)-N'-((2-phenyl-6,7-dihydro-SH-cyclopenta[b]pyridin-4-yl)carbamoyl)thiazole-5-sulfonimidamide | CHIRALPAK IF, 2*25 cm, 5 um | EtOH in Hex (0.1% FA) | 458 |
| 221 | 143a | 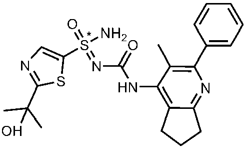 | (S) or (R)-2-(2-hydroxypropan-2-yl)-N'-((3-methyl-2-phenyl-6,7-dihydro-5H-cyclopenta[b]pyridin-4-yl)carbamoyl)thiazole-5-sulfonimidamide | CHIRAL ART Cellulose-SB S-5um, 2*25 cm, 5 um | EtOH in Hex (0.1% FA) | 472 |
| 222 | 143b | 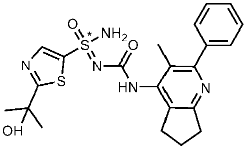 | (R) or (S)-2-(2-hydroxypropan-2-yl)-N'-((3-methyl-2-phenyl-6,7-dihydro-5H-cyclopenta[b]pyridin-4-yl)carbamoyl)thiazole-5-sulfonimidamide | CHIRAL ART Cellulose-SB S-5um, 2*25 cm, 5 um | EtOH in Hex (0.1% FA) | 472 |
| 223 | 184a | 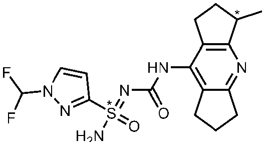 | (R, R) or (R, S)-1-(difluoromethyl)-N'-((3-methyl-1,2,3,5,6,7-hexahydrodicyclopenta[b,e]pyridi n-8-yl)carbamoyl)-1H-pyrazole-3-sulfonimidamide | 1^{st} peak Column, CHIRALPAK IG, 20*250 mm, 5 um | EtOH in MTBE (10 mM NH₃-MeOH) | 411 |
| 224 | 184b | 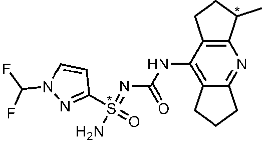 | (S, S) or (S, R)-1-(difluoromethyl)-N'-((3-methyl-1,2,3,5,6,7-hexahydrodicyclopenta[b,e]pyridi n-8-yl)carbamoyl)-1H-pyrazole-3-sulfonimidamide | Eluted as a mixture of 2^{nd} and 3^{rd} peaks CHIRAPAK IG, 20*250 mm, 5 um. EtOH in MTBE (10 mM NH₃-MeOH). Separated to individual isomers on CHIRALPAK IG, 2*25 cm (5 um), EtOH in Hex (8 mM NH₃-MeOH) | | 411 |
| 225 | 184c | 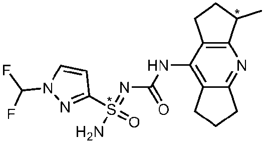 | (R, S) or (R, R)-1-(difluoromethyl)-N'-((3-methyl-1,2,3,5,6,7-hexahydrodicyclopenta[b,e]pyridi n-8-yl)carbamoyl)-1H-pyrazole-3-sulfonimidamide | | | 411 |
| 226 | 184d | 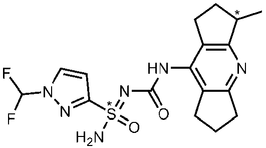 | (S, R) or (S, S)-1-(difluoromethyl)-N'-((3-methyl-1,2,3,5,6,7-hexahydrodicyclopenta[b,e]pyridi n-8-yl)carbamoyl)-1H-pyrazole-3-sulfonimidamide | Column, CHIRALPAK IG, 20*250 mm, 5 um | EtOH in MTBE (10 mM NH₃-MeOH) | 411 |
| 227 | 174a | 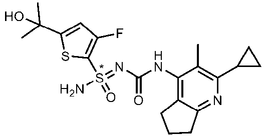 | (R) or (S)-N'-((2-cyclopropyl-3-methyl-6,7-dihydro-5H-cyclopenta[b]pyridin-4-yl)carbamoyl)-3-fluoro-5-(2-hydroxypropan-2-yl)thiophene-2-sulfonimidamide | CHIRAL ART Cellulose-SB, 2*25 cm, 5 um | IPA in Hex (8 mM NH₃.MeOH) | 453 |
| 228 | 174b | 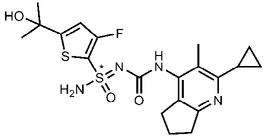 | (S) or (R)-N'-((2-cyclopropyl-3-methyl-6,7-dihydro-5H-cyclopenta[b]pyridin-4-yl)carbamoyl)-3-fluoro-5-(2-hydroxypropan-2-yl)thiophene-2-sulfonimidamide | CHIRAL ART Cellulose-SB, 2*25 cm, 5 um | IPA in Hex (8 mM NH₃.MeOH) | 453 |
| 229 | 175a | 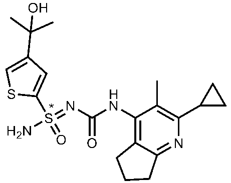 | (R) or (S)-N'-((2-cyclopropyl-3-methyl-6,7-dihydro-5H-cyclopenta[b]pyridin-4-yl)carbamoyl)-4-(2-hydroxypropan-2-yl)thiophene-2-sulfonimidamide | CHIRAL ART Cellulose-SB, 2*25 cm, 5 um | IPA in Hex (8 mM NH₃.MeOH) | 435 |
| 230 | 175b | 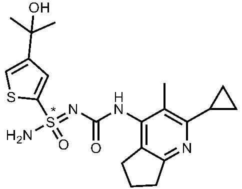 | (S) or (R)-N'-((2-cyclopropyl-3-methyl-6,7-dihydro-5H-cyclopenta[b]pyridin-4-yl)carbamoyl)-4-(2-hydroxypropan-2-yl)thiophene-2-sulfonimidamide | CHIRAL ART Cellulose-SB, 2*25 cm, 5 um | IPA in Hex (8 mM NH₃.MeOH) | 435 |
| 231 | 180a | 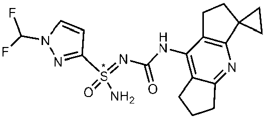 | (R) or (S)-1-(difluoromethyl)-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPAK IG, 20*250 mm, 5 um | MeOH(2mM NH₃-MeOH) in CO₂ | 423 |
| 232 | 180b | 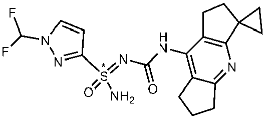 | (S) or (R)-1-(difluoromethyl)-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPAK IG, 20*250 mm, 5 um | MeOH(2mM NH₃-MeOH) in CO₂ | 423 |

| | | | | | | |
|---|---|---|---|---|---|---|
| ^{#} The amount of NH₃ in this chiral chromatographic solvent and similar solvents were adjusted by adding 2 M NH₃ in methanol to the desired NH₃ concentration. In this case, the resulting concentration of NH₃ in methanol is 8 mM. | | | | | | |

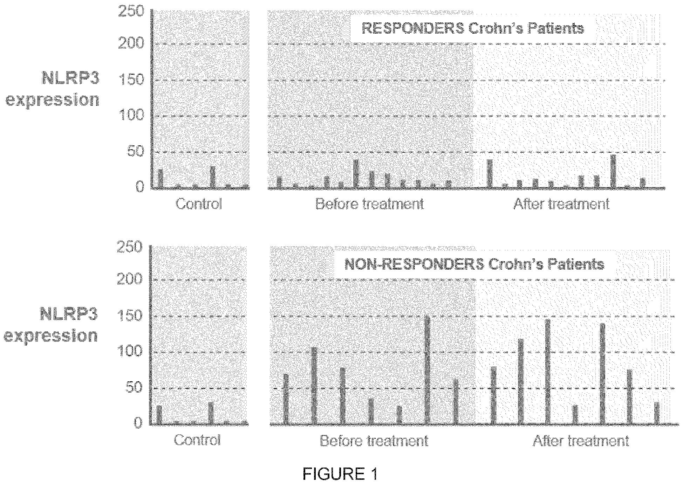

Single crystal X-ray crystallographic analysis was performed on compound **162bb** (Example 107 shown in Table 23 above). FIG 1 shows ball and stick models of the asymmetrical unit containing two crystallographically independent molecules of compound **162bb,** with hydrogen atoms omitted for clarity. Table M below shows fractional atomic coordinates of compound **162bb.** The X-ray crystal structure data of compound **162bb** shows (R) configuration at both the sulfur and carbon stereocenters.

**Table 25.**

| **Fractional Atomic Coordinates (×10⁴) and Equivalent Isotropic Displacement Parameters (Å²×10³) for Example 107. U_{eq} is defined as 1/3 of the trace of the orthogonalised U_{IJ} tensor.** | | | | |
|---|---|---|---|---|
| **Atom** | ***x*** | ***y*** | ***z*** | **U(eq)** |
| S4 | 5075(6) | 4544(8) | 4537(3) | 36.8(19) |
| S2 | 4781(6) | 4698(9) | -473(3) | 43(2) |
| S1 | 7558(7) | 5846(10) | -529(3) | 50(2) |
| S3 | 2293(7) | 5663(9) | 4517(3) | 46(2) |
| O2 | 4520(16) | 6060(20) | -694(8) | 39(5) |
| O5 | 5510(15) | 3200(20) | 4347(7) | 31(4) |
| O3 | 4619(17) | 5920(20) | 674(8) | 45(5) |
| N3 | 4140(18) | 4020(20) | 66(9) | 25(5) |
| O6 | 5356(18) | 3350(20) | 5721(9) | 50(5) |
| N9 | 5338(18) | 5480(30) | 6075(9) | 36(6) |
| N8 | 5550(20) | 5180(30) | 5117(11) | 48(7) |
| N4 | 4450(20) | 3850(30) | 1039(11) | 51(7) |
| O4 | -270(20) | 6350(30) | 4492(10) | 57(6) |
| N7 | 5257(19) | 5700(30) | 4048(10) | 37(6) |
| O1 | 10130(20) | 6580(30) | -616(10) | 66(7) |
| N2 | 4410(20) | 3530(30) | -970(9) | 32(5) |
| C00H | 5460(20) | 4710(30) | 5620(11) | 31(6) |
| C20 | 1070(20) | 4520(30) | 4713(11) | 35(7) |
| N10 | 5083(19) | 4460(20) | 7857(6) | 75(9) |
| C32 | 6064(18) | 3890(20) | 7545(8) | 66(10) |
| C33 | 6147(16) | 4230(20) | 6957(8) | 46(8) |
| C26 | 5250(17) | 5130(20) | 6679(6) | 41(7) |
| C27 | 4269(16) | 5700(20) | 6991(8) | 46(8) |
| C31 | 4186(16) | 5360(20) | 7579(8) | 47(8) |
| N1 | 8450(20) | 3680(30) | 38(10) | 40(6) |
| C2 | 6540(30) | 4700(40) | -317(13) | 47(7) |
| N6 | 1480(20) | 3260(30) | 4781(10) | 43(6) |
| C7 | 4370(30) | 4560(40) | 561(14) | 54(8) |
| C4 | 10220(20) | 5300(30) | -295(12) | 38(7) |
| C16 | 7300(30) | 5850(40) | 2595(15) | 59(9) |
| C21 | 3350(30) | 4350(40) | 4584(13) | 46(8) |
| C14 | 5804(16) | 4870(20) | 1845(8) | 45(8) |
| C8 | 4666(18) | 4160(20) | 1669(7) | 43(8) |
| C9 | 3784(16) | 3830(30) | 2079(9) | 66(10) |
| C13 | 4039(19) | 4200(30) | 2665(8) | 81(12) |
| N5 | 5180(20) | 4910(30) | 2841(7) | 78(9) |
| C15 | 6059(17) | 5240(30) | 2431(9) | 83(12) |
| C18 | 7080(30) | 5340(40) | 1585(13) | 46(8) |
| C22 | 2820(20) | 3160(40) | 4759(12) | 37(7) |
| C25 | -750(30) | 4770(50) | 5293(15) | 63(9) |
| C6 | 10910(30) | 5480(50) | 336(16) | 69(10) |
| C34 | 7230(30) | 3470(50) | 6749(15) | 66(10) |
| C5 | 10840(30) | 4200(50) | -671(17) | 74(11) |
| C30 | 3160(40) | 6000(50) | 7827(19) | 92(13) |
| C28 | 3190(30) | 6640(50) | 6797(16) | 70(11) |
| C23 | -290(30) | 4940(40) | 4703(13) | 49(8) |
| C3 | 7110(30) | 3580(40) | -17(12) | 43(8) |
| C17 | 7830(40) | 6370(50) | 2016(18) | 88(13) |
| C36 | 7060(30) | 2850(50) | 7758(16) | 70(11) |
| C35 | 7990(40) | 2950(50) | 7343(17) | 82(12) |
| C1 | 8870(20) | 4850(30) | -219(12) | 39(7) |
| C24 | -1130(30) | 4170(50) | 4203(16) | 71(11) |
| C12 | 2860(40) | 3740(60) | 3000(20) | 92(14) |
| C29 | 2320(40) | 6600(50) | 7354(17) | 79(12) |
| C10 | 2690(40) | 2920(50) | 1935(19) | 89(13) |
| C37 | 7650(60) | 2820(80) | 8380(30) | 160(30) |
| C11 | 2050(50) | 3380(70) | 2530(20) | 112(17) |
| C19 | 7290(50) | 7110(60) | 3030(20) | 113(17) |

### Example 233 (Compound 652)

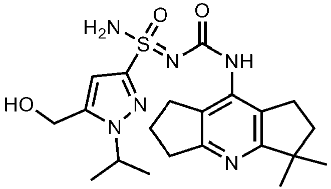

### N'-((3,3-dimethyl-1,2,3,5,6,7-hexahydrodicyclopenta[b,e]pyridin-8-yl)carbamoyl)-5-(hydroxymethyl)-1-isopropyl-1H-pyrazole-3-sulfonimidamide (Scheme I)

### Examples 234 (Compound 652b) and 235 (Compound 652a)

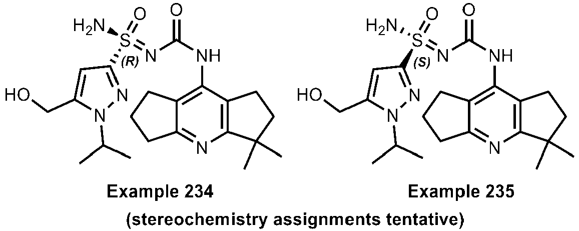

### (R)- and (S)-N'-((3,3-dimethyl-1,2,3,5,6,7-hexahydrodicyclopenta[b,e]pyridin-8-yl)carbamoyl)-5- (hydroxymethyl)-1-isopropyl-1H-pyrazole-3-sulfonimidamide

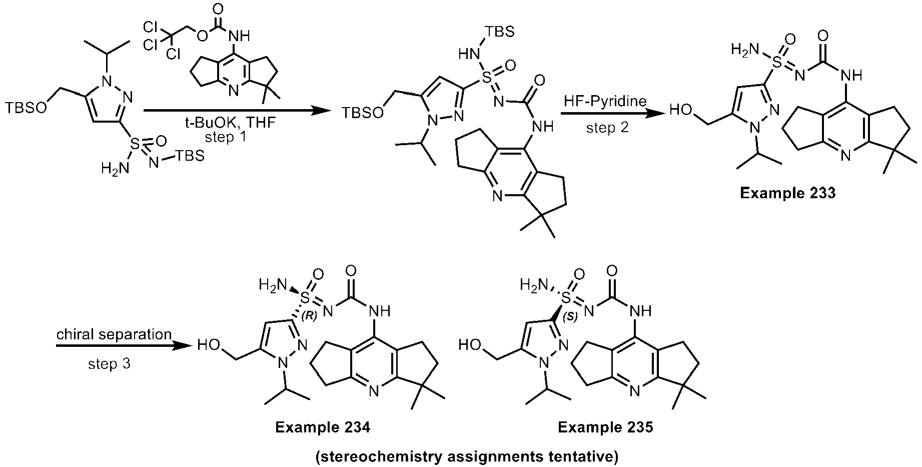

### Step 1: N-(tert-butyldimethylsilyl)-5-(((tert-butyldimethylsilyl)oxy)methyl)-N'-((3,3-dimethyl-1,2,3,5,6,7-hexahydrodicyclopenta[b,e]pyridin-8-yl)carbamoyl)-1-isopropyl-1-pyrazole-3-sulfonimidamide

To a stirred solution of N'-(tert-butyldimethylsilyl)-5-(((tert-butyldimethylsilyl)oxy)methyl)-1-isopropyl-1H -pyrazole-3-sulfonimidamide (1.0 g, 2.2 mmol) in THF (50 mL) in a 100-mL 3-necked round-bottom flask under nitrogen was added t-BuOK (493 mg, 4.4 mmol) in portions at 0 °C. The resulting solution was stirred for 15 min at 0 °C. To the above mixture was added 2,2,2-trichloroethyl (3,3-dimethyl-1,2,3,5,6,7- hexahydrodicyclopenta[b,e]pyridin-8-yl)carbamate (830 mg, 2.2 mmol) in THF (5 mL) dropwise at 0 °C. The resulting mixture was stirred overnight at RT. The resulting solution was then quenched with water (5 mL). The mixture was concentrated under vacuum. The residue was eluted from silica gel with DCM/MeOH (20:1). This resulted in 400 mg (27%) of the title compound as an off-white solid. MS-ESI: 675 (M+1).

### Step 2: N'-((3,3-dimethyl-1,2,3,5,6,7-hexahydrodicyclopenta[b,e]pyridin-8-yl)carbamoyl)-5-(hydroxymethyl)-1-isopropyl-1H-pyrazole-3-sulfonimidamide

To a stirred solution of N-(tert-butyldimethylsilyl)-5-(((tert-butyldimethylsilyl)oxy)methyl)-N'-((3,3- dimethyl-1,2,3,5,6,7-hexahydrodicyclopenta[b,e]pyridin-8-yl)carbamoyl)-1-isopropyl-1H-pyrazole-3-sulfonimidamide (400 mg, 0.59 mmol) in THF (20 mL) in a 100-mL round-bottom flask was added HF-pyridine (70% wt., 50 mg, 1.77 mmol) dropwise at 0 °C. The reaction solution was stirred for 2 h at RT. The resulting mixture was concentrated under vacuum. The crude product was purified by Prep-HPLC using the following conditions: XBridge Prep C18 OBD Column 19×150 mm 5um; Mobile Phase A: water (10 mM NH₄HCO₃), Mobile Phase B: ACN; Flow rate: 25 mL/min; Gradient: 9% B to 36% B over 7 min; UV 254/210 nm; Rt: 7.13 min. This resulted in 200 mg (75.6%) of Example 233 as a white solid. MS-ESI: 447 (M+1). ¹H NMR (400 MHz, MeOH-*d₄*) δ 6.70 (s, 1H), 4.79-4.74 (m, 1H), 4.66 (s, 2H), 2.95 (t, *J =* 7.6 Hz, 2H), 2.86-2.76 (m, 4H), 2.16-2.03 (m, 2H), 1.96 (t, *J* = 7.2 Hz, 2H), 1.52 (d, *J* = 6.6 Hz, 3H), 1.51 (d, *J* = 6.6 Hz, 3H), 1.27 (s, 6H).

### Step 3: Chiral resolution

**Example 233** (200 mg) was resolved by Prep-Chiral-HPLC with the following conditions: Column: CHIRALPAK AS, 2*25 cm (5 um); Mobile Phase A: CO₂, Mobile Phase B: EtOH (2 mM NH₃⁻MeOH); Flow rate: 40 mL/min; Gradient: 20% B; 220 nm; RT₁:4.54; RT₂:6.29; Injection Volume: 2.5 ml; Number of Runs: 10. This resulted in 81 mg of **Example 234** followed by 75 mg of **Example 235,** both as white solids.

**Example 234:** MS-ESI: 447 (M+1). ¹H NMR (400 MHz, MeOH-*d₄*) δ 6.70 (s, 1H), 4.79-4.72 (m, 1H), 4.66 (s, 2H), 2.95 (t, *J* = 7.6 Hz, 2H), 2.84-2.73 (m, 4H), 2.16-2.03 (m, 2H), 1.96 (t, *J* = 7.3 Hz, 2H), 1.52 (d, *J =* 6.6 Hz, 3H), 1.51 (d, *J =* 6.6 Hz, 3H), 1.27 (s, 6H).

**Example 235:** MS-ESI: 447 (M+1). ¹H NMR (400 MHz, MeOH-*d₄*) δ 6.70 (s, 1H), 4.81-4.74 (m, 1H), 4.66 (s, 2H), 2.95 (t, *J =* 7.6 Hz, 2H), 2.86-2.75 (m, 4H), 2.14-2.05 (m, 2H), 1.96 (t, *J =* 7.3 Hz, 2H), 1.52 (d, *J =* 6.6 Hz, 3H), 1.51 (d, *J =* 6.6 Hz, 3H), 1.27 (s, 6H).

### Example 236 (Compound 695)

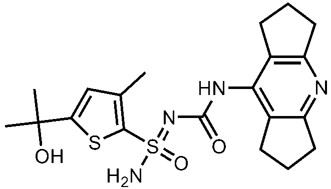

### N'-((1,2,3,5,6,7-hexahydrodicyclopenta[b,e]pyridin-8-yl)carbamoyl)-5-(2-hydroxypropan-2-yl)-3-methylthiophene-2-sulfonimidamide (Scheme II)

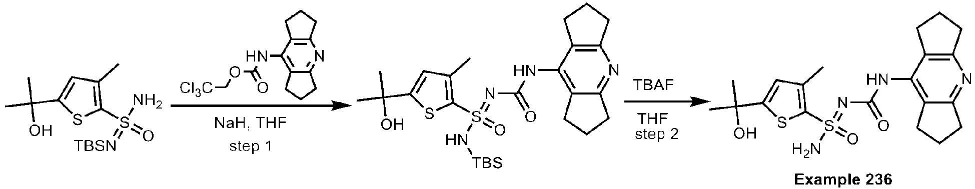

### Step 1: N-(tert-butyldimethylsilyl)-N'-((1,2,3,5,6,7-hexahydrodicyclopenta[b,e]pyridine-8-yl) carbamoyl)-5-(2-hydroxypropan-2-yl)-3-methylthiophene-2-sulfonimidamide

To a stirred solution of N-(tert-butyldimethylsilyl)-5-(2-hydroxypropan-2-yl)-3-methylthiophene-2- sulfonimidamide (150 mg, 0.43 mmol) in THF (10 mL) in a 50-mL round-bottom flask under nitrogen was added NaH (60%wt., dispersion in mineral oil, 34.4 mg, 0.86 mmol) at 0 °C. The resulting solution was stirred for 10 min at RT. Then 2,2,2-trichloroethyl (1,2,3,5,6,7-hexahydrodicyclopenta[b,e] pyridin-8-yl)carbamate (150 mg, 0.43 mmol) in THF (5 mL) was added dropwise at 0 °C into the mixture. The resulting solution was stirred for 1 h at RT. The reaction was then quenched by the addition of 1.0 mL water. The resulting mixture was concentrated under vacuum. The residue was eluted from silica gel with DCM/MeOH (10:1). This resulted in 230 mg (97.4%) of the title compound as a yellow solid. MS-ESI: 549 (M+1).

### Step 2: N'-((1,2,3,5,6,7-hexahydrodicyclopenta[b,e]pyridin-8-yl)carbamoyl)-5-(2-hydroxypropan-2-yl)-3-methylthiophene-2-sulfonimidamide

To a stirred solution of N-(tert-butyldimethylsilyl)-N'-((1,2,3,5,6,7-hexahydrodicyclopenta[b,e]pyridin-8-yl) carbamoyl)-5-(2-hydroxypropan-2-yl)-3-methylthiophene-2-sulfonimidamide (230 mg, 0.42 mmol) in THF (10 mL) in a 50-mL round-bottom flask was added TBAF (110 mg, 0.42 mmol) in portions at RT. The resulting solution was stirred for 1 h at RT. The resulting mixture was concentrated under vacuum. The crude product was purified by Prep-HPLC using the following conditions: XBridge Prep OBD C18 Column, 30x150 mm 5 um; mobile phase, water (10 mM NH₄HCO₃) and ACN (20% to 60% in 7 min); 254/210 nm; RT: 6.13 min. This resulted in 100 mg (55%) of **Example 236** as a white solid. MS-ESI: 435 (M+1). ¹H NMR (400 MHz, DMSO-*d₆*) δ 8.67 (s, 1H), 7.56 (br, s, 2H), 6.81 (s, 1H), 5.67 (s, 1H), 2.82-2.68 (m, 8H), 2.37 (s, 3H), 2.00-1.92 (m, 4H), 1.48 (s, 3H), 1.47 (s, 3H)

**Table 33. Examples in the following table were prepared using similar conditions as described in Example 236 and Scheme II from appropriate starting materials.**

| Example # | Final Target Number | Structure | IUPAC Name | Exact Mass [M+H]⁺ |
|---|---|---|---|---|
| 237 | 643 | 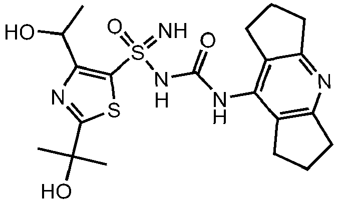 | N-((1,2,3,5,6,7-hexahydrodicyclopenta[b,e ]pyridin-8-yl)carbamoyl)-4-(1-hydroxyethyl)-2-(2-hydroxypropan-2-yl)thiazole-5-sulfonimidamide | 466 |
| 238 | 201 | 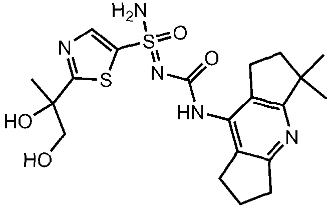 | 2-(1,2-Dihydroxypropan-2-yl)-N'-((3,3-dimethyl-1,2,3,5,6,7-hexahydrodicyclopenta[b,e ]pyridin-8-yl)carbamoyl)thiazole-5-sulfonimidamide | 466 |
| 239 | 640 | 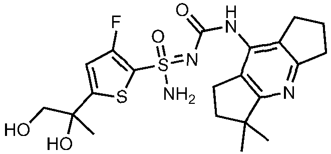 | 5-(1,2-Dihydroxypropan-2-yl)-N'-((3,3-dimethyl-1,2,3,5,6,7-hexahydrodicyclopenta[b,e ]pyridin-8-yl)carbamoyl)-3-fluorothiophene-2-sulfonimidamide | 483 |
| 240 | 605 | 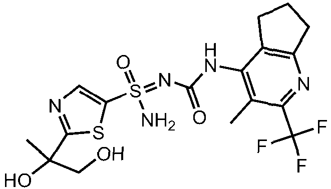 | 2-(1,2-Dihydroxypropan-2-yl)-N'-((3-methyl-2-(trifluoromethyl)-6,7-dihydro-5H-cyclopenta[b]pyridin-4-yl)carbamoyl)thiazole-5-sulfonimidamide | 480 |
| 241 | 605f | 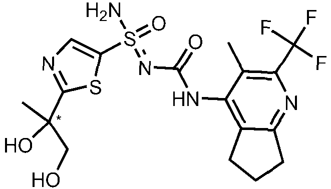 | (R, RS) or (S, RS) 2-(-1,2-dihydroxypropan-2-yl)-N'-((3-methyl-2-(trifluoromethyl)-6,7-dihydro-5H-cyclopenta[b]pyridin-4-yl)carbamoyl)thiazole-5-sulfonimidamide (from Intermediate 117A) | 480 |
| 242 | 605a | 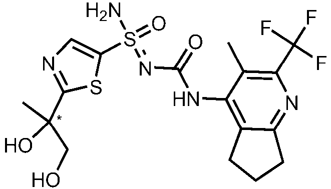 | (S, RS) or (R, RS) 2-(-1,2-dihydroxypropan-2-yl)-N'-((3-methyl-2-(trifluoromethyl)-6,7-dihydro-5H-cyclopenta[b]pyridin-4-yl)carbamoyl)thiazole-5-sulfonimidamide (from Intermediate 117B) | 480 |
| 243 | 691 | 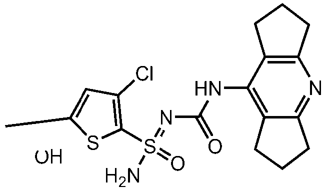 | 3-Chloro-N'-((1,2,3,5,6,7-hexahydrodicyclopenta[b,e ]pyridin-8-yl)carbamoyl)-5-(2-hydroxypropan-2-yl)thiophene-2-sulfonimidamide | 455 |
| 244 | 688 | 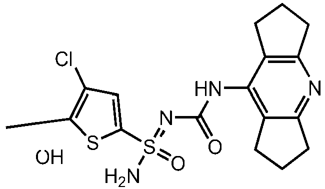 | 4-Chloro-N'-((1,2,3,5,6,7-hexahydrodicyclopenta[b,e ]pyridin-8-yl)carbamoyl)-5-(2-hydroxypropan-2-yl)thiophene-2-sulfonimidamide | 455 |
| 245 | 676 | 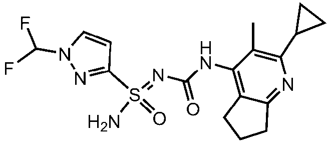 | N'-((2-cyclopropyl-3-methyl-6,7-dihydro-5H-cyclopenta[b]pyridin-4-yl)carbamoyl)-1-(difluoromethyl)-1H-pyrazole-3-sulfonimidamide | 411 |
| 246 | 669 | 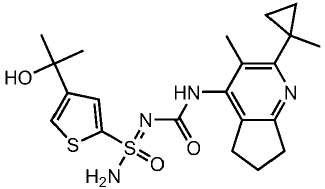 | 4-(2-Hydroxypropan-2-yl)-N'-((3-methyl-2-(1-methylcyclopropyl)-6,7-dihydro-5H-cyclopenta[b]pyridin-4-yl)carbamoyl)thiophene-2-sulfonimidamide | 449 |
| 247 | 612 | 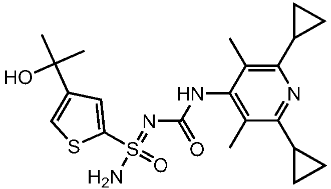 | N'-((2,6-dicyclopropyl-3,5-dimethylpyridin-4-yl)carbamoyl)-4-(2-hydroxypropan-2-yl)thiophene-2-sulfonimidamide | 449 |
| 248 | 627 | 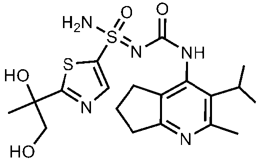 | 2-(1,2-Dihydroxypropan-2-yl)-N'-((3-isopropyl-2-methyl-6,7-dihydro-5H-cyclopenta[b]pyridin-4-yl)carbamoyl)thiazole-5-sulfonimidamide | 454 |
| 249 | 607 | 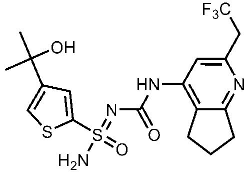 | 4-(2-Hydroxypropan-2-yl)-N'-((2-(2,2,2-trifluoroethyl)-6,7-dihydro-5H-cyclopenta[b]pyridin-4-yl)carbamoyl)thiophene-2-sulfonimidamide | 463 |

### Example 250 (Compound 665)

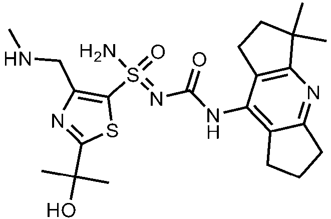

### N'-((3,3-dimethyl-1,2,3,5,6,7-hexahydrodicyclopenta(b,e]pyridin-8-yl)carbamoyl)-2-(2-hydroxypropan-2-yl)-4-((methylamino)methyl)thiazole-5-sulfonimidamide (Scheme III)

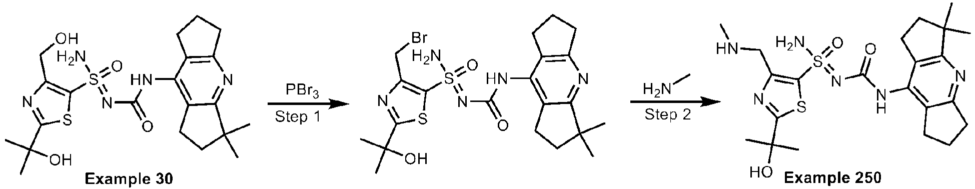

### Step 1: 4-(Bromomethyl)-N'-((3,3-dimethyl-1,2,3,5,6,7-hexahydrodicyclopenta[b,e]pyridin-8-yl) carbamoyl)-2-(2-hydroxypropan-2-yl)thiazole-5-sulfonimidamide

To a stirred solution of N'-((3,3-dimethyl-1,2,3,5,6,7-hexahydrodicyclopenta[b,e]pyridin-8-yl)carbamoyl)-4- (hydroxymethyl)-2-(2-hydroxypropan-2-yl)thiazole-5-sulfonimidamide (800 mg, 1.68 mmol) in THF (20 mL) in a 100-mL 3-necked round-bottom flask under nitrogen was added phosphorus tribromide (676 mg, 2.52 mmol) dropwise at 0 °C. The resulting solution was stirred for 4 h at RT. The solution was slowly poured into water/ice (20 mL). The resulting solution was extracted with 3x100 mL of ethyl acetate. The combined organic layer was dried over anhydrous sodium sulfate. The solids were filtered out. The resulting mixture was concentrated under vacuum. This resulted in 540 mg (60%) of crude title compound as an off-white solid. MS-ESI: 542/544 (M+1).

### Step 2: N'-((3,3-dimethyl-1,2,3,5,6,7-hexahydrodicyclopenta[b,e]pyridin-8-yl)carbamoyl)-2-(2- hydroxypropan-2-yl)-4-((methylamino)methyl)thiazole-5-sulfonimidamide

To a stirred solution of 4-(bromomethyl)-N'-((3,3-dimethyl-1,2,3,5,6,7-hexahydrodicyclopenta[b,e]pyridine -8-yl)carbamoyl)-2-(2-hydroxypropan-2-yl)thiazole-5-sulfonimidamide (500 mg, 0.92 mmol) in THF (10 mL) in a 50-mL round-bottom flask was added methanamine in THF (2 M, 2.31 mL, 4.62 mmol) dropwise at RT. The resulting solution was stirred for 4 h at 50 °C. The reaction was quenched with water (10 mL). The resulting solution was extracted with 3x50 mL of ethyl acetate and the organic layers were combined and dried with anhydrous Na₂SO₄. Then the organic phase was concentrated under vacuum. The residue was eluted from silica gel with EtOAc/PE (1:1). The crude product was further purified by Prep-HPLC with the following conditions: XBridge Prep C18 OBD Column 19×150 mm 5 um; Mobile Phase A: water (10 mM NH₄HCO₃+0.1%NH₃·H₂O), Mobile Phase B: ACN; Flow rate: 25 mL/min; Gradient: 10% B to 24% B over 7 min; 254/210 nm; Rt: 6.52 min. This resulted in 200 mg (44%) of **Example 250** as a yellow solid. MS-ESI: 493 (M+1).

### Example 251 (Compound 693)

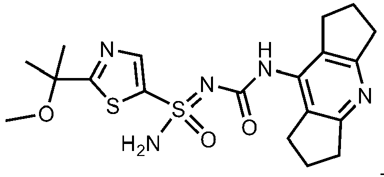

### N'-((1,2,3,5,6,7-hexahydrodicyclopenta[b,e]pyridin-8-yl)carbamoyl)-2-(2-methoxypropan-2-yl)thiazole-5-sulfonimidamide (Scheme IV)

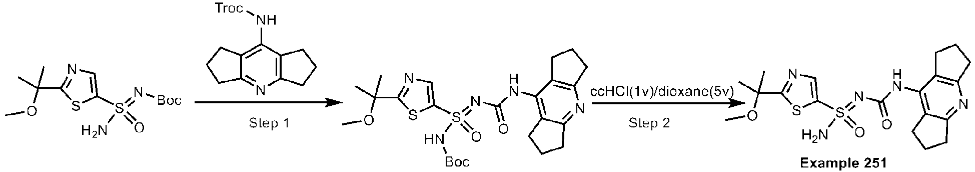

### Step 1: Tert-butyl (N-((1,2,3,5,6,7-hexahydrodicyclopenta[b,e]pyridin-8-yl)carbamoyl)-2-(2-methoxypropan-2-yl)thiazole-5-sulfonimidoyl)carbamate

To a stirred solution of *tert*-butyl (amino(2-(2-methoxypropan-2-yl)thiazol-5-yl)(oxo)-λ⁶-sulfaneylidene) carbamate (500 mg, 1.49 mmol) in THF (10 mL) in a 100-mL round-bottom flask under nitrogen was added NaH (60%wt., dispersion in mineral oil, 71.5 mg, 1.79 mmol) at 0 °C. The resulting solution was stirred for 10 min at RT. Then 2,2,2-trichloroethyl (1,2,3,5,6,7-hexahydrodicyclopenta[b,e]pyridin-8-yl) carbamate (521 mg, 1.49 mmol) in THF (5 mL) was added dropwise into the solution at 0 °C. The resulting solution was stirred for 2 h at RT. The reaction was then quenched by the addition of 5 mL water. The resulting solution was extracted with 3x50 mL of ethyl acetate. The combined organic layer was dried over anhydrous sodium sulfate. The solids were filtered out. The resulting mixture was concentrated under vacuum. This resulted in 400 mg (50%) of the title compound as a yellow solid. MS-ESI: 536 (M+1).

### Step 2: N'-((1,2,3,5,6,7-hexahydrodicyclopenta[b,e]pyridin-8-yl)carbamoyl)-2-(2-methoxypropan-2-yl) thiazole-5-sulfonimidamide

A stirred solution of tert-butyl (N-((1,2,3,5,6,7-hexahydrodicyclopenta [b,e]pyridin-8-yl)carbamoyl)-2- (2-methoxypropan-2-yl)thiazole-5-sulfonimidoyl)carbamate (300 mg, 0.56 mmol) in HCl/dioxane (4 M, 20 mL) was stirred for 15 h at RT. The resulting mixture was concentrated under vacuum. The crude product was purified by Prep-HPLC under the following conditions: XBridge Prep OBD C18 Column 30×150 mm 5 um; Mobile Phase A: water (10 mM NH₄HCO₃), Mobile Phase B: ACN; Flow rate: 60 mL/min; Gradient: 10% B to 20% B over 9.5 min; 254/210 nm; Rt: 7.32. This resulted in 130 mg (54%) of **Example 251** as a white solid. MS-ESI: 436 (M+1). ¹H NMR (300 MHz, DMSO-*d₆*) δ 8.87 (s, 1H), 8.14 (s, 1H), 7.97 (br s, 2H), 3.25 (s, 3H), 2.87-2.78 (m, 4H), 2.73-2.68 (m, 4H), 1.98-1.93 (m, 4H), 1.56 (s, 6H).

### Example 252 (Compound 645)

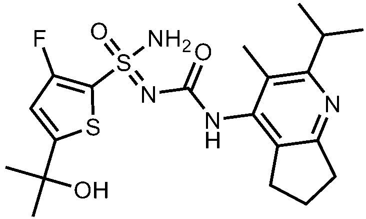

### 3-Fluoro-5-(2-hydroxypropan-2-yl)-N'-((2-isopropyl-3-methyl-6,7-dihydro-5H-cyclopenta[b]pyridin-4-yl)carbamoyl)thiophene-2-sulfonimidamide (Scheme V)

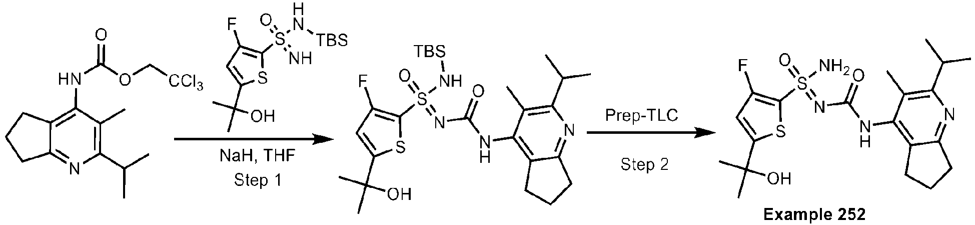

### Step 1: N-(tert-butyldimethylsilyl)-3-fluoro-5-(2-hydroxypropan-2-yl)-N'-((2-isopropyl-3-methyl-6,7- dihydro-5H-cyclopenta[b]pyridin-4-yl)carbamoyl)thiophene-2-sulfonimidamide

To a stirred solution of N-(tert-butyldimethylsilyl)-3-fluoro-5-(2-hydroxypropan-2-yl)thiophene-2- sulfonimidamide (300 mg, 0.85 mmol) in THF (20 mL) in a 50-mL round-bottom flask under nitrogen was added NaH (60%wt., dispersion in mineral oil, 102 mg, 2.56 mmol) at 0 °C. The resulting solution was stirred for 15 min at RT. 2,2,2-trichloroethyl (2-isopropyl-3-methyl-6,7-dihydro-5H-cyclopenta[b]-pyridin- 4-yl)carbamate (310 mg, 0.85 mmol) in THF (5 mL) was added dropwise to the solution. The resulting solution was stirred overnight at RT and quenched with water (2.0 mL). The resulting solution was concentrated under vacuum to get 580 mg of the title compound as an off-white crude solid which was used in the next step without further purification. MS-ESI: 569 (M+1).

### Step 2: 3-Fluoro-5-(2-hydroxypropan-2-yl)-N'-((2-isopropyl-3-methyl-6,7-dihydro-5H-cyclopenta[b] pyridin-4-yl)carbamoyl)thiophene-2-sulfonimidamide

The crude product (580 mg) of N-(tert-butyldimethylsilyl)-3-fluoro-5-(2-hydroxypropan-2-yl)-N'-((2- isopropyl-3-methyl-6,7- dihydro-5H-cyclopenta[b]pyridin-4-yl)carbamoyl)thiophene-2-sulfonimidamide was applied on a TLC and using DCM/MeOH=10:1 to keep Rf = 0.3~0.6, then left the product on TLC overnight at RT. The TBS group was removed on TLC to give the final product which was then eluted from TLC with DCM/MeOH=10:1. The final product was further purified by Prep-HPLC with the following conditions: XBridge Shield RP18 OBD Column, 19*250 mm,10 um; Mobile Phase A: water (10 mM NH₄HCO₃), Mobile Phase B: ACN; Flow rate: 25 mL/min; Gradient: 22% B to 23% B over 15 min; UV 210/254 nm; Rt: 16.03 min. This resulted in 130 mg (34%) of **Example 252** as a white solid. MS-ESI: 455 (M+1). ¹H NMR (400 MHz, DMSO-*d₆*) δ 8.58 (s, 1H), 7.80 (br, s, 2H), 6.98 (s, 1H), 5.86 (s, 1H), 3.24-3.17 (m, 1H), 2.84-2.80 (m, 2H), 2.77-2.66 (m, 2H), 2.09 (s, 3H), 1.98-1.90 (m, 2H), 1.48 (s, 3H), 1.47 (s, 3H), 1.15 (d, *J* = 6.7 Hz, 6H).

**Table 34. Examples in the following table were prepared using similar conditions as described in Example 252 and Scheme V from appropriate starting materials.**

| Example # | Final Target Number | Structure | IUPAC Name | Exact Mass [M+H]⁺ |
|---|---|---|---|---|
| 253 | 672 | 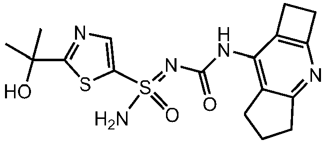 | 2-(2-Hydroxypropan-2-yl)-N'-((2,4,5,6-tetrahydro-1H-cyclobuta[b]cyclopenta[e]pyri din-7-yl)carbamoyl)thiazole-5-sulfonimidamide | 408 |
| 254 | 702 | 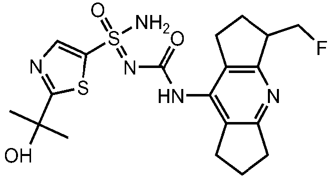 | N'-((3-(fluoromethyl)-1,2,3,5,6,7-hexahydrodicyclopenta[b,e]py ridin-8-yl)carbamoyl)-2-(2-hydroxypropan-2-yl)thiazole-5-sulfonimidamide | 454 |
| 255 | 692 | 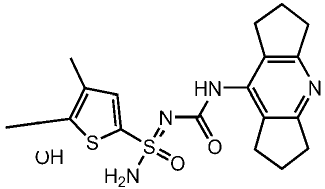 | N'-((1,2,3,5,6,7-hexahydrodicyclopenta[b,e]py ridin-8-yl)carbamoyl)-5-(2-hydroxypropan-2-yl)-4-methylthiophene-2-sulfonimidamide | 435 |
| 256 | 656 | 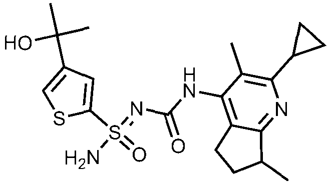 | N'-((2-cyclopropyl-3,7-dimethyl-6,7-dihydro-5H-cyclopenta[b]pyridin-4-yl)carbamoyl)-4-(2-hydroxypropan-2-yl)thiophene-2-sulfonimidamide | 449 |
| 257 | 681 | 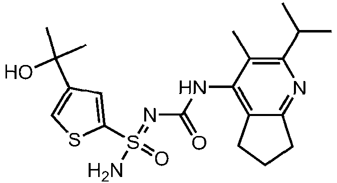 | 4-(2-Hydroxypropan-2-yl)-N'-((2-isopropyl-3-methyl-6,7-dihydro-5H-cyclopenta[b]pyridin-4-yl)carbamoyl)thiophene-2-sulfonimidamide | 437 |
| 258 | 668 | 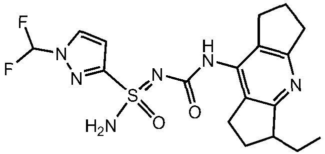 | 1-(Difluoromethyl)-N'-((3-ethyl-1,2,3,5,6,7-hexahydrodicyclopenta[b,e]py ridin-8-y1)carbamoy1)-1H-pyrazole-3-sulfonimidamide | 425 |
| 259 | 658 | 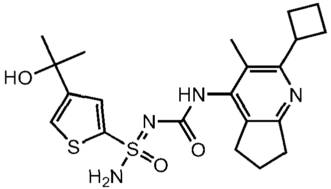 | N'-((2-cyclobutyl-3-methyl-6,7-dihydro-5H-cyclopenta[b]pyridin-4-yl)carbamoyl)-4-(2-hydroxypropan-2-yl)thiophene-2-sulfonimidamide | 449 |
| 260 | 667 | 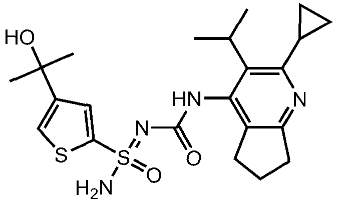 | N'-((2-cyclopropyl-3-isopropyl-6,7-dihydro-5H-cyclopenta[b]pyridin-4-yl)carbamoyl)-4-(2-hydroxypropan-2-yl)thiophene-2-sulfonimidamide | 463 |
| 261 | 703 | 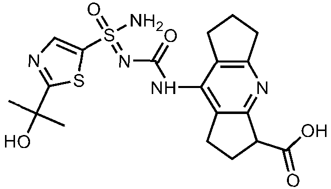 | 8-(3-(Amino(2-(2-hydroxypropan-2-yl)thiazol-5-yl)(oxo)-λ⁶-sulfaneylidene)ureido)-1,2,3,5,6,7-hexahydrodicyclopenta[b,e]py ridine-3-carboxylic acid | 466 |
| 262 | 664 | 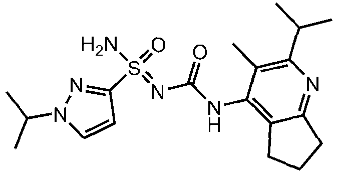 | 1-Isopropyl-N'-((2-isopropyl-3-methyl-6,7-dihydro-5H-cyclopenta[b]pyridin-4-yl)carbamoyl)-1H-pyrazole-3-sulfonimidamide | 405 |
| 263 | 632 | 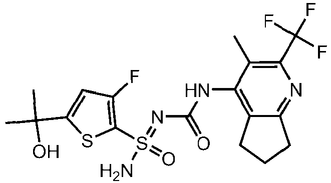 | 3-Fluoro-5-(2-hydroxypropan-2-yl)-N'-((3-methyl-2-(trifluoromethyl)-6,7-dihydro-5H-cyclopenta[b]pyridin-4-yl)carbamoyl)thiophene-2-sulfonimidamide | 481 |
| 264 | 624 | 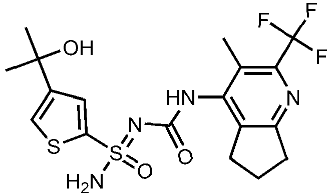 | 4-(2-Hydroxypropan-2-yl)-N'-((3-methyl-2-(trifluoromethyl)-6,7-dihydro-5H-cyclopenta[b]pyridin-4-yl)carbamoyl)thiophene-2-sulfonimidamide | 463 |
| 265 | 631 | 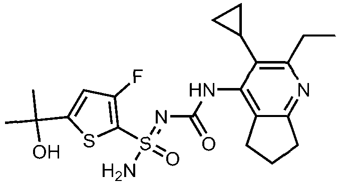 | N'-((3-cyclopropyl-2-ethyl-6,7-dihydro-5H-cyclopenta[b]pyridin-4-yl)carbamoyl)-3-fluoro-5-(2-hydroxypropan-2-yl)thiophene-2-sulfonimidamide | 467 |
| 266 | 630 | 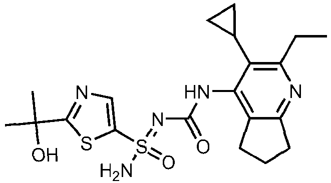 | N'-((3-cyclopropyl-2-ethyl-6,7-dihydro-5H-cyclopenta[b]pyridin-4-yl)carbamoyl)-2-(2-hydroxypropan-2-yl)thiazole-5-sulfonimidamide | 450 |
| 267 | 623 | 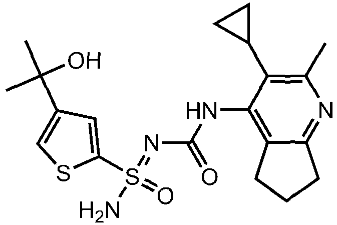 | N'-((3-cyclopropyl-2-methyl-6,7-dihydro-5H-cyclopenta[b]pyridin-4-yl)carbamoyl)-4-(2-hydroxypropan-2-yl)thiophene-2-sulfonimidamide | 435 |
| 268 | 621 | 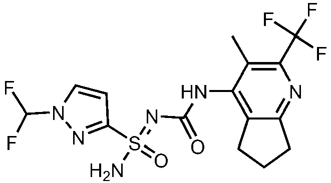 | 1-(Difluoromethyl)-N'-((3-methyl-2-(trifluoromethyl)-6,7-dihydro-5H-cyclopenta[b]pyridin-4-yl)carbamoyl)-1H-pyrazole-3-sulfonimidamide | 439 |
| 269 | 629 | 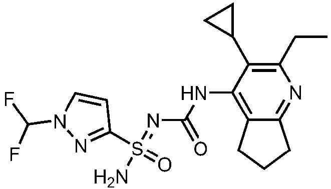 | N'-((3-cyclopropyl-2-ethyl-6,7-dihydro-5H-cyclopenta[b]pyridin-4-yl)carbamoyl)-1-(difluoromethyl)-1H-pyrazole-3-sulfonimidamide | 425 |
| 270 | 610 | 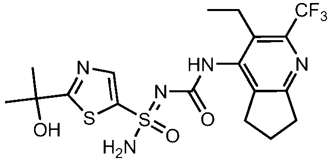 | N'-((3-ethyl-2-(trifluoromethyl)-6,7-dihydro-5H-cyclopenta[b]pyridin-4-yl)carbamoyl)-2-(2-hydroxypropan-2-yl)thiazole-5-sulfonimidamide | 478 |
| 271 | 611 | 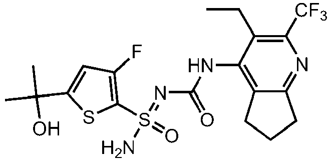 | N'-((3-ethyl-2-(trifluoromethyl)-6,7-dihydro-5H-cyclopenta[b]pyridin-4-yl)carbamoyl)-3-fluoro-5-(2-hydroxypropan-2-yl)thiophene-2-sulfonimidamide | 495 |
| 272 | 609 | 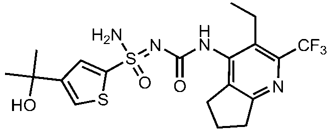 | N'-((3-ethyl-2-(trifluoromethyl)-6,7-dihydro-5H-cyclopenta[b]pyridin-4-yl)carbamoyl)-4-(2-hydroxypropan-2-yl)thiophene-2-sulfonimidamide | 477 |
| 273 | 608 | 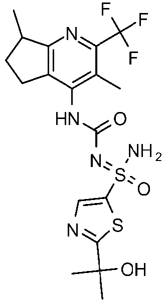 | N'-((3,7-dimethyl-2-(trifluoromethyl)-6,7-dihydro-SH-cyclopenta[b]pyridin-4-yl)carbamoyl)-2-(2-hydroxypropan-2-yl)thiazole-5-sulfonimidamide | 478 |
| 274 | 616 | 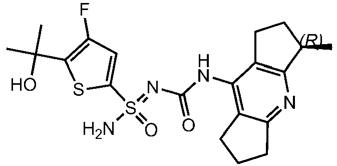 | 4-Fluoro-5-(2-hydroxypropan-2-yl)-N'-(((R)-3-methyl-1,2,3,5,6,7-hexahydrodicyclopenta[b,e]py ridin-8-yl)carbamoyl)thiophene-2-sulfonimidamide | 453 |
| 275 | 604 | 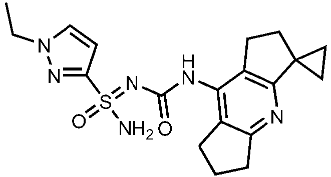 | 1-Ethyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 401 |
| 276 | 603 | 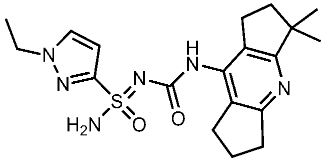 | N'-((3,3-dimethyl-1,2,3,5,6,7-hexahydrodicyclopenta[b,e]py ridin-8-yl)carbamoyl)-1-ethyl-1H-pyrazole-3-sulfonimidamide | 403 |
| 277 | 304 | 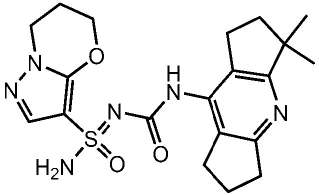 | N'-((3,3-dimethyl-1,2,3,5,6,7-hexahydrodicyclopenta[b,e]py ridin-8-yl)carbamoyl)-6,7-dihydro-5H-pyrazolo[5,1-b][1,3]oxazine-3-sulfonimidamide | 431 |
| 278 | 306 | 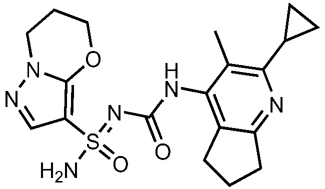 | N'-((2-cyclopropyl-3-methyl-6,7-dihydro-5H-cyclopenta[b]pyridin-4-yl)carbamoyl)-6,7-dihydro-5H-pyrazolo[5,1-b][1,3]oxazine-3-sulfonimidamide | 417 |
| 279 | 677 | 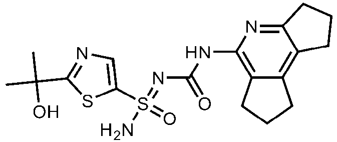 | N'-((1,2,3,6,7,8-hexahydrodicyclopenta[b,d]p yridin-5-yl)carbamoyl)-2-(2-hydroxypropan-2-yl)thiazole-5-sulfonimidamide | 422 |
| 280 | 661 | 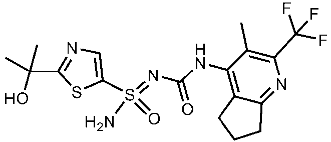 | 2-(2-Hydroxypropan-2-yl)-N'-((3-methyl-2-(trifluoromethyl)-6,7-dihydro-5H-cyclopenta[b]pyridin-4-yl)carbamoyl)thiazole-5-sulfonimidamide | 464 |
| 281 | 647 | 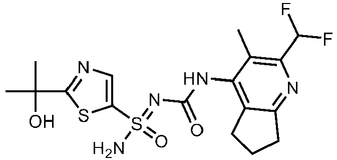 | N'-((2-(difluoromethyl)-3-methyl-6,7-dihydro-5H-cyclopenta[b]pyridin-4-yl)carbamoyl)-2-(2-hydroxypropan-2-yl)thiazole-5-sulfonimidamide | 446 |

### Example 282 (Compound 619)

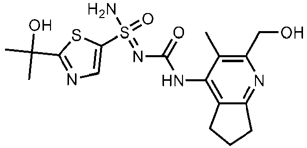

### N'-((2-(hydroxymethyl)-3-methyl-6,7-dihydro-5H-cyclopenta[b]pyridin-4-yl)carbamoyl)-2-(2-hydroxypropan-2-yl)thiazole-5-sulfonimidamide (Scheme VI)

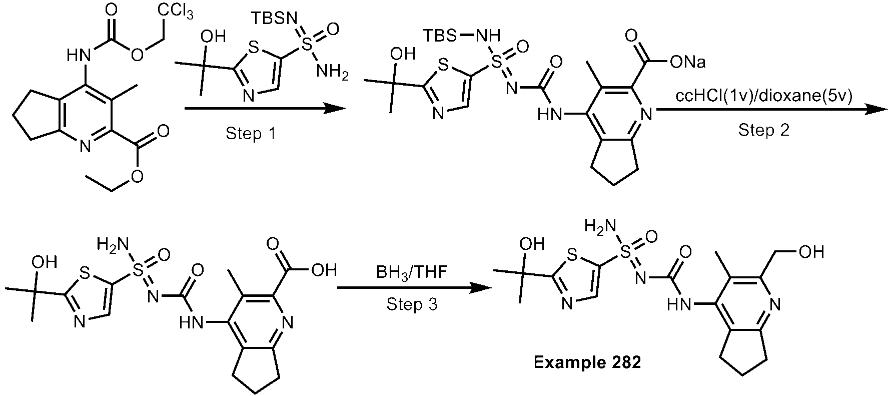

### Step 1: Sodium 4-(3-(((tert-butyldimethylsilyl)amino)(2-(2-hydroxypropan-2-yl)thiazol-5-yl)(oxo)- λ⁶-sulfaneylidene)ureido)-3-methyl-6,7-dihydro-5H-cyclopenta[b]pyridine-2-carboxylate

To a stirred solution of N-(tert-butyldimethylsilyl)-2-(2-hydroxypropan-2-yl)thiazole-5-sulfonimidamide (270 mg, 0.76 mmol) in THF (20 mL) in a 50-mL round-bottom flask under nitrogen was added NaH (60% wt., dispersion in mineral oil, 91.2 mg, 2.28 mmol) at 0 °C. The resulting solution was stirred for 5 min at RT. To the above solution was added ethyl 3-methyl-4-(((2,2,2-trichloroethoxy)carbonyl)amino)-6,7- dihydro-5H-cyclopenta[b]pyridine-2-carboxylate (300 mg, 0.76 mmol) in THF (5 mL) dropwise at 0 °C. The resulting solution was stirred overnight at RT. Then the resulting solution was quenched by addition of 2 mL water. The resulting mixture was stirred for 1 h at RT. The resulting solution was concentrated under vacuum. This resulted in 440 mg (crude) title compound as a white solid which was used in the next step without further purification. MS-ESI: 576 (M+1).

### Step 2: 4-(3-(Amino(2-(2-hydroxypropan-2-yl)thiazol-5-yl)(oxo)-λ⁶-sulfaneylidene)ureido)-3-methyl- 6,7-dihydro-5H-cyclopenta[b]pyridine-2-carboxylic acid

To a stirred solution of sodium 4-(3-(((tert-butyldimethylsilyl)amino)(2-(2-hydroxypropan-2-yl)thiazol-5-yl) (oxo)-λ⁶-sulfaneylidene)ureido)-3-methyl-6,7-dihydro-5H-cyclopenta[b]pyridine-2-carboxylate (440 mg, crude) in dioxane (10 mL) in a 50-mL round-bottom flask was added conc. HCl (2 mL) dropwise at 0 °C. The resulting solution was stirred for 30 min at RT. The resulting solution was concentrated under vacuum. The residue was eluted from silica gel with DCM/MeOH (10:1). This resulted in 174 mg (52%, over two steps) of the title compound as an off-white solid. MS-ESI: 440 (M+1).

### Step 3: N'-((2-(hydroxymethyl)-3-methyl-6,7-dihydro-5H-cyclopenta[b]pyridin-4-yl)carbamoyl)-2-(2-hydroxypropan-2-yl)thiazole-5-sulfonimidamide

To a stirred solution of 4-(3-(amino(2-(2-hydroxypropan-2-yl)thiazol-5-yl)(oxo)-λ⁶-sulfaneylidene)ureido)- 3-methyl-6,7-dihydro-5H-cyclopenta[b]pyridine-2-carboxylic acid (150 mg, 0.34 mmol) in THF (10 mL) in a 50-mL round-bottom flask under nitrogen was added BH₃/THF (1M, 1.0 mL, 1.0 mmol) dropwise at 0 °C. The resulting solution was stirred overnight at RT. The solution was quenched with MeOH (5 mL) and concentrated under vacuum. The crude product was purified by Prep-HPLC with the following condition: XBridge Prep OBD C18 Column, 30×150 mm 5 um; Mobile Phase A: water (10 mM NH₄HCO₃+0.1% NH₃·H₂O), Mobile Phase B: ACN; Flow rate: 60 mL/min; Gradient: 5% B to 17% B over 7 min; UV 254/210 nm; Rt: 6.63. This resulted in 100 mg (71%) of **Example 282** as a white solid. MS-ESI: 426 (M+1). ¹H NMR (400 MHz, MeOH-*d₄*) δ 7.82 (s, 1H), 4.48 (s, 2H), 2.84-2.81 (m, 2H), 2.71-2.69 (m, 2H), 2.07 (s, 3H), 1.96-1.93 (m, 2H), 1.49 (s, 6H).

### Example 283 (Compound 618)

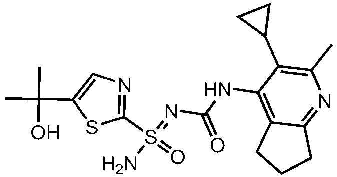

### N'-((3-cyclopropyl-2-methyl-6,7-dihydro-5H-cyclopenta[b]pyridin-4-yl)carbamoyl)-5-(2-hydroxypropan-2-yl)thiazole-2-sulfonimidamide (Scheme VII)

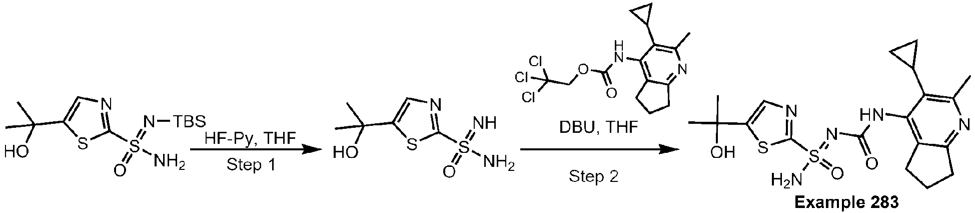

### Step 1: 5-(2-Hydroxypropan-2-yl)thiazole-2-sulfonimidamide

To a stirred solution of N'-(tert-butyldimethylsilyl)-5-(2-hydroxypropan-2-yl)thiazole-2-sulfonimidamide (1.0 g, 2.98 mmol) in THF (10 mL) in a 100-mL round-bottom flask was added HF/pyridine (70% wt., 255 mg, 8.94 mmol) dropwise at 0 °C. The resulting solution was stirred overnight at RT. The resulting mixture was concentrated under vacuum. The residue was eluted from silica gel with DCM/MeOH (20:1). This resulted in 630 mg (95.5%) of the title compound as an off-white solid. MS-ESI: 222(M+1).

### Step 2: N'-((3-cyclopropyl-2-methyl-6,7-dihydro-5H-cyclopenta[b]pyridin-4-yl)carbamoyl)-5-(2- hydroxypropan-2-yl)thiazole-2-sulfonimidamide

To a stirred solution of 5-(2-hydroxypropan-2-yl)thiazole-2-sulfonimidamide (500 mg, 2.26 mmol) in THF (20 mL) in a 100-mL round-bottom flask was added DBU (687 mg, 4.52 mmol) at RT. Then 2,2,2-trichloroethyl (3-cyclopropyl-2-methyl-6,7-dihydro-5H-cyclopenta[b]pyridin-4-yl)carbamate (818 mg, 2.26 mmol) in THF (5 mL) was added to the stirred solution at RT. The resulting solution was stirred overnight at RT, quenched by addition of water (2.0 mL) and concentrated under vacuum. The crude product was purified by Prep-HPLC using the following conditions: XSelect CSH Prep C18 OBD Column, 19*250 mm, 5 um; mobile phase, water (10 mM NH₄HCO₃+0.1% NH₃.H₂O) and ACN (10% to 22% over 7 min); 254/210 nm; Rt: 5.88 min. This resulted in 310 mg (32%) of **Example 283** as an off-white solid. MS-ESI: 436(M+1). ¹H NMR (400 MHz, MeOH-*d₄*) δ 7.68 (s, 1H), 2.89-2.86 (m, 4H), 2.54 (s, 3H), 2.08-1.99 (m, 2H), 1.62 (s, 6H), 1.62-1.50 (m, 1H) 1.02-1.00 (m, 2H), 0.42-0.40 (m, 2H).

**Table 35. Examples in the following table were prepared using similar conditions as described in Example 283 and Scheme VII from appropriate starting materials.**

| Example # | Final Target Number | Structure | IUPAC Name | Exact Mass [M+H]⁺ |
|---|---|---|---|---|
| 284 | 626 | 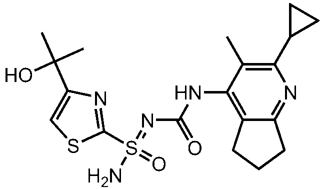 | N'-((2-cyclopropyl-3-methyl-6,7-dihydro-5H-cyclopenta[b]pyridin-4-yl)carbamoyl)-4-(2-hydroxypropan-2-yl)thiazole-2-sulfonimidamide | 436 |

**Table 36. Examples in the following table were prepared using similar conditions as described in Example 28 and Scheme 2A from appropriate starting materials.**

| Example # | Final Target Number | Structure | IUPAC Name | Exact Mass [M+H]⁺ |
|---|---|---|---|---|
| 285 | 696 | 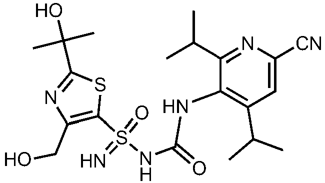 | N-((6-cyano-2,4-diisopropylpyridin-3-yl)carbamoyl)-4-(hydroxymethyl)-2-(2-hydroxypropan-2-yl)thiazole-5-sulfonimidamide | 481 |
| 286 | 682 | 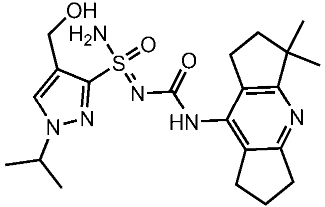 | N'-((3,3-dimethyl-1,2,3,5,6,7-hexahydrodicyclopenta[b,e]pyridin-8-yl)carbamoyl)-4-(hydroxymethyl)-1-isopropyl-1H-pyrazole-3-sulfonimidamide | 447 |
| 287 | 653 | 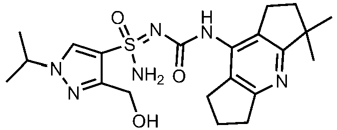 | N'-((3,3-dimethyl-1,2,3,5,6,7-hexahydrodicyclopenta[b,e]pyridin-8-yl)carbamoyl)-3-(hydroxymethyl)-1-isopropyl-1H-pyrazole-4-sulfonimidamide | 447 |
| 288 | 641 | 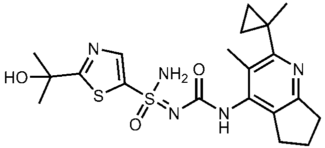 | 2-(2-Hydroxypropan-2-yl)-N'-((3-methyl-2-(1-methylcyclopropyl)-6,7-dihydro-5H-cyclopenta[b]pyridin-4-yl)carbamoyl)thiazole-5-sulfonimidamide | 450 |

**Table 37. Examples in the following table were prepared using similar conditions as described in Example 9 and Scheme 2 from appropriate starting materials.**

| Example # | Final Target Number | Structure | IUPAC Name | Exact Mass [M+H] ⁺ |
|---|---|---|---|---|
| 289 | 694 | 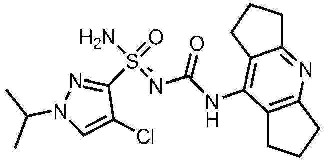 | 4-Chloro-N'-((1,2,3,5,6,7-hexahydrodicyclopenta[b,e] pyridin-8-yl)carbamoyl)-1-isopropyl-1H-pyrazole-3-sulfonimidamide | 423/42 5 |
| 290 | 687 | 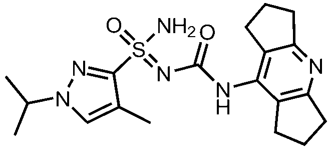 | N'-((1,2,3,5,6,7-hexahydrodicyclopenta[b,e] pyridin-8-yl)carbamoyl)-1-isopropyl-4-methyl-1H-pyrazole-3-sulfonimidamide | 403 |
| 291 | 660 | 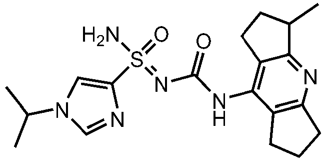 | 1-Isopropyl-N'-((3-methyl-1,2,3,5,6,7-hexahydrodicyclopenta[b,e] pyridin-8-yl)carbamoyl)-1H-imidazole-4-sulfonimidamide | 403 |
| 292 | 140c | 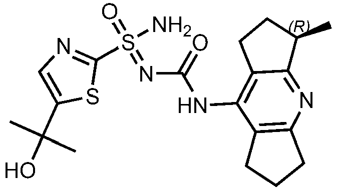 | 5-(2-Hydroxypropan-2-yl)-N'-(((R)-3-methyl-1,2,3,5,6,7-hexahydrodicyclopenta[b,e] pyridin-8-yl)carbamoyl)thiazole-2-sulfonimidamide | 436 |
| 293 | 635 | 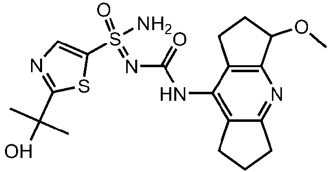 | 2-(2-Hydroxypropan-2-yl)-N'-((3-methoxy-1,2,3,5,6,7-hexahydrodicyclopenta[b,e] pyridin-8-yl)carbamoyl)thiazole-5-sulfonimidamide | 452 |
| 294 | 689 | 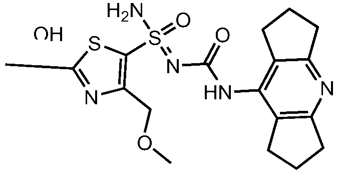 | N'-((1,2,3,5,6,7-hexahydrodicyclopenta[b,e] pyridin-8-yl)carbamoyl)-2-(2-hydroxypropan-2-yl)-4-(methoxymethyl)thiazole-5-sulfonimidamide | 466 |
| 295 | 642 | 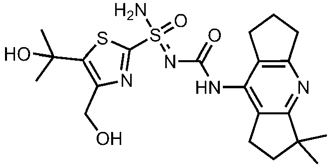 | N'-((3,3-dimethyl-1,2,3,5,6,7-hexahydrodicyclopenta[b,e] pyridin-8-yl)carbamoyl)-4-(hydroxymethyl)-5-(2-hydroxypropan-2-yl)thiazole-2-sulfonimidamide | 480 |
| 296 | 686 | 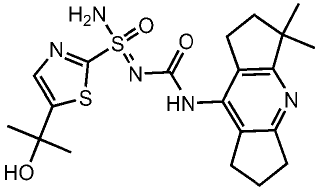 | N'-((3,3-dimethyl-1,2,3,5,6,7-hexahydrodicyclopenta[b,e] pyridin-8-yl)carbamoyl)-5-(2-hydroxypropan-2-yl)thiazole-2-sulfonimidamide | 450 |
| 297 | 680 | 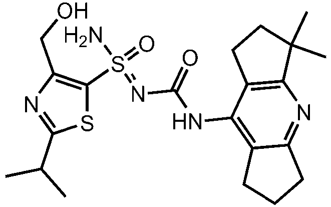 | N'-((3,3-dimethyl-1,2,3,5,6,7-hexahydrodicyclopenta[b,e] pyridin-8-yl)carbamoyl)-4-(hydroxymethyl)-2-isopropylthiazole-5-sulfonimidamide | 464 |
| 298 | 674 | 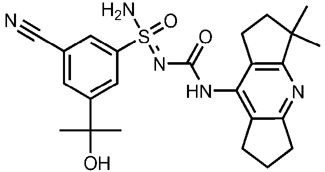 | 3-Cyano-N'-((3,3-dimethyl-1,2,3,5,6,7-hexahydrodicyclopenta[b,e] pyridin-8-yl)carbamoyl)-5-(2-hydroxypropan-2-yl)benzenesulfonimidamide | 468 |
| 299 | 684 | 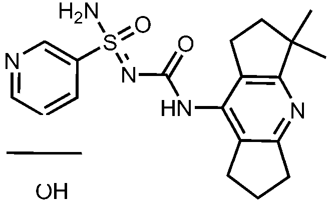 | N'-((3,3-dimethyl-1,2,3,5,6,7-hexahydrodicyclopenta[b,e] pyridin-8-yl)carbamoyl)-5-(2-hydroxypropan-2-yl)pyridine-3-sulfonimidamide | 444 |
| 300 | 683 | 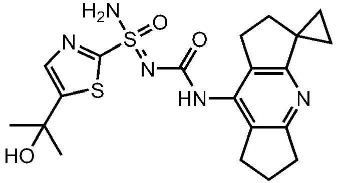 | 5-(2-Hydroxypropan-2-yl)-N'-((1',5',6',7'-tetrahydro-2'H-spiro[cyclopropane-1,3'-dicyclopenta[b,e]pyridin]-8'-yl)carbamoyl)thiazole-2-sulfonimidamide | 448 |
| 301 | 679 | 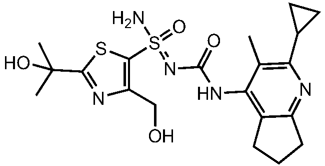 | N'-((2-cyclopropyl-3-methyl-6,7-dihydro-5H-cyclopenta[b]pyridin-4-yl)carbamoyl)-4-(hydroxymethyl)-2-(2-hydroxypropan-2-yl)thiazole-5-sulfonimidamide | 466 |
| 302 | 673 | 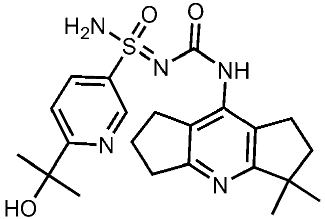 | N'-((3,3-dimethyl-1,2,3,5,6,7-hexahydrodicyclopenta[b,e] pyridin-8-yl)carbamoyl)-6-(2-hydroxypropan-2-yl)pyridine-3-sulfonimidamide | 444 |
| 303 | 704 | 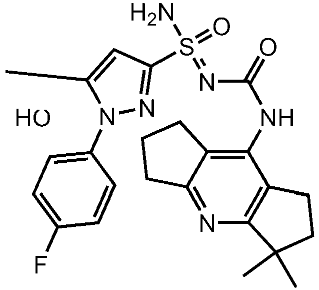 | N'-((3,3-dimethyl-1,2,3,5,6,7-hexahydrodicyclopenta[b,e] pyridin-8-yl)carbamoyl)-1-(4-fluorophenyl)-5-(2-hydroxypropan-2-yl)-1H-pyrazole-3-sulfonimidamide | 527 |
| 304 | 664 | 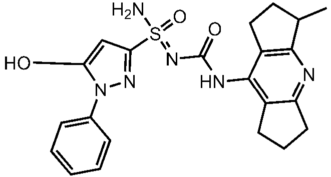 | 5-(2-Hydroxypropan-2-yl)-N'-((3-methyl-1,2,3,5,6,7-hexahydrodicyclopenta[b,e] pyridin-8-yl)carbamoyl)-1-phenyl-1H-pyrazole-3-sulfonimidamide | 495 |
| 305 | 663 | 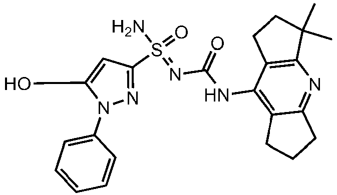 | N'-((3,3-dimethyl-1,2,3,5,6,7-hexahydrodicyclopenta[b,e] pyridin-8-yl)carbamoyl)-5-(2-hydroxypropan-2-yl)-1-phenyl-1H-pyrazole-3-sulfonimidamide | 509 |
| 306 | 651 | 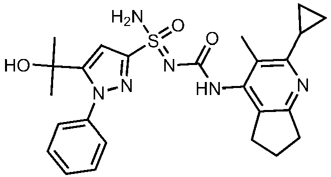 | N'-((2-cyclopropyl-3-methyl-6,7-dihydro-5H-cyclopenta[b]pyridin-4-yl)carbamoyl)-5-(2-hydroxypropan-2-yl)-1-phenyl-1H-pyrazole-3-sulfonimidamide | 495 |
| 307 | 659 | 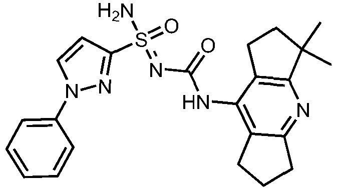 | N'-((3,3-dimethyl-1,2,3,5,6,7-hexahydrodicyclopenta[b,e] pyridin-8-yl)carbamoyl)-1-phenyl-1H-pyrazole-3-sulfonimidamide | 451 |
| 308 | 662 | 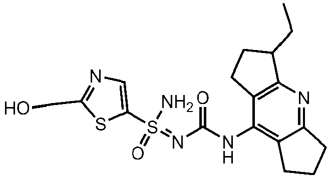 | N'-((3-ethyl-1,2,3,5,6,7-hexahydrodicyclopenta[b,e] pyridin-8-yl)carbamoyl)-2-(2-hydroxypropan-2-yl)thiazole-5-sulfonimidamide | 450 |
| 309 | 649 | 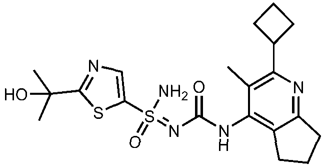 | N'-((2-cyclobutyl-3-methyl-6,7-dihydro-5H-cyclopenta[b]pyridin-4-yl)carbamoyl)-2-(2-hydroxypropan-2-yl)thiazole-5-sulfonimidamide | 450 |
| 310 | 650 | 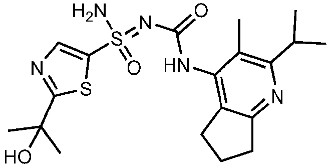 | 2-(2-Hydroxypropan-2-yl)-N'-((2-isopropyl-3-methyl-6,7-dihydro-5H-cyclopenta[b]pyridin-4-yl)carbamoyl)thiazole-5-sulfonimidamide | 438 |
| 311 | 648 | 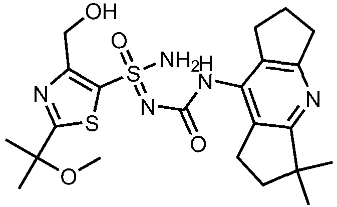 | N'-((3,3-dimethyl-1,2,3,5,6,7-hexahydrodicyclopenta[b,e] pyridin-8-yl)carbamoyl)-4-(hydroxymethyl)-2-(2-methoxypropan-2-yl)thiazole-5-sulfonimidamide | 494 |
| 312 | 615 | 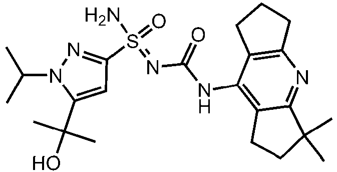 | N'-((3,3-dimethyl-1,2,3,5,6,7-hexahydrodicyclopenta[b,e] pyridin-8-yl)carbamoyl)-5-(2-hydroxypropan-2-yl)-1-isopropyl-1H-pyrazole-3-sulfonimidamide | 475 |
| 313 | 620 | 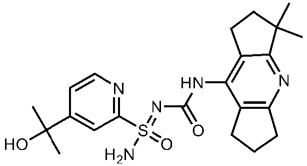 | N'-((3,3-dimethyl-1,2,3,5,6,7-hexahydrodicyclopenta[b,e] pyridin-8-yl)carbamoyl)-4-(2-hydroxypropan-2-yl)pyridine-2-sulfonimidamide | 444 |
| 314 | 185 | 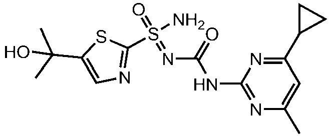 | N'-((4-cyclopropyl-6-methylpyrimidin-2-yl)carbamoyl)-5-(2-hydroxypropan-2-yl)thiazole-2-sulfonimidamide | 397 |
| 315 | 690 | 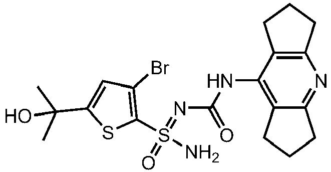 | 3-Bromo-N'-((1,2,3,5,6,7-hexahydrodicyclopenta[b,e] pyridin-8-yl)carbamoyl)-5-(2-hydroxypropan-2-yl)thiophene-2-sulfonimidamide | 499 |
| 316 | 675 | 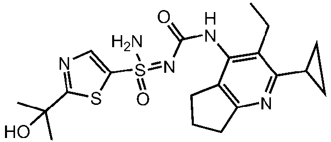 | N'-((2-cyclopropyl-3-ethyl-6,7-dihydro-5H-cyclopenta[b]pyridin-4-yl)carbamoyl)-2-(2-hydroxypropan-2-yl)thiazole-5-sulfonimidamide | 450 |
| 317 | 678 | 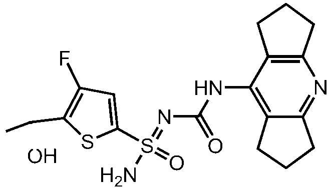 | 4-Fluoro-N'-((1,2,3,5,6,7-hexahydrodicyclopenta[b,e] pyridin-8-yl)carbamoyl)-5-(2-hydroxypropan-2-yl)thiophene-2-sulfonimidamide | 439 |
| 318 | 671 | 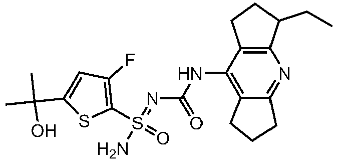 | N'-((3-ethyl-1,2,3,5,6,7-hexahydrodicyclopenta[b,e] pyridin-8-yl)carbamoyl)-3-fluoro-5-(2-hydroxypropan-2-yl)thiophene-2-sulfonimidamide | 467 |
| 319 | 657 | 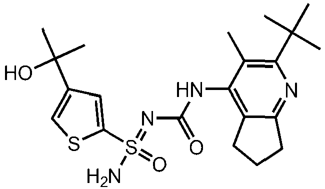 | N'-((2-(tert-butyl)-3-methyl-6,7-dihydro-5H-cyclopenta[b]pyridin-4-yl)carbamoyl)-4-(2-hydroxypropan-2-yl)thiophene-2-sulfonimidamide | 451 |
| 320 | 670 | 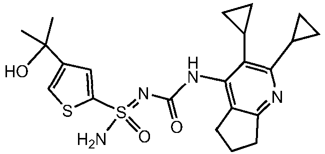 | N'-((2,3-dicyclopropyl-6,7-dihydro-5H-cyclopenta[b]pyridin-4-yl)carbamoyl)-4-(2-hydroxypropan-2-yl)thiophene-2-sulfonimidamide | 461 |
| 321 | 655 | 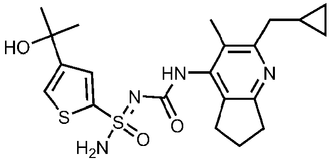 | N'-((2-(cyclopropylmethyl)-3-methyl-6,7-dihydro-5H-cyclopenta[b]pyridin-4-yl)carbamoyl)-4-(2-hydroxypropan-2-yl)thiophene-2-sulfonimidamide | 449 |
| 322 | 654 | 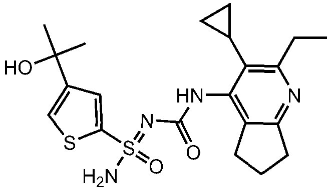 | N'-((3-cyclopropyl-2-ethyl-6,7-dihydro-5H-cyclopenta[b]pyridin-4-yl)carbamoyl)-4-(2-hydroxypropan-2-yl)thiophene-2-sulfonimidamide | 449 |
| 323 | 634 | 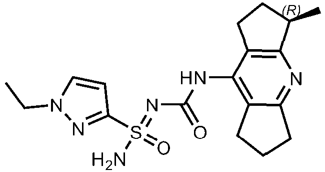 | 1-Ethyl-N'-(((R)-3-methyl-1,2,3,5,6,7-hexahydrodicyclopenta[b,e] pyridin-8-yl)carbamoyl)-1H-pyrazole-3-sulfonimidamide | 389 |
| 324 | 639 | 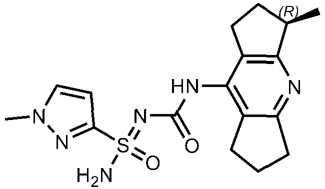 | 1-Methyl-N'-(((R)-3-methyl-1,2,3,5,6,7-hexahydrodicyclopenta[b,e] pyridin-8-yl)carbamoyl)-1H-pyrazole-3-sulfonimidamide | 375 |
| 325 | 646a | 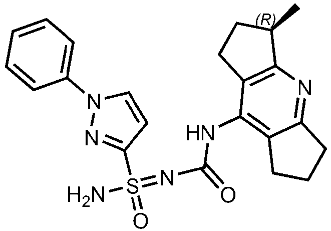 | N'-(((R)-3-methyl-1,2,3,5,6,7-hexahydrodicyclopenta[b,e] pyridin-8-yl)carbamoyl)-1-phenyl-1H-pyrazole-3-sulfonimidamide | 437 |
| 326 | 638 | 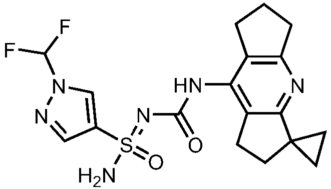 | 1-(Difluoromethyl)-N'-((1',5',6',7'-tetrahydro-2'H-spiro[cyclopropane-1,3'-dicyclopenta[b,e]pyridin]-8'-yl)carbamoyl)-1H-pyrazole-4-sulfonimidamide | 423 |
| 327 | 637 | 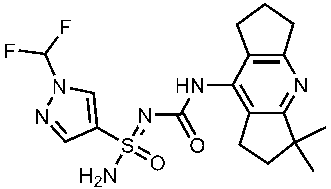 | 1-(Difluoromethyl)-N'-((3,3-dimethyl-1,2,3,5,6,7-hexahydrodicyclopenta[b,e] pyridin-8-yl)carbamoyl)-1H-pyrazole-4-sulfonimidamide | 425 |
| 328 | 633 | 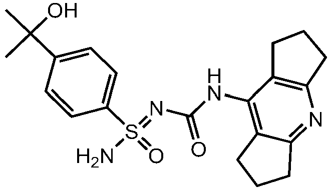 | N'-((1,2,3,5,6,7-hexahydrodicyclopenta[b,e] pyridin-8-yl)carbamoyl)-4-(2-hydroxypropan-2-yl)benzenesulfonimidamide | 415 |
| 329 | 625 | 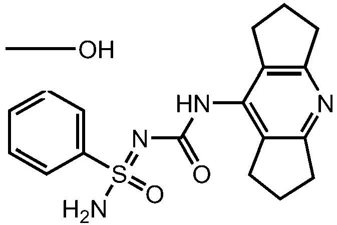 | N'-((1,2,3,5,6,7-hexahydrodicyclopenta[b,e] pyridin-8-yl)carbamoyl)-3-(2-hydroxypropan-2-yl)benzenesulfonimidamide | 415 |
| 330 | 622 | 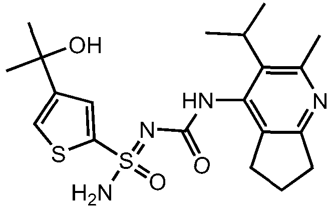 | 4-(2-Hydroxypropan-2-yl)-N'-((3-isopropyl-2-methyl-6,7-dihydro-5H-cyclopenta[b]pyridin-4-yl)carbamoyl)thiophene-2-sulfonimidamide | 437 |
| 331 | 628 | 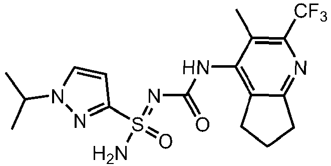 | 1-Isopropyl-N'-((3-methyl-2-(trifluoromethyl)-6,7-dihydro-5H-cyclopenta[b]pyridin-4-yl)carbamoyl)-1H-pyrazole-3-sulfonimidamide | 431 |
| 332 | 617 | 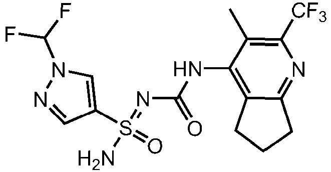 | 1-(Difluoromethyl)-N'-((3-methyl-2-(trifluoromethyl)-6,7-dihydro-5H-cyclopenta[b]pyridin-4-yl)carbamoyl)-1H-pyrazole-4-sulfonimidamide | 439 |
| 333 | 614 | 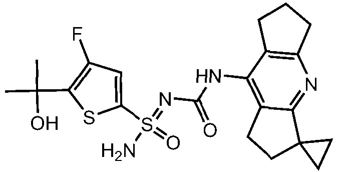 | 4-Fluoro-5-(2-hydroxypropan-2-yl)-N'-((1',5',6',7'-tetrahydro-2'H-spiro[cyclopropane-1,3'-dicyclopenta[b,e]pyridin]-8'-yl)carbamoyl)thiophene-2-sulfonimidamide | 465 |
| 334 | 613 | 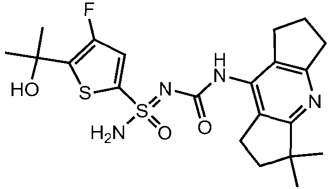 | N'-((3,3-dimethyl-1,2,3,5,6,7-hexahydrodicyclopenta[b,e] pyridin-8-yl)carbamoyl)-4-fluoro-5-(2-hydroxypropan-2-yl)thiophene-2-sulfonimidamide | 467 |
| 335 | 602 | 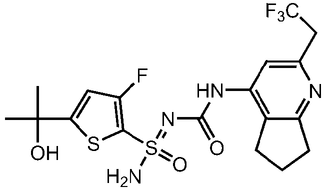 | 3-Fluoro-5-(2-hydroxypropan-2-yl)-N'-((2-(2,2,2-trifluoroethyl)-6,7-dihydro-5H-cyclopenta[b]pyridin-4-yl)carbamoyl)thiophene-2-sulfonimidamide | 481 |
| 336 | 636c | 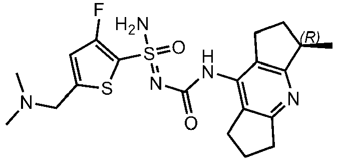 | 5-((Dimethylamino)methyl)-3-fluoro-N'-(((R)-3-methyl-1,2,3,5,6,7-hexahydrodicyclopenta[b,e] pyridin-8-yl)carbamoyl)thiophene-2-sulfonimidamide | 452 |
| 337 | 601 | 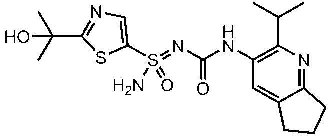 | 2-(2-Hydroxypropan-2-yl)-N'-((2-isopropyl-6,7-dihydro-5H-cyclopenta[b]pyridin-3-yl)carbamoyl)thiazole-5-sulfonimidamide | 424 |
| 338 | 685 | 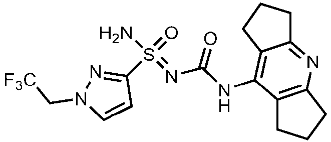 | N'-((1,2,3,5,6,7-hexahydrodicyclopenta[b,e] pyridin-8-yl)carbamoyl)-1-(2,2,2-trifluoroethyl)-1H-pyrazole-3-sulfonimidamide | 429 |

**Table 38. Examples in the following table were obtained from chiral HPLC resolutions of racemic and diastereomeric mixture examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. Assigned stereochemistry in compound names are tentative.**

| Ex. # | Final Target Number | Structure | IUPAC Name | Column | Eluents | LC-MS [M+H]⁺ |
|---|---|---|---|---|---|---|
| 339 | 685b | 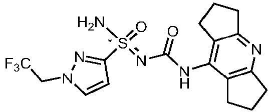 | (R) or (S)-N'-((1,2,3,5,6,7-hexahydrodicyclope nta[b,e]pyridin-8-yl)carbamoyl)-1-(2,2,2-trifluoroethy1)-1H-pyrazole-3-sulfonimidamide | CHIRALPA K IG, 20*250 mm, 5 um | 20% EtOH in MTBE (10 mM NH₃-MeOH) | 429 |
| 340 | 685a | 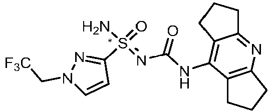 | (S) or (R)-N'-((1,2,3,5,6,7-hexahydrodicyclope nta[b,e]pyridin-8-yl)carbamoyl)-1-(2,2,2-trifluoroethy1)-1H-pyrazole-3-sulfonimidamide | CHIRALPA K IG, 20*250 mm, 5 um | 20% EtOH in MTBE (10 mM NH₃-MeOH) | 429 |
| 341 | 694b | 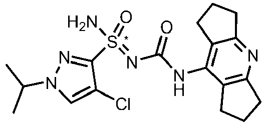 | (R) or (S)-4-Chloro-N'-((1,2,3,5,6,7-hexahydrodicyclope nta[b,e]pyridin-8-yl)carbamoyl)-1-isopropyl-1H-pyrazole-3-sulfonimidamide | CHIRALPA K IE, 2*25 cm, 5 um | 50% EtOH in Hex (8 mM NH₃·MeOH) | 423/425 |
| 342 | 694a | 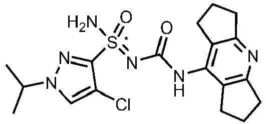 | (S) or (R)-4-Chloro-N'-((1,2,3,5,6,7-hexahydrodicyclope nta[b,e]pyridin-8-yl)carbamoyl)-1-isopropyl-1H-pyrazole-3-sulfonimidamide | CHIRALPA K IE, 2*25 cm, 5 um | 50% EtOH in Hex (8 mM NH₃·MeOH) | 423/425 |
| 343 | 687b | 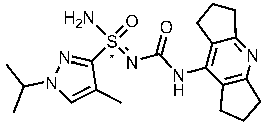 | (R) or (S)-N'-((1,2,3,5,6,7-hexahydrodicyclope nta[b,e]pyridin-8-yl)carbamoyl)-1-isopropyl-4-methyl-1H-pyrazole-3-sulfonimidamide | CHIRALPA K IG, 2*25 cm, 5 um | 50% EtOH in Hex (8 mM NH₃·MeOH) | 403 |
| 344 | 687a | 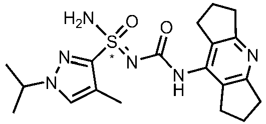 | (S) or (R)-N'-((1,2,3,5,6,7-hexahydrodicyclope nta[b,e]pyridin-8-yl)carbamoyl)-1-isopropyl-4-methyl-1H-pyrazole-3-sulfonimidamide | CHIRALPA K IG, 2*25 cm, 5 um | 50% EtOH in Hex (8 mM NH₃·MeOH) | 403 |
| 345 | 689b | 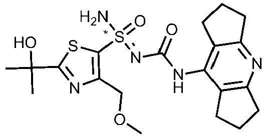 | (R) or (S)-N'-((1,2,3,5,6,7-hexahydrodicyclope nta[b,e]pyridin-8-yl)carbamoyl)-2-(2-hydroxypropan-2-yl)-4-(methoxymethyl)thia zole-5-sulfonimidamide | CHIRALPA K IG, 2*25 cm, 5 um | 30% EtOH in Hex (8 mM NH₃·MeOH) | 466 |
| 346 | 689a | 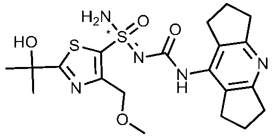 | (S) or (R)-N'-((1,2,3,5,6,7-hexahydrodicyclope nta[b,e]pyridin-8-yl)carbamoyl)-2-(2-hydroxypropan-2-yl)-4-(methoxymethyl)thia zole-5-sulfonimidamide | CHIRALPA K IG, 2*25 cm, 5 um | 30% EtOH in Hex (8 mM NH₃·MeOH) | 466 |
| 347 | 642b | 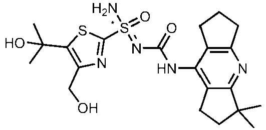 | (R) or (S)-N'-((3,3-dimethyl-1,2,3,5,6,7-hexahydrodicyclope nta[b,e]pyridin-8-yl)carbamoyl)-4-(hydroxymethyl)-5-(2-hydroxypropan-2-yl)thiazole-2-sulfonimidamide | CHIRALPA K IC, 2*25 cm, 5 um | 30% IPA in Hex (0.1% FA) | 480 |
| 348 | 642a | 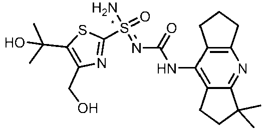 | (S) or (R)-N'-((3,3-dimethyl-1,2,3,5,6,7-hexahydrodicyclope nta[b,e]pyridin-8-yl)carbamoyl)-4-(hydroxymethyl)-5-(2-hydroxypropan-2-yl)thiazole-2-sulfonimidamide | CHIRALPA K IC, 2*25 cm, 5 um | 30% IPA in Hex (0.1% FA) | 480 |
| 349 | 686b | 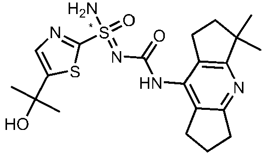 | (R) or (S)-N'-((3,3-dimethyl-1,2,3,5,6,7-hexahydrodicyclope nta[b,e]pyridin-8-yl)carbamoyl)-5-(2-hydroxypropan-2-yl)thiazole-2-sulfonimidamide | CHIRALPA K ID, 2*25 cm, 5 um | 30% MeOH:ACN=4 :1 in CO₂ | 450 |
| 350 | 686a | 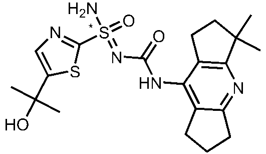 | (S) or (R)-N'-((3,3-dimethyl-1,2,3,5,6,7-hexahydrodicyclope nta[b,e]pyridin-8-yl)carbamoyl)-5-(2-hydroxypropan-2-yl)thiazole-2-sulfonimidamide | CHIRALPA K ID, 2*25 cm, 5 um | 30% MeOH:ACN=4 :1 in CO₂ | 450 |
| 351 | 170b | 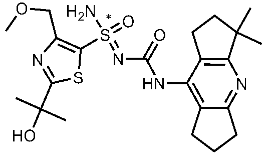 | (S) or (R)-N'-((3,3-dimethyl-1,2,3,5,6,7-hexahydrodicyclope nta[b,e]pyridin-8-yl)carbamoyl)-2-(2-hydroxypropan-2-yl)-4-(methoxymethyl)thia zole-5-sulfonimidamide | CHIRAL ART Cellulose-SB, 5 um, 250*20 mm | 30% EtOH in Hex (8 mM NH₃·MeOH) | 494 |
| 352 | 170a | 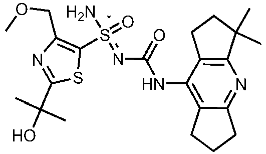 | (R) or (S)-N'-((3,3-dimethyl-1,2,3,5,6,7-hexahydrodicyclope nta[b,e]pyridin-8-yl)carbamoyl)-2-(2-hydroxypropan-2-yl)-4-(methoxymethyl)thia zole-5-sulfonimidamide | CHIRAL ART Cellulose-SB, 5 um, 250*20 mm | 30% EtOH in Hex (8 mM NH₃·MeOH) | 494 |
| 353 | 680b | 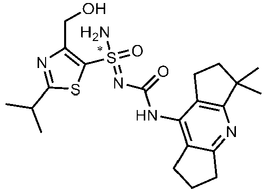 | (R) or (S)-N'-((3,3-dimethyl-1,2,3,5,6,7-hexahydrodicyclope nta[b,e]pyridin-8-yl)carbamoyl)-4-(hydroxymethyl)-2-isopropylthiazole-5-sulfonimidamide | CHIRALPA K IC, 2*25 cm, 5 um | 50% EtOH (0.1% NH₃·H₂O) in Hex | 464 |
| 354 | 680a | 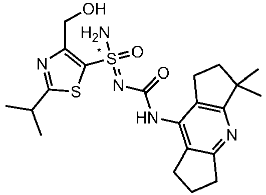 | (S) or (R)-N'-((3,3-dimethyl-1,2,3,5,6,7-hexahydrodicyclope nta[b,e]pyridin-8-yl)carbamoyl)-4-(hydroxymethyl)-2-isopropylthiazole-5-sulfonimidamide | CHIRALPA K IC, 2*25 cm, 5 um | 50% EtOH (0.1% NH₃·H₂O) in Hex | 464 |
| 355 | 682b | 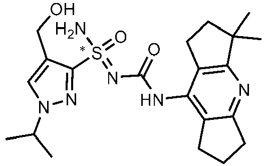 | (R) or (S)-N'-((3,3-dimethyl-1,2,3,5,6,7-hexahydrodicyclope nta[b,e]pyridin-8-yl)carbamoyl)-4-(hydroxymethyl)-1-isopropyl-1H-pyrazole-3-sulfonimidamide | Chiralpak AD, 2*25 cm, 5 um | 40% IPA (2 mM NH₃) in CO₂ | 447 |
| 356 | 682a | 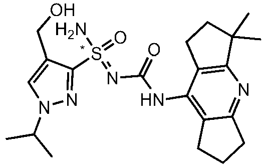 | (S) or (R)-N'-((3,3-dimethyl-1,2,3,5,6,7-hexahydrodicyclope nta[b,e]pyridin-8-yl)carbamoyl)-4-(hydroxymethyl)-1-isopropyl-1H-pyrazole-3-sulfonimidamide | Chiralpak AD, 2*25 cm, 5 um | 40% IPA (2 mM NH₃) in CO₂ | 447 |
| 357 | 653b | 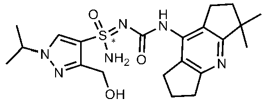 | (S) or (R)-N'-((3,3-dimethyl-1,2,3,5,6,7-hexahydrodicyclope nta[b,e]pyridin-8-yl)carbamoyl)-3-(hydroxymethyl)-1-isopropyl-1H-pyrazole-4-sulfonimidamide | CHIRALPA K AD, 2*25 cm, 5 um | 30% EtOH (2 mM NH₃-MeOH) in CO₂ | 447 |
| 358 | 653a | 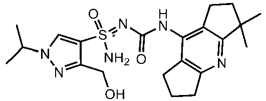 | (R) or (S)-N'-((3,3-dimethyl-1,2,3,5,6,7-hexahydrodicyclope nta[b,e]pyridin-8-yl)carbamoyl)-3-(hydroxymethyl)-1-isopropyl-1H-pyrazole-4-sulfonimidamide | CHIRALPA K AD, 2*25 cm, 5 um | 30% EtOH (2 mM NH₃-MeOH) in CO₂ | 447 |
| 359 | 674b | 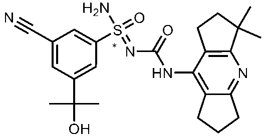 | (R) or (S)-3-Cyano-N'-((3,3-dimethyl-1,2,3,5,6,7-hexahydrodicyclope nta[b,e]pyridin-8-yl)carbamoyl)-5-(2-hydroxypropan-2-yl)benzenesulfonimi damide | Chiralpak IC, 2*25 cm, 5 um | 35% MeOH (2 mM NH₃-MeOH) in CO₂ | 468 |
| 360 | 674a | 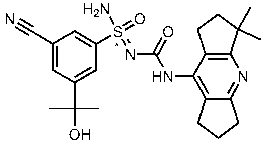 | (S) or (R)-3-Cyano-N'-((3,3-dimethyl-1,2,3,5,6,7-hexahydrodicyclope nta[b,e]pyridin-8-yl)carbamoyl)-5-(2-hydroxypropan-2-yl)benzenesulfonimi damide | Chiralpak IC, 2*25 cm, 5 um | 35% MeOH (2 mM NH₃-MeOH) in CO₂ | 468 |
| 361 | 684b | 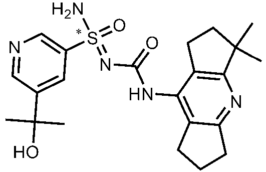 | (S) or (R)-N'-((3,3-dimethyl-1,2,3,5,6,7-hexahydrodicyclope nta[b,e]pyridin-8-yl)carbamoyl)-5-(2-hydroxypropan-2-yl)pyridine-3-sulfonimidamide | CHIRAL ART Cellulose-SB, 2*25 cm, 5 um | 25% EtOH in Hex | 444 |
| 362 | 684a | 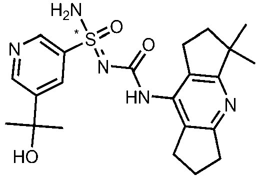 | (R) or (S)-N'-((3,3-dimethyl-1,2,3,5,6,7-hexahydrodicyclope nta[b,e]pyridin-8-yl)carbamoyl)-5-(2-hydroxypropan-2-yl)pyridine-3-sulfonimidamide | CHIRAL ART Cellulose-SB, 2*25 cm, 5 um | 25% EtOH in Hex | 444 |
| 363 | 683b | 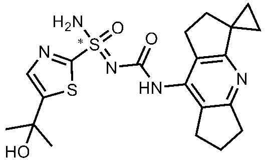 | (S) or (R)-5-(2-Hydroxypropan-2-yl)-N'-((1',5',6',1'-tetrahydro-2'H-spiro[cyclopropane-1,3'-dicyclopenta[b,e]pyr idin]-8'-yl)carbamoyl)thiazol e-2-sulfonimidamide | CHIRAL ART Cellulose-SB, 2*25 cm, 5 um | 40% IPA in EtOH (0.1% DEA) | 448 |
| 364 | 683a | 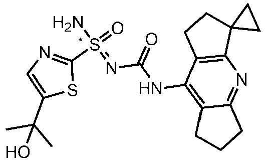 | (R) or (S)-5-(2-Hydroxypropan-2-yl)-N'-((1',5',6',1'-tetrahydro-2'H-spiro[cyclopropane-1,3'-dicyclopenta[b,e]pyr idin]-8'-yl)carbamoyl)thiazol e-2-sulfonimidamide | CHIRAL ART Cellulose-SB, 2*25 cm, 5 um | 40% IPA in EtOH (0.1% DEA) | 448 |
| 365 | 673b | 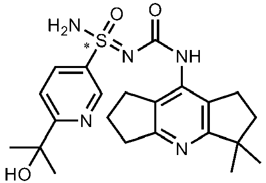 | (S) or (R)-N'-((3,3-dimethyl-1,2,3,5,6,7-hexahydrodicyclope nta[b,e]pyridin-8-yl)carbamoyl)-6-(2-hydroxypropan-2-yl)pyridine-3-sulfonimidamide | CHIRAL ART Cellulose-SB, 2*25 cm, 5 um | 30% EtOH in Hex | 444 |
| 366 | 673a | 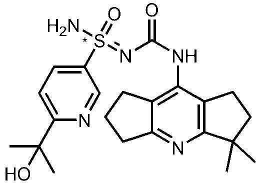 | (R) or (S)-N'-((3,3-dimethyl-1,2,3,5,6,7-hexahydrodicyclope nta[b,e]pyridin-8-yl)carbamoyl)-6-(2-hydroxypropan-2-yl)pyridine-3-sulfonimidamide | CHIRAL ART Cellulose-SB, 2*25 cm, 5 um | 30% EtOH in Hex | 444 |
| 367 | 705b | 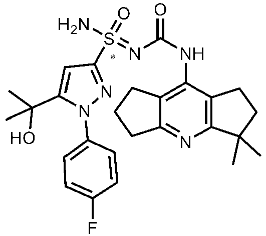 | (R) or (S)-N'-((3,3-dimethyl-1,2,3,5,6,7-hexahydrodicyclope nta[b,e]pyridin-8-yl)carbamoyl)-1-(4-fluorophenyl)-5-(2-hydroxypropan-2-yl)-1H-pyrazole-3-sulfonimidamide | Lux 5 um Cellulose-4, AXIA packed, 2.12*25 cm | 50% MeOH (2 mM NH₃-MeOH) in CO₂ | 527 |
| 368 | 705a | 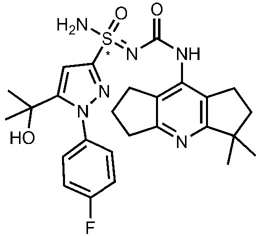 | (S) or (R)-N'-((3,3-dimethyl-1,2,3,5,6,7-hexahydrodicyclope nta[b,e]pyridin-8-yl)carbamoyl)-1-(4-fluorophenyl)-5-(2-hydroxypropan-2-yl)-1H-pyrazole-3-sulfonimidamide | Lux 5 um Cellulose-4, AXIA packed, 2.12*25 cm | 50% MeOH (2 mM NH₃-MeOH) in CO₂ | 527 |
| 369 | 664b | 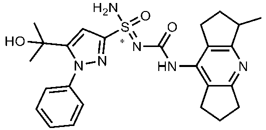 | (R) or (S)-5-(2-Hydroxypropan-2-yl)-N'-((3-methyl-1,2,3,5,6,7-hexahydrodicyclope nta[b,e]pyridin-8-yl)carbamoyl)-1-phenyl-1H-pyrazole-3-sulfonimidamide | CHIRALPA K IC, 2*25 cm, 5 um | 30% EtOH in Hex (0.1% FA) | 495 |
| 370 | 664a | 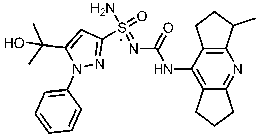 | (S) or (R)-5-(2-Hydroxypropan-2-yl)-N'-((3-methyl-1,2,3,5,6,7-hexahydrodicyclope nta[b,e]pyridin-8-yl)carbamoyl)-1-phenyl-1H-pyrazole-3-sulfonimidamide | CHIRALPA K IC, 2*25 cm, 5 um | 30% EtOH in Hex (0.1% FA) | 495 |
| 371 | 663b | 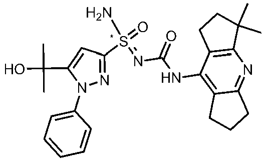 | (R) or (S)-N'-((3,3-dimethyl-1,2,3,5,6,7-hexahydrodicyclope nta[b,e]pyridin-8-yl)carbamoyl)-5-(2-hydroxypropan-2-yl)-1-phenyl-1H-pyrazole-3-sulfonimidamide | CHIRALPA K IC, 2*25 cm, 5 um | 30% EtOH in Hex (0.1% FA) | 509 |
| 372 | 663a | 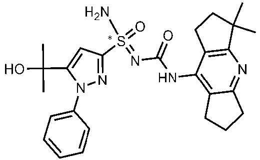 | (S) or (R)-N'-((3,3-dimethyl-1,2,3,5,6,7-hexahydrodicyclope nta[b,e]pyridin-8-yl)carbamoyl)-5-(2-hydroxypropan-2-yl)-1-phenyl-1H-pyrazole-3-sulfonimidamide | CHIRALPA K IC, 2*25 cm, 5 um | 30% EtOH in Hex (0.1% FA) | 509 |
| 373 | 651b | 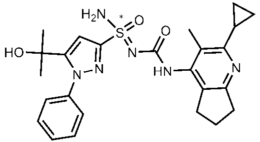 | (R) or (S)-N'-((2-cyclopropyl-3-methyl-6,7-dihydro-5H-cyclopenta[b]pyridin -4-yl)carbamoyl)-5-(2-hydroxypropan-2-yl)-1-phenyl-1H-pyrazole-3-sulfonimidamide | CHIRALPA K IG, 20*250 mm, 5 um | 30% EtOH in Hex (0.1% FA) | 495 |
| 374 | 651a | 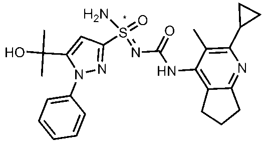 | (S) or (R)-N'-((2-cyclopropyl-3-methyl-6,7-dihydro-5H-cyclopenta[b]pyridin -4-yl)carbamoyl)-5-(2-hydroxypropan-2-yl)-1-phenyl-1H-pyrazole-3-sulfonimidamide | CHIRALPA K IG, 20*250 mm, 5 um | 30% EtOH in Hex (0.1% FA) | 495 |
| 375 | 659b | 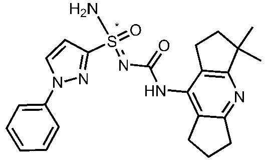 | (R) or (S)-N'-((3,3-dimethyl-1,2,3,5,6,7-hexahydrodicyclope nta[b,e]pyridin-8-yl)carbamoyl)-1-phenyl-1H-pyrazole-3-sulfonimidamide | CHIRALPA K IG, 5*25 cm, 5 um | 30% EtOH in Hex (0.1% FA) | 451 |
| 376 | 659a | 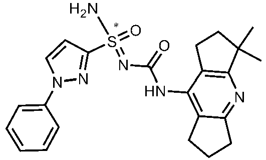 | (S) or (R)-N'-((3,3-dimethyl-1,2,3,5,6,7-hexahydrodicyclope nta[b,e]pyridin-8-yl)carbamoyl)-1-phenyl-1H-pyrazole-3-sulfonimidamide | CHIRALPA K IG, 5*25 cm, 5 um | 30% EtOH in Hex (0.1% FA) | 451 |
| 377 | 649b | 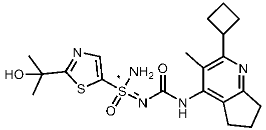 | (S) or (R)-N'-((2-cyclobutyl-3-methyl-6,7-dihydro-5H-cyclopenta[b]pyridin -4-yl)carbamoyl)-2-(2-hydroxypropan-2-yl)thiazole-5-sulfonimidamide | CHIRAL ART Cellulose-SB, 2*25 cm, 5 um | 30% EtOH in Hex (0.1% FA) | 450 |
| 378 | 649a | 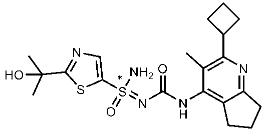 | (R) or (S)-N'-((2-cyclobutyl-3-methyl-6,7-dihydro-5H-cyclopenta[b]pyridin -4-yl)carbamoyl)-2-(2-hydroxvpropan-2-yl)thiazole-5-sulfonimidamide | CHIRAL ART Cellulose-SB, 2*25 cm, 5 um | 30% EtOH in Hex (0.1% FA) | 450 |
| 379 | 650b | 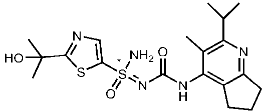 | (S) or (R)-2-(2-Hydroxypropan-2-yl)-N'-((2-isopropyl-3-methyl-6,7-dihydro-5H-cyclopenta[b]pyridin -4-yl)carbamoyl)thiazol e-5-sulfonimidamide | CHIRAL ART Cellulose-SB, 2*25 cm, 5 um | 30% EtOH in Hex (0.1% FA) | 438 |
| 380 | 650a | 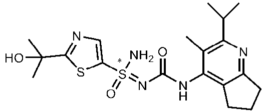 | (R) or (S)-2-(2-Hydroxypropan-2-yl)-N'-((2-isopropyl-3-methyl-6,7-dihydro-5H-cyclopenta[b]pyridin -4-yl)carbamoyl)thiazol e-5-sulfonimidamide | CHIRAL ART Cellulose-SB, 2*25 cm, 5 um | 30% EtOH in Hex (0.1% FA) | 438 |
| 381 | 648b | 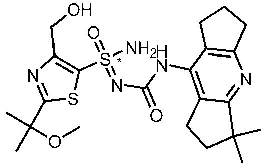 | (S) or (R)-N'-((3,3-dimethyl-1,2,3,5,6,7-hexahydrodicyclope nta[b,e]pyridin-8-yl)carbamoyl)-4-(hydroxymethyl)-2-(2-methoxypropan-2-yl)thiazole-5-sulfonimidamide | CHIRAL ART Cellulose-SB, 2*25 cm, 5 um | 20% EtOH in Hex (8 mM NH₃·MeOH) | 494 |
| 382 | 648a | 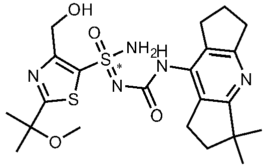 | (R) or (S)-N'-((3,3-dimethyl-1,2,3,5,6,7-hexahydrodicyclope nta[b,e]pyridin-8-yl)carbamoyl)-4-(hydroxymethyl)-2-(2-methoxypropan-2-yl)thiazole-5-sulfonimidamide | CHIRAL ART Cellulose-SB, 2*25 cm, 5 um | 20% EtOH in Hex (8 mM NH₃·MeOH) | 494 |
| 383 | 615b | 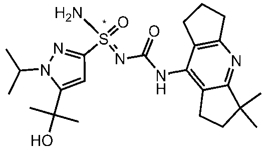 | (R) or (S)-N'-((3,3-dimethyl-1,2,3,5,6,7-hexahydrodicyclope nta[b,e]pyridin-8-yl)carbamoyl)-5-(2-hydroxypropan-2-yl)-1-isopropyl-1H-pyrazole-3-sulfonimidamide | CHIRALPA K IC, 2*25 cm, 5 um | 40% IPA in Hex (0.1% FA) | 475 |
| 384 | 615a | 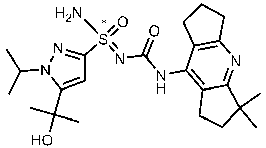 | (S) or (R)-N'-((3,3-dimethyl-1,2,3,5,6,7-hexahydrodicyclope nta[b,e]pyridin-8-yl)carbamoyl)-5-(2-hydroxypropan-2-yl)-1-isopropyl-1H-pyrazole-3-sulfonimidamide | CHIRALPA K IC, 2*25 cm, 5 um | 40% IPA in Hex (0.1% FA) | 475 |
| 385 | 620b | 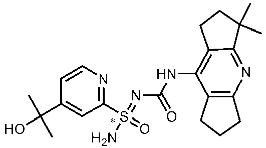 | (R) or (S)-N'-((3,3-dimethyl-1,2,3,5,6,7-hexahydrodicyclope nta[b,e]pyridin-8-yl)carbamoyl)-4-(2-hydroxypropan-2-yl)pyridine-2-sulfonimidamide | Chiralpak IC, 2*25 cm, 5 um | 30% EtOH in Hex (0.1% FA) | 444 |
| 386 | 620a | 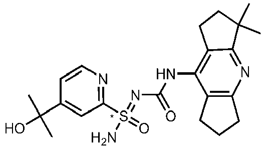 | (S) or (R)-N'-((3,3-dimethyl-1,2,3,5,6,7-hexahydrodicyclope nta[b,e]pyridin-8-yl)carbamoyl)-4-(2-hydroxypropan-2-yl)pyridine-2-sulfonimidamide | Chiralpak IC, 2*25 cm, 5 um | 30% EtOH in Hex (0.1% FA) | 444 |
| 387 | 185a | 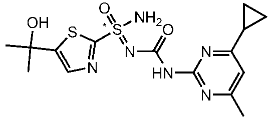 | (R) or (S)-N'-((4-cyclopropyl-6-methylpyrimidin-2-yl)carbamoyl)-5-(2-hydroxypropan-2-yl)thiazole-2-sulfonimidamide | Chiralpak ID, 2*25 cm, 5 um | 30% EtOH in Hex (0.1% FA) | 397 |
| 388 | 185b | 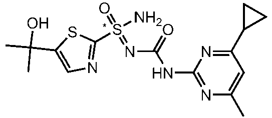 | (S) or (R)-N'-((4-cyclopropyl-6-methylpyrimidin-2-yl)carbamoyl)-5-(2-hydroxypropan-2-yl)thiazole-2-sulfonimidamide | Chiralpak ID, 2*25 cm, 5 um | 30% EtOH in Hex (0.1% FA) | 397 |
| 389 | 115b | 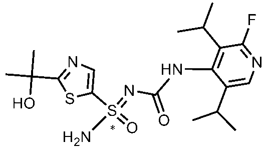 | (R) or (S)-N'-((2-fluoro-3,5-diisopropylpyridin-4-yl)carbamoyl)-2-(2-hydroxypropan-2-yl)thiazole-5-sulfonimidamide | Chiralpak IA, 2*25 cm, 5 um | 20% IPA in Hex (0.1% FA) | 444 |
| 390 | 115a | 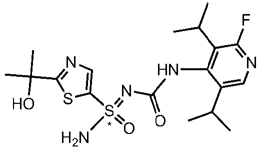 | (S) or (R)-N'-((2-fluoro-3,5-diisopropylpyridin-4-yl)carbamoyl)-2-(2-hydroxypropan-2-yl)thiazole-5-sulfonimidamide | Chiralpak IA, 2*25 cm, 5 um | 20% IPA in Hex (0.1% FA) | 444 |
| 391 | 701b | 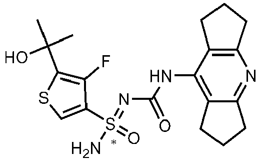 | (S) or (R)-4-Fluoro-N'-((1,2,3,5,6,7-hexahydrodicyclope nta[b,e]pyridin-8-yl)carbamoyl)-5-(2-hydroxypropan-2-yl)thiophene-3-sulfonimidamide | CHIRAL ART Cellulose-SB, 2*25 cm, 5 um | 30% EtOH in Hex (8 mM NH₃·MeOH) | 439 |
| 392 | 701a | 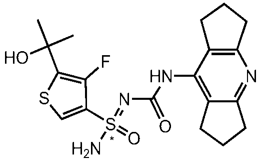 | (R) or (S)-4-Fluoro-N'-((1,2,3,5,6,7-hexahydrodicyclope nta[b,e]pyridin-8-yl)carbamoyl)-5-(2-hydroxypropan-2-yl)thiophene-3-sulfonimidamide | CHIRAL ART Cellulose-SB, 2*25 cm, 5 um | 30% EtOH in Hex (8 mM NH₃·MeOH) | 439 |
| 393 | 692b | 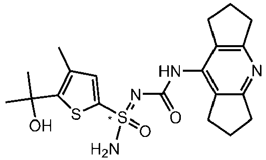 | (R) or (S)-N'-((1,2,3,5,6,7-hexahydrodicyclope nta[b,e]pyridin-8-yl)carbamoyl)-5-(2-hydroxypropan-2-yl)-4-methylthiophene-2-sulfonimidamide | CHIRALPA K IG, 2*25 cm, 5 um | 20% EtOH in MTBE (10 mM NH₃-MeOH) | 435 |
| 394 | 692a | 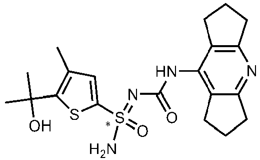 | (S) or (R)-N'-((1,2,3,5,6,7-hexahydrodicyclope nta[b,e]pyridin-8-yl)carbamoyl)-5-(2-hydroxypropan-2-yl)-4-methylthiophene-2-sulfonimidamide | CHIRALPA K IG, 2*25 cm, 5 um | 20% EtOH in MTBE (10 mM NH₃-MeOH) | 435 |
| 395 | 695b | 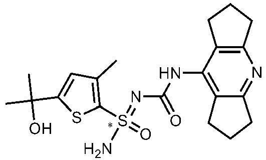 | (R) or (S)-N'-((1,2,3,5,6,7-hexahydrodicyclope nta[b,e]pyridin-8-yl)carbamoyl)-5-(2-hydroxypropan-2-yl)-3-methylthiophene-2-sulfonimidamide | CHIRALPA K IG, 2*25 cm, 5 um | 35% EtOH in Hex (8 mM NH₃·MeOH) | 435 |
| 396 | 695a | 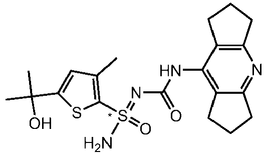 | (S) or (R)-N'-((1,2,3,5,6,7-hexahydrodicyclope nta[b,e]pyridin-8-yl)carbamoyl)-5-(2-hydroxypropan-2-yl)-3-methylthiophene-2-sulfonimidamide | CHIRALPA K IG, 2*25 cm, 5 um | 35% EtOH in Hex (8 mM NH₃·MeOH) | 435 |
| 397 | 691b | 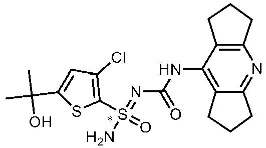 | (R) or (S)-3-Chloro-N'-((1,2,3,5,6,7-hexahydrodicyclope nta[b,e]pyridin-8-yl)carbamoyl)-5-(2-hydroxypropan-2-yl)thiophene-2-sulfonimidamide | CHIRALPA K IG, 2*25 cm, 5 um | 30% EtOH in Hex (8 mM NH₃·MeOH) | 455 |
| 398 | 691a | 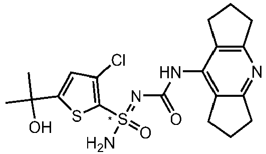 | (S) or (R)-3-Chloro-N'-((1,2,3,5,6,7-hexahydrodicyclope nta[b,e]pyridin-8-yl)carbamoyl)-5-(2-hydroxypropan-2-yl)thiophene-2-sulfonimidamide | CHIRALPA K IG, 22*25 cm, 5 um | 30% EtOH in Hex (8 mM NH₃·MeOH) | 455 |
| 399 | 688b | 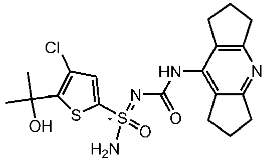 | (S) or (R)-4-Chloro-N'-((1,2,3,5,6,7-hexahydrodicyclope nta[b,e]pyridin-8-yl)carbamoyl)-5-(2-hydroxypropan-2-yl)thiophene-2-sulfonimidamide | CHIRAL ART Cellulose-SB, 2*25 cm, 5 um | 40% EtOH in Hex (8 mM NH₃·MeOH) | 455 |
| 400 | 688a | 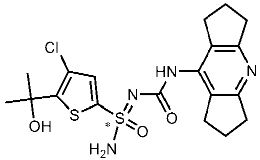 | (R) or (S)-4-Chloro-N'-((1,2,3,5,6,7-hexahydrodicyclope nta[b,e]pyridin-8-yl)carbamoyl)-5-(2-hydroxypropan-2-yl)thiophene-2-sulfonimidamide | CHIRAL ART Cellulose-SB, 2*25 cm, 5 um | 40% EtOH in Hex (8 mM NH₃·MeOH) | 455 |
| 401 | 690b | 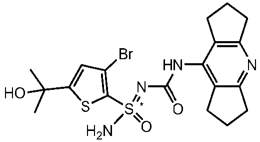 | (R) or (S)-3-Bromo-N'-((1,2,3,5,6,7-hexahydrodicyclope nta[b,e]pyridin-8-yl)carbamoyl)-5-(2-hydroxypropan-2-yl)thiophene-2-sulfonimidamide | CHIRALPA K IG, 2*25 cm, 5 um | 50% EtOH in Hex (8 mM NH₃·MeOH) | 499 |
| 402 | 690a | 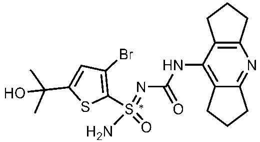 | (S) or (R)-3-Bromo-N'-((1,2,3,5,6,7-hexahydrodicyclope nta[b,e]pyridin-8-yl)carbamoyl)-5-(2-hydroxypropan-2-yl)thiophene-2-sulfonimidamide | CHIRALPA K IG, 2.0*25 cm, 5 um | 50% EtOH in Hex (8 mM NH₃·MeOH) | 499 |
| 403 | 661b | 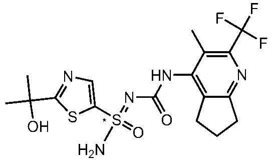 | (S) or (R)-2-(2-Hydroxypropan-2-yl)-N'-((3-methyl-2-(trifluoromethyl)-6,7-dihydro-5H-cyclopenta[b]pyridin -4-yl)carbamoyl)thiazol e-5-sulfonimidamide | CHIRAL ART Cellulose-SB, 2*25 cm, 5 um | 30% EtOH in Hex (8 mM NH₃·MeOH) | 464 |
| 404 | 661a | 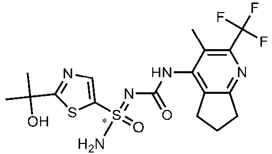 | (R) or (S)-2-(2-Hydroxypropan-2-yl)-N'-((3-methyl-2-(trifluoromethyl)-6,7-dihydro-5H-cyclopenta[b]pyridin -4-yl)carbamoyl)thiazol e-5-sulfonimidamide | CHIRAL ART Cellulose-SB, 2*25 cm, 5 um | 30% EtOH in Hex (8 mM NH₃·MeOH) | 464 |
| 405 | 647b | 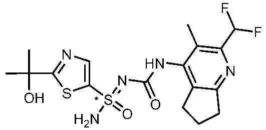 | (S) or (R)-N'-((2-(difluoromethyl)-3-methyl-6,7-dihydro-5H-cyclopenta[b]pyridin -4-yl)carbamoyl)-2-(2-hydroxypropan-2-yl)thiazole-5-sulfonimidamide | CHIRAL ART Cellulose-SB, 2*25 cm, 5 um | 30% EtOH in CO₂ | 446 |
| 406 | 647a | 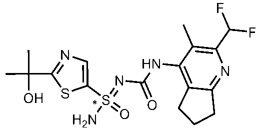 | (R) or (S)-N'-((2-(difluoromethyl)-3-methyl-6,7-dihydro-5H-cyclopenta[b]pyridin -4-yl)carbamoyl)-2-(2-hydroxypropan-2-yl)thiazole-5-sulfonimidamide | CHIRAL ART Cellulose-SB, 2*25 cm, 5 um | 30% EtOH in CO₂ | 446 |
| 407 | 676b | 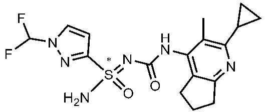 | (R) or (S)-N'-((2-cyclopropyl-3-methyl-6,7-dihydro-5H-cyclopenta[b]pyridin -4-yl)carbamoyl)-1-(difluoromethyl)-1H-pyrazole-3-sulfonimidamide | CHIRALPA K IG, 20*250 mm, 5 um | 10% EtOH in MTBE (10 mM NH₃-MeOH) | 411 |
| 408 | 676a | 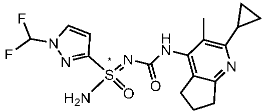 | (S) or (R)-N'-((2-cyclopropyl-3-methyl-6,7-dihydro-5H-cyclopenta[b]pyridin -4-yl)carbamoyl)-1-(difluoromethyl)-1H-pyrazole-3-sulfonimidamide | CHIRALPA K IG, 20*250 mm, 5 um | 10% EtOH in MTBE (10 mM NH₃-MeOH) | 411 |
| 409 | 181b | 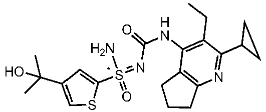 | (R) or (S)-N'-((2-cyclopropyl-3-ethyl-6,7-dihydro-5H-cyclopenta[b]pyridin -4-yl)carbamoyl)-4-(2-hydroxypropan-2-yl)thiophene-2-sulfonimidamide | Chiralpak AD, 2*25 cm 5 um | 30% IPA in Hex (8 mM NH₃.MeOH) | 449 |
| 410 | 181a | 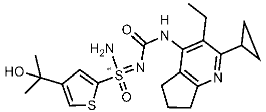 | (S) or (R)-N'-((2-cyclopropyl-3-ethyl-6,7-dihydro-5H-cyclopenta[b]pyridin -4-yl)carbamoyl)-4-(2-hydroxypropan-2-yl)thiophene-2-sulfonimidamide | Chiralpak AD, 2*25 cm 5 um | 30% IPA in Hex (8 mM NH₃.MeOH) | 449 |
| 411 | 675b | 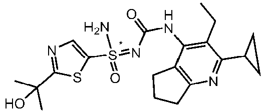 | (S) or (R)-N'-((2-cyclopropyl-3-ethyl-6,7-dihydro-5H-cyclopenta[b]pyridin -4-yl)carbamoyl)-2-(2-hydroxypropan-2-yl)thiazole-5-sulfonimidamide | CHIRAL ART Cellulose-SB, 2*25 cm, 5 um | 25% MeOH (2 mM NH₃-MeOH) in CO₂ | 450 |
| 412 | 675a | 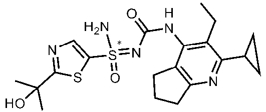 | (R) or (S)-N'-((2-cyclopropyl-3-ethyl-6,7-dihydro-5H-cyclopenta[b]pyridin -4-yl)carbamoyl)-2-(2-hydroxypropan-2-yl)thiazole-5-sulfonimidamide | CHIRAL ART Cellulose-SB, 2*25 cm, 5 um | 25% MeOH (2 mM NH₃-MeOH) in CO₂ | 450 |
| 413 | 681b | 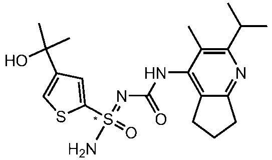 | (R) or (S)-4-(2-Hydroxypropan-2-yl)-N'-((2-isopropyl-3-methyl-6,7-dihydro-5H-cyclopenta[b]pyridin -4-yl)carbamoyl)thioph ene-2-sulfonimidamide | Chiralpak IC, 2*25 cm, 5 um | 10% EtOH in Hex:DCM (3:1, 10 mM NH₃-MeOH) | 437 |
| 414 | 681a | 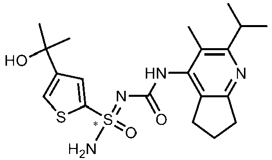 | (S) or (R)-4-(2-Hydroxypropan-2-yl)-N'-((2-isopropyl-3-methyl-6,7-dihydro-5H-cyclopenta[b]pyridin -4-yl)carbamoyl)thioph ene-2-sulfonimidamide | Chiralpak IC, 2*25 cm, 5 um | 10% EtOH in Hex:DCM (3:1, 10 mM NH₃-MeOH) | 437 |
| 415 | 669b | 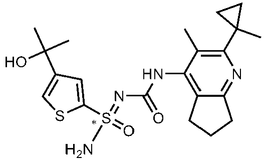 | (R) or (S)-4-(2-Hydroxypropan-2-yl)-N'-((3-methyl-2-(1-methylcyclopropyl)-6,7-dihydro-5H-cyclopenta[b]pyridin -4-yl)carbamoyl)thioph ene-2-sulfonimidamide | CHIRALPA K IG, 2*25 cm, 5 um | 30% EtOH in Hex (0.1% FA) | 449 |
| 416 | 669a | 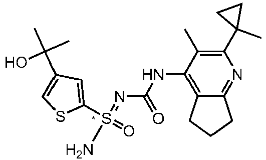 | (S) or (R)-4-(2-Hydroxypropan-2-yl)-N'-((3-methyl-2-(1-methylcyclopropyl)-6,7-dihydro-5H-cyclopenta[b]pyridin -4-yl)carbamoyl)thioph ene-2-sulfonimidamide | CHIRALPA K IG, 2*25 cm, 5 um | 30% EtOH in Hex (0.1% FA) | 449 |
| 417 | 176b | 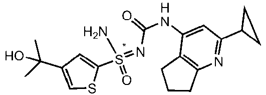 | (R) or (S)-N'-((2-cyclopropyl-6,7-dihydro-5H-cyclopenta[b]pyridin -4-yl)carbamoyl)-4-(2-hydroxypropan-2-yl)thiophene-2-sulfonimidamide | Lux 5 um Cellulose-4 AXIA packed, 2.12*25 cm | 40% EtOH in MeOH (2 mM NH₃-MeOH) | 421 |
| 418 | 176a | 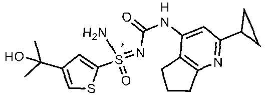 | (S) or (R)-N'-((2-cyclopropyl-6,7-dihydro-5H-cyclopenta[b]pyridin -4-yl)carbamoyl)-4-(2-hydroxypropan-2-yl)thiophene-2-sulfonimidamide | Lux 5 um Cellulose-4 AXIA packed, 2.12*25 cm | 40% EtOH in MeOH (2 mM NH₃-MeOH) | 421 |
| 419 | 182b | 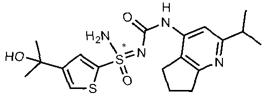 | (R) or (S)-4-(2-Hydroxypropan-2-yl)-N'-((2-isopropyl-6,7-dihydro-5H-cyclopenta[b]pyridin -4-yl)carbamoyl)thioph ene-2-sulfonimidamide | Lux 5 um Cellulose-4 AXIA packed, 2.12*25 cm | 40% MeOH (2 mM NH₃-MeOH) in CO₂ | 423 |
| 420 | 182a | 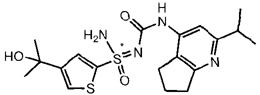 | (S) or (R)-4-(2-Hydroxypropan-2-yl)-N'-((2-isopropyl-6,7-dihydro-5H-cyclopenta[b]pyridin -4-yl)carbamoyl)thioph ene-2-sulfonimidamide | Lux 5 um Cellulose-4 AXIA packed, 2.12*25 cm | 40% MeOH (2 mM NH₃-MeOH) in CO₂ | 423 |
| 421 | 678b | 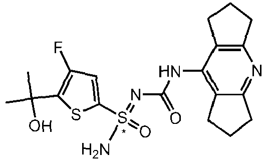 | (S) or (R)-4-Fluoro-N'-((1,2,3,5,6,7-hexahydrodicyclope nta[b,e]pyridin-8-yl)carbamoyl)-5-(2-hydroxypropan-2-yl)thiophene-2-sulfonimidamide | CHIRAL ART Cellulose-SB, 2*25 cm, 5 um | 30% EtOH in Hex (8 mM NH₃-MeOH) | 439 |
| 422 | 678a | 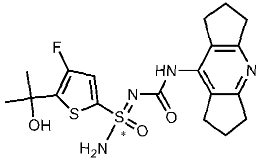 | (R) or (S)-4-Fluoro-N'-((1,2,3,5,6,7-hexahydrodicyclope nta[b,e]pyridin-8-yl)carbamoyl)-5-(2-hydroxypropan-2-yl)thiophene-2-sulfonimidamide | CHIRAL ART Cellulose-SB, 2*25 cm, 5 um | 30% EtOH in Hex (8 mM NH₃·MeOH) | 439 |
| 423 | 658b | 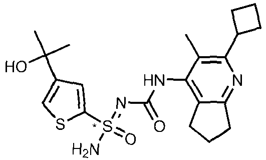 | (R) or (S)-N'-((2-cyclobutyl-3-methyl-6,7-dihydro-5H-cyclopenta[b]pyridin -4-yl)carbamoyl)-4-(2-hydroxypropan-2-yl)thiophene-2-sulfonimidamide | CHIRALPA K IG, 5*25 cm, 5 um | 30% EtOH in Hex (0.1% FA) | 449 |
| 424 | 658a | 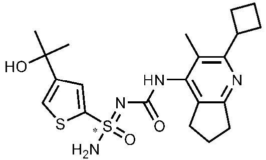 | (S) or (R)-N'-((2-cyclobutyl-3-methyl-6,7-dihydro-5H-cyclopenta[b]pyridin -4-yl)carbamoyl)-4-(2-hydroxypropan-2-yl)thiophene-2-sulfonimidamide | CHIRALPA K IG, 5*25 cm, 5 um | 30% EtOH in Hex (0.1% FA) | 449 |
| 425 | 667b | 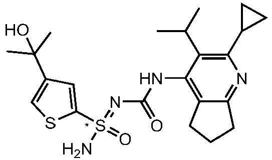 | (R) or (S)-N'-((2-cyclopropyl-3-isopropyl-6,7-dihydro-5H-cyclopenta[b]pyridin -4-yl)carbamoyl)-4-(2-hydroxypropan-2-yl)thiophene-2-sulfonimidamide | CHIRALPA K ID, 2*25 cm, 5 um | 30% MeOH (2mM NH₃-MeOH) in CO₂ | 463 |
| 426 | 667a | 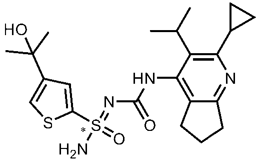 | (S) or (R)-N'-((2-cyclopropyl-3-isopropyl-6,7-dihydro-5H-cyclopenta[b]pyridin -4-yl)carbamoyl)-4-(2-hydroxypropan-2-yl)thiophene-2-sulfonimidamide | CHIRALPA K ID, 2*25 cm, 5 um | 30% MeOH (2mM NH₃-MeOH) in CO₂ | 463 |
| 427 | 657b | 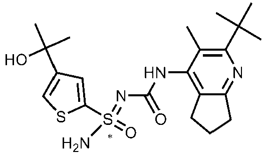 | (R) or (S)-N'-((2-(tert-butyl)-3-methyl-6,7-dihydro-5H-cyclopenta[b]pyridin -4-yl)carbamoyl)-4-(2-hydroxypropan-2-yl)thiophene-2-sulfonimidamide | CHIRALPA K IF, 2*25 cm, 5 um | 30% EtOH in Hex (0.1% FA) | 451 |
| 428 | 657a | 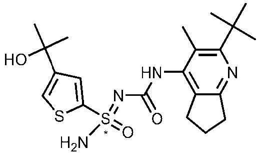 | (S) or (R)-N'-((2-(tert-butyl)-3-methyl-6,7-dihydro-5H-cyclopenta[b]pyridin -4-yl)carbamoyl)-4-(2-hydroxypropan-2-yl)thiophene-2-sulfonimidamide | CHIRALPA K IF, 2*25 cm, 5 um | 30% EtOH in Hex (0.1% FA) | 451 |
| 429 | 670b | 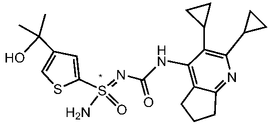 | (R) or (S)-N'-((2,3-dicyclopropyl-6,7-dihydro-5H-cyclopenta[b]pyridin -4-yl)carbamoyl)-4-(2-hydroxypropan-2-yl)thiophene-2-sulfonimidamide | Chiralpak ID, 2*25 cm, 5 um | 30% EtOH in Hex (0.1% FA) | 461 |
| 430 | 670a | 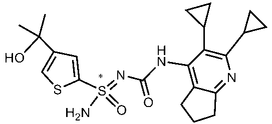 | (S) or (R)-N'-((2,3-dicyclopropyl-6,7-dihydro-5H-cyclopenta[b]pyridin -4-yl)carbamoyl)-4-(2-hydroxypropan-2-yl)thiophene-2-sulfonimidamide | Chiralpak ID, 2*25 cm, 5 um | 30% EtOH in Hex (0.1% FA) | 461 |
| 431 | 612b | 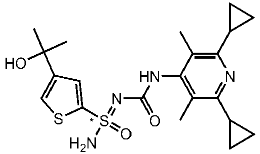 | (R) or (S)-N'-((2,6-dicyclopropyl-3,5-dimethylpyridin-4-yl)carbamoyl)-4-(2-hydroxypropan-2-yl)thiophene-2-sulfonimidamide | Chiralpak ID, 2*25 cm, 5 um | 10% IPA in Hex:DCM (3:1, 10 mM NH₃-MeOH) | 449 |
| 432 | 612a | 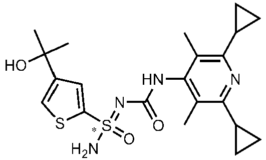 | (S) or (R)-N'-((2,6-dicyclopropyl-3,5-dimethylpyridin-4-yl)carbamoyl)-4-(2-hydroxypropan-2-yl)thiophene-2-sulfonimidamide | Chiralpak ID, 2*25 cm, 5 um | 10% IPA in Hex:DCM (3:1, 10 mM NH₃-MeOH) | 449 |
| 433 | 626b | 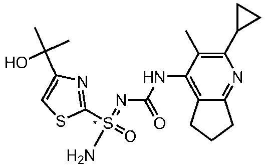 | (R) or (S)-N'-((2-cyclopropyl-3-methyl-6,7-dihydro-5H-cyclopenta[b]pyridin -4-yl)carbamoyl)-4-(2-hydroxypropan-2-yl)thiazole-2-sulfonimidamide | CHIRALPA K IG, 20*250 mm, 5 um | 10% EtOH in MTBE (0.1% FA) | 436 |
| 434 | 626a | 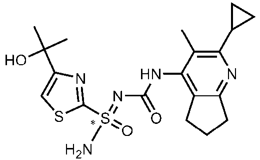 | (S) or (R)-N'-((2-cyclopropyl-3-methyl-6,7-dihydro-5H-cyclopenta[b]pyridin -4-yl)carbamoyl)-4-(2-hydroxypropan-2-yl)thiazole-2-sulfonimidamide | CHIRALPA K IG, 20*250 mm, 5 um | 10% EtOH in MTBE (0.1% FA) | 436 |
| 435 | 665b | 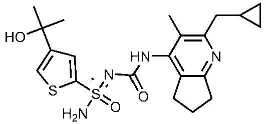 | (R) or (S)-N'-((2-(cyclopropylmethyl)-3-methyl-6,7-dihydro-5H-cyclopenta[b]pyridin -4-yl)carbamoyl)-4-(2-hydroxypropan-2-yl)thiophene-2-sulfonimidamide | CHIRALPA K IG, 2*25 cm, 5 um | 30% EtOH in Hex (0.1% FA) | 449 |
| 436 | 665a | 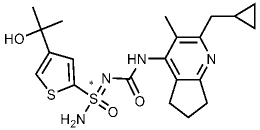 | (S) or (R)-N'-((2-(cyclopropylmethyl)-3-methyl-6,7-dihydro-5H-cyclopenta[b]pyridin -4-yl)carbamoyl)-4-(2-hydroxypropan-2-yl)thiophene-2-sulfonimidamide | CHIRALPA K IG, 2*25 cm, 5 um | 30% EtOH in Hex (0.1% FA) | 449 |
| 437 | 654b | 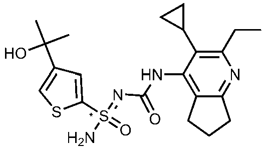 | (R) or (S)-N'-((3-cyclopropyl-2-ethyl-6,7-dihydro-5H-cyclopenta[b]pyridin -4-yl)carbamoyl)-4-(2-hydroxypropan-2-yl)thiophene-2-sulfonimidamide | CHIRALPA K IG, 2*25 cm, 5 um | 30% EtOH in Hex (0.1% FA) | 449 |
| 438 | 654a | 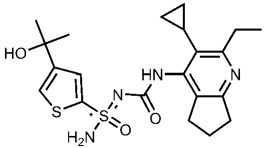 | (S) or (R)-N'-((3-cyclopropyl-2-ethyl-6,7-dihydro-5H-cyclopenta[b]pyridin -4-yl)carbamoyl)-4-(2-hydroxypropan-2-yl)thiophene-2-sulfonimidamide | CHIRALPA K IG, 2*25 cm, 5 um | 30% EtOH in Hex (0.1% FA) | 449 |
| 439 | 634b | 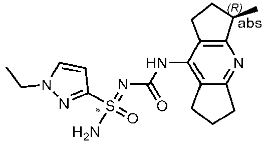 | (R) or (S)-1-Ethyl-N'-(((R)-3-methyl-1,2,3,5,6,7-hexahydrodicyclope nta[b,e]pyridin-8-yl)carbamoyl)-1H-pyrazole-3-sulfonimidamide | CHIRALPA K IE, 2*25 cm, 5 um | 50% EtOH in Hex (8 mM NH₃·MeOH) | 389 |
| 440 | 634a | 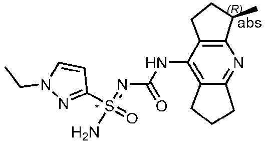 | (S) or (R)-1-Ethyl-N'-(((R)-3-methyl-1,2,3,5,6,7-hexahydrodicyclope nta[b,e]pyridin-8-yl)carbamoyl)-1H-pyrazole-3-sulfonimidamide | CHIRALPA K IE, 2*25 cm, 5 um | 50% EtOH in Hex (8 mM NH₃·MeOH) | 389 |
| 441 | 639b | 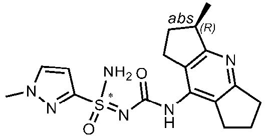 | (R) or (S)-1-Methyl-N'-(((R)-3-methyl-1,2,3,5,6,7-hexahydrodicyclope nta[b,e]pyridin-8-yl)carbamoyl)-1H-pyrazole-3-sulfonimidamide | CHIRALPA K IE, 2*25 cm, 5 um | 50% EtOH in Hex (8 mM NH₃·MeOH) | 375 |
| 442 | 639a | 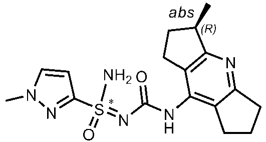 | (S) or (R)-1-Methyl-N'-(((R)-3-methyl-1,2,3,5,6,7-hexahydrodicyclope nta[b,e]pyridin-8-yl)carbamoyl)-1H-pyrazole-3-sulfonimidamide | CHIRALPA K IE, 2*25 cm, 5 um | 50% EtOH in Hex (8 mM NH₃·MeOH) | 375 |
| 443 | 700b | 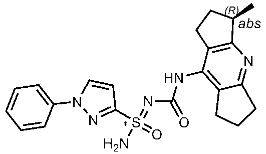 | (R) or (S)-N'-(((R)-3-methyl-1,2,3,5,6,7-hexahydrodicyclope nta[b,e]pyridin-8-yl)carbamoyl)-1-phenyl-1H-pyrazole-3-sulfonimidamide | CHIRALPA K IE, 2*25 cm, 5 um | 20% EtOH in MTBE (10 mM NH₃-MeOH) | 437 |
| 444 | 700a | 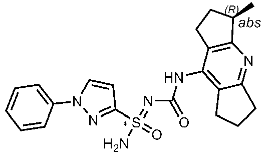 | (S) or (R)-N'-(((R)-3-methyl-1,2,3,5,6,7-hexahydrodicyclope nta[b,e]pyridin-8-yl)carbamoyl)-1-phenyl-1H-pyrazole-3-sulfonimidamide | CHIRALPA K IE, 2*25 cm, 5 um | 20% EtOH in MTBE (10 mM NH₃-MeOH) | 437 |
| 445 | 638b | 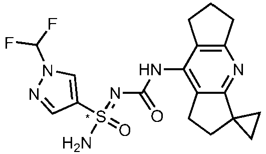 | (S) or (R)-1-(Difluoromethyl)-N'-((1',5',6',7'-tetrahydro-2'H-spiro[cyclopropane-1,3'-dicyclopenta[b,e]pyr idin]-8'-yl)carbamoyl)-1H-pyrazole-4-sulfonimidamide | CHIRALPA K IG, 5*25 cm, 5 um | 5% IPA in MTBE (10 mM NH₃-MeOH) | 423 |
| 446 | 638a | 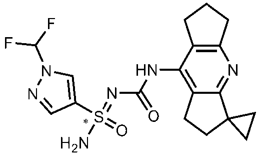 | (R) or (S)-1-(Difluoromethyl)-N'-((1',5',6',7'-tetrahydro-2'H-spiro[cyclopropane-1,3'-dicyclopenta[b,e]pyr idin]-8'-yl)carbamoyl)-1H-pyrazole-4-sulfonimidamide | CHIRALPA K IG, 5*25 cm, 5 um | 5% IPA in MTBE (10 mM NH₃-MeOH) | 423 |
| 447 | 637b | 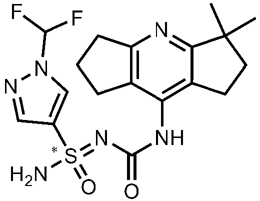 | (S) or (R)-1-(Difluoromethyl)-N'-((3,3-dimethyl-1,2,3,5,6,7-hexahydrodicyclope nta[b,e]pyridin-8-yl)carbamoyl)-1H-pyrazole-4-sulfonimidamide | CHIRALPA K IG, 5*25 cm, 5 um | 3% IPA in MTBE (10 mM NH₃-MeOH) | 425 |
| 448 | 637a | 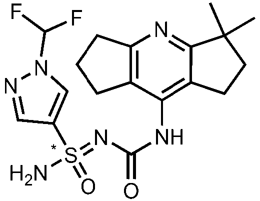 | (R) or (S)-1-(Difluoromethyl)-N'-((3,3-dimethyl-1,2,3,5,6,7-hexahydrodicyclope nta[b,e]pyridin-8-yl)carbamoyl)-1H-pyrazole-4-sulfonimidamide | CHIRALPA K IG, 5*25 cm, 5 um | 3% IPA in MTBE (10 mM NH₃-MeOH) | 425 |
| 449 | 645b | 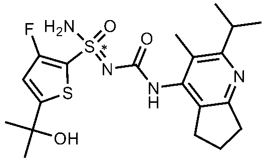 | (S) or (R)-3-Fluoro-5-(2-hydroxypropan-2-yl)-N'-((2-isopropyl-3-methyl-6,7-dihydro-5H-cyclopenta[b]pyridin -4-yl)carbamoyl)thioph ene-2-sulfonimidamide | CHIRAL ART Cellulose-SB, 2*25 cm, 5 um | 30% EtOH in Hex (8 mM NH₃·MeOH) | 455 |
| 450 | 645a | 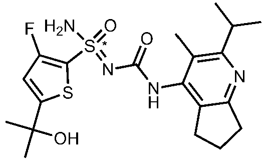 | (R) or (S)-3-Fluoro-5-(2-hydroxypropan-2-yl)-N'-((2-isopropyl-3-methyl-6,7-dihydro-5H-cyclopenta[b]pyridin -4-yl)carbamoyl)thioph ene-2-sulfonimidamide | CHIRAL ART Cellulose-SB, 2*25 cm, 5 um | 30% EtOH in Hex (8 mM NH₃·MeOH) | 455 |
| 451 | 644b | 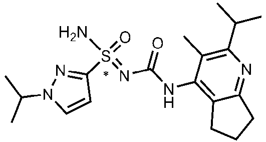 | (R) or (S)-1-Isopropyl-N'-((2-isopropyl-3-methyl-6,7-dihydro-5H-cyclopenta[b]pyridin -4-yl)carbamoyl)-1H-pyrazole-3-sulfonimidamide | CHIRALPA K IG, 2*25 cm, 5 um | 20% EtOH in Hex (8 mM NH₃·MeOH) | 405 |
| 452 | 644a | 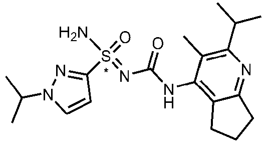 | (S) or (R)-1-Isopropyl-N'-((2-isopropyl-3-methyl-6,7-dihydro-5H-cyclopenta[b]pyridin -4-yl)carbamoyl)-1H-pyrazole-3-sulfonimidamide | CHIRALPA K IG, 2*25 cm, 5 um | 20% EtOH in Hex (8 mM NH₃·MeOH) | 405 |
| 453 | 633b | 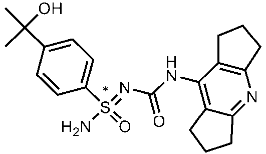 | (R) or (S)-N'-((1,2,3,5,6,7-hexahydrodicyclope nta[b,e]pyridin-8-yl)carbamoyl)-4-(2-hydroxypropan-2-yl)benzenesulfonimi damide | Chiralpak ID, 2*25 cm, 5 um | 50% IPA in Hex (8 mM NH₃·MeOH) | 415 |
| 454 | 633a | 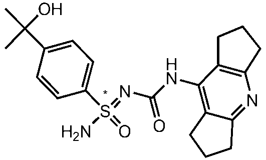 | (S) or (R)-N'-((1,2,3,5,6,7-hexahydrodicyclope nta[b,e]pyridin-8-yl)carbamoyl)-4-(2-hydroxypropan-2-yl)benzenesulfonimi damide | Chiralpak ID, 2*25 cm, 5 um | 50% IPA in Hex (8 mM NH₃·MeOH) | 415 |
| 455 | 625b | 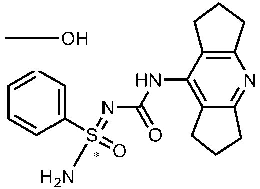 | (R) or (S)-N'-((1,2,3,5,6,7-hexahydrodicyclope nta[b,e]pyridin-8-yl)carbamoyl)-3-(2-hydroxypropan-2-yl)benzenesulfonimi damide | CHIRALPA K IG, 20*250 mm, 5 um | 50% EtOH in Hex (8 mM NH₃·MeOH) | 415 |
| 456 | 625a | 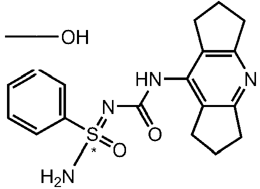 | (S) or (R)-N'-((1,2,3,5,6,7-hexahydrodicyclope nta[b,e]pyridin-8-yl)carbamoyl)-3-(2-hydroxypropan-2-yl)benzenesulfonimi damide | CHIRALPA K IG, 20*250 mm, 5 um | 50% EtOH in Hex (8 mM NH₃·MeOH) | 415 |
| 457 | 632b | 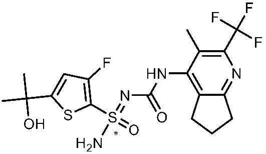 | (S) or (R)-3-Fluoro-5-(2-hydroxypropan-2-yl)-N'-((3-methyl-2-(trifluoromethyl)-6,7-dihydro-5H-cyclopenta[b]pyridin -4-yl)carbamoyl)thioph ene-2-sulfonimidamide | CHIRALPA K ID, 2*25 cm, 5 um | 10% IPA in MTBE (0.1% FA) | 481 |
| 458 | 632a | 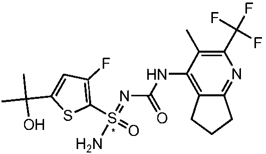 | (R) or (S)-3-Fluoro-5-(2-hydroxypropan-2-yl)-N'-((3-methyl-2-(trifluoromethyl)-6,7-dihydro-5H-cyclopenta[b]pyridin -4-yl)carbamoyl)thioph ene-2-sulfonimidamide | CHIRALPA K ID, 2*25 cm, 5 um | 10% IPA in MTBE (0.1% FA) | 481 |
| 459 | 624b | 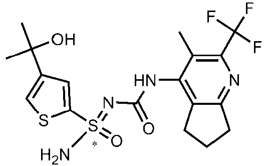 | (R) or (S)-4-(2-Hydroxypropan-2-yl)-N'-((3-methyl-2-(trifluoromethyl)-6,7-dihydro-5H-cyclopenta[b]pyridin -4-yl)carbamoyl)thioph ene-2-sulfonimidamide | CHIRALPA K IG, 2*25 cm, 5 um | 20% EtOH in Hex (0.1% FA) | 463 |
| 460 | 624a | 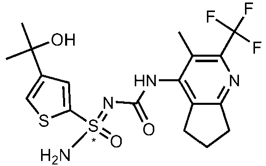 | (S) or (R)-4-(2-Hydroxypropan-2-yl)-N'-((3-methyl-2-(trifluoromethyl)-6,7-dihydro-5H-cyclopenta[b]pyridin -4-yl)carbamoyl)thioph ene-2-sulfonimidamide | CHIRALPA K IG, 2*25 cm, 5 um | 20% EtOH in Hex (0.1% FA) | 463 |
| 461 | 631b | 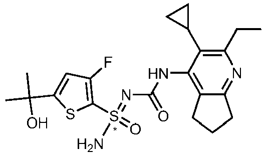 | (R) or (S)-N'-((3-cyclopropyl-2-ethyl-6,7-dihydro-5H-cyclopenta[b]pyridin -4-yl)carbamoyl)-3-fluoro-5-(2-hydroxypropan-2-yl)thiophene-2-sulfonimidamide | CHIRALPA K IC, 2*25 cm, 5 um | 30% EtOH in Hex (0.1% FA) | 467 |
| 462 | 631a | 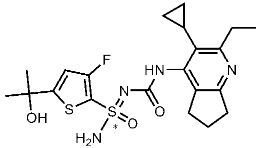 | (S) or (R)-N'-((3-cyclopropyl-2-ethyl-6,7-dihydro-5H-cyclopenta[b]pyridin -4-yl)carbamoyl)-3-fluoro-5-(2-hydroxypropan-2-yl)thiophene-2-sulfonimidamide | CHIRALPA K IC, 2*25 cm, 5 um | 30% EtOH in Hex (0.1% FA) | 467 |
| 463 | 630b | 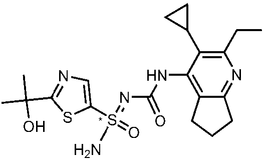 | (R) or (S)-N'-((3-cyclopropyl-2-ethyl-6,7-dihydro-5H-cyclopenta[b]pyridin -4-yl)carbamoyl)-2-(2-hydroxypropan-2-yl)thiazole-5-sulfonimidamide | CHIRALPA K IC, 2*25 cm, 5 um | 30% EtOH in Hex (0.1% FA) | 450 |
| 464 | 630a | 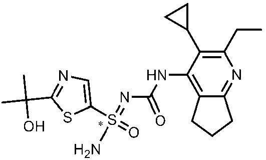 | (S) or (R)-N'-((3-cyclopropyl-2-ethyl-6,7-dihydro-5H-cyclopenta[b]pyridin -4-yl)carbamoyl)-2-(2-hydroxypropan-2-yl)thiazole-5-sulfonimidamide | CHIRALPA K IC, 2*25 cm, 5 um | 30% EtOH in Hex (0.1% FA) | 450 |
| 465 | 623b | 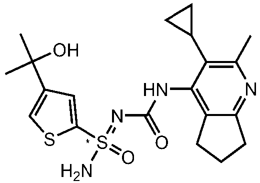 | (S) or (R)-N'-((3-cyclopropyl-2-methyl-6,7-dihydro-5H-cyclopenta[b]pyridin -4-yl)carbamoyl)-4-(2-hydroxypropan-2-yl)thiophene-2-sulfonimidamide | CHIRAL ART Cellulose-SB, 2*25 cm, 5 um | 10% EtOH in MTBE (0.1% FA) | 435 |
| 466 | 623a | 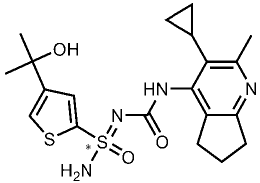 | (R) or (S)-N'-((3-cyclopropyl-2-methyl-6,7-dihydro-5H-cyclopenta[b]pyridin -4-yl)carbamoyl)-4-(2-hydroxypropan-2-yl)thiophene-2-sulfonimidamide | CHIRAL ART Cellulose-SB, 2*25 cm, 5 um | 10% EtOH in MTBE (0.1% FA) | 435 |
| 467 | 622b | 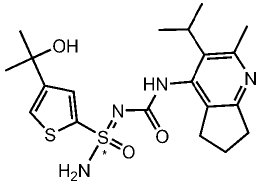 | (R) or (S)-4-(2-Hydroxypropan-2-yl)-N'-((3-isopropyl-2-methyl-6,7-dihydro-5H-cyclopenta[b]pyridin -4-yl)carbamoyl)thioph ene-2-sulfonimidamide | CHIRALPA K IC, 2*25 cm, 5 um | 50% IPA in MTBE (0.1% FA) | 437 |
| 468 | 622a | 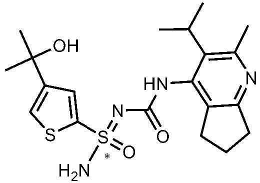 | (S) or (R)-4-(2-Hydroxypropan-2-yl)-N'-((3-isopropyl-2-methyl-6,7-dihydro-5H-cyclopenta[b]pyridin -4-yl)carbamoyl)thioph ene-2-sulfonimidamide | CHIRALPA K IC, 2*25 cm, 5 um | 50% IPA in MTBE (0.1% FA) | 437 |
| 469 | 621b | 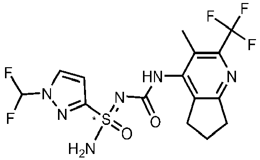 | (R) or (S)-1-(Difluoromethyl)-N'-((3-methyl-2-(trifluoromethyl)-6,7-dihydro-5H-cyclopenta[b]pyridin -4-yl)carbamoyl)-1H-pyrazole-3-sulfonimidamide | CHIRALPA K IG, 2*25 cm, 5 um | 20% MeOH (2 mM NH₃-MeOH) in CO₂ | 439 |
| 470 | 621a | 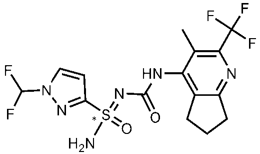 | (S) or (R)-1-(Difluoromethyl)-N'-((3-methyl-2-(trifluoromethyl)-6,7-dihydro-5H-cyclopenta[b]pyridin -4-yl)carbamoyl)-1H-pyrazole-3-sulfonimidamide | CHIRALPA K IG, 2*25 cm, 5 um | 20% MeOH (2 mM NH₃-MeOH) in CO₂ | 439 |
| 471 | 618b | 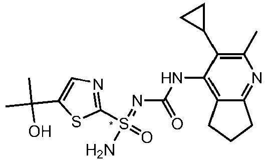 | (R) or (S)-N'-((3-cyclopropyl-2-methyl-6,7-dihydro-5H-cyclopenta[b]pyridin -4-yl)carbamoyl)-5-(2-hydroxypropan-2-yl)thiazole-2-sulfonimidamide | CHIRALPA K IC, 2*25 cm, 5 um | 30% EtOH in Hex (0.1% FA) | 436 |
| 472 | 618a | 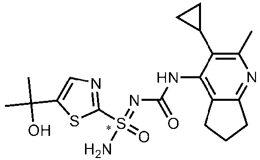 | (S) or (R)-N'-((3-cyclopropyl-2-methyl-6,7-dihydro-5H-cyclopenta[b]pyridin -4-yl)carbamoyl)-5-(2-hydroxypropan-2-yl)thiazole-2-sulfonimidamide | CHIRALPA K IC, 2*25 cm, 5 um | 30% EtOH in Hex (0.1% FA) | 436 |
| 473 | 628b | 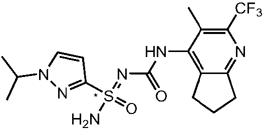 | (R) or (S)-1-Isopropyl-N'-((3-methyl-2-(trifluoromethyl)-6,7-dihydro-5H-cyclopenta[b]pyridin -4-yl)carbamoyl)-1H-pyrazole-3-sulfonimidamide | Chiralpak ID, 2*25 cm, 5 um | 30% EtOH in Hex (0.1% FA) | 431 |
| 474 | 628a | 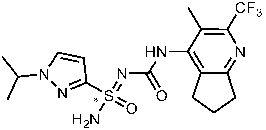 | (S) or (R)-1-Isopropyl-N'-((3-methyl-2-(trifluoromethyl)-6,7-dihydro-5H-cyclopenta[b]pyridin -4-yl)carbamoyl)-1H-pyrazole-3-sulfonimidamide | Chiralpak ID, 2*25 cm, 5 um | 30% EtOH in Hex (0.1% FA) | 431 |
| 475 | 617b | 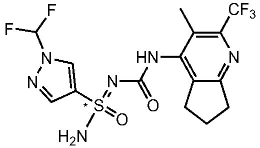 | (S) or (R)-1-(Difluoromethyl)-N'-((3-methyl-2-(trifluoromethyl)-6,7-dihydro-5H-cyclopenta[b]pyridin -4-yl)carbamoyl)-1H-pyrazole-4-sulfonimidamide | CHIRAL ART Cellulose-SB, 2*25 cm, 5 um | 25% EtOH in Hex (0.1% FA) | 439 |
| 476 | 617a | 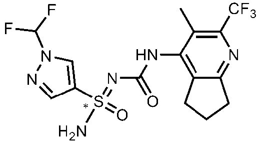 | (R) or (S)-1-(Difluoromethyl)-N'-((3-methyl-2-(trifluoromethyl)-6,7-dihydro-5H-cyclopenta[b]pyridin -4-yl)carbamoyl)-1H-pyrazole-4-sulfonimidamide | CHIRAL ART Cellulose-SB, 2*25 cm, 5 um | 25% EtOH in Hex (0.1% FA) | 439 |
| 477 | 610b | 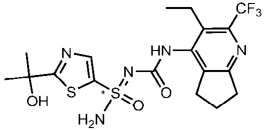 | (S) or (R)-N'-((3-ethyl-2-(trifluoromethyl)-6,7-dihydro-5H-cyclopenta[b]pyridin -4-yl)carbamoyl)-2-(2-hydroxypropan-2-yl)thiazole-5-sulfonimidamide | CHIRALPA K IE, 2*25 cm, 5 um | 15% EtOH in Hex (0.1% FA) | 478 |
| 478 | 610a | 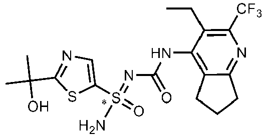 | (R) or (S)-N'-((3-ethyl-2-(trifluoromethyl)-6,7-dihydro-5H-cyclopenta[b]pyridin -4-yl)carbamoyl)-2-(2-hydroxypropan-2-yl)thiazole-5-sulfonimidamide | CHIRALPA K IE, 2*25 cm, 5 um | 15% EtOH in Hex (0.1% FA) | 478 |
| 479 | 611b | 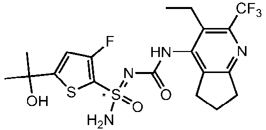 | (R) or (S)-N'-((3-ethyl-2-(trifluoromethyl)-6,7-dihydro-5H-cyclopenta[b]pyridin -4-yl)carbamoyl)-3-fluoro-5-(2-hydroxypropan-2-yl)thiophene-2-sulfonimidamide | CHIRALPA K IF, 4.6*50 mm, 3 um | 20% EtOH in Hex (0.1% FA ) | 495 |
| 480 | 611a | 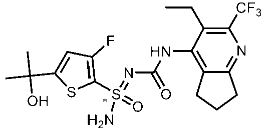 | (S) or (R)-N'-((3-ethyl-2-(trifluoromethyl)-6,7-dihydro-5H-cyclopenta[b]pyridin -4-yl)carbamoyl)-3-fluoro-5-(2-hydroxypropan-2-yl)thiophene-2-sulfonimidamide | CHIRALPA K IF, 4.6*50 mm, 3 um | 20% EtOH in Hex (0.1% FA ) | 495 |
| 481 | 698b | 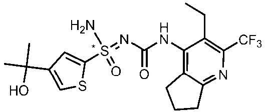 | (R) or (S)-N'-((3-ethyl-2-(trifluoromethyl)-6,7-dihydro-5H-cyclopenta[b]pyridin -4-yl)carbamoyl)-4-(2-hydroxypropan-2-yl)thiophene-2-sulfonimidamide | CHIRALPA K IG, 2.0*25 cm, 5 um | 20% EtOH in Hex (8 mM NH₃·MeOH) | 477 |
| 482 | 698a | 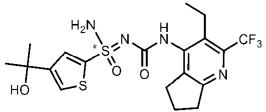 | (S) or (R)-N'-((3-ethyl-2-(trifluoromethyl)-6,7-dihydro-5H-cyclopenta[b]pyridin -4-yl)carbamoyl)-4-(2-hydroxypropan-2-yl)thiophene-2-sulfonimidamide | CHIRALPA K IG, 2.0*25 cm, 5 um | 20% EtOH in Hex (8 mM NH₃·MeOH) | 477 |
| 483 | 616b | 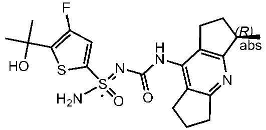 | (S) or (R)-4-Fluoro-5-(2-hydroxypropan-2-yl)-N'-(((R)-3-methyl-1,2,3,5,6,7-hexahydrodicyclope nta[b,e]pyridin-8-yl)carbamoyl)thioph ene-2-sulfonimidamide | CHIRAL ART Cellulose-SB, 2*25 cm, 5 um | 30% EtOH in Hex (8 mM NH₃·MeOH) | 453 |
| 484 | 616a | 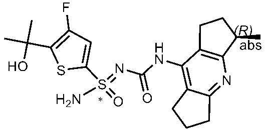 | (R) or (S)-4-Fluoro-5-(2-hydroxypropan-2-yl)-N'-(((R)-3-methyl-1,2,3,5,6,7-hexahydrodicyclope nta[b,e]pyridin-8-yl)carbamoyl)thioph ene-2-sulfonimidamide | CHIRAL ART Cellulose-SB, 2*25 cm, 5 um | 30% EtOH in Hex (8 mM NH₃·MeOH) | 453 |
| 485 | 614b | 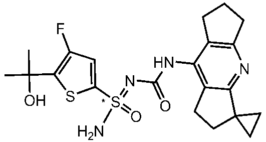 | (S) or (R)-4-Fluoro-5-(2-hydroxypropan-2-yl)-N'-((1',5',6',7'-tetrahydro-2'H-spiro[cyclopropane-1,3'-dicyclopenta[b,e]pyr idin]-8'-yl)carbamoyl)thioph ene-2-sulfonimidamide | CHIRAL ART Cellulose-SB, 2*25 cm, 5 um | 30% EtOH in Hex (8 mM NH₃·MeOH) | 465 |
| 486 | 614a | 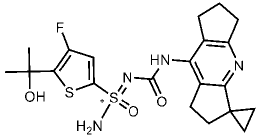 | (R) or (S)-4-Fluoro-5-(2-hydroxypropan-2-yl)-N'-((1',5',6',7'-tetrahydro-2'H-spiro[cyclopropane-1,3'-dicyclopenta[b,e]pyr idin]-8'-yl)carbamoyl)thioph ene-2-sulfonimidamide | CHIRAL ART Cellulose-SB, 2*25 cm, 5 um | 30% EtOH in Hex (8 mM NH₃·MeOH) | 465 |
| 487 | 613b | 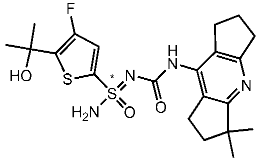 | -N'-((3,3-dimethyl-1,2,3,5,6,7-hexahydrodicyclope nta[b,e]pyridin-8-yl)carbamoyl)-4-fluoro-5-(2-hydroxypropan-2-yl)thiophene-2-sulfonimidamide | CHIRAL ART Cellulose-SB, 2*25 cm, 5 um | 40% EtOH in Hex (0.1% FA) | 467 |
| 488 | 613a | 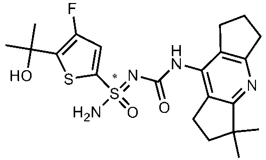 | (R) or (S)-N'-((3,3-dimethyl-1,2,3,5,6,7-hexahydrodicyclope nta[b,e]pyridin-8-yl)carbamoyl)-4-fluoro-5-(2-hydroxypropan-2-yl)thiophene-2-sulfonimidamide | CHIRAL ART Cellulose-SB, 2*25 cm, 5 um | 40% EtOH in Hex (0.1% FA) | 467 |
| 489 | 697b | 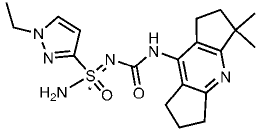 | (R) or (S)-N'-((3,3-dimethyl-1,2,3,5,6,7-hexahydrodicyclope nta[b,e]pyridin-8-yl)carbamoyl)-1-ethyl-1H-pyrazole-3-sulfonimidamide | **HIRALPAK** ID, 2*25 cm, 5 um | 30% EtOH in Hex (0.1% FA) | 403 |
| 490 | 697a | 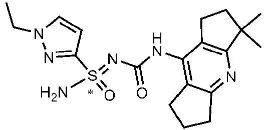 | (S) or (R)-N'-((3,3-dimethyl-1,2,3,5,6,7-hexahydrodicyclope nta[b,e]pyridin-8-yl)carbamoyl)-1-ethyl-1H-pyrazole-3-sulfonimidamide | HIRALPAK ID, 2*25 cm, 5 um | 30% EtOH in Hex (0.1% FA) | 403 |
| 491 | 607b | 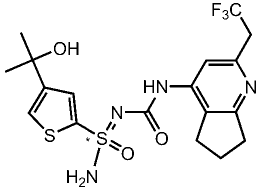 | (R) or (S)-4-(2-Hydroxypropan-2-yl)-N'-((2-(2,2,2-trifluoroethyl)-6,7-dihydro-5H-cyclopenta[b]pyridin -4-yl)carbamoyl)thioph ene-2-sulfonimidamide | CHIRALPA K IC, 2*25 cm, 5 um | 30% IPA in Hex (0.1% FA) | 463 |
| 492 | 607a | 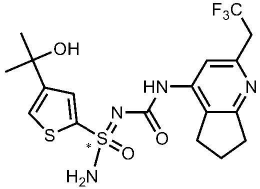 | (S) or (R)-4-(2-Hydroxypropan-2-yl)-N'-((2-(2,2,2-trifluoroethyl)-6,7-dihydro-5H-cyclopenta[b]pyridin -4-yl)carbamoyl)thioph ene-2-sulfonimidamide | CHIRALPA K IC, 2*25 cm, 5 um | 30% IPA in Hex (0.1% FA) | 463 |
| 493 | 636b | 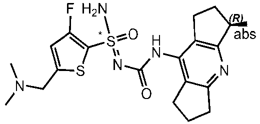 | (R) or (S)-5-((Dimethylamino)me thyl)-3-fluoro-N'-(((R)-3-methyl-1,2,3,5,6,7-hexahydrodicyclope nta[b,e]pyridin-8-yl)carbamoyl)thioph ene-2-sulfonimidamide | CHIRALPA K IG, 2.0*25 cm, 5 um | 15% EtOH in Hex:DCM (3:1, 10 mM NH₃-MeOH) | 452 |
| 494 | 636a | 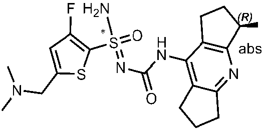 | (S) or (R)-5-((Dimethylamino)me thyl)-3-fluoro-N'-(((R)-3-methyl-1,2,3,5,6,7-hexahydrodicyclope nta[b,e]pyridin-8-yl)carbamoyl)thioph ene-2-sulfonimidamide | CHIRALPA K IG, 2.0*25 cm, 5 um | 15% EtOH in Hex:DCM (3:1, 10 mM NH₃-MeOH) | 452 |
| 495 | 304b | 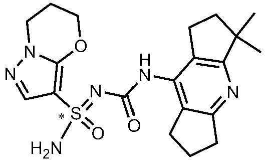 | (R) or (S)-N'-((3,3-dimethyl-1,2,3,5,6,7-hexahydrodicyclope nta[b,e]pyridin-8-yl)carbamoyl)-6,7-dihydro-5H-pyrazolo[5,1-b][1,3]oxazine-3-sulfonimidamide | CHIRALPA K ID, 2*25 cm, 5 um | 10% IPA in MTBE (0.1% FA) | 431 |
| 496 | 304a | 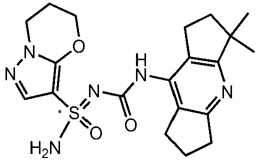 | (S) or (R)-N'-((3,3-dimethyl-1,2,3,5,6,7-hexahydrodicyclope nta[b,e]pyridin-8-yl)carbamoyl)-6,7-dihydro-5H-pyrazolo[5,1-b][1,3]oxazine-3-sulfonimidamide | CHIRALPA K ID, 2*25 cm, 5 um | 10% IPA in MTBE (0.1% FA) | 431 |
| 497 | 306b | 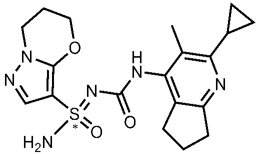 | (R) or (S)-N'-((2-cyclopropyl-3-methyl-6,7-dihydro-5H-cyclopenta[b]pyridin -4-yl)carbamoyl)-6,7-dihydro-5H-pyrazolo[5,1-b][1,3]oxazine-3-sulfonimidamide | CHIRALPA K IC, 2*25 cm, 5 um | 10% MeOH in MTBE (10 mM NH₃-MeOH) | 417 |
| 498 | 306a | 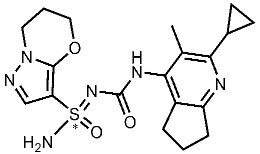 | (S) or (R)-N'-((2-cyclopropyl-3-methyl-6,7-dihydro-5H-cyclopenta[b]pyridin -4-yl)carbamoyl)-6,7-dihydro-5H-pyrazolo[5,1-b][1,3]oxazine-3-sulfonimidamide | CHIRALPA K IC, 2*25 cm, 5 um | 10% MeOH in MTBE (10 mM NH₃-MeOH) | 417 |
| 499 | 605e | 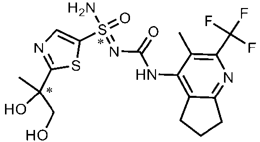 | (R, S) or (S, S)-2-(1,2-Dihydroxypropan-2-yl)-N'-((3-methyl-2-(trifluoromethyl)-6,7-dihydro-5H-cyclopenta[b]pyridin -4-yl)carbamoyl)thiazol e-5-sulfonimidamide (From Ex. 241) | CHIRAL ART Cellulose-SB, 2*25 cm, 5 um | 30% EtOH in Hex (0.1% FA) | 480 |
| 500 | 605c | 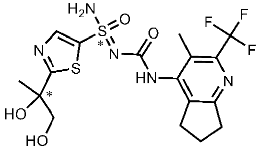 | (R, R) or (S, R)-2-(1,2-Dihydroxypropan-2-yl)-N'-((3-methyl-2-(trifluoromethyl)-6,7-dihydro-5H-cyclopenta[b]pyridin -4-yl)carbamoyl)thiazol e-5-sulfonimidamide (From Ex. 241) | CHIRAL ART Cellulose-SB, 2*25 cm, 5 um | 30% EtOH in Hex (0.1% FA) | 480 |
| 501 | 605g | 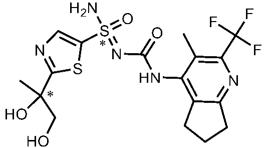 | (S, S) or (R, S)-2-(1,2-Dihydroxypropan-2-yl)-N'-((3-methyl-2-(trifluoromethyl)-6,7-dihydro-5H-cyclopenta[b]pyridin -4-yl)carbamoyl)thiazol e-5-sulfonimidamide (from Ex. 242) | CHIRAL ART Cellulose-SB, 2*25 cm, 5 um | 30% EtOH in Hex (0.1% FA) | 480 |
| 502 | 605h | 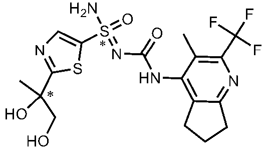 | (S, R) or (R, R)-2-(1,2-Dihydroxypropan-2-yl)-N'-((3-methyl-2-(trifluoromethyl)-6,7-dihydro-5H-cyclopenta[b]pyridin -4-yl)carbamoyl)thiazol e-5-sulfonimidamide (from Ex. 242) | CHIRAL ART Cellulose-SB, 2*25 cm, 5 um | 30% EtOH in Hex (0.1% FA) | 480 |

**Table 39. Examples in the following table were obtained from chiral HPLC resolutions of racemic examples described above. The chiral column and eluents are listed in the table. As a convention, the fastest-eluting diastereomer or isomer is always listed first in the table followed by the second-fastest-eluting enantiomer of the mixture, and so on. The symbol * at a chiral center denotes that this chiral center has been resolved and the absolute stereochemistry at that center has not been determined. Assigned stereochemistry in compound names are tentative.**

| Ex. # | Final Target Number | Structure | IUPAC Name | Column | Eluent s | LC-MS [M+H]⁺ |
|---|---|---|---|---|---|---|
| 503 | 660d | 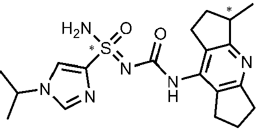 | (R, R) or (S, R) or (R, S) or (S, S)-1-Isopropyl-N'-((3-methyl-1,2,3,5,6,7-hexahydrodicycl openta[b,e]pyridi n-8-yl)carbamoyl)-1H-imidazole-4-sulfonimidamide | CHIRALP AK IA, 5*25 cm, 5 um | 50% EtOH: CAN (2:1, 2 mM NH₃-MeOH ) in CO₂ | 403 |
| 504 | 660c | 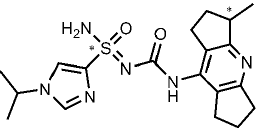 | (S, R) or (R, S) or (S, S) or (R, R)-1-Isopropyl-N'-((3-methyl-1,2,3,5,6,7-hexahydrodicycl openta[b,e]pyridi n-8-yl)carbamoyl)-1H-imidazole-4-sulfonimidamide | CHIRALP AK IA, 5*25 cm, 5 um | 50% EtOH: CAN (2:1, 2 mM NH₃-MeOH ) in CO₂ | 403 |
| 505 | 660b | 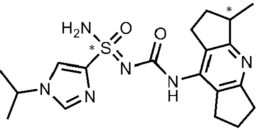 | (S, S) or (R, S) or (S, R) or (R, R)-1-Iisopropyl-N'-((3-methyl-1,2,3,5,6,7-hexahydrodicycl openta[b,e]pyridi n-8-yl)carbamoyl)-1H-imidazole-4-sulfonimidamide | CHIRALP AK IA, 5*25 cm, 5 um | 50% EtOH: CAN (2:1, 2 mM NH₃-MeOH ) in CO₂ | 403 |
| 506 | 660a | 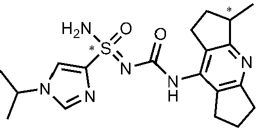 | (R, S) or (S, S) or (S, R) or (R, R)-1-Iisopropyl-N'-((3-methyl-1,2,3,5,6,7-hexahydrodicycl openta[b,e]pyridi n-8-yl)carbamoyl)-1H-imidazole-4-sulfonimidamide | CHIRALP AK IA, 5*25 cm, 5 um | 50% EtOH: CAN (2:1, 2 mM NH₃-MeOH ) in CO₂ | 403 |
| 507 | 201f | 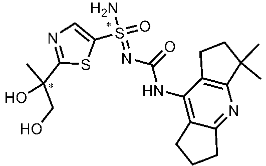 two diastereomers, most likely with the same configuration at sulfur | (R, S) and (S, S) or (S, R) and (R, R)-2-(1,2-Dihydroxypropan -2-yl)-N'-((3,3-dimethyl-1,2,3,5,6,7-hexahydrodicycl openta[b,e]pyridi n-8-yl)carbamoyl)thi azole-5-sulfonimidamide (mixture of two isomers) | CHIRAL ART Cellulose-SB, 2*25 cm, 5 um | 40% EtOH (2 mM NH₃-MeOH ) in CO₂ | 466 |
| 508 | 201e | 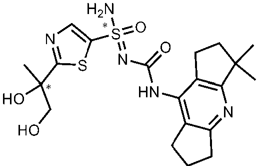 two diastereomers, most likely with the same configuration at sulfur | (R, R) and (S, R) or (R, S) and (S, S)-2-(1,2-Dihydroxypropan -2-yl)-N'-((3,3-dimethyl-1,2,3,5,6,7-hexahydrodicycl openta[b,e]pyridi n-8-yl)carbamoyl)thi azole-5-sulfonimidamide (mixture of two isomers) | CHIRAL ART Cellulose-SB, 2*25 cm, 5 um | 40% **EtOH** (2 mM NH₃-MeOH ) in CO₂ | 466 |
| 509 | 201d | 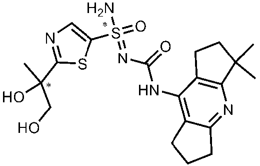 | (R, S) or (S, S) or (S, R) or (R, R)-2-(1,2-Dihydroxypropan -2-yl)-N'-((3,3-dimethyl-1,2,3,5,6,7-hexahydrodicycl openta[b,e]pyridi n-8-yl)carbamoyl)thi azole-5-sulfonimidamide (From Ex. 507) | CHIRALP AK IF, 2*25 cm, 5 um | 30% EtOH in Hex (0.1% FA) | 466 |
| 510 | 201c | 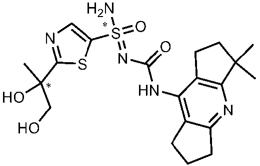 | (S, S) or (R, S) or (S, R) or (R, R)-2-(1,2-Dihydroxypropan -2-yl)-N'-((3,3-dimethyl-1,2,3,5,6,7-hexahydrodicycl openta[b,e]pyridi n-8-yl)carbamoyl)thi azole-5-sulfonimidamide (From Ex. 507) | CHIRALP AK IF, 2*25 cm, 5 um | 30% EtOH in Hex (0.1% FA) | 466 |
| 511 | 201a | 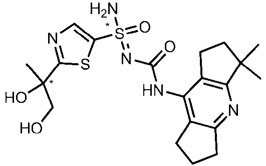 | (R, R) or (S, R) or (R, S) or (S, S)-2-(1,2-Dihydroxypropan -2-yl)-N'-((3,3-dimethyl-1,2,3,5,6,7-hexahydrodicycl openta[b,e]pyridi n-8-yl)carbamoyl)thi azole-5-sulfonimidamide (From Ex. 508) | CHIRAL ART Cellulose-SB, 2*25 cm, 5 um | 30% EtOH in Hex (0.1% FA) | 466 |
| 512 | 201b | 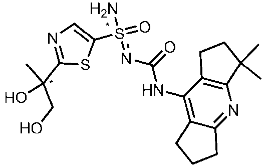 | (S, R) or (R, R) or (R, S) or (S, S)-2-(1,2-Dihydroxypropan -2-yl)-N'-((3,3-dimethyl-1,2,3,5,6,7-hexahydrodicycl openta[b,e]pyridi n-8-yl)carbamoyl)thi azole-5-sulfonimidamide (From Ex. 508) | CHIRAL ART Cellulose-SB, 2*25 cm, 5 um | 30% EtOH in Hex (0.1% FA) | 466 |
| 513 | 662c | 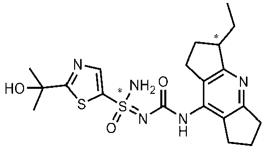 | (S, S) and (S, R) or (R, S) and (R, R)-N'-((3-Ethyl-1,2,3,5,6,7-hexahydrodicycl openta[b,e]pyridi n-8-yl)carbamoyl)-2-(2-hydroxypropan-2-yl)thiazole-5-sulfonimidamide (mixture of two isomers) | CHIRAL ART Cellulose-SB, 2*25 cm, 5 um | 30% EtOH in Hex (0.1% FA) | 450 |
| 514 | 662d | 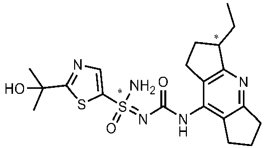 | (R, R) or (R, S) or (S, R) or (S, S)-N'-((3-Ethyl-1,2,3,5,6,7-hexahydrodicycl openta[b,e]pyridi n-8-yl)carbamoyl)-2-(2-hydroxypropan-2-yl)thiazole-5-sulfonimidamide | CHIRAL ART Cellulose-SB, 2*25 cm, 5 um | 30% EtOH in Hex (0.1% FA) | 450 |
| 515 | 662d | 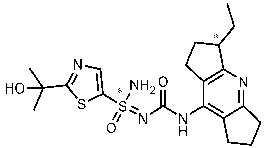 | (R, S) or (R, R) or (S, R) or (S, S)-N'-((3-Ethyl-1,2,3,5,6,7-hexahydrodicycl openta[b,e]pyridi n-8-yl)carbamoyl)-2-(2-hydroxypropan-2-yl)thiazole-5-sulfonimidamide | CHIRAL ART Cellulose-SB, 2*25 cm, 5 um | 30% EtOH in Hex (0.1% FA) | 450 |
| 516 | 662b | 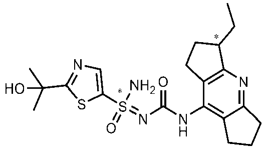 | (S, R) or (S, S) or (R, S) or (R, R)-N'-((3-Ethyl-1,2,3,5,6,7-hexahydrodicycl openta[b,e]pyridi n-8-yl)carbamoyl)-2-(2-hydroxypropan-2-yl)thiazole-5-sulfonimidamide (From Ex. 513) | CHIRALP AK IG, 2.0*25 cm, 5 um | 10%Et OH in MTBE (10 mM NH₃-MeOH ) | 450 |
| 517 | 662a | 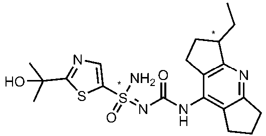 | (S, S) or (S, R) or (R, S) or (R, R)-N'-((3-Ethyl-1,2,3,5,6,7-hexahydrodicycl openta[b,e]pyridi n-8-yl)carbamoyl)-2-(2-hydroxypropan-2-yl)thiazole-5-sulfonimidamide (From Ex. 513) | CHIRALP AK IG, 2.0*25 cm, 5 um | 10%Et OH in MTBE (10 mM NH₃-MeOH ) | 450 |
| 518 | 699c | 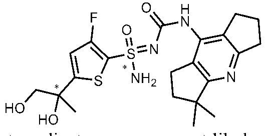 two diastereomers, most likely with the same configuration at sulfur | (R, S) and (S, S) or (S, R) and (R, R)-5-(1,2-Dihydroxypropan -2-yl)-N'-((3,3-dimethyl-1,2,3,5,6,7-hexahydrodicycl openta[b,e]pyridi n-8-yl)carbamoyl)-3-fluorothiophene-2-sulfonimidamide (mixture of two isomers) | CHIRAL ART Cellulose-SB, 2*25 cm, 5 um | 30% EtOH in Hex (0.1% FA) | 483 |
| 519 | 699b | 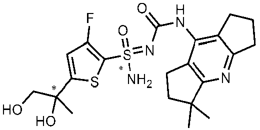 two diastereomers, most likely with the same configuration at sulfur | (S, R) and (R, R) or (R, S) and (S, S)-5-(1,2-Dihydroxypropan -2-yl)-N'-((3,3-dimethyl-1,2,3,5,6,7-hexahydrodicycl openta[b,e]pyridi n-8-yl)carbamoyl)-3-fluorothiophene-2-sulfonimidamide (mixture of two isomers) | CHIRAL ART Cellulose-SB, 2*25 cm, 5 um | 30% EtOH in Hex (0.1% FA) | 483 |
| 520 | 699a | 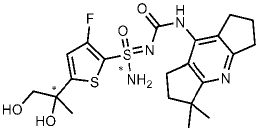 | (S, S) or (R, S) or (S, R) or (R, R)-5-(1,2-Dihydroxypropan -2-yl)-N'-((3,3-dimethyl-1,2,3,5,6,7-hexahydrodicycl openta[b,e]pyridi n-8-yl)carbamoyl)-3-fluorothiophene-2-sulfonimidamide (From Ex. 518) | CHIRALP AK IG, 20*250 mm, 5 um | 35% MeOH :ACN (2:8, 0.1% NH₃·H ₂O) in CO₂ | 483 |
| 521 | 640c | 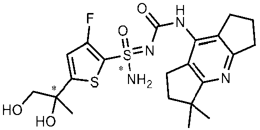 | (R, S) or (S, S) or (S, R) or (R, R)-5-(1,2-Dihydroxypropan -2-yl)-N'-((3,3-dimethyl-1,2,3,5,6,7-hexahydrodicycl openta[b,e]pyridi n-8-yl)carbamoyl)-3-fluorothiophene-2-sulfonimidamide (From Ex. 518) | CHIRALP AK IG, 20*250 mm, 5 um | 35% MeOH :ACN (2:8, 0.1% NH₃·H ₂O) in CO₂ | 483 |
| 522 | 640b | 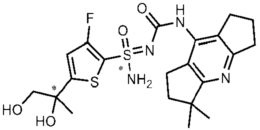 | (S, R) or (R, R) or (R, S) or (S, S)-5-(1,2-Dihydroxypropan -2-yl)-N'-((3,3-dimethyl-1,2,3,5,6,7-hexahydrodicycl openta[b,e]pyridi n-8-yl)carbamoyl)-3-fluorothiophene-2-sulfonimidamide (From Ex. 519) | CHIRALP AK AD, 5*25 cm, 5 um | 40% MeOH :ACN (2:8, 0.1% NH₃·H ₂O) in CO₂ | 483 |
| 523 | 640a | 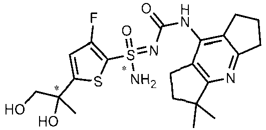 | (R, R) or (S, R) or (R, S) or (S, S)-5-(1,2-Dihydroxypropan -2-yl)-N'-((3,3-dimethyl-1,2,3,5,6,7-hexahydrodicycl openta[b,e]pyridi n-8-yl)carbamoyl)-3-fluorothiophene-2-sulfonimidamide (From Ex. 519) | CHIRALP AK AD, 5*25 cm, 5 um | 40% MeOH :ACN (2:8, 0.1% NH₃·H ₂O) in CO₂ | 483 |
| 524 | 656d | 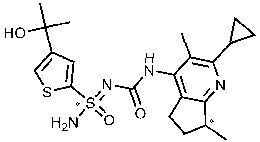 | (R, S) or (R, R) or (S, R) or (S, S)-N'-((2-Cyclopropyl-3,7-dimethyl-6,7-dihydro-5H-cyclopenta[b]pyri din-4-yl)carbamoyl)-4-(2-hydroxypropan-2-yl)thiophene-2-sulfonimidamide | CHIRAL ART Cellulose-SB, 2*25 cm, 5 um | 20% EtOH in Hex (0.1% FA) | 449 |
| 525 | 656b | 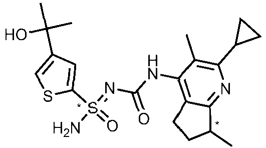 | (S, S) or (S, R) or (R, S) or (R, R)-N'-((2-Cyclopropyl-3,7-dimethyl-6,7-dihydro-5H-cyclopenta[b]pyri din-4-yl)carbamoyl)-4-(2-hydroxypropan-2-yl)thiophene-2-sulfonimidamide | CHIRAL ART Cellulose-SB, 2*25 cm, 5 um | 20% EtOH in Hex (0.1% FA) | 449 |
| 526 | 656a | 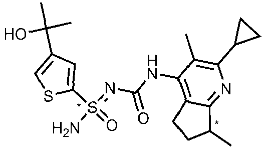 | (R, R) or (R, S) or (S, R) or (S, S)-N'-((2-Cyclopropyl-3,7-dimethyl-6,7-dihydro-5H-cyclopenta[b]pyri din-4-yl)carbamoyl)-4-(2-hydroxypropan-2-yl)thiophene-2-sulfonimidamide | CHIRAL ART Cellulose-SB, 2*25 cm, 5 um | 20% EtOH in Hex (0.1% FA) | 449 |
| 527 | 656c | 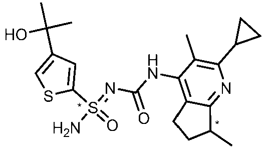 | (S, R) or (S, S) or (R, S) or (R, R)-N'-((2-Cyclopropyl-3,7-dimethyl-6,7-dihydro-5H-cyclopenta[b]pyri din-4-yl)carbamoyl)-4-(2-hydroxypropan-2-yl)thiophene-2-sulfonimidamide | CHIRAL ART Cellulose-SB, 2*25 cm, 5 um | 20% EtOH in Hex (0.1% FA) | 449 |
| 528 | 668c | 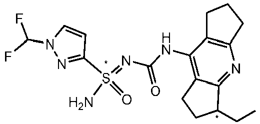 | (R, R) or (R, S) or (S, R) or (S, S)-1-(Difluoromethyl) -N'-((3-ethyl-1,2,3,5,6,7-hexahydrodicycl openta[b,e]pyridi n-8-yl)carbamoyl)-1H-pyrazole-3-sulfonimidamide | CHIRALP AK IG, 2.0*25 cm, 5 um | 30% EtOH in Hex (0.1% FA) | 425 |
| 529 | 668b | 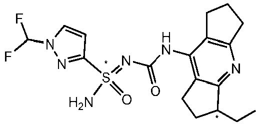 | (R, S) or (R, R) or (S, R) or (S, S)-1-(Difluoromethyl) -N'-((3-ethyl-1,2,3,5,6,7-hexahydrodicycl openta[b,e]pyridi n-8-yl)carbamoyl)-1H-pyrazole-3-sulfonimidamide | CHIRALP AK IG, 2.0*25 cm, 5 um | 30% EtOH in Hex (0.1% FA) | 425 |
| 530 | 668a | 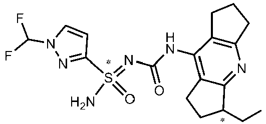 | (S, S) or (S, R) or (R, S) or (R, R)-1-(Difluoromethyl) -N'-((3-ethyl-1,2,3,5,6,7-hexahydrodicycl openta[b,e]pyridi n-8-yl)carbamoyl)-1H-pyrazole-3-sulfonimidamide | CHIRALP AK IG, 2.0*25 cm, 5 um | 30% EtOH in Hex (0.1% FA) | 425 |
| 531 | 668d | 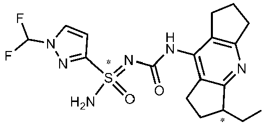 | (S, R) or (S, S) or (R, S) or (R, R)-1-(Difluoromethyl) -N'-((3-ethyl-1,2,3,5,6,7-hexahydrodicycl openta[b,e]pyridi n-8-yl)carbamoyl)-1H-pyrazole-3-sulfonimidamide | CHIRALP AK IG, 2.0*25 cm, 5 uM | 30% EtOH in Hex (0.1% FA) | 425 |
| 532 | 671d | 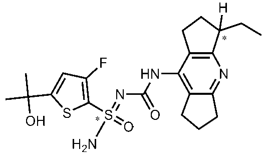 | (R, R) or (R, S) or (S, R) or (S, S)-N'-((3-ethyl-1,2,3,5,6,7-hexahydrodicycl openta[b,e]pyridi n-8-yl)carbamoyl)-3-fluoro-5-(2-hydroxypropan-2-yl)thiophene-2-sulfonimidamide | CHIRALP AK IG, 2.0*25 cm, 5 uM | 30% EtOH in Hex (8 mM NH₃·M eOH) | 467 |
| 533 | 671c | 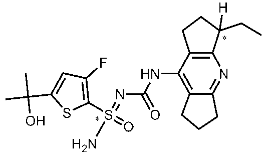 | (R, S) or (R, R) or (S, R) or (S, S)-N'-((3-ethyl-1,2,3,5,6,7-hexahydrodicycl openta[b,e]pyridi n-8-yl)carbamoyl)-3-fluoro-5-(2-hydroxypropan-2-yl)thiophene-2-sulfonimidamide | CHIRALP AK IG, 2.0*25 cm, 5 uM | 30% EtOH in Hex (8 mM NH₃·M eOH) | 467 |
| 534 | 671b | 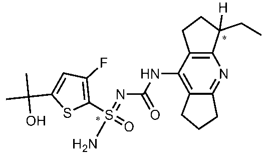 | (S, R) or (S, S) or (R, S) or (R, R)-N'-((3-ethyl-1,2,3,5,6,7-hexahydrodicycl openta[b,e]pyridi n-8-yl)carbamoyl)-3-fluoro-5-(2-hydroxypropan-2-yl)thiophene-2-sulfonimidamide | CHIRALP AK IG, 2.0*25 cm, 5 uM | 30% EtOH in Hex (8 mM NH₃·M eOH) | 467 |
| 535 | 671a | 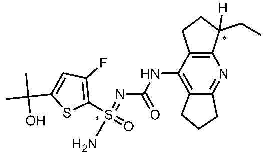 | (S, S) or (S, R) or (R, S) or (R, R)-N'-((3-ethyl-1,2,3,5,6,7-hexahydrodicycl openta[b,e]pyridi n-8-yl)carbamoyl)-3-fluoro-5-(2-hydroxypropan-2-yl)thiophene-2-sulfonimidamide | CHIRALP AK IG, 2.0*25 cm, 5 uM | 30% EtOH in Hex (8 mM NH₃·M eOH) | 467 |
| 536 | 605d | 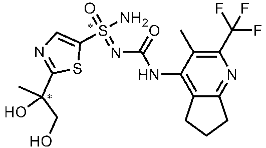 | (R, S) and (S, S) or (R, R) and (S, R)-2-(1,2-Dihydroxypropan -2-yl)-N'-((3-methyl-2-(trifluoromethyl)-6,7-dihydro-5H-cyclopenta[b]pyri din-4-yl)carbamoyl)thi azole-5-sulfonimidamide (from Ex. 240; mixture of two isomers) | CHIRAL ART Cellulose-SB, 2*25 cm, 5 uM | 30% EtOH in Hex (0.1% FA) | 480 |
| 537 | 605b | 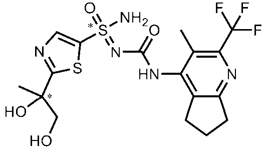 | (R, R) and (S, R) or (R, S) and (S, S)-2-(1,2-Dihydroxypropan -2-yl)-N'-((3-methyl-2-(trifluoromethyl)-6,7-dihydro-5H-cyclopenta[b]pyri din-4-yl)carbamoyl)thi azole-5-sulfonimidamide (from Ex. 240; mixture of two isomers) | CHIRAL ART Cellulose-SB, 2*25 cm, 5 uM | 30% EtOH in Hex (0.1% FA) | 480 |
| 538 | 643a | 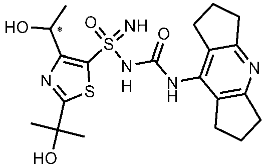 | (R, R/S) or (S, R/S)-N-((1,2,3,5,6,7-hexahydrodicycl openta[b,e]pyridi n-8-yl)carbamoyl)-4-(1-hydroxyethyl)-2-(2-hydroxypropan-2-yl)thiazole-5-sulfonimidamide (from Ex. 237; mixture of two isomers) | CHIRALP AK IG, 0.46*10 cm, 3 um | 30% EtOH in Hex (0.1% FA) | 466 |
| 539 | 643b | 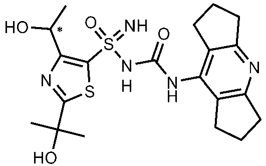 | (S, R/S) or (R, R/S)-N-((1,2,3,5,6,7-hexahydrodicycl openta[b,e]pyridi n-8-yl)carbamoyl)-4-(1-hydroxyethyl)-2-(2-hydroxypropan-2-yl)thiazole-5-sulfonimidamide (from Ex. 237; mixture of two isomers) | CHIRALP AK IG, 0.46*10 cm, 3 um | 30% EtOH in Hex (0.1% FA) | 466 |
| 540 | 171c | 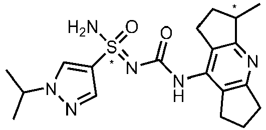 | (R, R) or (R, S) or (S, R) or (S, S)-1-Isopropyl-N'-((3-methyl-1,2,3,5,6,7-hexahydrodicycl openta[b,e]pyridi n-8-yl)carbamoyl)-1H-pyrazole-4-sulfonimidamide | Column 2: Reg-AD, 30*250 mm, 5 um, 20% IPA in Hex (8 mM NH₃·MeOH) to separate two fast-co-eluting isomers from Column 1 Chiralpak IC, 2*25 cm, 5 um, 30% MeOH (0.1% DEA) in CO₂ | 403 | |
| 541 | 171b | 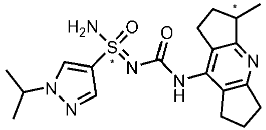 | (R, S) or (R, R) or (S, R) or (S, S)-1-Isopropyl-N'-((3-methyl-1,2,3,5,6,7-hexahydrodicycl openta[b,e]pyridi n-8-yl)carbamoyl)-1H-pyrazole-4-sulfonimidamide | Column 2: Reg-AD, 30*250 mm, 5 um, 20% IPA in Hex (8 mM NH₃·MeOH) to separate two fast-co-eluting isomers from Column 1 Chiralpak IC, 2*25 cm, 5 um, 30% MeOH (0.1% DEA) in CO₂ | 403 | |
| 542 | 171a | 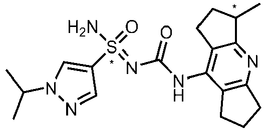 | (S, R) or (S, S) or (R, S) or (R, R)-1-Isopropyl-N'-((3-methyl-1,2,3,5,6,7-hexahydrodicycl openta[b,e]pyridi n-8-yl)carbamoyl)-1H-pyrazole-4-sulfonimidamide | Column 2: CHIRALPAK AD, 5*25 cm, 5 um, 50% EtOH in CO₂ to separate two slow-co-eluting isomers from Column 1 Chiralpak IC, 2*25 cm, 5 um, 30% MeOH (0.1% DEA) in CO₂ | 403 | |
| 543 | 171d | 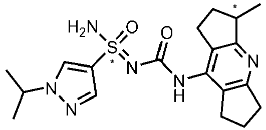 | (S, S) or (S, R) or (R, S) or (R, R)-1-Isopropyl-N'-((3-methyl-1,2,3,5,6,7-hexahydrodicycl openta[b,e]pyridi n-8-yl)carbamoyl)-1H-pyrazole-4-sulfonimidamide | Column 2: CHIRALPAK AD, 5*25 cm, 5 um, 50% EtOH in CO₂ to separate two slow-co-eluting isomers from Column 1 Chiralpak IC, 2*25 cm, 5 um, 30% MeOH (0.1% DEA) in CO₂ | 403 | |

### Example 544 (Compound 734)

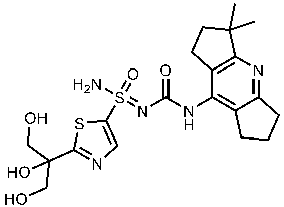

### N'-((3,3-dimethyl-1,2,3,5,6,7-hexahydrodicyclopenta[b,e]pyridin-8-yl)carbamoyl)-2-(1,2,3-trihydroxypropan-2-yl)thiazole-5-sulfonimidamide (Scheme VIII)

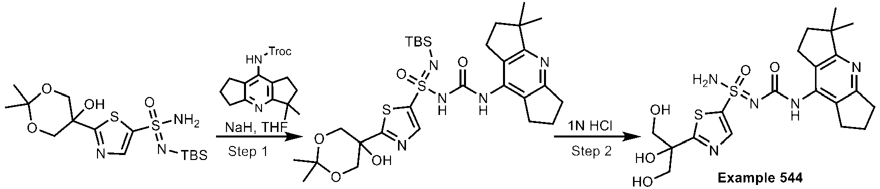

### Step 1: N'-(tert-butyldimethylsilyl)-N-((3,3-dimethyl-1,2,3,5,6,7-hexahydrodicyclopenta[b,e]pyridine -8-yl)carbamoyl)-2-(5-hydroxy-2,2-dimethyl-1,3-dioxan-5-yl)thiazole-5-sulfonimidamide

To a stirred solution of N'-(tert-butyldimethylsilyl)-2-(5-hydroxy-2,2-dimethyl-1,3-dioxan-5-yl)thiazole -5-sulfonimidamide (50 mg, 0.12 mmol) in THF (10 mL) in a 25-mL round-bottom flask under nitrogen was added NaH (60% wt. dispersion in mineral oil, 24 mg, 0.61 mmol) at 0 °C. The resulting solution was stirred for 10 min at 0 °C. To the solution was added 2,2,2-trichloroethyl (3,3-dimethyl-1,2,3,5,6,7-hexa- hydrodicyclopenta[b,e]pyridin-8-yl)carbamate (46 mg, 0.12 mmol) in portions at 0 °C. The resulting mixture was stirred for 2 h at 35 °C. The reaction was quenched by the addition of 10 mL water/ice at 0 °C and extracted with 3x15 mL of EtOAc. The combined organic layers were washed with brine (3x15 mL) and dried over anhydrous Na₂SO₄. After filtration, the filtrate was concentrated under reduced pressure. The residue was purified by Prep-TLC (PE/EtOAc = 3:1). This resulted in 60 mg (77%) of the title compound as an off-white solid. MS-ESI: 636 (M+1).

### Step 2: N'-((3,3-dimethyl-1,2,3,5,6,7-hexahydrodicyclopenta[b,e]pyridin-8-yl)carbamoyl)-2-(1,2,3- trihydroxypropan-2-yl)thiazole-5-sulfonimidamide

To a stirred solution of N'-(tert-butyldimethylsilyl)-N-((3,3-dimethyl-1,2,3,5,6,7-hexahydrodicyclopenta[b,e] pyridin-8-yl)carbamoyl)-2-(5-hydroxy-2,2-dimethyl-1,3-dioxan-5-yl)thiazole-5-sulfonimidamide (60 mg, 0.094 mmol) in THF (5 mL) in a 25-mL round-bottom flask was added HCl in 1,4-dioxane(4 M, 1.25 mL) dropwise at RT. The resulting mixture was stirred for 2 h at RT. The resulting mixture was concentrated under reduced pressure. The crude product was purified by Prep-HPLC with the following conditions: XBridge Prep OBD C18 Column, 30×150 mm 5 um; Mobile Phase A: water (10 mM NH₄HCO₃+0.1% NH₃·H₂O), Mobile Phase B: ACN; Flow rate: 60 mL/min; Gradient: 5% B to 25% B over 7 min; UV 254/210 nm; Rt: 4.95 min. This resulted in 17.1 mg (38%) of **Example 544** as a white solid. MS-ESI: 482 (M+1). ¹H NMR (400 MHz, MeOH-*d*₄) δ 8.22 (s, 1H), 3.91-3.83 (m, 4H), 2.98 (t*, J=* 7.6 Hz, 2H), 2.87-2.77 (m, 4H), 2.16-2.09 (m, 2H), 2.00-1.96 (m, 2H), 1.40 (s, 6H).

**Table 40. Examples in the following table were prepared using similar conditions as described in Example 9 and Scheme 2 from appropriate starting materials.**

| Example # | Final Target Number | Structure | IUPAC Name | Exact Mass [M+H]⁺ |
|---|---|---|---|---|
| 545 | 736 | 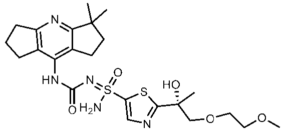 | N'-((3,3-dimethyl-1,2,3,5,6,7-hexahydrodicyclopent a[b,e]pyridin-8-yl)carbamoyl)-2-((R)-2-hydroxy-1-(2-methoxyethoxy)propa n-2-yl)thiazole-5-sulfonimidamide | 524 |
| 546 | 735 | 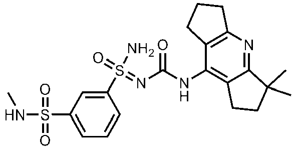 | 3-(N'-((3,3-dimethyl-1,2,3,5,6,7-hexahydrodicyclopent a[b,e]pyridin-8-yl)carbamoyl)sulfamid imidoyl)-N-methylbenzenesulfona mide | 478 |
| 547 | 721c | 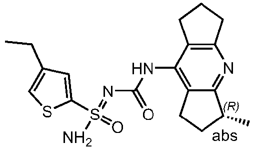 | 4-Ethyl-N'-(((R)-3-methyl-1,2,3,5,6,7-hexahydrodicyclopent a[b,e]pyridin-8-yl)carbamoyl)thiophen e-2-sulfonimidamide | 405 |

**Table 41. Examples in the following table were prepared using similar conditions as described in Example 236 and Scheme II from appropriate starting materials.**

| Example # | Structure | IUPAC Name | Exact Mass [M+H]⁺ |
|---|---|---|---|
| 548 | 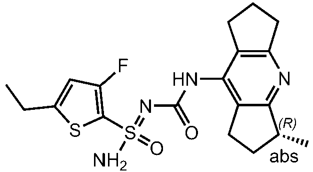 | 5-Ethyl-3-fluoro-N'-(((R)-3-methyl-1,2,3,5,6,7-hexahydrodicyclopenta[b,e]pyridin-8-yl)carbamoyl)thiophene-2-sulfonimidamide | 423 |

**Table 42. Examples in the following table were prepared using similar conditions as described in Example 252 and Scheme V from appropriate starting materials.**

| Example # | Structure | IUPAC Name | Exact Mass [M+H]⁺ |
|---|---|---|---|
| 549 | 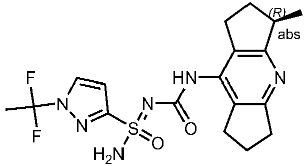 | 1-(1,1-Difluoroethyl)-N'-(((R)-3-methyl-1,2,3,5,6,7-hexahydrodicyclopenta[b,e]pyridin-8-yl)carbamoyl)-1H-pyrazole-3-sulfonimidamide | 425 |
| 550 | 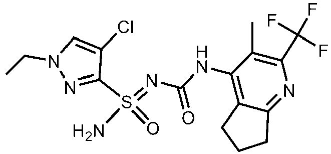 | 4-Chloro-1-ethyl-N'-((3-methyl-2-(trifluoromethyl)-6,7-dihydro-5H-cyclopenta[b]pyridin-4-yl)carbamoyl)-1H-pyrazole-3-sulfonimidamide | 451 |

**Table 43. Examples in the following table were obtained from chiral HPLC resolutions of racemic examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. Assigned stereochemistry in compound names are tentative.**

| Ex. # | Final Target Number | Structure | IUPAC Name | Column | Eluents | LC-MS [M+H]⁺ |
|---|---|---|---|---|---|---|
| 553 | 723b | 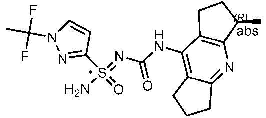 | (R) or (S)-1-(1,1-Difluoroethyl)-N'-(((R)-3-methyl-1,2,3,5,6,7-hexahydrodicyclopenta[b, e]pyridin-8-yl)carbamoyl)-1H-pyrazole-3-sulfonimidamide | CHIRA LPAK IE, 2*25 cm, 5 um | 50% IPA in Hex (0.1% FA) | 425 |
| 554 | 723a | 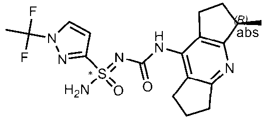 | (S) or (R)-1-(1,1-Difluoroethyl)-N'-(((R)-3-methyl-1,2,3,5,6,7-hexahydrodicyclopenta[b, e]pyridin-8-yl)carbamoyl)-1H-pyrazole-3-sulfonimidamide | CHIRA LPAK IE, 2*25 cm, 5 um | 50% IPA in Hex (0.1% FA) | 425 |
| 555 | 721b | 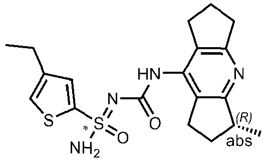 | (R) or (S)-4-Ethyl-N'-(((R)-3-methyl-1,2,3,5,6,7-hexahydrodicyclopenta[b, e]pyridin-8-yl)carbamoyl)thiophene-2-sulfonimidamide | CHIRA LPAK IC, 2*25 cm, 5 um | 30% EtOH in Hex (8 mM NH₃·MeOH ) | 405 |
| 556 | 721a | 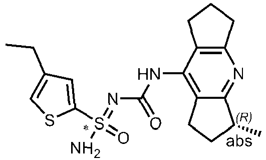 | (S) or (R)-4-Ethyl-N'-(((R)-3-methyl-1,2,3,5,6,7-hexahydrodicyclopenta[b, e]pyridin-8-yl)carbamoyl)thiophene-2-sulfonimidamide | CHIRA LPAK IC, 2*25 cm, 5 um | 30% EtOH in Hex (8 mM NH₃·MeOH ) | 405 |
| 557 | 720b | 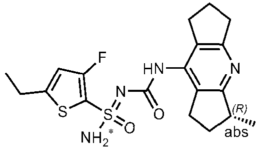 | (S) or (R)-5-Ethyl-3-fluoro-N'-(((R)-3-methyl-1,2,3,5,6,7-hexahydrodicyclopenta[b, e]pyridin-8-yl)carbamoyl)thiophene-2-sulfonimidamide | CHIRA L ART Cellulos e-SB, 2*25 cm, 5 um | 30% EtOH in Hex (8 mM NH₃·MeOH ) | 423 |
| 558 | 720a | 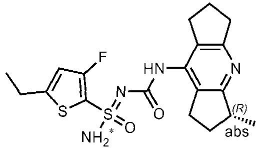 | (R) or (S)-5-Ethyl-3-fluoro-N'-(((R)-3-methyl-1,2,3,5,6,7-hexahydrodicyclopenta[b, e]pyridin-8-yl)carbamoyl)thiophene-2-sulfonimidamide | CHIRA L ART Cellulos e-SB, 2*25 cm, 5 um | 30% EtOH in Hex (8 mM NH₃·MeOH ) | 423 |
| 559 | 729b | 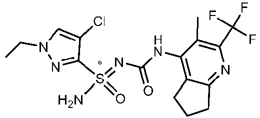 | (R) or (S)-4-Chloro-1-ethyl-N'-((3-methyl-2-(trifluoromethyl)-6,7-dihydro-5H-cyclopenta[b]pyridin-4-yl)carbamoyl)-1H-pyrazole-3-sulfonimidamide | CHIRA LPAK IE, 2*25 cm, 5 um | 30% EtOH in Hex (0.1% FA) | 451 |
| 560 | 729a | 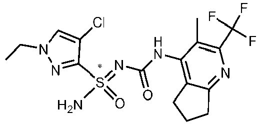 | (S) or (R)-4-Chloro-1-ethyl-N'-((3-methyl-2-(trifluoromethyl)-6,7-dihydro-5H-cyclopenta[b]pyridin-4-yl)carbamoyl)-1H-pyrazole-3-sulfonimidamide | CHIRA LPAK IE, 2*25 cm, 5 um | 30% EtOH in Hex (0.1% FA) | 451 |

### Example 561

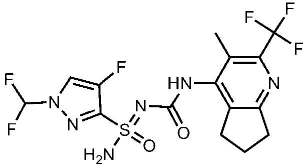

### 1-(Difluoromethyl)-4-fluoro-N'-((3-methyl-2-(trifluoromethyl)-6,7-dihydro-5H-cyclopenta[b]pyridin-4-yl)carbamoyl)-1H-pyrazole-3-sulfonimidamide (Scheme IX)

### Examples 562 and 563

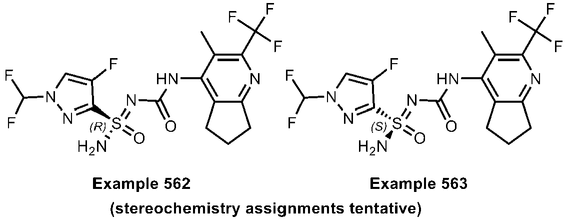

### (R)- and (S)- -1-(difluoromethyl)-4-fluoro-N'-((3-methyl-2-(trifluoromethyl)-6,7-dihydro-5H-cyclopenta[b]pyridin-4-yl)carbamoyl)-1H-pyrazole-3-sulfonimidamide

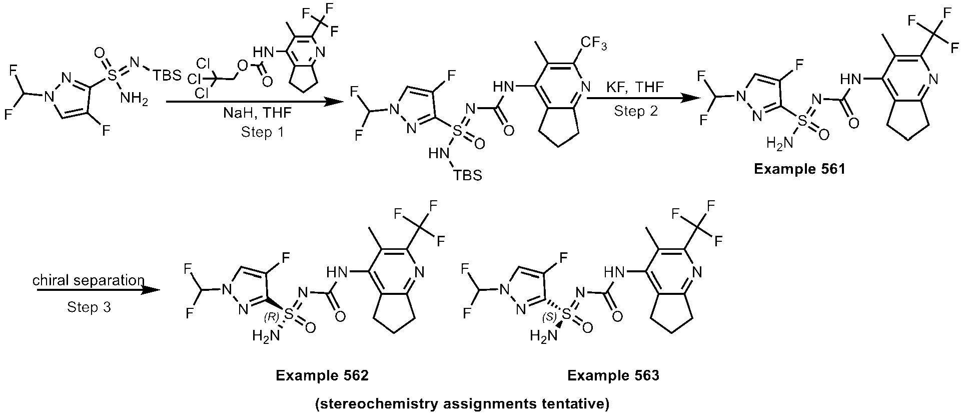

### Step 1: N-(tert-butyldimethylsilyl)-1-(difluoromethyl)-4-fluoro-N'-((3-methyl-2-(trifluoromethyl)-6,7- dihydro-5H-cyclopenta[b]pyridin-4-yl)carbamoyl)-1H-pyrazole-3-sulfonimidamide

To a stirred solution of N'-(tert-butyldimethylsilyl)-1-(difluoromethyl)-4-fluoro-1H-pyrazole-3-sulfonimidamide (150 mg, 0.46 mmol) in THF (10 mL) in a 50-mL round-bottom flask under nitrogen was added NaH (60% wt. dispersion in mineral oil, 54.8 mg, 1.37 mmol) at 0 °C in an ice/water bath. The resulting solution was stirred for 10 min at RT. Then 2,2,2-trichloroethyl (3-methyl-2-(trifluoromethyl)-6,7- dihydro-5H-cyclopenta[b]pyridin-4-yl)carbamate (179 mg, 0.46 mmol) was added to the stirred solution. The resulting solution was stirred for 20 h at RT. The reaction was then quenched by the addition of 1.0 mL of MeOH. The resulting mixture was concentrated. The residue was eluted from silica gel with DCM/MeOH (10:1). This resulted in 250 mg (96%) of the title compound as light yellow oil. MS-ESI: 571(M+1).

### Step 2: 1-(Difluoromethyl)-4-fluoro-N'-((3-methyl-2-(trifluoromethyl)-6,7-dihydro-5H-cyclopenta[b] pyridine-4-yl)carbamoyl)-1H-pyrazole-3-sulfonimidamide

To a stirred solution of N-(tert-butyldimethylsilyl)-1-(difluoromethyl)-4-fluoro-N'-((3-methyl-2-(trifluoromethyl)-6,7-dihydro-5H-cyclopenta[b]pyridin-4-yl)carbamoyl)-1H-pyrazole-3-sulfonimidamide (250 mg, 0.45 mmol) in THF (5.0 mL) in a 50-mL round-bottom flask was added KF (131 mg, 2.25 mmol) at RT. The resulting solution was stirred for 1 h at RT. The solids were filtered out. The resulting mixture was concentrated. The crude product was purified by Prep-HPLC using the following conditions: XBridge Prep C18 OBD Column, 19×150 mm 5 um; Mobile Phase A: water (10 mM NH₄HCO₃+0.1%NH₃.H₂O), Mobile Phase B: ACN; Flow rate: 25 mL/min; Gradient: 8% B to 28% B over 7 min; UV 210/254 nm; Rt: 5.32 min. This resulted in 155 mg (74%) of **Example 561** as an off-white solid. MS-ESI: 457 (M+1). ¹H NMR (400 MHz, DMSO-*d₆*) δ 9.08 (s, 1H), 8.65 (d, *J* = 4.8 Hz, 1H), 8.04 (br s, 2H), 7.86 (t, *J =* 58.4 Hz, 1H), 2.94-2.88 (m, 2H), 2.80-2.74 (m, 2H), 2.20 (s, 3H), 2.07-1.98 (m, 2H).

### Step 3: Chiral resolution

**Example 561** (150 mg) was resolved by prep-chiral-HPLC using the following conditions: CHIRALPAK IG, 20*250 mm, 5 um; Mobile Phase A: Hex (0.1% FA), Mobile Phase B: EtOH; Flow rate: 20 mL/min; Gradient: 20% B; 220/254 nm; Rt₁: 6.505 min (Example 562); Rt₂: 8.721 min (Example 563); This resulted in 37.0 mg of **Example 562** followed by 42.4 mg of **Example 563,** both as off-white solid.

**Example 562:** MS-ESI: 457 (M+1). ¹H NMR (400 MHz, DMSO-*d₆*) δ 9.08 (s, 1H), 8.65 (d, *J* = 4.8 Hz, 1H), 8.11 (br s, 2H), 7.86 (t, *J =* 58.4 Hz, 1H), 2.94-2.88 (m, 2H), 2.78-2.67 (m, 2H), 2.20 (s, 3H), 2.09-1.97 (m, 2H).

**Example 563:** MS-ESI: 457 (M+1). ¹H NMR (400 MHz, DMSO-*d₆*) δ 9.08 (s, 1H), 8.65 (d, *J* = 4.4 Hz, 1H), 8.11 (br s, 2H), 7.86 (t, *J =* 58.4 Hz, 1H), 2.94-2.87 (m, 2H), 2.81-2.71 (m, 2H), 2.20 (s, 3H), 2.09-1.96 (m, 2H).

**Table 49. Examples in the following table were prepared using similar conditions as described in Example 561 and Scheme IX from appropriate starting materials.**

| Example # | Structure | IUPAC Name | Exact Mass [M+H]⁺ |
|---|---|---|---|
| 564 | 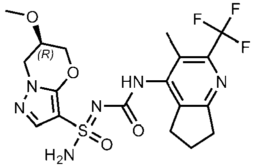 | (6R)-6-methoxy-N'-((3-methyl-2-(trifluoromethyl)-6,7-dihydro-5H-cyclopenta[b]pyridin-4-yl)carbamoyl)-6,7-dihydro-5H-pyrazolo[5,1-b][1,3]oxazine-3-sulfonimidamide | 475 |
| 565 | 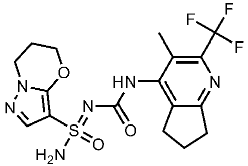 | N'-((3-methyl-2-(trifluoromethyl)-6,7-dihydro-5H-cyclopenta[b]pyridin-4-yl)carbamoyl)-6,7-dihydro-5H-pyrazolo[5,1-b][1,3]oxazine-3-sulfonimidamide | 445 |
| 566 | 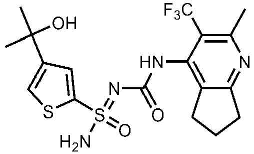 | 4-(2-Hydroxypropan-2-yl)-N'-((2-methyl-3-(trifluoromethyl)-6,7-dihydro-5H-cyclopenta[b]pyridin-4-yl)carbamoyl)thiophene-2-sulfonimidamide | 463 |
| 567 | 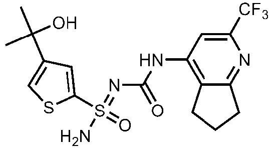 | 4-(2-Hydroxypropan-2-yl)-N'-((2-(trifluoromethyl)-6,7-dihydro-5H-cyclopenta[b]pyridin-4-yl)carbamoyl)thiophene-2-sulfonimidamide | 449 |
| 568 | 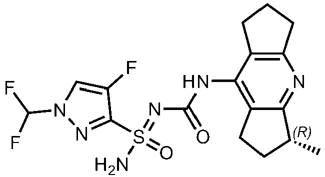 | 1-(Difluoromethyl)-4-fluoro-N'-(((R)-3-methyl-1,2,3,5,6,7-hexahydrodicyclopenta[b,e]pyridin-8-yl)carbamoyl)-1H-pyrazole-3-sulfonimidamide | 429 |
| 569 | 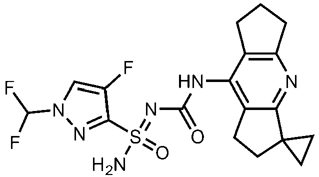 | 1-(Difluoromethyl)-4-fluoro-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 441 |
| 570 | 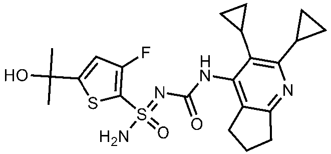 | N'-((2,3-dicyclopropyl-6,7-dihydro-5H-cyclopenta[b]pyridin-4-yl)carbamoyl)-3-fluoro-5-(2-hydroxypropan-2-yl)thiophene-2-sulfonimidamide | 479 |
| 571 | 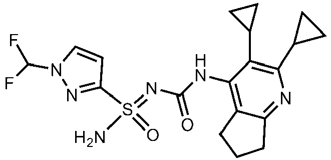 | N'-((2,3-dicyclopropyl-6,7-dihydro-5H-cyclopenta[b]pyridin-4-yl)carbamoyl)-1-(difluoromethyl)-1H-pyrazole-3-sulfonimidamide | 437 |
| 572 | 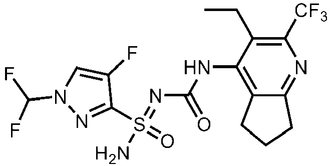 | 1-(Difluoromethyl)-N'-((3-ethyl-2-(trifluoromethyl)-6,7-dihydro-5H-cyclopenta[b]pyridin-4-yl)carbamoyl)-4-fluoro-1H-pyrazole-3-sulfonimidamide | 471 |

### Example 573

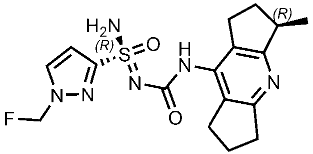

### (R)-1-(fluoromethyl)-N'-(((R)-3-methyl-1,2,3,5,6,7-hexahydrodicyclopenta[b,e]pyridin-8-yl)carbamoyl)-1H-pyrazole-3-sulfonimidamide (Scheme X)

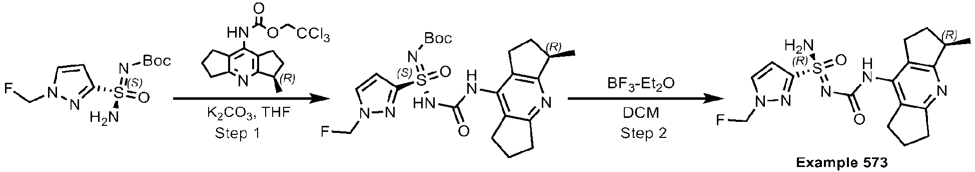

### Step 1: Tert-butyl((R)-(1-(fluoromethyl)-1H-pyrazol-3-yl)(3-((R)-3-methyl-1,2,3,5,6,7-hexahydrodicyclopenta[b,e]pyridin-8-yl)ureido)(oxo)-λ⁶-sulfaneylidene)carbamate

To a stirred solution of tert-butyl (S)-(amino(1-(fluoromethyl)-1H-pyrazol-3-yl)(oxo)-λ⁶-sulfaneylidene) carbamate (10 mg, 0.036 mmol) in THF (1.0 mL) in a 8-mL sealed tube was added K₂CO₃ (24.8 mg, 0.18 mmol) and 2,2,2-trichloroethyl (R)-(3-methyl-1,2,3,5,6,7-hexahydrodicyclopenta[b,e]pyridin-8-yl)carbamate (13 mg, 0.036 mmol). The resulting solution was stirred for 2 h at 80 °C. The reaction mixture was cooled to RT. The resulting solution was diluted with 20 mL of EtOAc. The solids were filtered out, the filtrate was concentrated under reduced pressure. This resulted in 11 mg (crude) of the title compound as a white solid. MS-ESI: 493 (M+1).

### Step 2: (R)-1-(fluoromethyl)-N'-(((R)-3-methyl-1,2,3,5,6,7-hexahydrodicyclopenta[b,e]pyridin-8-yl) carbamoyl)-1H-pyrazole-3-sulfonimidamide

To a stirred solution of tert-butyl ((R)-(1-(fluoromethyl)-1H-pyrazol-3-yl)(3-((R)-3-methyl-1,2,3,5,6,7- hexahydrodicyclopenta[b,e]pyridin-8-yl)ureido)(oxo)-λ⁶-sulfaneylidene)carbamate (10 mg) in DCM (10 mL) in a 20-mL sealed tube was added BF₃.Et₂O (47%wt., 0.050 mL, 0.40 mmol) dropwise at 0 °C. The resulting solution was stirred for 90 min at RT. The resulting solution was quenched with 2.0 mL of MeOH. The resulting mixture was concentrated. The crude product was purified by Prep-HPLC using the following conditions: XBridge Prep OBD C18 Column, 30*150 mm 5 um; mobile phase, water (10 mM NH₄HCO₃ + 0.1% NH₃·H₂O) and ACN (3% to 30% over 7 min); UV 254/220 nm; Rt: 6.13 min. This resulted in 0.80 mg (5.7% over 2 steps) of **Example 573** as a white solid. MS-ESI: 393 (M+1).

**Table 50. Examples in the following table were prepared using similar conditions as described in Example 573 and Scheme X from appropriate starting materials. The structures of Examples 575 and 576 were assigned based their proton NMR in comparison with similar literature compounds (Journal of Organic Chemistry. 2013, 78(11), 5349-5356).**

| Example # | Structure | IUPAC Name | Exact Mass [M+H]⁺ |
|---|---|---|---|
| 574 | 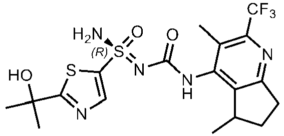 | Mixture of (R)-N'-((3,5-dimethyl-2-(trifluoromethyl)-6,7-dihydro-5H-cyclopenta[b]pyridin-4-yl)carbamoyl)-2-(2-hydroxypropan-2-yl)thiazole-5-sulfonimidamide and | 478 |
| | 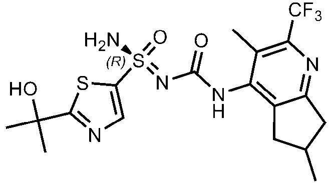 | (R)-N'-((3,6-dimethyl-2-(trifluoromethyl)-6,7-dihydro-5H-cyclopenta[b]pyridin-4-yl)carbamoyl)-2-(2-hydroxypropan-2-yl)thiazole-5-sulfonimidamide | |
| 575 | 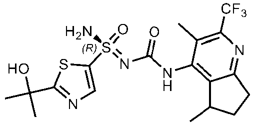 | (R)-N'-((3,5-dimethyl-2-(trifluoromethyl)-6,7-dihydro-5H-cyclopenta[b]pyridin-4-yl)carbamoyl)-2-(2-hydroxypropan-2-yl)thiazole-5-sulfonimidamide | 478 |
| 576 | 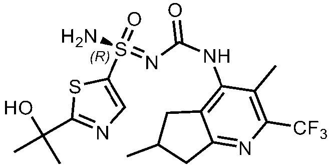 | (R)-N'-((3,6-dimethyl-2-(trifluoromethyl)-6,7-dihydro-5H-cyclopenta[b]pyridin-4-yl)carbamoyl)-2-(2-hydroxypropan-2-yl)thiazole-5-sulfonimidamide | 478 |

### Example 577

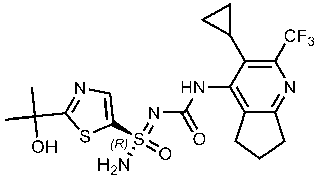

### (R)-N'-((3-cyclopropyl-2-(trifluoromethyl)-6,7-dihydro-5H-cyclopenta[b]pyridin-4-yl)carbamoyl)-2-(2-hydroxypropan-2-yl)thiazole-5-sulfonimidamide (Scheme XI)

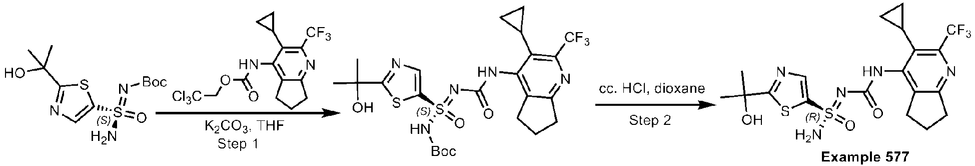

### Step 1: Tert-butyl(S)-(N-((3-cyclopropyl-2-(trifluoromethyl)-6,7-dihydro-5H-cyclopenta[b]pyridine-4-yl)carbamoyl)-2-(2-hydroxypropan-2-yl)thiazole-5-sulfonimidoyl)carbamate

To a stirred solution of tert-butyl (S)-(amino(2-(2-hydroxypropan-2-yl)thiazol-5-yl)(oxo)-λ⁶-sulfaneylidene) carbamate (90 mg, 0.28 mmol) in THF (10 mL) in a 20-mL sealed tube was added K₂CO₃ (193 mg, 1.4 mmol) and 2,2,2-trichloroethyl (3-cyclopropyl-2-(trifluoromethyl)-6,7-dihydro-5H-cyclopenta[b]pyridine -4-yl)carbamate (117 mg, 0.28 mmol) at RT. The resulting solution was stirred for 2 h at 80 °C. The reaction mixture was cooled to RT. The resulting solution was diluted with 50 mL of EtOAc. The solids were filtered out. The filtrate was concentrated. This resulted in 100 mg (crude) of the title compound as a white solid. MS-ESI: 590 (M+1).

### Step 2: (R)-N'-((3-cyclopropyl-2-(trifluoromethyl)-6,7-dihydro-5H-cyclopenta[b]pyridin-4-yl) carbamoyl)-2-(2-hydroxypropan-2-yl)thiazole-5-sulfonimidamide

A solution of tert-butyl (S)-(N-((3-cyclopropyl-2-(trifluoromethyl)-6,7-dihydro-5H-cyclopenta[b]pyridine -4-yl)carbamoyl)-2-(2-hydroxypropan-2-yl)thiazole-5-sulfonimidoyl)carbamate (100 mg, 0.17 mmol) in 4 M HCl/dioxane (4 mL) in a 50-mL round-bottom flask was stirred for 30 min at RT. The resulting mixture was concentrated under vacuum. The crude product was purified by Prep-HPLC using the following conditions: XBridge Prep C18 OBD Column, 19×150 mm 5 um; Mobile Phase A: water (10 mM NH₄HCO₃ + 0.1% NH₃·H₂O), Mobile Phase B: ACN; Flow rate: 25 mL/min; Gradient: 7% B to 20% B over 10 min; UV 210/254 nm; Rt₁: 11.03 min. This resulted in 30 mg (22% over 2 steps) of **Example 577** as a white solid. MS-ESI: 490 (M+1). ¹H NMR (400 MHz, DMSO-*d₆*) δ 8.98 (s, 1H), 8.09 (s, 1H), 7.92 (br s, 2H), 6.28 (s, 1H), 2.94-2.87 (m, 2H), 2.84-2.79 (m, 2H), 2.07-1.98 (m, 2H), 1.83-1.78 (m, 1H), 1.50 (s, 6H), 0.93-0.80 (m, 2H), 0.44-0.30 (m, 2H).

**Table 51. Examples in the following table were prepared using similar conditions as described in Example 577 and Scheme XI from appropriate starting materials.**

| Example # | Structure | IUPAC Name | Exact Mass [M+H]⁺ |
|---|---|---|---|
| 578 | 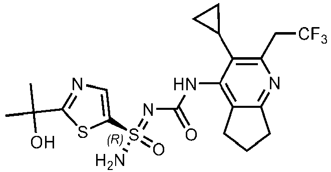 | (R)-N'-((3-cyclopropyl-2-(2,2,2-trifluoroethyl)-6,7-dihydro-5H-cyclopenta[b]pyridin-4-yl)carbamoyl)-2-(2-hydroxypropan-2-yl)thiazole-5-sulfonimidamide | 504 |
| 579 | 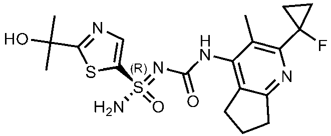 | (R)-N'-((2-(1 -fluorocyclopropyl)-3-methyl-6,7-dihydro-5H-cyclopenta[b]pyridin-4-yl)carbamoyl)-2-(2-hydroxypropan-2-yl)thiazole-5-sulfonimidamide | 454 |

### Example 580

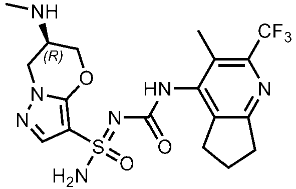

### (6R)-N'-((3-methyl-2-(trifluoromethyl)-6,7-dihydro-5H-cyclopenta[b]pyridin-4-yl)carbamoyl)-6-(methylamino)-6,7-dihydro-5H-pyrazolo[5,1-b][1,3]oxazine-3-sulfonimidamide (Scheme XII)

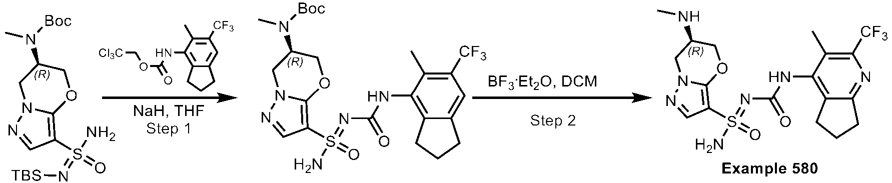

### Step 1: Tert-butylmethyl((6R)-3-(N'-((5-methyl-6-(trifluoromethyl)-2,3-dihydro-1H-inden-4-yl) carbamoyl)sulfamidimidoyl)-6,7-dihydro-5H-pyrazolo[5,1-b][1,3]oxazin-6-yl)carbamate

To a stirred solution of tert-butyl ((6R)-3-(N'-(tert-butyldimethylsilyl)sulfamidimidoyl)-6,7-dihydro-5H- pyrazolo[5,1-b][1,3]oxazin-6-yl)(methyl)carbamate (100 mg, 0.22 mmol) in THF (5.0 mL) in a 50-mL round-bottom flask under nitrogen was added NaH (60%wt. dispersion in mineral oil, 18 mg, 0.44 mmol) at 0 °C in an ice/water bath, followed by 2,2,2-trichloroethyl(5-methyl-6-(trifluoromethyl)-2,3-dihydro -1H-inden-4-yl)carbamate (88 mg, 0.22 mmol) in portions at 0 °C. The resulting solution was stirred for 3 h at RT. The reaction was then quenched by adding 5.0 mL of water. The resulting solution was extracted with 3x30 mL of EtOAc, the combined organic layers were dried over anhydrous sodium sulfate and concentrated under vacuum. This resulted in 100 mg (crude) of the title compound as a white solid. MS-ESI: 573 (M+1).

### Step 2: (6R)-N'-((3-methyl-2-(trifluoromethyl)-6,7-dihydro-5H-cyclopenta[b]pyridin-4-yl)carbamoyl) -6-(methylamino)-6,7-dihydro-5H-pyrazolo[5,1-b][1,3]oxazine-3-sulfonimidamide

To a stirred solution of tert-butyl methyl((6R)-3-(N'-((5-methyl-6-(trifluoromethyl)-2,3-dihydro-1H-inden -4-yl)carbamoyl)sulfamidimidoyl)-6,7-dihydro-5H-pyrazolo[5,1-b][1,3]oxazin-6-yl)carbamate (80 mg, 0.14 mmol) in DCM (3 mL) in a 25-mL round-bottom flask was added BF₃.Et₂O (47% wt., 0.05 mL, 0.40 mmol) dropwise with stirring at 0 °C in an ice/water bath. The resulting solution was stirred for 1 h at RT. The reaction was then quenched by adding 1.0 mL of water. The resulting solution was extracted with 3x10 mL of EtOAc and dried over anhydrous sodium sulfate. The solids were filtered out. The resulting mixture was concentrated under vacuum. The crude product was purified by Prep-HPLC using the following conditions: XSelect CSH Prep C18 OBD Column, 5 um, 19*150 mm; Mobile Phase A: water (10 mM NH₄HCO₃), Mobile Phase B: ACN; Flow rate: 25 mL/min; Gradient: 16% B to 31% B over 7 min; 210/254 nm; Rt₁: 6.05 min. This resulted in 50 mg (48%, over two steps) of **Example 580** as a white solid. MS-ESI: 474 (M+1). ¹H NMR (400 MHz, DMSO-*d₆*) δ 8.82 (s, 1H), 8.21 (s, 1H), 7.55 (s, 1H), 7.33 (br s, 2H), 4.40-4.19 (m, 3H), 3.99-3.90 (m, 1H), 3.20-3.14 (m, 1H), 2.92 *(t, J =* 7.6 Hz, 2H), 2.84 (t, *J =* 7.6 Hz, 2H), 2.34 (s, 3H), 2.23 (s, 3H), 2.13-1.99 (m, 2H).

### Example 581

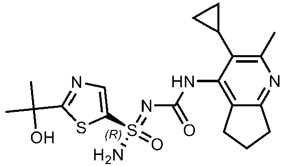

### (R)-N'-((3-cyclopropyl-2-methyl-6,7-dihydro-5H-cyclopenta[b]pyridin-4-yl)carbamoyl)-2-(2-hydroxypropan-2-yl)thiazole-5-sulfonimidamide (Scheme XIII)

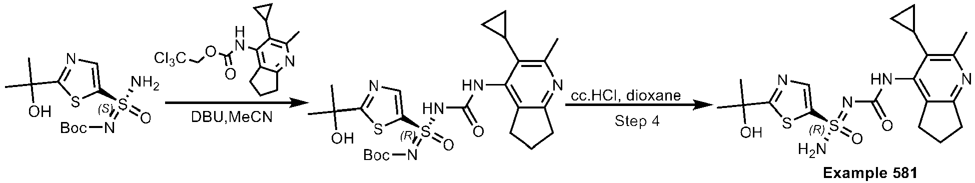

### Step 1: Tert-butyl (R)-((3-(3-cyclopropyl-2-methyl-6,7-dihydro-5H-cyclopenta[b]pyridin-4-yl)ureido) (2-(2-hydroxypropan-2-yl)thiazol-5-yl)(oxo)-λ⁶-sulfaneylidene)carbamate

To a stirred solution of tert-butyl (S)-(amino(2-(2-hydroxypropan-2-yl)thiazol-5-yl)(oxo)-λ⁶-sulfaneylidene) carbamate (71 mg, 0.22 mmol) in MeCN (10 mL) in a 25-mL round-bottom flask was added DBU (100 mg, 0.66 mmol) and 2,2,2-trichloroethyl (3-cyclopropyl-2-methyl-6,7-dihydro-5H-cyclopenta[b]pyridin-4-yl) carbamate (80 mg, 0.22 mmol). The resulting solution was stirred for 6 h at 35 °C in an oil bath. The reaction was then quenched by the addition of 6.0 mL of water. The resulting solution was extracted with 5x20 mL of EtOAc and the organic layers were combined and concentrated. This resulted in 160 mg (crude) of the title compound as a brown solid. MS-ESI: 536 (M+1).

### Step 2: (R)-N'-((3-cyclopropyl-2-methyl-6,7-dihydro-5H-cyclopenta[b]pyridin-4-yl)carbamoyl)-2-(2- hydroxypropan-2-yl)thiazole-5-sulfonimidamide

To a stirred solution of tert-butyl (R)-((3-(3-cyclopropyl-2-methyl-6,7-dihydro-5H-cyclopenta[b]pyridine -4-yl)ureido)(2-(2-hydroxypropan-2-yl)thiazol-5-yl)(oxo)-λ⁶-sulfaneylidene)carbamate (120 mg, 0.22 mmol) in dioxane (15 mL) was added conc. HCl (12 M, 0.10 mL, 1.2 mmol) dropwise at RT. The resulting solution was stirred for 2 h at RT. The pH value of the mixture was adjusted to 7 with Sat. NaHCO₃ (aq.) then extracted with 5x20 mL of EtOAc and the organic layers were combined, dried over Na₂SO₄ and concentrated under vacuum. The crude product was purified by Prep-HPLC using the following conditions: XBridge Prep OBD C18 Column, 30*150 mm* 5 um; mobile phase, water (10 mM NH₄HCO₃ + 0.1%NH₃·H₂O) and ACN (5% Phase B to 28% over 7 min); UV 210/254 nm; Rt₁: 6.05 min. This resulted in 45 mg (46% over two steps) of **Example 581** as a white solid. MS-ESI: 436 (M+1). ¹H NMR (400 MHz, DMSO-*d₆*) δ 8.56 (s, 1H), 8.07 (s, 1H), 7.86 (br s, 2H), 6.28 (s, 1H), 2.81-2.64 (m, 4H), 2.46 (s, 3H), 1.97-1.90 (m, 2H), 1.63-1.55 (m, 1H), 1.50 (s, 6H), 0.88-0.77 (m, 2H), 0.32-0.25 (m, 2H).

### Example 582

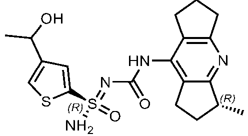

### (R)-4-(1-hydroxyethyl)-N'-(((R)-3-methyl-1,2,3,5,6,7-hexahydrodicyclopenta[b,e]pyridin-8-yl)carbamoyl)thiophene-2-sulfonimidamide (Scheme XIV)

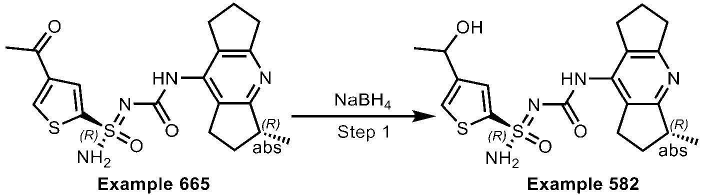

### Step 1: (R)-4-(1-hydroxyethyl)-N'-(((R)-3-methyl-1,2,3,5,6,7-hexahydrodicyclopenta[b,e]pyridin-8-yl) carbamoyl)thiophene-2-sulfonimidamide

To a stirred solution of (R)-4-acetyl-N'-(((R)-3-methyl-1,2,3,5,6,7-hexahydrodicyclopenta[b,e]pyridin-8-yl) carbamoyl)thiophene-2-sulfonimidamide (200 mg, 0.48 mmol) in MeOH (20 mL) in a 50-mL round-bottom flask under nitrogen was added NaBH₄ (54 mg, 1.43 mmol) in portions at 0 °C in an ice/water bath. The resulting solution was stirred overnight at RT. The reaction was then quenched by the addition of 10 mL of 1M HCl. The resulting solution was extracted with 2x50 mL of EtOAc and dried over anhydrous sodium sulfate and concentrated. The crude product was purified by Prep-HPLC using the following conditions: XBridge Prep C18 OBD Column, 19*150 mm 5 um; mobile phase, water (10 mM NH₄HCO₃ + 0.1% NH₃·H₂O) and ACN (5% Phase B to 25% over 7 min); UV 210/254 nm, Rt: 6.63 min. This resulted in 160 mg (80%) of **Example 582** as a white solid. MS-ESI: 421 (M+1). ¹H NMR (400 MHz, DMSO-*d₆*) δ 8.75 (s, 1H), 7.71 (br s, 2H), 7.60 (s, 2H), 5.33 (d, *J =* 4.8 Hz, 1H), 4.79-4.68 (m, 1H), 3.05-2.97 (m, 1H), 2.85-2.60 (m, 6H), 2.29-2.21 (m, 1H), 2.01-1.90 (m, 2H), 1.54-1.43 (m, 1H), 1.35 (d, *J =* 6.4 Hz, 3H), 1.20 (d, *J =* 7.2 Hz, 3H).

**Table 52 Examples in the following table were prepared using similar conditions as described in Example 582 and Scheme XIV from Example 666.**

| Example # | Structure | IUPAC Name | Exact Mass [M+H]⁺ |
|---|---|---|---|
| 583 | 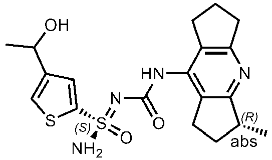 | (S)-4-(1-hydroxyethyl)-N'-(((R)-3-methyl-1,2,3,5,6,7-hexahydrodicyclopenta[b,e]pyridin-8-yl)carbamoyl)thiophene-2-sulfonimidamide | 421 |

### Example 584

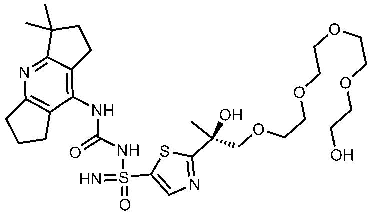

### 2-((S)-1,14-dihydroxy-3,6,9,12-tetraoxapentadecan-14-yl)-N-((3,3-dimethyl-1,2,3,5,6,7-hexahydrodicyclopenta[b,e]pyridin-8-yl)carbamoyl)thiazole-5-sulfonimidamide (Scheme XV)

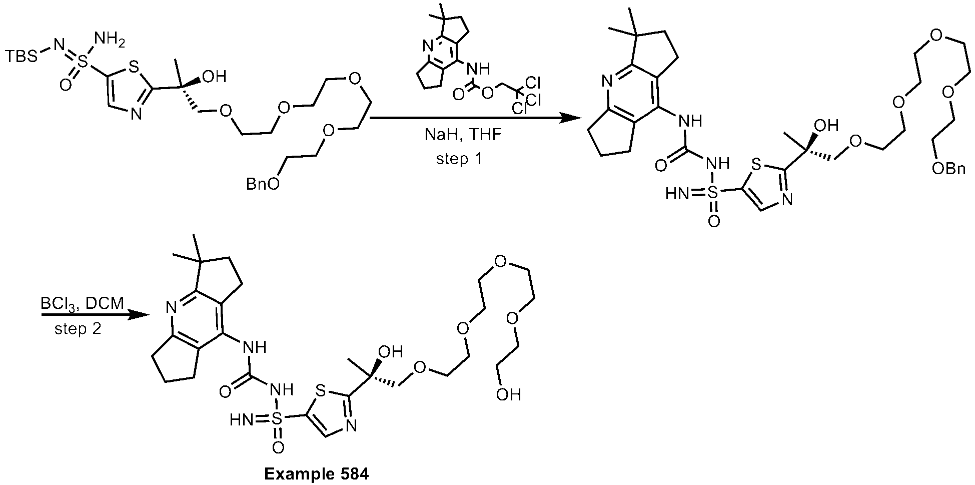

### Step 1: N-((3,3-dimethyl-1,2,3,5,6,7-hexahydrodicyclopenta[b,e]pyridin-8-yl)carbamoyl)-2-((S)-16- hydroxy-1-phenyl-2,5,8,11,14-pentaoxaheptadecan-16-yl)thiazole-5-sulfonimidamide

To a stirred solution of N'-(tert-butyldimethylsilyl)-2-((S)-16-hydroxy-1-phenyl-2,5,8,11,14-pentaoxahepta- decan-16-yl)thiazole-5-sulfonimidamide (100 mg, 0.16 mmol) in THF (5.0 mL) in a 50-mL round-bottom flask under nitrogen was added 2,2,2-trichloroethyl (3,3-dimethyl-1,2,3,5,6,7- hexahydrodicyclopenta[b,e]- pyridin-8-yl)carbamate (60 mg, 0.16 mmol) and NaH (60% wt. dispersion in mineral oil, 13 mg, 0.32 mmol) at 0 °C in an ice/water bath. The resulting solution was stirred for 3 h at RT. The reaction was then quenched by the addition of 5.0 mL of water. The resulting solution was extracted with 3x30 mL of EtOAc and the combined organic layers were dried over anhydrous sodium sulfate. The solids were filtered out. The resulting mixture was concentrated under vacuum. The residue was eluted from silica gel with DCM/MeOH (10:1). This resulted in 100 mg (85%) of the title compound as a white solid. MS-ESI: 732 (M+1).

### Step 2: 2-((S)-1,14-dihydroxy-3,6,9,12-tetraoxapentadecan-14-yl)-N-((3,3-dimethyl-1,2,3,5,6,7 -hexahydrodicyclopenta[b,e]pyridin-8-yl)carbamoyl)thiazole-5-sulfonimidamide

To a stirred solution of N-((3,3-dimethyl-1,2,3,5,6,7-hexahydrodicyclopenta[b,e]pyridin-8-yl)carbamoyl)-2- ((S)-16-hydroxy-1-phenyl-2,5,8,11,14-pentaoxaheptadecan-16-yl)thiazole-5-sulfonimidamide (80 mg, 0.11 mmol) in DCM (15 mL) was added BCl₃ in DCM (1 M, 0.46 mL, 0.46 mmol) dropwise at 0 °C in an ice/water bath under nitrogen. The resulting mixture was stirred for 5 h at 0 °C. The reaction was quenched with water/ice (10 mL) at 0 °C. The resulting mixture was extracted with DCM (3 × 30 mL). The combined organic layers were washed with brine (10 mL) and dried over anhydrous Na₂SO₄. After filtration, the filtrate was concentrated under reduced pressure. The crude product (80 mg) was purified by Prep-HPLC using the following conditions: XSelect CSH Prep C18 OBD Column, 5 um, 19*150 mm; Mobile Phase A: water (10 mM NH₄HCO₃ + 0.1% NH₃·H₂O), Mobile Phase B: ACN; Flow rate: 25 mL/min; Gradient: 18% B to 28% B over 7 min; UV 210/254 nm; Rt₁: 6.20 min. This resulted in 18.7 mg (27%) of **Example 584** as a white solid. MS-ESI: 642 (M+1). ¹H NMR (300 MHz, MeOH-*d₄*) δ 8.19 (s, 1H), 3.90-3.48 (m, 18H), 3.01-2.91 (m, 2H), 2.89-2.72 (m, 4H), 2.20-2.01 (m, 2H), 2.01-1.80 (m, 2H), 1.57 (s, 3H), 1.28 (s, 6H).

### Example 585

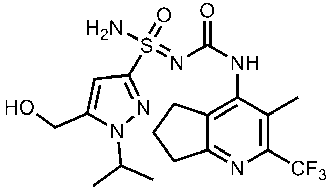

### 5-(Hydroxymethyl)-1-isopropyl-N'-((3-methyl-2-(trifluoromethyl)-6,7-dihydro-5H-cyclopenta[b]pyridin-4-yl)carbamoyl)-1H-pyrazole-3-sulfonimidamide (Scheme XVI)

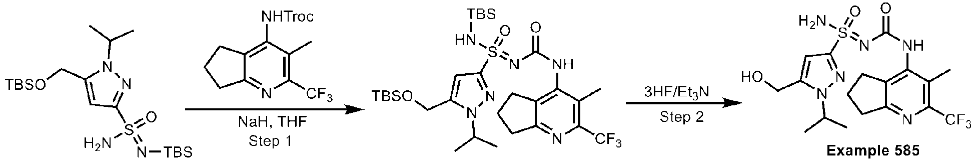

### Step 1: N-(tert-butyldimethylsilyl)-5-(((tert-butyldimethylsilyl)oxy)methyl)-1-isopropyl-N'-((3-methyl-2-(trifluoromethyl)-6,7-dihydro-5H-cyclopenta[b]pyridin-4-yl)carbamoyl)-1H-pyrazole-3-sulfonimidamide

To a stirred solution of N'-(tert-butyldimethylsilyl)-5-(((tert-butyldimethylsilyl)oxy)methyl)-1-isopropyl -1H-pyrazole-3-sulfonimidamide (300 mg, 0.67 mmol) in THF (5.0 mL) under nitrogen was added NaH (60% wt., dispersion in mineral oil, 80.4 mg, 2.01 mmol) in portions at 0 °C. The reaction mixture was stirred for 20 min at 0 °C. To the above mixture was added 2,2,2-trichloroethyl (3-methyl-2-(trifluoromethyl) -6,7-dihydro-5H-cyclopenta[b]pyridin-4-yl)carbamate (263 mg, 0.67 mmol) in THF (3.0 mL) dropwise at 0 °C. The resulting mixture was stirred for 2 h at RT. The reaction was quenched with water/ice (10 mL) at 0 °C. The resulting mixture was extracted with EtOAc (3 × 20 mL). The combined organic layers were dried over anhydrous Na₂SO₄ and concentrated under vacuum. The residue was purified by Prep-TLC with PE/EtOAc (4:1). This resulted in 375 mg (81%) of the title compound as a yellow solid. MS-ESI: 689 (M+1).

### Step 2: 5-(Hydroxymethyl)-1-isopropyl-N'-((3-methyl-2-(trifluoromethyl)-6,7-dihydro-5H-cyclopenta [b]pyridin-4-yl)carbamoyl)-1H-pyrazole-3-sulfonimidamide

To a stirred solution of N-(tert-butyldimethylsilyl)-5-(((tert-butyldimethylsilyl)oxy)methyl)-1-isopropyl-N'- ((3-methyl-2-(trifluoromethyl)-6,7-dihydro-5H-cyclopenta[b]pyridin-4-yl)carbamoyl)-1H-pyrazole-3-sulfonimidamide (370 mg, 0.54 mmol) in THF (5.0 mL) was added triethylamine trihydrofluoride (0.33 mL, 2.03 mmol, 4.50 equiv.) in portions at RT. The reaction mixture was stirred for 2 h at RT. The reaction mixture was concentrated under vacuum. The residue was purified by Prep-HPLC using the following conditions XBridge Prep OBD C18 Column, 30×150 mm, 5 um; Mobile Phase A: water (10 mM NH₄HCO₃ + 0.1% NH₃·H₂O), Mobile Phase B: ACN; Flow rate: 60 mL/min; Gradient: 5% B to 35% B over 7 min; UV 254/210 nm; Rt₁: 6.22 min. This resulted in 100 mg (40%) of **Example 585** as a white solid. MS-ESI: 461 (M+1). ¹H NMR (400 MHz, DMSO-*d₆*) δ 8.90 (s, 1H), 7.41 (br s, 2H), 6.55 (s, 1H), 5.46 (t, *J =* 5.4 Hz, 1H), 4.75-4.61 (m, 1H), 4.55 (d, *J* = 5.6 Hz, 2H), 2.90 (t, *J =* 7.4 Hz, 2H), 2.80 (t, *J =* 7.2 Hz, 2H), 2.21 (s, 3H), 2.08-1.94 (m, 2H), 1.39 (t, *J =* 6.6 Hz, 6H).

**Table 53 Examples in the following table were prepared using similar conditions as described in Example 544 and Scheme VIII from appropriate starting materials.**

| Example # | Structure | IUPAC Name | Exact Mass [M+H]⁺ |
|---|---|---|---|
| 586 | 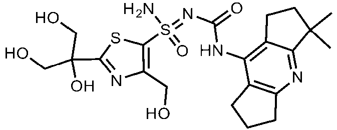 | N'-((3,3-dimethyl-1,2,3,5,6,7-hexahydrodicyclopenta[b,e]pyridin-8-yl)carbamoyl)-4-(hydroxymethyl)-2-(1,2,3-trihydroxypropan-2-yl)thiazole-5-sulfonimidamide | 512 |
| 587 | 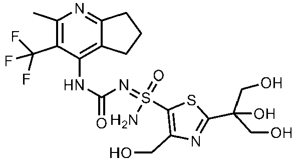 | 4-(Hydroxymethyl)-N'-((2-methyl-3-(trifluoromethyl)-6,7-dihydro-5H-cyclopenta[b]pyridin-4-yl)carbamoyl)-2-(1,2,3-trihydroxypropan-2-yl)thiazole-5-sulfonimidamide | 526 |
| 588 | 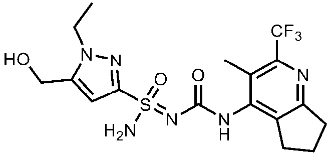 | 1-Ethyl-5-(hydroxymethyl)-N'-((3-methyl-2-(trifluoromethyl)-6,7-dihydro-5H-cyclopenta[b]pyridin-4-yl)carbamoyl)-1H-pyrazole-3-sulfonimidamide | 447 |
| 589 | 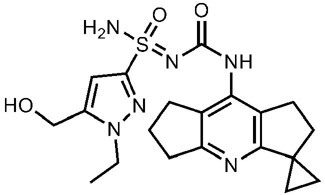 | 1-Ethyl-5-(hydroxymethyl)-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 431 |
| 590 | 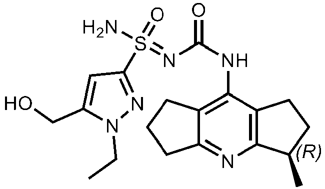 | 1-Ethyl-5-(hydroxymethyl)-N'-(((R)-3-methyl-1,2,3,5,6,7-hexahydrodicyclopenta[b,e]pyridin-8-yl)carbamoyl)-1H-pyrazole-3-sulfonimidamide | 419 |
| 591 | 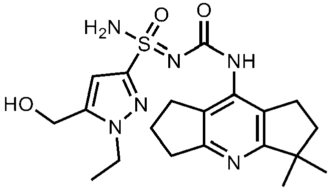 | N'-((3,3-dimethyl-1,2,3,5,6,7-hexahydrodicyclopenta[b,e]pyridin-8-yl)carbamoyl)-1-ethyl-5-(hydroxymethyl)-1H-pyrazole-3-sulfonimidamide | 433 |

**Table 54 Examples in the following table were prepared using similar conditions as described in Example 250 and Scheme III from appropriate starting materials.**

| Example # | Structure | IUPAC Name | Exact Mass [M+H]⁺ |
|---|---|---|---|
| 592 | 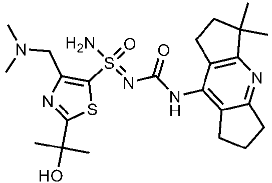 | N'-((3,3-dimethyl-1,2,3,5,6,7-hexahydrodicyclopenta[b,e]pyridin-8-yl)carbamoyl)-4-((dimethylamino)methyl)-2-(2-hydroxypropan-2-yl)thiazole-5-sulfonimidamide | 507 |

**Table 55. Examples in the following table were prepared using similar conditions as described in Example 49 and Scheme 3 from appropriate starting materials.**

| Example # | Structure | IUPAC Name | Exact Mass [M+H]⁺ |
|---|---|---|---|
| 593 | 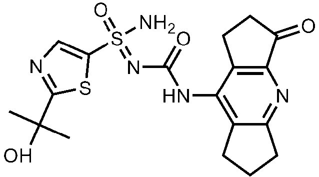 | 2-(2-Hydroxypropan-2-yl)-N'-((3-oxo-1,2,3,5,6,7-hexahydrodicyclopenta[b,e]pyridin-8-yl)carbamoyl)thiazole-5-sulfonimidamide | 436 |

**Table 56. Examples in the following table were prepared using similar conditions as described in Example 236 and Scheme II from appropriate starting materials.**

| Example # | Structure | IUPAC Name | Exact Mass [M+H]⁺ |
|---|---|---|---|
| 594 | 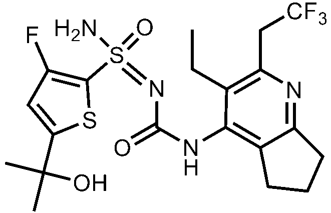 | N'-((3-ethyl-2-(2,2,2-trifluoroethyl)-6,7-dihydro-5H-cyclopenta[b]pyridin-4-yl)carbamoyl)-3-fluoro-5-(2-hydroxypropan-2-yl)thiophene-2-sulfonimidamide | 509 |
| 595 | 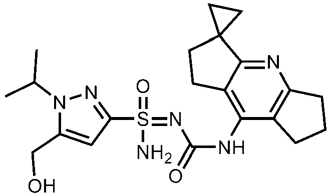 | 5-(Hydroxymethyl)-1-isopropyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 445 |
| 596 | 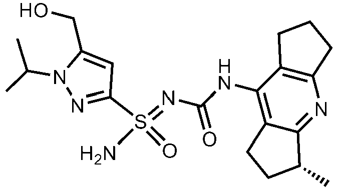 | 5-(Hydroxymethyl)-1-isopropyl-N'-(((R)-3-methyl-1,2,3,5,6,7-hexahydrodicyclopenta[b,e]pyridin-8-yl)carbamoyl)-1H-pyrazole-3-sulfonimidamide | 433 |
| 597 | 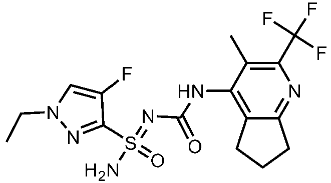 | 1-Ethyl-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | 435 |
| 598 | 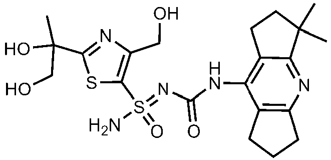 | 2-(1,2-Dihydroxypropan-2-yl)-N'-((3,3-dimethyl-1,2,3,5,6,7-hexahydrodicyclopenta[b,e]pyridin-8-yl)carbamoyl)-4-(hydroxymethyl)thiazole-5-sulfonimidamide | 496 |
| 599 | 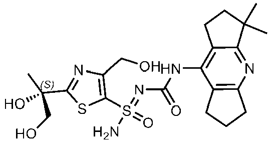 | 2-((S)-1,2-dihydroxypropan-2-yl)-N'-((3,3-dimethyl-1,2,3,5,6,7-hexahydrodicyclopenta[b,e]pyridin-8-yl)carbamoyl)-4-(hydroxymethyl)thiazole-5-sulfonimidamide | 496 |
| 600 | 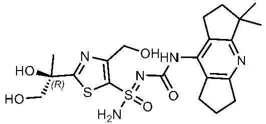 | 2-((R)-1,2-dihydroxypropan-2-yl)-N'-((3,3-dimethyl-1,2,3,5,6,7-hexahydrodicyclopenta[b,e]pyridin-8-yl)carbamoyl)-4-(hydroxymethyl)thiazole-5-sulfonimidamide | 496 |
| 601 | 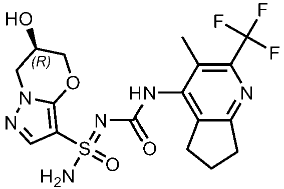 | (6R)-6-hydroxy-N'-((3-methyl-2-(trifluoromethyl)-6,7-dihydro-5H-cyclopenta[b]pyridin-4-yl)carbamoyl)-6,7-dihydro-5H-pyrazolo[5,1-b][1,3]oxazine-3-sulfonimidamide | 461 |
| 602 | 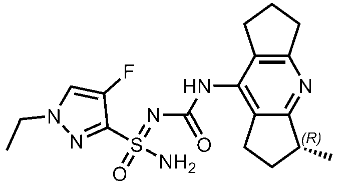 | 1-Ethyl-4-fluoro-N'-(((R)-3-methyl-1,2,3,5,6,7-hexahydrodicyclopenta[b,e]pyridin-8-yl)carbamoyl)-1H-pyrazole-3-sulfonimidamide | 407 |
| 603 | 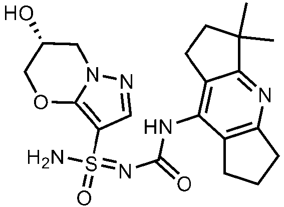 | (6R)-N'-((3,3-dimethyl-1,2,3,5,6,7-hexahydrodicyclopenta[b,e]pyridin-8-yl)carbamoyl)-6-hydroxy-6,7-dihydro-5H-pyrazolo[5,1-b][1,3]oxazine-3-sulfonimidamide | 447 |
| 604 | 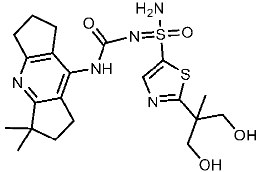 | 2-(1,3-Dihydroxy-2-methylpropan-2-yl)-N'-((3,3-dimethyl-1,2,3,5,6,7-hexahydrodicyclopenta[b,e]pyridin-8-yl)carbamoyl)thiazole-5-sulfonimidamide | 480 |
| 605 | 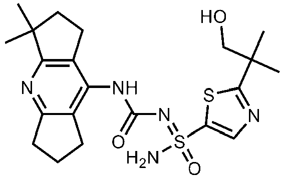 | N'-((3,3-dimethyl-1,2,3,5,6,7-hexahydrodicyclopenta[b,e]pyridin-8-yl)carbamoyl)-2-(1-hydroxy-2-methylpropan-2-yl)thiazole-5-sulfonimidamide | 464 |
| 606 | 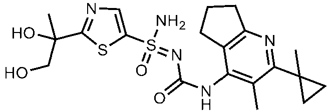 | 2-(1,2-Dihydroxypropan-2-yl)-N'-((3-methyl-2-(1-methylcyclopropyl)-6,7-dihydro-5H-cyclopenta[b]pyridin-4-yl)carbamoyl)thiazole-5-sulfonimidamide | 466 |
| 607 | 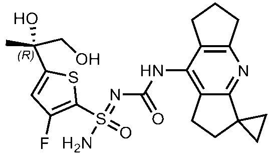 | 5-((R)-1,2-dihydroxypropan-2-yl)-3-fluoro-N'-((1',5',6',7'-tetrahydro-2'H-spiro[cyclopropane-1,3'-dicyclopenta[b,e]pyridin]-8'-yl)carbamoyl)thiophene-2-sulfonimidamide | 481 |
| 608 | 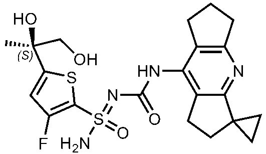 | 5-((S)-1,2-dihydroxypropan-2-yl)-3-fluoro-N'-((1',5',6',7'-tetrahydro-2'H-spiro[cyclopropane-1,3'-dicyclopenta[b,e]pyridin]-8'-yl)carbamoyl)thiophene-2-sulfonimidamide | 481 |

**Table 57. Examples in the following table were prepared using similar conditions as described in Example 252 and Scheme V from appropriate starting materials.**

| Example # | Structure | IUPAC Name | Exact Mass [M+H]⁺ |
|---|---|---|---|
| 609 | 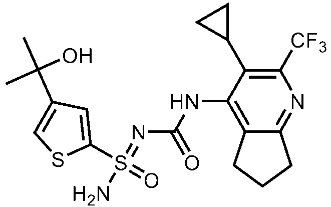 | N'-((3-cyclopropyl-2-(trifluoromethyl)-6,7-dihydro-5H-cyclopenta[b]pyridin-4-yl)carbamoyl)-4-(2-hydroxypropan-2-yl)thiophene-2-sulfonimidamide | 489 |
| 610 | 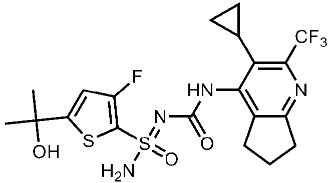 | N'-((3-cyclopropyl-2-(trifluoromethyl)-6,7-dihydro-5H-cyclopenta[b]pyridin-4-yl)carbamoyl)-3-fluoro-5-(2-hydroxypropan-2-yl)thiophene-2-sulfonimidamide | 507 |
| 611 | 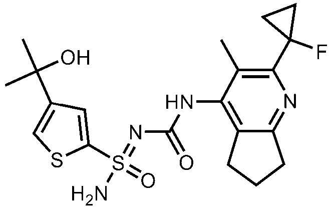 | N'-((2-(1-fluorocyclopropyl)-3-methyl-6,7-dihydro-5H-cyclopenta[b]pyridin-4-yl)carbamoyl)-4-(2-hydroxypropan-2-yl)thiophene-2-sulfonimidamide | 453 |
| 612 | 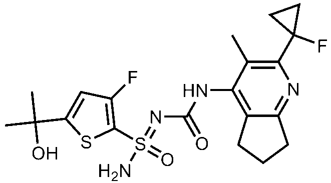 | 3-Fluoro-N'-((2-(1-fluorocyclopropyl)-3-methyl-6,7-dihydro-5H-cyclopenta[b]pyridin-4-yl)carbamoyl)-5-(2-hydroxypropan-2-yl)thiophene-2-sulfonimidamide | 471 |
| 613 | 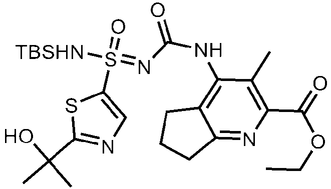 | Ethyl 4-(3-(((tert-butyldimethylsilyl)amino)(2-(2-hydroxypropan-2-yl)thiazol-5-yl)(oxo)-16-sulfaneylidene)ureido)-3-methyl-6,7-dihydro-5H-cyclopenta[b]pyridine-2-carboxylate | 582 |
| 614 | 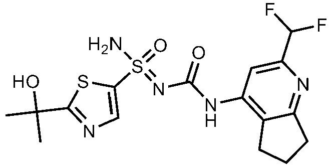 | N'-((2-(difluoromethyl)-6,7-dihydro-5H-cyclopenta[b]pyridin-4-yl)carbamoyl)-2-(2-hydroxypropan-2-yl)thiazole-5-sulfonimidamide | 432 |
| 615 | 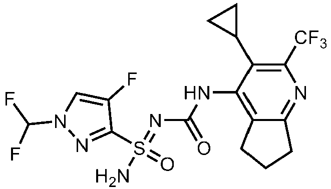 | N'-((3-cyclopropyl-2-(trifluoromethyl)-6,7-dihydro-5H-cyclopenta[b]pyridin-4-yl)carbamoyl)-1-(difluoromethyl)-4-fluoro-1H-pyrazole-3-sulfonimidamide | 483 |
| 616 | 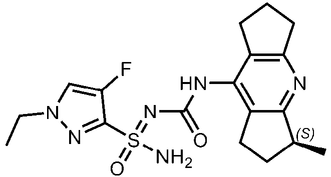 | 1-Ethyl-4-fluoro-N'-(((S)-3-methyl-1,2,3,5,6,7-hexahydrodicyclopenta[b,e]pyridin-8-yl)carbamoyl)-1H-pyrazole-3-sulfonimidamide | 407 |
| 617 | 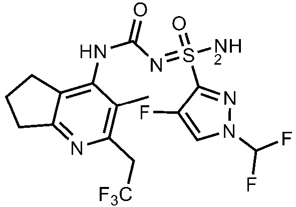 | 1-(Difluoromethyl)-4-fluoro-N'-((3-methyl-2-(2,2,2-trifluoroethyl)-6,7-dihydro-5H-cyclopenta[b]pyridin-4-yl)carbamoyl)-1H-pyrazole-3-sulfonimidamide | 471 |
| 618 | 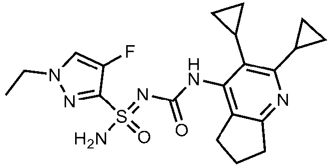 | N'-((2,3-dicyclopropyl-6,7-dihydro-5H-cyclopenta[b]pyridin-4-yl)carbamoyl)-1-ethyl-4-fluoro-1H-pyrazole-3-sulfonimidamide | 433 |
| 619 | 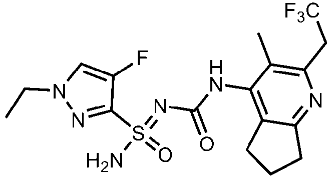 | 1-Ethyl-4-fluoro-N'-((3-methyl-2-(2,2,2-trifluoroethyl)-6,7-dihydro-5H-cyclopenta[b]pyridin-4-yl)carbamoyl)-1H-pyrazole-3-sulfonimidamide | 449 |
| 620 | 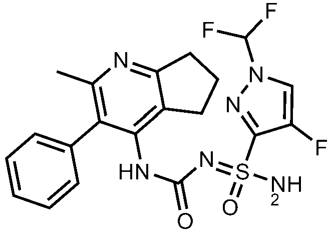 | 1-(Difluoromethyl)-4-fluoro-N'-((2-methyl-3-phenyl-6,7-dihydro-5H-cyclopenta[b]pyridin-4-yl)carbamoyl)-1H-pyrazole-3-sulfonimidamide | 465 |
| 621 | 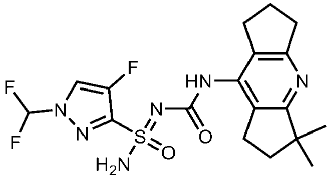 | 1-(Difluoromethyl)-N'-((3,3-dimethyl-1,2,3,5,6,7-hexahydrodicyclopenta[b,e]pyridin-8-yl)carbamoyl)-4-fluoro-1H-pyrazole-3-sulfonimidamide | 443 |

**Table 58 Examples in the following table were prepared using similar conditions as described in Example 9 and Scheme 2 from appropriate starting materials.**

| Example # | Structure | IUPAC Name | Exact Mass [M+H]⁺ |
|---|---|---|---|
| 622 | 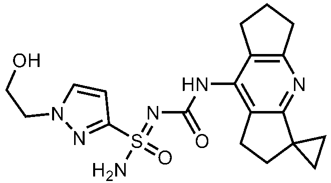 | 1-(2-Hydroxyethyl)-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 417 |
| 623 | 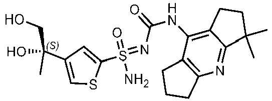 | 4-((S)-1,2-dihydroxypropan-2-yl)-N'-((3,3-dimethyl-1,2,3,5,6,7-hexahydrodicyclopenta[b,e]pyridin-8-yl)carbamoyl)thiophene-2-sulfonimidamide | 465 |
| 624 | 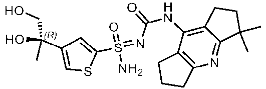 | 4-((R)-1,2-dihydroxypropan-2-yl)-N'-((3,3-dimethyl-1,2,3,5,6,7-hexahydrodicyclopenta[b,e]pyridin-8-yl)carbamoyl)thiophene-2-sulfonimidamide | 465 |
| 625 | 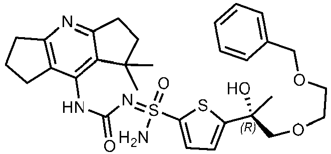 | 5-((R)-1-(2-(benzyloxy)ethoxy)-2-hydroxypropan-2-yl)-N'-((1,1-dimethyl-1,2,3,5,6,7-hexahydrodicyclopenta[b,e]pyridin-8-yl)carbamoyl)thiophene-2-sulfonimidamide | 599 |
| 626 | 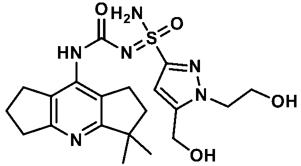 | N'-((3,3-dimethyl-1,2,3,5,6,7-hexahydrodicyclopenta[b,e]pyridin-8-yl)carbamoyl)-1-(2-hydroxyethyl)-5-(hydroxymethyl)-1H-pyrazole-3-sulfonimidamide | 449 |
| 627 | 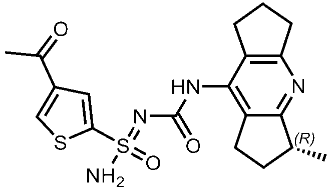 | 4-Acetyl-N'-(((R)-3-methyl-1,2,3,5,6,7-hexahydrodicyclopenta[b,e]pyridin-8-yl)carbamoyl)thiophene-2-sulfonimidamide | 419 |
| 628 | 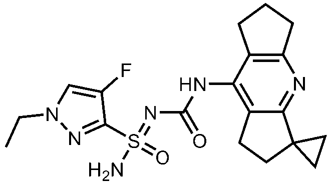 | 1-Ethyl-4-fluoro-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 419 |
| 629 | 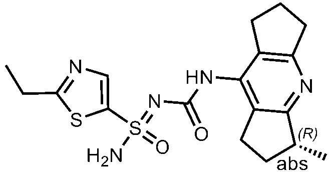 | 2-Ethyl-N'-(((R)-3-methyl-1,2,3,5,6,7-hexahydrodicyclopenta[b,e]pyridin-8-yl)carbamoyl)thiazole-5-sulfonimidamide | 406 |
| 630 | 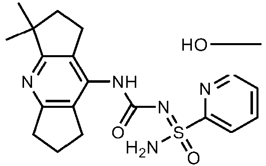 | N'-((3,3-dimethyl-1,2,3,5,6,7-hexahydrodicyclopenta[b,e]pyridin-8-yl)carbamoyl)-6-(2-hydroxypropan-2-yl)pyridine-2-sulfonimidamide | 444 |
| 631 | 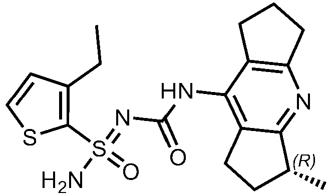 | 3-Ethyl-N'-(((R)-3-methyl-1,2,3,5,6,7-hexahydrodicyclopenta[b,e]pyridin-8-yl)carbamoyl)thiophene-2-sulfonimidamide | 404 |
| 632 | 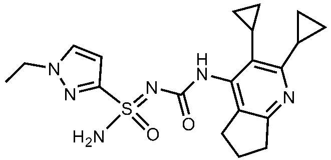 | N'-((2,3-dicyclopropyl-6,7-dihydro-5H-cyclopenta[b]pyridin-4-yl)carbamoyl)-1-ethyl-1H-pyrazole-3-sulfonimidamide | 415 |
| 633 | 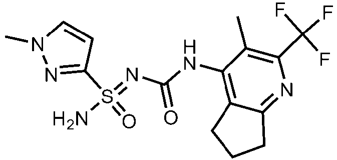 | 1-Methyl-N'-((3-methyl-2-(trifluoromethyl)-6,7-dihydro-5H-cyclopenta[b]pyridin-4-yl)carbamoyl)-1H-pyrazole-3-sulfonimidamide | 403 |
| 634 | 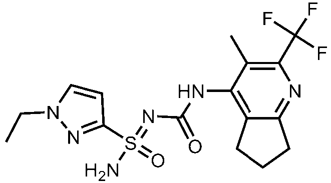 | 1-Ethyl-N'-((3-methyl-2-(trifluoromethyl)-6,7-dihydro-5H-cyclopenta[b]pyridin-4-yl)carbamoyl)-1H-pyrazole-3-sulfonimidamide | 417 |
| 635 | 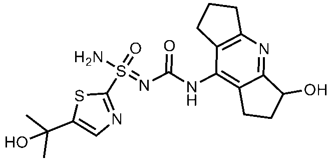 | N'-((3-hydroxy-1,2,3,5,6,7-hexahydrodicyclopenta[b,e]pyridin-8-yl)carbamoyl)-5-(2-hydroxypropan-2-yl)thiazole-2-sulfonimidamide | 438 |
| 636 | 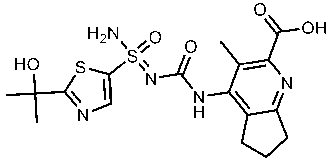 | 4-(3-(Amino(2-(2-hydroxypropan-2-yl)thiazol-5-yl)(oxo)-16-sulfaneylidene)ureido)-3-methyl-6,7-dihydro-5H-cyclopenta[b]pyridine-2-carboxylic acid | 440 |
| 637 | 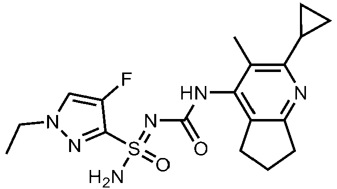 | N'-((2-cyclopropyl-3-methyl-6,7-dihydro-5H-cyclopenta[b]pyridin-4-yl)carbamoyl)-1-ethyl-4-fluoro-1H-pyrazole-3-sulfonimidamide | 407 |
| 638 | 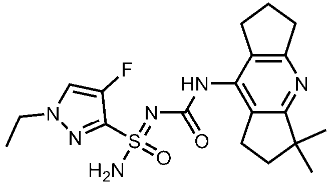 | N'-((3,3-dimethyl-1,2,3,5,6,7-hexahydrodicyclopenta[b,e]pyridin-8-yl)carbamoyl)-1-ethyl-4-fluoro-1H-pyrazole-3-sulfonimidamide | 421 |
| 854 | 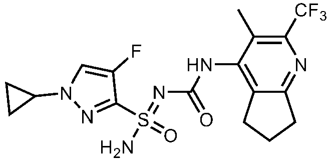 | 1-Cyclopropyl-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | 447 |
| 855 | 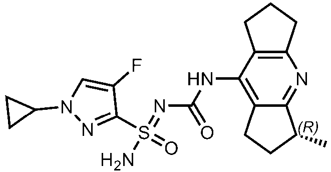 | 1-Cyclopropyl-4-fluoro-N'-(((R)-3-methyl-1,2,3,5,6,7-hexahydrodicyclopenta[b,e]pyridi n-8-yl)carbamoyl)-1H-pyrazole-3-sulfonimidamide | 419 |
| 856 | 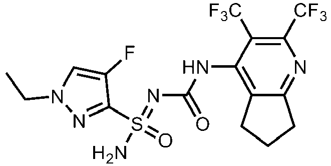 | N'-((2,3-bis(trifluoromethyl)-6,7-dihydro-5H-cyclopenta[b]pyridin-4-yl)carbamoyl)-1-ethyl-4-fluoro-1H-pyrazole-3-sulfonimidamide | 489 |
| 857 | 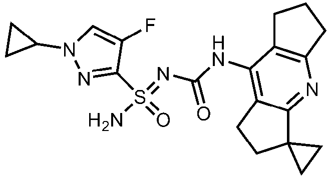 | 1-Cyclopropyl-4-fluoro-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 431 |
| 858 | 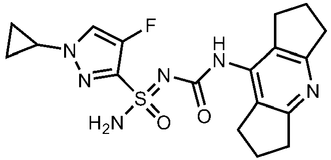 | 1-Cyclopropyl-4-fluoro-N'-((1,2,3,5,6,7-hexahydrodicyclopenta[b,e]pyridi n-8-yl)carbamoyl)-1H-pyrazole-3-sulfonimidamide | 405 |
| 859 | 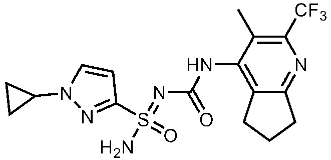 | 1-cyclopropyl-N'-((3-methyl-2-(trifluoromethyl)-6,7-dihydro-5H-cyclopenta[b]pyridin-4-yl)carbamoyl)-1H-pyrazole-3-sulfonimidamide | 429 |
| 860 | 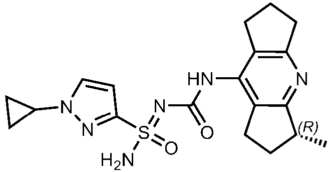 | 1-Cyclopropyl-N'-(((R)-3-methyl-1,2,3,5,6,7-hexahydrodicyclopenta[b,e]pyridi n-8-yl)carbamoyl)-1H-pyrazole-3-sulfonimidamide | 401 |
| 861 | 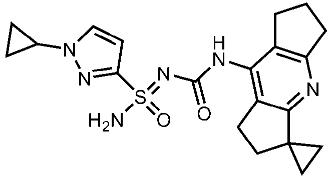 | 1-Cyclopropyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 413 |
| 862 | 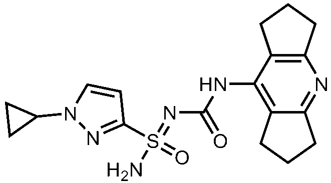 | 1-Cyclopropyl-N'-((1,2,3,5,6,7-hexahydrodicyclopenta[b,e]pyridi n-8-yl)carbamoyl)-1H-pyrazole-3-sulfonimidamide | 387 |

**Table 59 Examples in the following table were obtained from chiral HPLC resolutions of racemic and diastereomeric mixture examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. Assigned stereochemistry in compound names are tentative. Some carbon stereocenters were assigned arbitrarily for registration purpose, such as the alcohol centers for Examples 661~664.**

| Ex. # | Structure | IUPAC Name | Column | Eluents | LC-MS [M+H]⁺ |
|---|---|---|---|---|---|
| 639 | 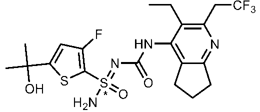 | (R) or (S)-N'-((3-ethyl-2-(2,2,2-trifluoroethyl)-6,7-dihydro-5H-cyclopenta[b]pyridin-4-yl)carbamoyl)-3-fluoro-5-(2-hydroxypropan-2-yl)thiophene-2-sulfonimidamide | CHIRALPA K IA, 4.6*50 mm; 3 um | 20% EtOH in Hex (0.1% FA) | 509 |
| 640 | 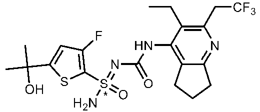 | (S) or (R)-N'-((3-ethyl-2-(2,2,2-trifluoroethyl)-6,7-dihydro-5H-cyclopenta[b]pyridin-4-yl)carbamoyl)-3-fluoro-5-(2-hydroxypropan-2-yl)thiophene-2-sulfonimidamide | CHIRALPA K IA, 4.6*50 mm; 3 um | 20% EtOH in Hex (0.1% FA) | 509 |
| 641 | 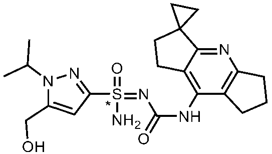 | (R) or (S)-5-(hydroxymethyl)-1-isopropyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IC, 2*25 cm, 5 um | 50% EtOH in Hex (0.1% FA) | 445 |
| 642 | 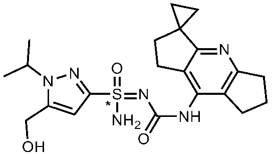 | (S) or (R)-5-(hydroxymethyl)-1-isopropyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IC, 2*25 cm, 5 um | 50% EtOH in Hex (0.1% FA) | 445 |
| 643 | 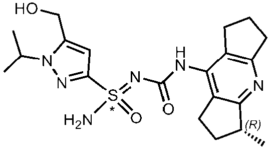 | (R) or (S)-5-(hydroxymethyl)-1-isopropyl-N'-(((R)-3-methyl-1,2,3,5,6,7-hexahydrodicyclopenta[b,e]pyridin-8-yl)carbamoyl)-1H-pyrazole-3-sulfonimidamide | CHIRALPA K IC, 2*25 cm, 5 um | 30% EtOH in Hex (0.1% FA) | 433 |
| 644 | 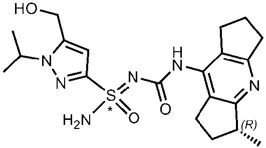 | (S) or (R)-5-(hydroxymethyl)-1-isopropyl-N'-(((R)-3-methyl-1,2,3,5,6,7-hexahydrodicyclopenta[b,e]pyridin-8-yl)carbamoyl)-1H-pyrazole-3-sulfonimidamide | CHIRALPA K IC, 2*25 cm, 5 um | 30% EtOH in Hex (0.1% FA) | 433 |
| 645 | 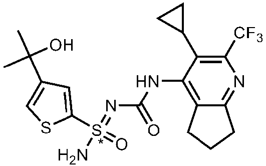 | (S) or (R)-N'-((3-cyclopropyl-2-(trifluoromethyl)-6,7-dihydro-5H-cyclopenta[b]pyridin-4-yl)carbamoyl)-4-(2-hydroxypropan-2-yl)thiophene-2-sulfonimidamide | CHIRALPA K IC, 2*25 cm, 5 um | 30% EtOH in Hex (0.2% DEA) | 489 |
| 646 | 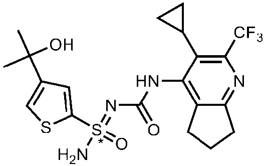 | (R) or (S)-N'-((3-cyclopropyl-2-(trifluoromethyl)-6,7-dihydro-5H-cyclopenta[b]pyridin-4-yl)carbamoyl)-4-(2-hydroxypropan-2-yl)thiophene-2-sulfonimidamide | CHIRALPA K IC, 2*25 cm, 5 um | 30% EtOH in Hex (0.2% DEA) | 489 |
| 647 | 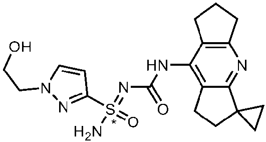 | (R) or (S)-1-(2-hydroxyethyl)-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IC, 2*25 cm, 5 um | 50% EtOH in Hex (0.1% FA) | 417 |
| 648 | 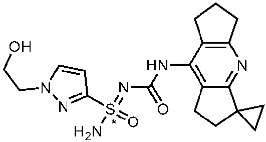 | (S) or (R)-1-(2-hydroxyethyl)-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IC, 2*25 cm, 5 um | 50% EtOH in Hex (0.1% FA) | 417 |
| 649 | 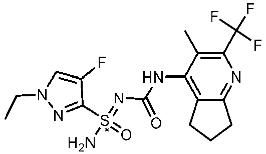 | (R) or (S)-1-ethyl-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K IE, 2*25 cm, 5 um; | 30% EtOH in Hex (0.1% FA) | 435 |
| 650 | 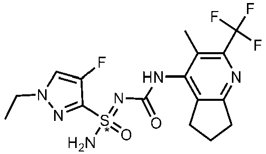 | (S) or (R)-1-ethyl-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K IE, 2*25 cm, 5 um; | 30% EtOH in Hex (0.1% FA) | 435 |
| 651 | 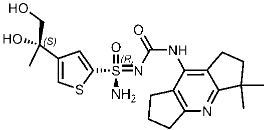 | (R)-4-((S)-1,2-dihydroxypropan-2-yl)-N'-((3,3-dimethyl-1,2,3,5,6,7-hexahydrodicyclopenta[b,e]pyridin-8-yl)carbamoyl)thiophene-2-sulfonimidamide | CHIRALPA K IG, 2*25 cm, 5 um | 30% EtOH in Hex (8 mM NH₃·MeOH) | 465 |
| 652 | 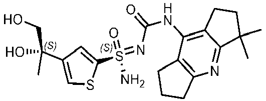 | (S)-4-((S)-1,2-dihydroxypropan-2-yl)-N'-((3,3-dimethyl-1,2,3,5,6,7-hexahydrodicyclopenta[b,e]pyridin-8-yl)carbamoyl)thiophene-2-sulfonimidamide | CHIRALPA K IG, 2*25 cm, 5 um | 30% EtOH in Hex (8 mM NH₃·MeOH) | 465 |
| 653 | 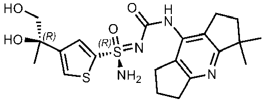 | (R)-4-((R)-1,2-dihydroxypropan-2-yl)-N'-((3,3-dimethyl-1,2,3,5,6,7-hexahydrodicyclopenta[b,e]pyridin-8-yl)carbamoyl)thiophene-2-sulfonimidamide | CHIRALPA K IG, 4.6*50mm,3 um | 30% EtOH in Hex (0.1% FA) | 465 |
| 654 | 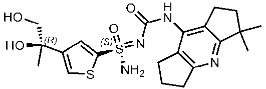 | (S)-4-((R)-1,2-dihydroxypropan-2-yl)-N'-((3,3-dimethyl-1,2,3,5,6,7-hexahydrodicyclopenta[b,e]pyridin-8-yl)carbamoyl)thiophene-2-sulfonimidamide | CHIRALPA K IG, 4.6*50mm,3 um | 30% EtOH in Hex (0.1% FA) | 465 |
| 655 | 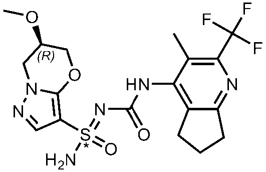 | (S) or (R)-(6R)-6-methoxy-N'-((3-methyl-2-(trifluoromethyl)-6,7-dihydro-5H-cyclopenta[b]pyridin-4-yl)carbamoyl)-6,7-dihydro-5H-pyrazolo[5,1-b][1,3]oxazine-3-sulfonimidamide | CHIRALPA K IE, 2*25 cm, 5 um | 50% EtOH in Hex (0.1% FA) | 475 |
| 656 | 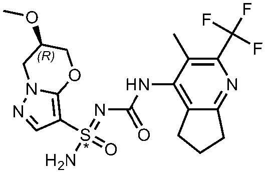 | (R) or (S)-(6R)-6-methoxy-N'-((3-methyl-2-(trifluoromethyl)-6,7-dihydro-5H-cyclopenta[b]pyridin-4-yl)carbamoyl)-6,7-dihydro-5H-pyrazolo[5,1-b][1,3]oxazine-3-sulfonimidamide | CHIRALPA K IE, 2*25 cm, 5 um | 50% EtOH in Hex (0.1% FA) | 475 |
| 657 | 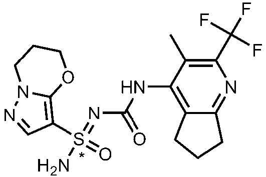 | (R) or (S)-N'-((3-methyl-2-(trifluoromethyl)-6,7-dihydro-5H-cyclopenta[b]pyridin-4-yl)carbamoyl)-6,7-dihydro-5H-pyrazolo[5,1-b][1,3]oxazine-3-sulfonimidamide | CHIRALPA K IC, 2*25 cm, 5 um | 40% EtOH in Hex (0.1% FA) | 445 |
| 658 | 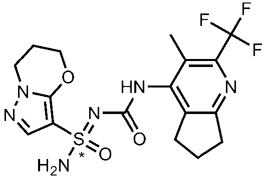 | (S) or (R)-N'-((3-methyl-2-(trifluoromethyl)-6,7-dihydro-5H-cyclopenta[b]pyridin-4-yl)carbamoyl)-6,7-dihydro-5H-pyrazolo[5,1-b][1,3]oxazine-3-sulfonimidamide | CHIRALPA K IC, 2*25 cm, 5 um | 40% EtOH in Hex (0.1% FA) | 445 |
| 659 | 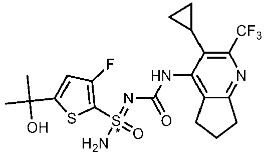 | (R) or (S)-N'-((3-cyclopropyl-2-(trifluoromethyl)-6,7-dihydro-5H-cyclopenta[b]pyridin-4-yl)carbamoyl)-3-fluoro-5-(2-hydroxypropan-2-yl)thiophene-2-sulfonimidamide | CHIRALPA K IF, 2*25 cm, 5 um | 30% EtOH in Hex (0.1% FA) | 507 |
| 660 | 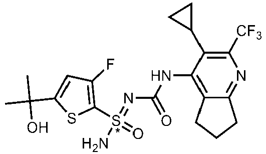 | (S) or (R)-N'-((3-cyclopropyl-2-(trifluoromethyl)-6,7-dihydro-5H-cyclopenta[b]pyridin-4-yl)carbamoyl)-3-fluoro-5-(2-hydroxypropan-2-yl)thiophene-2-sulfonimidamide | CHIRALPA K IF, 2*25 cm, 5 um | 30% EtOH in Hex (0.1% FA) | 507 |
| 661 | 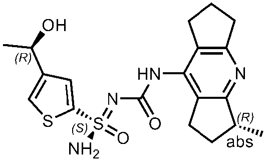 | (S)-4-((R) or (S)-1-hydroxyethyl)-N'-(((R)-3-methyl-1,2,3,5,6,7-hexahydrodicyclopenta[b,e]pyridin-8-yl)carbamoyl)thiophene-2-sulfonimidamide | CHIRAL ART Cellulose-SB, 2*25 cm, 5 um | 35%MeOH (8 mM NH₃·MeOH) in CO₂ | 421 |
| 662 | 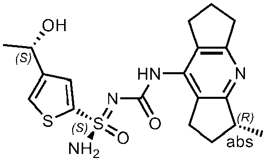 | (S)-4-((S) or (R)-1-hydroxyethyl)-N'-(((R)-3-methyl-1,2,3,5,6,7-hexahydrodicyclopenta[b,e]pyridin-8-yl)carbamoyl)thiophene-2-sulfonimidamide | CHIRAL ART Cellulose-SB, 2*25 cm, 5 um | 35%MeOH (8 mM NH₃·MeOH) in CO₂ | 421 |
| 663 | 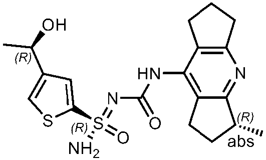 | (R)-4-((R) or (S)-1-hydroxyethyl)-N'-(((R)-3-methyl-1,2,3,5,6,7-hexahydrodicyclopenta[b,e]pyridin-8-yl)carbamoyl)thiophene-2-sulfonimidamide | Reg-AD, 3*25 cm, 5 um | 45% MeOH (8 mM NH₃·MeOH) in CO₂ | 421 |
| 664 | 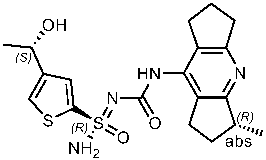 | (R)-4-((S) or (R)-1-hydroxyethyl)-N'-(((R)-3-methyl-1,2,3,5,6,7-hexahydrodicyclopenta[b,e]pyridin-8-yl)carbamoyl)thiophene-2-sulfonimidamide | Reg-AD, 3*25 cm, 5 um | 45% MeOH (8 mM NH₃·MeOH) in CO₂ | 421 |
| 665 | 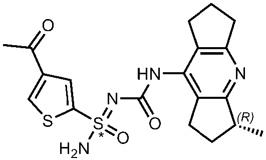 | (R) or (S)-4-acetyl-N'-(((R)-3-methyl-1,2,3,5,6,7-hexahydrodicyclopenta[b,e]pyridin-8-yl)carbamoyl)thiophene-2-sulfonimidamide | CHIRALPA K IC, 2*25 cm, 5 um | 40%MeOH (8 mM NH₃·MeOH) in CO₂ | 419 |
| 666 | 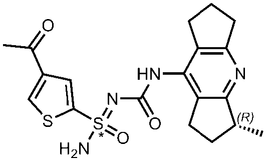 | (S) or (R)-4-acetyl-N'-(((R)-3-methyl-1,2,3,5,6,7-hexahydrodicyclopenta[b,e]pyridin-8-yl)carbamoyl)thiophene-2-sulfonimidamide | CHIRALPA K IC, 2*25 cm, 5 um | 40%MeOH (8 mM NH₃·MeOH) in CO₂ | 419 |
| 667 | 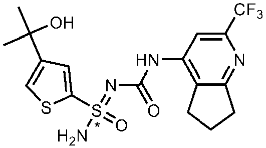 | (R) or (S)-4-(2-hydroxypropan-2-yl)-N'-((2-(trifluoromethyl)-6,7-dihydro-5H-cyclopenta[b]pyridin-4-yl)carbamoyl)thiophene-2-sulfonimidamide | Chiralpak IC, 2*25 cm, 5 um | 15% EtOH in Hex (0.1% FA) | 449 |
| 668 | 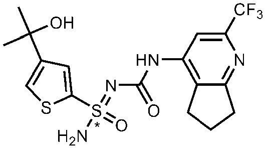 | (S) or (R)-4-(2-hydroxypropan-2-yl)-N'-((2-(trifluoromethyl)-6,7-dihydro-5H-cyclopenta[b]pyridin-4-yl)carbamoyl)thiophene-2-sulfonimidamide | Chiralpak IC, 2*25 cm, 5 um | 15% EtOH in Hex (0.1% FA) | 449 |
| 669 | 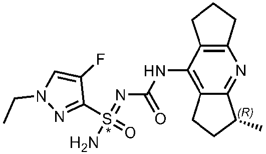 | (R) or (S)-1-ethyl-4-fluoro-N'-(((R)-3-methyl-1,2,3,5,6,7-hexahydrodicyclopenta[b,e]pyridin-8-yl)carbamoyl)-1H-pyrazole-3-sulfonimidamide | CHIRALPA K IG, 2*25 cm, 5 um | 50% EtOH in Hex (0.1% FA) | 407 |
| 670 | 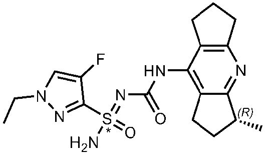 | (S) or (R)-1-ethyl-4-fluoro-N'-(((R)-3-methyl-1,2,3,5,6,7-hexahydrodicyclopenta[b,e]pyridin-8-yl)carbamoyl)-1H-pyrazole-3-sulfonimidamide | CHIRALPA K IG, 2*25 cm, 5 um | 50% EtOH in Hex (0.1% FA) | 407 |
| 671 | 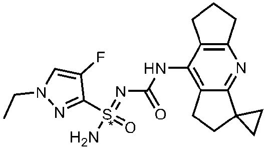 | (R) or (S)-1-ethyl-4-fluoro-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IC, 2*25 cm, 5 um | 50% EtOH in Hex (0.1% FA) | 419 |
| 672 | 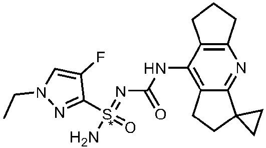 | (S) or (R)-1-ethyl-4-fluoro-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IC, 2*25 cm, 5 um | 50% EtOH in Hex (0.1% FA) | 419 |
| 673 | 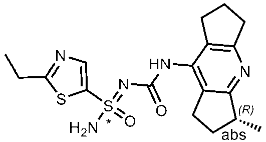 | (S) or (R)-2-ethyl-N'-(((R)-3-methyl-1,2,3,5,6,7-hexahydrodicyclopenta[b,e]pyridin-8-yl)carbamoyl)thiazole-5-sulfonimidamide | CHIRALPA K IE, 3*25 cm, 5 um | 50% EtOH in Hex (0.1% FA) | 406 |
| 674 | 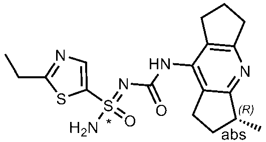 | (R) or (S)-2-ethyl-N'-(((R)-3-methyl-1,2,3,5,6,7-hexahydrodicyclopenta[b,e]pyridin-8-yl)carbamoyl)thiazole-5-sulfonimidamide | CHIRALPA K IE, 3*25 cm, 5 um | 50% EtOH in Hex (0.1% FA) | 406 |
| 675 | 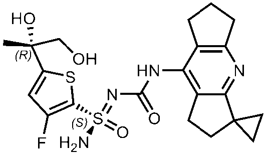 | (S)-5-((R)-1,2-dihydroxypropan-2-yl)-3-fluoro-N'-((1',5',6',7'-tetrahydro-2'H-spiro[cyclopropane-1,3'-dicyclopenta[b,e]pyridin]-8'-yl)carbamoyl)thiophene-2-sulfonimidamide | CHIRAL ART Cellulose-SB, 2*25 cm, 5 um | 30% EtOH in Hex (8 mM NH₃·MeOH) | 481 |
| 676 | 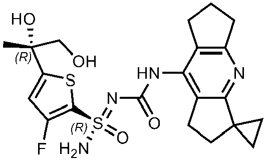 | (R)-5-((R)-1,2-dihydroxypropan-2-yl)-3-fluoro-N'-((1',5',6',7'-tetrahydro-2'H-spiro[cyclopropane-1,3'-dicyclopenta[b,e]pyridin]-8'-yl)carbamoyl)thiophene-2-sulfonimidamide | CHIRAL ART Cellulose-SB, 2*25 cm, 5 um | 30% EtOH in Hex (8 mM NH₃·MeOH) | 481 |
| 677 | 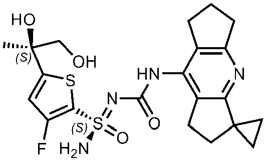 | (S)-5-((S)-1,2-dihydroxypropan-2-yl)-3-fluoro-N'-((1',5',6',7'-tetrahydro-2'H-spiro[cyclopropane-1,3'-dicyclopenta[b,e]pyridin]-8'-yl)carbamoyl)thiophene-2-sulfonimidamide | CHIRAL ART Cellulose-SB, 2*25 cm, 5 um | 30% EtOH in Hex (8 mM NH₃·MeOH) | 481 |
| 678 | 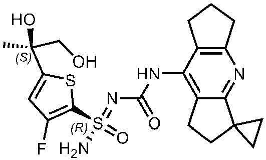 | (R)-5-((S)-1,2-dihydroxypropan-2-yl)-3-fluoro-N'-((1',5',6',7'-tetrahydro-2'H-spiro[cyclopropane-1,3'-dicyclopenta[b,e]pyridin]-8'-yl)carbamoyl)thiophene-2-sulfonimidamide | CHIRAL ART Cellulose-SB, 2*25 cm, 5 um | 30% EtOH in Hex (8 mM NH₃·MeOH) | 481 |
| 679 | 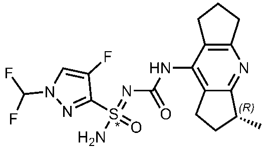 | (R) or (S)-1-(difluoromethyl)-4-fluoro-N'-(((R)-3-methyl-1,2,3,5,6,7-hexahydrodicyclopenta[b,e]pyridin-8-yl)carbamoyl)-1H-pyrazole-3-sulfonimidamide | CHIRALPA K IG, 4.6*50 mm, 3 um | 70% EtOH in Hex (0.1% FA) | 429 |
| 680 | 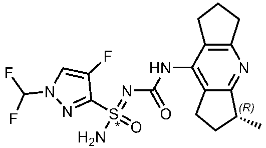 | (S) or (R)-1-(difluoromethyl)-4-fluoro-N'-(((R)-3-methyl-1,2,3,5,6,7-hexahydrodicyclopenta[b,e]pyridin-8-yl)carbamoyl)-1H-pyrazole-3-sulfonimidamide | CHIRALPA K IG, 4.6*50 mm, 3 um | 70% EtOH in Hex (0.1% FA) | 429 |
| 681 | 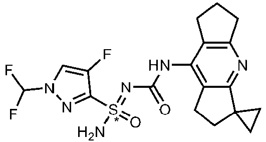 | (R) or (S)-1-(difluoromethyl)-4-fluoro-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IE, 2*25 cm, 5 um | 30% EtOH in Hex (0.1% FA) | 441 |
| 682 | 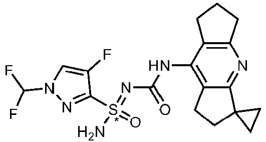 | (S) or (R)-1-(difluoromethyl)-4-fluoro-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IE, 2*25 cm, 5 um | 30% EtOH in Hex (0.1% FA) | 441 |
| 683 | 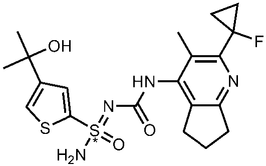 | (R) or (S)-N'-((2-(1-fluorocyclopropyl)-3-methyl-6,7-dihydro-5H-cyclopenta[b]pyridin-4-yl)carbamoyl)-4-(2-hydroxypropan-2-yl)thiophene-2-sulfonimidamide | CHIRALPA K IG, 4.6*50 mm, 3 um | 30% EtOH in Hex (0.1% FA) | 453 |
| 684 | 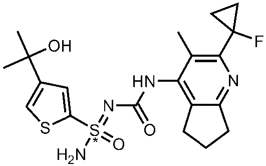 | (S) or (R)-N'-((2-(1-fluorocyclopropyl)-3-methyl-6,7-dihydro-5H-cyclopenta[b]pyridin-4-yl)carbamoyl)-4-(2-hydroxypropan-2-yl)thiophene-2-sulfonimidamide | CHIRALPA K IG, 4.6*50 mm, 3 um | 30% EtOH in Hex (0.1% FA) | 453 |
| 685 | 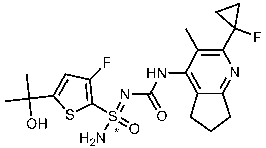 | (R) or (S)-3-fluoro-N'-((2-(1-fluorocyclopropyl)-3-methyl-6,7-dihydro-5H-cyclopenta[b]pyridin-4-yl)carbamoyl)-5-(2-hydroxypropan-2-yl)thiophene-2-sulfonimidamide | CHIRALPA K IE, 4.6*50 mm, 3 um | 30% EtOH in Hex (0.1% FA) | 471 |
| 686 | 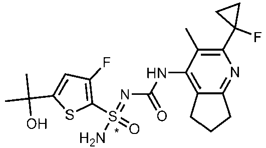 | (S) or (R)-3-fluoro-N'-((2-(1-fluorocyclopropyl)-3-methyl-6,7-dihydro-5H-cyclopenta[b]pyridin-4-yl)carbamoyl)-5-(2-hydroxypropan-2-yl)thiophene-2-sulfonimidamide | CHIRALPA K IE, 4.6*50 mm, 3 um | 30% EtOH in Hex (0.1% FA) | 471 |
| 687 | 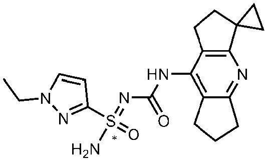 | (R) or (S)-1-Ethyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IC, 2*25 cm, 5 um | 50% EtOH in Hex (8 mM NH₃·MeOH) | 401 |
| 688 | 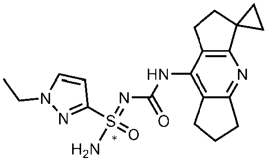 | (S) or (R)-1-Ethyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | CHIRALPA K IC, 2*25 cm, 5 um | 50% EtOH in Hex (8 mM NH₃·MeOH) | 401 |
| 689 | 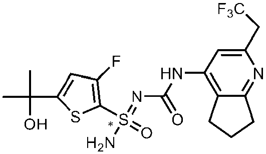 | (R) or (S)-3-Fluoro-5-(2-hydroxypropan-2-yl)-N'-((2-(2,2,2-trifluoroethyl)-6,7-dihydro-5H-cyclopenta[b]pyridin-4-yl)carbamoyl)thiophene-2-sulfonimidamide | Column: CHIRALPA K IC, 2*25 cm, 5 um | 20% EtOH in Hex (0.1% FA) | 481 |
| 690 | 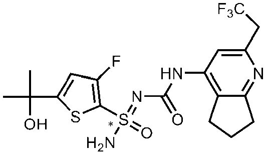 | (S) or (R)-3-Fluoro-5-(2-hydroxypropan-2-yl)-N'-((2-(2,2,2-trifluoroethyl)-6,7-dihydro-5H-cyclopenta[b]pyridin-4-yl)carbamoyl)thiophene-2-sulfonimidamide | CHIRALPA K IC, 2*25 cm, 5 um | 20% EtOH in Hex (0.1% FA) | 481 |
| 691 | 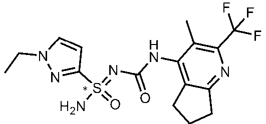 | (R) or (S)-1-Ethyl-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K ID, 2.0*25 cm, 5 um | 30% EtOH in Hex (0.1% FA) | 417 |
| 692 | 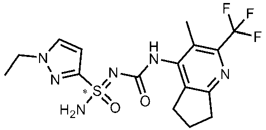 | (S) or (R)-1-Ethyl-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K ID, 2.0*25 cm, 5 um | 30% EtOH in Hex (0.1% FA) | 417 |
| 693 | 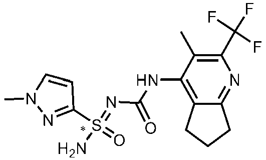 | (R) or (S)-1-Methyl-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K IE, 2*25 cm, 5 um | 30% EtOH in Hex (0.1% FA) | 403 |
| 694 | 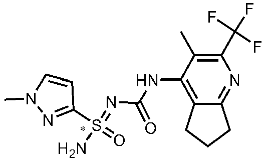 | (S) or (R)-1-Methyl-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K IE, 2*25 cm, 5 um | 30% EtOH in Hex (0.1% FA) | 403 |
| 695 | 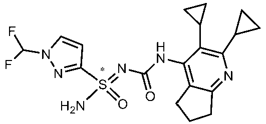 | (R) or (S)-N'-((2,3-dicyclopropyl-6,7-dihydro-5H-cyclopenta[b]pyridin-4-yl)carbamoyl)-1-(difluoromethyl)-1H-pyrazole-3-sulfonimidamide | CHIRALPA K ID, 2.0*25 cm, 5 um | 30% EtOH in Hex (0.1% FA) | 437 |
| 696 | 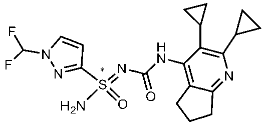 | (S) or (R)-N'-((2,3-dicyclopropyl-6,7-dihydro-5H-cyclopenta[b]pyridin-4-yl)carbamoyl)-1-(difluoromethyl)-1H-pyrazole-3-sulfonimidamide | CHIRALPA K ID, 2.0*25 cm, 5 um | 30% EtOH in Hex (0.1% FA) | 437 |
| 697 | 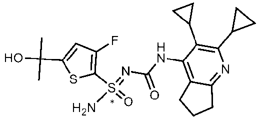 | (R) or (S)-N'-((2,3-dicyclopropyl-6,7-dihydro-5H-cyclopenta[b]pyridin-4-yl)carbamoyl)-3-fluoro-5-(2-hydroxypropan-2-yl)thiophene-2-sulfonimidamide | CHIRALPA K ID, 2.0*25 cm, 5 um | 50% IPA in Hex (0.1% FA) | 479 |
| 698 | 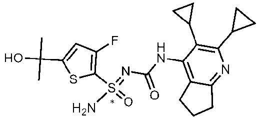 | (S) or (R)-N'-((2,3-dicyclopropyl-6,7-dihydro-5H-cyclopenta[b]pyridin-4-yl)carbamoyl)-3-fluoro-5-(2-hydroxypropan-2-yl)thiophene-2-sulfonimidamide | CHIRALPA K ID, 2.0*25 cm, 5 um | 50% IPA in Hex (0.1% FA) | 479 |
| 699 | 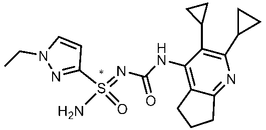 | (R) or (S)-N'-((2,3-dicyclopropyl-6,7-dihydro-5H-cyclopenta[b]pyridin-4-yl)carbamoyl)-1-ethyl-1H-pyrazole-3-sulfonimidamide | CHIRALPA K ID, 2*25 cm, 5 um | 30% EtOH in Hex:DCM=3 :1 (10 mM NH₃-MeOH) | 415 |
| 700 | 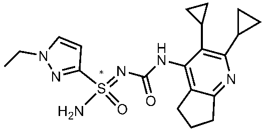 | (S) or (R)-N'-((2,3-dicyclopropyl-6,7-dihydro-5H-cyclopenta[b]pyridin-4-yl)carbamoyl)-1-ethyl-1H-pyrazole-3-sulfonimidamide | CHIRALPA K ID, 2*25 cm, 5 um | 30% EtOH in Hex:DCM=3 :1 (10 mM NH₃-MeOH) | 415 |
| 701 | 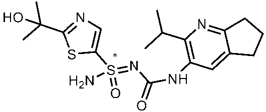 | (R) or (S)-2-(2-Hydroxypropan-2-yl)-N'-((2-isopropyl-6,7-dihydro-5H-cyclopenta[b]pyridin-3-yl)carbamoyl)thiazole-5-sulfonimidamide | CHIRALPA K IE, 2*25 cm, 5 um | 20% EtOH in Hex (0.1% FA) | 424 |
| 702 | 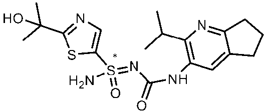 | (S) or (R)-2-(2-Hydroxypropan-2-yl)-N'-((2-isopropyl-6,7-dihydro-5H-cyclopenta[b]pyridin-3-yl)carbamoyl)thiazole-5-sulfonimidamide | CHIRALPA K IE, 2*25 cm, 5 um | 20% EtOH in Hex (0.1% FA) | 424 |
| 703 | 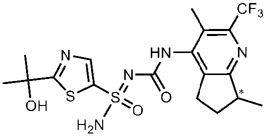 | (R/S, S) or (R/S, R)-N'-((3,7-dimethyl-2-(trifluoromethyl)-6,7-dihydro-5H-cyclopenta[b]pyridin-4-yl)carbamoyl)-2-(2-hydroxypropan-2-yl)thiazole-5-sulfonimidamide (from Example 273) | Chiralpak IA, 2*25 cm, 5 um | 10% EtOH in Hex (0.1% FA) | 478 |
| 704 | 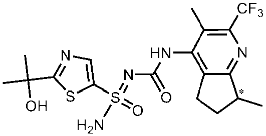 | (R/S, R) or (R/S, S)-N'-((3,7-dimethyl-2-(trifluoromethyl)-6,7-dihydro-5H-cyclopenta[b]pyridin-4-yl)carbamoyl)-2-(2-hydroxypropan-2-yl)thiazole-5-sulfonimidamide (from Example 273) | Chiralpak IA, 2*25 cm, 5 um | 10% EtOH in Hex (0.1% FA) | 478 |
| 705 | 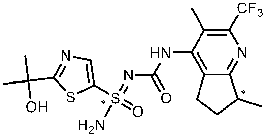 | (S, R) or (S, S)-N'-((3,7-dimethyl-2-(trifluoromethyl)-6,7-dihydro-5H-cyclopenta[b]pyridin-4-yl)carbamoyl)-2-(2-hydroxypropan-2-yl)thiazole-5-sulfonimidamide (from Example 704) | CHIRAL ART Cellulose-SB, 2*25 cm, 5 um | 20% EtOH in Hex (0.1% FA) | 478 |
| 706 | 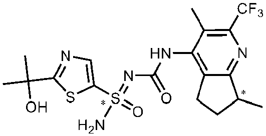 | (R, R) or (R, S)-N'-((3,7-dimethyl-2-(trifluoromethyl)-6,7-dihydro-5H-cyclopenta[b]pyridin-4-yl)carbamoyl)-2-(2-hydroxypropan-2-yl)thiazole-5-sulfonimidamide (from Example 704) | CHIRAL ART Cellulose-SB, 2*25 cm, 5 um | 20% EtOH in Hex (0.1% FA) | 478 |
| 707 | 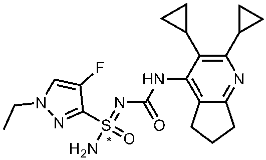 | (R) or (S)-N'-((2,3-dicyclopropyl-6,7-dihydro-5H-cyclopenta[b]pyridin-4-yl)carbamoyl)-1-ethyl-4-fluoro-1H-pyrazole-3-sulfonimidamide | Chiralpak ID, 2*25 cm, 5um | 30% EtOH in Hex (0.1% FA) | 433 |
| 708 | 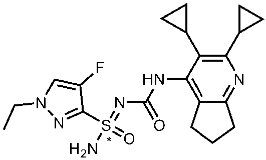 | (S) or (R)-N'-((2,3-dicyclopropyl-6,7-dihydro-5H-cyclopenta[b]pyridin-4-yl)carbamoyl)-1-ethyl-4-fluoro-1H-pyrazole-3-sulfonimidamide | Chiralpak ID, 2*25 cm, 5 um | 30% EtOH in Hex (0.1% FA) | 433 |
| 709 | 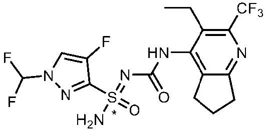 | (R) or (S)-1-(difluoromethyl)-N'-((3-ethyl-2-(trifluoromethyl)-6,7-dihydro-5H-cyclopenta[b]pyridin-4-yl)carbamoyl)-4-fluoro-1H-pyrazole-3-sulfonimidamide | Chiralpak ID, 2*25 cm, 5 um | 10% EtOH in Hex (0.1% FA) | 471 |
| 710 | 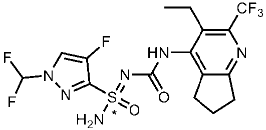 | (S) or (R)-1-(difluoromethyl)-N'-((3-ethyl-2-(trifluoromethyl)-6,7-dihydro-5H-cyclopenta[b]pyridin-4-yl)carbamoyl)-4-fluoro-1H-pyrazole-3-sulfonimidamide | Chiralpak ID, 2*25 cm, 5 um | 10% EtOH in Hex (0.1% FA) | 471 |
| 711 | 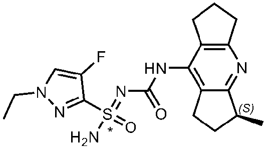 | (R) or (S)-1-ethyl-4-fluoro-N'-(((S)-3-methyl-1,2,3,5,6,7-hexahydrodicyclopenta[b,e]pyridin-8-yl)carbamoyl)-1H-pyrazole-3-sulfonimidamide | CHIRALPA K ID, 2*25 cm, 5 um | 50% IPA in Hex (0.1% FA) | 407 |
| 712 | 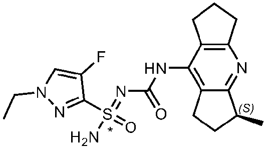 | (S) or (R)-1-ethyl-4-fluoro-N'-(((S)-3-methyl-1,2,3,5,6,7-hexahydrodicyclopenta[b,e]pyridin-8-yl)carbamoyl)-1H-pyrazole-3-sulfonimidamide | CHIRALPA K ID, 2*25 cm, 5 um | 50% IPA in Hex (0.1% FA) | 407 |
| 713 | 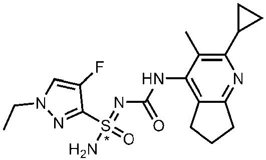 | (R) or (S)-N'-((2-cyclopropyl-3-methyl-6,7-dihydro-5H-cyclopenta[b]pyridin-4-yl)carbamoyl)-1-ethyl-4-fluoro-1H-pyrazole-3-sulfonimidamide | CHIRALPA K IE, 2*25 cm, 5 um | 40% EtOH in Hex (0.1% FA) | 407 |
| 714 | 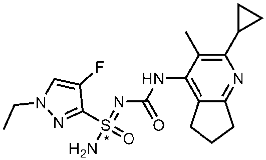 | (S) or (R)-N'-((2-cyclopropyl-3-methyl-6,7-dihydro-5H-cyclopenta[b]pyridin-4-yl)carbamoyl)-1-ethyl-4-fluoro-1H-pyrazole-3-sulfonimidamide | CHIRALPA K IE, 2*25 cm, 5 um | 40% EtOH in Hex (0.1% FA) | 407 |
| 715 | 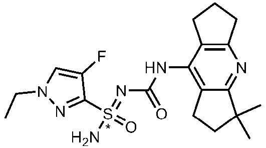 | (R) or (S)-N'-((3,3-dimethyl-1,2,3,5,6,7-hexahydrodicyclopenta[b,e]pyridin-8-yl)carbamoyl)-1-ethyl-4-fluoro-1H-pyrazole-3-sulfonimidamide | CHIRALPA K IE, 2*25 cm, 5 um | 40% EtOH in Hex (0.1% FA) | 421 |
| 716 | 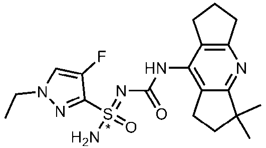 | (S) or (R)-N'-((3,3-dimethyl-1,2,3,5,6,7-hexahydrodicyclopenta[b,e]pyridin-8-yl)carbamoyl)-1-ethyl-4-fluoro-1H-pyrazole-3-sulfonimidamide | CHIRALPA K IE, 2*25 cm, 5 um | 40% EtOH in Hex (0.1% FA) | 421 |
| 717 | 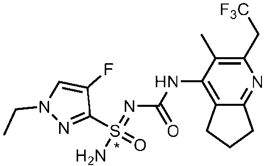 | (R) or (S)-1-ethyl-4-fluoro-N'-((3-methyl-2-(2,2,2-trifluoroethyl)-6,7-dihydro-5H-cyclopenta[b]pyridin-4-yl)carbamoyl)-1H-pyrazole-3-sulfonimidamide | CHIRALPA K IE, 2*25 cm, 5 um | 50% IPA in Hex (0.1% FA) | 449 |
| 718 | 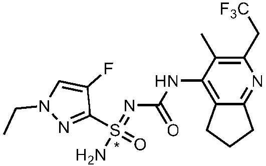 | (S) or (R)-1-ethyl-4-fluoro-N'-((3-methyl-2-(2,2,2-trifluoroethyl)-6,7-dihydro-5H-cyclopenta[b]pyridin-4-yl)carbamoyl)-1H-pyrazole-3-sulfonimidamide | CHIRALPA K IE, 2*25 cm, 5 um | 50% IPA in Hex (0.1% FA) | 449 |
| 719 | 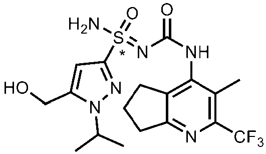 | (S) or (R)-5-(hydroxymethyl)-1-isopropyl-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K IC, 4.6*50 mm, 3 um | 15% EtOH in Hex (0.1% FA) | 461 |
| 720 | 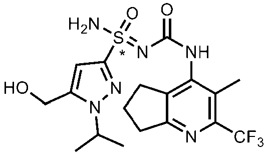 | (R) or (S)-5-(hydroxymethyl)-1-isopropyl-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K IC, 4.6*50 mm, 3 um | 15% EtOH in Hex (0.1% FA) | 461 |
| 721 | 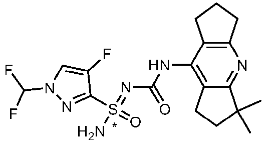 | (R) or (S)-1-(difluoromethyl)-N'-((3,3-dimethyl-1,2,3,5,6,7-hexahydrodicyclopenta[b,e]pyridin-8-yl)carbamoyl)-4-fluoro-1H-pyrazole-3-sulfonimidamide | CHIRALPA K IG, 2*25 cm, 5 um | 15% EtOH in Hex (0.1% FA) | 443 |
| 722 | 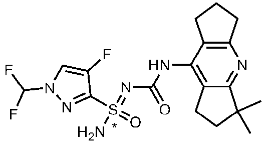 | (S) or (R)-1-(difluoromethyl)-N'-((3,3-dimethyl-1,2,3,5,6,7-hexahydrodicyclopenta[b,e]pyridin-8-yl)carbamoyl)-4-fluoro-1H-pyrazole-3-sulfonimidamide | CHIRALPA K IG, 2*25 cm, 5 um | 15% EtOH in Hex (0.1% FA) | 443 |
| 723 | 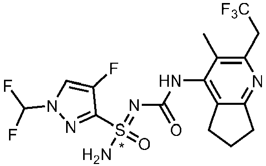 | (R) or (S)-1-(difluoromethyl)-4-fluoro-N'-((3-methyl-2-(2,2,2-trifluoroethyl)-6,7-dihydro-5H-cyclopenta[b]pyridin-4-yl)carbamoyl)-1H-pyrazole-3-sulfonimidamide | CHIRALPA K IE, 2*25 cm, 5 um | 20% EtOH in Hex (0.1% FA) | 471 |
| 724 | 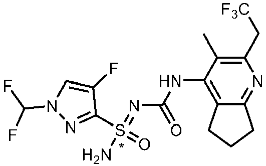 | (S) or (R)-1-(difluoromethyl)-4-fluoro-N'-((3-methyl-2-(2,2,2-trifluoroethyl)-6,7-dihydro-5H-cyclopenta[b]pyridin-4-yl)carbamoyl)-1H-pyrazole-3-sulfonimidamide | CHIRALPA K IE, 2*25 cm, 5 um | 20% EtOH in Hex (0.1% FA) | 471 |
| 725 | 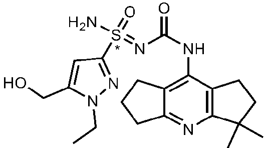 | (S) or (R)-N'-((3,3-dimethyl-1,2,3,5,6,7-hexahydrodicyclopenta[b,e]pyridin-8-yl)carbamoyl)-1-ethyl-5-(hydroxymethyl)-1H-pyrazole-3-sulfonimidamide | CHIRALPA K AS, 2*25 cm, 5 um | 20% EtOH (8 mM NH₃·MeOH) in CO₂ | 433 |
| 726 | 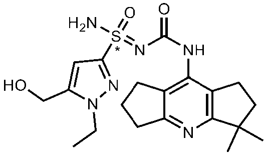 | (R) or (S)-N'-((3,3-dimethyl-1,2,3,5,6,7-hexahydrodicyclopenta[b,e]pyridin-8-yl)carbamoyl)-1-ethyl-5-(hydroxymethyl)-1H-pyrazole-3-sulfonimidamide | CHIRALPA K AS, 2*25 cm, 5 um | 20% EtOH (8 mM NH₃·MeOH) in CO₂ | 433 |
| 727 | 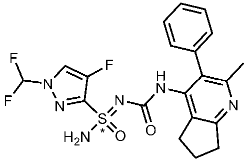 | (R) or (S)-1-(difluoromethyl)-4-fluoro-N'-((2-methyl-3-phenyl-6,7-dihydro-5H-cyclopenta[b]pyridin-4-yl)carbamoyl)-1H-pyrazole-3-sulfonimidamide | CHIRALPA K IE, 2*25 cm, 5 um | 30% EtOH in Hex (0.1% FA) | 465 |
| 728 | 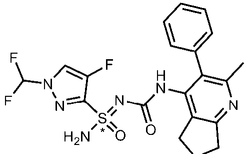 | (S) or (R)-1-(difluoromethyl)-4-fluoro-N'-((2-methyl-3-phenyl-6,7-dihydro-5H-cyclopenta[b]pyridin-4-yl)carbamoyl)-1H-pyrazole-3-sulfonimidamide | CHIRALPA K IE, 2*25 cm, 5 um | 30% EtOH in Hex (0.1% FA) | 465 |

**Compounds 908a, 952a, 983a** through **984,** and **986** through **1041a** in **Table 1E** can be prepared using one or more methods used to synthesize **Examples 1-728.**

**Table 64 Examples in the following table were prepared using similar conditions as described in Example 585 and Scheme XVI from appropriate starting materials. The carbon stereocenters of tertiary alcohol at thiazole for Examples 729~732 were assigned arbitrarily for compound registration purpose.**

| Example # | Structure | IUPAC Name | Exact Mass [M+H]⁺ |
|---|---|---|---|
| 729 | 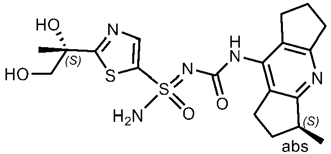 | 2-((S)-1,2-dihydroxypropan-2-yl)-N'-(((S)-3-methyl-1,2,3,5,6,7-hexahydrodicyclopenta[b,e]pyridin-8-yl)carbamoyl)thiazole-5-sulfonimidamide (from chiral Intermediate 117A and Intermediate 262) | 452 |
| 730 | 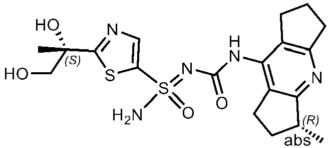 | 2-((S)-1,2-dihydroxypropan-2-yl)-N'-(((R)-3-methyl-1,2,3,5,6,7-hexahydrodicyclopenta[b,e]pyridin-8-yl)carbamoyl)thiazole-5-sulfonimidamide (from chiral Intermediate 117A and Intermediate 161) | 452 |
| 731 | 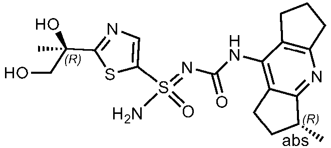 | 2-((R)-1,2-dihydroxypropan-2-yl)-N'-(((R)-3-methyl-1,2,3,5,6,7-hexahydrodicyclopenta[b,e]pyridin-8-yl)carbamoyl)thiazole-5-sulfonimidamide (from chiral Intermediate 117B and Intermediate 161) | 452 |
| 732 | 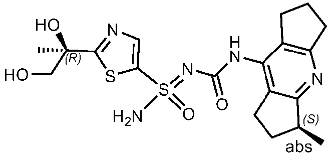 | 2-((R)-1,2-dihydroxypropan-2-yl)-N'-(((S)-3-methyl-1,2,3,5,6,7-hexahydrodicyclopenta[b,e]pyridin-8-yl)carbamoyl)thiazole-5-sulfonimidamide (from chiral Intermediate 117B and Intermediate 262) | 452 |

**Table 65. Examples in the following table were prepared using similar conditions as described in Example 252 and Scheme V from appropriate starting materials.**

| Example # | Structure | IUPAC Name | Exact Mass [M+H]⁺ |
|---|---|---|---|
| 733 | 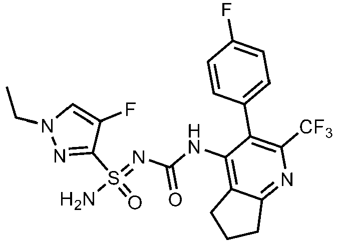 | 1-Ethyl-4-fluoro-N'-((3-(4-fluorophenyl)-2-(trifluoromethyl)-6,7-dihydro-5H-cyclopenta[b]pyridin-4-yl)carbamoyl)-1H-pyrazole-3-sulfonimidamide | 515 |
| 734 | 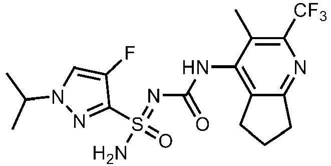 | 4-Fluoro-1-isopropyl-N'-((3-methyl-2-(trifluoromethyl)-6,7-dihydro-5H-cyclopenta[b]pyridin-4-yl)carbamoyl)-1H-pyrazole-3-sulfonimidamide | 449 |
| 735 | 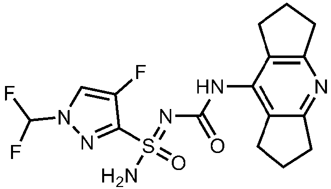 | 1-(Difluoromethyl)-4-fluoro-N'-((1,2,3,5,6,7-hexahydrodicyclopenta[b,e]pyridin-8-yl)carbamoyl)-1H-pyrazole-3-sulfonimidamide | 415 |
| 736 | 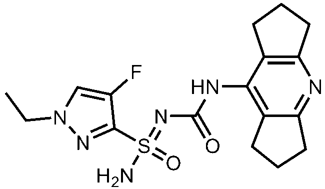 | 1-Ethyl-4-fluoro-N'-((1,2,3,5,6,7-hexahydrodicyclopenta[b,e]pyridin-8-yl)carbamoyl)-1H-pyrazole-3-sulfonimidamide | 393 |
| 737 | 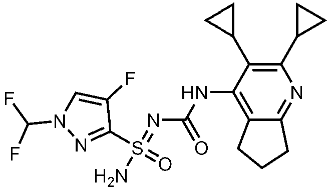 | N'-((2,3-dicyclopropyl-6,7-dihydro-5H-cyclopenta[b]pyridin-4-yl)carbamoyl)-1-(difluoromethyl)-4-fluoro-1H-pyrazole-3-sulfonimidamide | 455 |
| 738 | 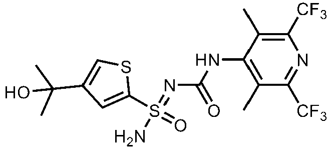 | N'-((3,5-dimethyl-2,6-bis(trifluoromethyl)pyridin-4-yl)carbamoyl)-4-(2-hydroxypropan-2-yl)thiophene-2-sulfonimidamide | *505* |
| 739 | 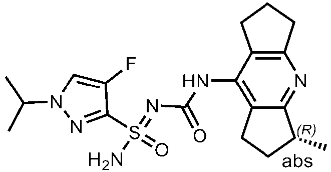 | 4-Fluoro-1-isopropyl-N'-(((R)-3-methyl-1,2,3,5,6,7-hexahydrodicyclopenta[b,e]pyridin-8-yl)carbamoyl)-1H-pyrazole-3-sulfonimidamide (from chiral Intermediate 161) | 421 |
| 740 | 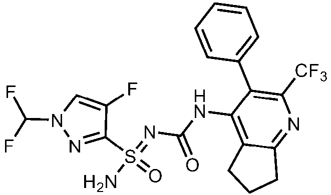 | 1-(Difluoromethyl)-4-fluoro-N'-((3-phenyl-2-(trifluoromethyl)-6,7-dihydro-SH-cyclopenta[b]pyridin-4-yl)carbamoyl)-1H-pyrazole-3-sulfonimidamide | 519 |
| 741 | 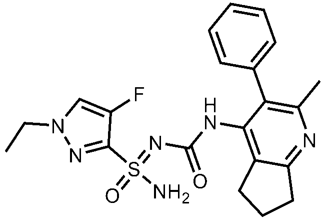 | 1-Ethyl-4-fluoro-N'-((2-methyl-3-phenyl-6,7-dihydro-5H-cyclopenta[b]pyridin-4-yl)carbamoyl)-1H-pyrazole-3-sulfonimidamide | 443 |
| 742 | 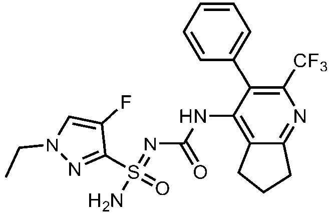 | 1-Ethyl-4-fluoro-N'-((3-phenyl-2-(trifluoromethyl)-6,7-dihydro-5H-cyclopenta[b]pyridin-4-yl)carbamoyl)-1H-pyrazole-3-sulfonimidamide | 497 |
| 743 | 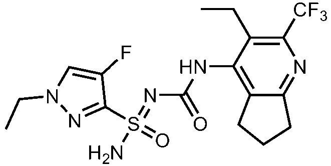 | 1-Ethyl-N'-((3-ethyl-2-(trifluoromethyl)-6,7-dihydro-5H-cyclopenta[b]pyridin-4-yl)carbamoyl)-4-fluoro-1H-pyrazole-3-sulfonimidamide | 449 |
| 744 | 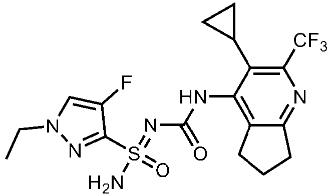 | N'-((3-cyclopropyl-2-(trifluoromethyl)-6,7-dihydro-5H-cyclopenta[b]pyridin-4-yl)carbamoyl)-1-ethyl-4-fluoro-1H-pyrazole-3-sulfonimidamide | 461 |
| 745 | 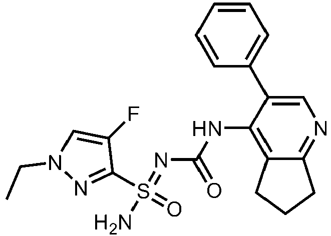 | 1-Ethyl-4-fluoro-N'-((3-phenyl-6,7-dihydro-5H-cyclopenta[b]pyridin-4-yl)carbamoyl)-1H-pyrazole-3-sulfonimidamide | 429 |
| 746 | 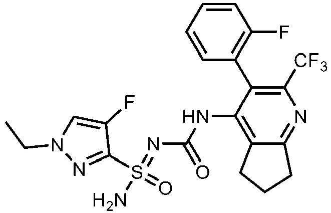 | 1-Ethyl-4-fluoro-N'-((3-(2-fluorophenyl)-2-(trifluoromethyl)-6,7-dihydro-5H-cyclopenta[b]pyridin-4-yl)carbamoyl)-1H-pyrazole-3-sulfonimidamide | 515 |
| 747 | 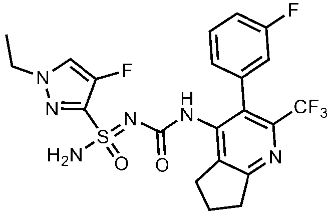 | 1-Ethyl-4-fluoro-N'-((3-(3-fluorophenyl)-2-(trifluoromethyl)-6,7-dihydro-5H-cyclopenta[b]pyridin-4-yl)carbamoyl)-1H-pyrazole-3-sulfonimidamide | 515 |
| 748 | 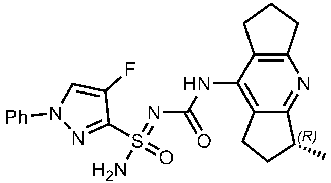 | 4-Fluoro-N'-(((R)-3-methyl-1,2,3,5,6,7-hexahydrodicyclopenta[b,e]pyridin-8-yl)carbamoyl)-1-phenyl-1H-pyrazole-3-sulfonimidamide | 455 |
| 749 | 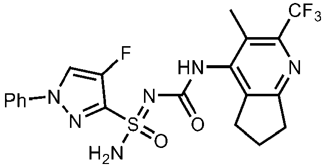 | 4-Fluoro-N'-((3-methyl-2-(trifluoromethyl)-6,7-dihydro-5H-cyclopenta[b]pyridin-4-yl)carbamoyl)-1-phenyl-1H-pyrazole-3-sulfonimidamide | 483 |
| 750 | 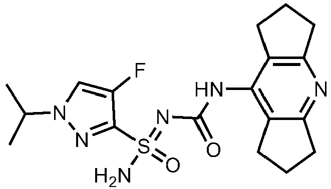 | 4-Fluoro-N'-((1,2,3,5,6,7-hexahydrodicyclopenta[b,e]pyridin-8-yl)carbamoyl)-1-isopropyl-1H-pyrazole-3-sulfonimidamide | 407 |

**Table 66 Examples in the following table were prepared using similar conditions as described in Example 9 and Scheme 2 from appropriate starting materials.**

| Example # | Structure | IUPAC Name | Exact Mass [M+H]⁺ |
|---|---|---|---|
| 751 | 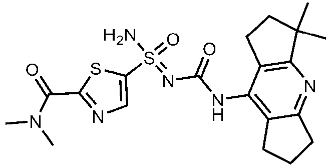 | 5-(N'-((3,3-dimethyl-1,2,3,5,6,7-hexahydrodicyclopenta[b,e]pyridin-8-yl)carbamoyl)sulfamidimidoyl)-N,N-dimethylthiazole-2-carboxamide | 463 |

**Table 67 Examples in the following table were prepared using similar conditions as described in Example 28 and Scheme 2A from appropriate starting materials.**

| Example # | Structure | IUPAC Name | Exact Mass [M+H]⁺ |
|---|---|---|---|
| 752 | 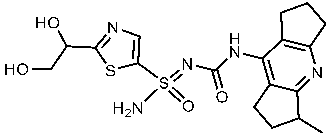 | 2-(1,2-Dihydroxyethyl)-N'-((3-methyl-1,2,3,5,6,7-hexahydrodicyclopenta[b,e]pyridin-8-yl)carbamoyl)thiazole-5-sulfonimidamide | 438 |
| 753 | 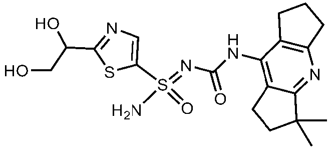 | 2-(1,2-Dihydroxyethyl)-N'-((3,3-dimethyl-1,2,3,5,6,7-hexahydrodicyclopenta[b,e]pyridin-8-yl)carbamoyl)thiazole-5-sulfonimidamide | 452 |

**Table 68 Examples in the following table were prepared using similar conditions as described in Example 251 and Scheme IV from appropriate starting materials.**

| Example # | Structure | IUPAC Name | Exact Mass [M+H]⁺ |
|---|---|---|---|
| 754 | 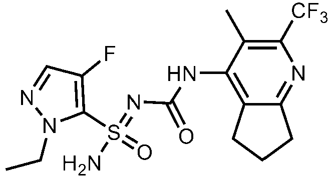 | 1-Ethyl-4-fluoro-N'-((3-methyl-2-(trifluoromethyl)-6,7-dihydro-5H-cyclopenta[b]pyridin-4-yl)carbamoyl)-1H-pyrazole-5-sulfonimidamide | 435 |

**Table 69 Examples in the following table were obtained from chiral HPLC resolutions of racemic and diastereomeric mixture examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. Assigned stereochemistry in compound names are tentative.**

| Ex. # | Structure | IUPAC Name | Column | Eluents | LC-MS [M+H]⁺ |
|---|---|---|---|---|---|
| 755 | 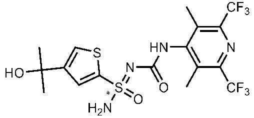 | (R) or (S) -N'-((3,5-dimethyl-2,6-bis(trifluoromethyl)pyridin-4-yl)carbamoyl)-4-(2-hydroxypropan-2-yl)thiophene-2-sulfonimidamide | CHIRALPA K IF, 2*25 cm, 5 um | 10% EtOH in Hex (0.1% FA) | 505 |
| 756 | 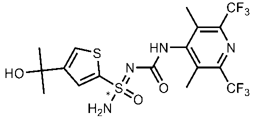 | (S) or (R) -N'-((3,5-dimethyl-2,6-bis(trifluoromethyl)pyridin-4-yl)carbamoyl)-4-(2-hydroxypropan-2-yl)thiophene-2-sulfonimidamide | CHIRALPA K IF, 2*25 cm, 5 um | 10% EtOH in Hex (0.1% FA) | 505 |
| 757 | 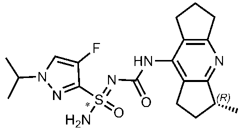 | (R) or (S) -4-fluoro-1-isopropyl-N'-(((R)-3-methyl-1,2,3,5,6,7-hexahydrodicyclopenta[b,e]pyridin-8-yl)carbamoyl)-1H-pyrazole-3-sulfonimidamide | CHIRALPA K IG, 2*25 cm, 5 um | 50% EtOH in Hex (8 mM NH₃·MeOH) | 421 |
| 758 | 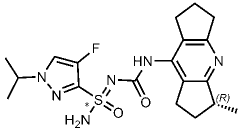 | (S) or (R) -4-fluoro-1-isopropyl-N'-(((R)-3-methyl-1,2,3,5,6,7-hexahydrodicyclopenta[b,e]pyridin-8-yl)carbamoyl)-1H-pyrazole-3-sulfonimidamide | CHIRALPA K IG, 2*25 cm, 5 um | 50% EtOH in Hex (8 mM NH₃·MeOH) | 421 |
| 759 | 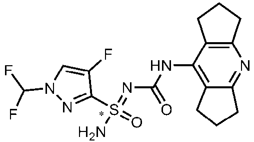 | (R) or (S) -1-(difluoromethyl)-4-fluoro-N'-((1,2,3,5,6,7-hexahydrodicyclopenta[b,e]pyridin-8-yl)carbamoyl)-1H-pyrazole-3-sulfonimidamide | CHIRALPA K IE, 2*25 cm, 5 um | 40% EtOH in Hex (0.1% FA) | 415 |
| 760 | 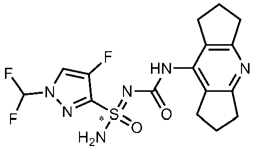 | (S) or (R) -1-(difluoromethyl)-4-fluoro-N'-((1,2,3,5,6,7-hexahydrodicyclopenta[b,e]pyridin-8-yl)carbamoyl)-1H-pyrazole-3-sulfonimidamide | CHIRALPA K IE, 2*25 cm, 5 um | 40% EtOH in Hex (0.1% FA) | 415 |
| 761 | 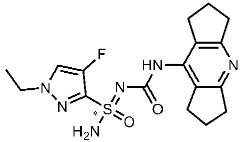 | (R) or (S) -1-ethyl-4-fluoro-N'-((1,2,3,5,6,7-hexahydrodicyclopenta[b,e]pyridin-8-yl)carbamoyl)-1H-pyrazole-3-sulfonimidamide | CHIRALPA K IG, 3*25 cm, 5 um | 50% EtOH in Hex (8 mM NH₃·MeOH) | 393 |
| 762 | 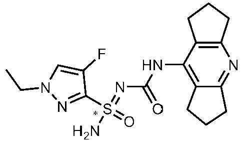 | (S) or (R) -1-ethyl-4-fluoro-N'-((1,2,3,5,6,7-hexahydrodicyclopenta[b,e]pyridin-8-yl)carbamoyl)-1H-pyrazole-3-sulfonimidamide | CHIRALPA K IG, 3*25 cm, 5 um | 50% EtOH in Hex (8 mM NH₃·MeOH) | 393 |
| 763 | 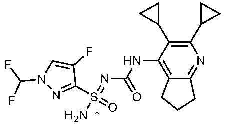 | (R) or (S) -N'-((2,3-dicyclopropyl-6,7-dihydro-5H-cyclopenta[b]pyridin-4-yl)carbamoyl)-1-(difluoromethyl)-4-fluoro-1H-pyrazole-3-sulfonimidamide | Chiralpak IC, 2*25 cm, 5 um | 15% EtOH in Hex (0.1% FA) | 455 |
| 764 | 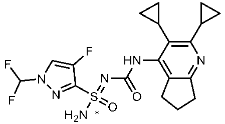 | (S) or (R) -N'-((2,3-dicyclopropyl-6,7-dihydro-5H-cyclopenta[b]pyridin-4-yl)carbamoyl)-1-(difluoromethyl)-4-fluoro-1H-pyrazole-3-sulfonimidamide | Chiralpak IC, 2*25 cm, 5 um | 15% EtOH in Hex (0.1% FA) | 455 |
| 765 | 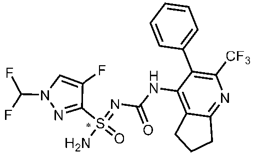 | (R) or (S) -1-(difluoromethyl)-4-fluoro-N'-((3-phenyl-2-(trifluoromethyl)-6,7-dihydro-SH-cyclopenta[b]pyridin-4-yl)carbamoyl)-1H-pyrazole-3-sulfonimidamide | CHIRALPA K IC, 2*25 cm, 5 um | 10% EtOH in Hex (0.1% FA) | 519 |
| 766 | 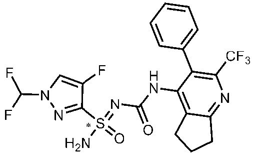 | (S) or (R) -1-(difluoromethyl)-4-fluoro-N'-((3-phenyl-2-(trifluoromethyl)-6,7-dihydro-SH-cyclopenta[b]pyridin-4-yl)carbamoyl)-1H-pyrazole-3-sulfonimidamide | CHIRALPA K IC, 2*25 cm, 5 um | 10% EtOH in Hex (0.1% FA) | 519 |
| 767 | 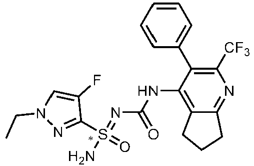 | (R) or (S) -1-ethyl-4-fluoro-N'-((3-phenyl-2-(trifluoromethyl)-6,7-dihydro-5H-cyclopenta[b]pyridin-4-yl)carbamoyl)-1H-pyrazole-3-sulfonimidamide | Chiralpak IC, 2*25 cm, 5 um | 20% EtOH in Hex (0.1% FA) | 497 |
| 768 | 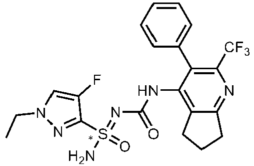 | (S) or (R) -1-ethyl-4-fluoro-N'-((3-phenyl-2-(trifluoromethyl)-6,7-dihydro-5H-cyclopenta[b]pyridin-4-yl)carbamoyl)-1H-pyrazole-3-sulfonimidamide | Chiralpak IC, 2*25 cm, 5 um | 20% EtOH in Hex (0.1% FA) | 497 |
| 769 | 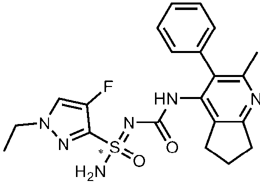 | (R) or (S) -1-ethyl-4-fluoro-N'-((2-methyl-3-phenyl-6,7-dihydro-5H-cyclopenta[b]pyridin-4-yl)carbamoyl)-1H-pyrazole-3-sulfonimidamide | CHIRALPA K IG, 2*25 cm, 5 um | 30% EtOH in Hex (0.1% FA) | 443 |
| 770 | 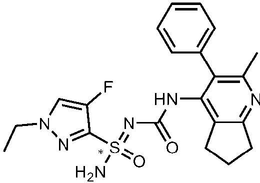 | (S) or (R) -1-ethyl-4-fluoro-N'-((2-methyl-3-phenyl-6,7-dihydro-5H-cyclopenta[b]pyridin-4-yl)carbamoyl)-1H-pyrazole-3-sulfonimidamide | CHIRALPA K IG, 2*25 cm, 5 um | 30% EtOH in Hex (0.1% FA) | 443 |
| 771 | 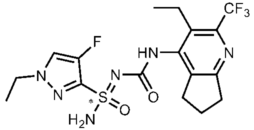 | (R) or (S) -1-ethyl-N'-((3-ethyl-2-(trifluoromethyl)-6,7-dihydro-5H-cyclopenta[b]pyridin-4-yl)carbamoyl)-4-fluoro-1H-pyrazole-3-sulfonimidamide | Chiralpak IC, 2*25 cm, 5 um | 30% EtOH in Hex (0.1% FA) | 449 |
| 772 | 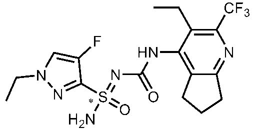 | (S) or (R) -1-ethyl-N'-((3-ethyl-2-(trifluoromethyl)-6,7-dihydro-5H-cyclopenta[b]pyridin-4-yl)carbamoyl)-4-fluoro-1H-pyrazole-3-sulfonimidamide | Chiralpak IC, 2*25 cm, 5 um | 30% EtOH in Hex (0.1% FA) | 449 |
| 773 | 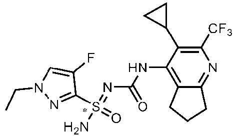 | (R) or (S) -N'-((3-cyclopropyl-2-(trifluoromethyl)-6,7-dihydro-5H-cyclopenta[b]pyridin-4-yl)carbamoyl)-1-ethyl-4-fluoro-1H-pyrazole-3-sulfonimidamide | Chiralpak IC, 2*25 cm, 5 um | 20% EtOH in Hex (0.1% FA) | 461 |
| 774 | 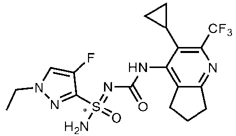 | (S) or (R) -N'-((3-cyclopropyl-2-(trifluoromethyl)-6,7-dihydro-5H-cyclopenta[b]pyridin-4-yl)carbamoyl)-1-ethyl-4-fluoro-1H-pyrazole-3-sulfonimidamide | Chiralpak IC, 2*25 cm, 5 um | 20% EtOH in Hex (0.1% FA) | 461 |
| 775 | 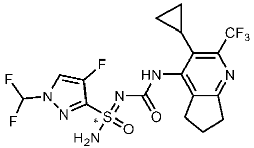 | (R) or (S) -N'-((3-cyclopropyl-2-(trifluoromethyl)-6,7-dihydro-5H-cyclopenta[b]pyridin-4-yl)carbamoyl)-1-(difluoromethyl)-4-fluoro-1H-pyrazole-3-sulfonimidamide | CHIRALPA K IC, 2*25 cm, 5 um | 10% EtOH in Hex (0.3% FA) | 483 |
| 776 | 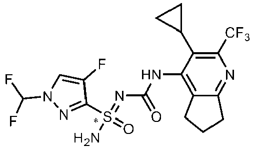 | (S) or (R) -N'-((3-cyclopropyl-2-(trifluoromethyl)-6,7-dihydro-5H-cyclopenta[b]pyridin-4-yl)carbamoyl)-1-(difluoromethyl)-4-fluoro-1H-pyrazole-3-sulfonimidamide | CHIRALPA K IC, 2*25 cm, 5 um | 10% EtOH in Hex (0.3% FA) | 483 |
| 777 | 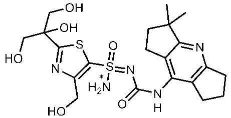 | (R) or (S) -N'-((3,3-dimethyl-1,2,3,5,6,7-hexahydrodicyclopenta[b,e]pyridin-8-yl)carbamoyl)-4-(hydroxymethyl)-2-(1,2,3-trihydroxypropan-2-yl)thiazole-5-sulfonimidamide | CHIRALPA K IG, 2*25 cm, 5 um | 20% EtOH in Hex (0.1% FA) | 512 |
| 778 | 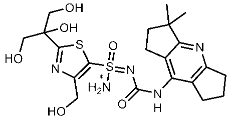 | (S) or (R) -N'-((3,3-dimethyl-1,2,3,5,6,7-hexahydrodicyclopenta[b,e]pyridin-8-yl)carbamoyl)-4-(hydroxymethyl)-2-(1,2,3-trihydroxypropan-2-yl)thiazole-5-sulfonimidamide | CHIRALPA K IG, 2*25 cm, 5 um | 20% EtOH in Hex (0.1% FA) | 512 |
| 779 | 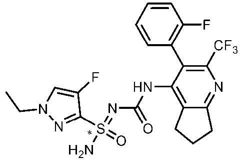 | (R) or (S) -1-ethyl-4-fluoro-N'-((3-(2-fluorophenyl)-2-(trifluoromethyl)-6,7-dihydro-5H-cyclopenta[b]pyridin-4-yl)carbamoyl)-1H-pyrazole-3-sulfonimidamide | CHIRALPA K IC, 2*25 cm, 5 um | 20% EtOH in Hex (0.1% FA) | 515 |
| 780 | 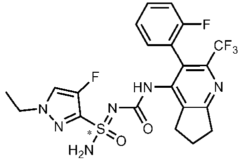 | (S) or (R) -1-ethyl-4-fluoro-N'-((3-(2-fluorophenyl)-2-(trifluoromethyl)-6,7-dihydro-5H-cyclopenta[b]pyridin-4-yl)carbamoyl)-1H-pyrazole-3-sulfonimidamide | CHIRALPA K IC, 2*25 cm, 5 um | 20% EtOH in Hex (0.1% FA) | 515 |
| 781 | 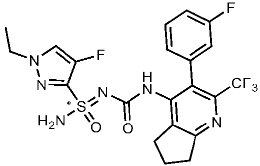 | (R) or (S) -1-ethyl-4-fluoro-N'-((3-(3-fluorophenyl)-2-(trifluoromethyl)-6,7-dihydro-5H-cyclopenta[b]pyridin-4-yl)carbamoyl)-1H-pyrazole-3-sulfonimidamide | CHIRALPA K IC, 3*25 cm, 5 um | 20% EtOH in Hex (0.1% FA) | 515 |
| 782 | 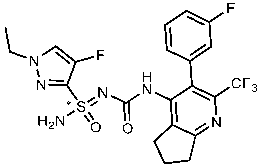 | (S) or (R) -1-ethyl-4-fluoro-N'-((3-(3-fluorophenyl)-2-(trifluoromethyl)-6,7-dihydro-5H-cyclopenta[b]pyridin-4-yl)carbamoyl)-1H-pyrazole-3-sulfonimidamide | CHIRALPA K IC, 3*25 cm, 5 um | 20% EtOH in Hex (0.1% FA) | 515 |
| 783 | 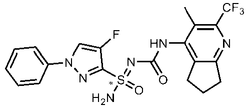 | (R) or (S) -4-fluoro-N'-((3-methyl-2-(trifluoromethyl)-6,7-dihydro-5H-cyclopenta[b]pyridin-4-yl)carbamoyl)-1-phenyl-1H-pyrazole-3-sulfonimidamide | Chiralpak IC, 2*25 cm, 5 um | 20% EtOH in Hex (0.1% FA) | 483 |
| 784 | 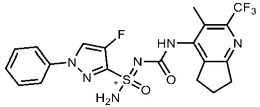 | (S) or (R) -4-fluoro-N'-((3-methyl-2-(trifluoromethyl)-6,7-dihydro-5H-cyclopenta[b]pyridin-4-yl)carbamoyl)-1-phenyl-1H-pyrazole-3-sulfonimidamide | Chiralpak IC, 2*25 cm, 5 um | 20% EtOH in Hex (0.1% FA) | 483 |
| 785 | 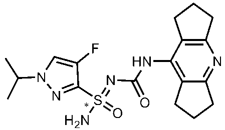 | (R) or (S) -4-fluoro-N'-((1,2,3,5,6,7-hexahydrodicyclopenta[b,e]pyridin-8-yl)carbamoyl)-1-isopropyl-1H-pyrazole-3-sulfonimidamide | CHIRALPA K IG, 3*25 cm, 5 um | 40% EtOH in Hex (8 mM NH₃·MeOH) | 407 |
| 786 | 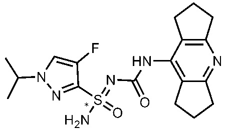 | (S) or (R) -4-fluoro-N'-((1,2,3,5,6,7-hexahydrodicyclopenta[b,e]pyridin-8-yl)carbamoyl)-1-isopropyl-1H-pyrazole-3-sulfonimidamide | CHIRALPA K IG, 3*25 cm, 5 um | 40% EtOH in Hex (8 mM NH₃·MeOH) | 407 |
| 787 | 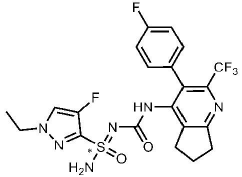 | (R) or (S) -1-ethyl-4-fluoro-N'-((3-(4-fluorophenyl)-2-(trifluoromethyl)-6,7-dihydro-5H-cyclopenta[b]pyridin-4-yl)carbamoyl)-1H-pyrazole-3-sulfonimidamide | Chiralpak IC, 2*25 cm, 5 um | 15% EtOH in Hex (0.1% FA) | 515 |
| 788 | 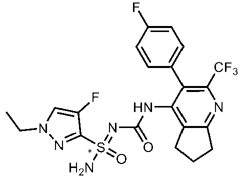 | (S) or (R) -1-ethyl-4-fluoro-N'-((3-(4-fluorophenyl)-2-(trifluoromethyl)-6,7-dihydro-5H-cyclopenta[b]pyridin-4-yl)carbamoyl)-1H-pyrazole-3-sulfonimidamide | Chiralpak IC, 2*25 cm, 5 um | 15% EtOH in Hex (0.1% FA) | 515 |
| 789 | 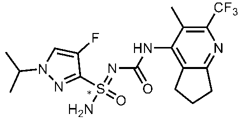 | (R) or (S) -4-fluoro-1-isopropyl-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K IE, 2*25 cm, 5 um | 20% EtOH in Hex (0.1% FA) | 449 |
| 790 | 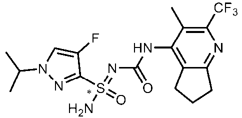 | (S) or (R) -4-fluoro-1-isopropyl-N'-((3-methyl-2-(trifluoromethyl)-6,7-dihydro-5H-cyclopenta[b]pyridin-4-yl)carbamoyl)-1H-pyrazole-3-sulfonimidamide | CHIRALPA K IE, 2*25 cm, 5 um | 20% EtOH in Hex (0.1% FA) | 449 |
| 791 | 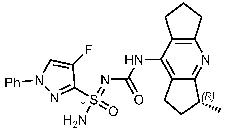 | (R) or (S) -4-fluoro-N'-(((R)-3-methyl-1,2,3,5,6,7-hexahydrodicyclopenta[b,e]pyridin-8-yl)carbamoyl)-1-phenyl-1H-pyrazole-3-sulfonimidamide | CHIRALPA K IE, 4.6*50 mm, 3 um | 50% EtOH in Hex (0.1% FA) | 455 |
| 792 | 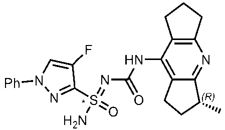 | (S) or (R) -4-fluoro-N'-(((R)-3-methyl-1,2,3,5,6,7-hexahydrodicyclopenta[b,e]pyridin-8-yl)carbamoyl)-1-phenyl-1H-pyrazole-3-sulfonimidamide | CHIRALPA K IE, 4.6*50 mm, 3 um | 50% EtOH in Hex (0.1% FA) | 455 |
| 793 | 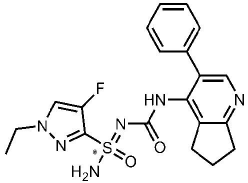 | (R) or (S)-1-ethyl-4-fluoro-N'-((3-phenyl-6,7-dihydro-5H-cyclopenta[b]pyridin-4-yl)carbamoyl)-1H-pyrazole-3-sulfonimidamide | CHIRALPA K IE, 2*25 cm, 5 um | 50% EtOH in Hex (0.1% FA) | 429 |
| 794 | 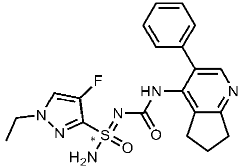 | (S) or (R)-1-ethyl-4-fluoro-N'-((3-phenyl-6,7-dihydro-5H-cyclopenta[b]pyridin-4-yl)carbamoyl)-1H-pyrazole-3-sulfonimidamide | CHIRALPA K IE, 2*25 cm, 5 um | 50% EtOH in Hex (0.1% FA) | 429 |

### Example 795 (compound 1100)

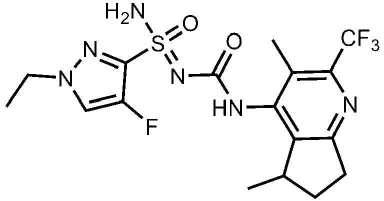

### N'-((3,5-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide (Scheme 2)

### Example 796 (compound 1101)

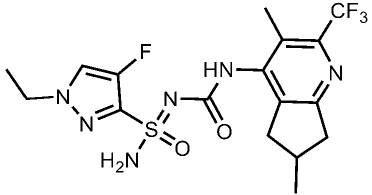

### N'-((3,5-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide (Scheme 2)

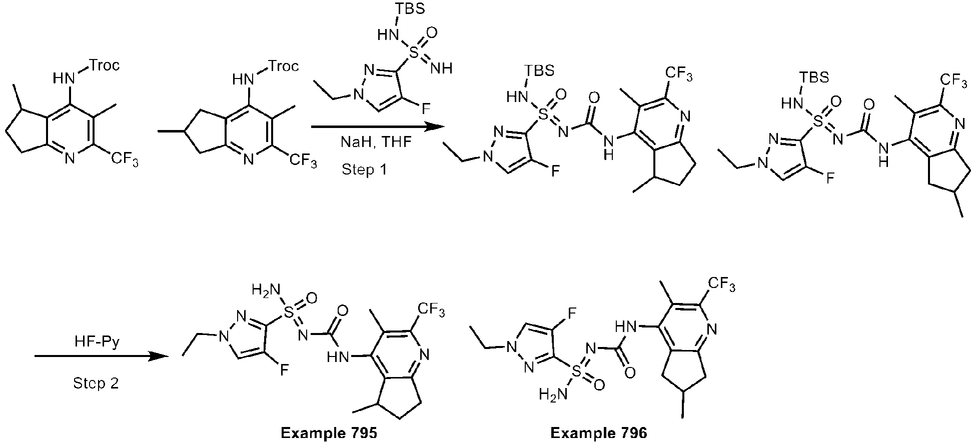

### Step 1: Mixture of N-(tert-butyldimethylsilyl)-N'-((3,5-dimethyl-2-(trifluoromethyl)-6,7-dihydro-5H- cyclopenta[b]pyridin-4-yl)carbamoyl)-1-ethyl-4-fluoro-1H-pyrazole-3-sulfonimidamide and N-(tert-butyldimethylsilyl)-N'-((3,6-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide

To a stirred solution of N-(tert-butyldimethylsilyl)-1-ethyl-4-fluoro-1H-pyrazole-3-sulfonimidamide (240 mg, 0.78 mmol) in THF (10 mL) under nitrogen was added NaH (60%wt, 62.8 mg, 1.57 mmol) in portions at 0 °C, and then to the above solution was added 2,2,2-trichloroethyl (3,5-dimethyl-2-(trifluoromethyl)-6,7- dihydro-5H-cyclopenta[b]pyridin-4-yl)carbamate and 2,2,2-trichloroethyl (3,6-dimethyl-2-(trifluoromethyl)- 6,7-dihydro-5H-cyclopenta[b]pyridin-4-yl)carbamate (~1:1 mixture, 349 mg, 0.86 mmol) in portions at 0 °C. The resulting solution was stirred for 1 h at RT. The reaction was then quenched with 10 mL of water and extracted with 3x10 mL of EtOAc. The organic layers were combined, dried over anhydrous Na₂SO₄, and concentrated under vacuum. This resulted in 600 mg (crude mixture) of the title compound as a yellow solid. MS-ESI: 563 (M+1).

### Step 2: N'-((3,5-dimethyl-2-(trifluoromethyl)-6,7-dihydro-5H-cyclopenta[b]pyridin-4-yl)carbamoyl)-1- ethyl-4-fluoro-1H-pyrazole-3-sulfonimidamide and N'-((3,6-dimethyl-2-(trifluoromethyl)-6,7-dihydro- 5H-cyclopenta[b]pyridin-4-yl)carbamoyl)-1-ethyl-4-fluoro-1H-pyrazole-3-sulfonimidamide

To a stirred solution of the mixture of N-(tert-butyldimethylsilyl)-N'-((3,5-dimethyl-2-(trifluoromethyl)-6,7- dihydro-5H-cyclopenta[b]pyridin-4-yl)carbamoyl)-1-ethyl-4-fluoro-1H-pyrazole-3-sulfonimidamide and N-(tert-butyldimethylsilyl)-N'-((3,6-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide (600 mg, crude from last step) in DCM (10 mL) was added HF-Pyridine (70%wt, 0.1 mL) dropwise at 0 °C. The resulting solution was stirred for 10 min at RT. The resulting mixture was concentrated under vacuum. The crude product was purified by Prep-HPLC using the following conditions: Column: Xselect CSH OBD Column 30*150 mm 5 um; Mobile Phase A: water (10mM NH₄HCO₃ + 0.1%NH₃·H₂O), Mobile Phase B: ACN; Flow rate: 60 mL/min; Gradient: 15% B to 25% B over 10 min; 254/210 nm; Rt₁: 8.82 min **(Example 795),** Rt₂: 9.26 min **(Example 796).** This resulted in 19.3 mg (5.5% over two steps) of **Example 795** and 19.5 mg (5.5% over two steps) of **Example 796** both as a white solid. MS-ESI: both 449 (M+1).

**Example 795 (compound 1100):** ¹H NMR (400 MHz, DMSO-*d*₆) δ 8.87 (s, 1H), 8.03 (d, *J* = 5.2 Hz, 1H), 7.61 (br s, 2H), 4.15-4.07 (m, 2H), 3.60-3.40 (m, 1H), 3.08-2.90 (m, 1H), 2.89-2.79 (m, 1H), 2.30-2.16 (m, 4H), 1.76-1.52 (m, 1H), 1.40-1.34 (m, 1.5H), 1.09 (d, *J* = 6.9 Hz, 3H).

**Example 796 (compound 1101):** ¹H NMR (400 MHz, DMSO-*d*₆) δ 8.89 (s, 1H), 8.04 (d, *J =* 4.4 Hz, 1H), 7.53 (br s, 2H), 4.12 (q, *J =* 7.2 Hz, 2H), 3.34-3.05 (m, 1H), 2.95-2.90 (m, 1H), 2.58-2.38 (m, 3H), 2.21 (s, 3H), 1.38 (t, *J =* 7.2 Hz, 3H), 1.09 (d, *J =* 6.4 Hz, 3H).

**Table 70 Examples in the following table were prepared using similar conditions as described in Example 9 and Scheme 2 from appropriate starting materials.**

| Example # | Compound # | Structure | IUPAC Name | Exact Mass [M+H]⁺ |
|---|---|---|---|---|
| 797 | **1102** | 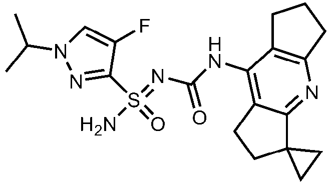 | 4-Fluoro-1-isopropyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 433 |
| 798 | **1103** | 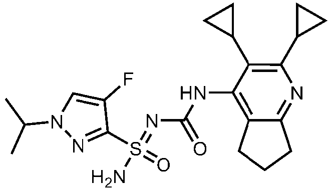 | N'-((2,3-dicyclopropyl-6,7-dihydro-5H-cyclopenta[b]pyridin-4-yl)carbamoyl)-4-fluoro-1-isopropyl-1H-pyrazole-3-sulfonimidamide | 447 |
| 799 | **1104** | 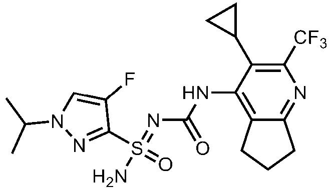 | N'-((3-cyclopropyl-2-(trifluoromethyl)-6,7-dihydro-5H-cyclopenta[b]pyridin-4-yl)carbamoyl)-4-fluoro-1-isopropyl-1H-pyrazole-3-sulfonimidamide | 475 |
| 800 | **1007** | 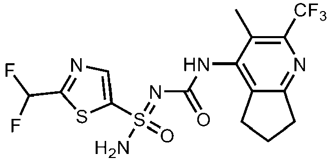 | 2-(Difluoromethyl)-N'-((3-methyl-2-(trifluoromethyl)-6,7-dihydro-5H-cyclopenta[b]pyridin-4-yl)carbamoyl)thiazole-5-sulfonimidamide | 456 |

**Table 71. Examples in the following table were prepared using similar conditions as described in Example 252 and Scheme V from appropriate starting materials.**

| Example # | Compound # | Structure | IUPAC Name | Exact Mass [M+H]⁺ |
|---|---|---|---|---|
| 801 | **1106** | 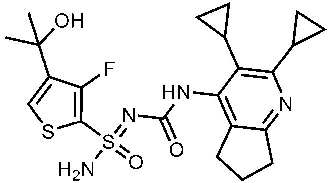 | N'-((2,3-dicyclopropyl-6,7-dihydro-5H-cyclopenta[b]pyridin-4-yl)carbamoyl)-3-fluoro-4-(2-hydroxypropan-2-yl)thiophene-2-sulfonimidamide | 479 |
| 802 | **1004** | 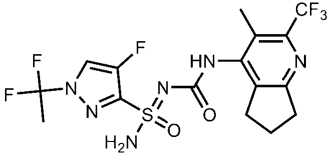 | 1-(1,1-Difluoroethyl)-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | 471 |
| 803 | **1105** | 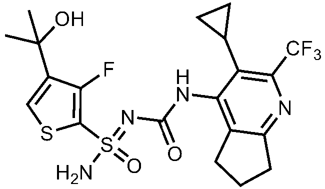 | N'-((3-cyclopropyl-2-(trifluoromethyl)-6,7-dihydro-5H-cyclopenta[b]pyridin-4-yl)carbamoyl)-3-fluoro-4-(2-hydroxypropan-2-yl)thiophene-2-sulfonimidamide | 507 |
| 804 | **1107** | 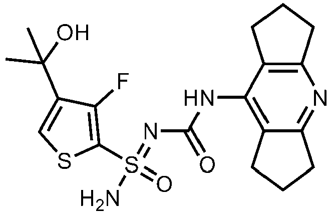 | 3-Fluoro-N'-((1,2,3,5,6,7-hexahydrodicyclopenta[b,e]pyr idin-8-yl)carbamoyl)-4-(2-hydroxypropan-2-yl)thiophene-2-sulfonimidamide | 439 |
| 805 | **1108** | 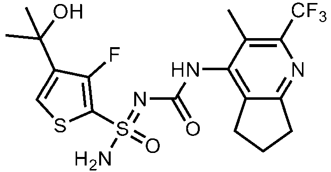 | 3-Fluoro-4-(2-hydroxypropan-2-yl)-N'-((3-methyl-2-(trifluoromethyl)-6,7-dihydro-5H-cyclopenta[b]pyridin-4-yl)carbamoyl)thiophene-2-sulfonimidamide | 481 |
| 806 | **1109** | 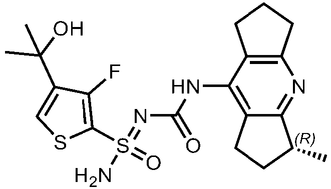 | 3-Fluoro-4-(2-hydroxypropan-2-yl)-N'-(((R)-3-methyl-1,2,3,5,6,7-hexahydrodicyclopenta[b,e]pyr idin-8-yl)carbamoyl)thiophene-2-sulfonimidamide | 453 |
| 807 | **1110** | 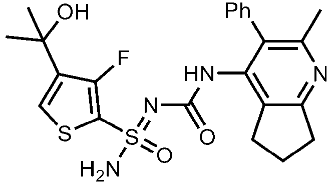 | 3-Fluoro-4-(2-hydroxypropan-2-yl)-N'-((2-methyl-3-phenyl-6,7-dihydro-5H-cyclopenta[b]pyridin-4-yl)carbamoyl)thiophene-2-sulfonimidamide | 489 |
| 808 | **1111** | 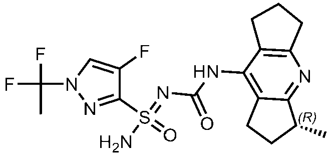 | 1-(1,1-Difluoroethyl)-4-fluoro-N'-(((R)-3-methyl-1,2,3,5,6,7-hexahydrodicyclopenta[b,e]pyr idin-8-yl)carbamoyl)-1H-pyrazole-3-sulfonimidamide | 443 |
| 809 | **1012** | 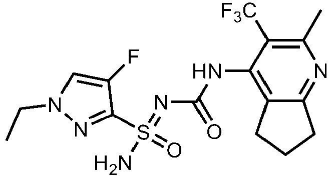 | 1-Ethyl-4-fluoro-N'-((2-methyl-3-(trifluoromethyl)-6,7-dihydro-5H-cyclopenta[b]pyridin-4-yl)carbamoyl)-1H-pyrazole-3-sulfonimidamide | 435 |
| 810 | **1112** | 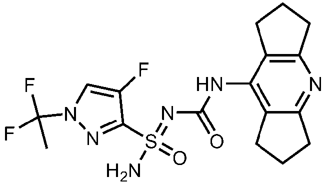 | 1-(1,1-Difluoroethyl)-4-fluoro-N'-((1,2,3,5,6,7-hexahydrodicyclopenta[b,e]pyr idin-8-yl)carbamoyl)-1H-pyrazole-3-sulfonimidamide | 429 |
| 811 | **1114** | 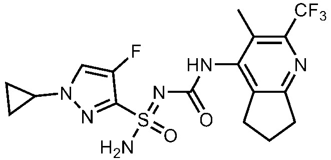 | 1-Cyclopropyl-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | 446 |

**Table 72. Examples in the following table were prepared using similar conditions as described in Example 68 and Scheme 6 from appropriate starting materials.**

| Example # | Compound # | Structure | IUPAC Name | Exact Mass [M+H]⁺ |
|---|---|---|---|---|
| 812 | **1113** | 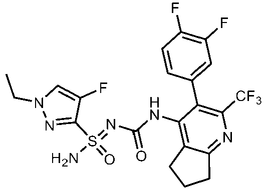 | N'-((3-(3,4-difluorophenyl)-2-(trifluoromethyl)-6,7-dihydro-5H-cyclopenta[b]pyridin-4-yl)carbamoyl)-1-ethyl-4-fluoro-1H-pyrazole-3-sulfonimidamide | 533 |

**Table 73 Examples in the following table were obtained from chiral HPLC resolutions of racemic and diastereomeric mixture examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. Assigned stereochemistry in compound names are tentative.**

| Ex. # | Compound # | Structure | IUPAC Name | Column | Eluents | LC-MS [M+H]⁺ |
|---|---|---|---|---|---|---|
| 813 | **1102a** | 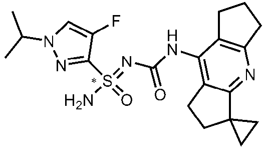 | (R) or (S)-4-Fluoro-1-isopropyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | Chiralpak ID, 2*25 cm, 5 um | 50% EtOH in Hex (0.1% FA) | 433 |
| 814 | **1102b** | 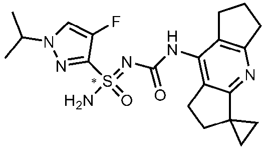 | (S) or (R)-4-Fluoro-1-isopropyl-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | Chiralpak ID, 2*25 cm, 5 um | 50% EtOH in Hex (0.1% FA) | 433 |
| 815 | **1103a** | 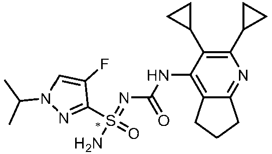 | (R) or (S)-N'-((2,3-dicyclopropyl-6,7-dihydro-5H-cyclopenta[b]pyridin-4-yl)carbamoyl)-4-fluoro-1-isopropyl-1H-pyrazole-3-sulfonimidamide | CHIRALP AK IC, 2*25 cm, 5 um | 30% EtOH in Hex (0.1% FA) | 447 |
| 816 | **1103b** | 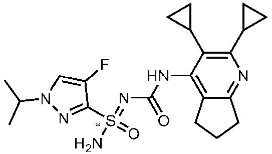 | (S) or (R)-N'-((2,3-dicyclopropyl-6,7-dihydro-5H-cyclopenta[b]pyridin-4-yl)carbamoyl)-4-fluoro-1-isopropyl-1H-pyrazole-3-sulfonimidamide | CHIRALP AK IC, 2*25 cm, 5 um | 30% EtOH in Hex (0.1% FA) | 447 |
| 817 | **1100a** | 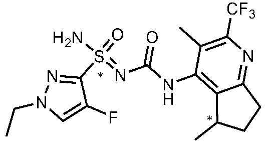 | (R, R) or (R, S)-N'-((3,5-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide | Chiralpak ID, 2*25 cm, 5 um | 20% EtOH in Hex (0.1% FA) | 449 |
| 818 | **1100b** | 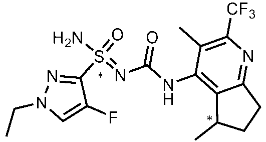 | (R, S) or (R, R)-N'-((3,5-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide | Chiralpak ID, 2*25 cm, 5 um | 20% EtOH in Hex (0.1% FA) | 449 |
| 819 | **1100c** | 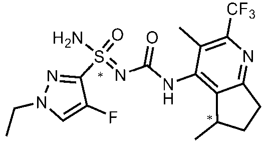 | (S, R) or (S, S)-N'-((3,5-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide | Chiralpak ID, 2*25 cm, 5 um | 20% EtOH in Hex (0.1% FA) | 449 |
| 820 | **1100d** | 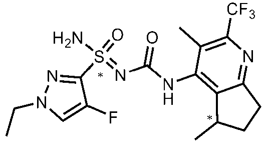 | (S, S) or (S, R)-N'-((3,5-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide | Chiralpak ID, 2*25 cm, 5 um | 20% EtOH in Hex (0.1% FA) | 449 |
| 821 | **1101a** | 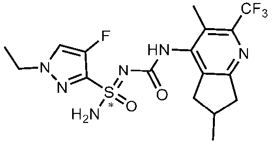 | (R, R/S)-N'-((3,6-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide | 1^{st} peak and 2^{nd} peak, Chiralpak ID, 2*25 cm, 5 um | 20% EtOH in Hex (0.1% FA) | 449 |
| 822 | **1101b** | 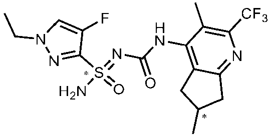 | (S, R) or (S, S)-N'-((3,6-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide | 3^{rd} peak, Chiralpak ID, 2*25 cm, 5 um | 20% EtOH in Hex (0.1% FA) | 449 |
| 823 | **1101c** | 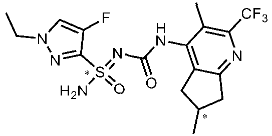 | (S, S) or (S, R)-N'-((3,6-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide | 4^{th} peak, Chiralpak ID, 2*25 cm, 5 um | 20% EtOH in Hex (0.1% FA) | 449 |
| 824 | **1101d** | 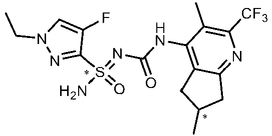 | (R, R) or (R, S)-N'-((3,6-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide (Separated from Example 821) | CHIRAL ART Amylose-C NEO, 3*25 cm, 5 um 30% EtOH:Hex=1:1 (2 mM NH₃·MeOH) in CO₂ | | 449 |
| 825 | **1101e** | 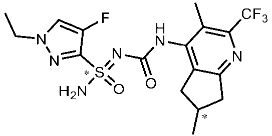 | (R, S) or (R, R)-N'-((3,6-dimethyl-2-(trifluoromethyl)-6,7-dihydro-5H-cyclopenta[b]pyridin-4-yl)carbamoyl)-1-ethyl-4-fluoro-1H-pyrazole-3-sulfonimidamide (Separated from Example 821) | CHIRAL ART Amylose-C NEO, 3*25 cm, 5 um 30% EtOH:Hex=1:1 (2 mM NH₃·MeOH) in CO₂ | | 449 |
| 826 | **1104a** | 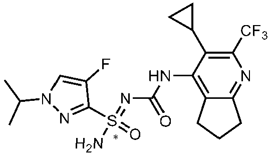 | (R) or (S)-N'-((3-cyclopropyl-2-(trifluoromethyl)-6,7-dihydro-5H-cyclopenta[b]pyridin-4-yl)carbamoyl)-4-fluoro-1-isopropyl-1H-pyrazole-3-sulfonimidamide | CHIRALP AK ID, 4.6*50 mm, 3 um | 30% EtOH in Hex (0.1% FA) | 475 |
| 827 | **1104b** | 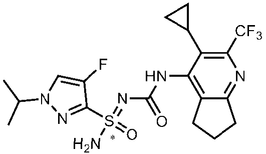 | (S) or (R)-N'-((3-cyclopropyl-2-(trifluoromethyl)-6,7-dihydro-5H-cyclopenta[b]pyridin-4-yl)carbamoyl)-4-fluoro-1-isopropyl-1H-pyrazole-3-sulfonimidamide | CHIRALP AK ID, 4.6*50 mm, 3 um | 30% EtOH in Hex (0.1% FA) | 475 |
| 828 | **1007b** | 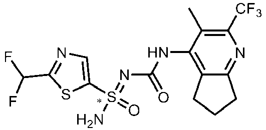 | (S) or (R)-2-(Difluoromethyl)-N'-((3-methyl-2-(trifluoromethyl)-6,7-dihydro-5H-cyclopenta[b]pyridin-4-yl)carbamoyl)thiazole-5-sulfonimidamide | CHIRALP AK IA, 3*25 cm, 5 um | 15% EtOH in Hex (0.1% FA) | 456 |
| 829 | **1007a** | 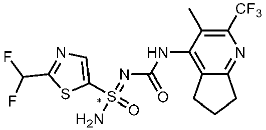 | (R) or (S)-2-(Difluoromethyl)-N'-((3-methyl-2-(trifluoromethyl)-6,7-dihydro-5H-cyclopenta[b]pyridin-4-yl)carbamoyl)thiazole-5-sulfonimidamide | CHIRALP AK IA, 3*25 cm, 5 um | 15% EtOH in Hex (0.1% FA) | 456 |
| 830 | **1106a** | 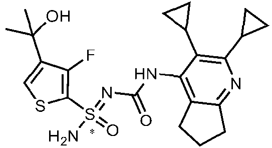 | (R) or (S)-N'-((2,3-dicyclopropyl-6,7-dihydro-5H-cyclopenta[b]pyridin-4-yl)carbamoyl)-3-fluoro-4-(2-hydroxypropan-2-yl)thiophene-2-sulfonimidamide | CHIRALP AK ID, 2.0*25 cm, 5 um | 40% IPA in Hex (0.1% FA) | 479 |
| 831 | **1106b** | 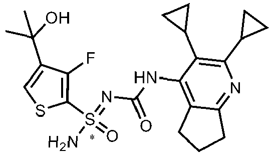 | (S) or (R)-N'-((2,3-dicyclopropyl-6,7-dihydro-5H-cyclopenta[b]pyridin-4-yl)carbamoyl)-3-fluoro-4-(2-hydroxypropan-2-yl)thiophene-2-sulfonimidamide | CHIRALP AK ID, 2.0*25 cm, 5 um | 40% IPA in Hex (0.1% FA) | 479 |
| 832 | **1004a** | 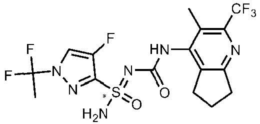 | (R) or (S)-1-(1,1-Difluoroethyl)-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | Chiralpak ID, 2*25 cm, 5 um | 20% IPA in Hex (0.1% FA) | 471 |
| 833 | **1004b** | 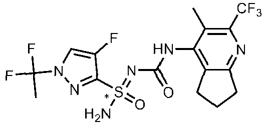 | (S) or (R)-1-(1,1-Difluoroethyl)-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | Chiralpak ID, 2*25 cm, 5 um | 20% IPA in Hex (0.1% FA) | 471 |
| 834 | **1105a** | 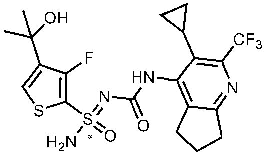 | (R) or (S)-N'-((3-cyclopropyl-2-(trifluoromethyl)-6,7-dihydro-5H-cyclopenta[b]pyridin-4-yl)carbamoyl)-3-fluoro-4-(2-hydroxypropan-2-yl)thiophene-2-sulfonimidamide | CHIRALP AK ID 4.6*50 mm, 3 um | 50% IPA in Hex (0.1% FA) | 507 |
| 835 | **1105b** | 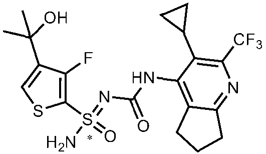 | (S) or (R) -N'-((3-cyclopropyl-2-(trifluoromethyl)-6,7-dihydro-5H-cyclopenta[b]pyridin-4-yl)carbamoyl)-3-fluoro-4-(2-hydroxypropan-2-yl)thiophene-2-sulfonimidamide | CHIRALP AK ID 4.6*50 mm, 3 um | 50% IPA in Hex (0.1% FA) | 507 |
| 836 | **1113a** | 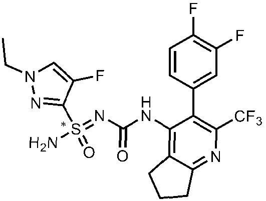 | (S) or (R)-N'-((3-(3,4-difluorophenyl)-2-(trifluoromethyl)-6,7-dihydro-5H-cyclopenta[b]pyridin-4-yl)carbamoyl)-1-ethyl-4-fluoro-1H-pyrazole-3-sulfonimidamide | CHIRALP AK IG, 2*25 cm,5 um | 20% IPA in Hex (0.1 %FA) | 533 |
| 837 | **1113b** | 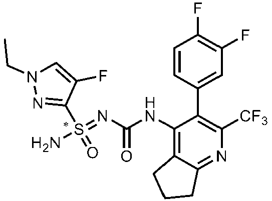 | (R) or (S)-N'-((3-(3,4-difluorophenyl)-2-(trifluoromethyl)-6,7-dihydro-5H-cyclopenta[b]pyridin-4-yl)carbamoyl)-1-ethyl-4-fluoro-1H-pyrazole-3-sulfonimidamide | CHIRALP AK IG, 2*25 cm,5 um | 20% IPA in Hex (0.1 %FA) | 533 |
| 838 | **1107a** | 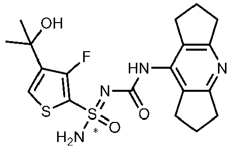 | (R) or (S)-3-fluoro-N'-((1,2,3,5,6,7-hexahydrodicyclopenta[b,e]pyridi n-8-yl)carbamoyl)-4-(2-hydroxypropan-2-yl)thiophene-2-sulfonimidamide | CHIRALP AK IG 2*25 cm, 5 um | 30% EtOH in Hex (0.3% FA) | 439 |
| 839 | **1107b** | 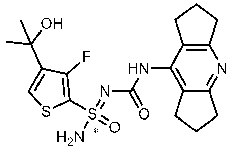 | (S) or (R)-3-fluoro-N'-((1,2,3,5,6,7-hexahydrodicyclopenta[b,e]pyridi n-8-yl)carbamoyl)-4-(2-hydroxypropan-2-yl)thiophene-2-sulfonimidamide | CHIRALP AK IG 2*25 cm, 5 um | 30% EtOH in Hex (0.3% FA) | 439 |
| 840 | **1108a** | 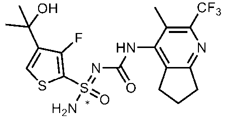 | (R) or (S)-3-fluoro-4-(2-hydroxypropan-2-yl)-N'-((3-methyl-2-(trifluoromethyl)-6,7-dihydro-5H-cyclopenta[b]pyridin-4-yl)carbamoyl)thiophene-2-sulfonimidamide | CHIRALP AK IG 2*25 cm, 5 um | 15% EtOH in Hex (0.1% FA) | 481 |
| 841 | **1108b** | 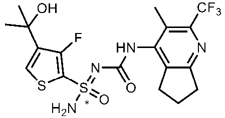 | (S) or (R)-3-fluoro-4-(2-hydroxypropan-2-yl)-N'-((3-methyl-2-(trifluoromethyl)-6,7-dihydro-5H-cyclopenta[b]pyridin-4-yl)carbamoyl)thiophene-2-sulfonimidamide | CHIRALP AK IG 2*25 cm, 5 um | 15% EtOH in Hex (0.1% FA) | 481 |
| 842 | **1109a** | 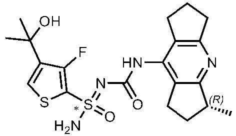 | (R) or (S)-3-fluoro-4-(2-hydroxypropan-2-yl)-N'-(((R)-3-methyl-1,2,3,5,6,7-hexahydrodicyclopenta[b,e]pyridi n-8-yl)carbamoyl)thiophene-2-sulfonimidamide | CHIRALP AK IG 2*25 cm, 5 um | 30% EtOH in Hex (0.3% FA) | 453 |
| 843 | **1109b** | 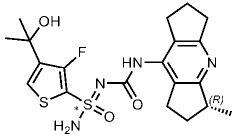 | (S) or (R)-3-fluoro-4-(2-hydroxypropan-2-yl)-N'-(((R)-3-methyl-1,2,3,5,6,7-hexahydrodicyclopenta[b,e]pyridi n-8-yl)carbamoyl)thiophene-2-sulfonimidamide | CHIRALP AK IG 2*25 cm, 5 um | 30% EtOH in Hex (0.3% FA) | 453 |
| 844 | **1110a** | 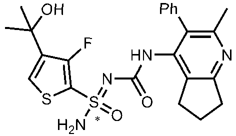 | (S) or (R)-3-fluoro-4-(2-hydroxypropan-2-yl)-N'-((2-methyl-3-phenyl-6,7-dihydro-5H-cyclopenta[b]pyridin-4-yl)carbamoyl)thiophene-2-sulfonimidamide | CHIRAL ART Cellulose-SB, 2*25 cm, 5 um | 15% EtOH in Hex (0.3% FA) | 489 |
| 845 | **1110b** | 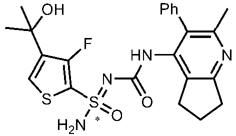 | (R) or (S)-3-fluoro-4-(2-hydroxypropan-2-yl)-N'-((2-methyl-3-phenyl-6,7-dihydro-5H-cyclopenta[b]pyridin-4-yl)carbamoyl)thiophene-2-sulfonimidamide | CHIRAL ART Cellulose-SB, 2*25 cm, 5 um | 15% EtOH in Hex (0.3% FA) | 489 |
| 846 | **1115a** | 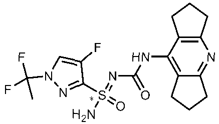 | (R) or (S)-1-(1,1-difluoroethyl)-4-fluoro-N'-((1,2,3,5,6,7-hexahydrodicyclopenta[b,e]pyridi n-8-yl)carbamoyl)-1H-pyrazole-3-sulfonimidamide | CHIRALP AK IG 2*25 cm, 5 um | 40% EtOH in Hex (0.1% FA) | 429 |
| 847 | **1115b** | 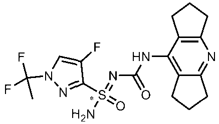 | (S) or (R)-1-(1,1-difluoroethyl)-4-fluoro-N'-((1,2,3,5,6,7-hexahydrodicyclopenta[b,e]pyridi n-8-yl)carbamoyl)-1H-pyrazole-3-sulfonimidamide | CHIRALP AK IG 2*25 cm, 5 um | 40% EtOH in Hex (0.1% FA) | 429 |
| 848 | **1116a** | 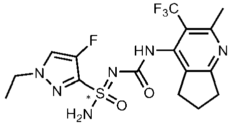 | (R) or (S) -1-ethyl-4-fluoro-N'-((2-methyl-3-(trifluoromethyl)-6,7-dihydro-5H-cyclopenta[b]pyridin-4-yl)carbamoyl)-1H-pyrazole-3-sulfonimidamide | CHIRALP AK IE, 2*25 cm, 5 um | 50% EtOH in Hex:DC M= 3:1 (10 mM NH₃·MeO H) | 435 |
| 849 | **1116b** | 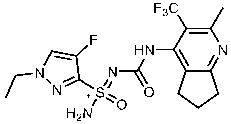 | (S) or (R) -1-ethyl-4-fluoro-N'-((2-methyl-3-(trifluoromethyl)-6,7-dihydro-5H-cyclopenta[b]pyridin-4-yl)carbamoyl)-1H-pyrazole-3-sulfonimidamide | CHIRALP AK IE, 2*25 cm, 5 um | 50% EtOH in Hex:DC M= 3:1 (10 mM NH₃·MeO H) | 435 |
| 850 | **1117a** | 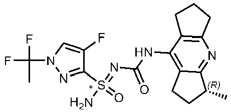 | (R) or (S) -1-(1,1-difluoroethyl)-4-fluoro-N'-(((R)-3-methyl-1,2,3,5,6,7-hexahydrodicyclopenta[b,e]pyridi n-8-yl)carbamoyl)-1H-pyrazole-3-sulfonimidamide | CHIRALP AK IE, 2*25 cm, 5 um | 50% IPA in Hex (0.1% FA) | 443 |
| 851 | **1117b** | 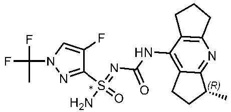 | (S) or (R) -1-(1,1-difluoroethyl)-4-fluoro-N'-(((R)-3-methyl-1,2,3,5,6,7-hexahydrodicyclopenta[b,e]pyridi n-8-yl)carbamoyl)-1H-pyrazole-3-sulfonimidamide | CHIRALP AK IE, 2*25 cm, 5 um | 50% IPA in Hex (0.1% FA) | 443 |

**Table 73A Examples in the following table were obtained from chiral HPLC resolutions of racemic and diastereomeric mixture examples described above. The chiral column and eluents are listed in the table. As a convention, the faster-eluting enantiomer is always listed first in the table followed by the slower-eluting enantiomer of the pair. The symbol * at a chiral center denotes that this chiral center has been resolved and the absolute stereochemistry at that center has not been determined. Assigned stereochemistry in compound names are tentative.**

| Ex. # | Structure | IUPAC Name | Column | Eluents | LC-MS [M+H]⁺ |
|---|---|---|---|---|---|
| 863 | 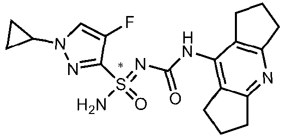 | (R) or (S)-1-cyclopropyl-4-fluoro-N'-((1,2,3,5,6,7-hexahydrodicyclopenta[b,e]pyridin -8-yl)carbamoyl)-1H-pyrazole-3-sulfonimidamide | Chiralpak IC, 2*25 cm, 5 um | 20% EtOH in Hex:DCM= 3:1 (10 mM NH₃·MeOH) | 405 |
| 864 | 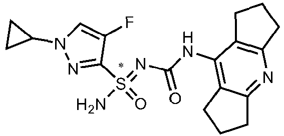 | (S) or (R)-1-cyclopropyl-4-fluoro-N'-((1,2,3,5,6,7-hexahydrodicyclopenta[b,e]pyridin -8-yl)carbamoyl)-1H-pyrazole-3-sulfonimidamide | Chiralpak IC, 2*25 cm, 5 um | 20% EtOH in Hex:DCM= 3:1 (10 mM NH₃·MeOH) | 405 |
| 865 | 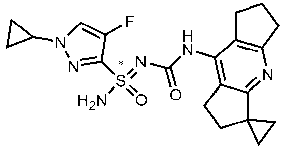 | (R) or (S)-1-cyclopropyl-4-fluoro-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | Chiralpak ID, 2*25 cm, 5 um | 20% EtOH in Hex:DCM= 3:1 (10 mM NH₃·MeOH) | 431 |
| 866 | 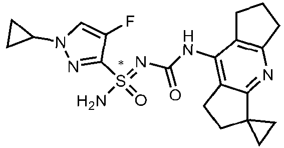 | (S) or (R)-1-cyclopropyl-4-fluoro-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | Chiralpak ID, 2*25 cm, 5 um | 20% EtOH in Hex:DCM= 3:1 (10 mM NH₃·MeOH) | 431 |
| 867 | 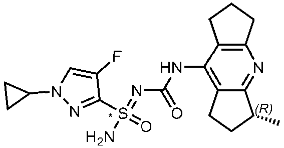 | (R) or (S)-1-cyclopropyl-4-fluoro-N'-(((R)-3-methyl-1,2,3,5,6,7-hexahydrodicyclopenta[b,e]pyridin -8-yl)carbamoyl)-1H-pyrazole-3-sulfonimidamide | CHIRALP AK ID, 2*25 cm, 5 um | 20% EtOH in Hex:DCM= 3:1 (10 mM NH₃·MeOH) | 419 |
| 868 | 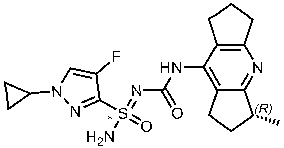 | (S) or (R)-1-cyclopropyl-4-fluoro-N'-(((R)-3-methyl-1,2,3,5,6,7-hexahydrodicyclopenta[b,e]pyridin -8-yl)carbamoyl)-1H-pyrazole-3-sulfonimidamide | CHIRALP AK ID, 2*25 cm, 5 um | 20% EtOH in Hex:DCM= 3:1 (10 mM NH₃·MeOH) | 419 |
| 869 | 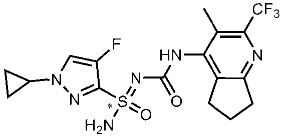 | (R) or (S)-1-cyclopropyl-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | CHIRALP AK IE, 2*25 cm, 5 um | 30% EtOH in Hex (0.1% FA) | 447 |
| 870 | 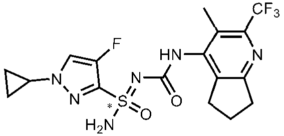 | (S) or (R)-1-cyclopropyl-4-fluoro-N'-((3-methyl-2-(trifluoromethyl)-6,7-dihydro-5H-cyclopenta[b]pyridin-4-yl)carbamoyl)-1H-pyrazole-3-sulfonimidamide | CHIRALP AK IE, 2*25 cm, 5 um | 30% EtOH in Hex (0.1% FA) | 447 |
| 871 | 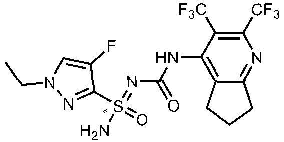 | (R) or (S)-N'-((2,3-bis(trifluoromethyl)-6,7-dihydro-5H-cyclopenta[b]pyridin-4-yl)carbamoyl)-1-ethyl-4-fluoro-1H-pyrazole-3-sulfonimidamide | CHIRALP AK IE, 2*25 cm, 5 um | 30% EtOH in Hex (0.1% FA) | 489 |
| 872 | 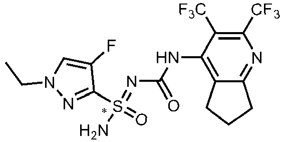 | (S) or (R)-N'-((2,3-bis(trifluoromethyl)-6,7-dihydro-5H-cyclopenta[b]pyridin-4-yl)carbamoyl)-1-ethyl-4-fluoro-1H-pyrazole-3-sulfonimidamide | CHIRALP AK IE, 2*25 cm, 5 um | 30% EtOH in Hex (0.1% FA) | 489 |

**Table 74. Examples in the following table were prepared using similar conditions as described in Example 252 and Scheme V from appropriate starting materials.**

| Example # | Compound # | Structure | IUPAC Name | Exact Mass [M+H]⁺ |
|---|---|---|---|---|
| 852 | **1118** | 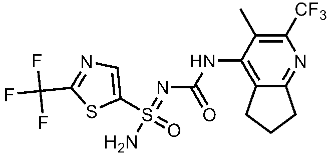 | N'-((3-methyl-2-(trifluoromethyl)-6,7-dihydro-5H-cyclopenta[b]pyridin-4-yl)carbamoyl)-2-(trifluoromethyl)thiazole-5-sulfonimidamide | 474 |
| 853 | **1119** | 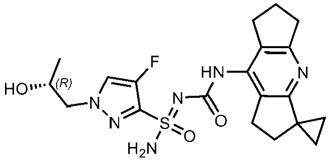 | 4-Fluoro-1-((R)-2-hydroxypropyl)-N'-((1',5',6',7'-tetrahydro-2'H-spiro[cyclopropane-1,3'-dicyclopenta[b,e]pyridin]-8'-yl)carbamoyl)-1H-pyrazole-3-sulfonimidamide | 449 |

The following protocol is suitable for testing the activity of the compounds disclosed herein.

### Procedure 1: IL-1β production in PMA-differentiated THP-1 cells stimulated with Gramicidin.

THP-1 cells were purchased from the American Type Culture Collection and sub-cultured according to instructions from the supplier. Cells were cultured in complete RPMI 1640 (containing 10% heat inactivated FBS, penicillin (100 units/ml) and streptomycin (100 µg/ml)), and maintained in log phase prior to experimental setup. Prior to the experiment, compounds were dissolved in dimethyl sulfoxide (DMSO) to generate a 30mM stock. The compound stock was first pre-diluted in DMSO to 3, 0.34, 0.042 and 0.0083 mM intermediate concentrations and subsequently spotted using Echo550 liquid handler into an empty 384-well assay plate to achieve desired final concentration (e.g. 100, 33, 11, 3.7, 1.2, 0.41, 0.14, 0.046, 0.015, 0.0051, 0.0017 µM). DMSO was backfilled in the plate to achieve a final DMSO assay concentration of 0.37%. The plate was then sealed and stored at room temperature until required.

THP-1 cells were treated with PMA (Phorbol 12-myristate 13-acetate) (20 ng/ml) for 16-18 hours. On the day of the experiment the media was removed and adherent cells were detached with trypsin for 5 minutes. Cells were then harvested, washed with complete RPMI 1640, spun down, and resuspended in RPMI 1640 (containing 2% heat inactivated FBS, penicillin (100 units/ml) and streptomycin (100 µg/ml). The cells were plated in the 384-well assay plate containing the spotted compounds at a density of 50,000 cells/well (final assay volume 50 µl). Cells were incubated with compounds for 1 hour and then stimulated with gramicidin (5µM) (Enzo) for 2 hours. Plates were then centrifuged at 340g for 5 min. Cell free supernatant (40µL) was collected using a 96-channel PlateMaster (Gilson) and the production of IL-1β was evaluated by HTRF (cisbio). The plates were incubated for 18 h at 4°C and read using the preset HTRF program (donor emission at 620 nm, acceptor emission at 668 nm) of the SpectraMax i3x spectrophotometer (Molecular Devices, software SoftMax 6). A vehicle only control and a dose titration of CRID3 (100 - 0.0017 µM) were run concurrently with each experiment. Data was normalized to vehicle-treated samples (equivalent to 0% inhibition) and CRID3 at 100 µM (equivalent to 100% inhibition). Compounds exhibited a concentration-dependent inhibition of IL-1β production in PMA-differentiated THP-1 cells.

### Procedure 2: IL-1β production in PMA-differentiated THP-1 cells stimulated with Gramicidin.

THP-1 cells were purchased from the American Type Culture Collection and sub-cultured according to instructions from the supplier. Prior to experiments, cells were cultured in complete RPMI 1640 (containing 10% heat inactivated FBS, penicillin (100 units/ml) and streptomycin (100 µg/ml)), and maintained in log phase prior to experimental setup. Prior to the experiment THP-1 were treated with PMA (Phorbol 12-myristate 13-acetate) (20 ng/ml) for 16-18 hours. Compounds were dissolved in dimethyl sulfoxide (DMSO) to generate a 30mM stock. On the day of the experiment the media was removed and adherent cells were detached with trypsin for 5 minutes. Cells were then harvested, washed with complete RPMI 1640, spun down, resuspended in RPMI 1640 (containing 2% heat inactivated FBS, penicillin (100 units/ml) and streptomycin (100 µg/ml). The cells were plated in a 384-well plate at a density of 50,000 cells/well (final assay volume 50 µl). Compounds were first dissolved in assay medium to obtain a 5x top concentration of 500µM. 10 step dilutions (1:3) were then undertaken in assay medium containing 1.67% DMSO. 5x compound solutions were added to the culture medium to achieve desired final concentration (e.g. 100, 33, 11, 3.7, 1.2, 0.41, 0.14, 0.046, 0.015, 0.0051, 0.0017 µM). Final DMSO concentration was at 0.37%. Cells were incubated with compounds for 1 hour and then stimulated with gramicidin (5µM) (Enzo) for 2 hours. Plates were then centrifuged at 340g for 5 min. Cell free supernatant (40µL) was collected using a 96-channel PlateMaster (Gilson) and the production of IL-1β was evaluated by HTRF (cisbio). A vehicle only control and a dose titration of CRID3 (100 - 0.0017 µM) were run concurrently with each experiment. Data was normalized to vehicle-treated samples (equivalent to 0% inhibition) and CRID3 at 100 µM (equivalent to 100% inhibition). Compounds exhibited a concentration-dependent inhibition of IL-1β production in PMA-differentiated THP-1 cells.

**Tables B1, B2, B3,** and **B4** show the biological activity of compounds in hTHP-1 assay containing 2% fetal bovine serum. Table **B5** and **B6** shows the biological activity of compounds in hTHP-1 assay containing 10% fetal bovine serum. Key: <0.008 µM = "++++++"; ≥0.008 and <0.04 µM = "+++++"; ≥0.04 and <0.2 µM = "++++"; ≥0.2 and <1 µM = "+++"; ≥1 and <5 µM = "++"; ≥5 and <30 µM = "+". ND = not determined.

**Table B1. Average IC₅₀ of compounds in hTHP-1 assay**

| Example # | Compound Number | hTHP-1 IC50 |
|---|---|---|
| 1 | 165 | ++ |
| 2 | 165a | +++ |
| 3 | 165b | + |
| 4 | 163 | +++ |
| 5 | 128 | +++ |
| 6 | 110 | + |
| 7 | 164 | ++ |
| 8 | 167 | ++ |
| 9 | 159 | ++ |
| 10 | 135 | ++ |
| 11 | 119 | ++ |
| 12 | 122 | +++ |
| 13 | 125 | ++ |
| 14 | 126 | ++++ |
| 15 | 101 | ++++ |
| 16 | 114 | ++++ |
| 17 | 117 | + |
| 18 | 116 | + |
| 19 | 130 | + |
| 20 | 170 | ++ |
| 21 | 171 | ++ |
| 22 | 172 | +++ |
| 23 | 173 | +++ |
| 24 | 174 | +++ |
| 25 | 175 | +++ |
| 26 | 176 | + |
| 27 | 177 | + |
| 28 | 118 | ++ |
| 29 | 150 | ++ |
| 30 | 121 | +++ |
| 31 | 178 | + |
| 32 | 179 | ++ |
| 33 | | |
| 34 | 107 | +++ |
| 35 | 155 | ++++ |
| 36 | 166 | + |
| 37 | 149 | +++ |
| 38 | 105 | ++++ |
| 39 | 141 | ++++ |
| 40 | 103 | ++++ |
| 41 | 127 | ++++ |
| 42 | 102 | ++++ |
| 43 | 131 | +++ |
| 44 | 123 | ++ |
| 45 | 180 | ++ |
| 46 | 113 | > 30 |
| 47 | 181 | +++ |
| 48 | 182 | > 14 |
| 49 | 183 | + |
| 50 | 154 | +++ |
| 51 | 120 | +++ |
| 52 | 162 | ++++ |
| 53 | 104 | +++ |
| 54 | 168 | +++ |
| 55 | 158 | ++ |
| 56 | 157 | + |
| 57 | 156 | > 30 |
| 58 | 160 | + |
| 59 | 129 | +++ |
| 60 | 161 | ++ |
| 61 | 153 | ++++ |
| 62 | 152 | +++ |
| 63 | 151 | +++ |
| 64 | 147 | + |
| 65 | 146 | + |
| 66 | 133 | > 14 |
| 67 | 112 | > 30 |
| 68 | 115 | ++ |
| 69 | 106 | ++++ |
| 70 | 148 | ++++ |
| 71 | 132 | > 27 |
| 72 | 140 | ++++ |
| 73 | 139 | +++ |
| 74 | 134 | ++ |
| 75 | 145 | + |
| 76 | 144 | > 30 |
| 77 | 143 | > 30 |
| 78 | 142 | > 30 |
| 79 | 138 | + |
| 80 | 137 | > 30 |
| 81 | 136 | ++++ |
| 82 | 124 | +++ |
| 83 | 128a | +++ |
| 84 | 128b | + |
| 85 | 150a | + |
| 86 | 150b | +++ |
| 87 | 163a | ++++ |
| 88 | 163b | + |
| 91 | 167a | + |
| 92 | 167b | +++ |
| 93 | 164b | + |
| 94 | 164a | ++ |
| 95 | 161a | + |
| 96 | 161b | ++ |
| 97 | 159a | ++ |
| 98 | 159b | + |
| 99 | 158a | ++ |
| 100 | 158b | > 30 |
| 101 | 157a | > 30 |
| 102 | 157b | + |
| 103 | 160a | ++ |
| 104 | 160b | + |
| 105 | 162ab | ++ |
| 106 | 162aa | + |
| 107 | 162bb | ++++ |
| 108 | 162ba | +++ |
| 109 | 156a | > 30 |
| 110 | 156b | ++ |
| 111 | 155a | ++++ |
| 112 | 155b | ++ |
| 113 | 149a | +++ |
| 114 | 149b | > 30 |
| 115 | 106a | ++++ |
| 116 | 106b | ++ |
| 117 | 148a | +++ |
| 118 | 148b | ++ |
| 119 | 147a | > 30 |
| 120 | 147b | ++ |
| 121 | 107a | +++ |
| 122 | 107b | + |
| 123 | 145a | +++ |
| 124 | 145b | ++ |
| 125 | 105a | ++++ |
| 126 | 105b | ++ |
| 127 | 153a | + |
| 128 | 153b | ++++ |
| 129 | 133a | > 30 |
| 130 | 133b | > 30 |
| 131 | 137a | > 10 |
| 132 | 137b | > 30 |
| 133 | 136a | ++ |
| 134 | 136b | ++++ |
| 135 | 140a | ++++ |
| 136 | 140b | + |
| 137 | 140aa | ++++ |
| 138 | 140a b | ++ |
| 139 | 140ba | + |
| 140 | 140bb | > 30 |
| 141 | 129aa | ++++ |
| 142 | 129ab | > 30 |
| 143 | 129ba | ++ |
| 144 | 129bb | + |
| 145 | 127a | ++ |
| 146 | 127b | ++++ |
| 147 | 130a | > 30 |
| 148 | 130b | ++ |
| 149 | 126a | ++++ |
| 150 | 126b | + |
| 151 | 168a | + |
| 152 | 168b | +++ |
| 153 | 141b | ++++ |
| 154 | 141a | ++ |
| 155 | 141aa | +++++ |
| 156 | 141ab | +++ |
| 157 | 141ba | ++ |
| 158 | 141bb | + |
| 159 | 104a | +++ |
| 160 | 104b | + |
| 161 | 168aa | + |
| 162 | 168ab | > 30 |
| 163 | 168ba | +++ |
| 164 | 168bb | ++ |
| 165 | 122a | ++++ |
| 166 | 122b | + |
| 167 | 103aa | ++ |
| 168 | 103ab | + |
| 169 | 103ba | +++++ |
| 170 | 103bb | +++ |
| 171 | 102a | ++ |
| 172 | 102b | +++++ |
| 173 | 101a | ++ |
| 174 | 101b | +++++ |
| 175 | 125a | > 30 |
| 176 | 125b | ++ |
| 177 | 132a | + |
| 178 | 132b | > 30 |
| 179 | 131b | + |
| 180 | 131a | +++ |
| 181 | 131aab | +++ |
| 182 | 131c | + |
| 183 | 131d | + |
| 184 | 131e | + |
| 185 | 131f | ++ |
| 186 | 131g | +++ |
| 187 | 121a | ++++ |
| 188 | 121b | + |
| 189 | 169a | + |
| 190 | 169b | ++++ |
| 191 | 119a | ++ |
| 192 | 119b | > 30 |
| 193 | 118a | +++ |
| 194 | 118b | > 24 |
| 195 | 134aa | > 30 |
| 196 | 134ab | > 30 |
| 197 | 134ba | ++ |
| 198 | 134bb | + |
| 199 | 117a | > 30 |
| 200 | 117b | + |
| 201 | 172a | + |
| 202 | 172b | +++ |
| 203 | 173a | +++ |
| 204 | 173b | > 20 |
| 205 | 183a | ++ |
| 206 | 183b | > 30 |
| 207 | 183c | + |
| 208 | 183d | > 30 |
| 209 | 116a | > 30 |
| 210 | 116b | + |
| 211 | 114a | ++++ |
| 212 | 114b | ++ |
| 213 | 124a | + |
| 214 | 124b | +++ |
| 215 | 154a | ++ |
| 216 | 154b | > 28 |
| 217 | 120a | > 30 |
| 218 | 120b | > 30 |
| 219 | 142a | > 30 |
| 220 | 142b | > 30 |
| 221 | 143a | > 30 |
| 222 | 143b | > 30 |
| 223 | 184a | ++++ |
| 224 | 184b | + |
| 225 | 184c | + |
| 226 | 184d | ++ |
| 227 | 174a | + |
| 228 | 174b | ++++ |
| 229 | 175a | + |
| 230 | 175b | +++ |
| 231 | 180a | +++ |
| 232 | 180b | > 30 |
| 33 | 111 | > 30 |
| | 185b | > 30 |
| | 185a | + |
| | 185 | > 30 |
| | 201a | ++++ |
| | | |

**Table B2**

| **Example #** | **Compound #** | **hTHP-1 IC50 (uM)** |
|---|---|---|
| 233 | 652 | ++ |
| 234 | 652b | > 30 |
| 235 | 652a | +++ |
| 236 | 695 | + |
| 237 | 643 | + |
| 238 | 201 | +++ |
| 239 | 640 | +++ |
| 240 | 605 | +++ |
| 241 | 605f | +++ |
| 242 | 605a | +++ |
| 243 | 691 | ++ |
| 244 | 688 | ++ |
| 245 | 676 | ++ |
| 246 | 669 | + |
| 247 | 612 | ++ |
| 248 | 627 | +++ |
| 249 | 607 | ++ |
| 250 | 665 | > 30 |
| 251 | 693 | ++ |
| 252 | 645 | ++++ |
| 253 | 672 | + |
| 254 | 702 | ++ |
| 255 | 692 | ++ |
| 256 | 656 | ++ |
| 257 | 681 | ++++ |
| 258 | 668 | + |
| 259 | 658 | + |
| 260 | 667 | +++ |
| 261 | 703 | + |
| 262 | 664 | ++ |
| 263 | 632 | +++ |
| 264 | 624 | ++++ |
| 265 | 631 | ++++ |
| 266 | 630 | +++ |
| 267 | 623 | +++ |
| 268 | 621 | ++ |
| 269 | 629 | ++ |
| 270 | 610 | + |
| 271 | 611 | +++ |
| 272 | 609 | ++ |
| 273 | 608 | ++ |
| 274 | 616 | ++++ |
| 275 | 604 | +++ |
| 276 | 603 | ++++ |
| 277 | 304 | ++ |
| 278 | 306 | + |
| 279 | 677 | ++ |
| 280 | 661 | ++ |
| 281 | 647 | ++ |
| 282 | 619 | + |
| 283 | 618 | ++ |
| 284 | 626 | ++++ |
| 285 | 696 | + |
| 286 | 682 | ++ |
| 287 | 653 | ND |
| 288 | 641 | + |
| 289 | 694 | ++ |
| 290 | 687 | + |
| 291 | 660 | + |
| 292 | 140c | +++ |
| 293 | 635 | + |
| 294 | 689 | ++ |
| 295 | 642 | ++++ |
| 296 | 686 | +++ |
| 297 | 680 | ++ |
| 298 | 674 | ++ |
| 299 | 684 | +++ |
| 300 | 683 | ++ |
| 301 | 679 | + |
| 302 | 673 | + |
| 303 | 704 | ++ |
| 304 | 664 | + |
| 305 | 663 | ++ |
| 306 | 651 | + |
| 307 | 659 | ++ |
| 308 | 662 | + |
| 309 | 649 | + |
| 310 | 650 | +++ |
| 311 | 648 | ++ |
| 312 | 615 | ++ |
| 313 | 620 | + |
| 314 | 185 | > 30 |
| 315 | 690 | ++ |
| 316 | 675 | ++ |
| 317 | 678 | ++ |
| 318 | 671 | ++ |
| 319 | 657 | + |
| 320 | 670 | +++ |
| 321 | 655 | + |
| 322 | 654 | +++ |
| 323 | 634 | ++++ |
| 324 | 639 | ++ |
| 325 | 646a | ++ |
| 326 | 638 | + |
| 327 | 637 | + |
| 328 | 633 | > 30 |
| 329 | 625 | + |
| 330 | 622 | + |
| 331 | 628 | +++ |
| 332 | 617 | + |
| 333 | 614 | +++ |
| 334 | 613 | +++ |
| 335 | 602 | ++ |
| 336 | 636c | +++ |
| 337 | 601 | + |
| 338 | 685 | ++ |
| 339 | 685b | ++ |
| 340 | 685a | > 30 |
| 341 | 694b | ++ |
| 342 | 694a | > 30 |
| 343 | 687b | + |
| 344 | 687a | > 30 |
| 345 | 689b | + |
| 346 | 689a | > 30 |
| 347 | 642b | ++++ |
| 348 | 642a | + |
| 349 | 686b | + |
| 350 | 686a | +++ |
| 351 | 170b | + |
| 352 | 170a | +++ |
| 353 | 680b | ++ |
| 354 | 680a | + |
| 355 | 682b | ++ |
| 356 | 682a | > 30 |
| 357 | 653b | > 30 |
| 358 | 653a | + |
| 359 | 674b | ++ |
| 360 | 674a | > 30 |
| 361 | 684b | + |
| 362 | 684a | ++++ |
| 363 | 683b | + |
| 364 | 683a | +++ |
| 365 | 673b | > 30 |
| 366 | 673a | ++ |
| 367 | 705b | ++ |
| 368 | 705a | + |
| 369 | 664b | ++ |
| 370 | 664a | > 30 |
| 371 | 663b | ++ |
| 372 | 663a | > 30 |
| 373 | 651b | ++ |
| 374 | 651a | + |
| 375 | 659b | +++ |
| 376 | 659a | + |
| 377 | 649b | > 30 |
| 378 | 649a | + |
| 379 | 650b | + |
| 380 | 650a | ++++ |
| 381 | 648b | > 30 |
| 382 | 648a | +++ |
| 383 | 615b | ++ |
| 384 | 615a | > 30 |
| 385 | 620b | ++ |
| 386 | 620a | > 30 |
| 387 | 185a | + |
| 388 | 185b | > 30 |
| 389 | 115b | ++ |
| 390 | 115a | + |
| 391 | 701b | > 30 |
| 392 | 701a | + |
| 393 | 692b | ++ |
| 394 | 692a | + |
| 395 | 695b | + |
| 396 | 695a | > 30 |
| 397 | 691b | ++ |
| 398 | 691a | + |
| 399 | 688b | + |
| 400 | 688a | +++ |
| 401 | 690b | ++ |
| 402 | 690a | > 30 |
| 403 | 661b | + |
| 404 | 661a | +++ |
| 405 | 647b | > 30 |
| 406 | 647a | ++ |
| 407 | 676b | ++ |
| 408 | 676a | > 30 |
| 409 | 181b | ++++ |
| 410 | 181a | ++ |
| 411 | 675b | + |
| 412 | 675a | ++ |
| 413 | 681b | +++ |
| 414 | 681a | ++ |
| 415 | 669b | + |
| 416 | 669a | > 30 |
| 417 | 176b | ++ |
| 418 | 176a | > 30 |
| 419 | 182b | + |
| 420 | 182a | > 30 |
| 421 | 678b | > 30 |
| 422 | 678a | ++ |
| 423 | 658b | ++ |
| 424 | 658a | > 30 |
| 425 | 667b | +++ |
| 426 | 667a | + |
| 427 | 657b | + |
| 428 | 657a | > 30 |
| 429 | 670b | ++++ |
| 430 | 670a | + |
| 431 | 612b | ++ |
| 432 | 612a | > 30 |
| 433 | 626b | ++++ |
| 434 | 626a | + |
| 435 | 665b | ++ |
| 436 | 665a | > 30 |
| 437 | 654b | +++ |
| 438 | 654a | ++ |
| 439 | 634b | ++++ |
| 440 | 634a | + |
| 441 | 639b | +++ |
| 442 | 639a | > 30 |
| 443 | 700b | +++ |
| 444 | 700a | > 30 |
| 445 | 638b | > 30 |
| 446 | 638a | ++ |
| 447 | 637b | > 30 |
| 448 | 637b | ++ |
| 449 | 645b | + |
| 450 | 645a | ++++ |
| 451 | 644b | ++ |
| 452 | 644a | > 30 |
| 453 | 633b | + |
| 454 | 633a | > 30 |
| 455 | 625b | ++ |
| 456 | 625a | > 30 |
| 457 | 632b | + |
| 458 | 632a | ++++ |
| 459 | 624b | ++++ |
| 460 | 624a | ++ |
| 461 | 631b | ++++ |
| 462 | 631a | + |
| 463 | 630b | +++ |
| 464 | 630a | + |
| 465 | 623b | + |
| 466 | 623a | ++++ |
| 467 | 622b | + |
| 468 | 622a | + |
| 469 | 621b | ++ |
| 470 | 621a | + |
| 471 | 618b | +++ |
| 472 | 618a | > 30 |
| 473 | 628b | +++ |
| 474 | 628a | > 30 |
| 475 | 617b | > 30 |
| 476 | 617a | + |
| 477 | 610b | + |
| 478 | 610a | ++ |
| 479 | 611b | +++ |
| 480 | 611a | + |
| 481 | 698b | +++ |
| 482 | 698a | ++ |
| 483 | 616b | + |
| 484 | 616a | ++++ |
| 485 | 614b | ++ |
| 486 | 614a | ++++ |
| 487 | 613b | ++ |
| 488 | 613a | ++++ |
| 489 | 697b | ++++ |
| 490 | 697a | + |
| 491 | 607b | ++ |
| 492 | 607a | > 30 |
| 493 | 636b | ++++ |
| 494 | 636a | + |
| 495 | 304b | ++ |
| 496 | 304a | > 30 |
| 497 | 306b | + |
| 498 | 306a | + |
| 499 | 605e | + |
| 500 | 605c | +++ |
| 501 | 605g | + |
| 502 | 605h | +++ |
| 503 | 660d | + |
| 504 | 660c | > 30 |
| 505 | 660b | > 30 |
| 506 | 660a | > 30 |
| 507 | 201f | + |
| 508 | 201e | ++++ |
| 509 | 201d | + |
| 510 | 201c | + |
| 511 | 201a | +++++ |
| 512 | 201b | ++++ |
| 513 | 662c | ND |
| 514 | 662e | ++ |
| 515 | 662d | + |
| 516 | 662b | > 30 |
| 517 | 662a | > 30 |
| 518 | 699c | + |
| 519 | 699b | +++++ |
| 520 | 699a | > 30 |
| 521 | 640c | ++ |
| 522 | 640b | ++++ |
| 523 | 640a | +++++ |
| 524 | 656d | ++ |
| 525 | 656b | + |
| 526 | 656a | +++ |
| 527 | 656c | + |
| 528 | 668c | + |
| 529 | 668b | + |
| 530 | 668a | > 30 |
| 531 | 668d | > 30 |
| 532 | 671d | +++ |
| 533 | 671c | ++ |
| 534 | 671b | + |
| 535 | 671a | > 30 |
| 536 | 605d | > 30 |
| 537 | 605b | +++ |
| 538 | 643a | + |
| 539 | 643b | + |
| 540 | 171c | ++ |
| 541 | 171b | + |
| 542 | 171a | > 30 |
| 543 | 171d | > 30 |
| 544 | 734 | +++ |
| 545 | 736 | ++ |
| 546 | 735 | ++ |
| 547 | 721c | ++++ |
| 548 | 720c | +++ |
| 549 | 723c | +++ |
| 550 | 729 | +++ |
| 553 | 723b | ++++ |
| 554 | 723a | + |
| 555 | 721b | ++++ |
| 556 | 721a | + |
| 557 | 720b | + |
| 558 | 720a | +++ |
| 559 | 729b | ++++ |
| 560 | 729a | + |
| | 601a | +++ |
| | 601b | + |
| | 602a | > 30.0000 |
| | 602b | ++ |
| | 604a | + |
| | 604b | +++ |
| | 608a | +++ |
| | 608b | + |
| | 608c | +++ |
| | 608d | ++ |
| | 706 | > 30.0000 |
| | 707 | > 30.0000 |
| | 708 | > 30.0000 |
| | 709 | > 30.0000 |
| | 710 | > 30.0000 |
| | 711 | > 30.0000 |
| | 712 | > 30.0000 |
| | 713 | ++ |
| | 716a | ++++ |
| | 716b | +++ |
| | 717 | +++ |
| | 718a | ++++ |
| | 719 | +++ |
| | 722 | ++ |
| | 724 | +++ |
| | 725a | ++ |
| | 726 | ++ |
| | 726a | > 30.0000 |
| | 726b | +++ |
| | 727 | +++ |
| | 727a | ++ |
| | 727b | ++++ |
| | 728 | ++ |
| | 728a | + |
| | 728b | +++ |
| | 730 | + |
| | 730a | + |
| | 730b | ++ |
| | 731 | ++ |
| | 731a | + |
| | 731b | +++ |
| | 733 | ++ |
| | 737 | +++ |
| | 738 | > 30.0000 |

**Table B3**

| **Example #** | **IC₅₀ (µM)** |
|---|---|
| 561 | +++ |
| 562 | +++ |
| 563 | + |
| 564 | ++ |
| 565 | + |
| 566 | +++ |
| 567 | + |
| 568 | ++++ |
| 569 | +++ |
| 570 | +++ |
| 571 | ++ |
| 572 | ++ |
| 573 | ++ |
| 574 | ++ |
| 575 | ++ |
| 576 | +++ |
| 577 | ++ |
| 578 | ++ |
| 579 | ++ |
| 580 | ++ |
| 581 | > 30 |
| 582 | ++++ |
| 583 | ++ |
| 584 | > 30 |
| 585 | ++ |
| 586 | +++ |
| 587 | + |
| 588 | ++ |
| 589 | ++ |
| 590 | ++ |
| 591 | ++ |
| 592 | > 30 |
| 593 | > 30 |
| 594 | ++ |
| 595 | ++ |
| 596 | ++ |
| 597 | ++++ |
| 598 | +++ |
| 599 | ++ |
| 600 | +++ |
| 601 | + |
| 602 | +++++ |
| 603 | ++ |
| 604 | ++++ |
| 605 | +++ |
| 606 | > 30 |
| 607 | +++ |
| 608 | ++++ |
| 609 | +++ |
| 610 | +++ |
| 611 | ++ |
| 612 | ++ |
| 613 | > 30 |
| 614 | > 30 |
| 615 | +++ |
| 616 | +++ |
| 617 | + |
| 618 | ++++ |
| 619 | ++ |
| 620 | +++ |
| 621 | ++++ |
| 622 | ++ |
| 623 | +++ |
| 624 | +++ |
| 625 | ++ |
| 626 | +++ |
| 627 | +++ |
| 628 | ++++ |
| 629 | +++ |
| 630 | + |
| 631 | ++ |
| 632 | ++ |
| 633 | + |
| 634 | ++ |
| 635 | > 30 |
| 636 | > 30 |
| 637 | +++ |
| 638 | +++++ |
| 639 | +++ |
| 640 | + |
| 641 | ++ |
| 642 | > 30 |
| 643 | ++ |
| 644 | > 30 |
| 645 | + |
| 646 | ++++ |
| 647 | +++ |
| 648 | + |
| 649 | ++++ |
| 650 | ++ |
| 651 | ++++ |
| 652 | + |
| 653 | ++++ |
| 654 | + |
| 655 | > 30 |
| 656 | ++ |
| 657 | ++ |
| 658 | > 30 |
| 659 | +++ |
| 660 | + |
| 661 | ++ |
| 662 | + |
| 663 | ++++ |
| 664 | ++++ |
| 665 | +++ |
| 666 | + |
| 667 | ++ |
| 668 | > 30 |
| 669 | +++++ |
| 670 | ++ |
| 671 | +++++ |
| 672 | ++ |
| 673 | + |
| 674 | +++ |
| 675 | + |
| 676 | +++ |
| 677 | + |
| 678 | +++++ |
| 679 | ++++ |
| 680 | + |
| 681 | ++++ |
| 682 | ++ |
| 683 | ++ |
| 684 | > 30 |
| 685 | +++ |
| 686 | > 30 |
| 687 | +++ |
| 688 | + |
| 689 | ++ |
| 690 | > 30 |
| 691 | +++ |
| 692 | + |
| 693 | ++ |
| 694 | > 30 |
| 695 | ++ |
| 696 | + |
| 697 | ++++ |
| 698 | ++ |
| 699 | +++ |
| 700 | + |
| 701 | + |
| 702 | > 30 |
| 703 | ++ |
| 704 | +++ |
| 705 | + |
| 706 | +++ |
| 707 | +++++ |
| 708 | + |
| 709 | ++ |
| 710 | + |
| 711 | ++++ |
| 712 | + |
| 713 | ++++ |
| 714 | ++ |
| 715 | ++++++ |
| 716 | ++ |
| 717 | ++ |
| 718 | > 30 |
| 719 | ++ |
| 720 | ^{~}30 |
| 721 | ++++ |
| 722 | ++ |
| 723 | ++ |
| 724 | > 30 |
| 725 | > 30 |
| 726 | +++ |
| 727 | ++++ |
| 728 | ++ |
| Compound 984a | + |
| Compound 984b | ++++ |
| Compound 983 | ++++ |
| Compound 985a | ++ |
| Compound 985b | +++ |

**Table B4**

| **Example #** | **hTHP-1 IC₅₀** |
|---|---|
| 729 | ++ |
| 730 | +++ |
| 731 | ++++ |
| 732 | ++ |
| 733 | ++++ |
| 734 | +++ |
| 735 | +++ |
| 736 | +++ |
| 737 | +++ |
| 738 | ++ |
| 739 | +++++ |
| 740 | ++++ |
| 741 | ++++ |
| 742 | +++++ |
| 743 | ++ |
| 744 | ++++ |
| 745 | ++ |
| 746 | +++++ |
| 747 | +++++ |
| 748 | +++ |
| 749 | ++ |
| 750 | +++ |
| 751 | ++ |
| 752 | ++ |
| 753 | +++ |
| 754 | ++ |
| 755 | +++ |
| 756 | + |
| 757 | +++++ |
| 758 | ++ |
| 759 | +++ |
| 760 | >30 |
| 761 | ++++ |
| 762 | + |
| 763 | +++ |
| 764 | + |
| 765 | +++++ |
| 766 | ++ |
| 767 | +++++ |
| 768 | +++ |
| 769 | + |
| 770 | ++++ |
| 771 | +++ |
| 772 | + |
| 773 | ++++ |
| 774 | + |
| 775 | ++++ |
| 776 | + |
| 777 | +++ |
| 778 | ++ |
| 779 | +++++ |
| 780 | +++ |
| 781 | +++++ |
| 782 | +++ |
| 783 | ++ |
| 784 | + |
| 785 | ++++ |
| 786 | + |
| 787 | +++++ |
| 788 | ++ |
| 789 | ++++ |
| 790 | + |
| 791 | +++ |
| 792 | >30 |
| 793 | +++ |
| 794 | + |

**Table B5.**

| **Example #** | **hTHP-1 IC₅₀ in 2% FBS** | **hTHP-1 IC₅₀ in 10% FBS** |
|---|---|---|
| 795 | ++ | ++ |
| 796 | +++++ | +++ |
| 797 | +++++ | +++++ |
| 798 | ++++ | ++++ |
| 799 | +++ | ND |
| 800 | ND | + |
| 801 | ND | ++++ |
| 802 | ND | +++ |
| 803 | ND | ++++ |
| 804 | ND | ++++ |
| 805 | ND | ++++ |
| 806 | ND | +++++ |
| 807 | ND | ++++ |
| 808 | ND | +++++ |
| 809 | ND | ++++ |
| 810 | ND | **ND** |
| 811 | ND | ++++ |
| 812 | ND | ++++ |
| 813 | +++++ | +++++ |
| 814 | ++ | ++ |
| 815 | ++++ | +++++ |
| 816 | + | ++ |
| 817 | + | ++ |
| 818 | +++ | ++++ |
| 819 | + | + |
| 820 | >30 µM | >30 µM |
| 821 | ++++ | ND |
| 822 | ++ | ++ |
| 823 | + | + |
| 824 | ND | +++ |
| 825 | ND | +++++ |
| 826 | ND | ++++ |
| 827 | ND | ++ |
| 828 | ND | + |
| 829 | ND | ++ |
| 830 | ND | ++++ |
| 831 | ND | ++ |
| 832 | ND | ++++ |
| 833 | ND | ++ |
| 834 | ND | ++++ |
| 835 | ND | +++ |
| 836 | ND | +++ |
| 837 | ND | +++++ |
| 838 | ND | ++++ |
| 839 | ND | ++ |
| 840 | ND | ++++ |
| 841 | ND | ++ |
| 842 | ND | +++++ |
| 843 | ND | +++ |
| 844 | ND | +++ |
| 845 | ND | ++++++ |
| 846 | ND | +++ |
| 847 | ND | + |
| 848 | ND | +++++ |
| 849 | ND | ++ |
| 850 | ND | +++++ |
| 851 | ND | +++ |
| 852 | ND | + |
| 853 | ND | +++++ |

**Table B6.**

| **Example #** | **hTHP-1 IC₅₀ in 10% FBS** |
|---|---|
| 854 | ++++ |
| 855 | +++++ |
| 856 | ++++ |
| 857 | ++++ |
| 858 | ++++ |
| 861 | ++++ |
| 863 | ++++ |
| 864 | +++ |
| 865 | ++++ |
| 866 | ++++ |
| 867 | ++++++ |
| 868 | +++ |
| 869 | +++++ |
| 870 | ++ |

### Study Example 1.

The CARD8 gene is located within the inflammatory bowel disease (IBD) 6 linkage region on chromosome 19. CARD8 interacts with NLRP3, and Apoptosis-associated Speck-like protein to form a caspase-1 activating complex termed the NLRP3 inflammasome. The NLRP3 inflammasome mediates the production and secretion of interleukin-1β, by processing pro-IL-1β into mature secreted IL-1β. In addition to its role in the inflammasome, CARD8 is also a potent inhibitor of nuclear factor NF-κB. NF-κB activation is essential for the production of pro-IL-1ϑ. Since over-production of IL-1β and dyregulation of NF-κB are hallmarks of Crohn's disease, CARD8 is herein considered to be a risk gene for inflammatory bowel disease. A significant association of CARD8 with Crohn's disease was detected in two British studies with a risk effect for the minor allele of the non-synonymous single-nucleotide polymorphism (SNP) of a C allele at rs2043211. This SNP introduces a premature stop codon, resulting in the expression of a severely truncated protein. This variant CARD8 protein is unable to suppress NF-κB activity, leading to constitutive production of pro-IL-1 β , which is a substrate for the NLRP3 inflammasome. It is believed that a gain-of-function mutation in an NLRP3 gene (e.g., any of the gain-of-function mutations described herein, e.g., any of the gain-of-function mutations in an NLRP3 gene described herein) combined with a loss-of-function mutation in a CARD8 gene (e.g., a C allele at rs2043211) results in the development of diseases related to increased NLRP3 inflammasome expression and/or activity. Patients having, e.g., a gain-of-function mutation in an NLRP3 gene and/or a loss-of-function mutation in a CARD8 gene are predicted to show improved therapeutic response to treatment with an NLRP3 antagonist.

A study is designed to determine: whether NLRP3 antagonists inhibit inflammasome function and inflammatory activity in cells and biopsy specimens from patients with Crohn's disease or ulcerative colitis; and whether the specific genetic variants identify patients with Crohn's disease or ulcerative colitis who are most likely to respond to treatment with an NLRP3 antagonist.

The secondary objectives of this study are to: determine if an NLRP3 antagonist reduces inflammasome activity in Crohn's disease and ulcerative biopsy samples (comparing Crohn's disease and ulcerative colitis results with control patient results); determine if an NLRP3 antagonist reduced inflammatory cytokine RNA and protein expression in Crohn's disease and ulcerative colitis samples; determine if baseline (no ex vivo treatment) RNA levels of NLRP3, ASC, and IL-1β are greater in biopsy samples from patients with anti-TNFα agent resistance status; and stratify the results according to presence of specific genetic mutations in genes encoding ATG16L1, NLRP3, and CARD8 (e.g., any of the mutations in the ATG16L1 gene, NLRP3 gene, and CARD8 gene described herein).

### Methods

- Evaluation of baseline expression of NLRP3 RNA and quantify inhibition of inflammasome activity by an NLRP3 antagonist in biopsies of disease tissue from patients with Crohn's disease and ulcerative colitis.
- Determine if NLRP3 antagonist treatment reduces the inflammatory response in biopsies of disease from patients with Crohn's disease based on decreased expression of inflammatory gene RNA measured with Nanostring.
- Sequence patient DNA to detect specific genetic mutations in the ATG16L1 gene, NLRP3 gene, and CARD8 gene (e.g., any of the exemplary mutations in these genes described herein) and then stratify the results of functional assays according to the presence of these genetic mutations.

### Experimental Design

- Human subjects and tissue:
   Endoscopic or surgical biopsies from areas of disease in patients with Crohn's disease and ulcerative colitis who are either anti-TNFα treatment naive or resistant to anti-TNFα treatment; additionally biopsies from control patients (surveillance colonoscopy or inflammation-free areas from patients with colorectal cancer) are studied.
- Ex vivo Treatment Model:
   Organ or LPMC culture as determined appropriate
- Endpoints to be measured:
   *Before ex vivo treatment--* NLRP3 RNA level
   *After ex vivo treatment-* inflammasome activity (either processed IL-1β, processed caspase-1, or secreted IL-1β); RNA for inflammatory cytokines (Nanostring); viable T cell number and/or T cell apoptosis.
- Data Analysis Plan:
   ▪ Determine if NLRP3 antagonist treatment decreases processed IL-1β, processed caspase-1 or secreted IL-1β, and inflammatory cytokine RNA levels.
   ▪ Stratify response data according to treatment status at biopsy and the presence of genetic mutations in the NLRP3 gene, CARD8 gene, and ATG16L1 gene (e.g., any of the exemplary genetic mutations of these genes described herein).

### Study Example 2. Treatment of anti-TNFα resistant patients with NLRP3 antagonists

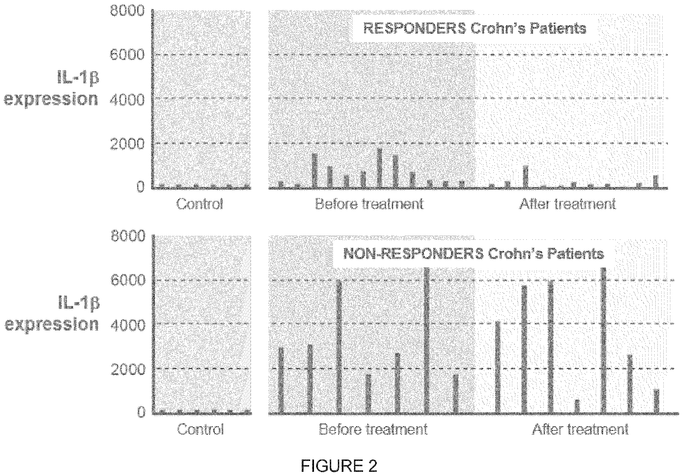

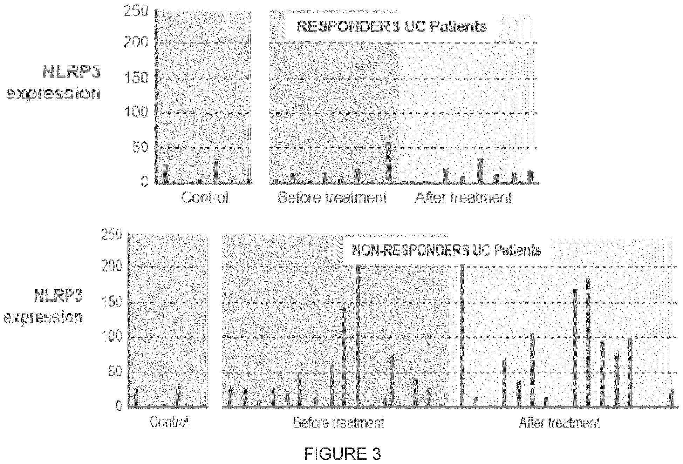

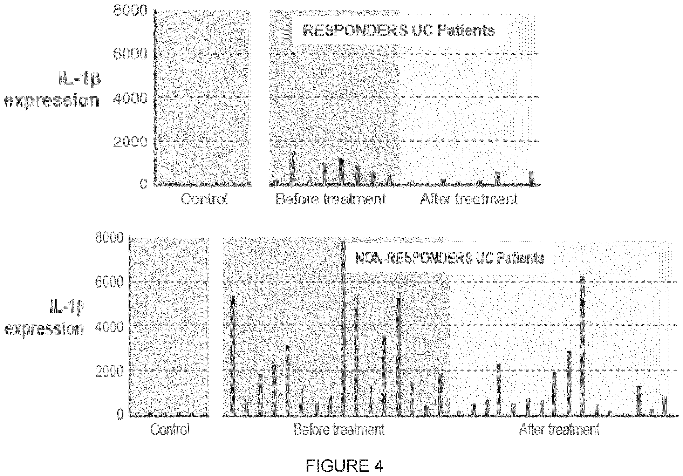

PLoS One 2009 Nov 24;4(11):e7984, describes that mucosal biopsies were obtained at endoscopy in actively inflamed mucosa from patients with Ulcerative Colitis, refractory to corticosteroids and/or immunosuppression, before and 4-6 weeks after their first infliximab (an anti-TNFα agent) infusion and in normal mucosa from control patients. The patients in this study were classified for response to infliximab based on endoscopic and histologic findings at 4-6 weeks after first infliximab treatment as responder or non-responder. Transcriptomic RNA expression levels of these biopsies were accessed by the inventors of the invention disclosed herein from GSE 16879, the publically available Gene Expression Omnibus (https://www.ncbi.nlm.nih.gov/geo/geo2r/?acc=GSE16879). Expression levels of RNA encoding NLRP3 and IL-1β were determined using GEO2R (a tool available on the same website), based on probe sets 207075_at and 205067_at, respectively. It was surprisingly found that in Crohn's disease patients that are non-responsive to the infliximab (an anti-TNFα agent) have higher expression of NLRP3 and IL-1β RNA than responsive patients (figures 1 and 2). Similar surprising results of higher expression of NLRP3 and IL-1β RNA in UC patients that are non-responsive to infliximab (an anti-TNFα agent) compared to infliximab (an anti-TNFα agent) responsive patients (figures 3 and 4) were found.

Said higher levels of NLRP3 and IL-1β RNA expression levels in anti-TNFα agent non-responders, is hypothesised herein to lead to NLRP3 activation which in turns leads ot release of IL-1β that induces IL-23 production, leading to said resistance to anti-TNFα agents. Therefore, treatment of Crohn's and UC anti-TNFα non-responders with an NLRP3 antagonist would prevent this cascade, and thus prevent development of non-responsiveness to anti-TNFα agents. Indeed, resistance to anti-TNFα agents is common in other inflammatory or autoimmune diseases. Therefore, use of an NLRP3 antagonist for the treatment of inflammatory or autoimmune diseases will block the mechanism leading to non-responsiveness to anti-TNFα □ agents. Consequently, use of NLRP3 antagonists will increase the sensitivity of patients with inflammatory or autoimmune diseases to anti-TNFα agents, resulting in a reduced dose of anti-TNFα agents for the treatment of these diseases. Therefore, a combination of an NLRP3 antagonist and an anti-TNFα agent can be used in the treatment of diseases wherein TNFα is overexpressed, such as inflammatory or autoimmune diseases, to avoid such non-responsive development of patients to anti-TNFα agents. Preferably, this combination threatment can be used in the treatment of IBD, for example Crohn's disease and UC.

Further, use of NLRP3 antagonists offers an alternative to anti-TNFα agents for the treatment of diseases wherein TNFα is overexpressed. Therefore, NLRP3 antagonists offers an alternative to anti-TNFα agents inflammatory or autoimmune diseases, such as IBD (e.g. Crohn's disease and UC).

Systemtic anti-TNFα agents are also known to increase the risk of infection. Gut restricted NLRP3 antagonists, however, offers a gut targeted treatment (i.e. non-systemic treatment), preventing such infections. Therefore, treatment of TNFα gut diseases, such as IBD (i.e. Crohn's disease and UC), with gut restricted NLRP3 antagonists has the additional advantage of reducing the risk of infection compared to anti-TNFα agents.

### Proposed Experiment:

Determine the expression of NLRP3 and caspase-1 in LPMCs and epithelial cells in patients with non-active disease, in patients with active disease, in patients with active disease resistant to corticosteroids, patients with active disease resistant to TNF-blocking agents. The expression of NLRP3 and caspase-1 in LPMCs and epithelial cells will be analyzed by RNAScope technology. The expression of active NLRP3 signature genes will be analyzed by Nanostring technology. A pilot analysis to determine feasibility will be performed with 5 samples from control, 5 samples from active CD lesions, and 5 samples from active UC lesions.

### Study Example 3.

It is presented that NLRP3 antagonists reverse resistance to anti-TNF induced T cell depletion/apoptosis in biopsy samples from IBD patients whose disease is clinically considered resistant or unresponsive to anti-TNF therapy.

A study is designed to determine: whether NLRP3 antagonists inhibit inflammasome function and inflammatory activity in cells and biopsy specimens from patients with Crohn's disease or ulcerative colitis; and whether an NLRP3 antagonist will synergize with anti-TNFα therapy in patients with Crohn's disease or ulcerative colitis.

The secondary objectives of this study are to: determine if an NLRP3 antagonist reduces inflammasome activity in Crohn's disease and ulcerative biopsy samples (comparing Crohn's disease and ulcerative colitis results with control patient results); determine if an NLRP3 antagonist reduced inflammatory cytokine RNA and protein expression in Crohn's disease and ulcerative colitis samples; determine if an NLRP3 antagonist in the absence of co-treatment with anti-TNFα antibody induces T cell depletion in Crohn's disease and ulcerative colitis biopsy samples; and determine if baseline (no ex vivo treatment) RNA levels of NLRP3, ASC, and IL-1β are greater in biopsy samples from patients with anti-TNFα agent resistance status.

### Methods

- Evaluation of baseline expression of NLRP3 RNA and quantify inhibition of inflammasome activity by an NLRP3 antagonist in biopsies of disease tissue from patients with Crohn's disease and ulcerative colitis.
- Determine if there is synergy between an NLRP3 antagonist and anti-TNF antibody with respect to effects on T cell depletion/apoptosis in biopsies of disease from patients with Crohn's disease and ulcerative colitis.
- Determine if NLRP3 antagonist treatment reduces the inflammatory response in biopsies of disease from patients with Crohn's disease based on decreased expression of inflammatory gene RNA measured with Nanostring.

### Experimental Design

- Human subjects and tissue:
   Endoscopic or surgical biopsies from areas of disease in patients with Crohn's disease and ulcerative colitis who are either anti-TNFα treatment naive or resistant to anti-TNFα treatment; additionally biopsies from control patients (surveillance colonoscopy or inflammation-free areas from patients with colorectal cancer) are studied.
- Ex vivo Treatment Model:
   Organ or LPMC culture as determined appropriate
- Ex vivo Treatments:
   NLRP3 antagonist (2 concentrations), negative control (vehicle), positive control (caspase-1 inhibitor) each in the presence or absence of anti-TNF antibody at a concentration appropriate to distinguish differences in the T cell apoptotic between biopsies from anti-TNF resistant and anti-TNF-sensitive Crohn's disease patients. Each treatment condition is evaluated in a minimum in duplicate samples.
- Endpoints to be measured:
   *Before ex vivo treatment--* NLRP3 RNA level
   *After ex vivo treatment-* inflammasome activity (either processed IL-1β, processed caspase-1, or secreted IL-1β); RNA for inflammatory cytokines (Nanostring); viable T cell number and/or T cell apoptosis.
- Data Analysis Plan:
   ▪ Determine if NLRP3 antagonist co-treatment increases T cell apoptosis/deletion in response to anti-TNF.
   ▪ Determine if the level of NLRP3 RNA expression is greater in TNF-resistant Crohn's disease and ulcerative colitis samples compared to anti-TNF treatment-naive samples.
   ▪ Determine if NLRP3 antagonist treatment decreases processed IL-1β, processed caspase-1 or secreted IL-1β, and inflammatory cytokine RNA levels.

### Biological Assay - Nigericin-stimulated IL-1β secretion assay in THP-1 cells

Monocytic THP-1 cells (ATCC: TIB-202) were maintained according to providers' instructions in RPMI media (RPMI/Hepes +10% fetal bovine serum + Sodium Pyruvate + 0.05 mM Beta-mercaptoethanol (1000x stock) + Pen-Strep). Cells were differentiated in bulk with 0.5 µM phorbol 12-myristate 13-acetate (PMA; Sigma # P8139) for 3 hours, media was exchanged, and cells were plated at 50,000 cells per well in a 384-well flat-bottom cell culture plates (Greiner, #781986), and allowed to differentiate overnight. Compound in a 1:3.16 serial dilution series in DMSO was added 1:100 to the cells and incubated for 1 hour. The NLRP3 inflammasome was activated with the addition of 15 µM (final concentration) Nigericin (Enzo Life Sciences, #BML-CA421-0005), and cells were incubated for 3 hours. 10 µL supernatant was removed, and IL-1β levels were monitored using an HTRF assay (CisBio, #62IL1PEC) according to manufacturers' instructions. Viability and pyroptosis was monitored with the addition of PrestoBlue cell viability reagent (Life Technologies, #A13261) directly to the cell culture plate.

A number of embodiments of the invention have been described. Nevertheless, it will be understood that various modifications may be made without departing from the spirit and scope of the invention. Accordingly, other embodiments are within the scope of the following claims.

### Numerical embodiments of the invention:

1. A compound of Formula AA 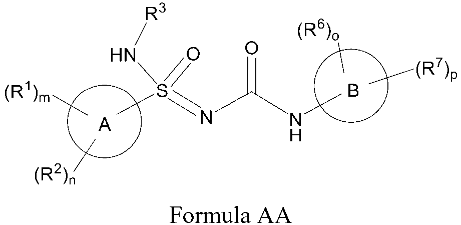 wherein
m = 0, 1, or 2;
n = 0, 1, or 2;
o = 1 or 2;
p = 0, 1, 2, or 3; wherein the sum of o and p is from 1 to 4;
wherein
A is a 5- to 10-membered heteroaryl or a C₆-C₁₀ aryl;
B is a 6-membered heteroaromatic ring containing 1-3 N atoms, or an N-oxide thereof; wherein at least one R⁶ is *ortho* to the bond connecting the B ring to the NHC(O) group of Formula AA;
R¹ and R² are each independently selected from C₁-C₆ alkyl, C₁-C₆ haloalkyl, C₁-C₆ alkoxy, C₁-C₆ haloalkoxy, halo, CN, NO₂, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NR⁸R⁹, C(O)R¹³, CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ alkoxy, C₁-C₆ haloalkyl, C₆-C₁₀ aryl, 5- to 10-membered heteroaryl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, R¹⁵*,* NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), and OCO(3- to 7-membered heterocycloalkyl);
wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, -O(C₀-C₃ alkylene)C₆-C₁₀ aryl, halo, NR⁸R⁹, or oxo;
wherein the 3- to 7-membered heterocycloalkyl, C₆-C₁₀ aryl, and 5- to 10-membered heteroaryl are each optionally substituted with one or more substituents independently selected from halo, C₁-C₆ alkyl, and OC₁-C₆ alkyl;
or one pair of R¹ and R² on adjacent atoms, taken together with the atoms connecting them, independently form at least one monocyclic or bicyclic C₄-C₁₂ carbocyclic ring or at least one monocyclic or bicyclic 5- to-12-membered heterocyclic ring wherein:
   a) when each of the adjacent atoms is a carbon atom, then the heterocyclic ring includes from 1-3 heteroatoms and/or heteroatomic groups independently selected from O, NH, NR¹³, S, S(O), and S(O)₂; and
   b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²), and
wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R⁶ and R⁷ are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, 5- to 10-membered heteroaryl, NHCOC₆-C₁₀ aryl, NHCO(5- to 10-membered heteroaryl) and NHCO(3- to 7-membered heterocycloalkyl) are optionally substituted with one or more substituents independently selected from halo, C₁-C₆ alkyl, and OC₁-C₆ alkyl;
or at least one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms independently selected from O, NH, NR¹³, and S, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R¹⁰ is C₁-C₆ alkyl;
each of R⁸ and R⁹ at each occurrence is independently selected from hydrogen, C₁-C₆ alkyl, C₂-C₆ alkenyl, C₃-C₇ cycloalkyl, C₁-C₆ haloalkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl, 3- to 7-membered heterocycloalkyl, or NR¹¹R¹²;
or R⁸ and R⁹ taken together with the nitrogen they are attached to form a 3- to 10-membered monocyclic or bicyclic ring optionally containing one or more heteroatoms in addition to the nitrogen they are attached to, wherein the ring is optionally substituted with one or more substituents independently selected from halo, C₁-C₆ alkyl, C₁-C₆ haloalkyl, C₁-C₆ alkoxy, oxo, N(C₁-C₆ alkyl)₂, NH₂, NH(C₁-C₆ alkyl), and hydroxy;
R¹³ is C₁-C₆ alkyl, C₁-C₆ haloalkyl, or -(Z¹-Z²)ₐ₁-Z³;
each of R¹¹ and R¹² at each occurrence is independently selected from hydrogen, C₁-C₆ alkyl, and -(Z¹-Z²)ₐ₁-Z³;
a1 is an integer selected from 0-10 (e.g., 0-5);
each Z¹ is independently C₁-C₆ alkylene optionally substituted with one or more substituents independently selected from oxo, halo, and hydroxy;
each Z² is independently a bond, NH, N(C₁-C₆ alkyl), -O-, -S-, or 5-10 membered heteroarylene;
Z³ is independently C₆-C₁₀ aryl, C₂-C₆ alkyenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
R³ is selected from hydrogen, cyano, hydroxy, CO₂C₁-C₆ alkyl, C₁-C₆ alkoxy, C₁-C₆ alkyl, and 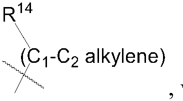 wherein the C₁-C₂ alkylene group is optionally substituted with oxo;
R¹⁴ is hydrogen, C₁-C₆ alkyl, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶;
R¹⁵ is -(Z⁴-Z⁵)ₐ₂-Z⁶;
a2 is an integer selected from 1-10 (e.g., 1-5 (e.g., 2-5));
each Z⁴ is independently selected from -O-, -S-, -NH-, and -N(C₁-C₃ alkyl)-;
provided that the Z⁴ group directly attached to R¹ or R² is -O- or -S-;
each Z⁵ is independently C₁-C₆ alkylene optionally substituted with one or more substituents independently selected from oxo, halo, and hydroxy; and
Z⁶ is OH, OC₁-C₆ alkyl, NH₂, NH(C₁-C₆ alkyl), N(C₁-C₆ alkyl)₂, NHC(O)(C₁-C₆ alkyl), NHC(O)(C₁-C₆ alkoxy), or an optionally substituted group selected from the group consisting of:
   C₆₋C₁₀ aryl, C₂-C₆ alkenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
   with the proviso that the compound of Formula AA is not a compound selected from the group consisting of: 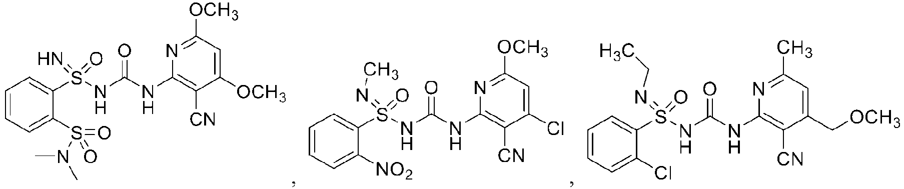 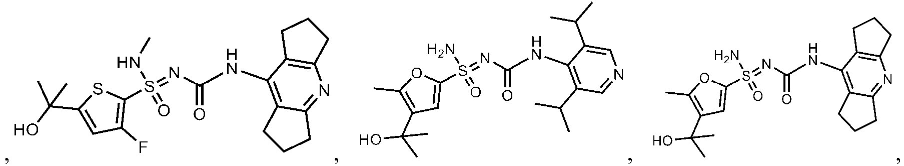 and 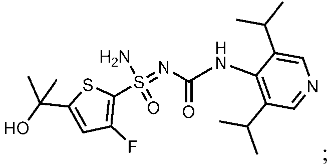
   or a pharmaceutically acceptable salt thereof.
2. The compound of claim 1, wherein A is a 5-membered heteroaryl.
3. The compound of claim 1, wherein A is a 6- to 10-membered heteroaryl.
4. The compound of claim 1, wherein A is a C₆-C₁₀ aryl.
5. The compound of any one of claims 1-2, wherein A is a 5-membered heteroaryl comprising 1 heteroatom or heteroatomic group selected from N, NH, and NR¹.
6. The compound of any one of claims 1-2, wherein A is a 5-membered heteroaryl comprising 1 heteroatom selected from O and S, wherein the heteroatom is not bonded to the position of the heteroaryl that is bonded to the S(O)(NHR³)=N moiety.
7. The compound of any one of claims 1-2, and 6, wherein A is thiophenyl.
8. The compound of any one of claims 1-2, wherein A is thiazolyl.
9. The compound of any one of claims 1-2, wherein A is pyrazolyl.
10. The compound of any one of claims 1 and 4, wherein A is phenyl.
11. The compound of any one of claims **1-10,** wherein m=1 and n=0.
12. The compound of any one of claims 1-2, 8, and **11,** wherein the optionally substituted ring A is 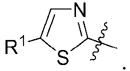 13. The compound of any one of claims 1-2, 8, and 11, wherein the optionally substituted ring A is 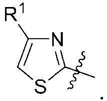 14. The compound of any one of claims 1-2, 8, and 11, wherein the optionally substituted ring A is 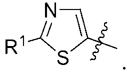 15. The compound of any one of claims 1-2, 8, and 11, wherein the optionally substituted ring A is 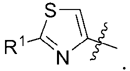 16. The compound of any one of claims 1-2, 9, and 11, wherein the optionally substituted ring A is 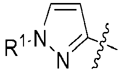 or 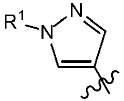 (e.g., 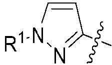 ).
17. The compound of any one of claims 1-2, and 11, wherein the optionally substituted ring A is 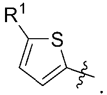 18. The compound of any one of claims 1-2, and 11, wherein the optionally substituted ring A is 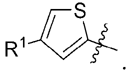 19. The compound of any one of claims 1, 4, and 11, wherein the optionally substituted ring A is 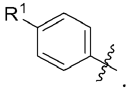 20. The compound of any one of claims 1-10, wherein m=1 and n=1.
21. The compound of any one of claims 1-2, 8, and 20, wherein the optionally substituted ring A is 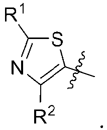 22. The compound of any one of claims 1-2, 9, and 20, wherein the optionally substituted ring A is 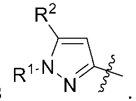 23. The compound of any one of claims 1-2, 9, and 20, wherein the optionally substituted ring A is 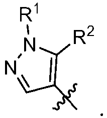 24. The compound of any one of claims 1-2, 9, and 20, wherein the optionally substituted ring A is 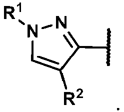 25. The compound of any one of claims 1-2, 8, and 20, wherein the optionally substituted ring A is 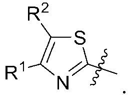 26. The compound of any one of claims 1, 2, 7, and 20, wherein the optionally substituted ring A is 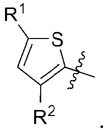 27. The compound of any one of claims 1, 2, 7, and 20, wherein the optionally substituted ring A is 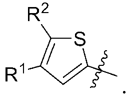 28. The compound of any one of claims 1, 4, 10, and 20, wherein the optionally substituted ring A is 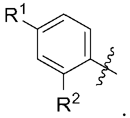 29. The compound of any one of claims 1-28, wherein R¹, when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.
30. The compound of any one of claims 1-29, wherein R¹, when present, is independently selected from the group consisting of C₁-C₆ alkyl optionally substituted with one or more hydroxy, halo, or NR⁸R⁹.
31. The compound of any one of claims 1-29, wherein R¹ is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².
32. The compound of any one of claims 1-28, wherein R¹ is selected from the group consisting of methyl; ethyl; difluoromethyl; 2-hydroxy-2-propyl; 1,2-dihydroxy-2-propyl; hydroxymethyl; (dimethylamino)methyl; (methylamino)methyl; and fluoro.
33. The compound of any one of claims 1-28 and 32, wherein R¹ is 2-hydroxy-2-propyl or 1,2-dihydroxy-2-propyl.
34. The compound of any one of claims 1-28 and 32, wherein R¹ is fluoro.
35. The compound of any one of claims 1-34, wherein R², when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.
36. The compound of any one of claims 1-34, wherein R², when present, is independently selected from the group consisting of C₁-C₆ alkyl optionally substituted with one or more hydroxyl, halo, or NR⁸R⁹.
37. The compound of any one of claims 1-34, wherein R², when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².
38. The compound of any one of claims 1-34, wherein R², when present, is selected from the group consisting of methyl; ethyl; difluoromethyl; 2-hydroxy-2-propyl; hydroxymethyl; 1,2-dihydroxy-2-propyl; (dimethylamino)methyl; (methylamino)methyl; and fluoro.
39. The compound of any one of claims 1-10, 20, 22-25, and 27, wherein R¹ and R² are on adjacent atoms, and taken together with the atoms connecting them, independently form one monocyclic or bicyclic C₄-C₁₂ carbocyclic ring (e.g., C₅ or C₆ carbocyclic ring) or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²), and wherein the carbocyclic ring or heterocyclic ring is optionally independently substituted with one or more substituents each independently selected from hydroxy, halo, oxo, C₁-C₆ alkyl (e.g., methyl), C₂-C₆ alkenyl, C₂-C₆ alkynyl, C₁-C₆ alkoxy (e.g., isopropoxyl), OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3-to 10-membered heterocycloalkyl (e.g., azetidinyl or oxetanyl), and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo (e.g., fluoro), C₁-C₆ alkyl, C₂-C₆ alkenyl, C₂-C₆ alkynyl, C₃-C₁₀ cycloalkyl, C₁-C₆ alkoxy, oxo, NR⁸R⁹ (e.g., amino, methylamino, or dimethylamino), =NR¹⁰, COOC₁-C₆ alkyl, C₆-C₁₀ aryl, and CONR⁸R⁹.
40. The compound of any one of claims 1-10, 20, 22-23, 25, and 27, wherein R¹ and R² are on adjacent atoms, and taken together with the atoms connecting them, independently form one monocyclic or bicyclic C₅-C₆ carbocyclic ring optionally independently substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino; or R¹ and R² are on adjacent atoms, and taken together, independently form: 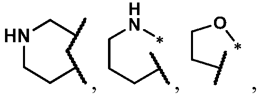 or 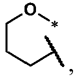 each of which is optionally substituted with one or more substituents independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino; wherein the asterisk represents a point of attachment to a carbon atom; and the 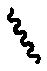 represents a point of attachment to a carbon or a nitrogen atom.
41. The compound of any one of claims 1-10, 20, 22-23, 25, and 27, wherein R¹ and R² are on adjacent atoms, and taken together with the atoms connecting them, independently form at least one bicyclic spirocyclic C₄-C₁₂ carbocyclic ring, wherein the carbocyclic ring is optionally substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino.
42. The compound of any one of claims 1-10, 20, 22-23, 25, and 27, wherein R¹ and R² are on adjacent atoms, and taken together with the atoms connecting them, independently form at least one bicyclic spirocyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²), and wherein the carbocyclic or heterocyclic ring is optionally substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino.
43. The compound of any one of claims 1-2 and 8, wherein A is thiazolyl (e.g., 2-thiazolyl or 5-thiazolyl); m is 1; n is 0 or 1; R¹ is C₁-C₆ alkyl optionally substituted with hydroxy (e.g., 2-hydroxy-2-propyl); and R², when present, is C₁-C₆ alkyl optionally substituted with hydroxy (e.g., methyl, hydroxymethyl, or hydroxyethyl).
44. The compound of any one of claims 1-2 and 9, wherein A is pyrazolyl (e.g., 3-pyrazolyl); m is 1; n is 0; and R¹ is C₁-C₆ alkyl optionally substituted with 1-3 halo (e.g., fluoro).
45. The compound of claim 1, wherein A is phenyl; m is 1; n is 0 or 1; and R¹ is C₁-C₆ alkyl optionally substituted with NR⁸R⁹ (e.g., dimethylamino); and R², when present, is halo (e.g., fluoro).
46. The compound of any one of claims 1-2 and 7, wherein A is thiophenyl (e.g., 2-thiophenyl); m is 1; n is 0; and R¹ is C₁-C₆ alkyl optionally substituted with hydroxyl or oxo (e.g., isopropyl, 2-hydroxy-2-propyl, or 1-propanoyl).
47. The compound of any one of claims 1-2, wherein the optionally substituted ring A is selected from the group consisting of a 5-membered heteroaryl comprising 2 or more heteroatoms, a 5-membered heteroaryl comprising 1 heteroatom or heteroatomic group selected from N, NH, and NR¹, and a 5-membered heteroaryl comprising 1 heteroatom selected from O and S, wherein the heteroatom is not bonded to the position of the heteroaryl that is bonded to the S(O)(NHR³)=N moiety; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²),and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.
48. The compound of any one of claims 1-2, 9, and 47, wherein the optionally substituted ring A is a pyrazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²),and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.
49. The compound of any one of claims 1-2 and 47, wherein the optionally substituted ring A is an imidazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂(in addition to the aforementioned nitrogen atom(s) attached to R¹ and/or R²), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.
50. The compound of any one of claims 1-2 and 7, wherein the optionally substituted ring A is a thiophenyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.
51. The compound of any one of claims 1-2 and 9, wherein the optionally substituted ring A is 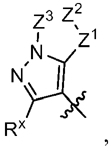 wherein R^{x} is selected from the group consisting of H and C₁-C₆ alkyl (e.g., methyl); Z¹ is selected from the group consisting of O, NH, and -CH₂- optionally substituted with 1-2 R²⁰; Z² is selected from the group consisting of NH and -CH₂-optionally substituted with 1-2 R²⁰; Z³ is selected from the group consisting of -CH₂-optionally substituted with 1-2 R²⁰, -CH₂CH₂- optionally substituted with 1-2 R²⁰, and - CH₂CH₂CH₂- optionally substituted with 1-2 R²⁰; R²⁰ is selected from the group consisting of hydroxy, halo (e.g., fluoro), oxo, C₁-C₆ alkyl (e.g., methyl or ethyl) optionally substituted with one R²¹, C₁-C₆ alkoxy (e.g., methoxy, ethoxy, or isopropoxy) optionally substituted with one R²¹, NR⁸R⁹, 3- to 10-membered heterocycloalkyl (e.g., azetidinyl or pyrrolidinyl) optionally substituted with one R²¹, or one pair of R²⁰ on the same atom, taken together with the atom connecting them, independently forms a monocyclic C₃-C₄ carbocyclic ring or a monocyclic 3- to 4-membered heterocyclic ring containing 1 O atom optionally substituted with OS(O)₂Ph; R²¹ is selected from the group consisting of halo (e.g., fluoro), NR⁸R⁹, C₂-C₆ alkynyl (e.g., ethynyl), and C₁-C₆ alkoxy (e.g., methoxy); R⁸ and R⁹ at each occurrence is independently selected from hydrogen, C₁-C₆ alkyl (e.g., methyl or ethyl), COR¹³, and CO₂R¹³; R¹³ is selected from the group consisting of: C₁-C₆ alkyl (e.g., methyl or t-butyl) and C₁-C₆ haloalkyl (e.g., trifluoromethyl).
52. The compound of any one of claims 1-2 and 9, wherein the optionally substituted ring A is 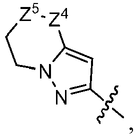 wherein Z⁴ is selected from the group consisting of -CH₂-, -C(O)-, and NH; Z⁵ is selected from the group consisting of O, NH, N-CH₃, and -CH₂-.
53. The compound of any one of claims 1-52, wherein B is pyridyl, or an N-oxide thereof.
54. The compound of any one of claims 1-53, wherein the B is 3-pyridyl.
55. The compound of any one of claims 1-54, wherein o=2 and p=1.
56. The compound of any one of claims 1-55, wherein the substituted ring B is 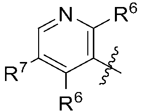 57. The compound of claim 1-56, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
58. The compound of any one of claims 1-57, wherein each R⁶ is independently selected from the group consisting of: C₁-C₆ alkyl, halo, C₃-C₇ cycloalkyl, and C₆-C₁₀ aryl.
59. The compound of any one of claims 1-58, wherein each R⁶ is independently selected from the group consisting of: methyl, isopropyl, cyclopropyl, fluoro, and phenyl.
60. The compound of any one of claims 1-59, wherein each R⁷ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
61. The compound of any one of claims 1-60, wherein each R⁷ is independently selected from the group consisting of: C₁-C₆ alkyl, C₃-C₇ cycloalkyl, halo, and C₆-C₁₀ aryl.
62. The compound of any one of claims 1-61, wherein each R⁷ is independently selected from the group consisting of: methyl, isopropyl, cyclopropyl, fluoro, and phenyl.
63. The compound of any one of claims 1-53, wherein the substituted ring B is 4-pyridyl.
64. The compound of any one of claims 1-53 and 63, wherein o=2 and p=0.
65. The compound of any one of claims 1-53 and 63-64, wherein the substituted ring B is 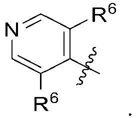 66. The compound of any one of claims 1-53 and 63-65, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
67. The compound of any one of claims 1-53 and 63-66, wherein each R⁶ is independently selected from the group consisting of: C₁-C₆ alkyl and C₃-C₇ cycloalkyl.
68. The compound of any one of claims 1-53 and 63-67, wherein each R⁶ is isopropyl.
69. The compound of any one of claims 1-53 and 63, wherein o=2 and p=2.
70. The compound of any one of claims 1-53, 63, and 69, wherein the substituted ring B is 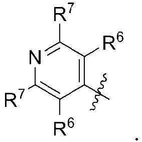 71. The compound of any one of claims 1-53, 63, and 69-70, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
72. The compound of any one of claims 1-53, 63, and 69-71, wherein each R⁶ is independently selected from the group consisting of: C₁-C₆ alkyl, C₃-C₇ cycloalkyl, halo, and C₆-C₁₀ aryl.
73. The compound of any one of claims 1-53, 63, and 69-72, wherein each R⁶ is independently selected from the group consisting of: methyl, isopropyl, cyclopropyl, fluoro, and phenyl.
74. The compound of any one of claims 1-53, 63, and 69-73, wherein each R⁷ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
75. The compound of any one of claims 1-53, 63, and 69-74, wherein each R⁷ is independently selected from the group consisting of: C₁-C₆ alkyl, C₃-C₇ cycloalkyl, halo, and C₆-C₁₀ aryl.
76. The compound of any one of claim 1-53, 63, and 69-75, wherein each R⁷ is independently selected from the group consisting of: methyl, isopropyl, cyclopropyl, fluoro, and phenyl.
77. The compound of any one of claims 1-53, 63, and 69-76, wherein one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, and S, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
78. The compound of any one of claims 1-53, 63, and 69-76, wherein one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
79. The compound of any one of claims 77-78, wherein the C₄-C₈ carbocyclic ring is a C₅ carbocyclic ring optionally substituted with one or more oxo, CH₃, or hydroxy.
80. The compound of claim 79, wherein the C₅ carbocyclic ring is substituted with one CH₃.
81. The compound of claim 79, wherein the C₅ carbocyclic ring is geminally substituted with two CH₃.
82. The compound of any one of claims 77-78, wherein the C₄-C₈ carbocyclic ring is a C₇ carbocyclic ring, wherein the C₇ carbocyclic ring is a bicyclic spirocycle, wherein the bicyclic spirocycle comprises a 5-membered ring and a 3-membered ring.
83. The compound of any one of claims 1 and 43-52, wherein the substituted ring B is 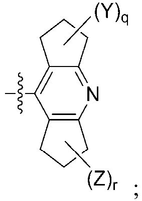 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to form a cyclopropyl ring.
84. The compound of any one of claims 1 and 43-52, wherein the substituted ring B is 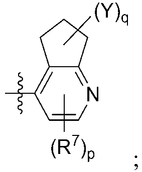 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.
85. The compound of any one of claims 1 and 43-52, wherein the substituted ring B is 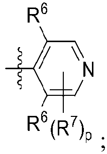 each R⁶ is independently selected from C₁-C₆ alkyl (e.g., isopropyl); each R⁷ is independently selected from halo (e.g., fluoro); p is 0 or 1.
86. The compound of any one of claims 1 and 43-52, wherein the substituted ring B is 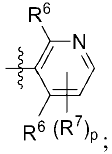 each R⁶ is independently selected from C₁-C₆ alkyl (e.g., isopropyl); each R⁷ is independently selected from halo (e.g., fluoro); p is 0 or 1.
87. The compound of any one of claims 1 and 43-52, wherein the substituted ring B is 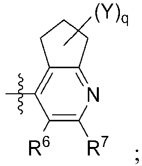 R⁶ is selected from C₁-C₆ alkyl (e.g., methyl, ethyl, or isopropyl) and C₃-C₁₀ cycloalkyl (e.g., cyclopropyl); R⁷ is selected from C₁-C₆ alkyl (e.g., methyl, ethyl, or isopropyl), C₁-C₆ haloalkyl (e.g., trifluoromethyl) and C₃-C₁₀ cycloalkyl (e.g., cyclopropyl or cyclobutyl); or R⁶ and R⁷, taken together with the atoms connecting them, independently form a C₅ carbocyclic ring optionally substituted with one or more C₁-C₆ alkyl (e.g., methyl); q is 0, 1, or 2; each Y is independently selected from C₁-C₆ alkyl (e.g., methyl); or when two Y are attached to the same carbon, the two Y are taken together with the carbon they are attached to to form a cyclopropyl ring.
88. A compound of Formula AA 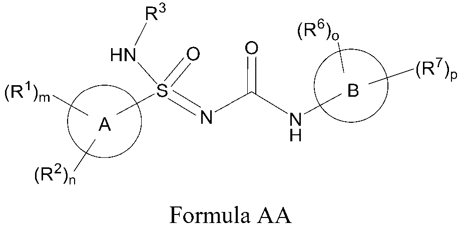 wherein the compound of Formula AA is selected from 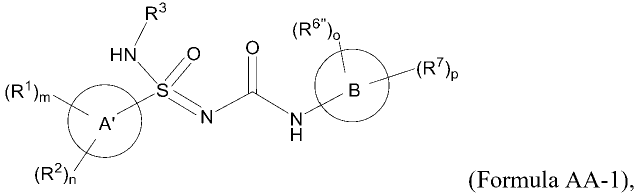 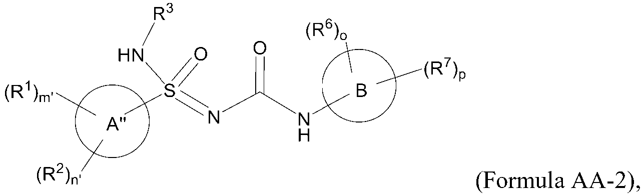 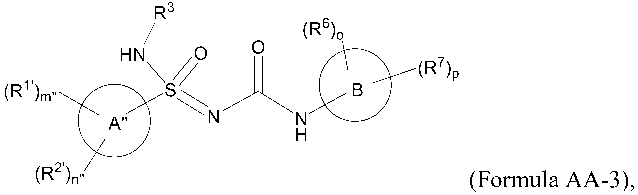 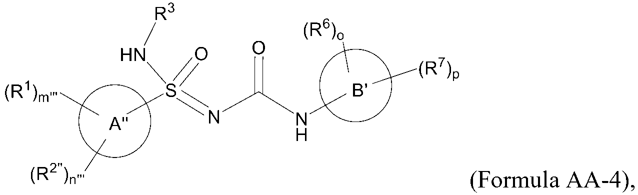 and 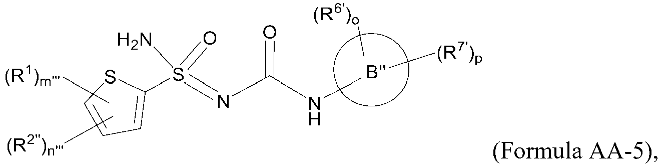 wherein
m = 0, 1, or 2;
n = 0, 1, or 2;
m' = 0, 1, or 2;
n' = 0, 1, or 2; wherein the sum of m' and n' is 0, 1, or 3;
m" = 0, 1, or 2;
n" = 0, 1, or 2; wherein the sum of m" and n" is 2;
m‴ = 1;
n‴ = 1;
o = 1 or 2;
p = 0, 1, 2, or 3; wherein the sum of o and p is from 1 to 4;
wherein
A' is selected from:
a 6- to 10-membered heteroaryl,
a C₆-C₁₀ aryl,
a 5-membered heteroaryl comprising 2 or more heteroatoms,
a 5-membered heteroaryl comprising 1 heteroatom or heteroatomic group selected from N, NH, and NR¹, and
a 5-membered heteroaryl comprising 1 heteroatom selected from O and S, wherein the heteroatom is not bonded to the position of the heteroaryl that is bonded to the S(O)(NHR³)=N moiety;
A" is a 5-membered heteroaryl comprising 1 heteroatom selected from O and S, wherein the heteroatom is bonded to the position of the heteroaryl that is bonded to the S(O)(NHR³)=N moiety;
B is a 6-membered heteroaromatic ring containing 1-3 N atoms, or an N-oxide thereof;
B' is 2-pyridyl, 3-pyridyl, or an N-oxide thereof;
B" is 4-pyridyl or an N-oxide thereof;
wherein
at least one R⁶ is *ortho* to the bond connecting the B ring to the NHC(O) group of Formula AA-2, Formula AA-3, and Formula AA-4;
at least one R^{6'} is *ortho* to the bond connecting the B ring to the NHC(O) group of Formula AA-5;
at least one R^{6"} is *ortho* to the bond connecting the B ring to the NHC(O) group of Formula AA-1;
R¹ and R² are each independently selected from C₁-C₆ alkyl, C₁-C₆ haloalkyl, C₁-C₆ alkoxy, C₁-C₆ haloalkoxy, halo, CN, NO₂, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NR⁸R⁹, C(O)R¹³, CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₁-C₆ alkyl, C₁-C₆ alkoxy, C₁-C₆ haloalkyl, C₆-C₁₀ aryl, 5- to 10-membered heteroaryl, C₃-C₇ cycloalkyl, and 3- to 7-membered heterocycloalkyl are optionally substituted with one or more substituents each independently selected from hydroxy, halo, CN, oxo, C₁-C₆ alkyl, C₁-C₆ alkoxy, R¹⁵, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), and OCO(3- to 7-membered heterocycloalkyl);
wherein each C₁-C₆ alkyl substituent and each C₁-C₆ alkoxy substituent of the R¹ or R² C₃-C₇ cycloalkyl or of the R¹ or R² 3- to 7-membered heterocycloalkyl is further optionally independently substituted with one to three hydroxy, -O(C₀-C₃ alkylene)C₆-C₁₀ aryl, halo, NR⁸R⁹, or oxo;
wherein the 3- to 7-membered heterocycloalkyl, C₆-C₁₀ aryl, and 5- to 10-membered heteroaryl, are each optionally substituted with one or more substituents independently selected from halo, C₁-C₆ alkyl, and OC₁-C₆ alkyl;
or one pair of R¹ and R² on adjacent atoms, taken together with the atoms connecting them, independently form one monocyclic or bicyclic C₄-C₁₂ carbocyclic ring or one monocyclic or bicyclic 5- to-12-membered heterocyclic ring wherein:
   a) when each of the adjacent atoms is a carbon atom, then the heterocyclic ring includes from 1-3 heteroatoms and/or heteroatomic groups independently selected from O, NH, NR¹³, S, S(O), and S(O)₂; and
   b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂(in addition to the aforementioned nitrogen atom(s) attached to R¹ and/or R²), and
wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R^{1'} and R^{2'} are each independently selected from C₂-C₆ alkyl, C₁-C₆ haloalkyl, C₁-C₆ alkoxy, C₁-C₆ haloalkoxy, Cl, Br, I, CN, NO₂, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NR⁸R⁹, C(O)R¹³, CONR⁸R⁹, SF₅, SC₁-C₆ alkyl, S(O₂)C₁-C₆ alkyl, S(O₂)NR¹¹R¹², S(O)C₁-C₆ alkyl, C₃-C₇ cycloalkyl and 3- to 7-membered heterocycloalkyl,
wherein the C₂-C₆ alkyl, C₁-C₆ alkoxy, C₁-C₆ haloalkyl, C₆-C₁₀ aryl, 5- to 10-membered heteroaryl, C₃-C₇ cycloalkyl, and 3- to 7-membered heterocycloalkyl is optionally substituted with one or more substituents each independently selected from hydroxy, halo, CN, oxo, C₁-C₆ alkyl, C₁-C₆ alkoxy, R¹⁵, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), and OCO(3- to 7-membered heterocycloalkyl);
wherein each C₁-C₆ alkyl substituent and each C₁-C₆ alkoxy substituent of the R^{1'} or R^{2'} C₃-C₇ cycloalkyl or of the R^{1'} or R^{2'} 3- to 7-membered heterocycloalkyl is further optionally independently substituted with one to three hydroxy, -O(C₀-C₃ alkylene)C₆-C₁₀ aryl, halo, NR⁸R⁹, or oxo;
wherein the 3- to 7-membered heterocycloalkyl, C₆-C₁₀ aryl, and 5- to 10-membered heteroaryl are optionally substituted with one or more substituents independently selected from halo, C₁-C₆ alkyl, and OC₁-C₆ alkyl;
or one pair of R^{1'} and R^{2'} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and
wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R^{2"} is F or CH₃; or
when the compound of Formula AA is a compound of Formula AA-4, one pair of R¹ and R^{2"} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and
wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹,
wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹;
R⁶ and R⁷ are each independently selected from C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R⁶ and R⁷ are each optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkyl or C₁-C₆ alkoxy that R⁶ or R⁷ is substituted with is optionally substituted with one or more hydroxyl, C₆-C₁₀ aryl or NR⁸R⁹, or wherein R⁶ or R⁷ is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R^{6'} and R^{7'} are each independently selected from unbranched C₁-C₆ alkyl, C₁-C₆ haloalkyl, C₁-C₆ alkoxy, C₁-C₆ haloalkoxy, halo, CN, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5- to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl and 3- to 10-membered heterocycloalkyl, and C₂-C₆ alkenyl,
wherein R^{6'} and R^{7'} are each optionally substituted with one or more substituents independently selected from hydroxy, halo, CN, oxo, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and wherein the C₁-C₆ alkoxy that R^{6'} or R^{7'} is substituted with is optionally substituted with one or more hydroxyl, C₆-C₁₀ aryl or NR⁸R⁹, or wherein R^{6'} or R^{7'} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R^{6"} is selected from C₁-C₆ alkyl, C₁-C₆ haloalkyl, C₁-C₆ alkoxy, C₁-C₆ haloalkoxy, halo, NO₂, COC₁-C₆ alkyl, CO₂C₁-C₆ alkyl, CO₂C₃-C₈ cycloalkyl, OCOC₁-C₆ alkyl, OCOC₆-C₁₀ aryl, OCO(5- to 10-membered heteroaryl), OCO(3- to 7-membered heterocycloalkyl), C₆-C₁₀ aryl, 5-to 10-membered heteroaryl, NH₂, NHC₁-C₆ alkyl, N(C₁-C₆ alkyl)₂, CONR⁸R⁹, SF₅, SC₁-C₆ alkyl, S(O₂)C₁-C₆ alkyl, C₃-C₁₀ cycloalkyl, 3- to 10-membered heterocycloalkyl, and C₂-C₆ alkenyl,
wherein R^{6"} is optionally substituted with one or more substituents independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 3- to 7-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(3- to 7-membered heterocycloalkyl), C₆-C₁₀ aryloxy, and S(O₂)C₁-C₆ alkyl; and
wherein the C₁-C₆ alkyl or C₁-C₆ alkoxy that R^{6"} is substituted with is optionally substituted with one or more hydroxyl, C₆-C₁₀ aryl or NR⁸R⁹, or wherein R^{6"} is optionally fused to a five- to -seven-membered carbocyclic ring or heterocyclic ring containing one or two heteroatoms independently selected from oxygen, sulfur and nitrogen;
wherein the 3- to 7-membered heterocycloalkyl, C₆-C₁₀ aryl, and 5- to 10-membered heteroaryl, are optionally substituted with one or more substituents independently selected from halo, C₁-C₆ alkyl, and OC₁-C₆ alkyl;
or one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ haloalkyl, C₁-C₆ alkoxy, NR⁸R⁹, CH₂NR⁸R⁹, =NR¹⁰, COOH, COOC₁-C₆ alkyl, C₆-C₁₀ aryl, and CONR⁸R⁹;
R¹⁰ is C₁-C₆ alkyl;
each of R⁸ and R⁹ at each occurrence is independently selected from hydrogen, C₁-C₆ alkyl, C₂-C₆ alkenyl, C₃-C₇ cycloalkyl, C₁-C₆ haloalkyl, (C=NR¹³)NR¹¹R¹², S(O₂)C₁-C₆ alkyl, S(O₂)NR¹¹R¹², COR¹³, CO₂R¹³ and CONR¹¹R¹²; wherein the C₁-C₆ alkyl is optionally substituted with one or more hydroxy, halo, C₁-C₆ alkoxy, C₆-C₁₀ aryl, 5- to 10-membered heteroaryl, C₃-C₇ cycloalkyl, 3- to 7-membered heterocycloalkyl, or NR¹¹R¹²;
or R⁸ and R⁹ taken together with the nitrogen they are attached to form a 3- to 10-membered monocyclic or bicyclic ring optionally containing one or more heteroatoms in addition to the nitrogen they are attached to, wherein the ring is optionally substituted with one or more substituents independently selected from halo, C₁₋C₆ alkyl, C₁-C₆ haloalkyl, C₁₋C₆ alkoxy, oxo, N(C₁-C₆ alkyl)₂, NH₂, NH(C₁-C₆ alkyl), and hydroxy;
R¹³ is C₁-C₆ alkyl, C₁-C₆ haloalkyl, or -(Z¹-Z²)ₐ₁-Z³;
each of R¹¹ and R¹² at each occurrence is independently selected from hydrogen, C₁-C₆ alkyl, and₋Z¹-Z²)ₐ₁-Z³;
a1 is an integer selected from 0-10 (e.g., 0-5);
each Z¹ is independently C₁-C₆ alkylene optionally substituted with one or more substituents independently selected from oxo, halo, and hydroxy;
each Z² is independently a bond, NH, N(C₁-C₆ alkyl), -O-, -S-, or 5-10 membered heteroarylene;
Z³ is independently C₆-C₁₀ aryl, C₂-C₆ alkyenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
R³ is selected from hydrogen, cyano, hydroxy, CO₂C₁-C₆ alkyl, C₁-C₆ alkoxy, C₁-C₆ alkyl, and 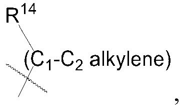 wherein the C₁-C₂ alkylene group is optionally substituted with oxo;
R¹⁴ is hydrogen, C₁-C₆ alkyl, 5- to 10-membered monocyclic or bicyclic heteroaryl or C₆-C₁₀ monocyclic or bicyclic aryl, wherein each C₁-C₆ alkyl, aryl or heteroaryl is optionally independently substituted with 1 or 2 R⁶;
R¹⁵ is -(Z⁴-Z⁵)ₐ₂-Z⁶;
a2 is an integer selected from 1-10 (e.g., 1-5 (e.g., 2-5));
each Z⁴ is independently selected from -O-, -S-, -NH-, and -N(C₁-C₃ alkyl)-;
provided that the Z⁴ group directly attached to R¹ or R² is -O- or -S-;
each Z⁵ is independently C₁-C₆ alkylene optionally substituted with one or more substituents independently selected from oxo, halo, and hydroxy; and
Z⁶ is OH, OC₁-C₆ alkyl, NH₂, NH(C₁-C₆ alkyl), N(C₁-C₆ alkyl)₂, NHC(O)(C₁-C₆ alkyl), NHC(O)(C₁-C₆ alkoxy), or an optionally substituted group selected from the group consisting of:
   C₆₋C₁₀ aryl, C₂-C₆ alkenyl, C₂-C₆ alkynyl, C₃-C₁₀ cycloalkyl, 5- to 10-membered heteroaryl, or 3- to 10-membered heterocycloalkyl, each of which is optionally substituted with one or more substituents independently selected from halo, C₁-C₆ alkyl, C₁₋₆ haloalkyl, C₁-C₆ alkoxy, oxo, N(C₁-C₆ alkyl)₂, NH₂, NH(C₁-C₆ alkyl), and hydroxy;
   or a pharmaceutically acceptable salt thereof.
89. The compound of claim 88, wherein the compound of Formula AA is a compound of 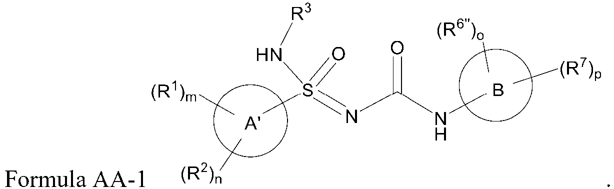 90. The compound of any one of claims 88-89, wherein A' is a 6- to 10-membered heteroaryl.
91. The compound of any one of claims 88-89, wherein A' is a C₆-C₁₀ aryl.
92. The compound of any one of claims 88-89, wherein A' is a 5-membered heteroaryl comprising 2 or more heteroatoms and/or heteroatomic groups.
93. The compound of any one of claims 88-89, wherein A' is a 5-membered heteroaryl comprising 1 heteroatom or heteroatomic group selected from N, NH, and NR¹.
94. The compound of any one of claims 88-89, wherein A' is a 5-membered heteroaryl comprising 1 heteroatom selected from O and S, wherein the heteroatom is not bonded to the position of the heteroaryl that is bonded to the S(O)(NHR³)=N moiety.
95. The compound of any one of claims 88-89 and 94, wherein A' is thiophenyl.
96. The compound of any one of claims 88-89 and 92, wherein A' is thiazolyl.
97. The compound of any one of claims 88-89 and 92, wherein A' is pyrazolyl.
98. The compound of any one of claims 88-89 and 91, wherein A' is phenyl.
99. The compound of any one of claims 88-98, wherein m=1 and n=0.
100. The compound of any one of claims 88-89, 92, 96, and 99, wherein the optionally substituted ring A' is 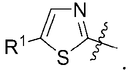 101. The compound of any one of claims 88-89, 92, 96, and 99, wherein the optionally substituted ring A' is 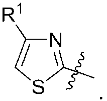 102. The compound of any one of claims 88-89, 92, 96, and 99, wherein the optionally substituted ring A' is 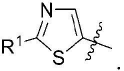 103. The compound of any one of claims 88-89, 92, 96, and 99, wherein the optionally substituted ring A' is 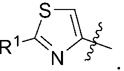 104. The compound of any one of claims 88-89, 92, 97, and 99, wherein the optionally substituted ring A' is 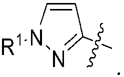 105. The compound of any one of claims 88-89, 91, 98, and 99, wherein the optionally substituted ring A' is 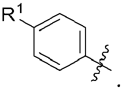 106. The compound of any one of claims 88-98, wherein m=1 and n=1.
107. The compound of any one of claims 88-89, 92, 96, and 106, wherein the optionally substituted ring A' is 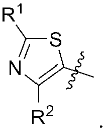 108. The compound of any one of claims 88-89, 92, 97, and 106, wherein the optionally substituted ring A is 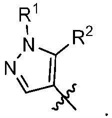 109. The compound of any one of claims 88-89, 92, 97, and 106, wherein the optionally substituted ring A is 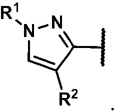 110. The compound of any one of claims 88-89, 92, 96, and 106, wherein the optionally substituted ring A' is 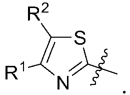 111. The compound of any one of claims 88-89, 91, 98, and 106, wherein the optionally substituted ring A' is 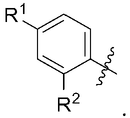 112. The compound of any one of claims 88-111, wherein R¹, when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁₋C₆ alkoxy, C₁₋C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O²)NR¹¹R¹²; S(O)C₁-C₆ alkyl; and S(O₂)C₁-C₆ alkyl.
113. The compound of any one of claims 88-112, wherein R¹, when present, is independently selected from the group consisting of C₁-C₆ alkyl optionally substituted with one or more hydroxyl, halo, or NR⁸R⁹.
114. The compound of any one of claims 88-112, wherein R¹, when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².
115. The compound of any one of claims 88-114, wherein R¹, when present, is selected from the group consisting of methyl; ethyl; difluoromethyl; 2-hydroxy-2-propyl; 1,2-dihydroxy-2-propyl; hydroxymethyl; (dimethylamino)methyl; (methylamino)methyl; and fluoro.
116. The compound of any one of claims 88-115, wherein R¹ is 2-hydroxy-2-propyl or 1,2-dihydroxy-2-propyl.
117. The compound of any one of claims 88-115, wherein R¹ is fluoro.
118. The compound of any one of claims 88-98 and 106-117, wherein R², when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.
119. The compound of any one of claims 88-98 and 106-118, wherein R², when present, is independently selected from the group consisting of C₁-C₆ alkyl optionally substituted with one or more hydroxyl, halo, or NR⁸R⁹.
120. The compound of any one of claims 88-98 and 106-118, wherein R², when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².
121. The compound of any one of claims 88-98 and 106-120, wherein R², when present, is selected from the group consisting of methyl; ethyl; difluoromethyl; 2-hydroxy-2-propyl; hydroxymethyl; 1,2-dihydroxy-2-propyl; (dimethylamino)methyl; (methylamino)methyl; and fluoro.
122. The compound of any one of claims 88-98, 106, 108, and 110, wherein R¹ and R² are on adjacent atoms, and taken together with the atoms connecting them, independently form one monocyclic or bicyclic C₄-C₁₂ carbocyclic ring (e.g., C₅ or C₆ carbocyclic ring) or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²), and wherein the carbocyclic ring or heterocyclic ring is optionally independently substituted with one or more substituents each independently selected from hydroxy, halo, oxo, C₁-C₆ alkyl (e.g., methyl), C₂-C₆ alkenyl, C₂-C₆ alkynyl, C₁-C₆ alkoxy (e.g., isopropoxyl), OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl (e.g., azetidinyl or oxetanyl), and CONR⁸R⁹, wherein the C₁₋C₆ alkyl, C₁₋C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo (e.g., fluoro), C₁-C₆ alkyl, C₂-C₆ alkenyl, C₂-C₆ alkynyl, C₃-C₁₀ cycloalkyl, C₁₋C₆ alkoxy, oxo, NR⁸R⁹ (e.g., amino, methylamino, or dimethylamino), =NR¹⁰, COOC₁-C₆ alkyl, C₆-C₁₀ aryl, and CONR⁸R⁹.
123. The compound of any one of claims 88-98, 106, 108, and 110, wherein R¹ and R² are on adjacent atoms, and taken together with the atoms connecting them, independently form one monocyclic or bicyclic C₅-C₆ carbocyclic ring optionally independently substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino; or R¹ and R² are on adjacent atoms, and taken together, independently form: 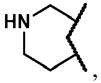 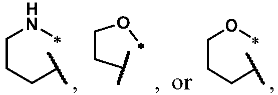 each of which is optionally substituted with one or more substituents independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino; wherein the asterisk represents a point of attachment to a carbon atom; and the 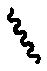 represents a point of attachment to a carbon or a nitrogen atom.
124. The compound of any one of claims 88-98, 106, 108, and 110, wherein R¹ and R² are on adjacent atoms, and taken together with the atoms connecting them, independently form at least one bicyclic spirocyclic C₄-C₁₂ carbocyclic ring, wherein the carbocyclic ring is optionally substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino.
125. The compound of any one of claims 88-98, 106, 108, and 110, wherein R¹ and R² are on adjacent atoms, and taken together with the atoms connecting them, independently form at least one bicyclic spirocyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²), and wherein the carbocyclic or heterocyclic ring is optionally substituted with one or more substituents each independently selected from hydroxy, halo, oxo, methyl, isopropoxyl, azetidinyl, oxetanyl, wherein the methyl, isopropoxyl, azetidinyl, and oxetanyl are optionally substituted with one or more substituents each independently selected from hydroxy, fluoro, amino, methylamino, and dimethylamino.
126. The compound of any one of claims 88-89, 92, and 96, wherein A' is thiazolyl (e.g., 2-thiazolyl or 5-thiazolyl); m is 1; n is 0 or 1; R¹ is C₁₋C₆ alkyl optionally substituted with hydroxy (e.g., 2-hydroxy-2-propyl); and R², when present, is C₁₋C₆ alkyl optionally substituted with hydroxy (e.g., methyl, hydroxymethyl, or hydroxyethyl).
127. The compound of any one of claims 88-89, 92, and 97, wherein A' is pyrazolyl (e.g., 3-pyrazolyl); m is 1; n is 0; and R¹ is C₁-C₆ alkyl optionally substituted with 1-3 halo (e.g., fluoro).
128. The compound of any one of claims 88-89, 91, and 98, wherein A' is phenyl; m is 1; n is 0 or 1; and R¹ is C₁-C₆ alkyl optionally substituted with NR⁸R⁹ (e.g., dimethylamino); and R², when present, is halo (e.g., fluoro).
129. The compound of any one of claims 88-89 and 94-95, wherein A' is thiophenyl; m is 1; n is 0; and R¹ is C₁₋C₆ alkyl optionally substituted with hydroxyl or oxo (e.g., isopropyl, 2-hydroxy-2-propyl, or 1-propanoyl).
130. The compound of any one of claims 88-89, wherein the optionally substituted ring A' is selected from the group consisting of a 5-membered heteroaryl comprising 2 or more heteroatoms, a 5-membered heteroaryl comprising 1 heteroatom or heteroatomic group selected from N, NH, and NR¹, and a 5-membered heteroaryl comprising 1 heteroatom selected from O and S, wherein the heteroatom is not bonded to the position of the heteroaryl that is bonded to the S(O)(NHR³)=N moiety; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂ (in addition to the aforementioned nitrogen atom(s) attached to R¹ and/or R²),and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁₋C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁₋C₆ alkyl, C₂-C₆ alkenyl, C₂-C₆ alkynyl, C₃-C₁₀ cycloalkyl, C₁₋C₆ alkoxy, oxo, NR⁸R⁹, =NR¹⁰, COOC₁-C₆ alkyl, C₆-C₁₀ aryl, and CONR⁸R⁹.
131. The compound of any one of claims 88-89, 92, 97, and 130, wherein the optionally substituted ring A' is a pyrazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂(in addition to the aforementioned nitrogen atom(s) attached to R¹ and/or R²), and wherein the carbocyclic ring or heterocyclic ring is optionally independently substituted with one or more substituents each independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁₋C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁₋C₆ alkoxy, oxo, NR⁸R⁹, =NR¹⁰, COOC₁-C₆ alkyl, C₆-C₁₀ aryl, and CONR⁸R⁹.
132. The compound of any one of claims 88-89, 92, and 130, wherein the optionally substituted ring A' is an imidazolyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring wherein a) when each of the adjacent atoms is a carbon atom, then the heterocyclic ring includes from 1-3 heteroatoms and/or heteroatomic groups independently selected from O, NH, NR¹³, S, S(O), and S(O)₂; and b) when one or both of the adjacent atoms is/are a nitrogen atom(s), then the heterocyclic ring includes from 0-2 heteroatoms and/heteroatomic groups independently selected from O, NH, NR¹³, S, S(O), and S(O)₂(in addition to the aforementioned nitrogen atom(s) attached to R¹ and/or R²), and wherein the carbocyclic ring or heterocyclic ring is optionally independently substituted with one or more substituents each independently selected from hydroxy, halo, oxo, C₁₋C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁₋C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁₋C₆ alkoxy, oxo, NR⁸R⁹, =NR¹⁰, COOC₁-C₆ alkyl, C₆-C₁₀ aryl, and CONR⁸R⁹.
133. The compound of any one of claims 88-89 and 94-95, wherein the optionally substituted ring A' is a thiophenyl; m is 1; n is 1; R¹ and R² are on adjacent atoms, and taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂, and wherein the carbocyclic ring or heterocyclic ring is optionally independently substituted with one or more substituents each independently selected from hydroxy, halo, oxo, C₁₋C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, S(O₂)C₆-C₁₀ aryl, C₆-C₁₀ aryl, 5- to 10-membered heteroaryl, C₃-C₁₀ cycloalkyl, 3-to 10-membered heterocycloalkyl, and CONR⁸R⁹, wherein the C₁-C₆ alkyl, C₁₋C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.
134. The compound of any one of claims 88-89, 92, and 97, wherein the optionally substituted ring A is 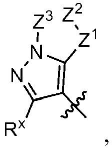 wherein R^{x} is selected from the group consisting of H and C₁-C₆ alkyl (e.g., methyl); Z¹ is selected from the group consisting of O, NH, and -CH₂-optionally substituted with 1-2 R²⁰; Z² is selected from the group consisting of NH and - CH₂- optionally substituted with 1-2 R²⁰; Z³ is selected from the group consisting of -CH₂-optionally substituted with 1-2 R²⁰, -CH₂CH₂- optionally substituted with 1-2 R²⁰, and - CH₂CH₂CH₂- optionally substituted with 1-2 R²⁰; R²⁰ is selected from the group consisting of hydroxy, halo (e.g., fluoro), oxo, C₁-C₆ alkyl (e.g., methyl or ethyl) optionally substituted with one R²¹, C₁₋C₆ alkoxy (e.g., methoxy, ethoxy, or isopropoxy) optionally substituted with one R²¹, NR⁸R⁹, 3- to 10-membered heterocycloalkyl (e.g., azetidinyl or pyrrolidinyl) optionally substituted with one R²¹, or one pair of R²⁰ on the same atom, taken together with the atom connecting them, independently forms a monocyclic C₃-C₄ carbocyclic ring or a monocyclic 3- to 4-membered heterocyclic ring containing 1 O atom optionally substituted with OS(O)₂Ph; R²¹ is selected from the group consisting of halo (e.g., fluoro), NR⁸R⁹, C₂-C₆ alkynyl (e.g., ethynyl), and C₁-C₆ alkoxy (e.g., methoxy); R⁸ and R⁹ at each occurrence is independently selected from hydrogen, C₁-C₆ alkyl (e.g., methyl or ethyl), COR¹³, and CO₂R¹³; R¹³ is selected from the group consisting of: C₁-C₆ alkyl (e.g., methyl or t-butyl) and C₁-C₆ haloalkyl (e.g., trifluoromethyl).
135. The compound of any one of claims 88-89, 92, and 97, wherein the optionally substituted ring A is 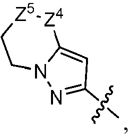 wherein Z⁴ is selected from the group consisting of - CH₂-, -C(O)-, and NH; Z⁵ is selected from the group consisting of O, NH, N-CH₃, and - CH₂-.
136. The compound of any one of claims 88-135, wherein B is pyridyl, or an N-oxide thereof.
137. The compound of any one of claims 88-136, wherein B is 3-pyridyl, or an N-oxide thereof.
138. The compound of any one of claims 88-137, wherein o=2 and p=1.
139. The compound of any one of claims 88-138, wherein the substituted ring B is 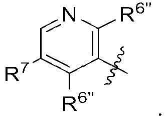 140. The compound of any one of claims 88-139, wherein each R^{6"} is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
141. The compound of any one of claims 88-140, wherein each R^{6"} is independently selected from the group consisting of: C₁-C₆ alkyl, halo, C₃-C₇ cycloalkyl, and C₆-C₁₀ aryl.
142. The compound of any one of claims 88-141, wherein each R^{6"} is independently selected from the group consisting of: methyl, isopropyl, cyclopropyl, fluoro, and phenyl.
143. The compound of any one of claims 88-142, wherein each R⁷ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
144. The compound of any one of claims 88-143, wherein each R⁷ is independently selected from the group consisting of: C₁-C₆ alkyl, C₃-C₇ cycloalkyl, halo, and C₆-C₁₀ aryl.
145. The compound of any one of claims 88-144, wherein each R⁷ is independently selected from the group consisting of: methyl, isopropyl, cyclopropyl, fluoro, and phenyl.
146. The compound of any one of claims 88-136, wherein B is 4-pyridyl, or an N-oxide thereof.
147. The compound of any one of claims 88-136 and 146, wherein o=2 and p=0.
148. The compound of any one of claims 88-136 and 146-147, wherein the substituted ring B is 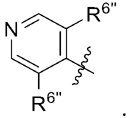 149. The compound of any one of claims 88-136 and 146-148, wherein each R^{6"} is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
150. The compound of any one of claims 88-136 and 146-149, wherein each R^{6"} is independently selected from the group consisting of: C₁-C₆ alkyl and C₃-C₇ cycloalkyl.
151. The compound of any one of claims 88-136 and 146-150, wherein each R^{6"} is isopropyl.
152. The compound of any one of claims 88-136 and 146, wherein o=2 and p=2.
153. The compound of any one of claims 88-136, 146, and 152, wherein the substituted ring B is 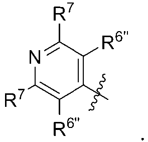 154. The compound of any one of claim 88-136, 146, and 152-153, wherein each R^{6"} is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
155. The compound of any one of claims 88-136, 146, and 152-154, wherein each R^{6"} is independently selected from the group consisting of: C₁-C₆ alkyl, C₃-C₇ cycloalkyl, halo, and C₆-C₁₀ aryl.
156. The compound of any one of claims 88-136, 146, and 152-155, wherein each R^{6"} is independently selected from the group consisting of: methyl, isopropyl, cyclopropyl, fluoro, and phenyl.
157. The compound of any one of claims 88-136, 146, and 152-156, wherein each R⁷ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
158. The compound of any one of claims 88-136, 146, and 152-157, wherein each R⁷ is independently selected from the group consisting of: C₁-C₆ alkyl, C₃-C₇ cycloalkyl, halo, and C₆-C₁₀ aryl.
159. The compound of any one of claims 88-136, 146, and 152-158, wherein each R⁷ is independently selected from the group consisting of: methyl, isopropyl, cyclopropyl, fluoro, and phenyl.
160. The compound of any one of claims 88-89, 139-142, and 153-159, wherein one pair of R^{6"} and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
161. The compound of any one of claims 88-89, 139-142, and 153-159, wherein one pair of R^{6"} and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
162. The compound of any one of claims 160-161, wherein the C₄-C₈ carbocyclic ring is a C₅ carbocyclic ring optionally substituted with one or more oxo, CH₃, or hydroxy.
163. The compound of claim 162, wherein the C₅ carbocyclic ring is substituted with one CH₃.
164. The compound of claim 162, wherein the C₅ carbocyclic ring is geminally substituted with two CH₃.
165. The compound of any one of claims 160-161, wherein the C₄-C₈ carbocyclic ring is a C₇ carbocyclic ring, wherein the C₇ carbocyclic ring is a bicyclic spirocycle, wherein the bicyclic spirocycle comprises a 5-membered ring and a 3-membered ring.
166. The compound of any one of claims 88-89 and 96, wherein the optionally substituted ring A' is thiazolyl (e.g., 2-thiazolyl or 5-thiazolyl); m is 1; n is 0 or 1 (e.g., n is 1); R¹ is C₁-C₆ alkyl optionally substituted with hydroxy (e.g., 2-hydroxy-2-propyl); and R², when present, is C₁-C₆ alkyl optionally substituted with hydroxyl (e.g., methyl, hydroxymethyl, or hydroxyethyl).
167. The compound of any one of claims 88-89 and 97, wherein the optionally substituted ring A' is pyrazolyl (e.g., 3-pyrazolyl); m is 1; n is 0; and R¹ is C₁-C₆ alkyl optionally substituted with 1-3 halo (e.g., fluoro (e.g., R¹ is C₁-C₆ alkyl substituted with 2-3 fluoro)).
168. The compound of any one of claims 88-89 and 98, wherein the optionally substituted ring A' is phenyl; m is 1; n is 0 or 1; and R¹ is C₁-C₆ alkyl optionally substituted with NR⁸R⁹ (e.g., dimethylamino); and R², when present, is halo (e.g., fluoro (e.g., R¹ is C₁-C₆ alkyl substituted with 2-3 fluoro)).
169. The compound of any one of claims 88-89, 126-135 and, 166-168, wherein the substituted ring B is 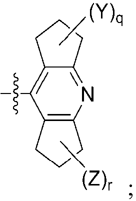 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.
170. The compound of any one of claims 88-89, 126-135 and, 166-168, wherein the substituted ring B is 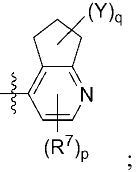 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.
171. The compound of any one of claims 88-89, 126-135 and, 166-168, wherein the substituted ring B is 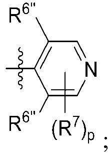 each R^{6"} is independently selected from C₁-C₆ alkyl (e.g., isopropyl); each R⁷ is independently selected from halo (e.g., fluoro); p is 0 or 1.
172. The compound of any one of claims 88-89, 126-135 and, 166-168, wherein the substituted ring B is 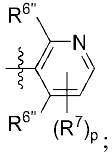 each R^{6"} is independently selected from C₁-C₆ alkyl (e.g., isopropyl); each R⁷ is independently selected from halo (e.g., fluoro); p is 0 or 1.
173. The compound of claim 88, wherein the compound of Formula AA is a compound of 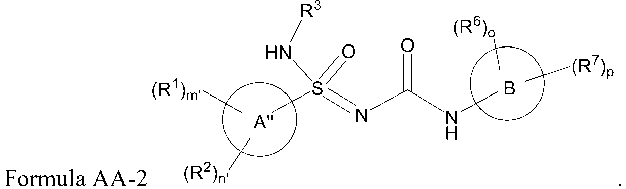 174. The compound of any one of claims 88 and 173 wherein A" is thiophenyl.
175. The compound of any one of claims 88 and 173-174, wherein m'=1 and n'=0.
176. The compound of any one of claims 88 and 173-175, wherein the optionally substituted ring A" is 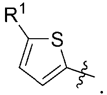 177. The compound of any one of claims 88 and 173-175, wherein the optionally substituted ring A" is 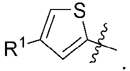 178. The compound of any one of claims 88 and 173-177, wherein R¹, when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.
179. The compound of any one of claims 88 and 173-178, wherein R¹, when present, is independently selected from the group consisting of C₁-C₆ alkyl optionally substituted with one or more hydroxyl, halo, or NR⁸R⁹.
180. The compound of any one of claims 88 and 173-178, wherein R¹, when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².
181. The compound of any one of claims 88 and 173-180, wherein R¹, when present, is selected from the group consisting of methyl; ethyl; difluoromethyl; 2-hydroxy-2-propyl; 1,2-dihydroxy-2-propyl; hydroxymethyl; (dimethylamino)methyl; (methylamino)methyl; and fluoro.
182. The compound of any one of claims 88 and 173-181, wherein R¹, when present, is 2-hydroxy-2-propyl or 1,2-dihydroxy-2-propyl.
183. The compound of any one of claims 88 and 173-181, wherein R¹ is fluoro.
184. The compound of any one of claims 88, 173, 174, and 178-183, wherein R², when present, is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.
185. The compound of any one of claims 88, 173, 174, and 178-184, wherein R², when present, is independently selected from the group consisting of C₁-C₆ alkyl optionally substituted with one or more hydroxyl, halo, or NR⁸R⁹.
186. The compound of any one of claims 88, 173, 174, and 178-184, wherein R², when present, is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².
187. The compound of any one of claims 88, 173, 174, and 178-186, wherein R², when present, is selected from the group consisting of methyl; ethyl; difluoromethyl; 2-hydroxy-2-propyl; 1,2-dihydroxy-2-propyl; hydroxymethyl; (dimethylamino)methyl; (methylamino)methyl; and fluoro.
188. The compound of any one of claims 88 and 173-187, wherein B is pyridyl, or an N-oxide thereof.
189. The compound of any one of claims 88 and 173-188, wherein B is 3-pyridyl, or an N-oxide thereof.
190. The compound of any one of claims 88 and 173-189, wherein o=2 and p=1.
191. The compound of any one of claims 88 and 173-190, wherein the substituted ring B is 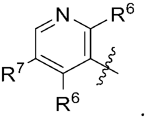 192. The compound of any one of claims 88 and 173-191, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
193. The compound of any one of claims 88 and 173-192, wherein each R⁶ is independently selected from the group consisting of: C₁-C₆ alkyl, halo, C₃-C₇ cycloalkyl, and C₆-C₁₀ aryl.
194. The compound of any one of claims 88 and 173-193, wherein each R⁶ is independently selected from the group consisting of: methyl, isopropyl, cyclopropyl, fluoro, and phenyl.
195. The compound of any one of claims 88, 173-189, and 192-194, wherein each R⁷, when present, is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
196. The compound of any one of claims 88, 173-189, and 192-195, wherein each R⁷, when present, is independently selected from the group consisting of: C₁-C₆ alkyl, C₃-C₇ cycloalkyl, halo, and C₆-C₁₀ aryl.
197. The compound of any one of claims 88, 173-189, and 192-196, wherein each R⁷, when present, is independently selected from the group consisting of: methyl, isopropyl, cyclopropyl, fluoro, and phenyl.
198. The compound of any one of claims 88 and 173-188, wherein B is 4-pyridyl, or an N-oxide thereof.
199. The compound of claim 88, 173-188, and 198, wherein o=2 and p=0.
200. The compound of any one of claims 88, 173-188, and 198-199, wherein the substituted ring B is 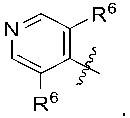 201. The compound of any one of claims 88, 173-188, and 198-200, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
202. The compound of any one of claims 88, 173-188, and 198-201, wherein each R⁶ is independently selected from the group consisting of: C₁-C₆ alkyl and C₃-C₇ cycloalkyl.
203. The compound of any one of claims 88, 173-188, and 198-202, wherein each R⁶ is isopropyl.
204. The compound of claim 88, 173-188, and 198, wherein o=2 and p=2.
205. The compound of claims 88, 173-188, 198, and 204, wherein the substituted ring B is 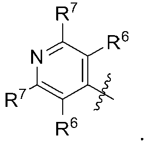 206. The compound of claim 88, 173-188, 198, and 204-205, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
207. The compound of claim 88, 173-188, 198, and 204-206, wherein each R⁶ is independently selected from the group consisting of: C₁-C₆ alkyl, C₃-C₇ cycloalkyl, halo, and C₆-C₁₀ aryl.
208. The compound of any one of claims 88, 173-188, 198, and 204-207, wherein each R⁶ is independently selected from the group consisting of: methyl, isopropyl, cyclopropyl, fluoro, and phenyl.
209. The compound of any one of claims 88, 173-188, 198, and 204-208, wherein each R⁷, when present, is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5-to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
210. The compound of any one of claims 88, 173-188, 198, and 204-209, wherein each R⁷, when present, is independently selected from the group consisting of: C₁-C₆ alkyl, C₃-C₇ cycloalkyl, halo, and C₆-C₁₀ aryl.
211. The compound of claim 88, 173-188, 198, and 204-210 wherein each R⁷, when present, is independently selected from the group consisting of: methyl, isopropyl, cyclopropyl, fluoro, and phenyl.
212. The compound of any one of claims 88, 173-188, 198, and 204-210, wherein one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
213. The compound of any one of claims 88, 173-188, 198, and 204-210, wherein one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
214. The compound of any one of claims 212-213, wherein the C₄-C₈ carbocyclic ring is a C₅ carbocyclic ring optionally substituted with one or more oxo, CH₃, or hydroxy.
215. The compound of claim 214, wherein the C₅ carbocyclic ring is substituted with one CH₃.
216. The compound of claim 214, wherein the C₅ carbocyclic ring is geminally substituted with two CH₃.
217. The compound of any one of claims 212-213, wherein the C₄-C₈ carbocyclic ring is a C₇ carbocyclic ring, wherein the C₇ carbocyclic ring is a bicyclic spirocycle, wherein the bicyclic spirocycle comprises a 5-membered ring and a 3-membered ring.
218. The compound of claim 173, wherein A" is thiophenyl (e.g., 2-thiophenyl); m' is 1; n' is 0; and R¹ is C₁-C₆ alkyl optionally substituted with hydroxyl or oxo (e.g., isopropyl, 2-hydroxy-2-propyl, or 1-propanoyl).
219. The compound of any one of claims 173 and 218, wherein A" is 2-thiophenyl.
220. The compound of any one of claims 173 and 218, wherein the substituted ring B is 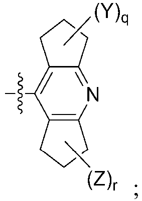 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.
221. The compound of any one of claims 173 and 218, wherein the substituted ring B is 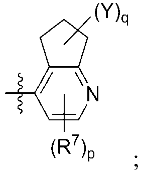 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.
222. The compound of any one of claims 173 and 218, wherein the substituted ring B is 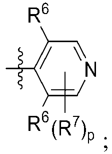 each R⁶ is independently selected from C₁-C₆ alkyl (e.g., isopropyl); each R⁷ is independently selected from halo (e.g., fluoro); p is 0 or 1.
223. The compound of any one of claims 173 and 218, wherein the substituted ring B is 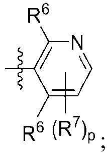 each R⁶ is independently selected from C₁-C₆ alkyl (e.g., isopropyl); each R⁷ is independently selected from halo (e.g., fluoro); p is 0 or 1.
224. The compound of claim 88, wherein the compound of Formula AA is a compound of 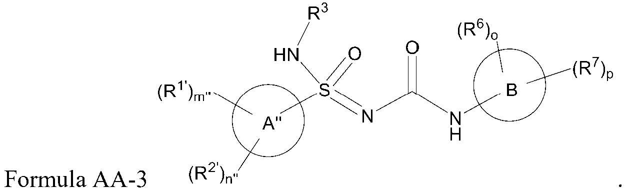 225. The compound of any one of claims 88 and 224, wherein A" is thiophenyl.
226. The compound of any one of claims 88 and 224-225, wherein m"=1 and n"=1.
227. The compound of any one of claims 88 and 224-226, wherein the optionally substituted ring A" is 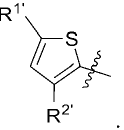 228. The compound of any one of claims 88 and 224-226, wherein the optionally substituted ring A" is 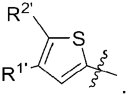 229. The compound of any one of claims 88 and 224-226, the optionally substituted ring A" is 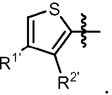 230. The compound of any one of claims 88 and 224-229, wherein R^{1'}, when present, is independently selected from the group consisting of C₂-C₆ alkyl optionally substituted with one or more hydroxy, C₂-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; Cl; Br; I; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.
231. The compound of any one of claims 88 and 224-230, wherein R^{1'}, when present, is independently selected from the group consisting of C₂-C₆ alkyl optionally substituted with one or more hydroxyl, or NR⁸R⁹.
232. The compound of any one of claims 88 and 224-230, wherein R^{1'}, when present, is selected from the group consisting of 1-hydroxy-2-methylpropan-2-yl; 1,2-dihydroxy-2-propyl; isopropyl; ethyl; 2-hydroxy-2-propyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; 1-(dimethylamino)ethyl; chloro; phenyl; pyridyl; pyrazolyl; S(O₂)CH₃; and S(O₂)NR¹¹R¹².
233. The compound of any one of claims 88 and 224-232, wherein R^{1'} is 2-hydroxy-2-propyl or 1,2-dihydroxy-2-propyl.
234. The compound of any one of claims 88 and 224-233, wherein R^{2'}, when present, is independently selected from the group consisting of C₂-C₆ alkyl optionally substituted with one or more hydroxy, C₂-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; Cl; Br; I; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.
235. The compound of any one of claims 88 and 224-234, wherein R^{2'}, when present, is independently selected from the group consisting of C₂-C₆ alkyl optionally substituted with one or more hydroxyl, halo, or NR⁸R⁹.
236. The compound of any one of claims 88 and 224-234, wherein R^{2'}, when present, is selected from the group consisting of 1-hydroxy-2-methylpropan-2-yl; 1,2-dihydroxy-2-propyl; isopropyl; ethyl; 2-hydroxy-2-propyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; 1-(dimethylamino)ethyl; chloro; phenyl; pyridyl; pyrazolyl; S(O₂)CH₃; and S(O₂)NR¹¹R¹².
237. The compound of any one of claims 88 and 224-236, wherein R^{2'} is 2-hydroxy-2-propyl or 1,2-dihydroxy-2-propyl.
238. The compound of any one of claims 88, 224-226, and 228-229, wherein one pair of R^{1'} and R^{2'} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂,wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.
239. The compound of any one of claims 88 and 224-238, wherein B is pyridyl, or an N-oxide thereof.
240. The compound of any one of claims 88 and 224-239, wherein B is 3-pyridyl, or an N-oxide thereof.
241. The compound of any one of claims 88 and 224-240, wherein o=2 and p=1.
242. The compound of any one of claims 88 and 224-241, wherein the substituted ring B is 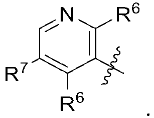 243. The compound of claims 88 and 224-242, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
244. The compound of any one of claims 88 and 224-243, wherein each R⁶ is independently selected from the group consisting of: C₁-C₆ alkyl, halo, C₃-C₇ cycloalkyl, and C₆-C₁₀ aryl.
245. The compound of any one of claims 88 and 224-244, wherein each R⁶ is independently selected from the group consisting of: methyl, isopropyl, cyclopropyl, fluoro, and phenyl.
246. The compound of any one of claims 88 and 224-245, wherein each R⁷ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
247. The compound of any one of claims 88 and 224-246, wherein each R⁷, when present, is independently selected from the group consisting of: C₁-C₆ alkyl, C₃-C₇ cycloalkyl, halo, and C₆-C₁₀ aryl.
248. The compound of any one of claims 88 and 224-247, wherein each R⁷, when present, is independently selected from the group consisting of: methyl, isopropyl, cyclopropyl, fluoro, and phenyl.
249. The compound of any one of claims 88 and 224-239 , wherein B is 4-pyridyl, or an N-oxide thereof.
250. The compound of any one of claims 88, 224-239, and 249, wherein o=2 and p=0.
251. The compound of any one of claims 88, 224-239, and 249-250, wherein the substituted ring B is 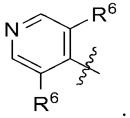 252. The compound of any one of claims 88, 224-239, and 249-251, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
253. The compound of any one of claims 88, 224-239, and 249-252, wherein each R⁶ is independently selected from the group consisting of: C₁-C₆ alkyl and C₃-C₇ cycloalkyl.
254. The compound of any one of claims 88, 224-239, and 249-253, wherein each R⁶ is isopropyl.
255. The compound of any one of claims 88, 224-239, and 249-254, wherein o=2 and p=2.
256. The compound of any one of claims 88, 224-239, and 249-255, wherein the substituted ring B is 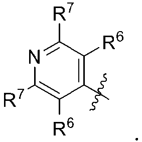 257. The compound of any one of claims 88, 224-239, and 249-256, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
258. The compound of any one of claims 88, 224-239, and 249-257, wherein each R⁶ is independently selected from the group consisting of: C₁-C₆ alkyl, C₃-C₇ cycloalkyl, halo, and C₆-C₁₀ aryl.
259. The compound of any one of claims 88, 224-239, and 249-258, wherein each R⁶ is independently selected from the group consisting of: methyl, isopropyl, cyclopropyl, fluoro, and phenyl.
260. The compound of any one of claims 88, 224-239, and 249-259, wherein each R⁷, when present, is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
261. The compound of any one of claims 88, 224-239, and 249-260, wherein each R⁷, when present, is independently selected from the group consisting of: C₁-C₆ alkyl, C₃-C₇ cycloalkyl, halo, and C₆-C₁₀ aryl.
262. The compound of any one of claims 88, 224-239, and 249-261, wherein each R⁷, when present, is independently selected from the group consisting of: methyl, isopropyl, cyclopropyl, fluoro, and phenyl.
263. The compound of any one of claims 88, 224-239, and 249-262, wherein one pair of R⁶ and R⁷ on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
264. The compound of any one of claims 88, 224-239, and 249-262, wherein one pair of R⁶ and R⁷ on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
265. The compound of any one of claims 263-264, wherein the C₄-C₈ carbocyclic ring is a C₅ carbocyclic ring optionally substituted with one or more oxo, CH₃, or hydroxy.
266. The compound of claim 265, wherein the C₅ carbocyclic ring is substituted with one CH₃.
267. The compound of claim 265, wherein the C₅ carbocyclic ring is geminally substituted with two CH₃.
268. The compound of any one of claims 263-264, wherein the C₄-C₈ carbocyclic ring is a C₇ carbocyclic ring, wherein the C₇ carbocyclic ring is a bicyclic spirocycle, wherein the bicyclic spirocycle comprises a 5-membered ring and a 3-membered ring.
269. The compound of any one of claims 88 and 224, wherein A" is thiophenyl (e.g., 2-thiophenyl); m" is 1; n" is 1; R^{1'} is C₂-C₆ alkyl optionally substituted with hydroxyl or oxo (e.g., 2-hydroxy-2-propyl); and R^{2'} is C₂-C₆ alkyl optionally substituted with hydroxyl or oxo (e.g., 2-hydroxy-2-propyl).
270. The compound of any one of claims 88, 224, and 269, wherein the substituted ring B is 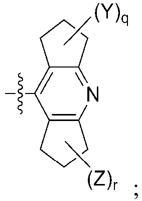 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.
271. The compound of any one of claims 88, 224, and 269, wherein the substituted ring B is 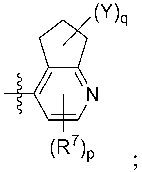 R⁷ is selected from C₁-C₆ alkyl (e.g., methyl, ethyl, or isopropyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.
272. The compound of any one of claims 88, 224, and 269, wherein the substituted ring B is 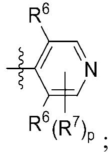 each R⁶ is independently selected from C₁-C₆ alkyl (e.g., isopropyl); each R⁷ is independently selected from halo (e.g., fluoro); p is 0 or 1.
273. The compound of any one of claims 88, 224, and 269, wherein the substituted ring B is 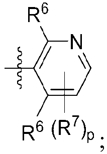 each R⁶ is independently selected from C₁-C₆ alkyl (e.g., isopropyl); each R⁷ is independently selected from halo (e.g., fluoro); and p is 0 or 1.
274. The compound of claim 88, wherein the compound of Formula AA is a compound of 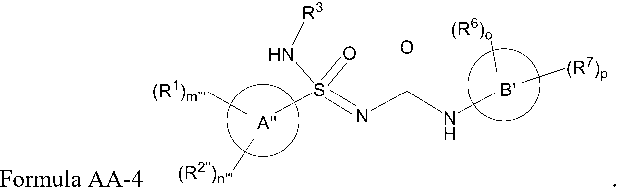 275. The compound of any one of claims 88 and 274, wherein A" is thiophenyl.
276. The compound of any one of claims 88 and 274-275, wherein the optionally substituted ring A" is 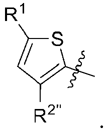 277. The compound of any one of claims 88 and 274-275, wherein the optionally substituted ring A" is 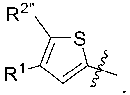 278. The compound of any one of claims 88 and 274-275, the optionally substituted ring A" is 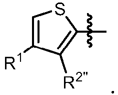 279. The compound of any one of claims 88 and 274-278, wherein R¹ is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.
280. The compound of any one of claims 88 and 274-279, wherein R¹ is independently selected from the group consisting of C₁-C₆ alkyl optionally substituted with one or more hydroxyl, halo, or NR⁸R⁹.
281. The compound of any one of claims 88 and 274-279, wherein R¹ is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².
282. The compound of any one of claims 88 and 274-281, wherein R¹ is selected from the group consisting of methyl; ethyl; difluoromethyl; 2-hydroxy-2-propyl; 1,2-dihydroxy-2-propyl; hydroxymethyl; (dimethylamino)methyl; (methylamino)methyl; and fluoro.
283. The compound of any one of claims 88 and 274-282, wherein R¹ is 2-hydroxy-2-propyl or 1,2-dihydroxy-2-propyl.
284. The compound of any one of claims 88 and 274-282, wherein R¹ is fluoro.
285. The compound of any one of claims 88 and 274-284, wherein R^{2"} is fluoro.
286. The compound of any one of claims 88 and 274-284, wherein R^{2"} is methyl.
287. The compound of any one of claims 88, 274-275, and 277-278, wherein one pair of R¹ and R^{2"} on adjacent atoms, taken together with the atoms connecting them, independently form one monocyclic or bicyclic C₄-C₁₂ carbocyclic ring or one monocyclic or bicyclic 5- to-12-membered heterocyclic ring that includes from 1-3 heteroatoms and/or heteroatomic groups independently selected from O, NH, NR¹³, S, S(O), and S(O)₂ and wherein the carbocyclic ring or heterocyclic ring is optionally independently substituted with one or more substituents independently selected from hydroxy, halo, oxo, C₁-C₆ alkyl, C₂-C₆ alkenyl, C₂-C₆ alkynyl, C₁-C₆ alkoxy, OC₃-C₁₀ cycloalkyl, NR⁸R⁹, =NR¹⁰, CN, COOC₁-C₆ alkyl, OS(O₂)C₆-C₁₀ aryl, S(O₂)C₆-C₁₀ aryl, C₆-C₁₀ aryl, 5- to 10-membered heteroaryl, C₃-C₁₀ cycloalkyl, 3- to 10-membered heterocycloalkyl, and CONR⁸R⁹, wherein the C₁-C₆ alkyl, C₁-C₆ alkoxy, S(O₂)C₆-C₁₀ aryl, C₆-C₁₀ aryl, 5- to 10-membered heteroaryl, C₃-C₁₀ cycloalkyl, and 3- to 10-membered heterocycloalkyl are optionally substituted with one or more substituents each independently selected from hydroxy, halo, C₁-C₆ alkyl, C₂-C₆ alkenyl, C₂-C₆ alkynyl, C₃-C₁₀ cycloalkyl, C₁-C₆ alkoxy, oxo, NR⁸R⁹, =NR¹⁰, COOC₁-C₆ alkyl, C₆-C₁₀ aryl, and CONR⁸R⁹.
288. The compound of any one of claims 88 and 274-287, wherein o=2 and p=1.
289. The compound of any one of claims 88 and 274-288, wherein the substituted ring B is 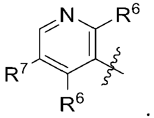 290. The compound of any one of claims 88 and 274-289, wherein each R⁶ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
291. The compound of any one of claims 88 and 274-290, wherein each R⁶ is independently selected from the group consisting of: C₁-C₆ alkyl, halo, C₃-C₇ cycloalkyl, and C₆-C₁₀ aryl.
292. The compound of any one of claims 88 and 274-291, wherein each R⁶ is independently selected from the group consisting of: methyl, isopropyl, cyclopropyl, fluoro, and phenyl.
293. The compound of any one of claims 88 and 274-292, wherein each R⁷ is independently selected from the group consisting of: C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkyl, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
294. The compound of any one of claims 88 and 274-293, wherein each R⁷ is independently selected from the group consisting of: C₁-C₆ alkyl, C₃-C₇ cycloalkyl, halo, and C₆-C₁₀ aryl.
295. The compound of any one of claims 88 and 274-294, wherein each R⁷ is independently selected from the group consisting of: methyl, isopropyl, cyclopropyl, fluoro, and phenyl.
296. The compound of any one of claims88 and 274-278, wherein the optionally substituted ring A" is thiophenyl (e.g., 2-thiophenyl); R¹ is C₁-C₆ alkyl optionally substituted with hydroxyl or oxo (e.g., methyl or 2-hydroxy-2-propyl); and R²" is methyl.
297. The compound of any one of claims 88, 274-278, and 296, wherein the substituted ring B' is 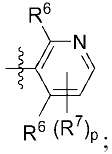 each R⁶ is independently selected from C₁-C₆ alkyl (e.g., isopropyl); each R⁷ is independently selected from halo (e.g., fluoro); p is 0 or 1.
298. The compound of any one of claims 1-297, wherein R³ is hydrogen.
299. The compound of any one of claims 1-297, wherein R³ is CHO.
300. The compound of claim 88, wherein the compound of Formula AA is a compound of 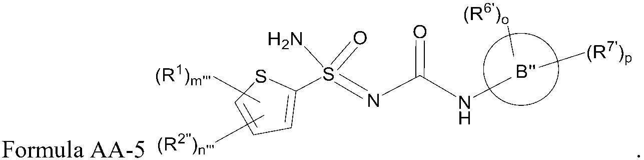 301. The compound of any one of claims 88 and 300, wherein 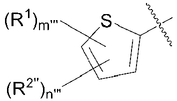 is 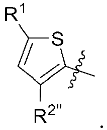 302. The compound of any one of claims 88 and 300, wherein 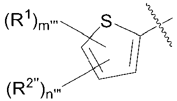 is 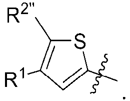 303. The compound of any one of claims 88 and 300-302, wherein R¹ is independently selected from the group consisting of C₁-C₆ alkyl optionally substituted with one or more hydroxy, C₁-C₆ alkyl optionally substituted with one or more halo, oxo, C₁-C₆ alkoxy, or NR⁸R⁹; C₃-C₇ cycloalkyl optionally substituted with one or more hydroxy, halo, oxo, C₁-C₆ alkoxy, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; 3- to 7-membered heterocycloalkyl optionally substituted with one or more hydroxy, halo, oxo, C₁-C₆ alkyl, or NR⁸R⁹ wherein the C₁-C₆ alkoxy or C₁-C₆ alkyl is further optionally substituted with one to three hydroxy, halo, NR⁸R⁹, or oxo; C₁-C₆ haloalkyl; C₁-C₆ alkoxy; C₁-C₆ haloalkoxy; halo; CN; CO-C₁-C₆ alkyl; CO-C₆-C₁₀ aryl; CO(5- to 10-membered heteroaryl); CO₂C₁-C₆ alkyl; CO₂C₃-C₈ cycloalkyl; OCOC₁-C₆ alkyl; OCOC₆-C₁₀ aryl; OCO(5- to 10-membered heteroaryl); OCO(3- to 7-membered heterocycloalkyl); C₆-C₁₀ aryl; 5- to 10-membered heteroaryl; NH₂; NHC₁-C₆ alkyl; N(C₁-C₆ alkyl)₂; CONR⁸R⁹; SF₅; S(O₂)NR¹¹R¹²; S(O)C₁-C₆ alkyl; and S(O₂)C₁-C₆ alkyl.
304. The compound of any one of claims 88 and 300-302, wherein R¹ is independently selected from the group consisting of C₁-C₆ alkyl optionally substituted with one or more hydroxyl, halo, or NR⁸R⁹.
305. The compound of any one of claims 88 and 300-302, wherein R¹ is selected from the group consisting of 1-hydroxy-2-methylpropan-2-yl; 1,2-dihydroxy-2-propyl; methyl; ethyl; difluoromethyl; isopropyl; 2-hydroxy-2-propyl; hydroxymethyl; 1-hydroxyethyl; 2-hydroxyethyl; 1-hydroxy-2-propyl; 1-hydroxy-1-cyclopropyl; 1-hydroxy-1-cyclobutyl; 1-hydroxy-1-cyclopentyl; 1-hydroxy-1-cyclohexyl; morpholinyl; 1,3-dioxolan-2-yl; COCH₃; COCH₂CH₃; 2-methoxy-2-propyl; (dimethylamino)methyl; (methylamino)methyl; 1-(dimethylamino)ethyl; fluoro; chloro; phenyl; pyridyl; pyrazolyl; S(O₂)CH₃; and S(O₂)NR¹¹R¹².
306. The compound of any one of claims 88 and 300-305, wherein R¹ is selected from the group consisting of methyl; ethyl; difluoromethyl; 2-hydroxy-2-propyl; hydroxymethyl; 1,2-dihydroxy-2-propyl; (dimethylamino)methyl; (methylamino)methyl; and fluoro.
307. The compound of any one of claims 88 and 300-305, wherein R¹ is 2-hydroxy-2-propyl or 1,2-dihydroxy-2-propyl;.
308. The compound of any one of claims 88 and 300-305, wherein R¹ is fluoro.
309. The compound of any one of claims 88 and 300-308, wherein R^{2"} is F.
310. The compound of any one of claims 88 and 300-308, wherein R^{2"} is methyl.
311. The compound of any one of claims 88 and 300-310, wherein o=2 and p=0.
312. The compound of any one of claims 88 and 300-311, wherein the substituted ring B is 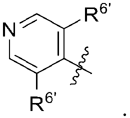 313. The compound of any one of claims 88 and 300-312, wherein each R^{6'} is independently selected from the group consisting of: unbranched C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the unbranched C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
314. The compound of any one of claims 88 and 300-313, wherein each R^{6'} is independently selected from the group consisting of: unbranched C₁-C₆ alkyl and C₃-C₇ cycloalkyl.
315. The compound of any one of claims 88 and 300-310, wherein o=2 and p=2.
316. The compound of any one of claims 88, 300-310, and 315, wherein the substituted ring B is 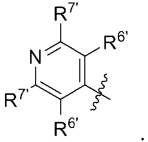 317. The compound of any one of claims 88, 300-310, and 315-316, wherein each R^{6'} is independently selected from the group consisting of: unbranched C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the unbranched C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
318. The compound of any one of claims 88, 300-310, and 315-317, wherein each R^{6'} is independently selected from the group consisting of: unbranched C₁-C₆ alkyl, C₃-C₇ cycloalkyl, halo, and C₆-C₁₀ aryl.
319. The compound of any one of claims 88, 300-310, and 315-317, wherein each R^{6'} is independently selected from the group consisting of: methyl, cyclopropyl, fluoro, and phenyl.
320. The compound of any one of claims 88, 300-310, and 315-319, wherein each R^{7'}, when present, is independently selected from the group consisting of: unbranched C₁-C₆ alkyl, C₃-C₇ cycloalkyl, C₁-C₆ haloalkyl, C₁-C₆ alkoxy, C₁-C₆ haloalkoxy, halo, CN, C₆-C₁₀ aryl, 5- to 10-membered heteroaryl, CO-C₁-C₆ alkyl; CONR⁸R⁹, and 4- to 6-membered heterocycloalkyl, wherein the unbranched C₁-C₆ alkyl, C₁-C₆ haloalkyl, C₃-C₇ cycloalkyl and 4- to 6-membered heterocycloalkyl is optionally substituted with one or more substituents each independently selected from hydroxy, halo, CN, oxo, C₁-C₆ alkoxy, NR⁸R⁹, =NR¹⁰, COOC₁-C₆ alkyl, CONR⁸R⁹, 4- to 6-membered heterocycloalkyl, C₆-C₁₀ aryl, 5- to 10-membered heteroaryl, OCOC₁-C₆ alkyl, OCOC₆-C₁₀ aryl, OCO(5- to 10-membered heteroaryl), OCO(4- to 6-membered heterocycloalkyl), NHCOC₁-C₆ alkyl, NHCOC₆-C₁₀ aryl, NHCO(5- to 10-membered heteroaryl), NHCO(4- to 6-membered heterocycloalkyl), and NHCOC₂-C₆ alkynyl.
321. The compound of any one of claims 88, 300-310, and 315-320, wherein each R^{7'}, when present, is independently selected from the group consisting of: unbranched C₁-C₆ alkyl, C₃-C₇ cycloalkyl, halo, and C₆-C₁₀ aryl.
322. The compound of any one of claims 88, 300-310, and 315-321, wherein each R^{7'}, when present, is independently selected from the group consisting of: methyl, cyclopropyl, fluoro, and phenyl.
323. The compound of any one of claims 88, 300-310, and 315-322, wherein one pair of R^{6'} and R^{7'} on adjacent atoms, taken together with the atoms connecting them, independently form at least one C₄-C₈ carbocyclic ring or at least one 5- to 8-membered heterocyclic ring containing 1 or 2 heteroatoms and/or heteroatomic groups independently selected from O, NH, NR¹³, S, S(O), and S(O)₂, wherein the carbocyclic ring or heterocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
324. The compound of any one of claims 88, 300-310, and 315-322, wherein one pair of R^{6'} and R^{7'} on adjacent atoms, taken together with the atoms connecting them, independently form at least one C₄-C₈ carbocyclic ring, wherein the carbocyclic ring is optionally independently substituted with one or more substituents independently selected from hydroxy, hydroxymethyl, halo, oxo, C₁-C₆ alkyl, C₁-C₆ alkoxy, NR⁸R⁹, CH₂NR⁸R⁹, =NR¹⁰, COOC₁-C₆ alkyl, C₆-C₁₀ aryl, and CONR⁸R⁹.
325. The compound of any one of claims 323-324, wherein the C₄-C₈ carbocyclic ring is a C₅ carbocyclic ring optionally substituted with one or more oxo, CH₃, or hydroxy.
326. The compound of claim 325, wherein the C₅ carbocyclic ring is substituted with one CH₃.
327. The compound of claim 325, wherein the C₅ carbocyclic ring is geminally substituted with two CH₃.
328. The compound of any one of claims 323-324, wherein the C₄-C₈ carbocyclic ring is a C₇ carbocyclic ring, wherein the C₇ carbocyclic ring is a bicyclic spirocycle, wherein the bicyclic spirocycle comprises a 5-membered ring and a 3-membered ring.
329. The compound of claim 300, wherein the substituted ring B" is 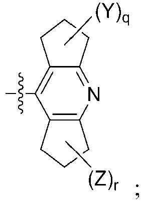 q is 0, 1, or 2; r is 0, 1, or 2; wherein each of Y and Z is independently selected from C₁-C₆ alkyl (e.g., methyl) and hydroxy; or wherein when two Y are attached to the same carbon, the two Y are taken together with the carbon they are attached to to form a cyclopropyl ring; or wherein when two Z are attached to the same carbon, the two Z are taken together with the carbon they are attached to to form a cyclopropyl ring.
330. The compound of claim 300, wherein the substituted ring B" is 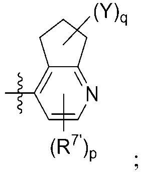 R^{7'} is selected from unbranched C₁-C₆ alkyl (e.g., methyl or ethyl), C₆-C₁₀ aryl (e.g., phenyl), and C₃-C₁₀ cycloalkyl (e.g., cyclopropyl); p is 0, 1, or 2; q is 0, 1, or 2; wherein each Y is independently selected from C₁-C₆ alkyl (e.g., methyl) and hydroxy; or when two Y are attached to the same carbon, the two Y are taken together with the carbon they are attached to to form a cyclopropyl ring.
331. A compound selected from the group consisting of the compounds below:

| **Cmpd #** | **Structure** | **Cmpd #** | **Structure** |
|---|---|---|---|
| **101** | 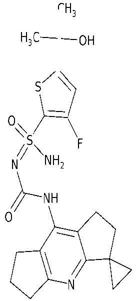 | **141aa** | 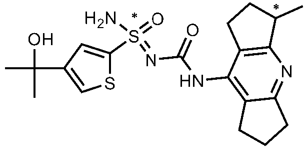 |
| **101a** | 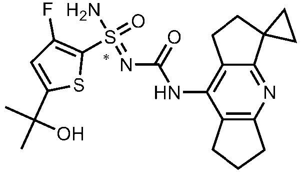 | **141ab** | 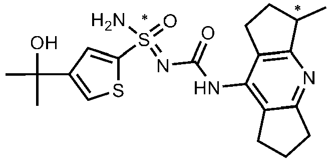 |
| **101b** | 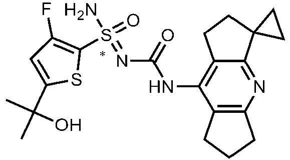 | **141b** | 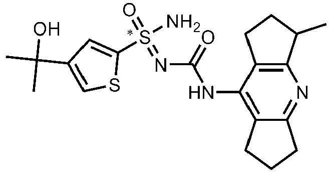 |
| **102** | 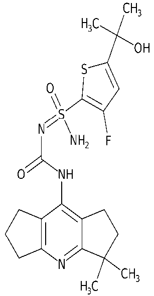 | **141ba** | 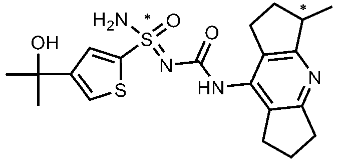 |
| **102a** | 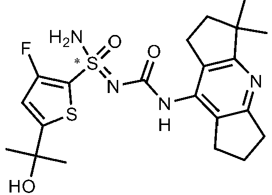 | **141bb** | 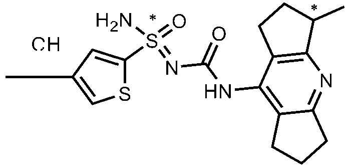 |
| **102b** | 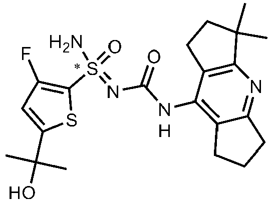 | **145** | 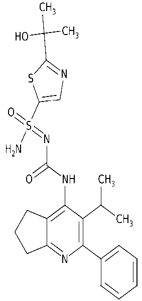 |
| **103** | 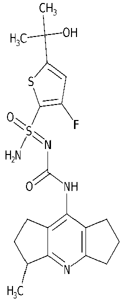 | **145a** | 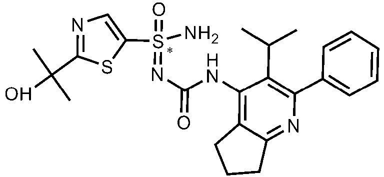 |
| **103aa** | 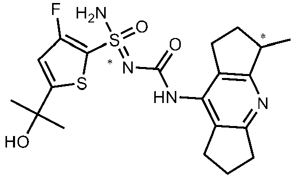 | **145b** | 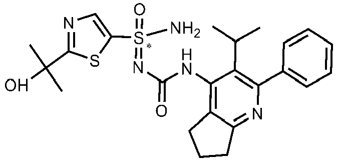 |
| **103ab** | 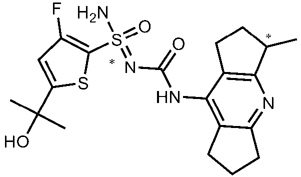 | **147** | 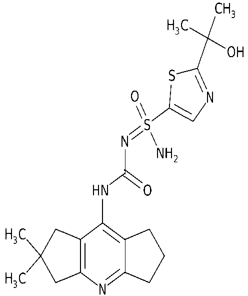 |
| **103ba** | 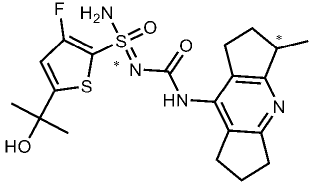 | **147b** | 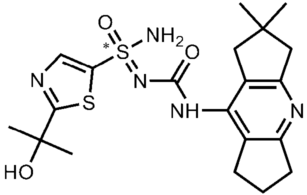 |
| **103bb** | 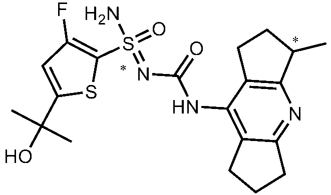 | **148** | 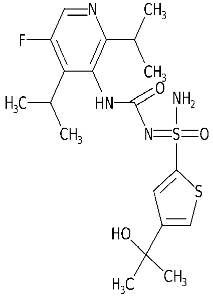 |
| **104** | 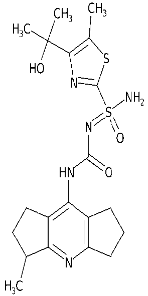 | **148a** | 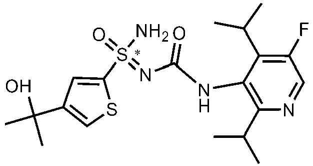 |
| **104a** | 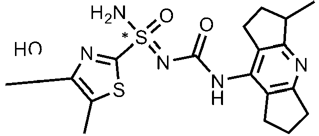 | **148b** | 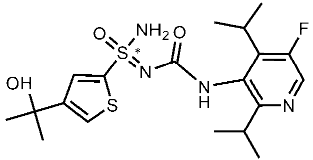 |
| **104b** | 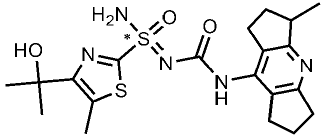 | **149** | 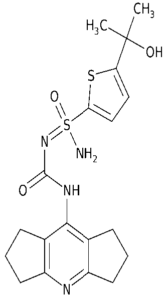 |
| **105** | 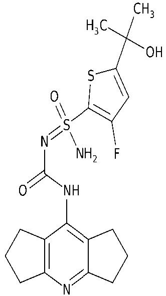 | **149a** | 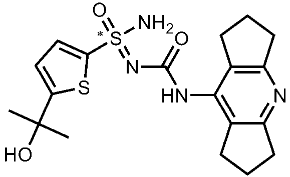 |
| **105a** | 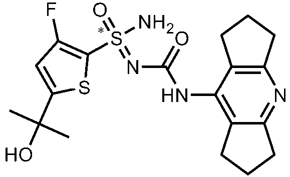 | **150** | 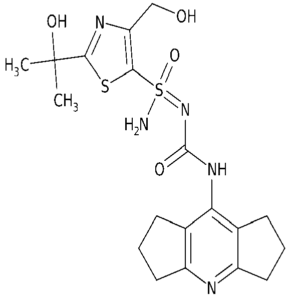 |
| **105b** | 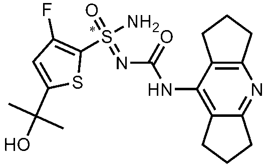 | **150a** | 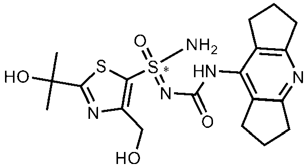 |
| **106** | 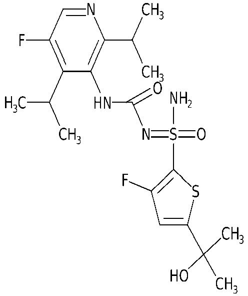 | **150b** | 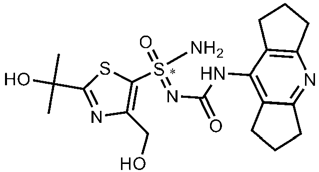 |
| **106a** | 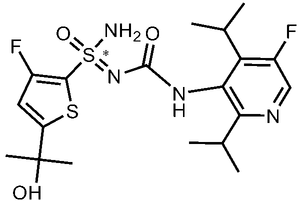 | **151** | 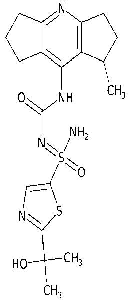 |
| **106b** | 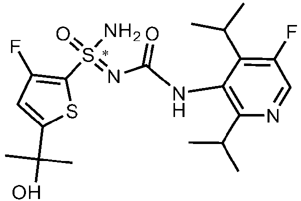 | **152** | 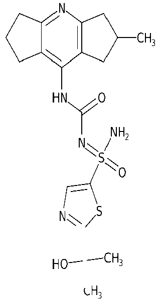 |
| **107** | 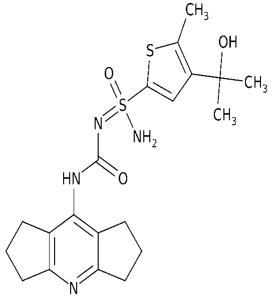 | **153** | 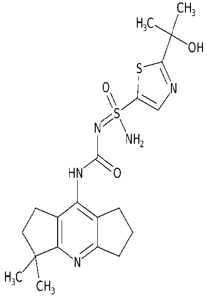 |
| **107a** | 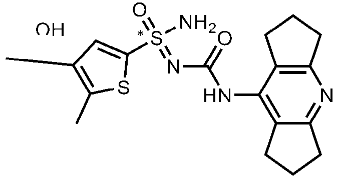 | **153a** | 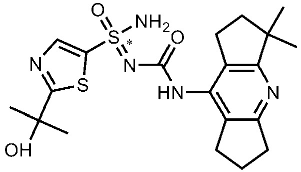 |
| **107b** | 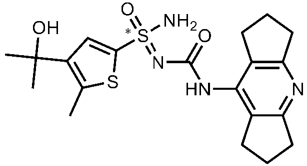 | **153b** | 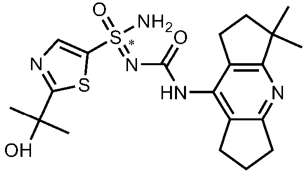 |
| **110** | 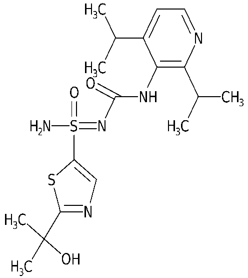 | **154** | 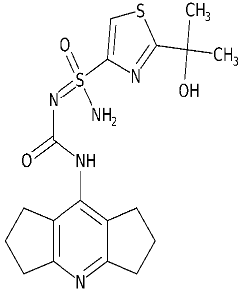 |
| **114** | 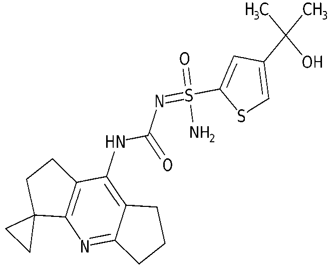 | **154a** | 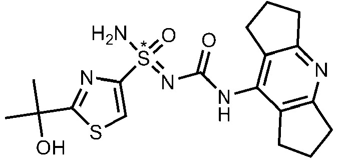 |
| **114a** | 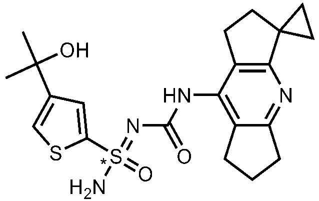 | **154b** | 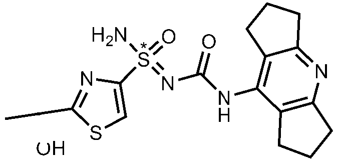 |
| **114b** | 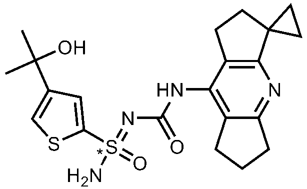 | **155** | 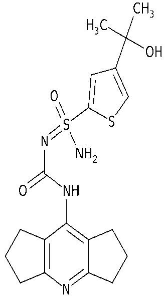 |
| **115** | 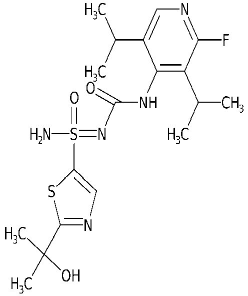 | **155a** | 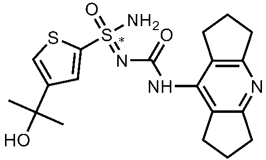 |
| **116** | 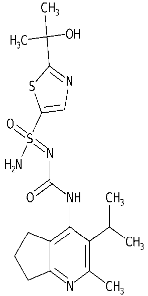 | **155b** | 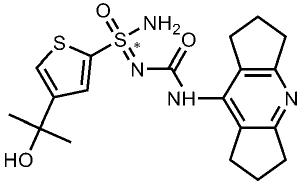 |
| **116a** | 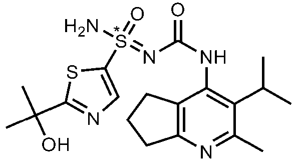 | **156b** | 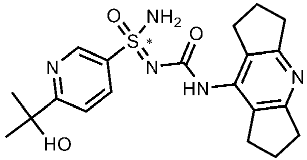 |
| **116b** | 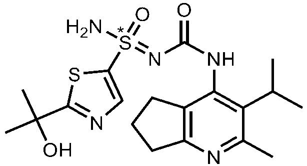 | **157b** | 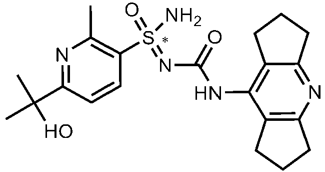 |
| **117** | 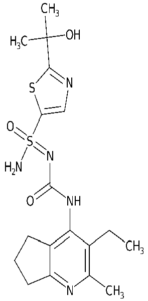 | **158** | 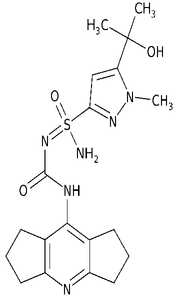 |
| **117a** | 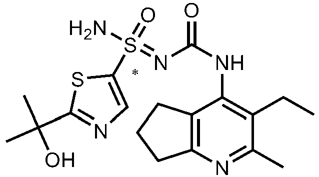 | **158a** | 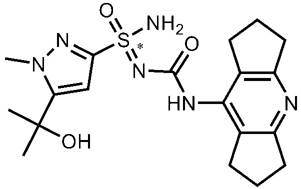 |
| **117b** | 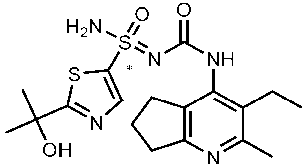 | **159** | 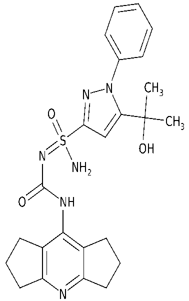 |
| **118** | 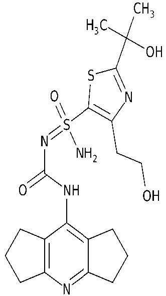 | **159a** | 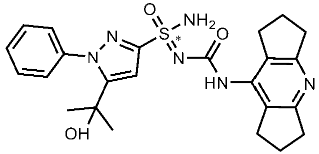 |
| **118a** | 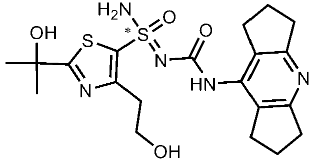 | **159b** | 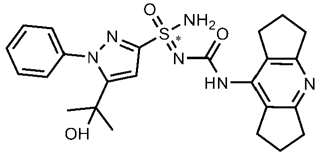 |
| **118b** | 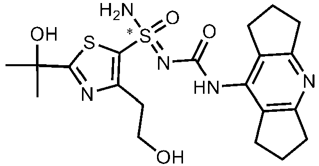 | **160** | 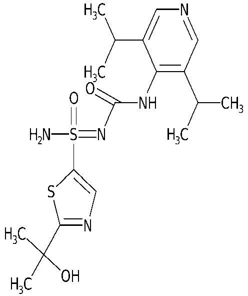 |
| **119** | 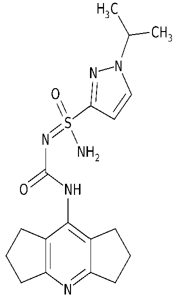 | **160a** | 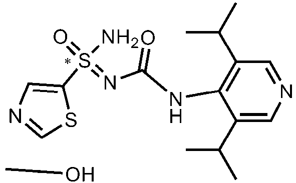 |
| **119a** | 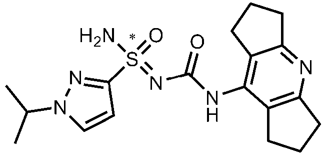 | **161** | 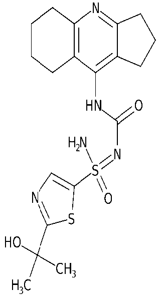 |
| **119b** | 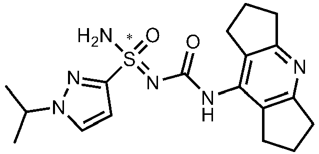 | **161a** | 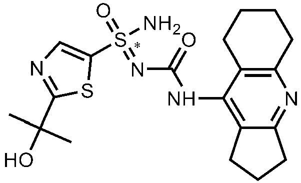 |
| **120** | 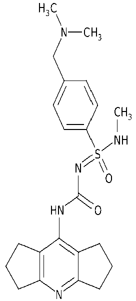 | **161b** | 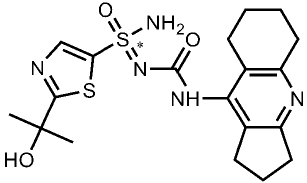 |
| **121** | 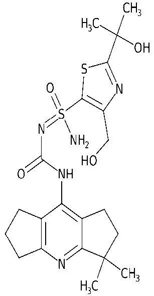 | **162** | 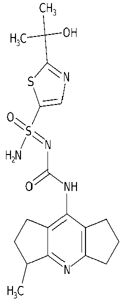 |
| **121a** | 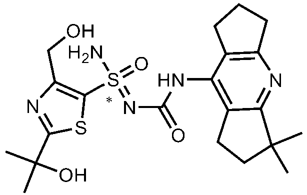 | **162aa** | 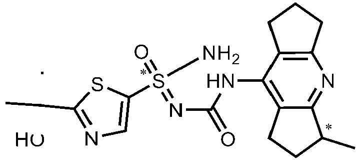 |
| **121b** | 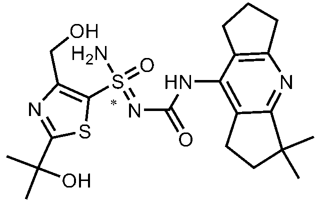 | **162ab** | 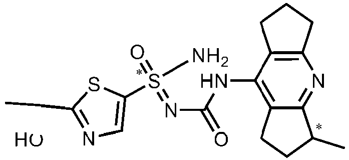 |
| **122** | 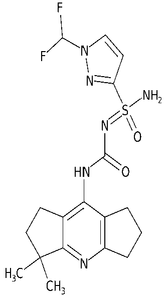 | **162ba** | 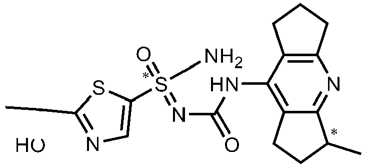 |
| **122a** | 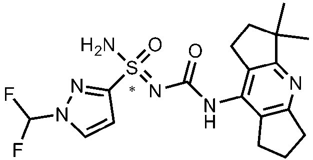 | **162bb** | 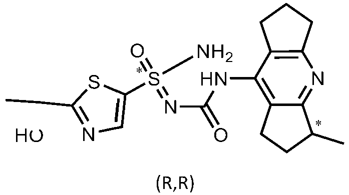 |
| **122b** | 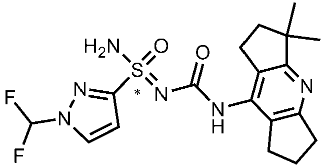 | **163** | 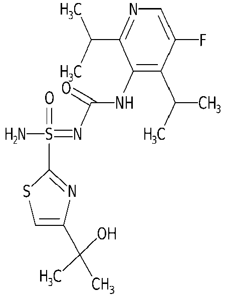 |
| **123** | 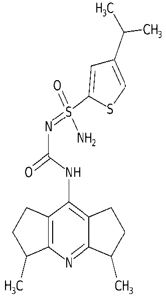 | **163a** | 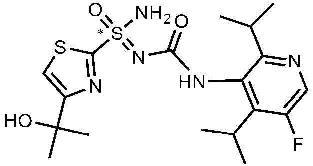 |
| **124** | 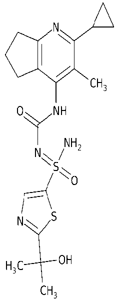 | **163b** | 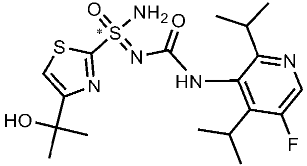 |
| **124a** | 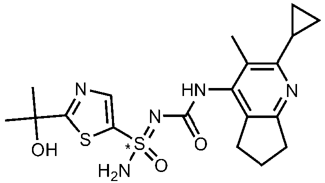 | **164** | 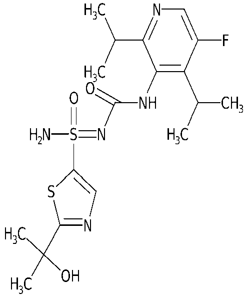 |
| **124b** | 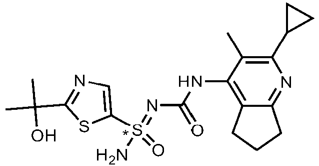 | **164a** | 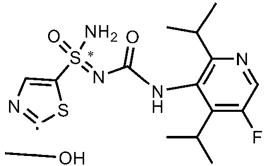 |
| **125** | 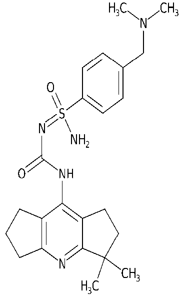 | **165** | 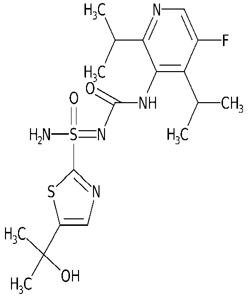 |
| **125a** | 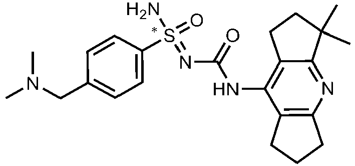 | **165a** | 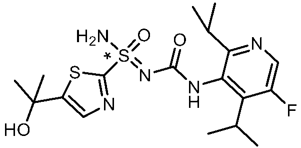 |
| **125b** | 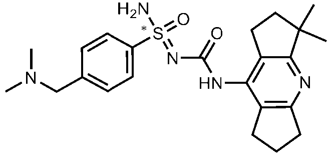 | **165b** | 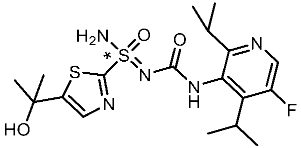 |
| **126** | 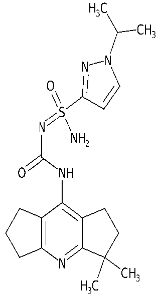 | **166** | 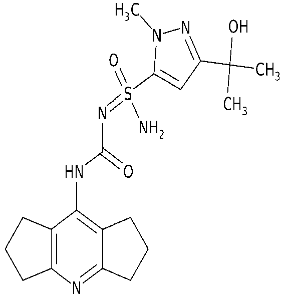 |
| **126a** | 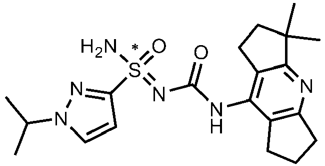 | **167** | 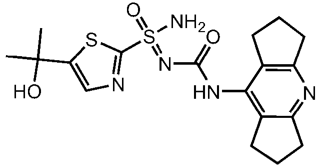 |
| **126b** | 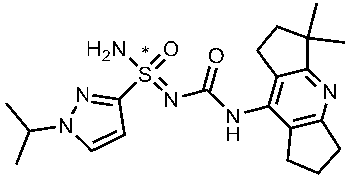 | **167a** | 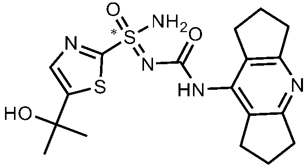 |
| **127** | 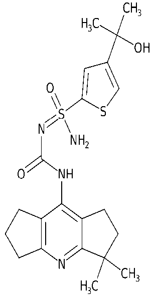 | **167b** | 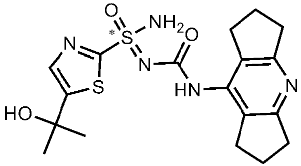 |
| **127a** | 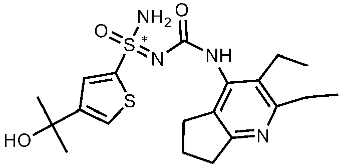 | **168** | 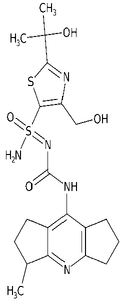 |
| **127b** | 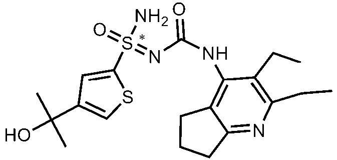 | **168a** | 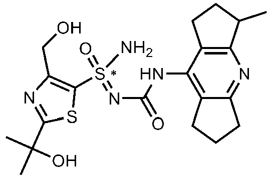 |
| **128** | 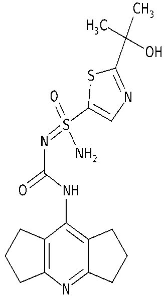 | **168aa** | 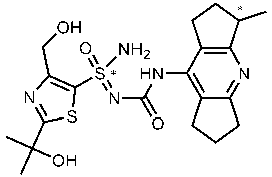 |
| **128a** | 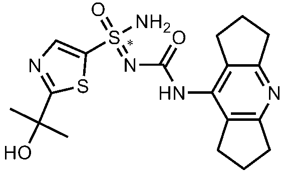 | **168ab** | 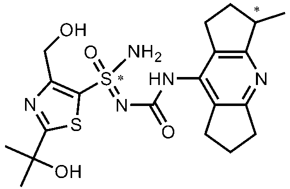 |
| **128b** | 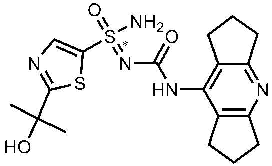 | **168b** | 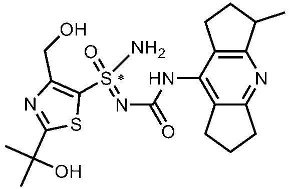 |
| **129** | 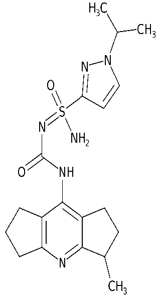 | **168ba** | 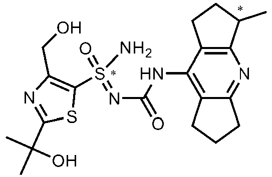 |
| **129aa** | 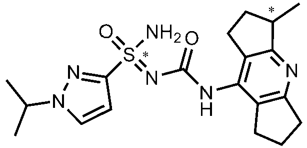 | **168bb** | 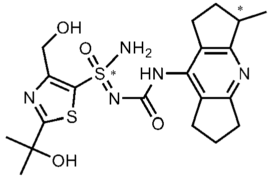 |
| **129ba** | 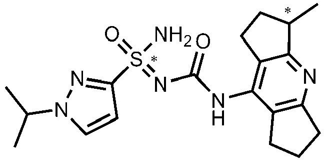 | **169a** | 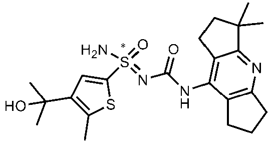 |
| **129bb** | 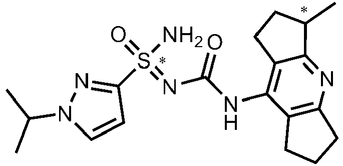 | **169b** | 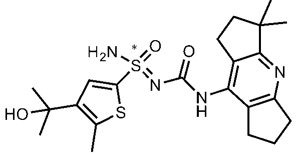 |
| **130** | 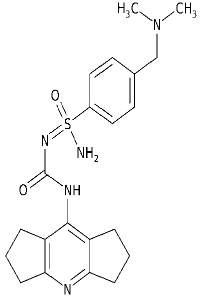 | **170** | 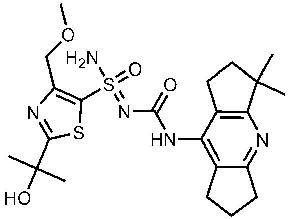 |
| **130b** | 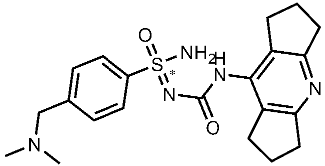 | **171** | 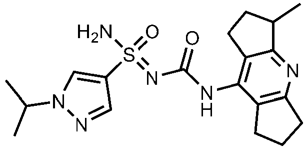 |
| **131** | 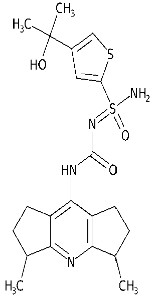 | **172** | 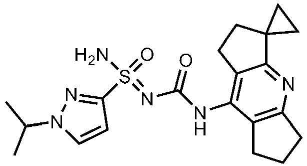 |
| **131a** | 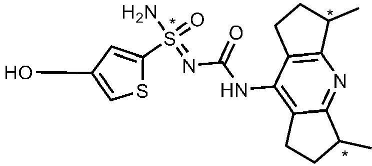 | **172a** | 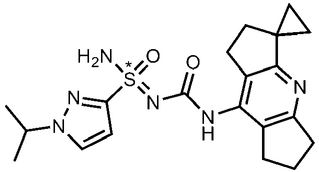 |
| **131aab** | 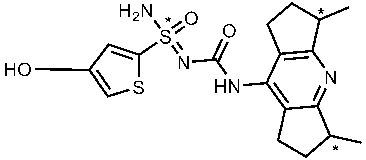 | **172b** | 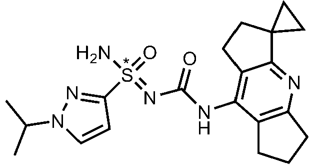 |
| **131b** | 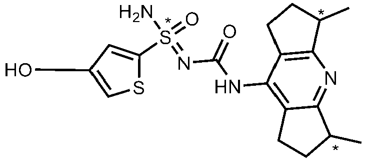 | **173** | 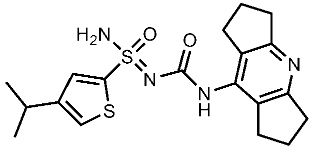 |
| **131c** | 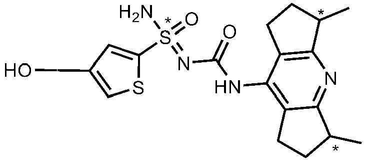 | **173a** | 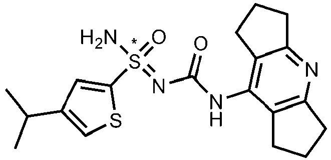 |
| **131d** | 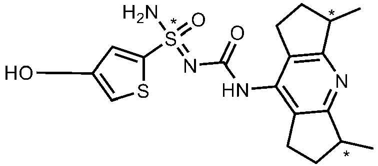 | **173b** | 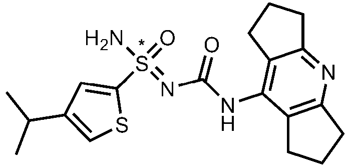 |
| **131e** | 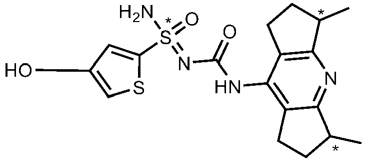 | **174** | 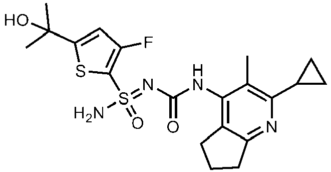 |
| **131f** | 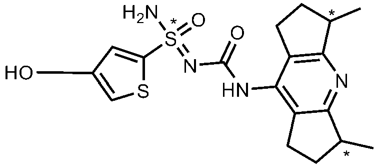 | **174a** | 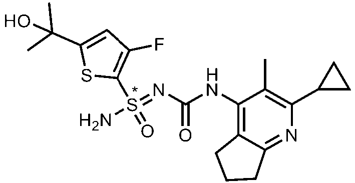 |
| **131g** | 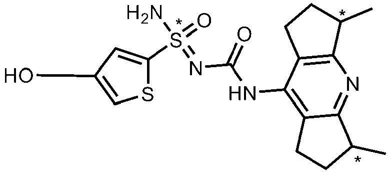 | **174b** | 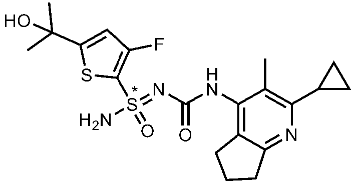 |
| **132b** | 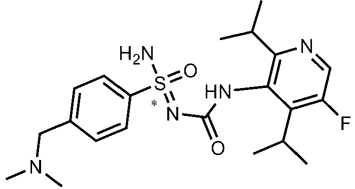 | **175** | 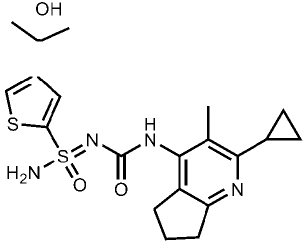 |
| **133** | 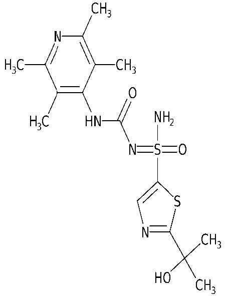 | **175a** | 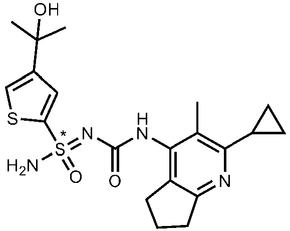 |
| **134** | 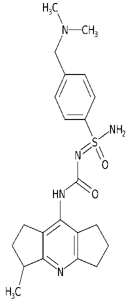 | **175b** | 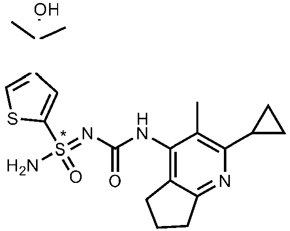 |
| **134aa** | 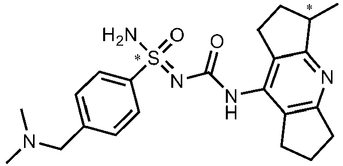 | **176** | 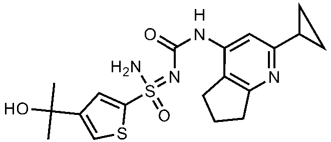 |
| **134ab** | 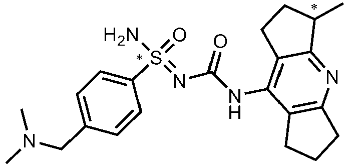 | **177** | 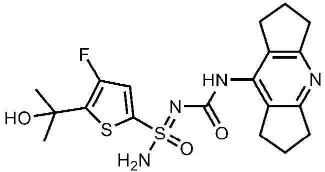 |
| **134ba** | 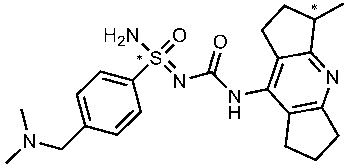 | **178** | 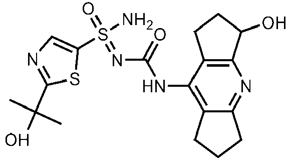 |
| **134bb** | 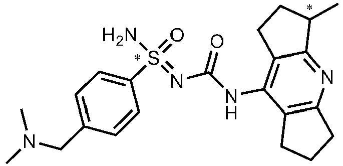 | **179** | 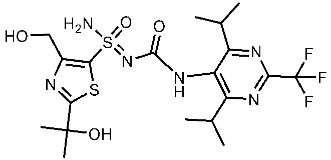 |
| **135** | 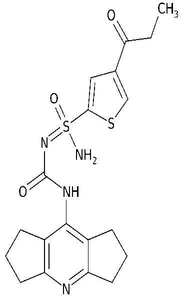 | **180** | 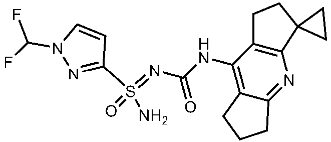 |
| **136** | 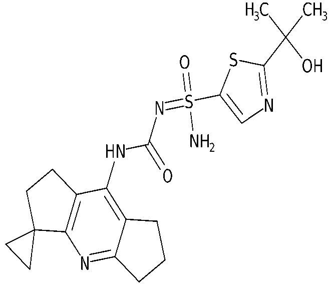 | **180a** | 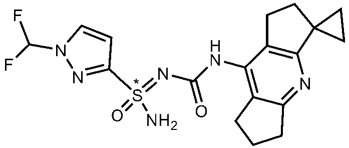 |
| **136a** | 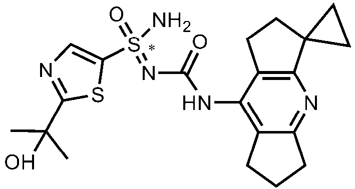 | **180b** | 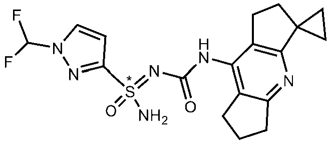 |
| **136b** | 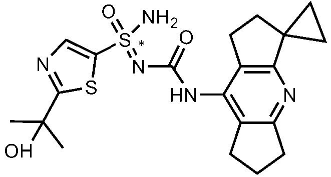 | **181** | 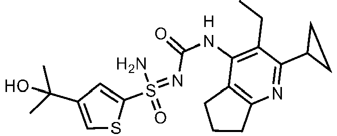 |
| **137a** | 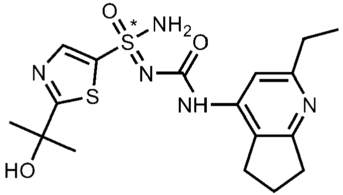 | **182** | 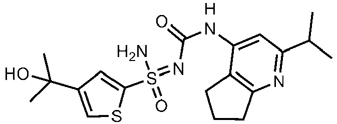 |
| **138** | 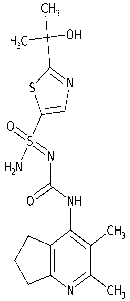 | **183** | 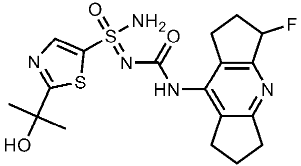 |
| **139** | 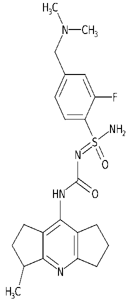 | **183a** | 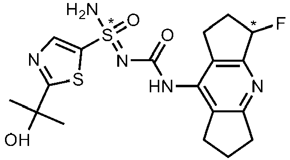 |
| **140** | 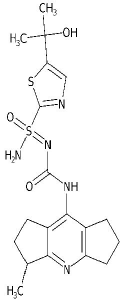 | **183b** | 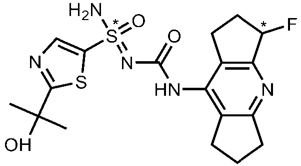 |
| **140a** | 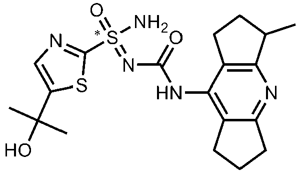 | **183c** | 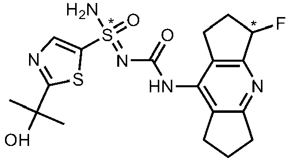 |
| **140aa** | 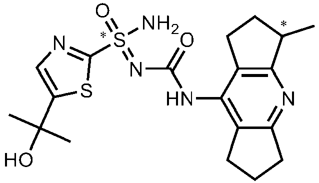 | **183d** | 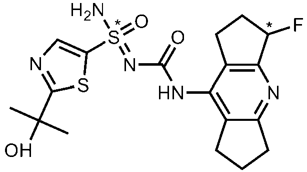 |
| **140ab** | 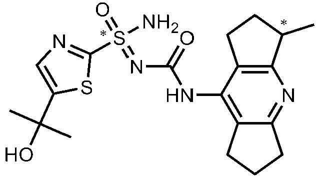 | **184a** | 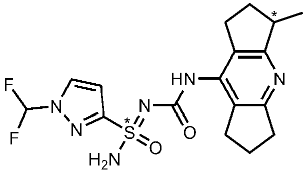 |
| **140b** | 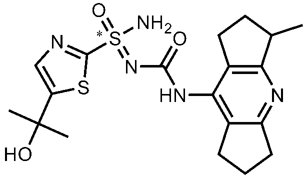 | **184b** | 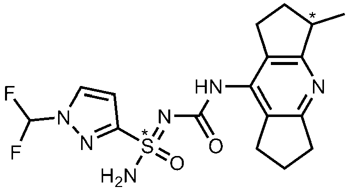 |
| **140ba** | 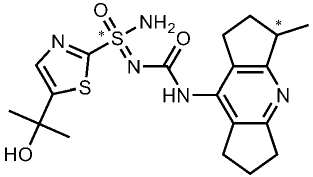 | **184c** | 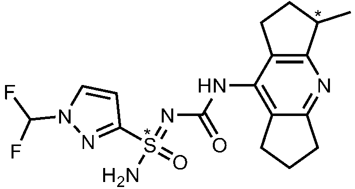 |
| **141** | 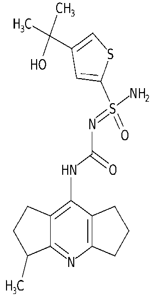 | **184d** | 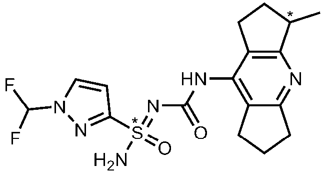 |
| **141a** | 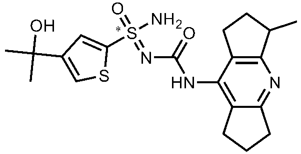 | **201** | 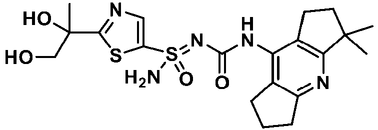 |
| | | **201a** | 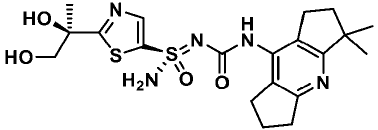 |

and pharmaceutically acceptable salts thereof.
332. A compound selected from the group consisting of the compounds in **Table 1C** and pharmaceutically acceptable salts thereof.
333. A compound selected from the group consisting of the compounds in **Table 1D** and pharmaceutically acceptable salts thereof.
334. A compound selected from the group consisting of the compounds in **Table 1E** or **Table 1F** (e.g., **Table 1E;** e.g., **Table 1F**) and pharmaceutically acceptable salts thereof.
335. The compound of any one of claims 1-330, wherein the sulfur in the moiety S(=O)(NHR³)=N- has (S) stereochemistry.
336. The compound of any one of claims 1-330, wherein the sulfur in the moiety S(=O)(NHR³)=N- has (R) stereochemistry.
337. A pharmaceutical composition comprising a compound or salt as claimed in any one of claims 1-336 and one or more pharmaceutically acceptable excipients.
338. A method for modulating NLRP3 activity, the method comprising contacting NLRP3 with an effective amount of a compound as claimed in any one of claims 1-336 or a pharmaceutical composition as claimed in claim 337.
339. The method of claim 338, wherein the modulating comprises antagonizing NLRP3.
340. The method of any one of claims 338-339, which is carried out *in vitro.*
341. The method of claim 338-340, wherein the method comprises contacting a sample comprising one or more cells comprising NLRP3 with the compound.
342. The method of any one of claims 338-339 and 341, which is carried out *in vivo.*
343. The method of claim 342, wherein the method comprises administering the compound to a subject having a disease in which NLRP3 signaling contributes to the pathology and/or symptoms and/or progression of the disease.
344. The method of claim 343, wherein the subject is a human.
345. A method of treating a disease, disorder or condition that is a metabolic disorder, comprising administering to a subject in need of such treatment an effective amount of a compound as claimed in any one of claims 1-336 or a pharmaceutical composition as claimed in claim 337.
346. The method of claim 345, wherein the metabolic disorder is Type 2 diabetes, atherosclerosis, obesity or gout.
347. A method of treating a disease, disorder or condition that is a disease of the central nervous system, comprising administering to a subject in need of such treatment an effective amount of a compound as claimed in any one of claims 1-336 or a pharmaceutical composition as claimed in claim 337.
348. The method of claim 347, wherein the disease of the central nervous system is Alzheimer's disease, multiple sclerosis, Amyotrophic Lateral Sclerosis or Parkinson's disease.
349. A method of treating a disease, disorder or condition that is lung disease, comprising administering to a subject in need of such treatment an effective amount of a compound as claimed in any one of claims 1-336 or a pharmaceutical composition as claimed in claim 337.
350. The method of claim 349, wherein the lung disease is asthma, COPD or pulmonary idiopathic fibrosis.
351. A method of treating a disease, disorder or condition that is liver disease, comprising administering to a subject in need of such treatment an effective amount of a compound as claimed in any one of claims 1-336 or a pharmaceutical composition as claimed in claim 337.
352. The method of claim 351, wherein the liver disease is NASH syndrome, viral hepatitis or cirrhosis.
353. A method of treating a disease, disorder or condition that is pancreatic disease, comprising administering to a subject in need of such treatment an effective amount of a compound as claimed in any one of claims 1-336 or a pharmaceutical composition as claimed in claim 337.
354. The method of claim 353, wherein the pancreatic disease is acute pancreatitis or chronic pancreatitis.
355. A method of treating a disease, disorder or condition that is kidney disease, comprising administering to a subject in need of such treatment an effective amount of a compound as claimed in any one of claims 1-336 or a pharmaceutical composition as claimed in claim 337.
356. The method of claim 355, wherein the kidney disease is acute kidney injury or chronic kidney injury.
357. A method of treating a disease, disorder or condition that is intestinal disease, comprising administering to a subject in need of such treatment an effective amount of a compound as claimed in any one of claims 1-336 or a pharmaceutical composition as claimed in claim 337.
358. The method of claim 357, wherein the intestinal disease is Crohn's disease or Ulcerative Colitis.
359. A method of treating a disease, disorder or condition that is skin disease, comprising administering to a subject in need of such treatment an effective amount of a compound as claimed in any one of claims 1-336 or a pharmaceutical composition as claimed in claim 337.
360. The method of claim 359, wherein the skin disease is psoriasis.
361. A method of treating a disease, disorder or condition that is musculoskeletal disease, comprising administering to a subject in need of such treatment an effective amount of a compound as claimed in any one of claims 1-336 or a pharmaceutical composition as claimed in claim 337.
362. The method of claim 361, wherein the musculoskeletal disease is scleroderma.
363. A method of treating a disease, disorder or condition that is a vessel disorder, comprising administering to a subject in need of such treatment an effective amount of a compound as claimed in any one of claims 1-336 or a pharmaceutical composition as claimed in claim 337.
364. The method of claim 363, wherein the vessel disorder is giant cell arteritis.
365. A method of treating a disease, disorder or condition that is a disorder of the bones, comprising administering to a subject in need of such treatment an effective amount of a compound as claimed in any one of claims 1-336 or a pharmaceutical composition as claimed in claim 337.
366. The method of claim 365, wherein the disorder of the bones is osteoarthritis, osteoporosis or osteopetrosis disorders.
367. A method of treating a disease, disorder or condition that is eye disease, comprising administering to a subject in need of such treatment an effective amount of a compound as claimed in any one of claims 1-336 or a pharmaceutical composition as claimed in claim 337.
368. The method of claim 367, wherein the eye disease is glaucoma or macular degeneration.
369. A method of treating a disease, disorder or condition that is a disease caused by viral infection, comprising administering to a subject in need of such treatment an effective amount of a compound as claimed in any one of claims 1-336 or a pharmaceutical composition as claimed in claim 337.
370. The method of claim 369, wherein the diseases caused by viral infection is HIV or AIDS.
371. A method of treating a disease, disorder or condition that is an autoimmune disease, comprising administering to a subject in need of such treatment an effective amount of a compound as claimed in any one of claims 1-336 or a pharmaceutical composition as claimed in claim 337.
372. The method of claim 371, wherein the autoimmune disease is Rheumatoid Arthritis, Systemic Lupus Erythematosus, Autoimmune Thyroiditis, , Addison's disease, pernicious anemia, cancer and aging.
373. A method of treating a disease, disorder or condition that is cancer or aging, comprising administering to a subject in need of such treatment an effective amount of a compound as claimed in any one of claims 1-336 or a pharmaceutical composition as claimed in claim 337.
374. A method of treating a disease, disorder or condition that is a cancer selected from: myelodysplastic syndromes (MDS); non-small cell lung cancer, such as non-small cell lung cancer in patients carrying mutation or overexpression of NLRP3; acute lymphoblastic leukemia (ALL), such as ALL in patients resistant to glucocorticoids treatment; Langerhan's cell histiocytosis (LCH); multiple myeloma; promyelocytic leukemia; acute myeloid leukemia (AML); chronic myeloid leukemia (CML); gastric cancer; and lung cancer metastasis, comprising administering to a subject in need of such treatment an effective amount of a compound as claimed in any one of claims 1-336 or a pharmaceutical composition as claimed in claim 337.
375. The method of claim 374, wherein the cancer is MDS.
376. The method of claim 374, wherein the cancer is non-small lung cancer.
377. The method of claim 374, wherein the cancer is acute lymphoblastic leukemia.
378. The method of claim 374, wherein the cancer is LCH.
379. The method of claim 374, wherein the cancer is multiple myeloma.
380. The method of claim 374, wherein the cancer is promyelocytic leukemia.
381. The method of claim 374, wherein the cancer is acute myeloid leukemia (AML).
382. The method of claim 374, wherein the cancer is chronic myeloid leukemia (CML).
383. The method of claim 374, wherein the cancer is gastric cancer.
384. The method of claim 374, wherein the cancer is lung cancer metastasis.
385. The method of any one of claims 338-384, further comprising administering a therapeutically effective amount of an anti-TNFα agent to the subject.
386. The method of claim 385, wherein the NLRP3 antagonist is administered to the subject prior to administration of the anti-TNFα agent to the subject.
387. The method of claim 385, wherein the anti-TNFα agent is administered to the subject prior to the administration of the NLRP3 antagonist to the subject.
388. The method of claim 385, wherein the NLRP3 antagonist and the anti-TNFα agent are administered to the subject at substantially the same time.
389. The method of claim 385, wherein the NLRP3 antagonist and the anti-TNFα agent are formulated together in a single dosage form.
390. The compound of claim 1, wherein
m = 0, 1, or 2;
n = 0, 1, or 2;
o = 1 or 2;
p = 0, 1, 2, or 3; wherein the sum of o and p is from 1 to 4;
wherein
A is a pyrazolyl,
B is a pyridinyl.
391. The compound of claim 390, wherein A is pyrazolyl optionally substituted with 1 or 2 R1 and optionally substituted with 1 or 2 R2.
392. The compound of claim 390, wherein A is pyrazolyl optionally substituted with 1 R1 and optionally substituted with 1 or 2 R2.
393. The compound of claim 390, wherein A is pyrazolyl optionally substituted with 1 or 2 R1 and optionally substituted with 1 R2.
394. The compound of any one of claims 390 to 393, wherein B is a pyridin-4-yl.
395. The compound of any one of claims 390 to 394, wherein B is 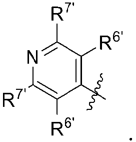 396. The compoudn of any one of claims 390 to 395, wherein B is 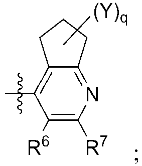 R⁶ is selected from C₁-C₆ alkyl (e.g., methyl, ethyl, or isopropyl) and C₃-C₁₀ cycloalkyl (e.g., cyclopropyl); R⁷ is selected from C₁-C₆ alkyl (e.g., methyl, ethyl, or isopropyl), C₁-C₆ haloalkyl (e.g., trifluoromethyl) and C₃-C₁₀ cycloalkyl (e.g., cyclopropyl or cyclobutyl); or R⁶ and R⁷, taken together with the atoms connecting them, independently form a C₅ carbocyclic ring optionally substituted with one or more C₁-C₆ alkyl (e.g., methyl); q is 0, 1, or 2; each Y is independently selected from C₁-C₆ alkyl (e.g., methyl); or when two Y are attached to the same carbon, the two Y are taken together with the carbon they are attached to to form a cyclopropyl ring.

## Claims

1. A compound selected from the group consisting of: 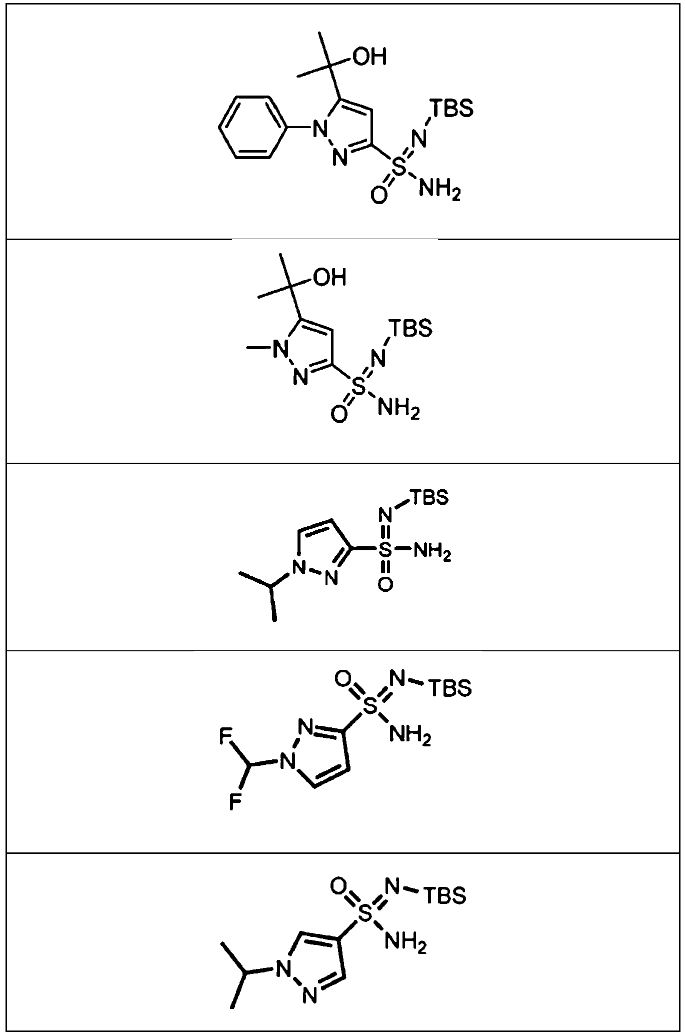 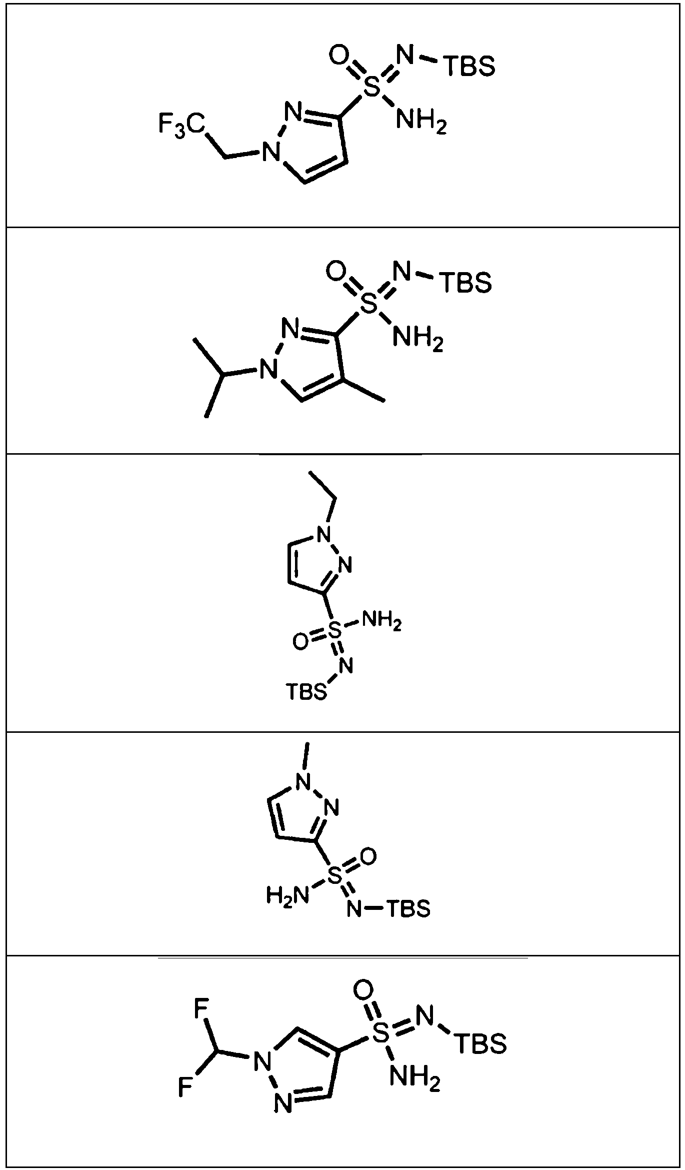 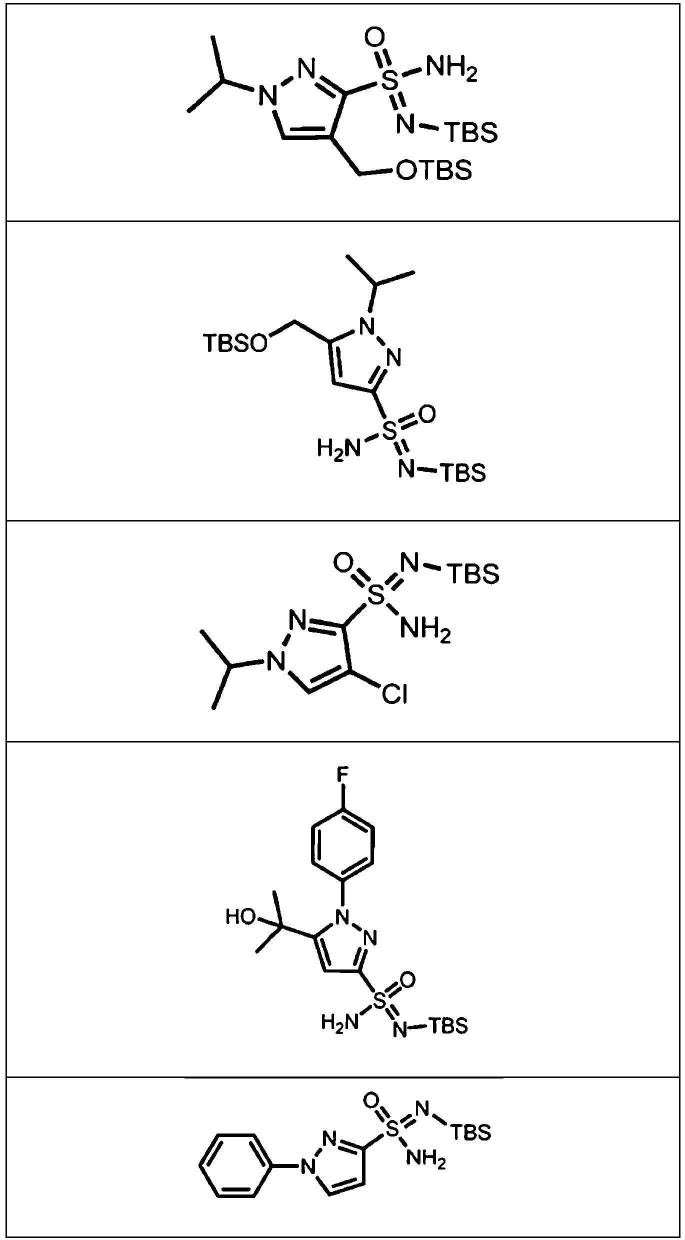 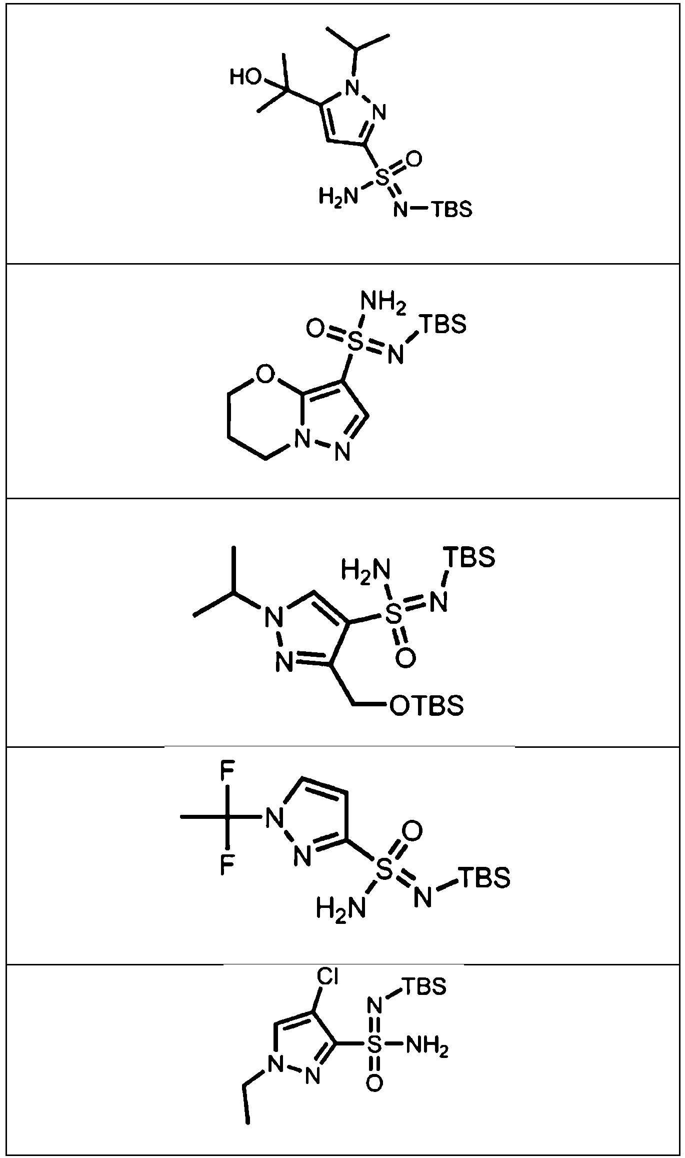 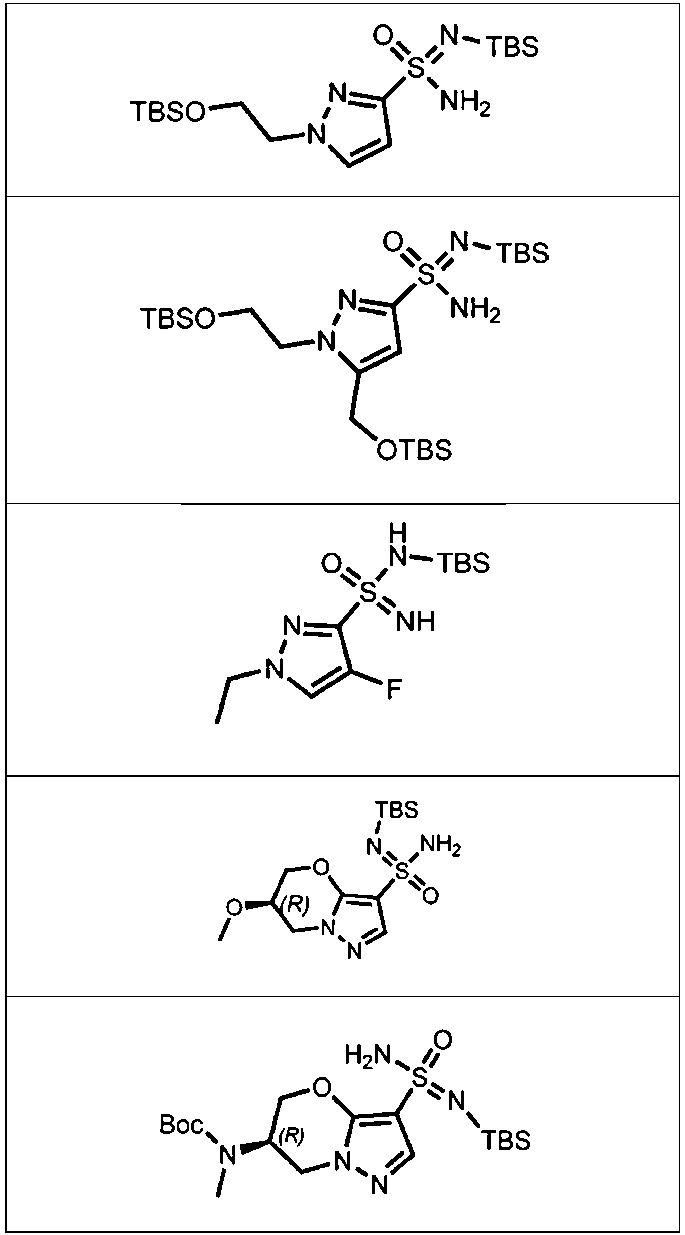 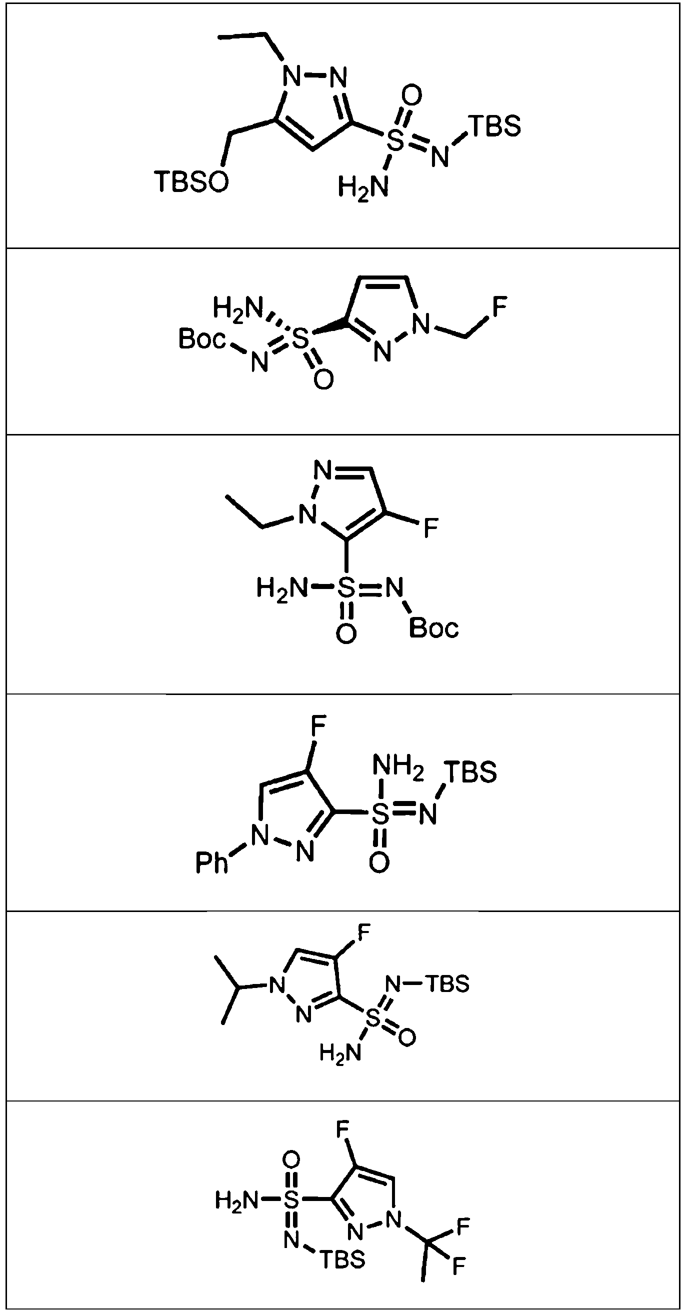 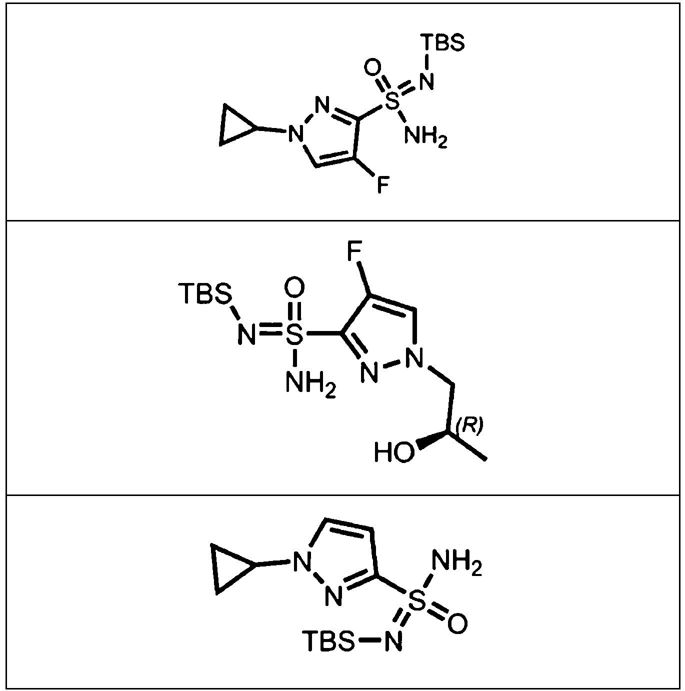 wherein TBS is tert-butyldimethylsilyl and Boc is *tert*-butyloxy carbonyl,
or a pharmaceutically acceptable salt thereof.

2. The compound of claim 1, selected from the group consisting of: 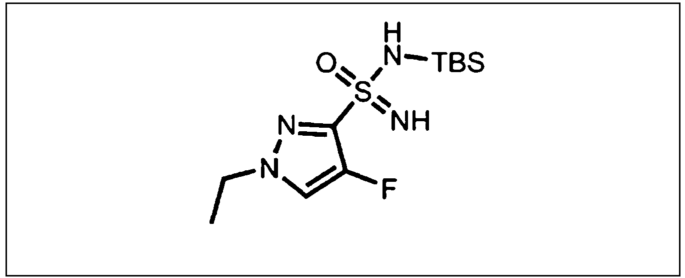 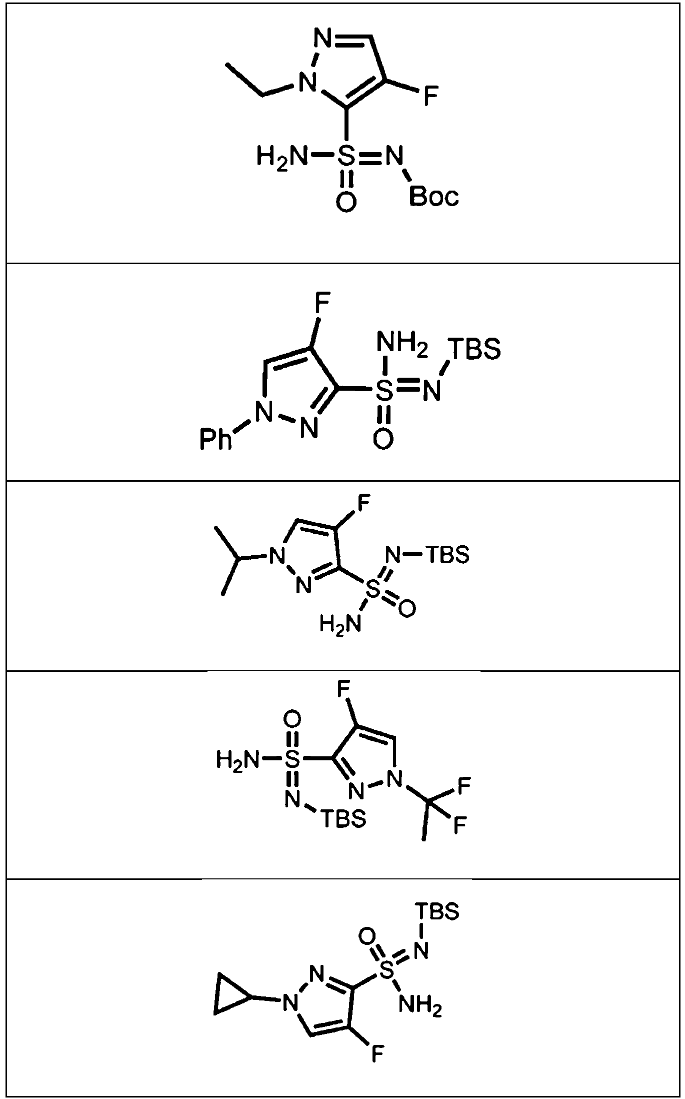 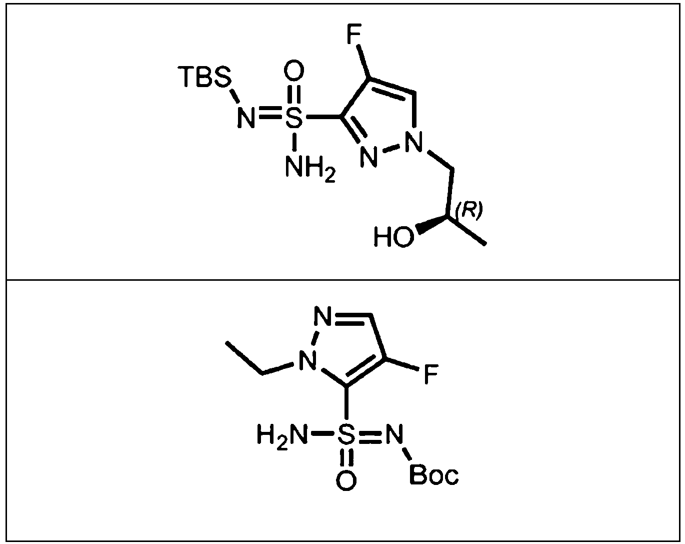 or a pharmaceutically acceptable salt thereof.

3. The compound of claim 1 or 2, selected from the group consisting of: 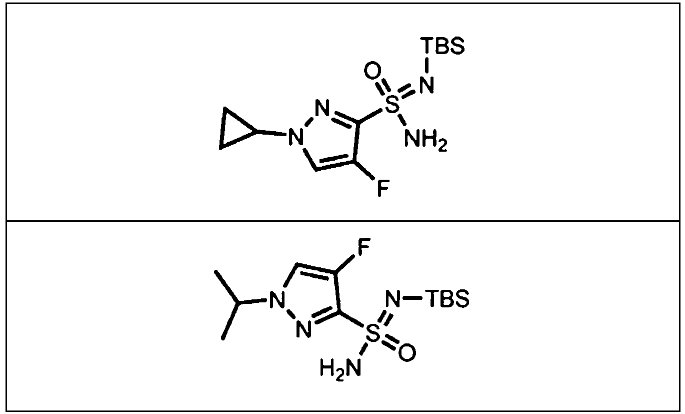 or a pharmaceutically acceptable salt thereof.
